# EUROPEAN PATENT SPECIFICATION

(11) **EP 2 422 201 B1**
(45) Date of publication and mention of the grant of the patent: **22.04.2015**
(21) Application number: 10766481.5
(22) Date of filing: 09.03.2010
(51) Int. Cl.: G01N 33/68, C40B 30/04, G06F 17/50, C07K 14/72, G01N 23/20, G06Q 50/00

(54) **STRUCTURE OF THE C-TERMINAL REGION OF THE INSULIN RECEPTOR ALPHA -CHAIN AND OF THE INSULIN-LIKE GROWTH FACTOR RECEPTOR ALPHA -CHAIN**
STRUKTUR DER C-ENDREGION DER INSULINREZEPTOR-ALPHA-KETTE UND DER ALPHA-KETTE DES INSULINÄHNLICHEN WACHSTUMSFAKTORS
STRUCTURE DE LA REGION C TERMINALE DE LA CHAÎNE ALPHA DU RECEPTEUR D'INSULINE ET DE LA CHAÎNE ALPHA DU RECEPTEUR DE FACTEUR DE CROISSANCE DE TYPE INSULINE

(30) Priority: 22.04.2009 US 214472 P
(43) Date of publication of application: 29.02.2012
(73) Proprietor: The Walter and Eliza Hall Institute of Medical Research, Melbourne, VIC 3052 (AU)
(72) Inventor: LAWRENCE, Michael, Colin, Melbourne, Victoria 3052 (AU); SMITH, Brian, John, Melbourne, Victoria 3052 (AU); MENTING, John, Gerbrandt, Tasman, Melbourne, Victoria 3052 (AU); WARD, Colin, Wesley, Melbourne, Victoria 3052 (AU)
(74) Representative: Sweetinburgh, Mark Roger
(86) International application number: PCT/AU2010/000271
(87) International publication number: WO 2010/121288

(56) References cited:
- EP-B1- 0 957 164
- WO-A1-99/28347
- WO-A1-2007/147213
- WO-A2-99/42127
- TAKESHI KUROSE ET AL: "Cross-linking of a B25 Azidophenylalanine Insulin Derivative to the Carboxyl-terminal Region of the a-Subunit of the Insulin Receptor", JOURNAL OF BIOLOGICAL CHEMISTRY, vol. 269, no. 46, 18 November 2004 (2004-11-18), pages 29190-29197, XP55043668,
- COLIN W. WARD ET AL: "Ligand-induced activation of the insulin receptor: a multi-step process involving structural changes in both the ligand and the receptor", BIOESSAYS, vol. 31, no. 4, 1 April 2009 (2009-04-01), pages 422-434, XP55043655, ISSN: 0265-9247, DOI: 10.1002/bies.200800210
- WHITTEN A E ET AL: "Solution Structure of Ectodomains of the Insulin Receptor Family: The Ectodomain of the Type 1 Insulin-Like Growth Factor Receptor Displays Asymmetry of Ligand Binding Accompanied by Limited Conformational Change", JOURNAL OF MOLECULAR BIOLOGY, ACADEMIC PRESS, UNITED KINGDOM, vol. 394, no. 5, 18 December 2009 (2009-12-18), pages 878-892, XP026802527, ISSN: 0022-2836, DOI: 10.1016/J.JMB.2009.10.011 [retrieved on 2009-10-14]
- JOHN G. MENTING ET AL: "A Thermodynamic Study of Ligand Binding to the First Three Domains of the Human Insulin Receptor: Relationship between the Receptor [alpha]-Chain C-Terminal Peptide and the Site 1 Insulin Mimetic Peptides", BIOCHEMISTRY, vol. 48, no. 23, 16 June 2009 (2009-06-16) , pages 5492-5500, XP55043611, ISSN: 0006-2960, DOI: 10.1021/bi900261q
- MENTING, J. ET AL.: 'A Thermodynamic Study of Ligand Binding to the First Three Domains of the Human Insulin Receptor: Relationship between the Receptor a-Chain C-Terminal Peptide and the Site 1 Insulin Mimetic Peptides' BIOCHEMISTRY vol. 48, no. 23, 2009, pages 5492 - 5500, XP055043611
- MCKERN, N. M. ET AL.: 'Structure of the Insulin Receptor Ectodomain Reveals a Folded-Over Conformation' NATURE vol. 443, 2006, pages 218 - 221, XP008102956
- LOU, M. ET AL.: 'The First Three Domains of the Insulin Receptor Differ Structurally from the Insulin-Like Growth Factor 1 Receptor in the Regions Governing Ligand Specificity' PROC. NATL. ACAD. SCI. USA vol. 103, no. 33, 2006, pages 12429 - 12434, XP008102973
- MUNSHI, S. ET AL.: 'Crystal Structure of the Apo, Unactivated Insulin-Like Growth Factor-I Receptor Kinase. Implication for Inhibitor Specificity' J. BIOL. CHEM. vol. 277, no. 41, 2002, pages 38797 - 38802, XP055043633

## Description

### Field of the Invention

The present invention relates generally to structural studies of the insulin binding site of the insulin receptor (IR) and the insulin-like growth factor 1 receptor (IGF-1R). More particularly, the present invention relates to methods of using the crystal structure of the low affinity insulin binding site of the IR ectodomain comprising the C-terminal region of the IR α-chain, as well as the corresponding region of IGF-1R, and related structural information to screen for and design compounds that interact with or modulate the function of IR and/or IGF-1R.

### Background to the Invention

The insulin receptor (IR) and its homologue the type 1 insulin-like growth factor 1 receptor (IGF-1R), are closely related members of the tyrosine kinase receptor family and are large, transmembrane, glycoprotein dimers consisting of several structural domains.

The key role of the insulin receptor (IR) is in glucose uptake and metabolism by muscle and fat. Mouse knockout studies have also shown IR to be important in adipogenesis, neovascularization, the regulation of hepatic glucose synthesis and glucose-induced pancreatic insulin secretion (Kitamura *et al.,* 2003). IR signalling is also important in the brain, being involved in the regulation of food intake, peripheral fat deposition and the reproductive endocrine axis as well as in learning and memory (Wada *et al.,* 2005). Dysfunctional IR signalling has been implicated in diseases including type I and type II diabetes, dementia and cancer.

IR exists as two splice variant isoforms, IR-A and IR-B, which respectively lack or contain the 12 amino acids coded by exon 11. The longer variant, IR-B, is the isoform responsible for signalling metabolic responses. In contrast, IR-A signals predominantly mitogenic responses, is the preferentially expressed isoform in several cancers (Denley *et al.,* 2003) and is capable of binding insulin-like growth factor 2 (IGF-11) with high affinity (Denley *et al.,* 2004).

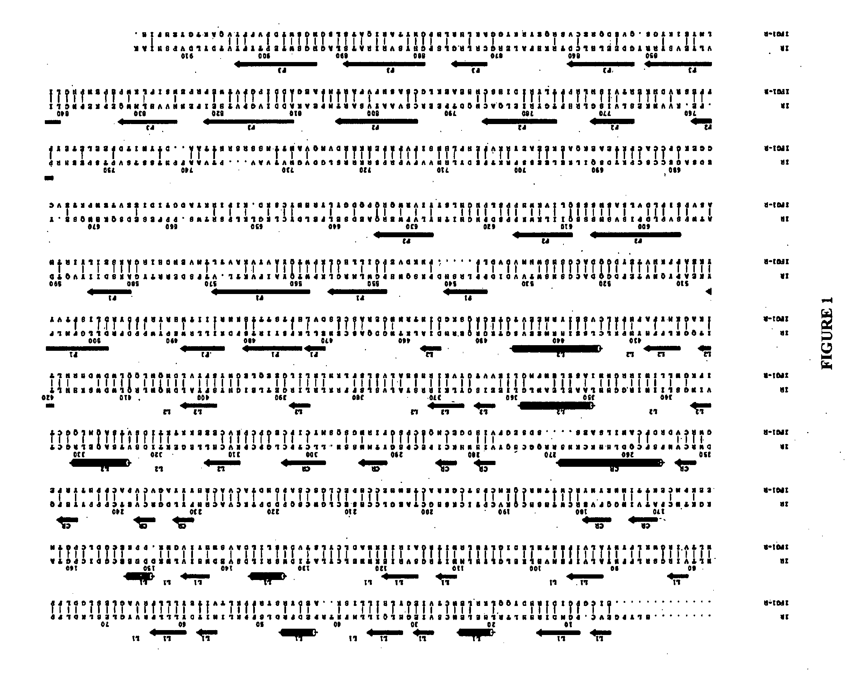

The sequence of IR is highly homologous to the sequence of IGF-1R, indicating that the three-dimensional structures of both receptors are most likely closely similar. The mature human IR and IGF-1R molecules are each homodimers comprising two α-chains and two β-chains, the α- and β-chains arising from the post-translational cleavage at the furin cleavage site at residues 720-723 (IR-A numbering with the mature N-terminal residue numbered 1) or 707-710 (IGF-1R). The structural organization of IR and IGF-1R has been reviewed extensively (Adams *et al.,* 2000; De Meyts and Whittaker, 2002; Ward *et al.*, 2003; Lawrence *et al.,* 2007; Ward and Lawrence, 2009). The sequence relationship and domain organization of these receptors are presented in Figure 1.

The extracellular part of each IR or IGF-1R monomer contains (sequentially from N- to C-terminus) a leucine-rich repeat domain (L1), a cysteine-rich region (CR) and a second leucine-rich repeat domain (L2), followed by three fibronectin type III domains, (FnIII-1, -2 and -3). The FnIII-2 domain contains a large insert domain (ID) of approximately 120 residues, within which lies the α-β cleavage site. Intracellularly, each monomer contains a tyrosine kinase catalytic domain flanked by two regulatory regions that contain the phosphotyrosine binding sites for signalling molecules. Each α-chain is linked to its partner β-chain via a disulphide bond between residues Cys647 and Cys860 (Sparrow *et al.,* 1997) in the case of IR and/or Cys633-Cys849 in the case of IGF-1R. The α-chains of both IR and IGF-1R are cross-linked by disulphide bonds in two places. The first is at Cys524 (IR) or Cys514 (IGF-1R) in the FnIII-1 domain, cross-linked to its counterpart in the opposite monomer, and the second involves one or more of the residues Cys682, Cys683 and Cys685 (IR) or Cys669, Cys670 and Cys672 (IGF-1R) in the insert region of each FnIII-2 domain, cross-linked to their counterparts in the opposite monomer (Sparrow *et al.,* 1997).

The domains of IR and IGF-1R exhibit high (47-67%) amino acid sequence identity indicative of high conservation of three-dimensional structure. The crystal structure of the first three domains of IGF-1R (L1-CR-L2) has been determined (Garrett *et al.,* 1998) and revealed that the L domains consist of a single-stranded right-handed β-helix (a helical arrangement of β-stands), while the cysteine-rich region is composed of eight related disulfide-bonded modules. The crystal structure of the first three domains of IR (L1-CR-L2) has also been determined (WO 07/147213, Lou et al., 2006) and as anticipated is closely similar to that of its IGF-1R counterpart. Other evidence for the close structural similarity of IR and IGF-1R arises from : (i) electron microscopic analyses (Tulloch *et al.,* 1999), (ii) the fact that hybrid receptors (heterodimers of one IR monomer disulphide-bonded to one of IGF-1R monomer) exist naturally and are commonly found in tissues expressing both receptors (Bailyes *et al.,* 1997); and (iii) the fact that receptor chimeras can be constructed which have whole domains or smaller segments of polypeptide from one receptor replaced by the corresponding domain or sequence from the other (reviewed in Adams *et al.,* 2000).

The current model for insulin binding proposes that, in the basal state, the IR homodimer contains two identical pairs of binding sites (referred to as Site 1 and Site 2) on each monomer (De Meyts and Whittaker, 2002; Schaffer, 1994; De Meyts, 1994; De Meyts, 2004; Kiselyov *et al.,* 2009). Binding of insulin to a low affinity site (Site 1) on one α-subunit is followed by a second binding event between the bound insulin and a different region of the second IR α-subunit (Site 2). This ligand-mediated bridging between the two α-subunits generates the high affinity state that results in signal transduction. In contrast, soluble IR ectodomain, which is not tethered at its C-terminus, cannot generate the high affinity receptor-ligand complex. The soluble IR ectodomain can bind two molecules of insulin simultaneously at its two Site Is, but only with low affinity (Adams *et al.,* 2000). The model for IGF-I or IGF-II binding to IGF-1R is the same as that just described for insulin binding to IR and involves IGF-I (or IGF-II) binding to an initial low affinity site (Site 1) and subsequent cross-linking to a second site (Site 2) on the opposite monomer to form the high affinity state, as described for the IR. However, the values of the kinetic parameters describing these events are somewhat different in the two systems (Surinya *et al.,* 2008; Kiselyov et *al.,* 2009).

While similar in structure, IGF-1R and IR serve different physiological function. IGF-1R is expressed in almost all normal adult tissue except for liver, which is itself the major site of IGF-I production (Buttel *et al.,* 1999). A variety of signalling pathways are activated following binding of IGF-I or IGF-II to IGF-1R, including Src and Ras, as well as downstream pathways, such as the MAP kinase cascade and the P13K/AKT axis (Chow *et al.,* 1998). IR is primarily involved in metabolic functions whereas IGF-1R mediates growth and differentiation. Consistent with this, ablation of IGF-I (i.e. in IGF-I knock-out mice) results in embryonic growth deficiency, impaired postnatal growth, and infertility. In addition, IGF-1R knock-out mice were only 45% of normal size and died of respiratory failure at birth (Liu *et al.,* 1993). However, both insulin and IGF-I can induce both mitogenic and metabolic effects.

Various non-crystallographic 3-D structural analyses of the IR and the interaction of insulin with the IR have been undertaken using electron microscopic techniques (Luo *et al.,* 1999; Ottensmeyer *et al.,* 2000, 2001; Yip and Ottensmeyer, 2001). However, due to the low resolution information obtained (>20 angstrom), the conclusions of these studies have been questioned (De Meyts and Whittaker, 2002).

Crystal structures of the ectodomain of IR have been presented previously (WO 07/147213, McKern et al., 2006; Lou *et al.,* 2006) and have elucidated some potential ligand/IR interactions, in particular part of the low affinity site on the surface of IR L1. However, an area of ambiguous electron density on the surface of the IR L1 domain could not be resolved (WO 07/147213, McKern et al., 2006). Accordingly, there is a need in the art to more fully resolve the structures of both IR and IGF-1R in order to elucidate all potential ligand/receptor interactions. This information would provide a more complete understanding of the mechanisms of action of both IR and IGF-1R necessary for the development of IR and IGF-1R agonists/antagonists.

### Summary of the Invention

The present inventors have determined the crystal structure of the low affinity insulin binding site of human IR. In particular, the crystal structure of the low affinity insulin binding site of human IR ectodomain comprising the C-terminal region of the insulin receptor α-chain has been determined. This structure allows visualisation, for the first time, of the intact low affinity insulin receptor binding site region controlling the initial binding of insulin and the subsequent formation of the high affinity insulin-IR complex that leads to signal transduction. The structure shows, for the first time, the way in which the C-terminal region of the insulin receptor α-chain associates with the first leucine-rich repeat (L1) domain of the receptor to form the complete low affinity insulin binding site. The structure also provides direct insight, for the first time, into the way the so-called Site 1 insulin mimetic peptides bind to the low affinity binding site of the insulin receptor and also provides a basis for designing insulin mimetic peptides that interact with the low affinity insulin binding site of IR. The structural information presented also indicates, by analogy, the corresponding regions in the closely related IGF-1R that are involved in insulin growth factor (IGF) binding.

The identification of molecular structures having a high degree of specificity for only one of IR or IGF-1R is important in the development of efficacious and safe therapeutics. For example, a molecule developed as an insulin agonist should have little or no IGF-I activity in order to avoid the mitogenic activity of IGF-I and a potential for facilitating neoplastic growth. The determination of which regions of IR and IGF-1R have sufficient differences to confer selectivity for their respective ligands or for therapeutic molecules such as chemical entities or biological reagents is therefore an important and significant advancement. Similarly, it is believed that the ability to be able to identify molecular structures that mimic the active binding regions of insulin and/or IGF-I and which impart selective agonist or antagonist activity will also aid and advance the development of new drugs.

To assist in the design of agonists/antagonists of IR and/or IGF-1R, the present inventors have used the structure of human IR ectodomain comprising the C-terminal region of the insulin receptor α-chain (Appendix I) to place a model of the C-terminal region of the insulin receptor α-chain in the 3D structure of IGF-1R ectodomain (Appendix II). The present inventors have also used these models to place a model of the C-terminal region of the IGF-1R α-chain in the 3D structure of IR ectodomain and IGF-1R ectodomain (Appendixes III and IV, respectively). The present inventors used all of these structures to place a model of an insulin mimetic peptide (S519C16) in the binding site of IR and IGF-1R (Appendixes V and VI, respectively). The models, with coordinates in Appendixes II to VI, are oriented relative to atomic coordinates found in Appendix I and may be used in conjunction with atomic coordinates of Appendix I to design a compound which binds to the insulin binding site of IR and/or a compound which binds to the IGF binding site of IGF-1R.

With regards to defining structures by combining subsets of coordinates from Appendix I to Appendix VI, such combinations may be achieved by methods such as assembling combinations of complete domains from each set, assembling combinations of complete domains from each set wherein the coordinates and corresponding amino acid sequence from one structure are transposed onto those of the other, refining less resolved regions of one crystal using the corresponding coordinates of the other.

Accordingly, the present invention provides a computer assisted method of identifying, designing or screening for a compound that can potentially interact with insulin receptor (IR) and/or insulin-like growth factor-1 receptor (IGF-1R), comprising performing structure-based identification, design or screening of a compound based on the compound's interactions with a structure defined by the atomic coordinates of one or more of Appendixes I to VI, or a subset of atomic coordinates of one or more thereof at least representing the C-terminal region of the α-chain of IR, the C-terminal region of the α-chain of IGF-1R, or a mimetic of the C-terminal region of the α-chain of IR and/or IGF-1R.

In one embodiment, the method comprises identifying, designing or screening for a compound which interacts with the three-dimensional structure of; (i) the low affinity insulin binding site of IR, the structure being defined by the atomic coordinates shown in one or more of Appendixes I, III and V, and/or (ii) the low affinity insulin-like growth factor (IGF) binding site of IGF-1R, the structure being defined by the atomic coordinates shown in one or more of Appendixes II, IV and VI, wherein interaction of the compound with the structure is favoured energetically.

In another embodiment, the method further comprises synthesising or obtaining an identified or designed candidate compound and determining the ability of the candidate compound to interact with IR and/or IGF-1R.

The atomic coordinates define one or more regions of the low affinity binding site of IR for insulin, and/or the low affinity binding site of IGF-1R for IGF, comprising the C-terminal region of the α-chain of IR, the C-terminal region of the α-chain of IGF-1R, or a mimetic of the C-terminal region of the α-chain of IR and/or IGF-1R.

The C-terminal region of the α-chain of IR comprises amino acids 693 to 710 of IR α-chain (SEQ ID NO: 13).

In another preferred embodiment, the atomic coordinates defining the low affinity insulin binding site of IR further comprise the leucine-rich repeat 1 (L1) domain and/or the cysteine-rich (CR) domain of the IR ectodomain.

In yet another preferred embodiment, the atomic coordinates define portions of the molecular surface of the central β-sheet of the L1 domain and portions of the molecular surface of the second leucine-rich repeat (LRR) which contain Phe39 and/or the loop in the fourth LRR rung of the L1 domain.

In yet another preferred embodiment, the atomic coordinates define module 6 of the CR domain of IR.

In another embodiment, the atomic coordinates further define one or more amino acid sequences selected from IR amino acid residues 1-156, 157-310, 594 and 794.

In a preferred embodiment, the one or more amino acids selected from IR amino acid residues 1-156 comprise at least one amino acid selected from Arg14, Asn15, Gln34, Leu36, Leu37, Phe39, Pro43, Phe46, Leu62, Phe64, Leu87, Phe88, Phe89, Asn90, Phe96, Glu97, Arg118, Glu120 and His144.

In another embodiment, the one or more amino acids selected from IR amino acid residues 157-310 comprise at least one of the amino acid sequences selected from 192-310, 227-303 and 259-284.

The crystal structure of the first three domains of the ectodomain of IGF-1R has been previously reported (WO 99/028347). The crystal structure of the first three domains of the ectodomain of IR was subsequently reported (WO 07/147213), enabling, for the first time, direct comparison of the regions controlling ligand specificity in the closely related IGF-1R and IR. However, the structure of the intact low affinity insulin binding site (i.e. inclusive of the C-terminal region of the receptor α-chain) could not be elucidated. As will be evident to the skilled person, the findings presented here on the intact insulin binding site of IR ectodomain structure, shape and orientation can be transposed onto the IGF binding site of IGF-1R ectodomain structure, shape and orientation.

The present invention has enabled the identification of previously unrecognised regions of the insulin binding site of IR ectodomain. By analogy, the present invention also identifies the equivalent regions in the IGF-1R, given the structural organisation of domains in the two receptors is effectively the same. The present invention has identified the critical regions of IR involved in the binding of insulin and in mediating the subsequent formation of the high affinity insulin-IR complex that leads to signal transduction. Once again, it will be evident to the skilled person that these findings can be transposed onto IGF-1R.

The present invention is therefore also useful in the identification and/or design of compounds which bind to the low affinity IGF binding site of IGF-1R.

In one embodiment, the atomic coordinates defining one or more regions of the low affinity binding site of IGF-1R for IGF, comprise the C-terminal region of the α-chain of IGF-1R. The C-terminal region of the α-chain of IGF-1R comprises amino acids 681 to 697 of IGF-1R α-chain (SEQ ID NO: 15).

In another embodiment, the atomic coordinates defining the low affinity IGF binding site of IGF-1R further comprise the L1 domain and/or the CR domain of IGF-1R ectodomain.

In a preferred embodiment, the atomic coordinates define the central β-sheer of the L1 domain, and/or that part of the second LRR containing Ser35, and/or the loop in the fourth LRR rung of the L1 domain.

In another preferred embodiment, the atomic coordinates define module 6 of the CR domain of IGF-1R.

The mimetic of the C-terminal region of the α-chain of IR and/or IGF-1R is S519C16 (SEQ ID NO: 18).

In further embodiment, the compound substitutes for the C-terminal region of the α-chain of IR and/or the C-terminal region of the α-chain of IGF-1R in the formation of the low affinity binding site of IR or IGF-1R. Such compounds may act as either agonists or antagonists of these receptors. In one alternative of this embodiment, insulin and/or IGF-1R binds the low affinity binding site of IR and/or IGF-1R in the presence of the compound. In another alternative of this embodiment, insulin and/or IGF-1R does not bind, or has reduced binding to, the low affinity binding site of IR and/or IGF-1R in the presence of the compound.

In another embodiment, a candidate compound for interacting with IR and/or IGF-1R is chemically modified as a result of structure-based evaluation.

In a further embodiment, the chemical modification is designed to either:
i) reduce the potential for the candidate compound to bind to IR whilst maintaining binding to IGF-1R; or
ii) reduce the potential for the candidate compound to bind to IGF-1R, whilst maintaining binding to IR.

Candidate compounds and compounds identified or designed using a method of the present invention may be any suitable compound, including naturally occurring compounds, *de novo* designed compounds, library generated compounds (chemically or recombinantly generated), mimetics etc., and include organic compounds, new chemical entities, antibodies, binding proteins other than antibody-based molecules (nonimmunoglobulin proteins) including, for example, protein scaffolds such as lipocalins, designed ankyrin repeat proteins (DARPins, Stumpp *et al.,* 2007) and protein A domains (reviewed in Binz *et al,* 2005), avimers (Silverman *et al.,* 2005), and other new biological entities such as nucleic acid aptamers (reviewed in Ulrich, 2006).

The present invention is also useful for improving the properties of known ligands for the low affinity binding sites of IR and/or IGF-1R. For example, existing IR or IGF-1 R low affinity binding site ligands can be screened against the 3D structure of the insulin binding site of IR ectodomain or a region of the insulin binding site of IR ectodomain defined by the atomic coordinates of Appendix I or a portion thereof (optionally utilising the atomic coordinates given in Appendixes II to VI to further refine the screen and/or the assessment of the potential to energetically interact with IR), and an assessment made of the potential to energetically interact with the insulin binding site of IR.

Thus, the present invention also provides a computer assisted method for redesigning a compound which is known to bind to IR and/or IGF-1R comprising performing structure-based evaluation of the compound based on the compound's interactions with a structure defined by the atomic coordinates of one or more of Appendixes I to VI, or a subset of atomic coordinates of one or more thereof at least representing the C-terminal region of the α-chain of IR, the C-terminal region of the α-chain of IGF-1R, or a mimetic of the C-terminal region of the α-chain of IR and/or IGF-1R, and redesigning or chemically modifying the compound as a result of the evaluation.

In one embodiment, the compound which is known to bind to IR and/or IGF-1R is redesigned or chemically modified to (i) improve affinity for binding to IR, and/or (ii) lower affinity for binding to IGF-IR.

In another embodiment, the compound which is known to bind to IR and/or IGF-1R is redesigned or chemically modified to (i) improve affinity for binding to IGF-1R, and/or (ii) lower affinity for binding to IR.

When screening potential ligands or compounds for selectivity for binding to the insulin binding site of IR or IGF-1R, it will be important to concentrate on those areas of difference in the 3D structure between the low affinity binding site of ectodomains of IR and IGF-1R. Such areas are identified and described herein. In particular, it will be important to concentrate on those areas of difference which are identified as being potentially important in the binding of insulin to the receptors.

Accordingly, in a further embodiment the compound is redesigned or modified so as to lower the affinity to IR or IGF-1R by virtue of the structural differences between IR and IGF-1R at or in the vicinity of the C-terminal region of the α-chain of IR and the C-terminal region of the α-chain of IGF-1R.

Also described herein is a computer system for identifying one or more compounds that can potentially interact with IR and/or IGF-1R, the system containing data representing the structure of: (i) the low affinity insulin binding site of IR, the structure being defined by the atomic coordinates shown in one or more of Appendixes I, III and V; (ii) the low affinity IGF binding site of IGF-1R, the structure being defined by the atomic coordinates shown in one or more of Appendixes II, IV and VI; and/or (iii) the C-terminal region of the α-chain of IR, the C-terminal region of the α-chain of IGF-1R, or a mimetic of the C-terminal region of the α-chain of IR and/or IGF-1R, the structure being defined by a subset of atomic coordinates shown in one or more of Appendixes I to VI.

Also described is a computer-readable medium having recorded thereon data representing a model and/or the atomic coordinates as shown in one or more of Appendixes I to VI, or a subset of atomic coordinates of one or more thereof at least representing:
i) the C-terminal region of the α-chain of IR;
ii) the C-terminal region of the α-chain of IGF-1R; and/or
iii) a mimetic of the C-terminal region of the α-chain of IR and/or IGF-1R,
as any one of i) to iii) associates with IR and/or IGF-1R.

Further described are a set of coordinates as shown in one or more of Appendixes I to VI, or a subset of atomic coordinates of one or more thereof at least representing:
i) the C-terminal region of the α-chain of IR;
ii) the C-terminal region of the α-chain of IGF-1R; and/or
iii) a mimetic of the C-terminal region of the α-chain of IR and/or IGF-1R,
as any one of i) to iii) associates with IR and/or IGF-1R.

The three-dimensional structure of the C-terminal region of the IR and/or IGF-1R α-chain may be used to develop models useful for drug design and/or *in silico* screening of candidate compounds that interact with and/or modulate IR and/or IGF-1R. Other physicochemical characteristics may also be used in developing the model, e.g. bonding, electrostatics, etc.

Generally the term *"in silico"* refers to the creation in a computer memory, i.e., on a silicon or other like chip. Stated otherwise *"in silico"* means "virtual". When used herein the term *"in silico"* is intended to refer to screening methods based on the use of computer models rather than *in vitro* or *in vivo* experiments.

Accordingly, the present invention also provides a computer-assisted method of identifying a compound that potentially interacts with IR and/or IGF-1R, which method comprises fitting the structure of: (i) the low affinity insulin binding site of IR, the structure being defined by the atomic coordinates shown in one or more of Appendixes I, III and V; (ii) the low affinity IGF binding site of IGF-1R, the structure being defined by the atomic coordinates shown in one or more of Appendixes II, IV and VI; and/or (iii) the C-terminal region of the α-chain of IR, the C-terminal region of the α-chain of IGF-1R, or a mimetic of the C-terminal region of the α-chain of IR and/or IGF-1R, the structure being defined by a subset of atomic coordinates shown in one or more of Appendixes I to VI, to the structure of a candidate compound.

Also provided by the present invention is a computer-assisted method for identifying a compound able to interact with IR and/or IGF-1R using a programmed computer comprising a processor, which method comprises the steps of: (a) generating, using computer methods, a set of atomic coordinates of a structure that possesses energetically favourable interactions with the atomic coordinates of: (i) the low affinity insulin binding site of IR, the structure being defined by the atomic coordinates shown in one or more of Appendixes I, III and V; (ii) the low affinity IGF binding site of IGF-1R, the structure being defined by the atomic coordinates shown in one or more of Appendixes II, IV and VI; and/or (iii) the C-terminal region of the α-chain of IR, the C-terminal region of the α-chain of IGF-1R, or a mimetic of the C-terminal region of the α-chain of IR and/or IGF-1R, the structure being defined by a subset of atomic coordinates shown in one or more of Appendixes I to VI, which coordinates are entered into the computer thereby generating a criteria data set; (b) comparing, using the processor, the criteria data set to a computer database of chemical structures; (c) selecting from the database, using computer methods, chemical structures which are complementary or similar to a region of the criteria data set; and optionally, (d) outputting, to an output device, the selected chemical structures which are complementary to or similar to a region of the criteria data set.

The present invention further provides a computer-assisted method for identifying potential mimetics of IR and/or IGF-1R using a programmed computer comprising a processor, the method comprising the steps of (a) generating a criteria data set from a set of atomic coordinates of: (i) the low affinity insulin binding site of IR, the structure being defined by the atomic coordinates shown in one or more of Appendixes I, III and V; (ii) the low affinity IGF binding site of IGF-1R, the structure being defined by the atomic coordinates shown in one or more of Appendixes II, IV and VI; and/or (iii) the C-terminal region of the α-chain of IR, the C-terminal region of the α-chain of IGF-1R, or a mimetic of the C-terminal region of the α-chain of IR and/or IGF-1R, the structure being defined by a subset of atomic coordinates shown in one or more of Appendixes I to VI, which coordinates are entered into the computer; (b) (i) comparing, using the processor, the criteria data set to a computer database of chemical structures stored in a computer data storage system and selecting from the database, using computer methods, chemical structures having a region that is structurally similar to the criteria data set; or (ii) constructing, using computer methods, a model of a chemical structure having a region that is structurally similar to the criteria data set; and, optionally, (c) outputting to an output device: (i) the selected chemical structures from step (b)(i) having a region similar to the criteria data set; or (ii) the constructed model from step (b)(ii).

The present invention further provides a method for evaluating the ability of a compound to interact with IR and/or IGF-1R, the method comprising the steps of: (a) employing computational means to perform a fitting operation between the compound and the binding surface of a computer model of the low affinity binding site for insulin on IR ectodomain, and/or the low affinity binding site for IGF on IGF-1R ectodomain, using atomic coordinates wherein the root mean square deviation between the atomic coordinates and a subset of atomic coordinates of one or more of Appendixes I to VI or a subset of atomic coordinates of one or more thereof at least representing the C-terminal region of the α-chain of IR, the C-terminal region of the α-chain of IGF-1R, or a mimetic of the C-terminal region of the α-chain of IR and/or IGF-1R, is not more than 1.5 Å; and (b) analysing the results of the fitting operation to quantify the association between the compound and the binding surface model.

The present invention also provides a method of using molecular replacement to obtain structural information about a molecule or a molecular complex of unknown structure, comprising the steps of: (i) generating an X-ray diffraction pattern of the crystallized molecule or molecular complex; and (ii) applying the atomic coordinates of one or more of Appendixes I to VI, or a subset of atomic coordinates of one or more thereof at least representing the C-terminal region of the α-chain of IR, the C-terminal region of the α-chain of IGF-1R, or a mimetic of the C-terminal region of the α-chain of IR and/or IGF-1R, to the X-ray diffraction pattern to generate a three-dimensional electron density map of at least a region of the molecule or molecular complex whose structure is unknown.

Also described herein is a compound that binds to IR and/or IGF-1R ectodomain designed, redesigned or modified using the methods of the invention. Preferably, such compounds have an affinity (K_{d}) for IR and/or IGF-1R of less than 10⁻⁵ M. In a particularly preferred example, the compound binds to the low affinity binding site of IR and/or to the low affinity binding site of IGF-1R.

Further described is an isolated peptide or mimetic thereof which binds the L1 domain of IR and/or the L1 domain of IGF-1R, the peptide comprising: (i) an amino acid sequence as provided in SEQ ID NO: 13 or SEQ ID NO: 15; (ii) an amino acid sequence which is at least 50% identical, more preferably at least 80% identical, more preferably at least 90% identical, more preferably at least 95% identical, to SEQ ID NO: 13 and/or SEQ ID NO: 15; or (iii) a fragment of i) or ii) which binds the L1 domain of IR and/or the L1 domain of IGF-1R, wherein the peptide has a helical structure.

Further described is an isolated polynucleotide encoding the isolated peptide or mimetic thereof, as well as a vector comprising said polynucleotide and a host cell comprising said vector.

Additionally described herein is a composition comprising a compound, a peptide or mimetic, and/or a polynucleotide as described herein, and optionally an acceptable carrier or diluent, more preferably a pharmaceutically acceptable carrier or diluent.

Further described herein is a method for preventing or treating a disease associated with aberrant IR and/or IGF-1R functioning and/or signalling, the method comprising administering to a subject in need thereof a compound, a peptide or mimetic, and/or a polynucleotide as described herein.

Also described herein is use of a compound, a peptide or mimetic, and/or a polynucleotide as described herein, for the manufacture of a medicament for treating a disease in a subject associated with aberrant IR and/or IGF-1R functioning and/or signalling.

Examples of diseases associated with aberrant IR and/or IGF-1R functioning and/or signalling include, but are not limited to, obesity, type I and type II diabetes, cardiovascular disease, osteoporosis, dementia and cancer.

Also described are manufacturing steps such as incorporating the compound, such as a peptide, into a pharmaceutical composition in the manufacture of a medicament.

Throughout this specification, preferred aspects and embodiments apply, as appropriate, separately, or in combination, to other aspects and embodiments, *mutatis mutandis,* whether or not explicitly stated as such.

The present invention will now be described further with reference to the following examples, which are illustrative only and non-limiting.

### Brief Description of the Figures

Some figures contain colour representations or entities. Coloured versions of the figures are available from the Patentee upon request or from an appropriate patent Office. A fee may be imposed if obtained from a Patent Office.
**Figure 1****:** Shows the sequence alignment of the ectodomains of human insulin receptor (IR, exon 11-isoform) and human IGF1 receptor (IGF-1R). Residues conserved-between the sequences are indicated by vertical bars and potential N-linked glycosylation sites are indicated by shading. Disulphide links are indicated by square braces above the alignment. Sequence sources were: IR (Ullrich *et al.,* 1985), human type 1 IGF receptor (Ullrich *et al.,* 1986).
**Figure 2****.** ITC curves for the titration of (a) IR classical αCT peptide against IR485, (b) IGF-1R classical aCT peptide against IR485, and (c) IR classical αCT.714A peptide against IR485.
**Figure 3****.** ITC curves for the titration of (a) ZFP-insulin against IR485 pre-complexed with a 10-fold molar ratio of IR classical aCT peptide, (b) ZFP-insulin against IR485 pre-complexed with a 10-fold molar ratio of IGF-1R classical aCT peptide, (c) IGF-1R against IR485 pre-complexed with a 10-fold molar ratio of IR classical aCT peptide, and (d) IGF-1R against IR485 pre-complexed with a 10-fold molar ratio of IGF-1R classical aCT peptide.
**Figure 4****.** ITC curves for the titration of (a) S519C16 against IR485, (b) S519C16 against IR485 pre-complexed with a 10-fold molar ratio of IR classical αCT peptide, (c) S519N20 against IR485, and (d) S519 against IR485.
**Figure 5****.** Dynamic light scattering volume distribution curves obtained from samples of (a) IR485 at 6 mg/ml, (b) IR485 at 0.5 mg/ml; (c) IR485 at 6 mg/ml plus a 3-fold molar ratio of IR αCT peptide, (d) IR485 at 6 mg/ml plus a 3-fold molar ratio of IR classical aCT peptide and a 2-fold molar ratio of ZFP-insulin.
**Figure 6****.** The crystal structure of IR ectodomain comprising the C-terminal region of the α-chain of IR. (a) Negative B-factor enhanced (Fₒ-F_{c}) electron density overlaid with the final model of IR residues 693-710; (b) Detail of the interaction between IR residues 693-710 (yellow backbone, green carbons, non-bold numbering) and the surface of L1-β2 (pink backbones, cyan carbons, bold numbering); (c) Sequence alignment of the C-terminal regions of the α-chains of IR and IGF-1R and the S519C16 peptide (Menting et al., 2009). Shaded
regions show conservation between the three sequences and boxed regions show segments predicted to be helical in conformation (Menting et al., 2009).

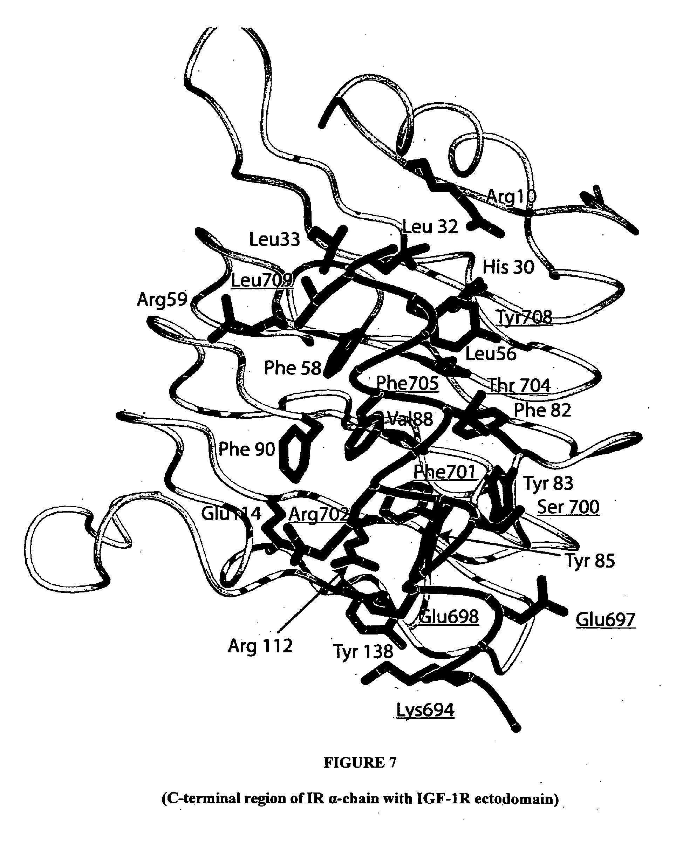

Figure 7. Model structure of the C-terminal region of IR α-chain bound to the L1 domain of IGF-1R (Appendix II) generated from the crystal structure of IR ectodomain inclusive of residues 693-710. The backbone of the respective L1 domain is shown as an orange coil, the side chains of residues within the L1 domain that interact with the respective bound peptide are shown with green carbon atoms, red oxygen atoms and blue nitrogen atoms, and the backbone of the bound peptide helix is shown as a blue coiL Selected peptide residues that interact with the L1 domain are shown with cyan carbon atoms, red oxygen atoms and blue nitrogen atoms. The remaining peptide residues, which have more limited or no interaction with the L1 domain are represented only by their α-carbon atoms shown as spheres embedded in the peptide coil, with other atoms within these residues omitted for clarity. Residues that lie in the C-terminal region of IR α-chain are underlined for clarity.
**Figure 8****.** Model structure of the C-terminal region of IGF-1R α-chain bound to the L1 domain of IR (Appendix III). Colouring and style is as described above for Figure 7.
**Figure 9****.** Model structure of the C-terminal region of IGF-1R α-chain bound to the L1 domain of IGF-1R (Appendix IV). Colouring and style is as described above for Figure 7.
**Figure 10****.** Model structure of the S519C16 peptide bound to the L1 domain of IR (Appendix V). Colouring and style is as described above for Figure 7.
**Figure 11****.** Model structure of the S519C16 peptide bound to the L1 domain of IGF-1R (Appendix VI). Colouring and style is as described above for Figure 7.
**Figure 12****.** Sample isothermal titration calorimetry curves obtained for the titration against insulin mini-receptor IR485 of N-terminally biotinylated aCT peptide 698-719 containing the following respective mutations: (A) wild type, (B) T704Y, (C) R702W, (D) R702Y, (E) T704W and (F) R702Y / T704W.

### Key to the Sequence Listing

SEQ ID NO: 1 - Amino acid sequence of mature human insulin receptor ectodomain (isoform A).
SEQ ID NO: 2 - Amino acid sequence of mature human insulin receptor ectodomain (isoform B).
SEQ ID NO: 3 - Amino acid sequence of mouse insulin receptor.
SEQ ID NO: 4 - Amino acid sequence of rhesus monkey insulin receptor, predicted.
SEQ ID NO: 5 - Amino acid sequence of bovine insulin receptor, predicted.
SEQ ID NO: 6 - Amino acid sequence of mature human insulin-like growth factor receptor 1 (IGF-1R) ectodomain.
SEQ ID NO: 7 - Amino acid sequence of mouse insulin-like growth factor receptor 1 (IGF-1R).
SEQ ID NO: 8 - Amino acid sequence of rhesus monkey insulin-like growth factor receptor 1 (IGF-1R), predicted.
SEQ ID NO: 9 - Amino acid sequence of bovine insulin-like growth factor receptor 1 (IGF-1R), predicted.
SEQ ID NO: 10 - Amino acid sequence of IR485.
SEQ ID NO: 11 - Amino acid sequence of the classical α-chain C-terminal peptide (αCT) of human IR.
SEQ ID NO: 12 - Amino acid sequence of the F714A mutant of the classical α-chain C-terminal peptide ('αCT') of human IR.
SEQ ID NO: 13 - Amino acid sequence of the C-terminal region of the α-chain of human IR.
SEQ ID NO: 14 - Amino acid sequence of the classical α-chain C-terminal peptide (αCT) of human IGF-1R.
SEQ ID NO: 15 - Amino acid sequence of the C-terminal region of the α-chain of human IGF-1R.
SEQ ID NO: 16 - Amino acid sequence of the S519 peptide.
SEQ ID NO: 17 - Amino acid sequence of the S519N20 peptide.
SEQ ID NO: 18 - Amino acid sequence of the S519C16 peptide.
SEQ ID NO:19 - FYXWF motif.

### Detailed Description of the Invention

Unless defined otherwise, all technical and scientific terms used herein have the same meaning as commonly understood by one of ordinary skill in the art (e.g. in molecular biology, biochemistry, structural biology, and computational biology). Standard techniques are used for molecular and biochemical methods (see generally, Sambrook *et al.,* 2001, and Ausubel *et al.,* 1999, and chemical methods.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

### IR ectodomain crystals and crystal structure

The present invention relates to use of a crystal comprising a C-terminal region of the IR α-chain based on the IRΔβ construct (see Examples).

As used herein, the term "crystal" means a structure (such as a three dimensional (3D) solid aggregate) in which the plane faces intersect at definite angles and in which there is a regular structure (such as internal structure) of the constituent chemical species. The term "crystal" refers in particular to a solid physical crystal form such as an experimentally prepared crystal.

Crystals may be prepared using any IR ectodomain, i.e. the IR polypeptide containing the extracellular domain and lacking the transmembrane domain and the intracellular tyrosine kinase domain. Typically, the extracellular domain comprises residues 1 to 917 (mature receptor numbering) of human IR, or the equivalent thereof together with any post-translational modifications of these residues such as N- or O-linked glycosylation.

In a preferred embodiment the IR polypeptide is human IR (SEQ ID NOs: 1 and 2). However, the IR polypeptide may also be obtained from other species, such as other mammalian, vertebrate or invertebrate species. Examples of IR polypeptides from other species are given in SEQ ID NOs: 3 to 5.

Crystals may be constructed with wild-type IR polypeptide ectodomain sequences or variants thereof, including allelic variants and naturally occurring mutations as well as genetically engineered variants. Typically, variants have at least 95 or 98% sequence identity with a corresponding wild-type IR ectodomain polypeptide.

Optionally, the crystal of IR ectodomain may comprise one or more molecules which bind to the ectodomain, or otherwise soaked into the crystal or cocrystallised with IR ectodomain. Such molecules include ligands or small molecules, which may be candidate pharmaceutical agents intended to modulate the interaction between IR and its biological targets. The crystal of IR ectodomain may also be a molecular complex with other receptors of the IGF receptor family such as IGF-1R. The complex may also comprise additional molecules such as the ligands to these receptors.

The production of IR ectodomain crystals is described below.

In a preferred embodiment, an IR ectodomain crystal comprising the C-terminal segment of the IR α-chain has the atomic coordinates set forth in Appendix I. As used herein, the term "atomic coordinates" or "set of coordinates" refers to a set of values which define the position of one or more atoms with reference to a system of axes. It will be understood by those skilled in the art that atomic coordinates may be varied, without affecting significantly the accuracy of models derived therefrom.

It will be understood that any reference herein to the atomic coordinates or subset of the atomic coordinates shown in Appendix I shall include, unless specified otherwise, atomic coordinates having a root mean square deviation of backbone atoms of not more than 1.5 Å, preferably not more than 1 Å, when superimposed on the corresponding backbone atoms described by the atomic coordinates shown in Appendix I. Also, any reference to the atomic coordinates or subset of the atomic coordinates shown in Appendixes II to VI shall include, unless specified otherwise, atomic coordinates having a root mean square deviation of backbone atoms of not more than 2.5 Å when superimposed on the corresponding backbone atoms described by the atomic coordinates shown in Appendixes II to VI.

The following defines what is intended by the term "root mean square deviation (RMSD)" between two data sets. For each element in the first data set, its deviation from the corresponding item in the second data set is computed. The squared deviation is the square of that deviation, and the mean squared deviation is the mean of all these squared deviations.
The root mean square deviation is the square root of the mean squared deviation.

Preferred variants are those in which the RMSD of the x, y and z coordinates for all backbone atoms other than hydrogen is less than 1.5 Å (preferably less than 1 Å, 0.7 Å or less than 0.3 Å) compared with the coordinates given in Appendix I. It will be readily appreciated by those skilled in the art that a 3D rigid body rotation and/or translation of the atomic coordinates does not alter the structure of the molecule concerned.

In a highly preferred embodiment, the crystal has the atomic coordinates as shown in Appendix I.

The present invention also relates to a crystal structure of the low affinity insulin binding site of IR ectodomain polypeptide comprising the C-terminal region of the IR α-chain, or a region thereof.

The atomic coordinates obtained experimentally for amino acids 4 to 655, 693 to 710 (the "C-terminal region of the IR α-chain"), and 755 to 909 of human IR-A (mature receptor numbering; SEQ ID NO: 1) are shown in Appendix I. However, a person skilled in the art will appreciate that a set of atomic coordinates determined by X-ray crystallography is not without standard error. Accordingly, any set of structure coordinates for an IR ectodomain polypeptide comprising the C-terminal region of the IR α-chain that has a root mean square deviation of protein backbone atoms of less than 0.75 Å when superimposed (using backbone atoms) on the atomic coordinates listed in Appendix I shall be considered identical.

The present invention also comprises the atomic coordinates of the C-terminal region of the IR α-chain that substantially conforms to the atomic coordinates listed in Appendix I.

A structure that "substantially conforms" to a given set of atomic coordinates is a structure wherein at least about 50% of such structure has an RMSD of less than about 1.5 Å for the backbone atoms in secondary structure elements in each domain, and more preferably, less than about 1.3 Å for the backbone atoms in secondary structure elements in each domain, and, in increasing preference, less than about 1.0 Å, less than about 0.7 Å, less than about 0.5 Å, and most preferably, less than about 0.3 Å for the backbone atoms in secondary structure elements in each domain.

In a more preferred embodiment, a structure that substantially conforms to a given set of atomic coordinates is a structure wherein at least about 75% of such structure has the recited RMSD value, and more preferably, at least about 90% of such structure has the recited RMSD value, and most preferably, about 100% of such structure has the recited RMSD value.

In an even more preferred embodiment, the above definition of "substantially conforms" can be extended to include atoms of amino acid side chains. As used herein, the phrase "common amino acid side chains" refers to amino acid side chains that are common to both the structure which substantially conforms to a given set of atomic coordinates and the structure that is actually represented by such atomic coordinates.

According to the present invention the C-terminal region of the IR ectodomain α-chain spans residues 693 to 710 (SEQ ID NO: 13).

As used herein, the term "IR ectodomain" refers to the extracellular domain of IR lacking the transmembrane domain and the intracellular tyrosine kinase domain of IR, typically comprising residues 1 to 917 (mature IR-A receptor numbering) of human IR, or the equivalent thereof, together with any post-translational modifications of these residues such as N- or O-linked glycosylation.

As used herein, the term "low affinity binding site" for IR means the regions of IR involved in forming the low affinity binding site (also known as "Site 1") of IR for insulin, comprising the C-terminal region of the IR α-chain and additionally one or both of the L1 domain of IR and the CR domain of IR. Insulin binding to the low affinity binding site of IR induces formation of the high affinity insulin binding site of IR and subsequent signal transduction.

As used herein, the term "C-terminal region" of the IR α-chain refers to amino acids 693-710 of isoform A (IR-A) of the human IR α-chain as given in SEQ ID NO: 13, with numbering according to mature isoform A of human IR (SEQ ID NO: 1).

As used herein, the term "classical α-chain C-terminal peptide", or "αCT", refers in IR to a region of the C-terminal α-chain of IR previously described in the literature as being important for insulin binding (Kurose et al., 1994; Kristensen et al, 2002), and comprising amino acids 704-719 (mature IR-A receptor numbering) as given in SEQ ID NO: 11.

As used herein, the term "leucine-rich repeat domain 1" or "L1 domain" refers in IR to a leucine-rich domain comprising amino acids 1-156 of mature human IR (SEQ ID NO: 1). The L1 domain of IR comprises a central β-sheer, which comprises amino acids selected from 10-15, 32-37, 60-65, 88-97, 116-121 and 142-147 of mature human IR (SEQ ID NO: 1).

As used herein, the term "leucine-rich repeat domain 2" or "L2 domain" refers in IR to a leucine-rich domain comprising amino acids 310-469 of mature human IR (SEQ ID NO: 1).

As used herein, the term "loop in the fourth leucine-rich repeat (LRR) rung of the L1 domain", or variations thereof, refers in IR to a leucine-rich domain comprising amino acids 85-91 of mature human IR (SEQ ID NO: 1).

As used herein, the term "cysteine-rich domain" or "CR domain" refers in IR to a cysteine-rich domain comprising amino acids 157-309 of mature human IR (SEQ ID NO: 1). The CR domain contains many different modules. As used herein, the term "module 6 of the CR domain" refers in IR to amino acids 256-286 of mature human IR (SEQ ID NO: 1).

### IGF-1R ectodomain structure

Due to the high sequence homology and structural similarity between IR and IGF-1R, the present invention also provides a model for the C-terminal region of IGF-1R α-chain as it associates with IGF-1R to form the low affinity IGF binding site. According to the present invention the C-terminal region of the IGF-1R ectodomain α-chain comprises residues 681-697 (SEQ ID NO: 15).

As used herein, the term "IGF-1R ectodomain" refers to the extracellular domain of IGF-1R lacking the transmembrane domain and the intracellular tyrosine kinase domain of IGF-1R, typically comprising residues 1 to 905 (mature receptor numbering) of human IGF-1R, or the equivalent thereof, together with any post-translational modifications of these residues such as N- or O-linked glycosylation.

As used herein, the term "low affinity binding site" for IGF-1R means the regions of IGF-1R involved in forming the low affinity binding site (also known as "Site 1") of IGF-1R for IGF, comprising the C-terminal region of the IGF-1R α-chain and additionally one or both of the L1 domain of IGF-1R and the CR domain of IGF-1R. IGF binding to the low affinity binding site of IGF-1R induces formation of the high affinity IGF binding site of IGF-1R and subsequent signal transduction.

As used herein, the term "C-terminal region" of the IGF-1R α-chain refers to amino acids 681-697 of human IGF-1R α-chain as given in SEQ ID NO: 15, with numbering according to mature human IGF-1R (SEQ ID NO: 6).

As used herein, the term "classical α-chain C-terminal peptide", or "αCT", refers in IGF-1R to a region of IGF-1R corresponding to the C-terminal α-chain of IR previously described in the literature as being important for insulin binding (Kurose et al., 1994; Kristensen et al, 2002), and comprising amino acids 691-706 of IGF-1R (mature IGF-1R numbering) as given in SEQ ID NO: 14.

As used herein, the term "leucine-rich repeat domain 1" or "L1 domain" refers in IGF-1R to a leucine-rich domain comprising amino acids 1-149 of mature human IGF-1R (SEQ ID NO: 6).

As used herein, the term "leucine-rich repeat domain 2" or "L2 domain" refers in IGF-1R to a leucine-rich domain comprising amino acids 300-459 of mature human IGF-1 R (SEQ ID NO: 6).

As used herein, the term "that part of the second LRR containing Ser35" refers in IGF-1R to amino acids 35-41 of mature human IGF-1R (SEQ ID NO: 6).

As used herein, the term "cysteine-rich domain" or "CR domain" refers in IGF-1R to a cysteine-rich domain comprising amino acids 150-299 of mature human IGF-1R (SEQ ID NO: 6). The CR domain contains many different modules. As used herein, the term "module 6 of the CR domain" refers to amino acids 249-275 of mature human IGF-1R (SEQ ID NO: 6).

### Manipulation of the atomic coordinates

It will be appreciated that a set of atomic coordinates for a polypeptide is a relative set of points that define a shape in three dimensions. Thus, it is possible that an entirely different set of coordinates could define a similar or identical shape. Moreover, slight variations in the individual coordinates will have little effect on overall shape.

The variations in coordinates may be generated due to mathematical manipulations of the atomic coordinates. For example, the atomic coordinates set forth in Appendix I could be manipulated by crystallographic permutations of the atomic coordinates, fractionalisation of the atomic coordinates, integer additions or subtractions to sets of the structure coordinates, inversion of the atomic coordinates, or any combination thereof.

Alternatively, modification in the crystal structure due to mutations, additions, substitutions, and/or deletions of amino acids, or other changes in any of the components that make up the crystal could also account for variations in atomic coordinates.

Various computational analyses are used to determine whether a molecular complex or a portion thereof is sufficiently similar to all or parts of the structure of the extracellular domain of IR described above. Such analyses may be carried out in current software applications, such as the Sequoia program (Bruns et al., 1999).

The Molecular Similarity program permits comparisons between different structures, different conformations of the same structure, and different parts of the same structure.

Comparisons typically involve calculation of the optimum translations and rotations required such that the root mean square deviation of the fit over the specified pairs of equivalent atoms is an absolute minimum. This number is given in Angstroms.

Accordingly, atomic coordinates of an IR and/or IGF-1R ectodomain comprising the low affinity binding site of the present invention include atomic coordinates related to the atomic coordinates listed in Appendixes I to VI by whole body translations and/or rotations. Accordingly, RMSD values listed above assume that at least the backbone atoms of the structures are optimally superimposed which may require translation and/or rotation to achieve the required optimal fit from which to calculate the RMSD value.

A three dimensional structure of an IR and/or IGF-1R ectodomain polypeptide or region thereof which substantially conforms to a specified set of atomic coordinates can be modelled by a suitable modeling computer program such as MODELLER (Sali & Blundell, 1993), using information, for example, derived from the following data: (1) the amino acid sequence of the human IR and/or IGF-1R ectodomain polypeptide; (2) the amino acid sequence of the related portion(s) of the protein represented by the specified set of atomic coordinates having a three dimensional configuration; and, (3) the atomic coordinates of the specified three dimensional configuration. A three dimensional structure of an IR and/or IGF-1R ectodomain polypeptide which substantially conforms to a specified set of atomic coordinates can also be calculated by a method such as molecular replacement, which is described in detail below.

Atomic coordinates are typically loaded onto a machine-readable medium for subsequent computational manipulation. Thus models and/or atomic coordinates are advantageously stored on machine-readable media, such as magnetic or optical media and random-access or read-only memory, including tapes, diskettes, hard disks, CD-ROMs and DVDs, flash memory cards or chips, servers and the internet. The machine is typically a computer.

The atomic coordinates may be used in a computer to generate a representation, e.g. an image, of the three-dimensional structure of the IR and/or IGF-1R ectodomain crystal which can be displayed by the computer and/or represented in an electronic file.

The atomic coordinates and models derived therefrom may also be used for a variety of purposes such as drug discovery, biological reagent (binding protein) selection and X-ray crystallographic analysis of other protein crystals.

### Molecular replacement/binding

The structure coordinates of IR and/or IGF-1R comprising the C-terminal region of the α-chain, such as those set forth in Appendixes I to IV, can also be used for determining the three-dimensional structure of a molecular complex which contains at least the C-terminal region of the α-chain of IR and/or IGF-1R. In particular, structural information about another crystallised molecular complex may be obtained. This may be achieved by any of a number of well-known techniques, including molecular replacement.

Methods of molecular replacement are generally known by those of skill in the art (generally described in Brunger, 1997; Navaza & Saludjian, 1997; Tong & Rossmann, 1997; Bentley, 1997; Lattman, 1985; Rossmann, 1972; McCoy, 2007).

Generally, X-ray diffraction data are collected from the crystal of a crystallised target structure. The X-ray diffraction data is transformed to calculate a Patterson function. The Patterson function of the crystallised target structure is compared with a Patterson function calculated from a known structure (referred to herein as a search structure). The Patterson function of the search structure is rotated on the target structure Patterson function to determine the correct orientation of the search structure in the crystal. A translation function is then calculated to determine the location of the search structure with respect to the crystal axes. Once the search structure has been correctly positioned in the unit cell, initial phases for the experimental data can be calculated. These phases are necessary for calculation of an electron density map from which structural differences can be observed and for refinement of the structure. Preferably, the structural features (e.g., amino acid sequence, conserved di-sulphide bonds, and beta-strands or beta-sheets) of the search molecule are related to the crystallised target structure.

The electron density map can, in turn, be subjected to any well-known model building and structure refinement techniques to provide a final, accurate structure of the unknown (i.e. target) crystallised molecular complex (e.g. see Jones *et al.,* 1991; Brünger *et al.,* 1998).

Obtaining accurate values for the phases, by methods other than molecular replacement, is a time-consuming process that involves iterative cycles of approximations and refinements and greatly hinders the solution of crystal structures. However, when the crystal structure of a protein containing at least a homologous portion has been solved, the phases from the known structure provide a satisfactory starting estimate of the phases for the unknown structure.

By using molecular replacement, all or part of the structure coordinates of IR and/or IGF-1R comprising the C-terminal region of the α-chain provided herein (and set forth in Appendixes I to IV) can be used to determine the structure of a crystallised molecular complex whose structure is unknown more rapidly and efficiently than attempting to determine such information *ab initio.* This method is especially useful in determining the structure of IR and/or IGF-1R mutants and homologues.

The structure of any portion of any crystallised molecular complex that is sufficiently homologous to any portion of the extracellular domain of IR and/or IGF-1R can be solved by this method..

Such structure coordinates are also particularly useful to solve the structure of crystals of IR and/or IGF-1R co-complexed with a variety of molecules, such as chemical entities. For example, this approach enables the determination of the optimal sites for the interaction between chemical entities, and the interaction of candidate IR and/or IGF-1R agonist or antagonists.

All of the complexes referred to above may be studied using well-known X-ray diffraction techniques and may be refined against 1.5-3.5 Å resolution X-ray data to an R value of about 0.25 or less using computer software, such as X-PLOR (Yale University, distributed by Molecular Simulations, Inc.; see Brunger, 1996). This information may thus be used to optimize known IR and/or IGF-1R agonist/antagonists, such as anti-IR and/or anti-IF-1R antibodies, and more importantly, to design new or improved IR and/or IGF-1R agonists/antagonists.

### Target sites for compound identification, design or screening

The three-dimensional structure of the low affinity binding site of IR and/or IGF-1R provided by the present invention (Appendixes I to IV) can be used to identify potential target binding sites in the low affinity insulin binding site of IR and/or IGF-1R (i.e. to identify those regions of the low affinity binding site of IR and/or IGF-1R involved in and important to the binding of insulin and/or IGF and subsequent signal transduction) as well as in methods for identifying or designing compounds which interact with the low affinity binding site of IR and/or IGF-1R e.g. potential modulators of IR and/or IGF-1R.

The three-dimensional structure of IR and/or IGF-1R provided by the present invention (Appendixes I to IV) can be used to identify potential target binding sites in the L1 domain of IR and/or IGF-1R important for binding to the C-terminal region of the IR and/or IGF-1R α-chain (i.e. to identify those regions of the L1 domain of IR and/or IGF-1R involved in and important to the binding of C-terminal region of the IR and/or IGF-1R α-chain) as well as in methods for identifying or designing compounds which interact with the L1 domain of IR and/or IGF-1R in a manner similar to the C-terminal region of the IR and/or IGF-1R α-chain e.g. potential modulators of IR and/or IGF-1R.

The low affinity binding site of IR is a region of IR ectodomain involved in insulin docking to the receptor. Preferred low affinity target binding sites comprise the C-terminal region of the α-chain and and one or more regions from the L1 domain and/or the CR domain of IR ectodomain. With regards to the L1 domain, the target binding site preferably comprises portions of the molecular surface of the central β-sheet of L1 and portions of the molecular surface of the second leucine-rich repeat (LRR) which contain Phe39 or the loop in the fourth LRR rung of L1, or preferably both, as defined above. With regards the CR domain, the target binding site preferably comprises module 6 of the CR domain, as defined above.

The low affinity target binding site in IR may comprise amino acids 693-710 (encompassing the C-terminal region of the IR α-chain) plus one or more of the following amino acid sequences: (i) amino acids 1-156; (ii) amino acids 157-310, and; (iii) amino acids 594 and 794.

With regards to amino acids 1-156, the target binding site preferably comprises at least one amino acid selected from Arg14, Asn15, Gln34, Leu36, Leu37, Phe39, Pro43, Phe46, Leu62, Phe64, Leu87, Phe88, Phe89, Asn90, Phe96, Glu97, Arg118, Glu120 or His144.

With regards to amino acids 157-310, the target binding site preferably comprises at least one amino acid from the amino acid sequence 192-310, more preferably at least one amino acid from the sequence 227-303, yet more preferably least one amino acid selected from the sequence 259-284.

With regards to amino acids 594 and 794, the target binding site preferably comprises at least one amino acid selected from Asn594 or Arg794.

In a preferred embodiment, van der Waals and/or hydrophobic interactions account for the major portion of the binding energy between a compound and a low affinity insulin binding site of IR.

The three-dimensional structure of the C-terminal region of the IR α-chain provided by the present invention can also be used to identify or more clearly elucidate potential target binding sites on IGF-1R ectodomain (i.e. to identify those regions, or at least more accurately elucidate those regions, of IGF-1R ectodomain involved in and important to the binding of IGF and signal transduction) as well as in methods used for identifying or designing compounds which interact with potential target binding sites of IGF-1R ectodomain, e.g. potential modulators of IGF-1R.

Preferred target binding sites are those governing specificity, i.e. those regions of IGF-1R ectodomain involved in the initial low affinity binding of IGF (i.e. the initial binding of IGF to IGF-1R).

The low affinity binding site of IGF-1R is a region of IGF-1R ectodomain involved in IGF-I binding to the receptor. Preferred low affinity target binding sites comprise the C-terminal region of IGF-1R α-chain and one or more regions from the L1 domain and/or the CR domain of IGF-1R ectodomain. With regards to the L1 domain, the target binding site preferably comprises the central β-sheet of the L1 domain, and/or that part of the second LRR containing Ser35, and/or the loop in the fourth LRR rung of the L1 domain, or preferably all of these, as defined above. With regards the CR domain, the target binding site preferably comprises module 6 of the CR domain, as defined above.

The low affinity IGF binding site may comprise amino acids 681-697 (encompassing the C-terminal region of the IGF-1R α-chain) plus one or more amino acids from the following amino acid sequences: (i) amino acids 1-149; and (ii) amino acids 150-298.

With regards to amino acids 1-149, the target binding site preferably comprises at least one amino acid from the amino acid sequence 1-62, preferably 1-49, and more preferably amino acid sequence 23-49. With regards to amino acids 150-298, the target binding site preferably comprises at least one amino acid from the amino acid sequence 185-298, more preferably at least one amino acid from the sequence 220-294, yet more preferably least one amino acid selected from the sequence 252-273. The target binding site preferably comprises at least one amino acid selected from Arg10, His30, Leu32, Leu33, Leu56, Phe58, Arg59, Phe82, Tyr83, Asn84, Tyr85, Va188, Phe90, Argy112 and Asp136.

In a preferred embodiment, van der Waals and/or hydrophobic interactions account for the major portion of the binding energy between a compound and a low affinity binding site of IGF-1R.

Additional preferred binding sites in the case of both IR and IGF-1R, particularly for biological macromolecules such as proteins or aptamers, are those that are devoid of glycosylation or devoid of steric hindrance from glycan covalently attached to the polypeptide at sites in the spatial vicinity.

### Design, selection, fitting and assessment of chemical entities that bind IR and/or IGF-1R

Using a variety of known modelling techniques, the crystal structure can be used to produce a model for the low affinity binding site of IR and/or IGF-1R, or at least part of the C-terminal region of the α-chain of IR or IGF-1R.

As used herein, the term "modelling" includes the quantitative and qualitative analysis of molecular structure and/or function based on atomic structural information and interaction models. The term "modelling" includes conventional numeric-based molecular dynamic and energy minimisation models, interactive computer graphic models, modified molecular mechanics models, distance geometry and other structure-based constraint models.

Molecular modelling techniques can be applied to the atomic coordinates of the low affinity binding site of IR and/or IGF-1R, or at least part of the C-terminal region of the α-chain of IR or IGF-1R, or a region thereof to derive a range of 3D models and to investigate the structure of binding sites, such as the binding sites of monoclonal antibodies, nonimmunoglobulin binding proteins and inhibitory peptides.

These techniques may also be used to screen for or design small and large chemical entities which are capable of binding IR and modulating the ability of IR to interact with extracellular biological targets, such as insulin or members of the IGF receptor family e.g. which modulate the ability of IR to heterodimerise. The screen may employ a solid 3D screening system or a computational screening system.

Such modelling methods are to design or select chemical entities that possess stereochemical complementary to the low affinity binding site of IR and/or IGF-1R, or to the regions of the L1 domain of IR and/or IGF-1R with which the C-terminal region of the α-chain of IR and/or IGF-1R interact: By "stereochemical complementarity" we mean that the compound or a portion thereof makes a sufficient number of energetically favourable contacts with the receptor as to have a net reduction of free energy on binding to the receptor.

Such stereochemical complementarity is characteristic of a molecule that matches intra-site surface residues lining the groove of the receptor site as enumerated by the coordinates set out in Appendix I, optionally also utilising the coordinates set out in Appendixes II to VI. By "match" we mean that the identified portions interact with the surface residues, for example, via hydrogen bonding or by non-covalent Van der Waals and Coulomb interactions (with surface or residue) which promote desolvation of the molecule within the site, in such a way that retention of the molécule at the binding site is favoured energetically.

It is preferred that the stereochemical complementarity is such that the compound has a K_{d} for the receptor site of less than 10⁻⁴M, more preferably less than 10⁻⁵M and more preferably 10⁻⁶M. In a most preferred embodiment, the K_{d} value is less than 10⁻⁸M and more preferably less than 10⁻⁹M.

Chemical entities which are complementary to the shape and electrostatics or chemistry of the receptor site characterised by amino acids positioned at atomic coordinates set out in Appendixes I to IV will be able to bind to the receptor, and when the binding is sufficiently strong, substaritially prohibit the interaction of the IR and/or IGF-1R ectodomain with biological target molecules such as insulin or IGF.

It will be appreciated that it is not necessary that the complementarity between chemical entities and the receptor site extend over all residues of the receptor site in order to inhibit binding of a molecule or complex that naturally interacts with IR and/or IF-1R ectodomain.

A number of methods may be used to identify chemical entities possessing stereochemical complementarity to the low affinity binding site of IR and/or IGF-1R, or to the regions of the L1 domain of IR and/or IGF=1R with which the C-terminal region of the α-chain of IR and/or IGF-1R interact. For instance, the process may begin by visual inspection of the entire low affinity insulin binding site comprising the C-terminal region of the α-chain of IR, or the equivalent region in IGF-1R, on the computer screen based on the coordinates in Appendixes I to IV generated from the machine-readable storage medium. Alternatively, selected fragments or chemical entities may then be positioned in a variety of orientations, or docked, within the low affinity binding site of IR and/or IGF-1R, or within the L1 domain of IR and/or IGF-1R in a manner similar to the C-terminal region of the α-chain of IR or IGF-1R, as defined supra. Similar methods could be used to identify chemical entities or compounds that may interact with the L1 domain of IR and/or IGF-1R in a manner similar to that of the C-terminal region of the α-chain of IR and/or IGF-1R.

Modelling software that is well known and available in the art may be used (Guida, 1994). These include Discovery Studio (Accelrys Software Inc., San Diego), SYBYL (Tripos Associates, Inc., St. Louis, Mo., 1992), Maestro (Schrodinger LLC, Portland), MOE (Chemical Computing Group Inc., Montreal, Canada). This modelling step may be followed by energy minimization with standard molecular mechanics force fields such as AMBER (Weiner *et al.,* 1984), OPLS (Jorgensen and Tirado-Rives, 1988) and CHARMM (Brooks *et al.,* 1983). In addition, there are a number of more specialized computer programs to assist in the process of selecting the binding moieties of this invention.

Specialised computer programs may also assist in the process of selecting fragments or chemical entities. These include, inter alia:
1. GRID (Goodford, 1985). GRID is available from Molecular Discovery Ltd., Italy.
2. AUTODOCK (Goodsell & Olsen, 1990). AUTODOCK is available from Scripps Research Institute, La Jolla, CA.
3. DOCK (Kuntz *et al.,* 1982). DOCK is available from University of California, San Francisco, CA.
4. GLIDE (Friesner *et al.,* 2004). GLIDE is available from Schrödinger LLC, Portland.
5. GOLD (Cole *et al.,* 2005). GOLD is avaible from The Cambridge, Crystallographic Data Centre, Cambridge, UK.

Once suitable chemical entities or fragments have been selected, they can be assembled into a single compound. In one embodiment, assembly may proceed by visual inspection of the relationship of the fragments to each other on the three-dimensional image displayed on a computer screen in relation to the structure coordinates of the low affinity binding site of IR and/or IGF-1 R, or the L1 domain to which the C-terminal region of the α-chain of IR or IGF-1R binds. This is followed by manual model building using software such as Discovery Studio, Maestro, MOE or Sybyl. Alternatively, fragments may be joined to additional atoms using standard chemical geometry.

The above-described evaluation process for chemical entities may be performed in a similar fashion for chemical compounds.

Useful programs to aid one of skilled in the art in connecting the individual chemical entities or fragments include:
1. CAVEAT (Bartlett *et al.,* 1989). CAVEAT is available from the University of California, Berkeley, CA.
2. GANDI (Day and Caflisch, 2008). GANDI is available from the University of Zurich.

Other molecular modeling techniques may also be employed in accordance with this invention, see, e.g., Cohen *et al.* (1990) and Navia & Murcko (1992).

There are two preferred approaches to designing a molecule that complement the stereochemistry of the low affinity binding site of IR and/or IGF-1R, or the L1 domain to which the C-terminal region of the α-chain of IR or IGF-1R binds. The first approach is to *in silico* directly dock molecules from a three-dimensional structural database, to the target binding site, using mostly, but not exclusively, geometric criteria to assess the goodness-of-fit of a particular molecule to the site. In this approach, the number of internal degrees of freedom (and the corresponding local minima in the molecular conformation space) is reduced by considering only the geometric (hard-sphere) interactions of two rigid bodies, where one body (the active site) contains "pockets" or "grooves" that form binding sites for the second body (the complementing molecule).

Flexibility of the receptor, IR or IGFR, can be incorporated into the *in silico* screening by the application of multiple conformations of the receptor (Totrov and Abagyan, 2008). The multiple conformations of the receptor can be generated from the coordinates listed in Appendixes 1 to VI computationally by use of molecular dynamics simulation or similar approaches.

This approach is illustrated by Kuntz *et al.* (1982) and Ewing *et al.* (2001), whose algorithm for ligand design is implemented in a commercial software package, DOCK version 4.0, distributed by the Regents of the University of California and further described in a document, provided by the distributor, which is entitled "Overview of the DOCK program suite" the contents of which are hereby incorporated by reference. Pursuant to the Kuntz algorithm, the shape of the cavity in which the C-terminal region of the α-chain of IR or IGF-1R fits is defined as a series of overlapping spheres of different radii. One or more extant databases of crystallographic data, such as the Cambridge Structural Database System (The Cambridge Crystallographic Data Centre, Cambridge, U.K.), the Protein Data Bank maintained by the Research Collaboratory for Structural Bioinformatics (Rutgers University, N.J., U.S.A.), LeadQuest (Tripos Associates, Inc., St. Louis, MO), Available Chemicals Directory (Symyx Technologies Inc.), and the NCI database (National Cancer Institute, U.S.A) is then searched for molecules which approximate the shape thus defined.

Molecules identified on the basis of geometric parameters, can then be modified to satisfy criteria associated with chemical complementarity, such as hydrogen bonding, ionic interactions and van der Waals interactions. Different scoring functions can be employed to rank and select the best molecule from a database. See for example Bohm & Stahl (1999). The software package FlexX, marketed by Tripos Associates, Inc. (St. Louis, MO) is another program that can be used in this direct docking approach (see Rarey *et al.,* 1996).

The second preferred approach entails an assessment of the interaction of respective chemical groups ("probes") with the active site at sample positions within and around the site, resulting in an array of energy values from which three-dimensional contour surfaces at selected energy levels can be generated. The chemical-probe approach to ligand design is described, for example, by Goodford, (1985), and is implemented in several commercial software packages, such as GRID (product of Molecular Discovery Ltd., Italy).

Pursuant to this approach, the chemical prerequisites for a site-complementing molecule are identified at the outset, by probing the active site with different chemical probes, e.g., water, a methyl group, an amine nitrogen, a carboxyl oxygen, or a hydroxyl. Favoured sites for interaction between the active site and each probe are thus determined, and from the resulting three-dimensional pattern of such sites a putative complementary molecule can be generated. This may be done either by programs that can search three-dimensional databases to identify molecules incorporating desired pharmacophore patterns or by programs which use the favoured sites and probes as input to perform de novo design. Suitable programs for determining and designing pharmacophores include CATALYST (Accelrys Software, Inc), and CERIUS2, DISCO (Abbott Laboratories, Abbott Park, IL; distributed by Tripos Associates Inc.).

The pharmacophore can be used to screen in silico compound libraries/ three-dimensional databases, using a program such as CATALYST (Accelrys Software, Inc) and Sybyl/3DB Unity (Tripos Associates, Inc., St. Louis, MO).

Databases of chemical structures are available from a number of sources including Cambridge Crystallographic Data Centre (Cambridge, U.K.), Molecular Design, Ltd., (San Leandro, CA), Tripos Associates, Inc. (St. Louis, MO), Chemical Abstracts Service (Columbus, OH), the Available Chemical Directory (Symyx Technologies, Inc.), the Derwent World Drug Index (WDI), BioByteMasterFile, the National Cancer Institute database (NCI), Medchem Database (BioByte Cortp.), and the Maybridge catalogue.

De novo design programs include LUDI (Accelrys Software Inc., San Diego, CA), Leapfrog (Tripos Associates, Inc.), and LigBuilder (Peking University, China).

Once an entity or compound has been designed or selected by the above methods, the efficiency with which that entity or compound may bind to IR and/or IGF-1R can be tested and optimised by computational evaluation. For example, a compound that has been designed or selected to function as an IR binding compound must also preferably traverse a volume not overlapping that occupied by the binding site when it is bound to the native IR. An effective IR binding compound must preferably demonstrate a relatively small difference in energy between its bound and free states (i.e., a small deformation energy of binding). Thus, the most efficient IR and/or IGF-1R binding compound should preferably be designed with a deformation energy of binding of not greater than about 10 kcal/mole, preferably, not greater than -7 kcal/mole. IR and/or IGF-1R binding compounds may interact with IR and/or IGF-1R in more than one conformation that are similar in overall binding energy. In those cases, the deformation energy of binding is taken to be the difference between the energy of the free compound and the average energy of the conformations observed when the compound binds to the protein.

A compound designed or selected as binding to IR and/or IGF-1R may be further computationally optimised so that in its bound state it would preferably lack repulsive electrostatic interaction with the target protein.

Such non-complementary (e.g., electrostatic) interactions include repulsive charge-charge, dipole-dipole and charge-dipole interactions. Specifically, the sum of all electrostatic interactions between the compound and the protein when the compound is bound to IR and/or IGF-1R, preferably make a neutral or favourable contribution to the enthalpy of binding.

Once an IR and/or IGF-1R-binding compound has been optimally selected or designed, as described above, substitutions may then be made in some of its atoms or side groups to improve or modify its binding properties. Generally, initial substitutions are conservative, i.e., the replacement group will have approximately the same size, shape, hydrophobicity and charge as the original group. It should, of course, be understood that components known in the art to alter conformation should be avoided. Such substituted chemical compounds may then be analysed for efficiency of fit to IR by the same computer methods described in detail above.

Specific computer software is available in the art to evaluate compound deformation energy and electrostatic interaction. Examples of programs designed for such uses include: Gaussian 03, (Frisch, Gaussian, Inc., Pittsburgh, PA); GAMESS (Gordon et al., Iowa State University); Jaguar (Schrödinger LLC, Portland);AMBER, version 9.0 (Case et al, University of California at San Francisco); CHARMM (Accelrys Software, Inc., San Diego, CA); and.GROMACS version 4.0 (van der Spoel et al.).

The screening/design methods may be implemented in hardware or software, or a combination of both. However, preferably, the methods are implemented in computer programs executing on programmable computers each comprising a processor, a data storage system (including volatile and non-volatile memory and/or storage elements), at least one input device, and at least one output device. Program code is applied to input data to perform the functions described above and generate output information. The output information is applied to one or more output devices, in known fashion. The computer may be, for example, a personal computer, microcomputer, or workstation of conventional design.

Each program is preferably implemented in a high level procedural or object-oriented programming language to communicate with a computer system. However, the programs can be implemented in assembly or machine language, if desired. In any case, the language may be compiled or interpreted language.

Each such computer program is preferably stored on a storage medium or device (e.g., ROM or magnetic diskette) readable by a general or special purpose programmable computer, for configuring and operating the computer when the storage media or device is read by the computer to perform the procedures described herein. The system may also be considered to be implemented as a computer-readable storage medium, configured with a computer program, where the storage medium so configured causes a computer to operate in a specific and predefined manner to perform the functions described herein.

### Compounds

Compounds described herein include both those designed or identified using a screening method of the invention and those which are capable of recognising and binding to the low affinity binding sites of IR and/or IGF-1R, as defined above. Also described are compounds that bind to the L1 domain of IR and/or IGF-1R in a manner similar to that of the C-terminal region of the α-chain of IR and/or IGF-1R, i.e. compounds which mimic the C-terminal region of the α-chain of IR and/or IGF-1R.

Compounds capable of recognising and binding to the low affinity binding site of IR and/or IGF-1R may be produced using a screening method based on use of the atomic coordinates corresponding to the 3D structure of the the low affinity binding site of IR and/or IGF-1R, or alternatively may be identified by screening against a specific target molecule which is indicative of the capacity to bind to the low affinity binding site of IR and/or IGF-1R.

Compounds capable of recognising and binding to the L1 domain of IR and/or IGF-1R in a manner similar to that of the C-terminal region of the α-chain of IR and/or IGF-1R (i.e. compounds which mimic the C-terminal region of the α-chain of IR and/or IGF-1R) may be produced using a screening method based on use of the atomic coordinates corresponding to the 3D structure of the the C-terminal region of the α-chain of IR and/or IGF-1R in isolation or as it associates with IR and/or IGF-1R, or alternatively may be identified by screening against a specific target molecule which is indicative of the capacity to bind to the low affinity binding site of IR and/or IGF-1R.

The candidate compounds and/or compounds identified or designed using a method of the present invention may be any suitable compound, synthetic or naturally occurring, preferably synthetic. In one embodiment, a synthetic compound selected or designed by the methods of the invention preferably has a molecular weight equal to or less than about 5000, 4000, 3000, 2000, 1000 or 500 daltons. A compound as described herein is preferably soluble under physiological conditions.

The compounds may encompass numerous chemical classes, though typically they are organic molecules, preferably small organic compounds having a molecular weight of more than 50 and less than about 2,500 daltons, preferably less than 1500, more preferably less than 1000 and yet more preferably less than 500. Such compounds can comprise functional groups necessary for structural interaction with proteins, particularly hydrogen bonding, and typically include at least an amine, carbonyl, hydroxyl or carboxyl group, preferably at least two of the functional chemical groups. The compound may comprise cyclical carbon or heterocyclic structures and/or aromatic or polyaromatic structures substituted with one or more of the above functional groups. Compounds can also comprise biomolecules including peptides, saccharides, fatty acids, steroids, purines, pyrimidines, derivatives, structural analogs, or combinations thereof.

Compounds may include, for example: (1) Peptides such as soluble peptides, including Ig-tailed fusion peptides and members of random peptide libraries (see, e.g., Lam *et al.,* 1991; Houghten *et al.,* 1991) and combinatorial chemistry-derived molecular libraries made of D-and/or L-configuration amino acids; (2) Phosphopeptides (e.g., members of random and partially degenerate, directed phosphopeptide libraries, see, e.g., Songyang *et al.,* 1993); (3) Antibodies (e.g., polyclonal, monoclonal, humanized, anti-idiotypic, chimeric, and single chain antibodies as well as Fab, (Fab)_{2'}, Fab expression library and epitope-binding fragments of antibodies); (4) Nonimmunoglobulin binding proteins such as but not restricted to avimers, DARPins and lipocalins; (5) Nucleic acid-based aptamers; and (6) Small organic and inorganic molecules.

Ligands can be obtained from a wide variety of sources including libraries of synthetic or natural compounds. Synthetic compound libraries are commercially available from, for example, Maybridge Chemical Co. (Tintagel, Cornwall, UK), AMRI (Budapest, Hungary) and ChemDiv (San Diego, CA), Specs (Delft, The Netherlands).

Natural compound libraries comprising bacterial, fungal, plant or animal extracts are available from, for example, Pan Laboratories (Bothell, WA), TimTec (Newark, DE). In addition, numerous means are available for random and directed synthesis of a wide variety of organic compounds and biomolecules, including expression of randomized oligonucleotides.

Alternatively, libraries of natural compounds in the form of bacterial, fungal, plant and animal extracts can be readily produced. Methods for the synthesis of molecular libraries are readily available (see, e.g., DeWitt *et al.,* 1993; Erb *et al.,* 1994; Zuekermann *et al.,* 1994; Cho *et al.,* 1993; Carell *et al.,* 1994a; Carell *et al.,* 1994b; and Gallop *et al.,* 1994). In addition, natural or synthetic compound libraries and compounds can be readily modified through conventional chemical, physical and biochemical means (see, e.g., Blondelle and Houghton, 1996), and may be used to produce combinatorial libraries. In another approach, previously identified pharmacological agents can be subjected to directed or random chemical modifications, such as acylation, alkylation, esterification, amidification, and the analogs can be screened for IR and/or IGF-1R-modulating activity.

Numerous methods for producing combinatorial libraries are known in the art, including those involving biological libraries; spatially addressable parallel solid phase or solution phase libraries ; synthetic library methods requiring deconvolution ; the "one-bead one-compound" library method; and synthetic library methods using affinity chromatography selection. The biological library approach is limited to polypeptide or peptide libraries, while the other four approaches are applicable to polypeptide, peptide, non-peptide oligomer, or small molecule libraries of compounds (Lam, 1997).

Compounds also include those that may be synthesized from leads generated by fragment-based drug design, wherein the binding of such chemical fragments is assessed by soaking or co-crystallizing such screen fragments into crystals provided by the invention and then subjecting these to an X-ray beam and obtaining diffraction data. Difference Fourier techniques are readily applied by those skilled in the art to determine the location within the IR ectodomain and/or IGF-1R ectodomain structure at which these fragments bind, and such fragments can then be assembled by synthetic chemistry into larger compounds with increased affinity for the receptor.

### Isolated peptides or mimetics thereof

Compounds identified or designed using the methods of the invention can be a peptide or a mimetic thereof. In one example, there is described an isolated peptide or mimetic thereof which binds the L1 domain of IR and/or the L1 domain of IGF-1R, the peptide comprising:
i) an amino acid sequence as provided in SEQ ID NO: 13 or SEQ ID NO: 15;
ii) an amino acid sequence which is at least 50% identical to SEQ ID NO: 13 and/or SEQ ID NO: 15; or
iii) a fragment of i) or ii) which binds the L1 domain of IR and/or the L1 domain of IGF-1R,
wherein the peptide has a helical structure.

The isolated peptides or mimetics may be conformationally constrained molecules or alternatively molecules which are not conformationally constrained such as, for example, non-constrained peptide sequences. The term "conformationally constrained molecules" means conformationally constrained peptides and conformationally constrained peptide analogues and derivatives.

The term "analogues" refers to molecules having a chemically analogous structure to naturally occurring α-amino acids. Examples include molecules containing gemdiaminoalkyl groups or alklylmalonyl groups.

The term "derivatives" includes α-amino acids wherein one or more side groups found in the naturally occurring α-amino acids have been modified. Thus, for example the amino acids may be replaced with a variety of uncoded or modified amino acids such as the corresponding D-amino acid or N-methyl amino acid. Other modifications include substitution of hydroxyl, thiol, amino and carboxyl functional groups with chemically similar groups.

With regard to peptides and mimetics thereof, other examples of other unnatural amino acids or chemical amino acid analogues/derivatives which can be introduced as a substitution or addition include, but are not limited to, 2,4-diaminobutyric acid, α-amino isobutyric acid, 4-aminobutyric acid, 2-aminobutyric acid, 6-amino hexanoic acid, 2-amino isobutyric acid, 3-amino propionic acid, ornithine, norleucine, norvaline, hydroxyproline, sarcosine, citrulline, homocitrulline, cysteic acid, t-butylglycine, t-butylalanine, phenylglycine, cyclohexylalanine, β-alanine, fluoro-amino acids, designer amino acids such as β-methyl amino acids, Cα-methyl amino acids, Nα-methyl amino acids, and amino acid analogues in general.

The mimetic may be a peptidomimetic. A "peptidomimetic" is a molecule that mimics the biological activity of a peptide but is no longer peptidic in chemical nature. By strict definition, a peptidomimetic is a molecule that no longer contains any peptide bonds (that is, amide bonds between amino acids). However, the term peptide mimetic is sometimes used to describe molecules that are no longer completely peptidic in nature, such as pseudo-peptides, semi-peptides and peptoids. Whether completely or partially non-peptide, peptidomimetics, provide a spatial arrangement of reactive chemical moieties that closely resembles the three-dimensional arrangement of active groups in the peptide on which the peptidomimetic is based. As a result of this similar active-site geometry, the peptidomimetic has effects on biological systems which are similar to the biological activity of the peptide.

There are sometimes advantages for using a mimetic of a given peptide rather than the peptide itself, because peptides commonly exhibit two undesirable properties: (1) poor bioavailability; and (2) short duration of action. Peptide mimetics offer an obvious route around these two major obstacles, since the molecules concerned are small enough to be both orally active and have a long duration of action. There are also considerable cost savings and improved patient compliance associated with peptide mimetics, since they can be administered orally compared with parenteral administration for peptides. Furthermore, peptide mimetics are generally cheaper to produce than peptides.

Suitable peptidomimetics based on the C-terminal region of the α-chain of IR and/or IGR-1R can be developed using readily available techniques. Thus, for example, peptide bonds can be replaced by non-peptide bonds that allow the peptidomimetic to adopt a similar structure, and therefore biological activity, to the original peptide. Further modifications can also be made by replacing chemical groups of the amino acids with other chemical groups of similar structure. The development of peptidomimetics derived from peptides of the C-terminal region of the IR and/or IGF-1R α-chain can be aided by reference to the three dimensional structure of these residues as provided in Appendixes I to IV. This structural information can be used to search three-dimensional databases to identify molecules having a similar structure, using programs such as Sybyl/3DB Unity (Tripos Associates, St. Louis, MO).

Those skilled in the art will recognize that the design of a peptidomimetic may require slight structural alteration or adjustment of a chemical structure designed or identified using the methods of the invention. In general, chemical compounds identified or designed using the methods of the invention can be synthesized chemically and then tested for ability to modulate IR and/or IGF-1R activity using any of the methods described herein. The methods of the invention are particularly useful because they can be used to greatly decrease the number potential mimetics which must be screened for their ability to modulate IR and/or IGF-1R activity.

The peptides or peptidomimetics can be used in assays for screening for candidate compounds which bind to regions of IR and/or IGF-1R and potentially interfere with the binding of insulin to IR and/or signal transduction and/or the binding of IGF to IGF-1R and/or signal transduction. Peptides or peptidomimetics which mimic target binding sites are particularly useful as specific target molecules for identifying potentially useful ligands for IR and/or IGF-1R.

Standard solid-phase ELISA assay formats are particularly useful for identifying compounds that bind to the receptor. In accordance with this embodiment, the peptide or peptidomimetic immobilized on a solid matrix, such as, for example an array of polymeric pins or a glass support. Conveniently, the immobilized peptide or peptidomimetic is a fusion polypeptide comprising Glutathione-S-transferase (GST; e.g. a CAP-ERK fusion), wherein the GST moiety facilitates immobilization of the protein to the solid phase support. This assay format can then be used to screen for candidate compounds that bind to the immobilised peptide or peptidomimetic and/or interefere with binding of a natural binding partner of IR and/or IGF-1R to the immobilised peptide or peptidomimetic.

As used herein a "fragment" is a portion of a peptide which maintains a defined activity of the "full-length" peptide, namely the ability to bind to the low affinity binding site of IR and/or IGF-1R, or to bind to the L1 domain of IR and/or IGF-1R. Fragments can be any size as long as they maintain the defined activity. Preferably, the fragment maintains at least 50%, more preferably at least 75%, of the activity of the full length polypeptide.

The % identity of a peptide is determined by GAP (Needleman and Wunsch, 1970) analysis (GCG program) with a gap creation penalty=5, and a gap extension penalty=0.3. The query sequence is at least 10 amino acids in length, and the GAP analysis aligns the two sequences over a region of at least 10 amino acids. More preferably, the GAP analysis aligns two sequences over their entire length.

With regard to a defined peptide, it will be appreciated that % identity figures higher than those provided above will encompass preferred embodiments. Thus, where applicable, in light of the minimum % identity figures, it is preferred that the peptide comprises an amino acid sequence which is at least 50%, more preferably at least 55%, more preferably at least 60%, more preferably at least 65%, more preferably at least 70%, more preferably at least 75%, more preferably at least 80%, more preferably at least 85%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably at least 99.1%, more preferably at least 99.2%, more preferably at least 99.3%, more preferably at least 99.4%, more preferably at least 99.5%, more preferably at least 99.6%, more preferably at least 99.7%, more preferably at least 99.8%, and even more preferably at least 99.9% identical to the relevant nominated SEQ ID NO.

Amino acid sequence mutants of the peptides identified or designed using the methods of the invention, can be prepared by introducing appropriate nucleotide changes into a nucleic acid as described herein, or by *in vitro* synthesis of the desired peptide. Such mutants include, for example, deletions, insertions or substitutions of residues within the amino acid sequence. A combination of deletion, insertion and substitution can be made to arrive at the final construct, provided that the final peptide product possesses the desired characteristics.

In designing amino acid sequence mutants, the location of the mutation site and the nature of the mutation will depend on characteristic(s) to be modified. The sites for mutation can be modified individually or in series, e.g., by (1) substituting first with conservative amino acid choices and then with more radical selections depending upon the results achieved, (2) deleting the target residue, or (3) inserting other residues adjacent to the located site.

Substitution mutants have at least one amino acid residue in the peptide removed and a different residue inserted in its place. Sites of interest are those in which particular residues obtained from various strains or species are identical. These sites, especially those falling within a sequence of at least three other identically conserved sites, are preferably substituted in a relatively conservative manner. Such conservative substitutions are shown in Table 1 under the heading of "exemplary substitutions".

**Table 1 - Exemplary substitutions.**

| **Original Residue** | **Exemplary Substitutions** |
|---|---|
| Ala (A) | val; leu; ile; gly |
| Arg (R) | lys |
| Asn (N) | gln; his |
| Asp (D) | glu |
| Cys (C) | ser |
| Gln (Q) | asn; his |
| Glu (E) | asp |
| Gly (G) | pro, ala |
| His (H) | asn; gln |
| Ile (I) | leu; val; ala |
| Leu (L) | ile; val; met; ala; phe |
| Lys (K) | arg |
| Met (M) | leu; phe |
| Phe (F) | leu; val; ala |
| Pro (P) | gly |
| Ser (S) | thr |
| Thr (T) | ser |
| Trp (W) | tyr |
| Tyr (Y) | trp; phe |
| Val (V) | ile; leu; met; phe, ala |

In a preferred embodiment a mutant/variant peptide has one or two or three or four conservative amino acid changes when compared to a peptide defined herein. Details of conservative amino acid changes are provided in Table 1.

Also described herein are peptides which are differentially modified during or after synthesis, e.g., by biotinylation, benzylation, glycosylation, acetylation, phosphorylation, amidation, derivatization by known protecting/blocking groups, proteolytic cleavage, linkage to an antibody molecule or other cellular ligand, etc. These modifications may serve to increase the stability and/or bioactivity of the peptide.

The residues that form the IR segment 693-710 can be grouped into three classes :-Class A: those whose side chains are completely buried in the interface with L1 (viz. F701 and F705); Class B: those whose side chains lie at the periphery of the interface with L1 (viz. K694, E697, E698, S700, R702, T704, Y708 and L709); and Class C: those whose side chains appear to have no interaction with L1 (viz. L693, E695, L696, S699, K703, E706, D707 and H710). In terms of using the 693-710 peptide itself as a scaffold for mimetics that might compete with the C-terminal region of the IR α-chain peptide in its binding to the L1 domain, design might focus in the first instance on substitution of those residues in belonging to Class B, given that the two residues lying in Class A are relatively optimally packed within the interface and already have few degrees of freedom. Higher affinity binding might be achieved by substitution of one or more of the Class B residues with either naturally-occuring amino acids or non-natural amino acids. For example, the substitution of one or more of the charged residues K694, E697, E698 and R702 with residues that have reduced rotameric degrees of freedom (i.e. reduced entropy) may lead to higher affinity binding **or** altered physicochemical properties of the compound. Such modification for example may include substitution by the naturally occuring amino acids Phe, Tyr or Trp. As a further example, in the case of K694 and R702, it may be possible to substitute these residues by more bulky non-natural amino acids that retain the terminal cationic character, for example substitution by the basic phenyl propanoic acid derivatives App (L-2-amino-3-(4-aminophenyl)propanoic acid) and/or Gpp, (L-2-amino-3-(4-guanidinophenyl)propanoic acid) (Svenson *et al.,* 2009). A further strategy for design might involve substitutions to improve the overall stability of the helical structure (for example helix stapling - see Danial *et al.,* 2008). Such substiutions would likely be made within Class C residues. A yet further strategy to improve affinity or physicochemical properties might involve truncation of the helical segment and/or attaching an N- or C-terminal group also designed to improve affinity. Similar principles of design may be applied to generate modified peptides based on the native IGF-1R peptide 681-697 as outlined above for the IR peptide.

In a particularly preferred example, a peptide or mimetic thereof does not comprise any one of the following described in US 7,173,005:
a) X₁ X₂ X₃ X₄ X₅ wherein X₁, X₂, X₄ and X₅ are aromatic amino acids, and X₃ is any polar amino acid;
b) X₆ X₇ X₈ X₉ X₁₀ X₁₁ X₁₂ X₁₃ wherein X₆ and X₇ are aromatic amino acids, X₈, X₉, X₁₁ and X₁₂ are any amino acid, and X₁₀ and X₁₃ are hydrophobic amino acids;
c) X₁₄ X₁₅ X₁₆ X₁₇ X₁₈ X₁₉ X₂₀ X₂₁ wherein X₁₄, and X₁₇ are hydrophobic amino acids, X₁₅, X₁₆, X₁₈ and X₁₉ are any amino acid, and X₂₀ and X₂₁, are aromatic amino acids;
d) X₂₂ X₂₃ X₂₄ X₂₅ X₂₆ X₂₇ X₂₈ X₂₉ X₃₀ X₃₁ X₃₂ X₃₃ X₃₄ X₃₅ X₃₆ X₃₇ X₃₈ X₃₉ X₄₀ X₄₁ wherein X₂₂, X₂₅, X₂₈, X₂₉, X₃₀, X₃₃, X₃₄, X₃₅, X₃₆, X₃₇, X₃₈, X₄₀, and X₄₁ are any amino acid, X₃₅ and X₃₇ may be any amino acid for binding to IR, whereas X₃₅ is preferably a hydrophobic amino acid and X₃₇ is preferably glycine for binding to IGF-1R and possess agonist or antagonist activity. X₂₃ and X₂₆ are hydrophobic amino acids. This sequence further comprises at least two cysteine residues, preferably at X₂₅ and X₄₀ X₃₁ and X₃₂ are small amino acids;
e) X₄₂ X₄₃ X₄₄ S₄₅ X₄₆ X₄₇ X₄₈ X₄₉ X₅₀ X₅₁ X₅₂ X₅₃ X₅₄ X₅₅ X₅₆ X₅₇ X₅₈ X₅₉ X₆₀ X₆₁ wherein X₄₂, X₄₃, X₄₄, X₄₅, X₅₃₃ X₅₅, X₅₆, X₅₈, X₆₀ and X₆₁ may be any amino acid, X₄₃, X₄₆, X₄₉, X₅₀, X₅₄ are hydrophobic amino acids, X₄₇ and X₅₉ are preferably cysteines, X₄₈ is a polar amino acid, and X₅₁, X₅₂ and X₅₇ are small amino acids;
f) X₆₂ X₆₃ X₆₄ X₆₅ X₆₆ X₆₇ X₆₈ X₆₉ X₇₀ X₇₁ X₇₂ X₇₃ X₇₄ X₇₅ X₇₆ X₇₇ X₇₈ X₇₉ X₈₀ X₈₁ wherein X₆₂, X₆₅, X₆₈, X₆₉, X₇₁, X₇₃, X₇₆, X₇₇, X₇₈, X₈₀, and X₈₁ may be any amino acid; X₆₃, X₇₀, X₇₄ are hydrophobic amino acids; X₆₄ is a polar amino acid, X₆₇ and X₇₅ are aromatic amino acids and X₇₂ and X₇₉ are preferably cysteines capable of forming a loop;
g) H X₈₂ X₈₃ X₈₄ X₈₅ X₈₆ X₈₇ X₈₈ X₈₉ X₉₀ X₉₁ X₉₂ wherein X₈₂ is proline or alanine, X₈₃ is a small amino acid, X₈₄ is selected from leucine, serine or threonine, X₈₅ is a polar amino acid, X₈₆, X₈₈, X₈₉ and X₉₀ are any amino acid, and X₈₇, X₉₁ and X₉₂ are an aliphatic amino acid;
h) X₁₀₄ X₁₀₅ X₁₀₆ X₁₀₇ X₁₀₈ X₁₀₉ X₁₁₀ Y₁₁₁ X₁₁₂ X₁₁₃ X₁₁₄ wherein at least one of the amino acids of X₁₀₆ through X₁₁₁, and preferably two, are tryptophan separated by three amino acids, and wherein at least one of X₁₀₄, X₁₀₅ and X₁₀₆ and at least one of X₁₁₂, X₁₁₃ and X₁₁₄ are cysteine;
i) an amino acid sequence comprising the sequence JBA5: DYKDLCQSWGVRIGWLAGLCPKK or JBA5 minus FLAG® tag and terminal lysines: LCQSWGVRIGWLAGLCP (Formula 9); and
j) W X₁₂₃ G Y X₁₂₄ W X₁₂₅ X₁₂₆ wherein X₁₂₃ is selected from proline, glycine, serine, arginine, alanine or leucine, but more preferably proline; X₁₂₄ is any amino acid, but preferably a charged or aromatic amino acid; X₁₂₅ is a hydrophobic amino acid preferably leucine or phenylalanine, and most preferably leucine. X₁₂₆ is any amino acid, but preferably a small amino acid.

In a further preferred example, a peptide or mimetic thereof is more structurally similar to the native C-terminal region of the α-chain of IR and/or the C-terminal region of the α-chain of IGF-1R than it is to any one of a) to j) above (such as the peptides provided as SEQ ID Nos: 16 to 18).

The design of synthetic non-peptide mimetics of α-helices is an established art (see for example Davis *et al*., 2006). In particular, methods of mimicry of i, i+4, i+7 motifs (such as those identified within the C-terminal helical region of the α-chain of IR and IGF-1R and which interact the respective L1 domains (i.e. IR residues Phe701, Phe705, Tyr708 and IGF-1R residues Tyr688, Phe 692, Phe 695) are known. For example, these motifs may be mimicked by terphenyl, oligophenyl, chalcone or 1,4-benzodiazepine-2,5-dione scaffolds (Davis *et al.,* 2006) or by benzoylurea scaffolds (US 2008153802). Non-peptide mimetics of α-helices have been investigated as therapeutics in a number of disease contexts, for example HIV1 infection (disruption of the assembly of the hexameric helical bundle (Ernst *et al*., 2001)) and cancer (disruption of the assembly of the HDM2-p53 complex (Yin *et al*., 2005); inhibitors of Bcl-2 family heterodimerisation (Degterev *et al*., 2001).

With regard to redesigning compounds using a method of the invention, in an embodiment the compound is redesigned to be more structurally similar to the native C-terminal region of the α-chain of IR and/or the C-terminal region of the α-chain of IGF-1 R. Examples of peptides which could be redesigned in this manner include, but are not limited to, those described by Schäffer et al. (2003) and/or US 7,173,005 (see above).

### Interaction of compounds with IR and/or IGF-1R

A compound may interact with the low affinity binding site of IR and/or IGF-1R by binding either directly or indirectly to that region. A compound which binds directly, binds to the specified region. A compound which binds indirectly, binds to a region in close proximity to or adjacent to the low affinity binding site of IR and/or IGF-1R with the result that it interferes with the ability of IR to bind to insulin, or IGF-1R to bind IGF, either antagonistically or agonistically. Such interference may be steric, electrostatic, or allosteric. Preferably, a compound interacts with the low affinity binding site of IR and/or IGF-1R by binding directly to the specified region. In the case of compounds that bind to specific target molecules, such compounds bind directly to the specific target molecule.

A compound may alternatively interact with the L1 domain of IR and/or IGF-1R in a manner similar to that of the C-terminal region of the α-chain of IR and/or IGF-1R by binding either directly or indirectly to that region. A compound which binds directly, binds to the specified region. A compound which binds indirectly, binds to a region in close proximity to or adjacent to the L1 domain of IR and/or IGF-1R in a manner similar to that of the C-terminal region of the α-chain of IR and/or IGF-1R with the result that it interferes with the ability of IR to bind to insulin, or IGF-1R to bind IGF, either antagonistically or agonistically. Such interference may be steric, electrostatic, or allosteric. Preferably, a compound interacts with the L1 domain of IR and/or IGF-1R in a manner similar to that of the C-terminal region of the α-chain of IR and/or IGF-1R by binding directly to the specified region. In the case of compounds that bind to specific target molecules, such compounds bind directly to the specific target molecule.

Binding can be either by covalent or non-covalent interactions, or both. Examples of non-covalent interactions include electrostatic interactions, van der Waals interactions, hydrophobic interactions and hydrophilic interactions.

When a compound interacts with IR and/or IGF-1R, it preferably "modulates" IR or IGF-1R, respectively. By "modulate" we mean that the compound changes an activity of IR or IGF-1R by at least 10%. Suitably, a compound modulates IR or IGF-1R by increasing or decreasing signal transduction via IR or IGF-1R, respectively. The phrase "decreases signal transduction" is intended to encompass partial or complete inhibition of signal transduction via IR or IGF-1R. The ability of a candidate compound to increase or decrease signal transduction via IR or IGF-1R can be assessed by any one of the IR or IGF-1R cell-based assays described herein.

Compounds may act as antagonists or agonists for insulin binding to IR or as antagonists or agonists for IGF binding to IGF-1R.

Compounds preferably have an affinity for IR or IGF-1R sufficient to provide adequate binding for the intended purpose. Suitably, such compounds and compounds which bind to specific target molecules of IR or IGF-1R have an affinity (K_{d}) of from 10⁻⁵ to 10⁻¹⁵ M. For use as a therapeutic, the compound suitably has an affinity (K_{d}) of from 10⁻⁷ to 10⁻¹⁵ M, preferably from 10⁻⁸ to 10⁻¹² M and more preferably from 10⁻¹⁰ to 10⁻¹² M. Where a compound is to be used as a reagent in a competitive assay to identify other ligands, the compound suitably has an affinity (K_{d}) of from 10⁻⁵ to 10⁻¹² M.

As will be evident to the skilled person, the crystal structures presented herein have enabled, for the first time, direct comparison of the regions controlling insulin or IGF binding in the closely related IR and IGF-1R. The structures have enabled the identification of the C-terminal region of the α-chain of IR and IGF-1R, critical for the initial binding of insulin or IGF, respectively, and in the subsequent formation of the high affinity insulin-IR or IGF-IGF-1R complex that leads to signal transduction.

In one preferred embodiment, a compound has a high specificity for IR and/or a specific target molecule of IR but not for IGF-1R, i.e. a compound selectively binds to IR or has enhanced selectivity for IR over IGF-1R. In this respect, a compound suitably has an affinity (K_{d}) for IR and/or a specific target molecule of IR of no more than 10⁻⁵ M, preferably no more than 10⁻⁷ M, and an affinity for IGF-1R of at least 10⁻⁵ M, preferably at least 10⁻³ M. Such compounds are desirable as, for example, IR agonists where the propensity to interact with IGF-1R and thus, for example, promote undesirable cell proliferation, is reduced.

In a preferred embodiment, the (IR or specific target molecule of IR)/IGF-1R binding affinity ratio for a compound is at least 10 and preferably at least 100.

In another preferred embodiment, a compound has a high specificity for IGF-1R and/or a specific target molecule of IGF-1R but not for IR, i.e. a compound selectively binds to IGF-1R or has enhanced selectivity for IGF-1R over IR. In this respect, a compound suitably has an affinity (K_{d}) for IGF-1R and/or a specific target molecule of IGF-1R of no more than 10⁻⁵ M, preferably no more than 10⁻⁷ M, and an affinity for IR of at least 10⁻⁵ M, preferably at least 10⁻³ M. Such compounds are desirable as, for example, IGF-1R agonists where there propensity to interact with IR and thus, for example, promote glucose uptake and metabolism, is reduced.

In a preferred embodiment, the (IGF-1R or specific target molecule of IGF-1 R)/(IR) binding affinity ratio for a compound is at least 10 and preferably at least 100.

### Screening assays and confirmation of binding

Compounds described herein may be subjected to further confirmation of binding to IR and/or IGF-1R by cocrystallization of the compound with IR and/or IGF-1R and structural determination, as described herein.

Compounds designed or selected according to the methods of the present invention are preferably assessed by a number of *in vitro* and *in vivo* assays of IR and/or IGF-1R function to confirm their ability to interact with and modulate IR and/or IGF-1R activity. For example, compounds may be tested for their ability to bind to IR and/or IGF-1R and/or for their ability to modulate e.g. disrupt, IR and/or IGF-1R signal transduction.

Libraries may be screened in solution by methods generally known in the art for determining whether ligands competitively bind at a common binding site. Such methods may include screening libraries in solution (e.g., Houghten, 1992), or on beads (Lam, 1991), chips (Fodor, 1993), bacteria or spores (U.S. 5,223,409), plasmids (Cull *et al.,* 1992), or on phage (Scott & Smith, 1990; Devlin, 1990; Cwirla *et al.,* 1990; Felici, 1991; U.S. 5,223,409).

Where the screening assay is a binding assay, IR or IGF-1R may be joined to a label, where the label can directly or indirectly provide a detectable signal. Various labels include radioisotopes, fluorescent molecules, chemiluminescent molecules, enzymes, specific binding molecules, particles, e.g., magnetic particles, and the like. Specific binding molecules include pairs, such as biotin and streptavidin, digoxin and antidigoxin, etc. For the specific binding members, the complementary member would normally be labeled with a molecule that provides for detection, in accordance with known procedures.

A variety of other reagents may be included in the screening assay. These include reagents like salts, neutral proteins, e.g., albumin, detergents, etc., which are used to facilitate optimal protein-protein binding and/or reduce non-specific or background interactions. Reagents that improve the efficiency of the assay, such as protease inhibitors, nuclease inhibitors, antimicrobial agents, etc., may be used. The components are added in any order that produces the requisite binding. Incubations are performed at any temperature that facilitates optimal activity, typically between 4 and 40 °C.

Direct binding of compounds to IR or IGF-1R can also be done by Surface Plasmon Resonance (BIAcore) (reviewed in Morton & Myszka, 1998). Here the receptor is immobilized on a CM5 or other sensor chip by either direct chemical coupling using amine or thiol-disulphide exchange coupling (Nice & Catimel, 1999) or by capturing the receptor ectodomain as an Fc fusion protein to an appropriately derivatised sensor surface (Morten & Myszka, 1998). The potential binding molecule (called an analyte) is passed over the sensor surface at an appropriate flow rate and a range of concentrations. ' The classical method of analysis is to collect responses for a wide range of analyte concentrations. A range of concentrations provides sufficient information about the reaction, and by using a fitting algorithm such as CLAMP (see Morton & Myszka, 1998), rate constants can be determined (Morton & Myszka, 1998; Nice & Catimel, 1999). Normally, the ligand surface is regenerated at the end of each analyte binding cycle. Surface regeneration ensures that the same number of ligand binding sites is accessible to the analyte at the beginning of each cycle.

Incubation periods are selected for optimum activity, but may also be optimized to facilitate rapid high-throughput screening. Normally, between 0.1 and 1 hour will be sufficient. In general, a plurality of assay mixtures is run in parallel with different test agent concentrations to obtain a differential response to these concentrations. Typically, one of these concentrations serves as a negative control, i.e. at zero concentration or below the level of detection.

The basic format of an *in vitro* competitive receptor binding assay as the basis of a heterogeneous screen for small organic molecular replacements for insulin may be as follows: occupation of the low affinity binding site of IR and/or IGF-1R is quantified by time-resolved fluorometric detection (TRFD) as described by Denley *et al.,* 2004. R⁻IR-A, R⁻IR-B and P6 cells are used as sources of IR-A, IR-B and IGF-1R respectively. Cells are lysed with lysis buffer (20 mM HEPES, 150 mM NaCl, 1.5 mM MgCl₂, 10% (v/v) glycerol, 1% (v/v) Triton X-100, 1 mM EGTA pH 7.5) for 1 hour at 4°C. Lysates are centrifuged for 10 minutes at 3500 rpm and then 100 µl is added per well to a white Greiner Lumitrac 600 plate previously coated with anti-insulin receptor antibody 83-7 or anti-IGF-1R antibody 24-31. Neither capture antibody interferes with receptor binding by insulin, IGF-I or IGF-II. Approximately 100,000 fluorescent counts of europium-labelled insulin or europium-labelled IGF-I are added to each well along with various amounts of unlabelled competitor and incubated for 16 hours at 4°C. Wells are washed with 20 mM Tris, 150 mM NaCl, 0.05% (v/v) Tween 20 (TBST) and DELFIA enhancement solution (100 µl/well) is added. Time-resolved fluorescence is measured using 340 nm excitation and 612 nm emission filters with a BMG Lab Technologies Polarstar^{™} Fluorimeter or a Wallac Victor II (EG & G Wallac, Inc.).

Examples of other suitable assays which may be employed to assess the binding and biological activity of compounds to and on IR are well known in the art. For example, suitable assays may be found in PCT International Publication Number WO 03/027246. Examples of suitable assays include the following:
(i) Receptor autophosphorylation (as described by Denley *et al*., 2004). R⁻ IR-A, R⁻IR-B cells or P6 cells are plated in a Falcon 96 well flat bottom plate at 2.5 x 10⁴ cells/well and grown overnight at 37°C, 5% CO₂. Cells are washed for 4 hours in serum-free medium before treating with one of either insulin, IGF-I or IGF-II in 100µl DMEM with 1% BSA for 10 minutes at 37°C, 5% CO₂. Lysis buffer containing 2mM Na₃VO₄ and 1 mg/ml NaF is added to cells and receptors from lysates are captured on 96 well plates precoated with antibody 83-7 or 24-31 and blocked with 1x TBST/0.5% BSA. After overnight incubation at 4°C, the plates are washed with 1 x TBST. Phosphorylated receptor is detected with europium-labelled antiphosphotyrosine antibody PY20 (130 ng/well, room temperature, 2 hours). DELFIA enhancement solution (100 µl/well) is added and time resolved fluorescence detected as described above.
(ii) Glucose uptake using 2-deoxy-[U-14C] glucose (as described by Olefsky, 1978). Adipocytes between days 8-12 post-differentation in 24-well plates are washed twice in Krebs-Ringer Bicarbonate Buffer (25mM Hepes, pH 7.4 containing 130 mM NaCl, 5 mM KCl, KH₂PO₄, 1.3 mM MgSO₄.7H₂O, 25·mM NaHCO₃ and 1.15 mM CaCl₂) supplemented with 1% (w/v) RIA-grade BSA and 2 mM sodium pyruvate. Adipocytes are equilibrated for 90 min at 37°C prior to insulin addition, or for 30 min prior to agonist or antagonist addition. Insulin (Actrapid, Novogen) is added over a concentration range of 0.7 to 70 nM for 30 min at 37°C. Agonist or antagonist (0 to 500 µM) is added to adipocytes for 90 min followed by the addition of insulin in the case of antagonists. Uptake of 50 µM 2-deoxy glucose and 0.5 µCi 2-deoxy-[U-¹⁴C] glucose (NEN, PerkinElmer Life Sciences) per well is measured over the final 10 min of agonist stimulation by scintillation counting.
(iii) Glucose transporter GLUT4 translocation using plasma membrane lawns (as described by Robinson & James (1992) and Marsh *et al.* (1995)).
(iv) GLUT4 translocation using plasma membrane lawns (as described by Marsh *et al.,* 1995). 3T3-L1 fibroblasts are grown on glass coverslips in 6-well plates and differentiated into adipocytes. After 8-12 days post-differentiation, adipocytes are serum-starved for 18 hrs in DMEM containing 0.5% FBS. Cells are washed twice in Krebs-Ringer Bicarbonate Buffer, pH 7.4 and equilibrated for 90 min at 37°C prior to insulin (100nM) addition, or for 30 min prior to compound (100µM) addition. After treatments, adipocytes are washed in 0.5 mg/ml poly-L-lysine in PBS, shocked hypotonically by three washes in 1:3 (v/v) membrane buffer (30 mM Hepes, pH 7.2 containing 70 mM KCl, 5 mM MgCl₂, 3 mM EGTA and freshly added 1 mM DTT and 2 mM PMSF) on ice. The washed cells are then sonicated using a probe sonicator (Microson) at setting 0 in 1:1 (v/v) membrane buffer on ice, to generate a lawn of plasma membrane fragments that remain attached to the coverslip. The fragments are fixed in 2% (w/v) paraformaldehyde in membrane buffer for 20 min at 22°C and the fixative quenched by 100 mM glycine in PBS. The plasma membrane fragments are then blocked in 1% (w/v) Blotto in membrane buffer for 60 min at 22°C and immunolabelled with an in-house rabbit affinity purified anti-GLUT4 polyclonal antibody (clone R10, generated against a peptide encompassing the C-terminal 19 amino acids of GLUT4) and Alexa 488 goat anti-rabbit secondary antibody (Molecular Probes; 1:200). Coverslips are mounted onto slides using FluoroSave reagent (Calbiochem), and imaged using an OptiScan confocal laser scanning immunofluoroscence microscope (Optiscan, VIC., Australia). Data are analysed using ImageJ (NIH) imaging software. At least six fields are examined within each experiment for each condition, and the confocal microscope gain settings over the period of experiments are maintained to minimise between-experiment variability.

Insulin agonist activity may be determined using an adipocyte assay. Insulin increases uptake of ³H glucose into adipocytes and its conversion into lipid. Incorporation of ³H into a lipid phase is determined by partitioning of lipid phase into a scintillant mixture, which excludes water-soluble ³H products. The effect of compounds on the incorporation of ³H glucose at a sub-maximal insulin dose is determined, and the results expressed as increase relative to full insulin response. The method is adapted from Moody *et al*., (1974). Mouse epididymal fat pads are dissected out, minced into digestion buffer (Krebs-Ringer 25 mM HEPES, 4% HSA, 1.1 mM glucose, 0.4 mg/ml Collagenase Type 1, pH 7.4), and digested for up to 1.5 hours at 36.5 C. After filtration, washing (Krebs-Ringer HEPES, 1% HSA) and resuspension in assay buffer (Krebs-Ringer HEPES, 1% HSA), free fat cells are pipetted into 96-well Picoplates containing test solution and approximately an ED₂₀ insulin.

The assay is started by addition of ³H glucose (e.g. ex. Amersham TRK 239), in a final concentration of 0.45 mM glucose. The assay is incubated for 2 hours at 36.5 °C, in a Labshaker incubation tower, 400 rpm, then terminated by the addition of Permablend/Toluene scintillant (or equivalent), and the plates sealed before standing for at least 1 hour and detection in a Packard Top Counter or equivalent. A full insulin standard curve (8 dose) is run as control on each plate.

Data are presented graphically, as the effect of the compound on an (approximate) ED₂₀ insulin response, with data normalized to a full insulin response. The assay can also be run at basal or maximal insulin concentration.

To test the *in vivo* activity of a compound, an intravenous blood glucose test may be carried out on Wistar rats as follows. Male Mol:Wistar rats, weighing about 300 g, are divided into two groups. A 10 µl sample of blood is taken from the tail vein for determination of blood glucose concentration. The rats are then anaesthetized (e.g. with Hypnorm/Dormicum) at t =30 min and blood glucose measured again at t =-20 min and at t = 0 min. After the t = 0 sample is taken, the rats are injected into the tail vein with vehicle or test substance in an isotonic aqueous buffer at a concentration corresponding to a 1ml/kg volume of injection. Blood glucose is measured at times 10, 20, 30, 40, 60, 80, 120 and 180 min. The anaesthetic administration is repeated at 20 min intervals.

Additional assays to determine the effect of binding molecules on IGF-1R activity are as follows:
(i) Cell Viability Assay on HT29 cells with induction of Apoptosis: The ability of compounds to inhibit IGF-mediated rescue from apoptosis is measured using the colorectal cell line HT29 cells (ATCC: HTB 38) after induction with Na Butyrate. The HT29 cells are plated out onto white Fluoronunc 96 well plates (Nunc) at 12,000cells/ml and incubated at 37°C, 5% CO₂ for 48 hours. Media is aspirated and 100µl/well of serum free DMEM/F12 is added for 2 hours to serum starve cells. IGF (100µl/ well 0.05-50 nM dilutions) in the presence and the absence of inhibitory compound is added in 0.1% BSA solution (Sigma) in DMEM/F12 (Gibco) in triplicate. A final concentration of 5mM Butyrate (Sigma) is added to each well. Plates are incubated at 37°C, 5% CO₂ for a further 48 hours. Plates are brought to room temperature and developed (as per instructions for CTG Assay (Promega)). Luminescence signal is measured on the Polarstar plate reader and data is evaluated using table curve to obtain the specific ED50.
(ii) Cell Migration Assay: The migration assays are performed in the modified 96-well Boyden chamber (Neuroprobe, Bethesda, MA). An 8 µM polycarbonate filter, which is pre-soaked in 25 µg/ml of collagen in 10 mM acetic acid overnight at 4 °C, is placed so as to divide the chamber into an upper & lower compartment. Varying concentrations of the IGF-I analogues (25 µl of 0-100 nM) diluted in RPMI (Gibco) with 0.5% BSA (Sigma) tested for their migration inducing ability, are placed in the lower compartment in quadruplicates. The wells of the upper chamber are seeded with 50µl/well of 2x10⁵ SW480 (ATCC:CCL 228) pre-incubated for 30mins/37°C with 1.1µl of 2µM Calcein (Molecular Probes). Cells migrate for 8 hours at 37°C, 5% CO₂. Unmigrated cells are removed by wiping the filter. The filter is then analysed in the Polarstar for fluorescence at excitation wavelength of 485nm and emission wavelength of 520nm. Data is evaluated using table curve to obtain the specific ED50 value.
(iii) Mouse Xenograft studies for anti-IGF-1R antibodies: *In vivo* studies are performed in 56-week-old female athymic BALBc nude mice, homozygous for the nunu allele. Mice are maintained in autoclaved micro-isolator cages housed in a positive pressure containment rack (Thoren Caging Systems Inc., Hazelton, PA, USA. To establish xenografts, mice are injected subcutaneously into the left inguinal mammary line with 3 x 10⁶ or 5 x 10⁶ cells in 100 µl of PBS. Tumour volume (TV) is calculated by the formula (length x width²)/2 (Clarke *et al.,* 2000), where length is the longest axis and width the measurement at right angles to length.
   Initial biodistribution of potential binding molecules are ascertained by injecting 40 BALBc nude mice with established xenografts with radiolabelled ¹¹¹In- or ¹²⁵I-anti-IGFR antibody (3 µg, 10 µCi) intravenously via the tail vein (total volume=0.1 ml). At designated time points after injection of the radioconjugates (*t*=4 h, days 1, 2, 3, 5 and 7), groups of mice (*n*=35) are killed by Ethrane anaesthesia. Mice are then exsanguinated by cardiac puncture, and tumours and organs (liver, spleen, kidney, muscle, skin, bone (femur), lungs, heart, stomach, brain, small bowel, tail and colon) are resected immediately. All samples are counted in a dual gamma scintillation counter (Packard Instruments). Triplicate standards prepared from the injected material are counted at each time point with tissue and tumour samples enabling calculations to be corrected for the physical decay of the isotopes. The tissue distribution data are calculated as the mean ± s.d. percent injected dose per gram tissue (%ID g⁻¹) for the candidate molecule per time point.
   Pharmacokinetics for the candidate compounds are ascertained as follows: Serum obtained from mice bearing xenografts, following infusion of radiolabelled-binding molecule as described above, is aliquoted in duplicate and counted in a gamma scintillation counter (Packard Instruments, Melbourne, Australia). Triplicate standards prepared from the injected material are counted at each time point with serum samples to enable calculations to be corrected for the isotope physical decay. The results of the serum are expressed as % injected dose per litre (% ID 1⁻¹). Pharmacokinetic calculations are performed of serum data using a curve fitting program (WinNonlin, Pharsight Co., Mountain View, CA, USA). A two-compartment model is used to calculate serum pharmacokinetic parameters of AUC (area under the serum concentration curve extrapolated to infinite time), CL (total serum clearance), T_{12α} and T_{12β} (half-lives of the initial and terminal phases of disposition) for ¹²⁵I- and ¹¹¹In-labelled molecule.
(iv) Therapeutic *in vivo* studies: Tumour cells (3 x 10⁶) in 100 µl of media are inoculated subcutaneously into both flanks of 46-week-old female nude mice (*n*=5 group⁻¹). Candidate molecule treatment commences day 7 post-tumour cell inoculations (mean ± s.e. tumour volume=60x15 mm³) and consists of six intraperitoneal injections over 2 weeks of appropriate amounts of the candidate molecule or vehicle control. Tumour volume in mm³ is determined as described previously. Data is expressed as mean tumour volume for each treatment group. Differences in tumour size between control and test groups are tested for statistical significance (*P*<0.05) by *t*-test.

### Uses of compounds

Compounds/chemical entities designed or selected by the methods of the invention described above may be used to modulate IR and/or IGF-1R activity in cells, i.e. activate or inhibit IR and/or IGF-1R activity. Such compounds may interact with the low affinity binding sites of IR and/or IGF-1R as defined herein, or mimic the C-terminal region of the α-chain of IR and/or IGF-1R as defined herein. They may also be used to modulate homodimerisation of IR and/or IGF-1R.

Modulation of homodimerisation of IR and/or IGF-1R may be achieved by direct binding of the chemical entity to a homodimerisation surface of IR and/or IGF-1R, and/or by an allosteric interaction elsewhere in the IR and/or IGF-1R extracellular domain.

Given that aberrant IR and/or IGF-1R activity is implicated in a range of disorders, the compounds described above may also be used to treat, ameliorate or prevent disorders characterised by abnormal IR and/or IGF-1R signalling. Examples of such disorders include malignant conditions including tumours of the brain, head and neck, prostate, ovary, breast, cervix, lung, pancreas and colon; and melanoma, rhabdomyosarcoma, mesothelioma, squamous carcinomas of the skin and glioblastoma.

The compounds designed to interact or identified as interacting with the extracellular domain of IR and/or IGF-1R, and in particular to interact with the target binding sites, are useful as agonists or antagonists against the action of insulin on IR and/or IGF on IGF-1R. The compounds are useful as assay reagents for identifying other useful ligands by, for example, competition assays, as research tools for further analysis of IR and/or IGF-1R and as potential therapeutics in pharmaceutical compositions.

Compounds described herein are also useful as lead compounds for identifying other more potent or selective compounds. The mimetic compounds described herein are also potentially useful as inhibitors of the action of insulin and in the design of assay kits directed at identifying compounds capable of binding to the low affinity binding site for insulin on IR. The mimetic compounds are also potentially useful as inhibitors of the action of IGF and in the design of assay kits directed at identifying compounds capable of binding to the low affinity binding site for IGF on IGF-1R. In particular, it is envisaged that compounds described herein will prove particularly useful in selecting/designing ligands which are specific for IR or IGF-1R.

In one example, one or more of the compounds can be provided as components in a kit for identifying other ligands (e.g., small, organic molecules) that bind to IR or IGF-1R. Such kits may also comprise IR or IGF-1R, or functional fragments thereof. The compound and receptor components of the kit may be labeled (e.g. by radioisotopes, fluorescent molecules, chemiluminescent molecules, enzymes or other labels), or may be unlabeled and labelling reagents may be provided. The kits may also contain peripheral reagents such as buffers, stabilizers, etc. Instructions for use can also be provided.

IR and IGF-1R agonists and antagonists, and in particular antagonists, provided by this invention are potentially useful as therapeutics. For example, compounds are potentially useful as treatments for cancers, including, but not limited to, breast, prostate, colorectal, and ovarian cancers. Human and breast cancers are responsible for over 40,000 deaths per year, as present treatments such as surgery, chemotherapy, radiation therapy, and immunotherapy show limited success. Recent reports have shown that a previously identified IGF-1R antagonist can suppress retinal neovascularization, which causes diabetic retinopathy (Smith *et al*., 1999). IGF-1R agonist compounds (i.e. existing IGF-1R compounds which have been modified employing methods of the present invention) are useful for development as treatments for neurological disorders, including stroke and diabetic neuropathy. Reports of several different groups implicate IGF-1R in the reduction of global brain ischemia, and support the use of IGF-I for the treatment of diabetic neuropathy (reviewed in Auer *et al*., 1998; Apfel, 1999). A number of therapeutics directed against IGF-1R are currently undergoing clinical trial as anti-cancer agents (Hewish *et al*., 2009)

The IGF-1R agonist peptides described herein may be useful for enhancing the survival of cells and/or blocking apoptosis in cells.

### Administration

Compounds described herein, i.e. ligands or modulators of IR and/or IGF-1R identified or identifiable by the screening methods of the invention, may preferably be combined with various components to produce the compositions described herein. Preferably the compositions are combined with a pharmaceutically acceptable carrier or diluent to produce a pharmaceutical composition (which may be for human or animal use).

The formulation will depend upon the nature of the compound and the route of administration but typically they can be formulated for topical, parenteral, intramuscular, oral, intravenous, intra-peritoneal, intranasal inhalation, lung inhalation, intradermal or intra-articular administration. The compound may be used in an injectable form. It may therefore be mixed with any vehicle which is pharmaceutically acceptable for an injectable formulation, preferably for a direct injection at the site to be treated, although it may be administered systemically.

The pharmaceutically acceptable carrier or diluent may be, for example, sterile isotonic saline solutions, or other isotonic solutions such as phosphate-buffered saline. The compounds may be admixed with any suitable binder(s), lubricant(s), suspending agent(s), coating agent(s), solubilising agent(s). It is also preferred to formulate the compound in an orally active form.

In general, a therapeutically effective daily oral or intravenous dose of the compounds, including compounds and their salts, is likely to range from 0.01 to 50 mg/kg body weight of the subject to be treated, preferably 0.1 to 20 mg/kg. The compounds and their salts may also be administered by intravenous infusion, at a dose which is likely to range from 0.001-10 mg/kg/hr.

Tablets or capsules of the compounds may be administered singly or two or more at a time, as appropriate. It is also possible to administer the compounds in sustained release formulations.

Typically, the physician will determine the actual dosage which will be most suitable for an individual patient and it will vary with the age, weight and response of the particular patient. The above dosages are exemplary of the average case. There can, of course, be individual instances where higher or lower dosage ranges are merited.

For some applications, the compositions are administered orally in the form of tablets containing excipients such as starch or lactose, or in capsules or ovules either alone or in admixture with excipients, or in the form of elixirs, solutions or suspensions containing flavouring or colouring agents.

The compositions (as well as the compounds alone) can also be injected parenterally, for example intravenously, intramuscularly or subcutaneously. In this case, the compositions will comprise a suitable carrier or diluent.

For parenteral administration, the compositions are best used in the form of a sterile aqueous solution which may contain other substances, for example enough salts or monosaccharides to make the solution isotonic with blood.

For buccal or sublingual administration the compositions may be administered in the form of tablets or lozenges which can be formulated in a conventional manner.

For oral, parenteral, buccal and sublingual administration to subjects (such as patients), the daily dosage level of the compounds and their pharmaceutically acceptable salts and solvates may typically be from 10 to 500 mg (in single or divided doses). Thus, and by way of example, tablets or capsules may contain from 5 to 100 mg of active compound for administration singly, or two or more at a time, as appropriate. As indicated above, the physician will determine the actual dosage which will be most suitable for an individual patient and it will vary with the age, weight and response of the particular patient.

The routes of administration and dosages described are intended only as a guide since a skilled practitioner will be able to determine readily the optimum route of administration and dosage for any particular patient depending on, for example, the age, weight and condition of the patient.

### Examples

### Example 1: A thermodynamic study of ligand binding to the first three domains of the human insulin receptor (IR): relationship between the IR α-chain C-terminal peptide (αCT) and the Site 1 insulin mimetic peptides

### 1.1 Introduction

In order to study ligand binding to the first three domains of IR ectodomain, isothermal titration calorimetry (ITC) was used. ITC allowed a direct assay of the interactions between the IR ectodomain classical αCT peptide (residues 704-719; SEQ ID NO: 11) and IR485 (a construct consisting of the first three N-terminal domains of the IR; SEQ ID NO: 10), as well as the interaction between the analogous peptide of human IGF-1R and IR485. At the same time the thermodynamics of binding of the N- and C-terminal segments of the insulin mimetic peptide S519 (Schaffer et al., 2003) to IR485, as well as the binding of S519 itself to IR485 were examined. S519 (SLEEEWAQVECEVYGRGCPSGSLDESFYDWFERQLG; SEQ ID NO: 16) is a 36-residue peptide resulting from the affinity-optimization of two covalently-linked peptides, S371, a so-called "Site 1" peptide, and S446, a so-called "Site 2" peptide (Pillutla et al., 2002). S519 binds human IR with K_{d}=2.0 x 10⁻¹¹ (Schaffer et al., 2003). Taken together, the results revealed a remarkable relationship between the IR αCT peptide and the Site 1 mimetic peptide, and suggested a previously undetected structural similarity between the mimetic peptides and insulin itself (Ward and Lawrence, 2009). Finally, the binding to IR485 of the IR αCT peptide carrying a F714A mutation (residues 704-719, TFEDYLHNVVAVPRPS; SEQ ID NO: 12) was examined.

### 1.2 Materials and methods for thermodynamic experiments .

### Reagents

The IR485 construct of human IR was expressed in Lec8 mutant CHO cells and purified by gel filtration chromatography as described previously (Lou et al., 2006). IR485 consists of the first 485 residues of the mature human insulin receptor followed by the 16-residue sequence SDDDDKEQKLISEEDLN (SEQ ID NO: 10), which comprises a serine residue followed by an enterokinase cleavage site and a c-myc epitope tag. The c-myc tag was not removed for the ITC studies described here. IR485 was concentrated to 30 mg/ml in Tris-buffered saline (TBSA; 24.8 mM Tris-HCl (pH 8.0), 137 mM NaCl, 2.7 mM KCl and 0.02% sodium azide) using an Ultrafree centrifugal concentrator (Millipore, USA). Porcine insulin (Sigma-Aldrich, USA) was prepared in a zinc-free-form (termed ZFP-insulin) by extensive dialysis against 0.1% (v/v) acetic acid followed by lyophilization. Human IGF-I was obtained from Novozymes GroPep (Australia) as "receptor grade" material.

The IR classical aCT peptide (residues 704-719, TFEDYLHNVVFVPRPS; SEQ ID NO: 11), the IGF-1R classical αCT peptide (residues 691-706, VFENFLHNSIFVPRPE; SEQ ID NO: 14) as well as the F714A mutant of IR aCT (denoted IR αCT.714A; SEQ ID NO: 12) were obtained from Genscript Corporation (USA) at the >98% level of purity. The S519N20 peptide (SLEEEWAQVECEVYGRGCPS; SEQ ID NO: 17) and the S519C16 peptide (GSLDESFYDWFERQLG; SEQ ID NO: 18) were obtained from AusPep (Australia) at the >90% level of purity. The S519 peptide (SLEEEWAQVECEVYGRGCPSGSLDESFYDWFERQLG; SEQ ID NO: 16) was obtained from Activotec (UK) at the >85% purity. Peptides were prepared by dissolving in 10 mM HCl at ~4 mg/ml concentration and then diluting with TBSA. Oxidation of peptides S519N20 and S519 (which contain two cysteine residues) was carried out by incubating the respective peptide (prepared at 1 mg/ml in 50 mM ammonium bicarbonate adjusted to pH 8.5 with ammonia solution) in the dark at room temperature for 2 days and then lyophilizing before use. Oxidation was complete as determined by analysis with Ellman's reagent (5,5'-dithiobis-2-nitrobenzoate), All protein concentrations were determined by absorbance measurement at 280 nm using a NanoDrop 1000 spectrophotometer (Thermo Scientific, USA).

### Isothermal Titration Calorimetry

ITC experiments were performed using a VP-ITC isothermal titration calorimeter (MicroCal Inc., USA) with the calorimeter cell held at 25 °C. All samples were degassed prior to injection or placement into the cell, and the instrument was temperature equilibrated prior to the start of the injections. In all experiments the volume of sample placed in the cell was 1.4 ml and the titrant was injected in 7 µl volumes over 14 s at 3 min intervals, with the total number of injections being 40. The sample contents were stirred at a speed of 310 rpm over the duration of the titration. All titrants (*i.e.* ZFP-insulin, IGF-I, IR αCT peptide, IGF-1R aCT peptide, IR aCT.714A peptide, S519, S519N20, and S519C20) were first injected into a solution of TBSA alone in order ascertain the heat of dilution, which was then subtracted from the data of interest as appropriate. Data were analyzed using the instrument's software incorporated within the Origin 7 software (OriginLab, USA) and in all cases fitted as a single-site interaction using the methodologies outlined in the instrument's manual. All measurements showing quantifiable interaction were done in triplicate (in some cases at varying concentrations) and the resultant ITC-derived thermodynamic parameters averaged.

### Dynamic light scattering (DLS)

DLS measurements were carried out using a Zetasizer NanoZS (Malvern Instruments Ltd, England). Samples of IR485 and IR485 in combination with IR aCT and/or ZFP-insulin at various concentrations were prepared in TBSA and equilibrated overnight at 4 °C. Samples were spun at 13,000 g using a benchtop centrifuge to pellet any macroscopic particulates and then pipetted into a 45 µl glass cuvette held at 20 °C. Data were analysed using the instrument's Dispersion Technology Software version 5.0.2 to yield a volume distribution for the scattering particles present in the solution. The results presented were representative of several sets of experiments.

### 1.3 Results from thermodynamic experiments

### The pairwise interactions of IR485, hormones and αCT peptides

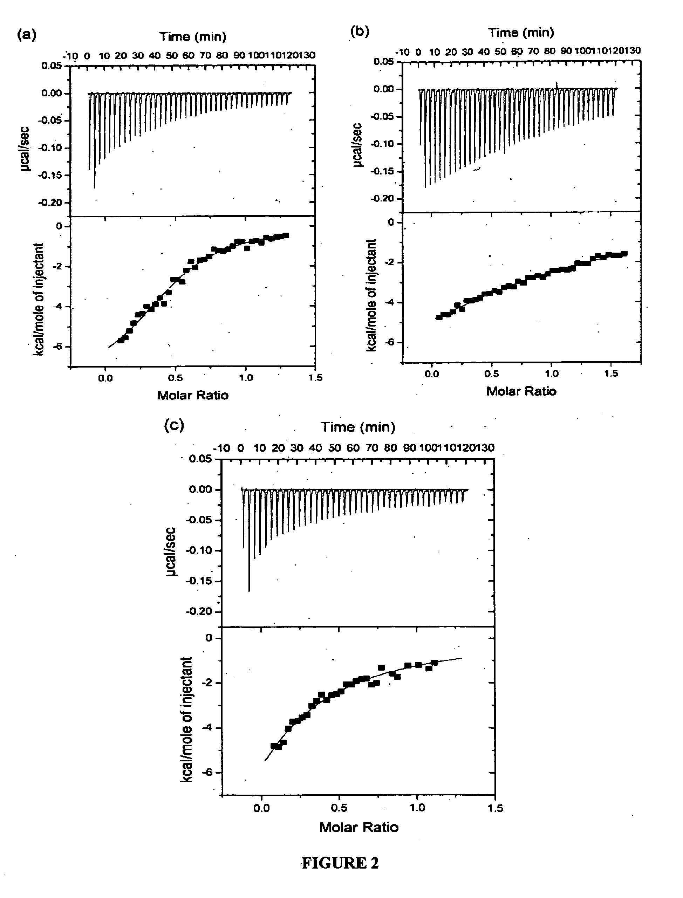

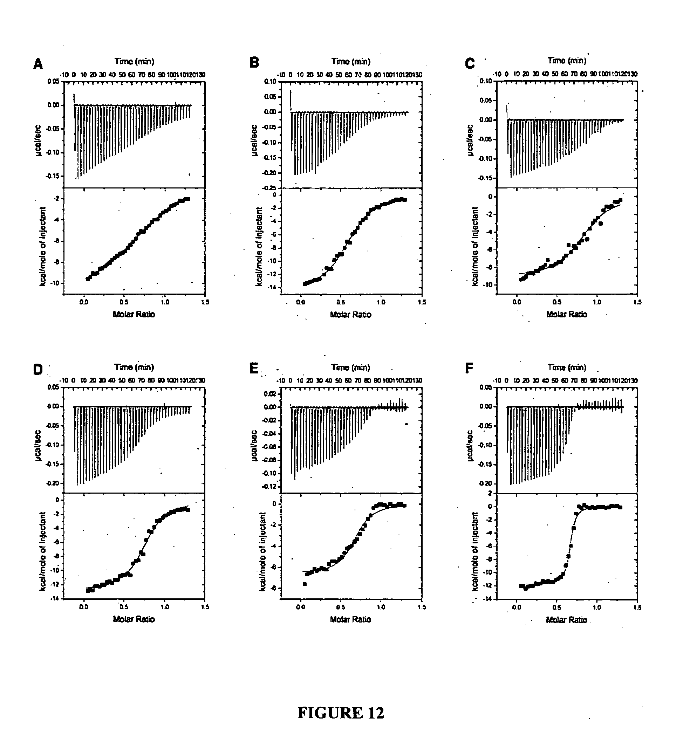

ITC experiments investigated the pairwise interaction between selected combinations of IR485, ZFP-insulin, IGF-I, IR aCT peptide, IGF-1R αCT peptide and IR aCT.714A peptide. The results of the analyses are presented in Table 2. The mean dissociation constants (K_{d}) of the interaction between (i) IR αCT and IR485, (ii) IGF-1R aCT and IR 485, and (iii) IR aCT.714A and IR485 were determined to be 3.9 ± 1.1 µM, 17.6 to µM and 6.5 ± 1.7 µM respectively. Representative ITC profiles for these measurements are presented in Figure 2. The dissociation constant (K_{d}) for the interactions between (i) ZFP-insulin and the IR αCT peptide, (ii) IGF-I and the IGF-1R aCT peptide, (iii)-ZFP-insulin and IR485 and (iv) IGF-I and IR485 was in all cases estimated to be weaker than 1 mM.

**Table 2 - ITC analysis of pairwise interactions of IR485, hormone and αCT peptides^{a}.**

| Sample | [Sample] (µM) | Titrant | [Titrant] (µM) | K_{d} (µM) |
|---|---|---|---|---|
| ZFP-insulin | 20 | IR-αCT | 200 | 1000 |
| IGF-IR | 20 | IGF1R-αCT | 200 | 1000 |
| IR485 | 5 | ZFP-insulin | 50 | 1000 |
| IR485 | 10 | IGF-IR | 100 | 1000 |
| IR485 | 10 | IR-αCT | 60 | 3.9 ± 1.1 |
| IR485 | 20 | IGF1R-αCT | 120 - 150*^{b}* | 17.6 ± 2.1 |
| IR485 | 10 | IR-αCT.714A | 60 | 6.5 ± 1.7 |

| | | | | |
|---|---|---|---|---|
| *^{a}*K_{d} values are of the mean of three experiments with each set of experimental data being modeled as a single-site interaction between titrant and sample (see Materials and Methods). The error stated is the standard error of the mean. The relatively low value of the Wiseman parameter c coupled with the noise in the data precludes accurate determination of ΔH⁰ (and hence of -TΔS⁰). *^{b}*Ranges are quoted for sample or titrant concentration where these vary across the three measurements. | | | | |

### The interaction of hormone with aCT peptide pre-complexed IR485

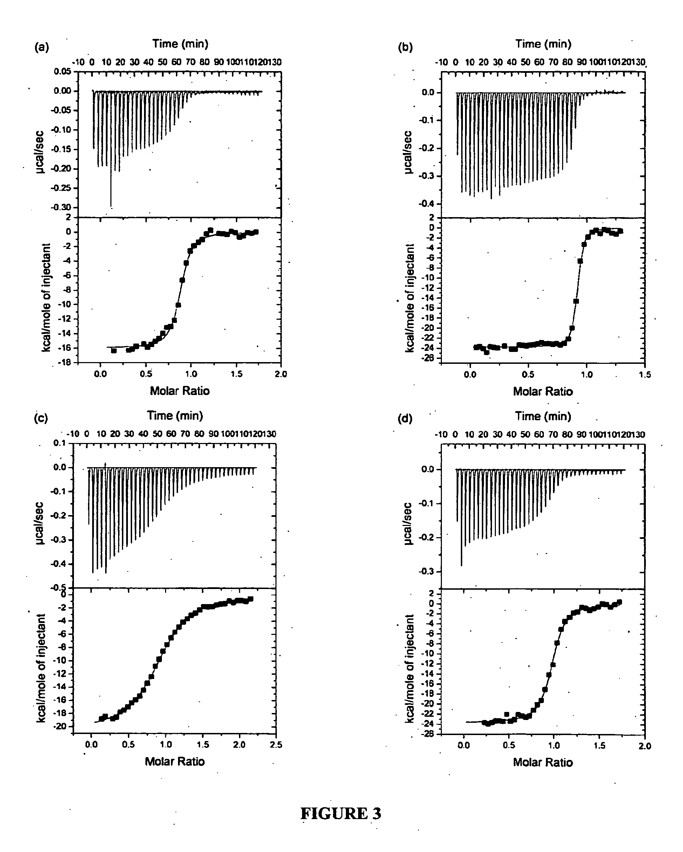

ITC experiments investigated the interaction of ZFP-insulin and IGF-I with IR485 pre-complexed with a ten-fold molar ratio of either IR aCT or IGF-1R aCT peptide. Given that the αCT peptides displayed micromolar affinity for IR485 (see above), it was calculated that, at the concentrations of IR485 and aCT peptide employed in this set of experiments, ≥90% of the IR485 molecules within the ITC cell were in complex with aCT peptide prior to injection of hormone. The results of the ITC investigations are presented in Table 3 with representative ITC curves being presented in Figure 3. The dissociation constant of ZFP-insulin with respect to IR485 in the presence of a 10-fold molar ratio of IR aCT peptide was 17 ± 4nM and in the presence of a 10-fold molar ratio of IGF-1R aCT peptide was 5.7 ± 1.1nM. In contrast, the dissociation constant of IGF-I with respect to IR485 in the presence of a 10-fold excess of IR aCT peptide was 490 ± 75nM and in the presence of a 10-fold excess of IGF-1R aCT peptide was 22 ± 3nM, with no correction being made for the incomplete saturation of IR485 with peptide prior to injection. The thermodynamic parameters presented in Table 3 show that in all four cases the binding of hormone to aCT peptide pre-complexed IR485 is enthalpically driven.

**Table 3 - Isothermal titration of ZFP-insulin and IGF-IR against IR485 pre-mixed with a 10-fold molar ratio of either IR or IGF-1R αCT peptide^{a}.**

| Sample | [Sample] (*µ*M) | Titrant | [Titrant] (*µ*M) | K_{d} (nM) | ΔH⁰ (kJ/mol) | -TΔS⁰ (kJ/mol) |
|---|---|---|---|---|---|---|
| IR485 +(10×IR-αCT) | 5 | ZFP-insulin | 50 | 17 ± 4 | -67 ± 1 | 22 ± 2 |
| IR485 +(10×IGF1R-αCT) | 4-10*^{b}* | ZFP-insulin | 32 - 60 | 5.7 ± 1.1 | -97 ± 2 | 50 ± 2 |
| IR485 +(10×IR-αCT) | 5 - 10 | IGF-IR | 50 - 80 | 490 ± 75 | -89 ± 2 | 53 ± 2 |
| IR485 +(10×IGF1R-αCT) | 4-5 | IGF-IR | 40 - 50 | 22 ± 3 | -101 ± 1 | 57 ± 1 |

| | | | | | | |
|---|---|---|---|---|---|---|
| *^{a}*K_{d}, ΔH⁰ and -TΔS⁰ values are of the mean of three experiments with each set of experimental data being modeled as a single-site interaction between titrant and sample (see Materials and Methods). The error stated is the standard error of the mean. *^{b}* Ranges are quoted for sample or titrant concentration where these vary across the three measurements. | | | | | | |

### The interaction of insulin mimetic peptides with IR485

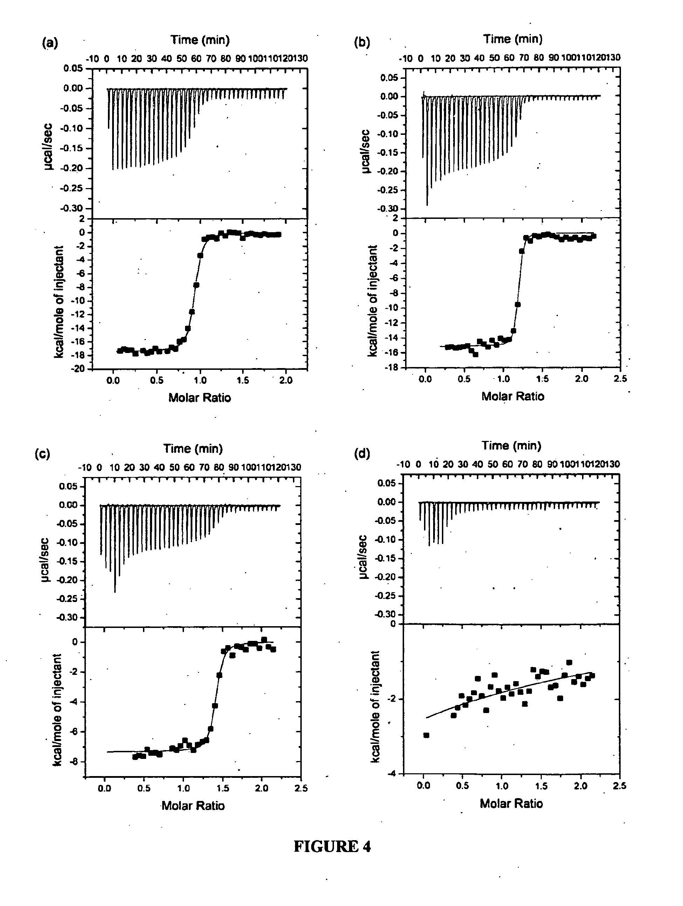

ITC experiments investigated the interaction of the insulin mimetic peptides S519 (SEQ ID NO: 16), S519N20 (a Site 2 peptide, corresponding to the 20 N-terminal residues of S519; SEQ ID NO: 17) and S519C16 (a Site 1 peptide, corresponding to the 16 C-terminal residues of S519; SEQ ID NO: 18) with IR485. The results are presented in Table 4, with representative ITC curves being presented in Figure 4. S519 was found to bind IR485 with a dissociation constant of 11 ± 3nM, whilst S519C16 bound IR485 with somewhat higher affinity (K_{d} = 2.6 ± 0.7nM). The binding of both S519 and S519C16 to IR485 appeared to be enthalpically driven. When IR485 was in the presence of a 10-fold molar ratio of IR αCT peptide, the dissociation constant of S519C16 increased to 16 ± 6nM. The dissociation constant for the interaction between S519N20 and IR485 was estimated to be weaker than 1mM, with accurate determination of K_{d} precluded at the concentrations of reactants employed.

**Table 4 - Isothermal titration of insulin mimetics peptides S519, S519N20 and S519C16 against IR485^{a}.**

| Sample | [Sample] (*µ*M) | Titrant | [Titrant] (*µ*M) | K_{d} (nM) | ΔH⁰ (kJ/mol) | -TΔS⁰ (kJ/mol) |
|---|---|---|---|---|---|---|
| R485 | 4.5-5*^{b}* | S519 | 40-50 | 11 ± 3 | -69 ± 6 | 24 ± 6 |
| R485 | 5 | S519C16 | 50 | 2.6 ± 0.7 | -61 ± 4 | 12 ± 4 |
| R485 10×IR-αCT) | + 3-5 | S519C16 | 45-50 | 16 ± 6 | | -11 ± 3 |
| R485 | 5 | S519N20 | 50 | 1 mM | ^{(*c*)} | |

| | | | | | | |
|---|---|---|---|---|---|---|
| *^{a}* K_{d}, ΔH⁰ and -TΔS⁰ values are of the mean of three experiments with each set of experimental data being modeled as a single-site interaction between titrant and sample (see Materials and Methods). The error stated is the standard error of the mean. *^{b}*Ranges are quoted for sample or titrant concentration where these vary across the three measurements.*^{c}* The affinity of interaction of S519N20 with IR485 is too weak to allow meaningful calculation of either ΔH⁰ or -TΔS⁰. | | | | | | |

### The multimeric state of IR485 in the presence of IR aCT peptide

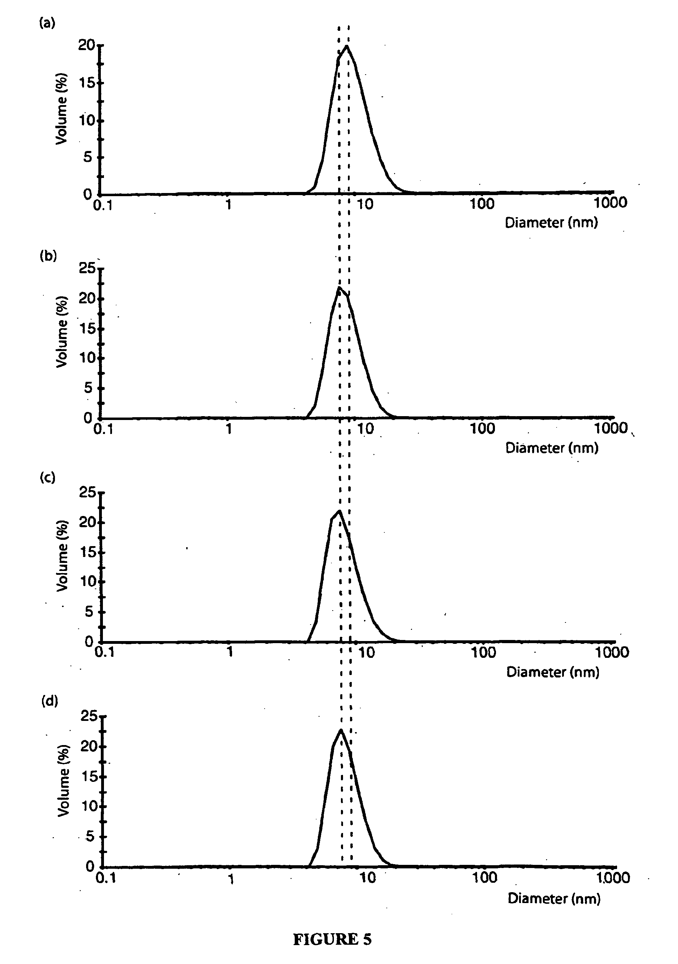

The DLS-determined volume distribution of IR485 at 6 mg/ml (a concentration at which IR485 is overwhelmingly dimeric (Lou et al., 2006) showed a single broad peak centred at a particle diameter of 9.7 nm (Figure 5a). The half-width of the peak was 3.1 nm and the assumption of a spherical particle lead to a calculated scattering particle molecular weight of 136 kDa, closely similar to the molecular weight of ~140 kDa estimated by size-exclusion chromatography. Instrumental limitations precluded DLS measurement of IR485 at concentrations at which IR485 was known to be overwhelmingly monomeric (*i.e.* at < 0.025 mg/ml, (Lou et al., 2006). However, at 0.5 mg/ml, the DLS-determined volume distribution of IR485 showed a single broad peak at a particle diameter of 8.2 nm (Figure 5b). The half-width of the peak was 2.2 nm and the assumption that the scattering arose from a single species of spherical scattering particle lead to a calculated molecular weight of 91 kDa, consistent with the solution being predominantly monomeric. Using this pair of observations as reference values, it was then observed that (i) an addition of a three-food molar ratio of IR αCT peptide to a 6 mg/ml solution of IR485 resulted in a DLS-determined volume distribution which had a single peak centred at 8.6 nm (Figure 5c), and (ii) an addition of a three-fold molar ratio of IR αCT peptide together with a 2-fold molar ratio of ZFP-insulin to a 6 mg/ml solution of IR485 resulted in a volume distribution which had a single peak centred at 8.2 nm (Figure 5d). The half-width of these latter two peaks was 2.4 nm and the calculated molecular weights of the scattering particles (again assuming a single species of spherical scatterers) was 102 kDa and 92 kDa respectively. These data implied that addition of either (i) IR αCT peptide or (ii) IR aCT peptide plus ZFP-insulin to a 6 mg/ml solution of IR485 resulted in a change in its hydrodynamic diameter consistent with the construct undergoing a transition from being overwhelmingly dimeric in solution to predominantly monomeric in solution.

### Example 2: Solving the crystal structure of the C-terminal region of the α-chain of IR

### 2.1 Introduction: ambiguous electron density

As described previously (WO 07/147213), an area of strand-like ambiguous electron density was present near the L1-β2 face of IR. However, despite numerous different processing and refinement protocols, the density was impossible to interpret in terms of any peptide sequence. The data described in Example 1 above implied a surprising and previously unexpected sequence relationship between the S519C16 peptide (SEQ ID NO: 18) and the 'classical' αCT peptide region (residues 704-719; SEQ ID NO: 11) described previously in the literature (Kurose et al., 1994). X-ray data obtained in WO 07/147213 were revisited and further reviewed with the possibility then in mind that the ambiguous electron density near the L1-β2 face of IR could have been due to the 'classical' αCT peptide region of the IR α-chain.

### 2.2 Protein production, crystallisation and data collection from IR ectodomain crystals

Production of IR ectodomain protein, subsequent crystallization and data collection were performed as described previously (WO 07/147213).

### 2.3 Diffraction data processing and crystallographic refinement

The diffraction images used were those used in the native structure determination of the human insulin receptor ectodomain homodimers ("Native 2" data set, see Table 3 of WO 07/147213). The diffraction images were re-processed using XDS (version 10-September-2008; Kabsch, 1993), including reflections to a maximum resolution of 3.8 Å. Diffraction data processing statistics from XDS are shown in Table 5. About a 1 Å difference in the longest cell dimension was observed as compared to analysis previously with the D*trek (Pflugrath, 1999) program.

The receptor ectodomain monomer complexed with Fab 83-7 and Fab 83-14 (Protein Data Bank entry 2DTG, with minor prior in house improvement) was subjected to individual domain rigid body crystallographic refinement against the XDS-processed diffraction data set using PHENIX v1.3b (Adams et al., 2002). A total of fourteen rigid body domains were defined for this purpose (i) chain E and residues 4-191, (ii) chain E and residues 192-311, (iii) chain E and residues 312-464, (iv) chain E and residues 465-594, (v) chain E and residues 595-817, (vi) chain E and residues 818-909, (vii) chain A and residues 1-112, (viii) chain B and residues 1-109, (ix) chain A and residues 113-220, (x) chain B and residues 110-219, (xi) chain C and residues 1-109, (xii) chain C and residues 110-207, (xiii) chain D and residues 1-106, (xiv) chain D and residues 107-214}. Rigid body refinement was then followed by atomic coordinate refinement and finally by TLS (translation, libration and screw-rotation displacement) refinement, using default protocols within PHENIX. A σ_{A}-weighted 2Fₒ-F_{c} difference electron map was then calculated using structure factors whose amplitudes had been artificially inflated by the application of a B value (-153 Å²) equal to the negative of that determined by a Wilson plot (Brünger et al., 2009). The map was then visually inspected using O v12.0 (Jones, 2004). At this stage the segment of electron density previously discerned on the L1-β2 face of IR (WO 07/147213) had a surprisingly clear helical conformation with sufficient variation in side chain electron density to suggest that sequence assignment was possible (see below). O was then used for all subsequent model building of the IR segment 693-710, with model building being iterated with crystallographic refinement within PHENIX. Final crystallographic refinement statistics are shown in Table 5.

### 2.4 Assigning structure to the C-terminal region of the IR α-chain

Following the refinement protocol detailed above, sequence assignment to the C-terminal region of the IR α-chain was possible and based on the following observations (i) the segment Glu698 to Tyr708 was the only region of the insert domain predicted to have helix-forming propensity (Ward and Lawrence, 2009), (ii) inspection of the density at the side-chain positions i, i+4 and i+7 showed that they likely arose from three large (most likely aromatic) residues, that these could in all likelihood be used to define the sequence register, and that the discerned helix would then span residues i-8 to i+9, (iii) the direction of the polypeptide chain within the helix was readily apparent from helical "tree" averaging of the difference electron density (Jones, 2004), and (iv) the only possible assignment of the sequence to the i, i+4, i+7 all-aromatic motif of (ii) above was to associate these residues with Phe701, Phe705 and Tyr708, respectively.

**Table 5 - Data processing and crystallographic refinement statistics from IR ectodomain crystal IR IRΔβ data processed using XDS.**

| **Data processing** | |
|---|---|
| Space group | C222₁ |
| Cell dimensions | |
| *a*, *b*, *c* (Å) | 123.03, 318.65, 204.64 |
| Resolution (Å) | 30.64 (3.80)* |
| *R_{sym}* | 14.6 (198.9) |
| *I* / σ*I* | 0.96 |
| Completeness (%) | 93.8 (93.4) |
| Redundancy | 5.47 (5.35) |
| | |

| **Refinement** | |
|---|---|
| Resolution (Å) | 30.64 - 3.80 |
| No. reflections | 39268 |
| *R*_{work} / *R*_{free} | 0.225 / 0.289 |
| No. atoms | |
| Protein | 13041 |
| *B*-factors | |
| Protein (Å²) | 180.68 |
| R.m.s. deviations | |
| Bond lengths (Å) | 0.011 |
| Bond angles (°) | 1.558 |

| | |
|---|---|
| *Values in parentheses are for highest-resolution shell. Data were collected from a single crystal. | |

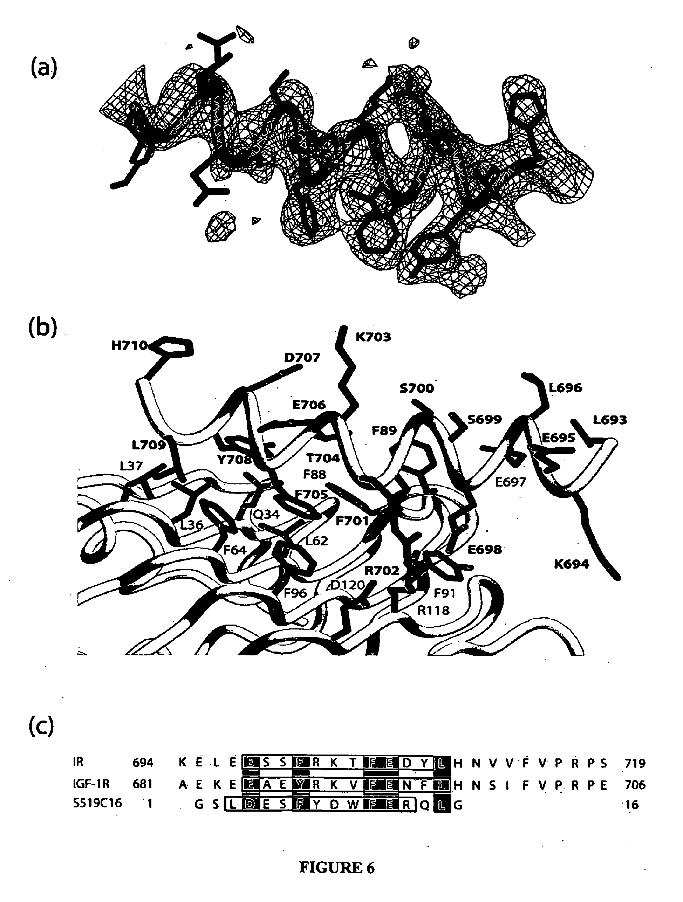

The observed helical region of density spanned IR residues 693 to 710 (SEQ ID NO: 13; Figure 6). The direction of the polypeptide was consistent with having the inter-monomer disulphide bond-forming triplet Cys682 / Cys683 / Cys685 (Sparrow et al., 1997) in close proximity to the ectodomain two-fold axis (McKern et al., 2006). Subsequent model-building of insulin receptor residues 693-710 (SEQ ID NO: 13) into the difference electron density further supported the correctness of the sequence register assignment by virtue of the shape and electrostatic complementarity of the packing at the interface between L1 and the helical segment.

The side-chains of Phe701 and Phe705 pack adjacent to each other into a hydrophobic pocket formed by the side-chains of the L1 residues Phe64, Phe88, Phe96, Tyr91 and Arg 118 (Figure 6b). The side-chain of Tyr708 is packed approximately parallel to the surface and interacts with the L1 residues Leu62, Gln34 and Phe64. The side-chains of the residue pair Glu698 and Arg702 lie in close proximity and are juxtaposed against the side chains of the L1 residue pair Arg118 and Asp 120 respectively, the four side chains forming a symmetric charge-compensating cluster. The final interaction between the helix and the surface of the central b-sheet of L1 arises from an interaction between the side chain of Leu709 with the side chains of the L1 residues Leu37 and Phe64. On the opposite surface of the helix side chains of the residue pair Lys703 and Asp707 are in proximity to each other and also likely charge compensate. Further crystallographic refinement of the (IR + Fab 83-7 + Fab 83-14) structure, now inclusive of IR residues 693-710, lead to a reduction of 0.5% in the free R-factor. The shape complementarity (Lawrence and Colman, 1993) of the interface between the insulin receptor residues 693-710 and the L1 domain of the receptor, computed after crystallographic refinement, is high (0.72). Taken together, these results gave overwhelming support to the correctness of the assignment of sequence to the helical density segment.

The structure provided herein (Appendix I) enables, for the first time, a view of the intact low affinity insulin receptor binding site that includes the critically-important C-terminal region of the receptor α-chain (SEQ ID NO: 13). The atomic coordinates of IR + Fab 83-7 + Fab 83-14 inclusive of the helical segment are now included in Appendix I and are depicted in Figure 6. The modelled helical segment of the IR α-chain (residues 693-710, herein termed 'the C-terminal region of the α-chain of IR'; LKELEESSFRKTFEDYLH; SEQ ID NO: 13) surprisingly encompasses residues N-terminal of the "classical" aCT peptide of IR (residues 704-719; TFEDYLHNVVFVPRPS; SEQ ID NO: 11) described previously in the literature (Kurose et al., 1994; Figure 6c). The original demarcation of this segment arose from a tryptic digest aimed at isolating receptor segments that were experimentally cross-linked to bound insulin. Tryptic cleavage at Lys703 resulted in the isolation of the segment 704-719 crosslinked to insulin. The involvement of residues immediately N-terminal of 704 in both the formation of the low affinity insulin binding site and in attachment of α-chain C-terminus to the first three domains of the receptor had not been contemplated previously.

### Example 3: Molecular modelling

### 3.1 Introduction

There is a high level of sequence identity between, on the one hand, the L1 domains of IGF-1R and IR, and, on the other hand, between the C-terminal regions of the α-chain of IGF-1R and IR (Figure 1 and Figure 6c). Accordingly, models of IR in complex with S519C16 and the IGF-1R α-chain residues 681-697, respectively, were constructed using the MODELLER program (Sali and Blundell, 1993) with the crystallographic structure of IR ectodomain presented in the main text as a template. Models of the ectodomain of IGF-1R in complex, respectively, with the IGF-1R α-chain residues 681-697 (SEQ ID NO: 15). S519C16 (SEQ ID NO: 18) and the IR α-chain residues 693-710 (SEQ ID NO: 13) were constructed employing the crystal structure of the first three domains of IGF-1R (Garrett et al., 1998), the structure of the IR ectodomain presented here and the known sequence relationship between IR and IGF-1R (Adams et al., 2000).

### 3.2 Materials and methods for modelling

Twenty-five instances of each model were prepared and the structure with lowest DOPE score (Eramian et al., 2006) selected for further molecular dynamics (MD) simulation. The L1-domains of IR and IGF-1R, residues Pro4-Gln189 and residues Glul-Gln189, respectively, in complex with the various peptides were excised from the full-length models prepared by MODELLER for use in the MD calculations. MD simulations were performed using the GROMACS v4.0 suite (van der Spoel et al., 2005) with the OPLS-aa force field (Jorgensen and Tirado-Rives, 1988). The proteins were solvated in a box of water and the total charge of the system neutralized by replacing water molecules with sodium ions. The LINCS algorithm was used to constrain bond lengths (Hess et al., 1977). Protein and solvent (including ions) were coupled separately to a thermal bath at 300 K using velocity rescaling (Bussi et al., 2007) applied with a coupling time of 0.1 ps. All simulations were performed with a single non-bonded cutoff of 10 Å, applying a neighbour-list update frequency of 10 steps (20 fs). The particle mesh Ewald method was applied to deal with long-range electrostatics with a grid width of 1.2 Å and fourth-order spline interpolation. All simulations consisted of an initial minimization to remove close contacts, followed by 100 ps of positional restrained MD to equilibrate the water molecules with the protein fixed. The time step used in all the simulations was 2 fs. MD simulations for each system were run for a total length of 2.0 ns.

### 3.3 Molecular models

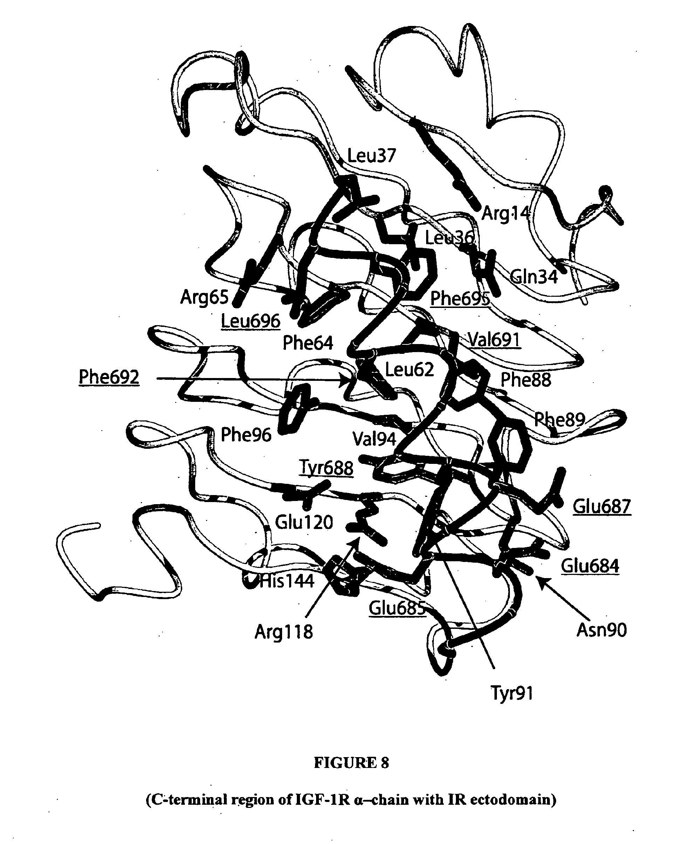

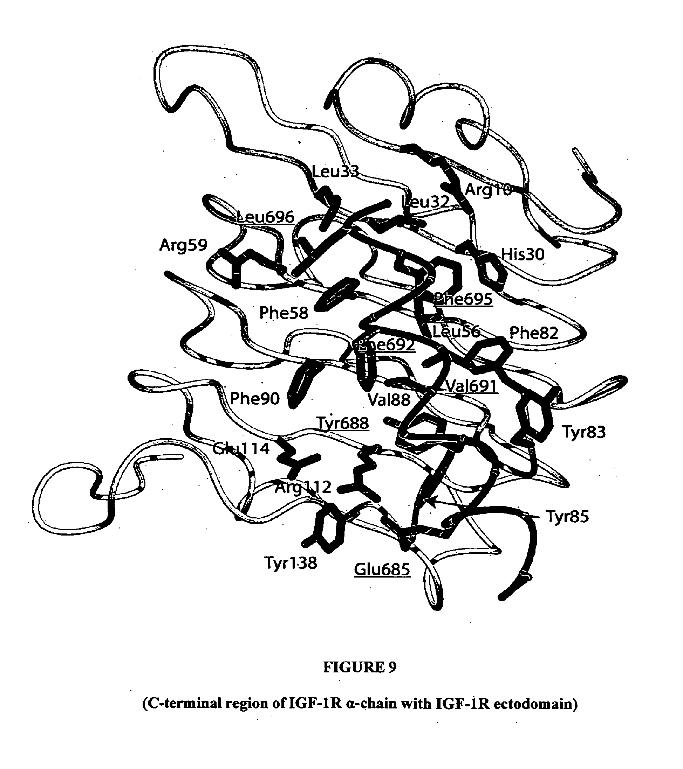

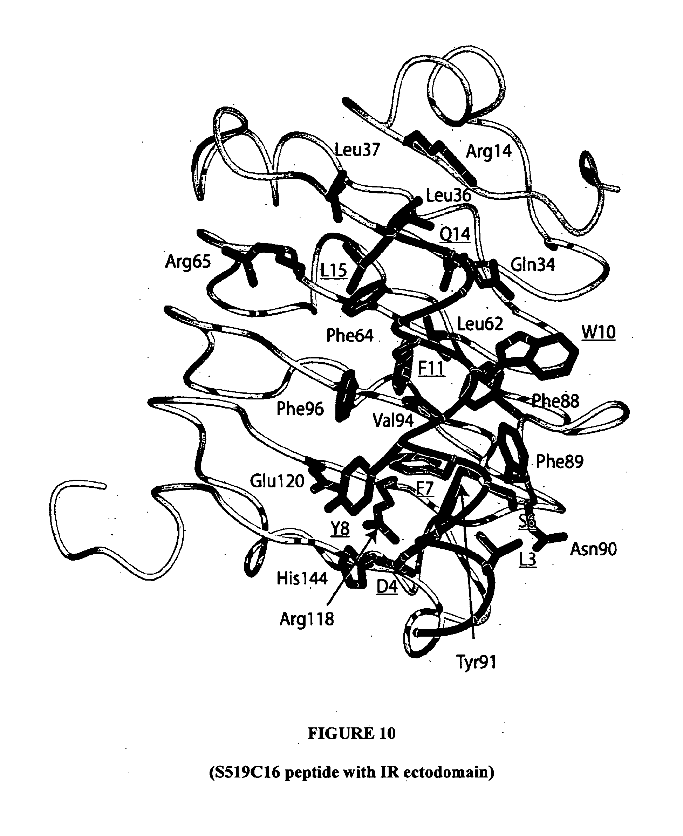

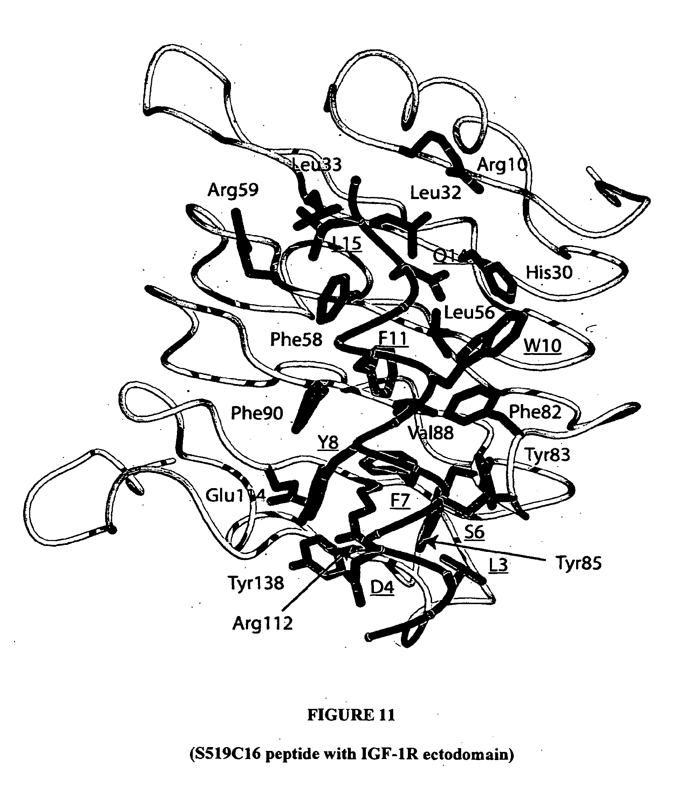

The following models were created using MODELLER and subjected to MD simulations as described above:
the C-terminal region of IR α-chain (SEQ ID NO: 13) bound to the L1 domain of IGF-1R (Appendix II; Figure 7),
the C-terminal region of IGF-1R α-chain (SEQ ID NO: 15) bound to the L1 domain of IR (Appendix III; Figure 8),
the C-terminal region of IGF-1R α-chain (SEQ ID NO:15) bound to the L1 domain of IGF-1R (Appendix IV; Figure 9),
S519C16 mimetic peptide (SEQ ID NO: 18) bound to the L1 domain of IR (Appendix V; Figure 10), and
S519C16 mimetic peptide (SEQ ID NO: 18) bound to the L1 domain of IGF-1R (Appendix VI; Figure 11).

The above models are presented schematically in Figures 7 to 11, with coordinates in Appendixes II to VI, respectively. These models define a common binding surface on IR and IGF-1R capable of binding the C-terminal region of the α-chain of the receptors. The following interactions were observed:
*(i) IGF-1R*/*IR α-chain residues 693-710 (**Figure* 7): This interaction is characterized by several polar and ionic interactions - Y83 of the receptor hydrogen bonds the hydroxyl side-chains of S700 and T704 of the ligand, and E698 and R702 of the ligand form salt bridges with R112 and E114 of the receptor, respectively. Hydrophobic residues on the ligand pack into a hydrophobic pocket on the receptor formed by in part by L32, L33, L56, F58, F82, Y83, Y85, V88 and F90.
*(ii) IR*/*IGF-1R* α-*chain residues 681-697 (**Figure 8**):* The complex between IR and the IGF-1R α-chain (residues 681-697) consists of charged interactions between R118 and E120 of IR, and E685 and Y688 of the peptide, respectively - the latter of these interactions can be mediated by a water molecule. There are a large number of hydrophobic residues on IR that contact the peptide, formed in part by L36, L37, L62, F64, F88, F89, Y91, V94 and F96.
*(iii) IGF-1R*/*IGF-1R* α-*chain residues 681-697 (**Figure 9**):* This interaction is characterized by a salt bridge between R112 of the receptor and E685 of the peptide. Additionally, the hydroxy group on the side-chain of Y688 in hydrogen bonded to a water molecule (not shown) that is itself hydrogen bonded to E 114 of the receptor. The hydrophobic resides Y688, F 192, F 195 and L196 pack into the hydrophobic pocket on the surface of the receptor formed in part by the side-chains of L32, L56, F58, F82, Y83, Y85, V88 and F90.
*(iv) IR*/*S519C16 (**Figure 10**)*: Throughout the MD simulation of the complex between IR (L1 domain) and the S519C16 peptide several hydrogen-bond interactions are observed - between R119 of IR and D4 of the peptide, between E120 of IR and Y8 of the peptide, and between Q14 of the peptide with both residues R14 and Q34 of IR. The interaction between receptor and peptide involves the aromatic side-chains of several residues - F7 and F11 of the peptide bind a pocket on the surface of IR flanked by the residues L62, F64, F88, F89, Y91 and F96. Additionally, L 15 of the peptide packs against the hydrophobic side-chains of L37 and F64.
*(v) IGF-1R*/*S519C16 (**Figure 11**):* D4 and Y8 of the peptide hydrogen bonds R112 and E 114 of the receptor, respectively. The aromatic side-chains of F7 and F11 of the peptide bind the hydrophobic pocket on the receptor formed by L56, F58, F82, Y83, V88 and F90. W10 of the peptide can conceivably contact the hydrophobic side-chains of L32 and F82, increasing its affinity.

The models presented herein can now be used to improve the insulin mimetic peptides developed by Schaffer and co-workers (Schäffer et al., 2003). Isothermal titration calorimetry experiments (see Example 1 above) indicated that a prototypical Site 1 mimetic peptide S519C16 competes with the IR C-terminal peptide 704-719 in binding to a construct consisting of the first three domains of the insulin receptor. The two major residues involved in the interaction between the C-terminal segment of the insulin receptor α-chain and the L1 domain (viz. Phe701 and Phe705) are conserved in S519C16 (Figure 3). Of the remaining residues involved in the interaction between the C-terminus of the α-chain and L1, Tyr708 is replaced with an asparagine residue in S519C16, Glu698 is either conserved or replaced by an aspartate residue, Arg702 is replaced by a tyrosine residue and Leu709 is conserved. The two phenylalanine residues form part of the motif FYXWF (SEQ ID NO: 19) that characterizes the Site 1 mimetic peptides (Schäffer et al., 2003). Modelling undertaken with MODELLER and subsequent MD showed that it is possible to dock the S519C16 peptide onto the L1 surfaces of IR and IGF-1R in a way analogous to the docking of cognate peptides (see Figures 6-11).

### Example 4: Binding of mutant αCT peptides to an insulin mini-receptor (IR485 construct)

### 4.1 Introduction

Insulin-mimetic peptides have been discovered by phage display technology and classified as "Site" 1, 2 or 3 on the basis of competition of binding to insulin receptor (Pillutla *et al.,* 2002). The affinity-matured Site 1 peptides are characterized by a FYXWF motif (SEQ ID NO: 19) (Pillutla *et al.,* 2002); selected Site 1 and 2 peptides have been covalently tethered to yield agonists with up to picomolar affinity for insulin receptor (Schäffer *et al.,* 2003). A sequence relationship has been shown (see Figure 6c) between the aCT region and the prototypic Site 1 mimetic peptide that places the aCT region residues Phe701 and Phe705 in respective alignment with the two flanking phenylalanine residues in the FYXWF motif. This relationship was used to explain the competitive binding of the aCT peptide and the prototypic Site 1 mimetic peptide to the insulin mini-receptor IR485, a construct which consists of the receptor L1-CR-L2 domains only. If this relationship was correct, then mutation of Arg702 and/or Thr704 within the aCT segment to either tyrosine or tryptophan would lead to significantly higher affinity of the segment for the insulin mini-receptor. It would also then be possible to model these substitutions directly onto the structure reported here. These hypotheses were tested as follows.

### 4.2 Materials and methods for thermodynamic experiments

Biotinylated aCT peptides at >75 % purity spanning residues 698-719 of the insulin receptor were obtained from Genscript Inc. (USA). The insulin mini-receptor IR485 construct was produced and purified as previously described (Lou *et al.,* 2006), omitting the final ion-exchange chromatography step. Isothermal titration calorimetry (ITC) experiments were performed using a VP-ITC isothermal titration calorimeter (MicroCal Inc., USA) with the calorimeter cell held at about 25° C. The ITC cell contained insulin mini-receptor IR485 prepared at about 10 µM concentration in Tris-buffered saline plus azide (TBSA; about 24.8 mM Tris-HCl (pH 8.0), 137 mM NaCl, 2.7 mM KCl, and 0.02% sodium azide), and the syringe contained the peptide prepared at about 60 µM concentration in TBSA. All samples were degassed prior to injection or placement into the cell, and the instrument was temperature equilibrated prior to the start of the injections.

In all experiments the volume of the insulin mini-receptor IR485 sample placed in the cell was about 1.4 ml and the titrant was injected in about 7 µl volumes over 14 s at 3 min intervals, with the total number of injections being 40. The sample contents were stirred at a speed of about 310 rpm over the duration of the titration. All titrants were first injected into a solution of TBSA alone in order ascertain the heat of dilution, which was then subtracted from the data of interest as appropriate. Each experiment was performed three times, except for that employing the native peptide, which was performed twice. Data were analyzed using the instrument's software incorporated within the Origin 7 software (OriginLab, USA) and in all cases fitted as a single-site interaction using the methodologies outlined in the instrument's manual.

### 4.3 Results from thermodynamic experiments

Sample individual titration curves are provided in Figure 12. Table 6 below presents ITC-derived dissociation constants and thermodynamic parameters for a set of mutant aCT peptides titrated against the insulin mini-receptor IR485.

**Table 6 - Derived thermodynamic parameters for the titration against IR485 of the N-terminally biotinylated IR peptide 698-719 containing the following respective mutations: wild-type, T704Y, R702W, R702Y, T704W and R702Y / T704W.**

| **Titrant peptide*** | ***K_{d}* peptide (nM)** | **Δ*H*⁰ (kJ mol⁻¹)** | ***T*Δ*S⁰* (kJ mol⁻¹)** | **Δ*G⁰* (kJ mol⁻¹)** |
|---|---|---|---|---|
| wt | 1340 ± 230^{†} | -48 ± 1 | -14 ± 1 | -33.5 ± 0.4 |
| T704Y | 740 ± 210 | -46 ± 9 | -11 ± 9 | -35. ± 0.7 |
| R702W | 309 ± 36 | -35 ± 5 | -3 ± 5 | -37.2 ± 0.3 |
| R702Y | 249 ± 3 | -50 ± 3 | -12 ± 2 | -37.67 ± 0.03 |
| T704W | 201 ± 16 | -26 ± 2 | 11 ± 2 | -38.2 ± 0.2 |
| R702Y / T704W | 18 ± 3 | -49 ± 2 | -5 ± 2 | -44.2 ± 0.4 |

| | | | | |
|---|---|---|---|---|
| *αCT peptides span residues 698-719 and have a biotin moiety attached at the N-terminus. ^{†}Error estimates are the standard error of the mean. | | | | |

Progressive inclusion of aromatic residues at positions 702 and 704 is seen to result in an up to 100-fold increase in affinity, supporting the view that there is a structural relationship between the Site-1 mimetic peptides and the native aCT segment. The docking of the single- and double-mutant αCT peptides to the surface of the L1 domain was investigated by molecular dynamics simulation using the GROMACS v4.0 suite (van der Spoel *et al.,* 2005) with the OPLS-aa force field (Jorgensen *et al.,* 1988) and revealed that the aromatic side chains of mutant aCT residues Trp702 and Tyr704 are likely to interact with the surface of the L1 domain and enhance the affinity of the interaction. At position 702 the aromatic side chain is docked into a pocket formed by the Phe96 and the alkyl portion of L1 side-chain Lys121; the hydroxyl group of a variant tyrosine residue can in addition form a hydrogen bond with the Lys121 ε-amino group. At position 704 the aromatic side chain of the variants is docked against L1 side chains Phe88 and Phe89.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed before the priority date of each claim of this application.

### References

Adams et al. (2000) Cell. Mol. Life Sci., 57, 1050-1093.
Adams et al. (2002) Acta Cryst. D58, 1948-54.
Apfel (1999) Am. J. Med., 107, 34S-42S.
Auer (1998) Neurology, 51, S39-S43.
Ausubel et al. (1999) Short Protocols in Molecular Biology, 4th Ed, John Wiley & Sons, Inc.; and the full version entitled Current Protocols in Molecular Biology.
Bailyes et al. (1997) Biochein. J., 327, 209-215.
Bartlett et al. (1989) Royal Chem. Soc., 78, 182-196.
Bentley (1997) Methods Enzymol., 276, 611-619.
Binz et al. (2005) Nature Biotech., 23, 1257-1268.
Blondelle and Houghten (1996) Trends Biotechnol., 14, 60-65.
Bohm and Stahl (1999) M. Med. Chem. Res., 9,445.
Brooks et al. (1983) Comp. Chem., 4, 187-217.
Brünger et al. (1998) Acta Cryst. D54, 905-921.
Brünger (1997) Methods Enzymol., 276, 558-580.
Brünger et al. (2009) Acta Cryst., D65, 128-33.
Bruns et al. (1999) J Mol Biol., 288, 427-439.
Bussi et al. (2007) M. J. Chem. Phys., 126, 14101.
Buttel et al. (1999) Immunol. Cell Biol., 77, 256-262.
Carell et al. (1994a) Angew.Chem. Int. Ed. Engl., 33,2059.
Carell et al. (1994b) Angew. Chem. Int. Ed. Engl., 33, 2061.
Cho et al. (1993) Science, 261, 1303.
Chow et al. (1998) Biol. Chem., 273, 4672-4680.
Clarke et al. (2000) Cancer Res., 60, 4804-4811.
Cohen et al. (1990) J. Med. Chem., 33, 883-894.
Cole et al. (2005) in "Virtual Screening in Drug Discovery (Eds. B. Shoichet, J. Alvarez)", Taylor & Francis CRC Press, Florida, USA.
Cull et al. (1992) Proc. Natl. Acad. Sci. USA, 89, 1865-1869.
Cwirla et al. (1990) Proc. Natl. Acad. Sci. USA, 97, 6378-6382.
Danial et al. (2008) Nat. Med. 14, 144-153.
Davis et al. (2006) Chem. Soc. Rev. 36, 326-334.
Day and Caflisch (2008) J. Chem. Inform. Model. 48,679-90.
Degterev et al. (2001) Nat. Cell. Biol. 3, 173-182.
De Meyts (1994) Diabetologia, 37, S135-S148.
De Meyts and Whittaker (2002) Nat. Rev. Drug Discos., 1, 769-783.
De Meyts (2004) Bioessays, 26, 1351-1362.
Denley et al. (2003) Horm. Metab. Res., 35, 778-785.
Denley et al. (2004) Mol. Endocrinol., 18, 2502-2512.
Devlin (1990) Science, 249, 404-406.
DeWitt et al. (1993) Proc. Natl. Acad. Sci. USA, 90, 6909.
Eramian et al. (2006) Protein Sci. 15, 1653-66.
Erb et al. (1994) Proc. Natl. Acad. Sci. USA, 91, 11422.
Ernst et al. (2000) J. Magn. Reson. Imaging, 12, 859-865.
Ewing et al. (2001) J. Comput-Aid. Mol. Design, 15,411.
Felici (1991) J. Mol. Biol., 222, 301-310.
Fodor (1993) Nature, 364, 555-556.
Friesner et al. (2004) J. Med. Chem. 47, 1739-1749.
Garrett et al. (1998) Nature, 394, 395-399.
Gallop et al. (1994). J. Med. Chem., 37, 1233.
Goodford (1985) J. Med. Chem., 28, 849-857 (1985).
Goodsell and Olsen (1990) Proteins: Struct. Funct. Genet., 8, 195-202.
Guida (1994) Curr. Opin. Struct. Biol., 4, 777-781.
Hess et al. (1977) Comp. Chem. 18,1463-1472.
Hewish et al. (2009) Recent Patents Anticancer Drug Discov, 4, 54-72.
Houghten et al. (1991) Nature, 354, 84-86.
Houghten (1992) Biotechniques, 13,412-421.
Jones et al. (1991) Acta Cryst. A47, 110-119.
Jones (2004) Acta Cryst. D60, 2115-25.
Jorgensen and Tirado-Rives (1988) Am. Chem. Soc., 110, 1657-1666
Kabsch (1993) J. Appl. Cryst., 26, 795-800.
Kiselyov et al. (2009) Mol. Sys. Biol., 5, 243.
Kitamura et al. (2003) Annu. Rev. Physiol., 65, 313-332.
Kristensen et al. (2002) J. Biol. Chem., 277, 18340-18345.
Kuntz et al. (1982) J. Mol. Biol., 161, 269-288.
Kurose et al. (1994) J. Biol. Chem., 269, 29190-29197.
Lam et al. (1991) Nature, 354, 82-84.
Lam (1997) Anticancer Drug Des., 12, 145.
Lawrence and Colman (1993) J. Mol. Biol., 234, 946-950.
Lawrence et al. (2007) Curr. Opin. Struct. Biol., 17, 699-705.
Liu et al. (1993) Cell, 75, 59-72.
Lou et al. (2006) Proc. Natl. Acad. Sci. USA 103, 12429-12434.
Luo et al. (1999) Science, 285, 1077-1080.
Marsh et al. (1995) J. Cell Biol., 130, 1081-1091.
Martin (1992) J. Med. Chem., 35, 2145-2154.
McCoy (2007) Act Cryst D63, 32-41.
McKern et al. (2006) Nature, 443, 218-221.
Menting et al. (2009) Biochemistry (submitted).
Miranker and Karplus (1991) Proteins: Struct. Funct. Genet., 11, 29-34.
Moody et al. (1974) Horm. Metab. Res., 6, 12-16.
Morton and Myszka (1998) Methods Enzymol., 295, 268-294.
Navaza and Saludjian (1997) Methods Enzymol., 276, 581-594.
Navia and Murcko (1992) Curr. Opin. Struct. Biol., 2, 202-210.
Nice and Catimel (1999) Bioessays, 21, 339-352.
Olefsky (1978) Biochem. J., 172, 137-145.
Ottensmeyer et al. (2000) Biochemistry, 39, 12103-12112.
Ottensmeyer et al. (2001) Biochemistry, 40, 6988-6988.
Pflugrath (1999) Acta Cryst. D55, 1718-1725.
Pillutla et al. (2002) J. Biol. Chem., 277, 22590-22594.
Rarey et al. (1996) J. Mol. Biol., 261, 470.
Robinson and James (1992) Am. J. Physiol., 263, E383-E393.
Rossmann, end. (1972) The Molecular Replacement Method, Int. Sci. Rev. Ser., No. 13, Gordon & Breach, New York.
Sali and Blundell (1993) J. Mol. Biol., 234, 779-815.
Sambrook et al. (2001) Molecular Cloning: A Laboratory Manual, 3rd ed. Cold Spring Harbor Laboratory Press, Cold Spring Harbor, N.Y.
Schäffer (1994) Eur. J. Biochem., 221, 1127-1132.
Schäffer et al. (2003) Proc. Natl. Acad. Sci. USA, 100,4435-4439.
Scott and Smith (1990) Science, 249, 386-390.
Silverman et al. (2005) Nature Biotech., 23, 1556-1561.
Smith et al. (1999) Nat. Med., 5, 1390-1395.
Songyang et al. (1993) Cell, 72, 767-778.
Sparrow et al. (1997) J. Biol. Chem., 272, 29460-29467.
Stumpp et al. (2007) Curr. Opin. Drug Discov. Develop., 10, 153-159.
Surinya et al. (2008) J. Biol. Chem. 283, 5355-5363.
Svenson et al. (2009) Mol. Pharm., published online 2 April 2009 (DOI: 10.1021/mp900057k).
Tong and Rossmann, (1997) Methods Enzymol., 276, 594-611.
Totrov and Abagyan (2008) Curr. Opin. Struct. Biol., 18, 178-184.
Tulloch et al. (1999) J. Struct. Biol., 125, 11-18.
Ullrich et al. (1985) Nature, 313, 756-761.
Ullrich et al. (1986) EMBO J., 5, 2503-2512.
Ulrich (2006) Handb Exp Pharmacol., 173, 305-326.
van der Spoel et al. (2005) J. Comp. Chem., 26, 1701-1718.
Wada et al. (2005) J Pharmacol Sci., 99, 128-143.
Ward et al. (2003) Insulin-like growth factors (LeRoith D, Zumkeller, W. & Baxter R. (eds), Eurekah.com and Kluwer Academic/Plenum Publishers, 1-21.
Ward and Lawrence (2009) BioEssays, 31, 422-434.
Weiner et al. (1984) J. Am. Chem. Soc., 106, 765-784.
Yin et al. (2005) Angew. Chem. Int. Ed. Engl., 44, 2704-2707.
Yip et al. (1988) Biochem. Biophys. Res. Commun., 157, 321-329.
Yip and Ottensmeyer (2003) J. Biol. Chem., 278, 27329-27332.
Zuckermann et al. (1994) J. Med. Chem., 37, 2678.

### APPENDIX I : ATOMIC COORDINATES FOR IRΔβ MONOMER (CHAIN CONTAINING THE C-TERMINAL REGION OF THE IR α-CHAIN (CHAIN) WITH ATTACHED Fab 83-7 (CHAINS A AND B) AND Fab 83-14 (CHAIN AND D)

Note: The coordinates in this Appendix describe the asymmetric unit of the cry unit cell. The coordinates of the dimeric form of the above are generated by the application of the appropriate crystallographic two-fold operation, and these coordinates are hence included implicitly in this Appendix.

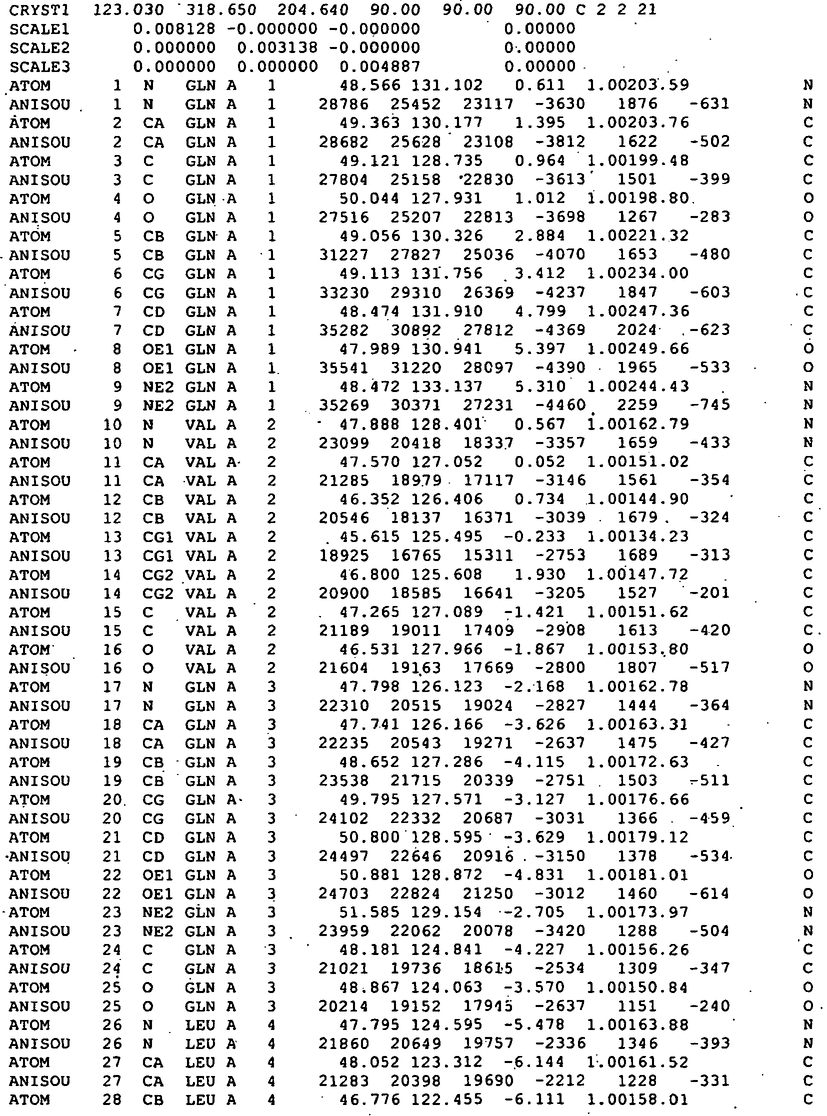

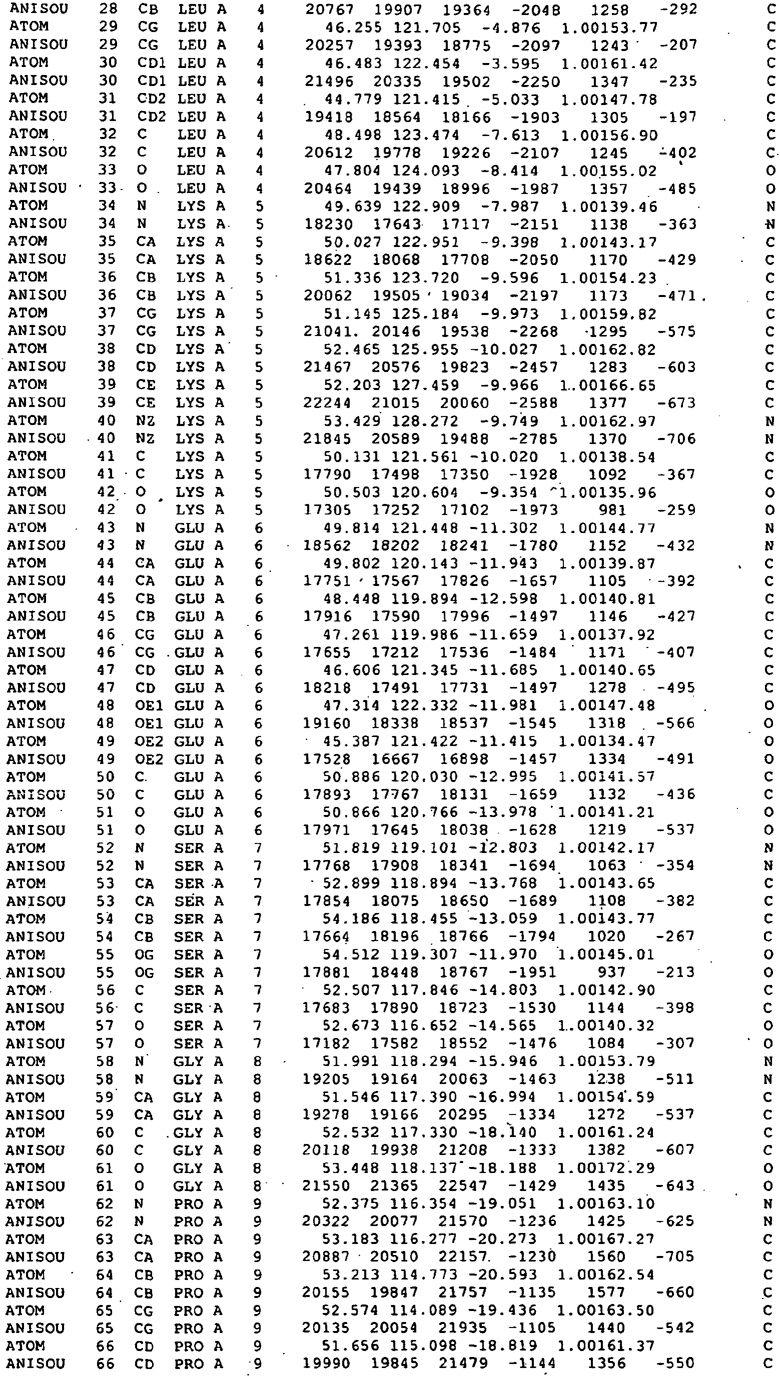

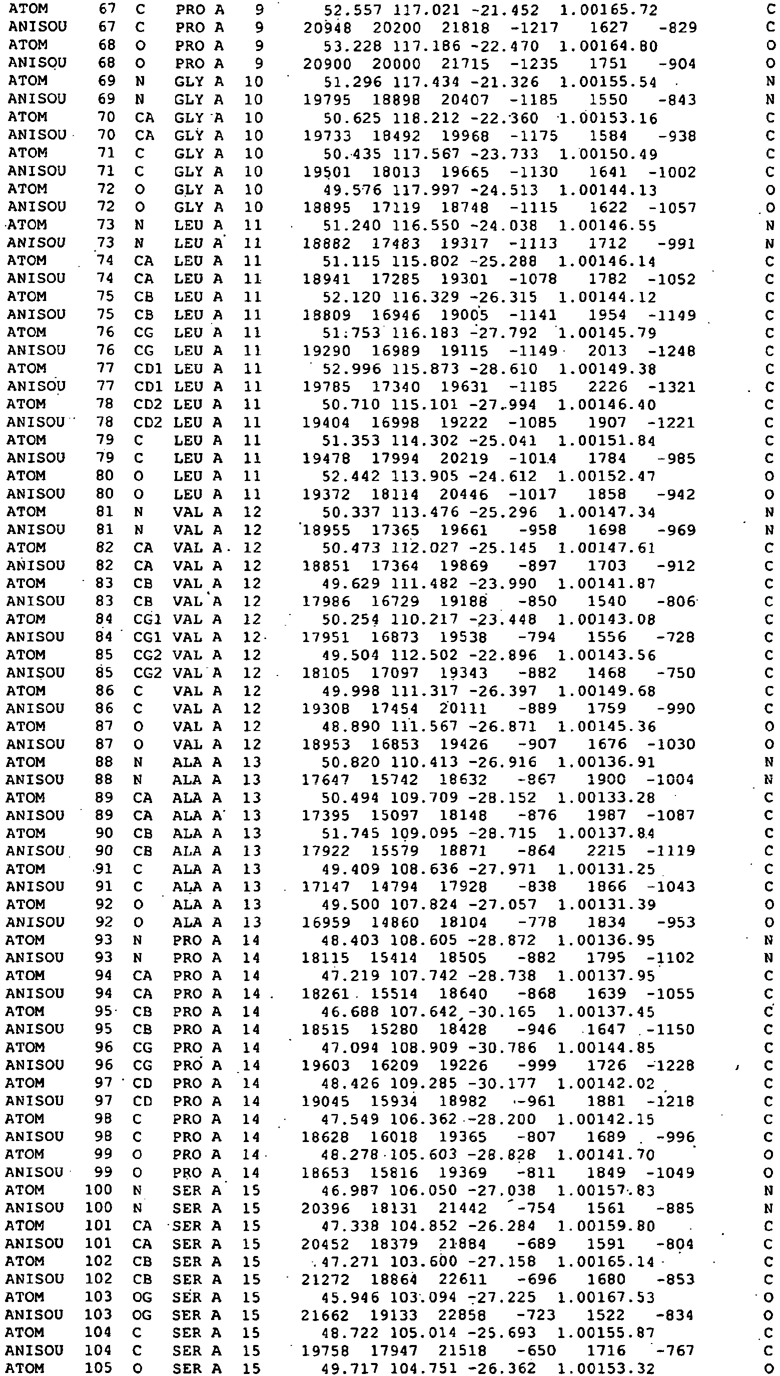

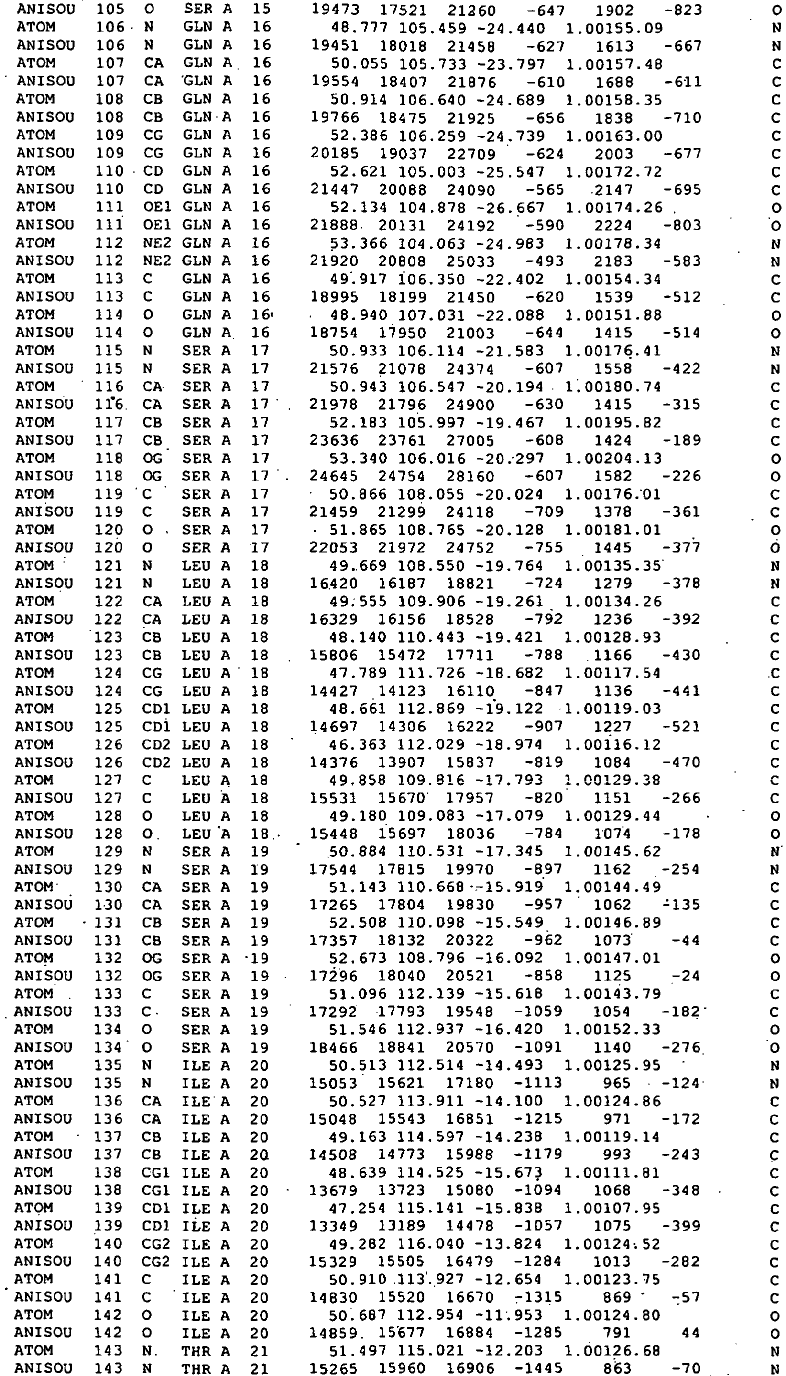

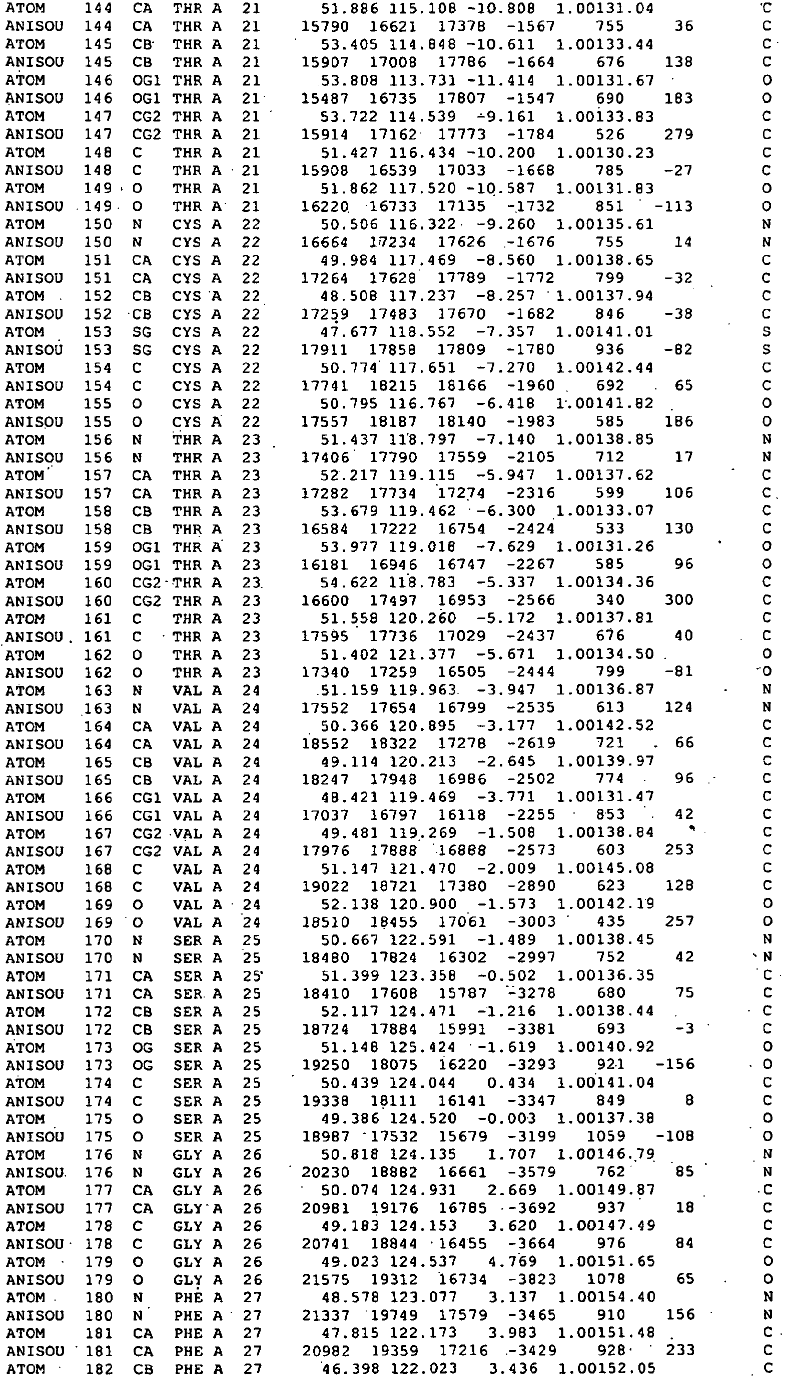

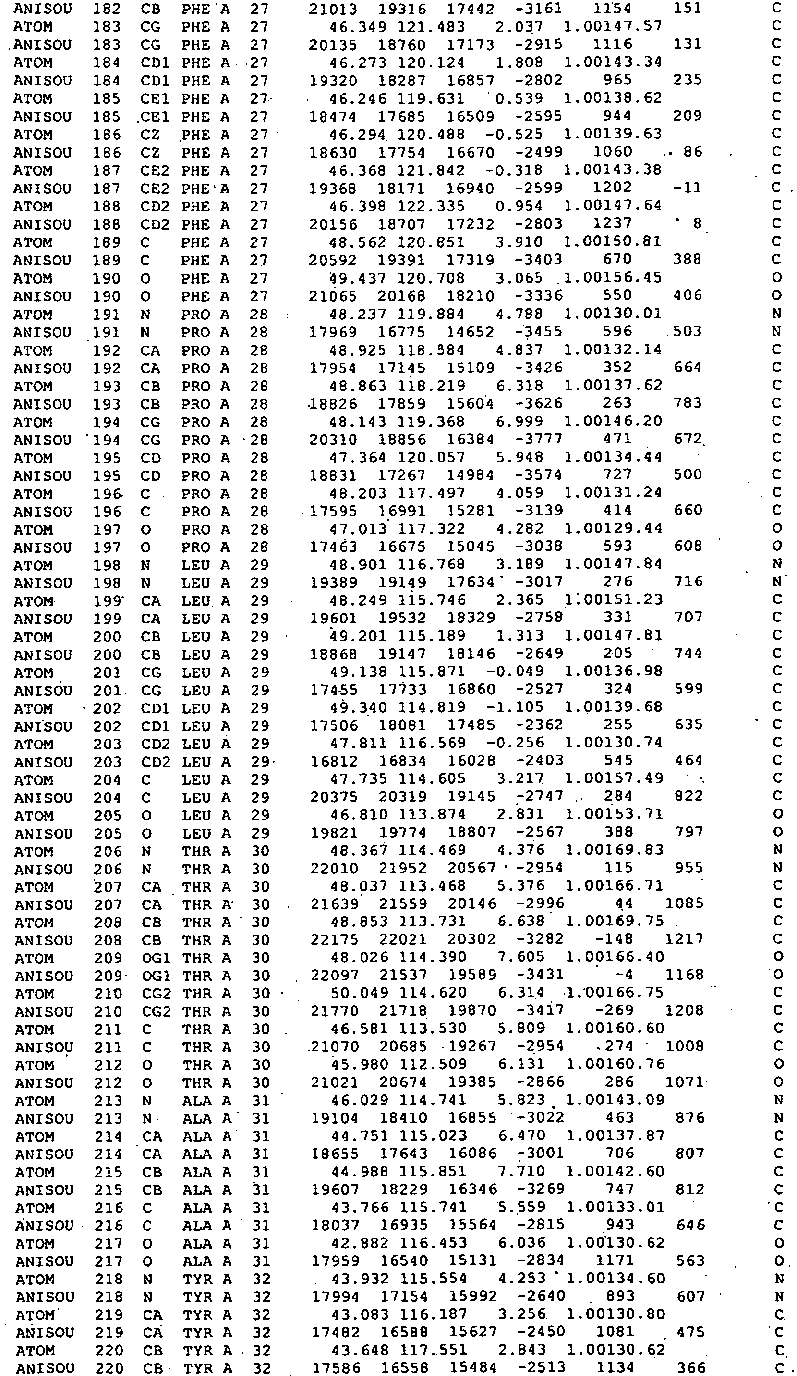

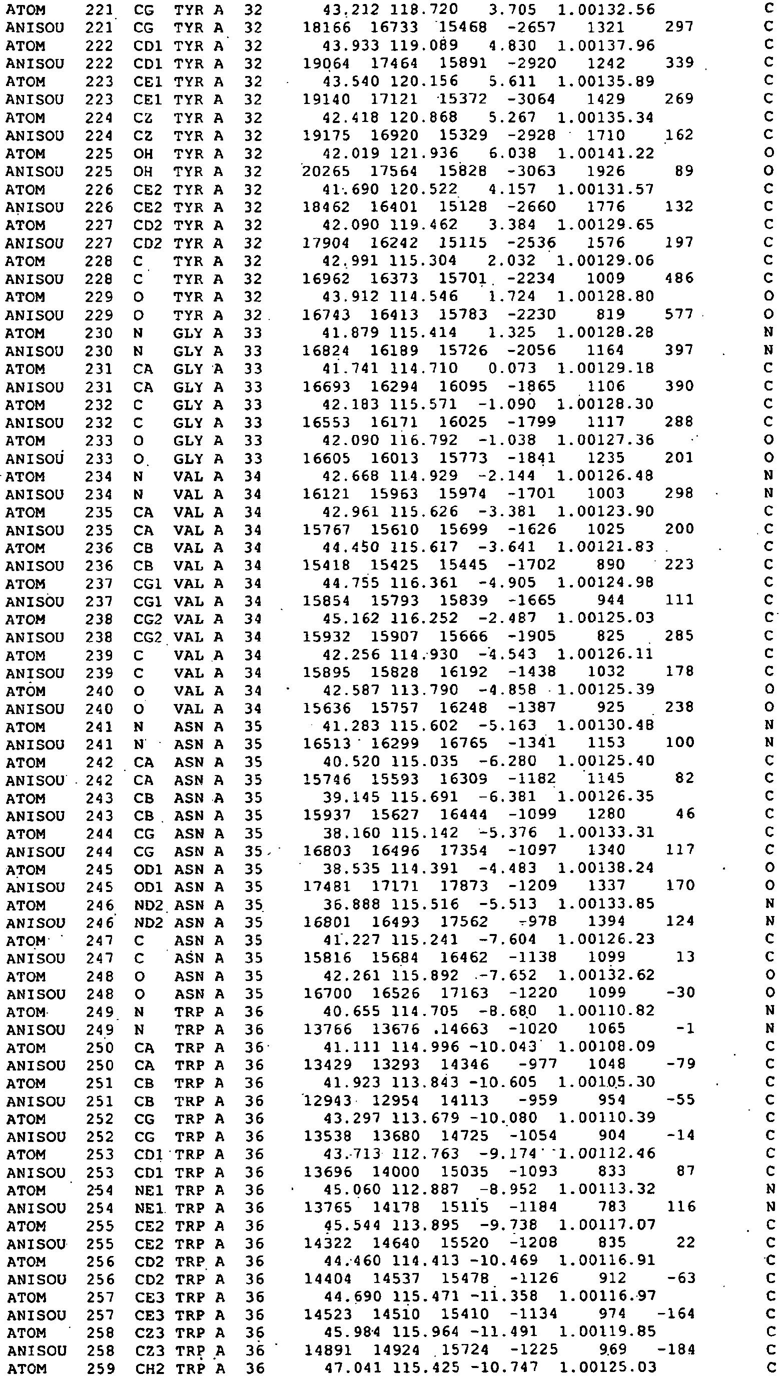

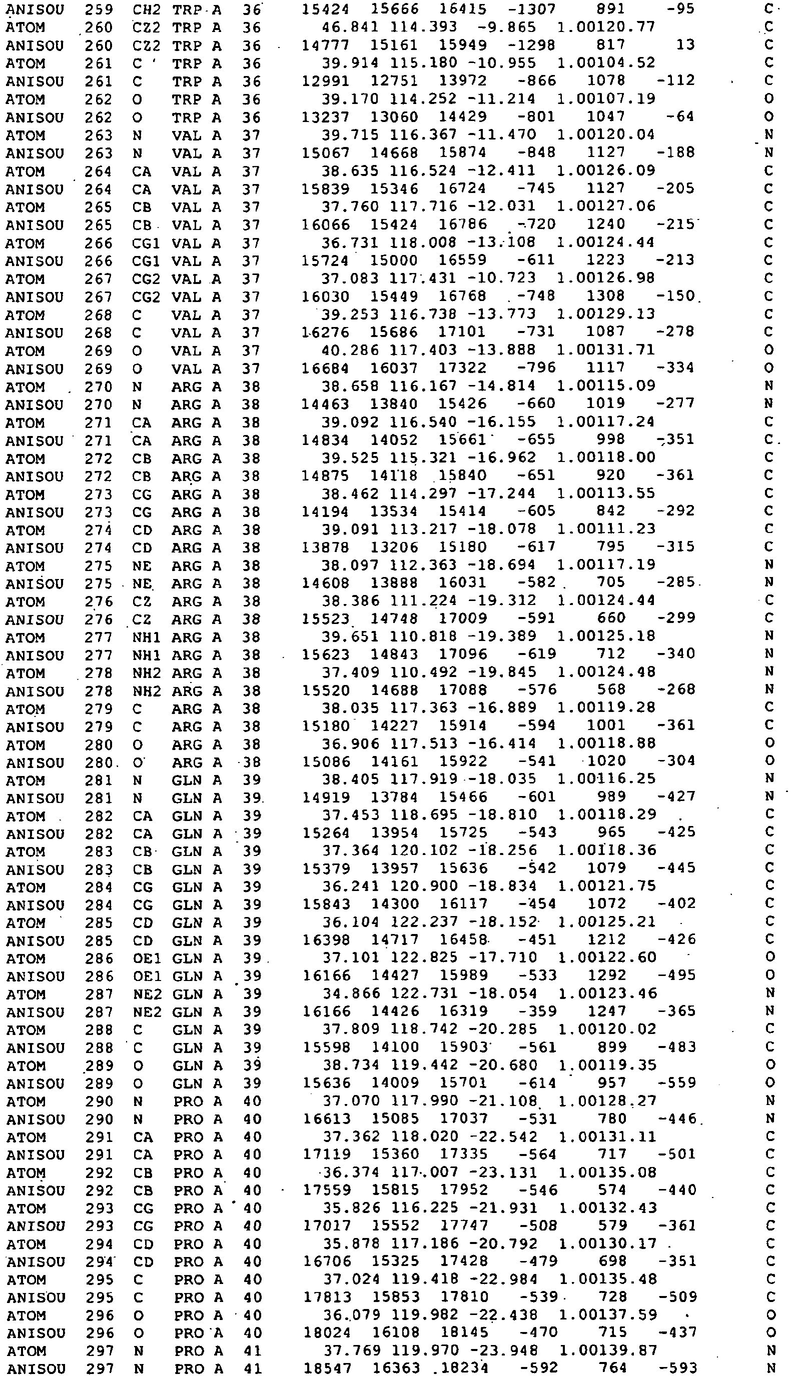

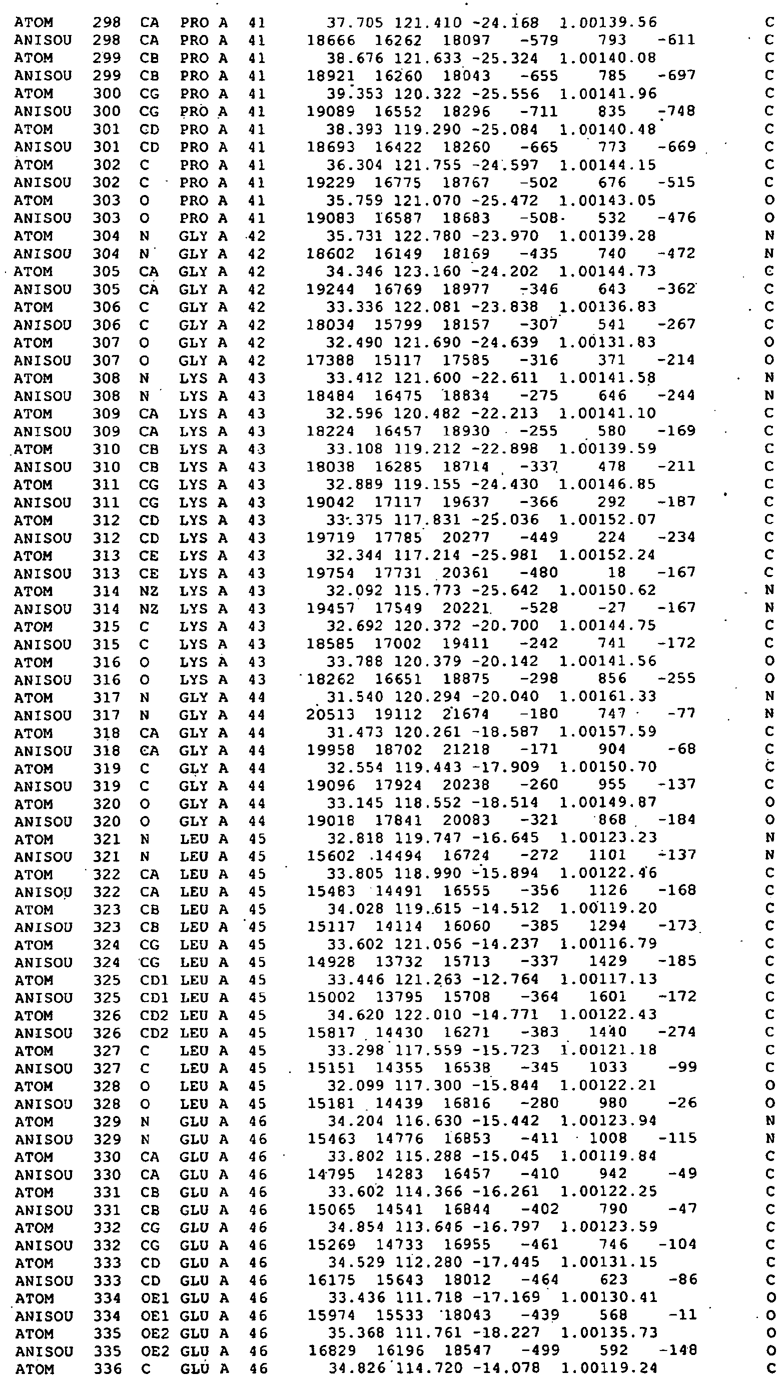

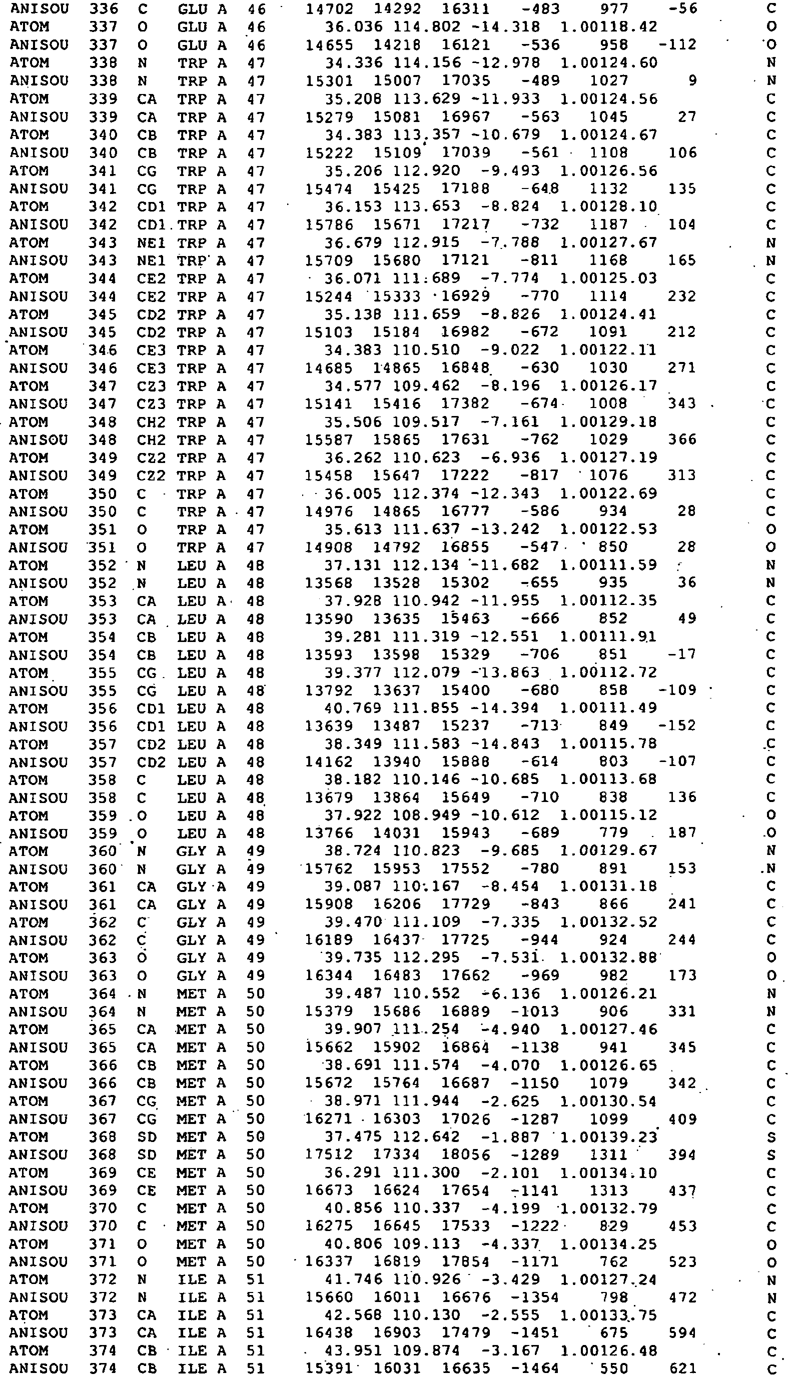

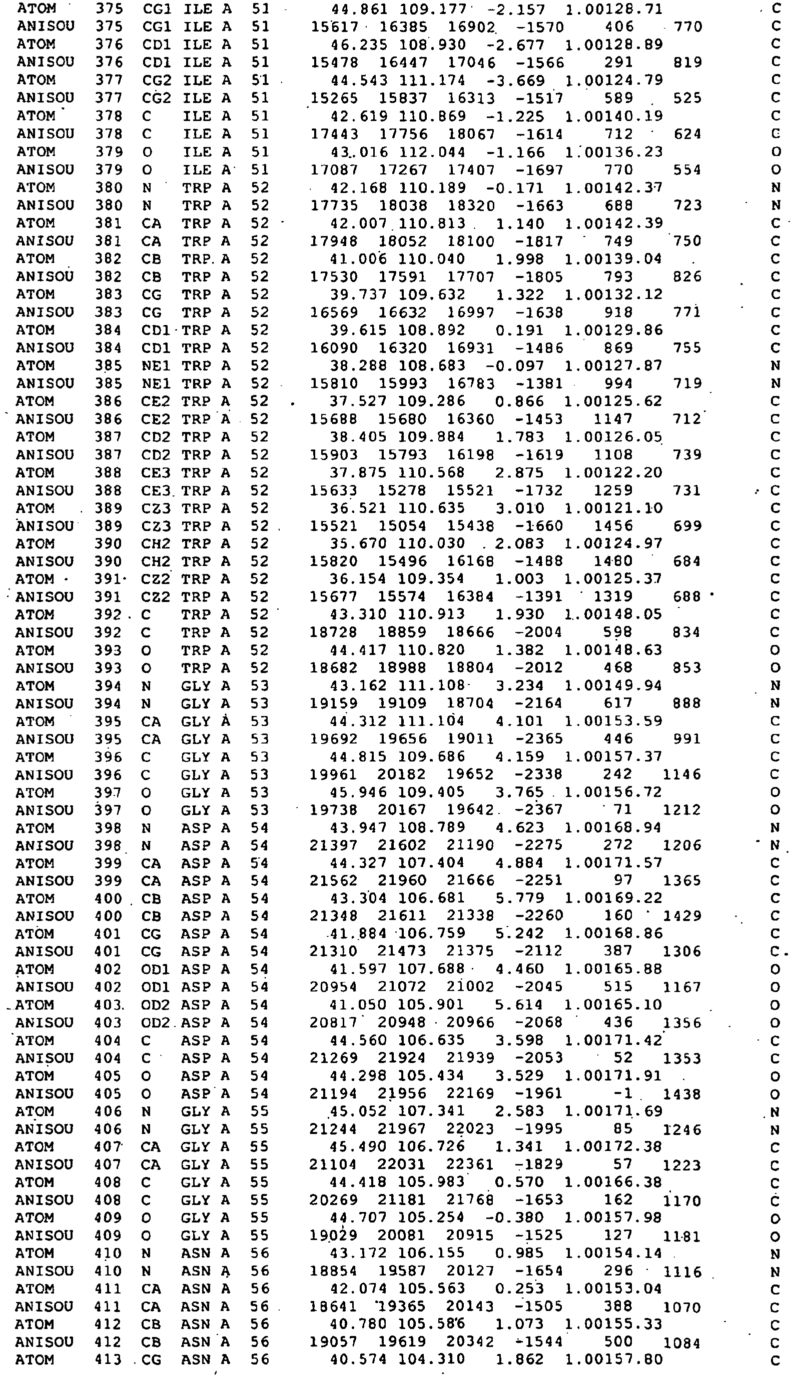

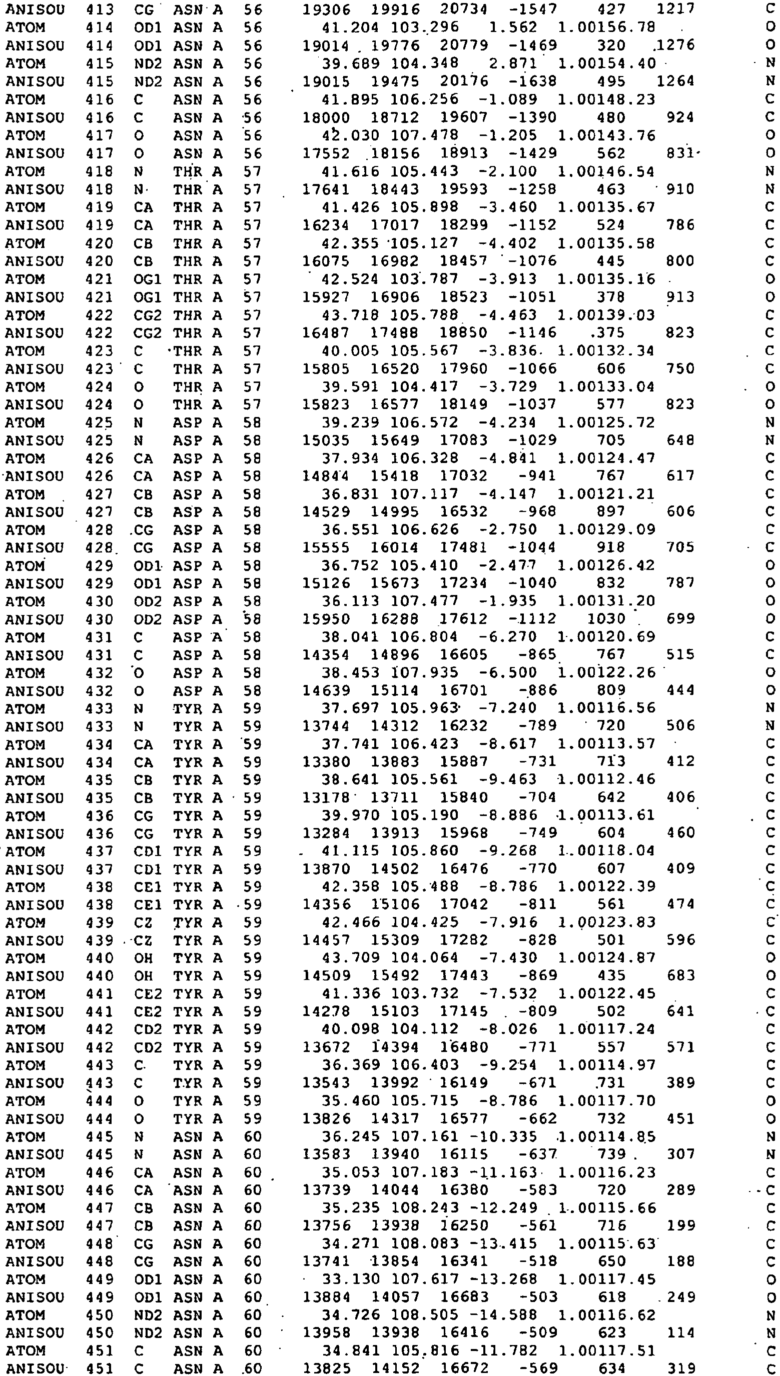

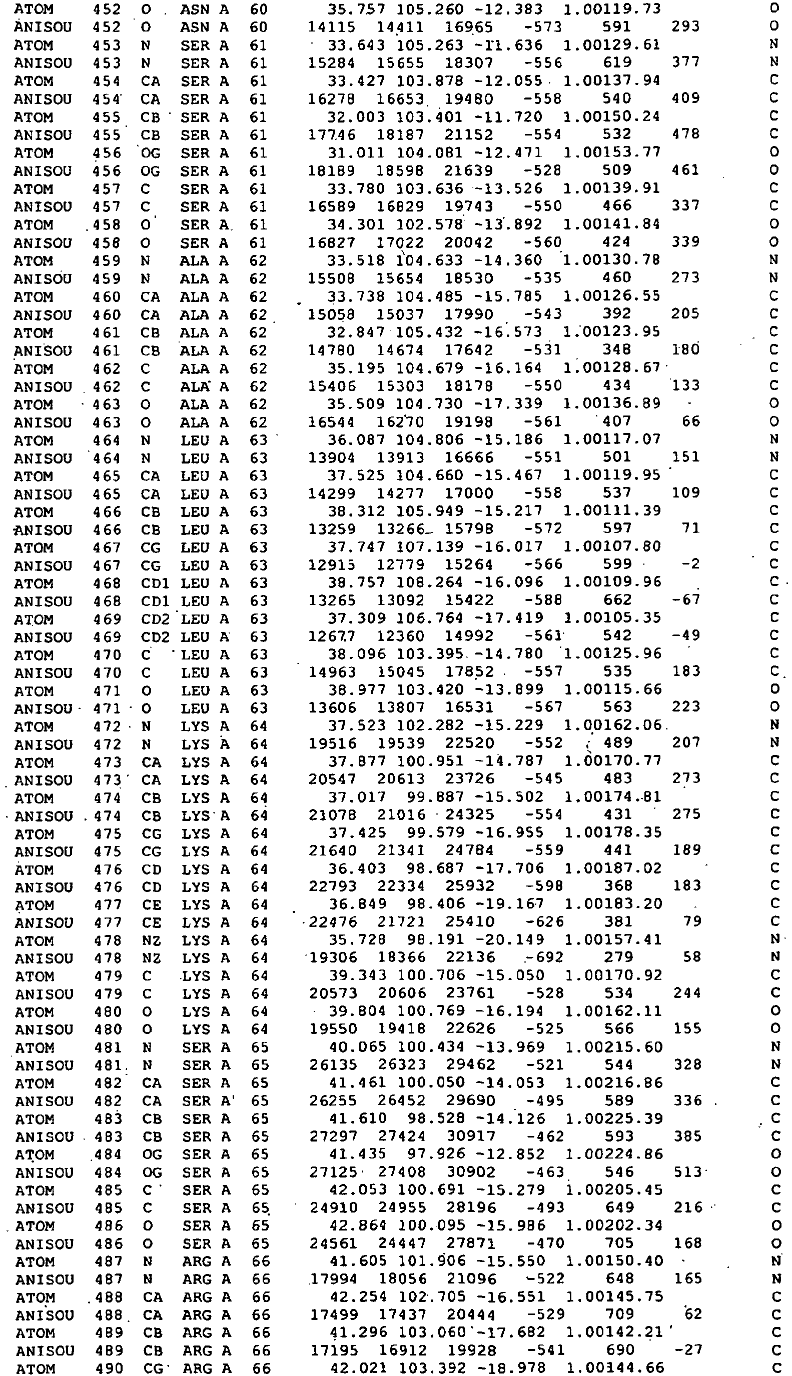

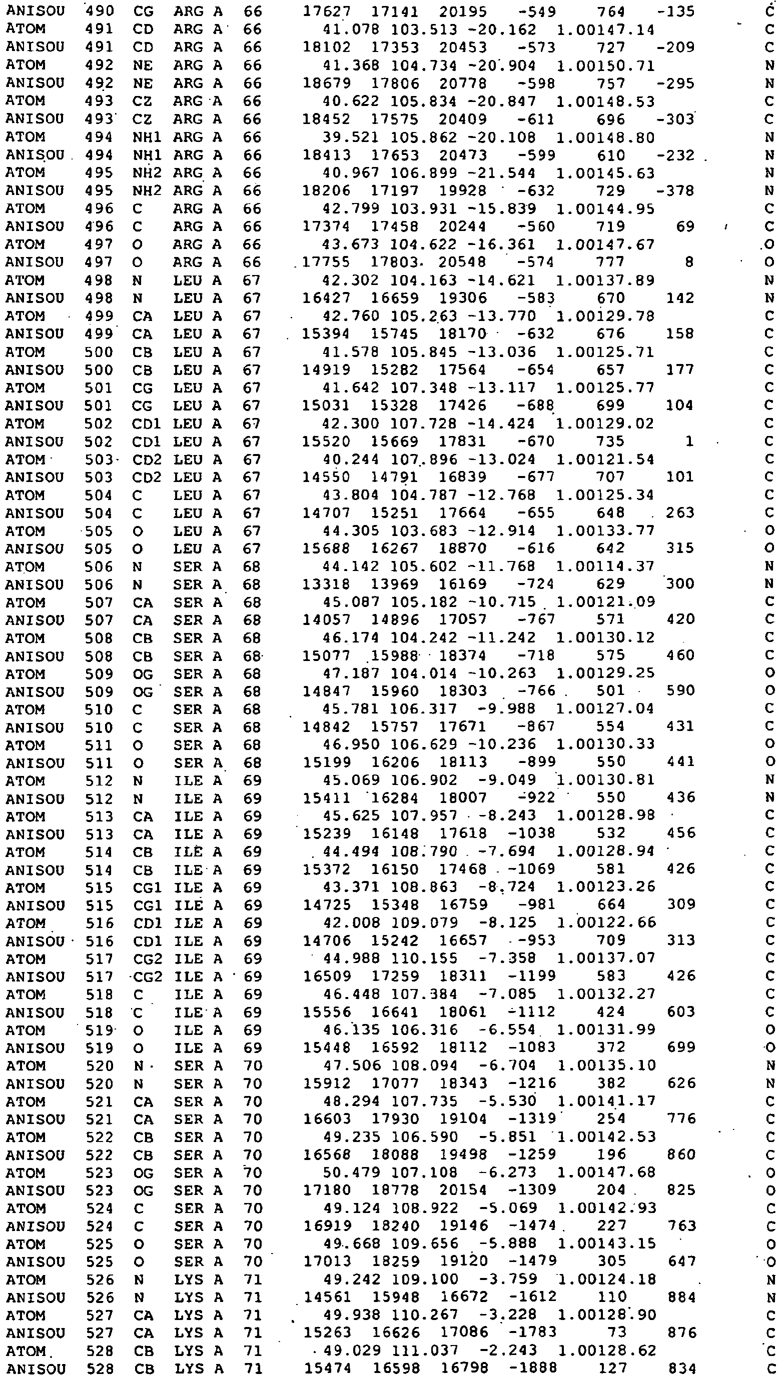

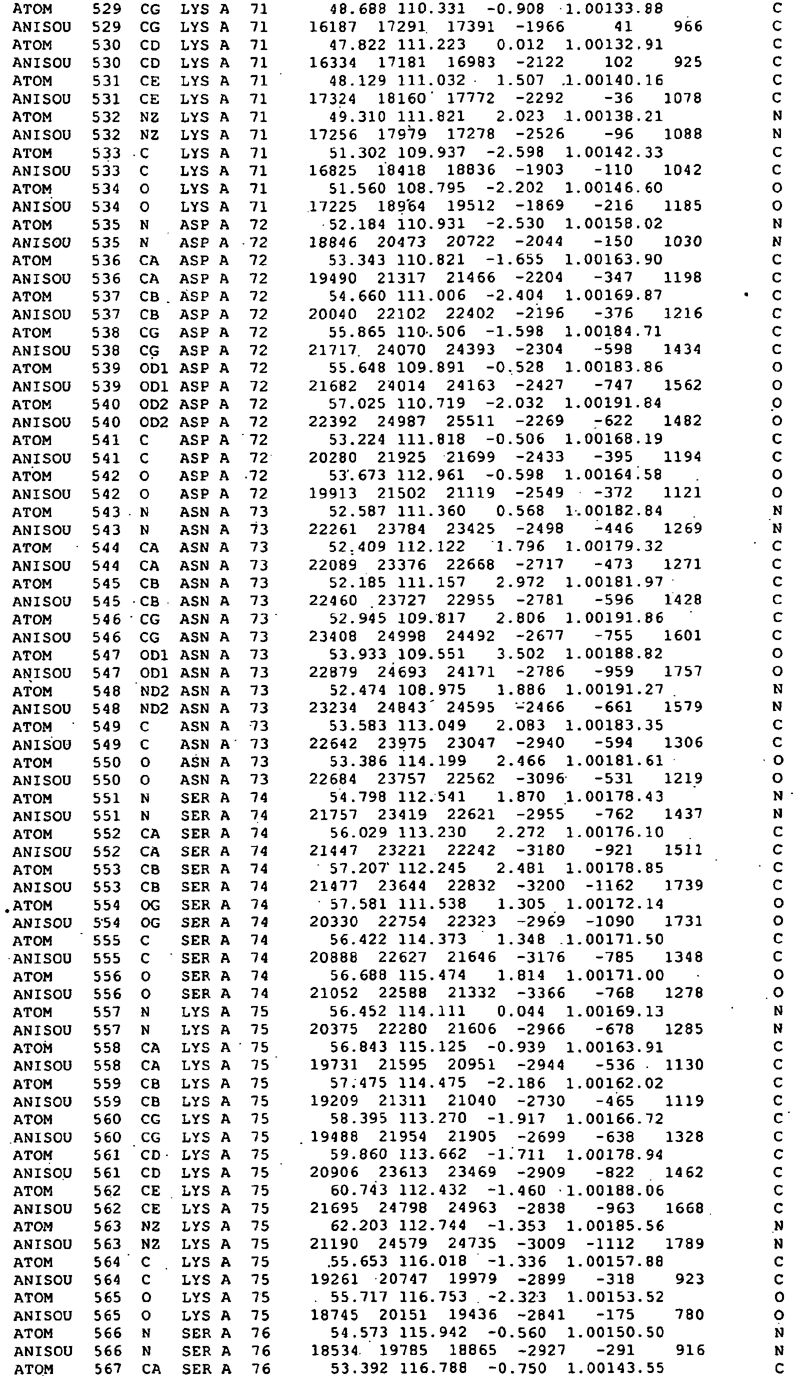

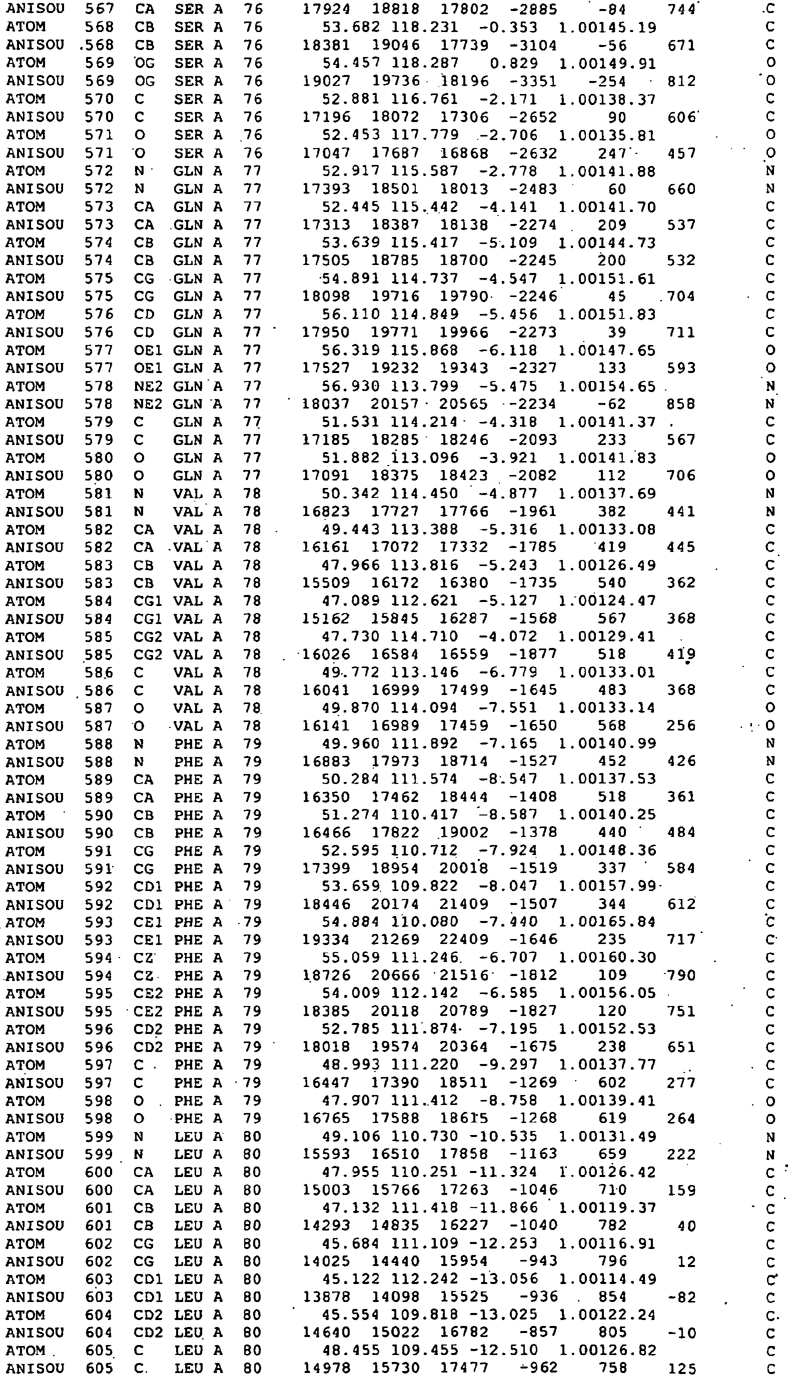

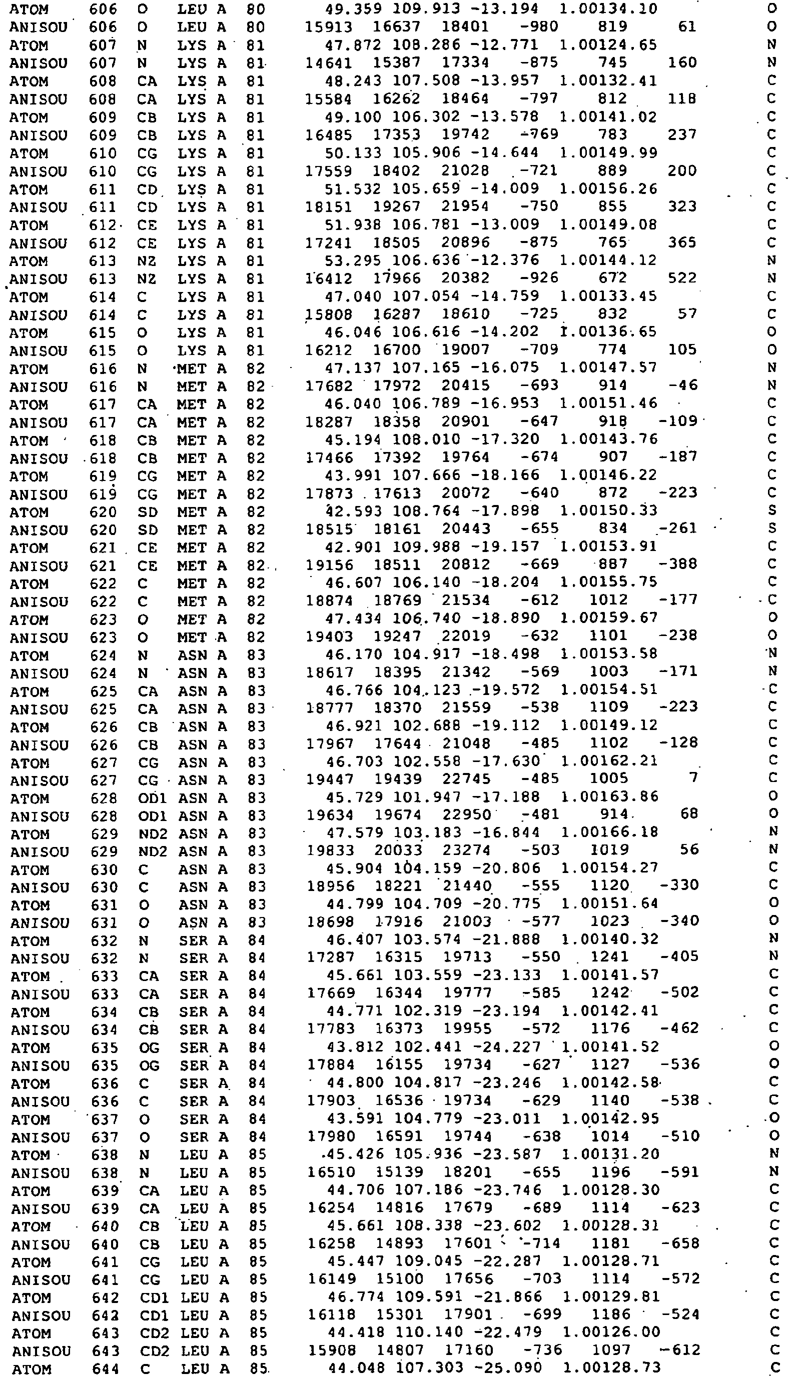

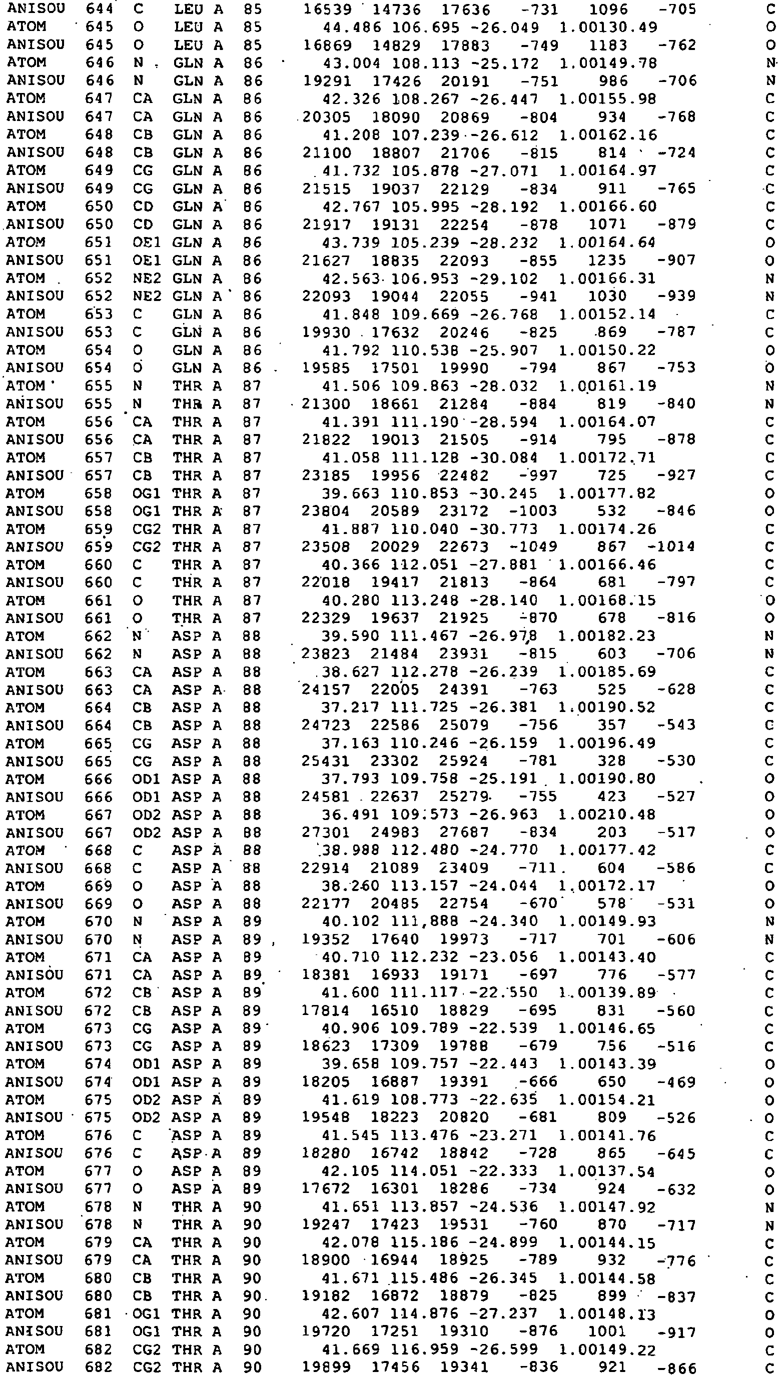

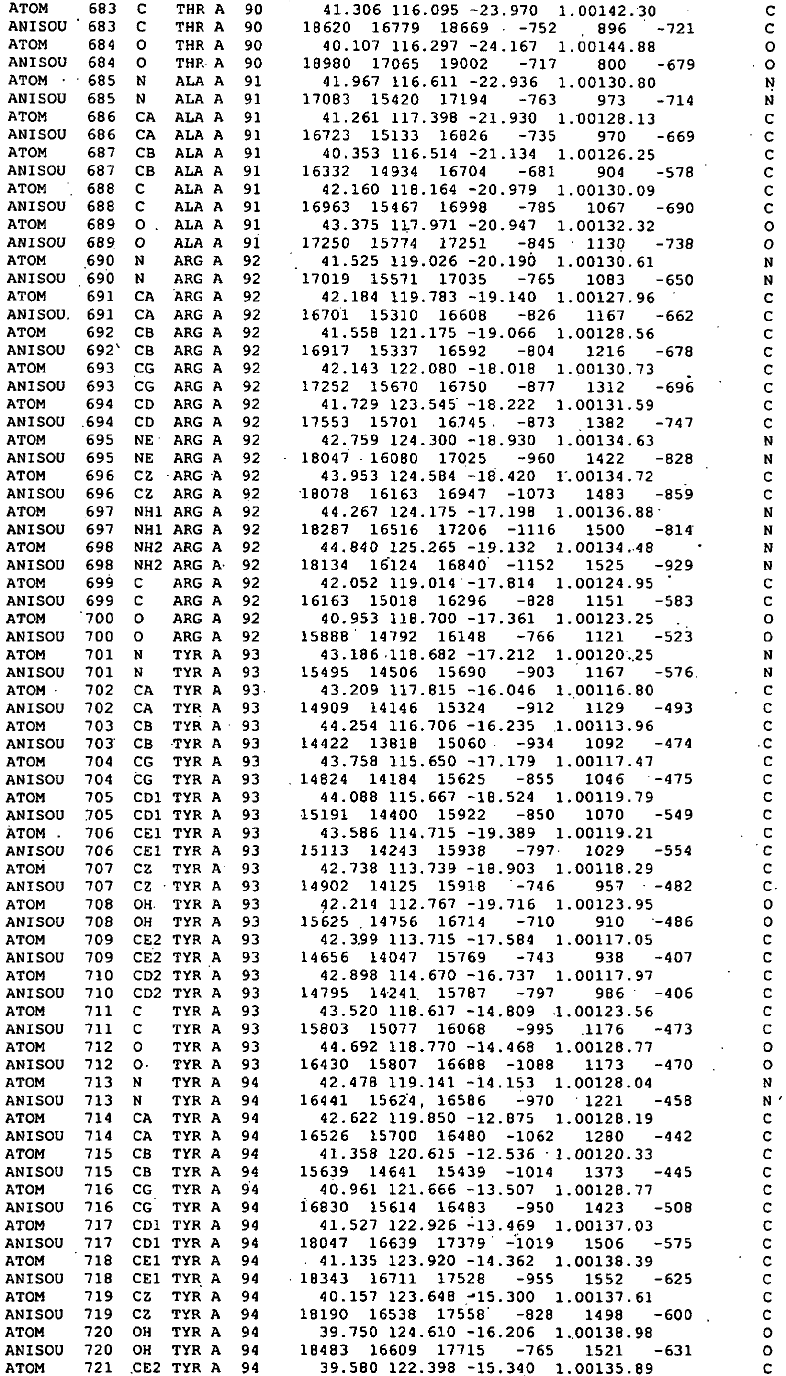

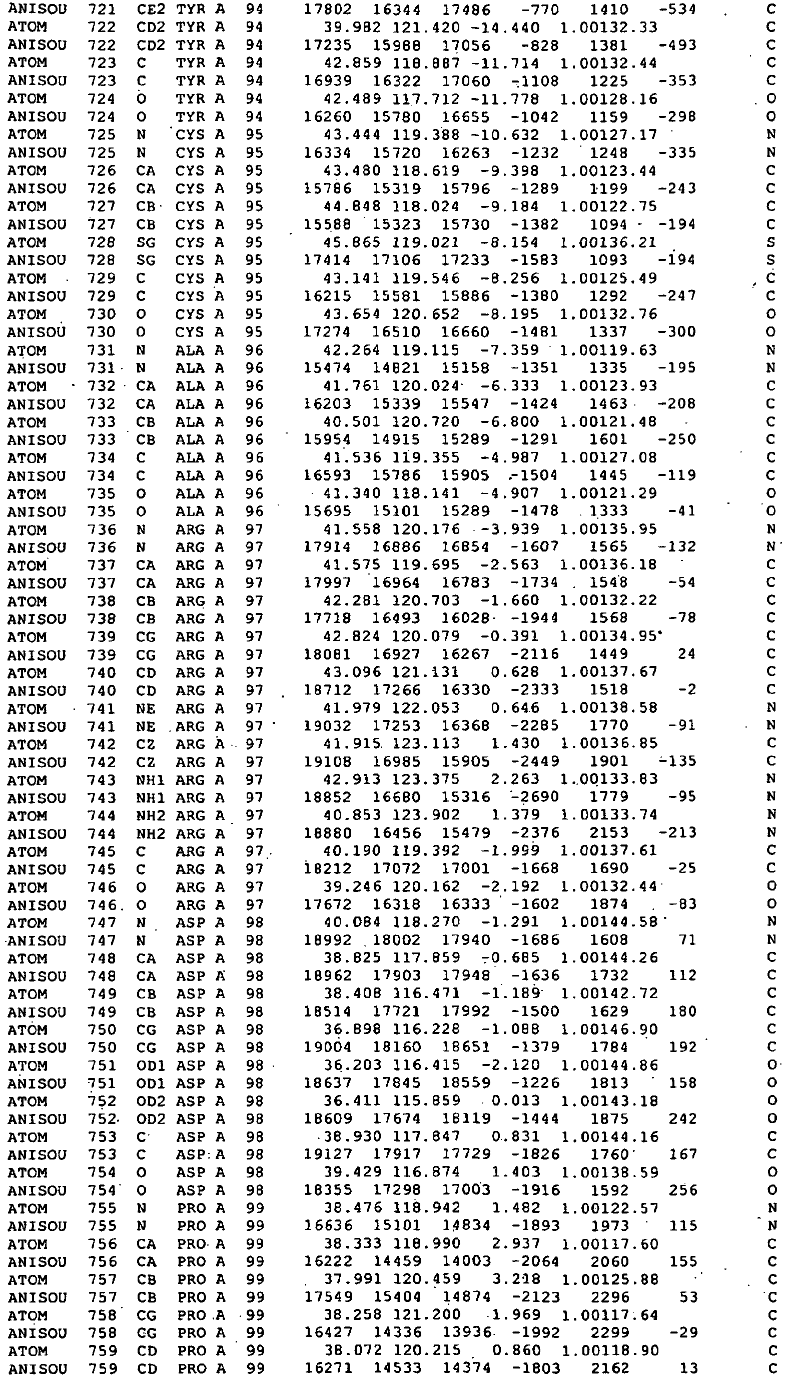

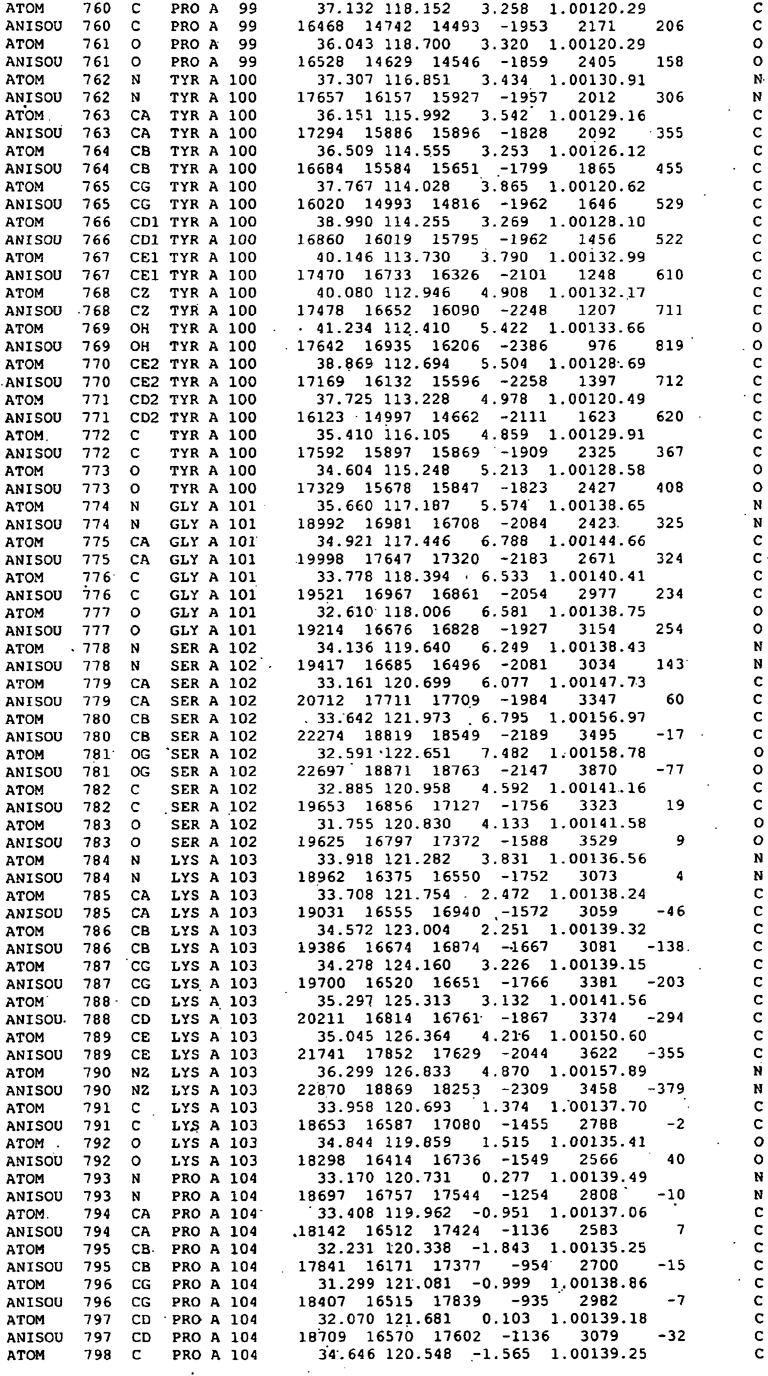

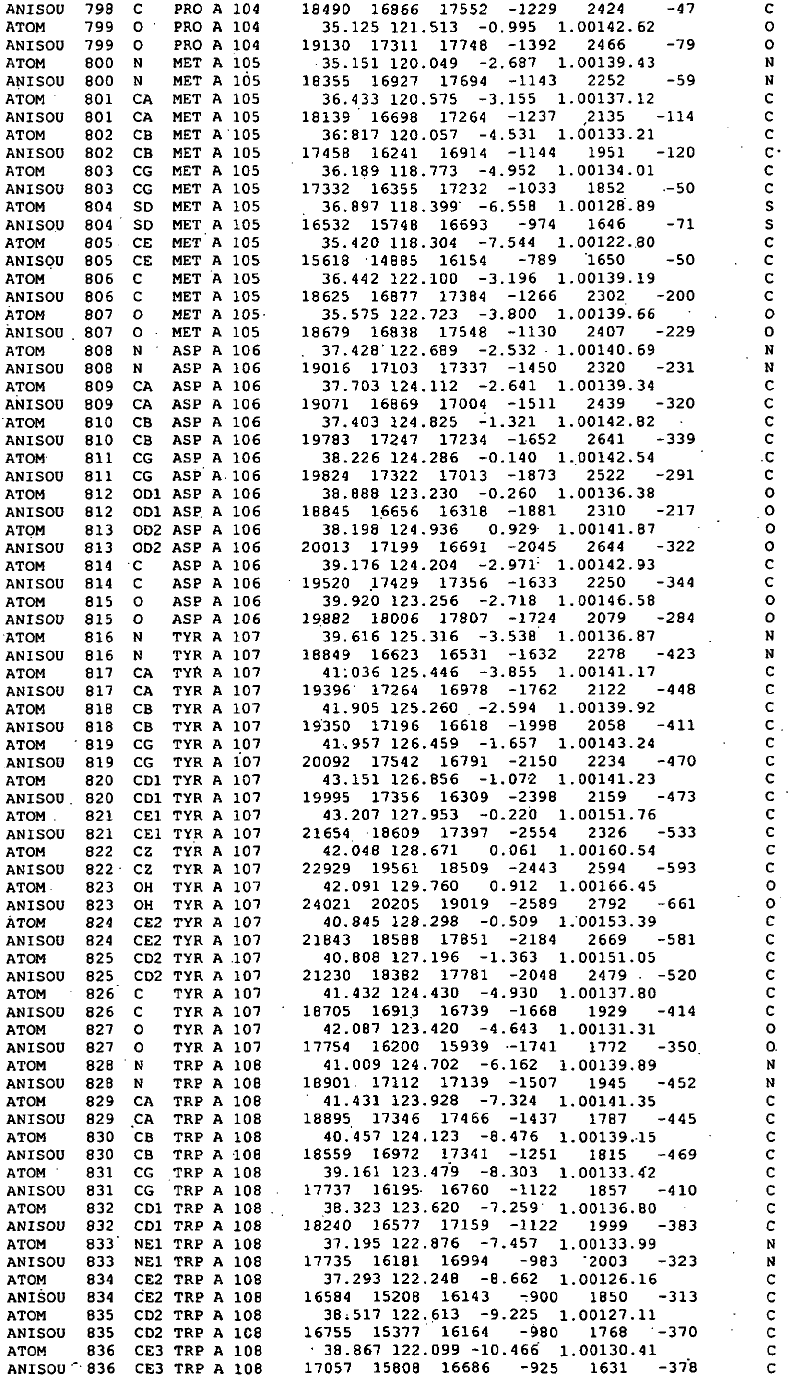

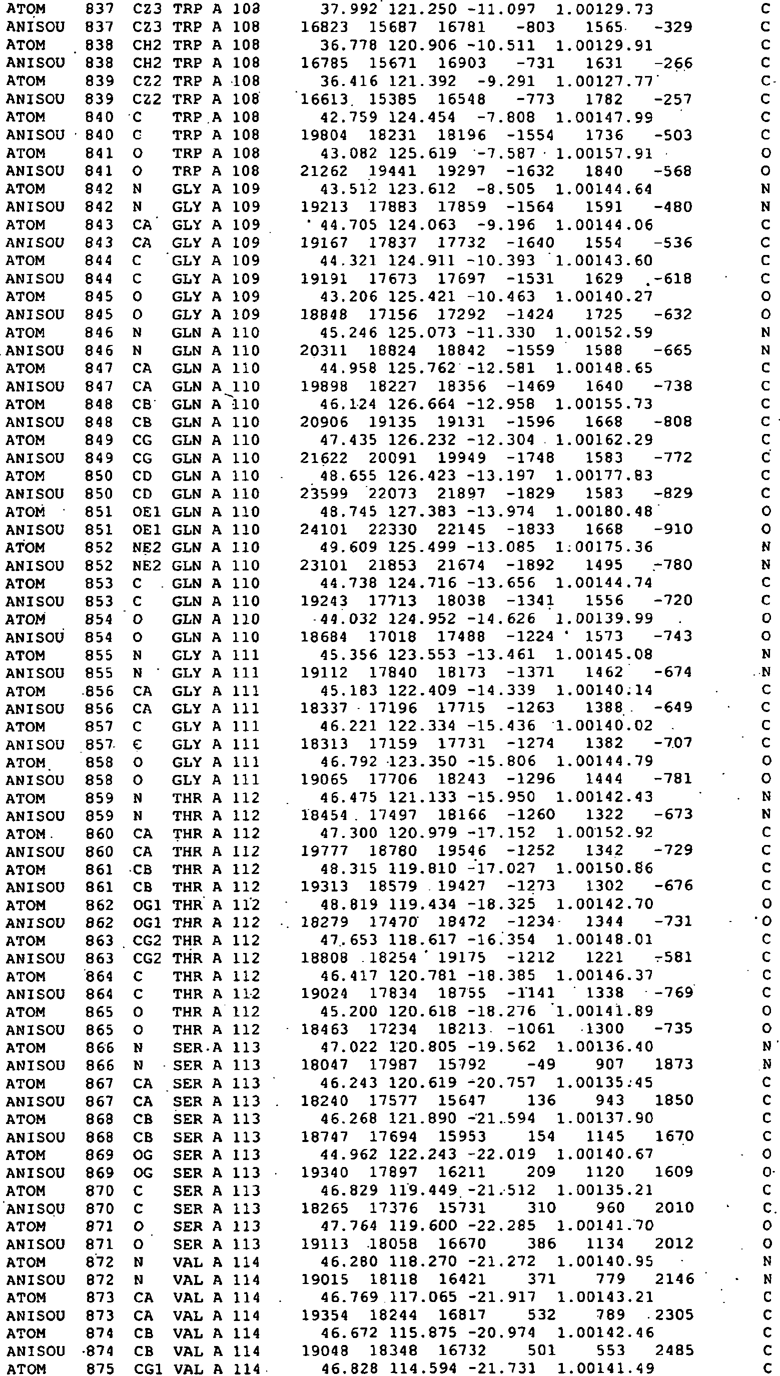

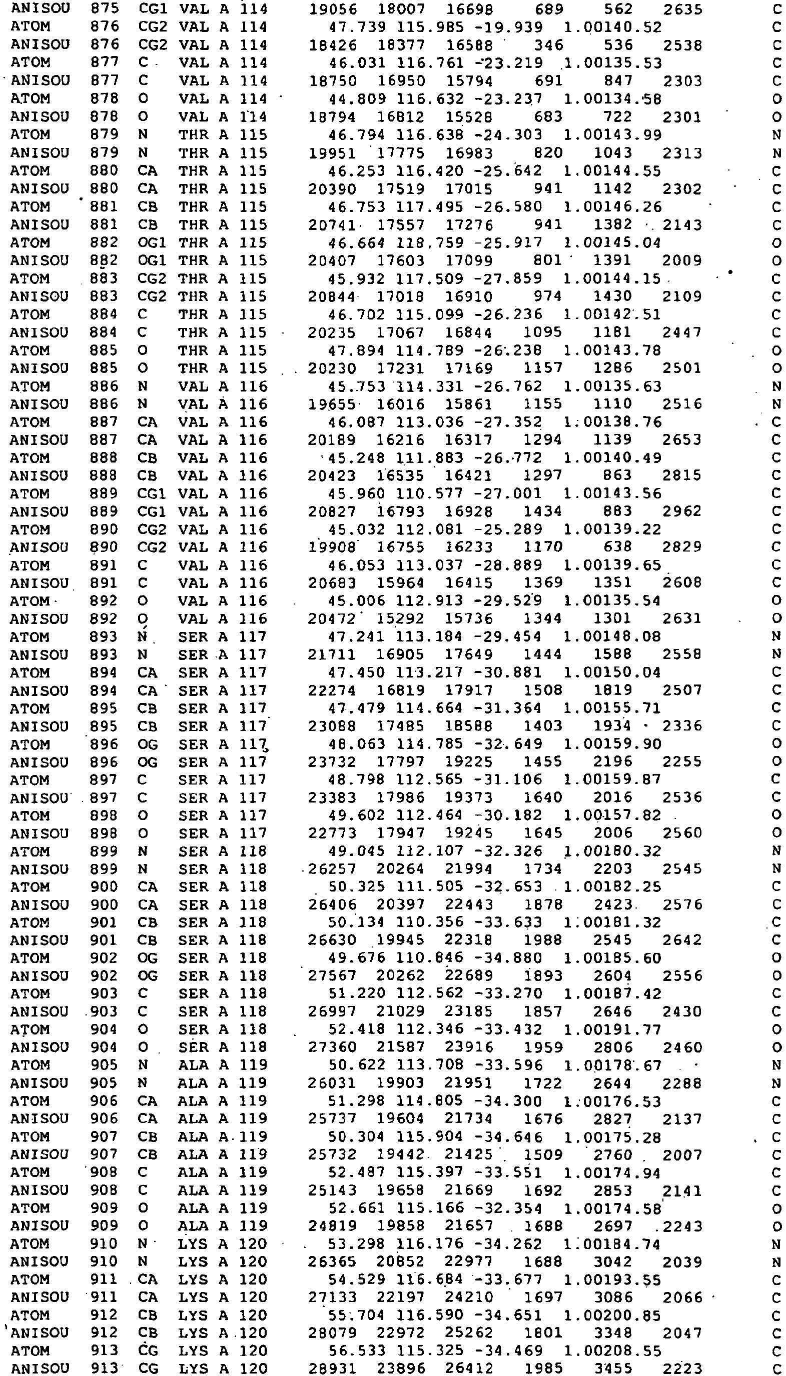

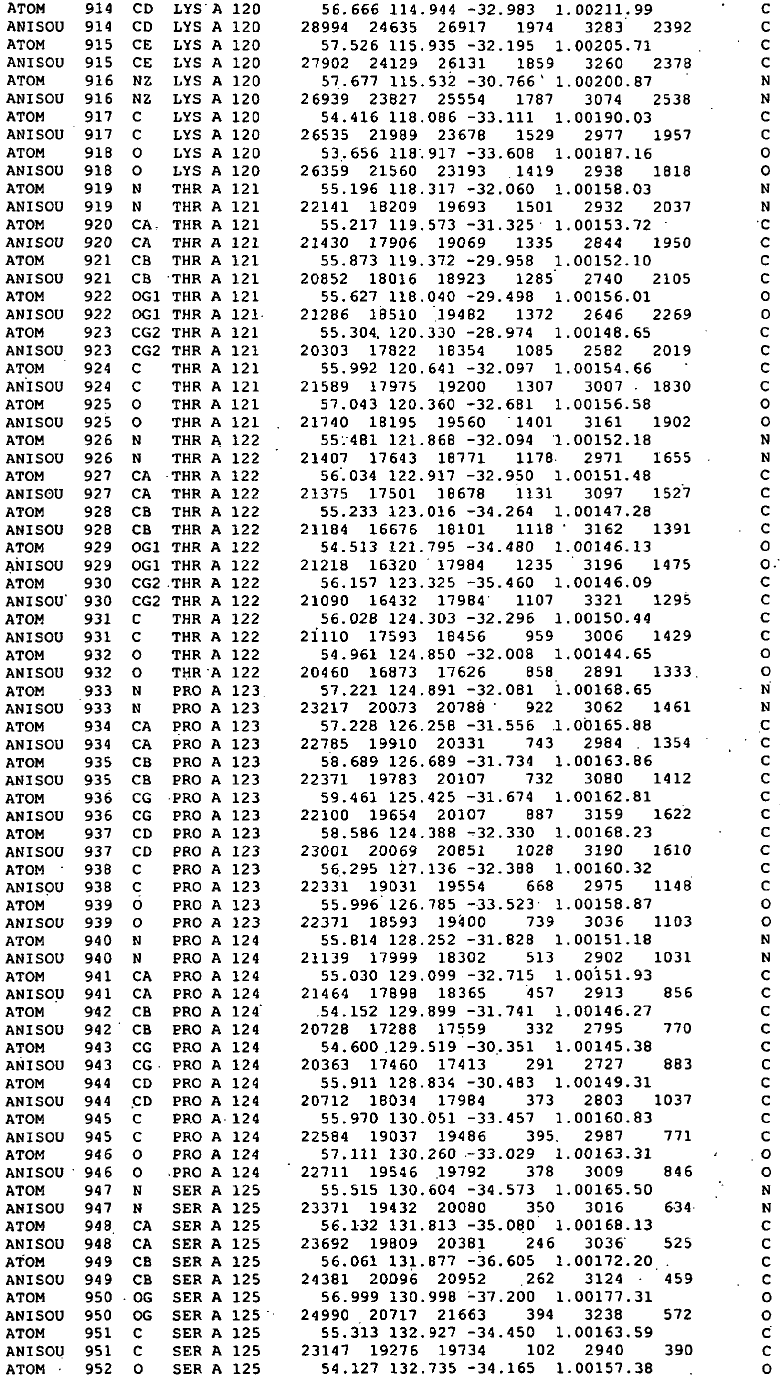

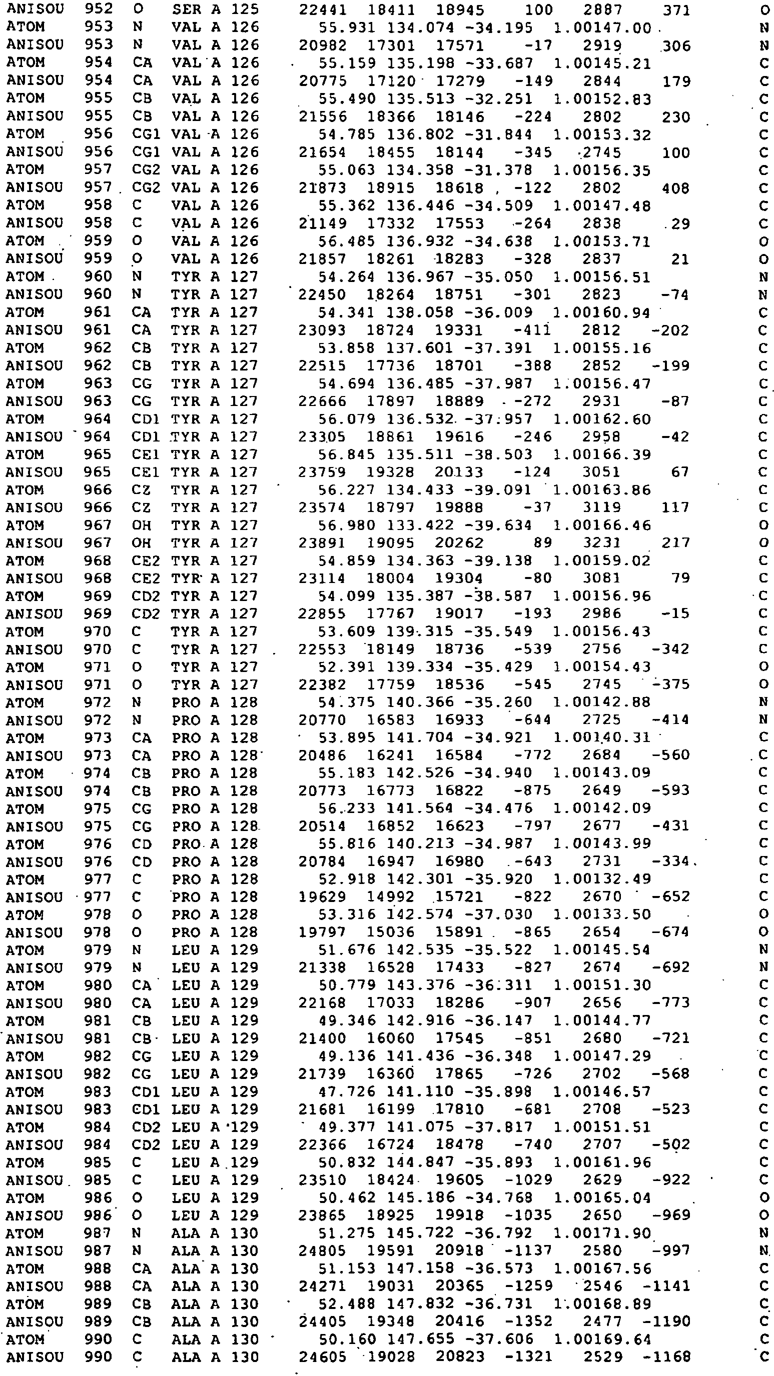

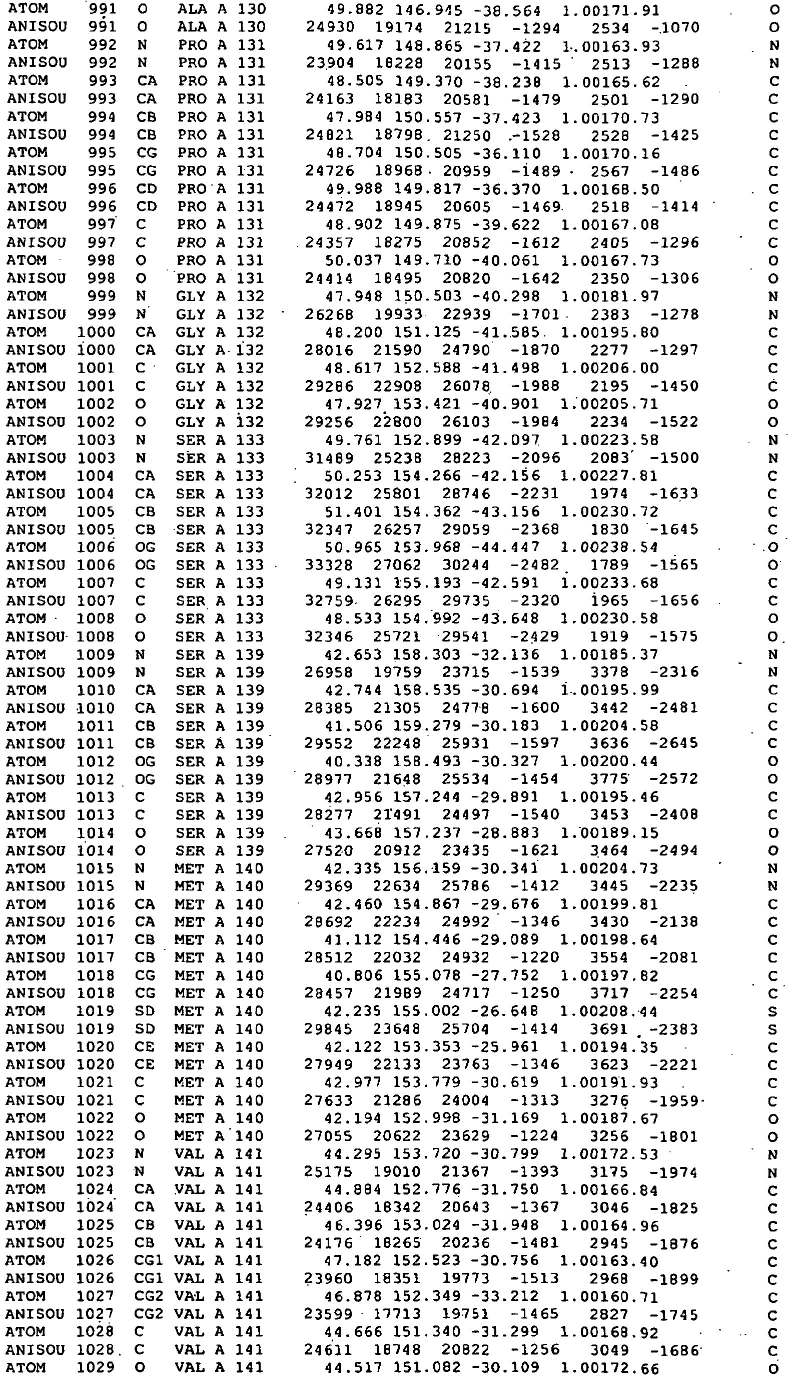

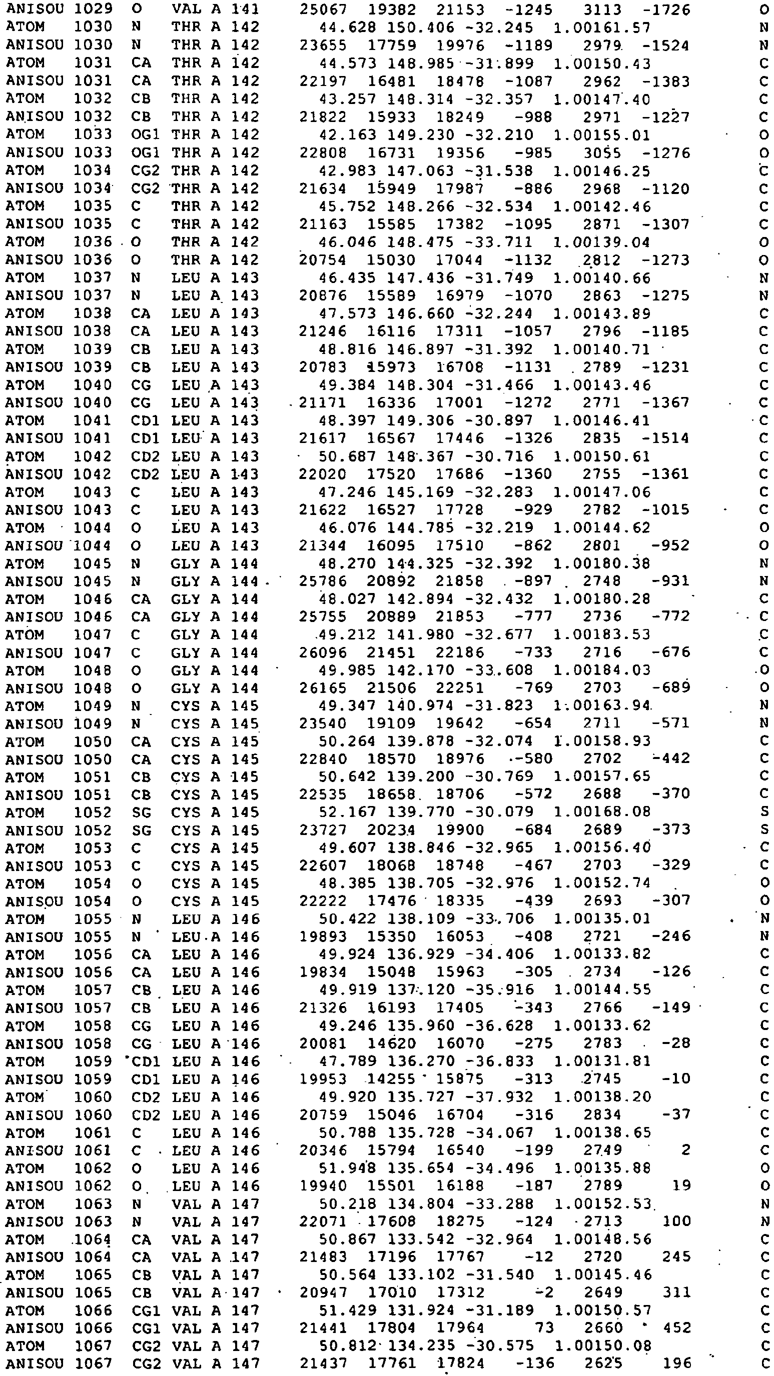

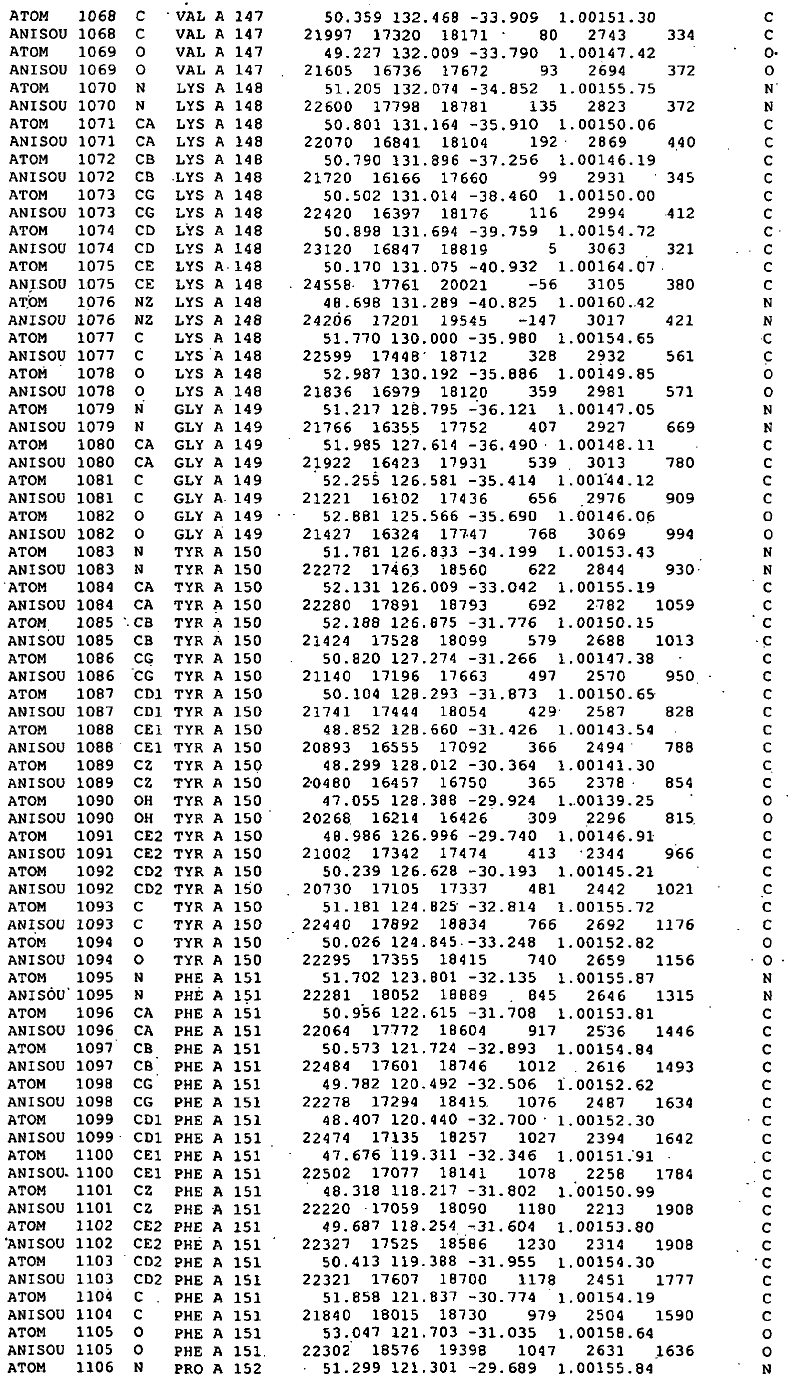

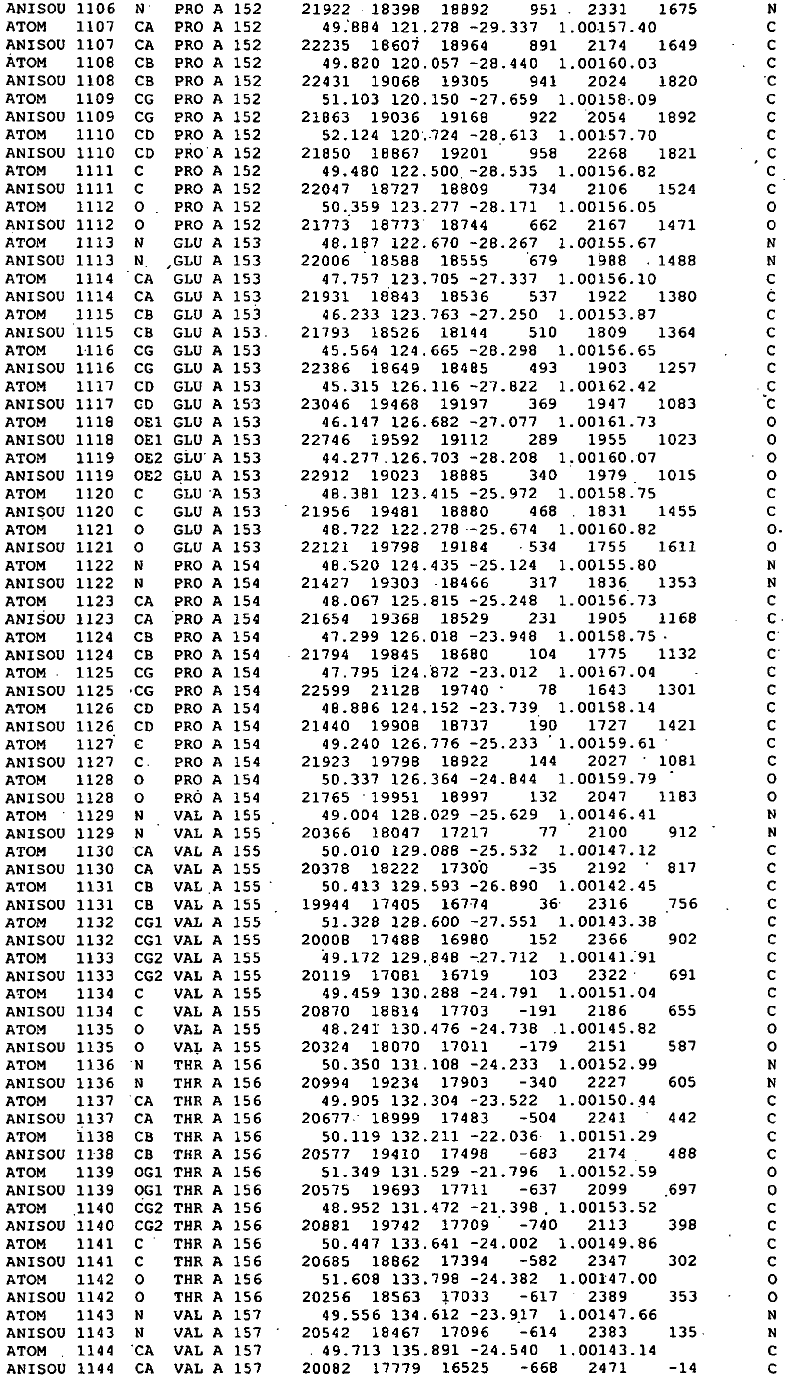

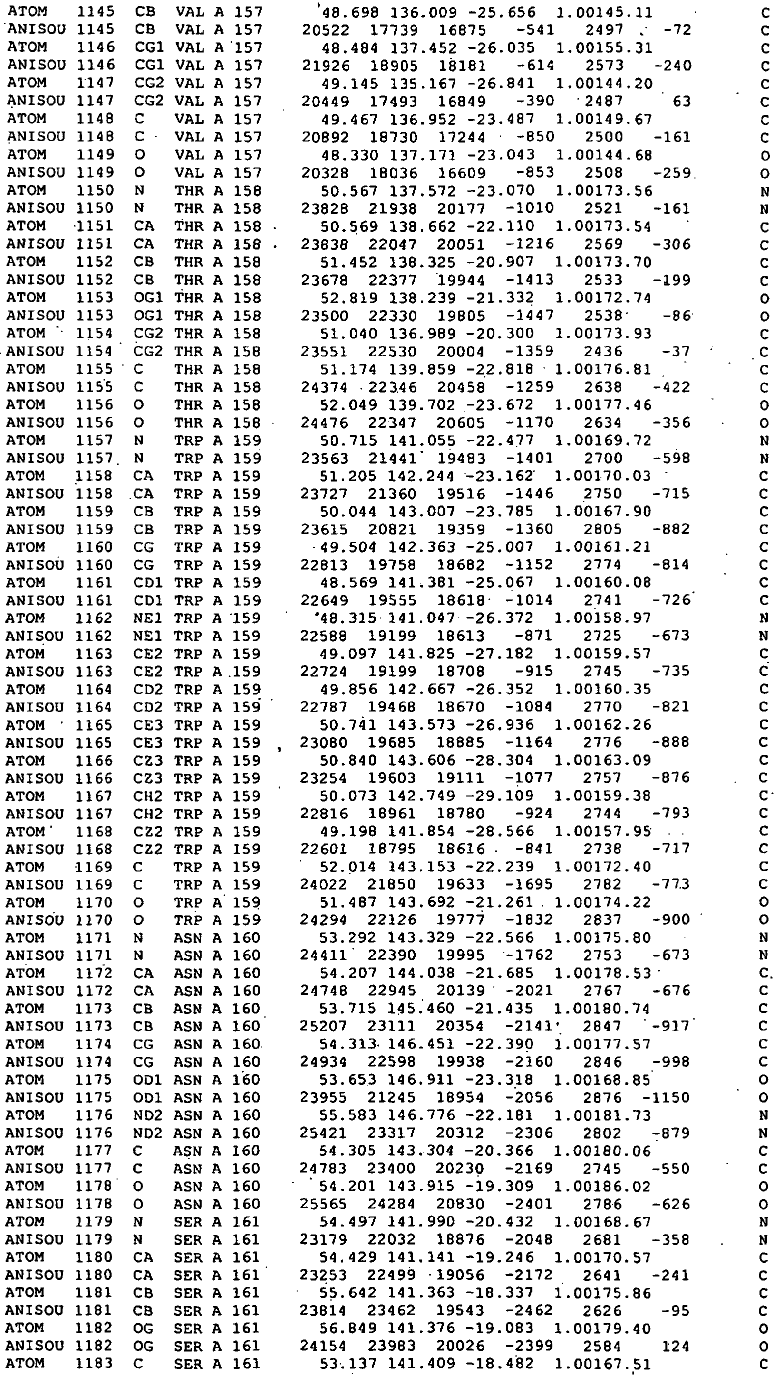

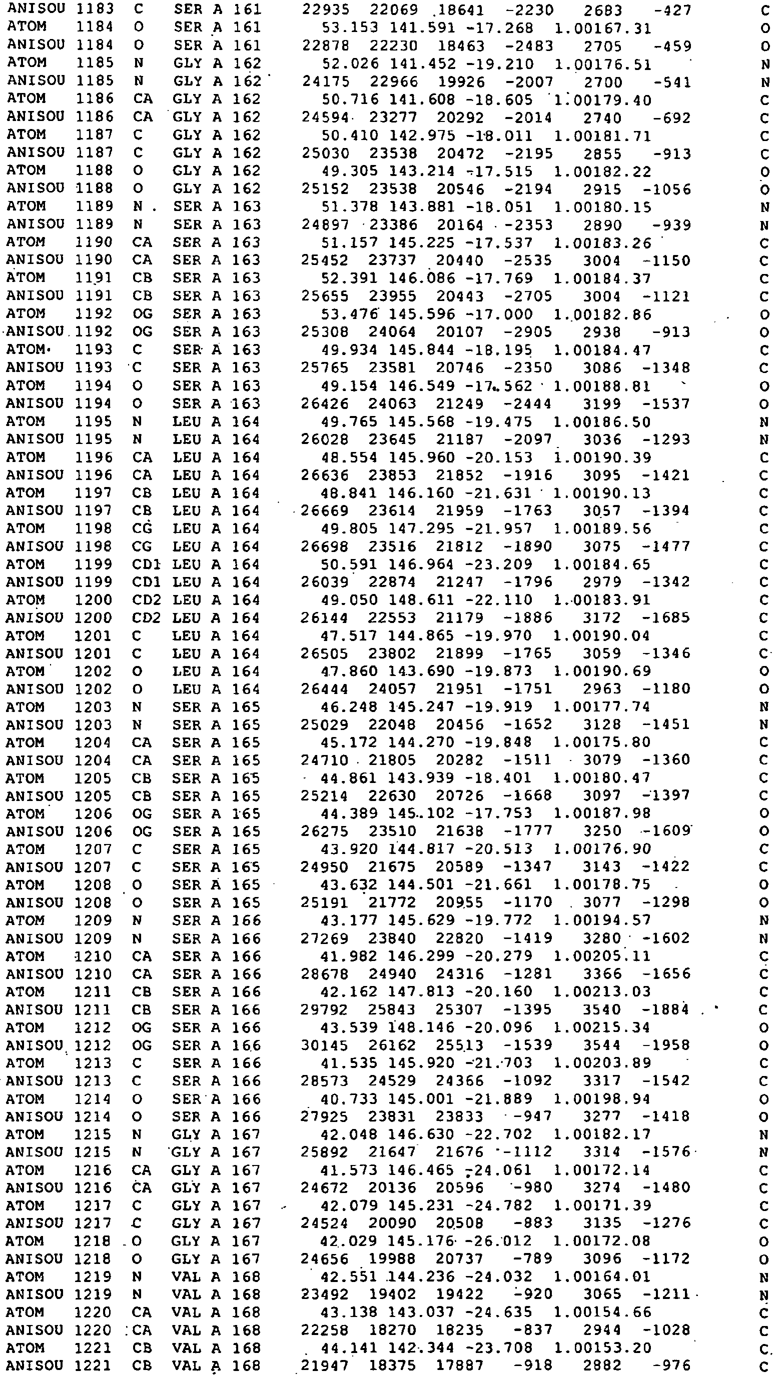

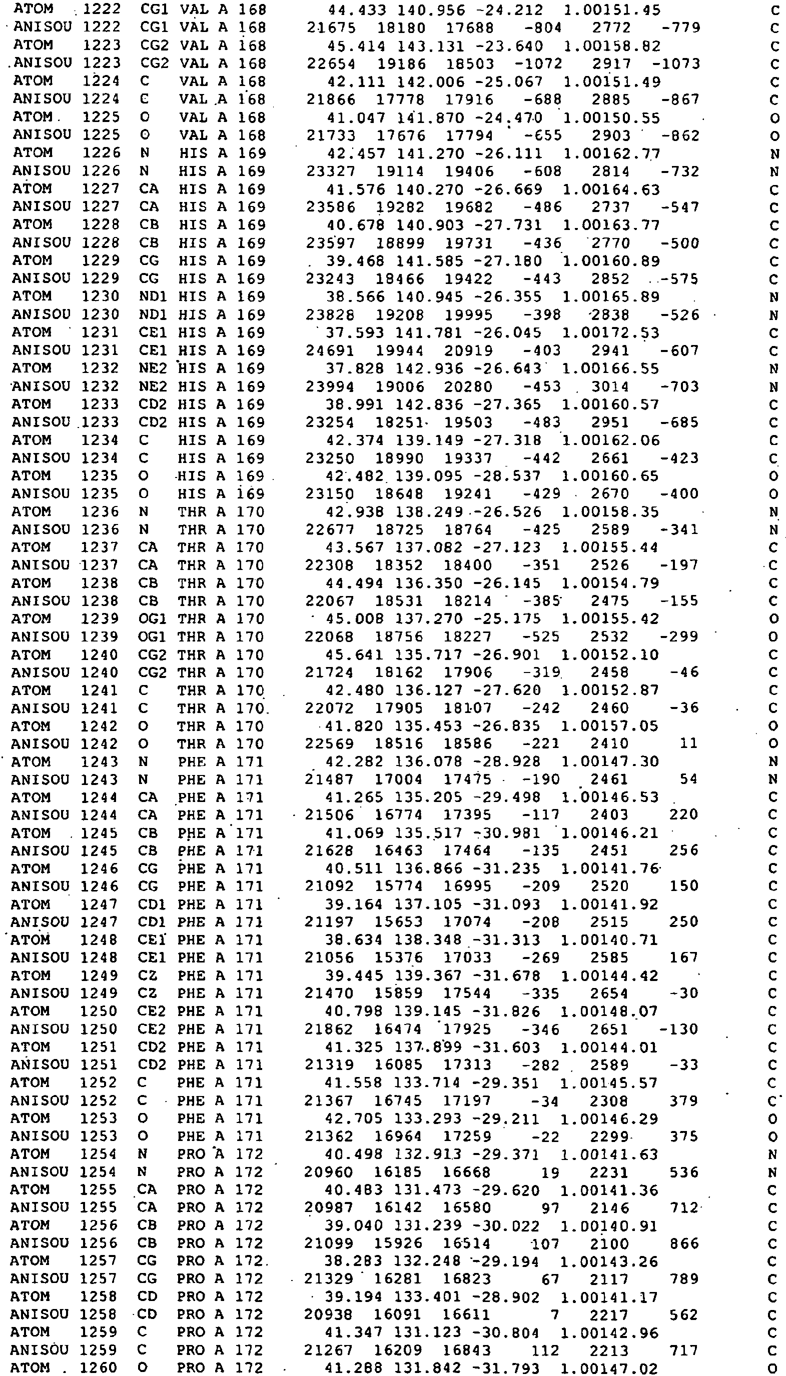

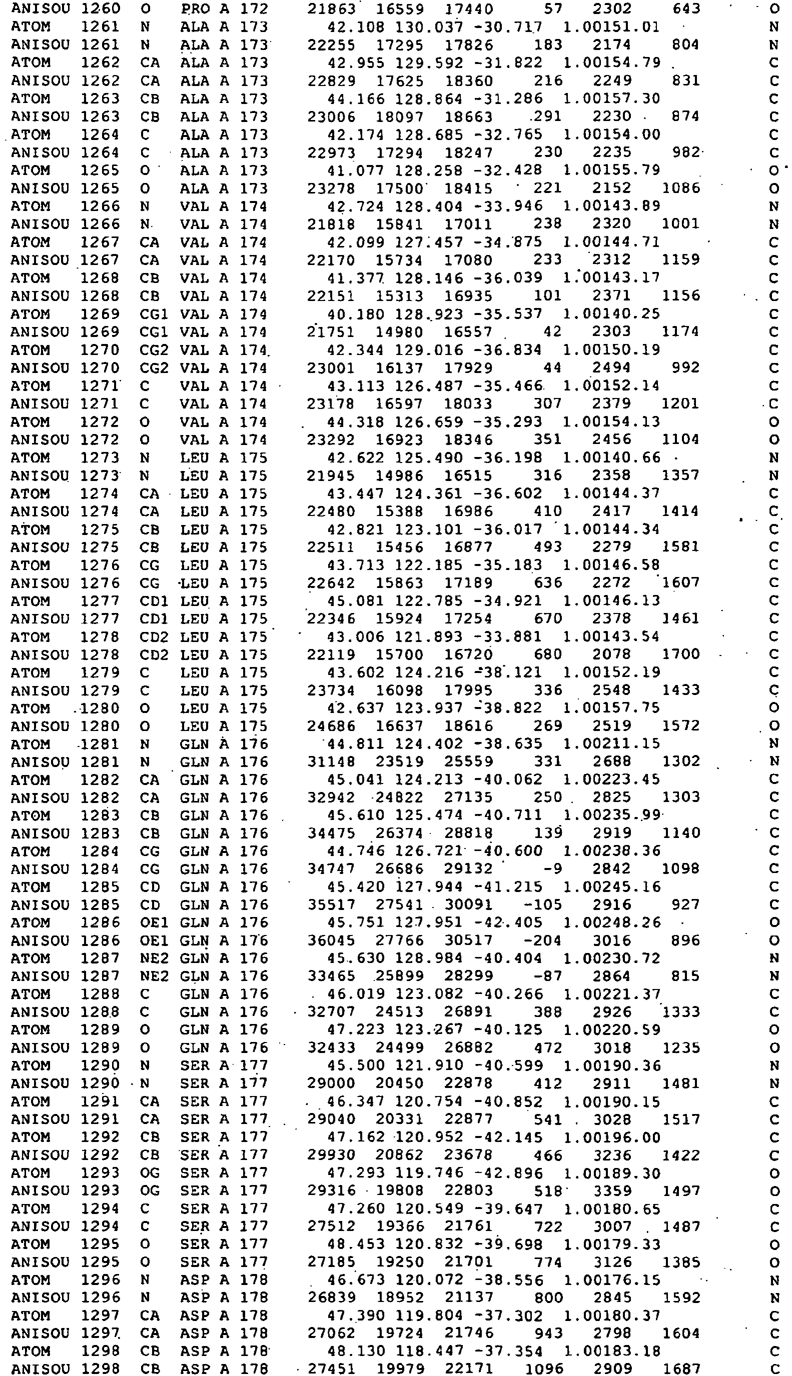

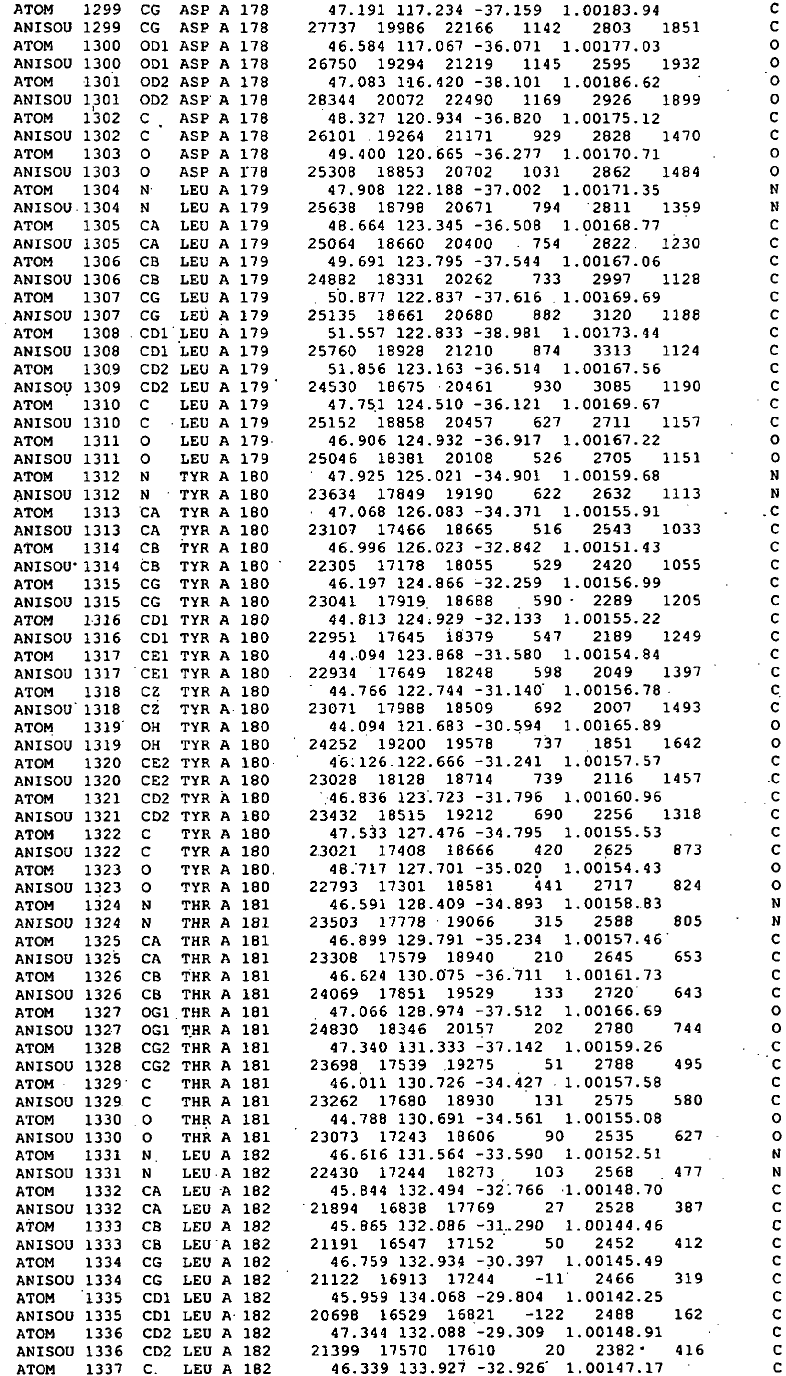

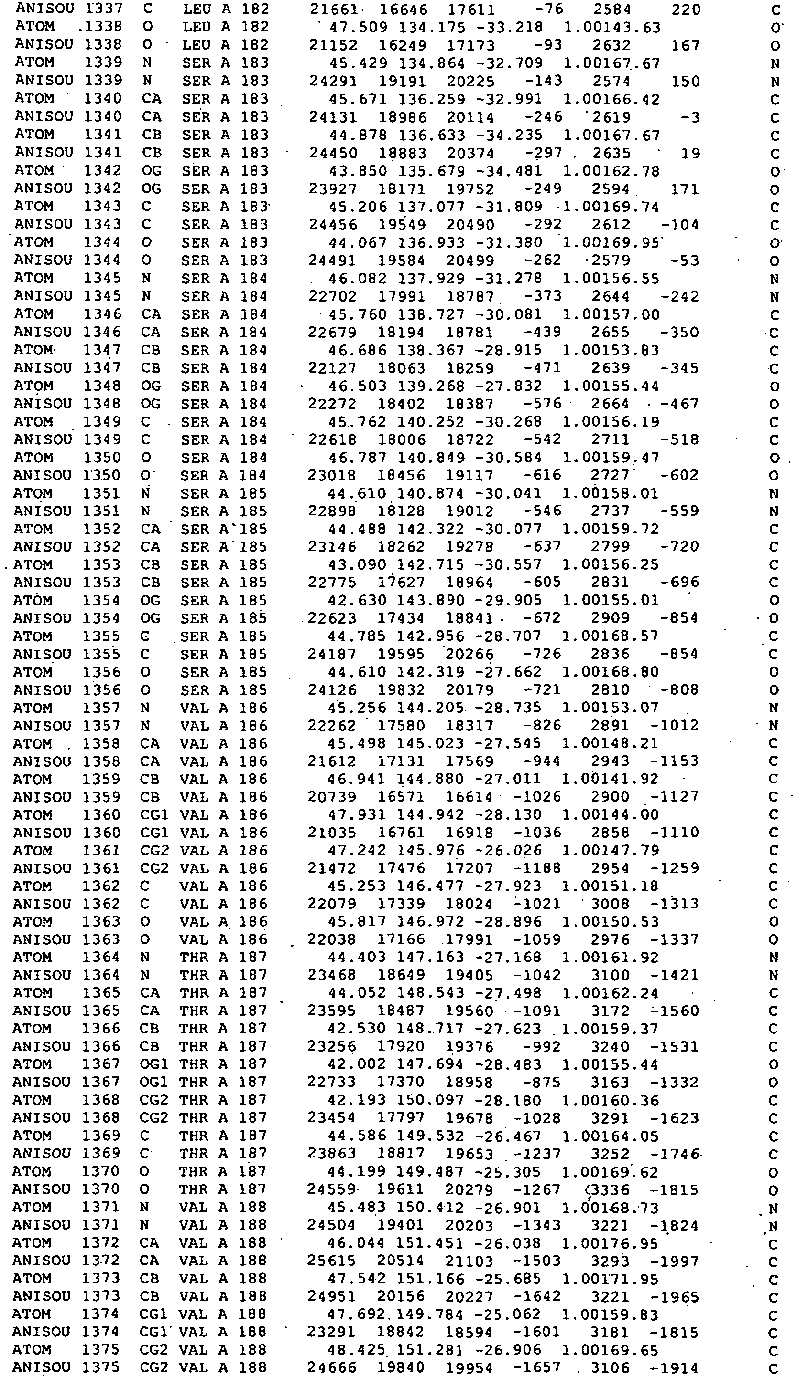

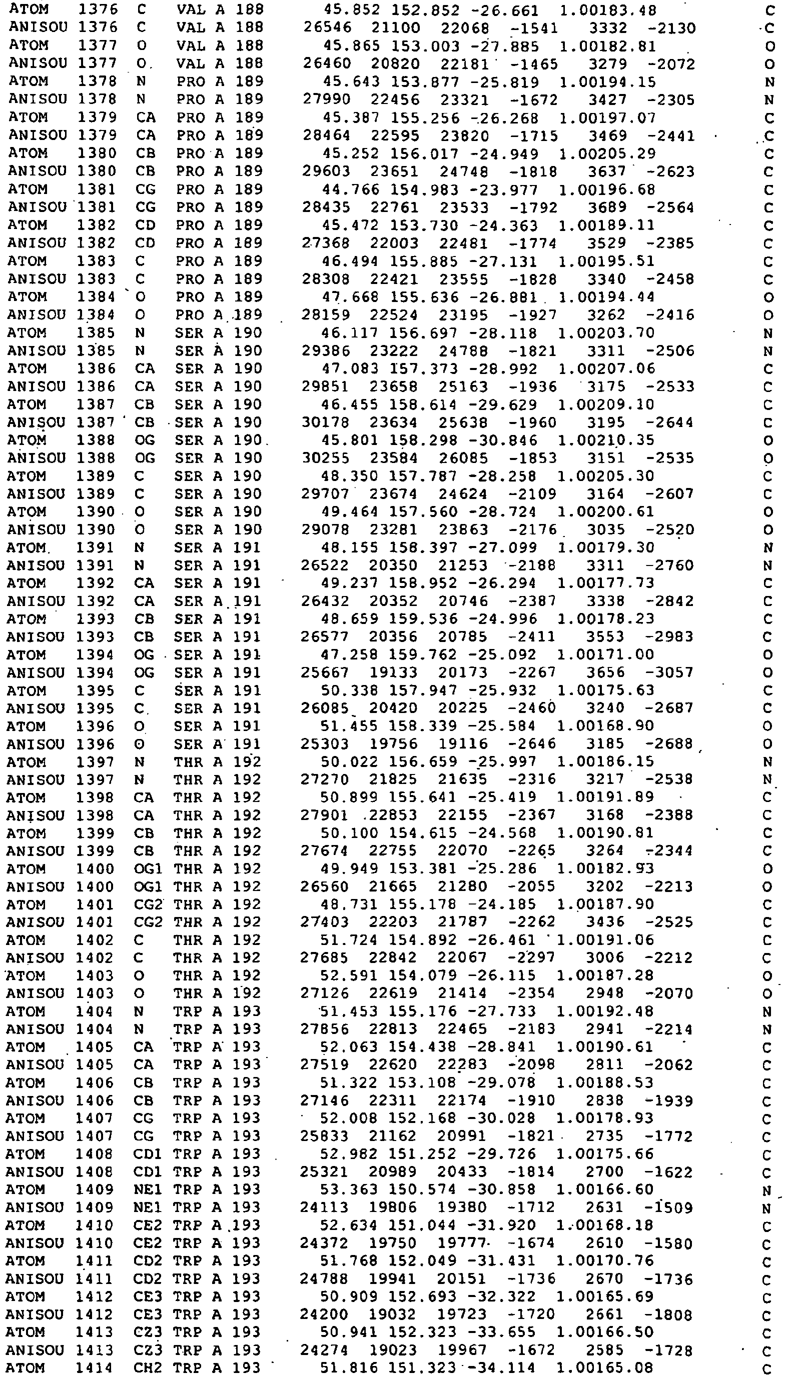

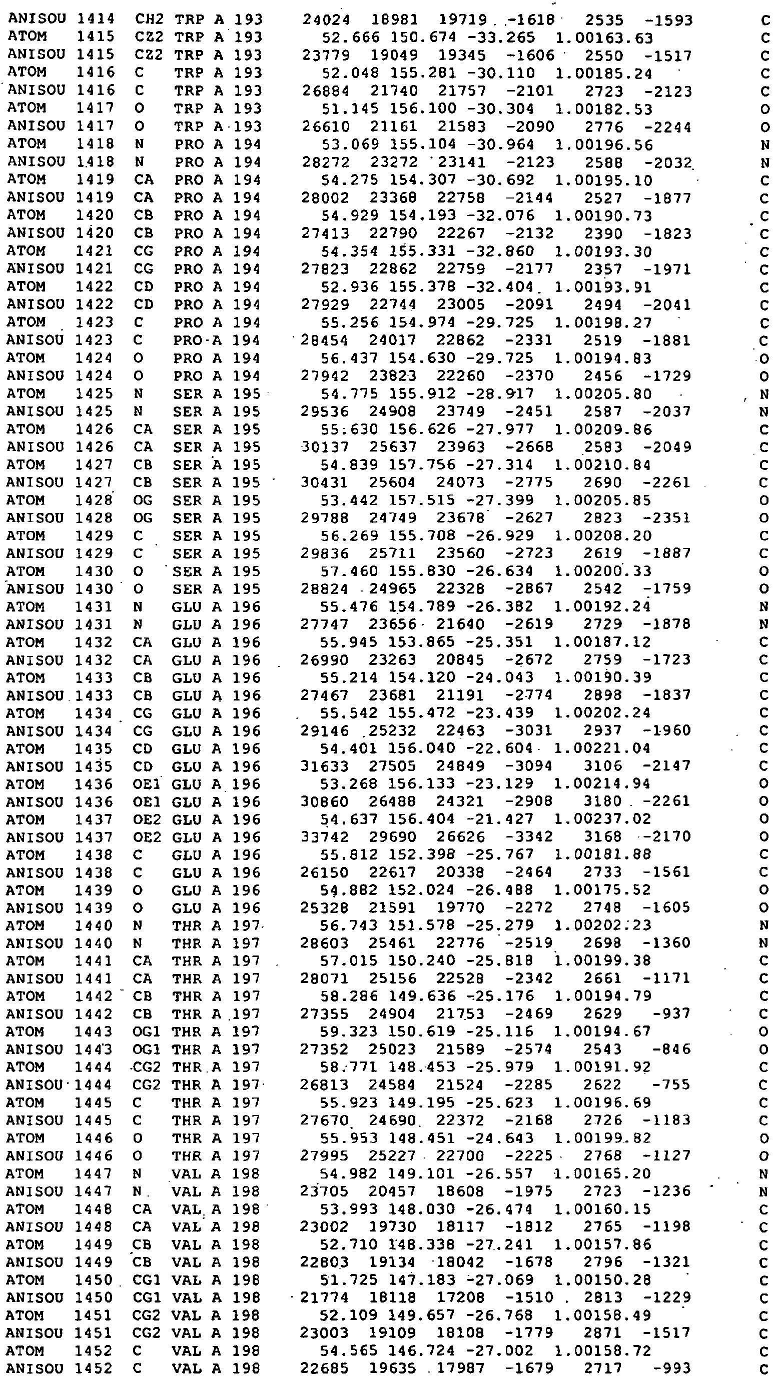

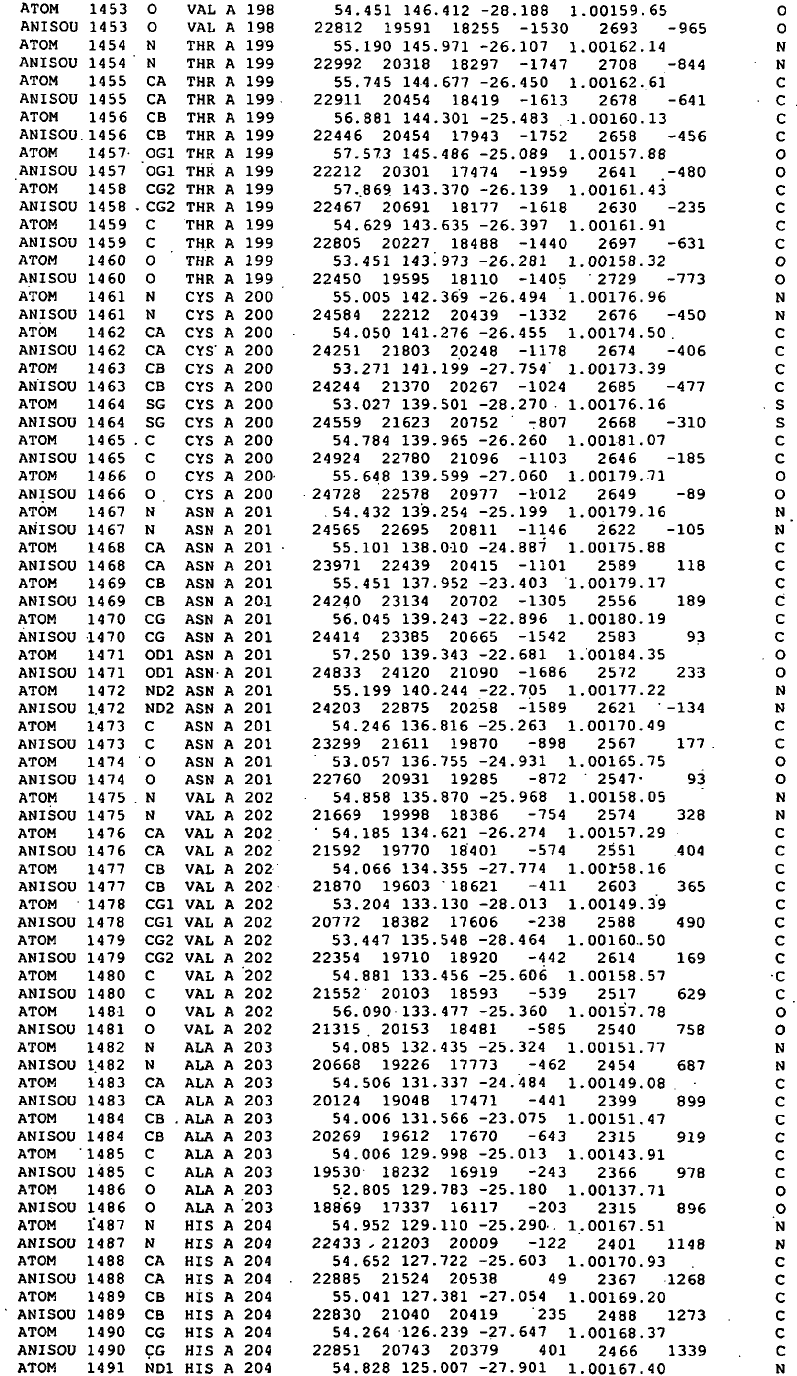

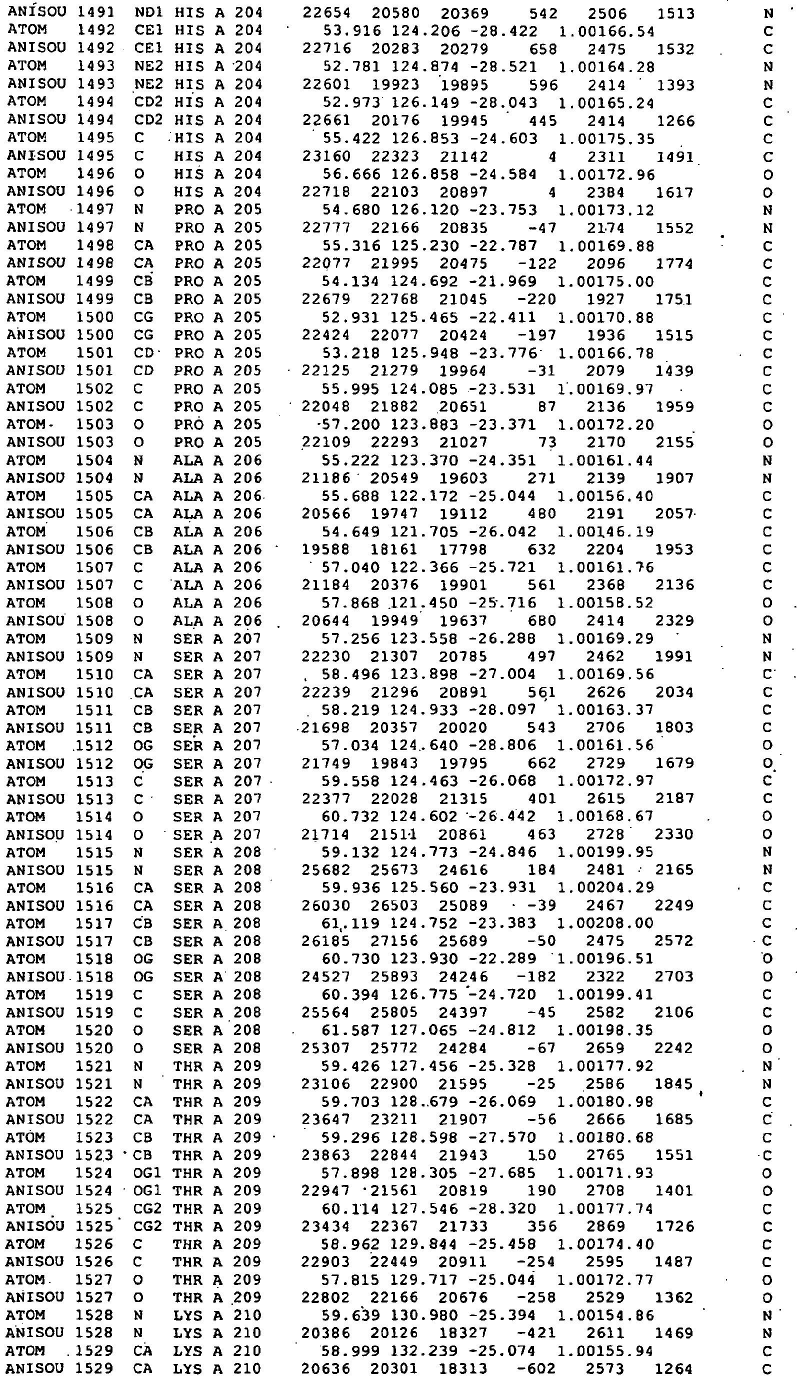

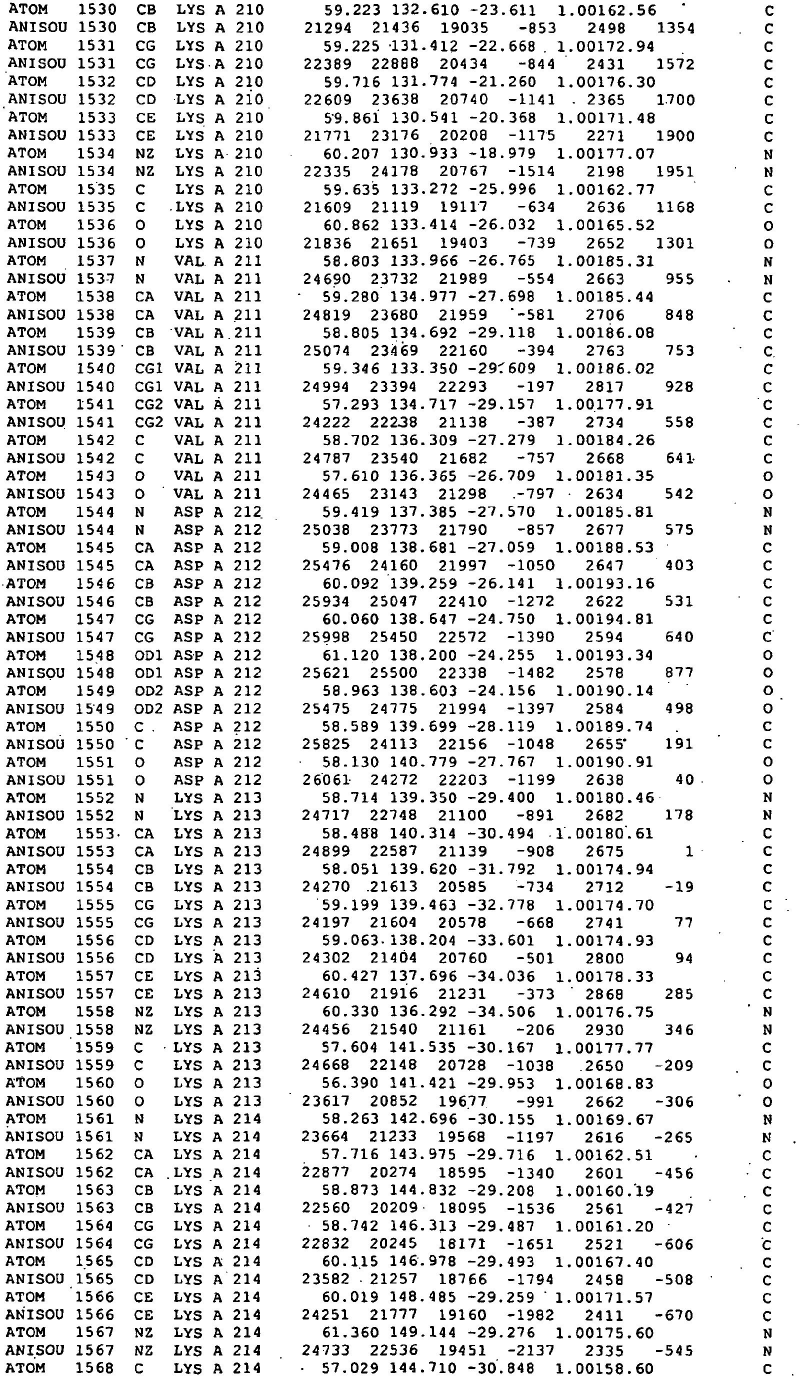

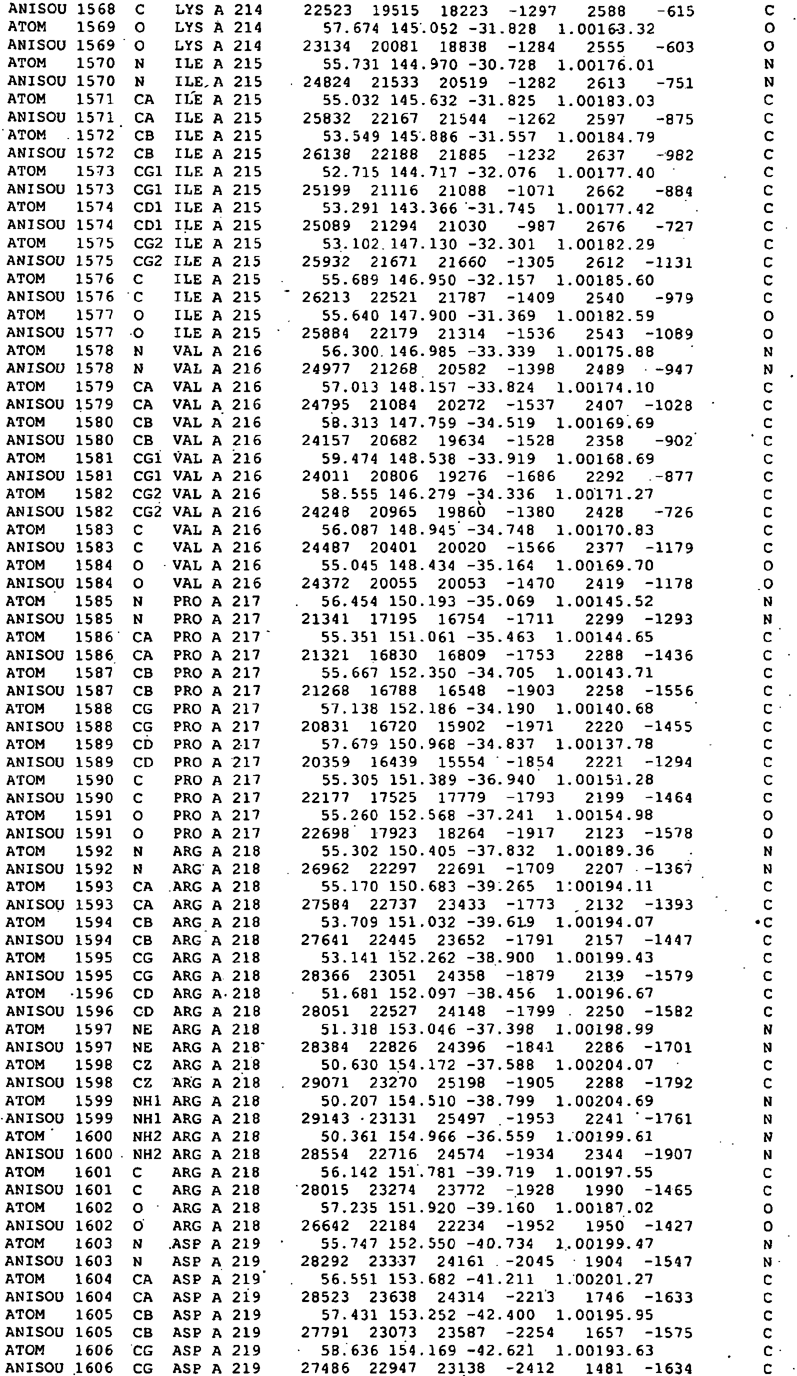

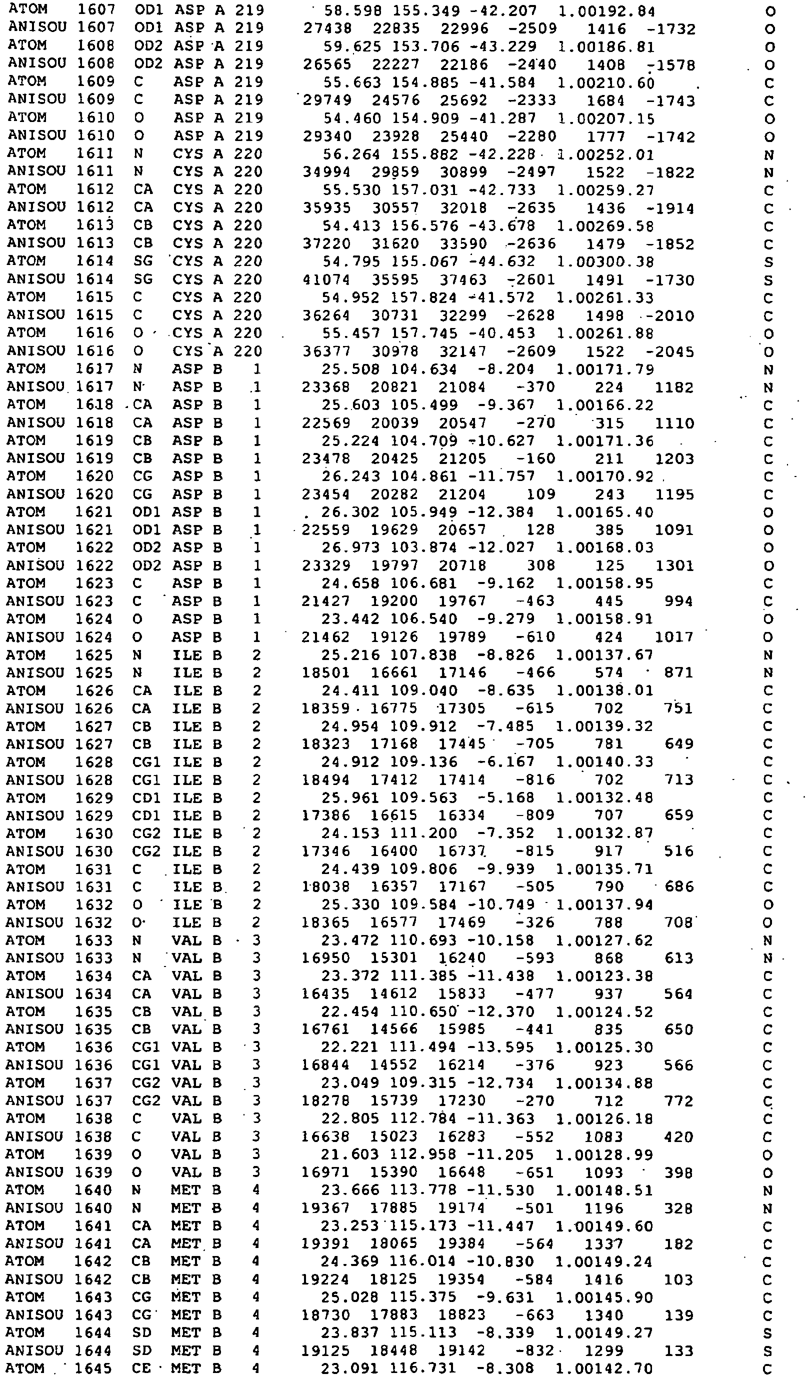

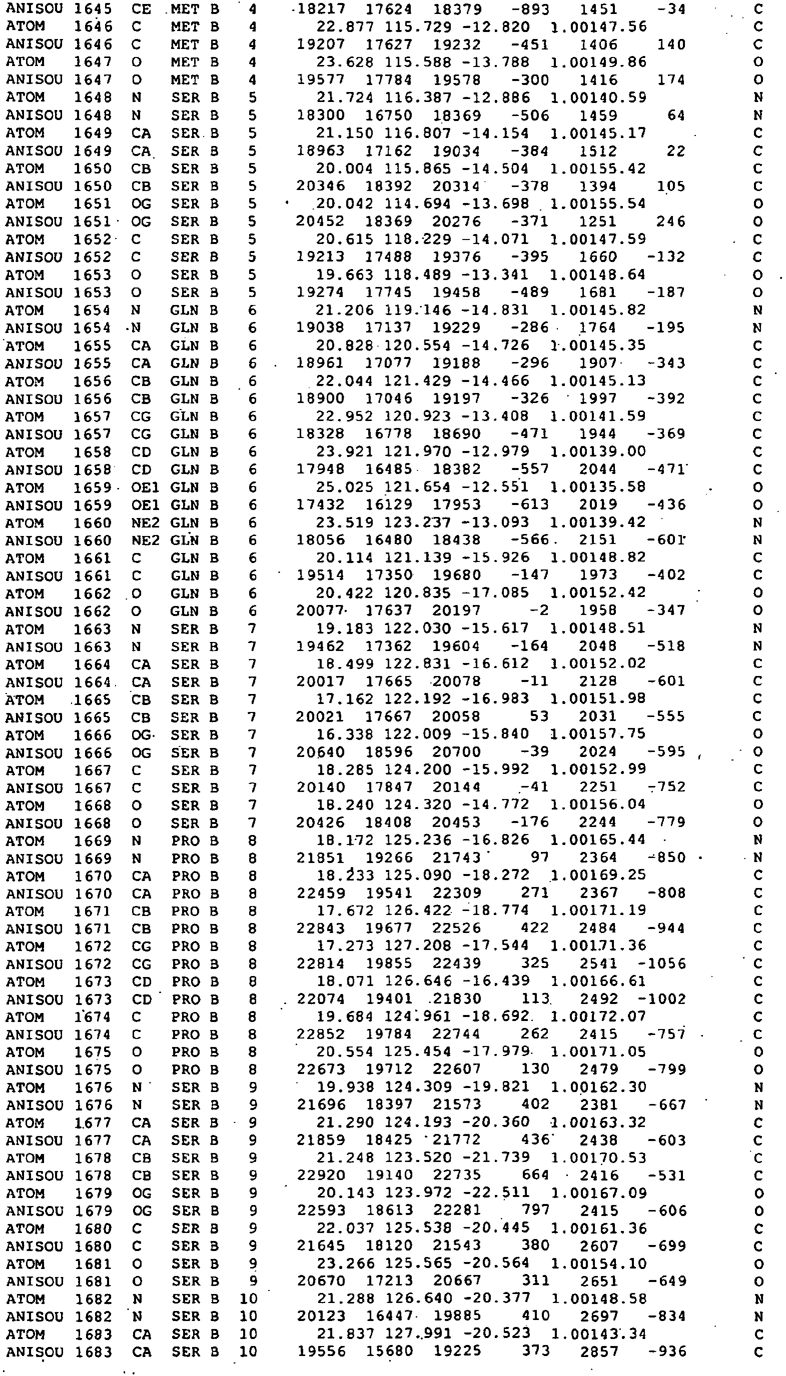

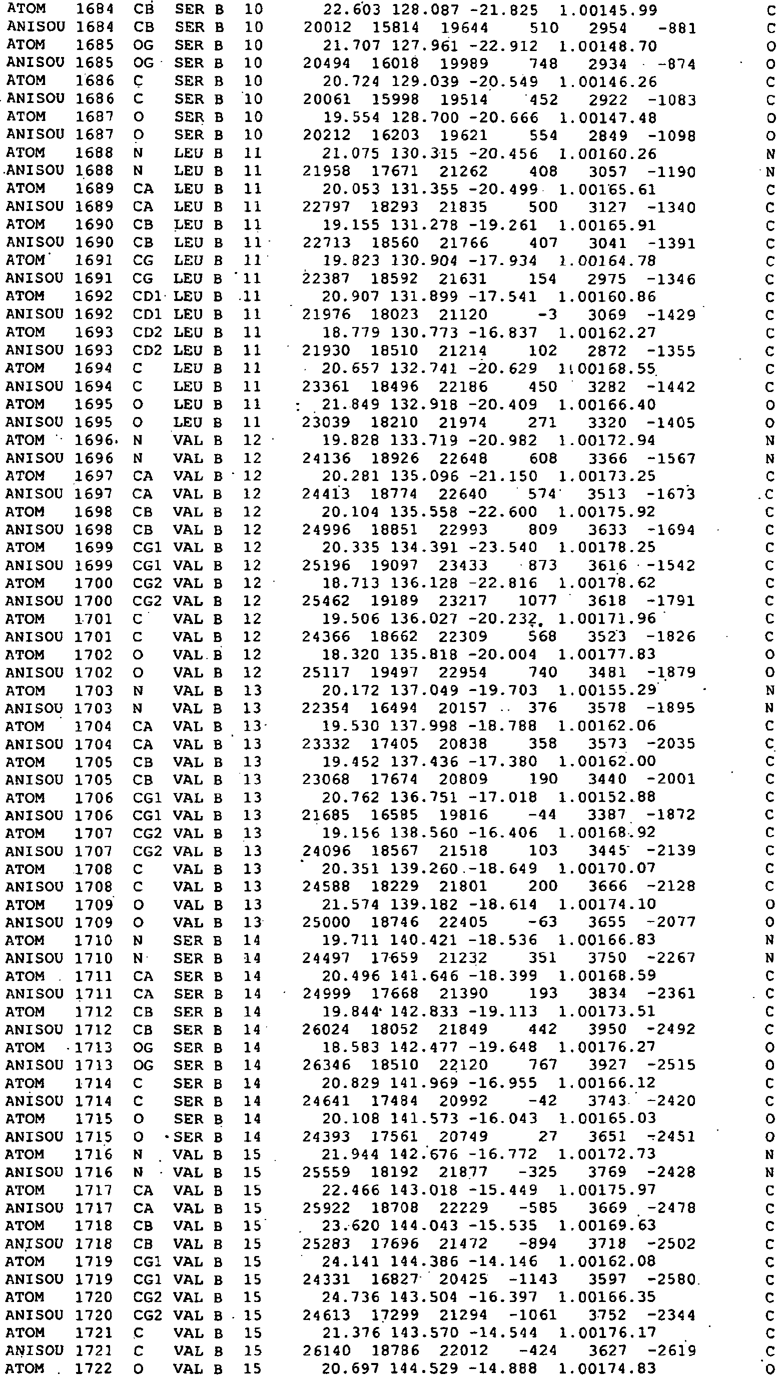

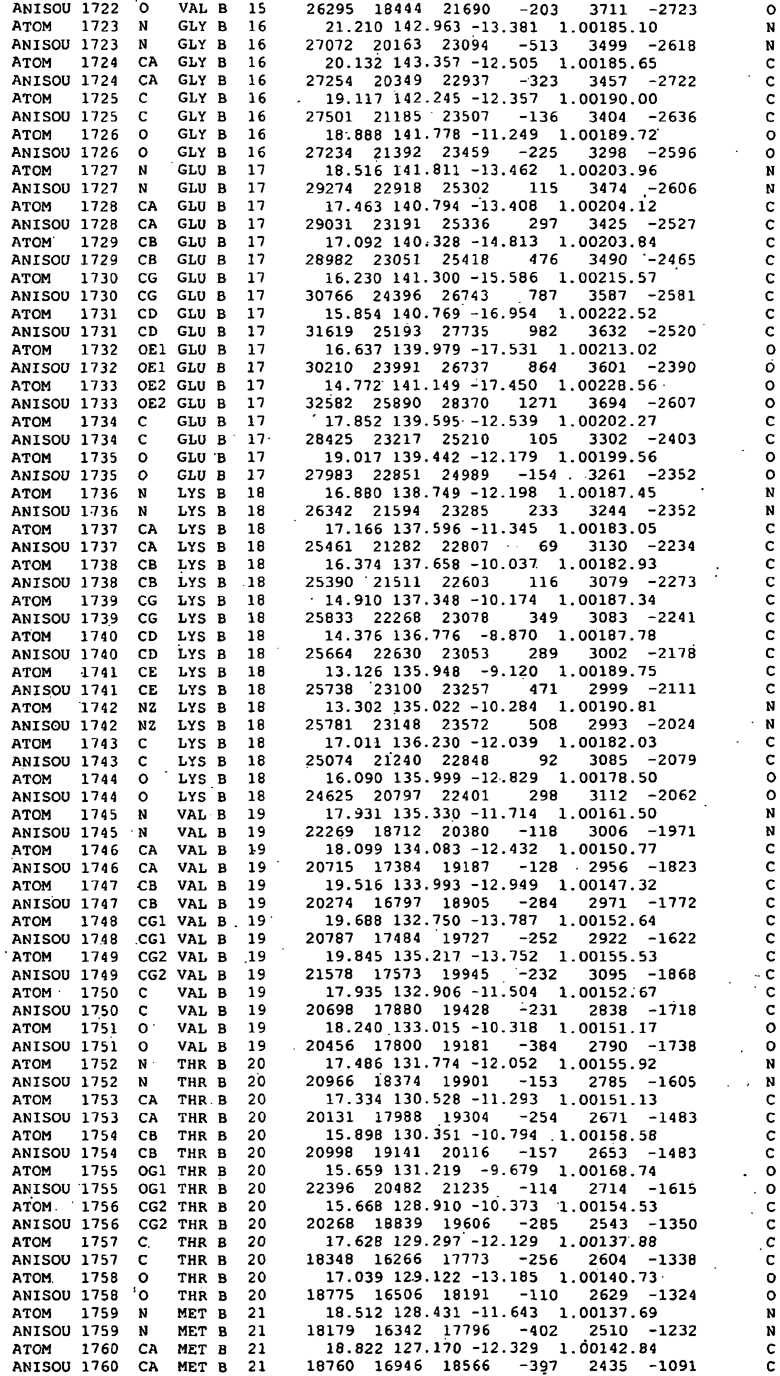

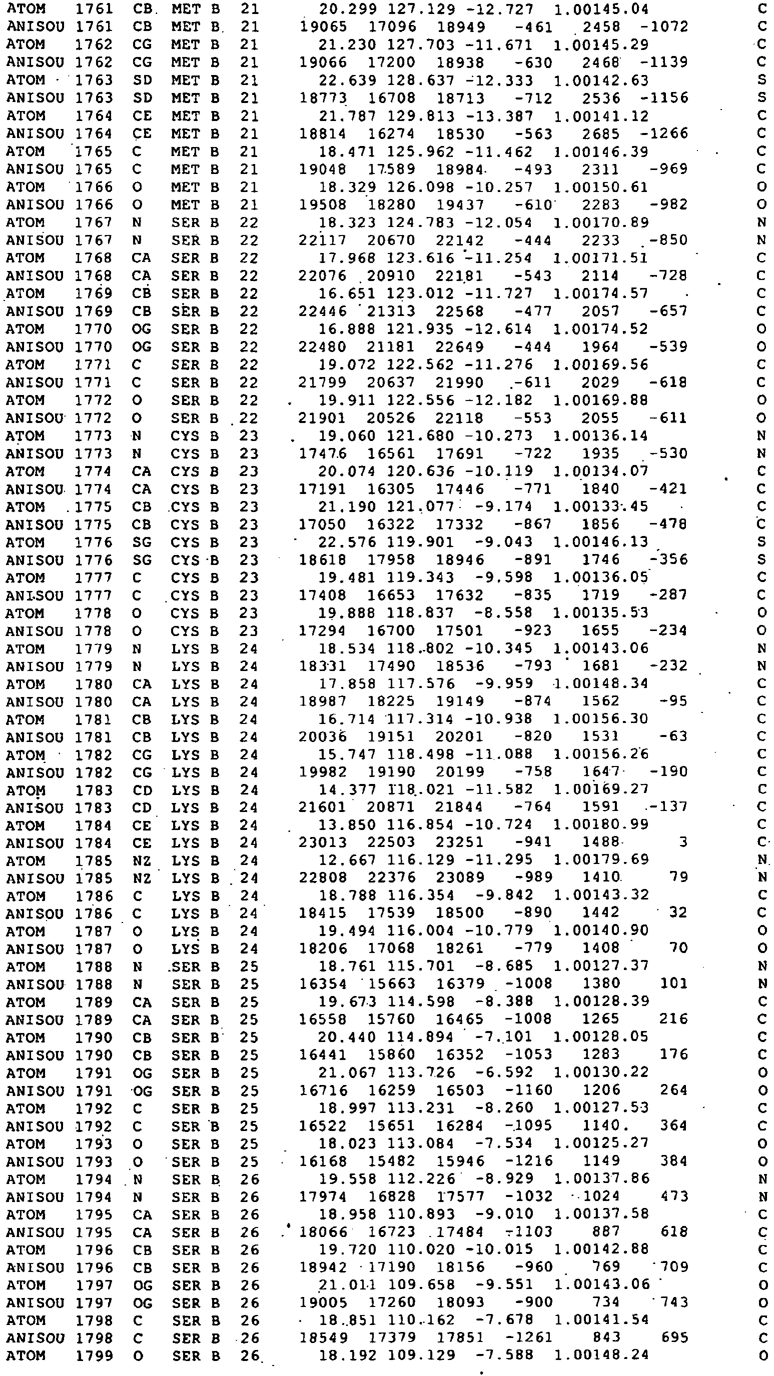

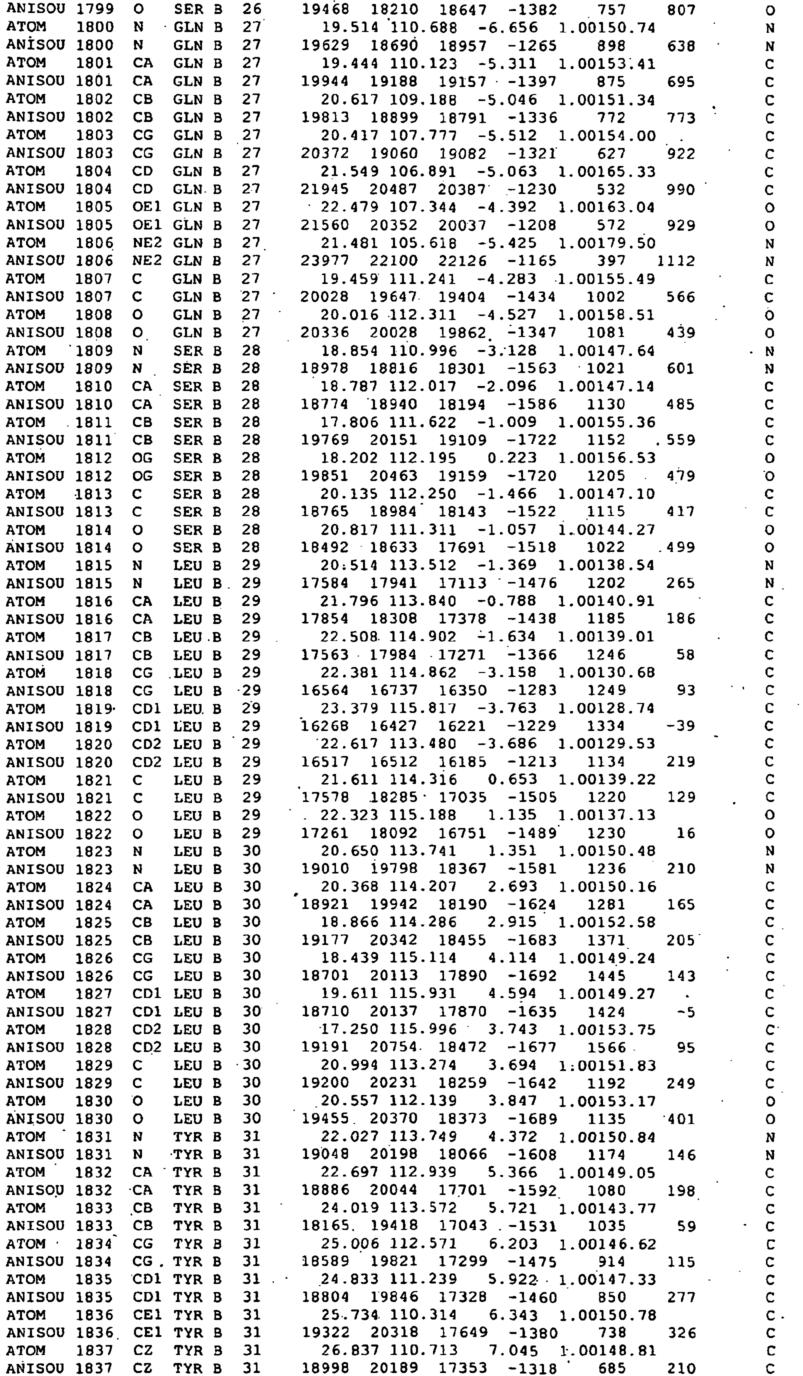

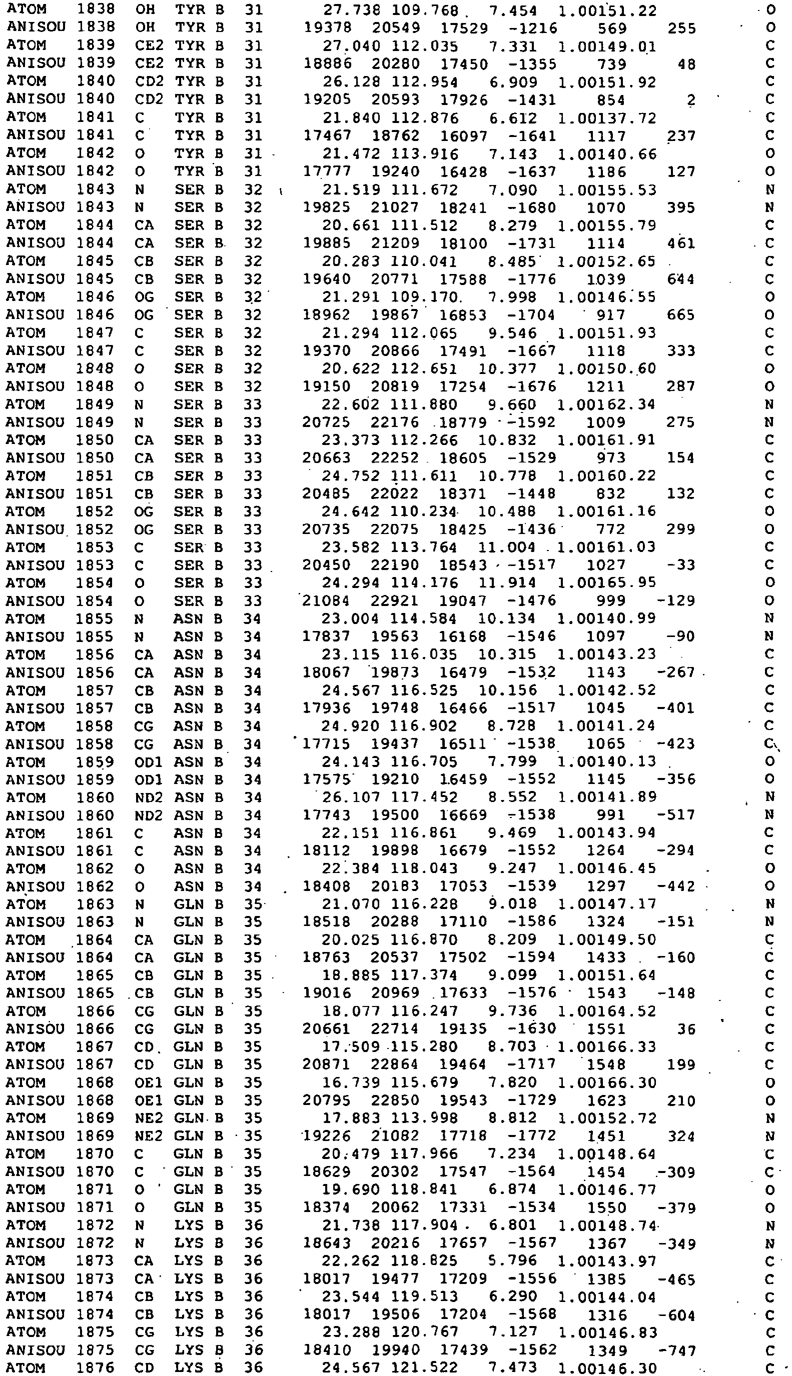

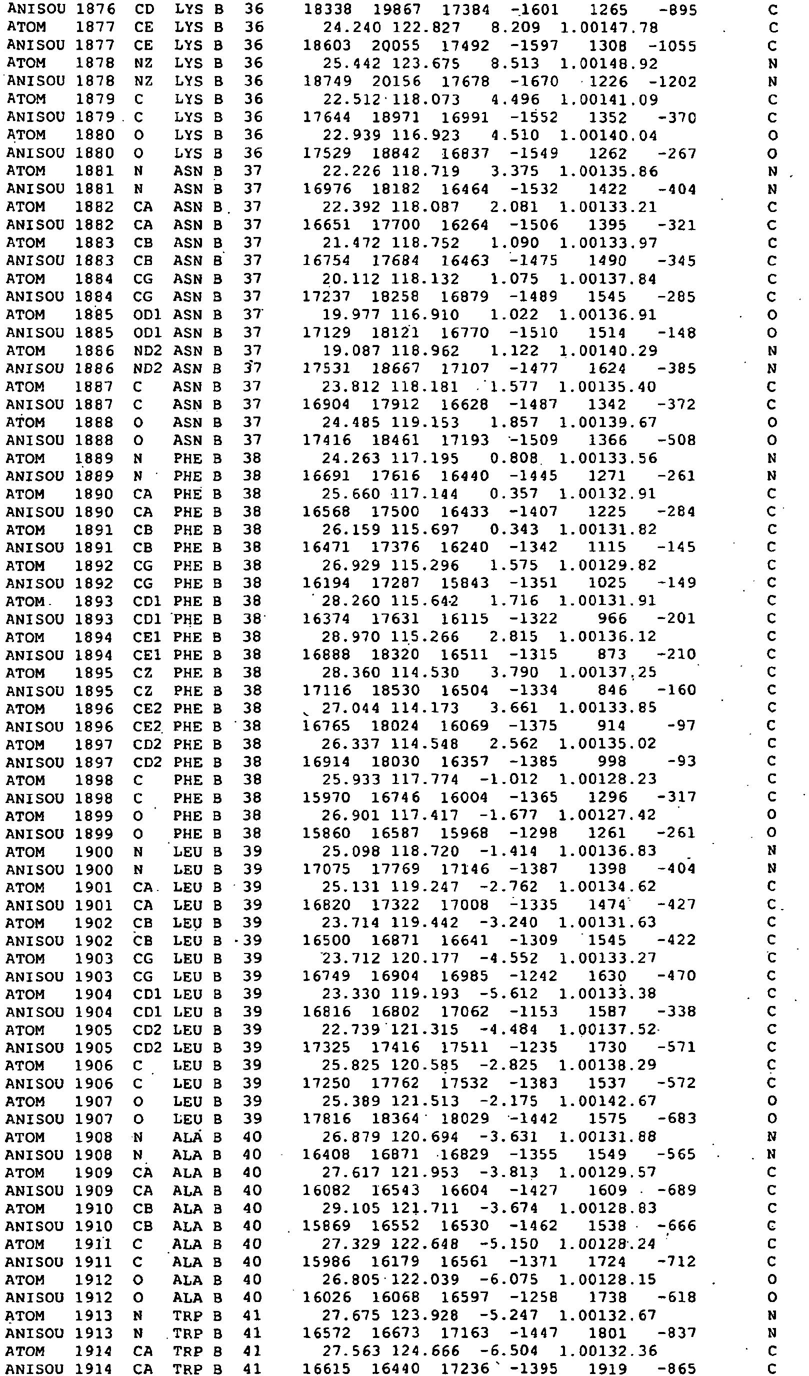

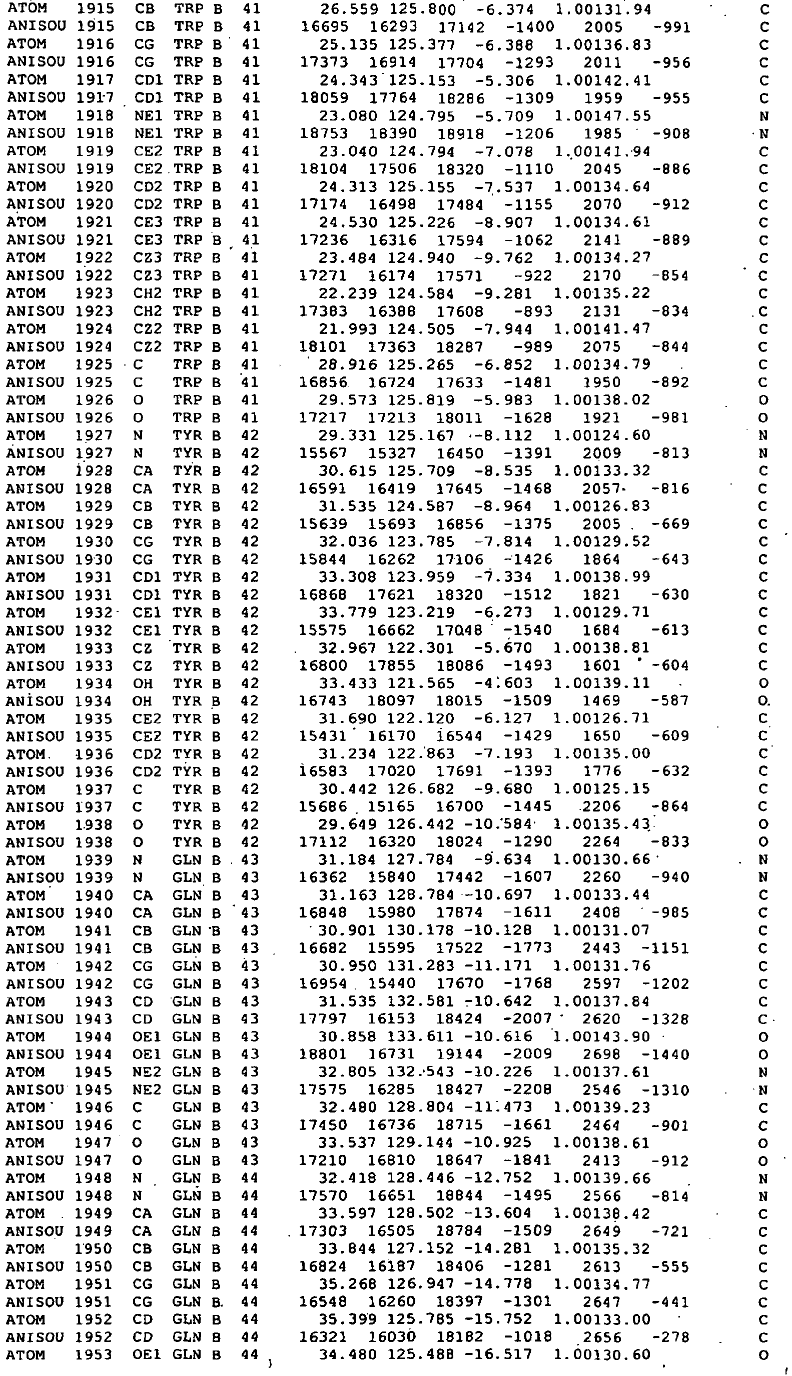

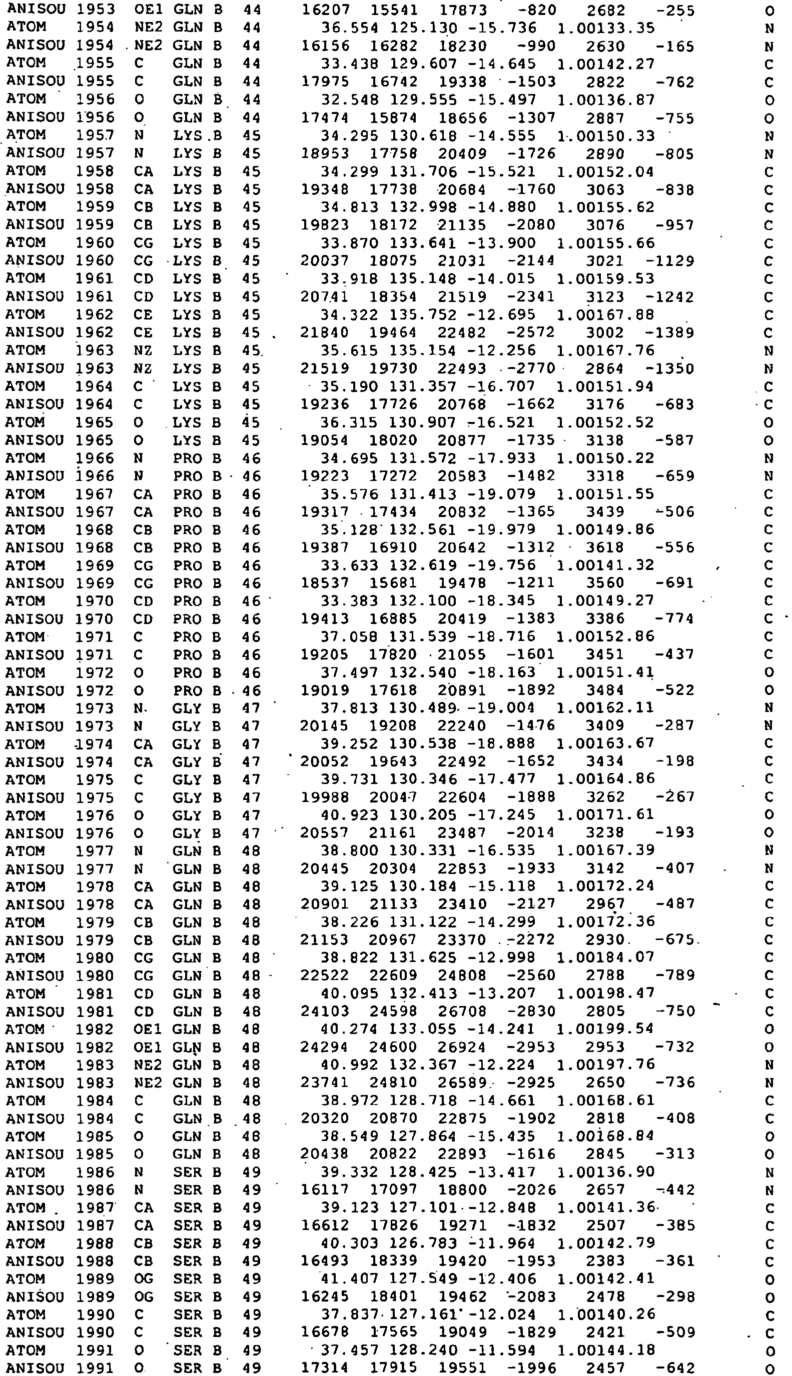

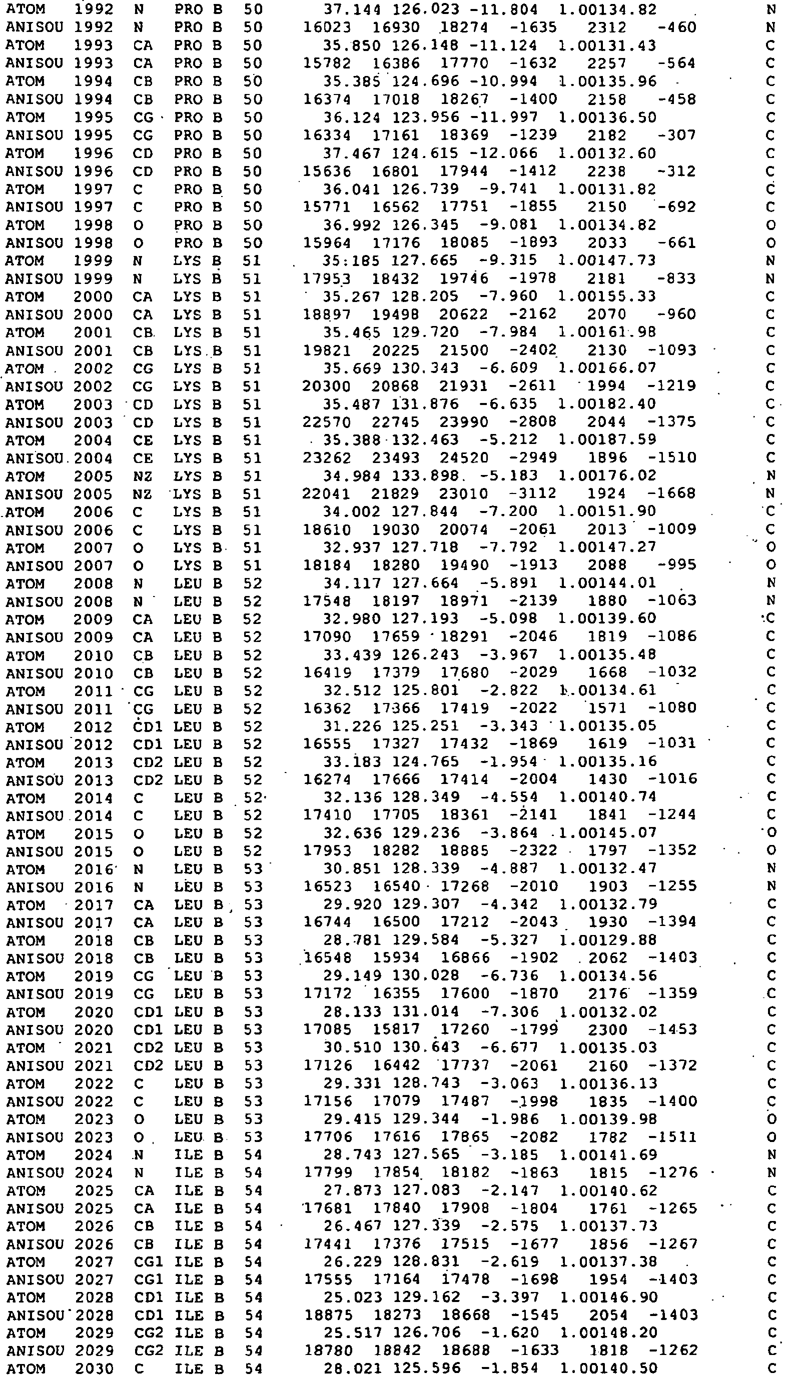

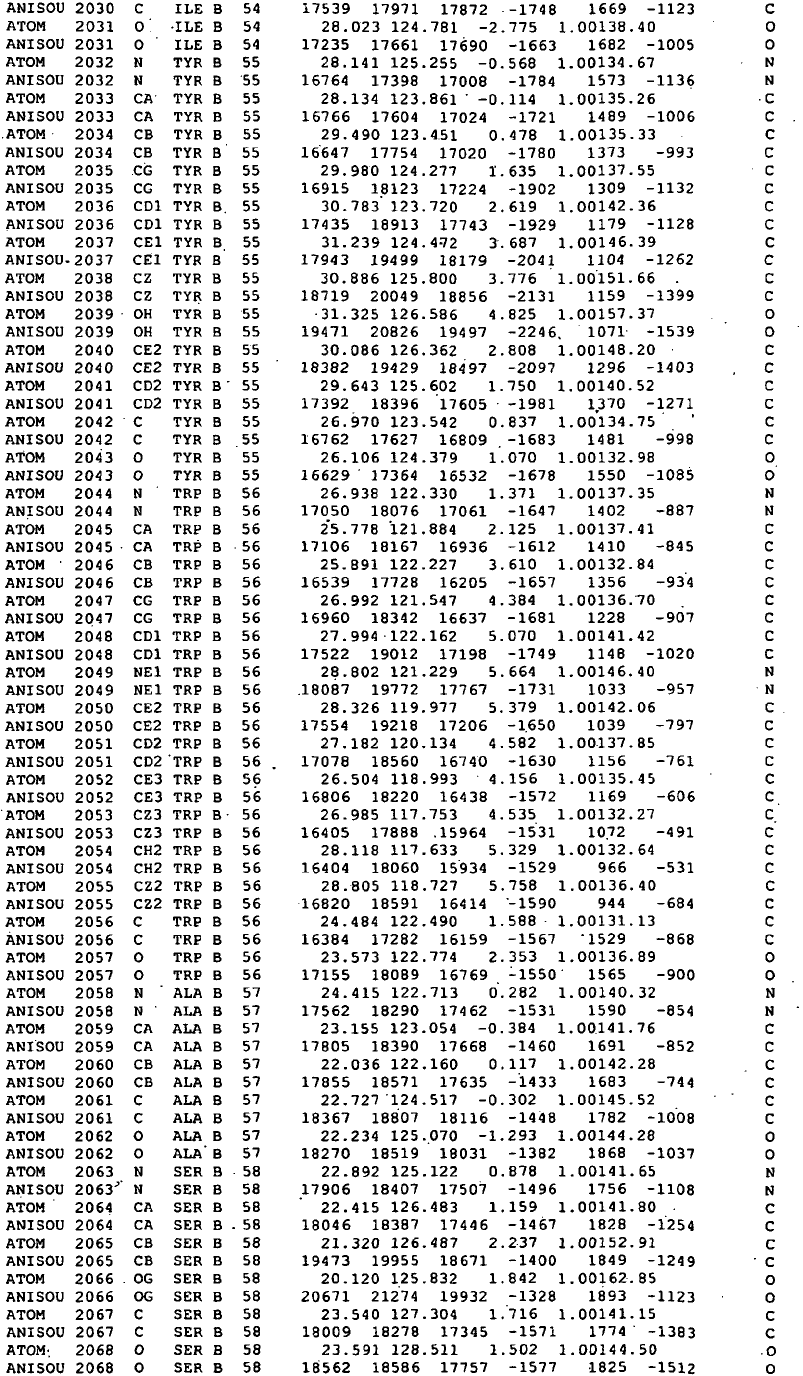

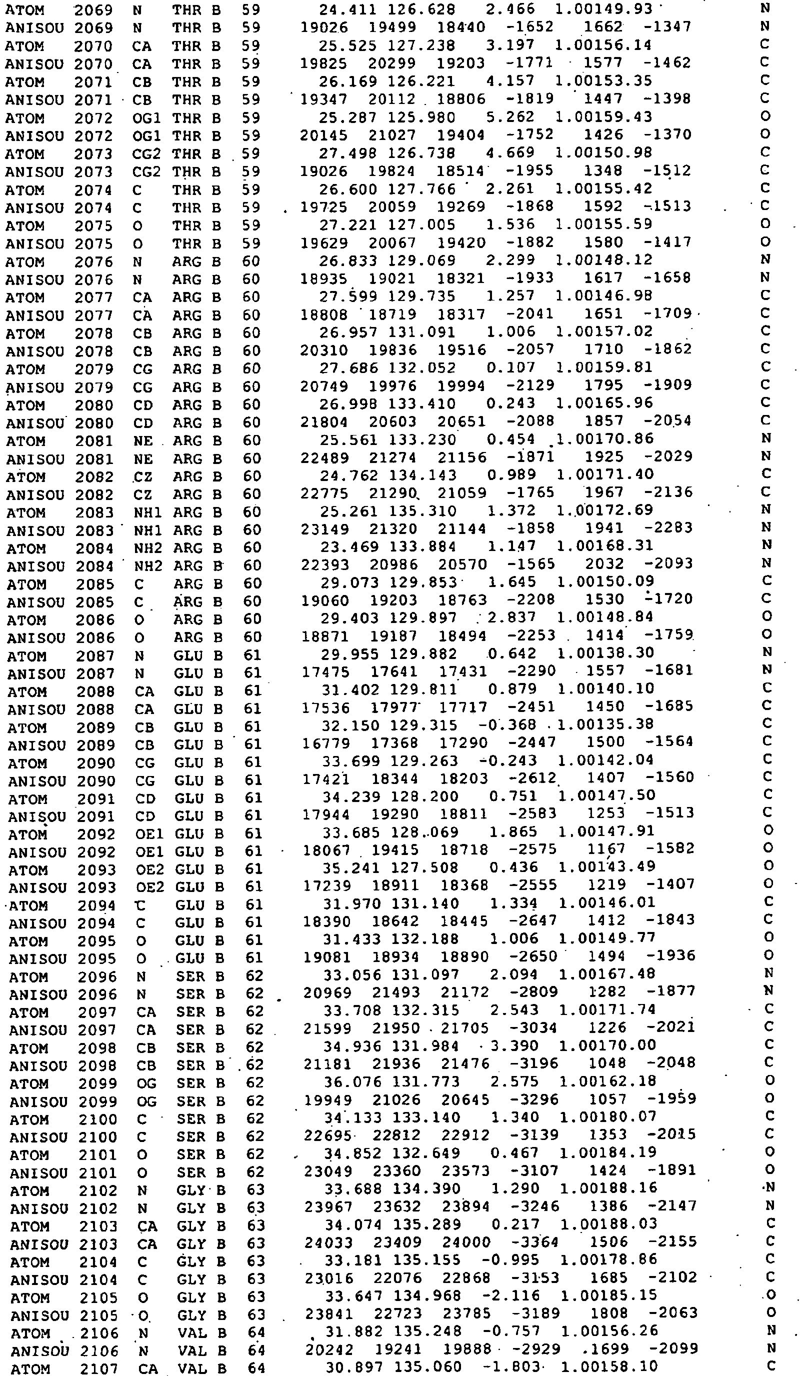

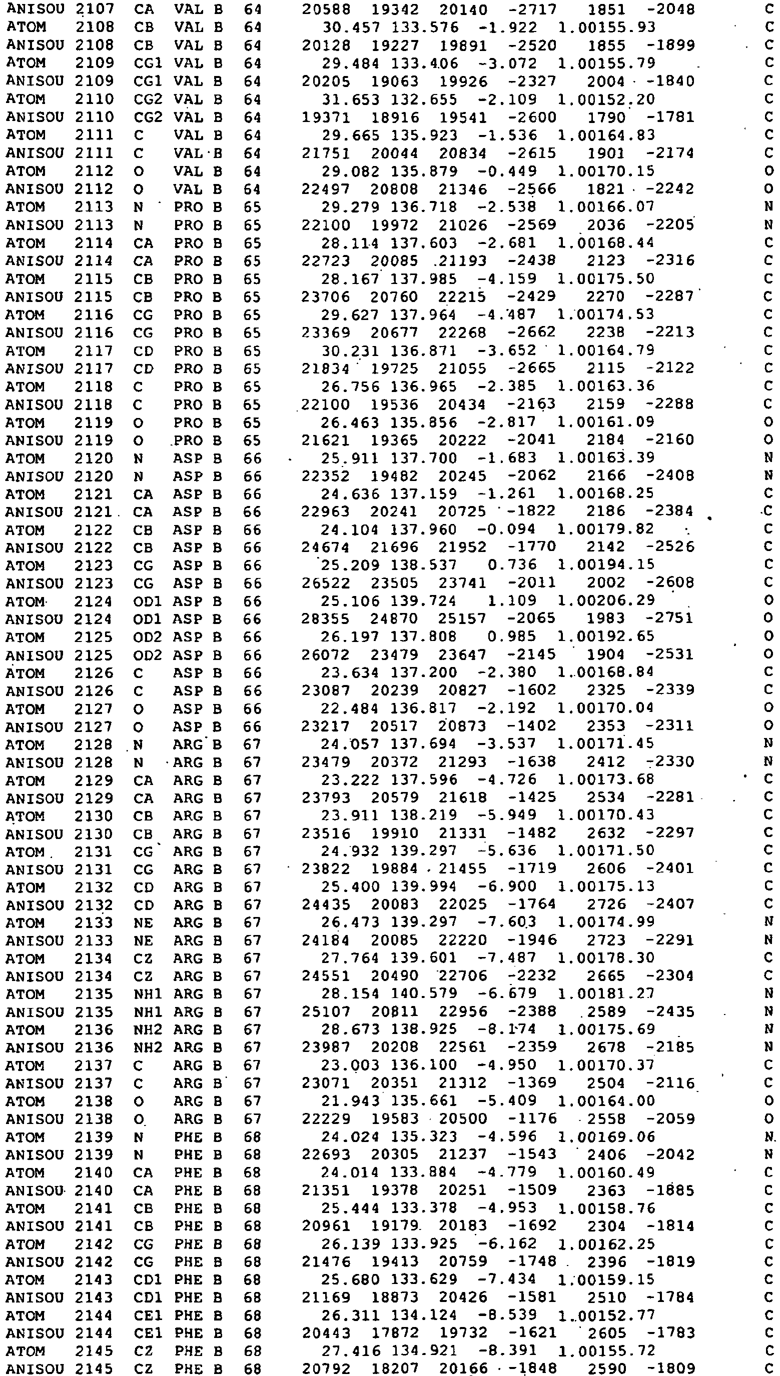

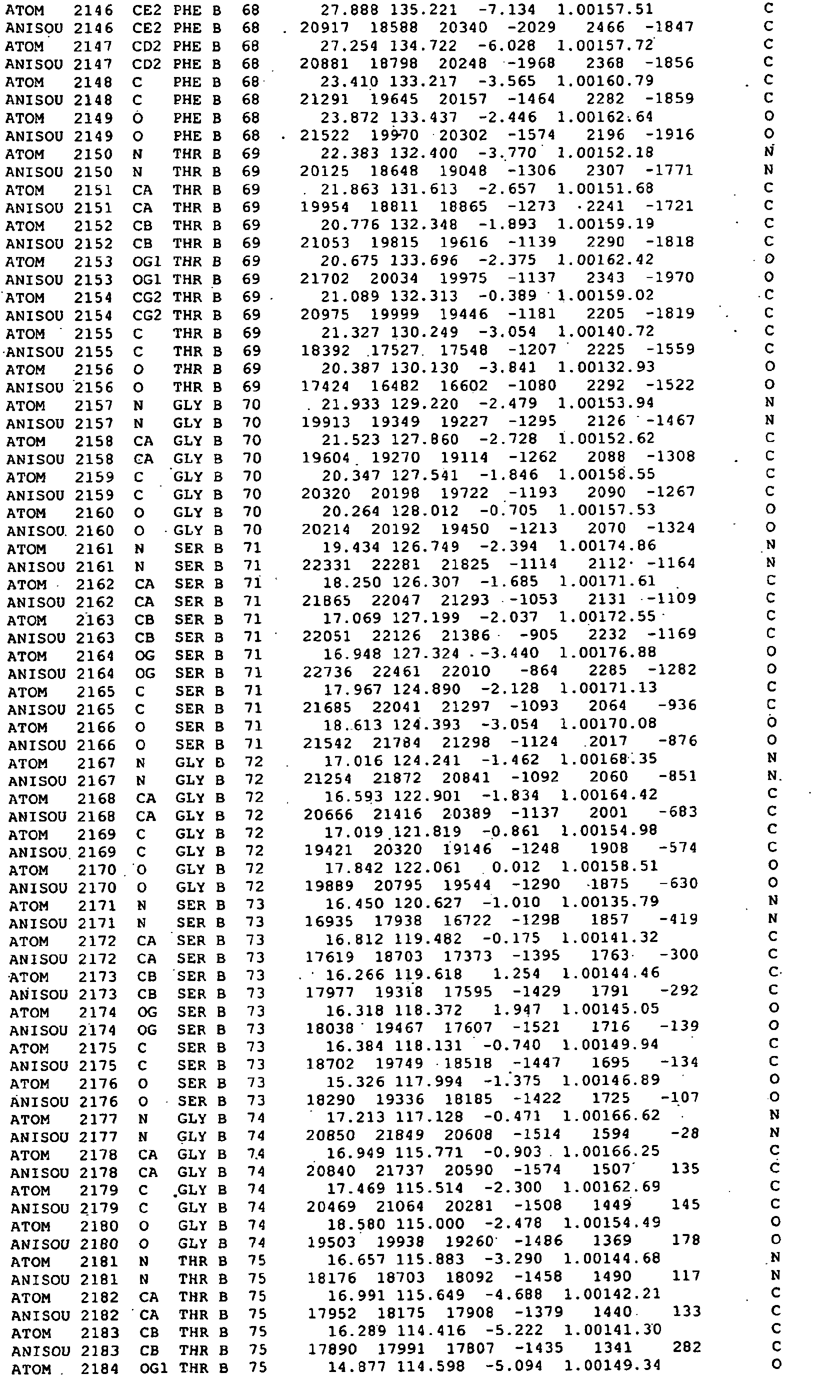

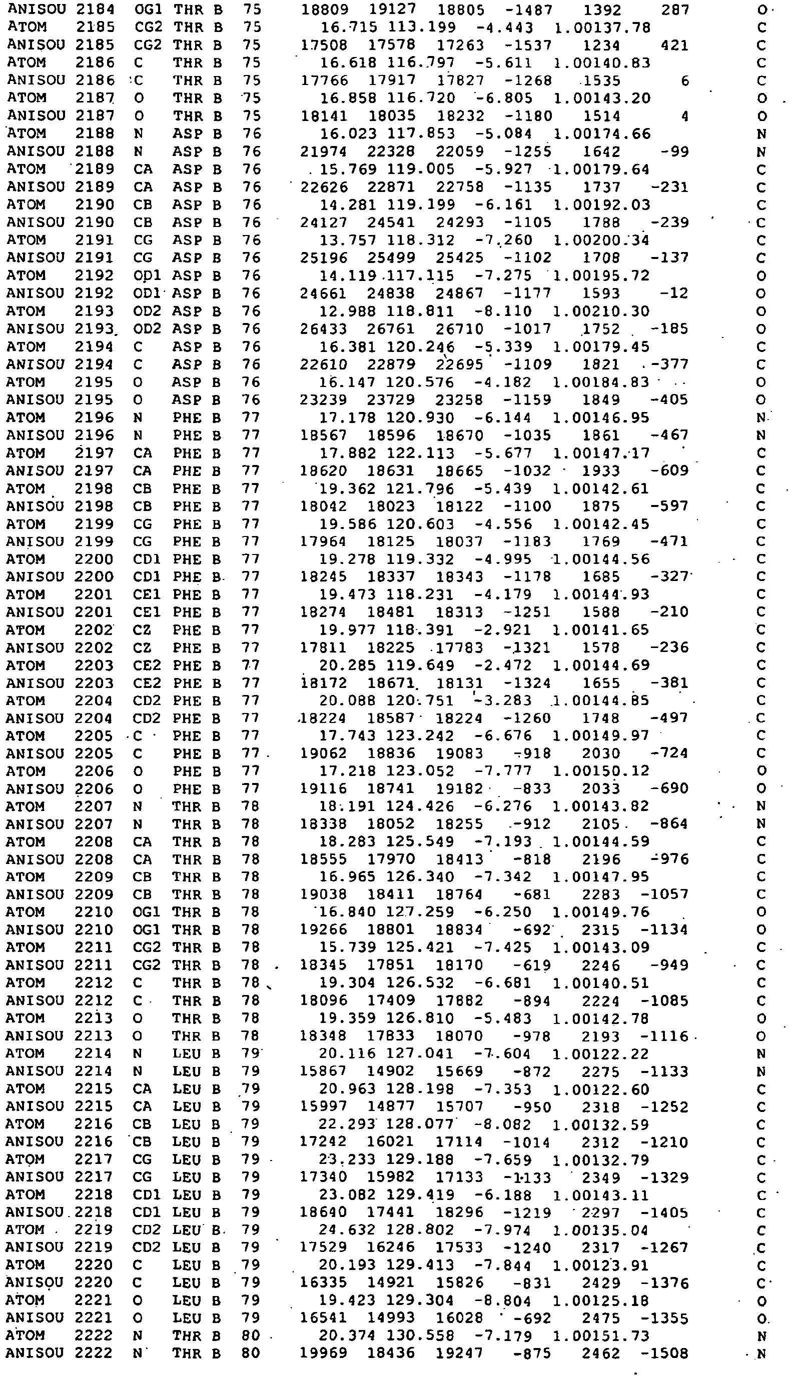

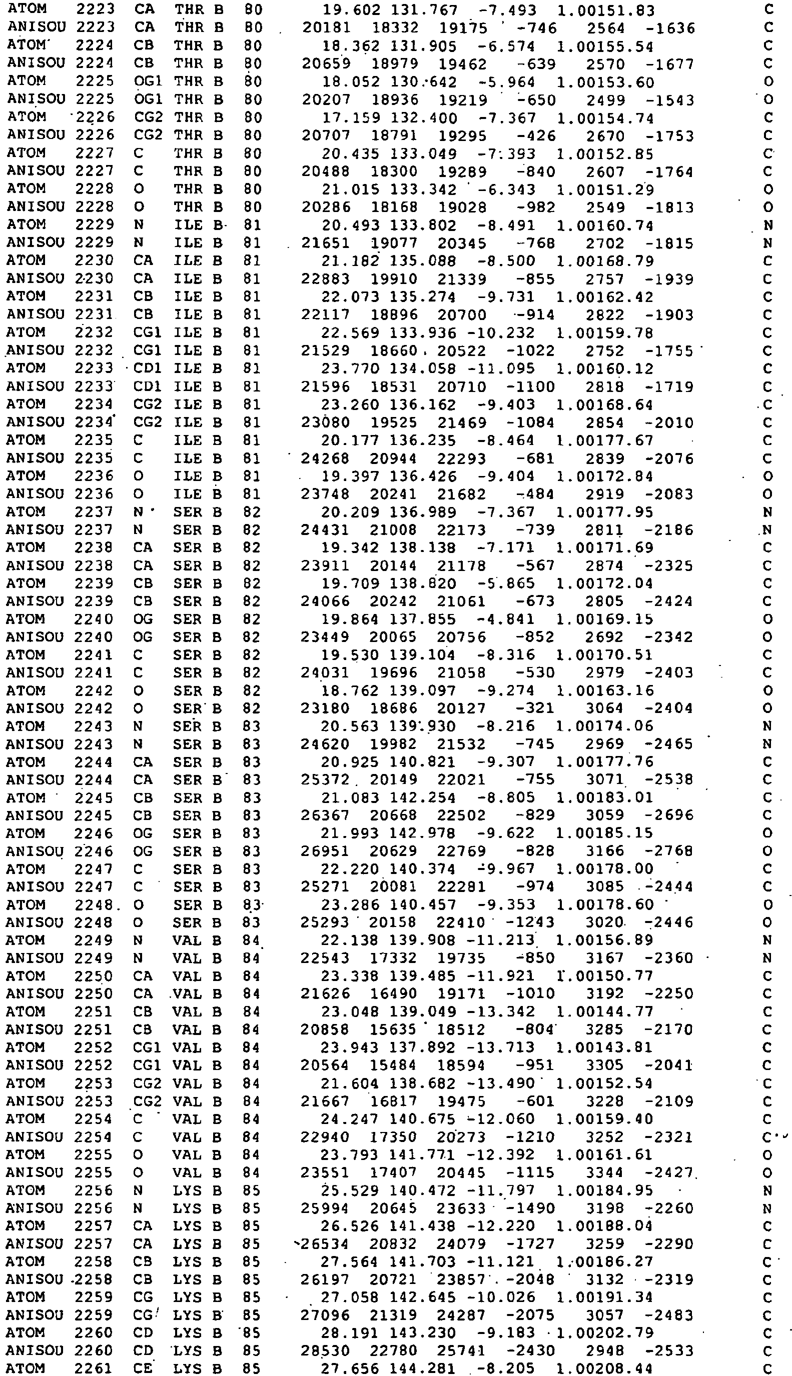

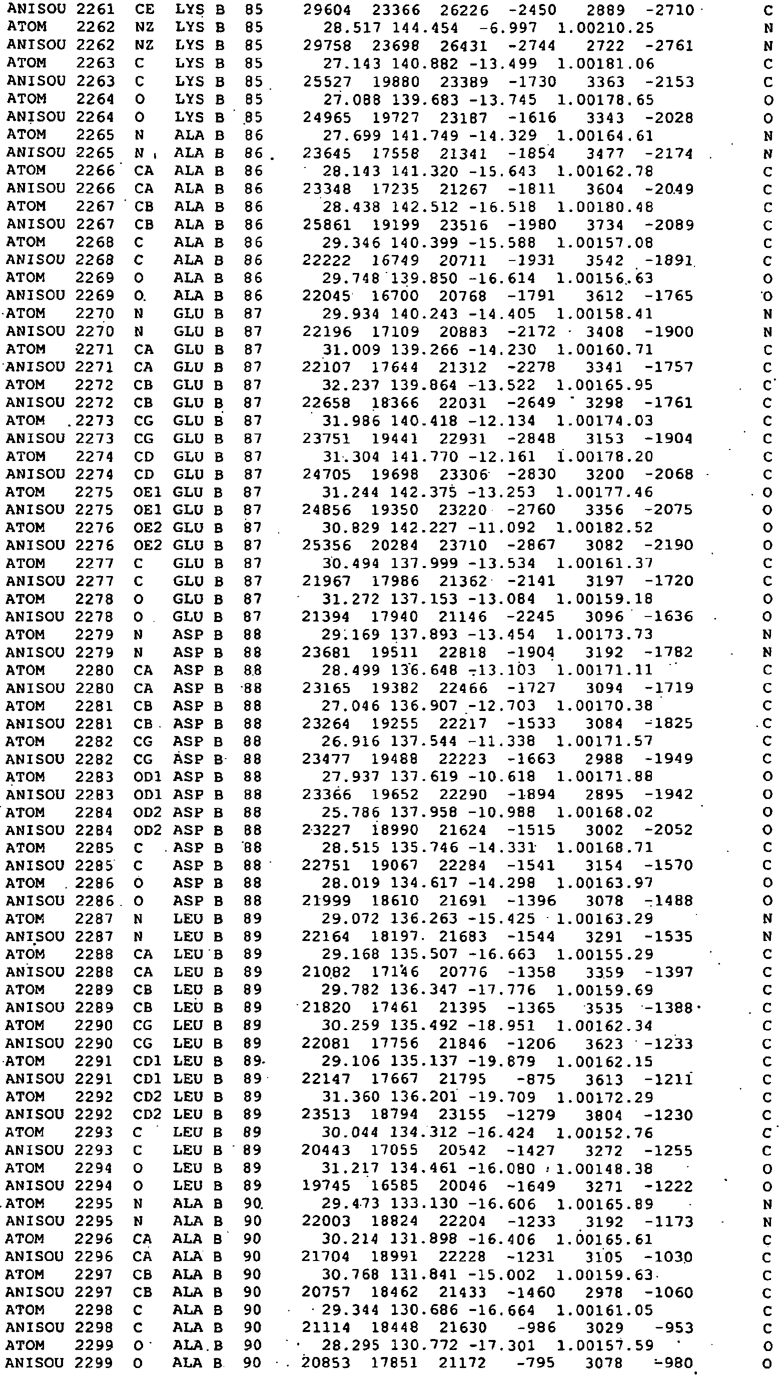

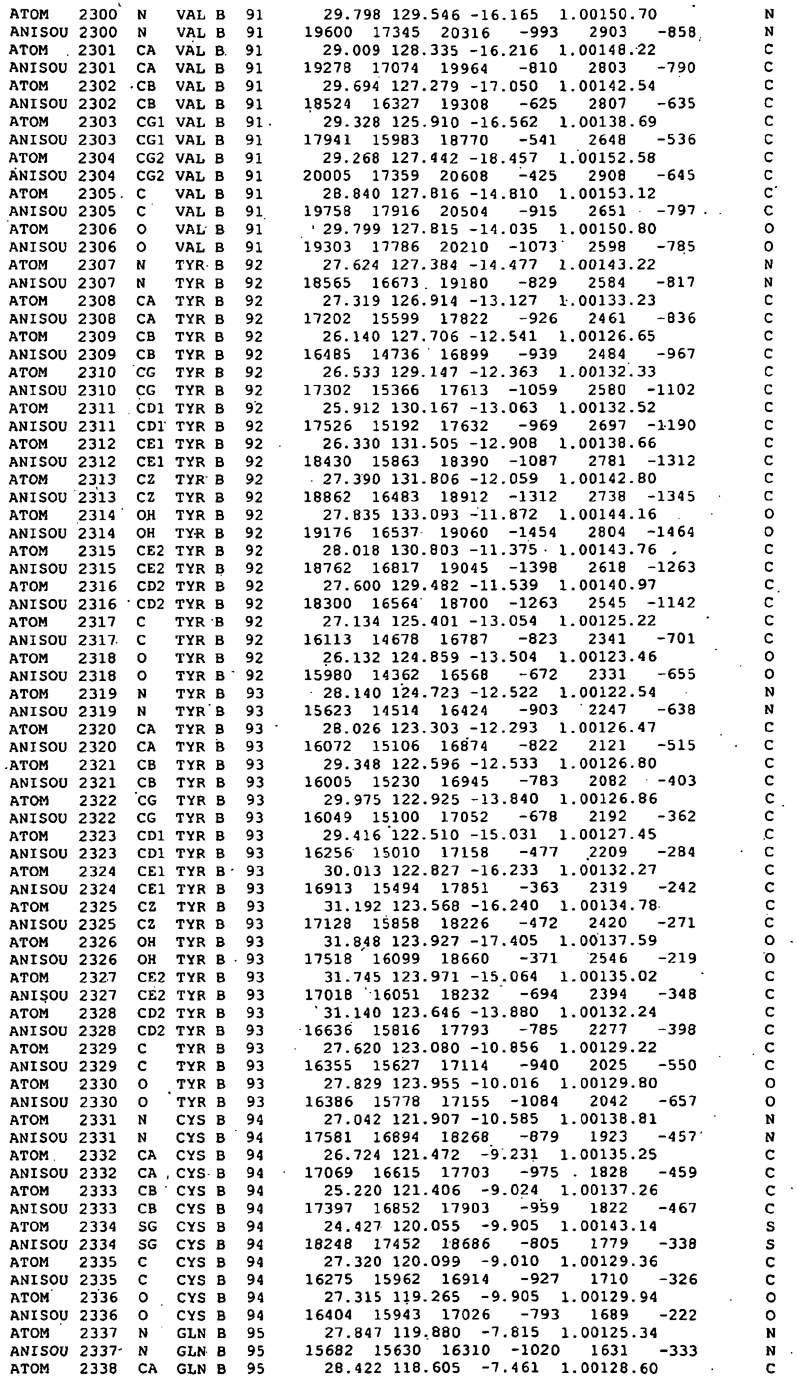

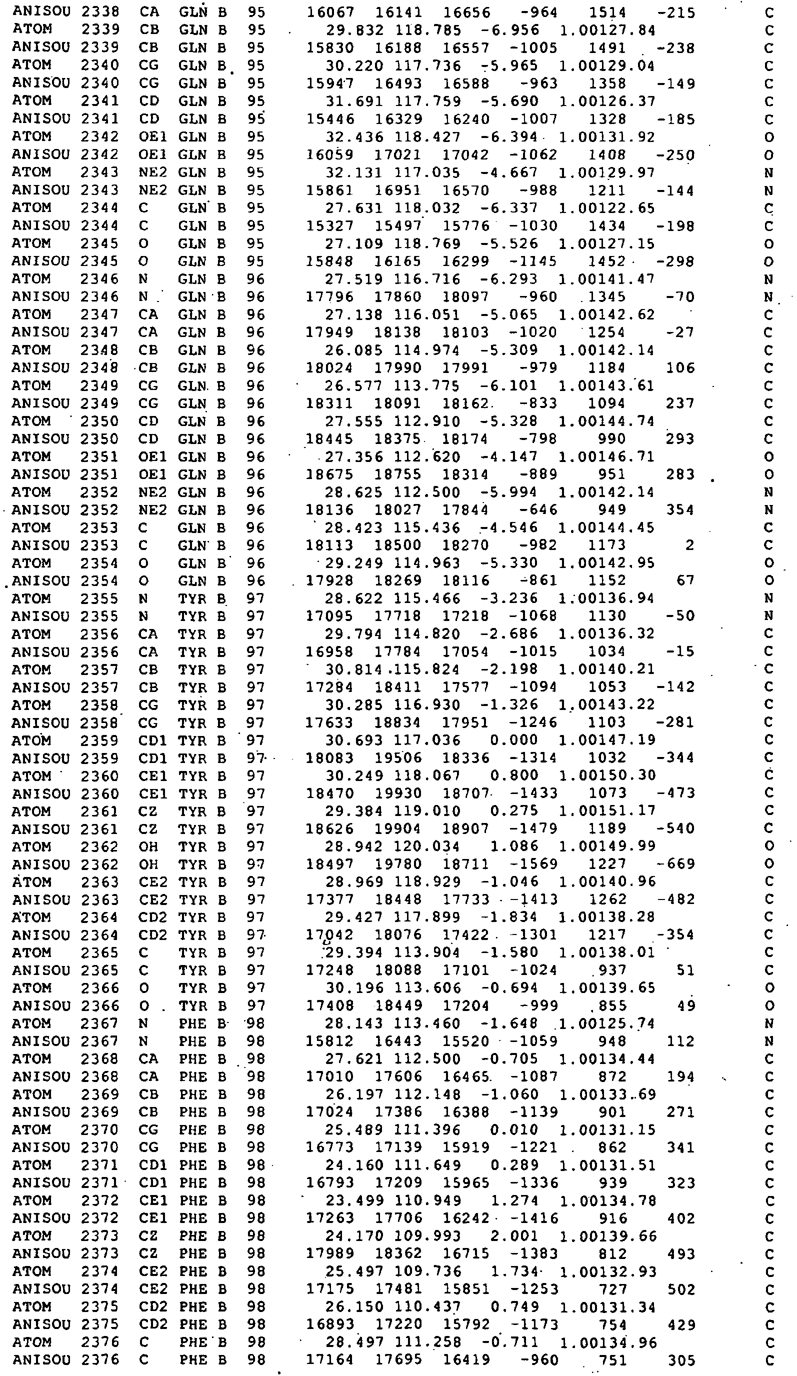

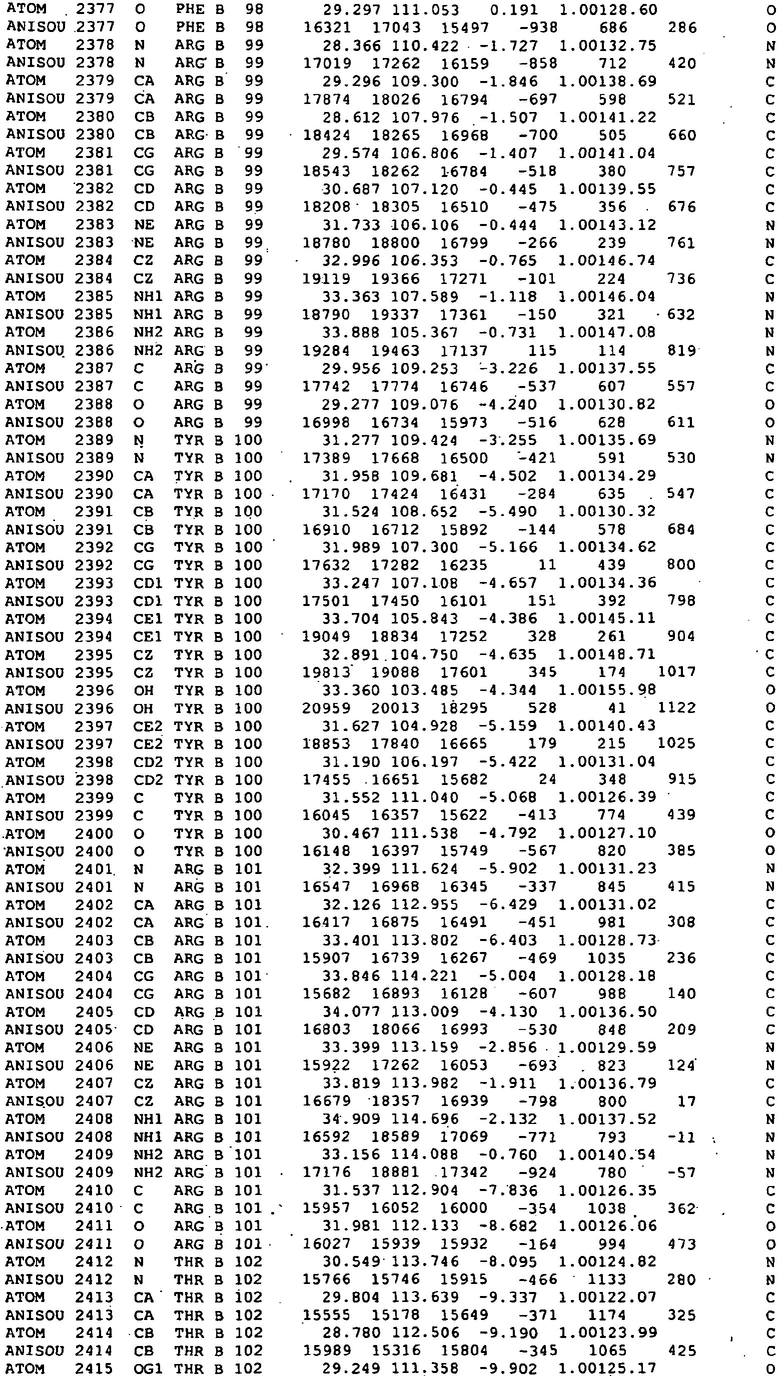

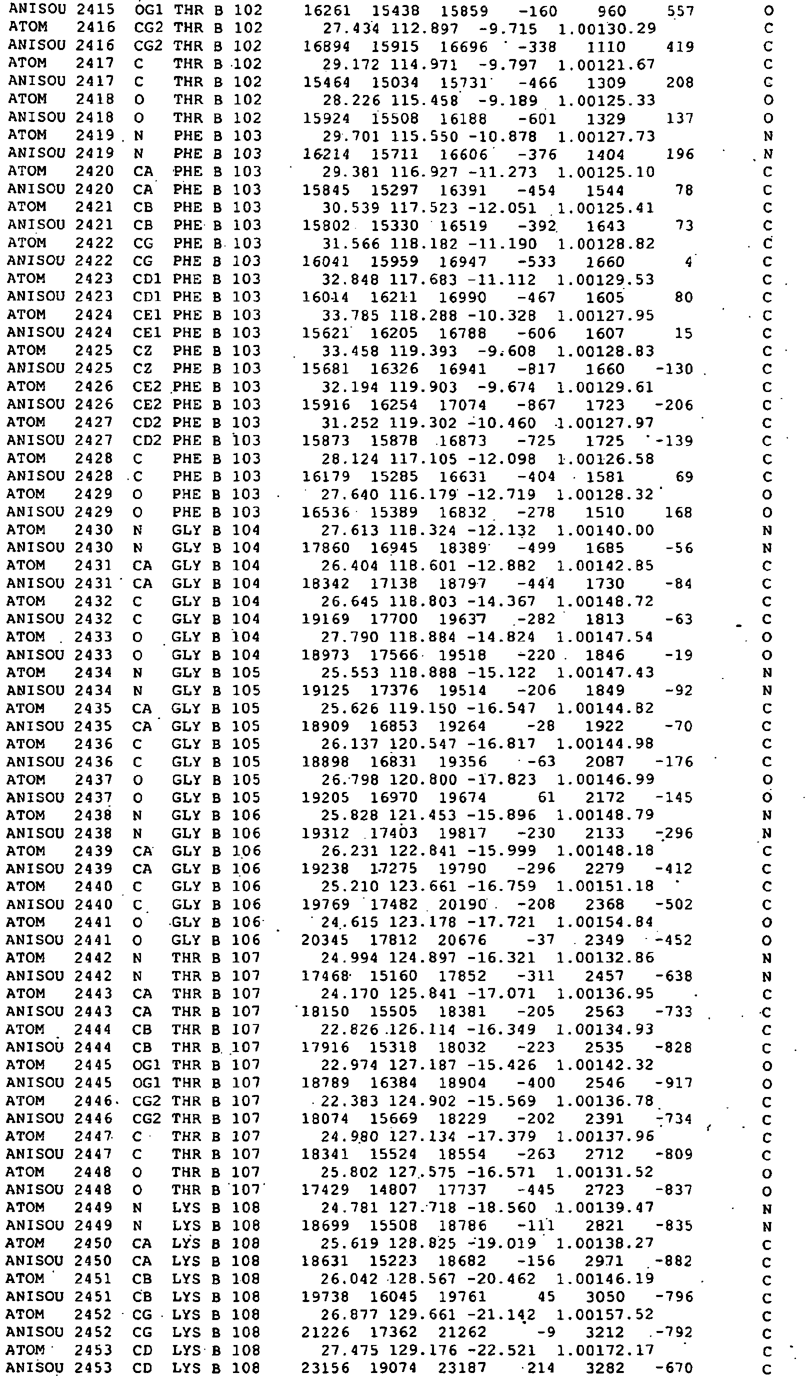

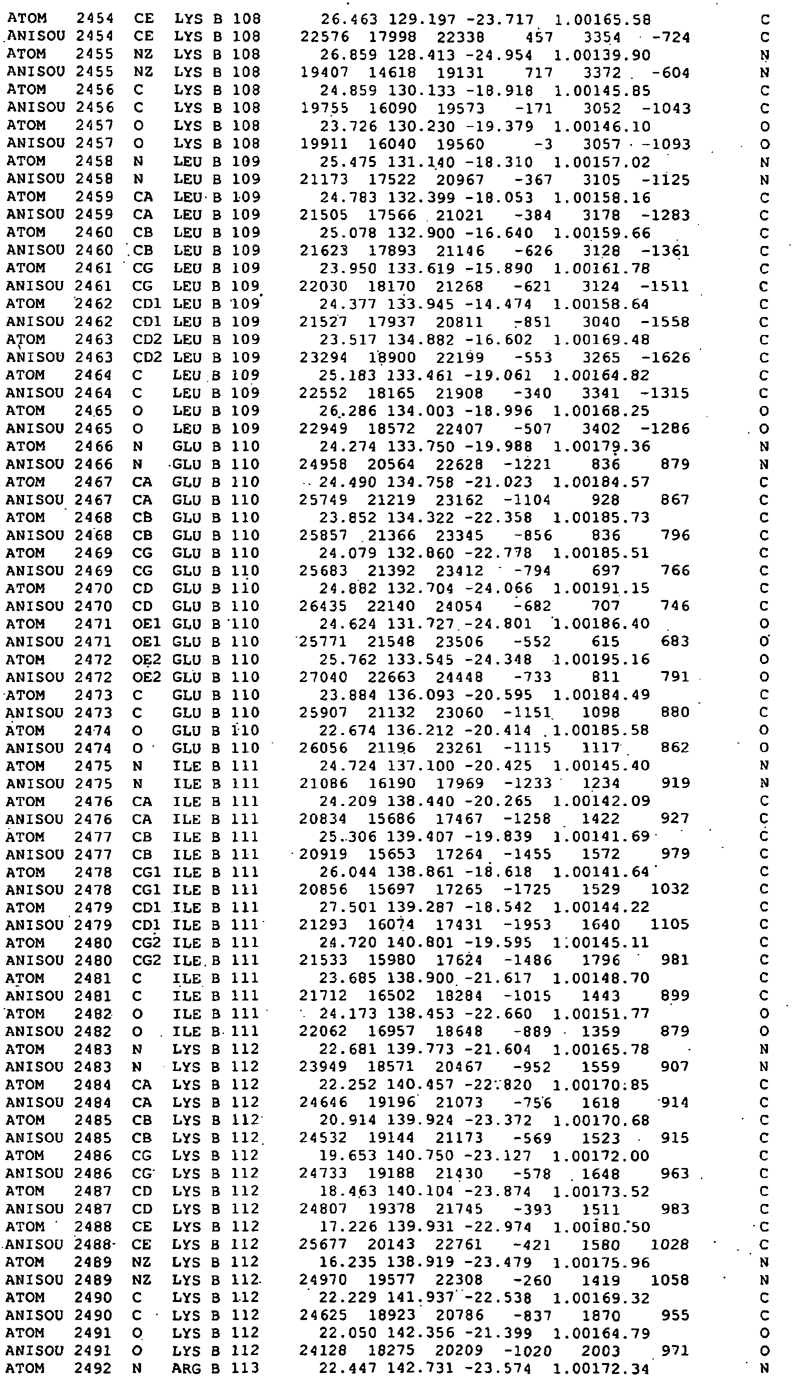

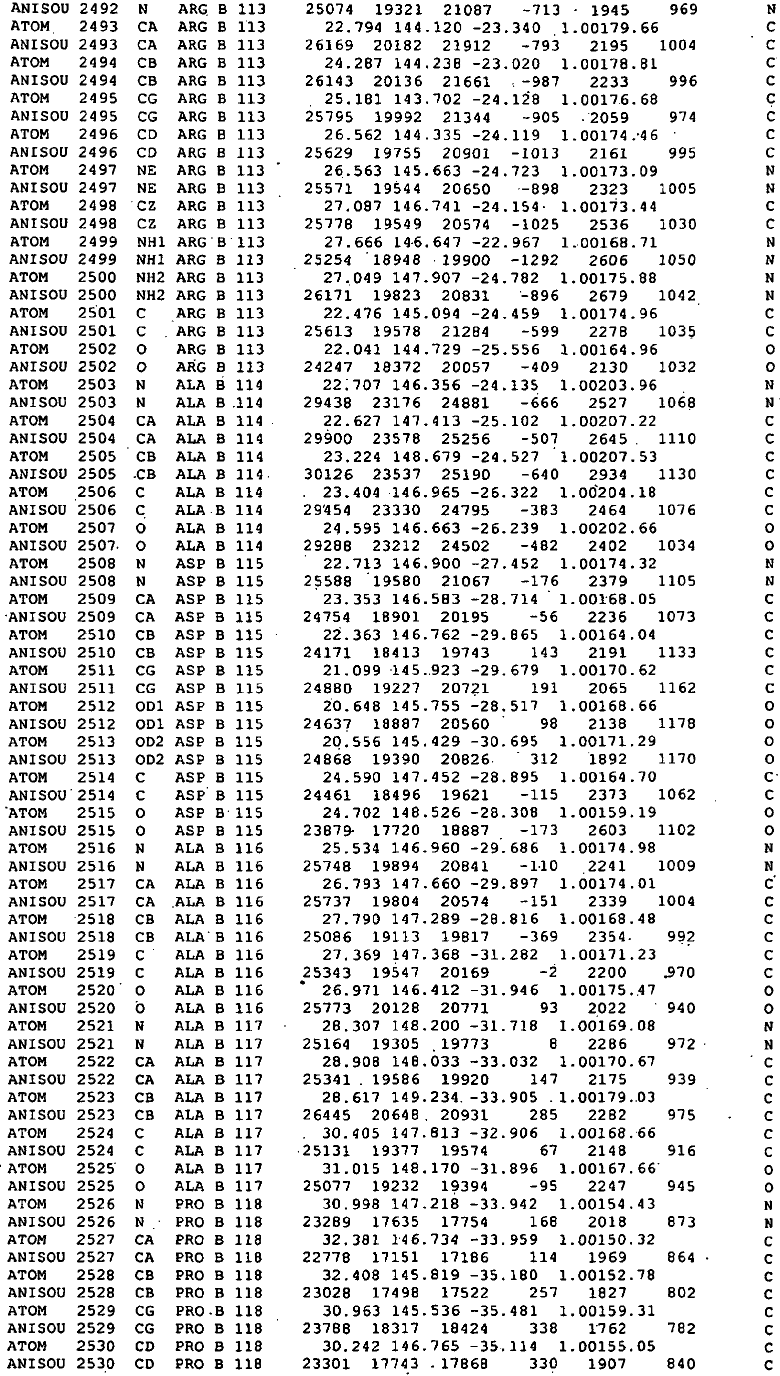

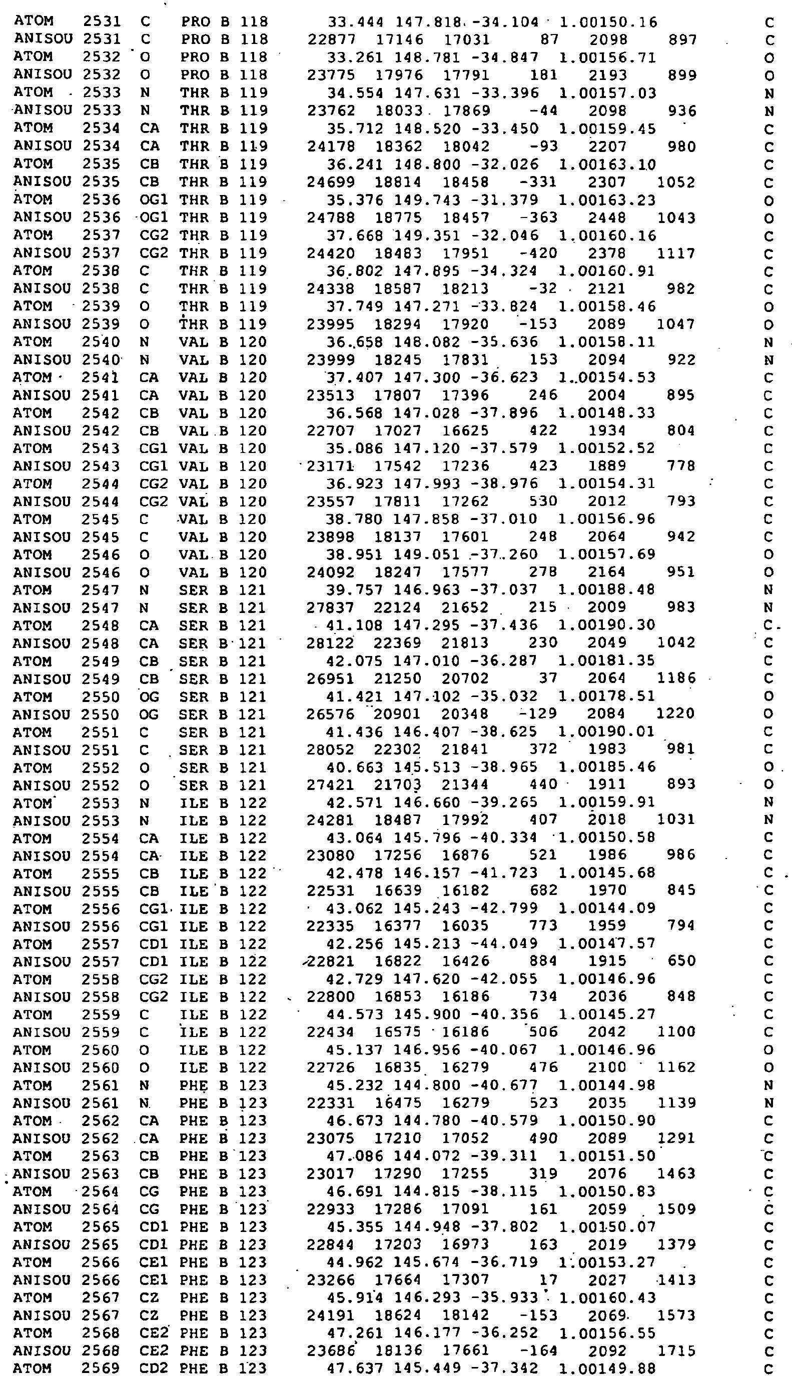

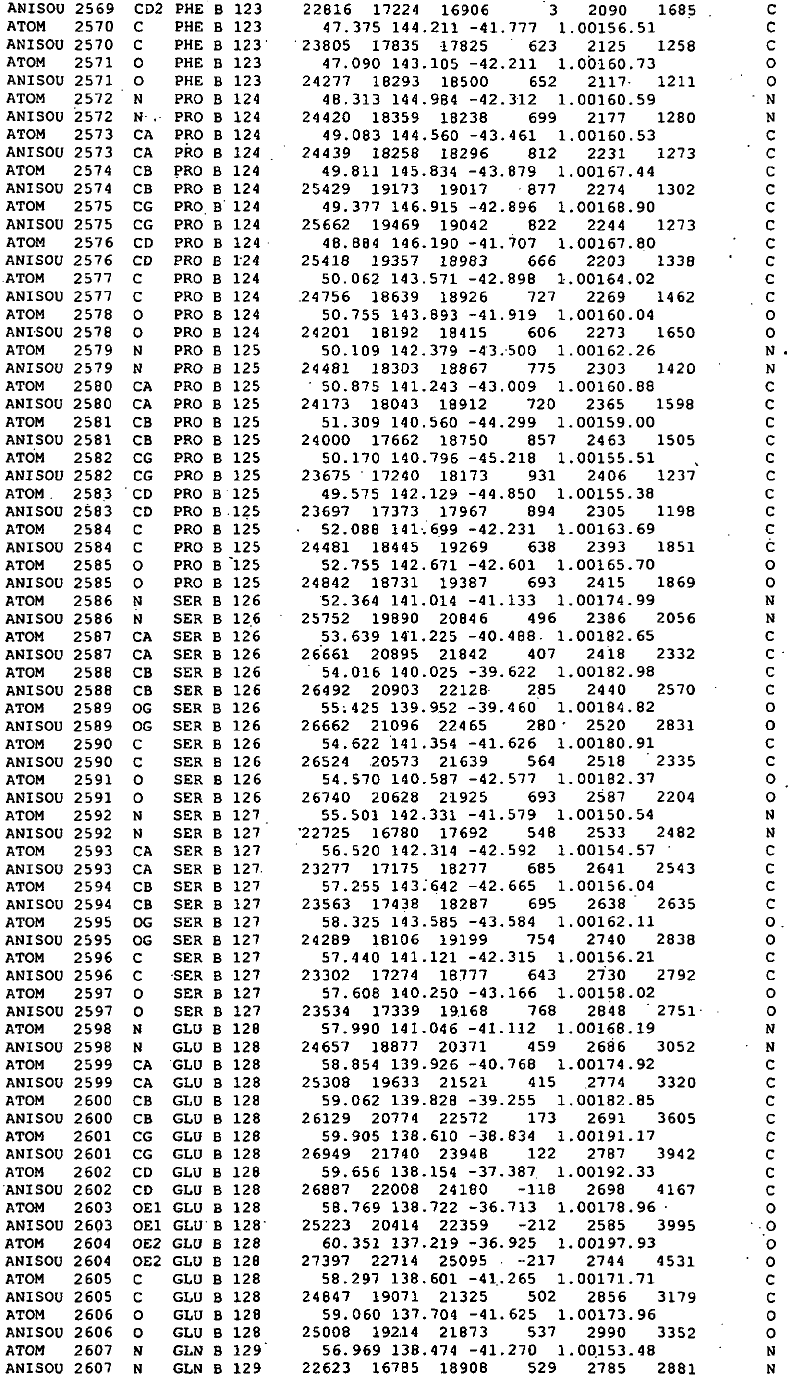

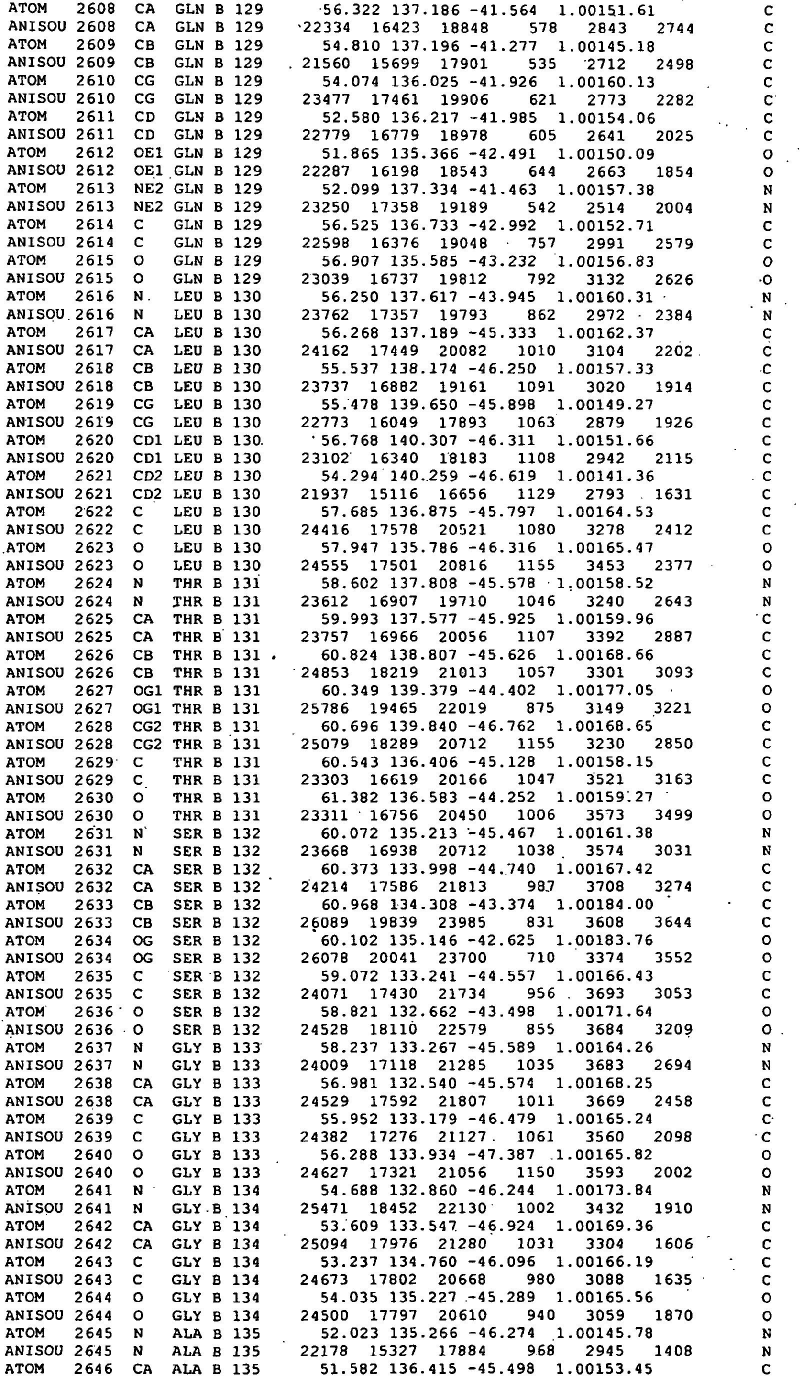

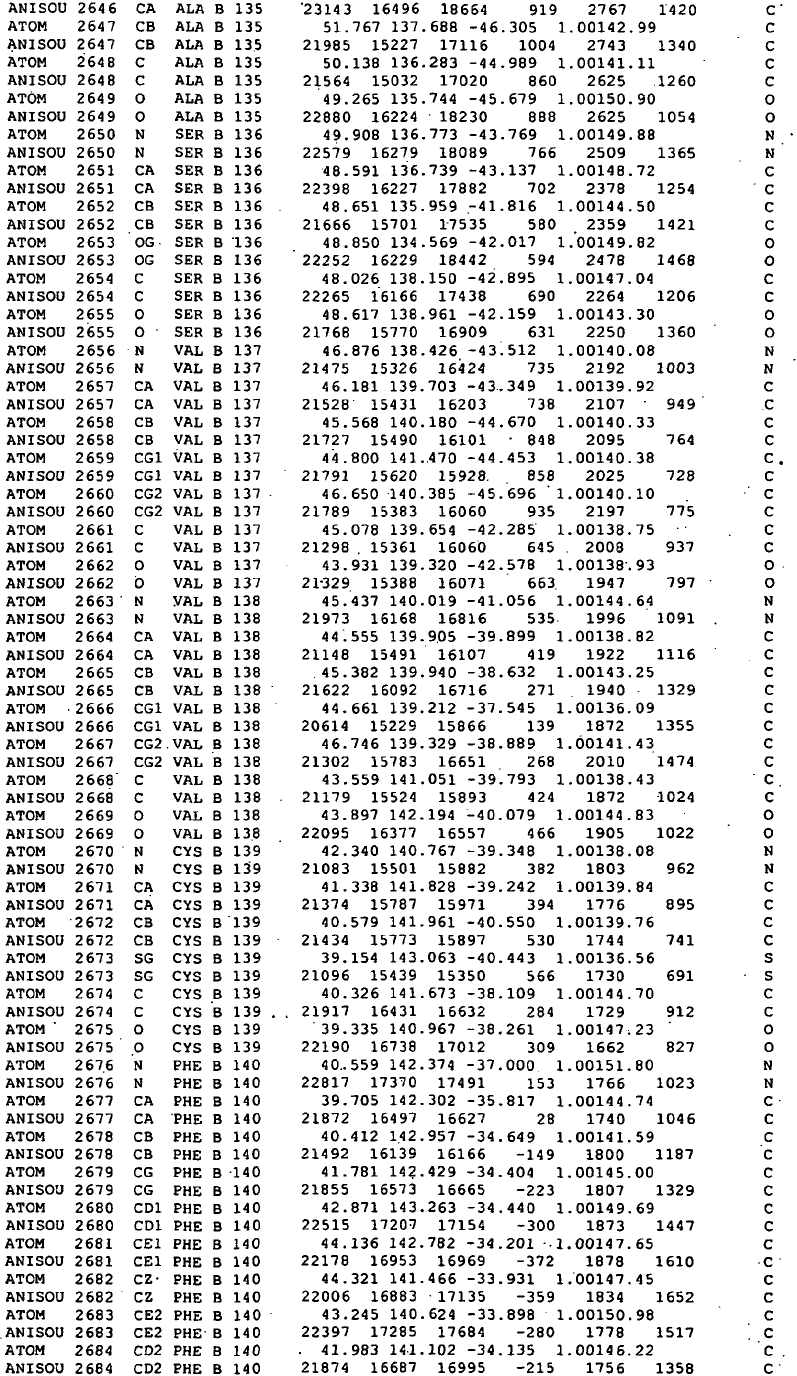

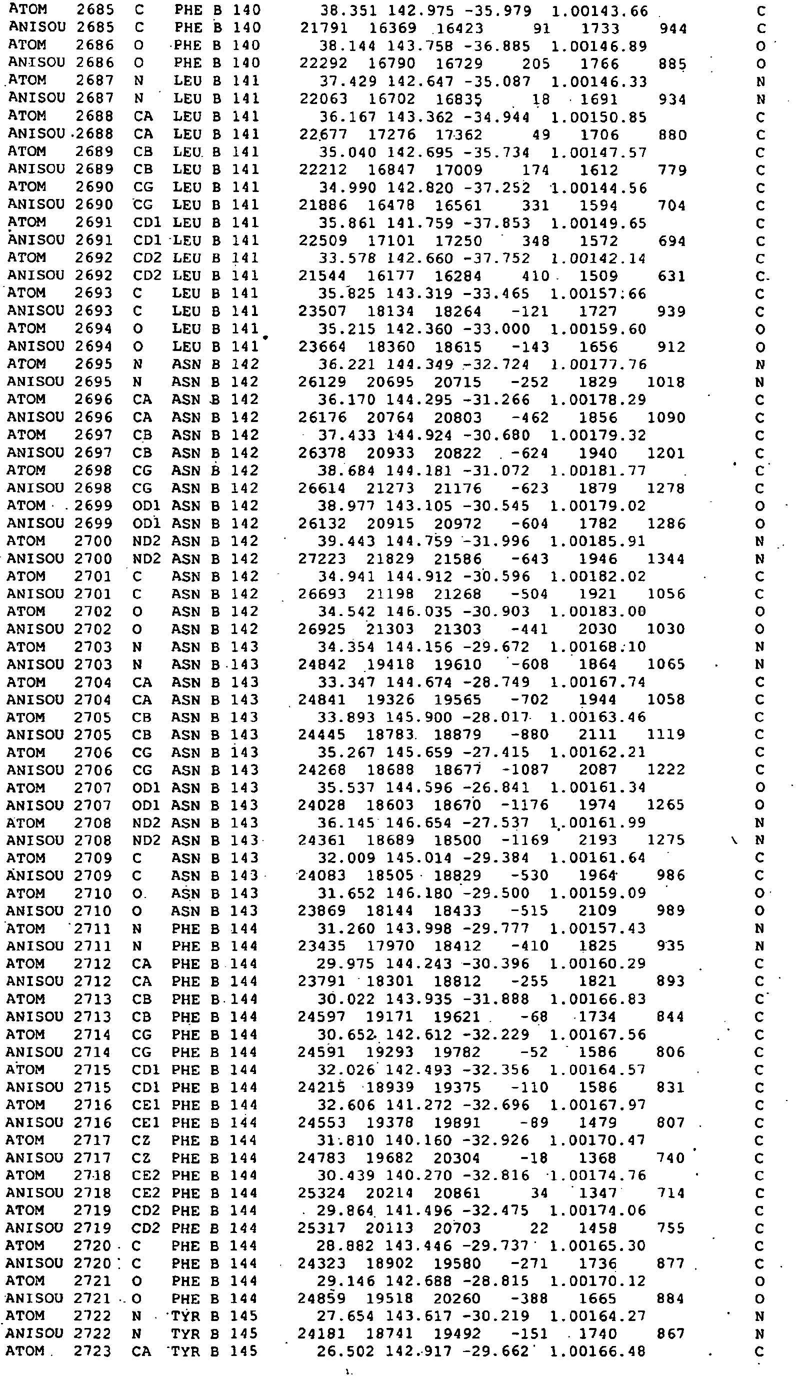

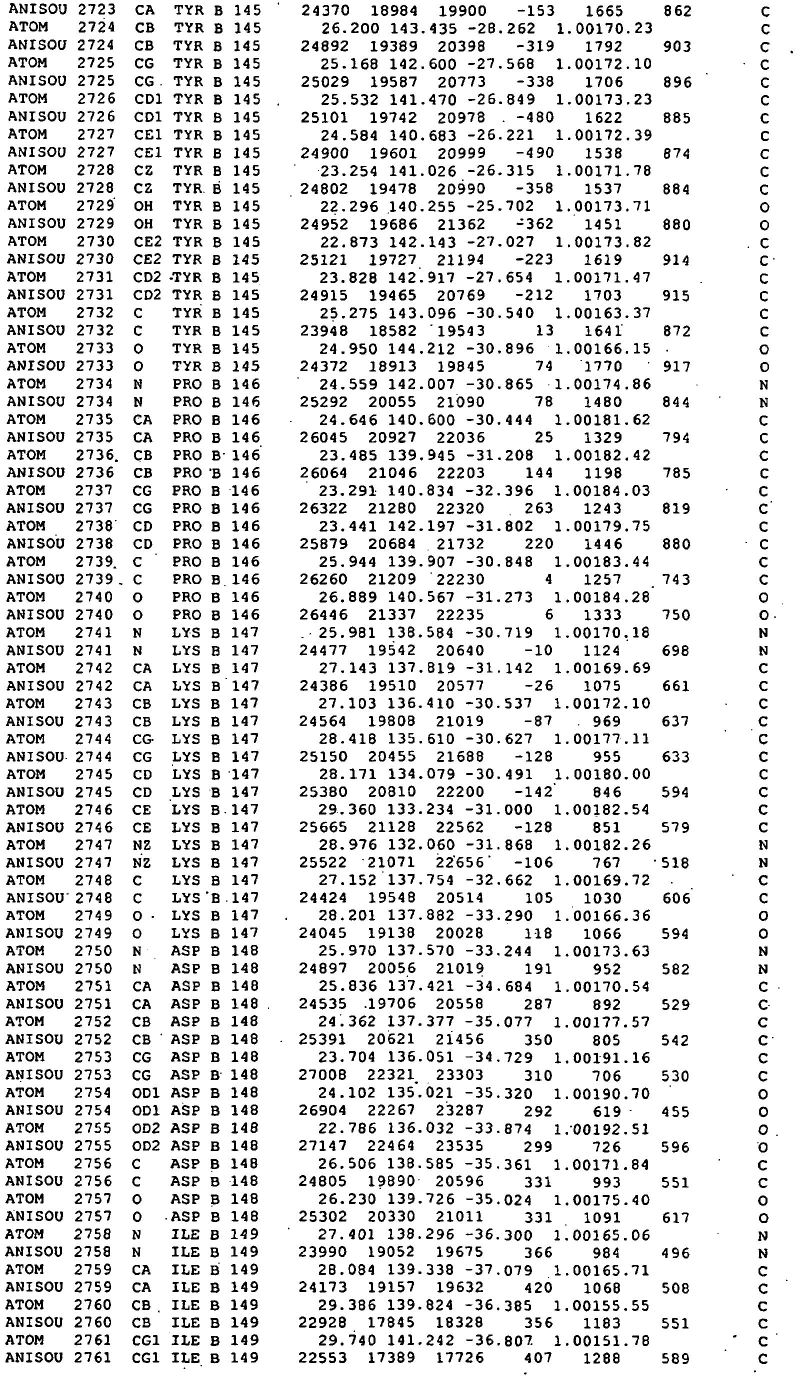

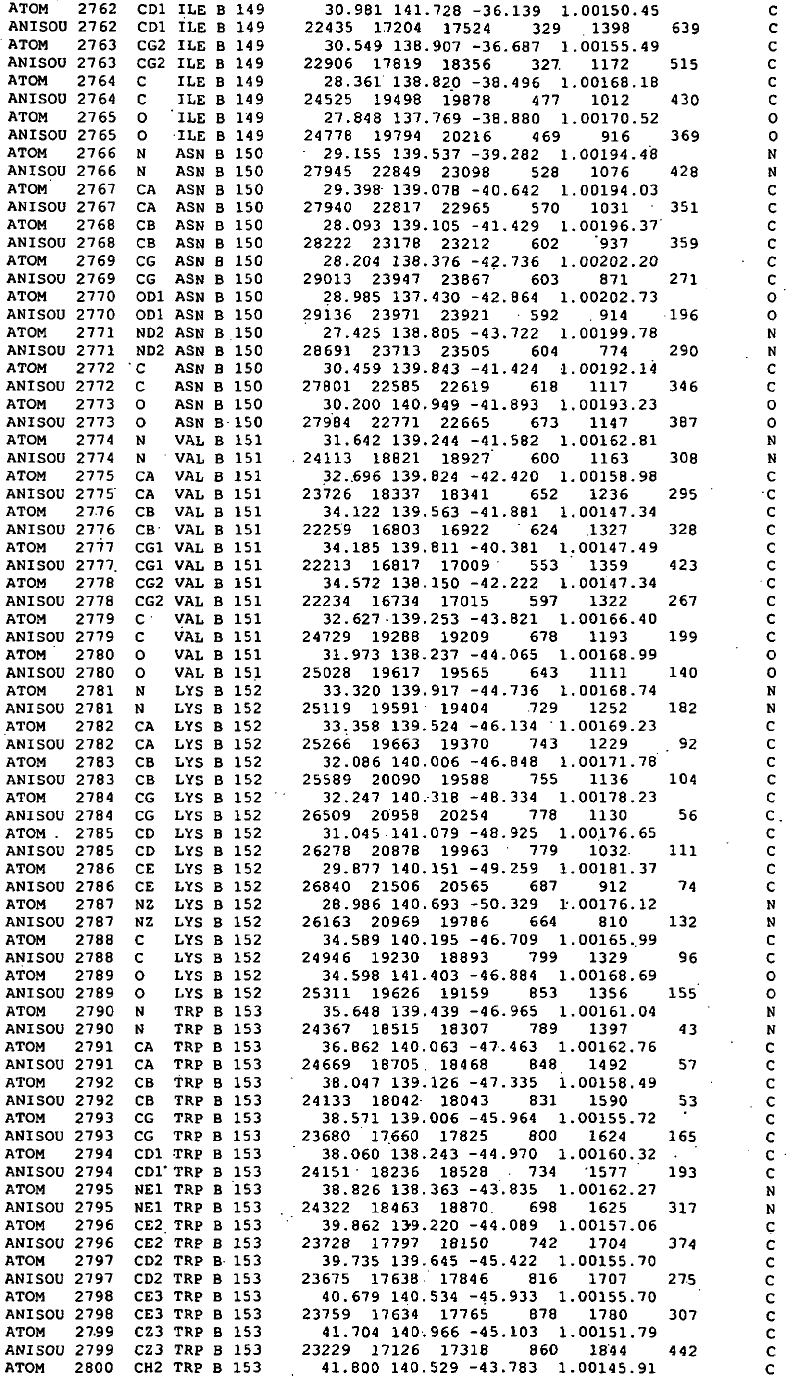

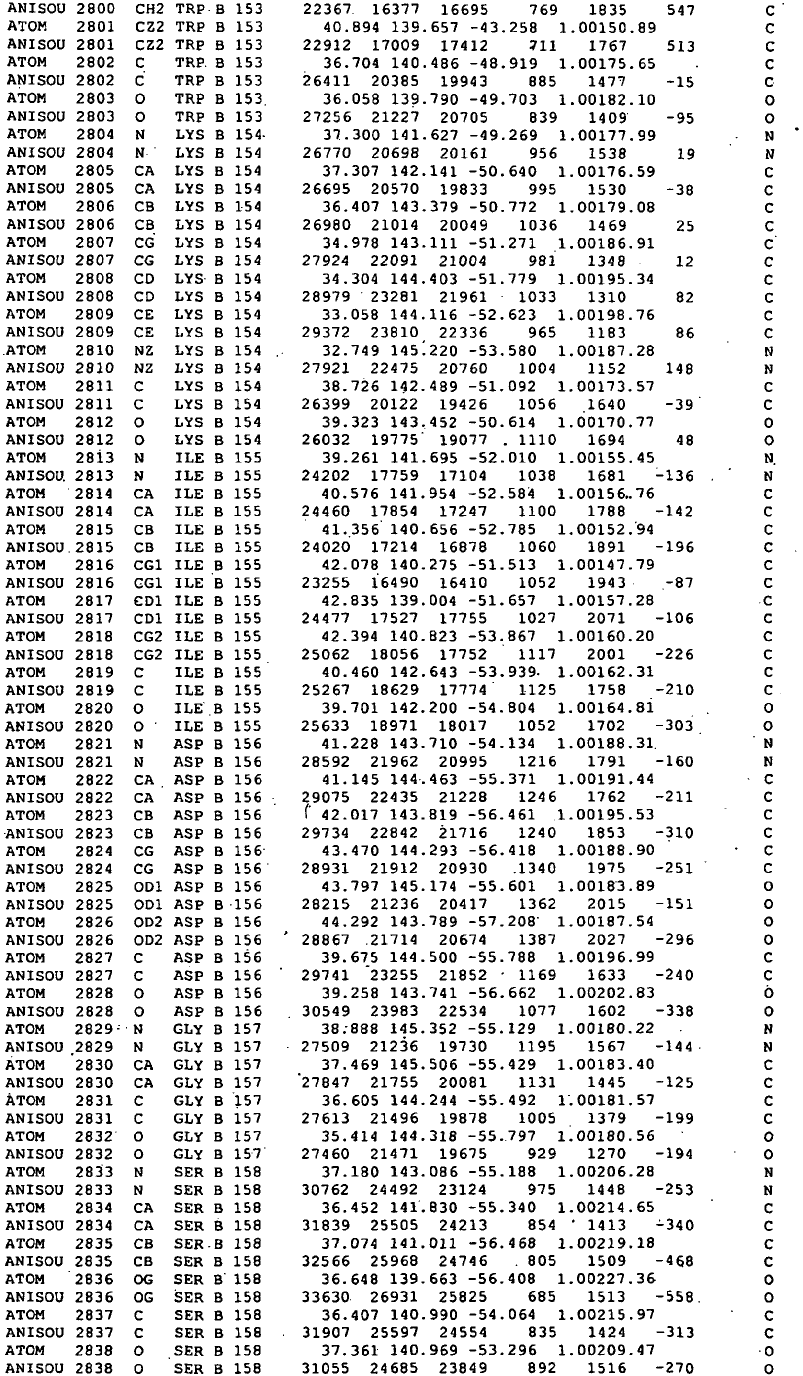

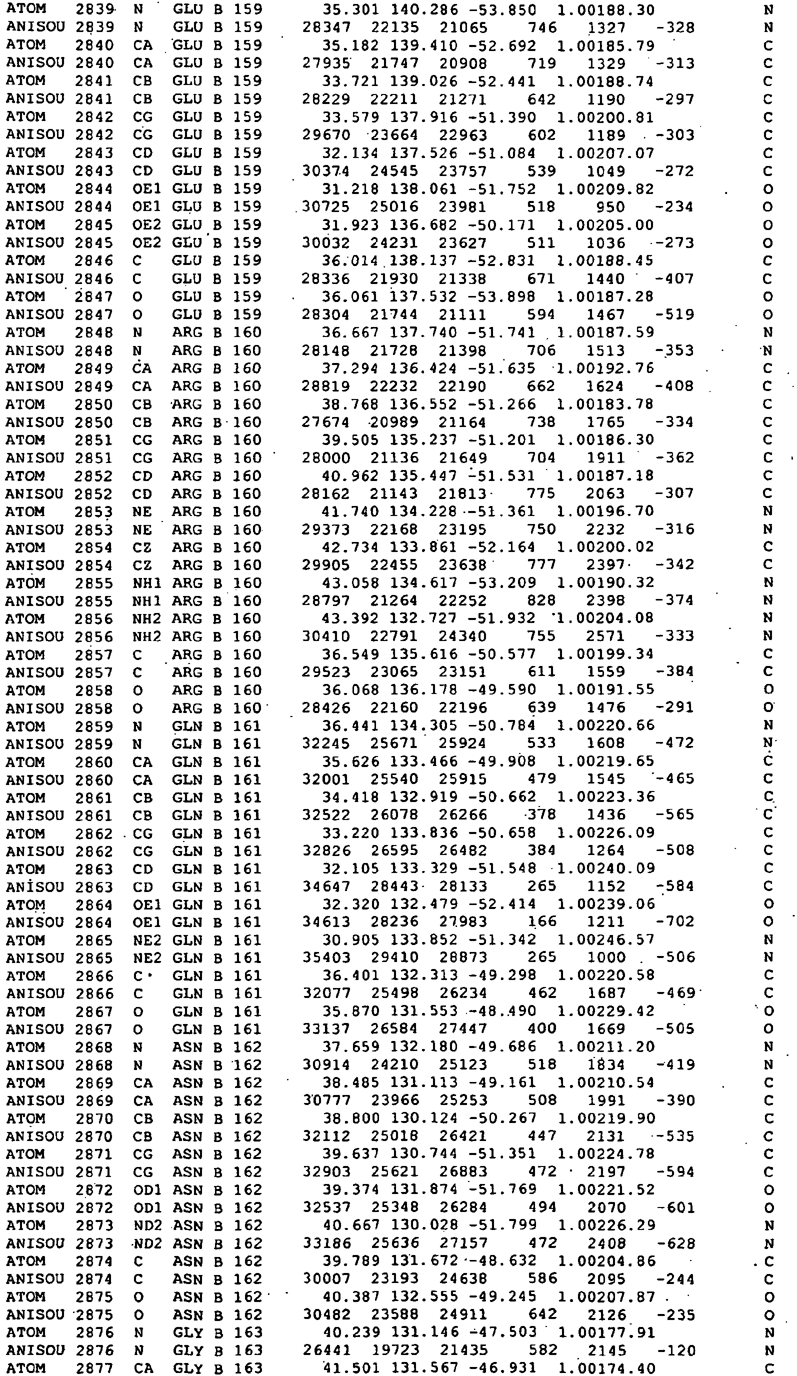

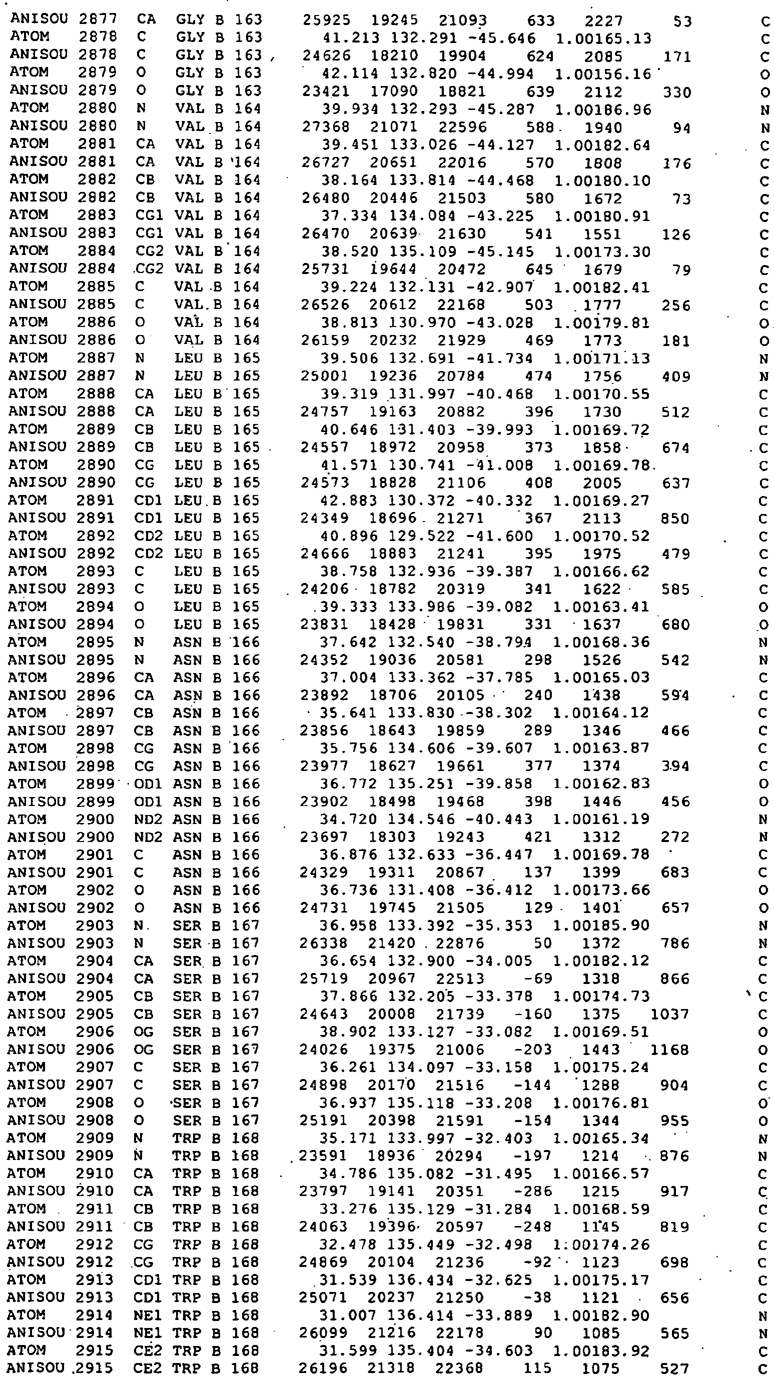

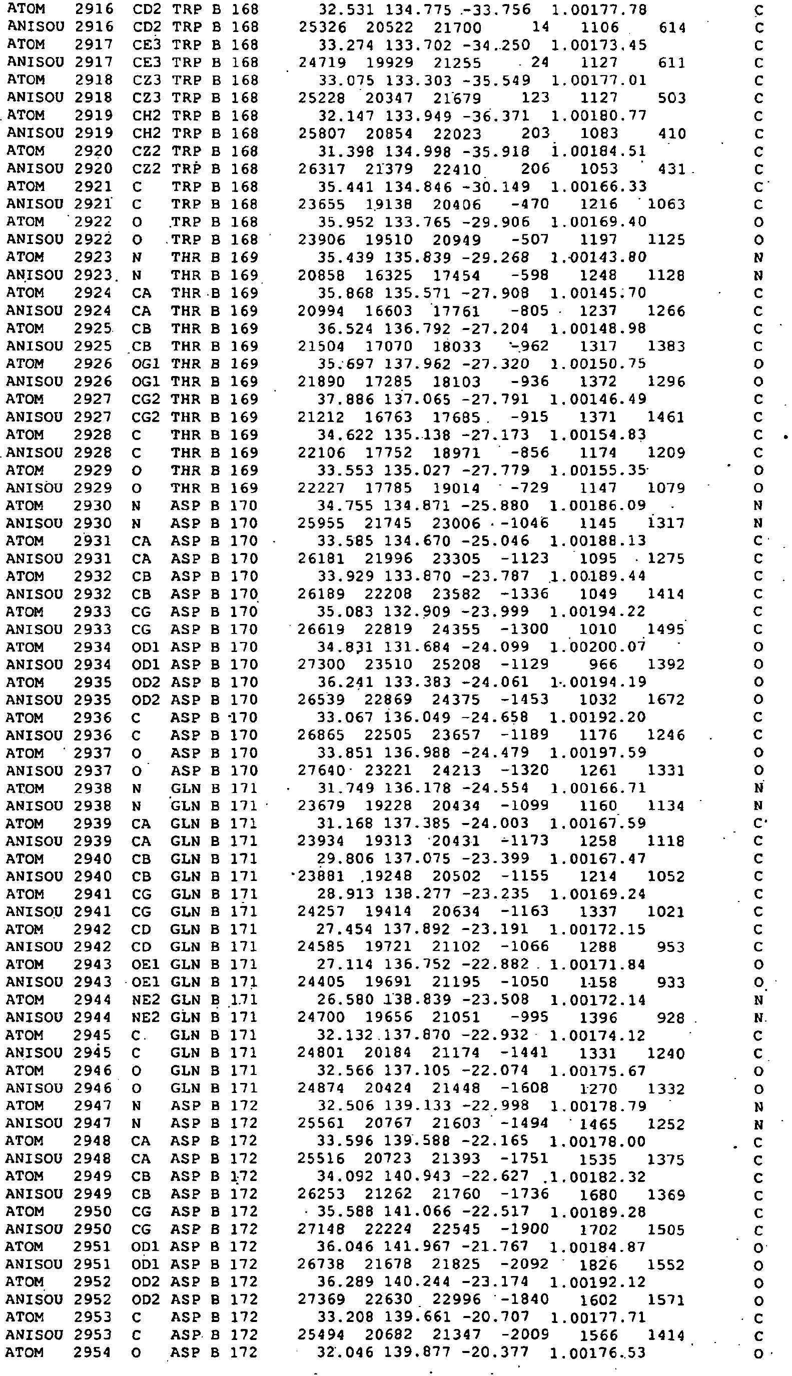

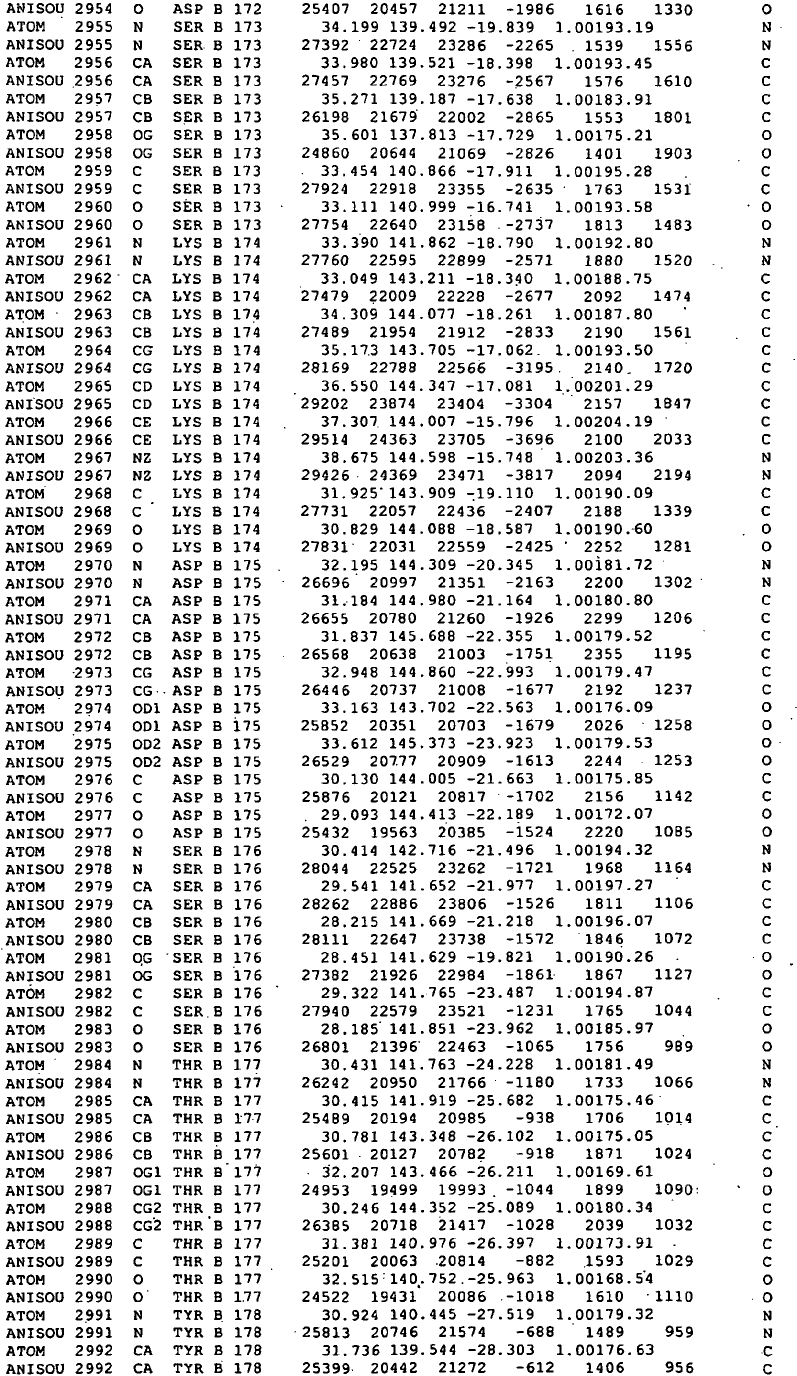

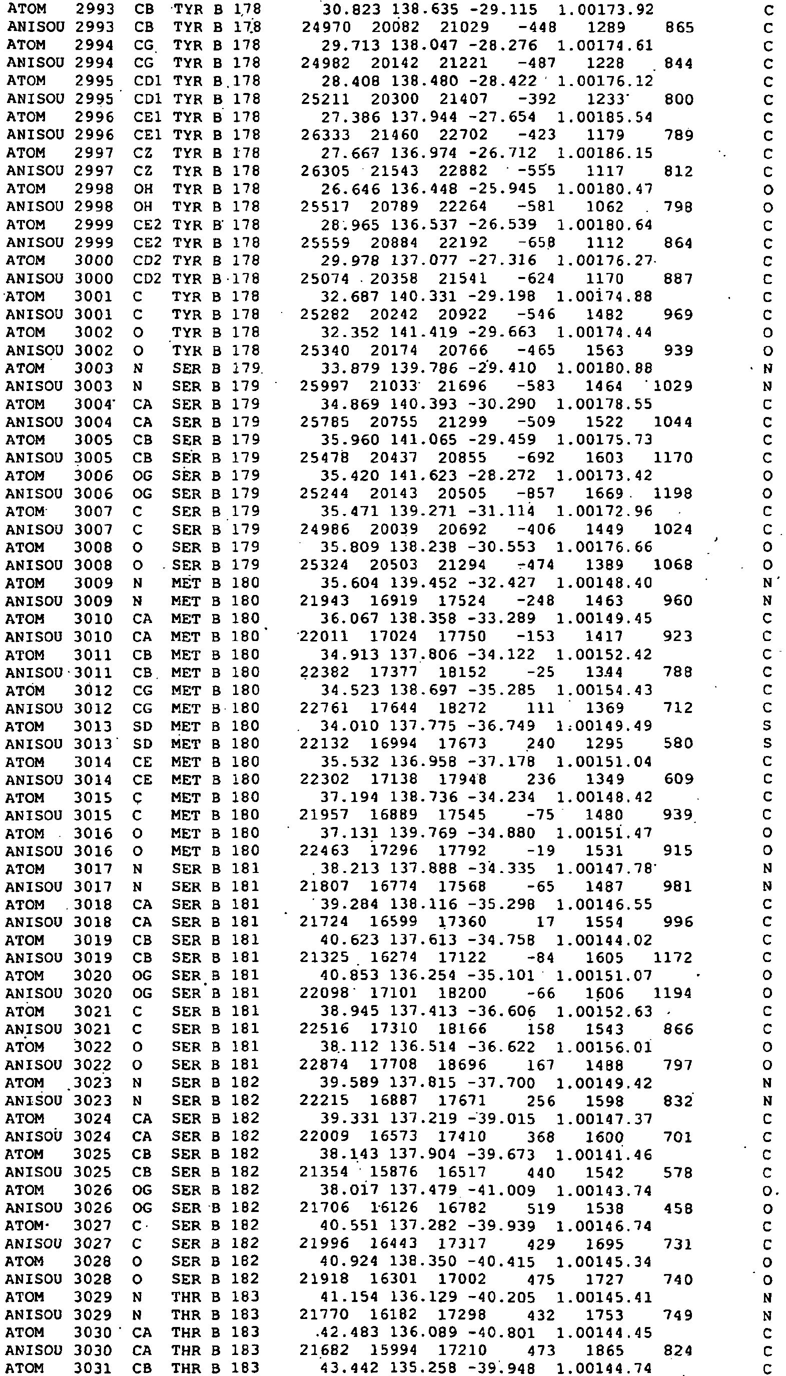

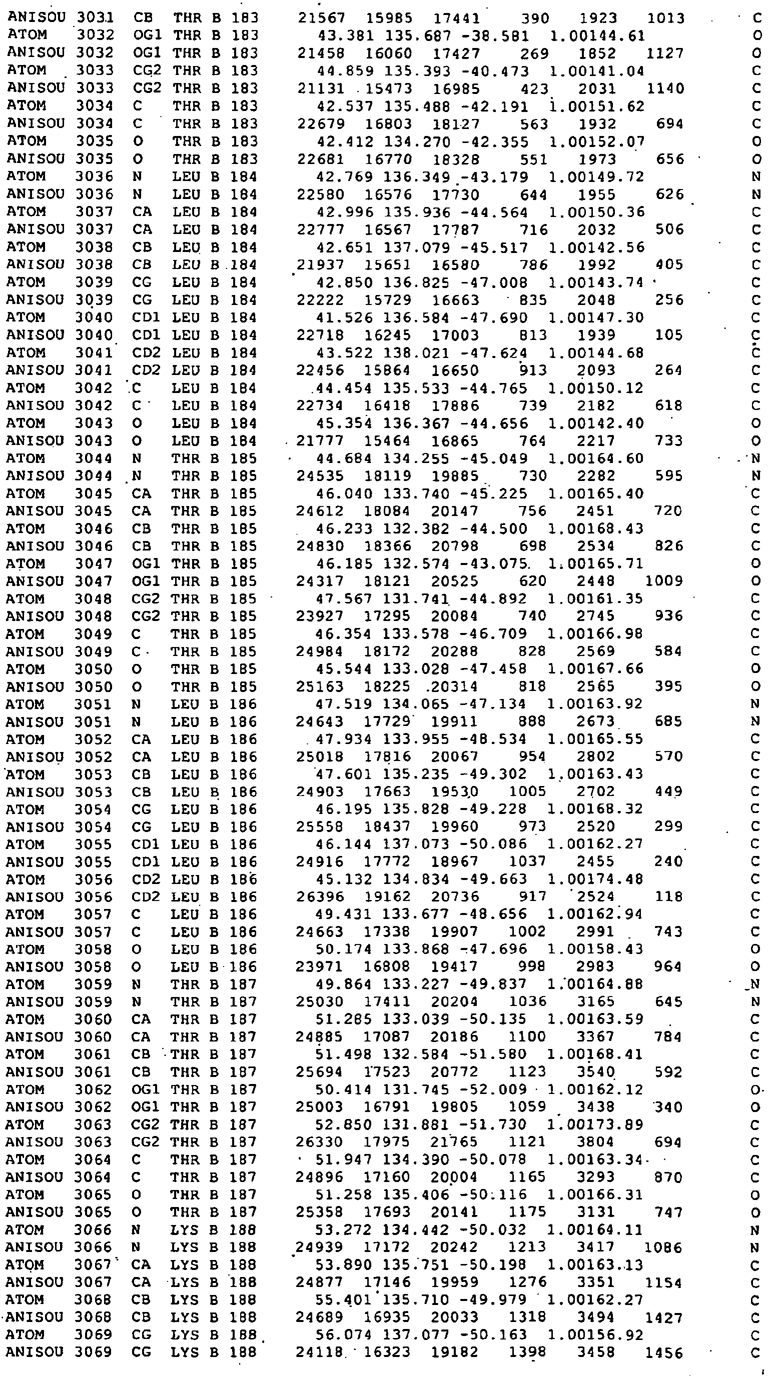

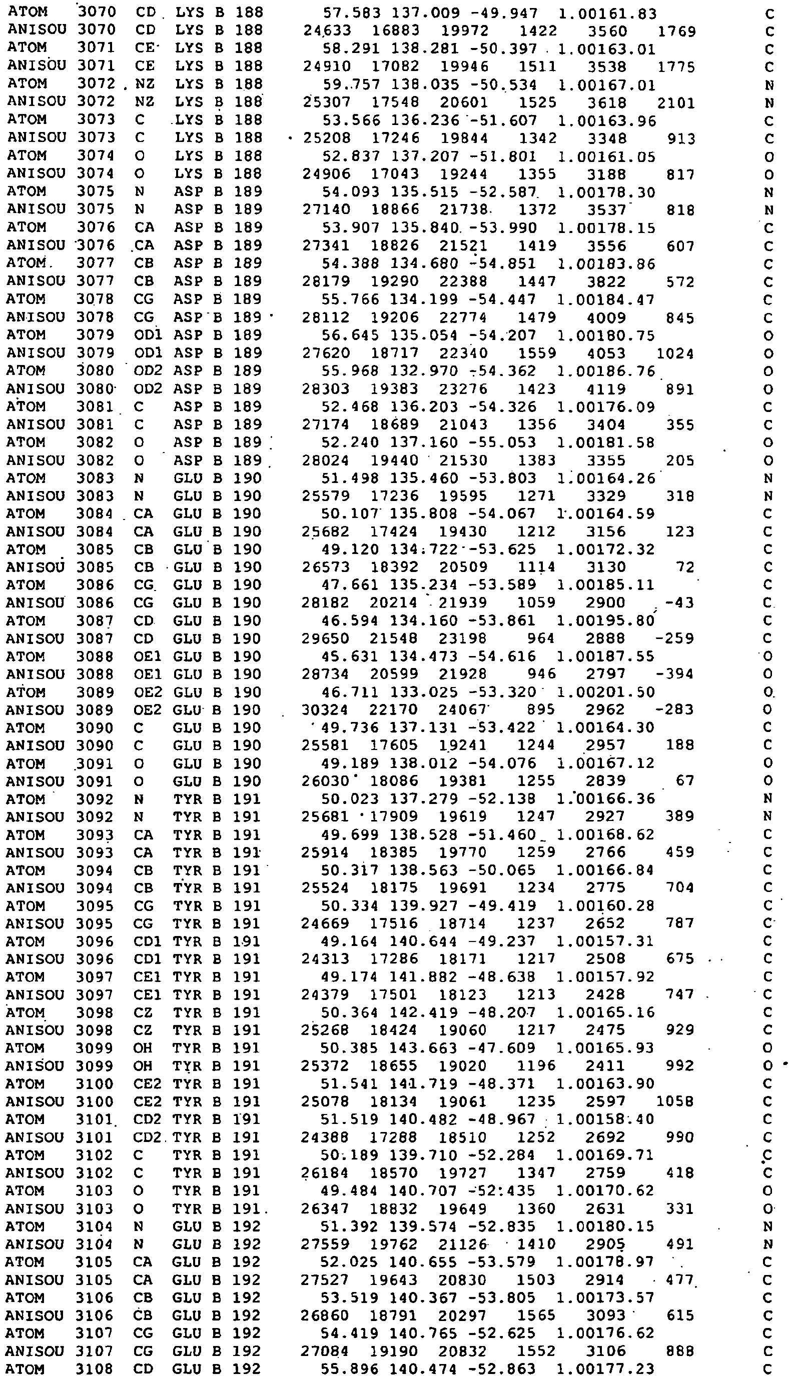

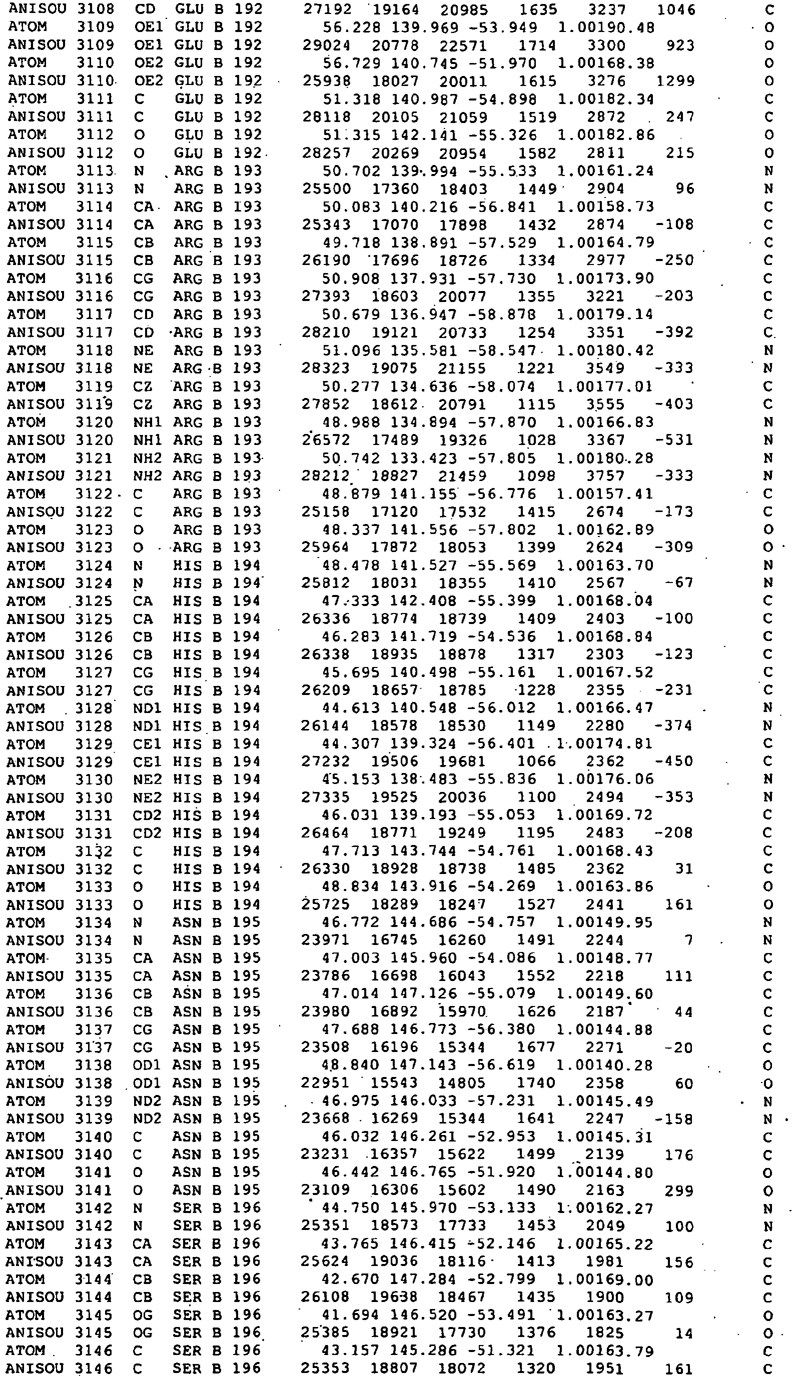

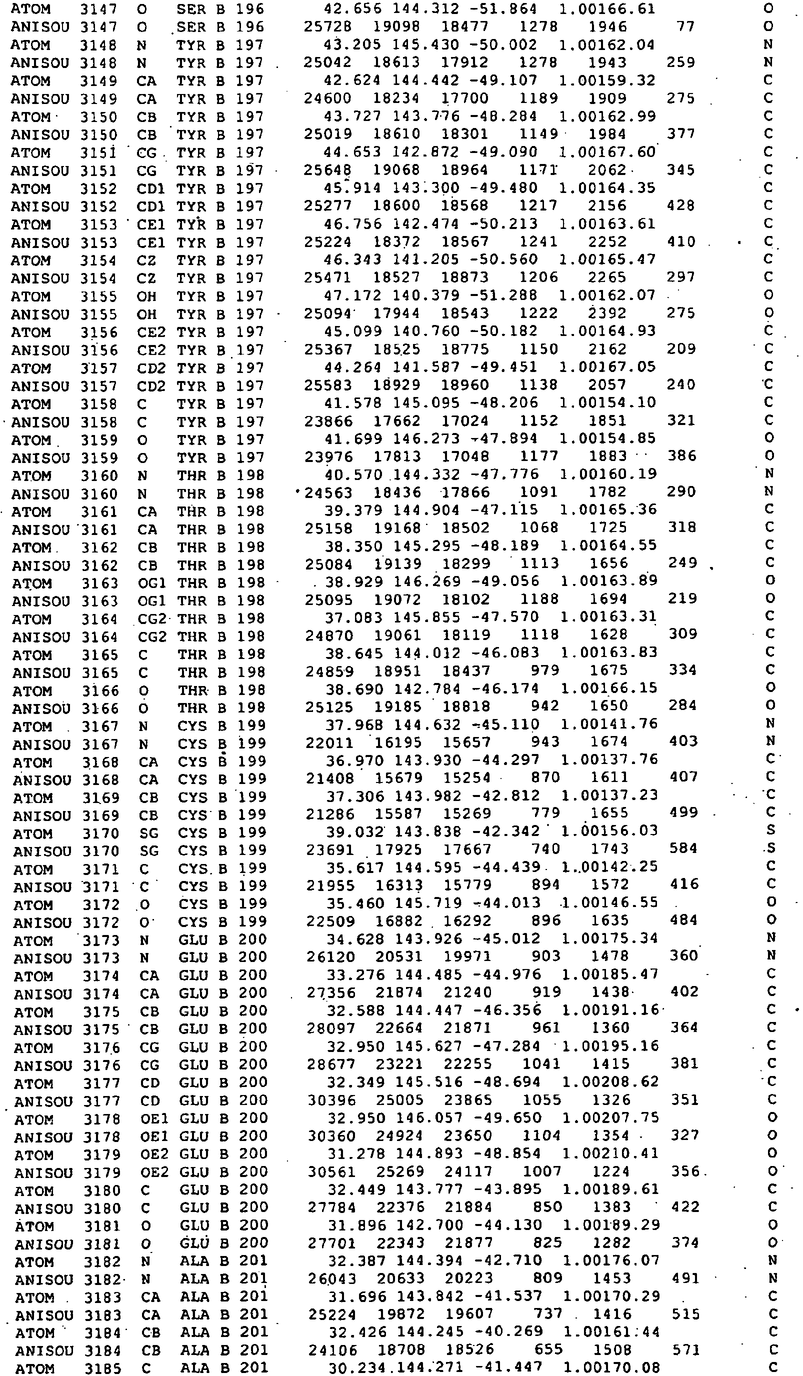

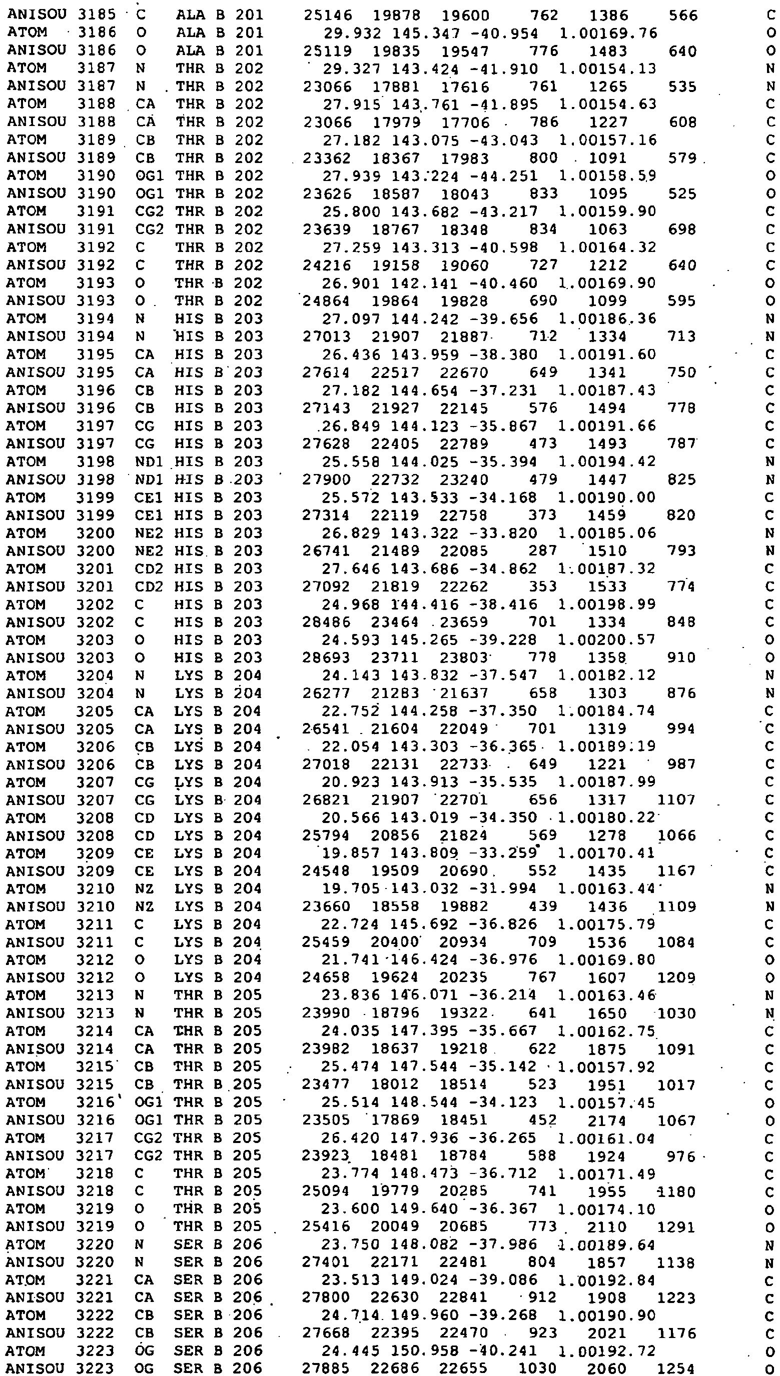

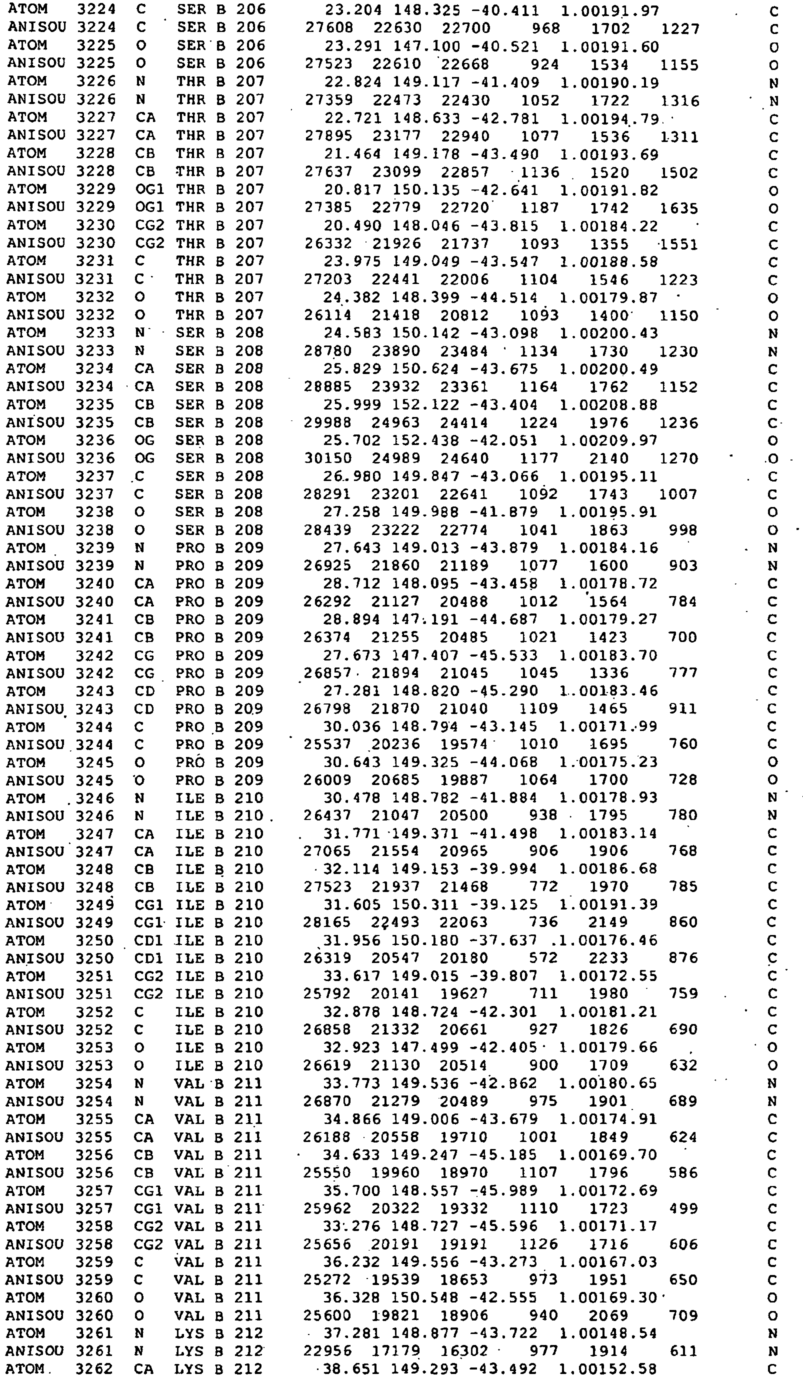

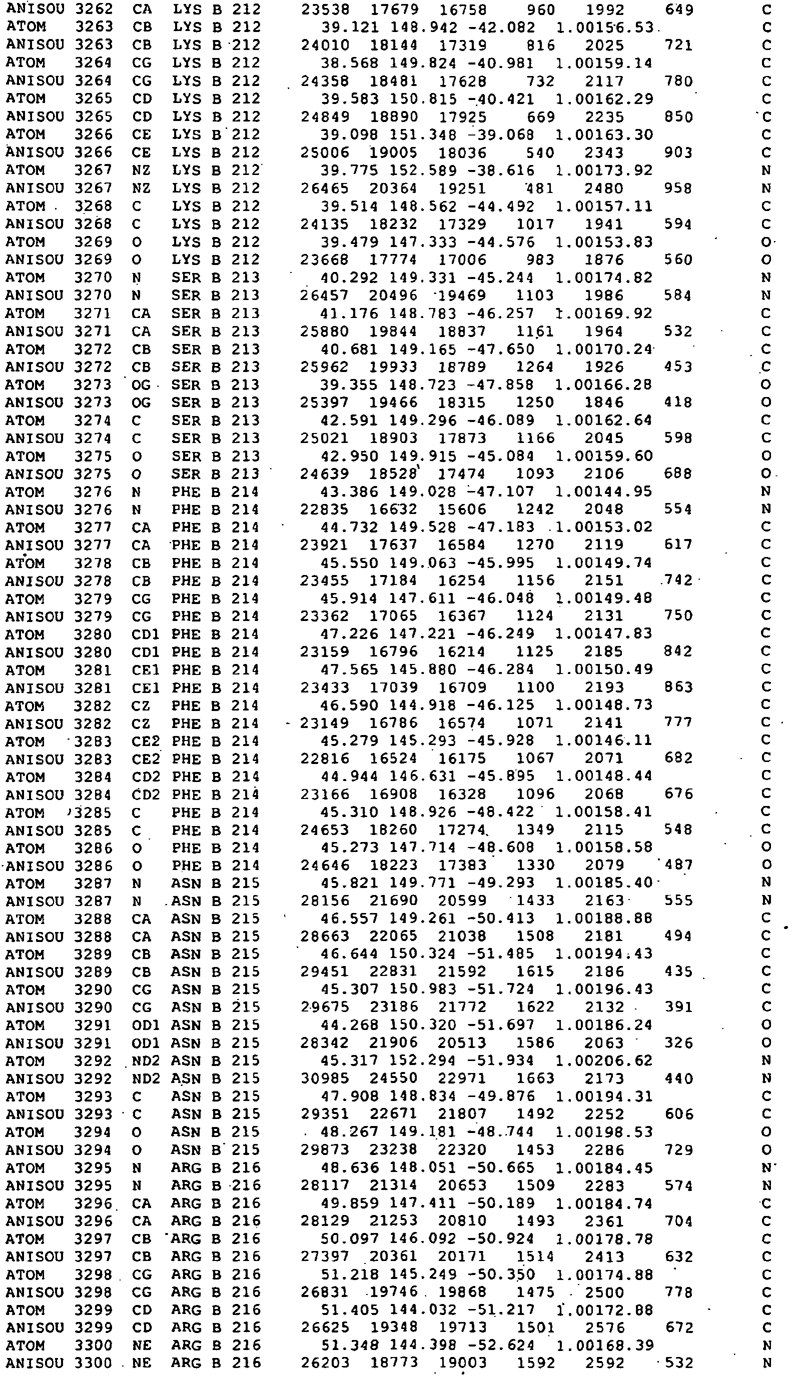

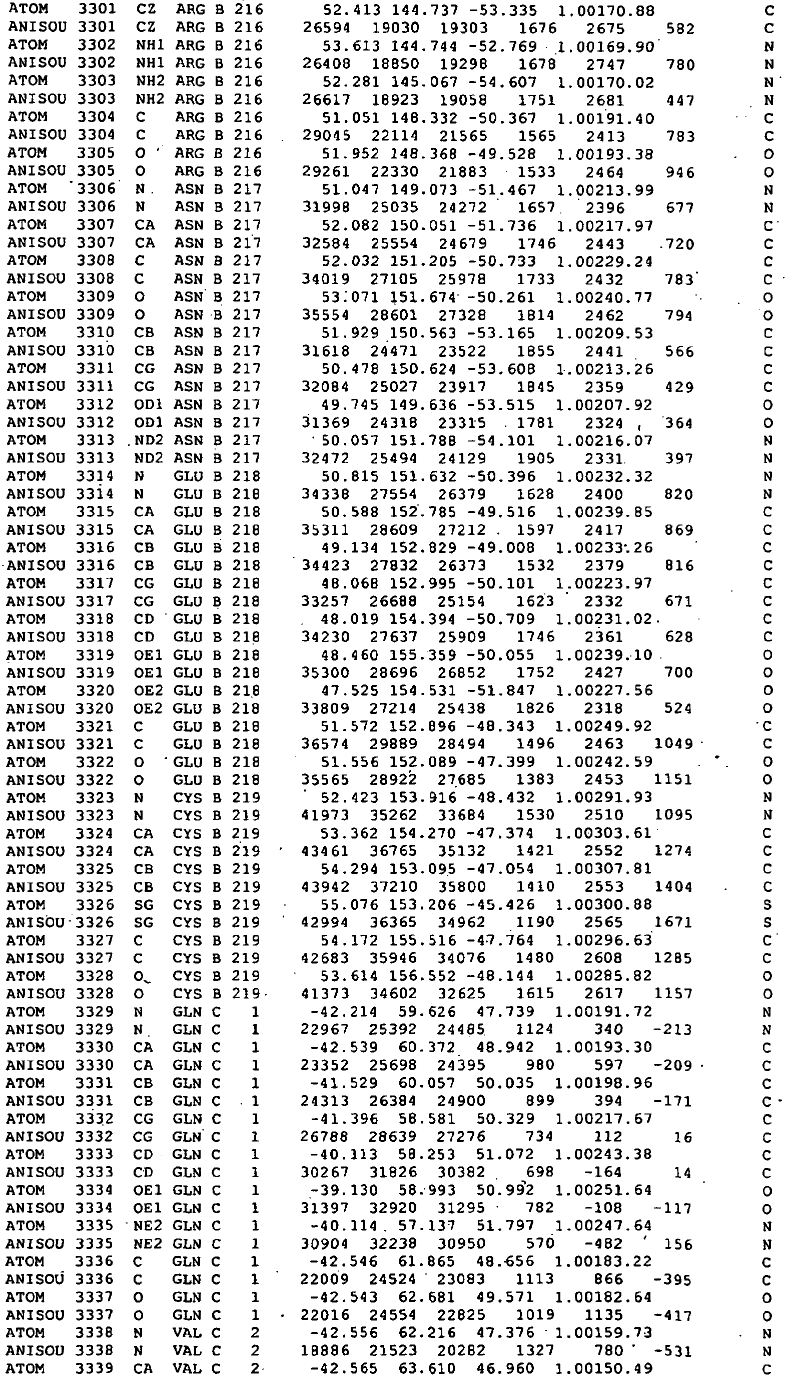

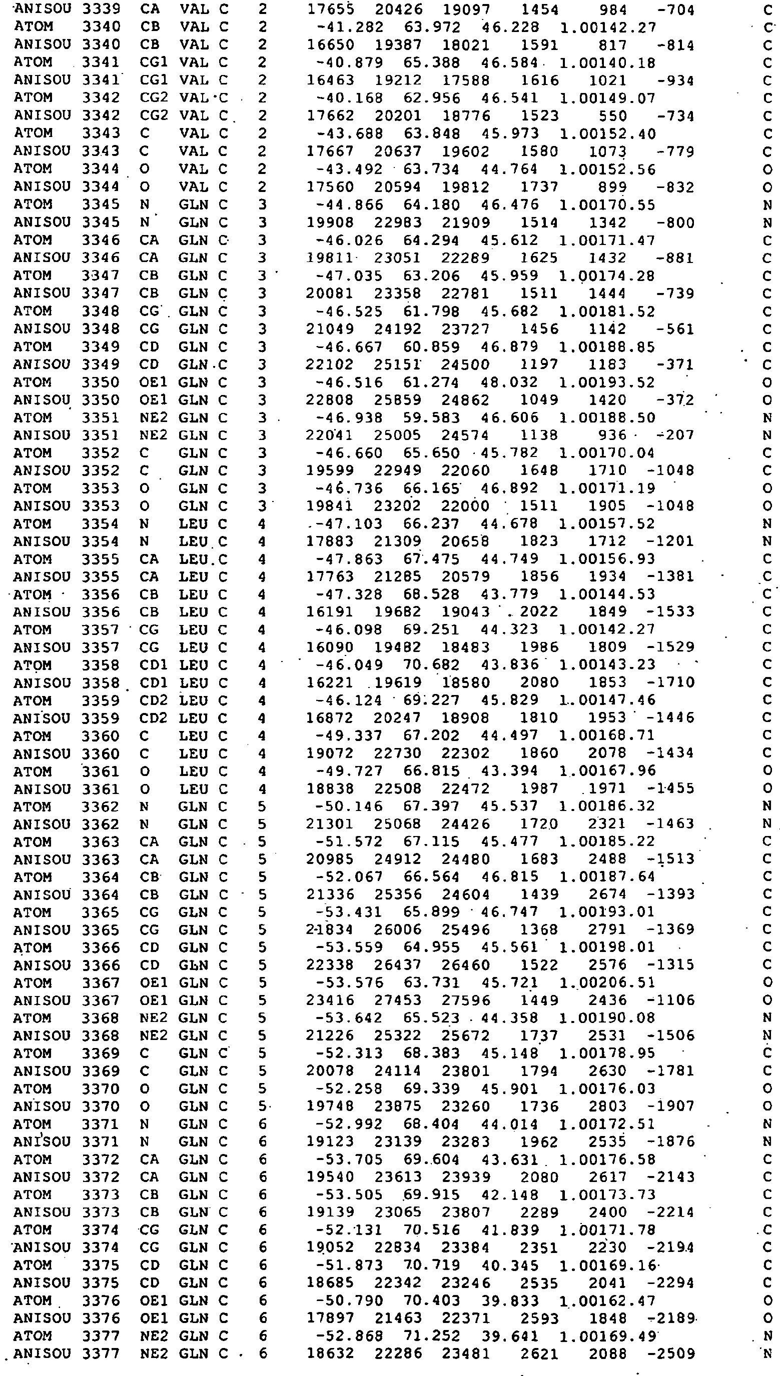

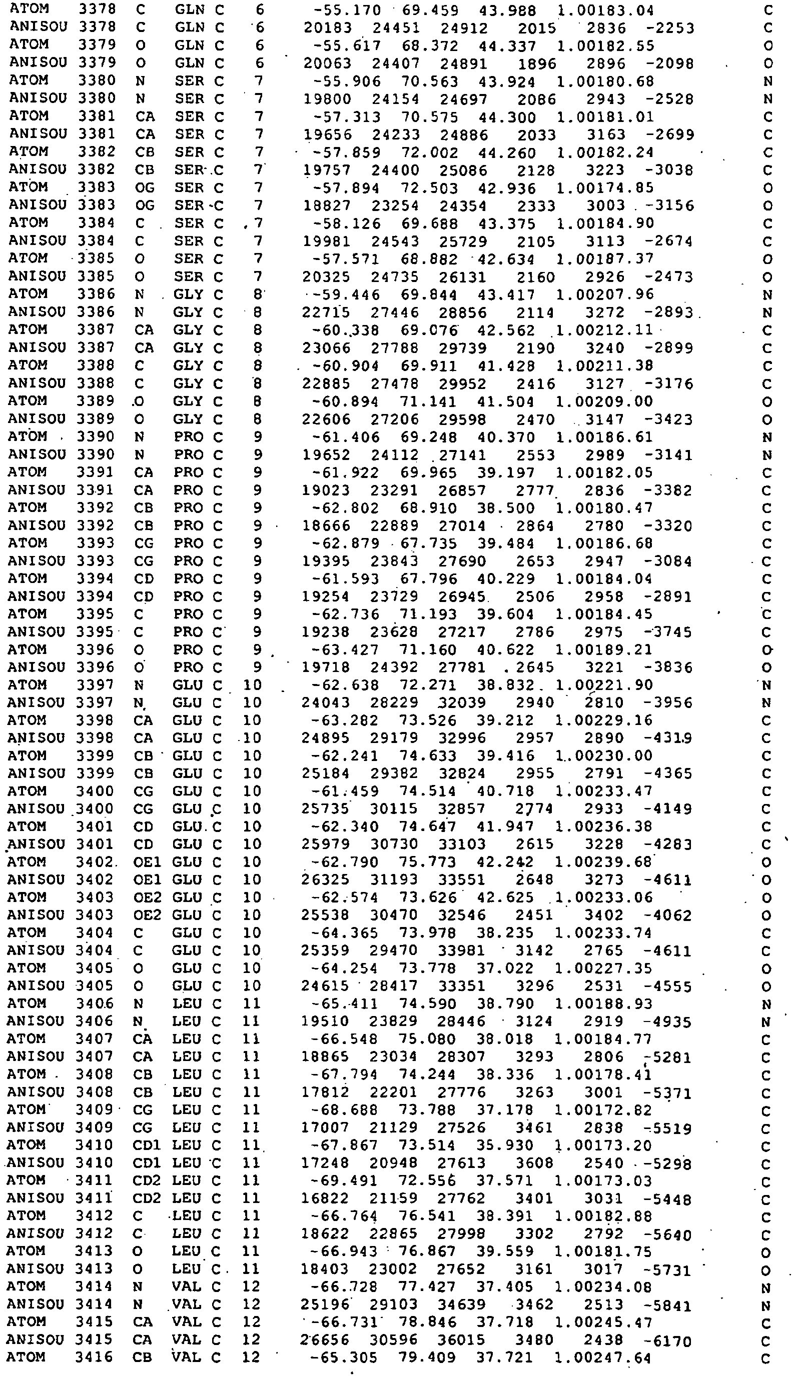

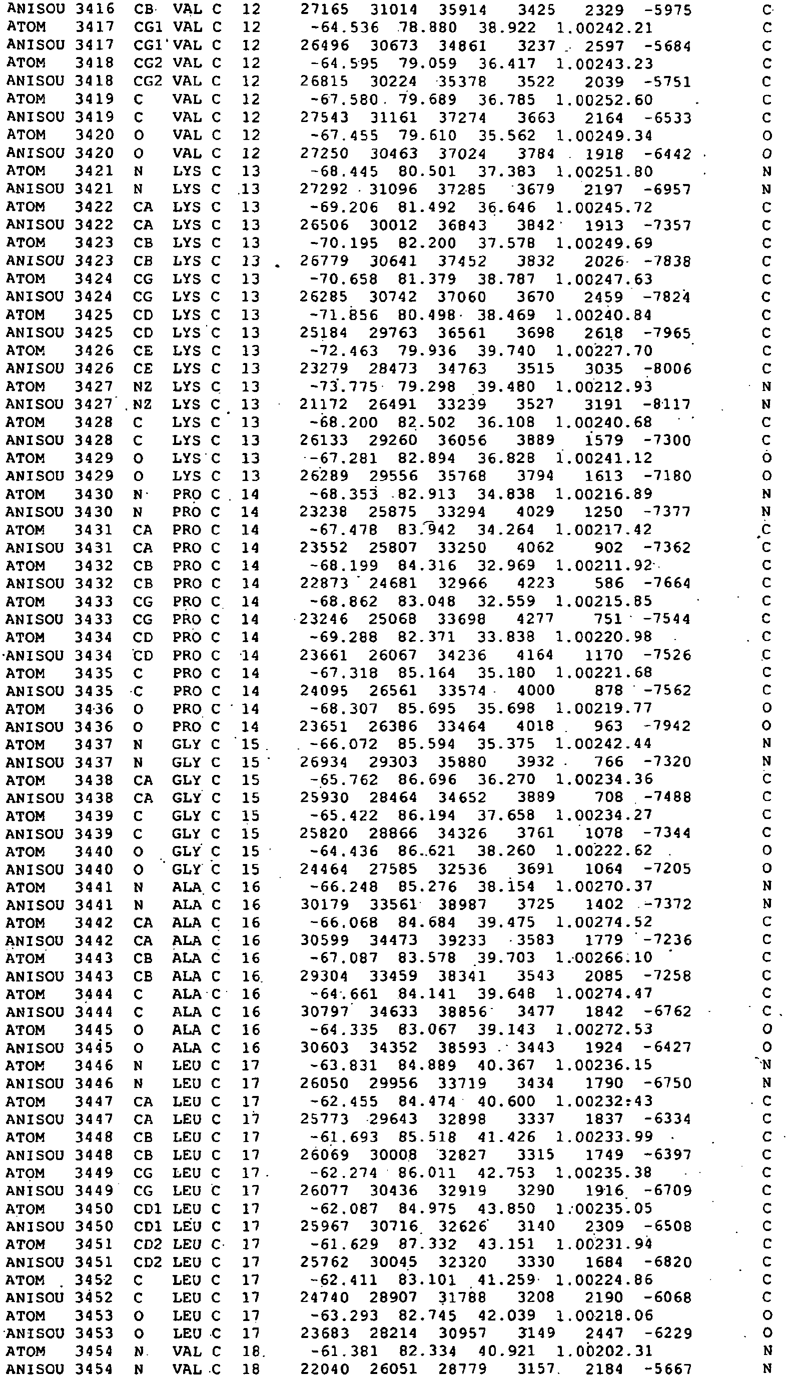

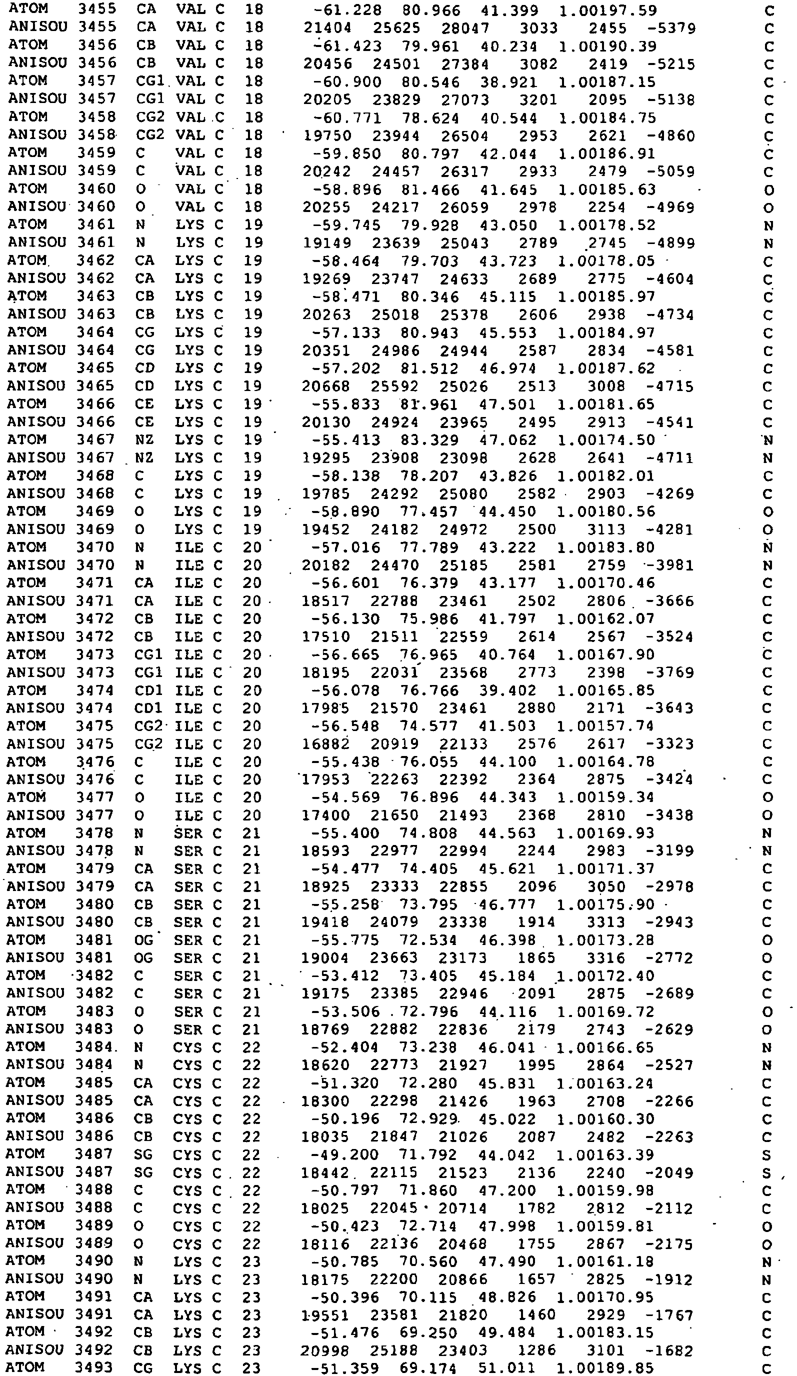

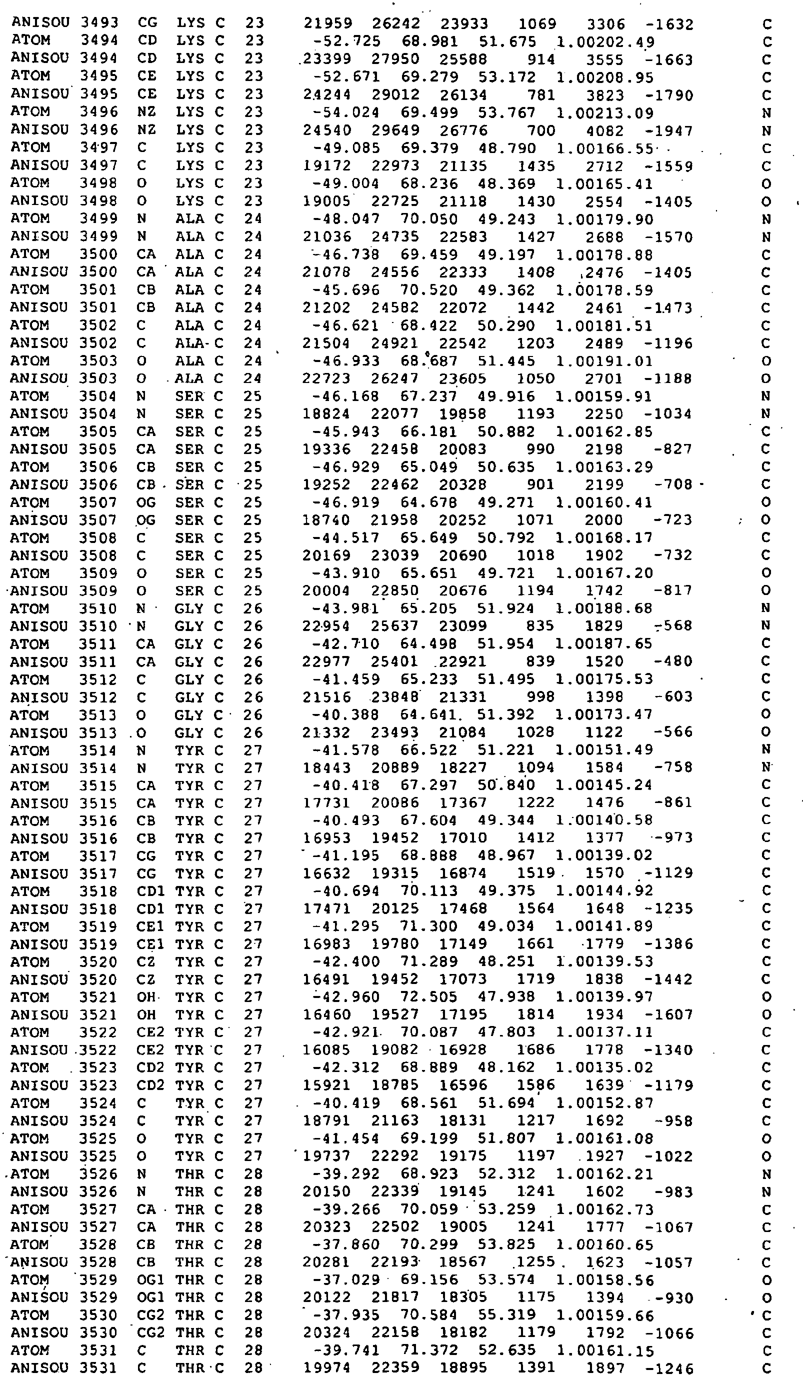

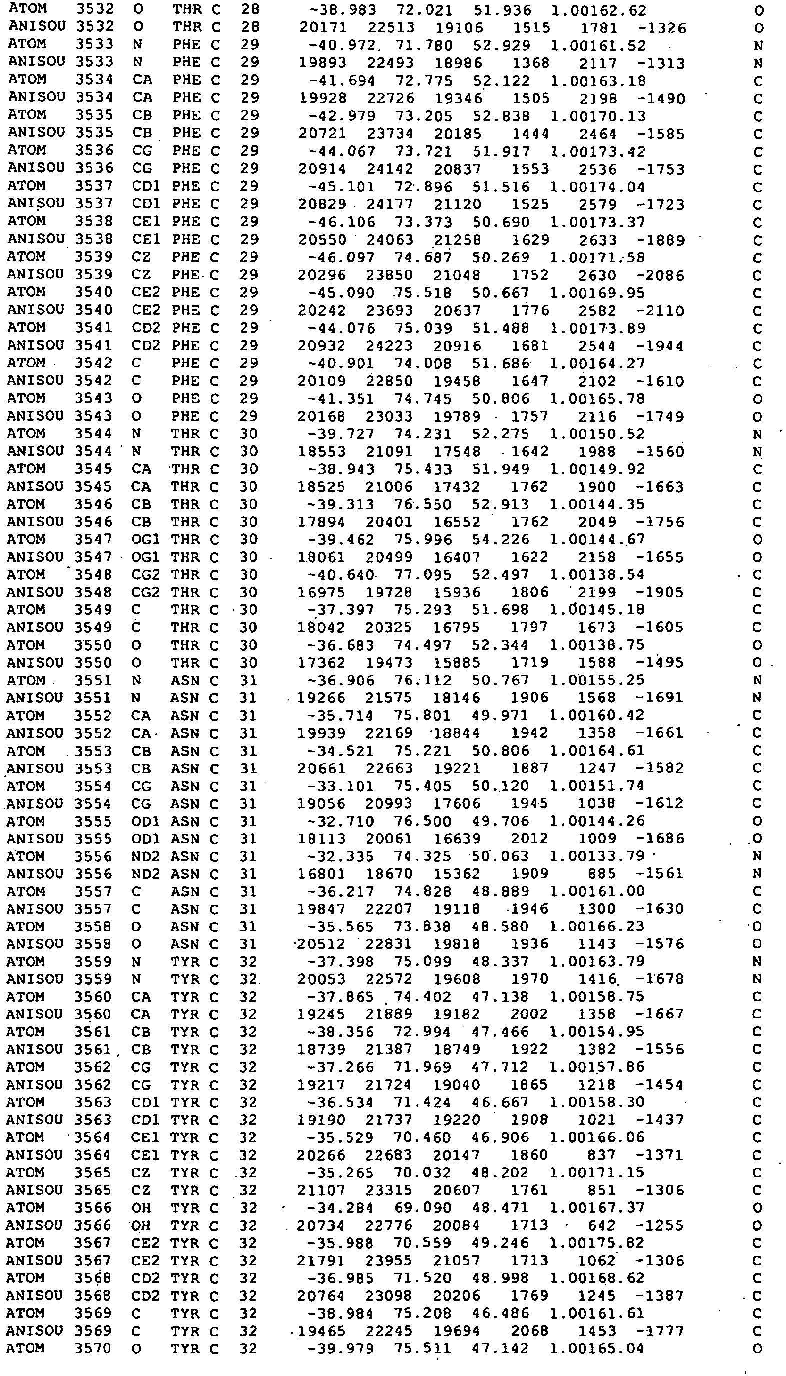

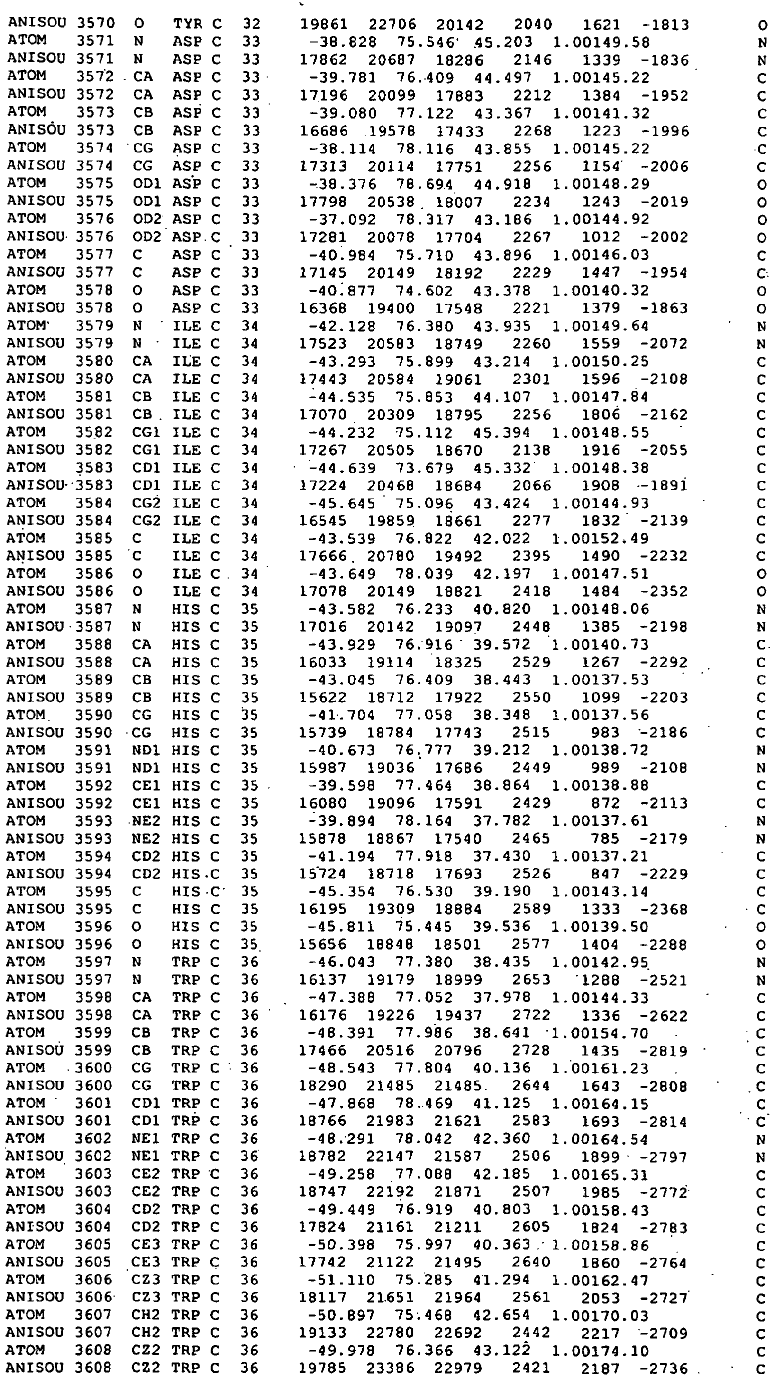

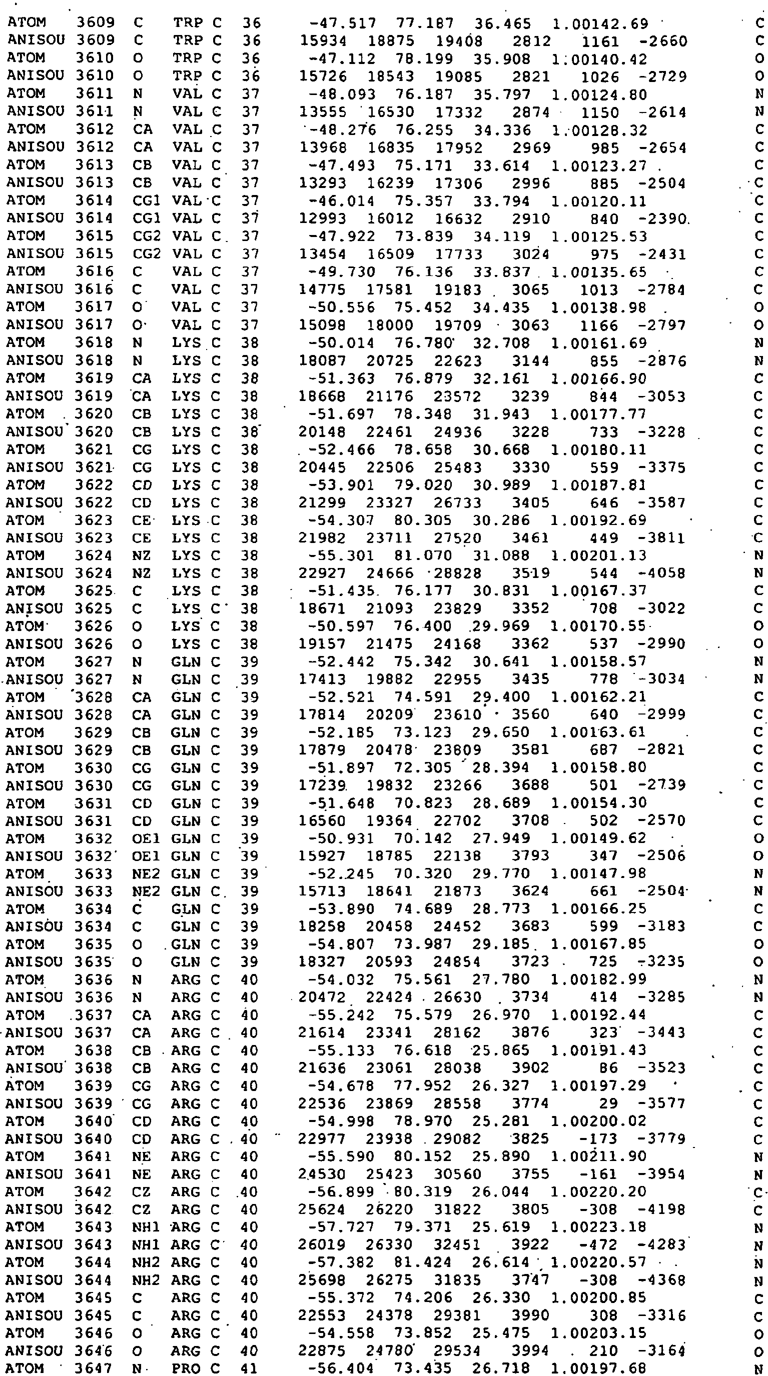

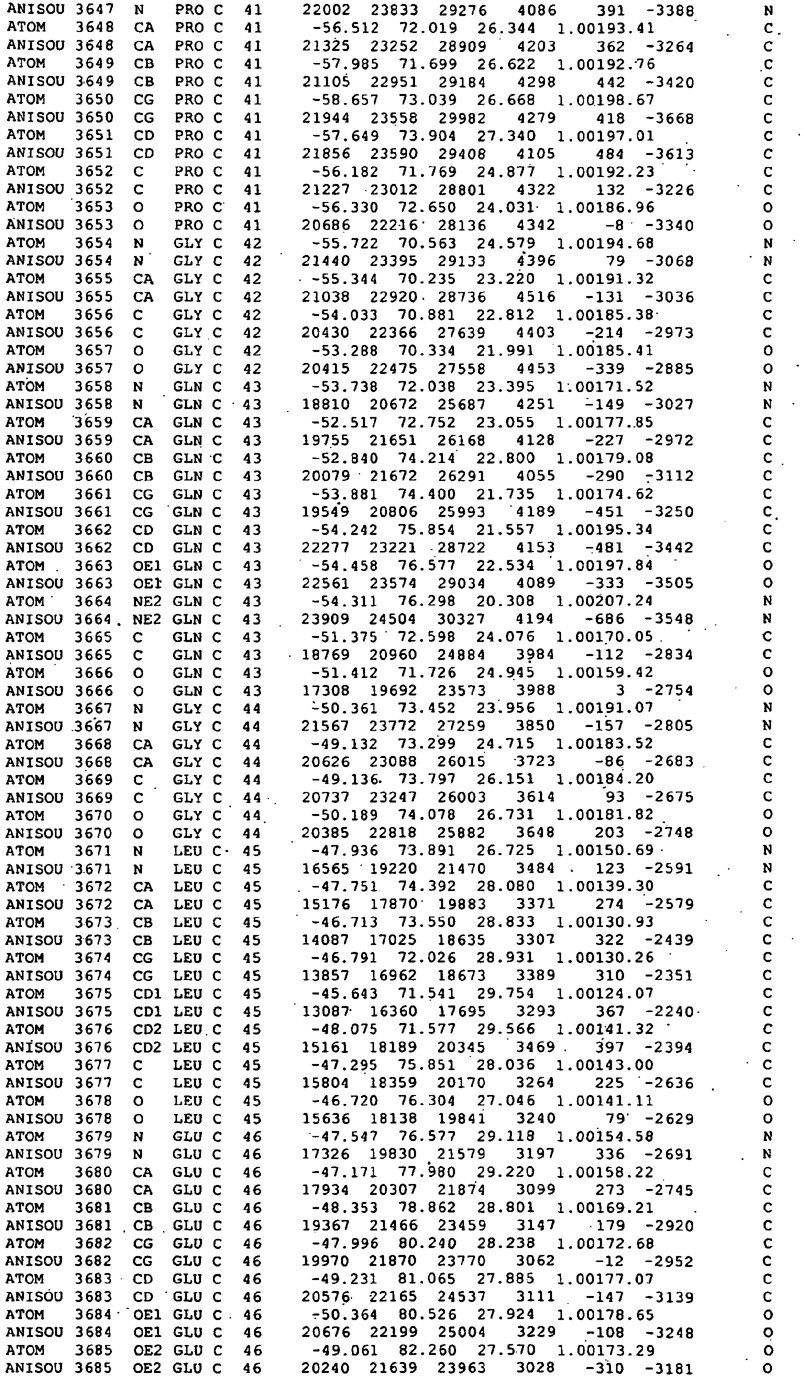

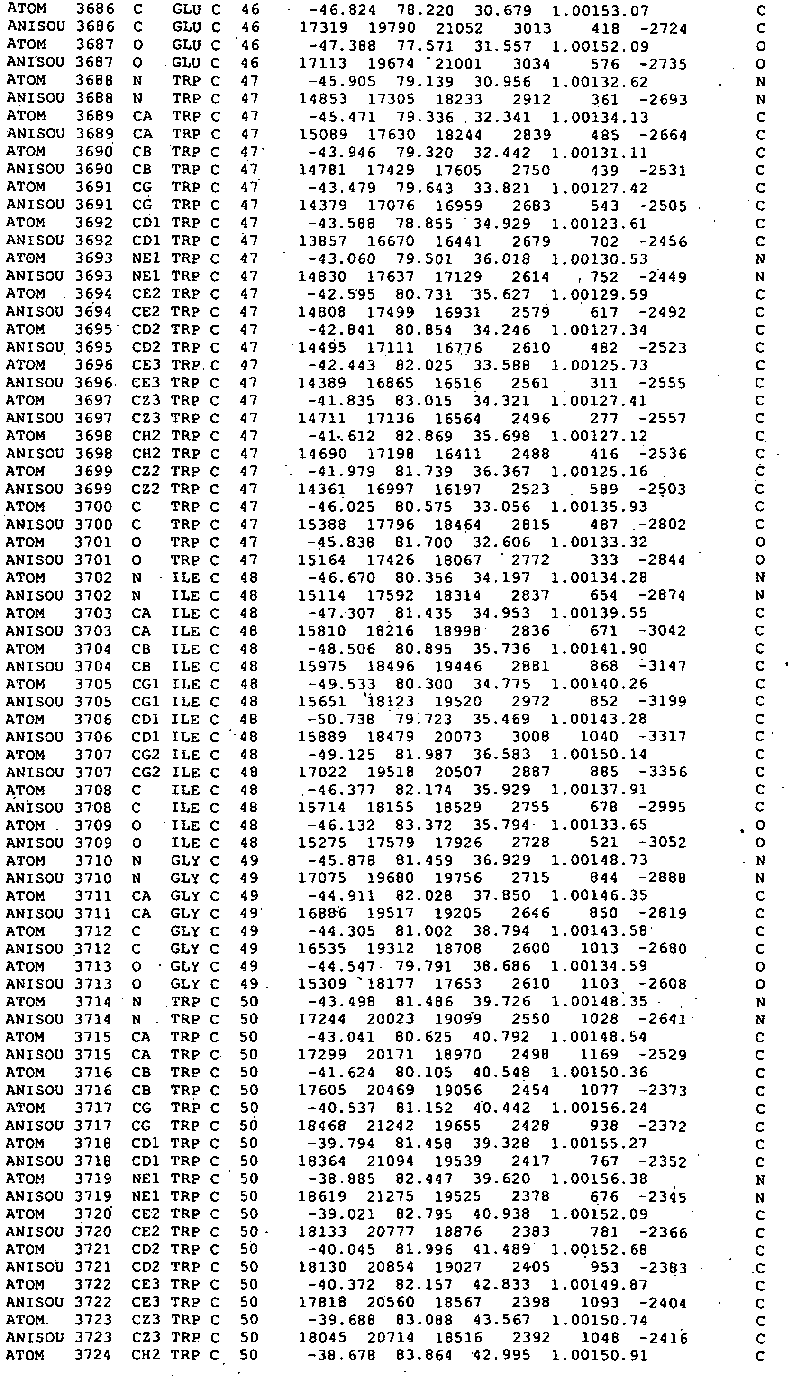

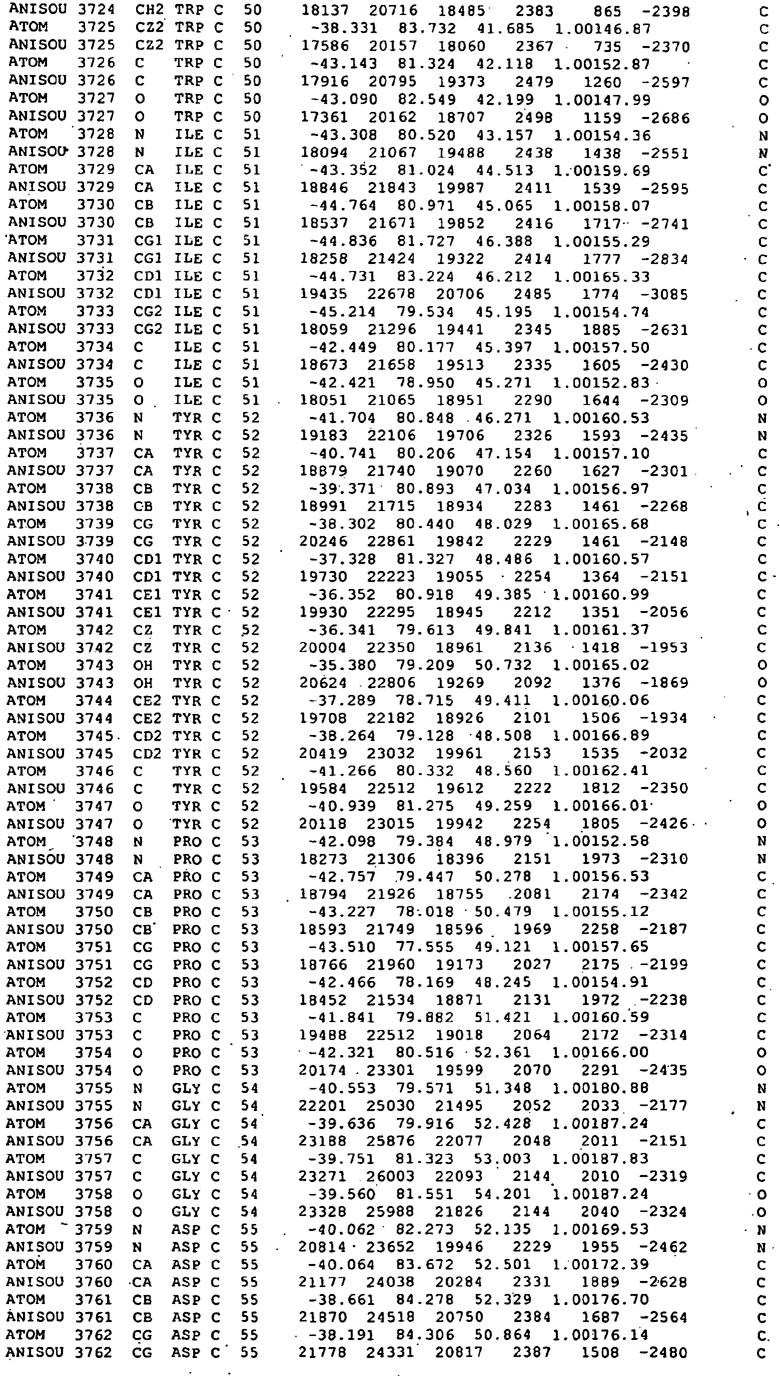

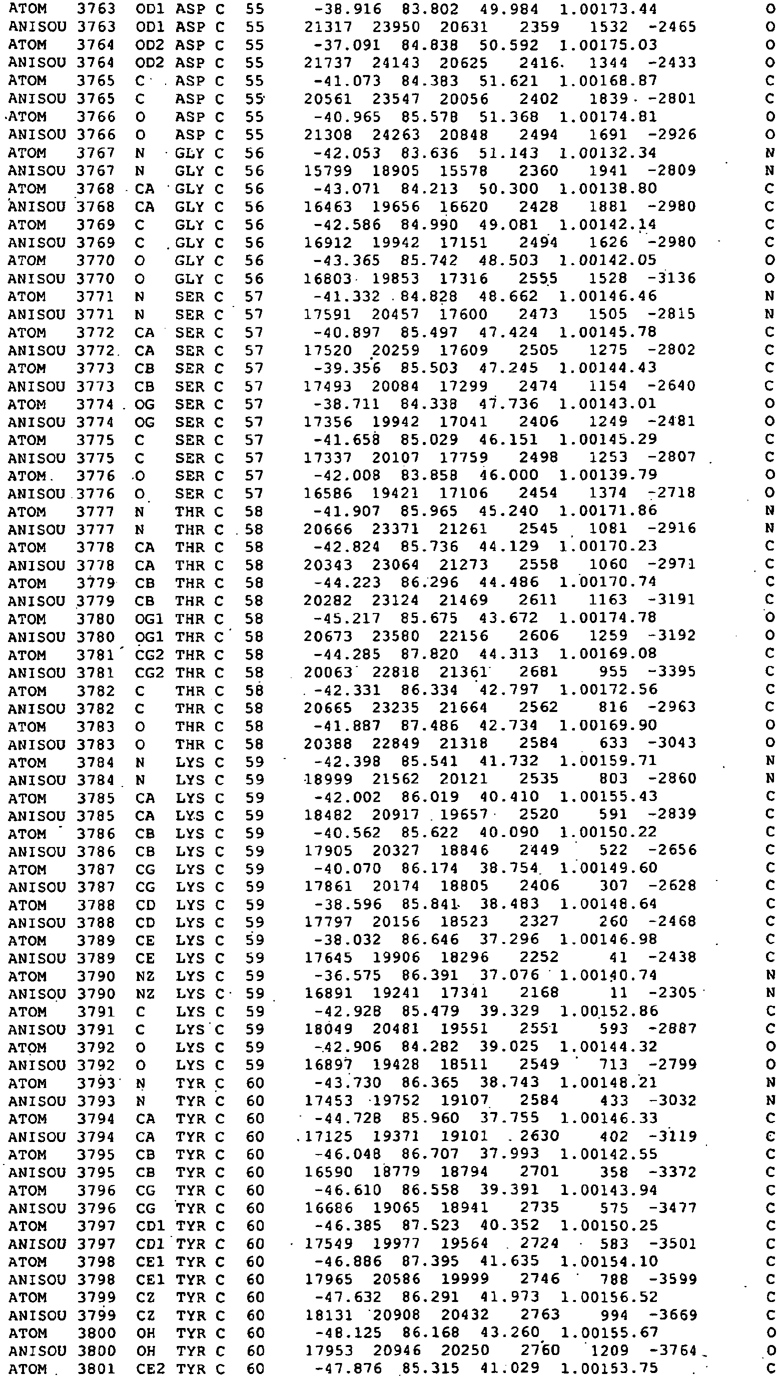

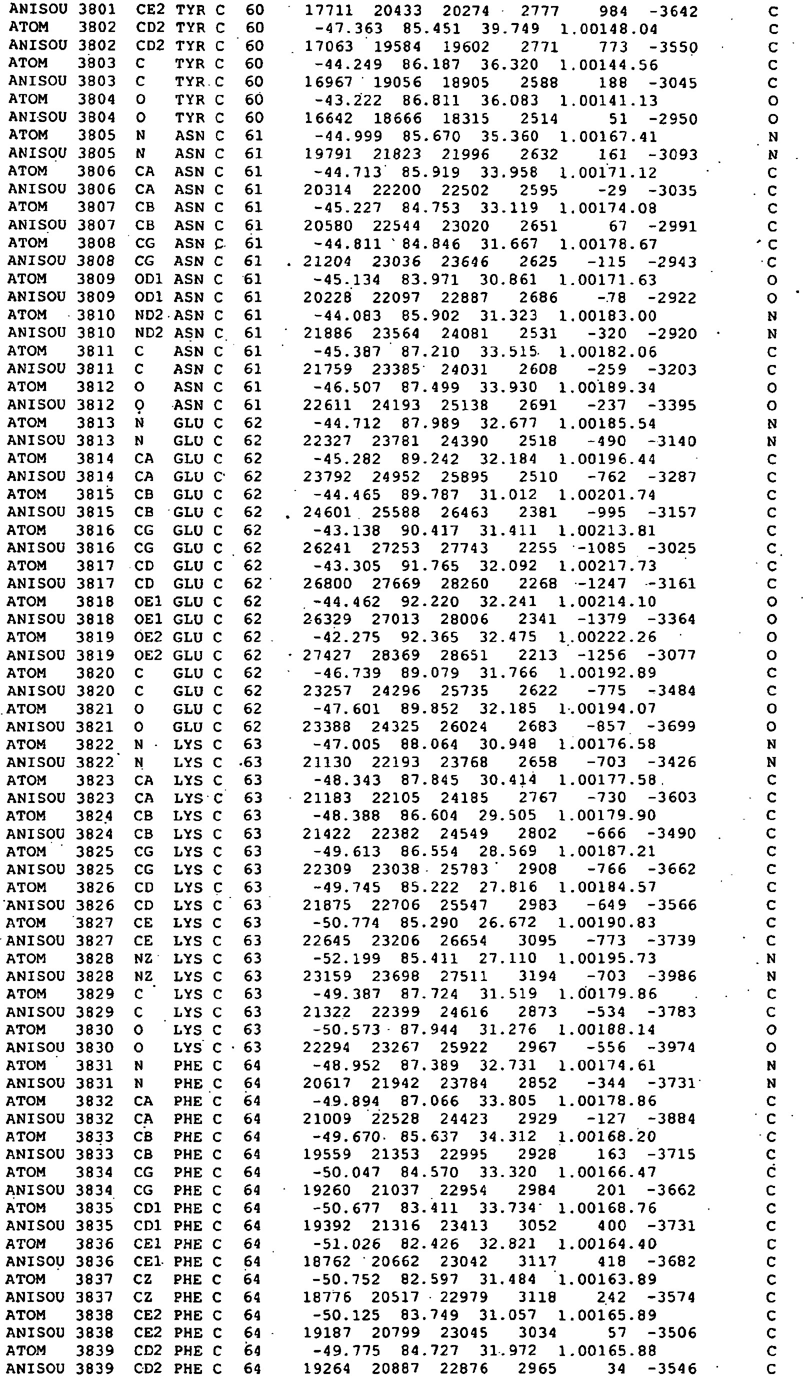

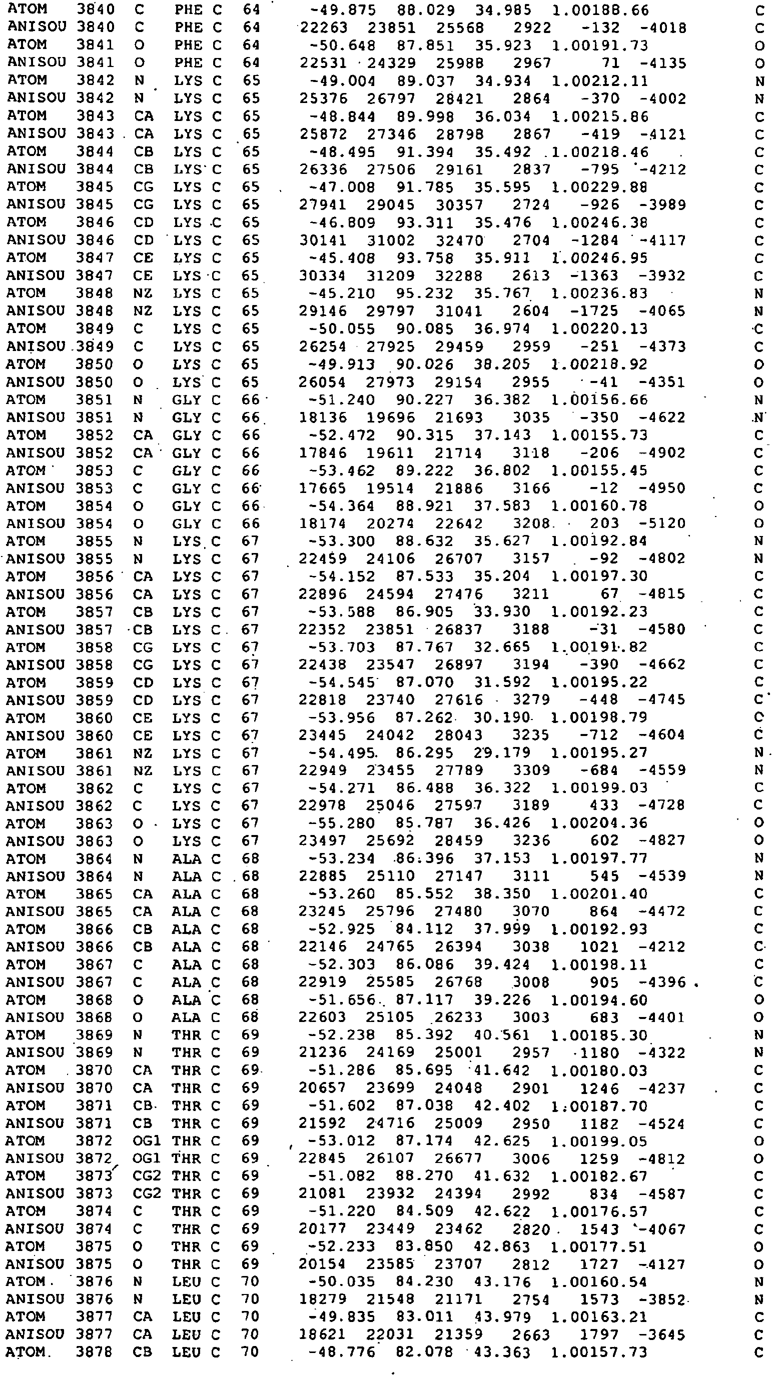

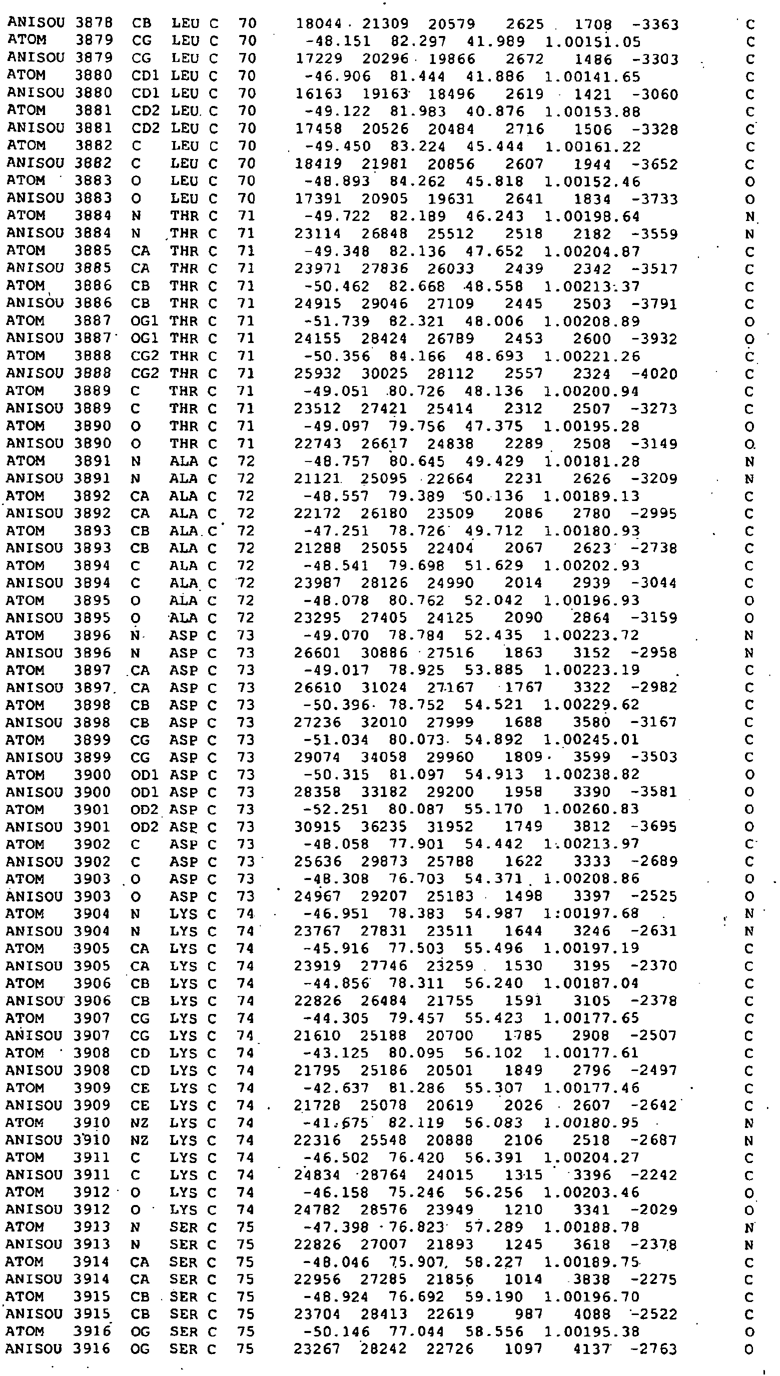

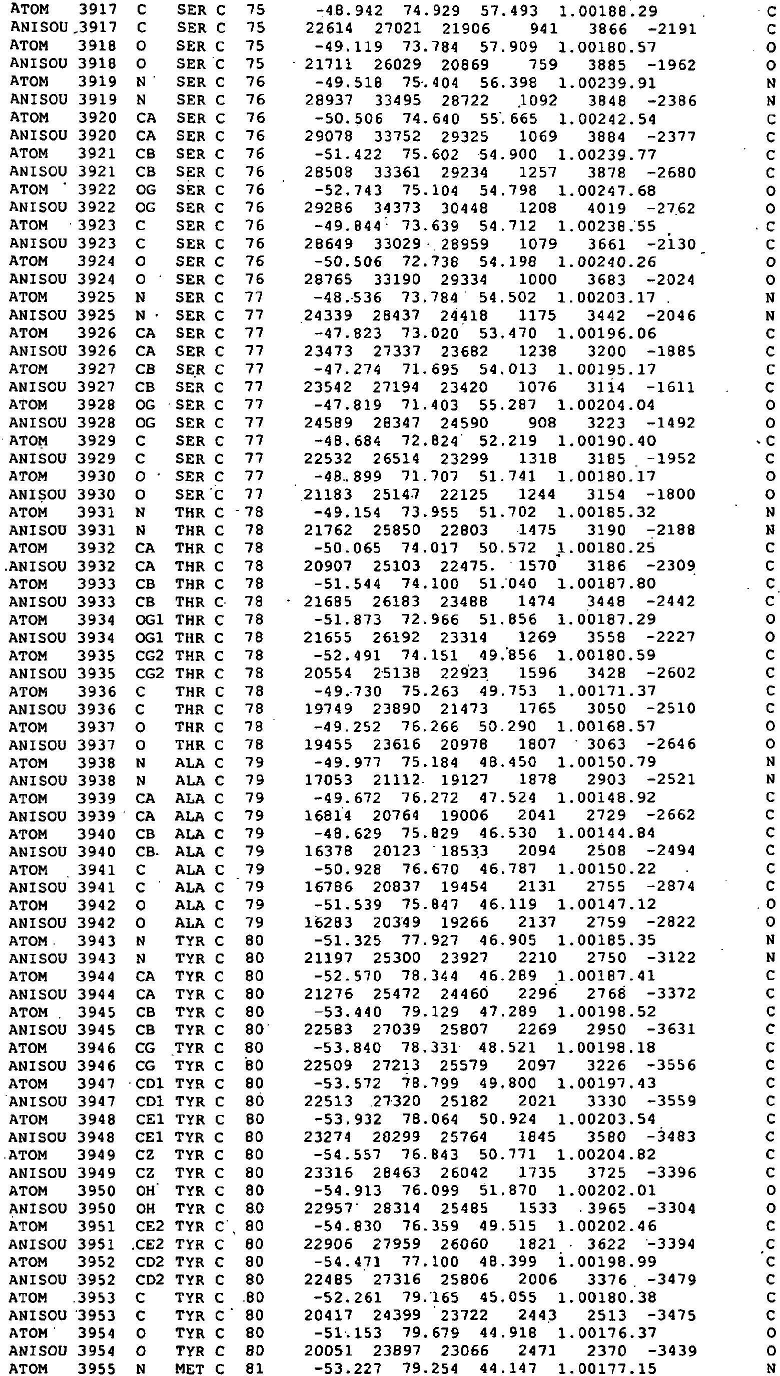

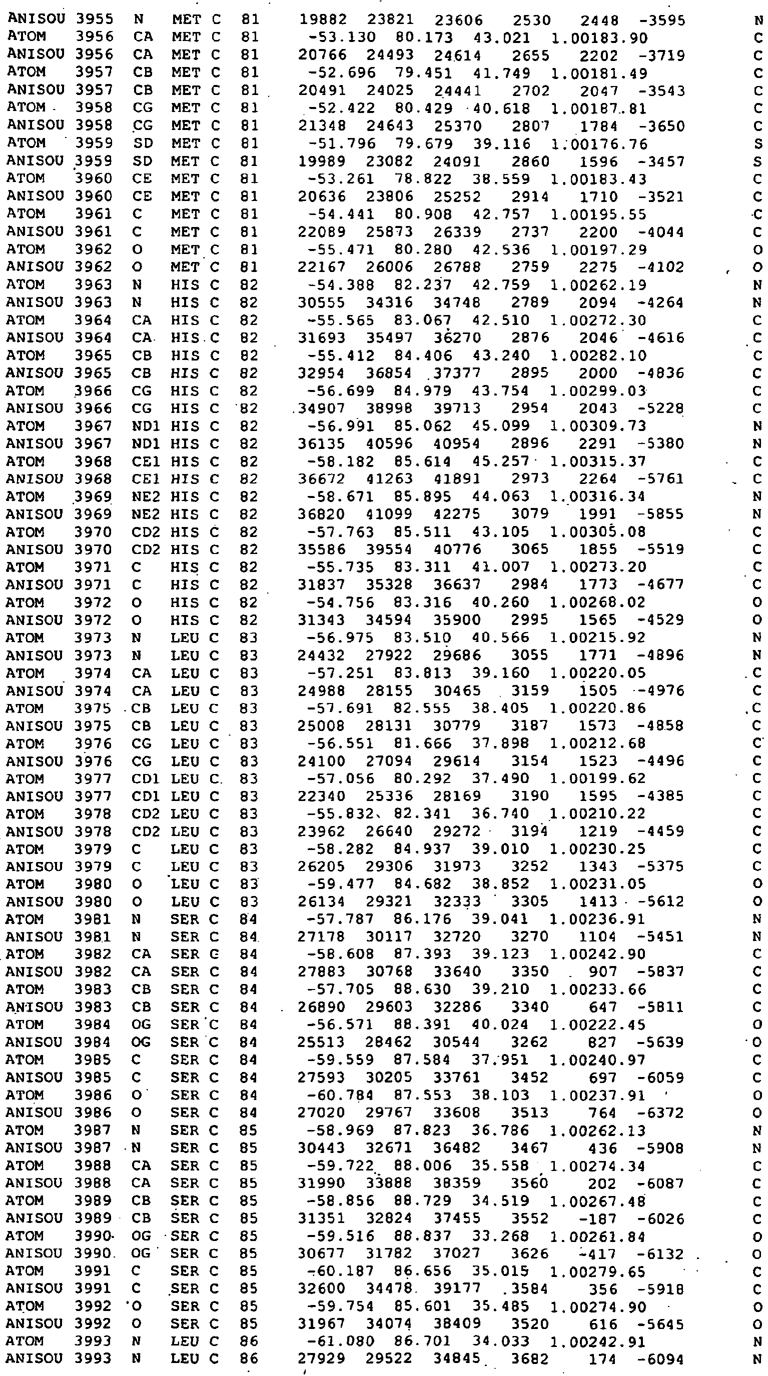

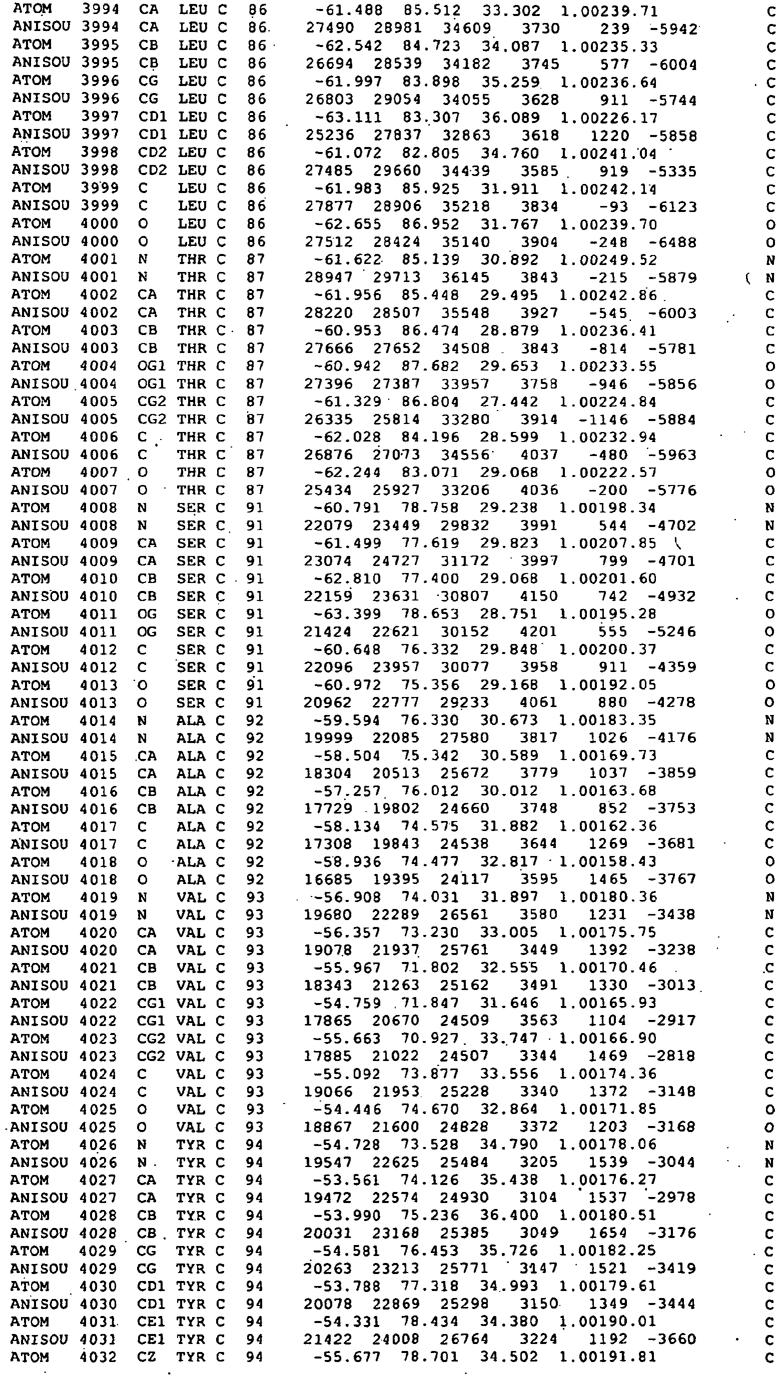

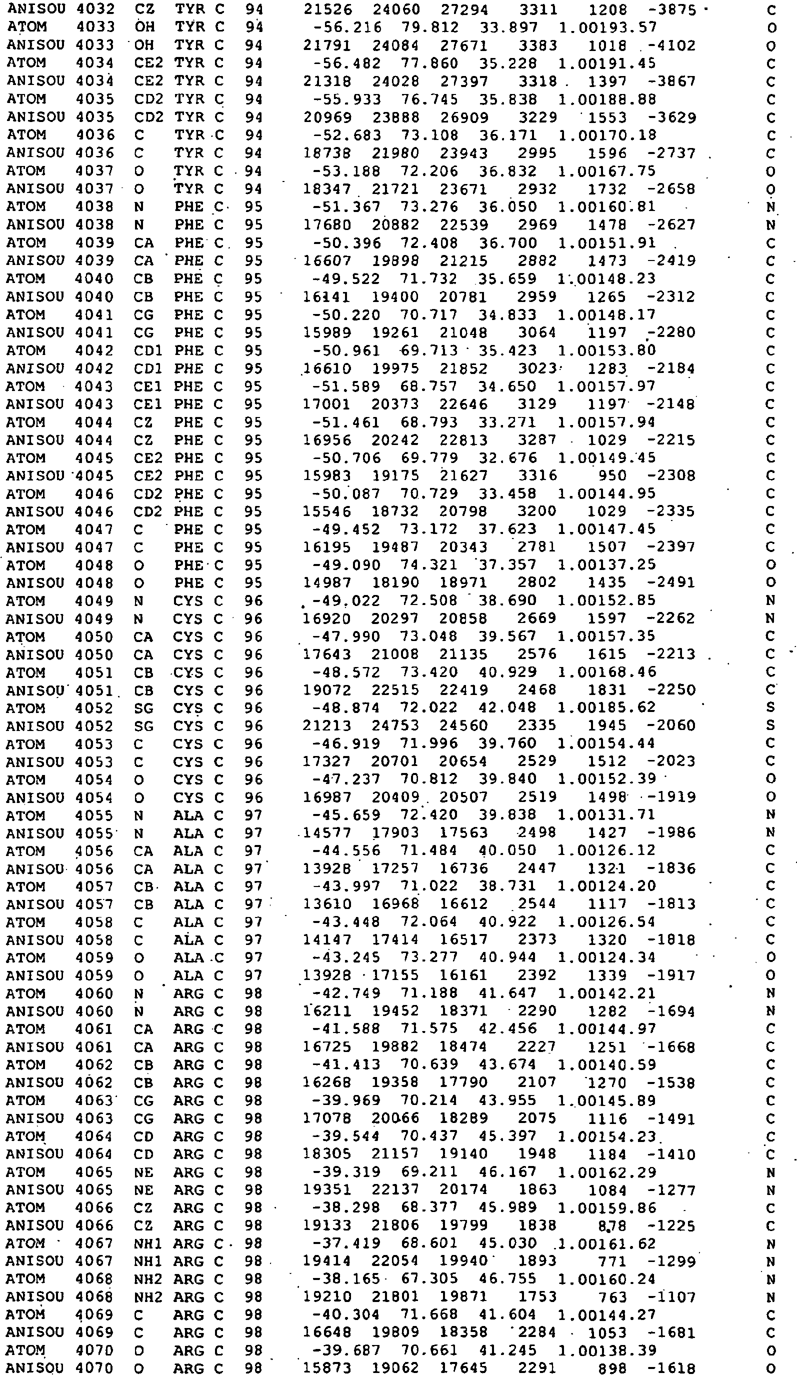

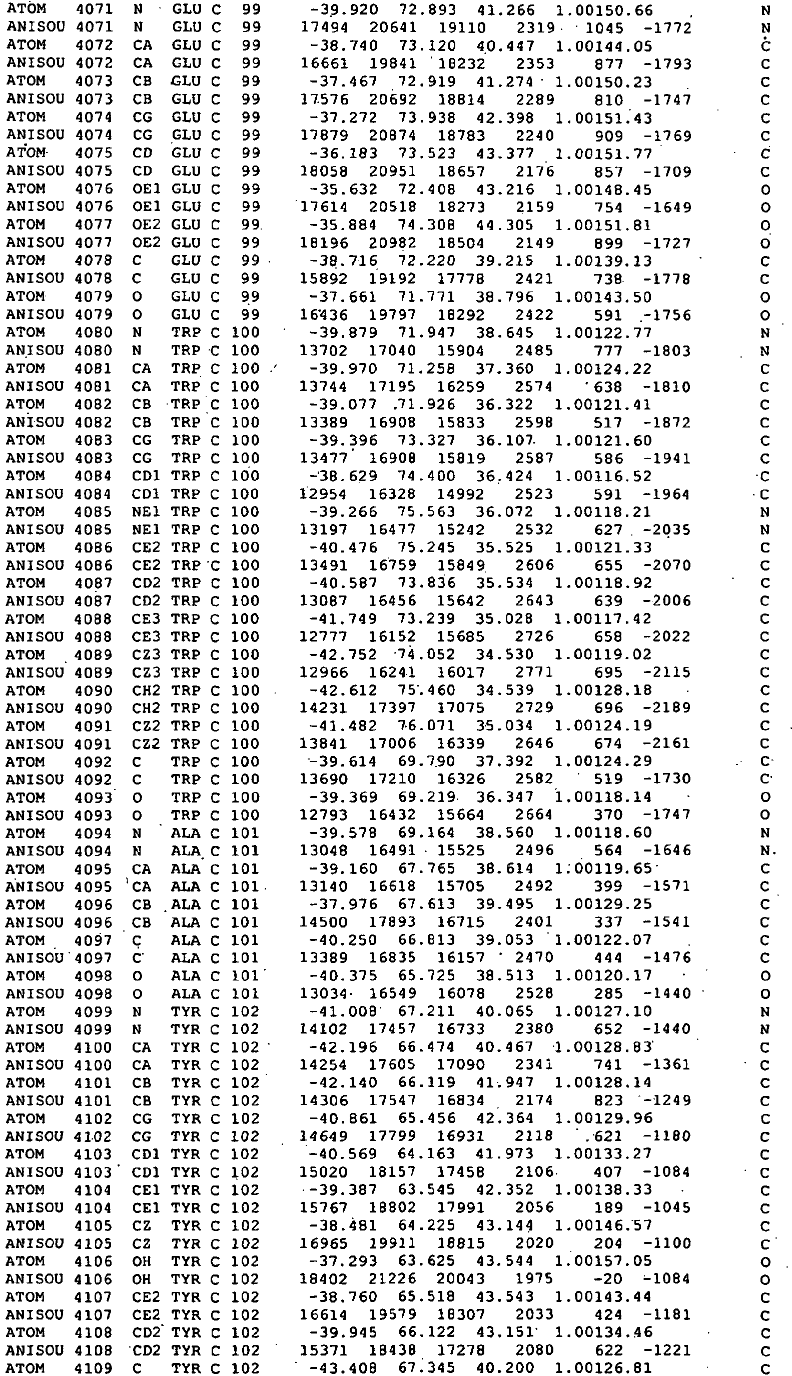

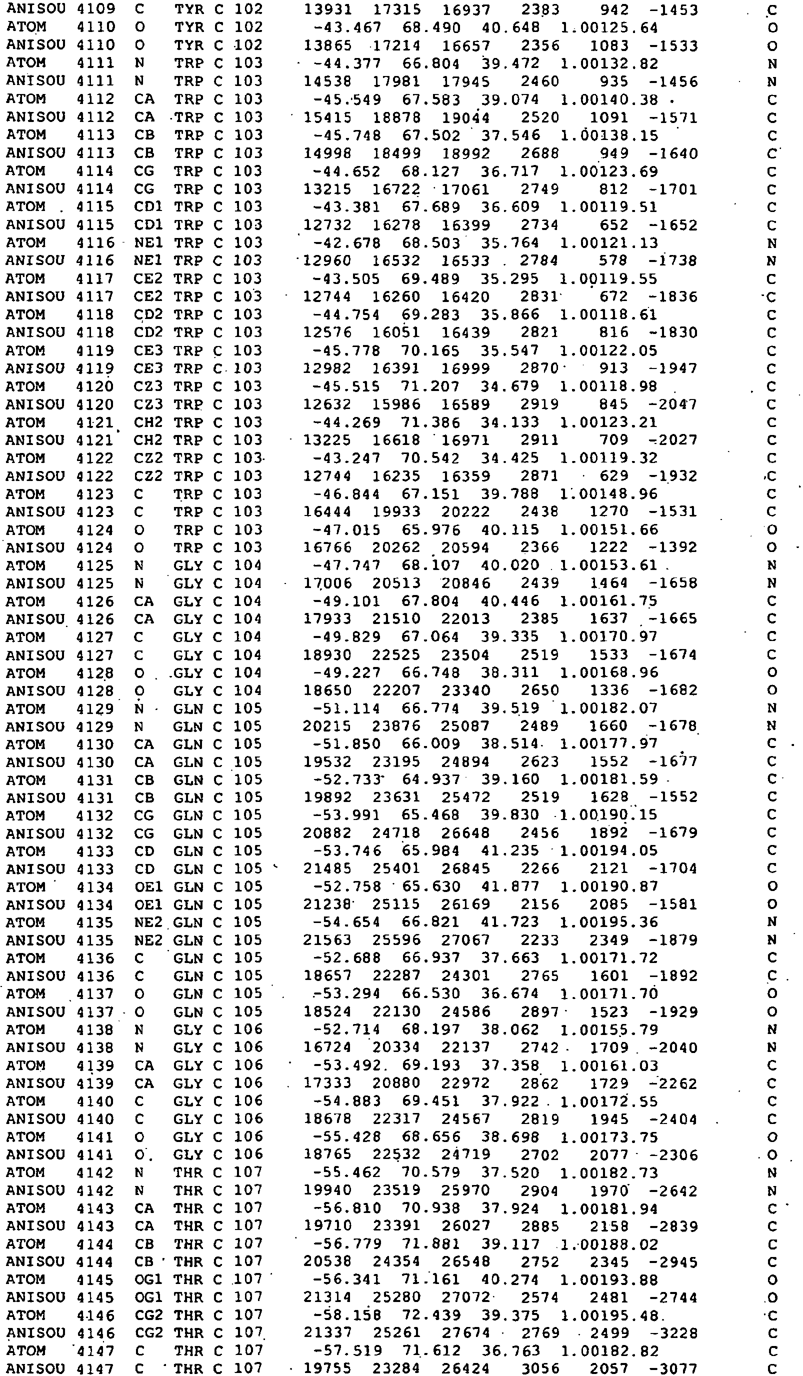

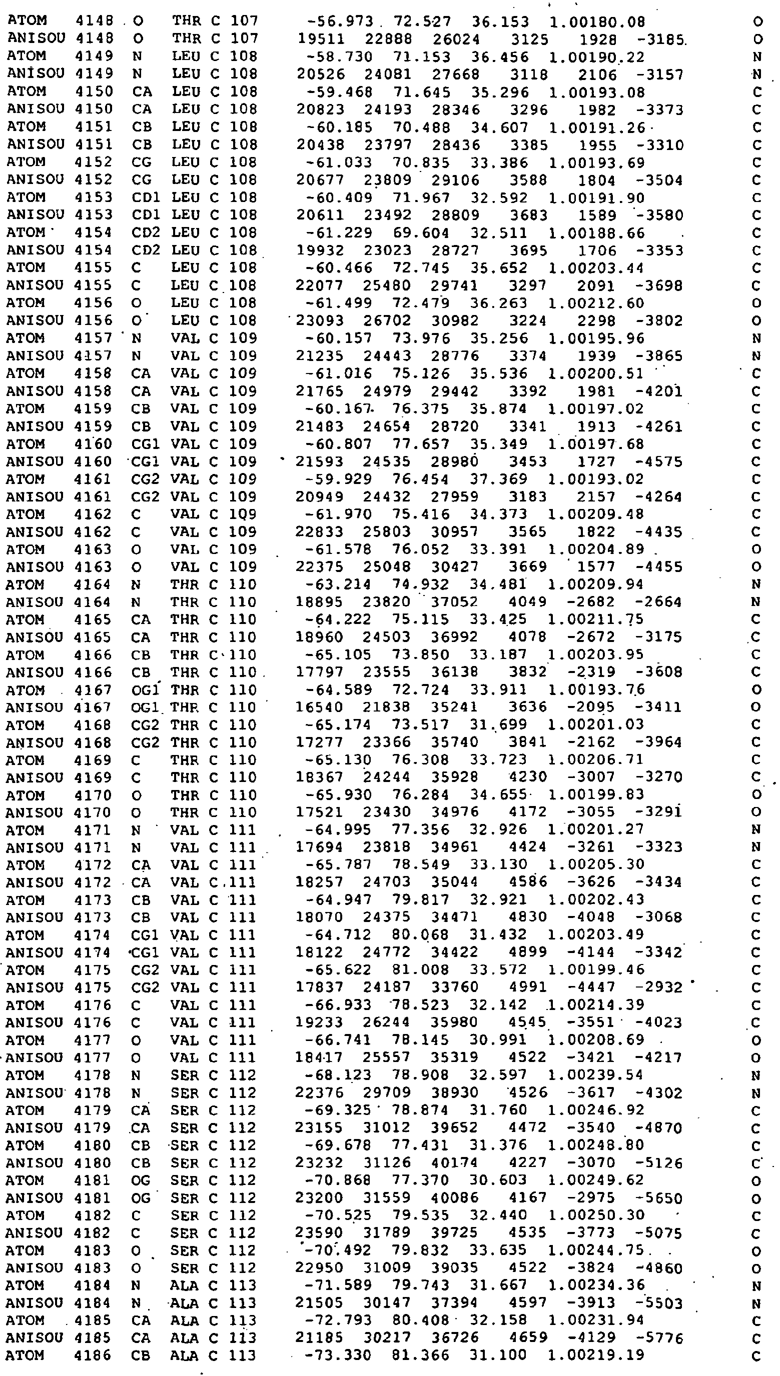

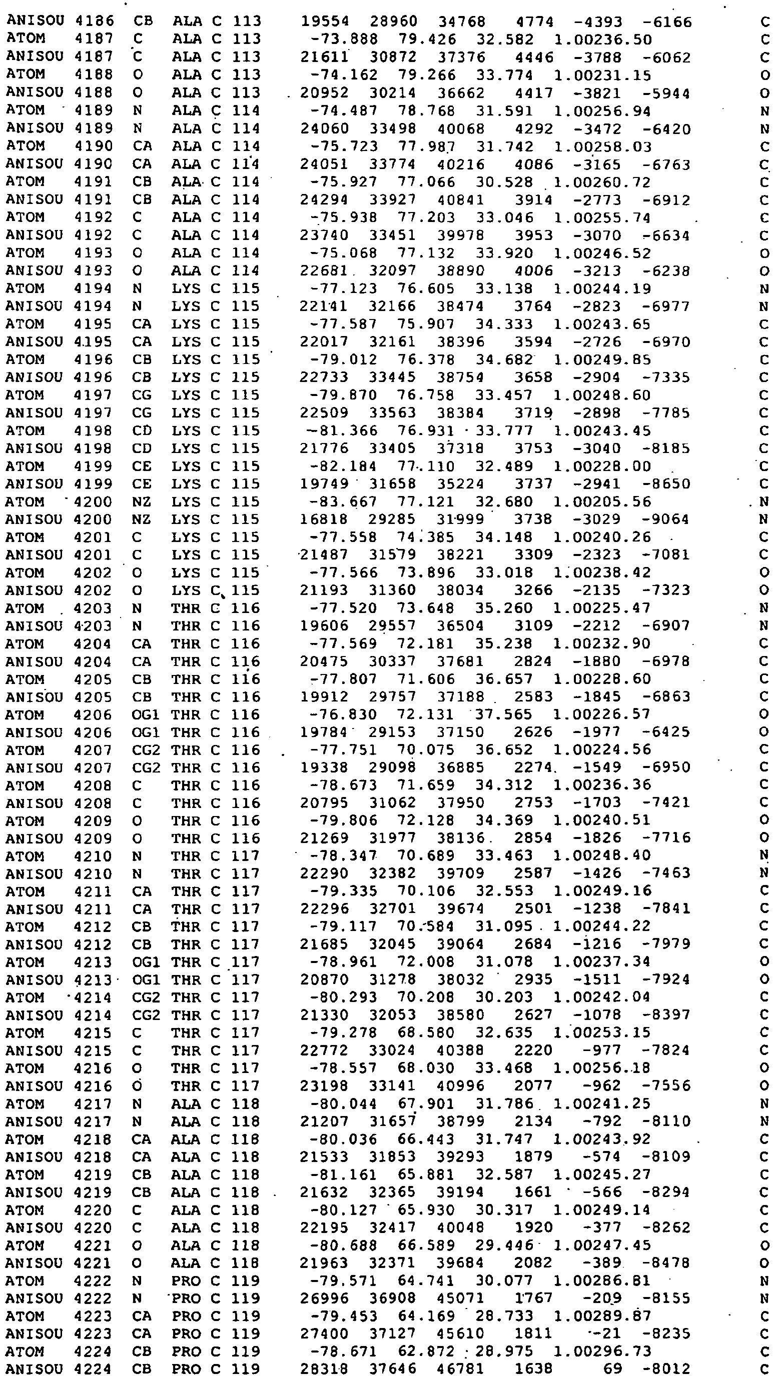

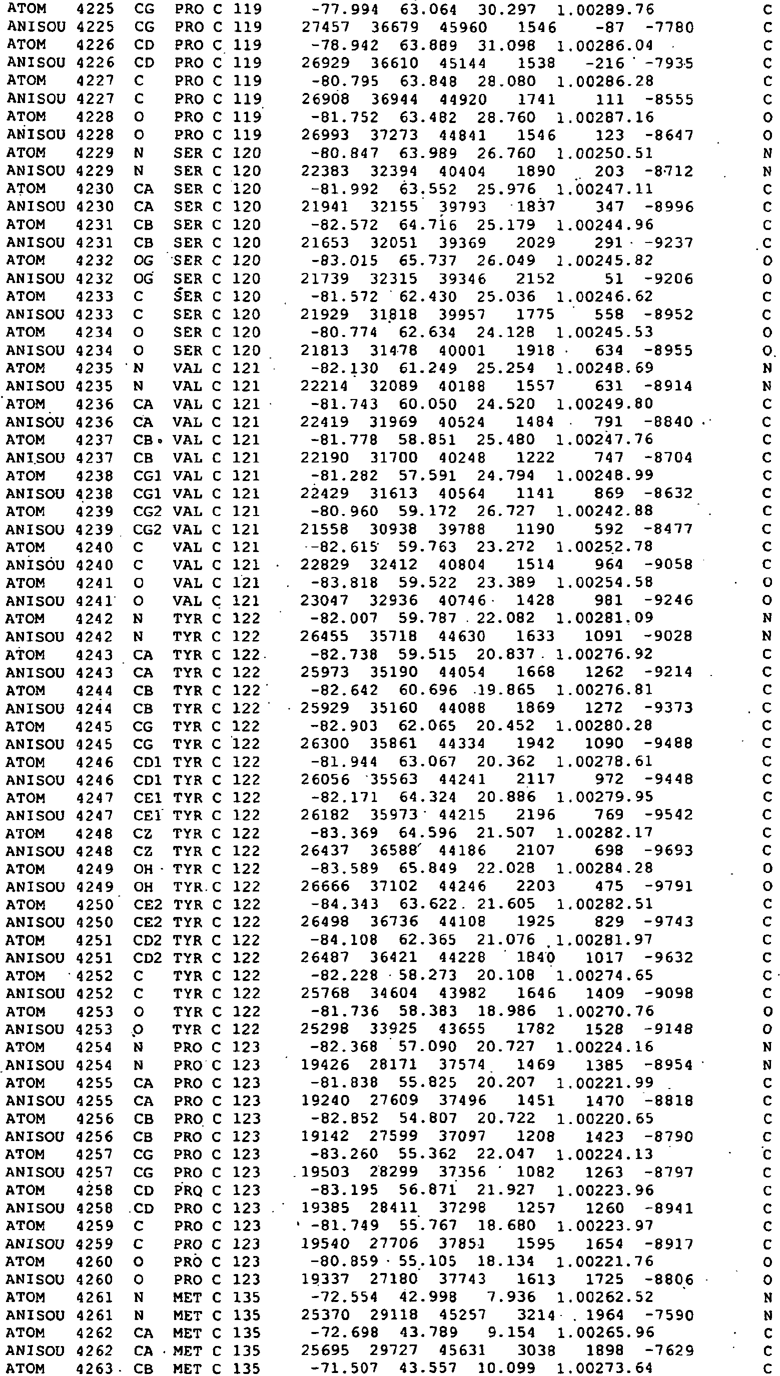

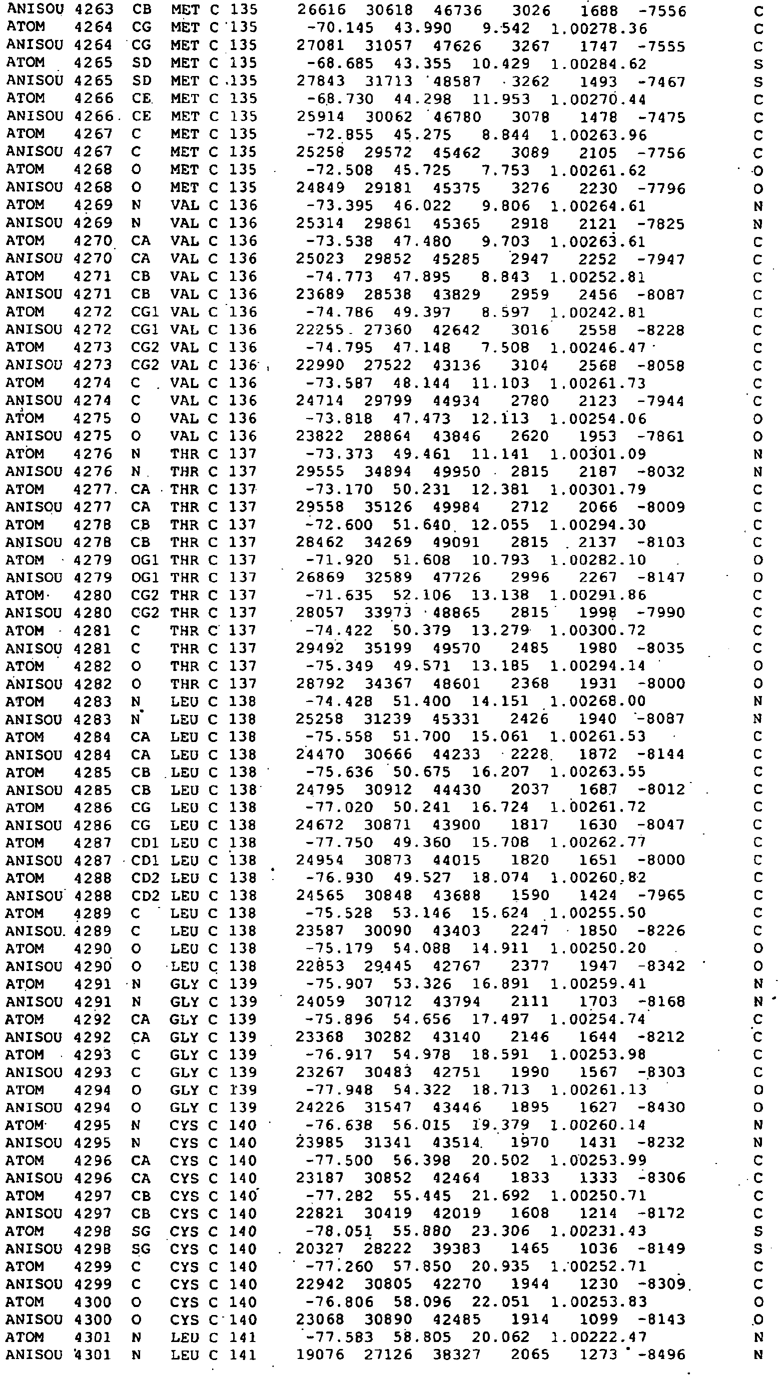

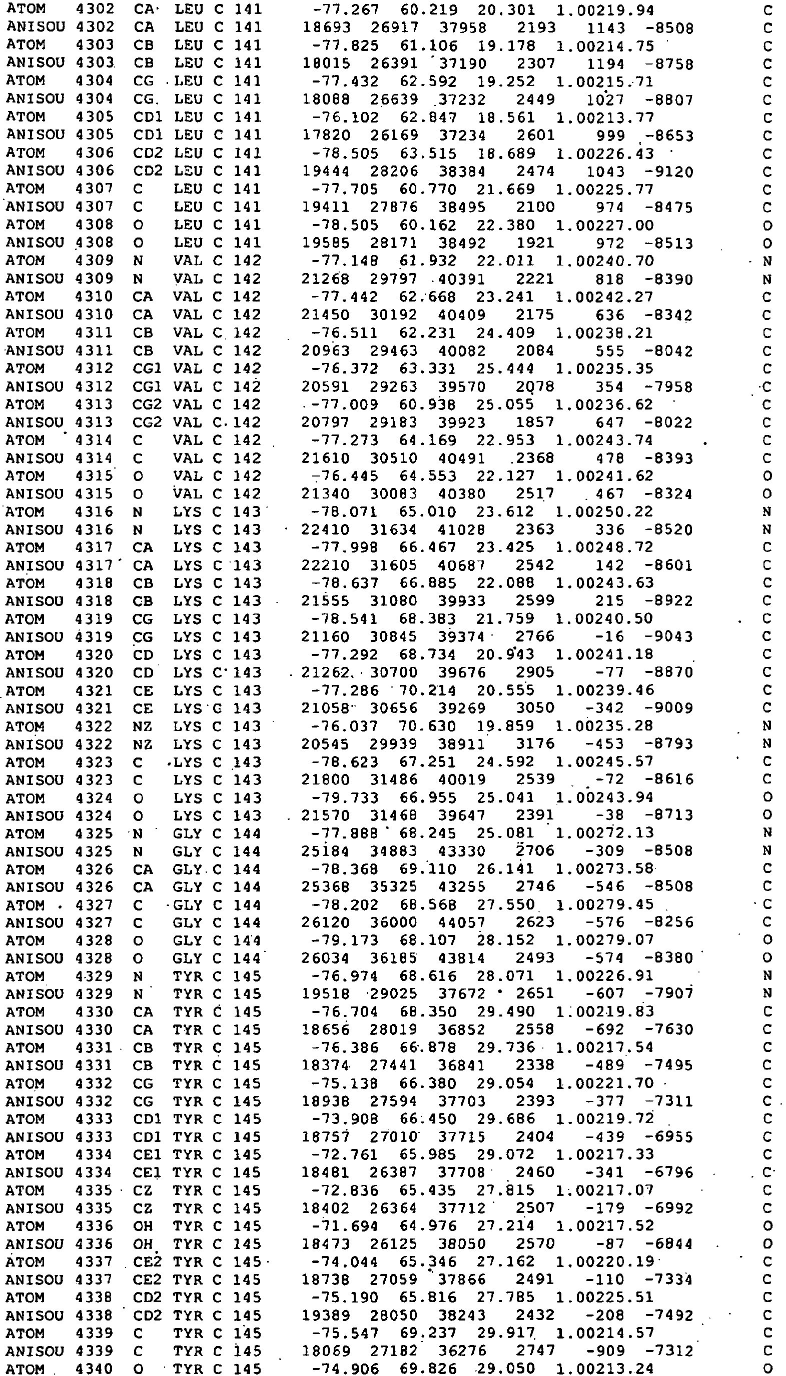

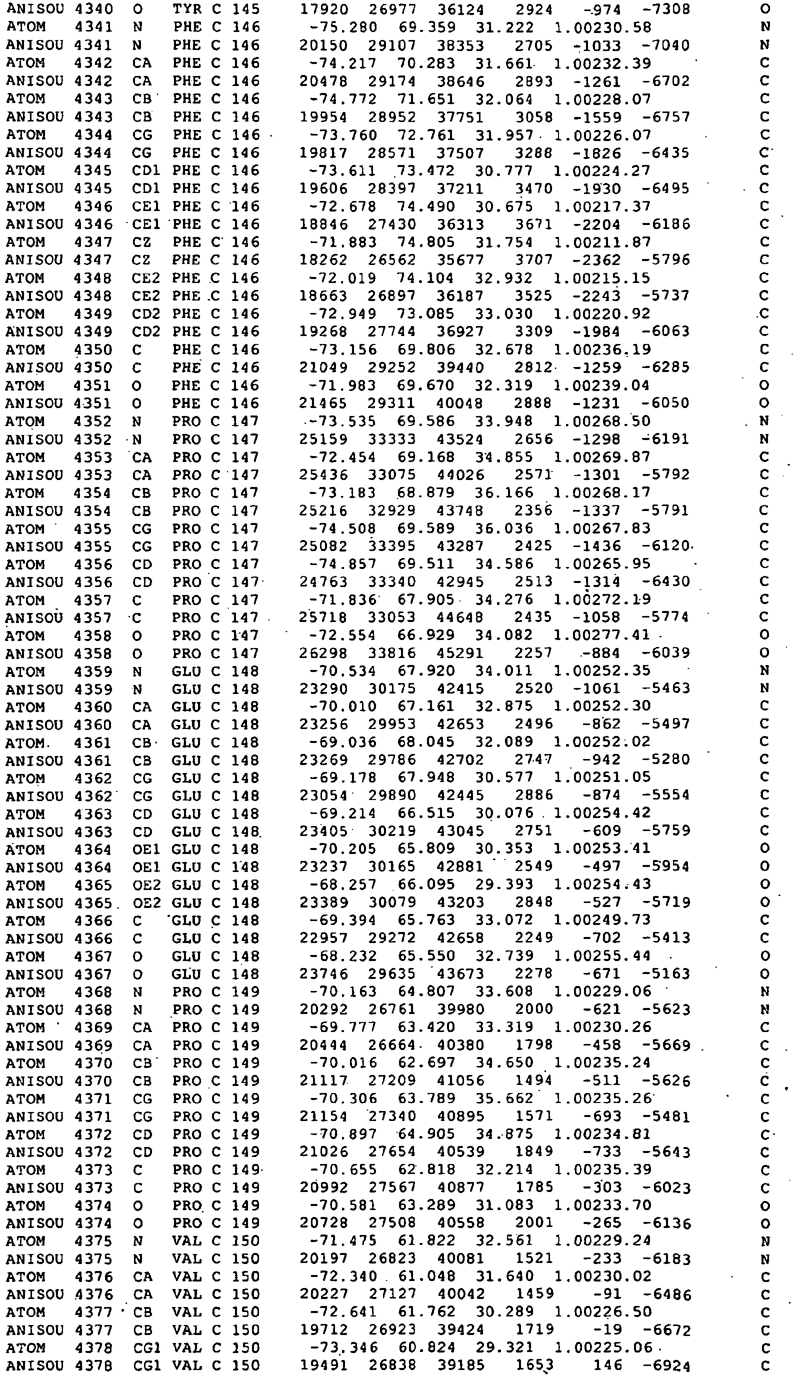

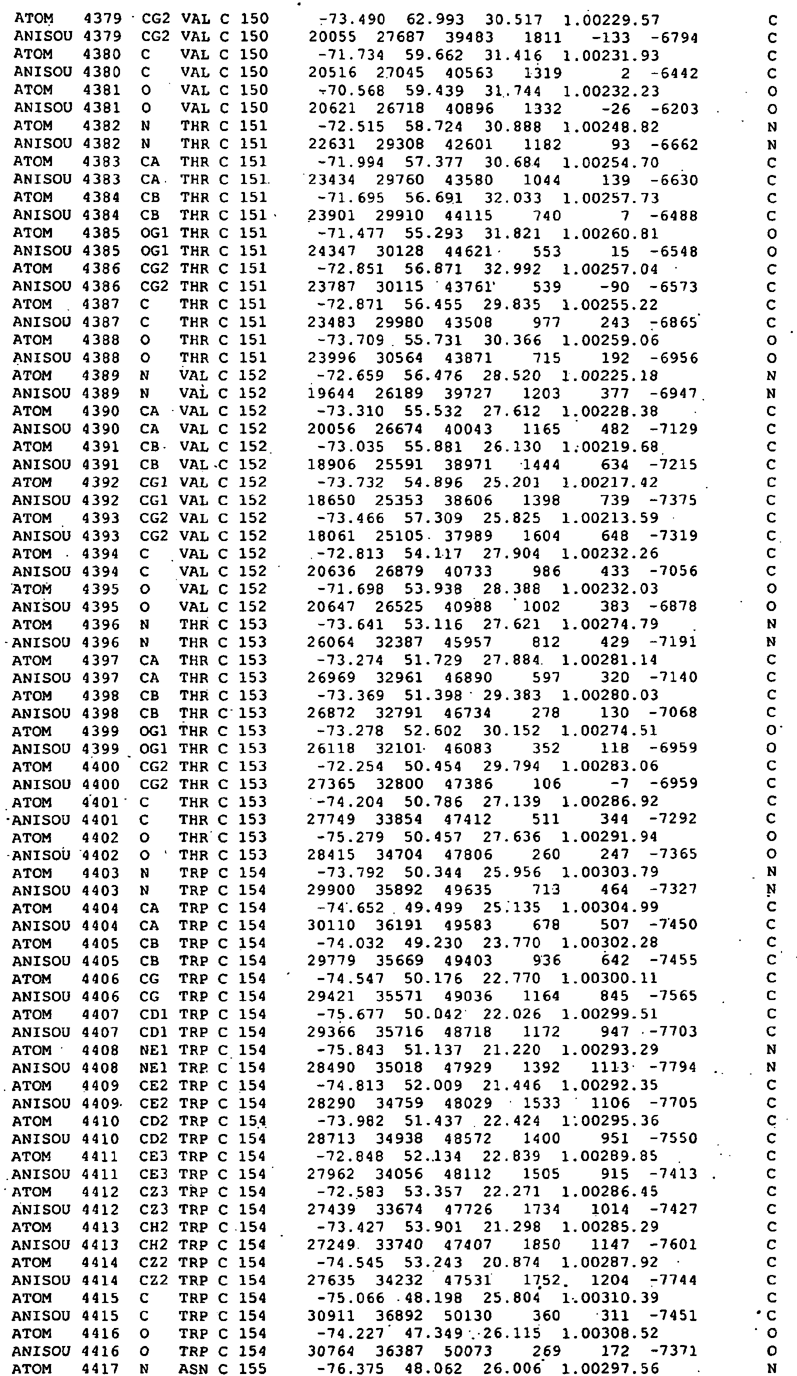

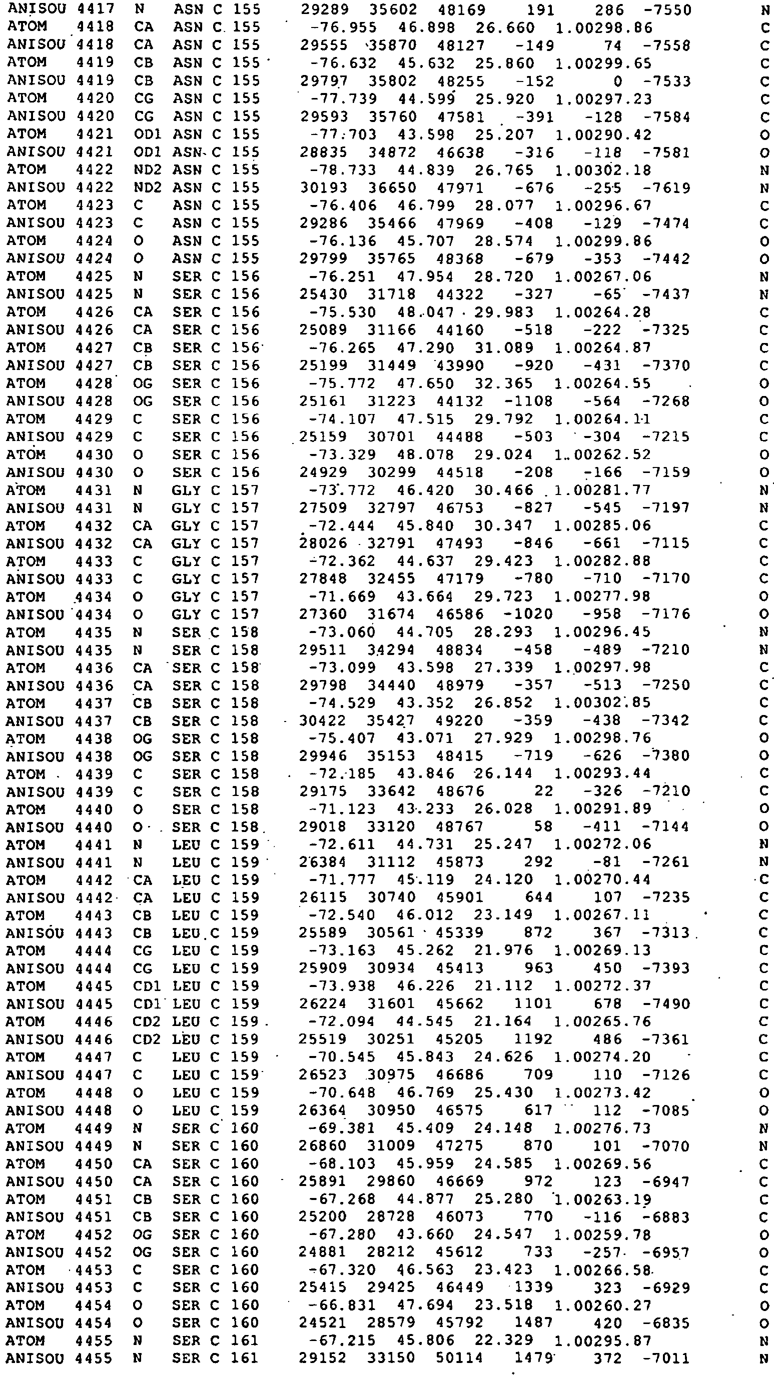

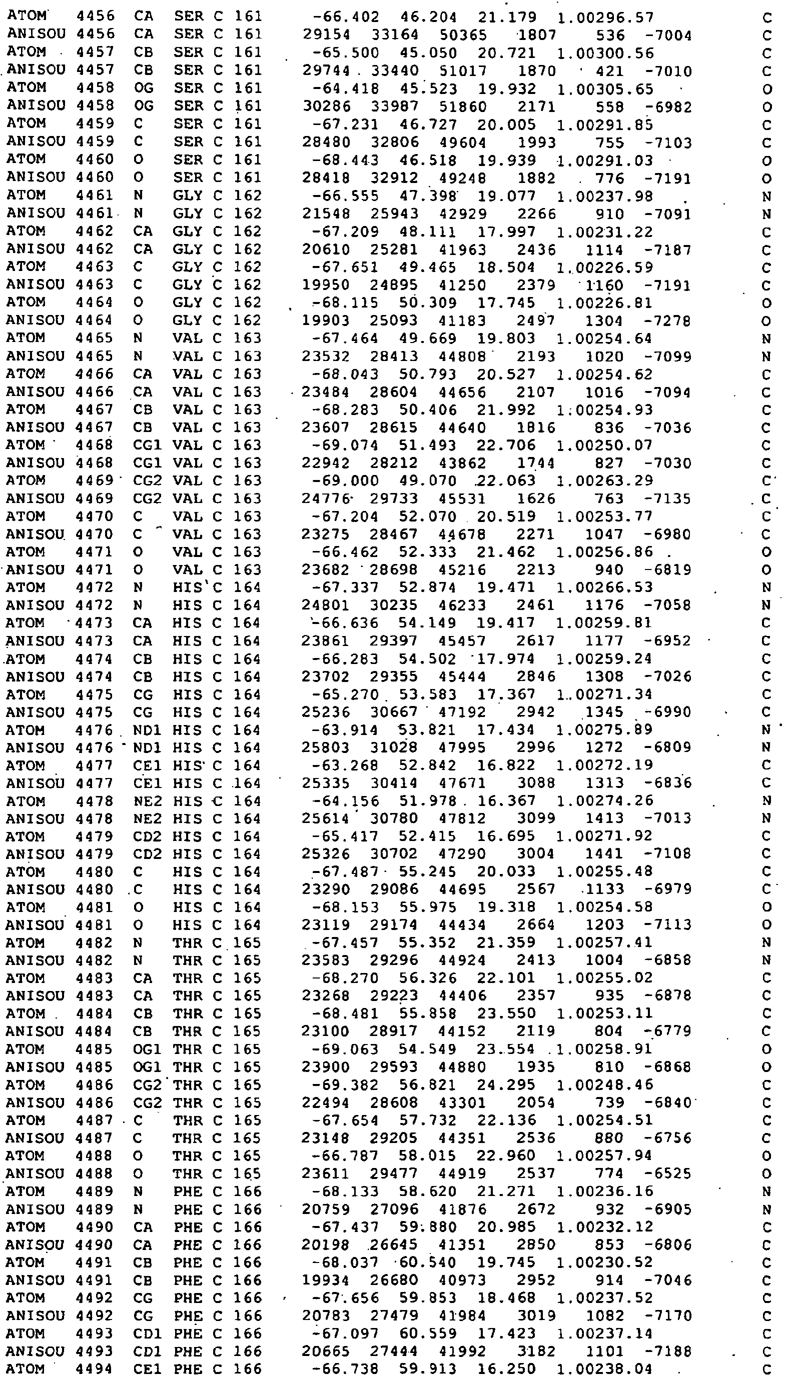

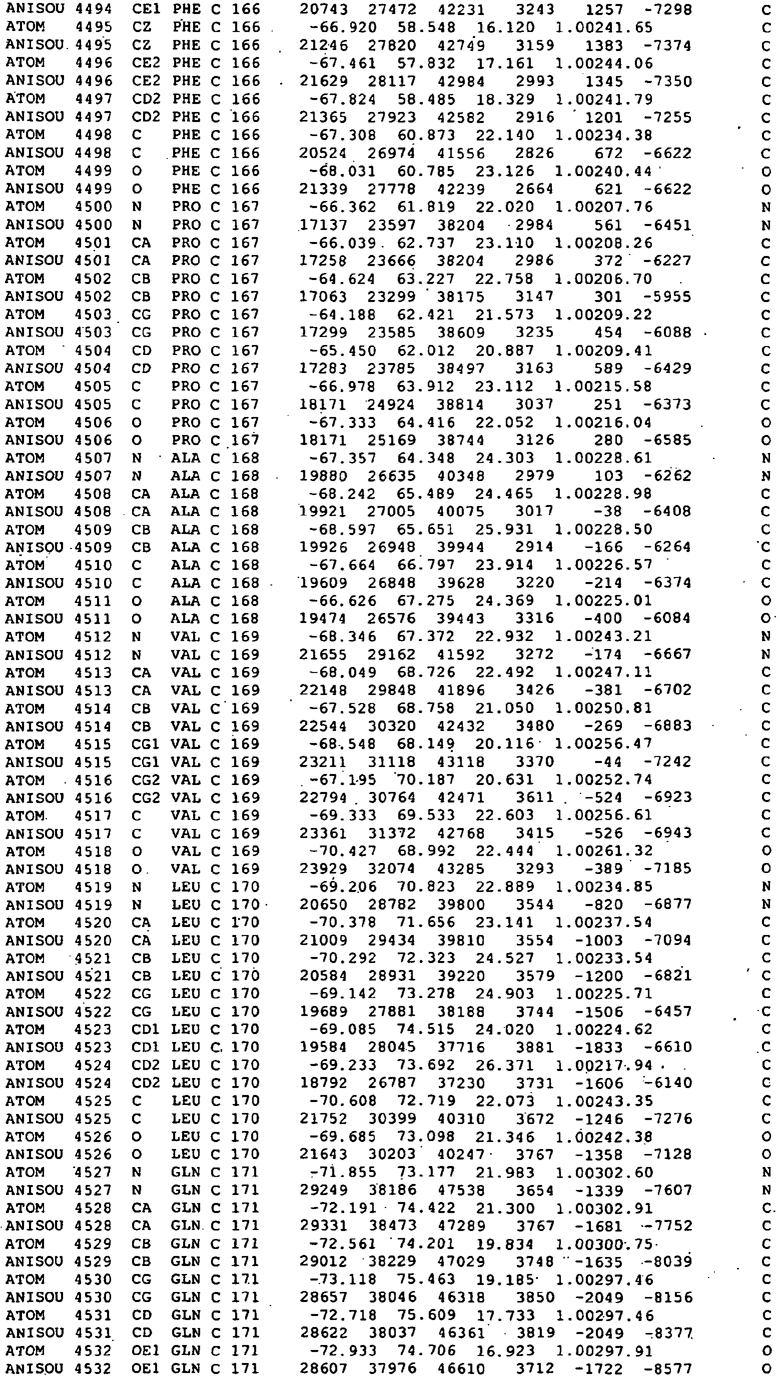

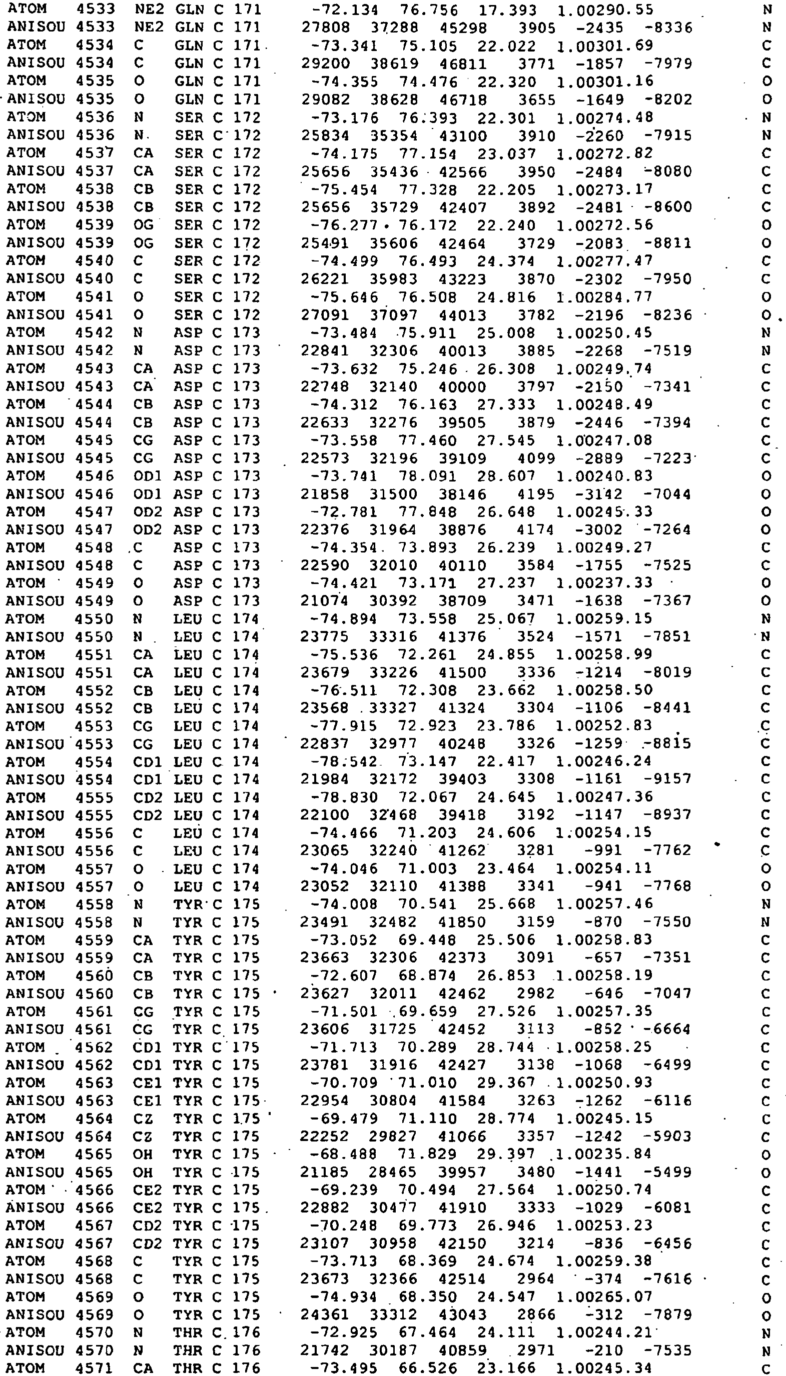

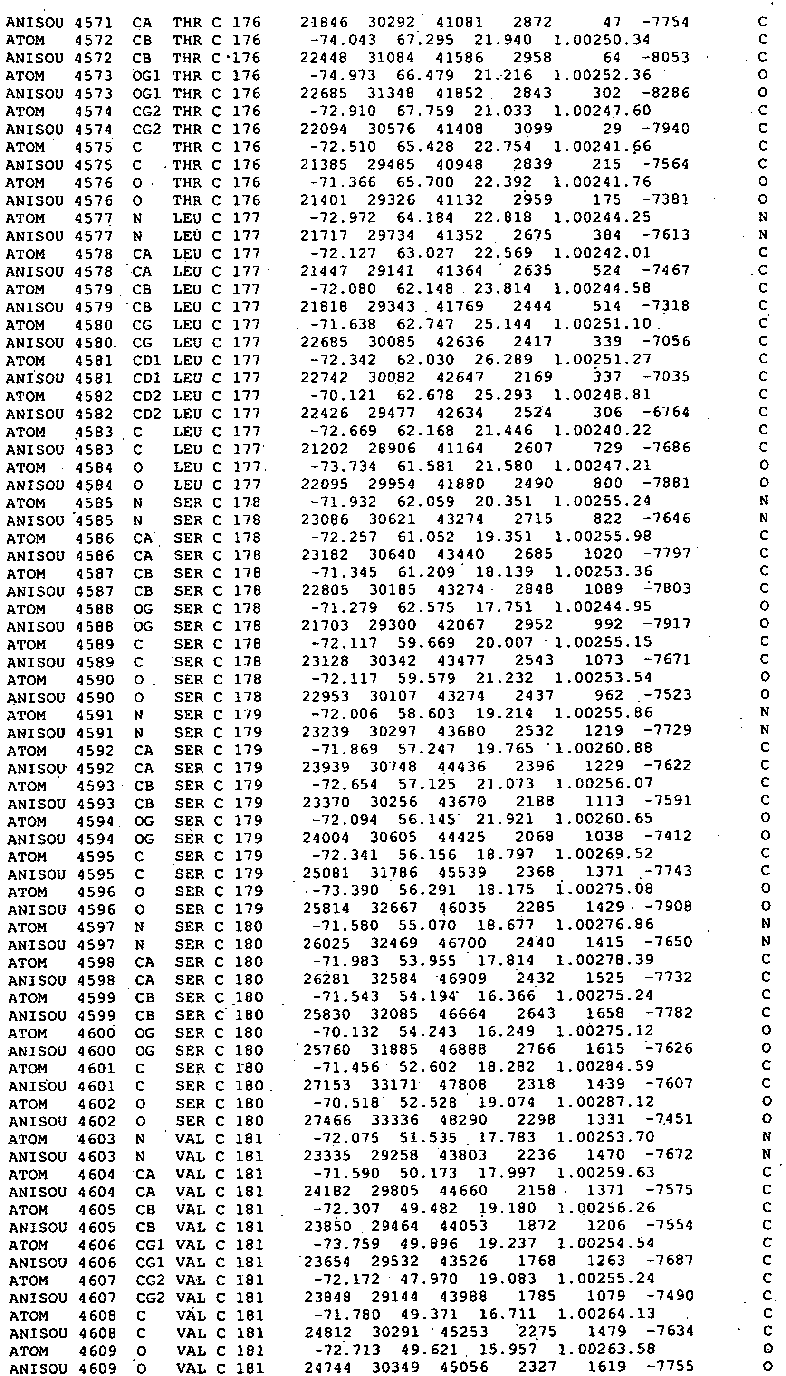

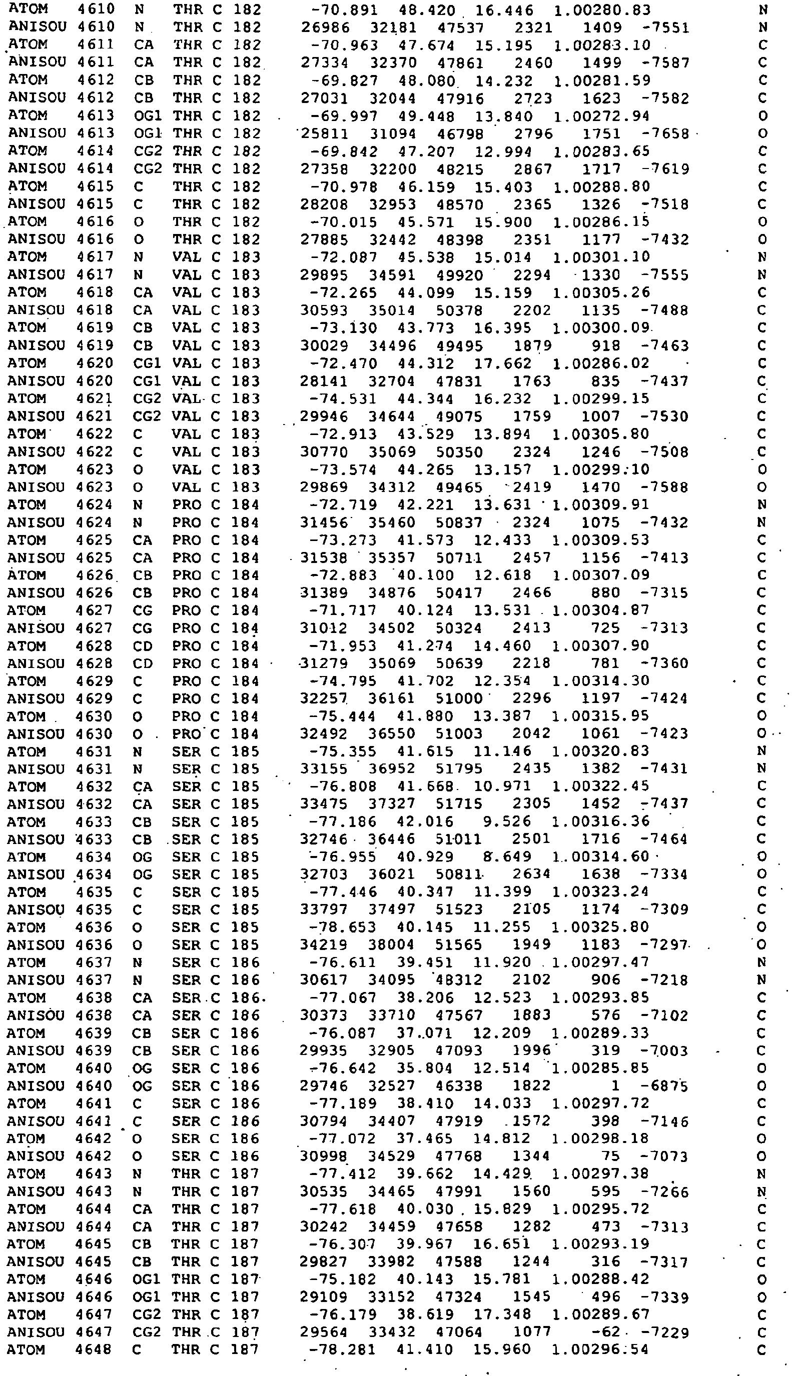

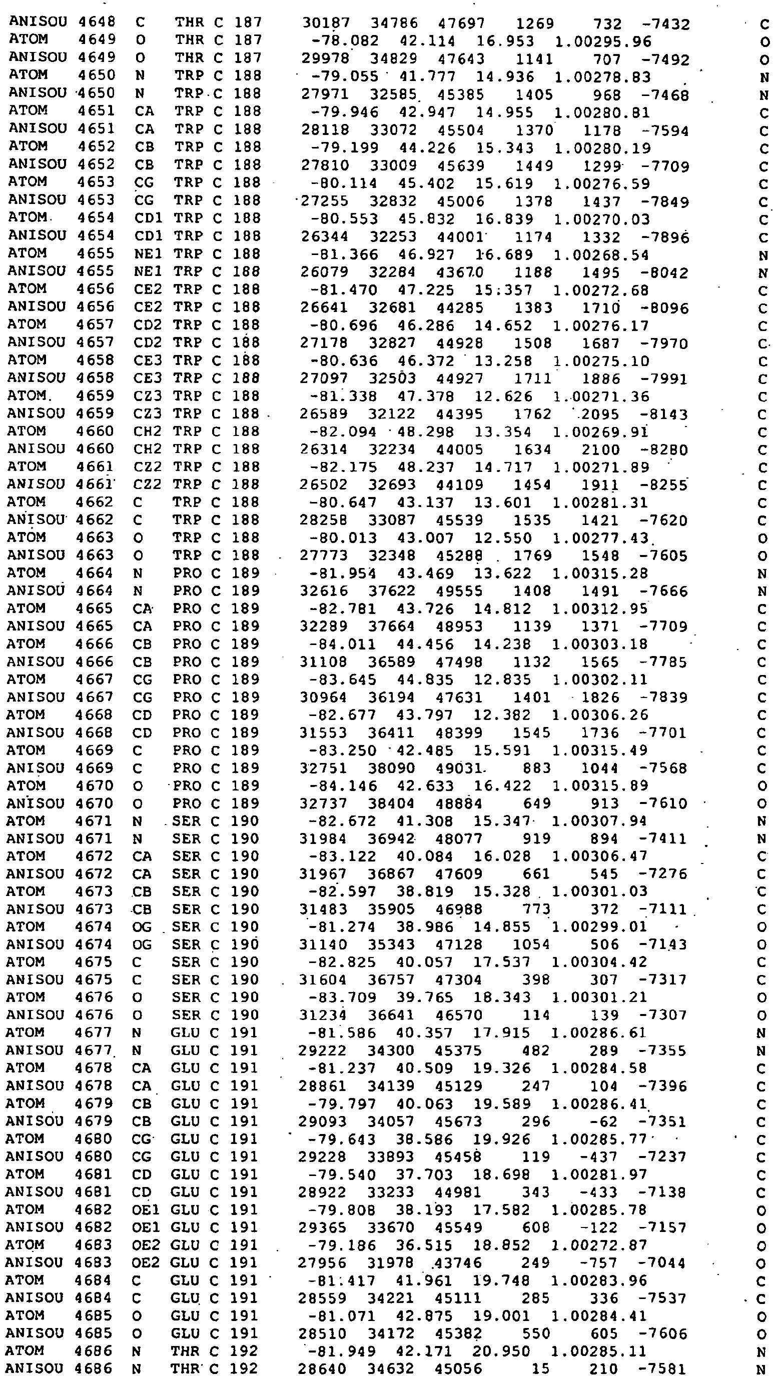

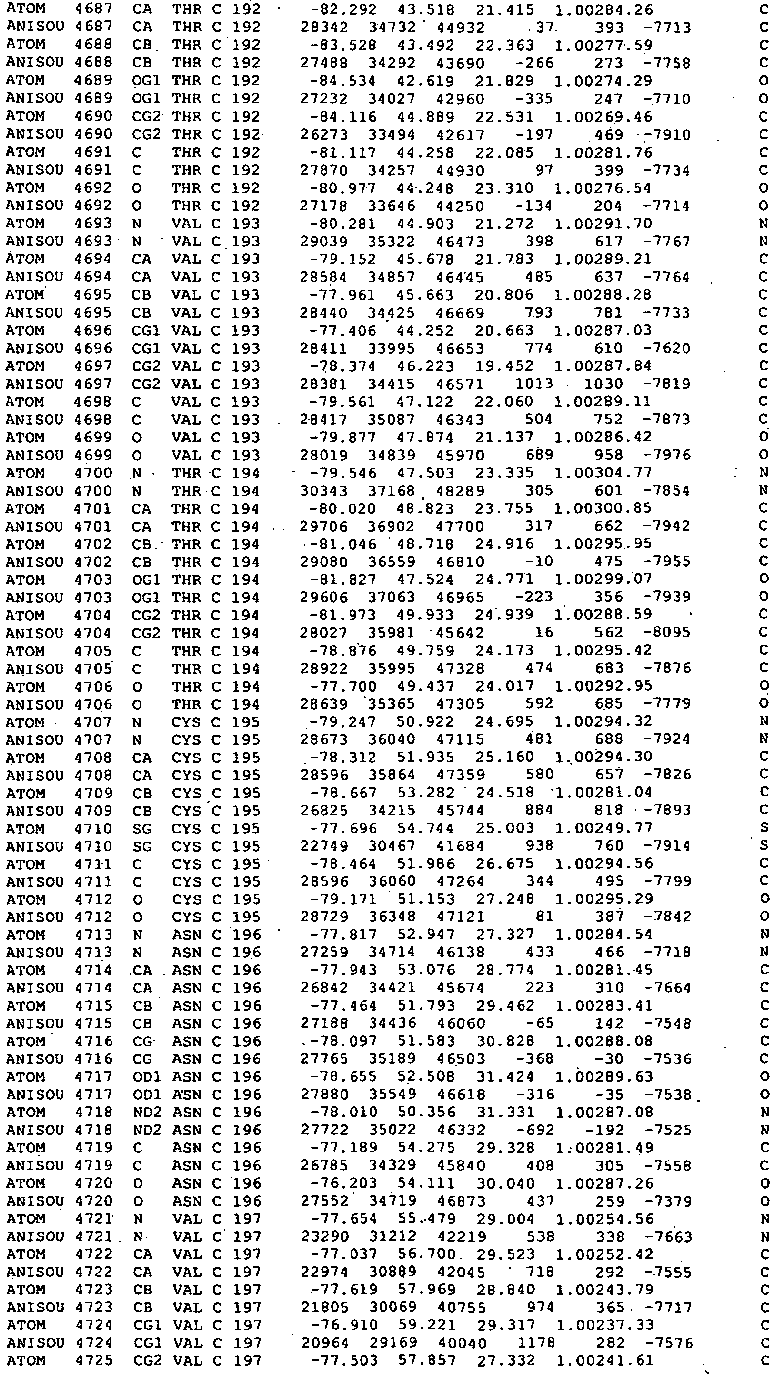

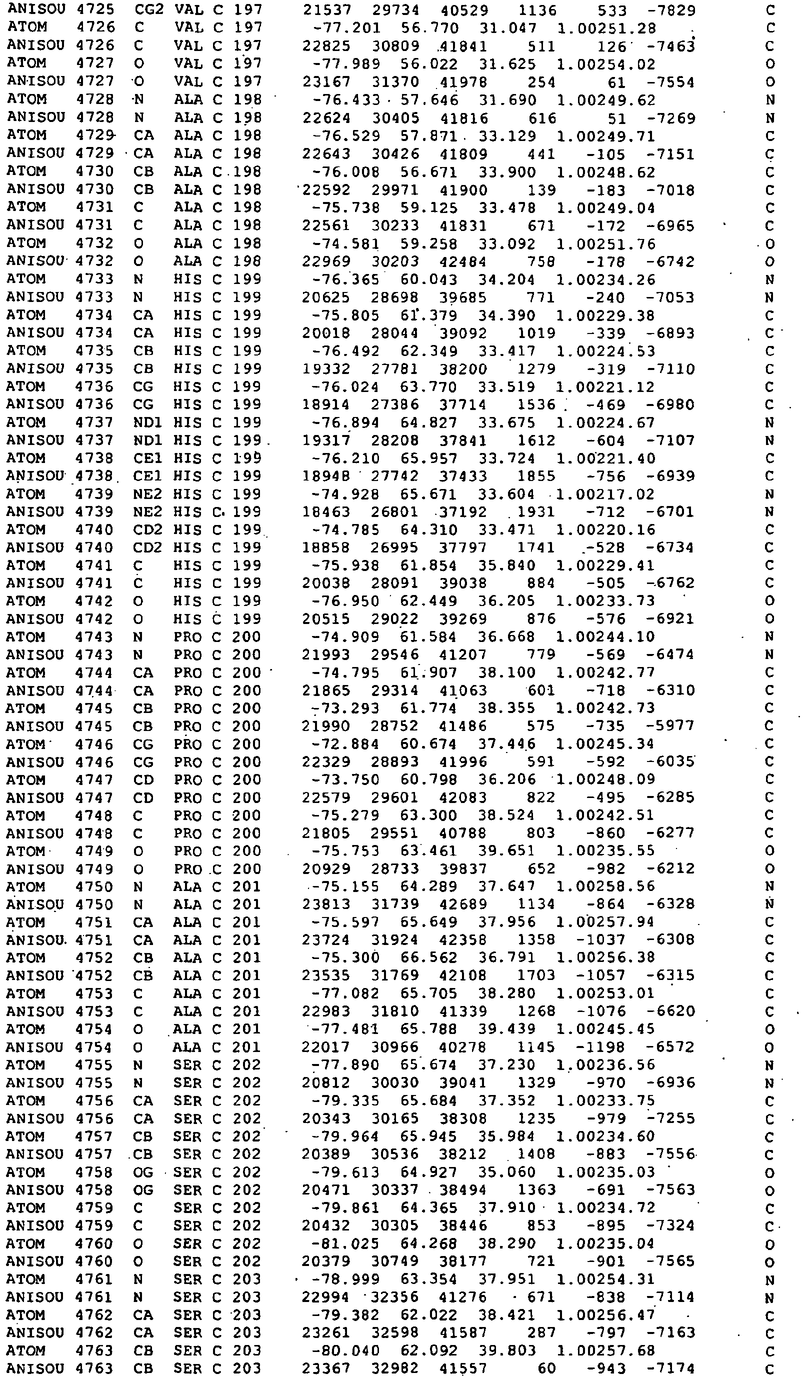

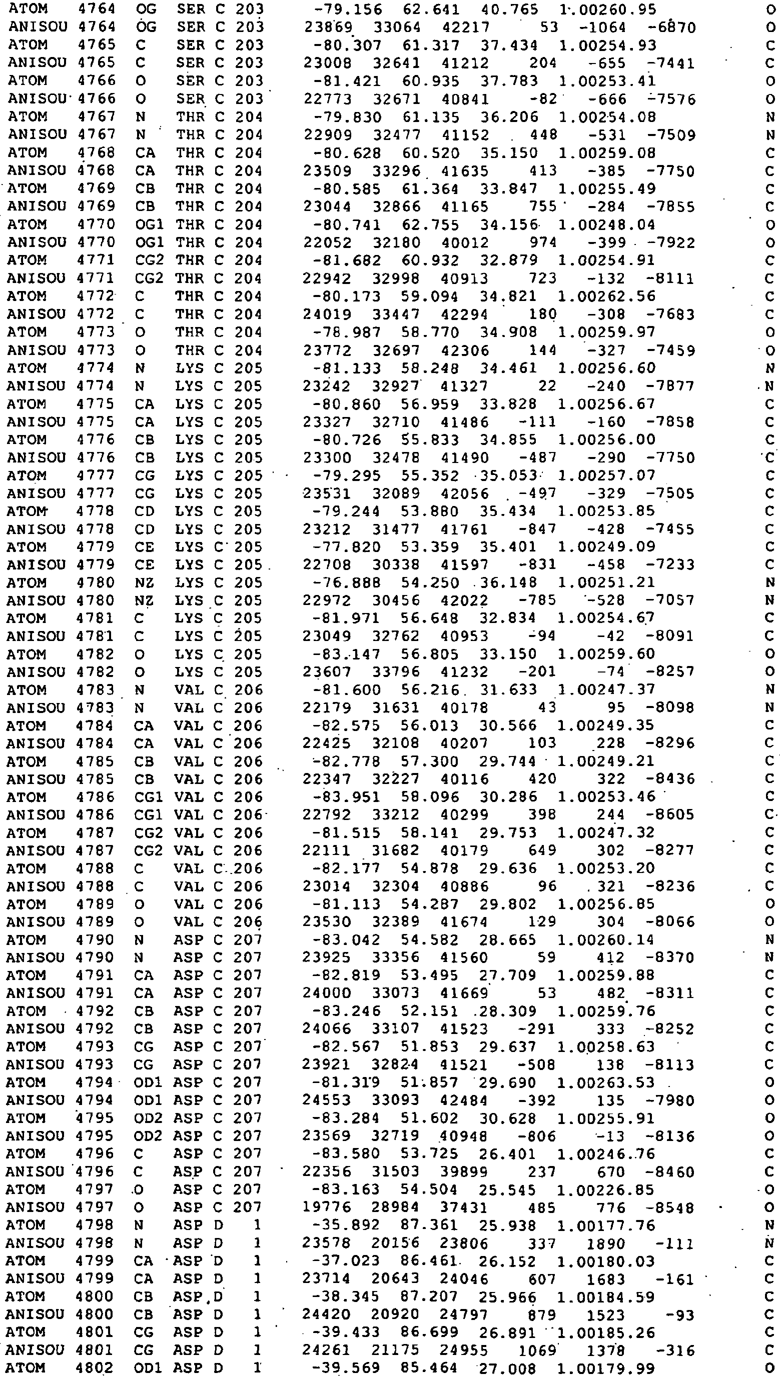

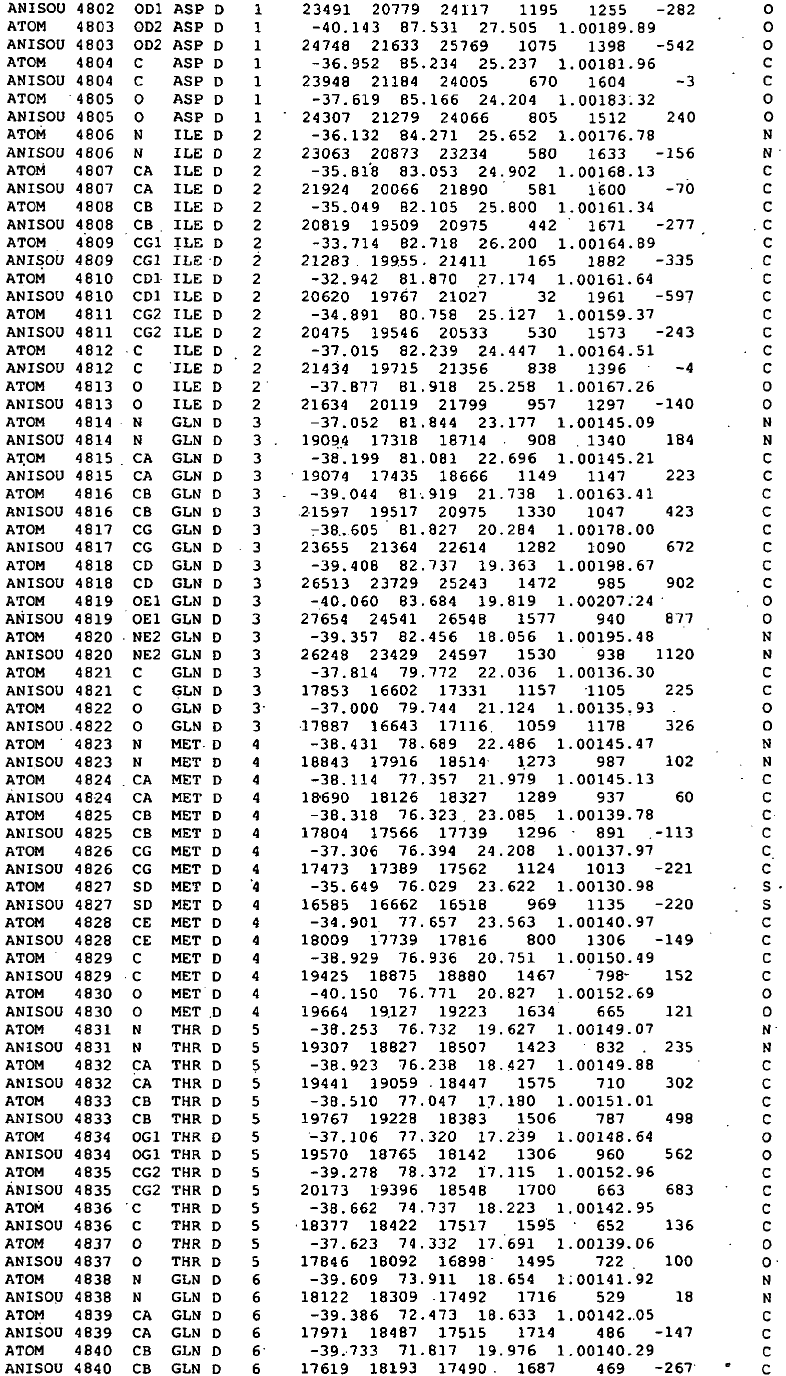

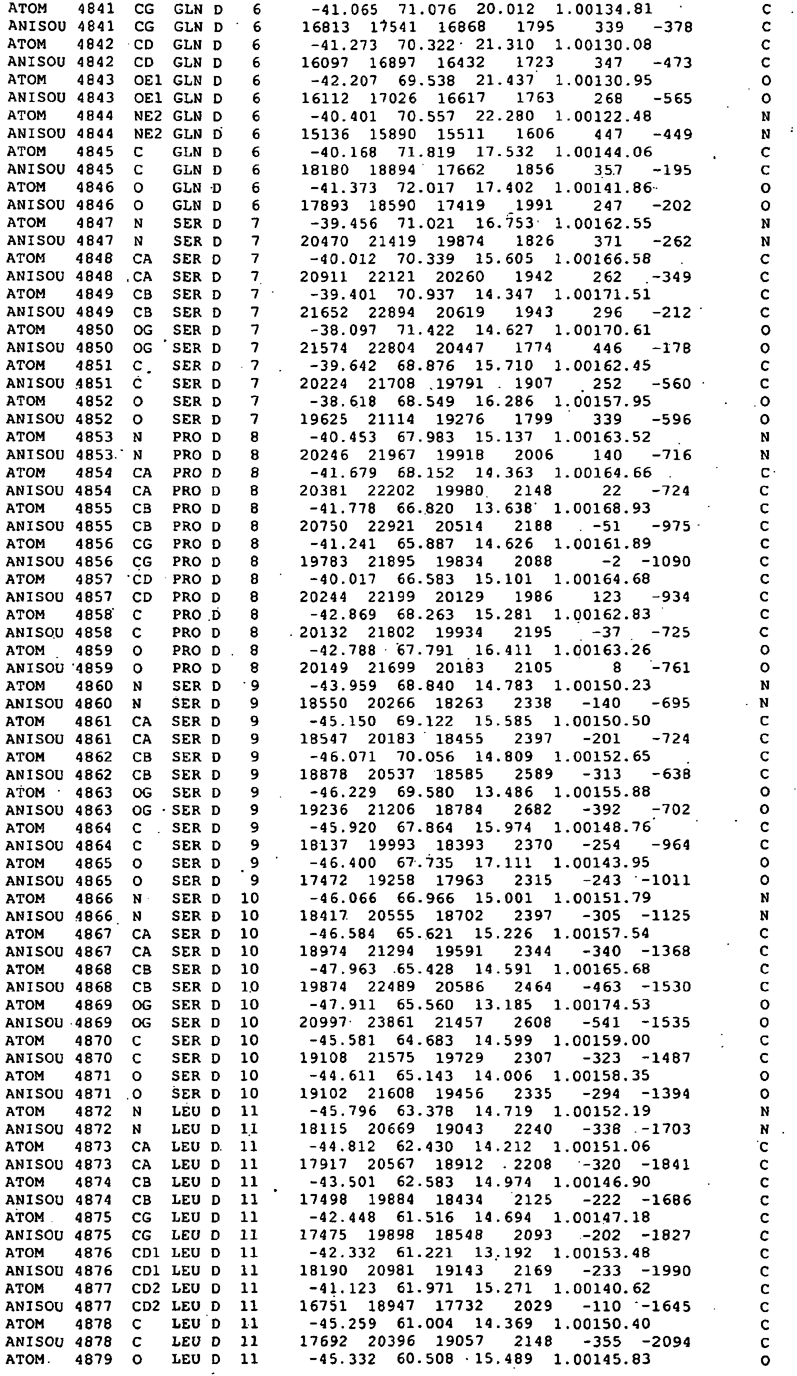

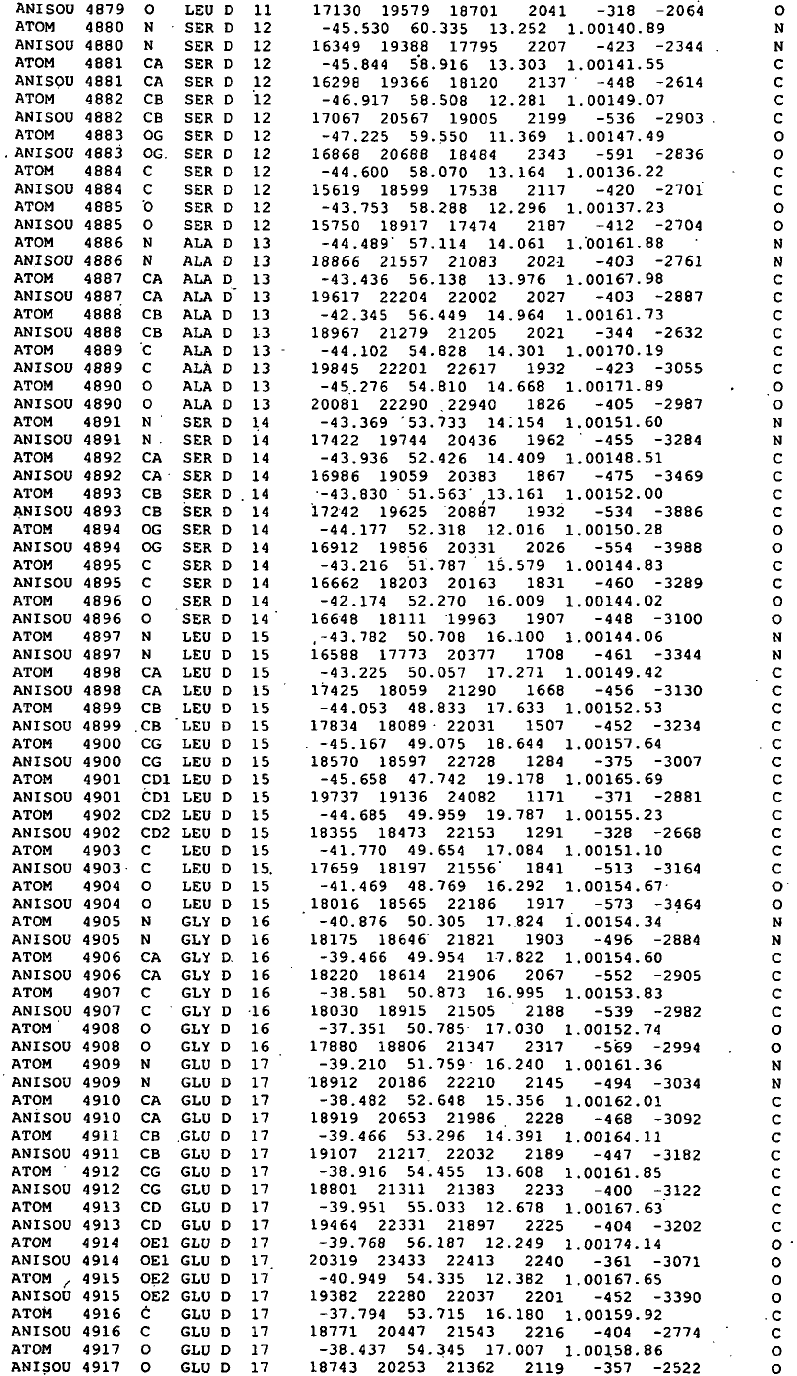

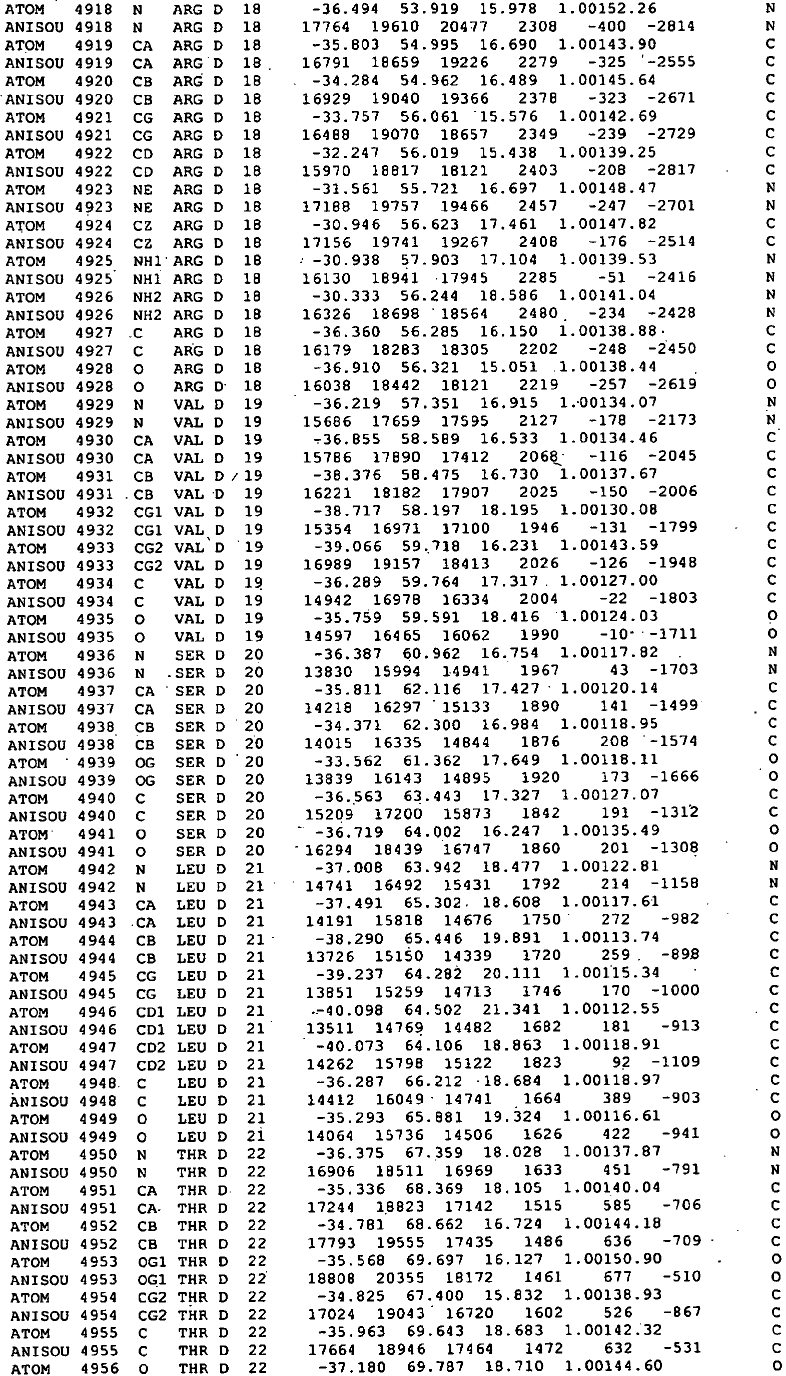

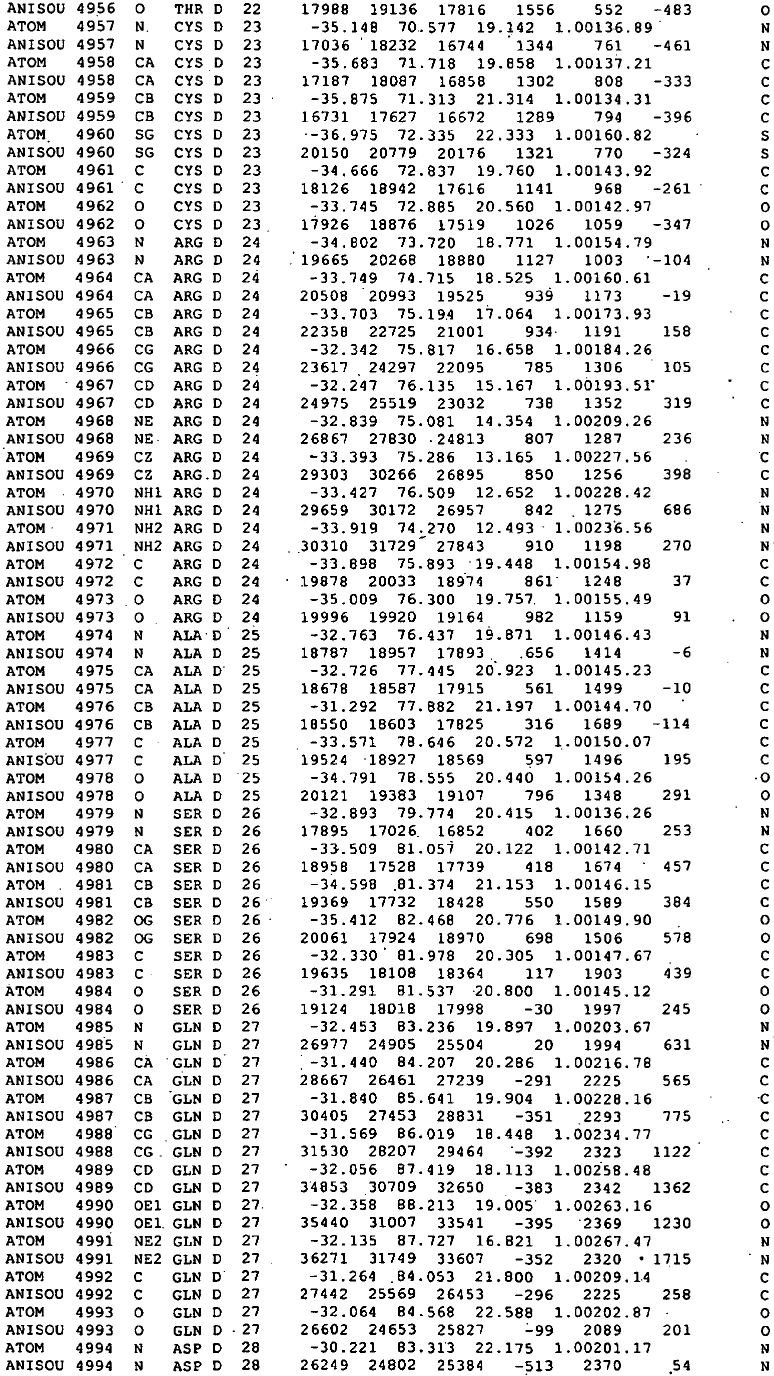

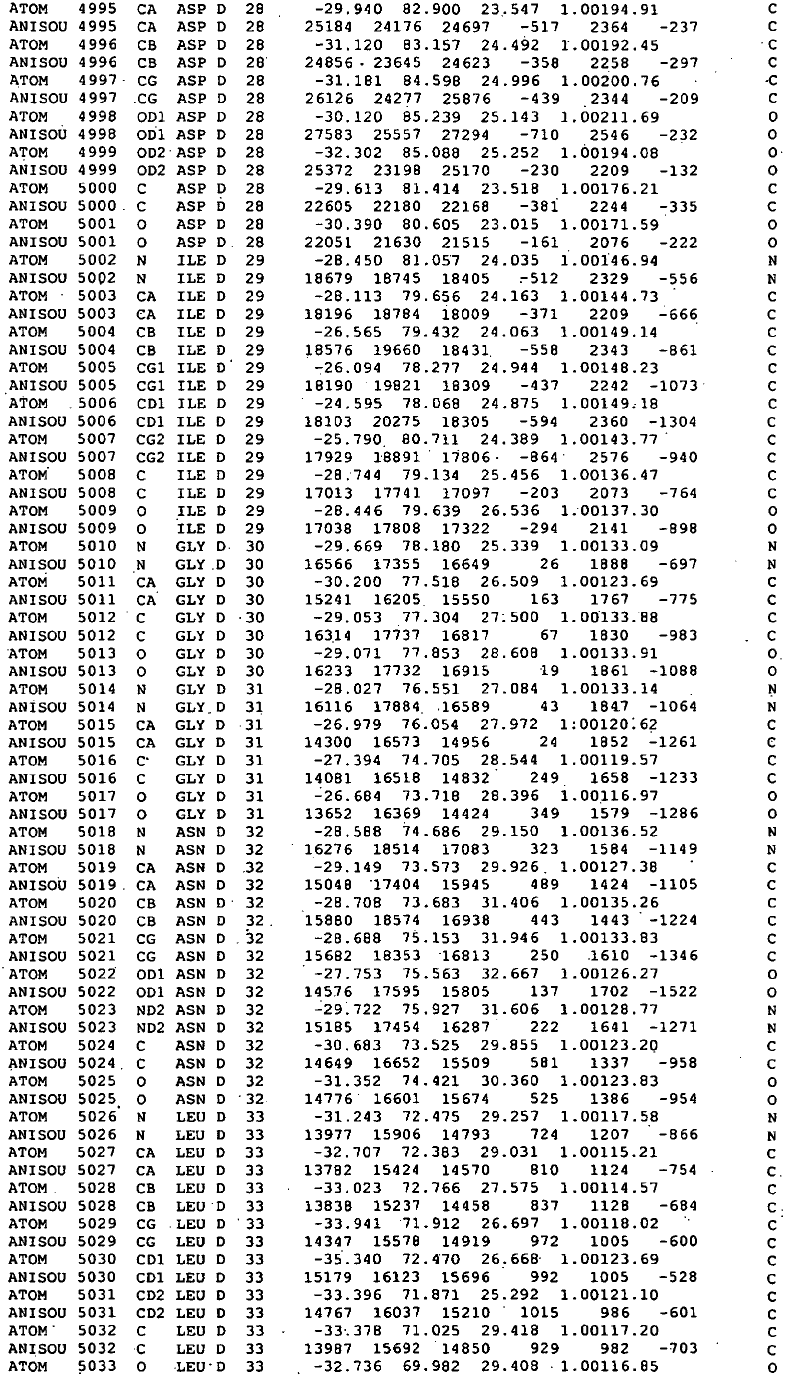

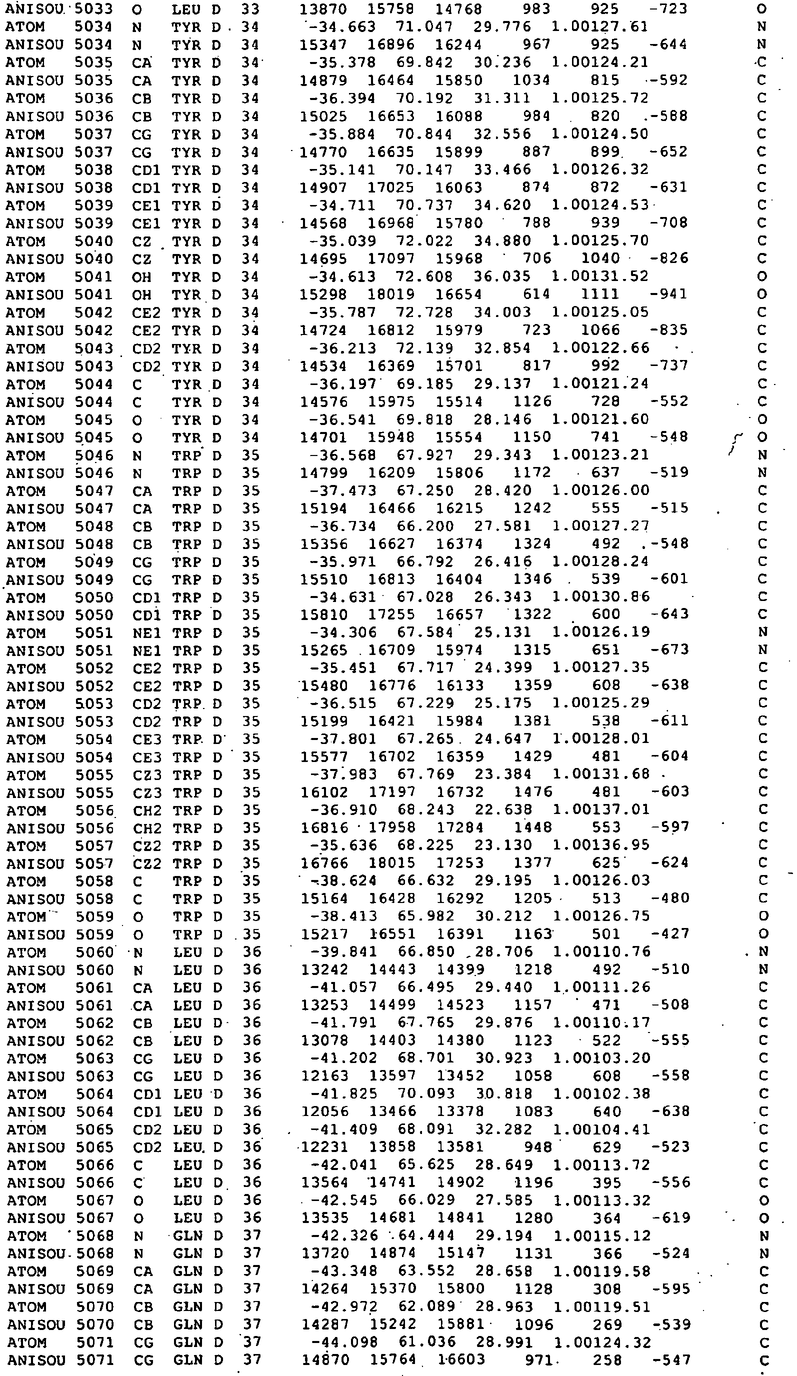

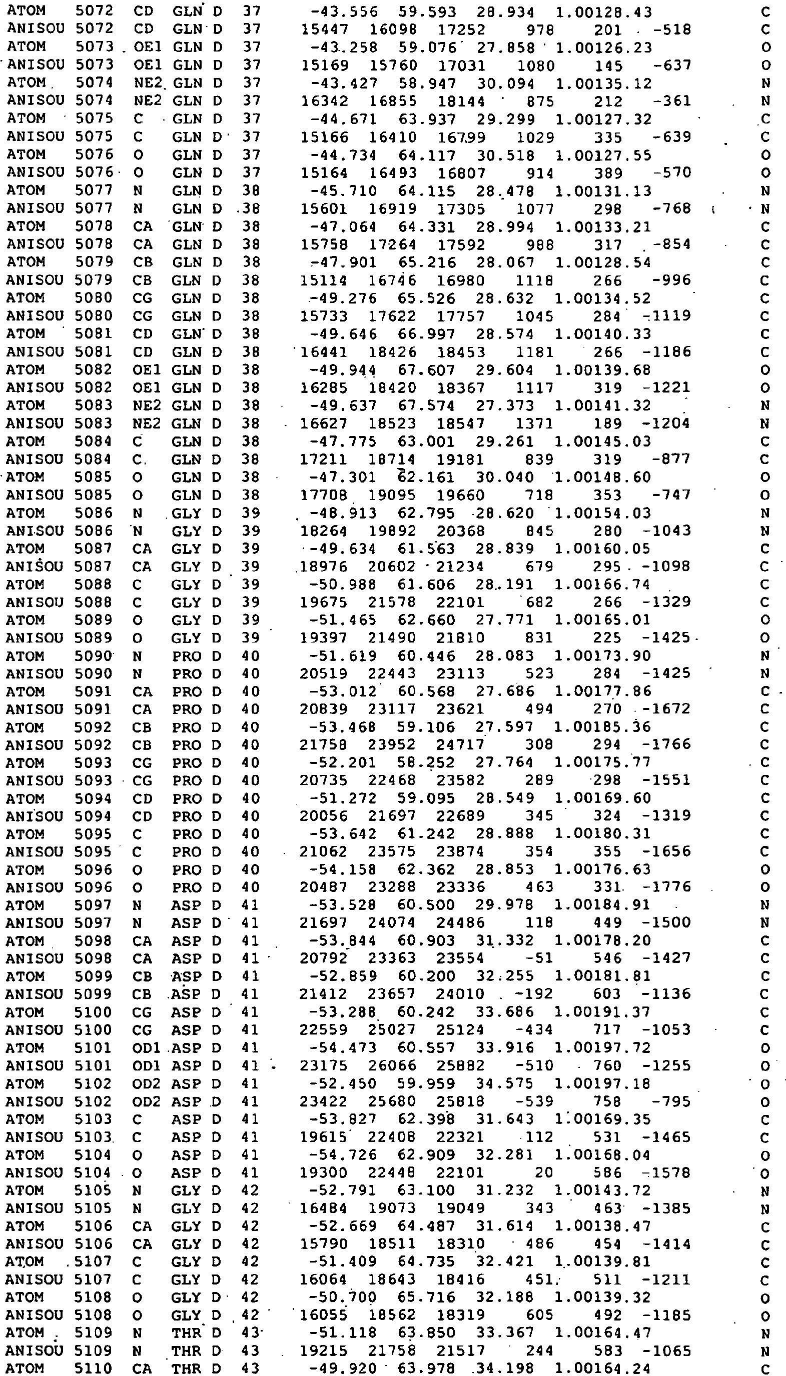

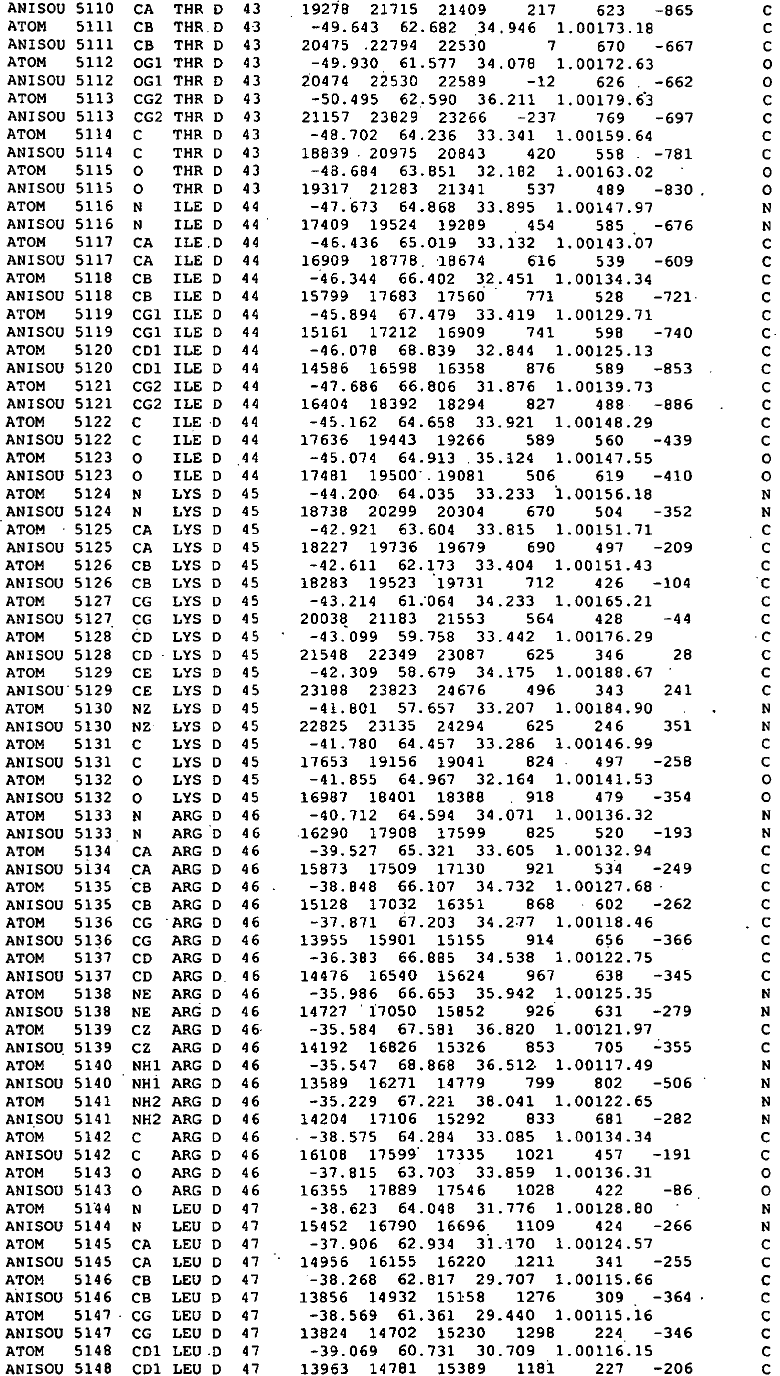

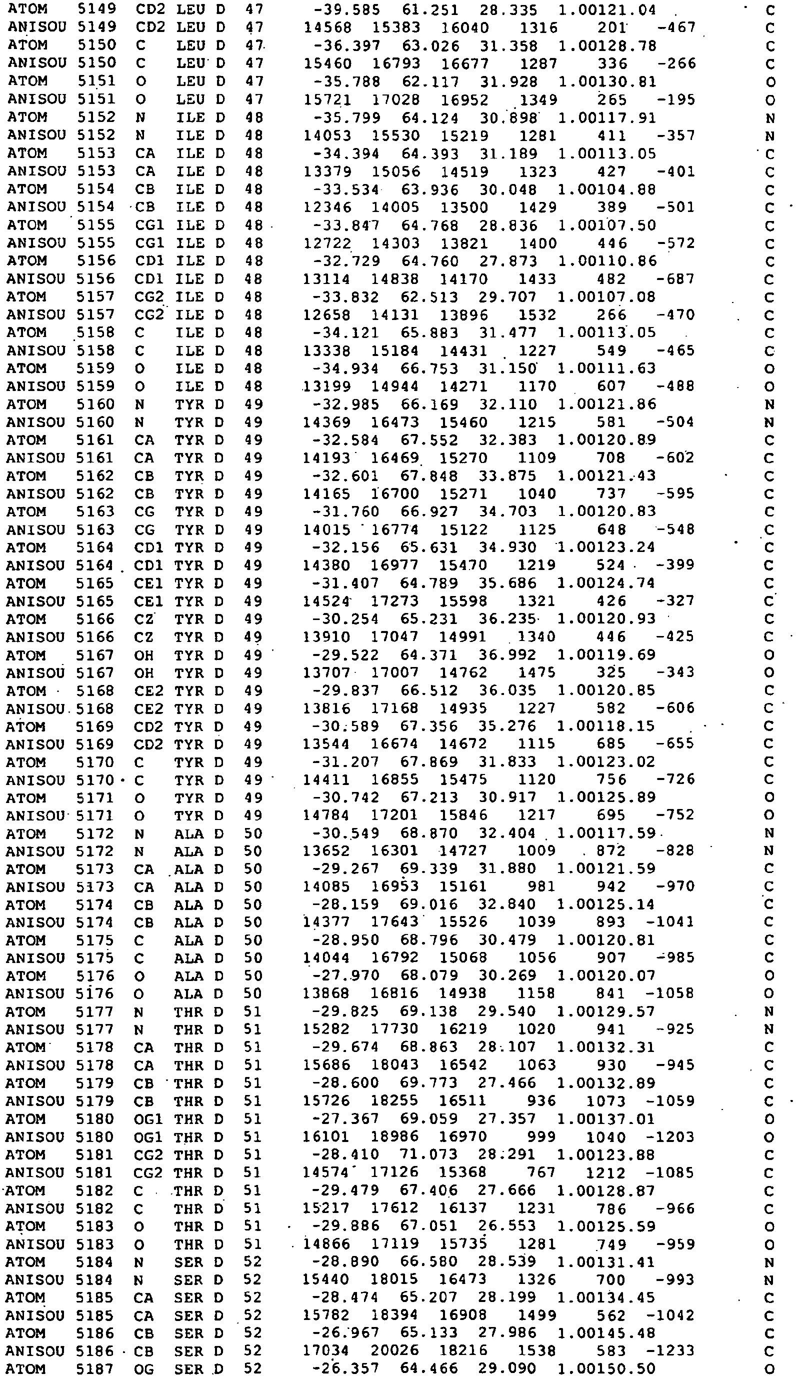

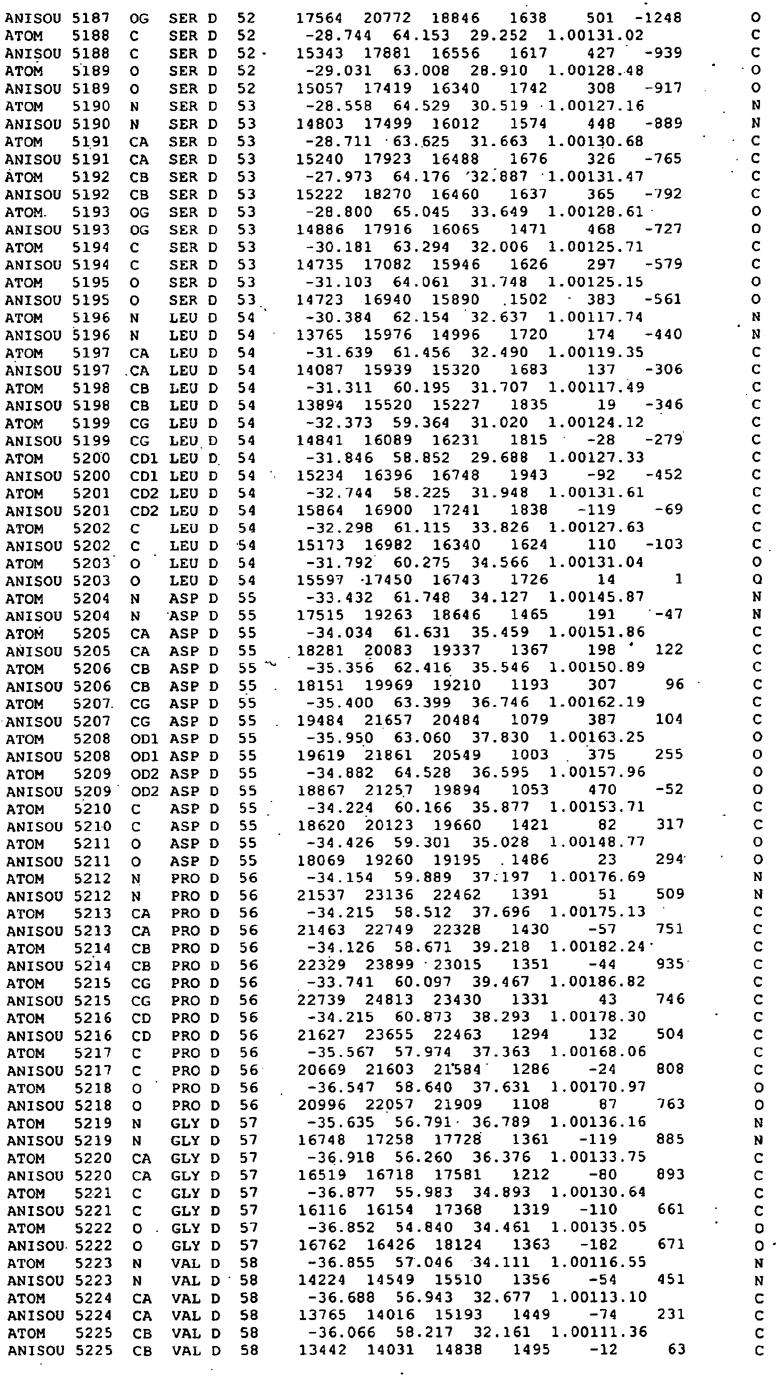

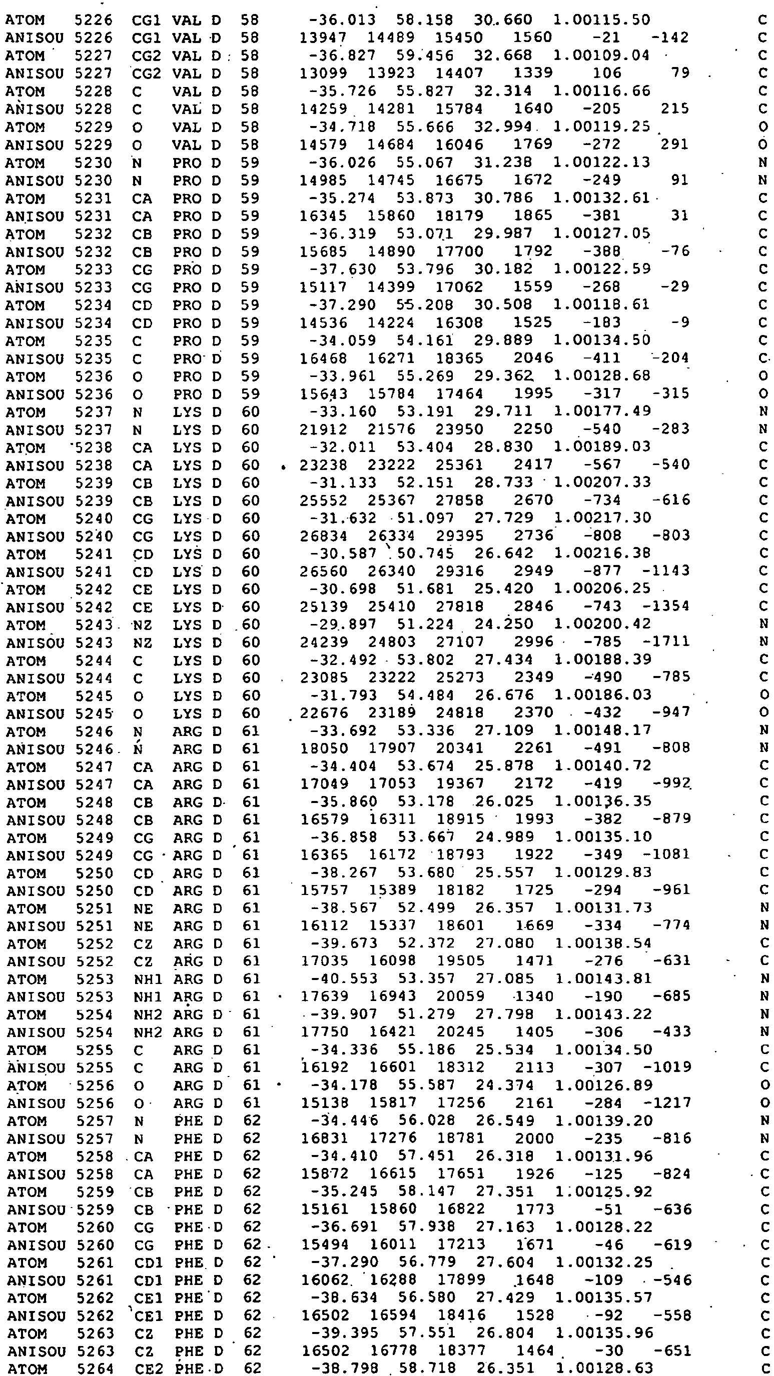

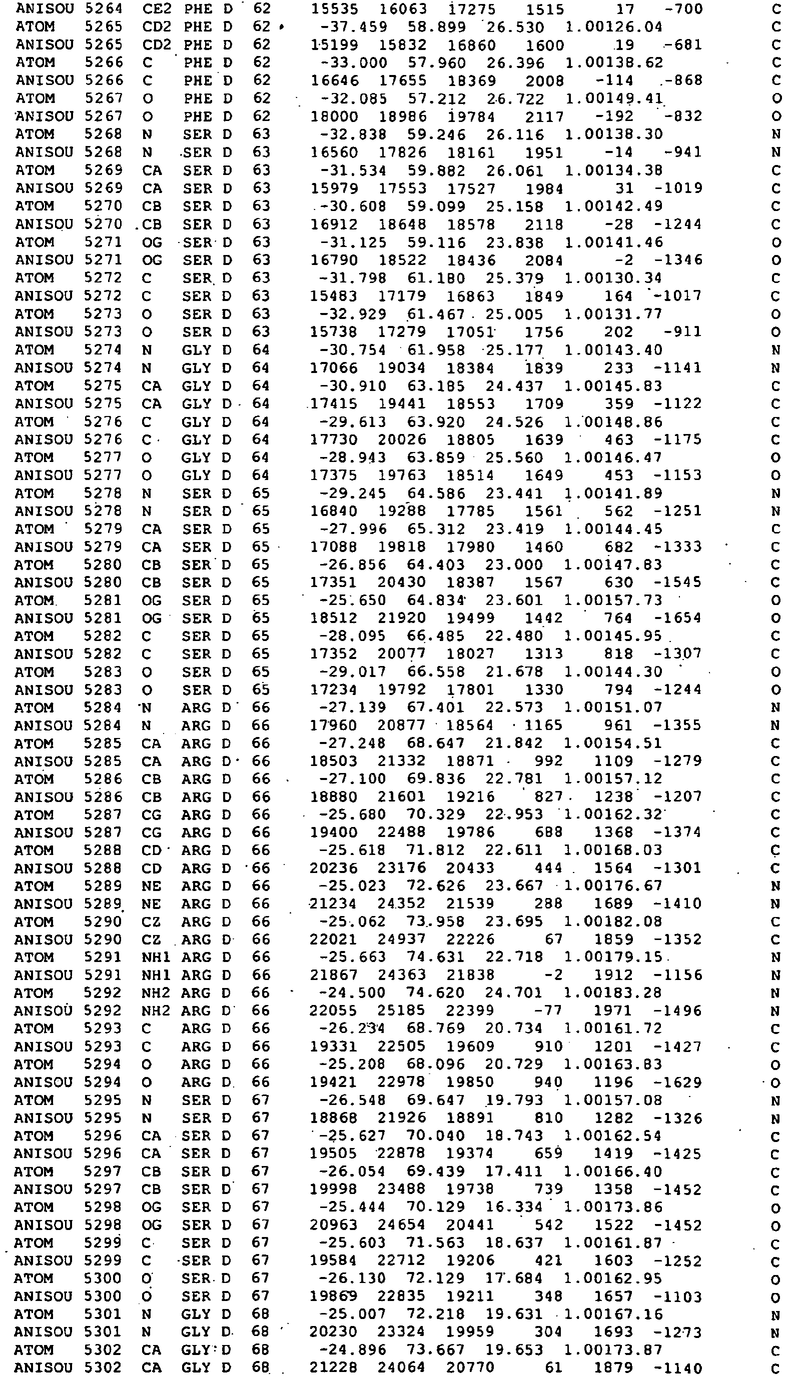

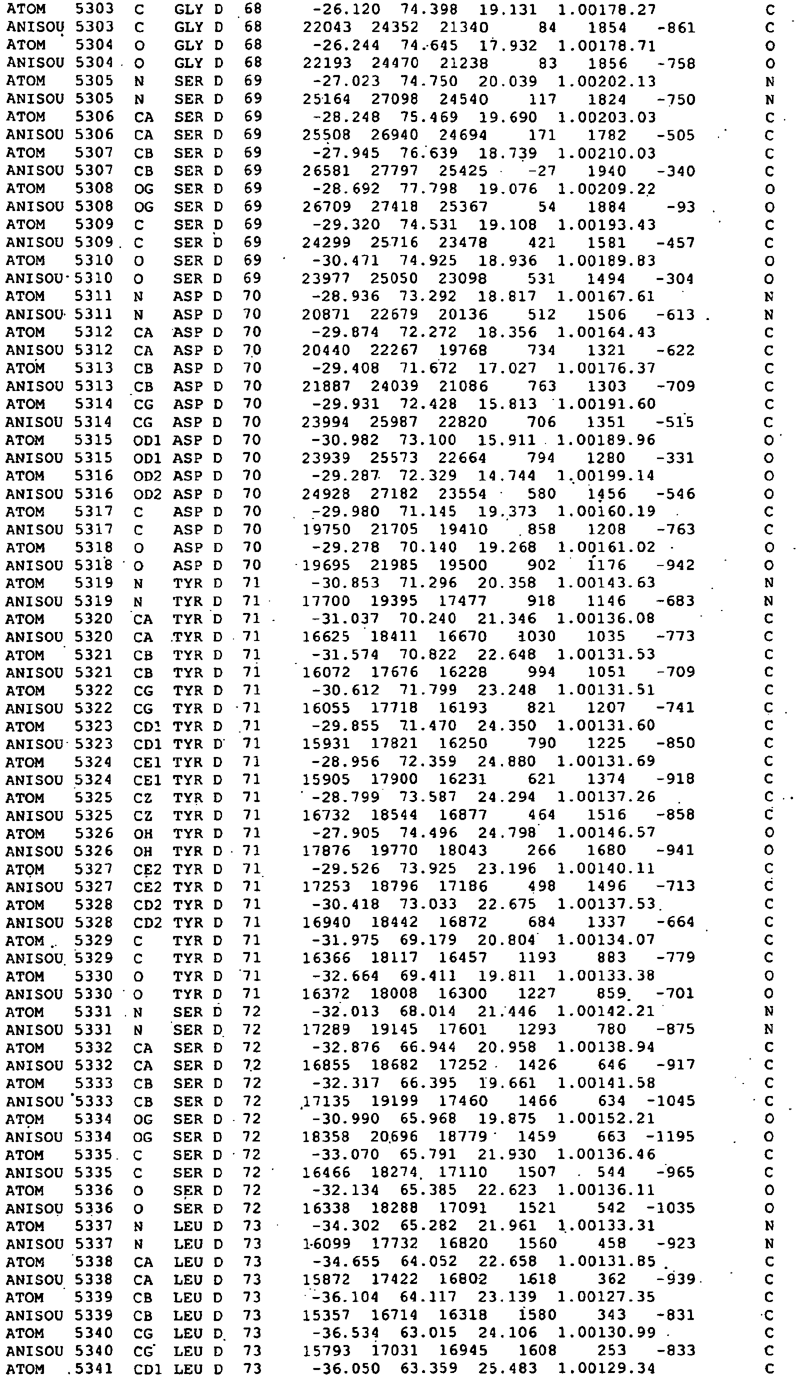

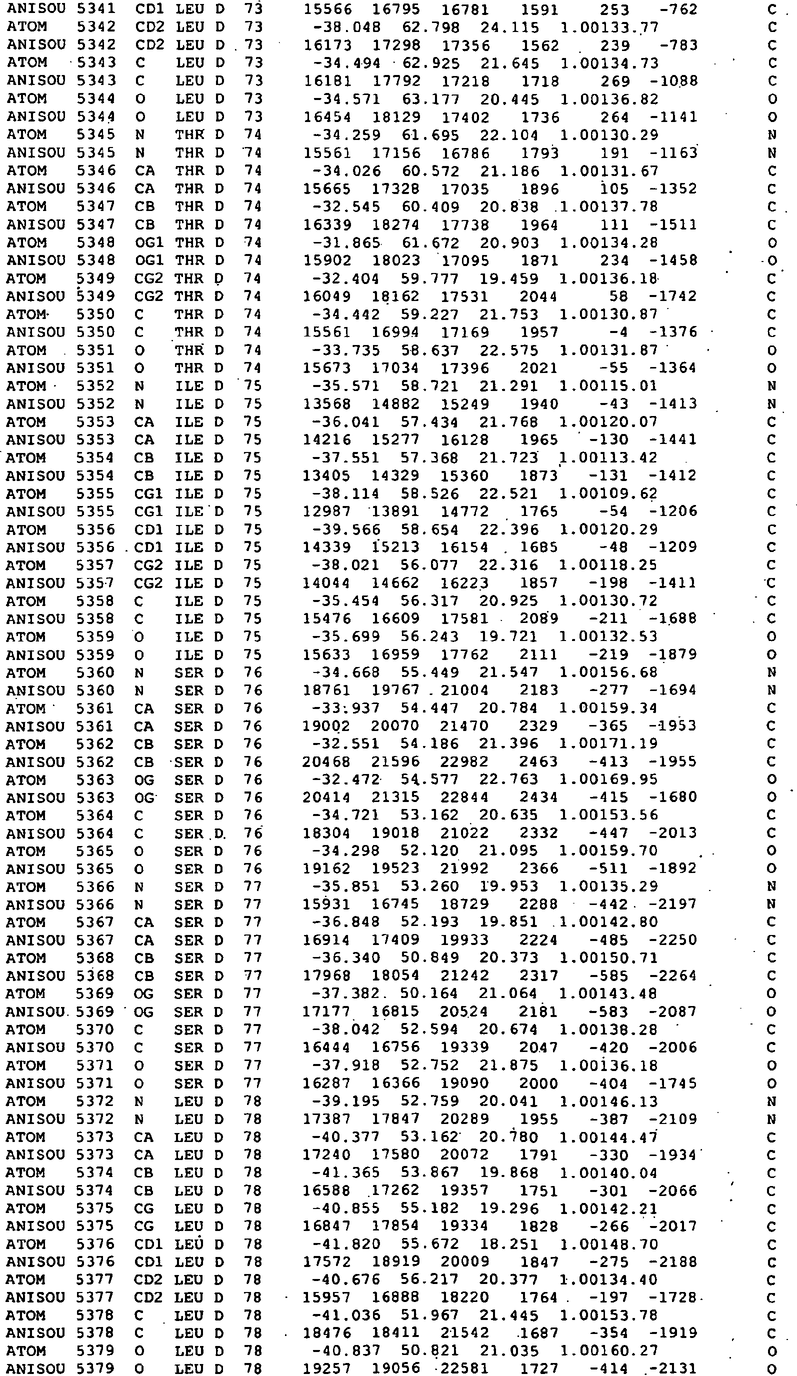

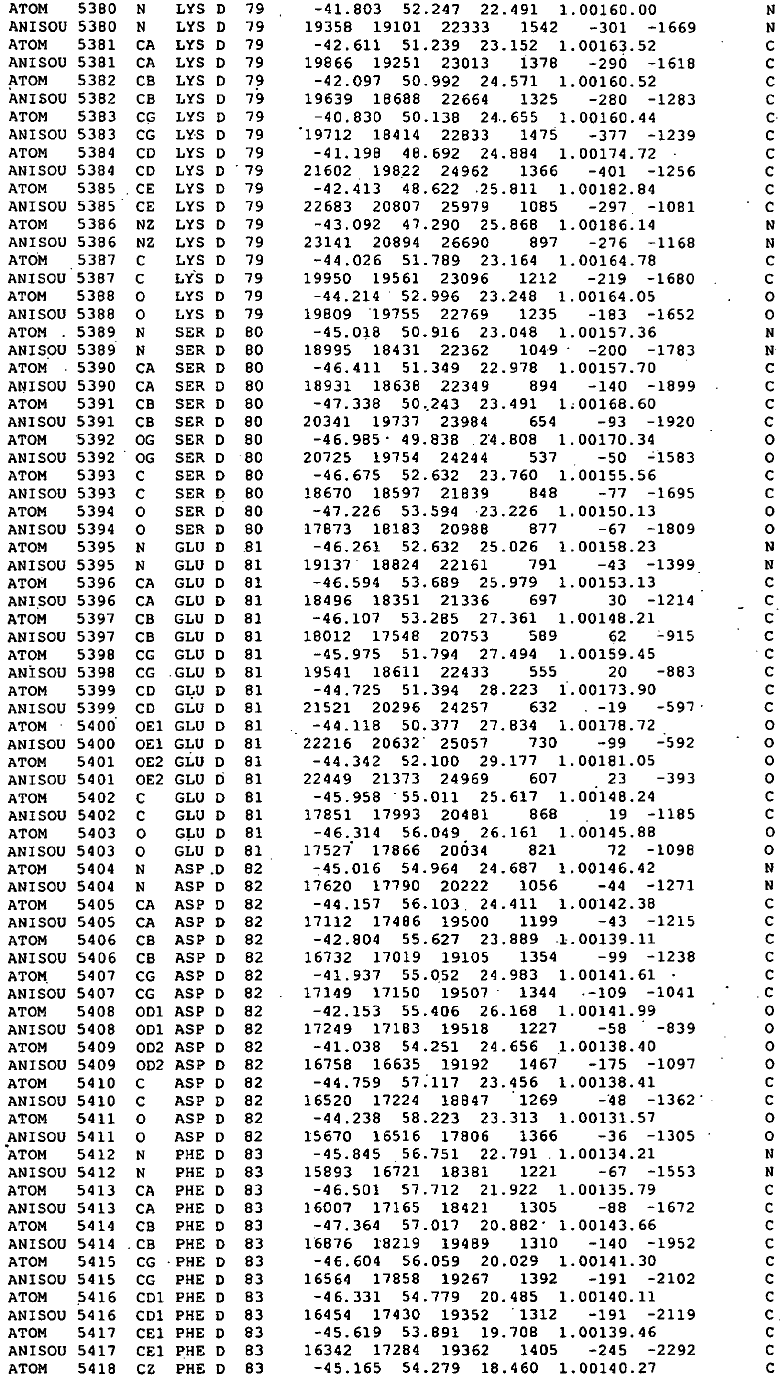

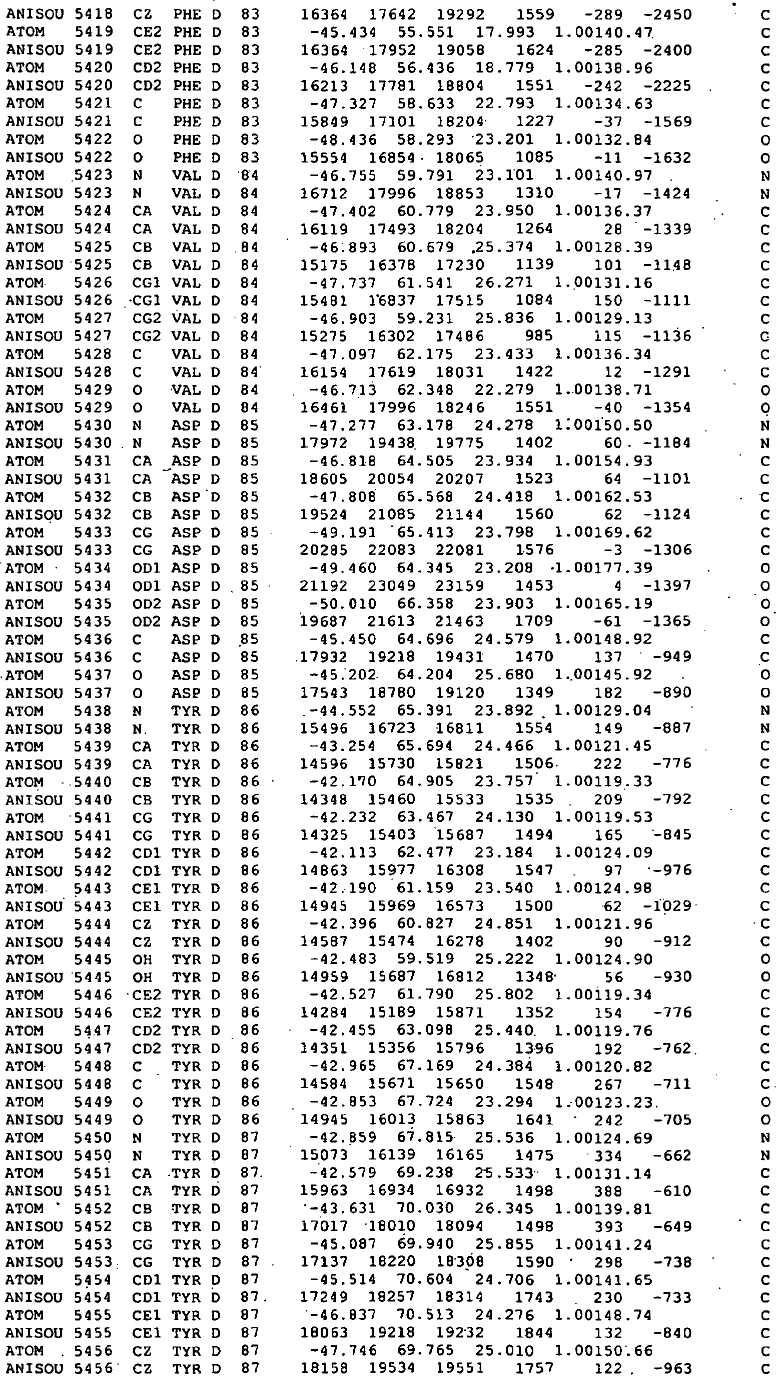

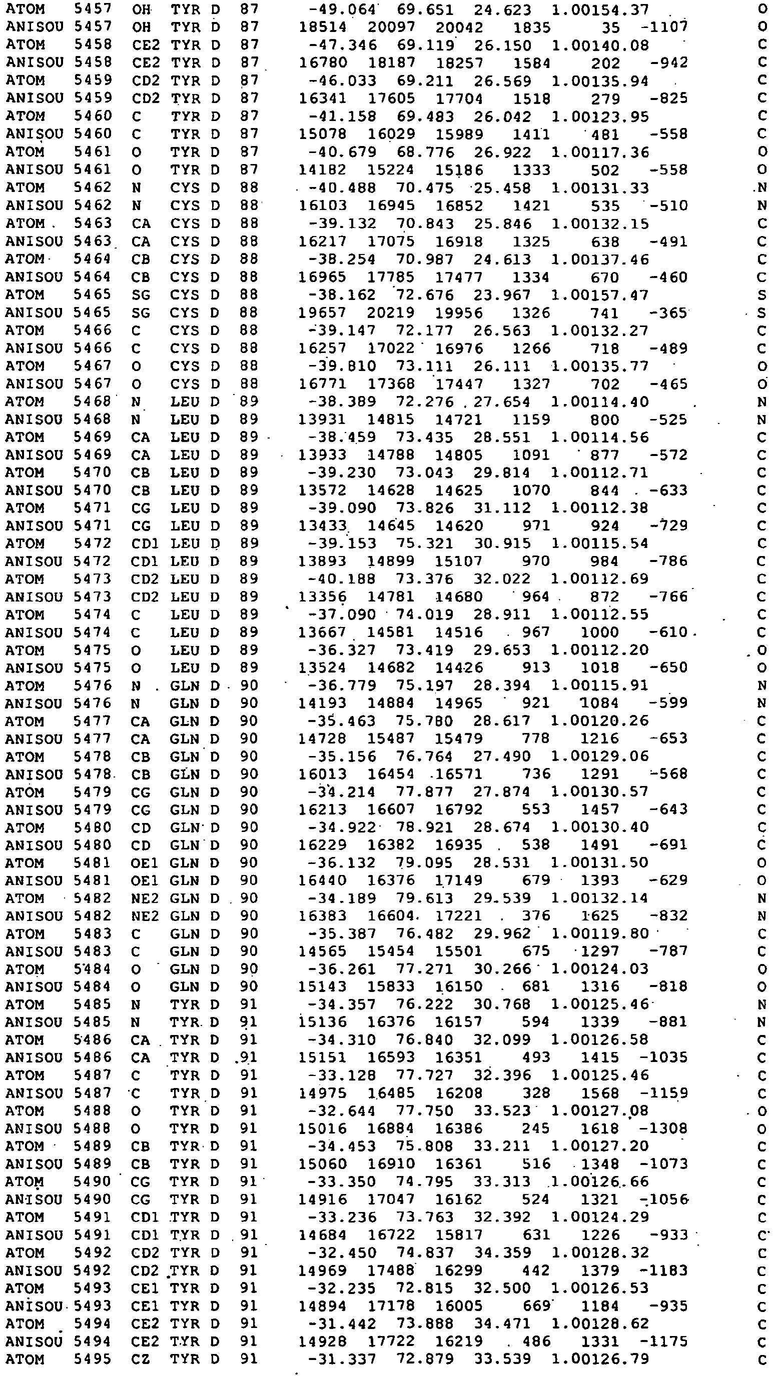

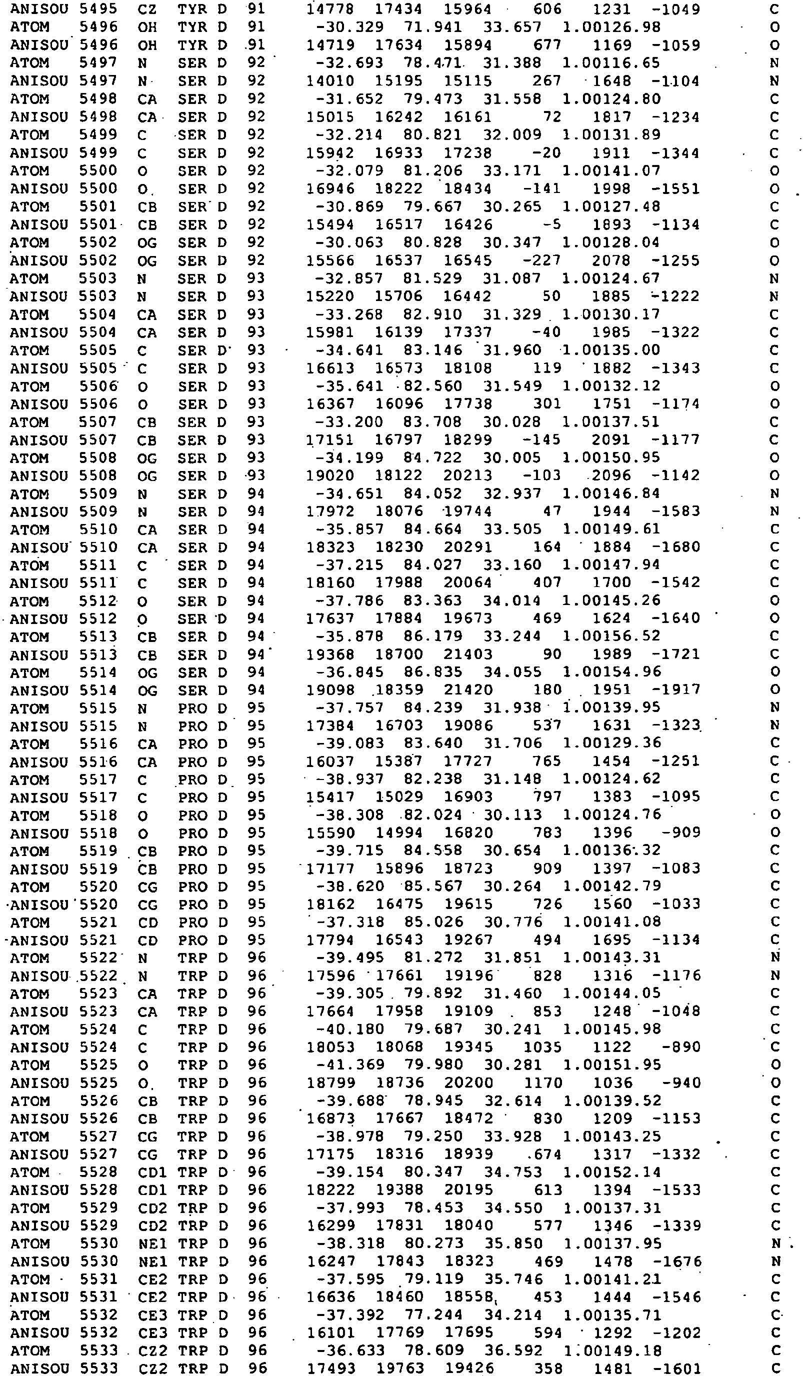

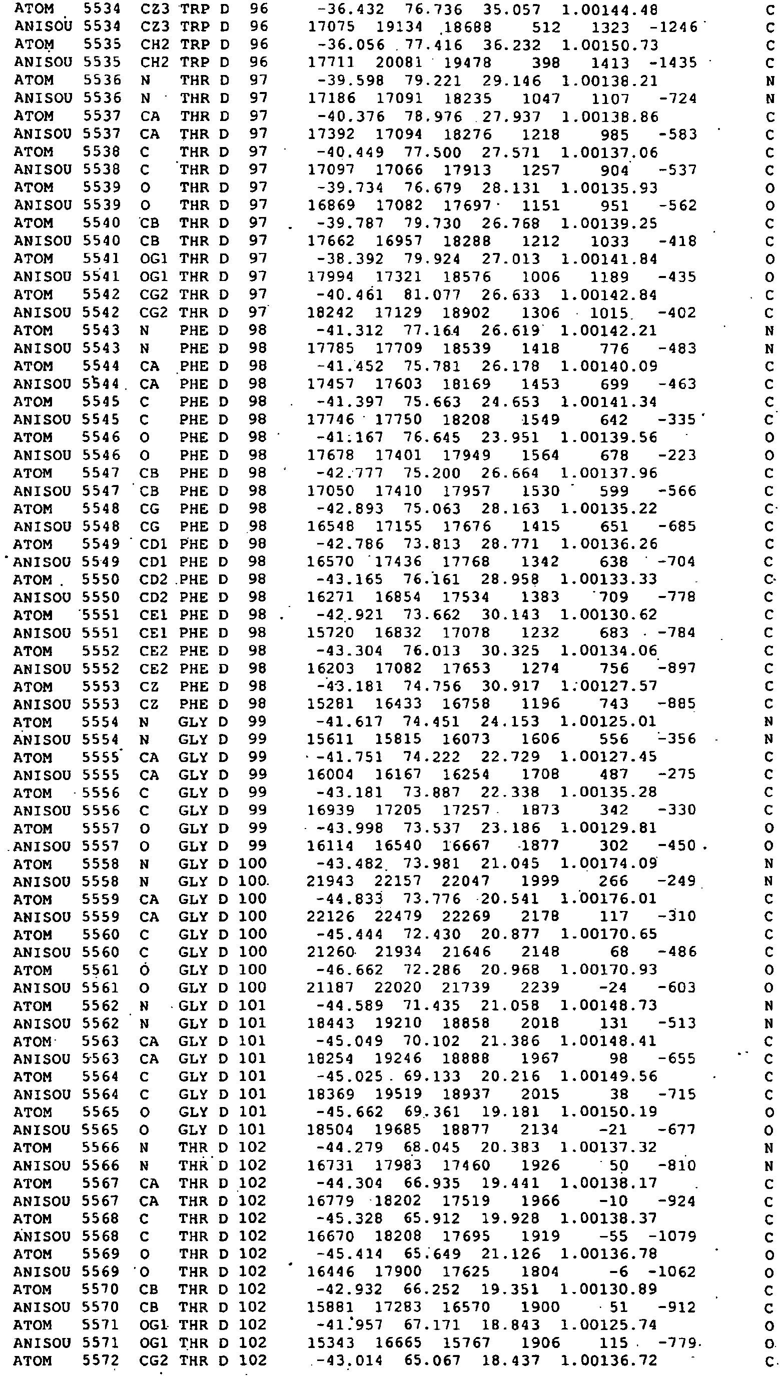

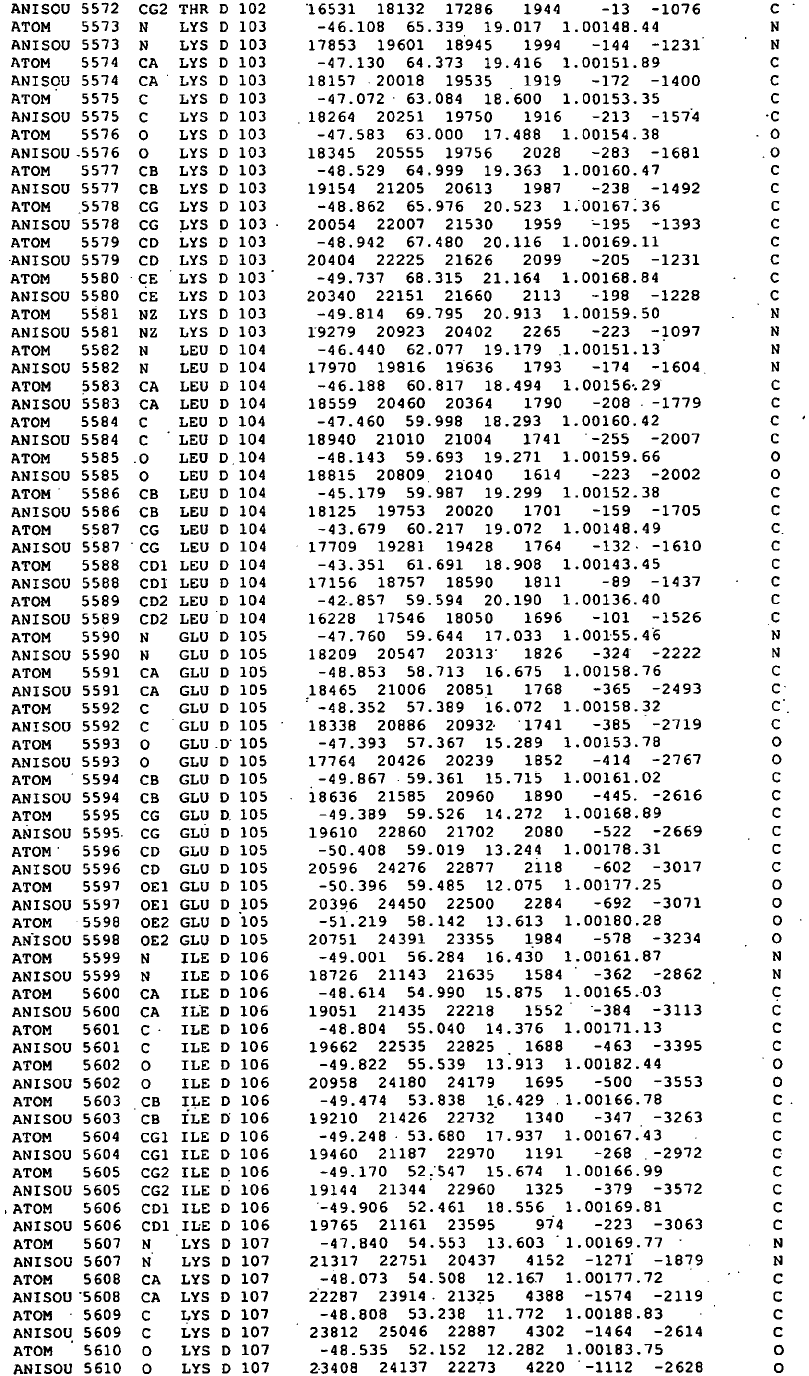

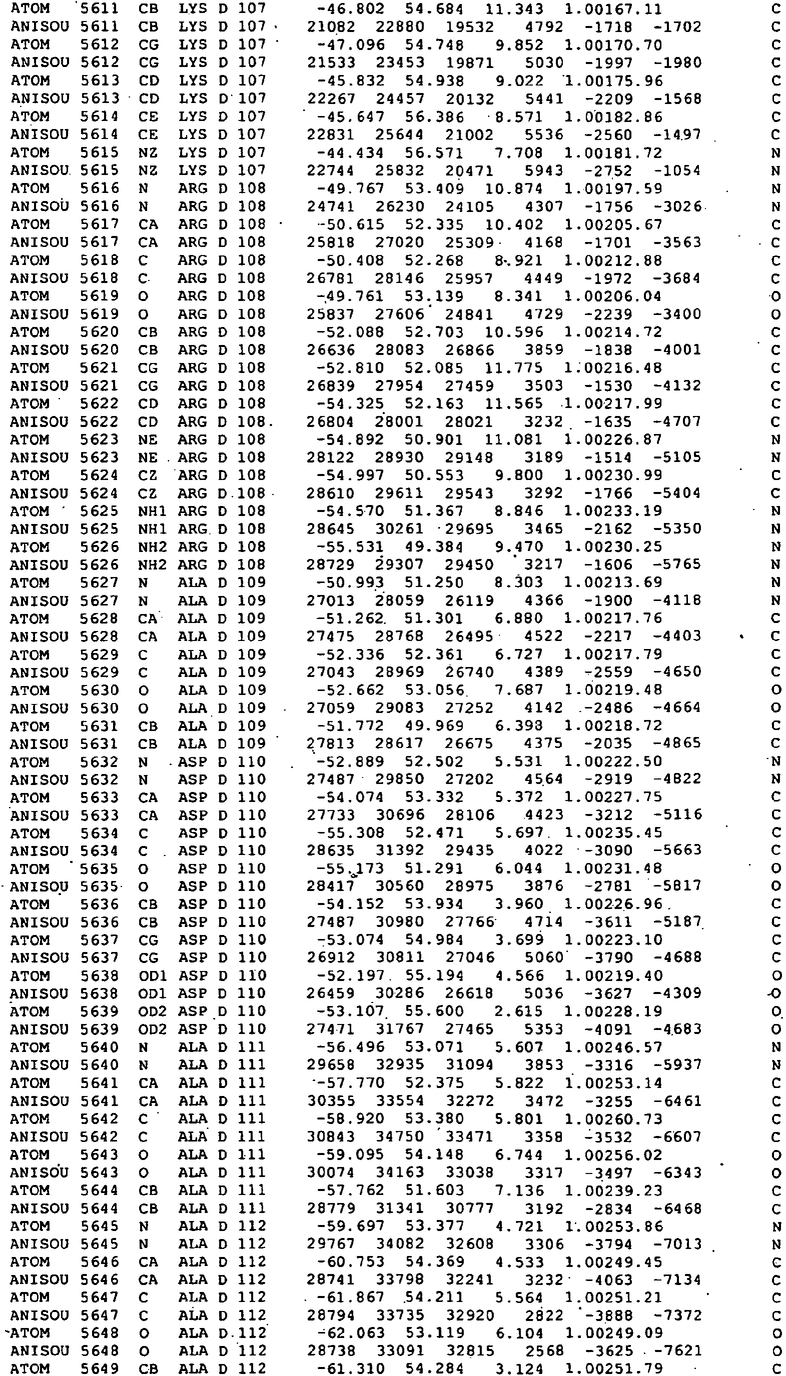

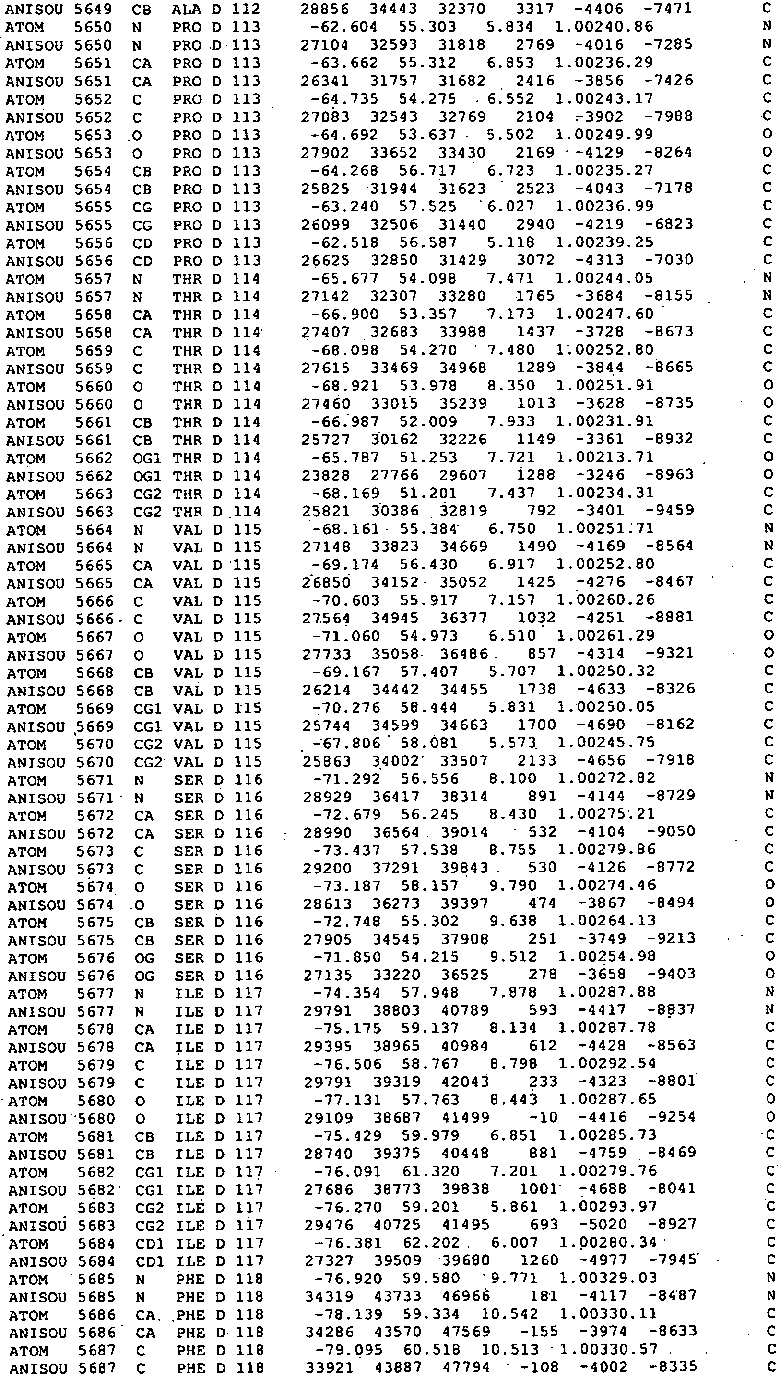

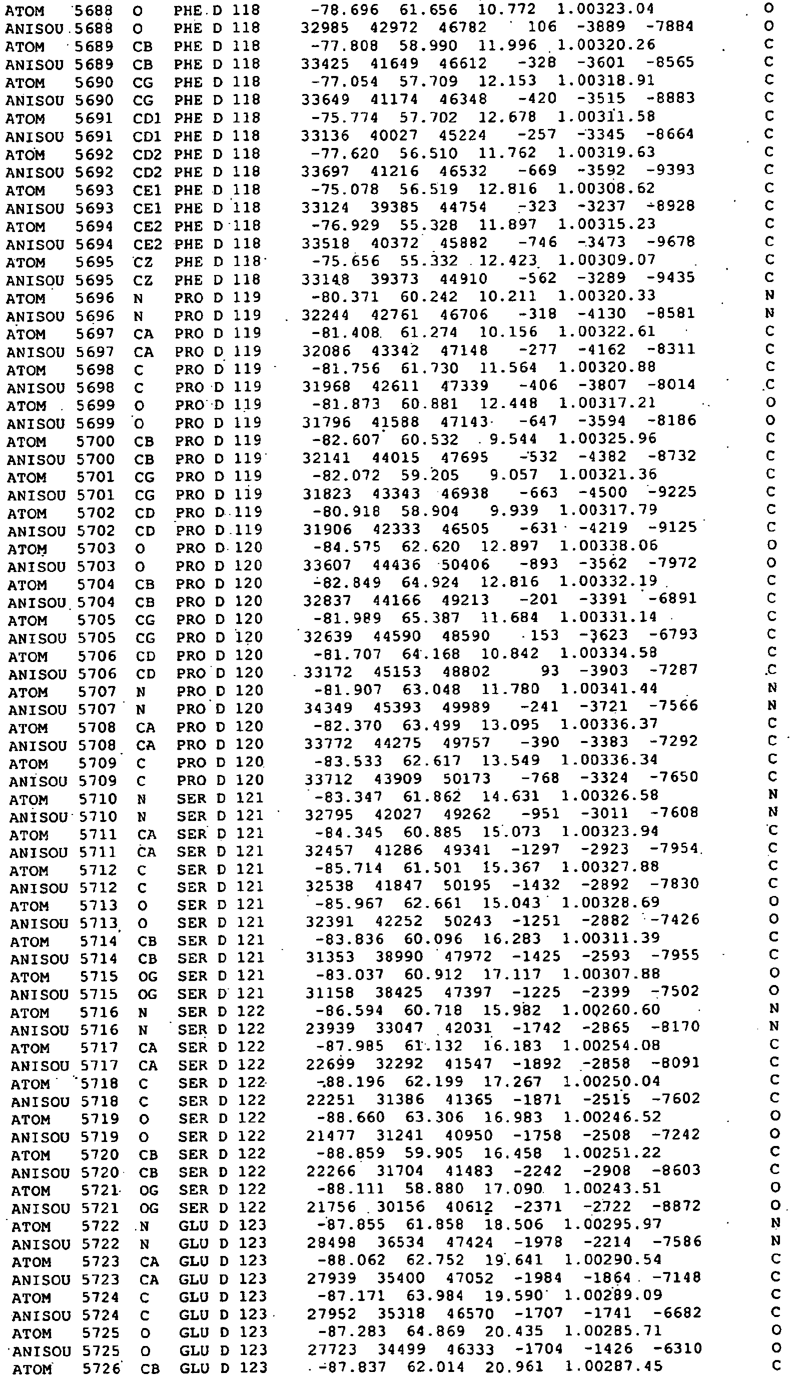

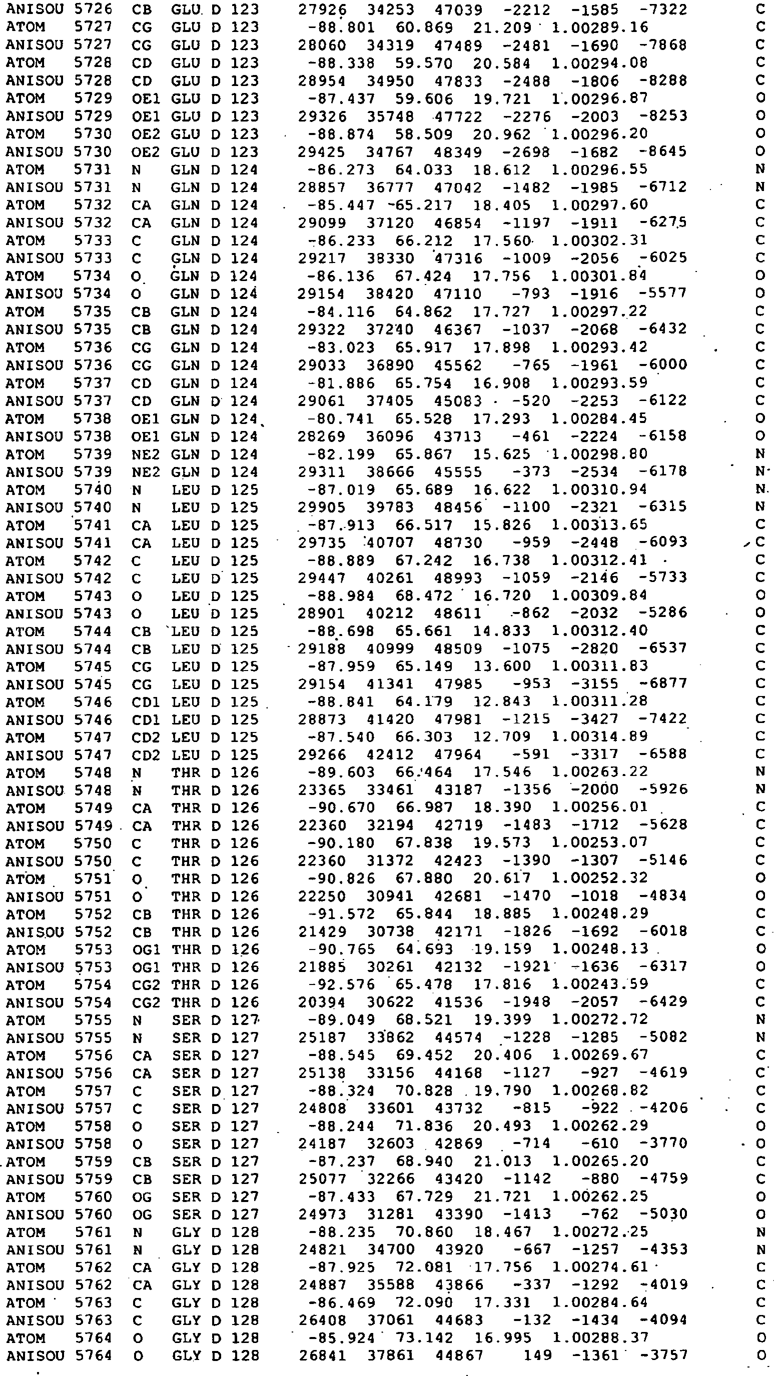

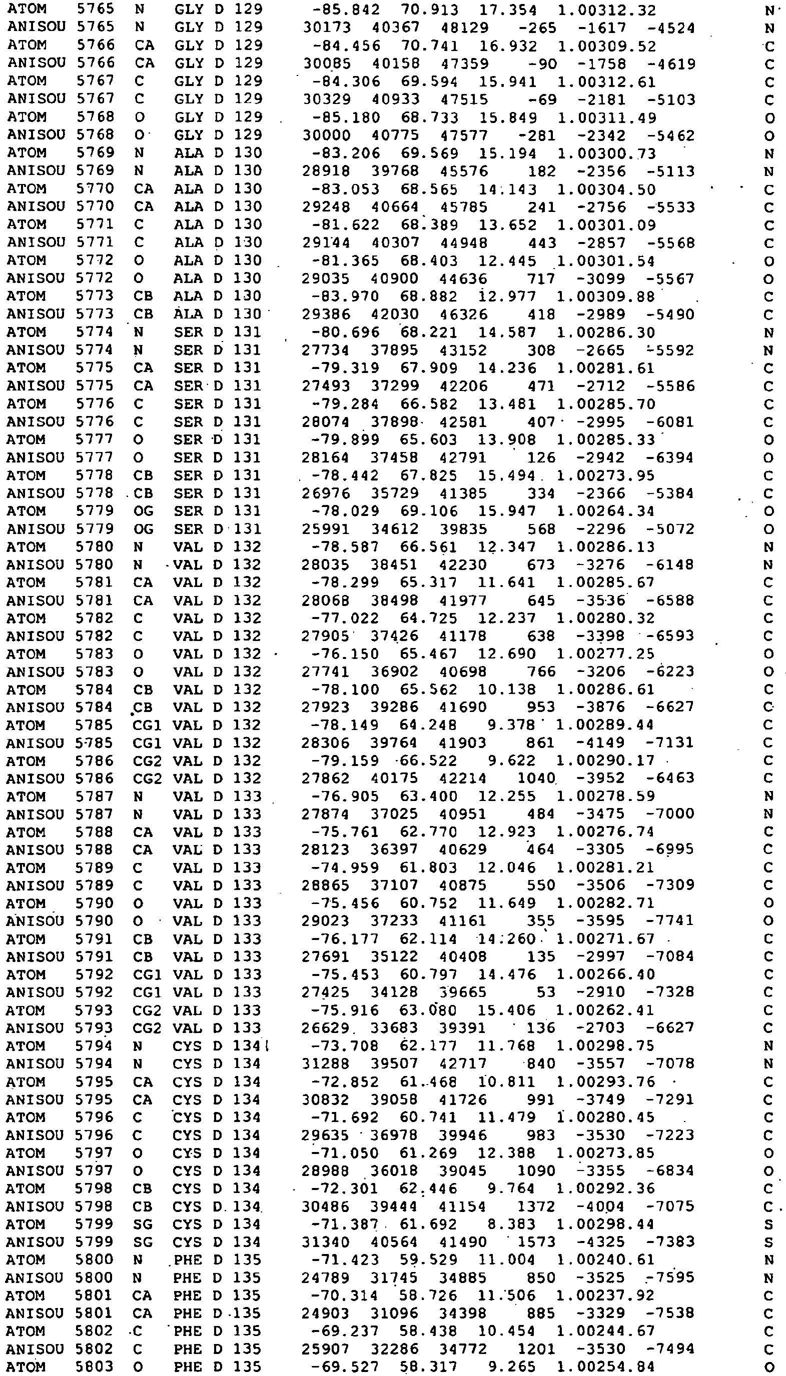

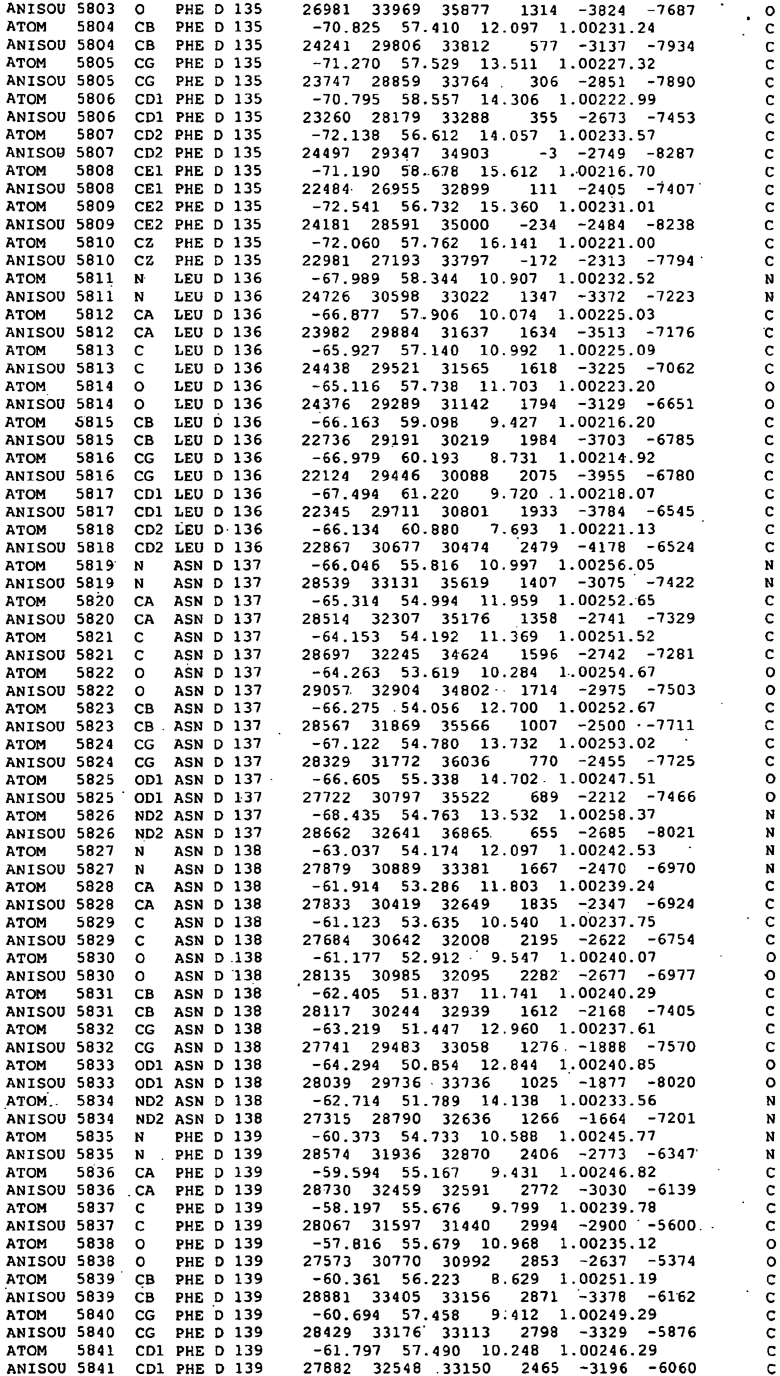

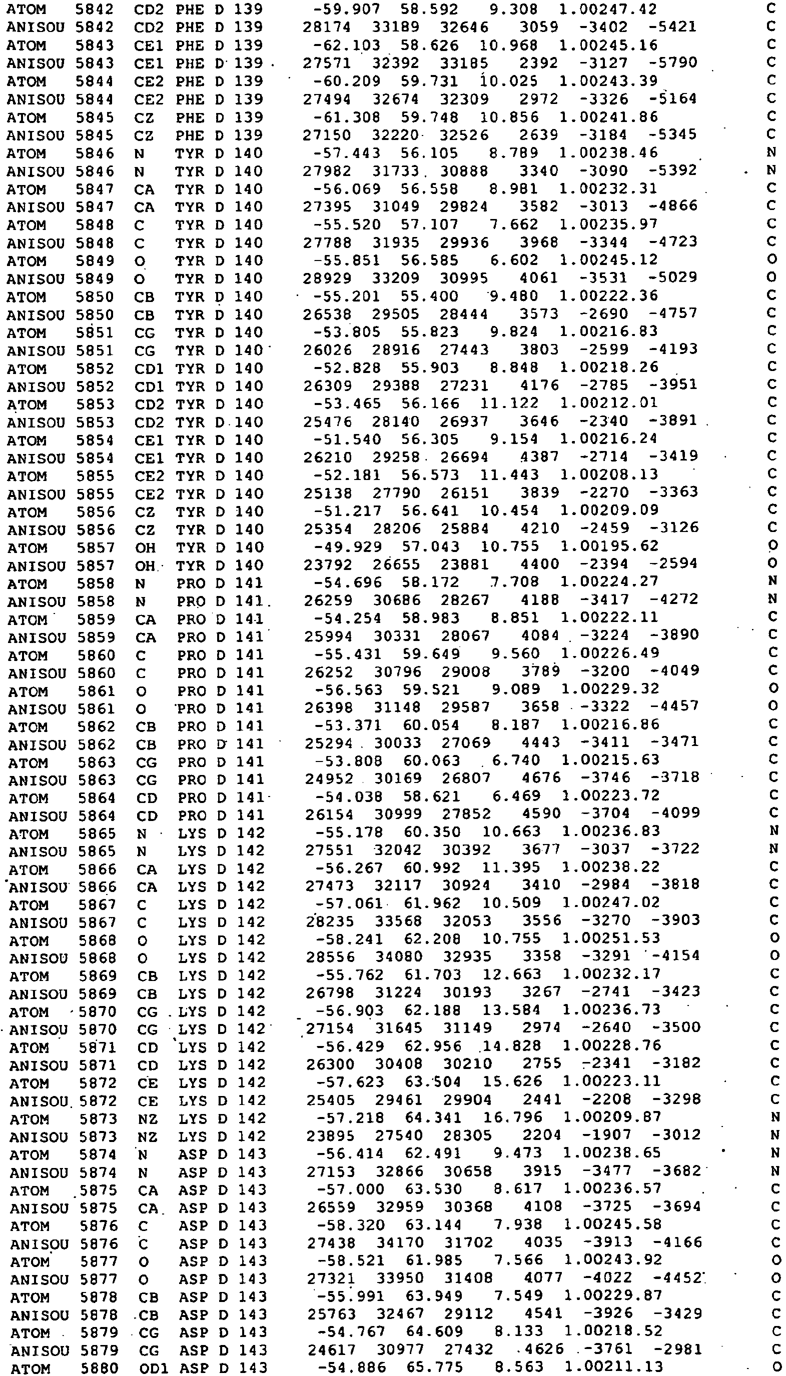

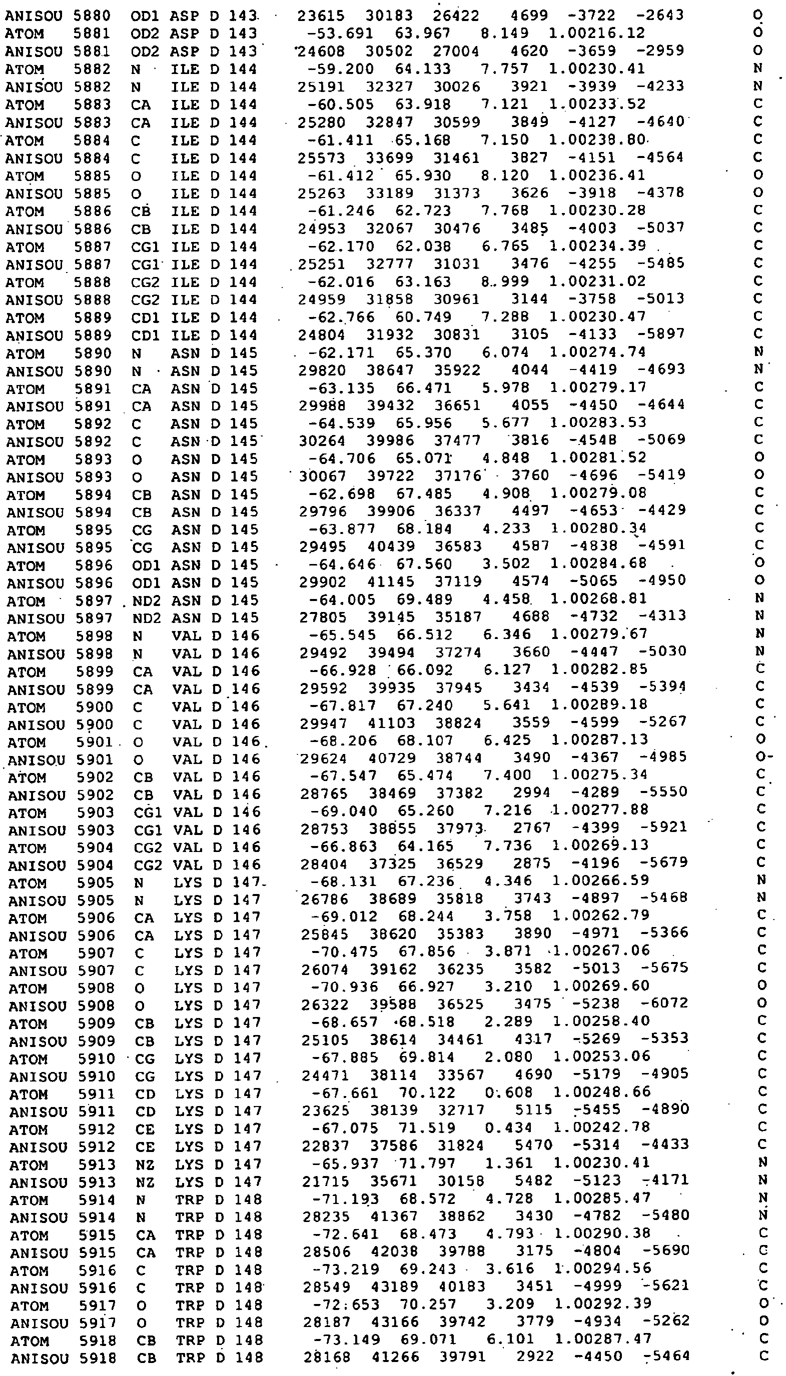

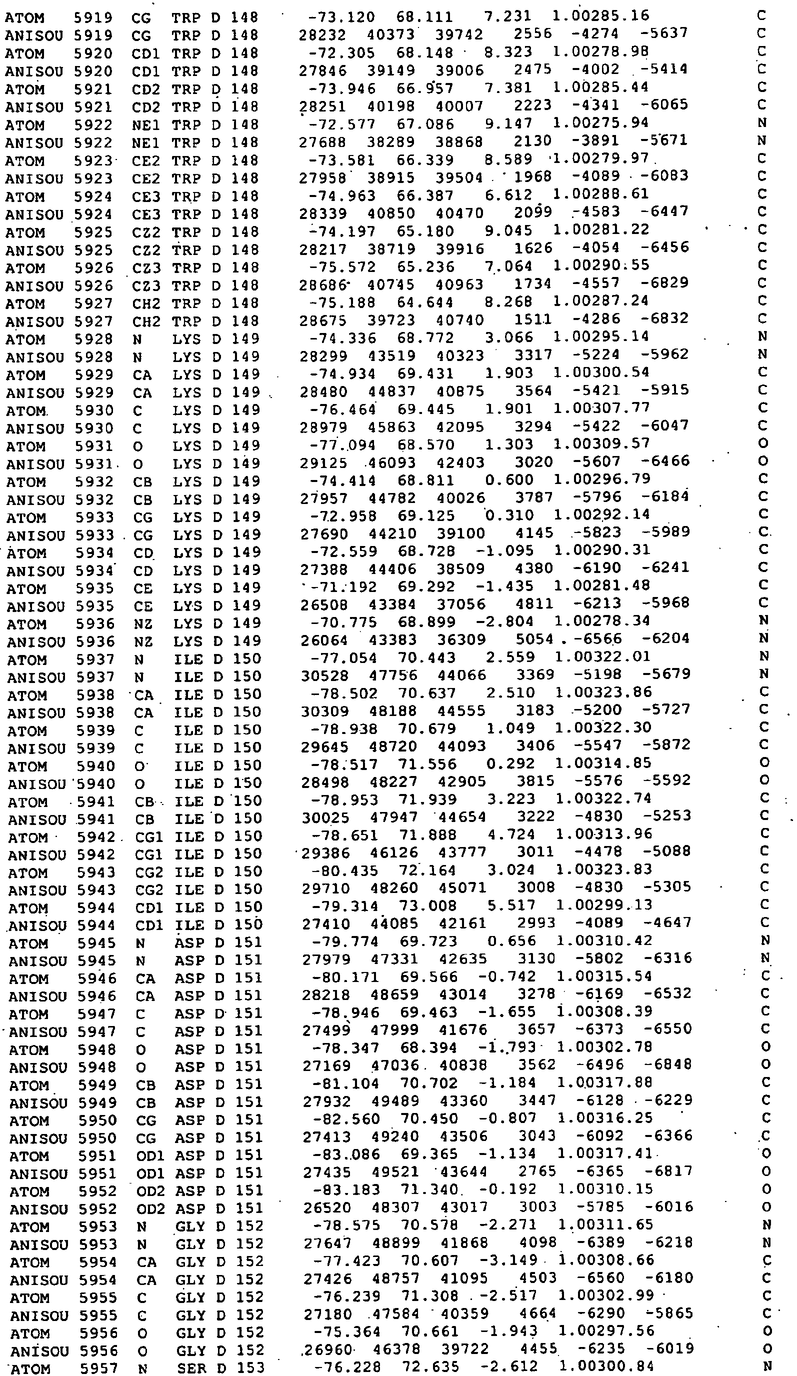

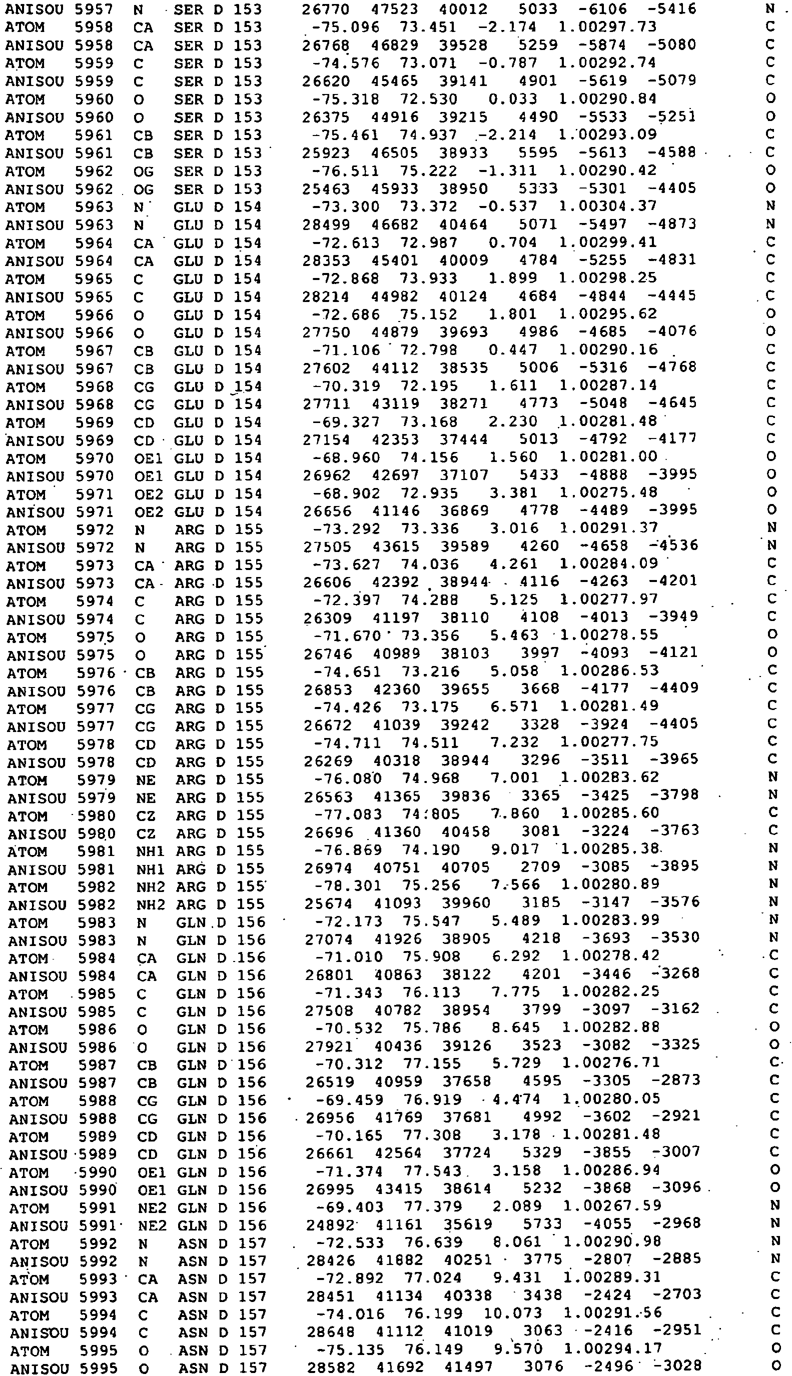

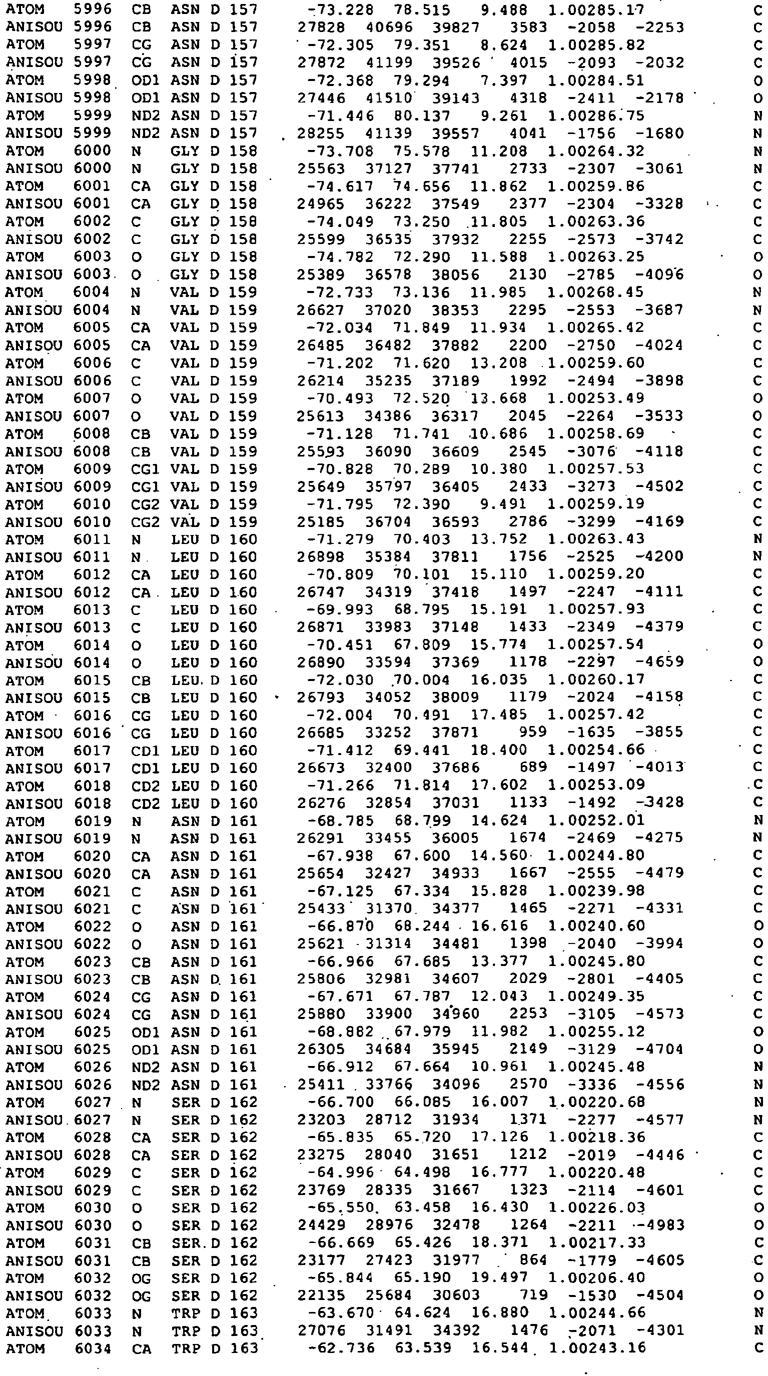

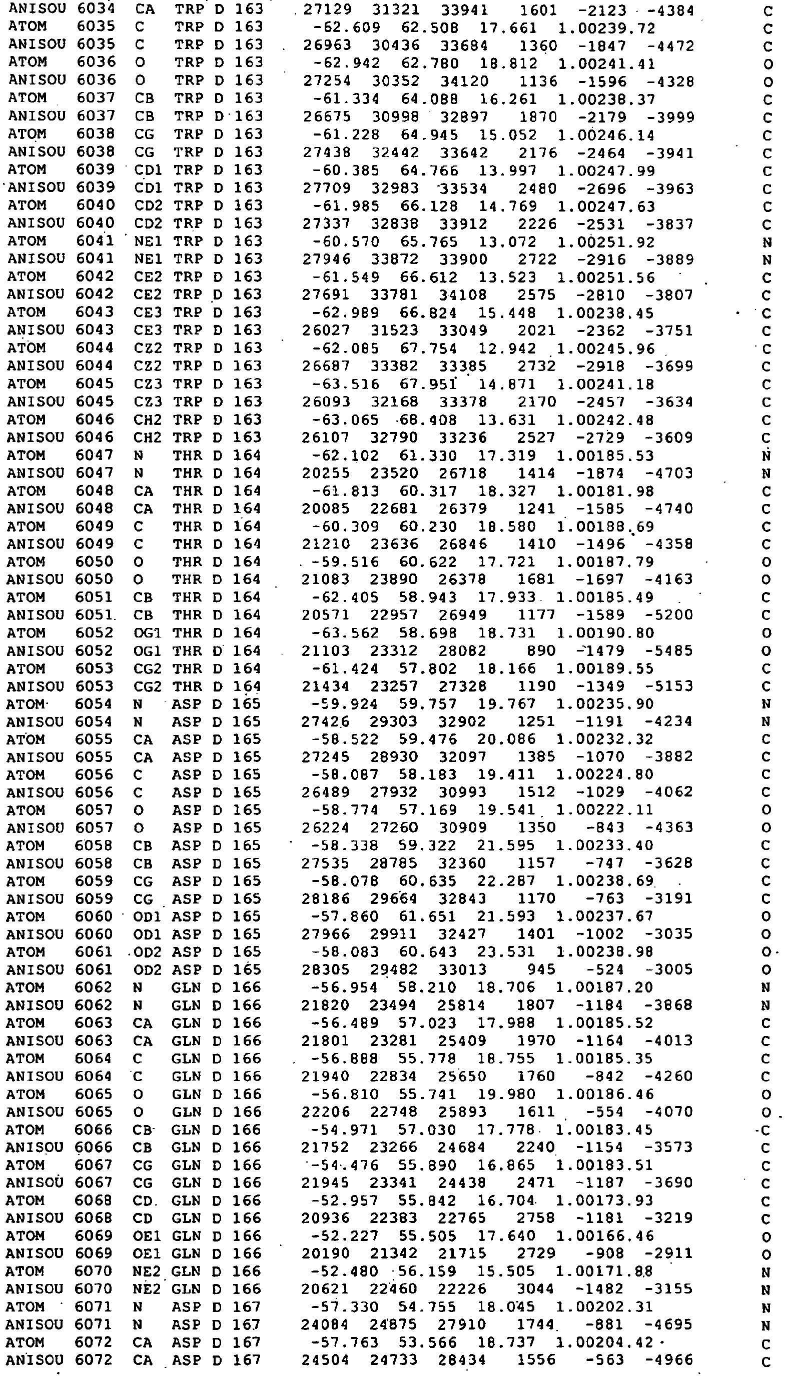

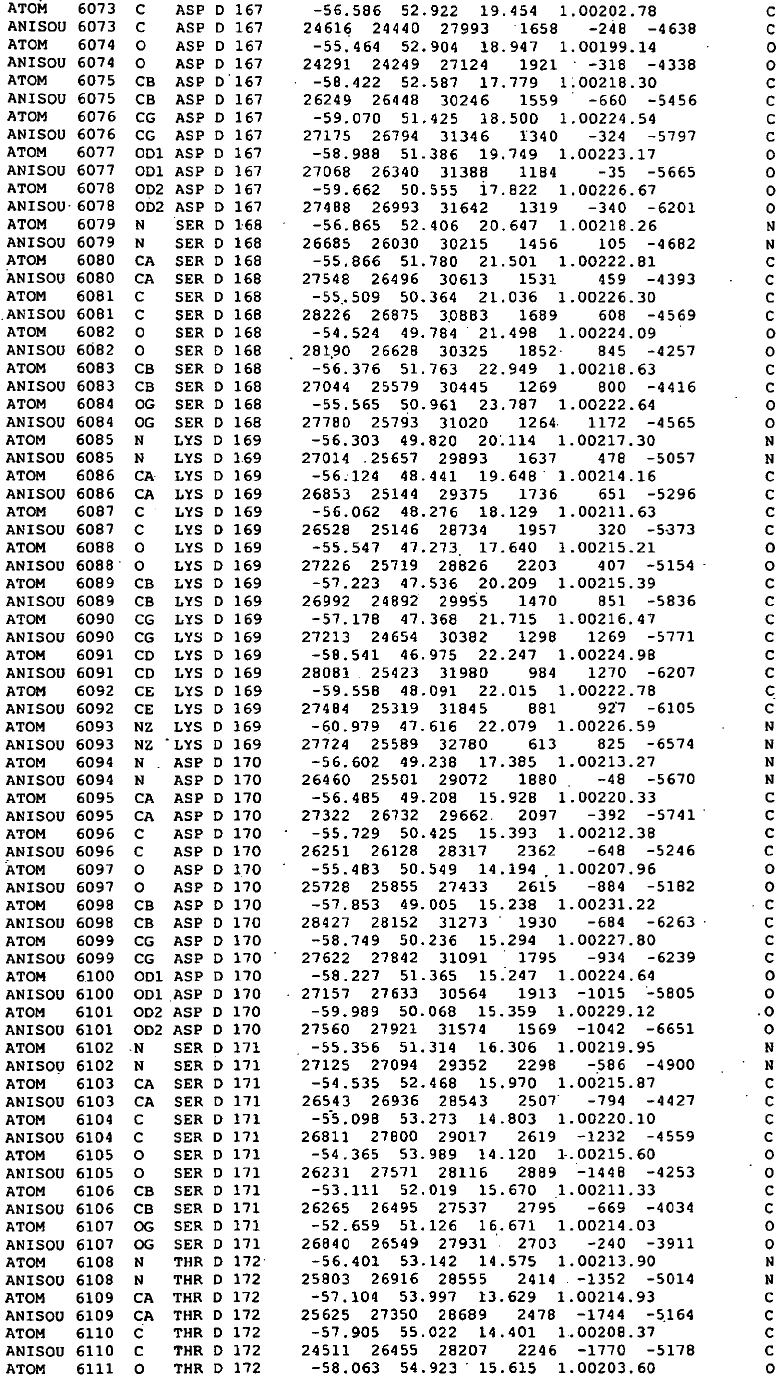

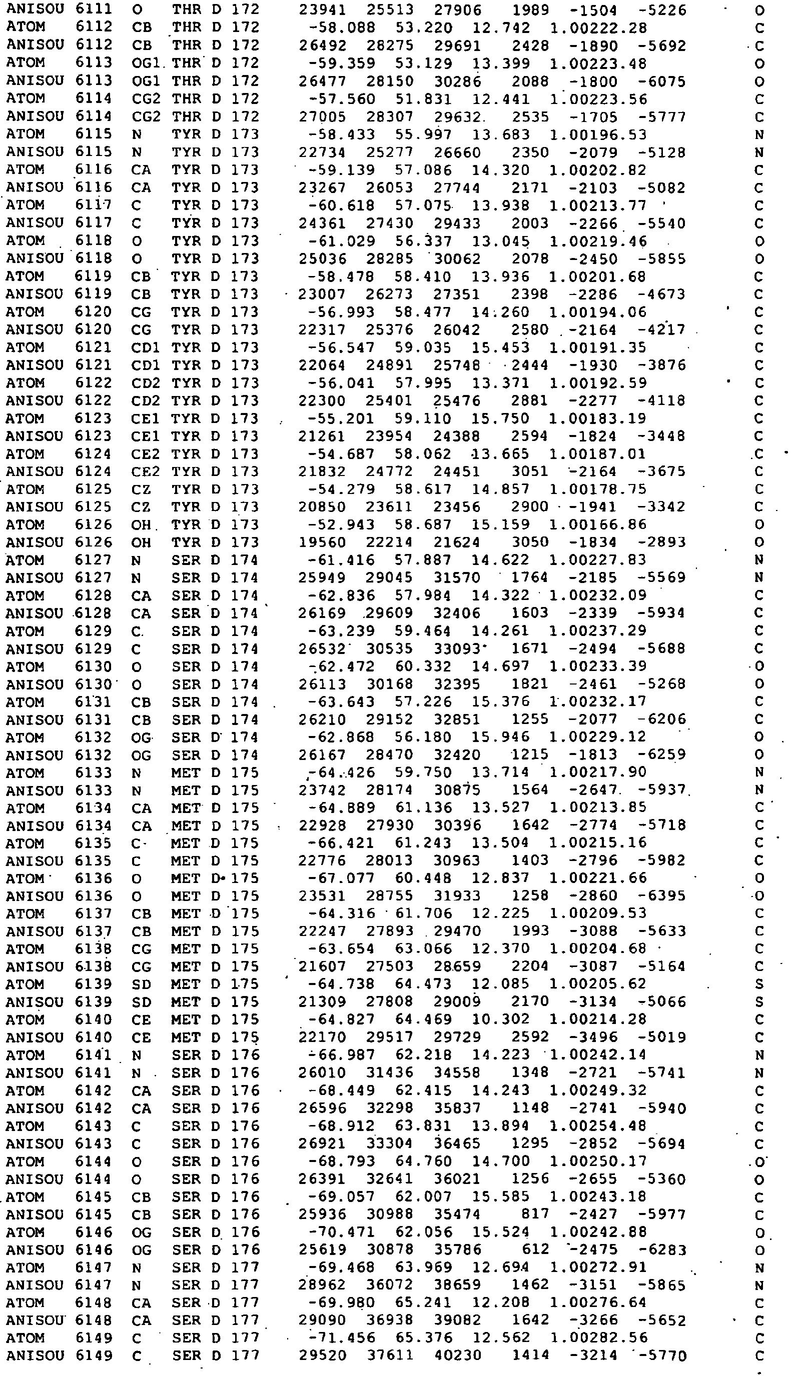

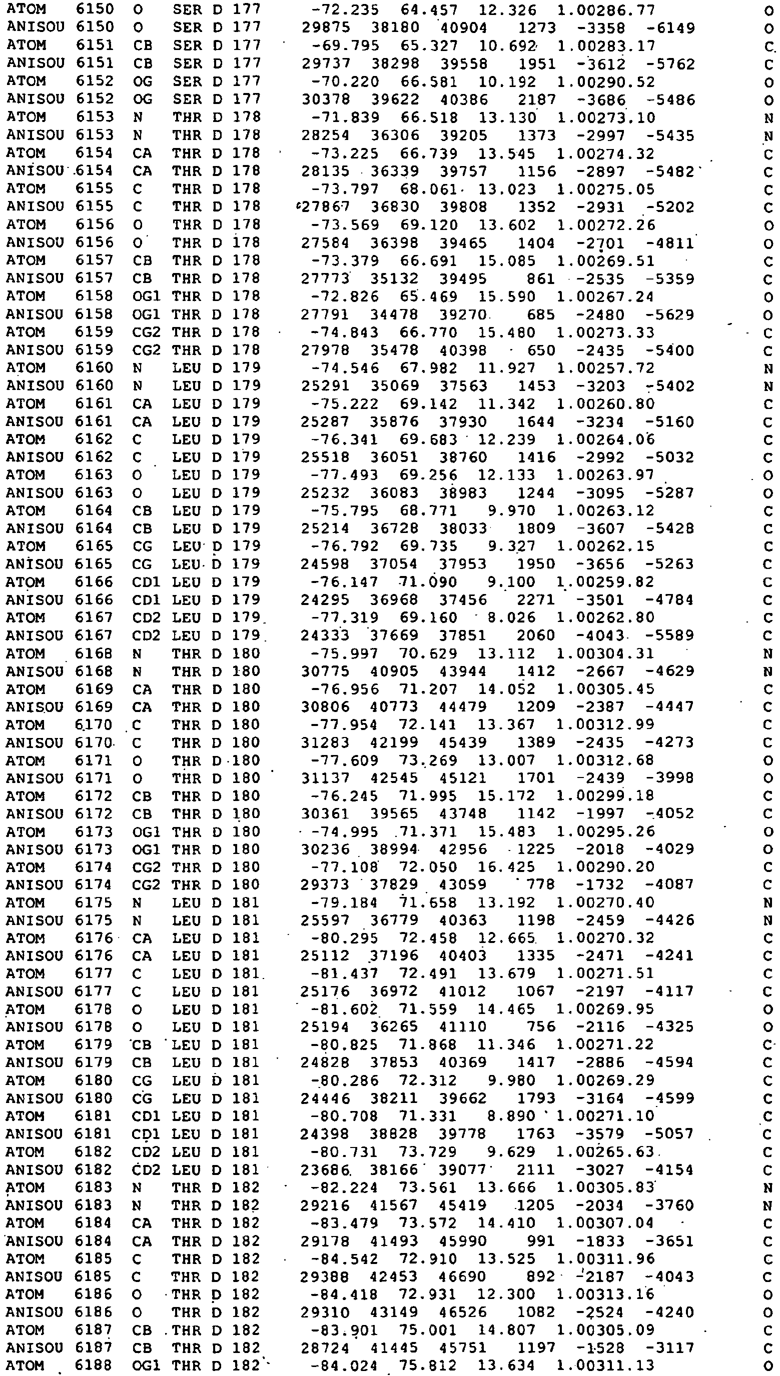

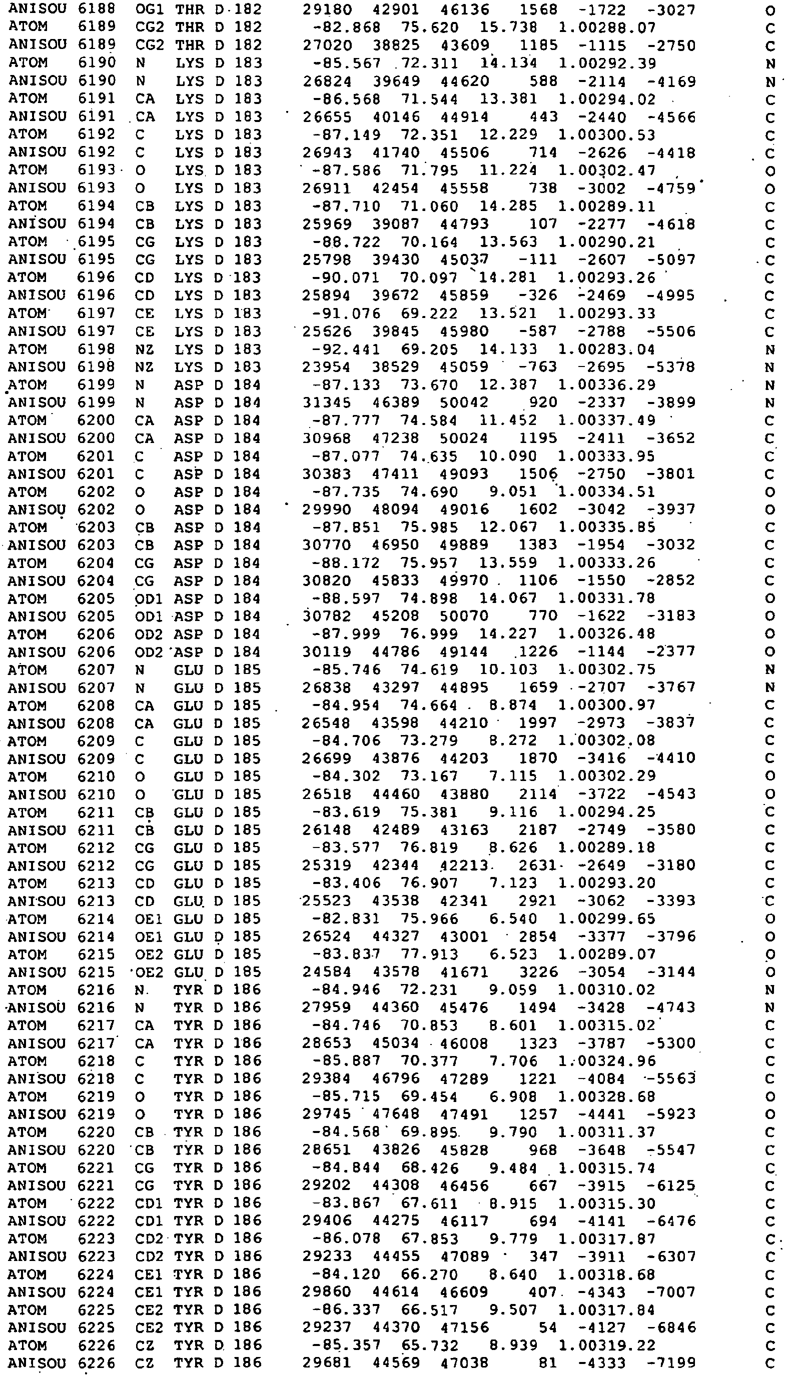

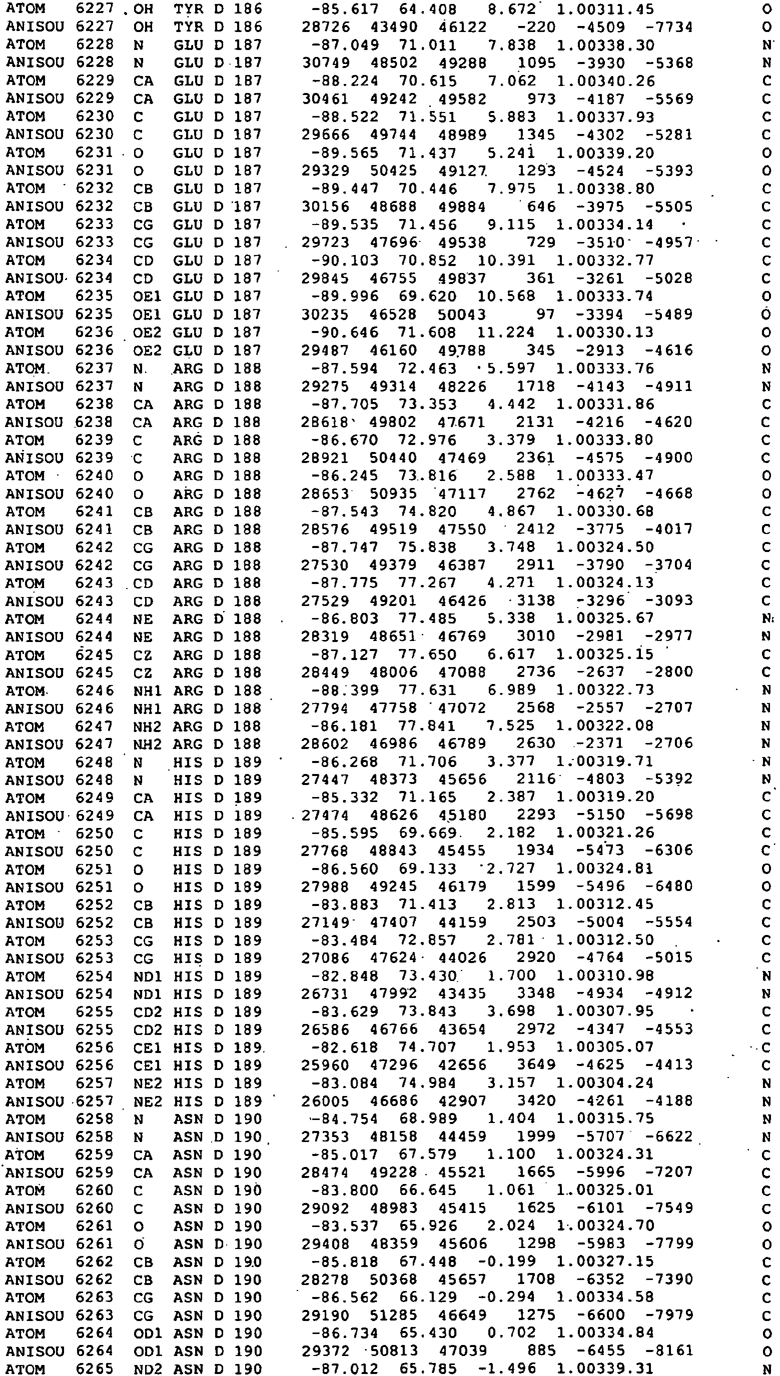

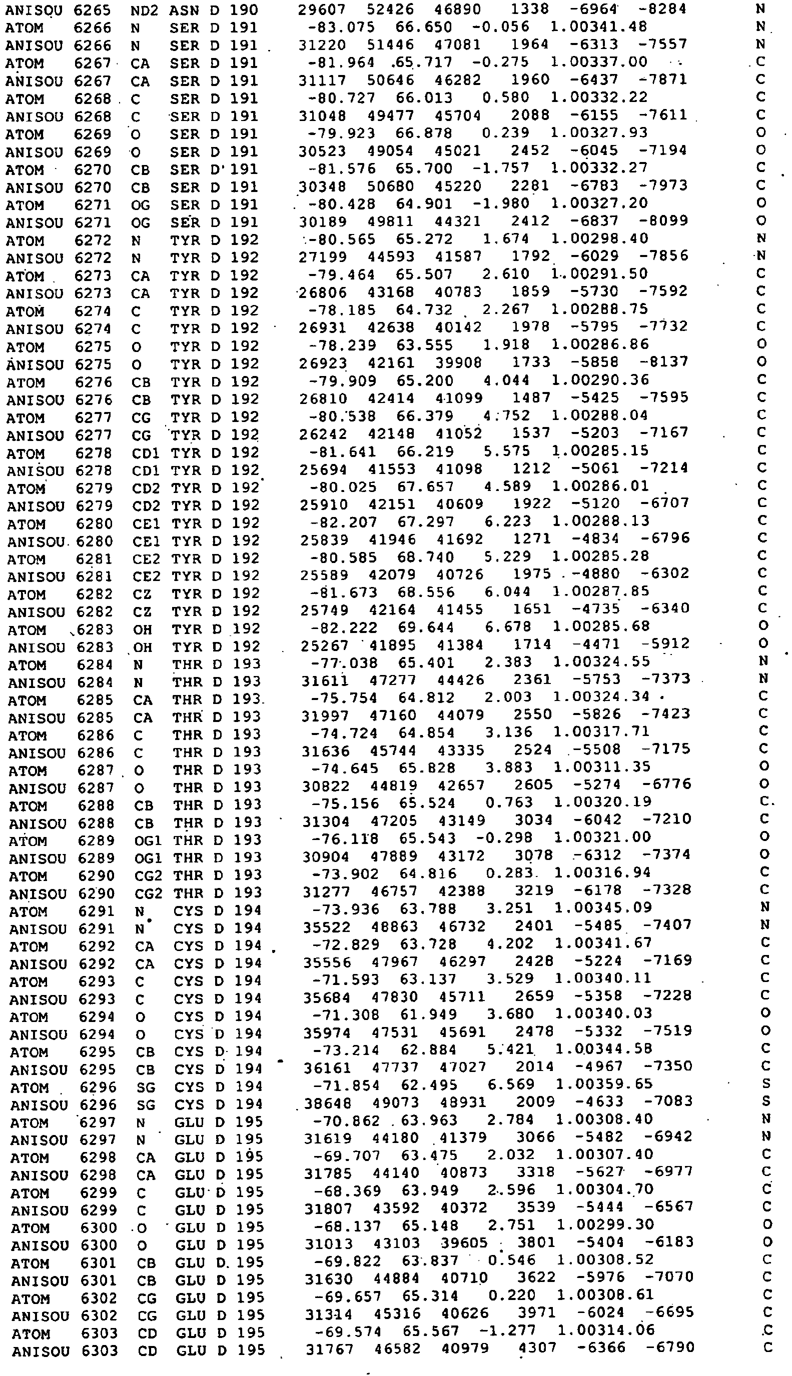

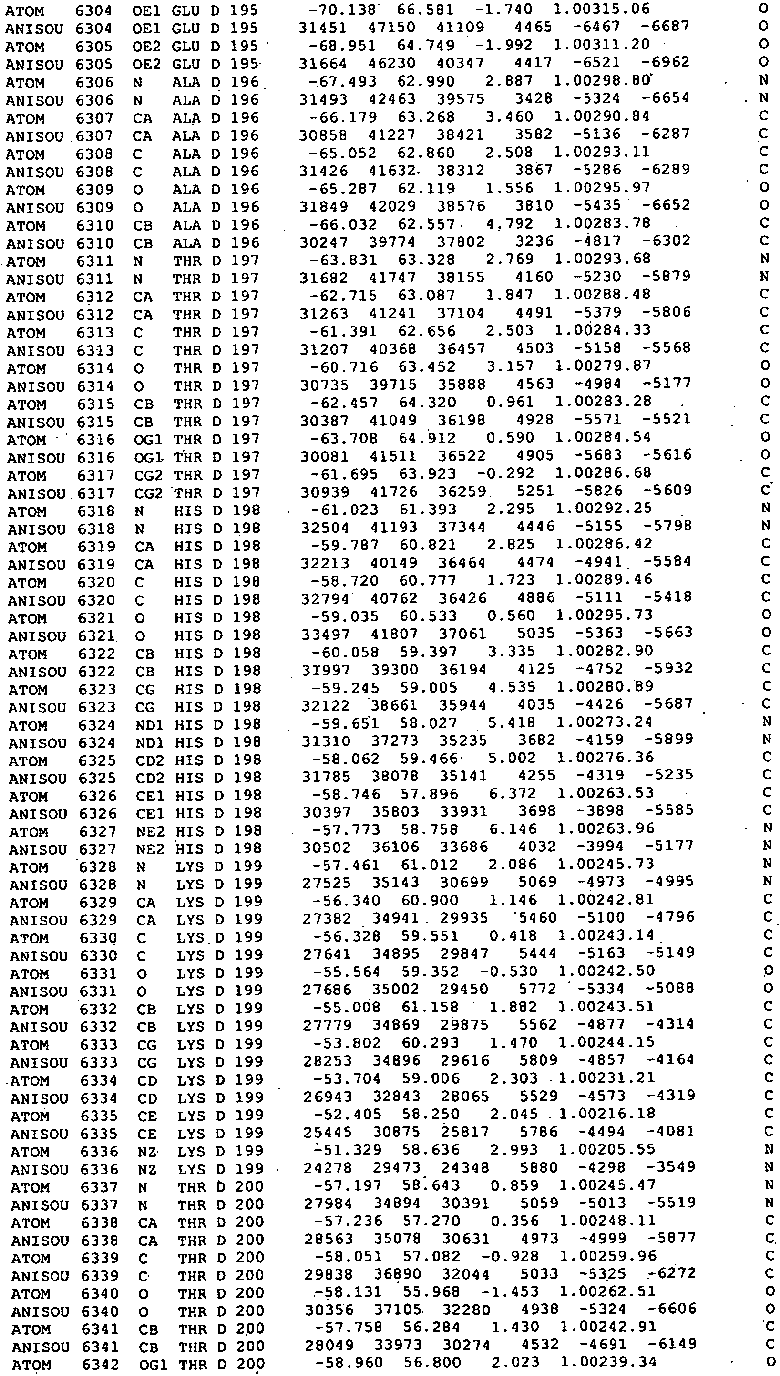

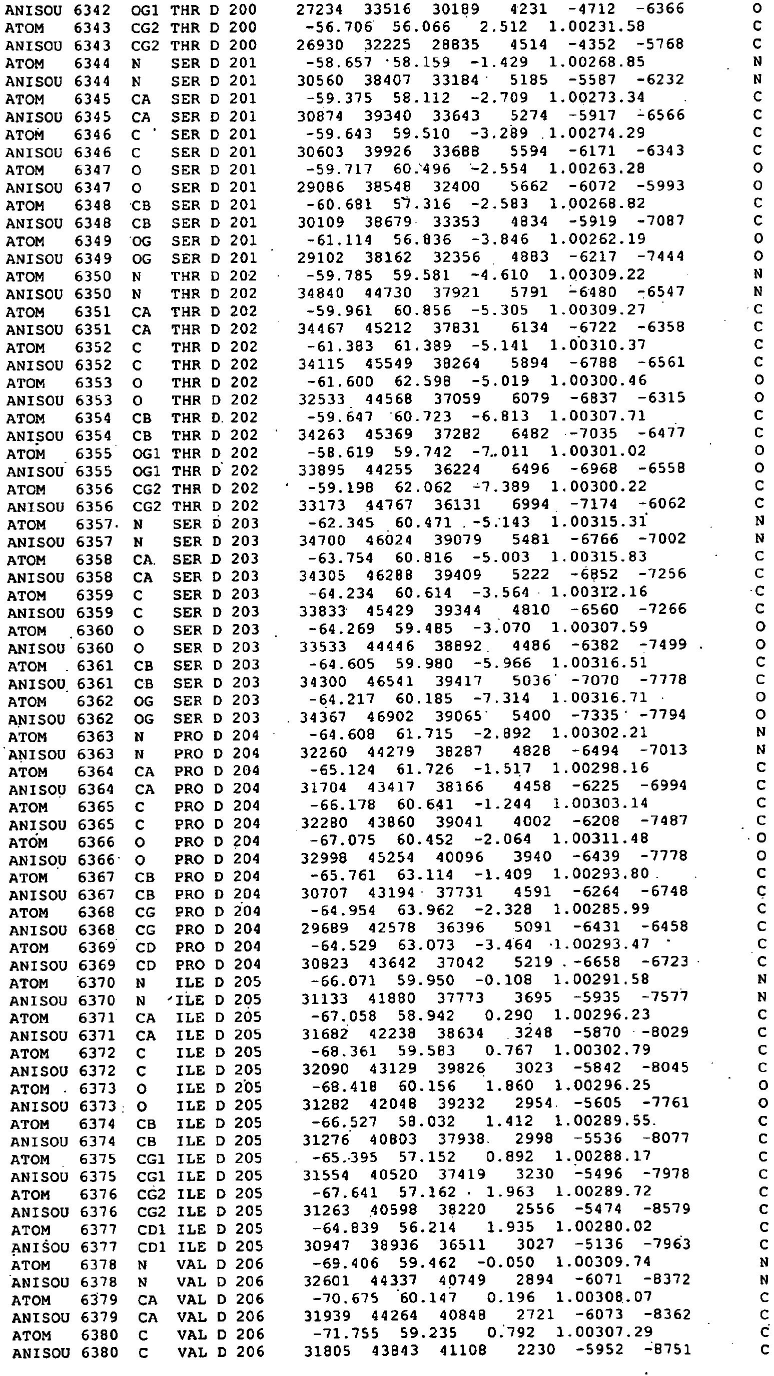

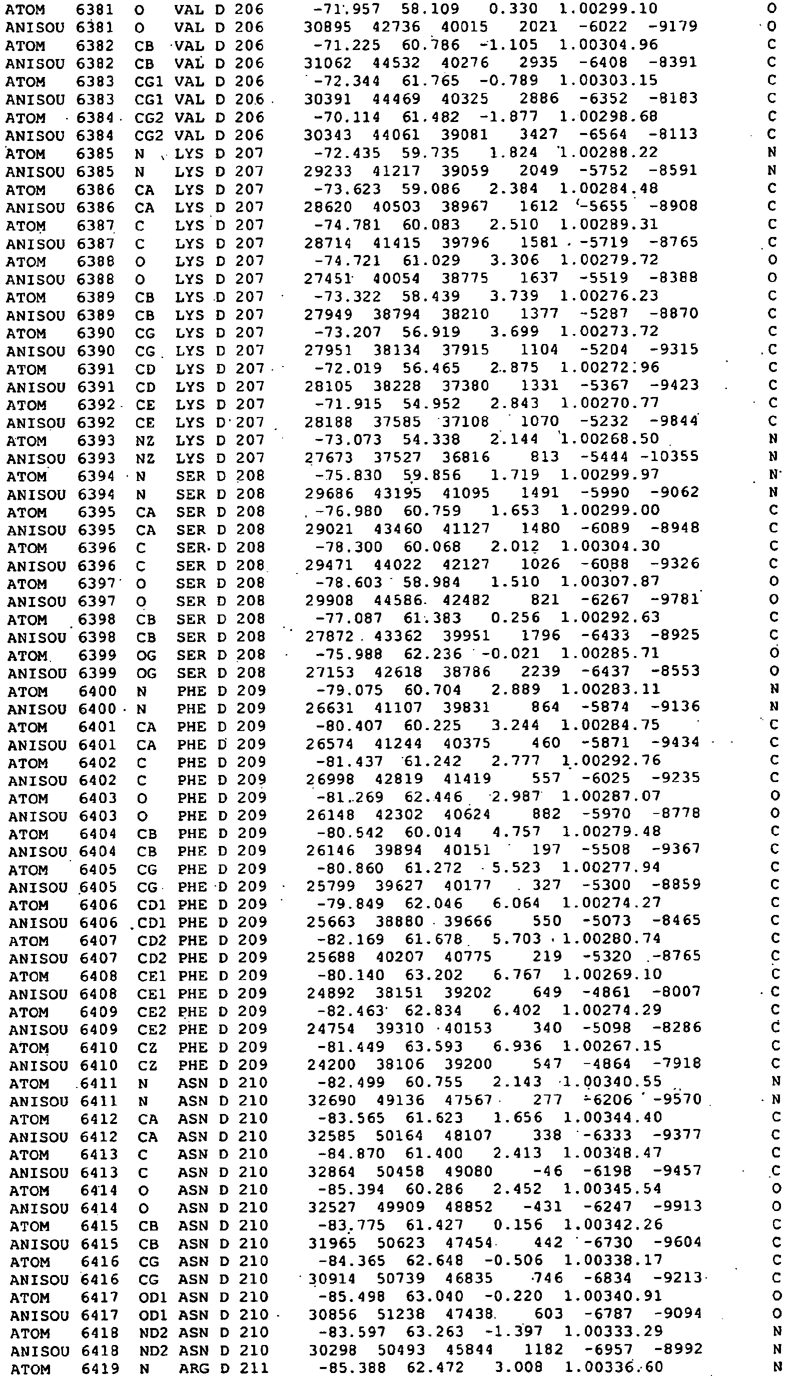

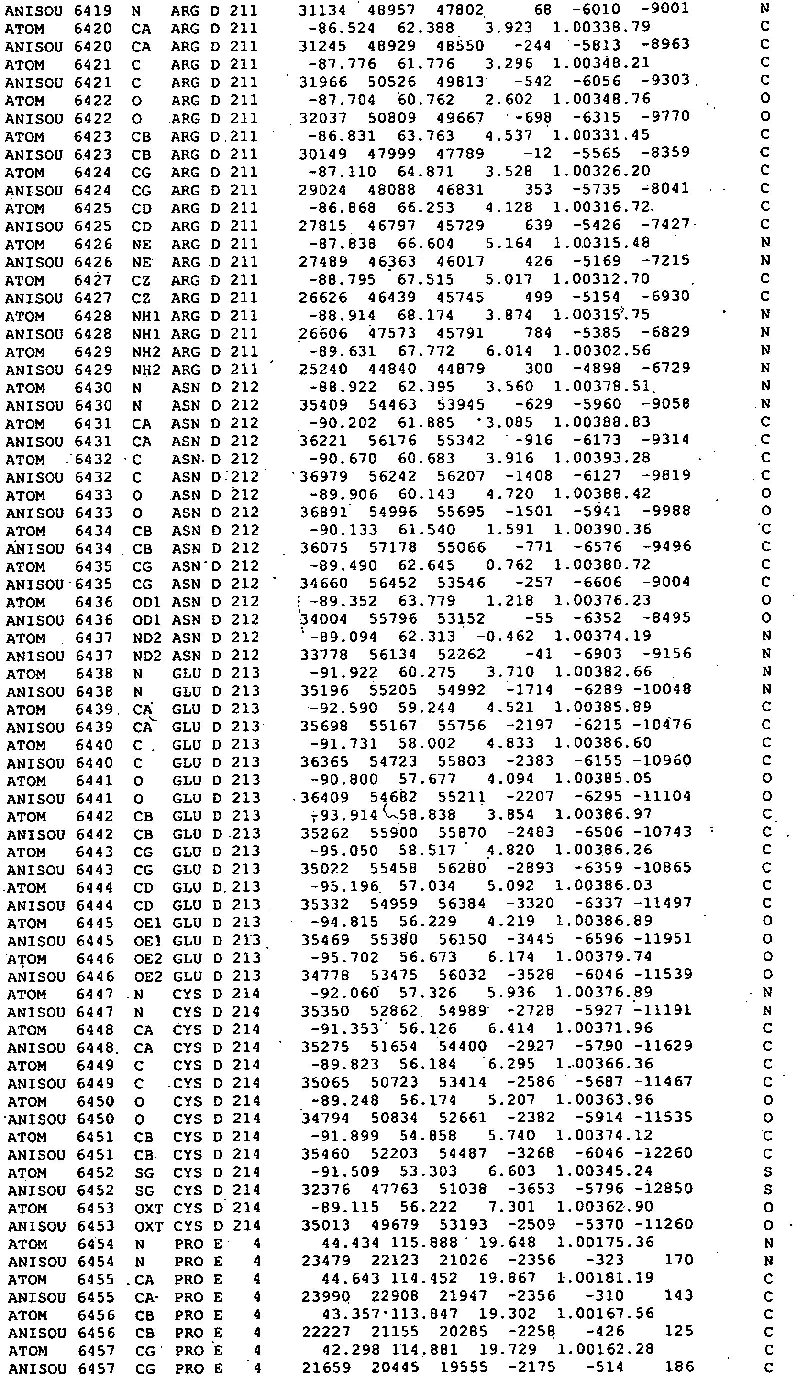

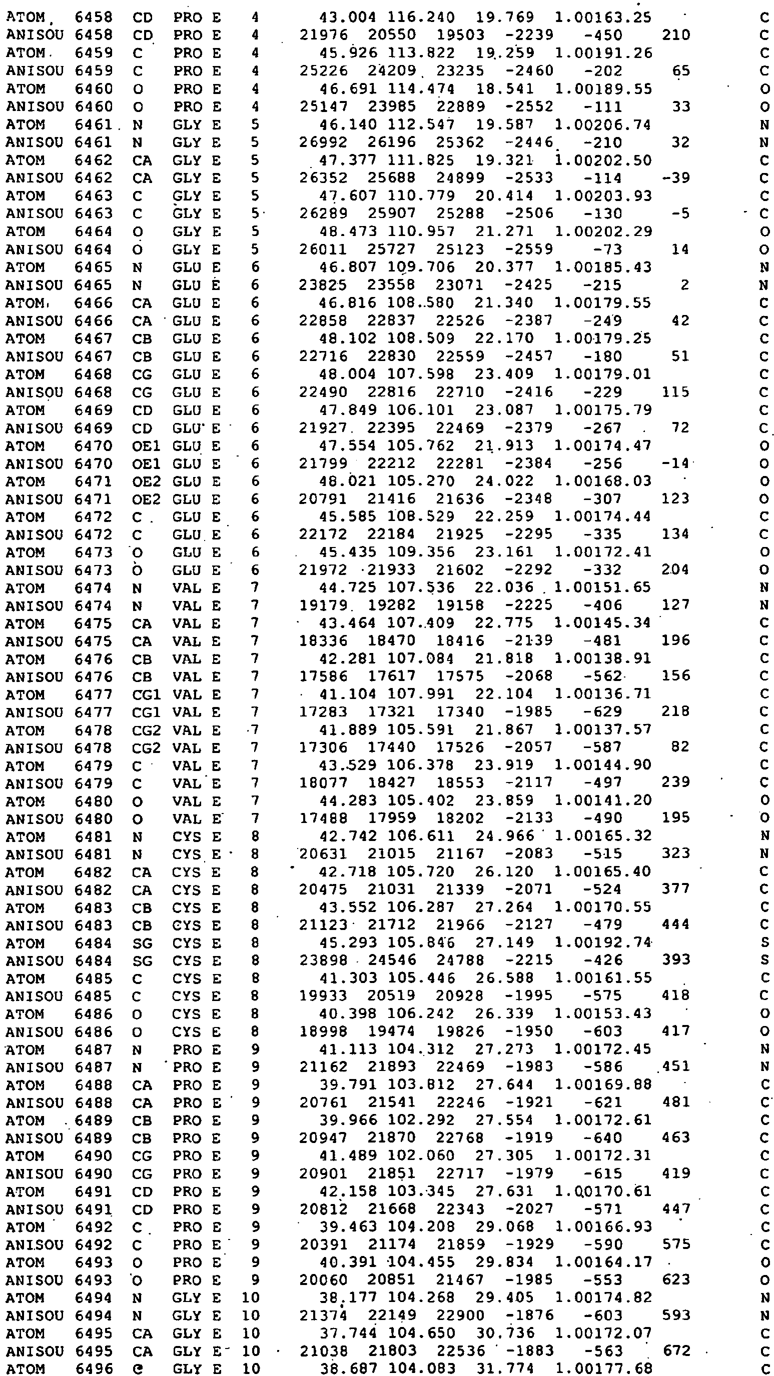

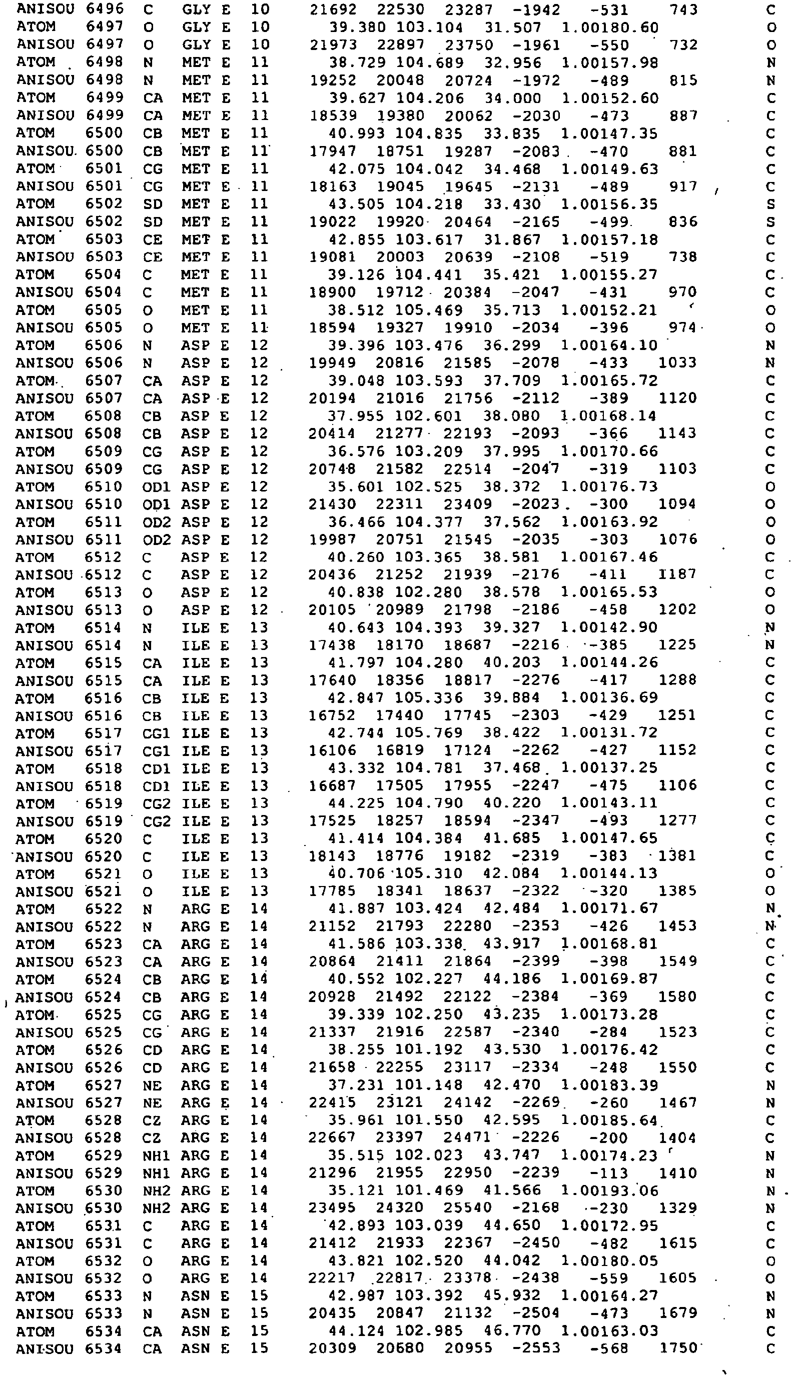

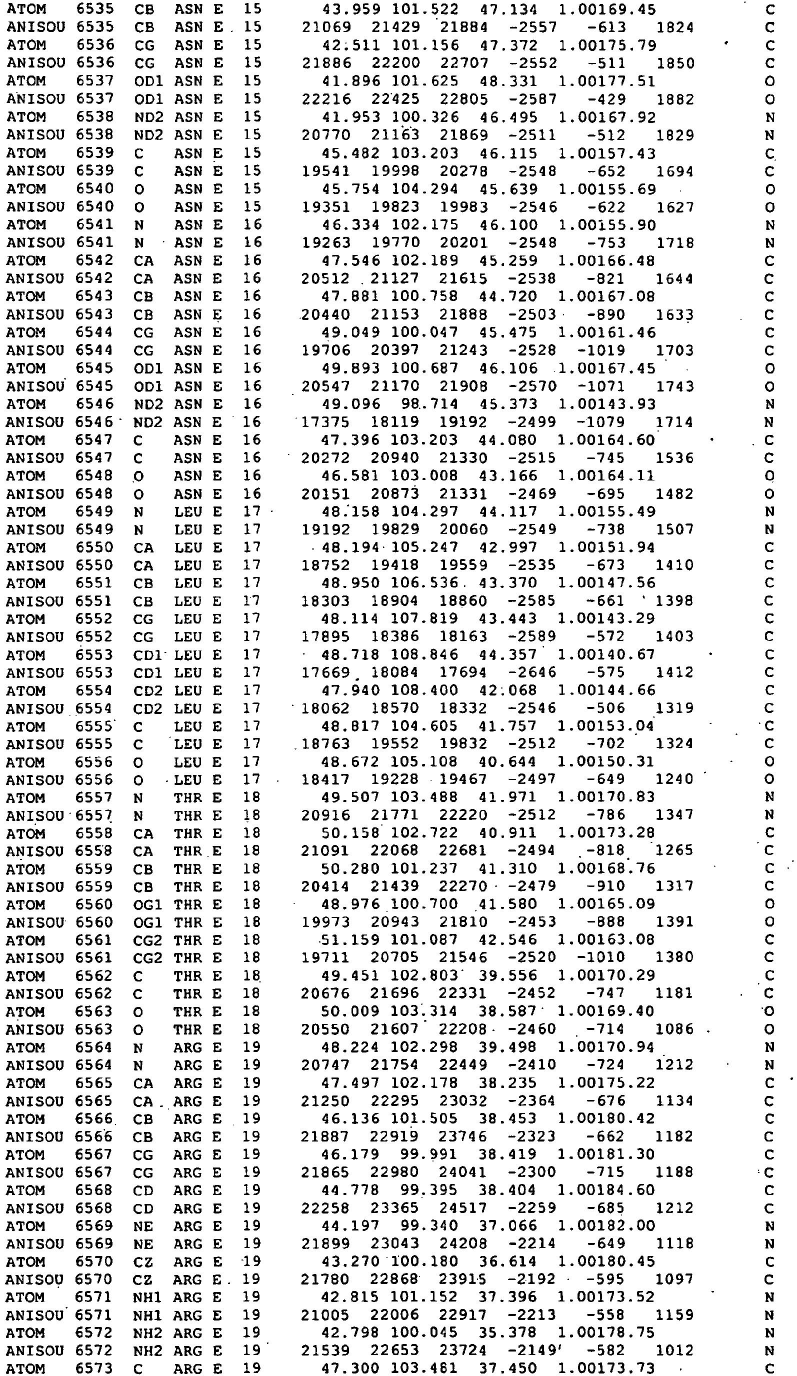

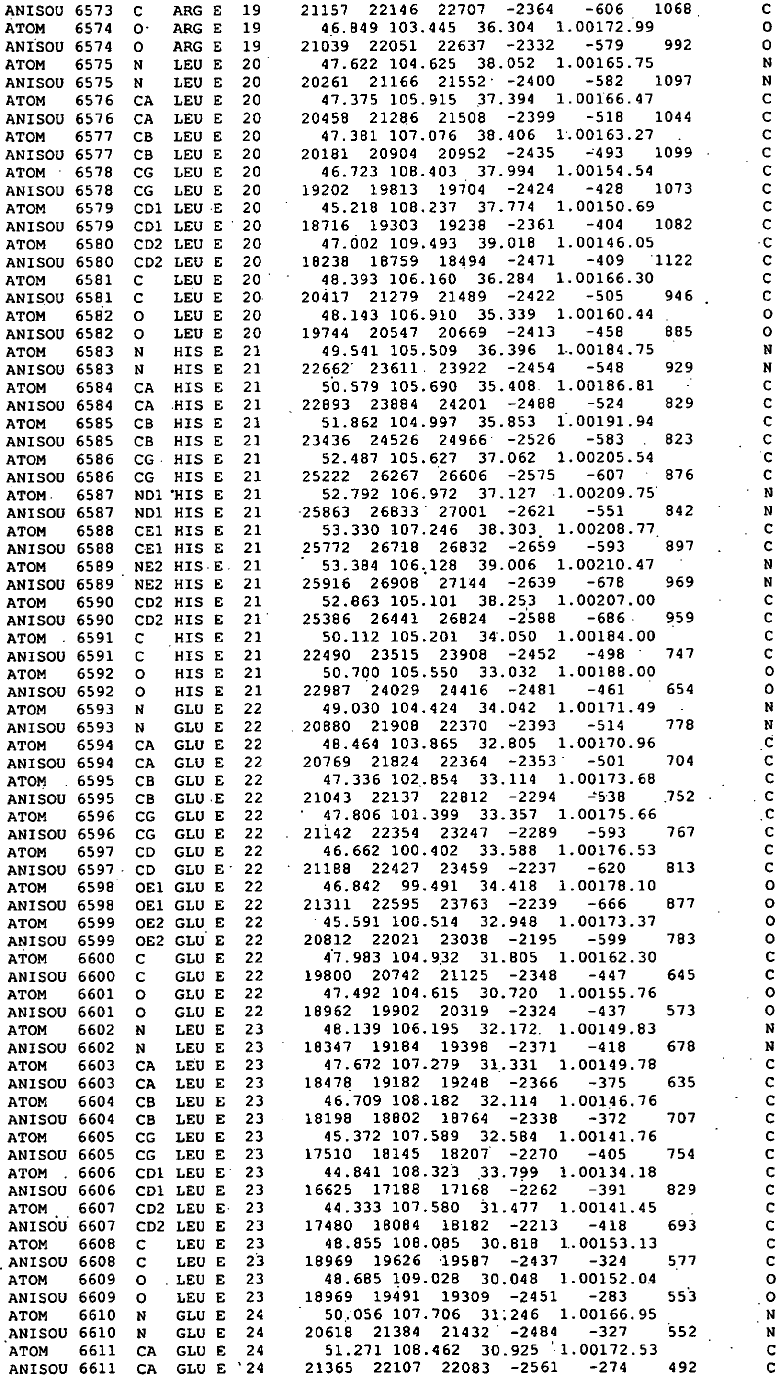

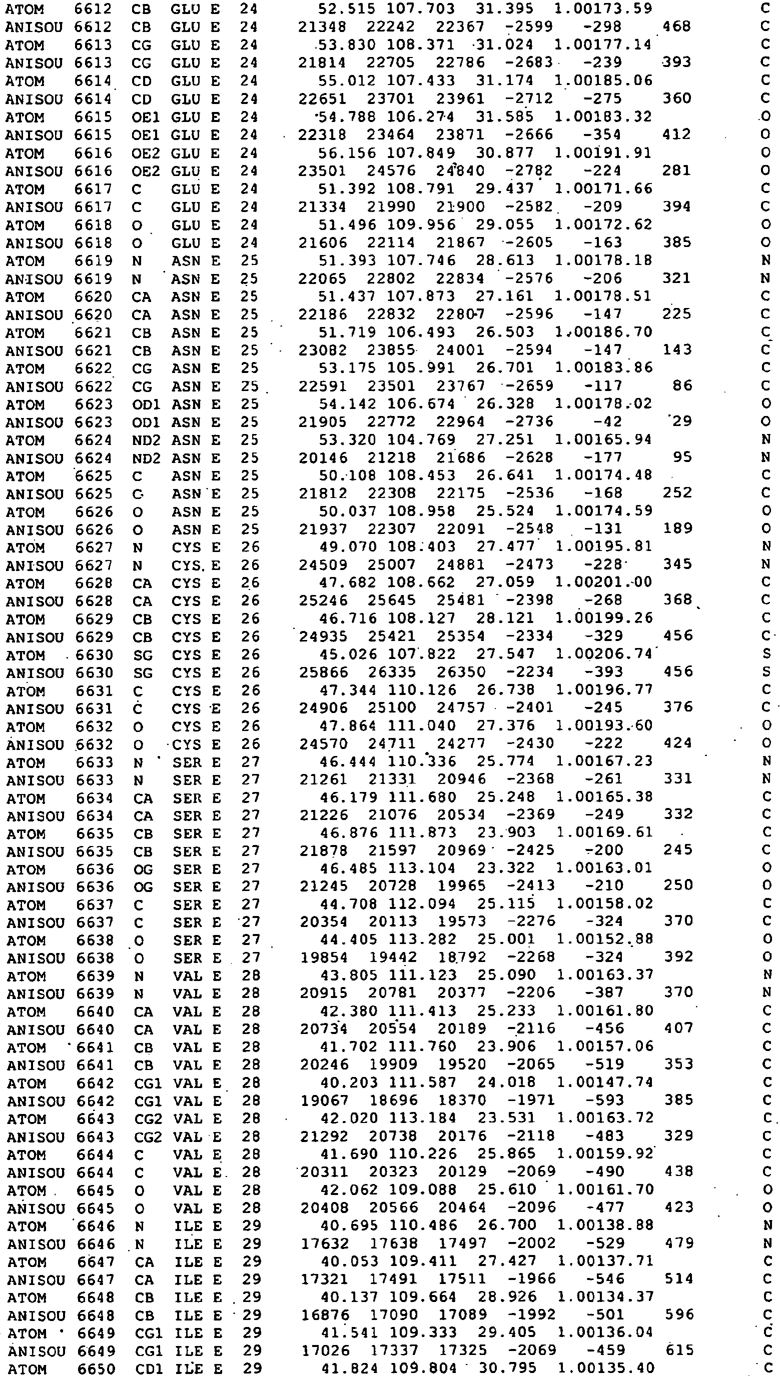

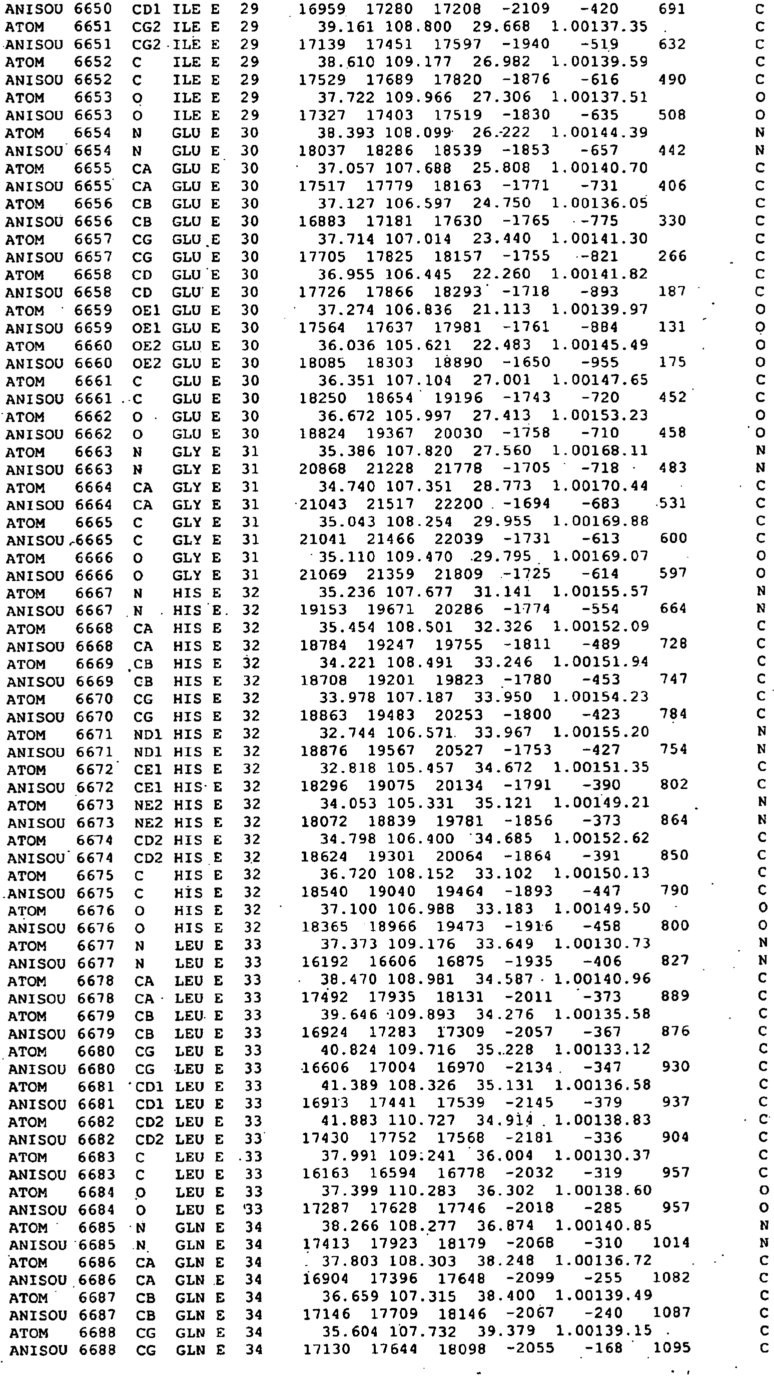

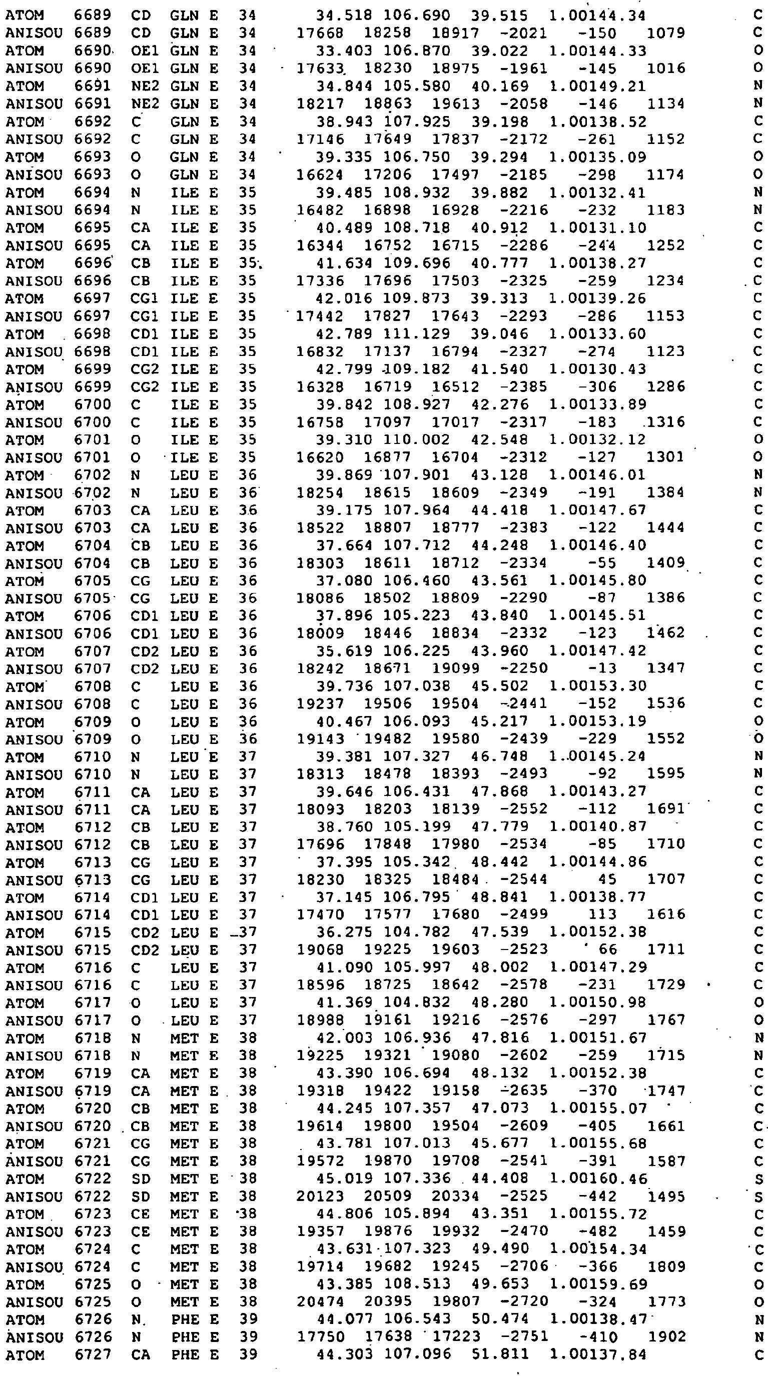

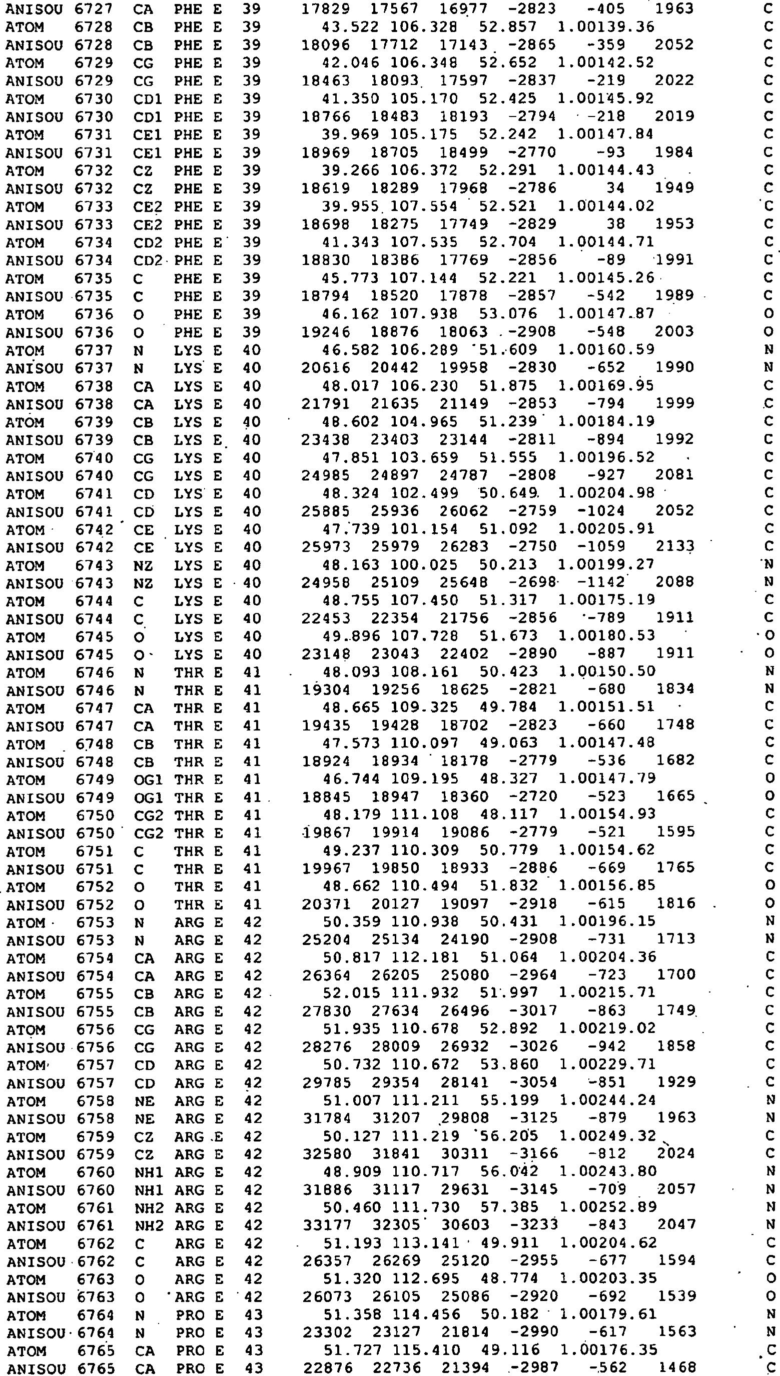

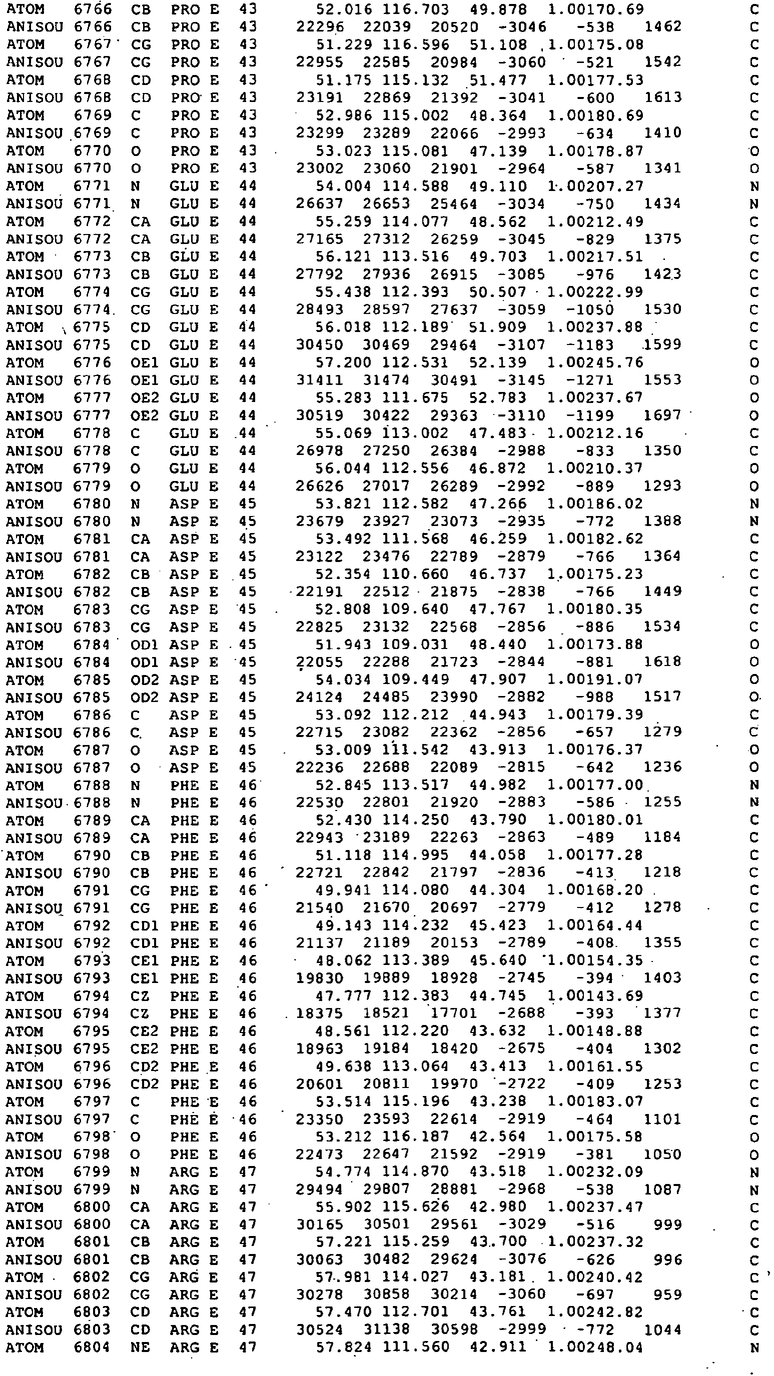

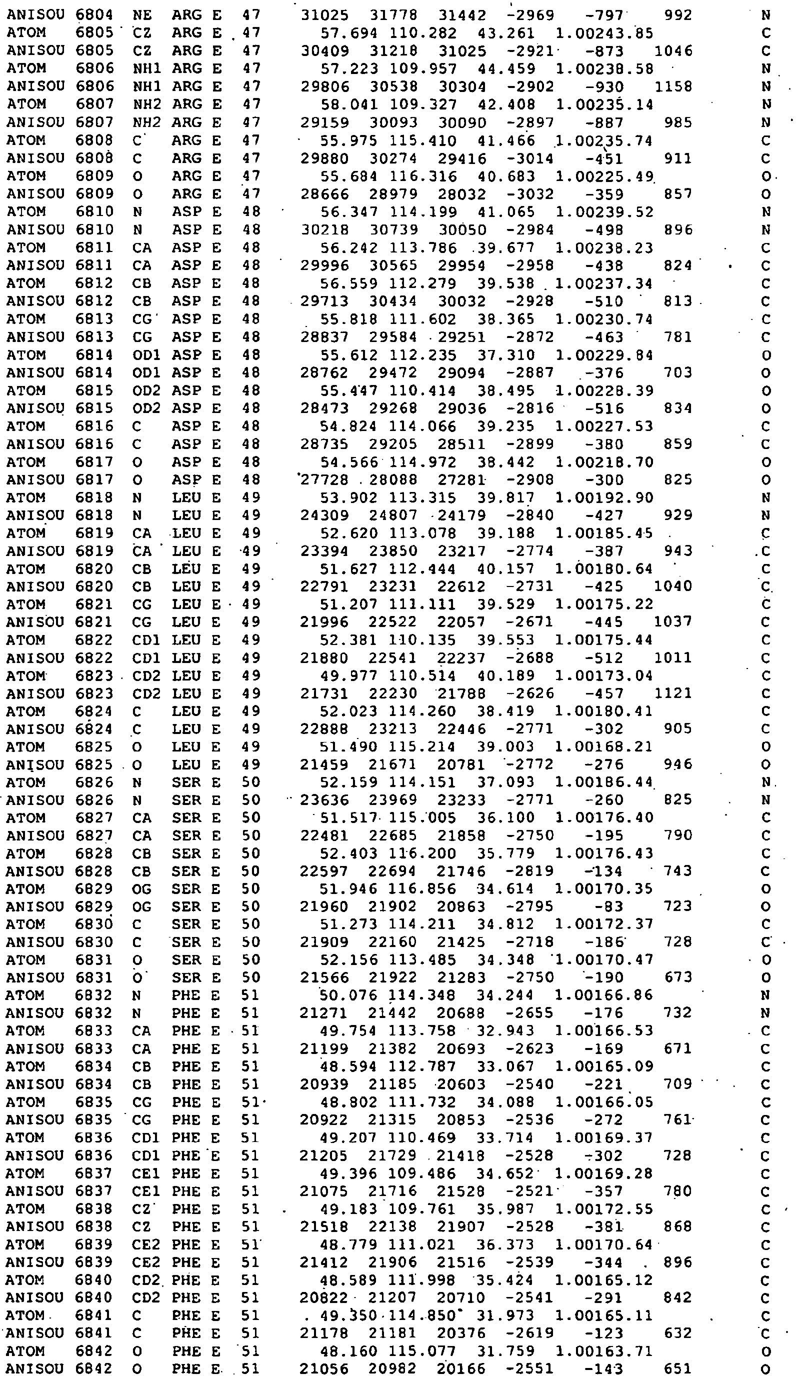

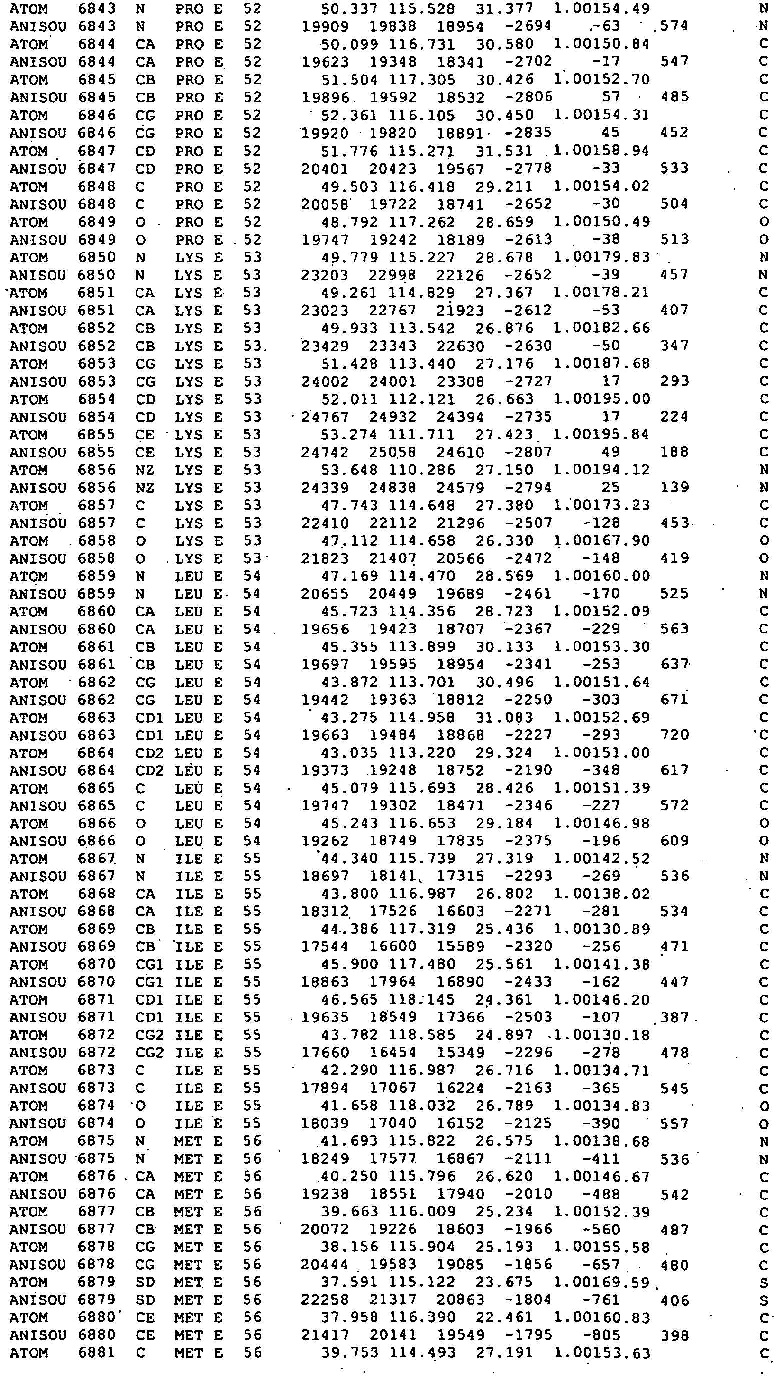

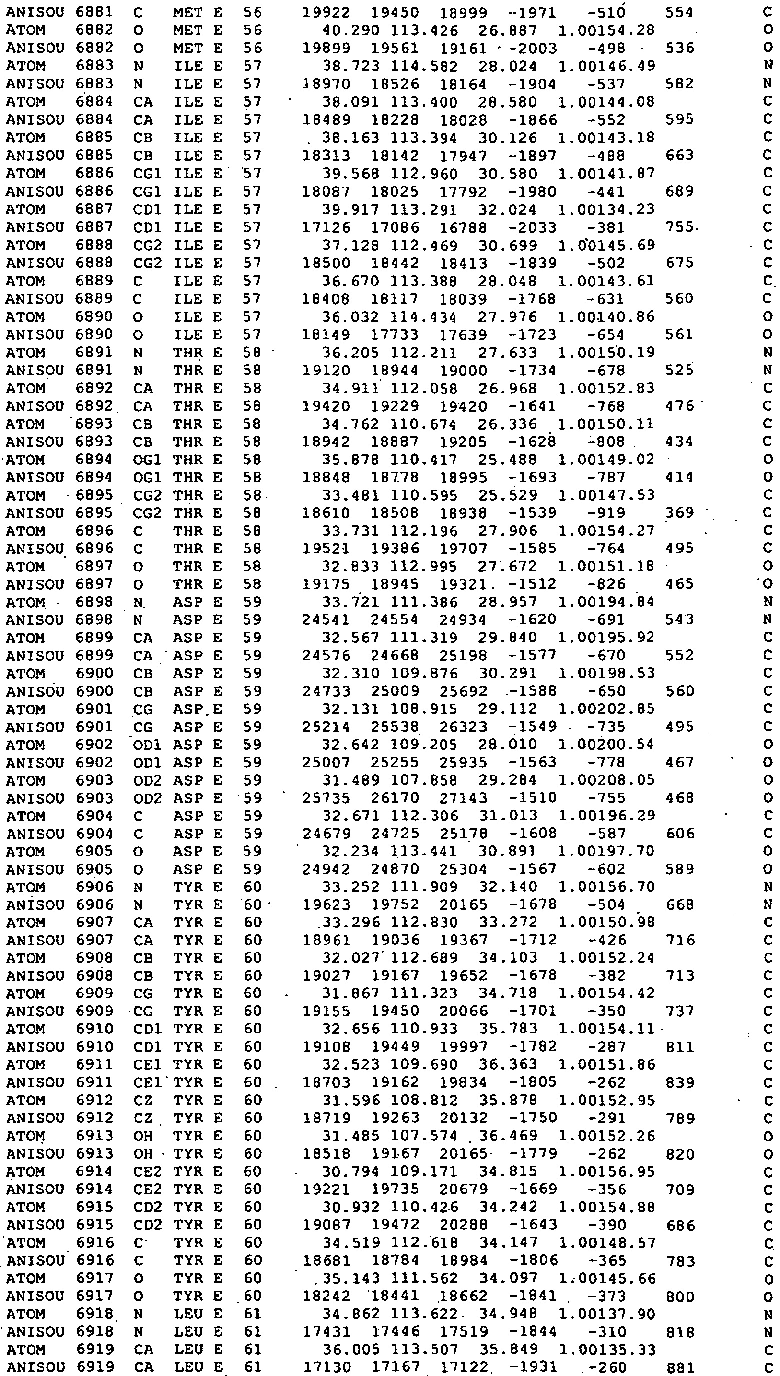

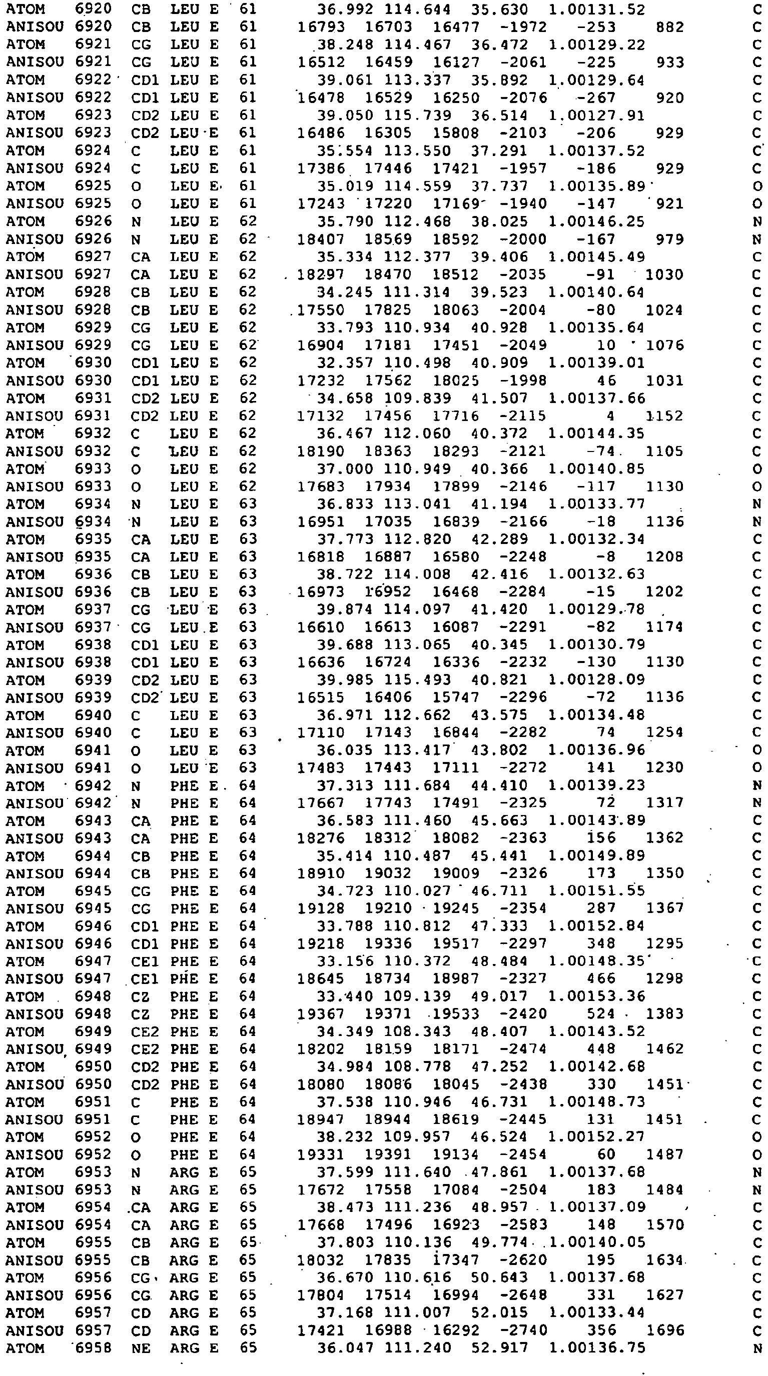

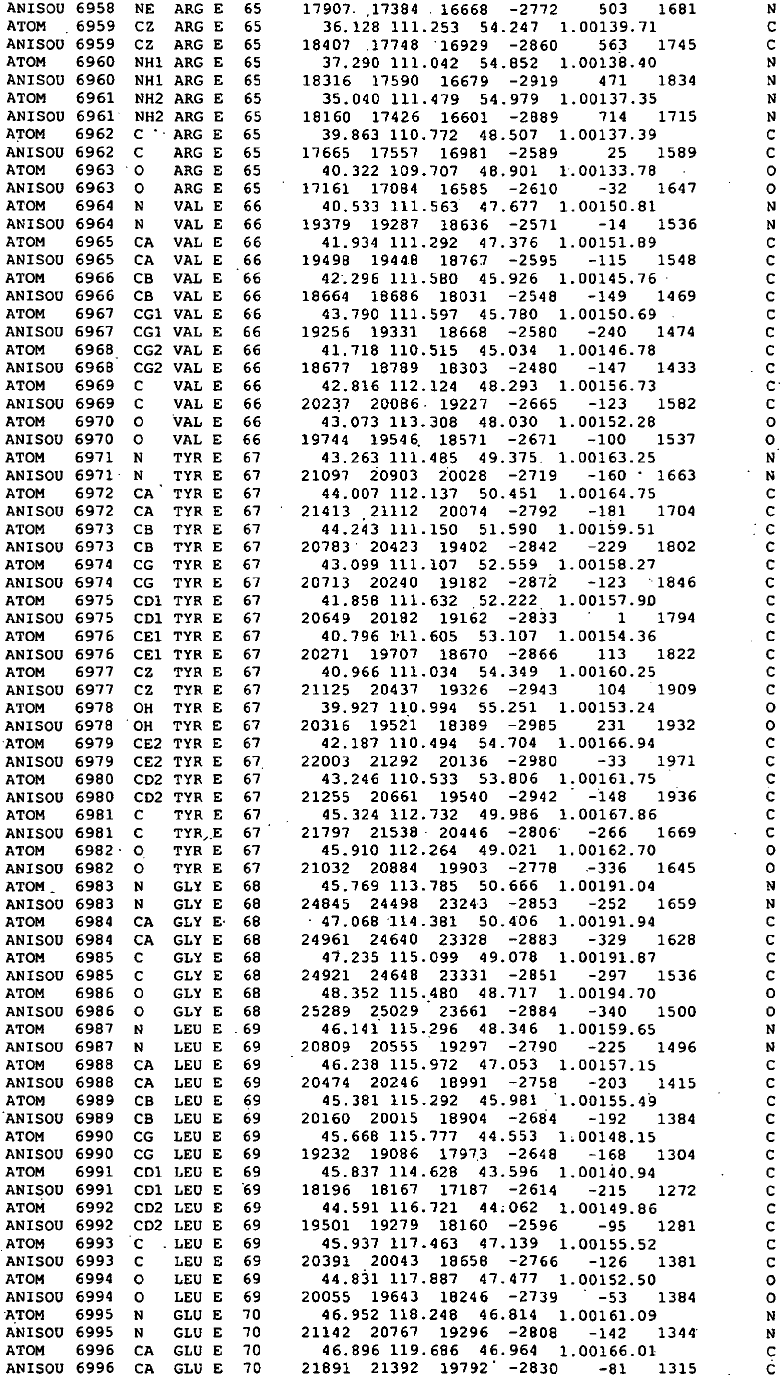

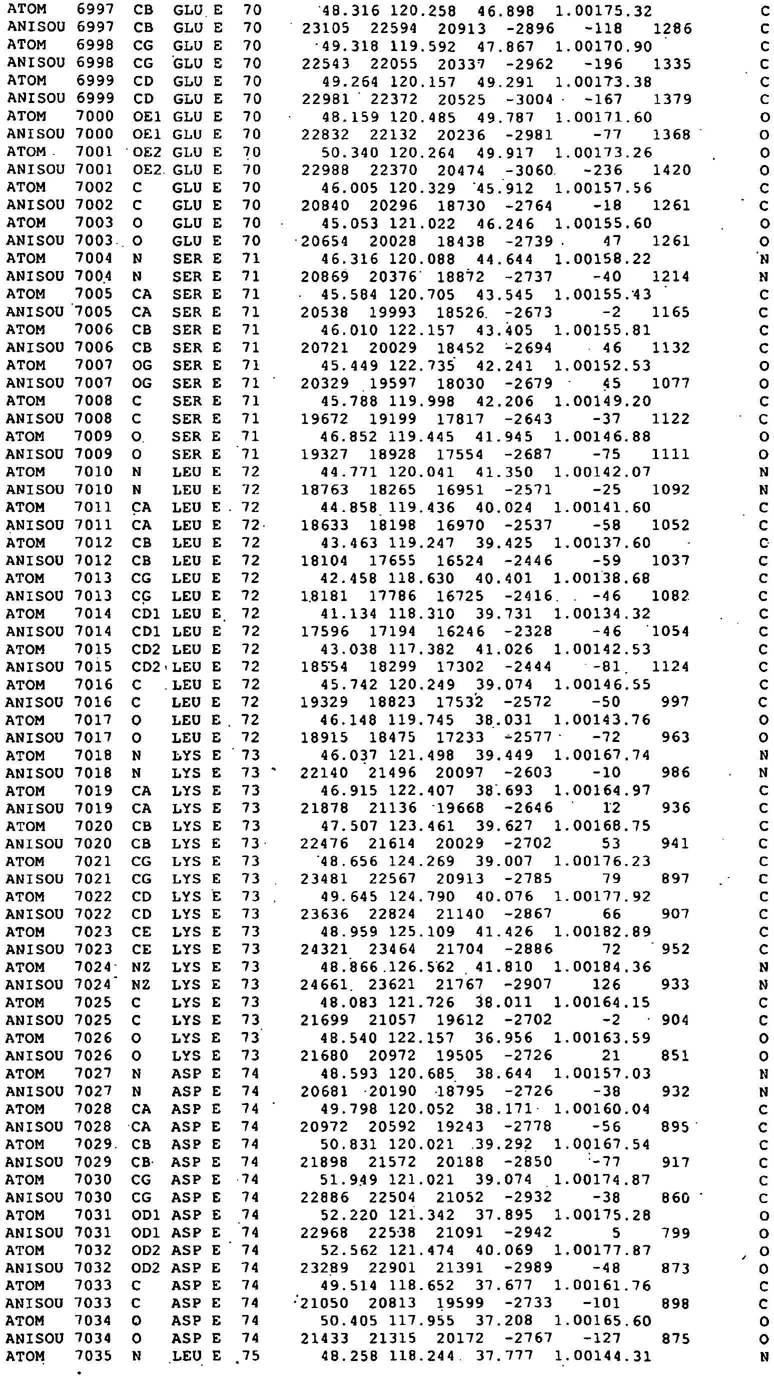

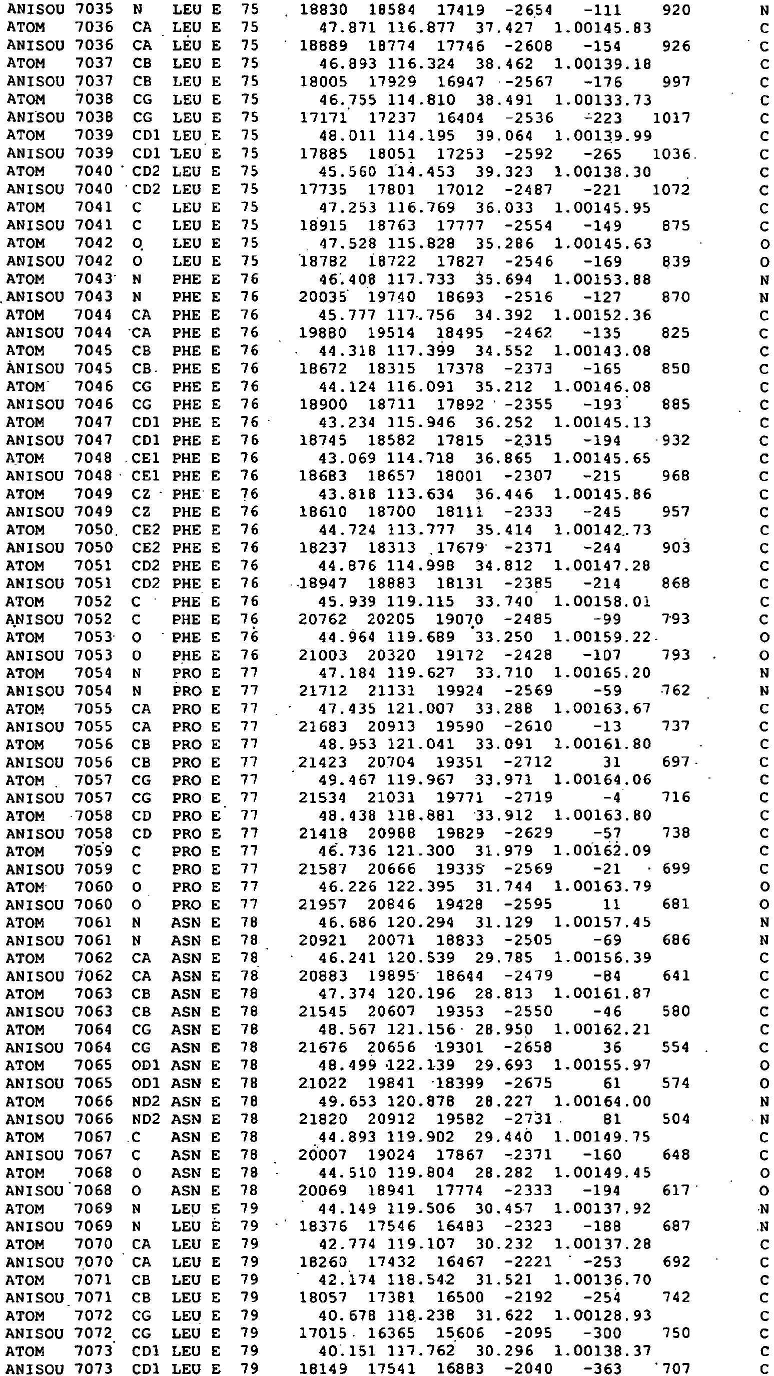

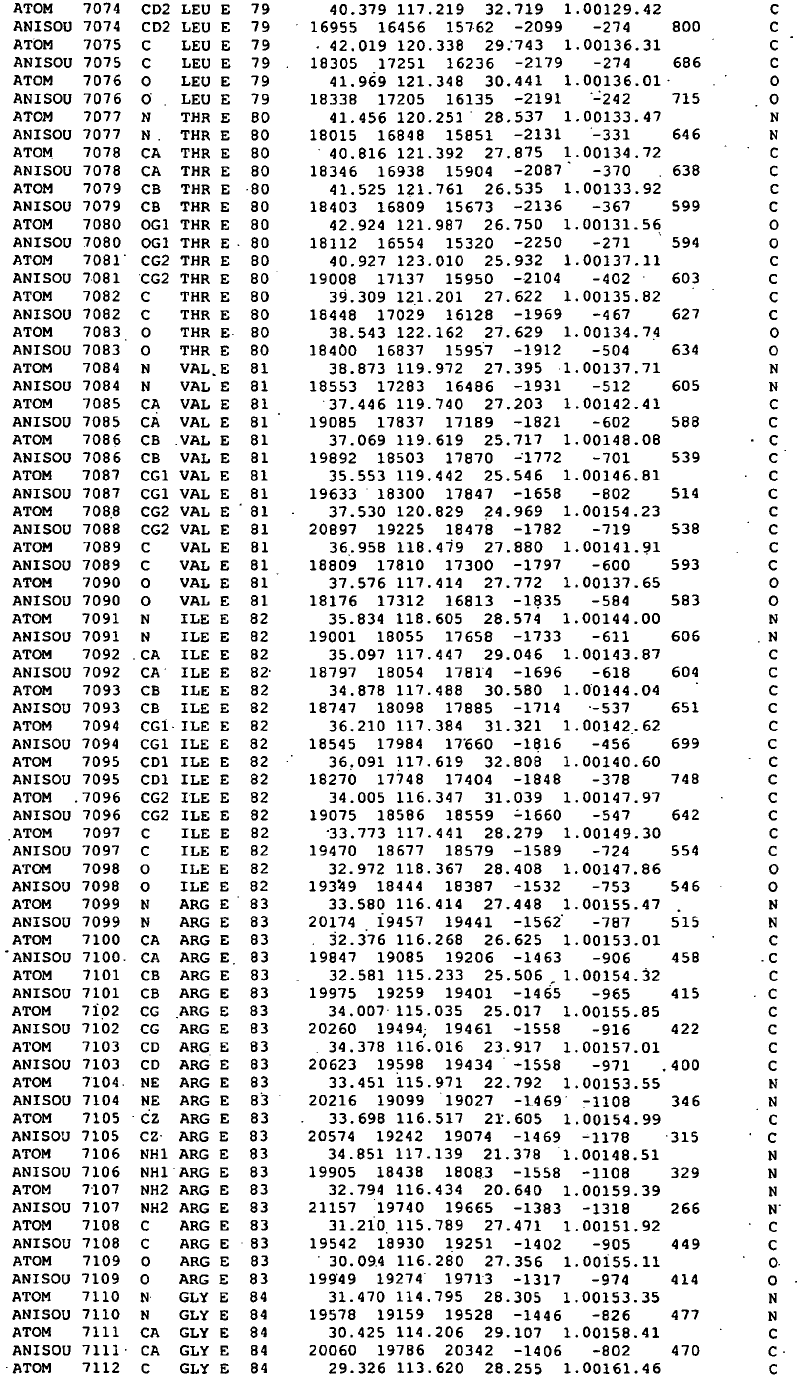

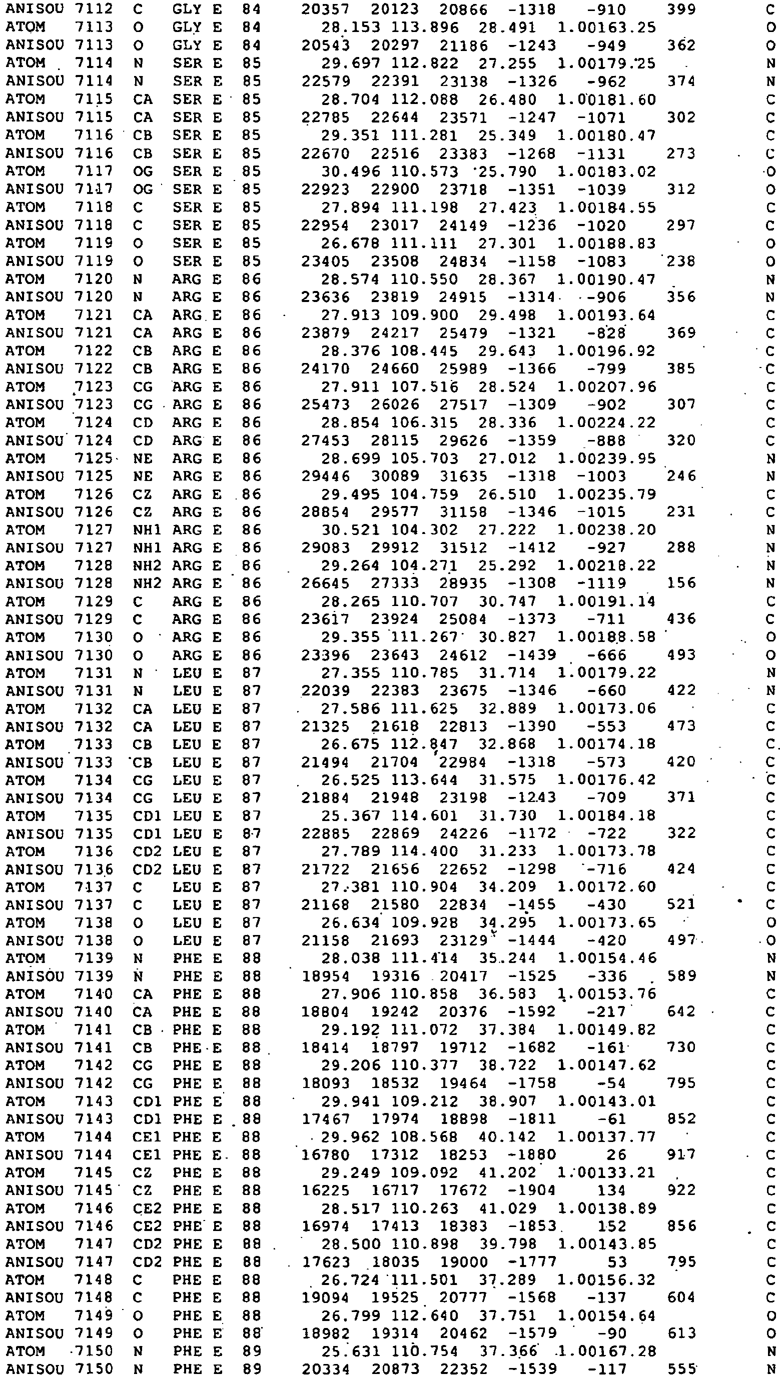

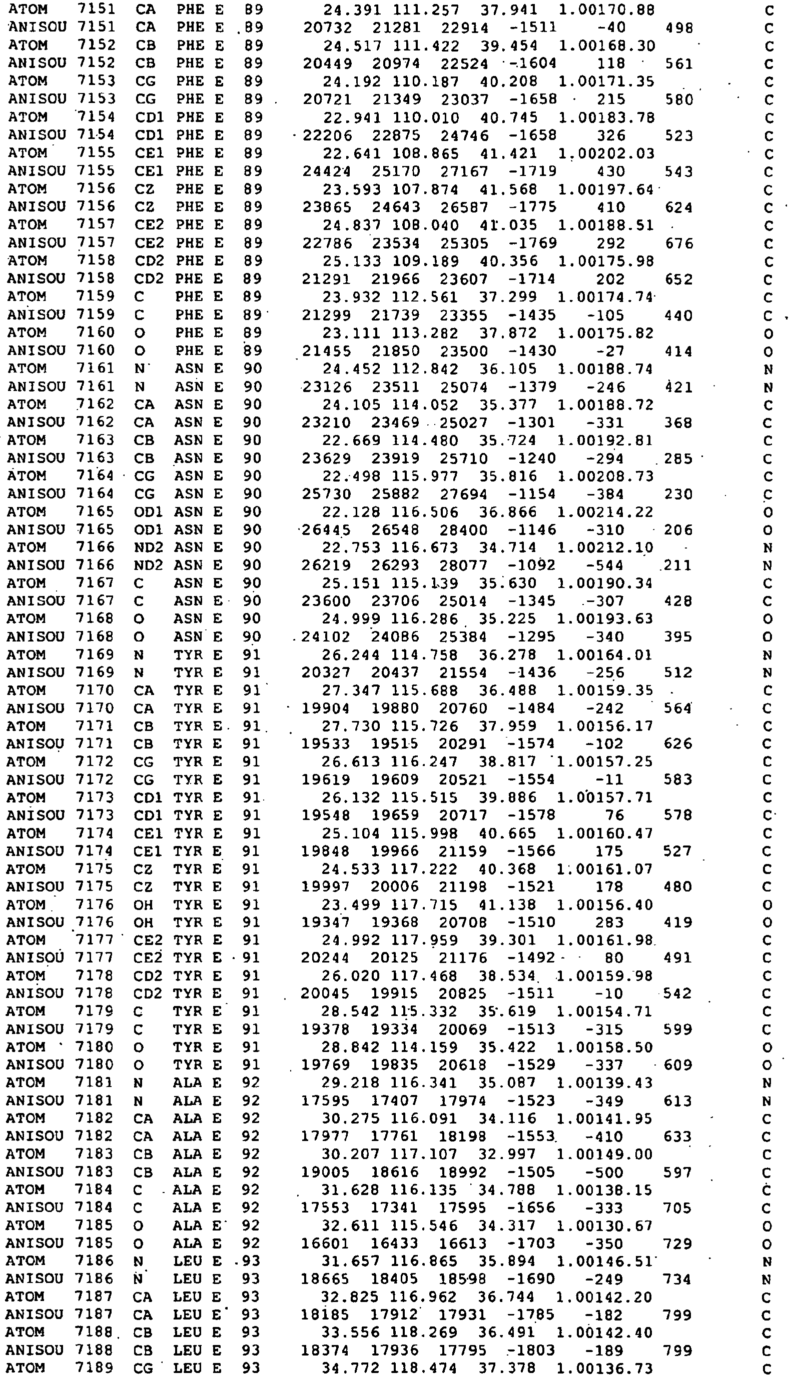

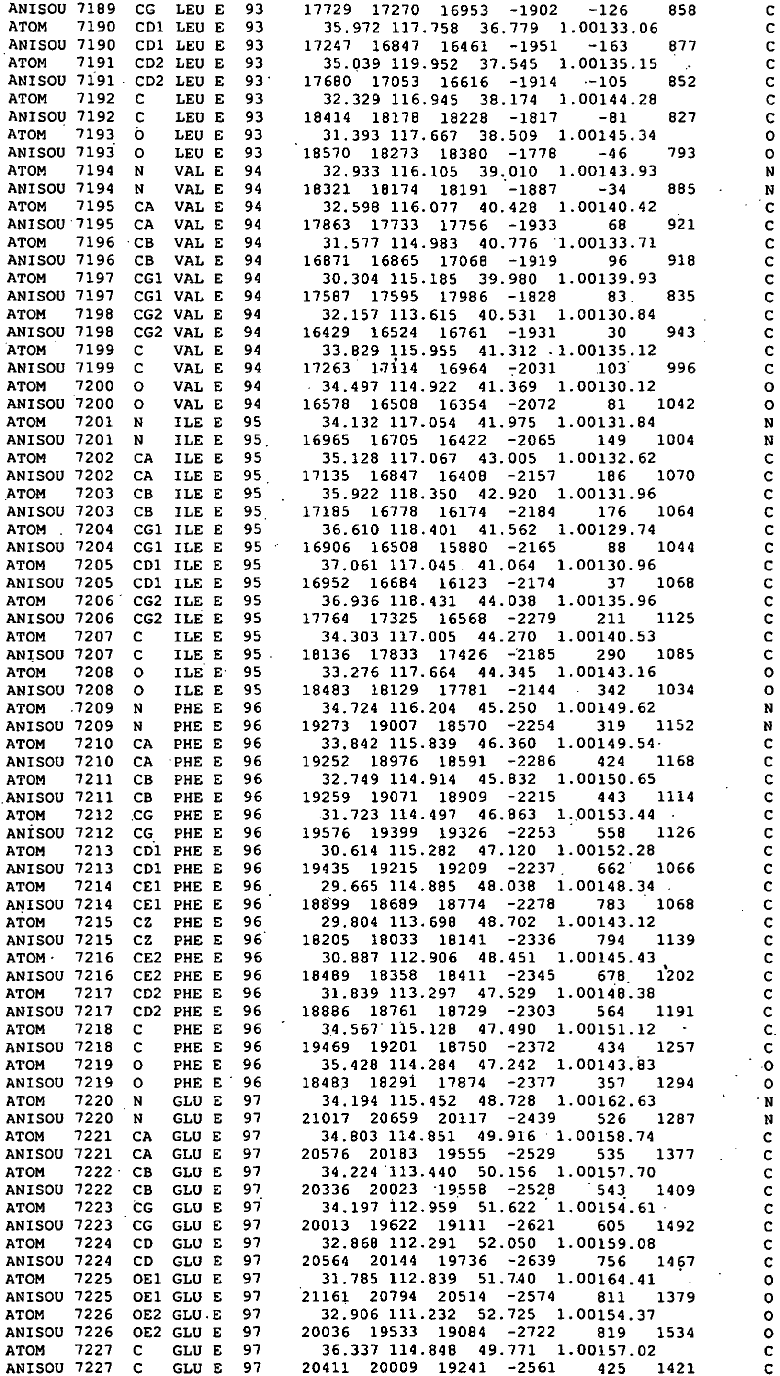

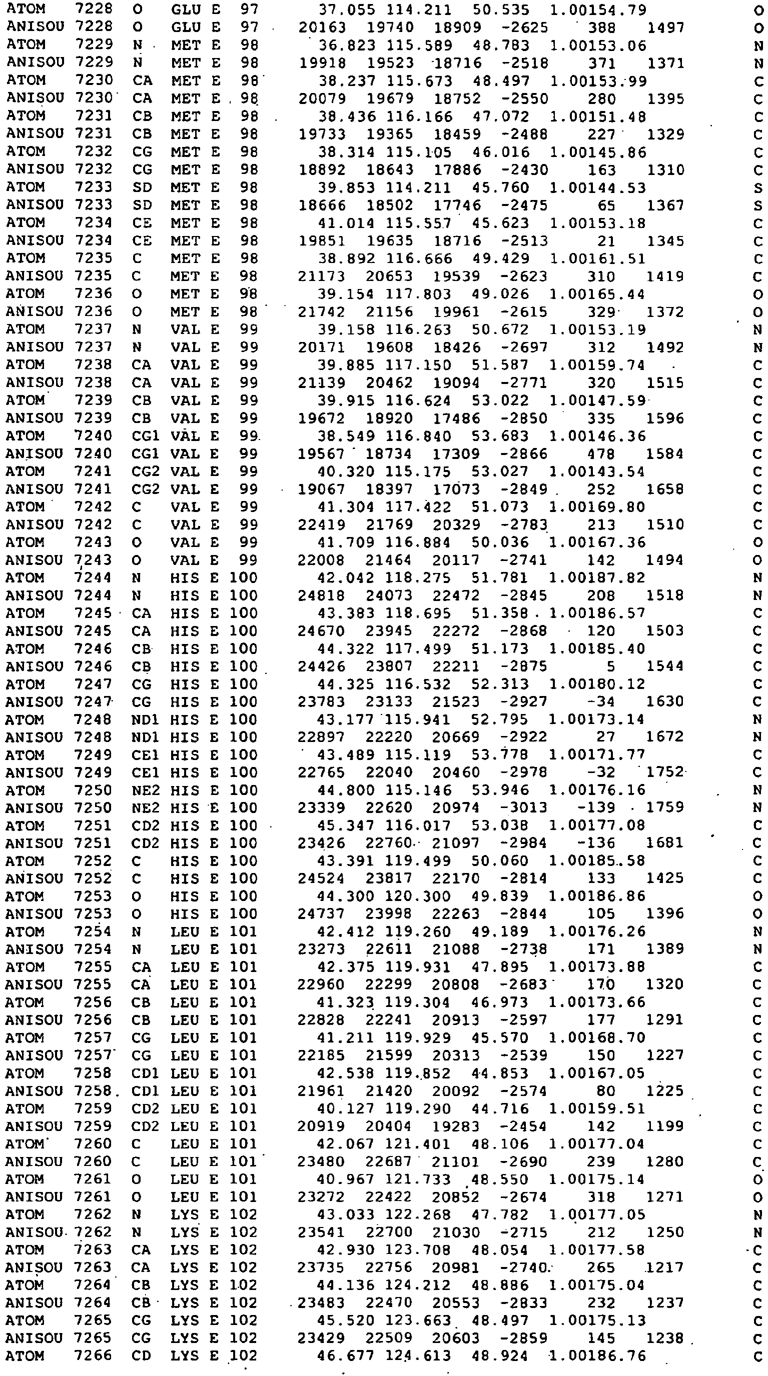

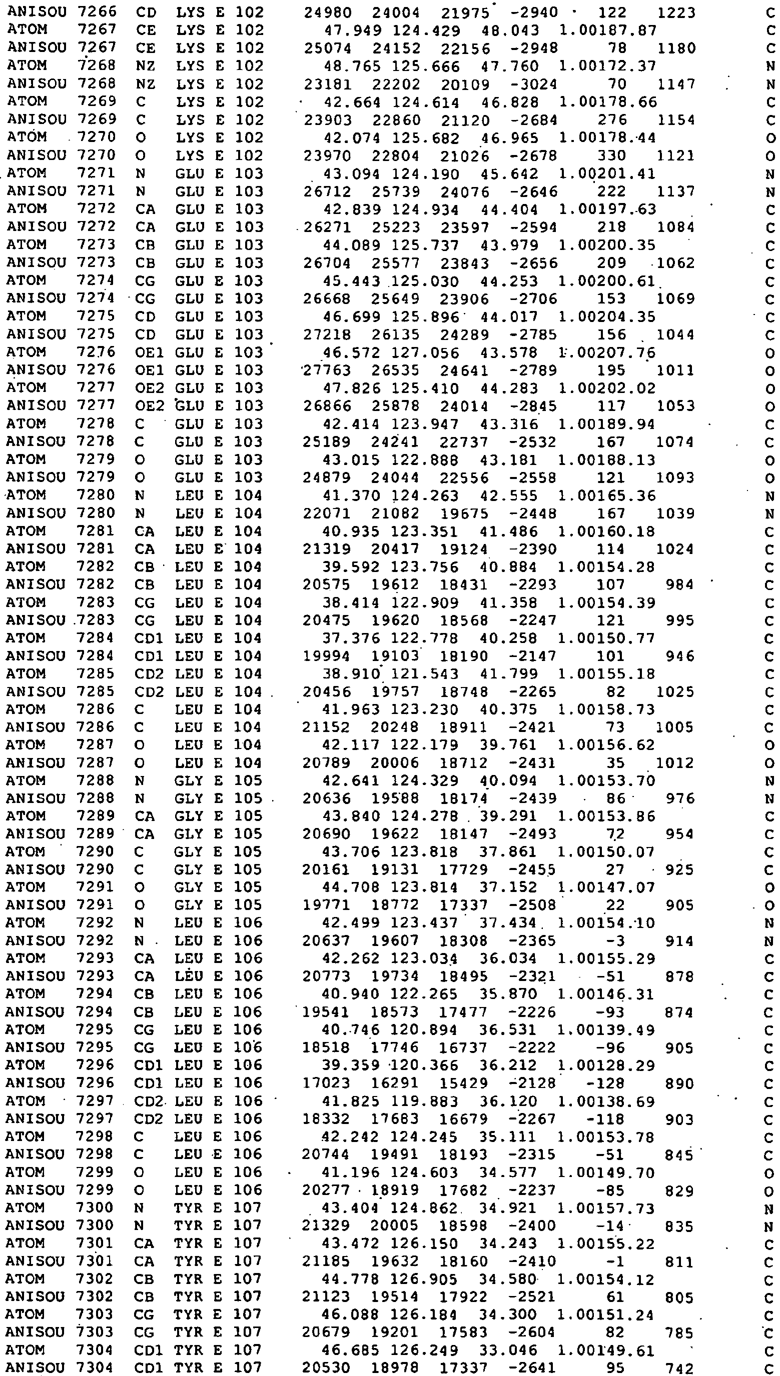

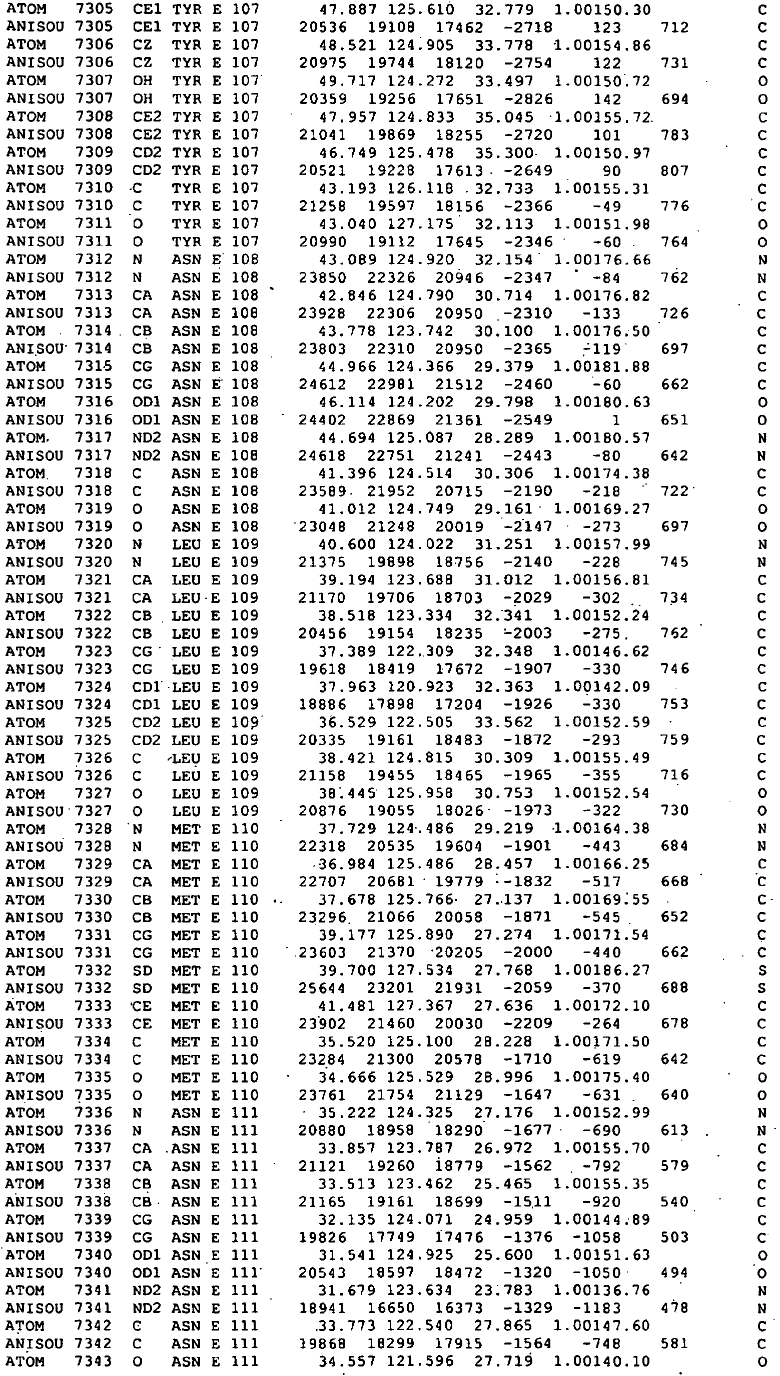

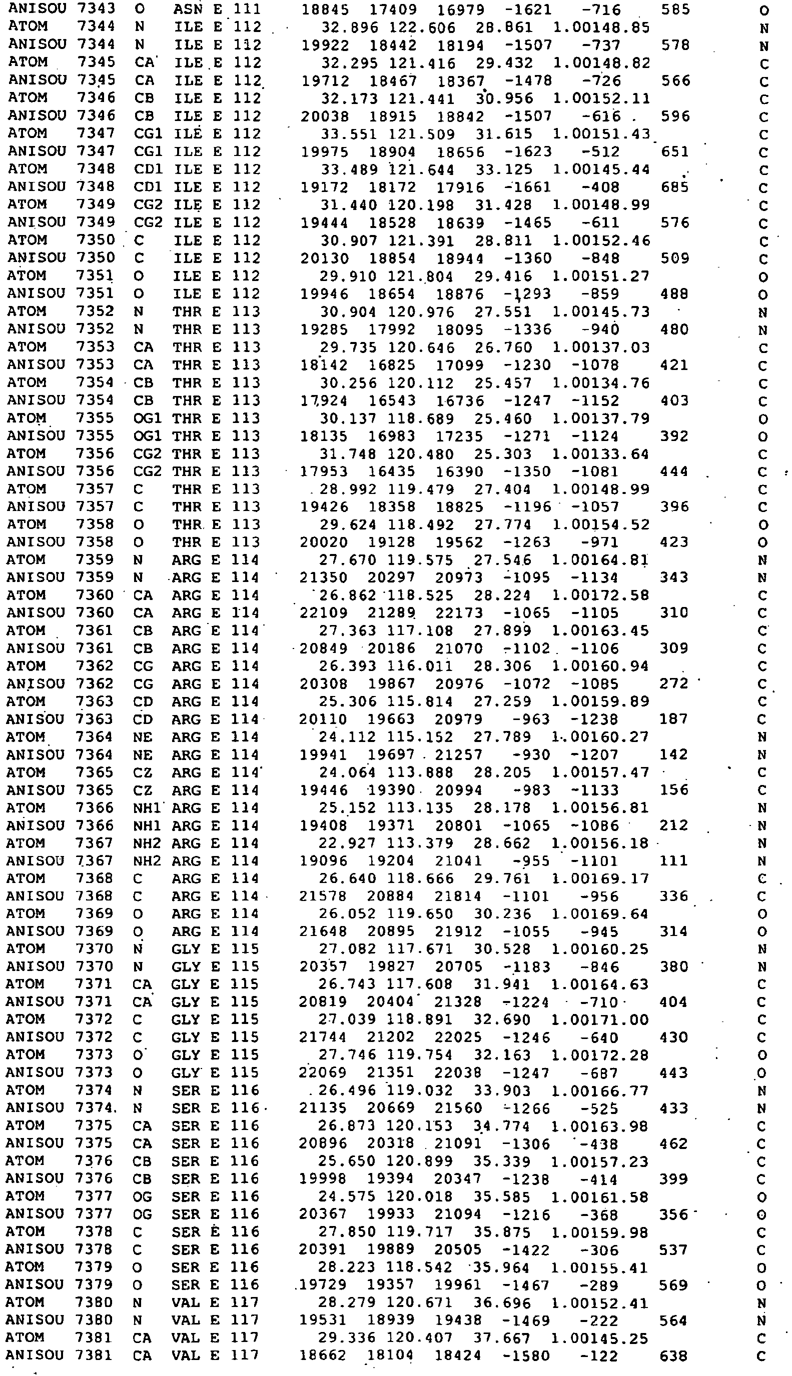

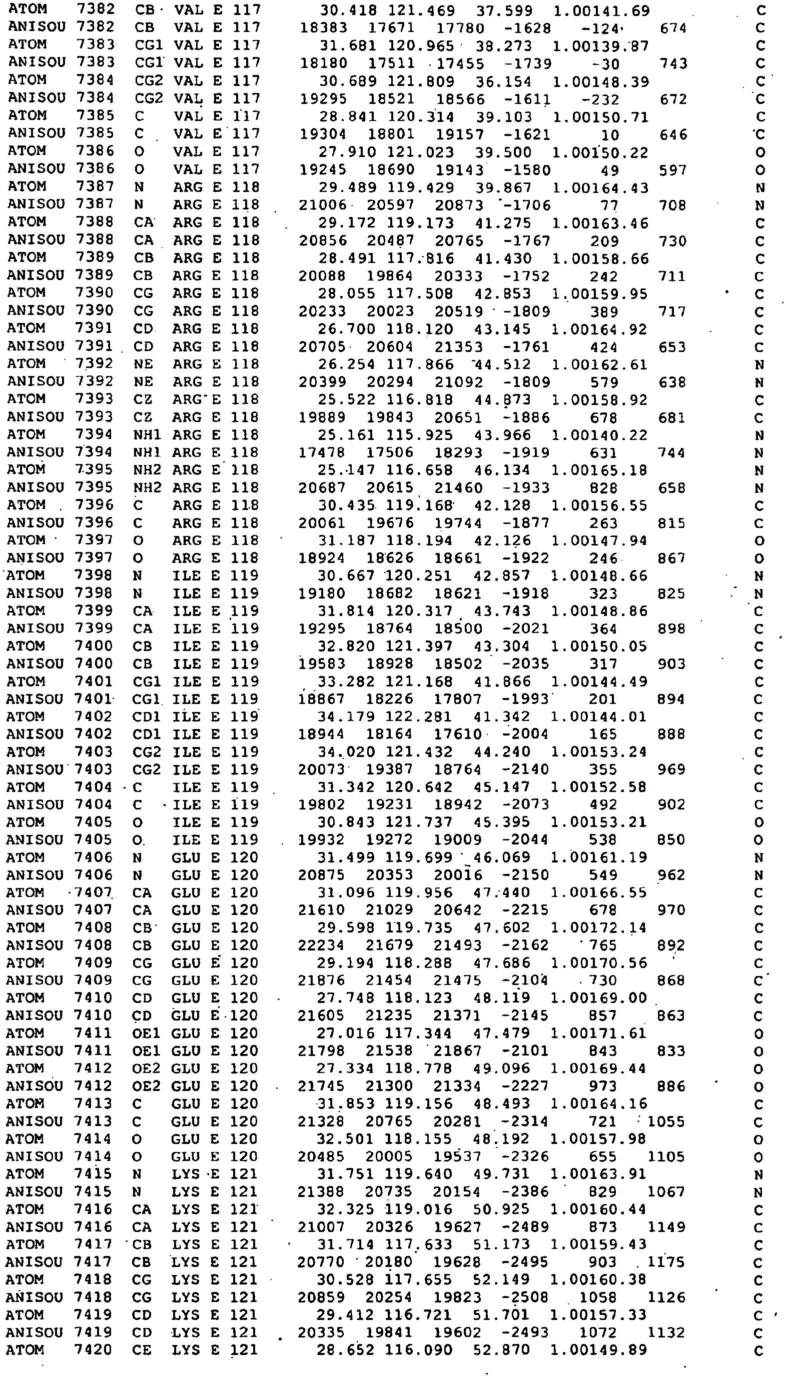

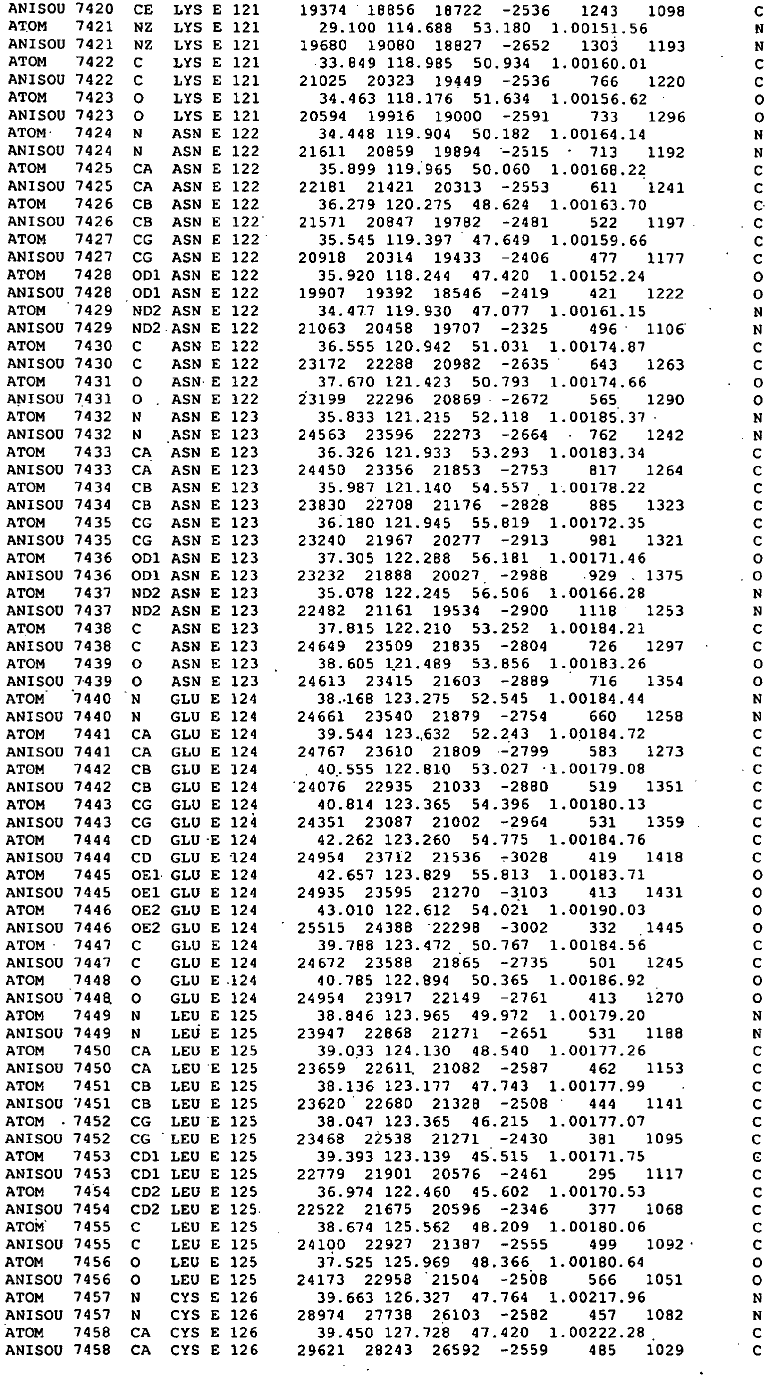

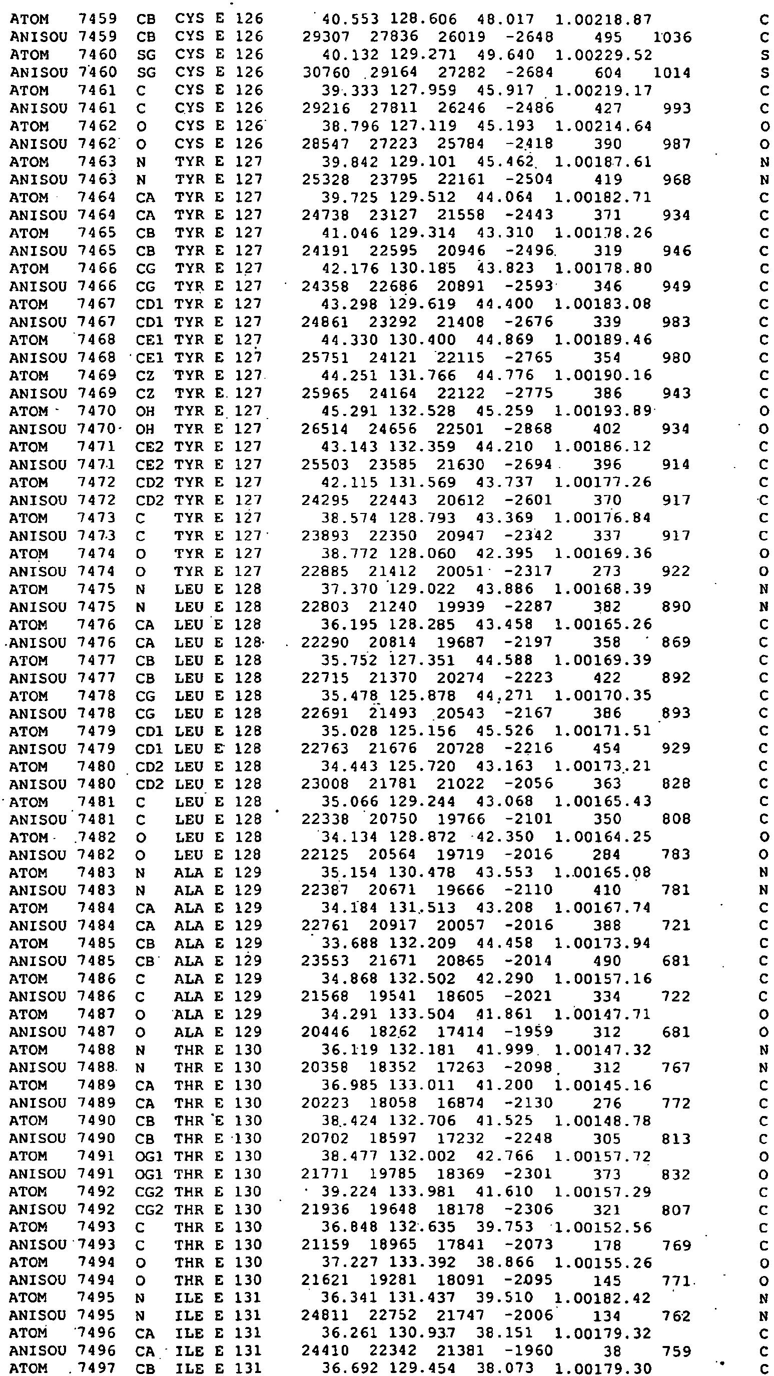

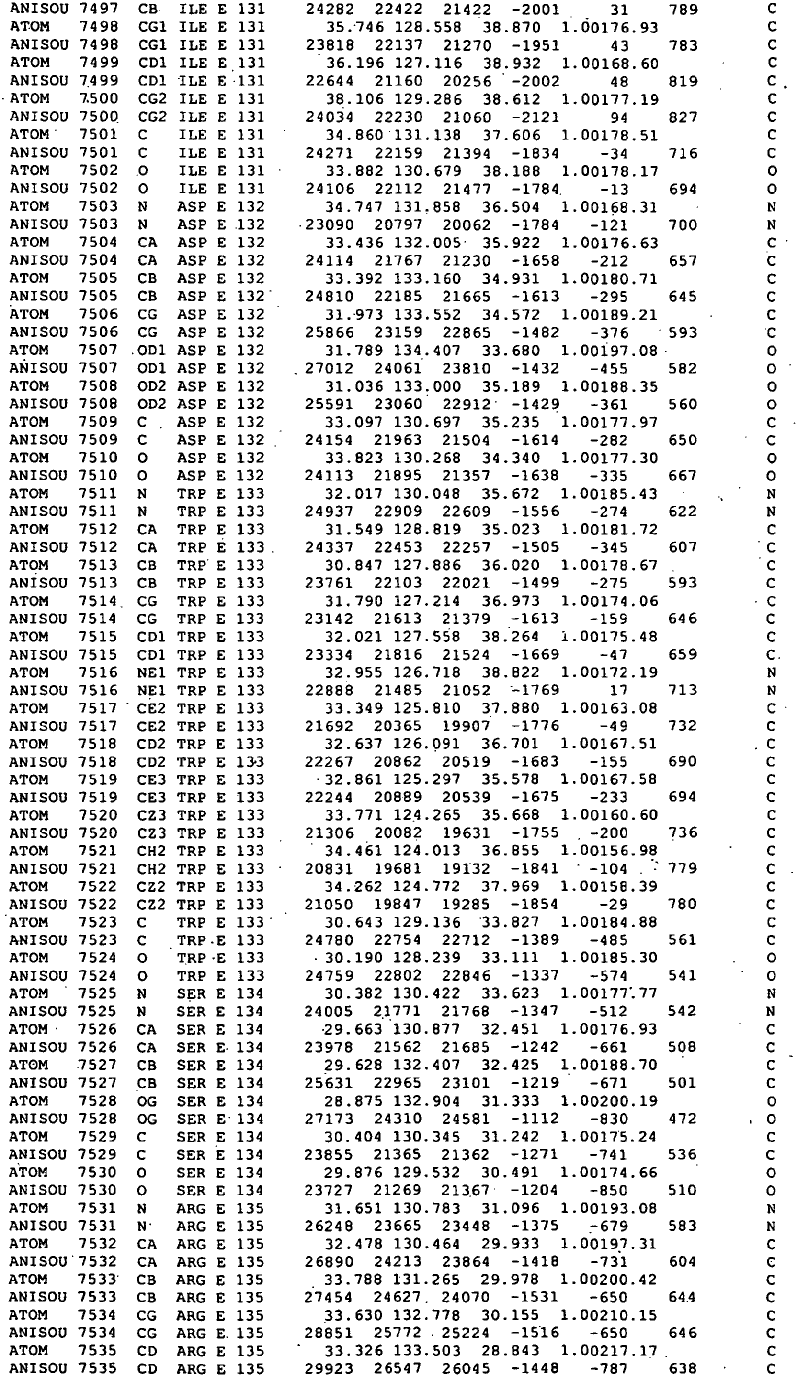

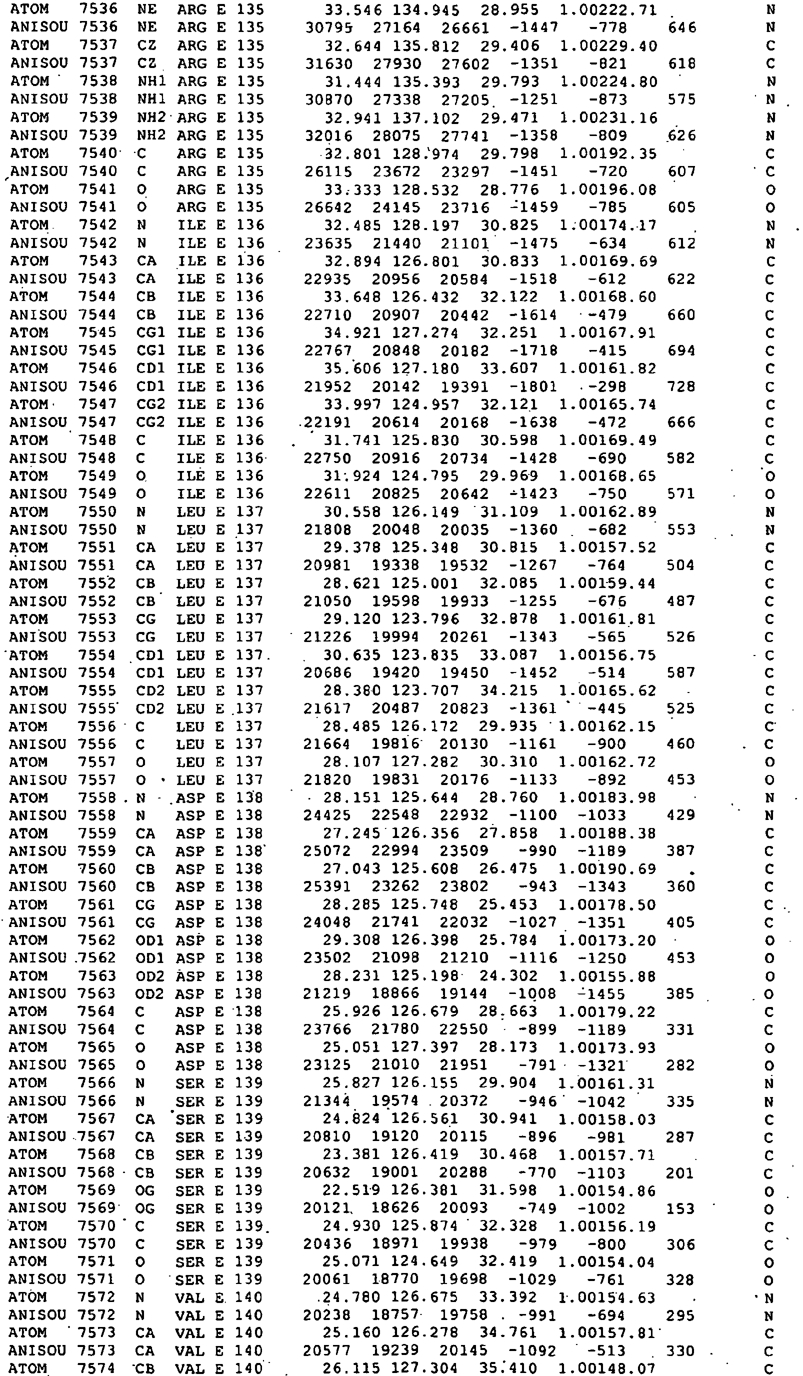

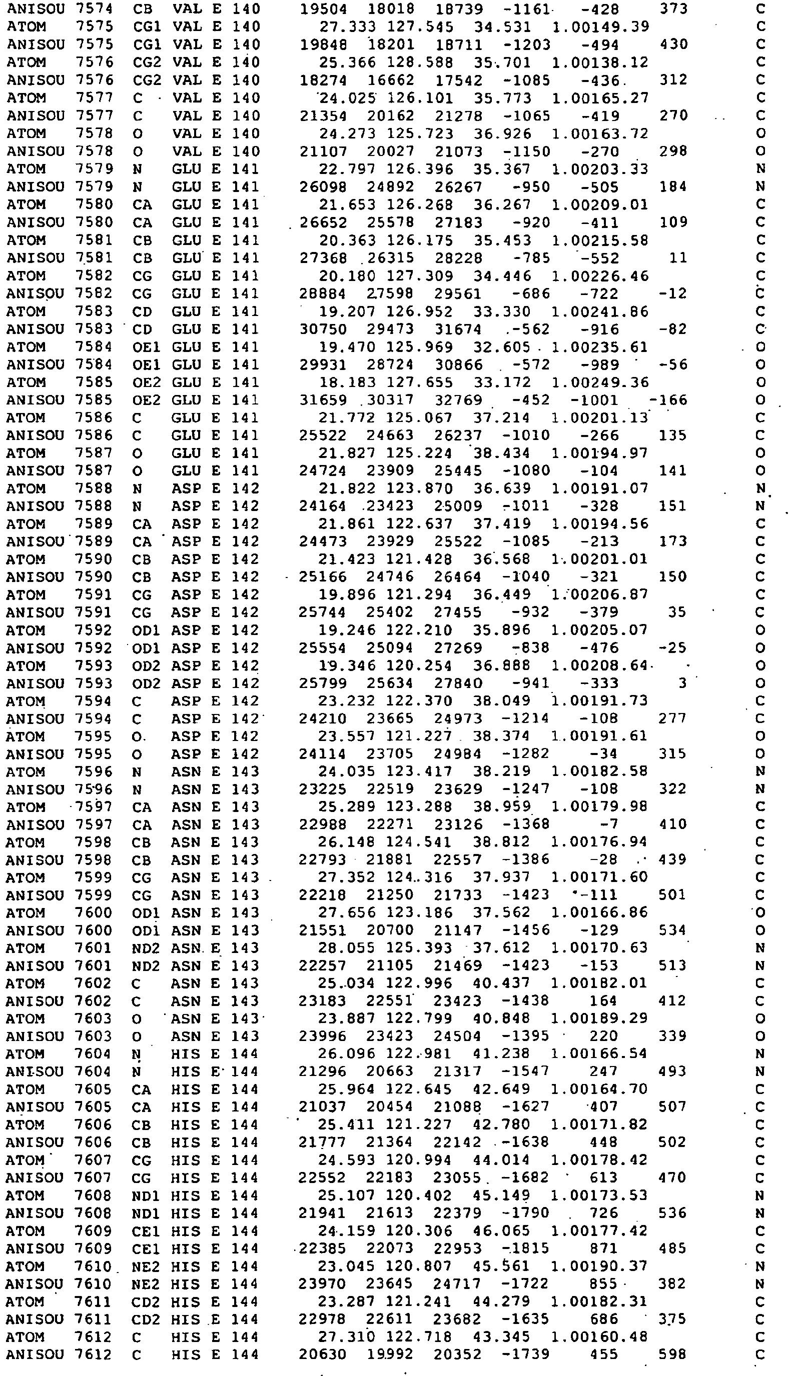

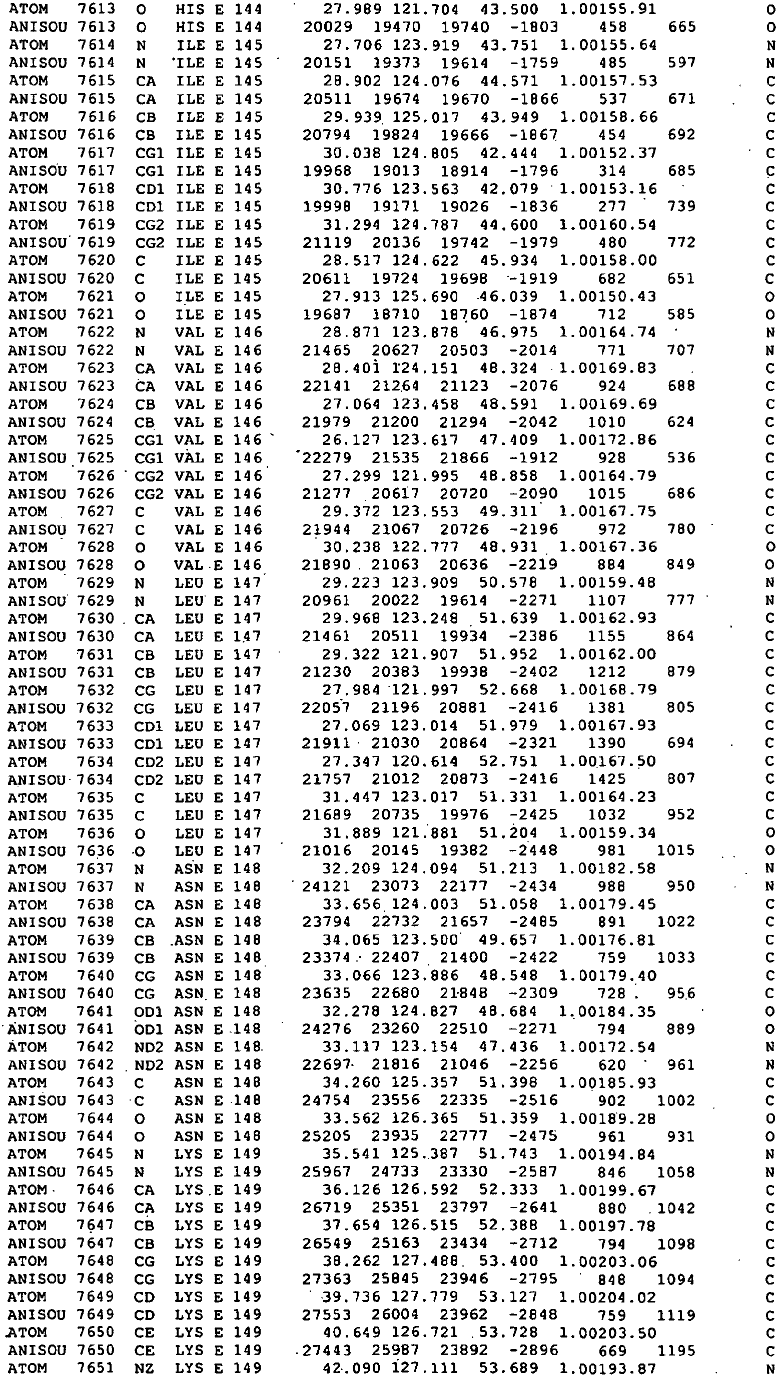

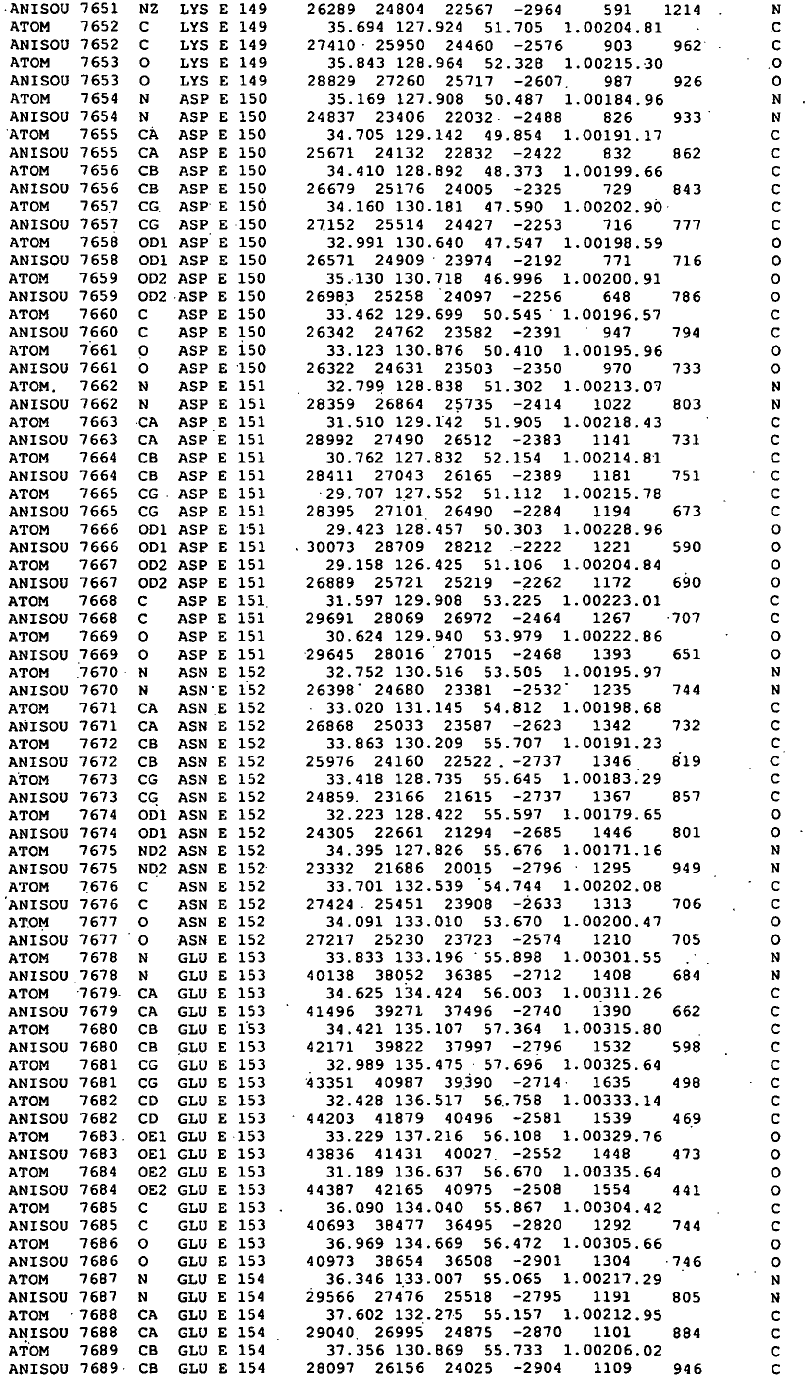

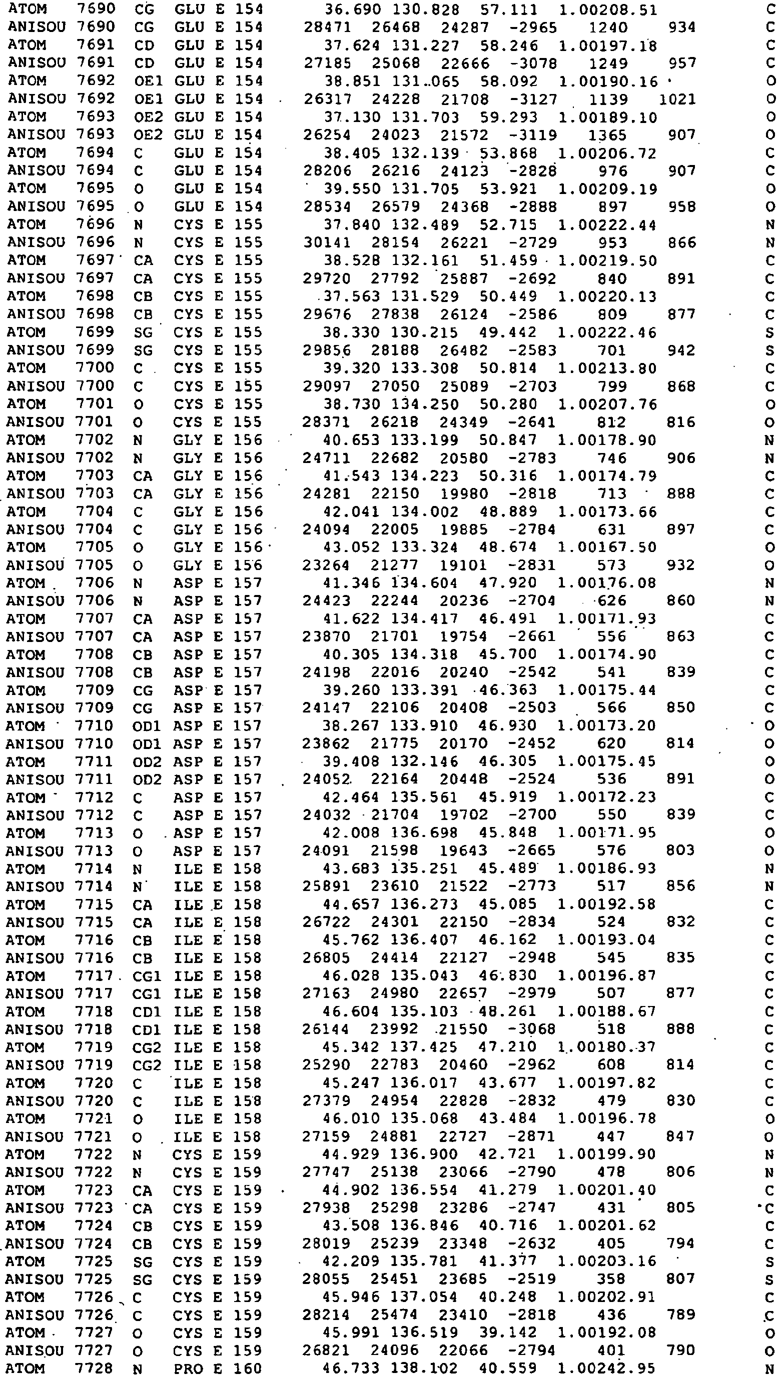

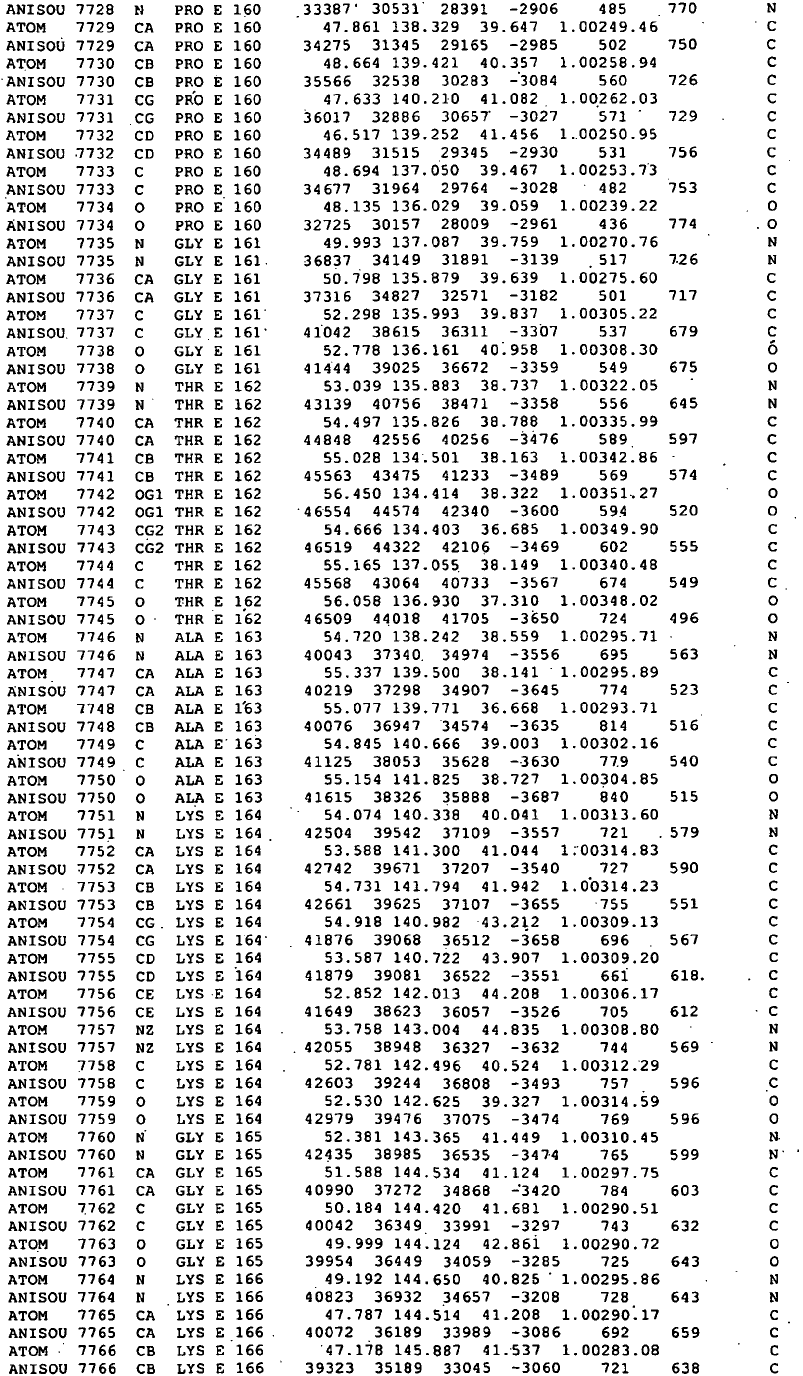

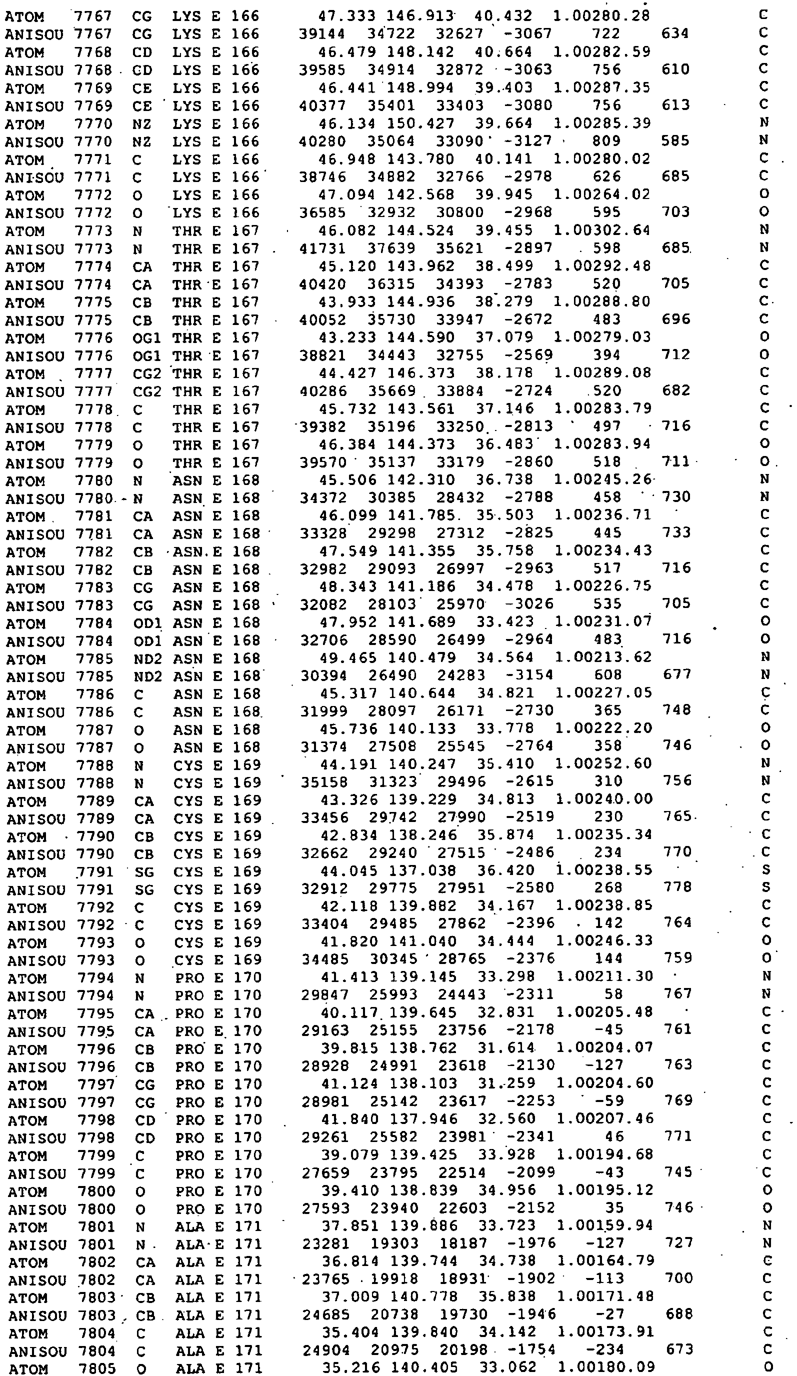

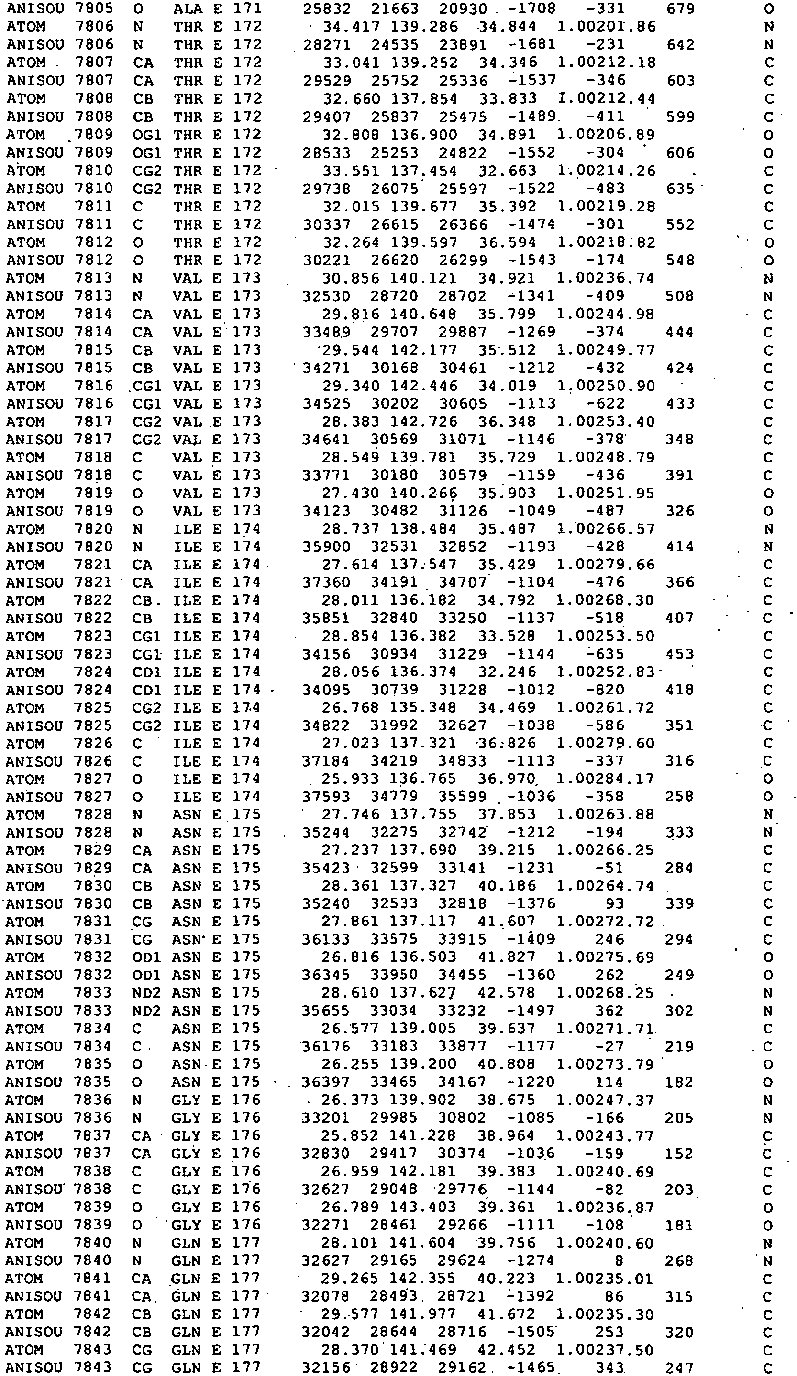

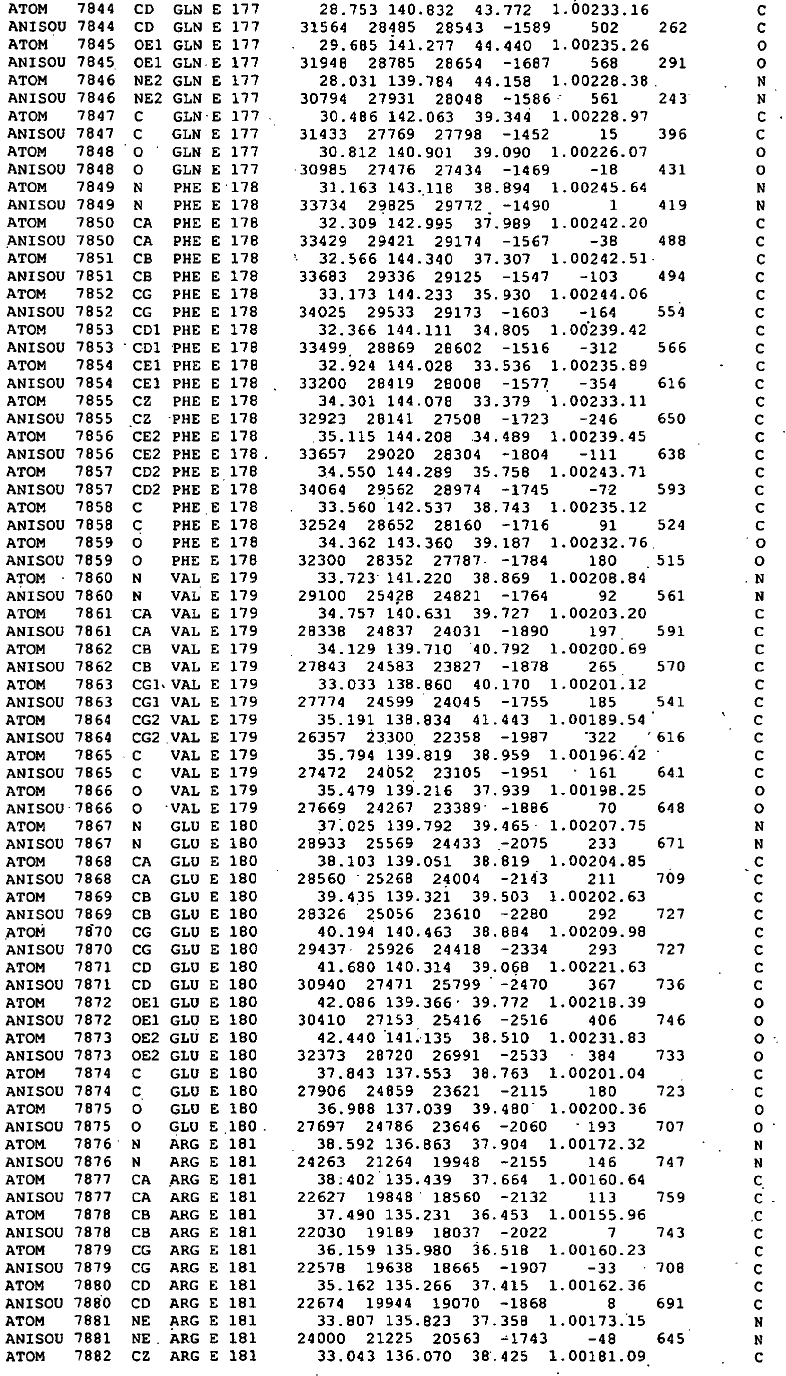

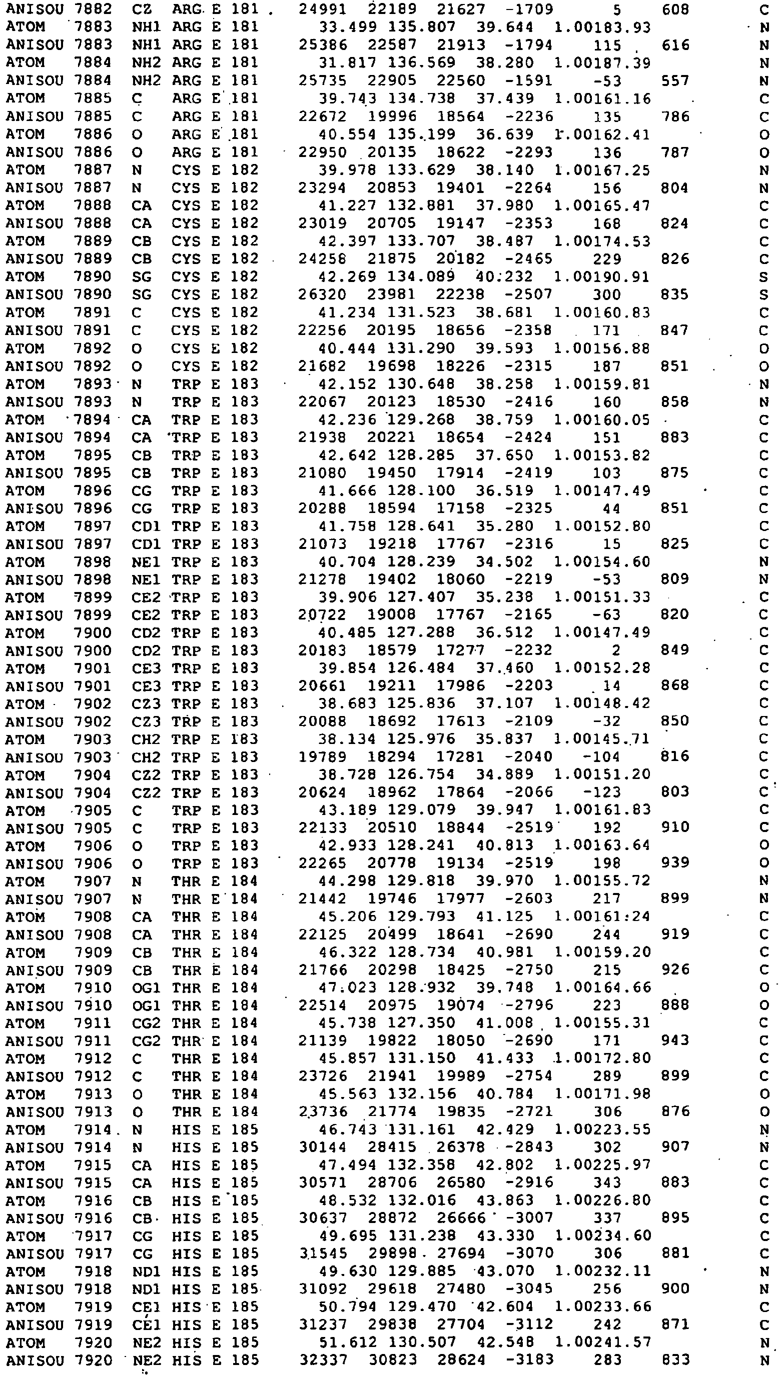

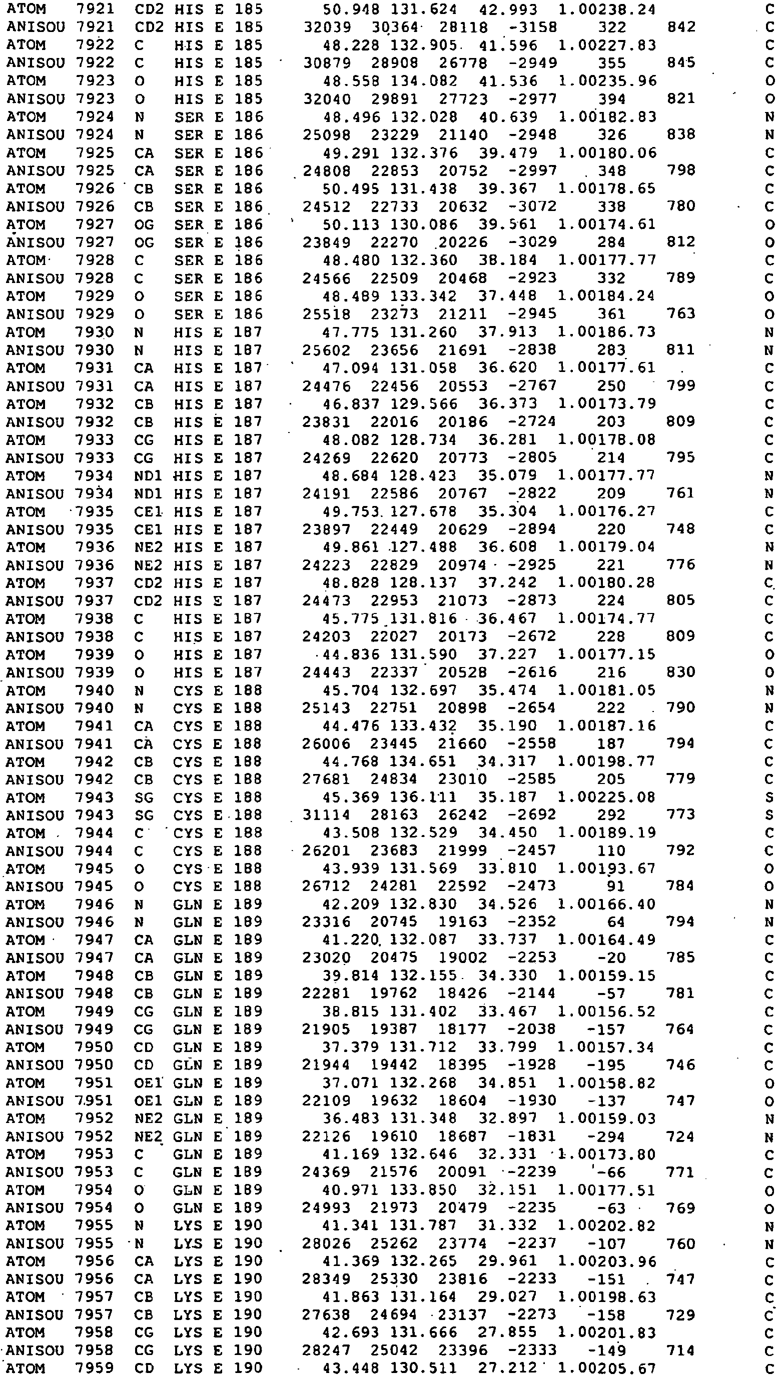

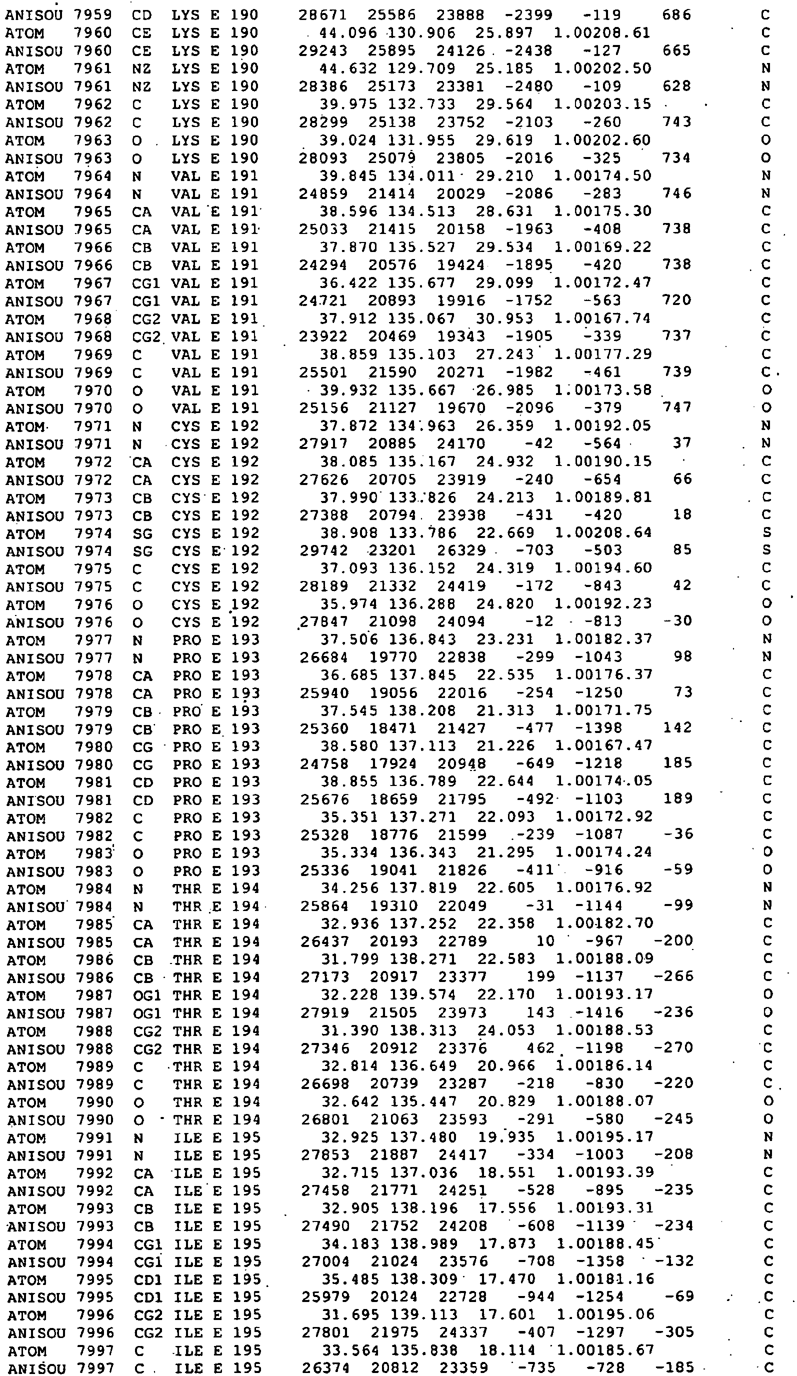

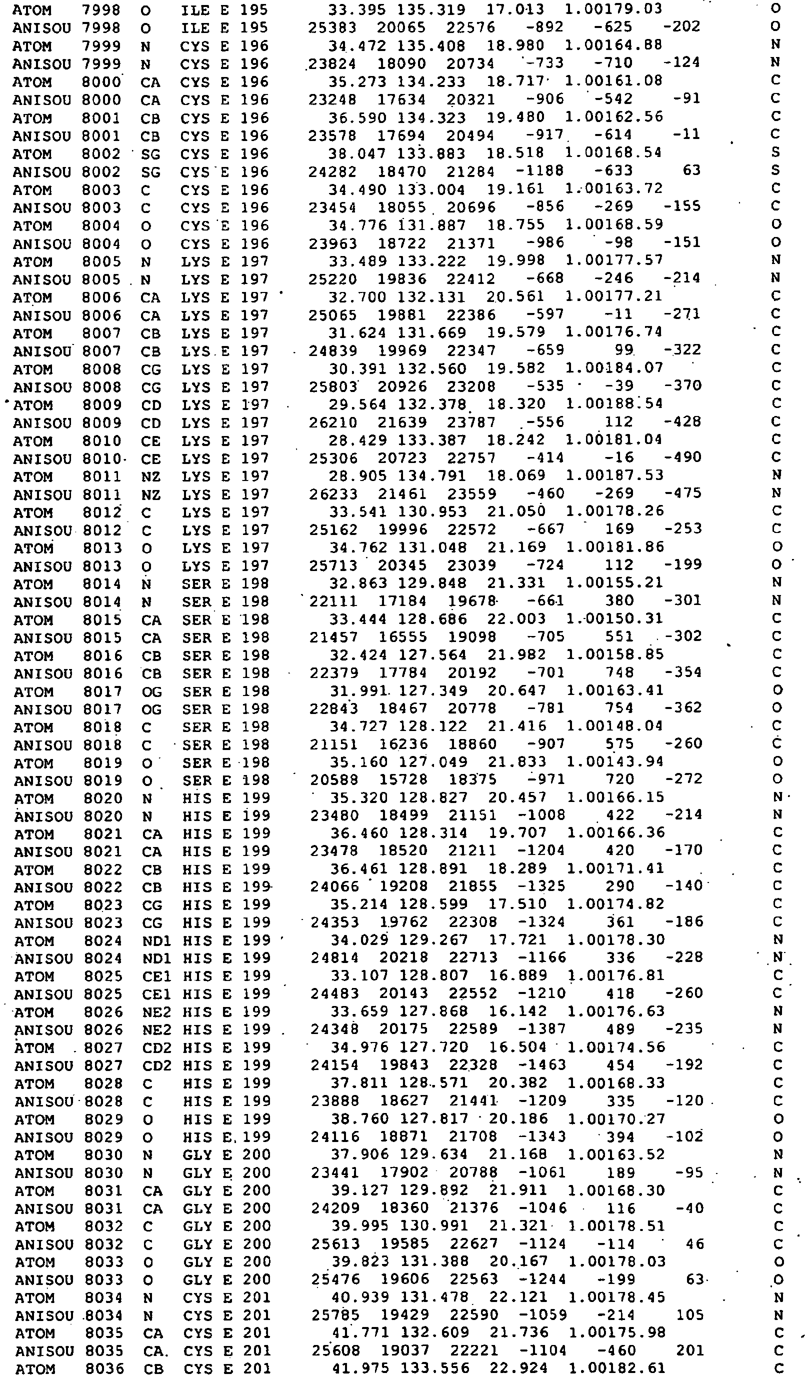

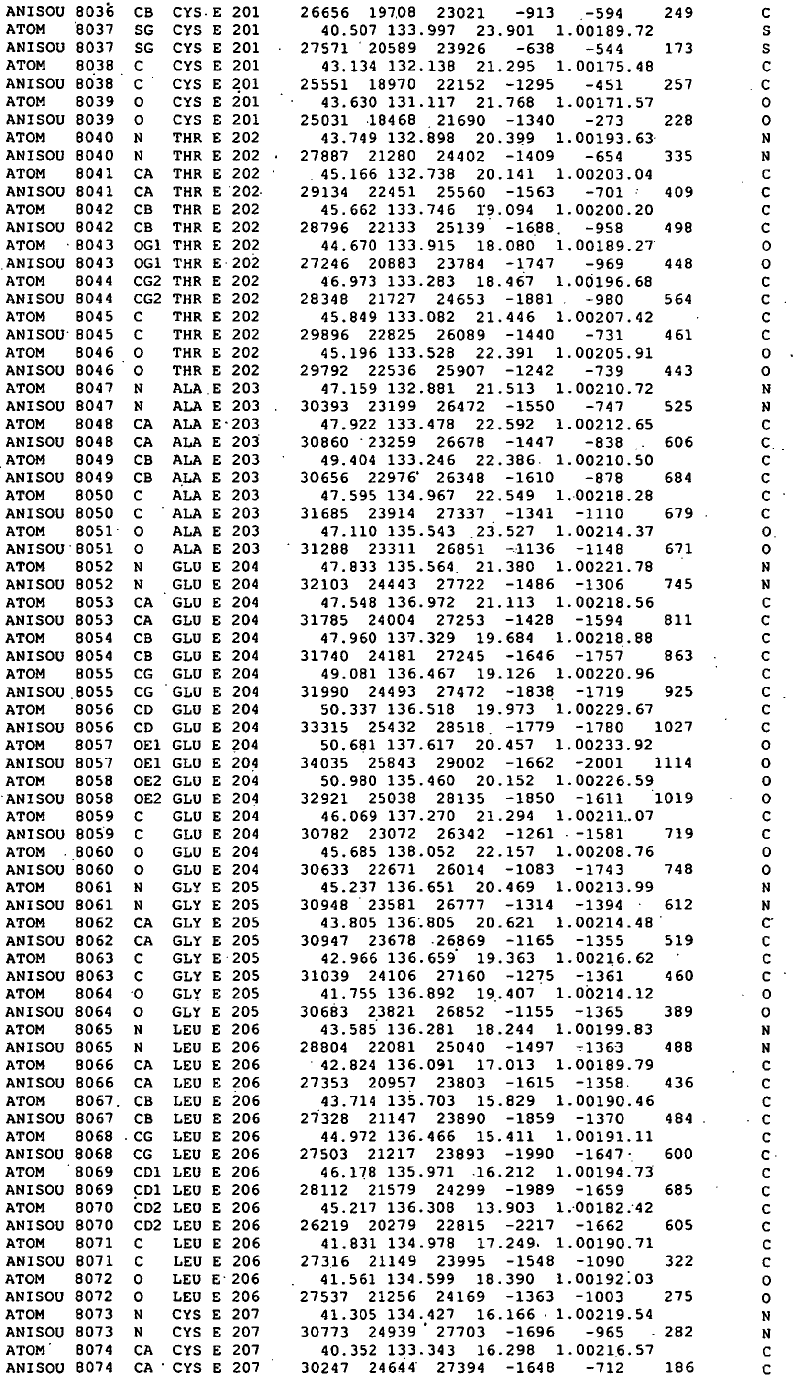

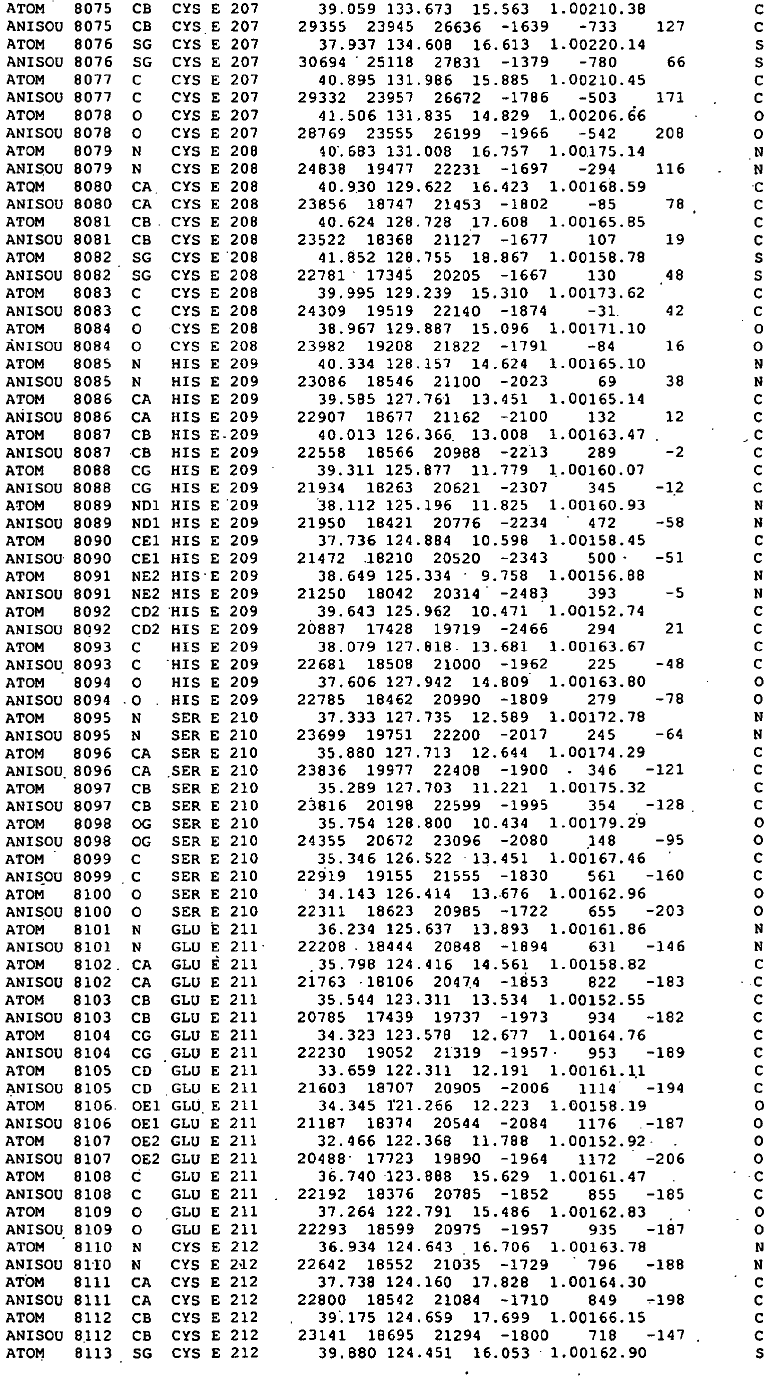

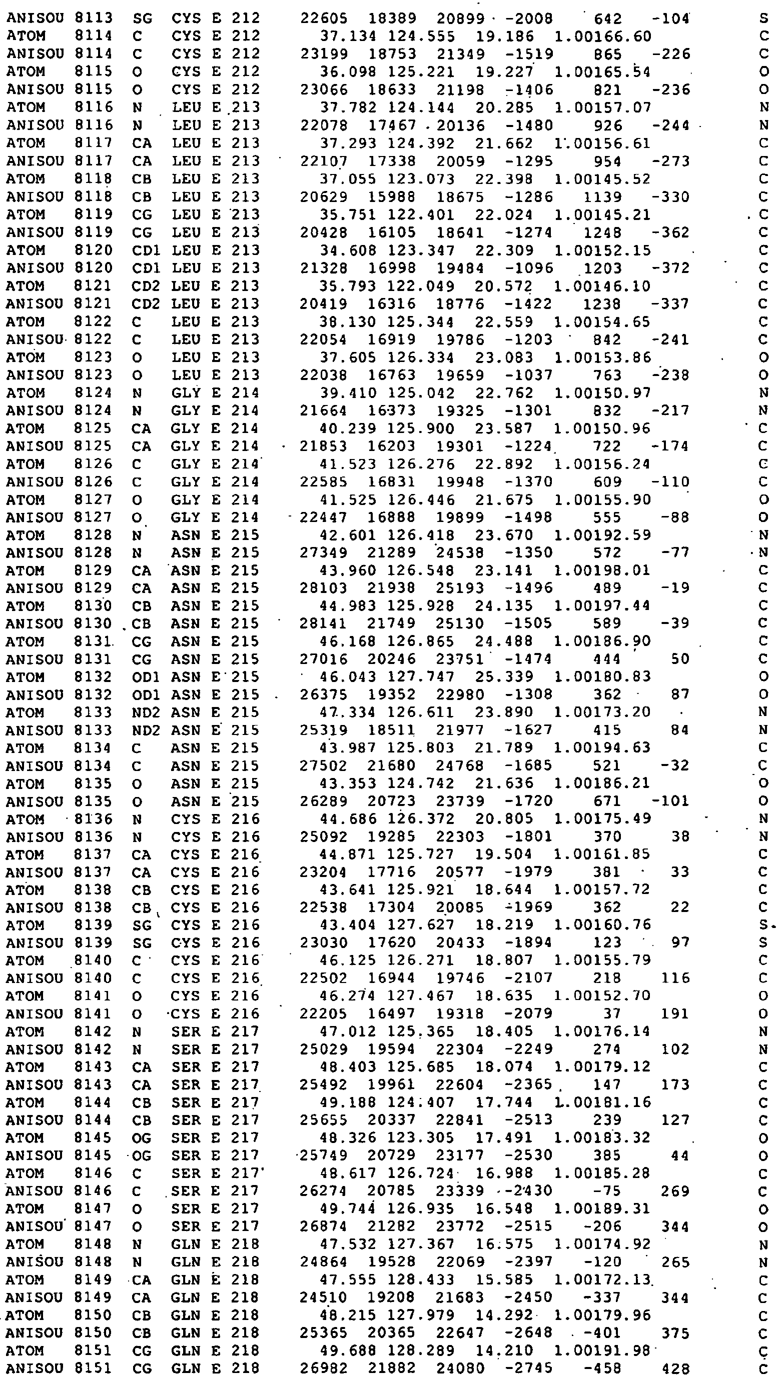

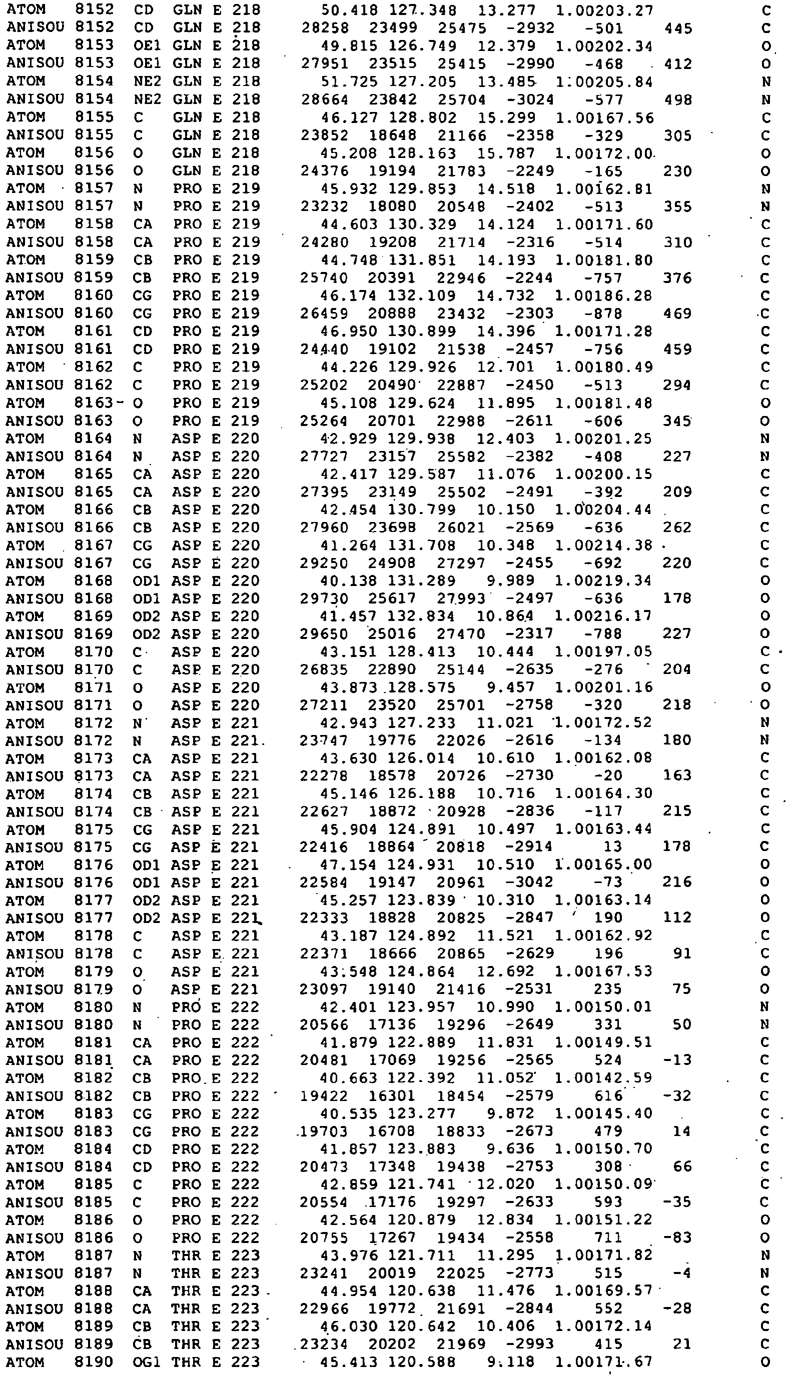

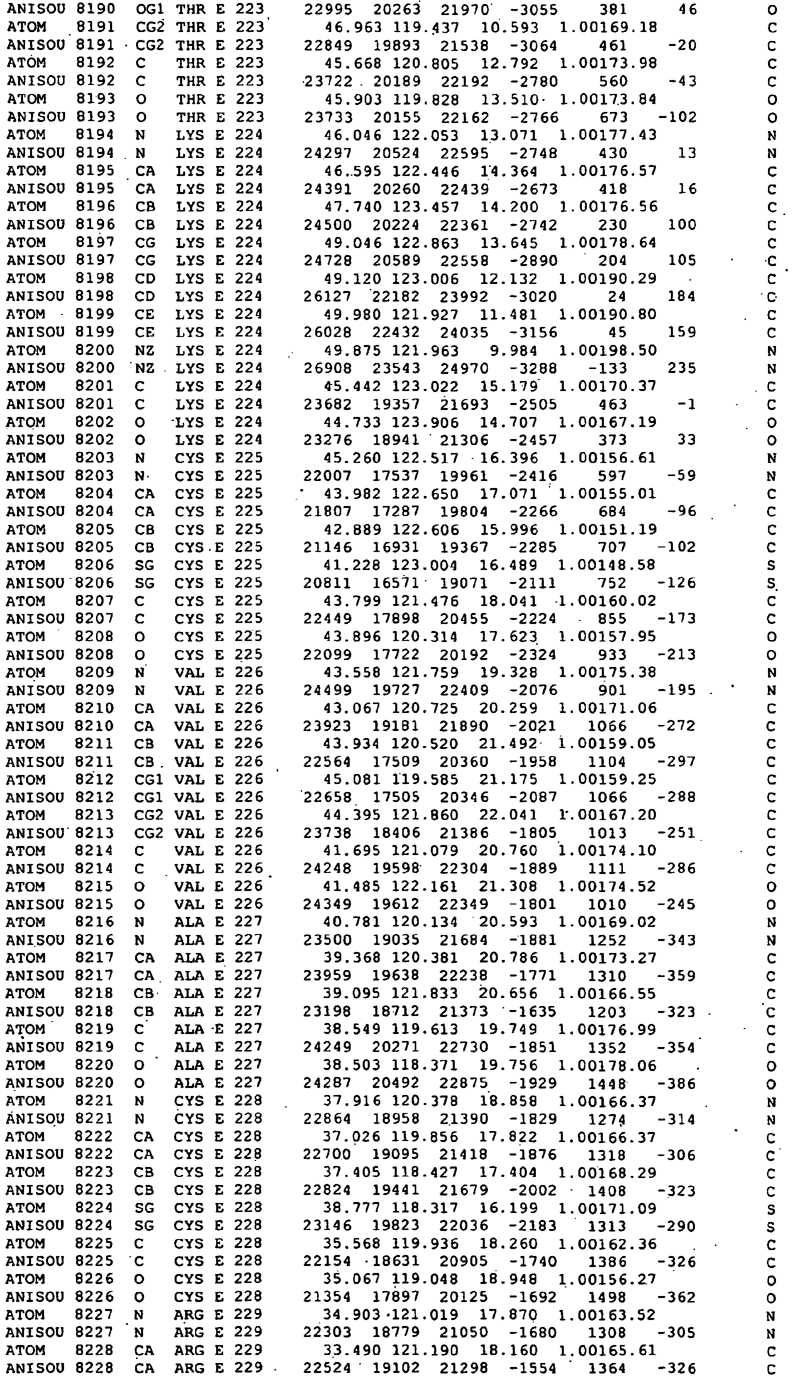

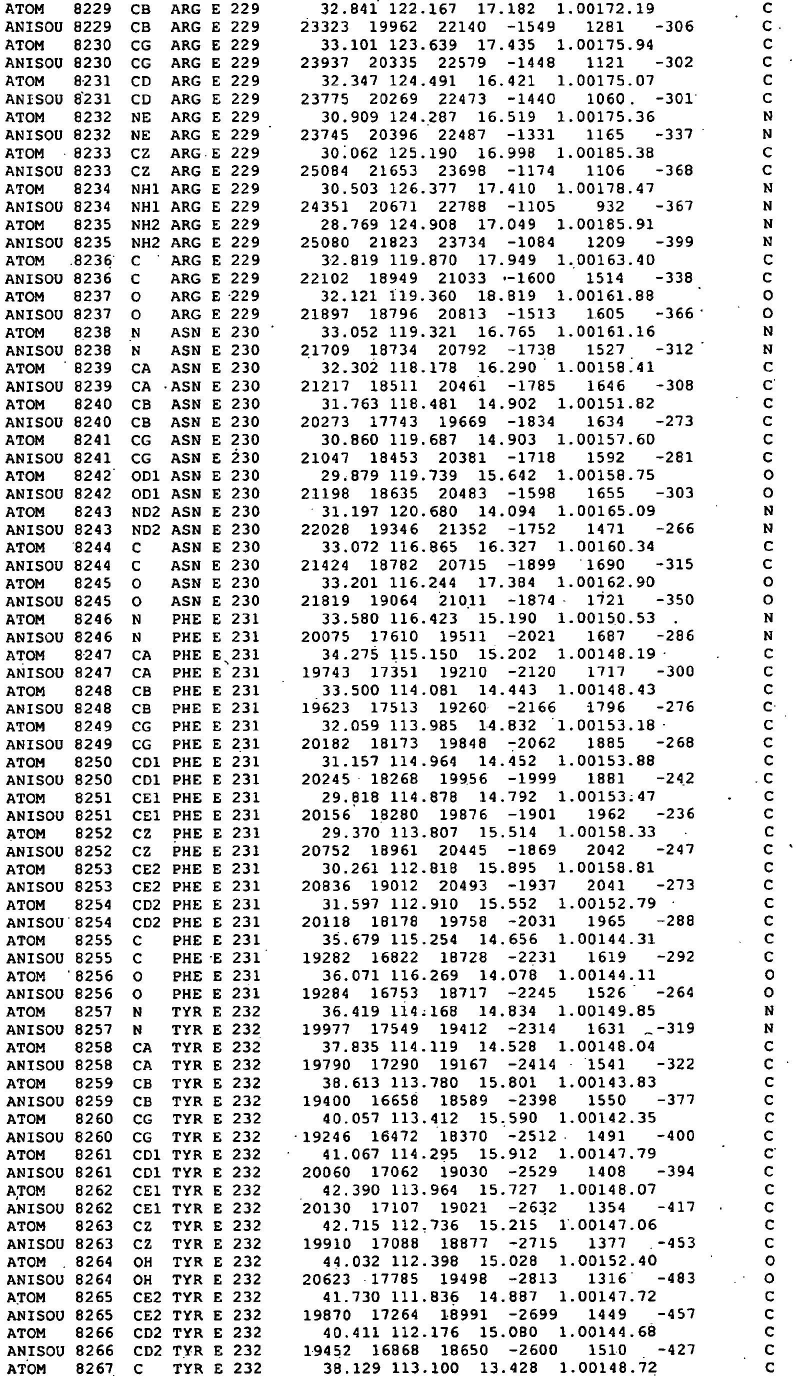

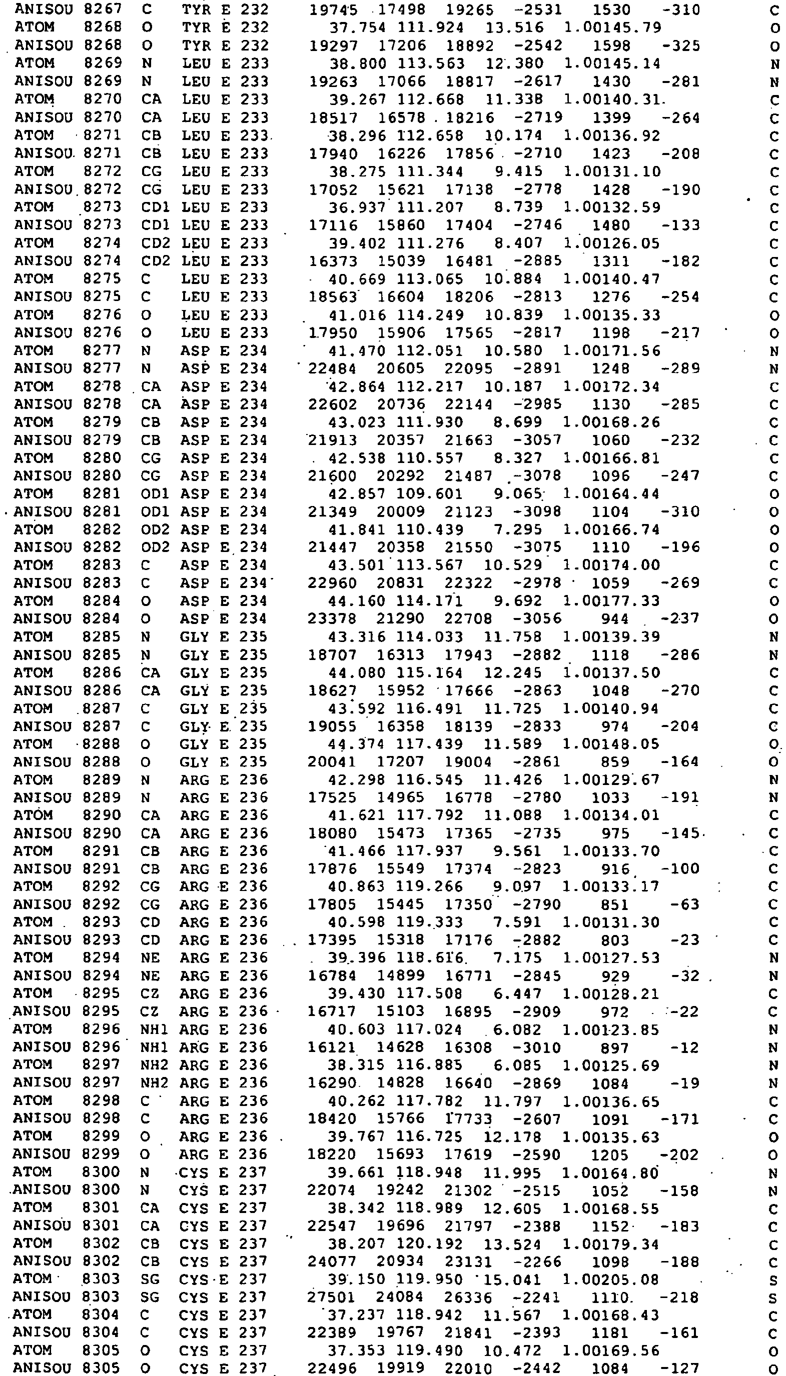

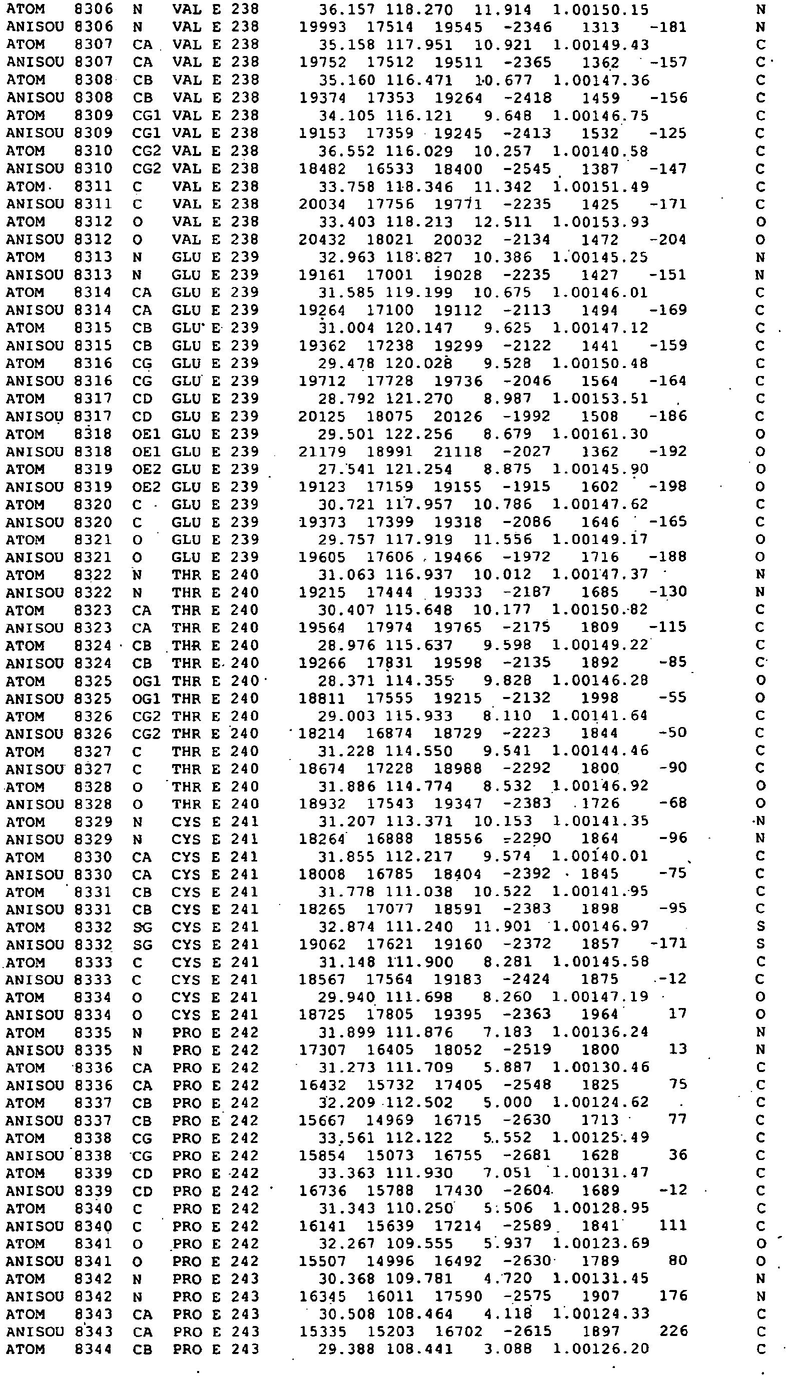

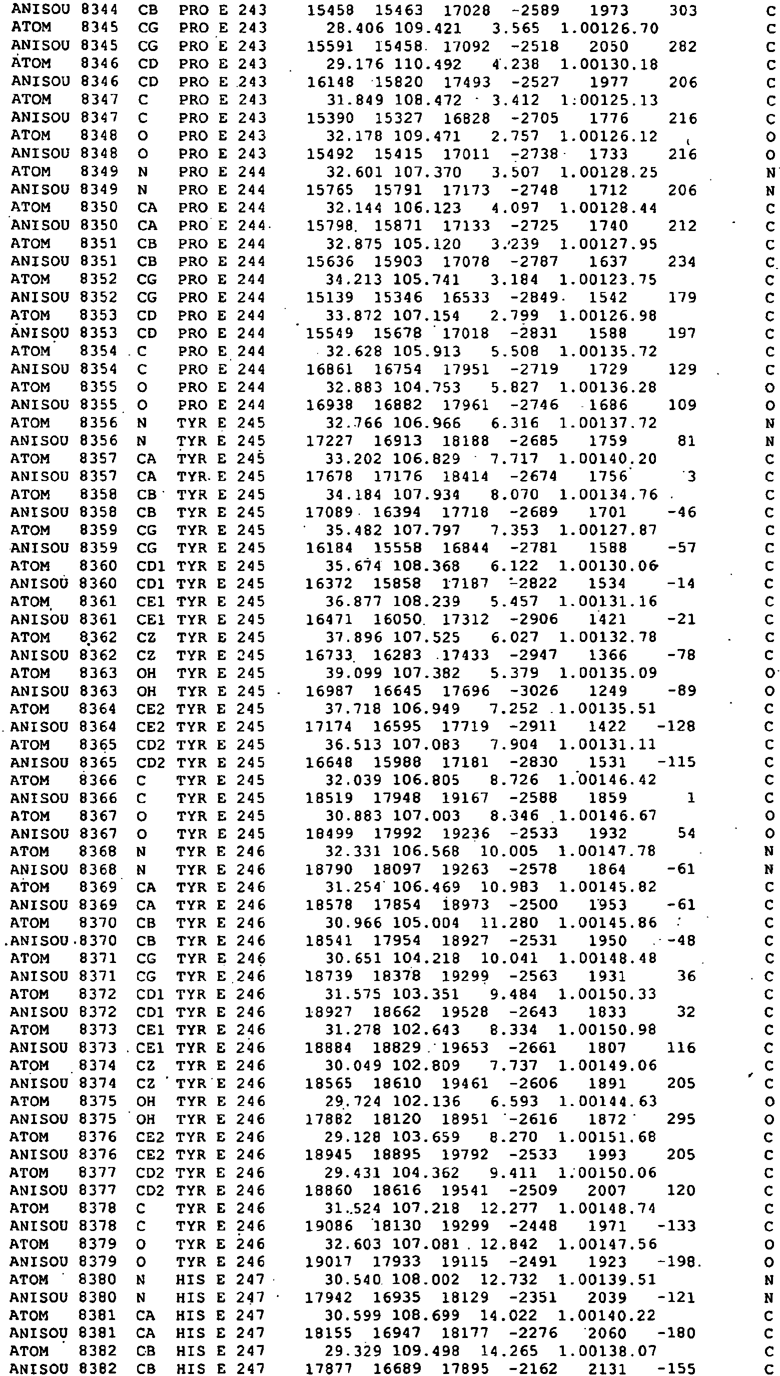

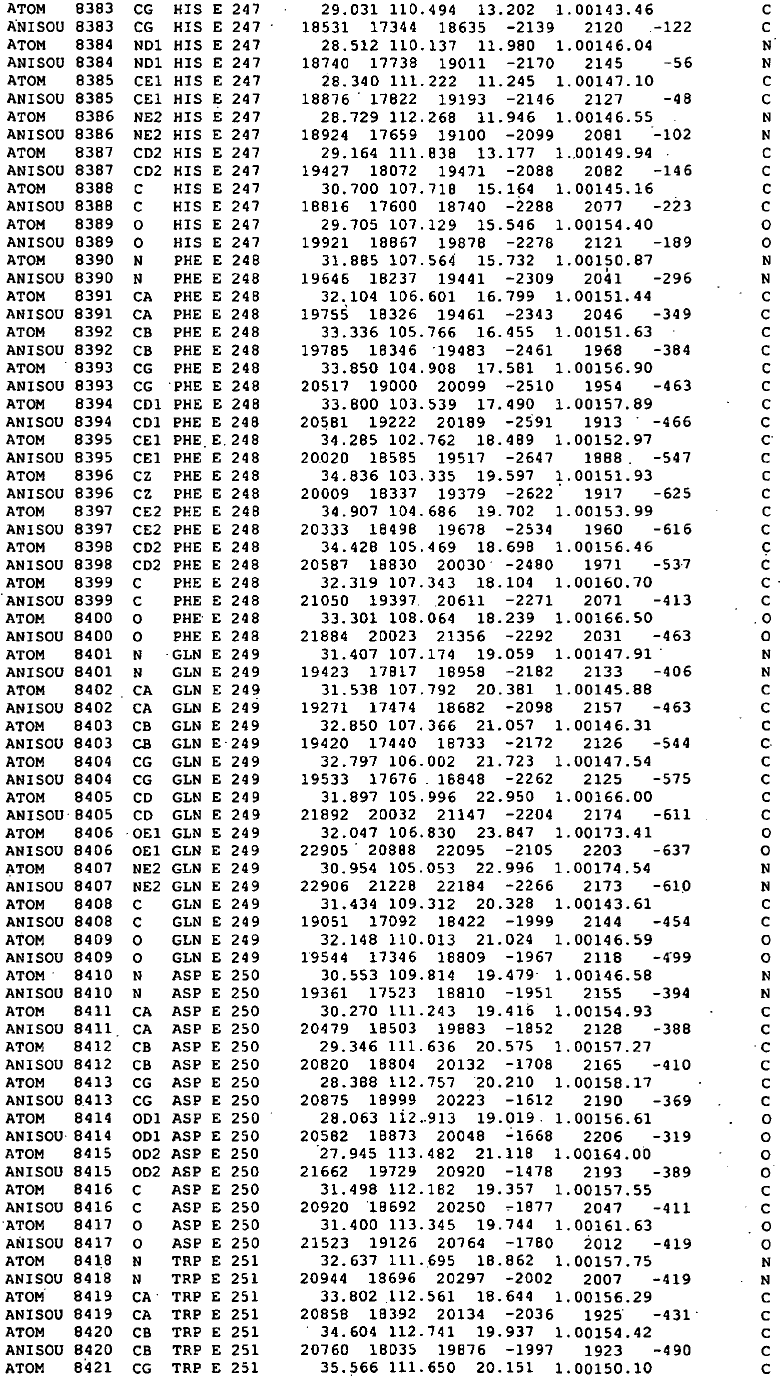

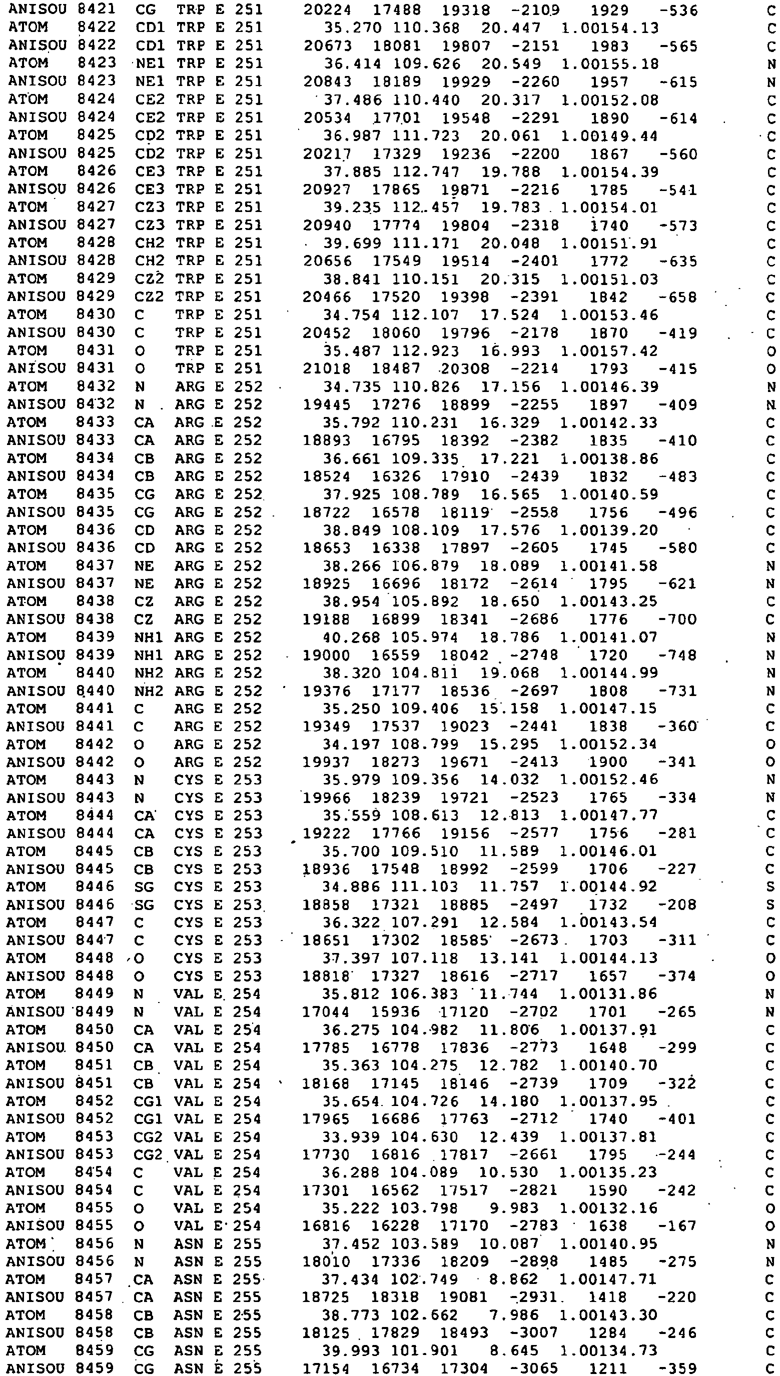

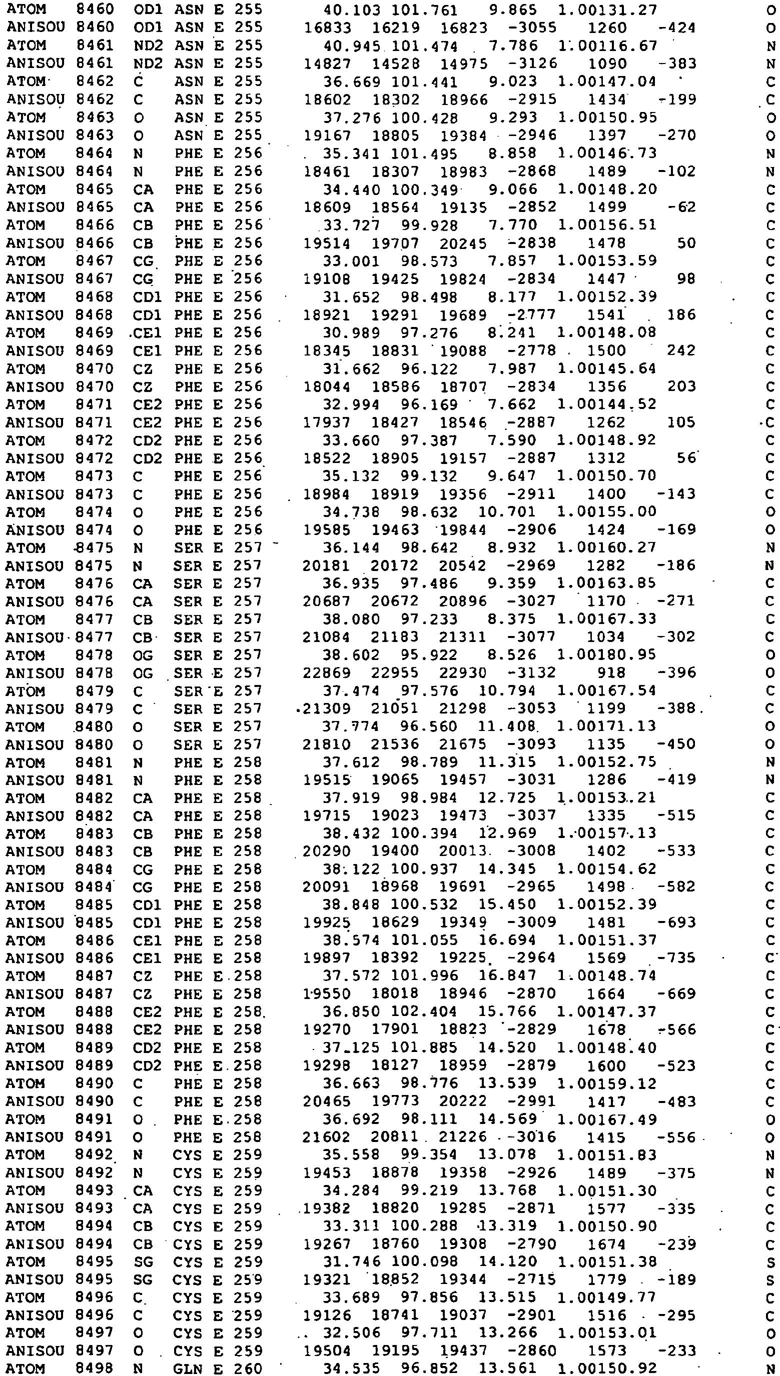

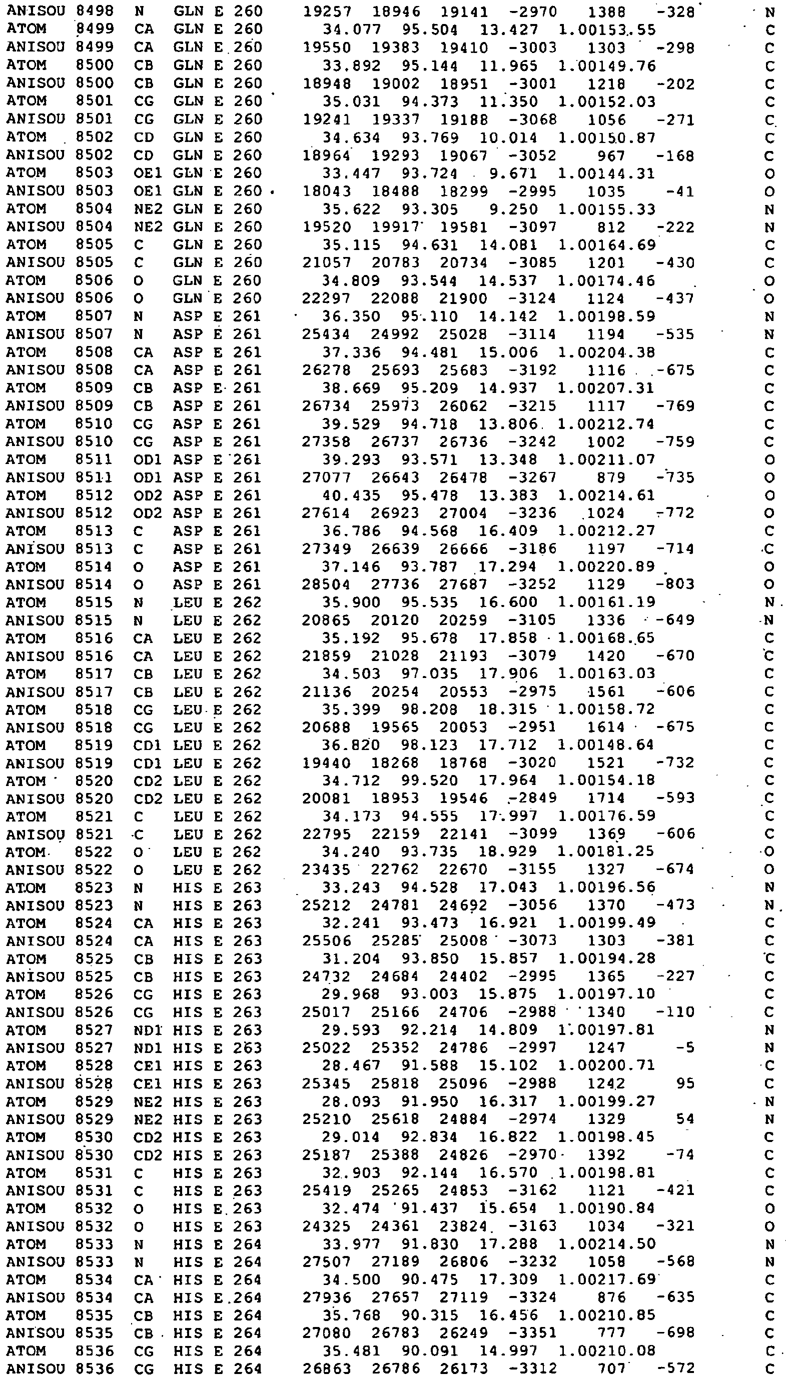

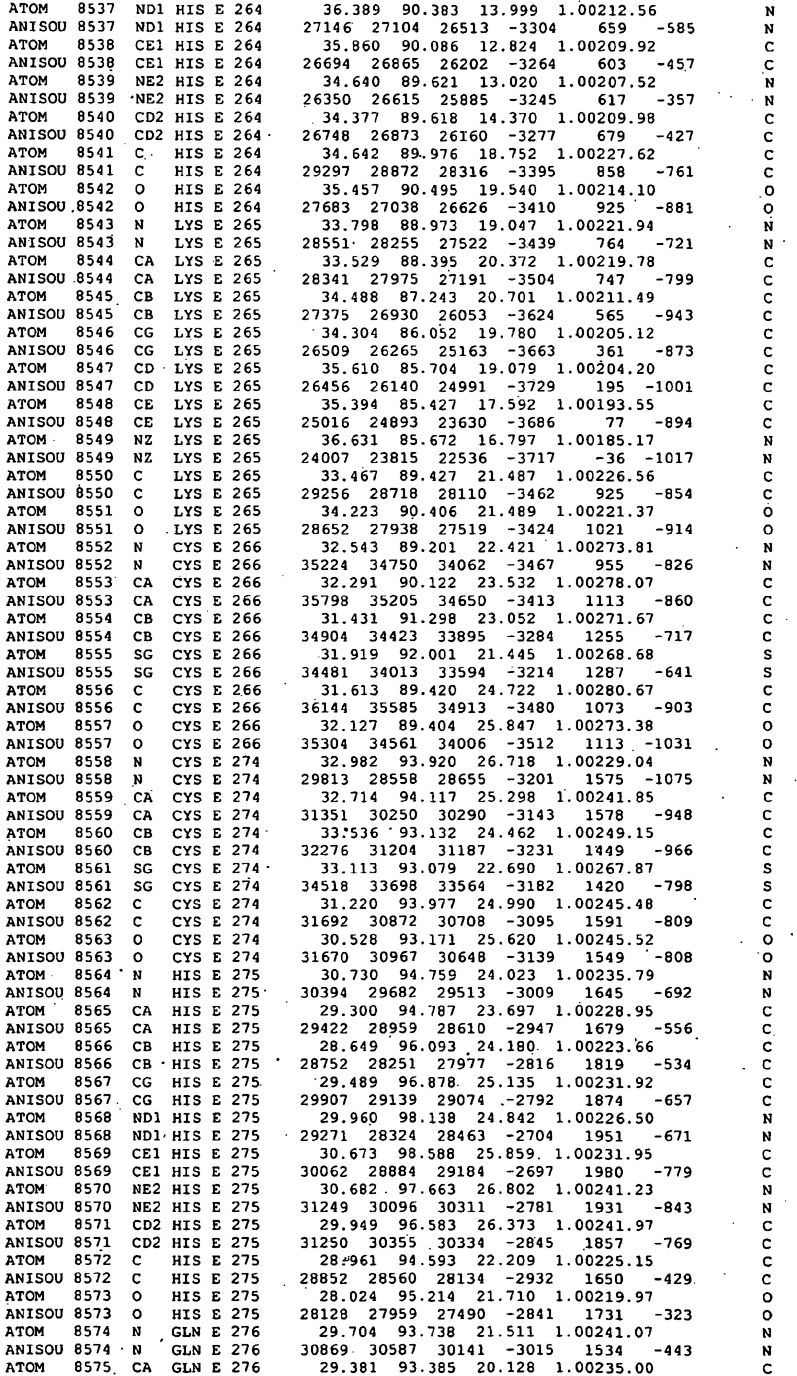

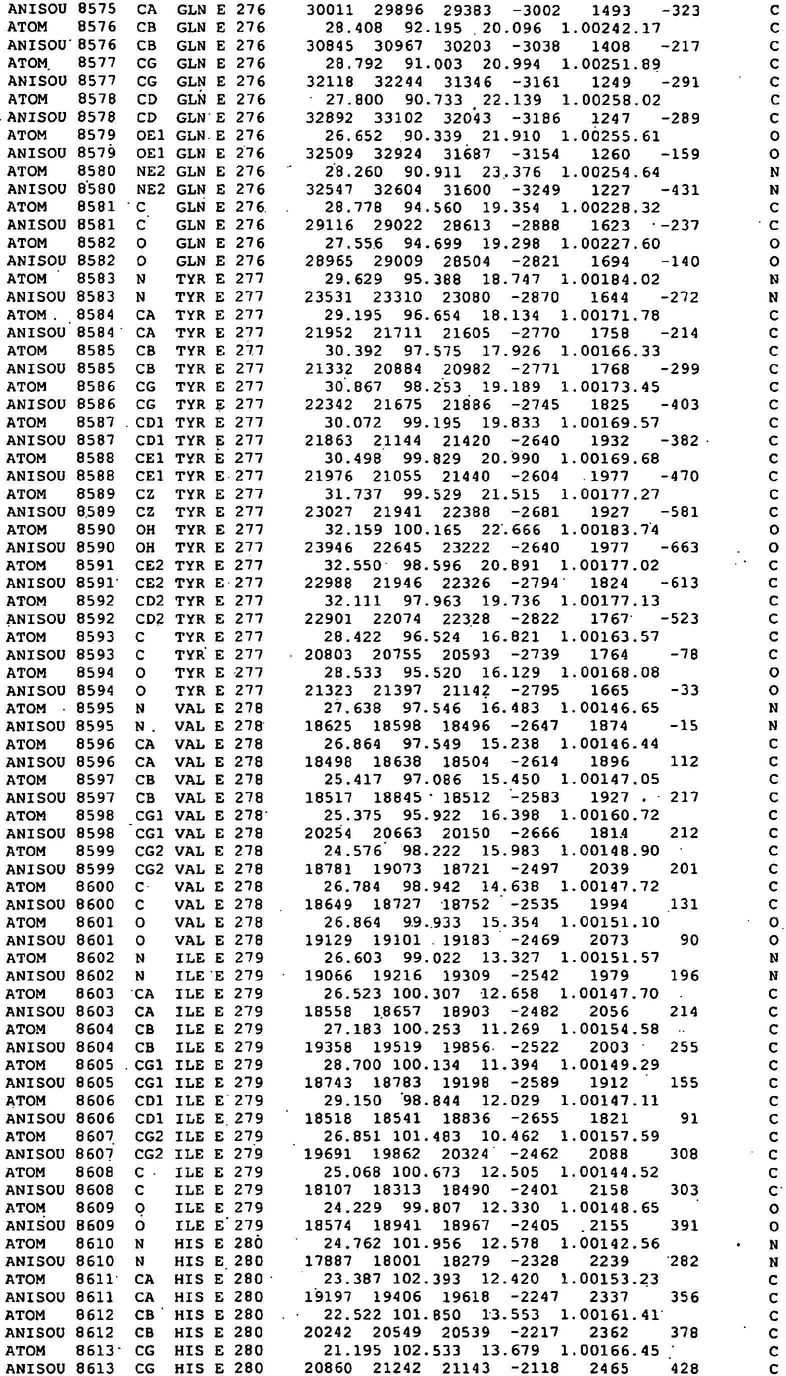

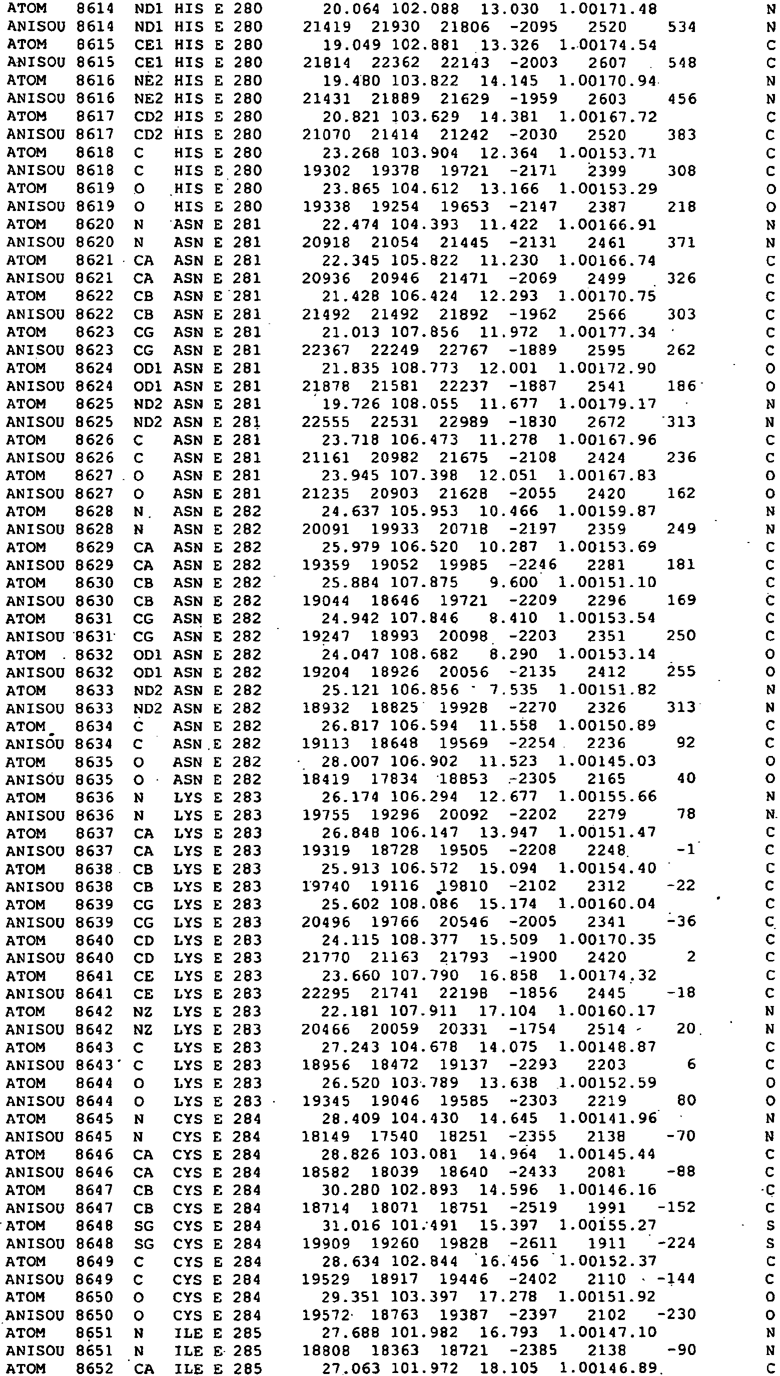

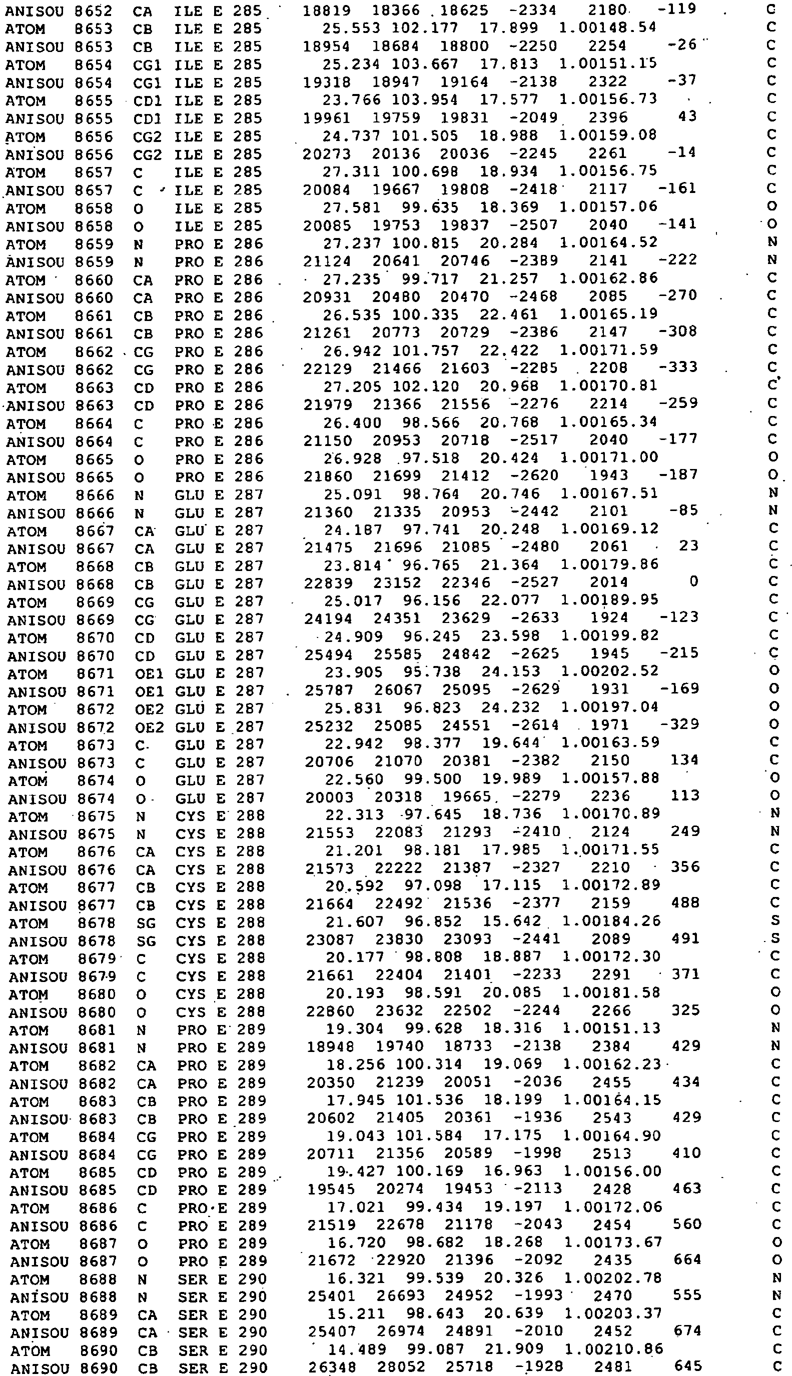

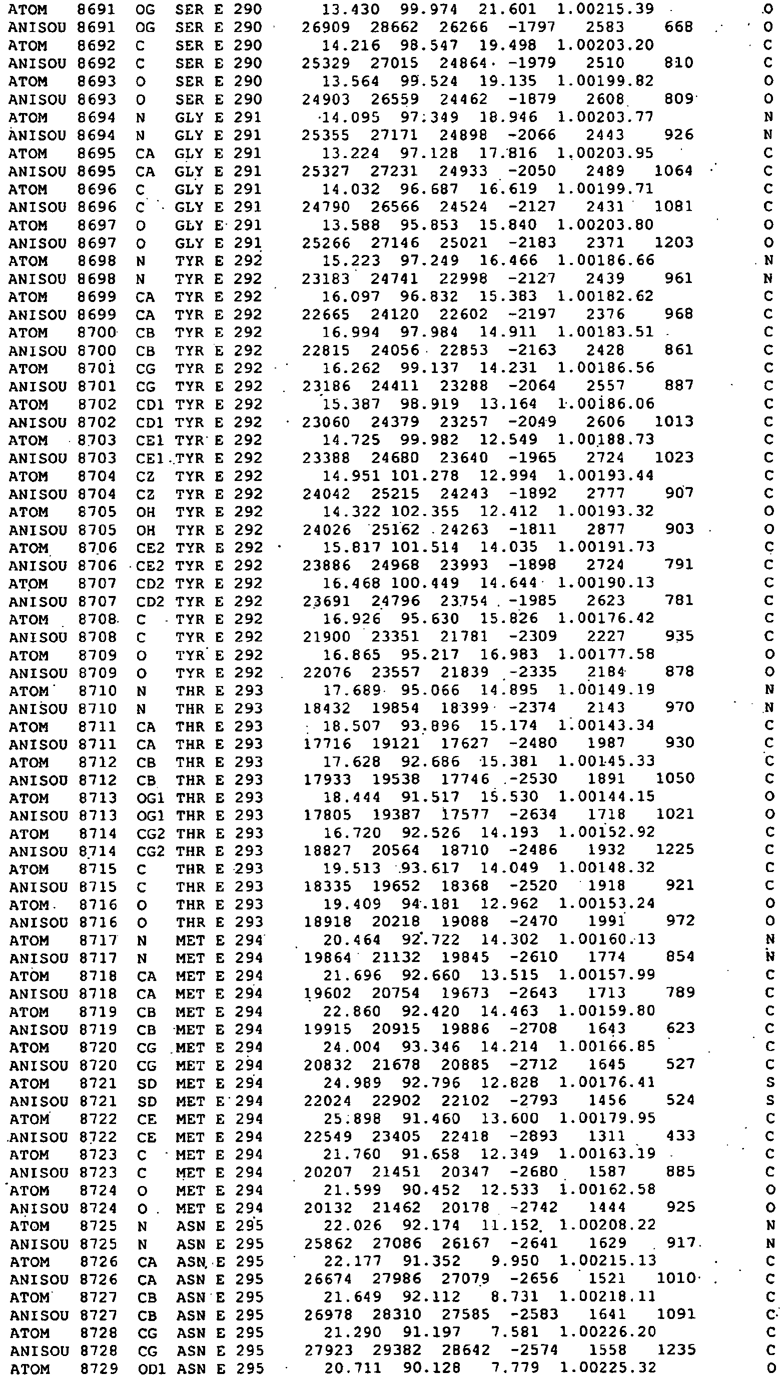

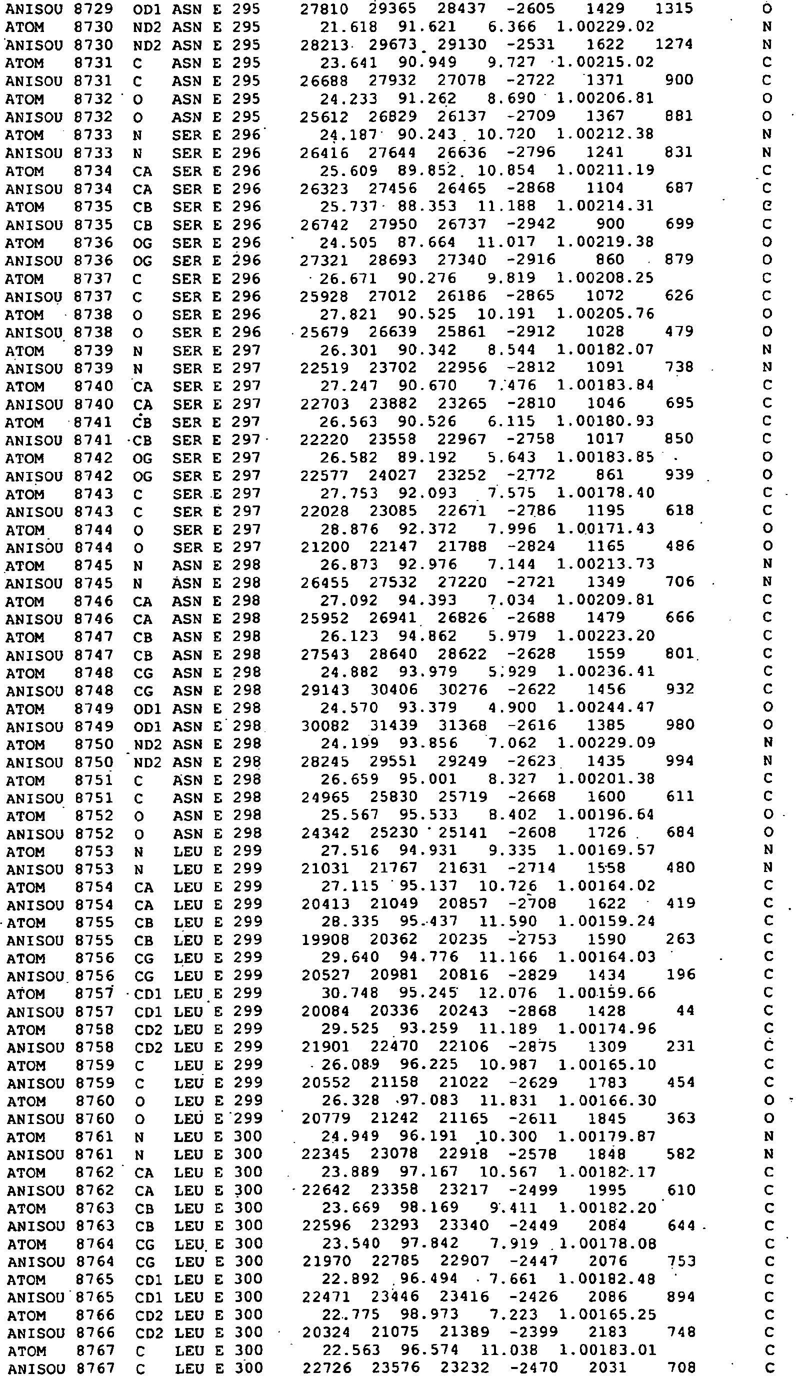

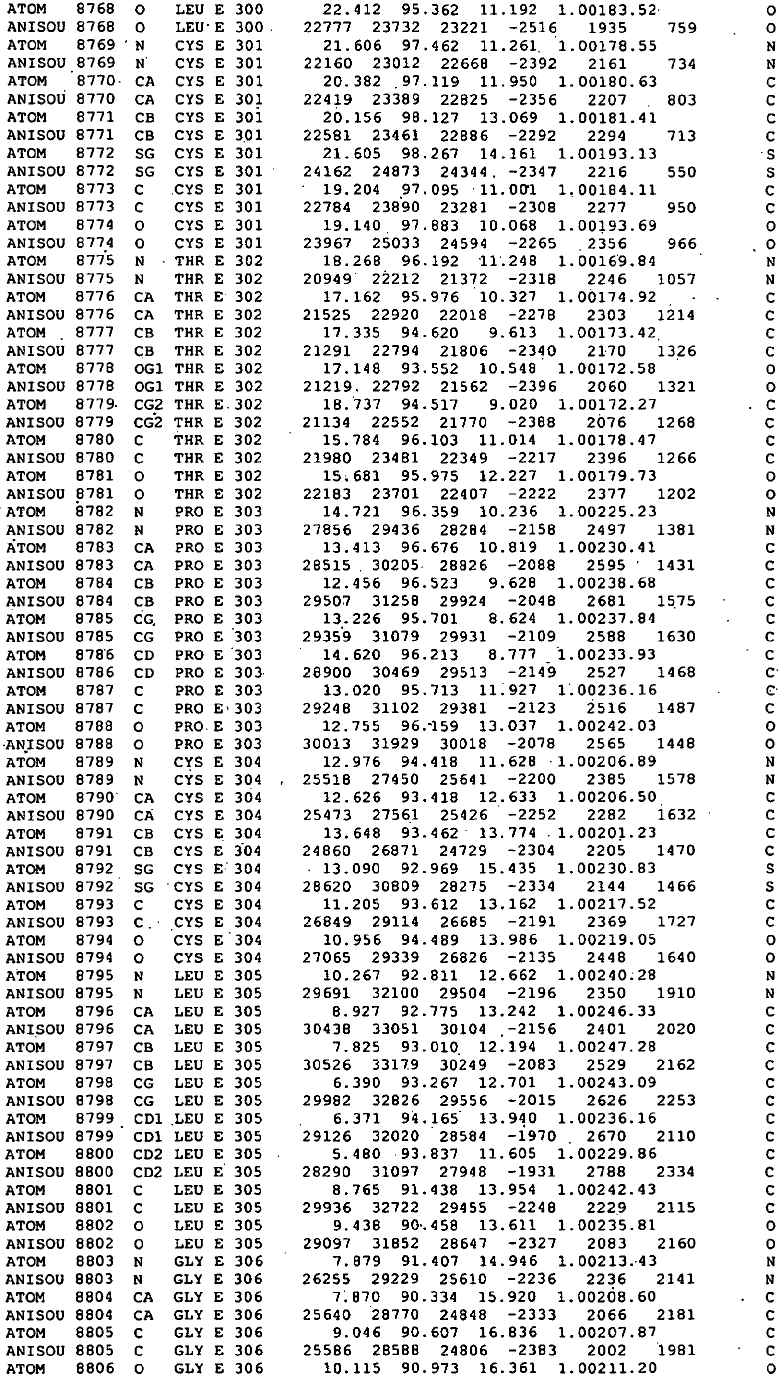

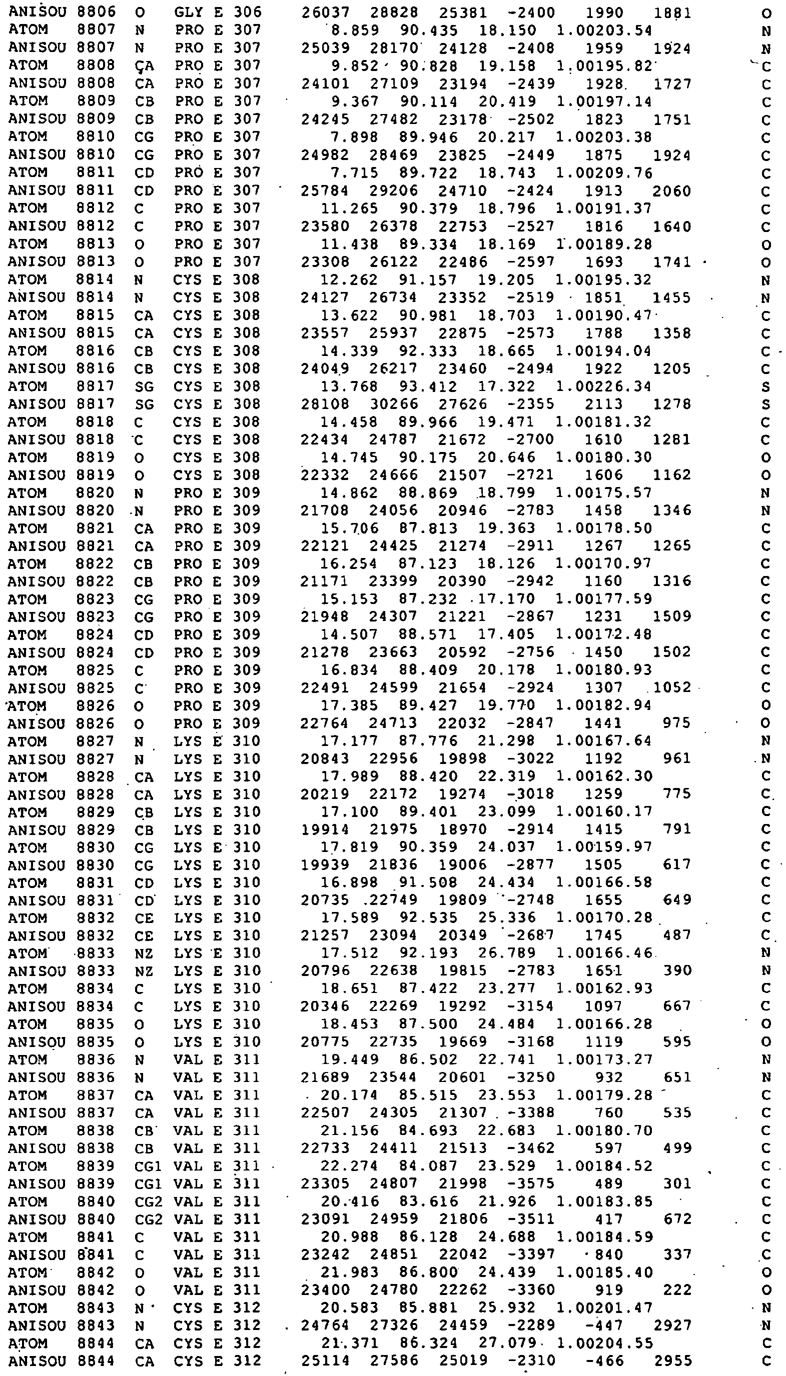

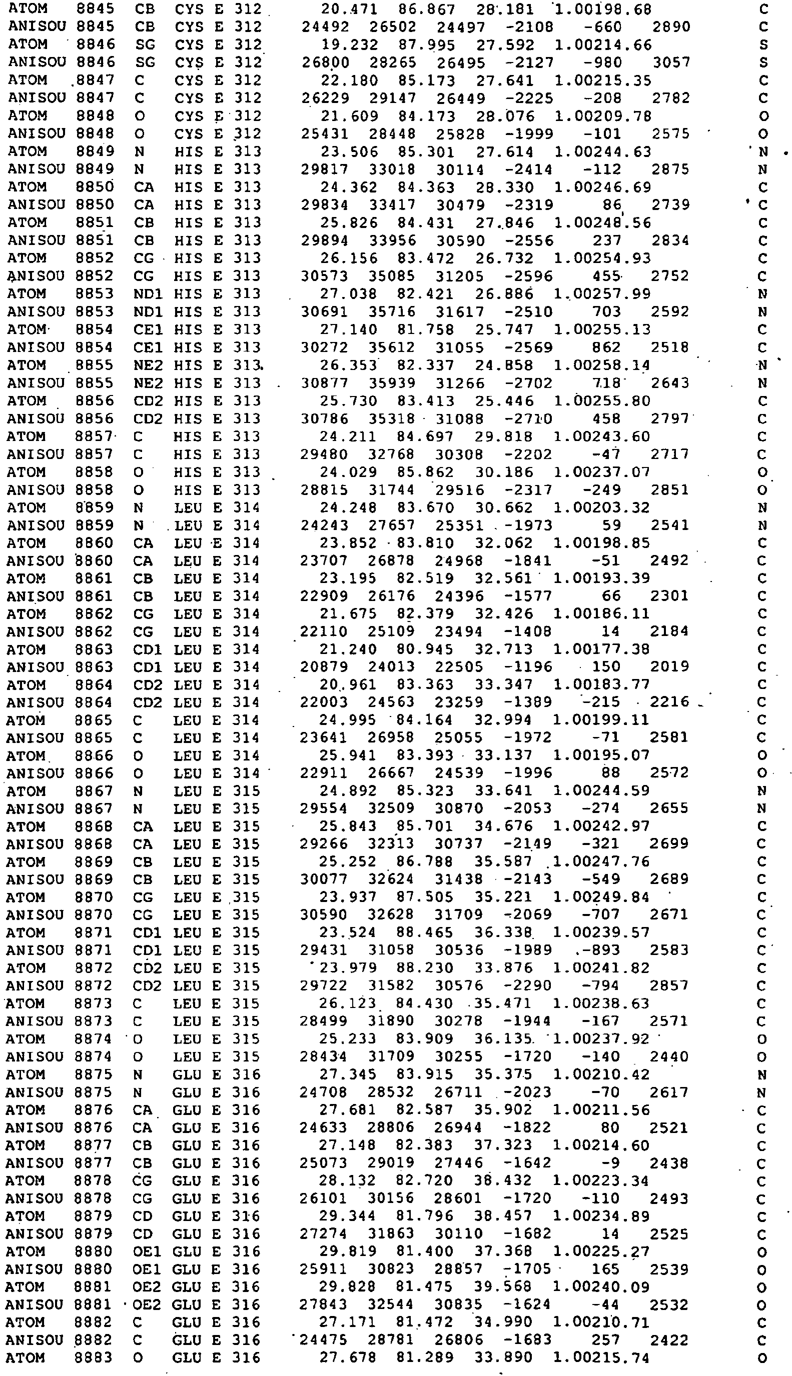

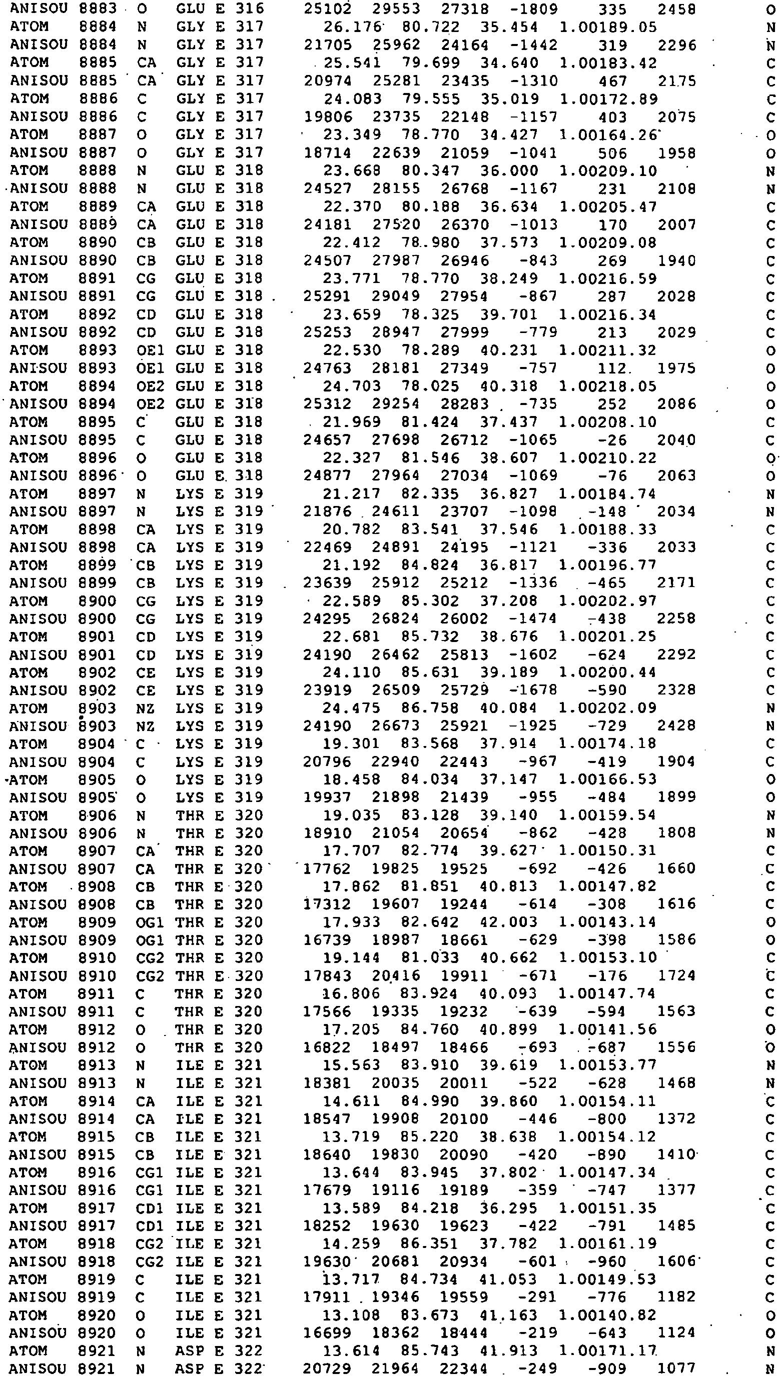

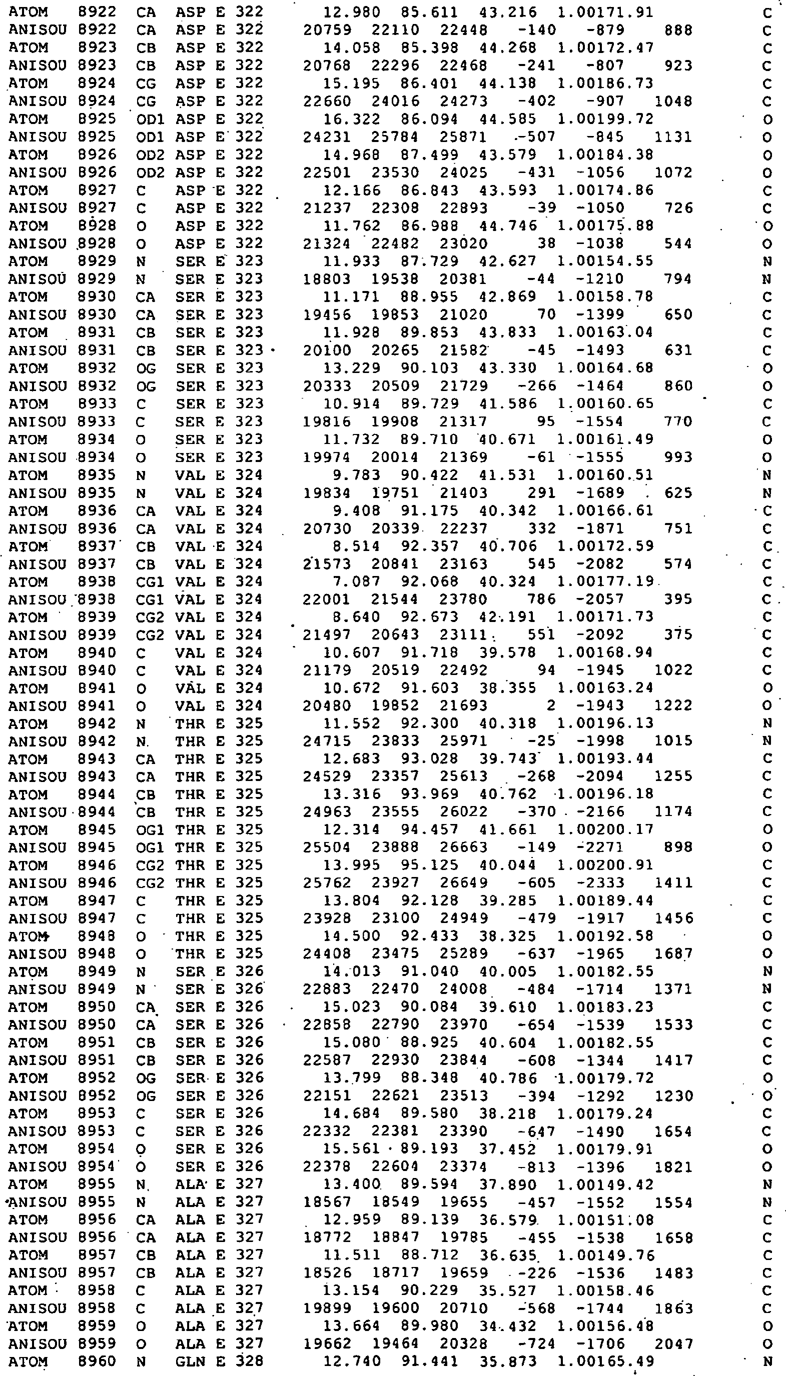

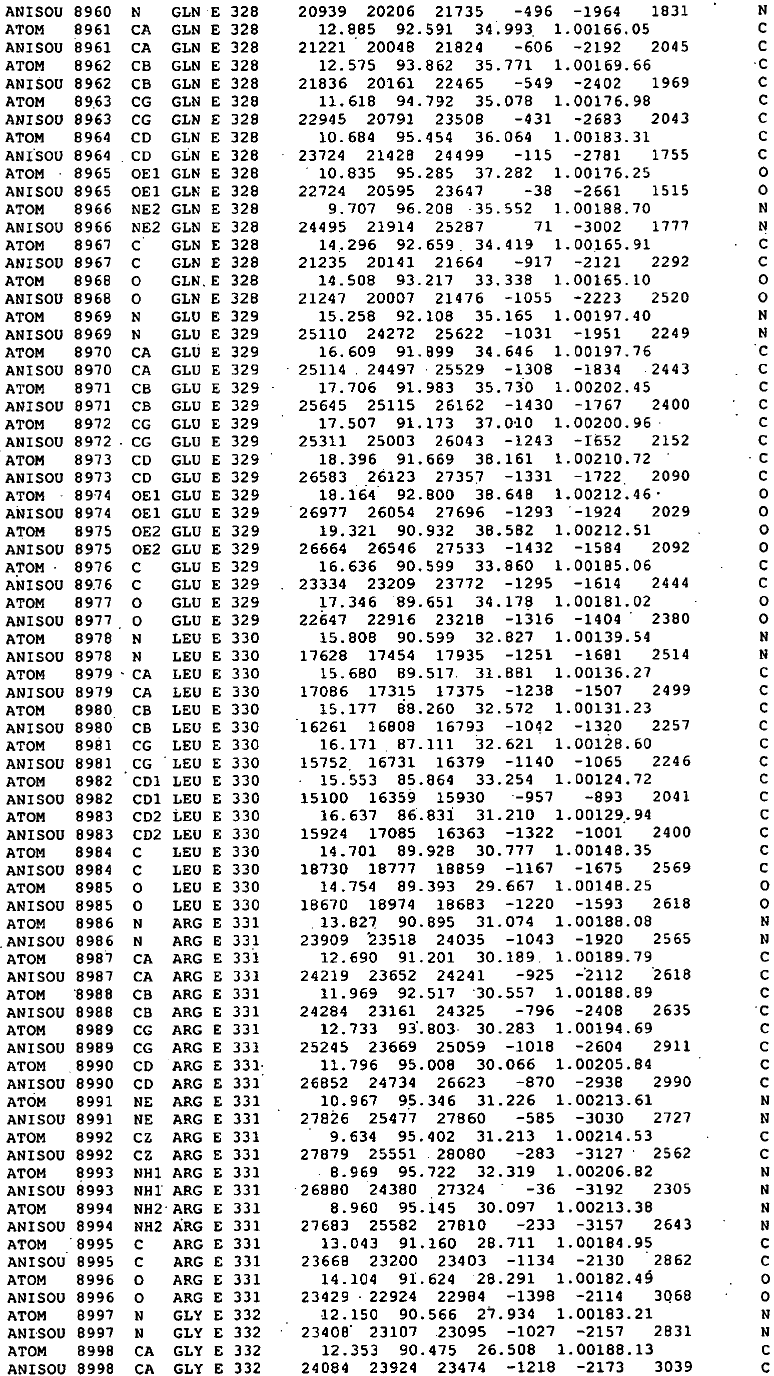

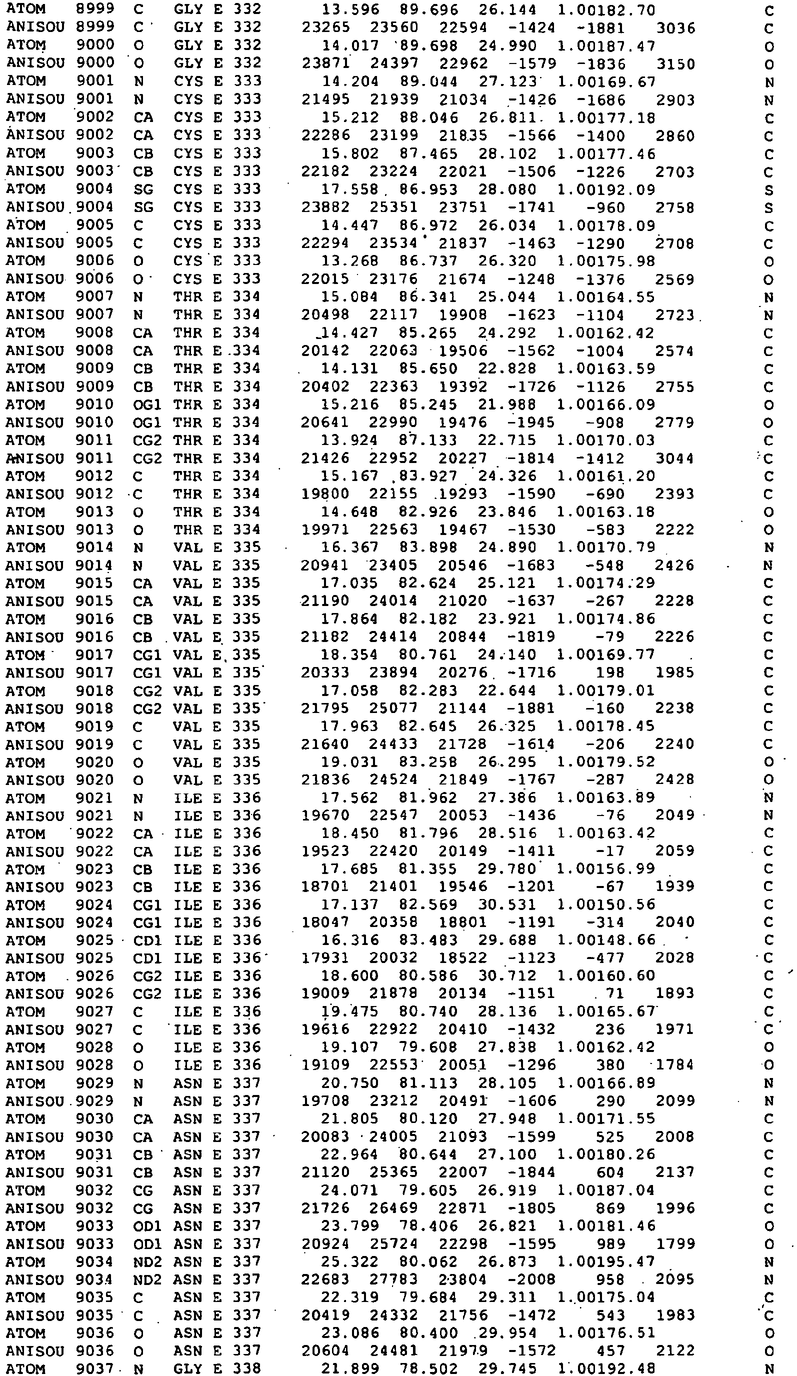

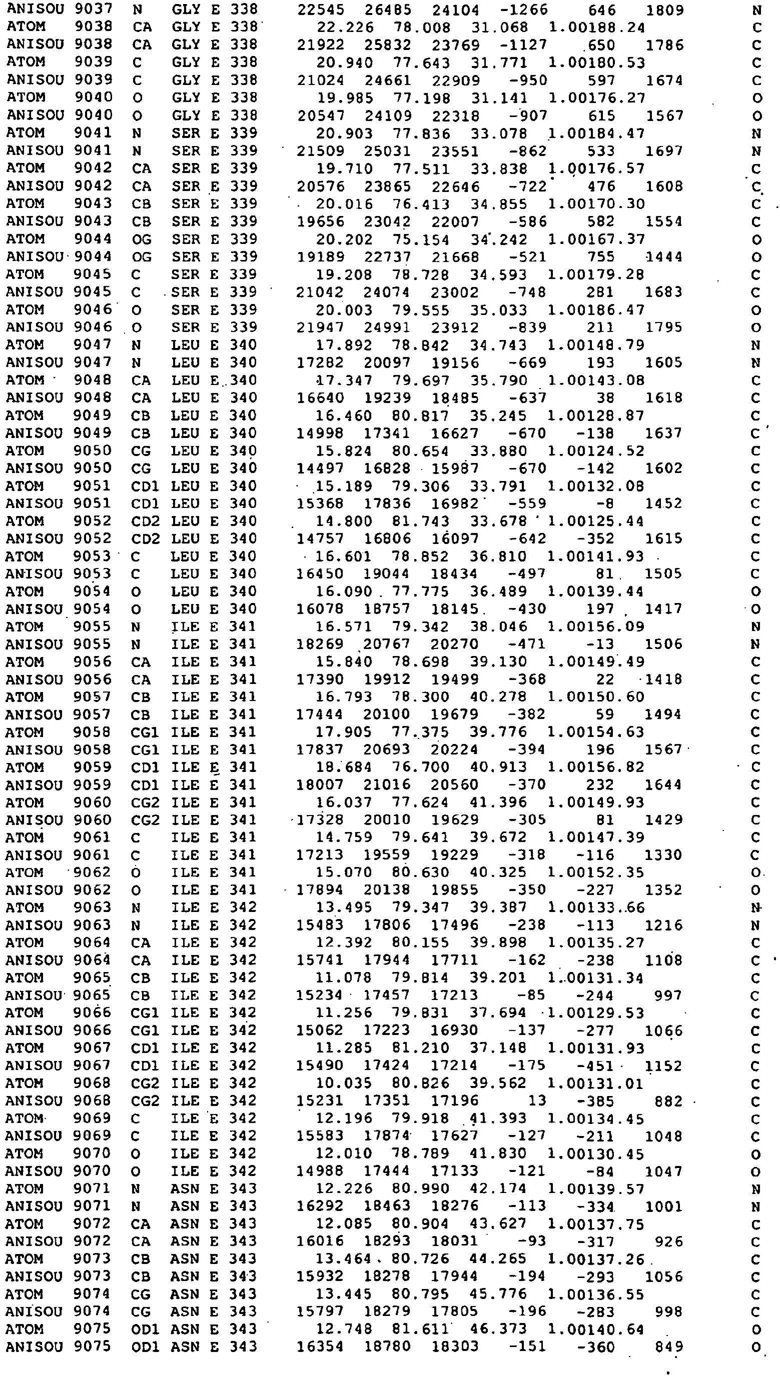

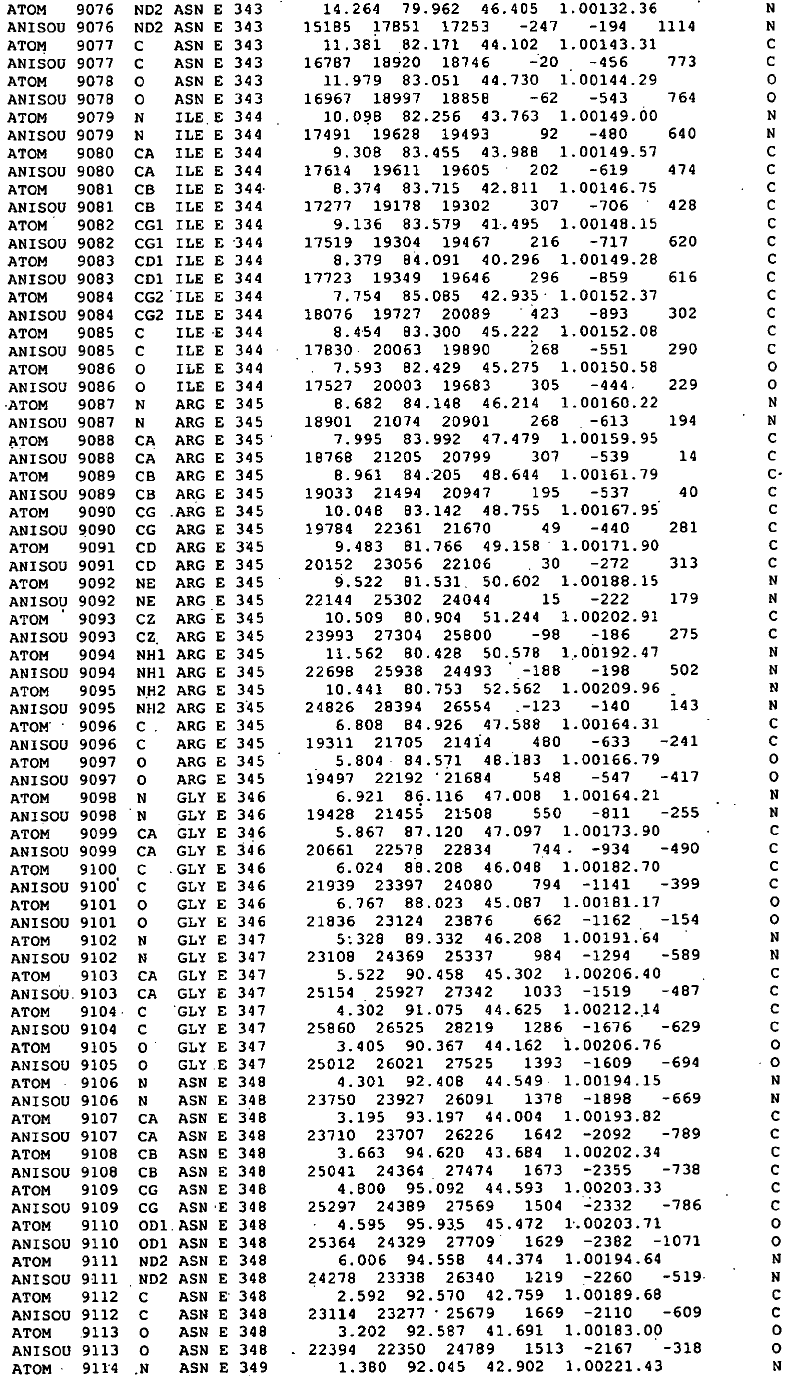

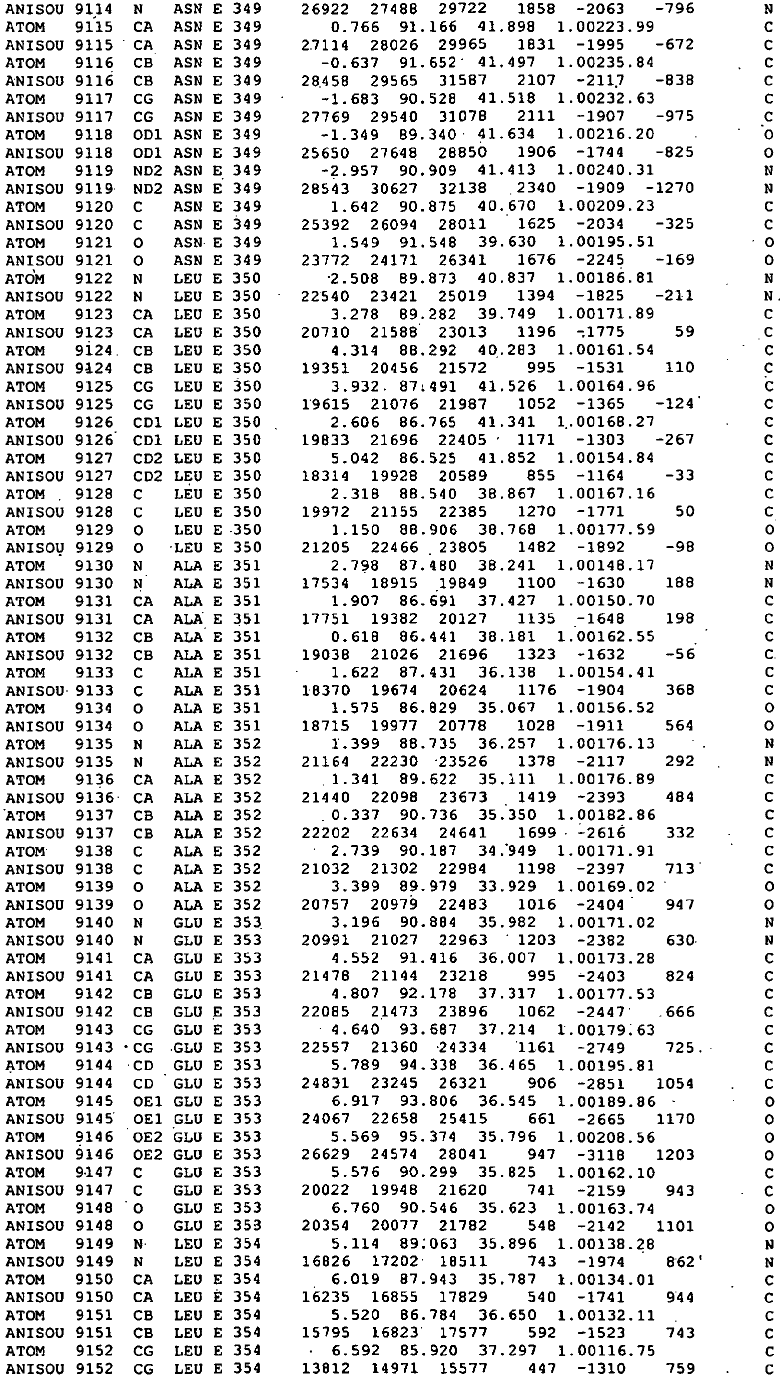

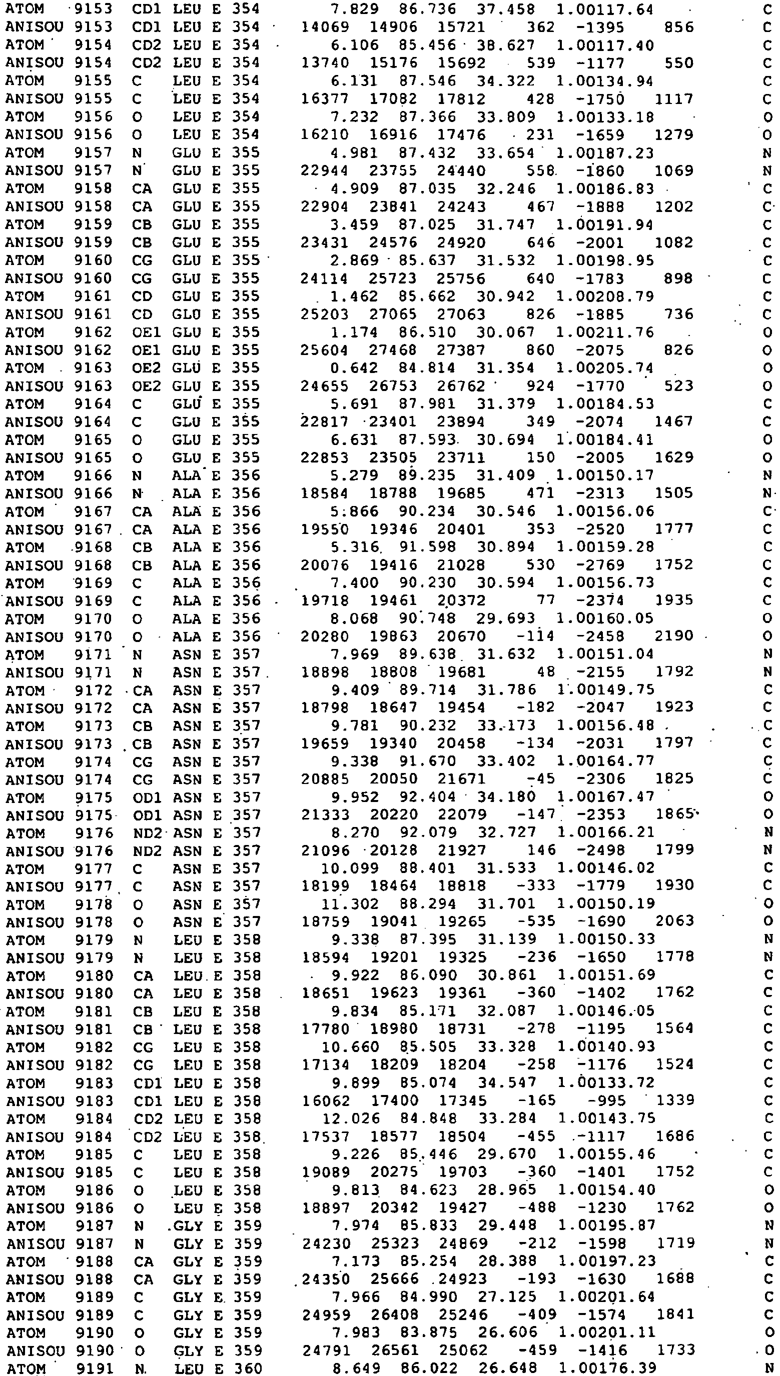

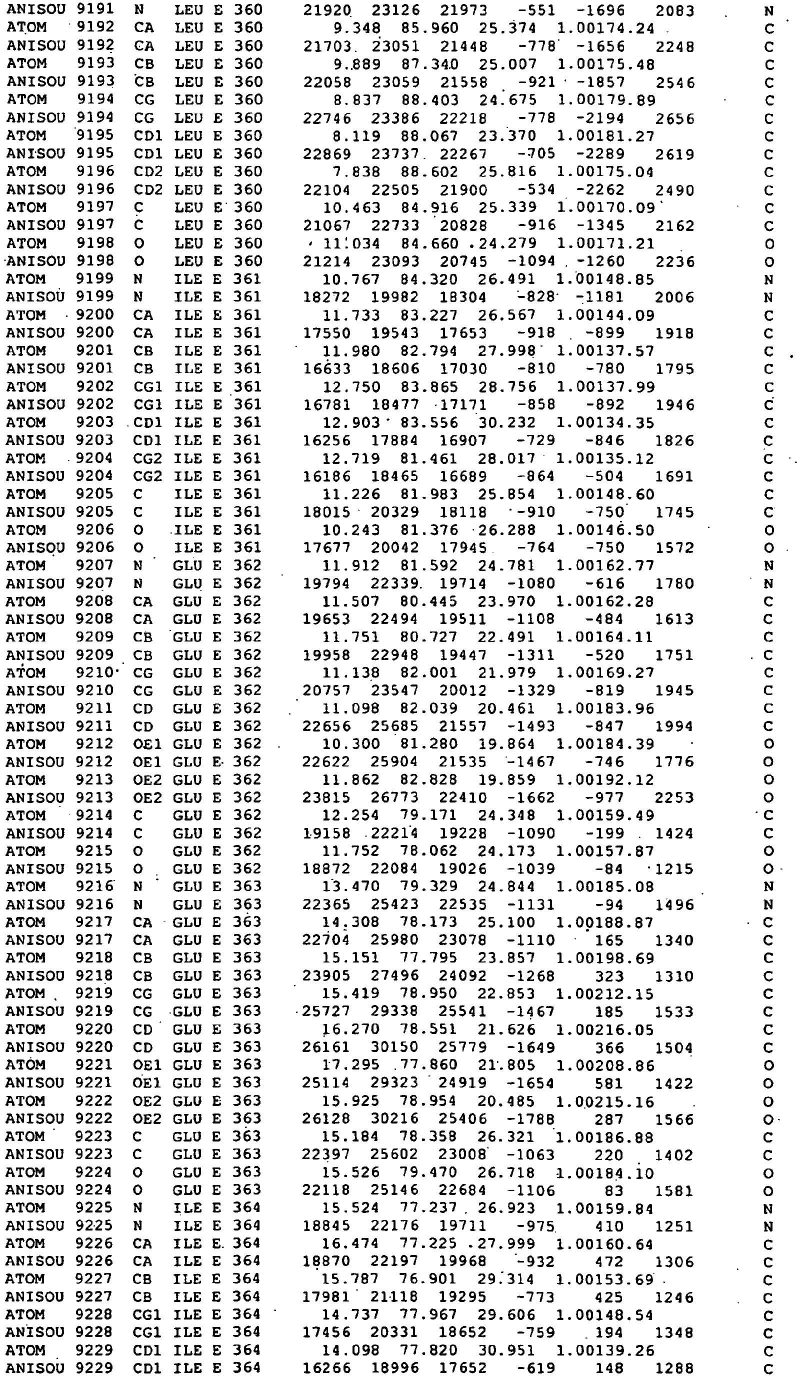

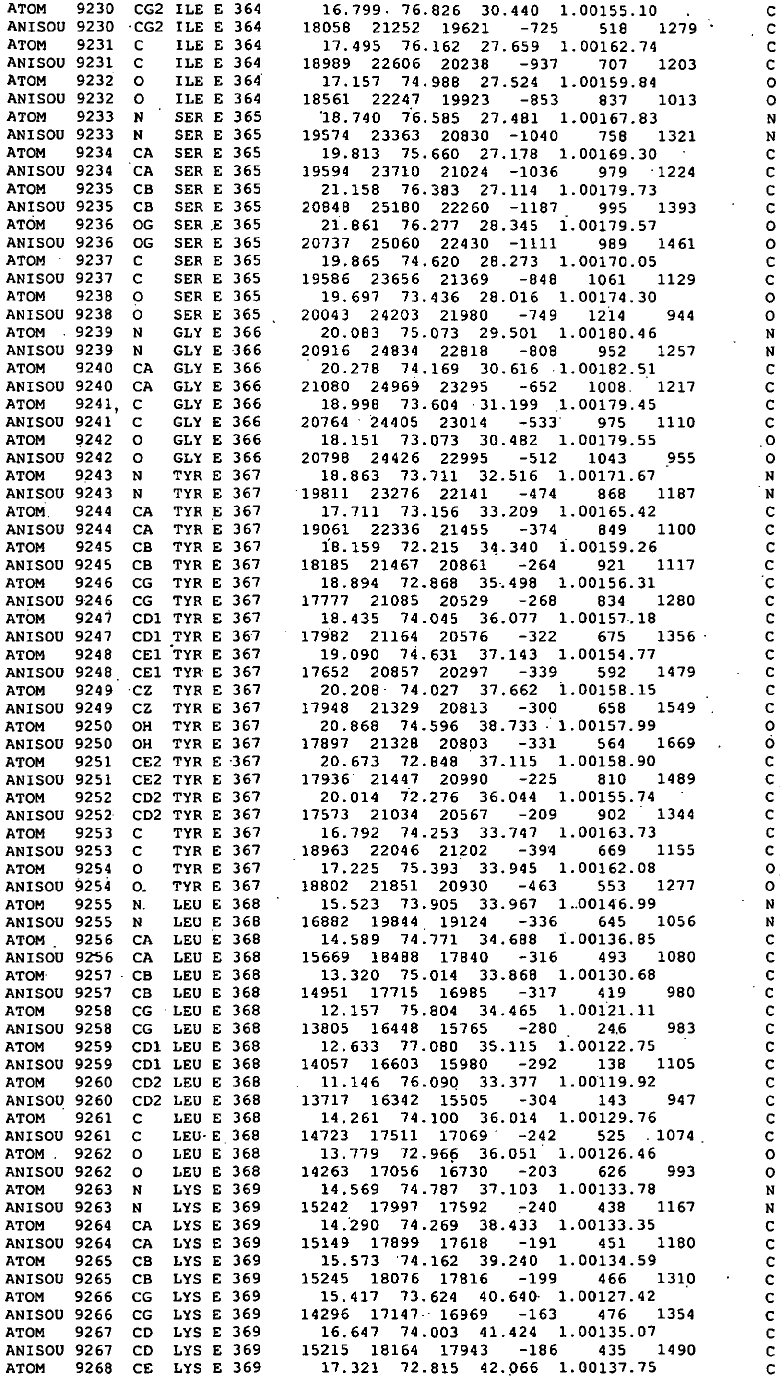

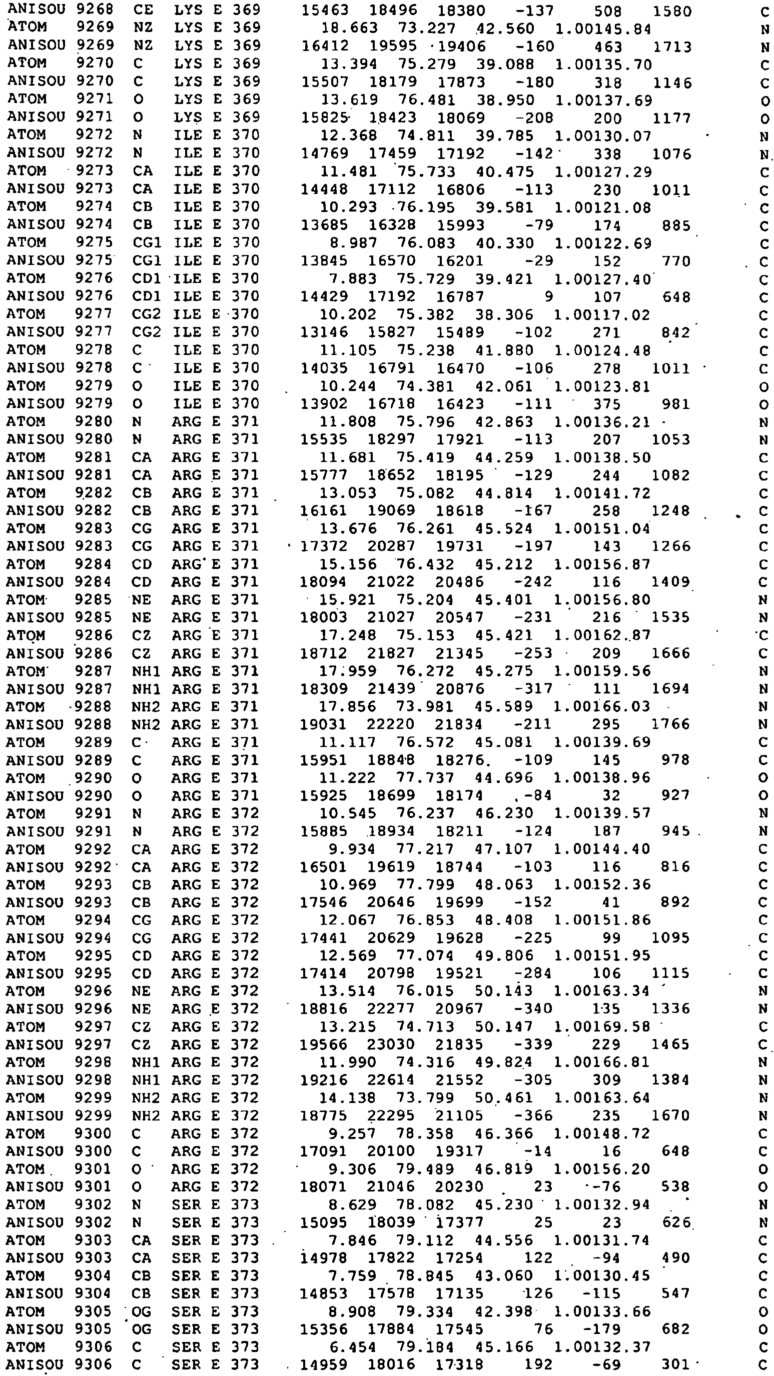

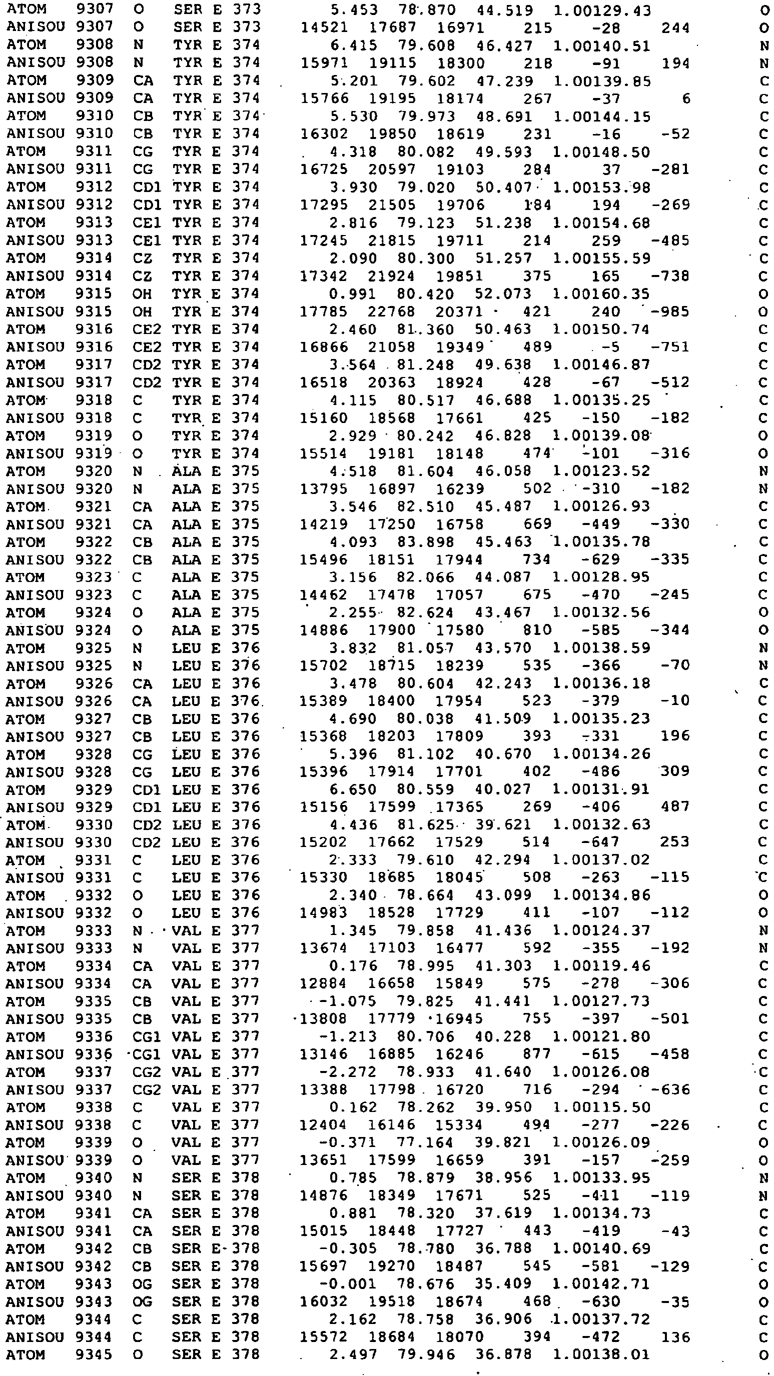

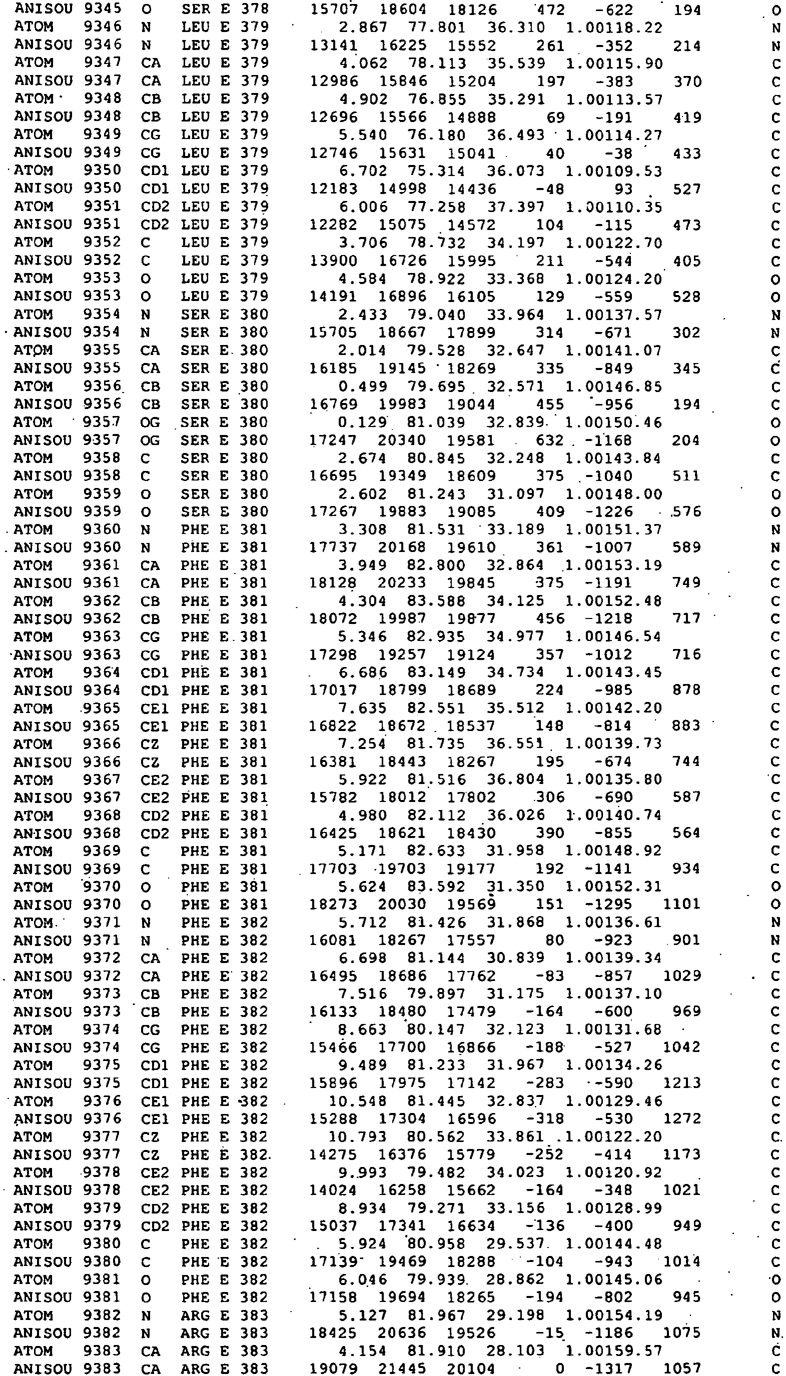

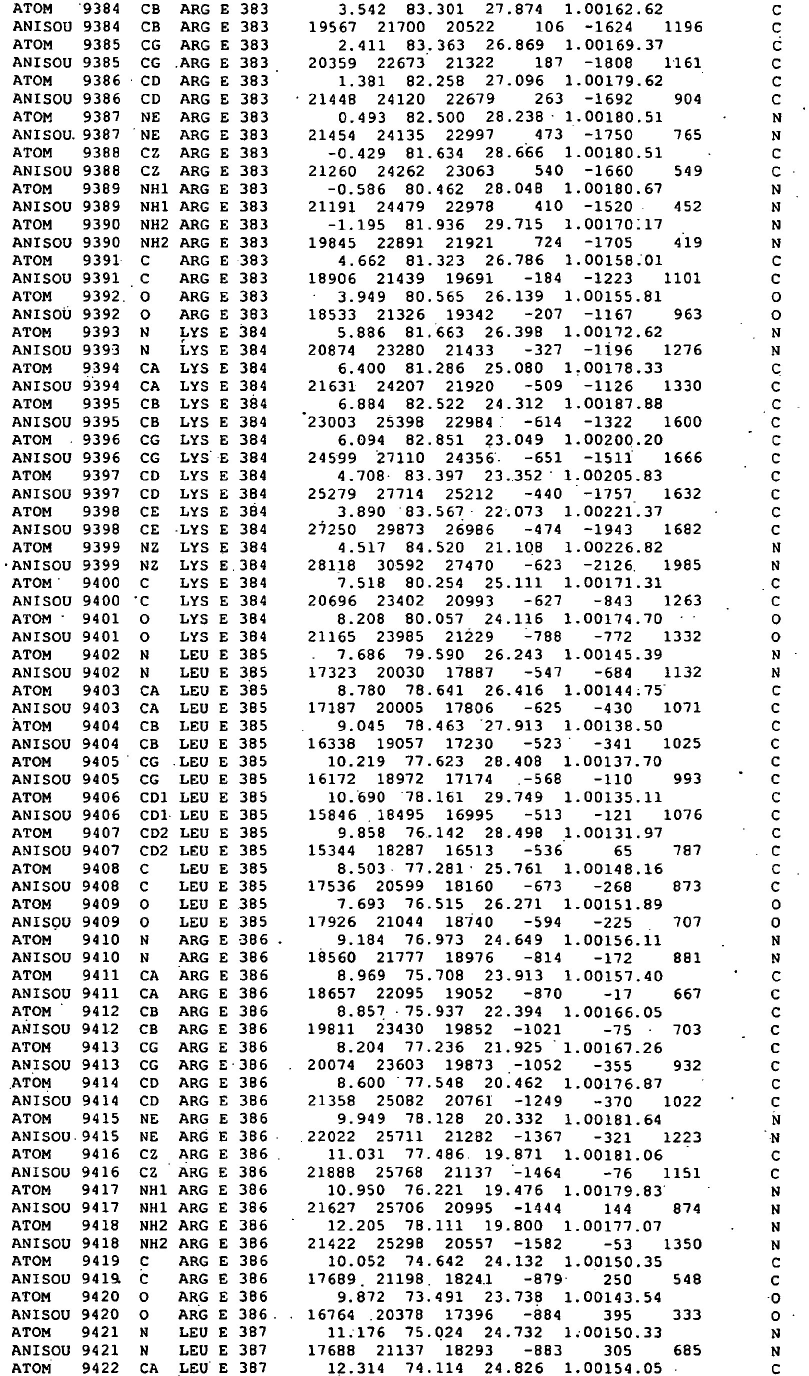

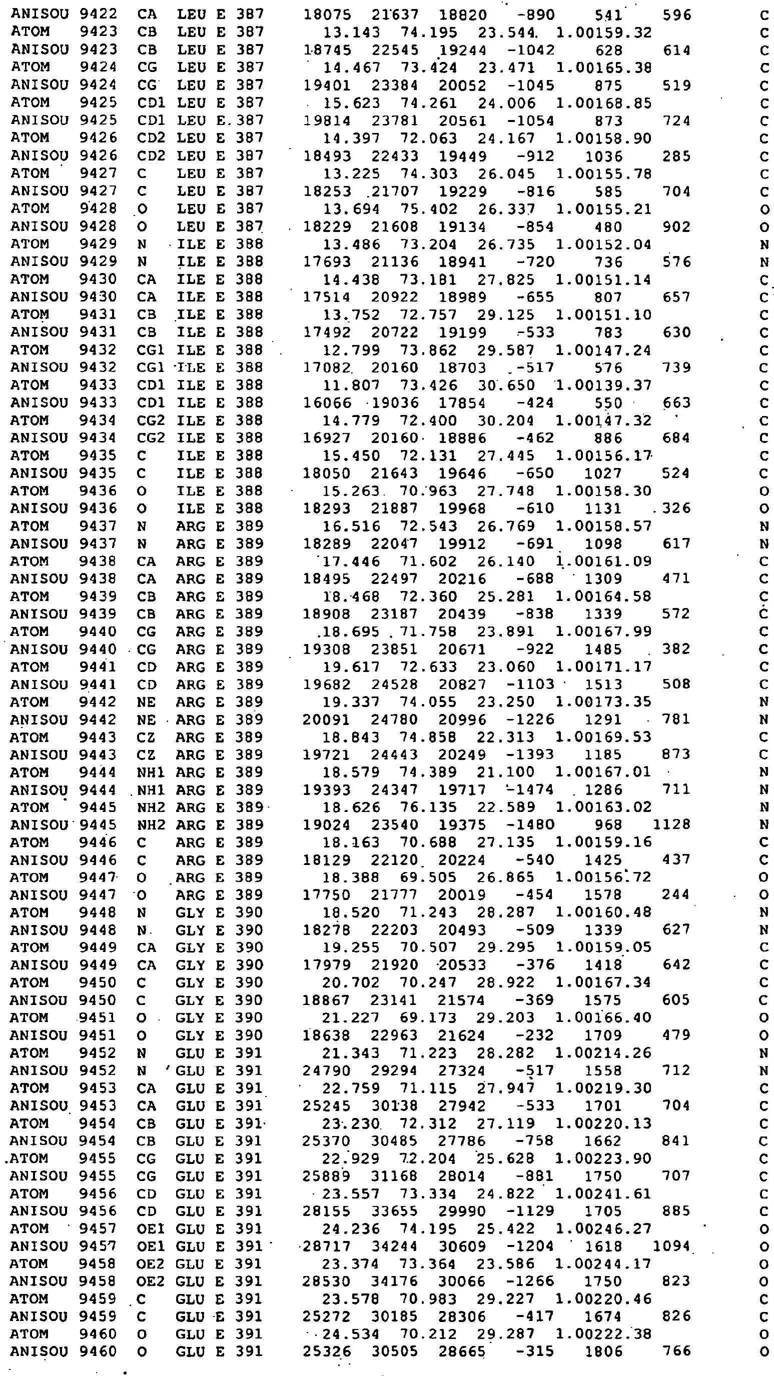

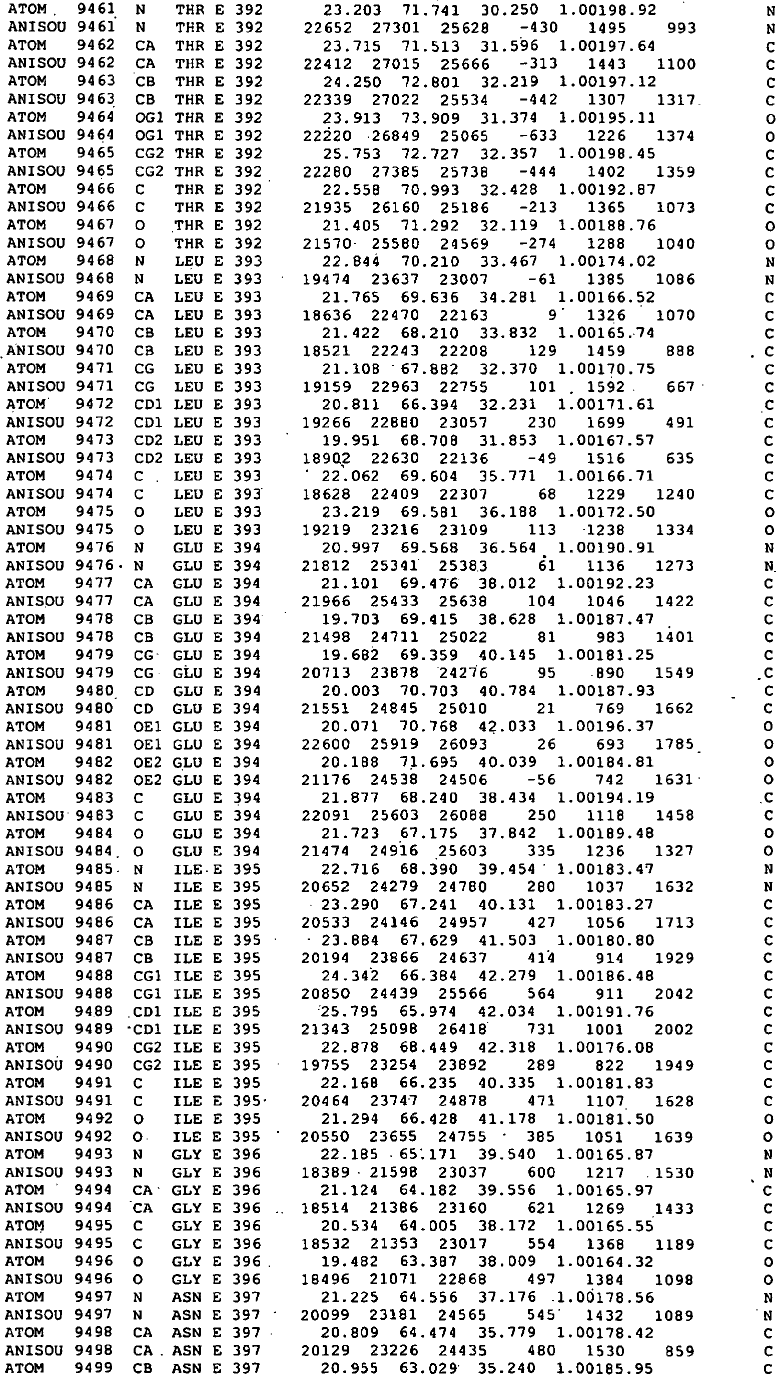

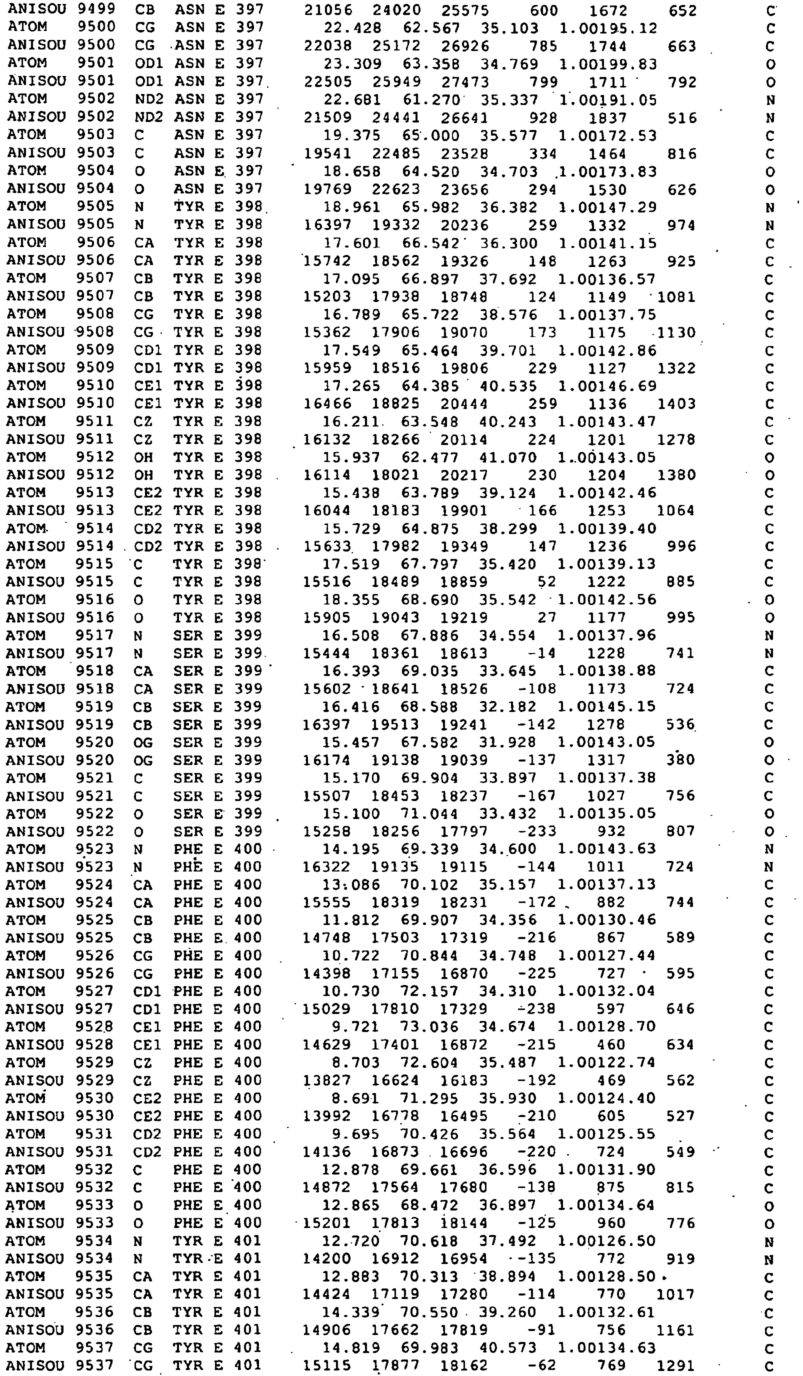

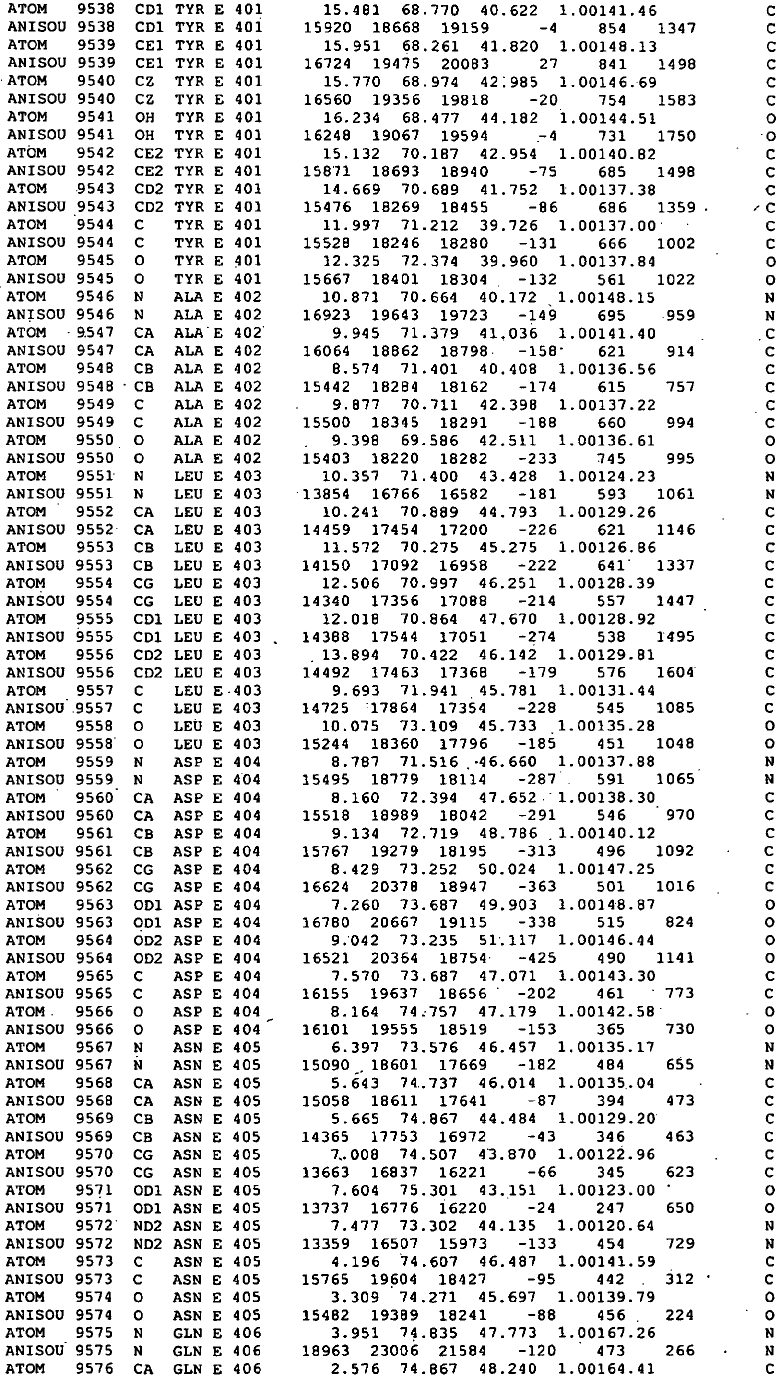

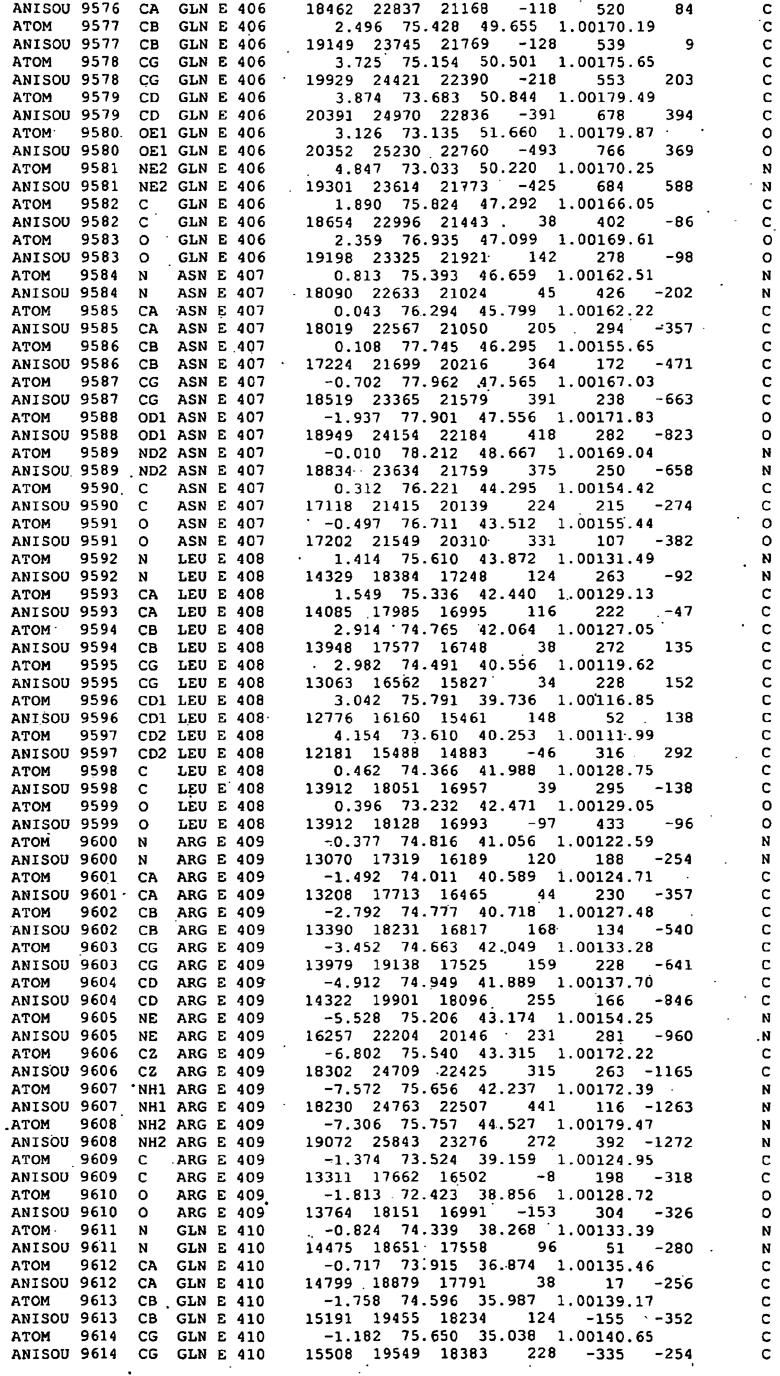

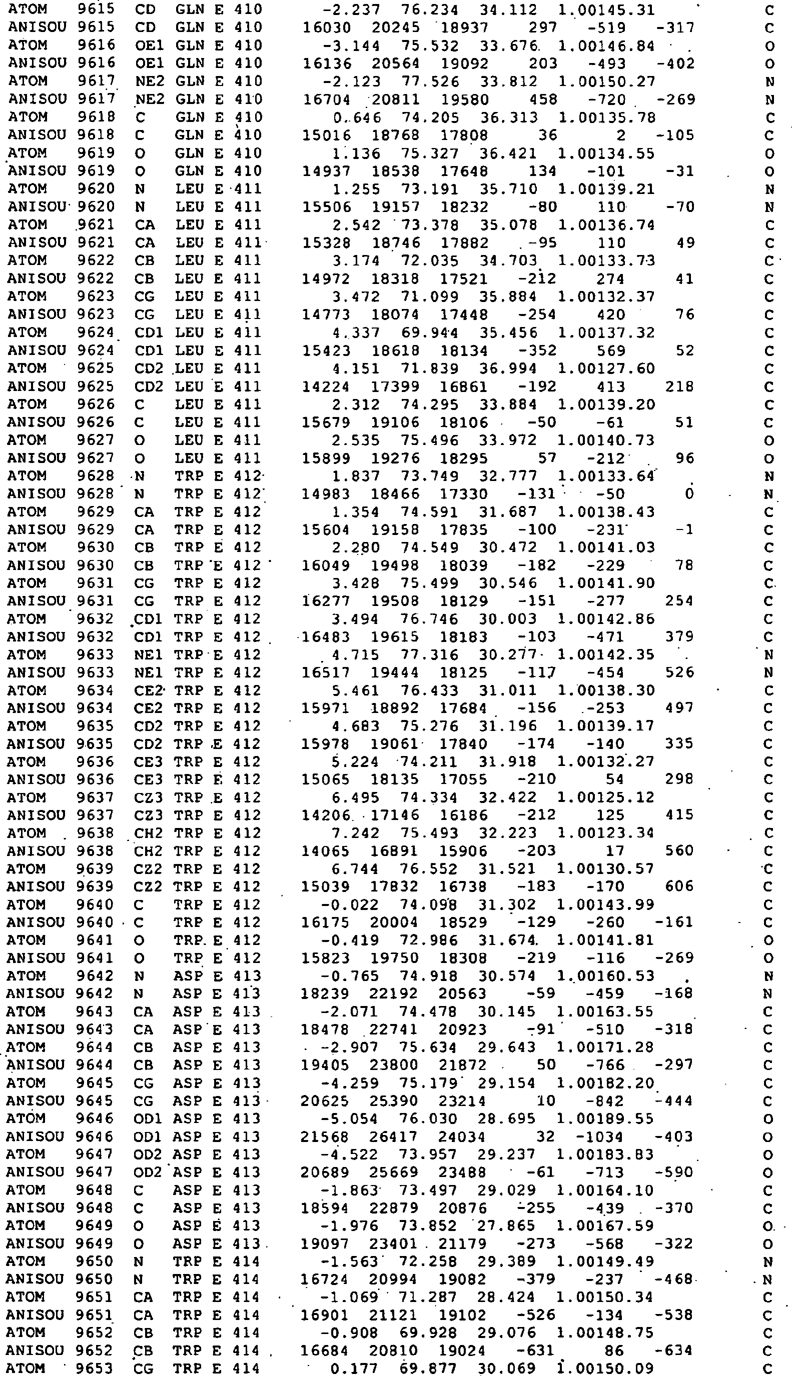

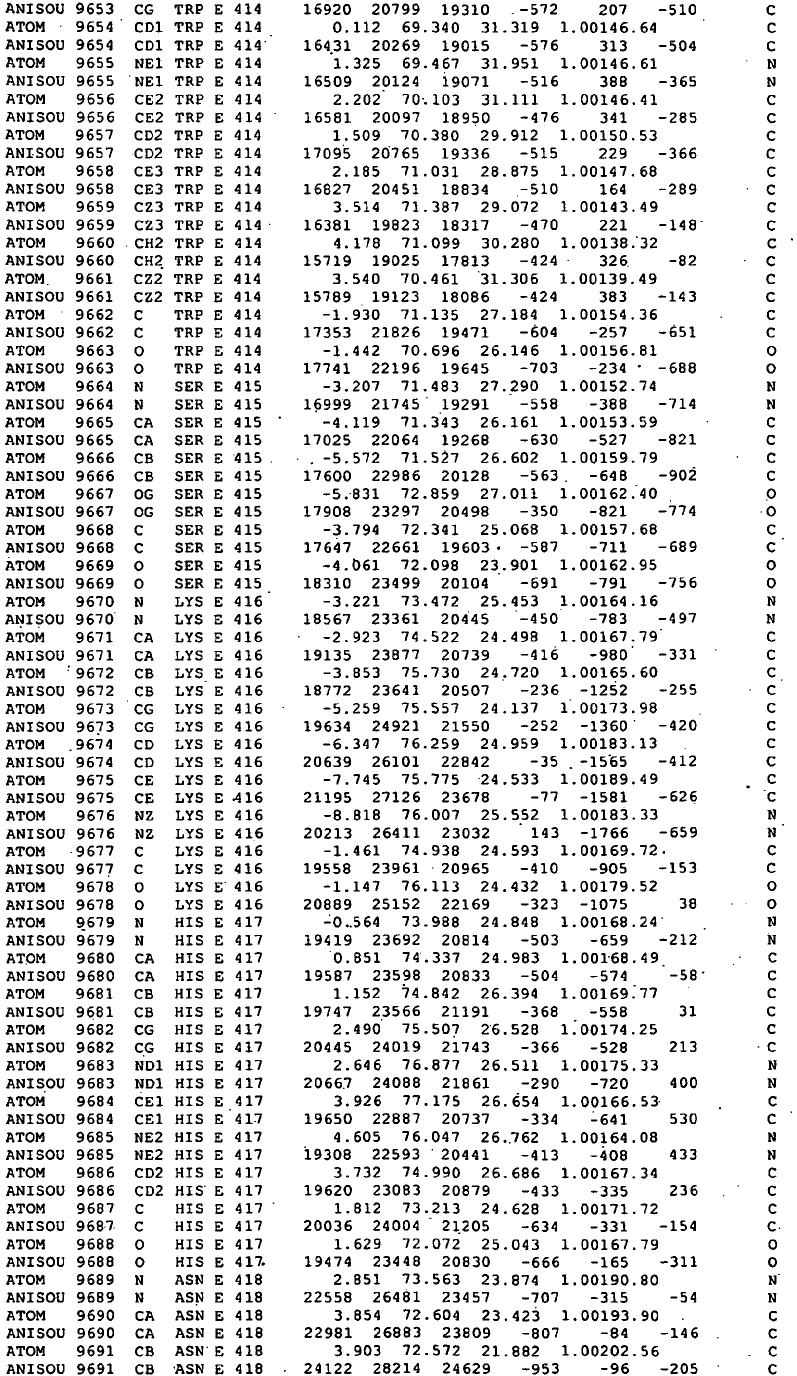

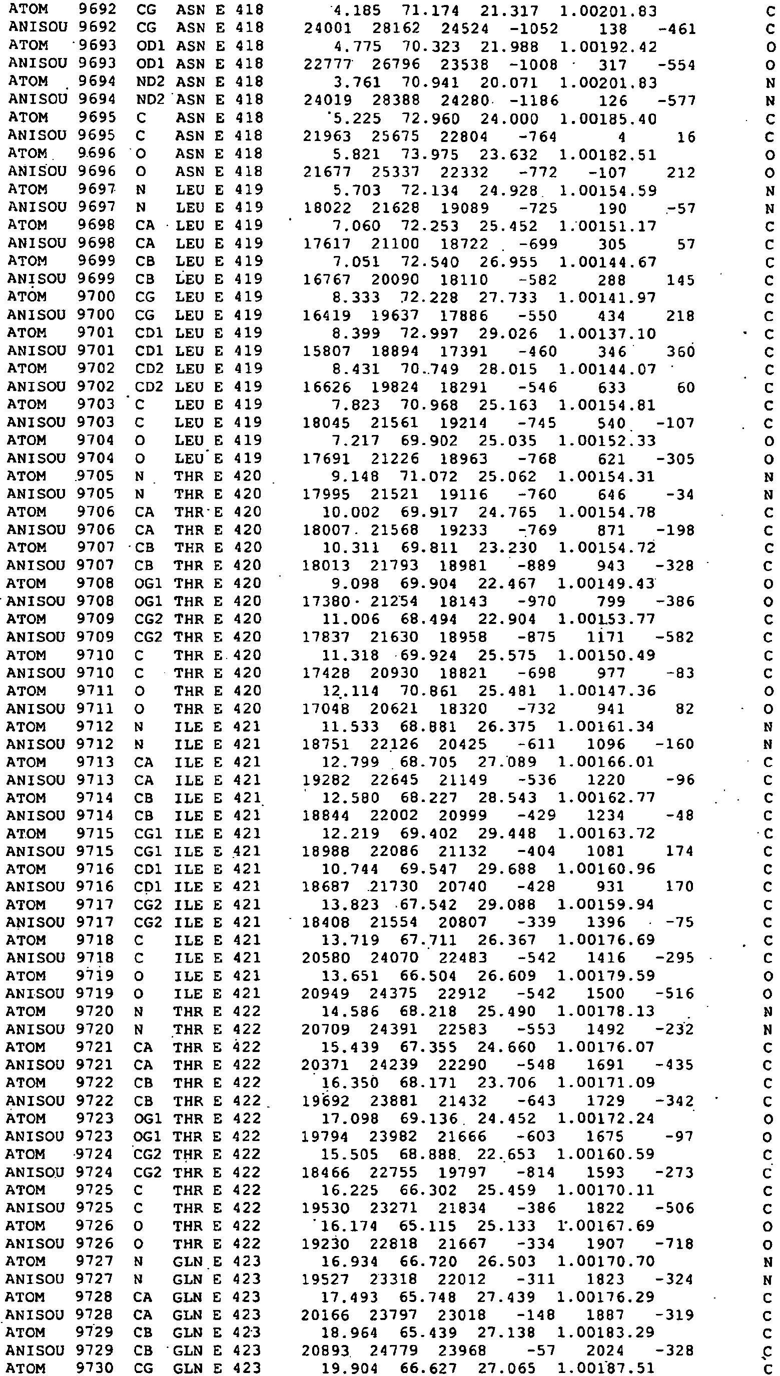

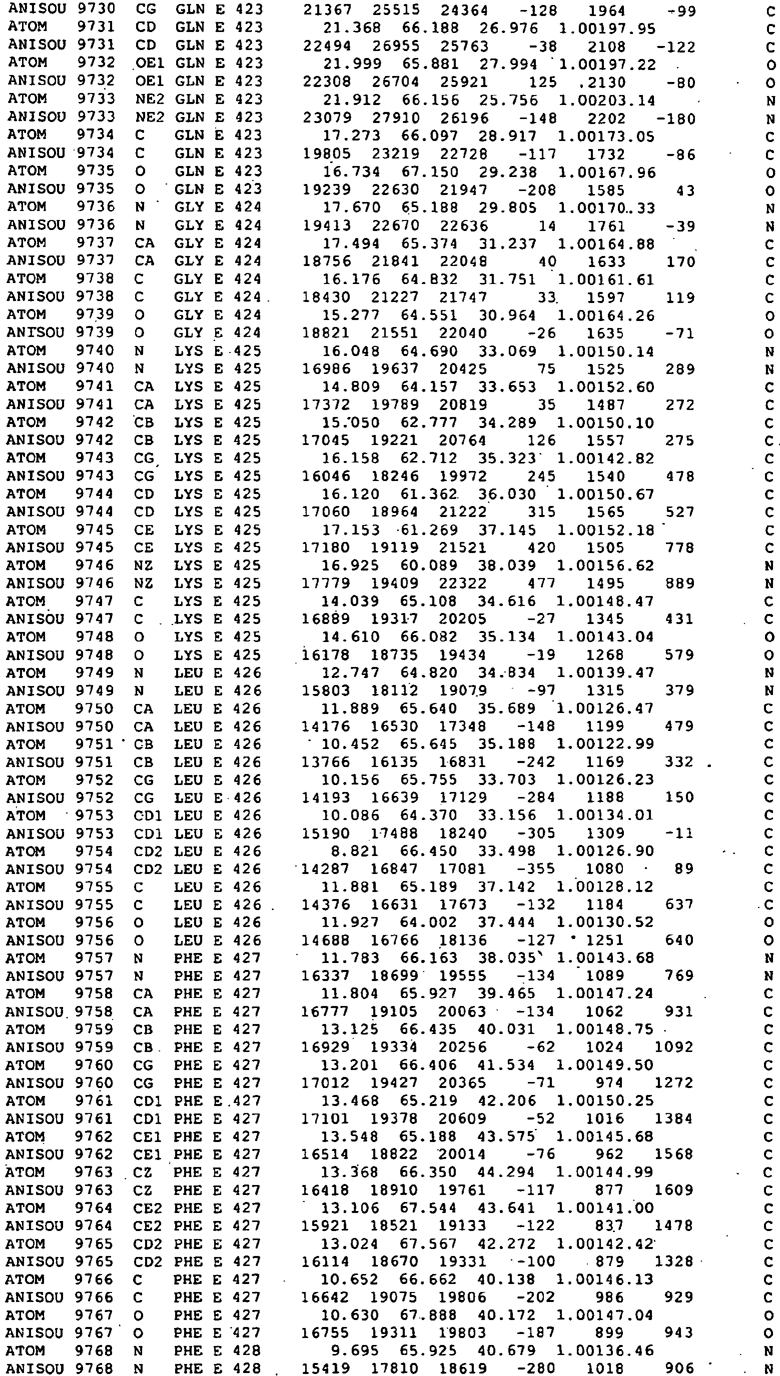

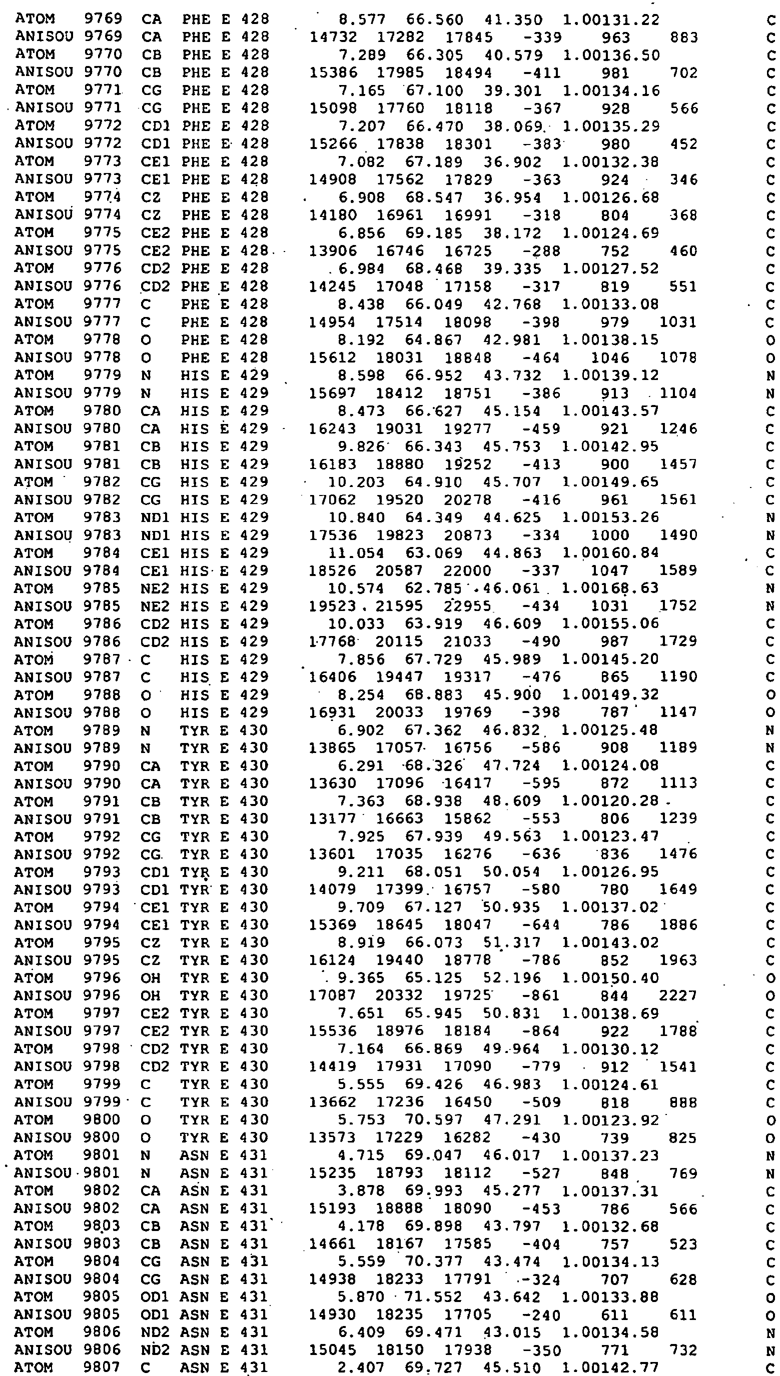

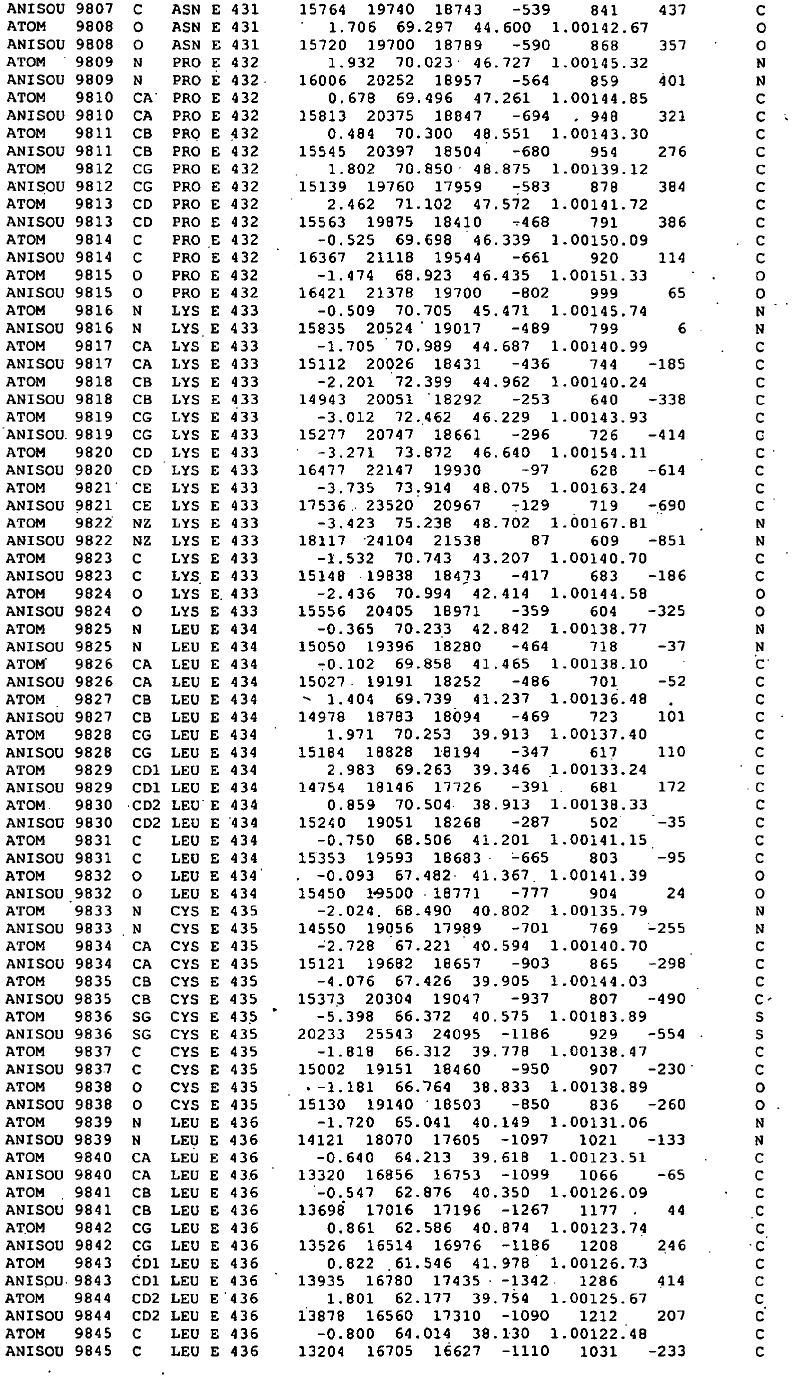

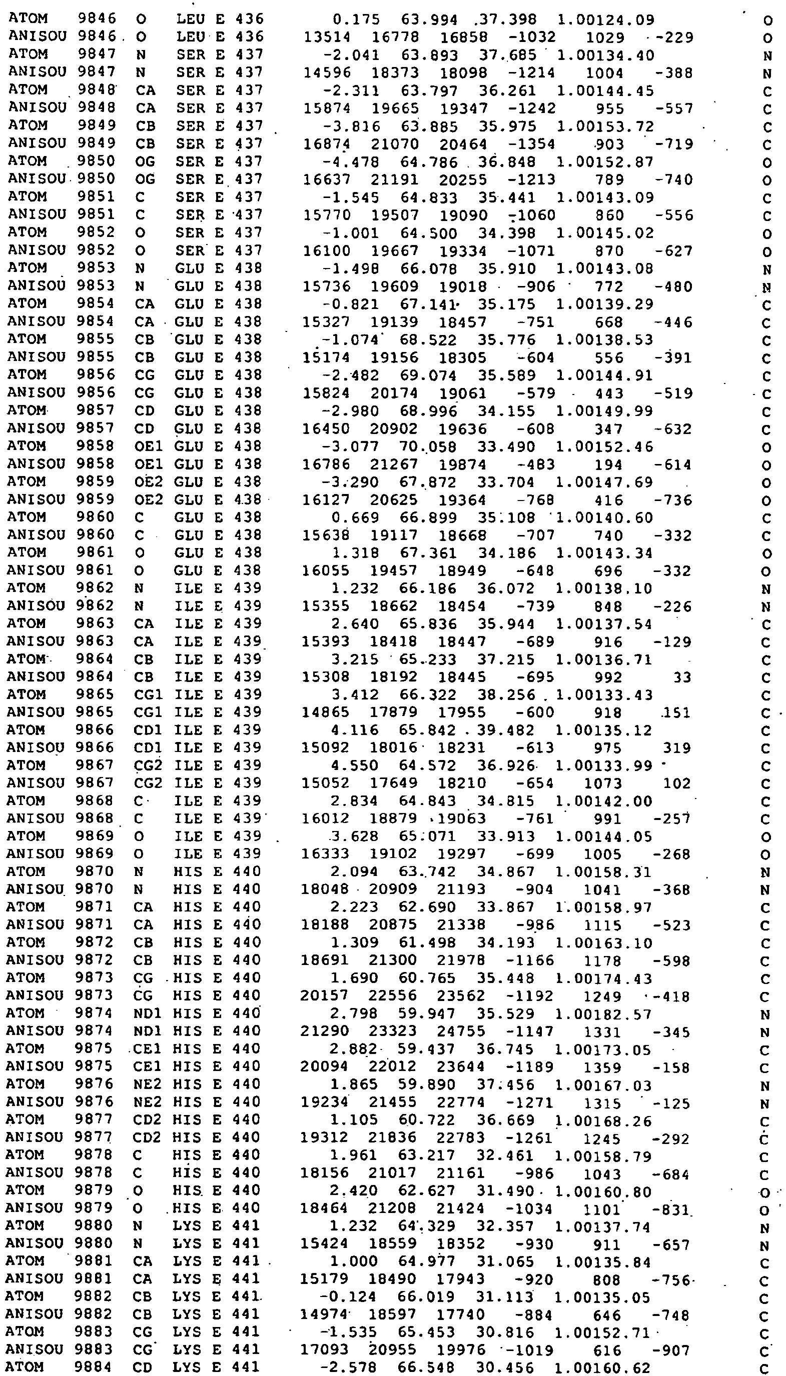

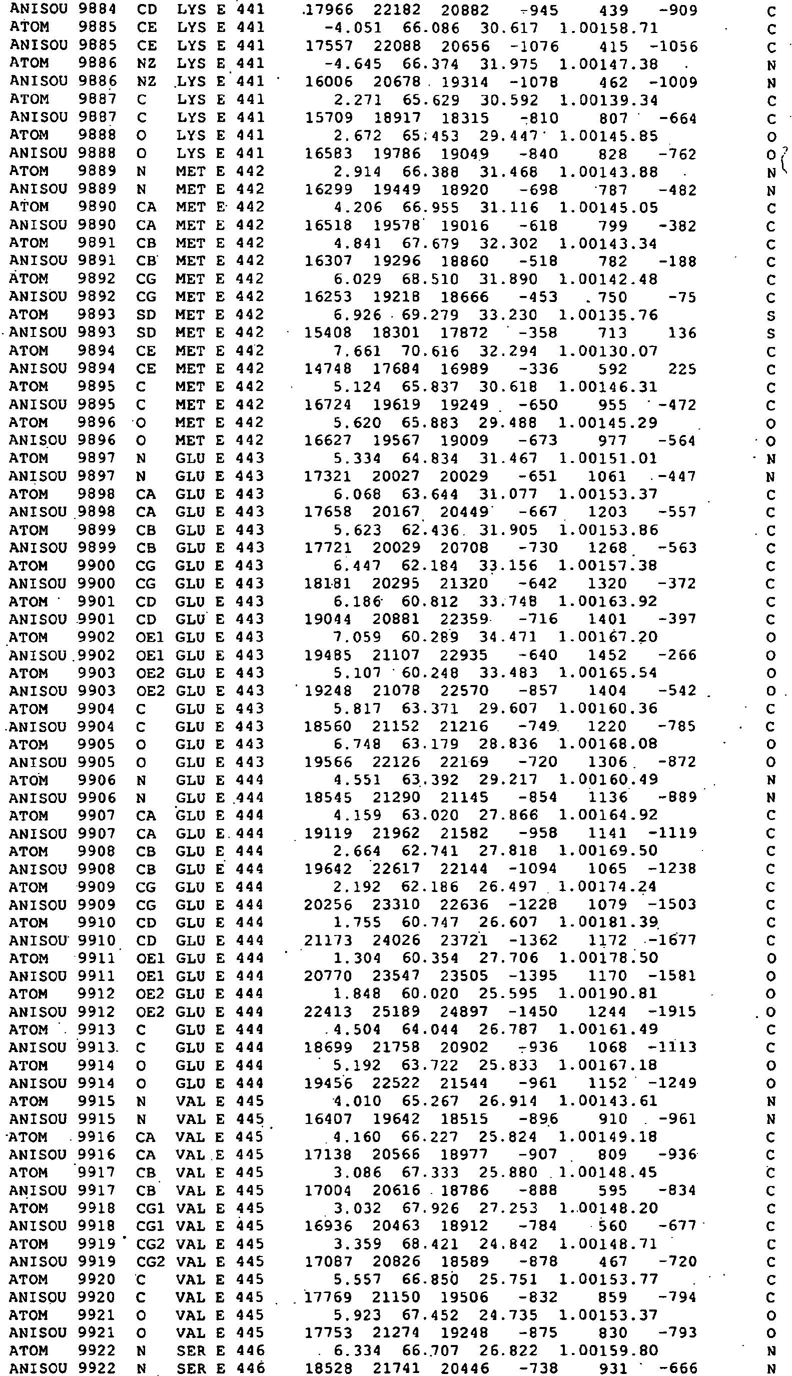

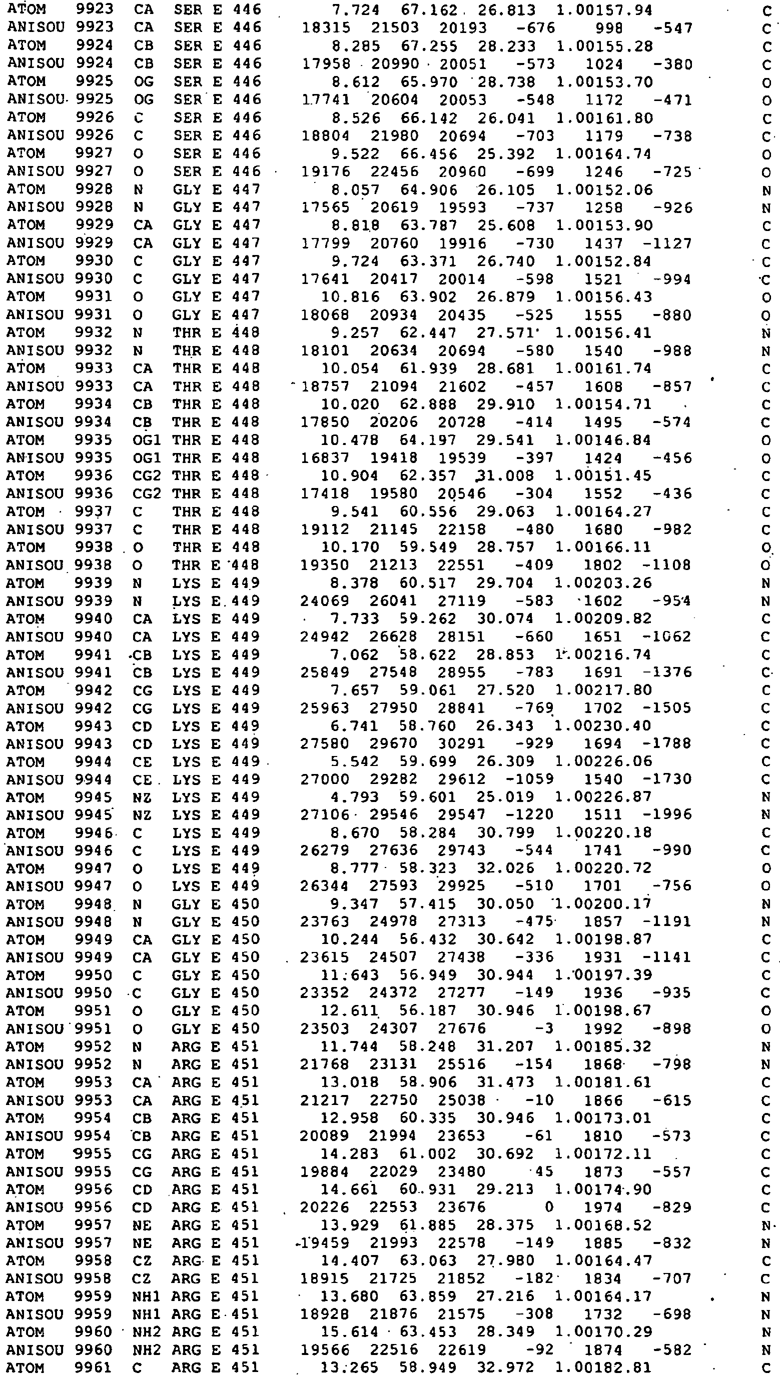

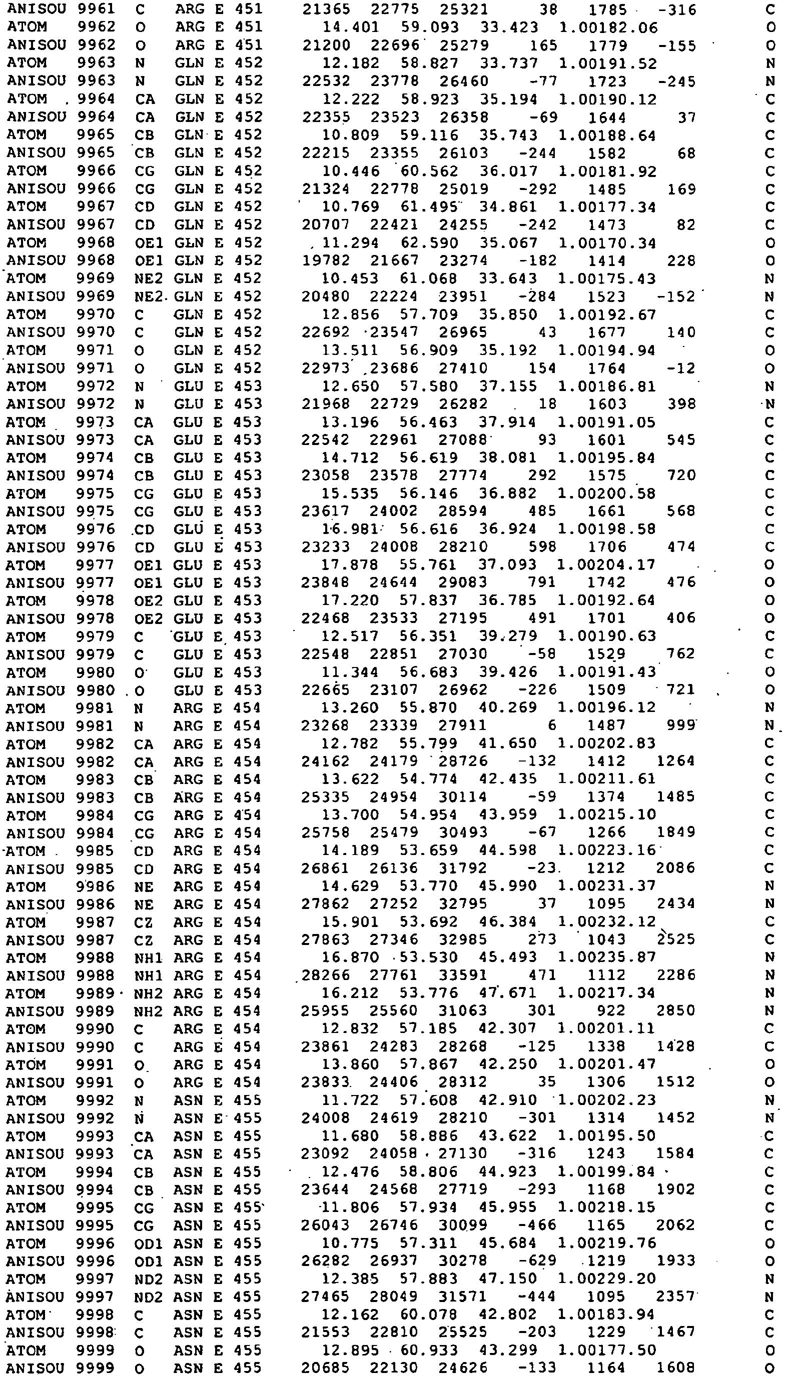

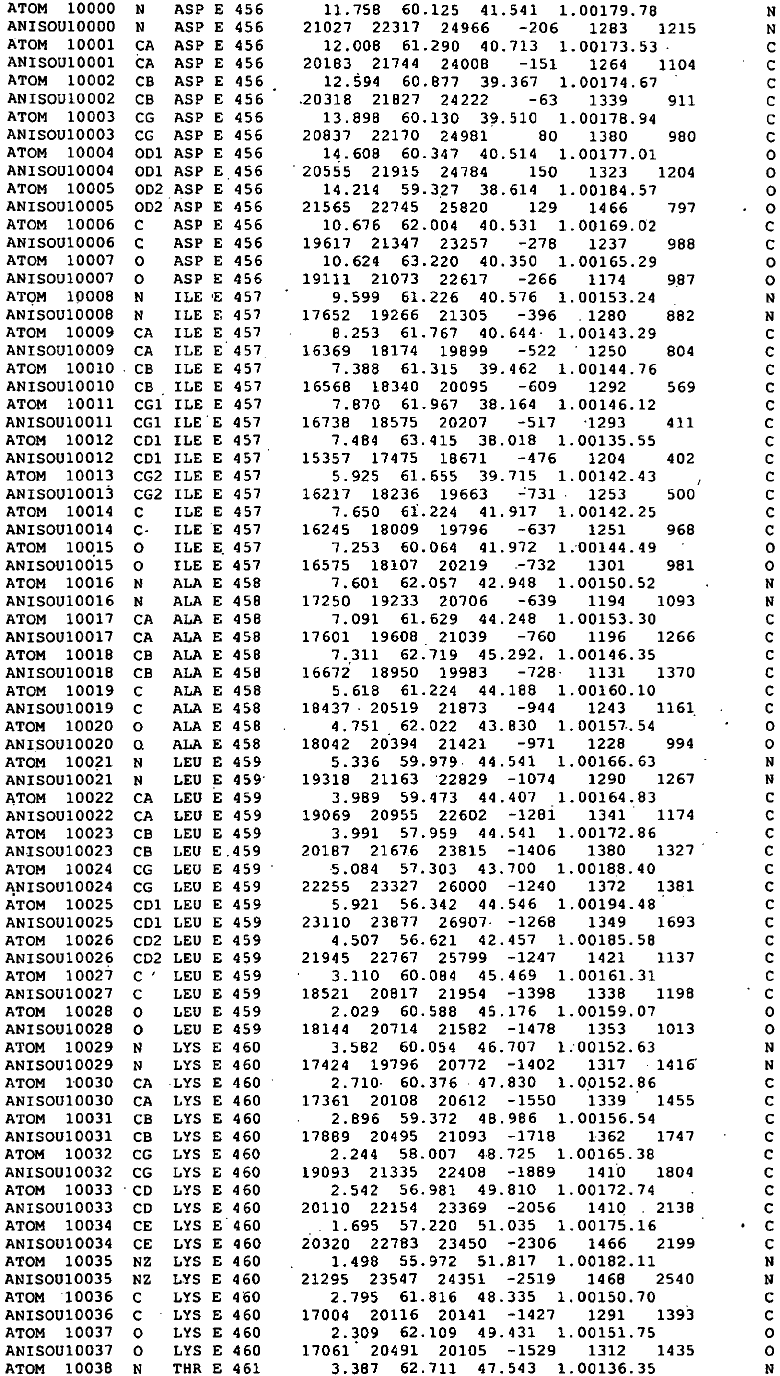

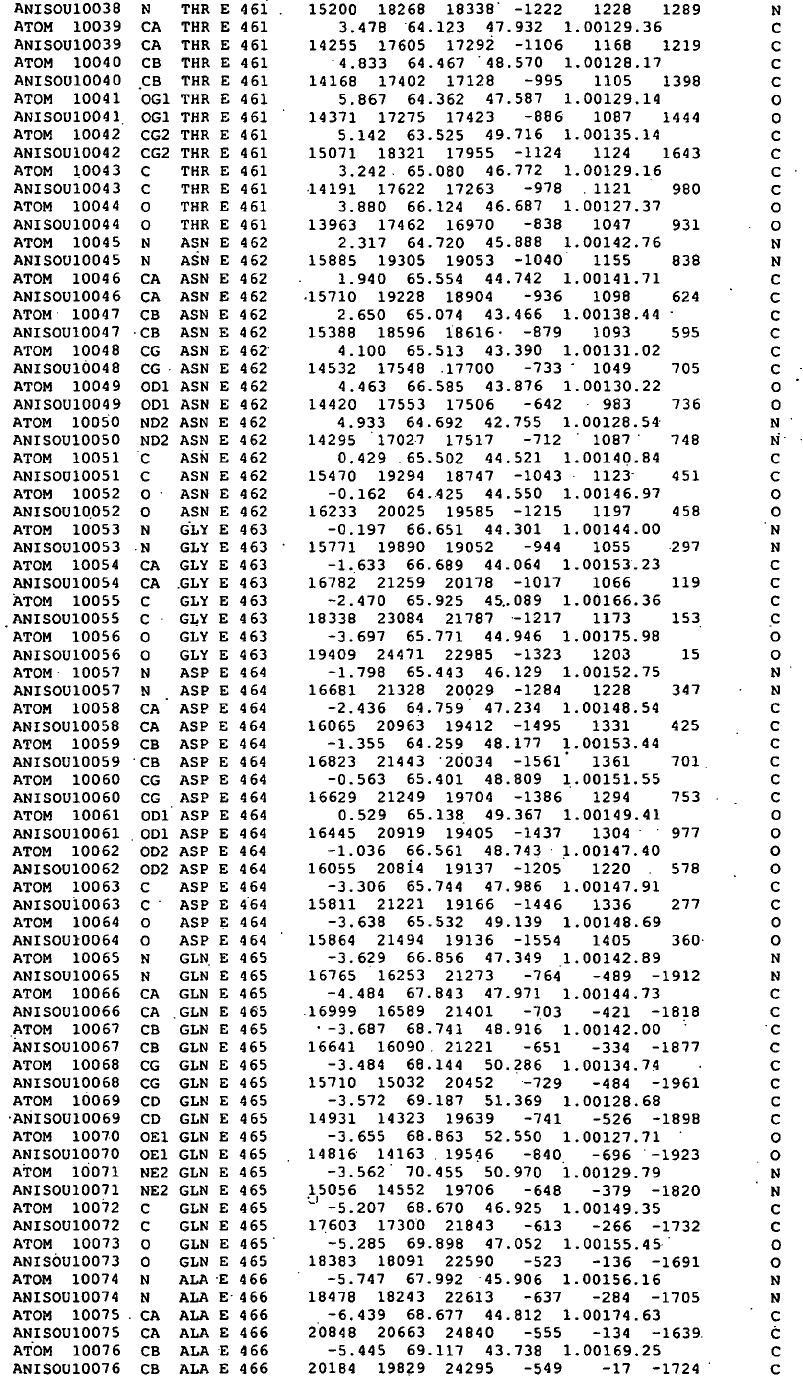

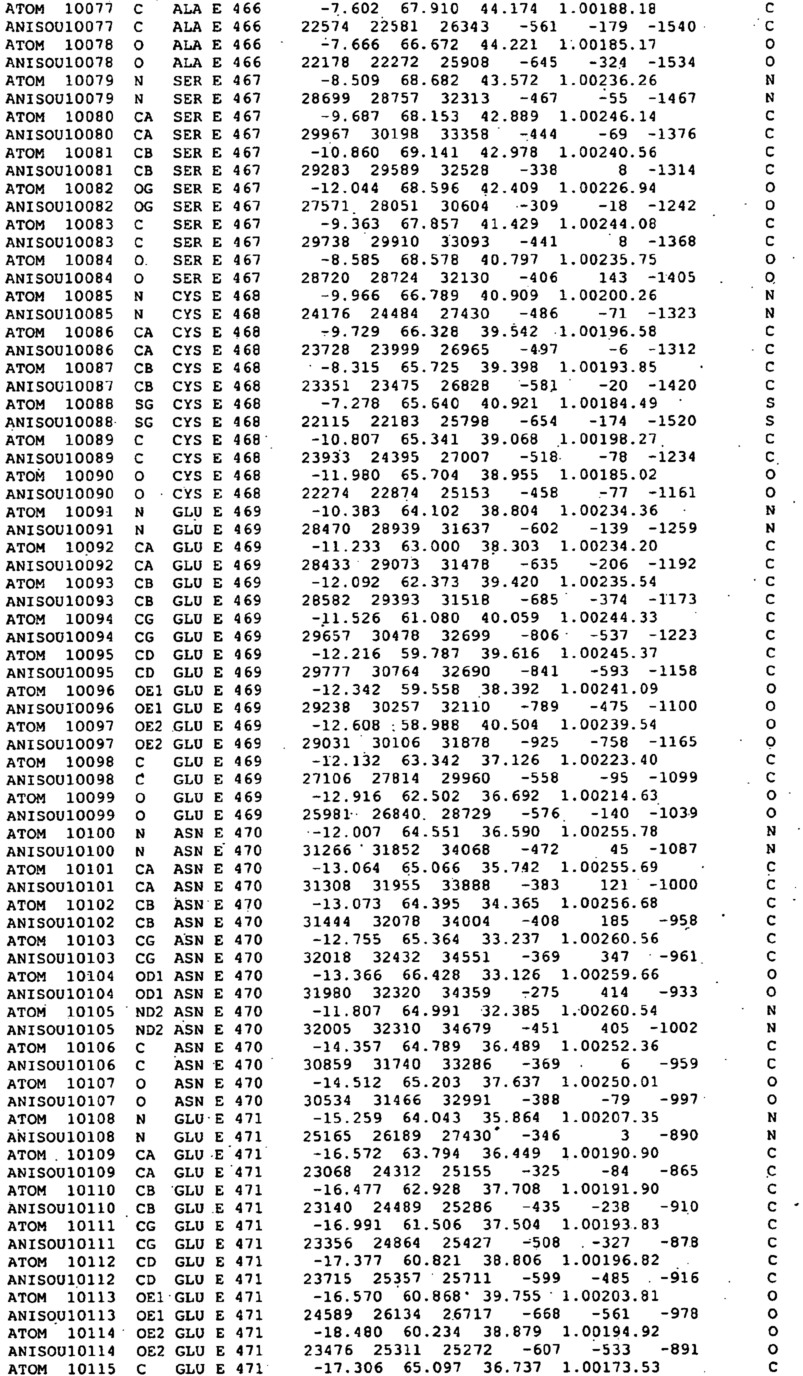

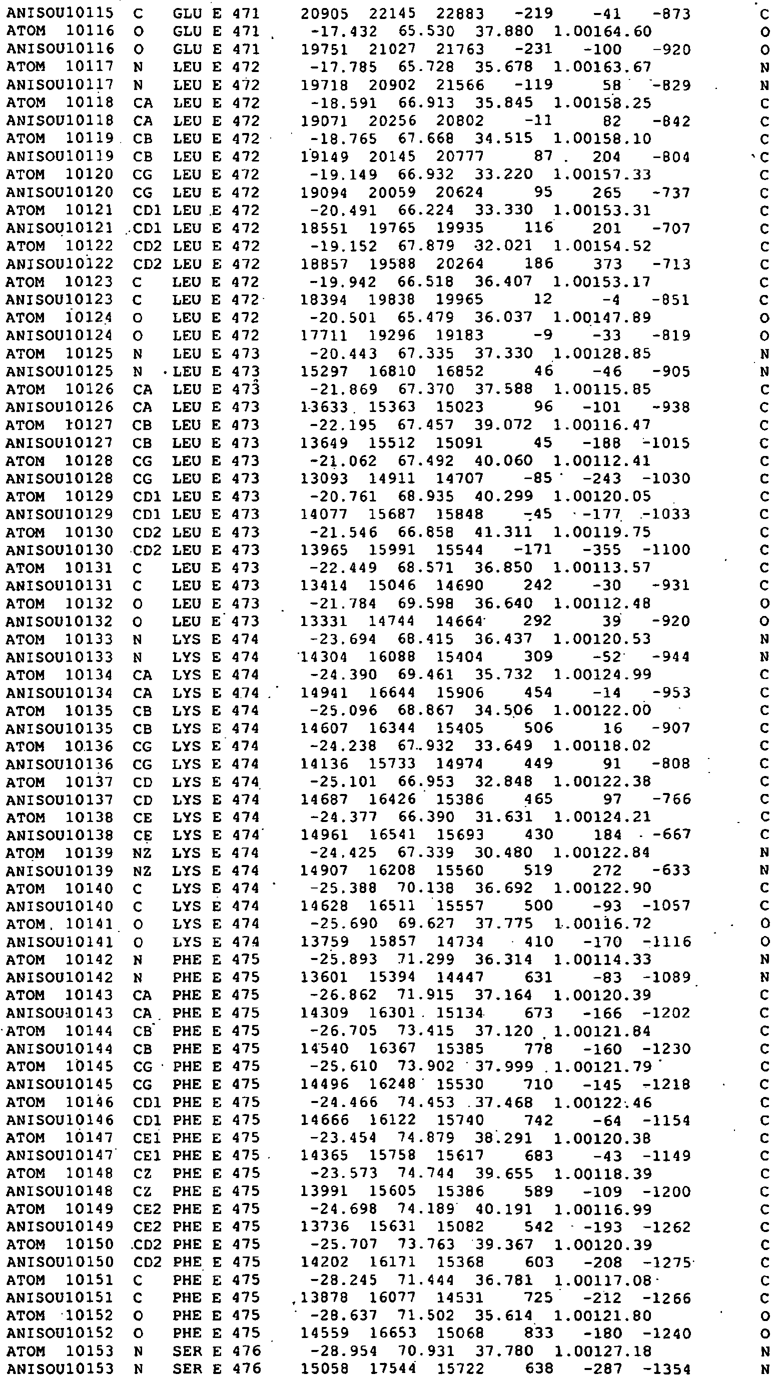

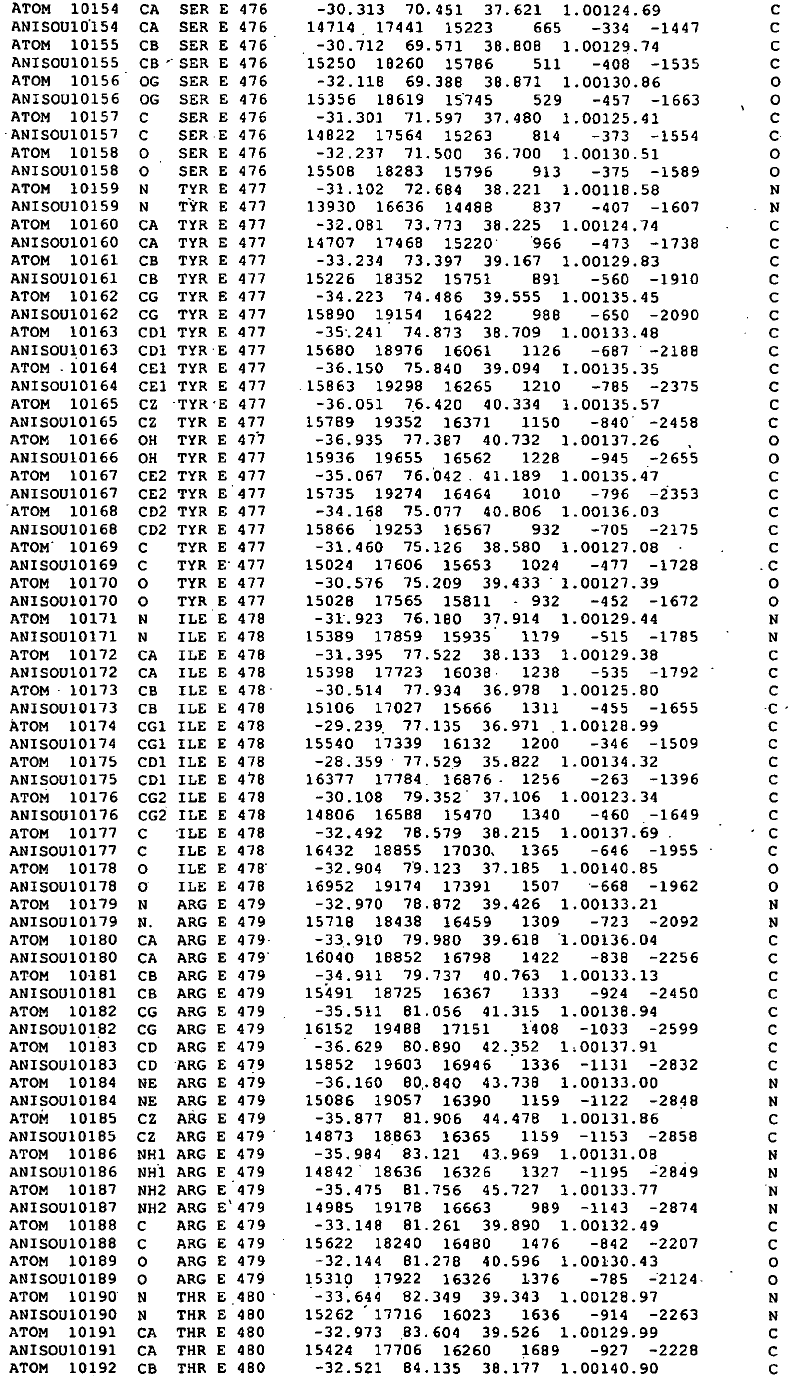

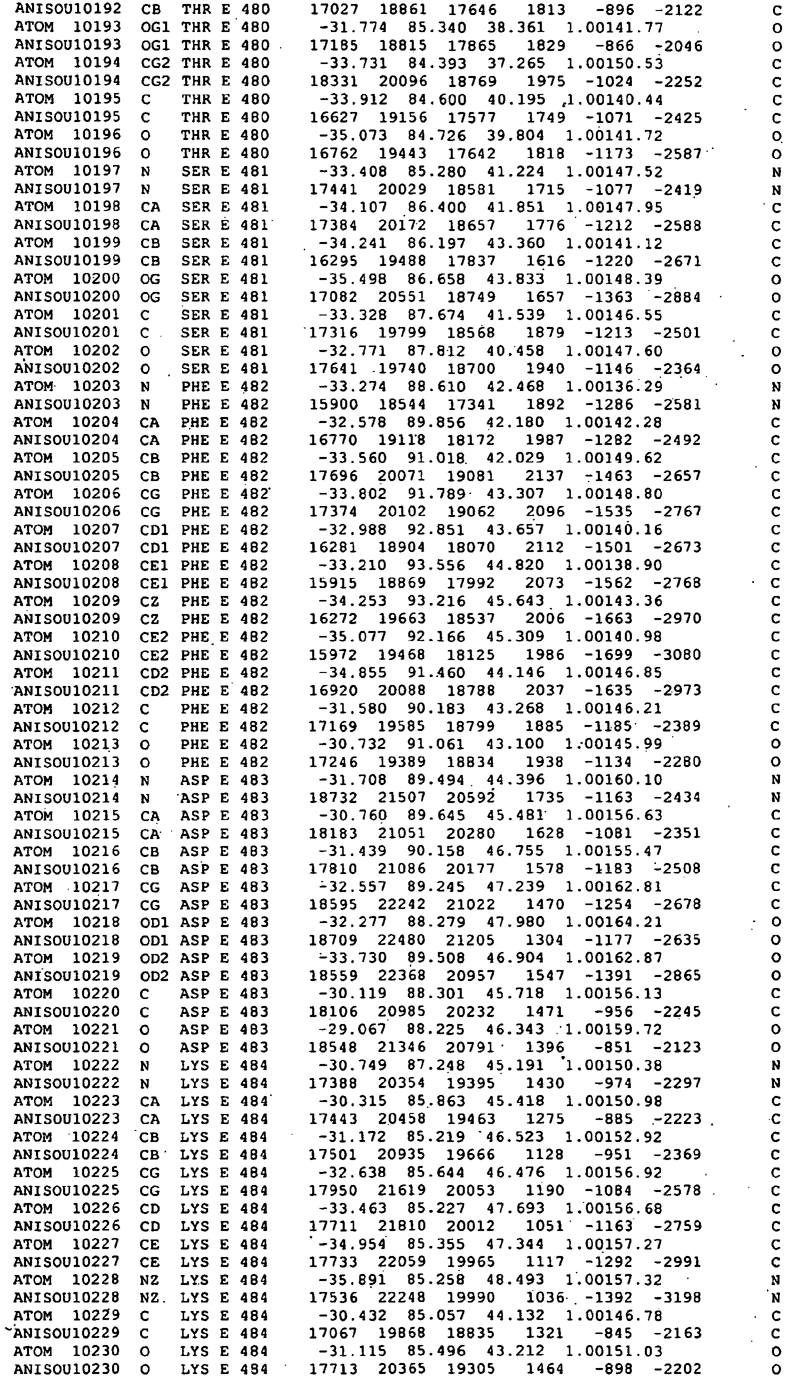

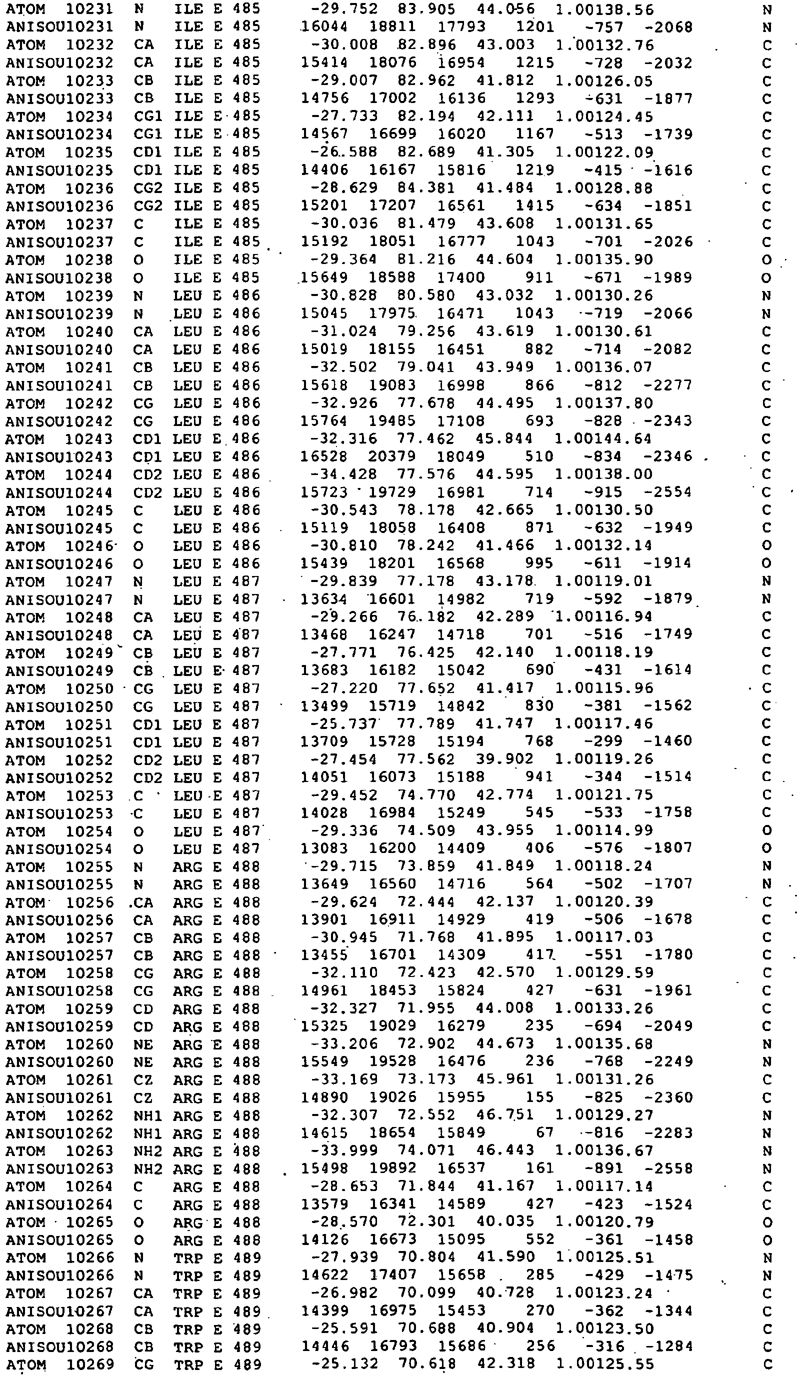

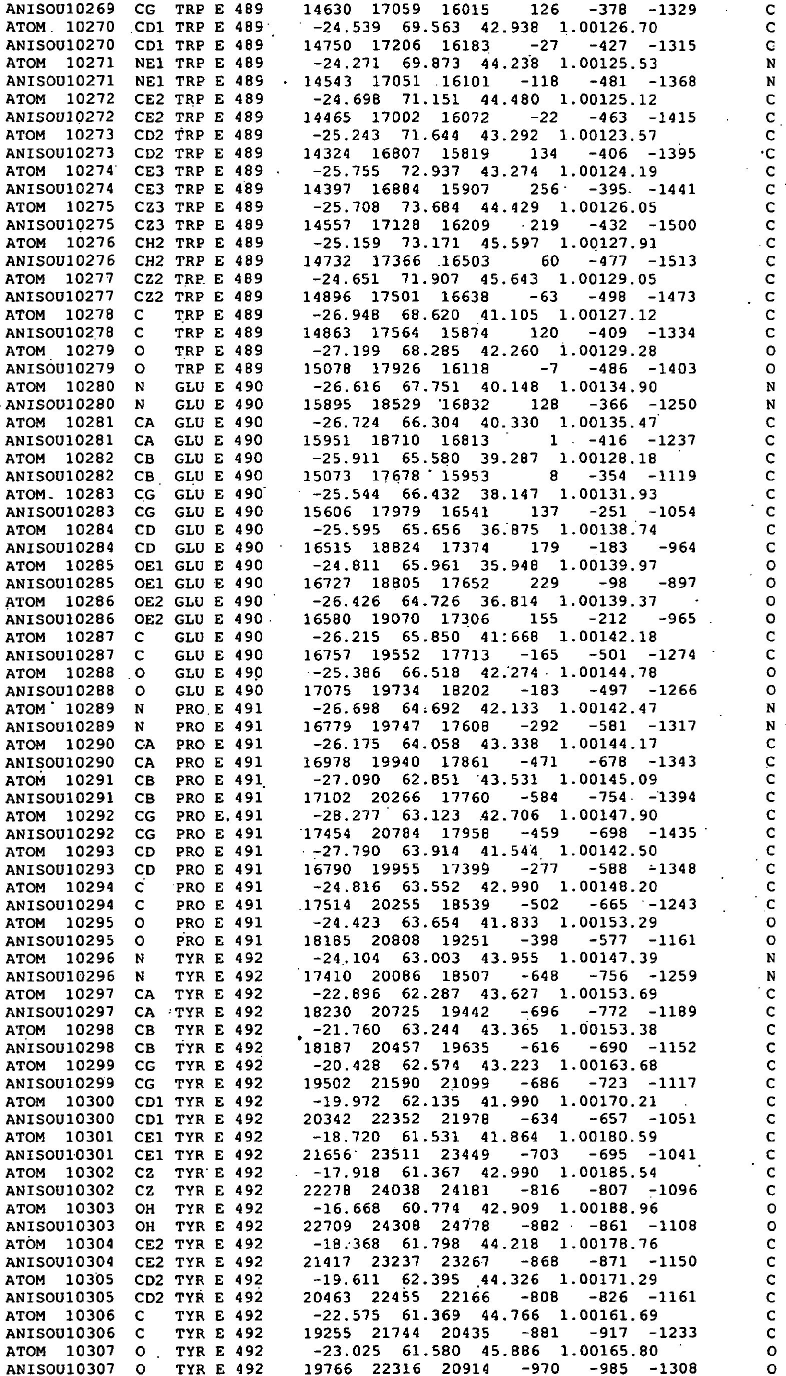

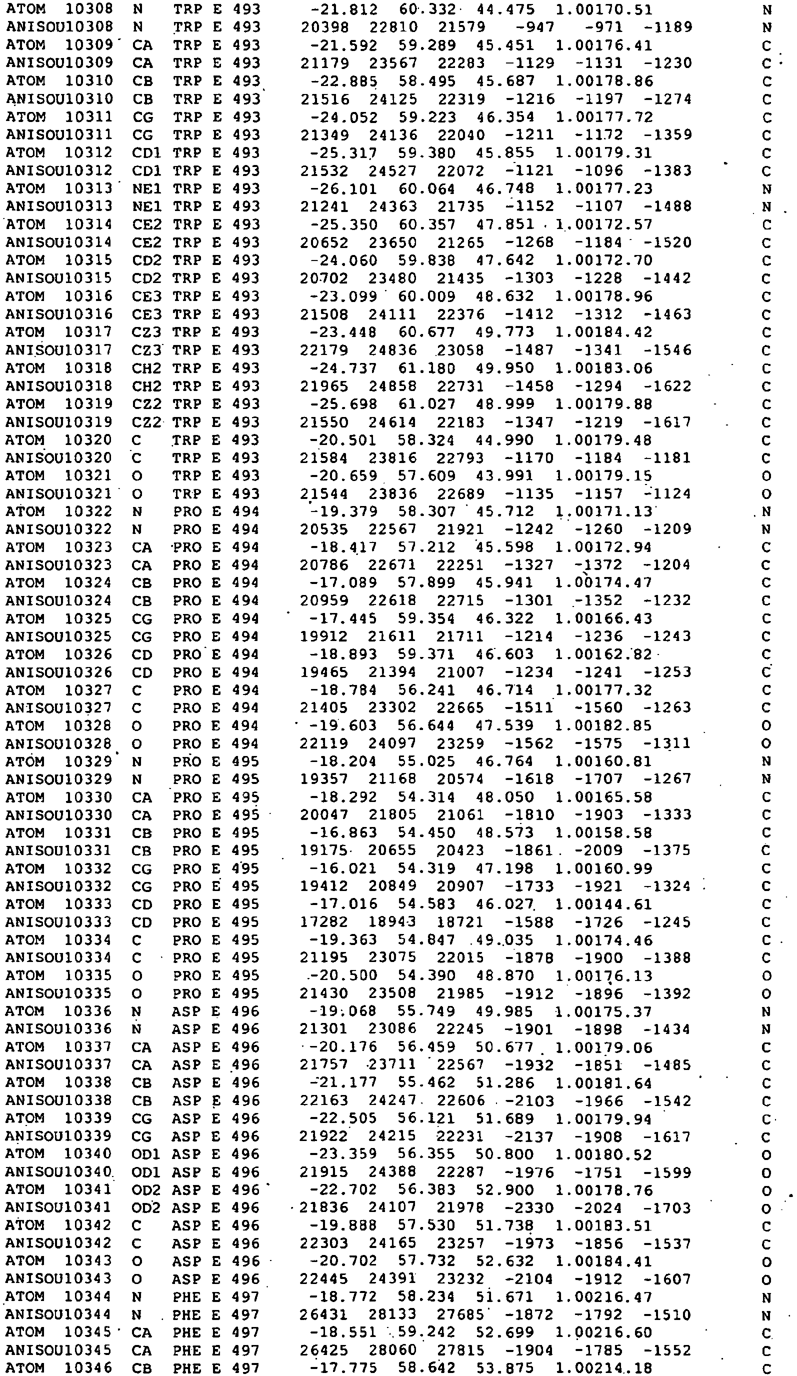

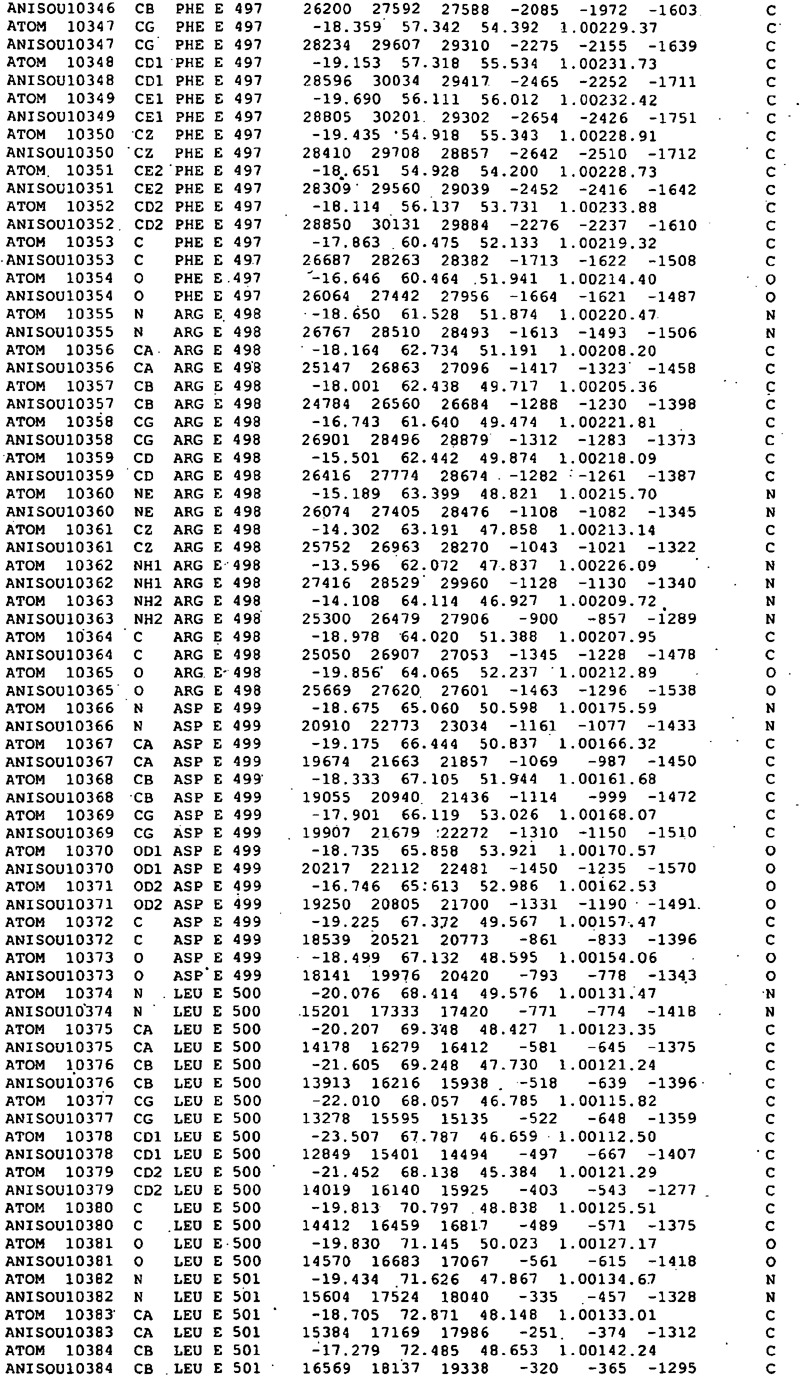

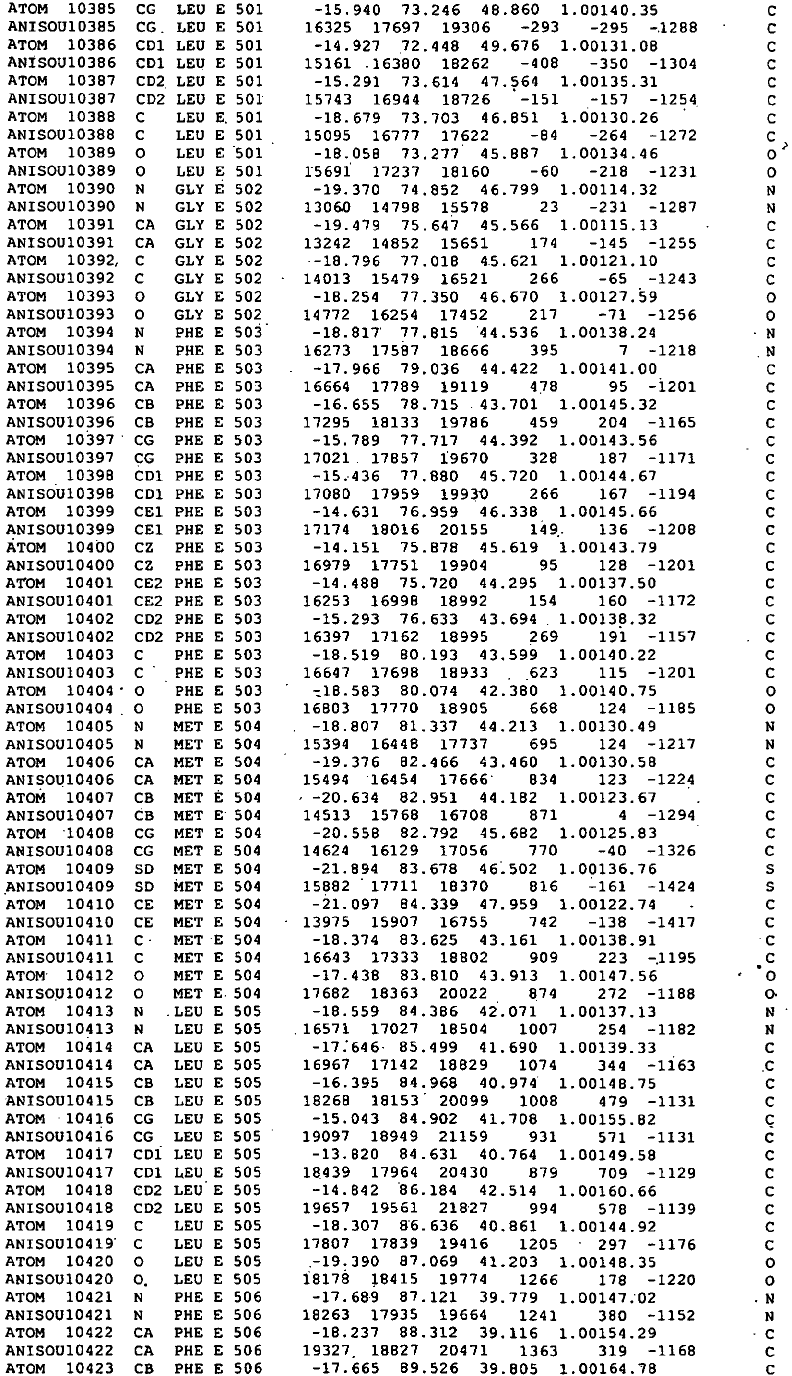

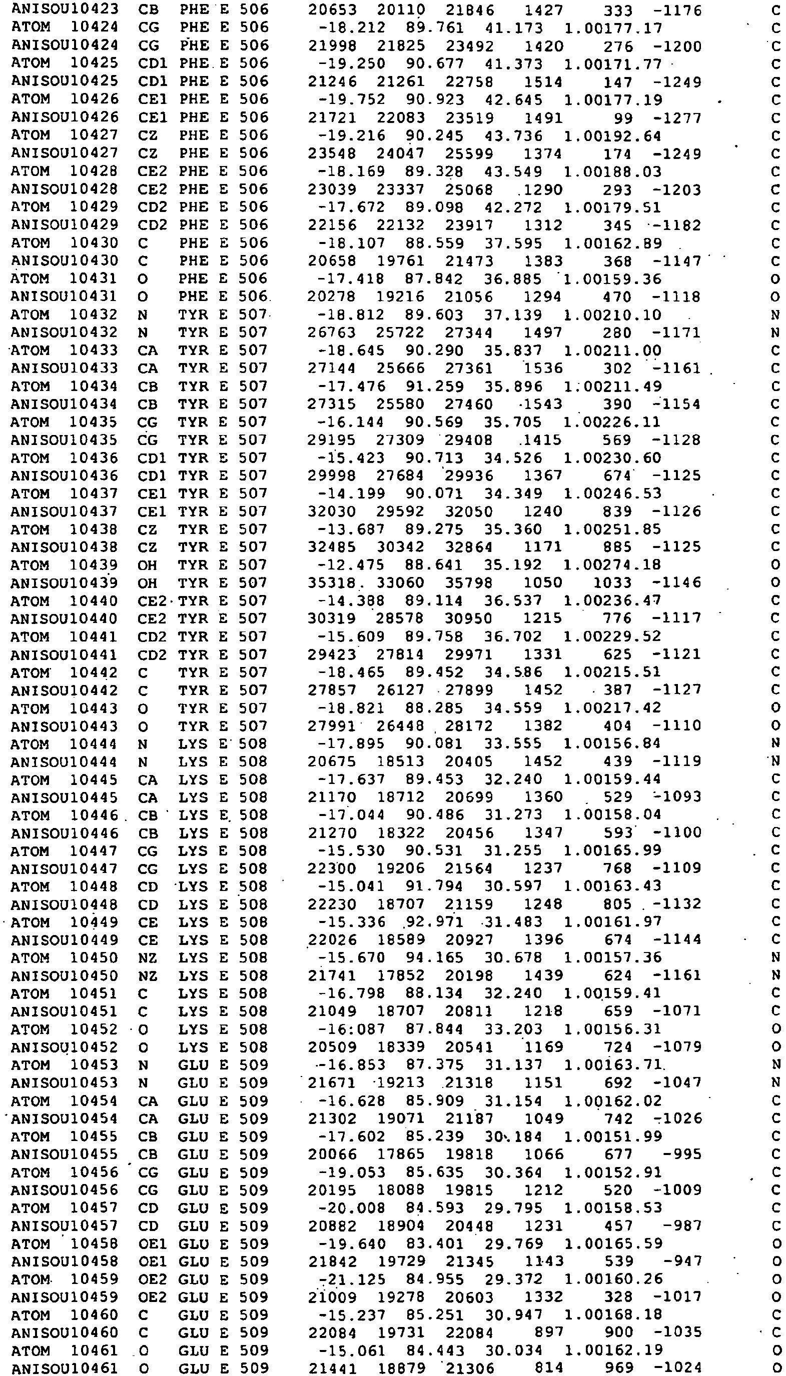

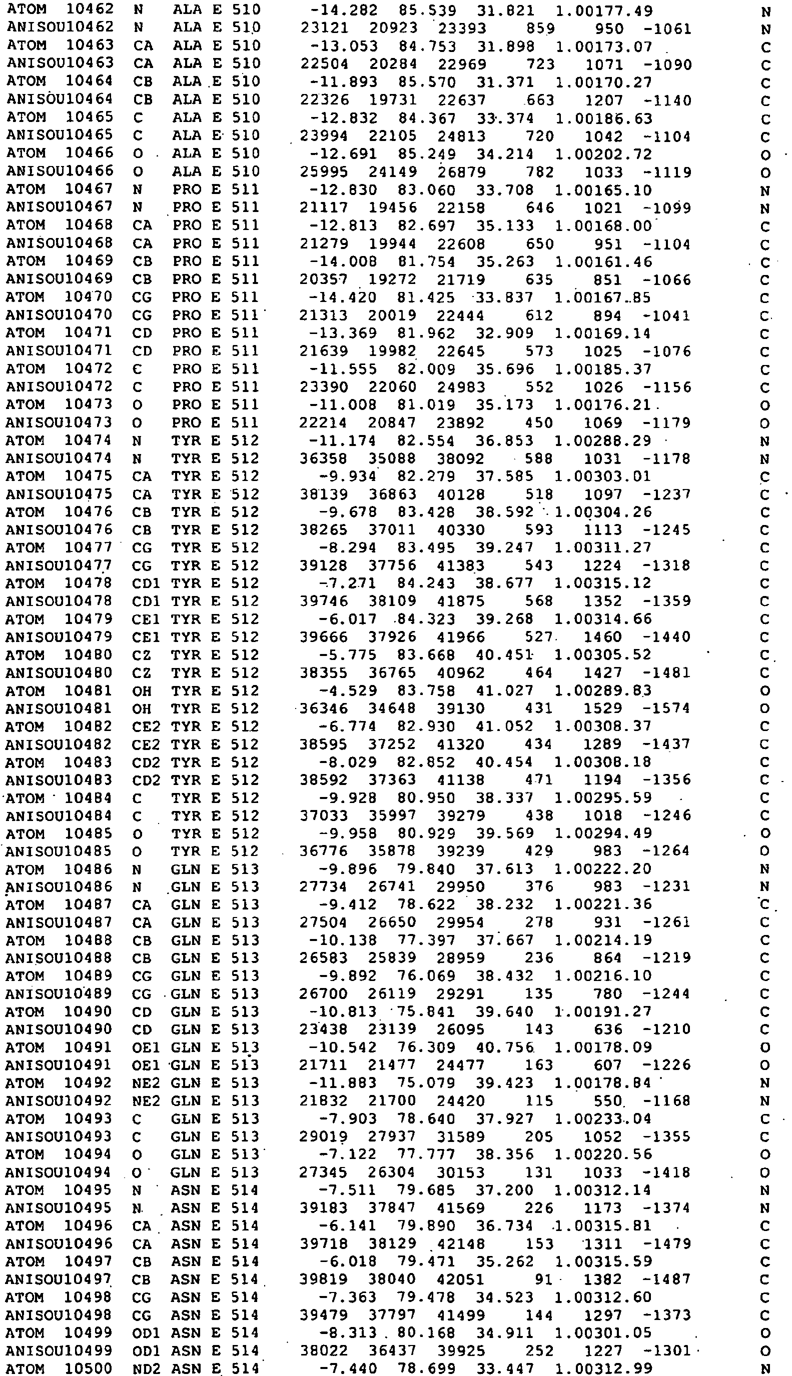

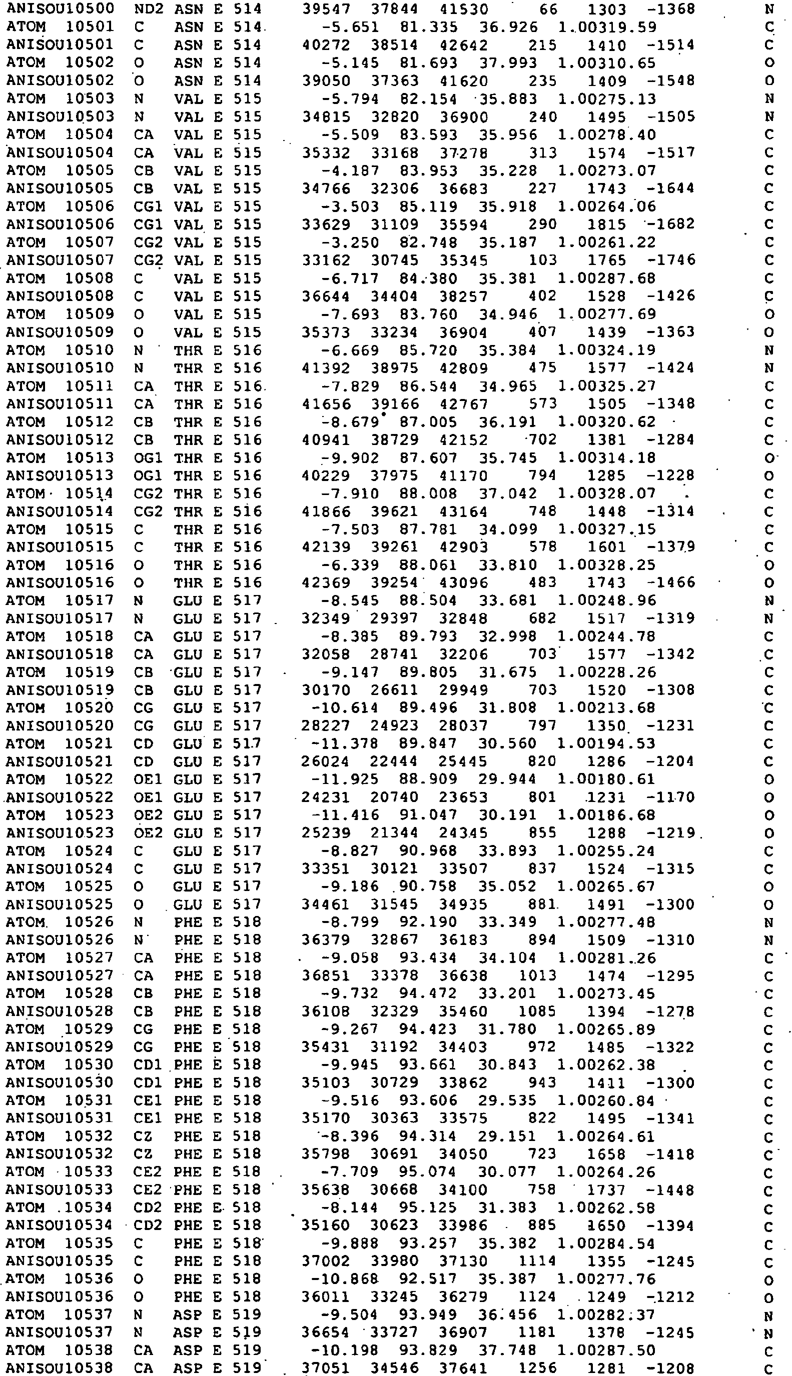

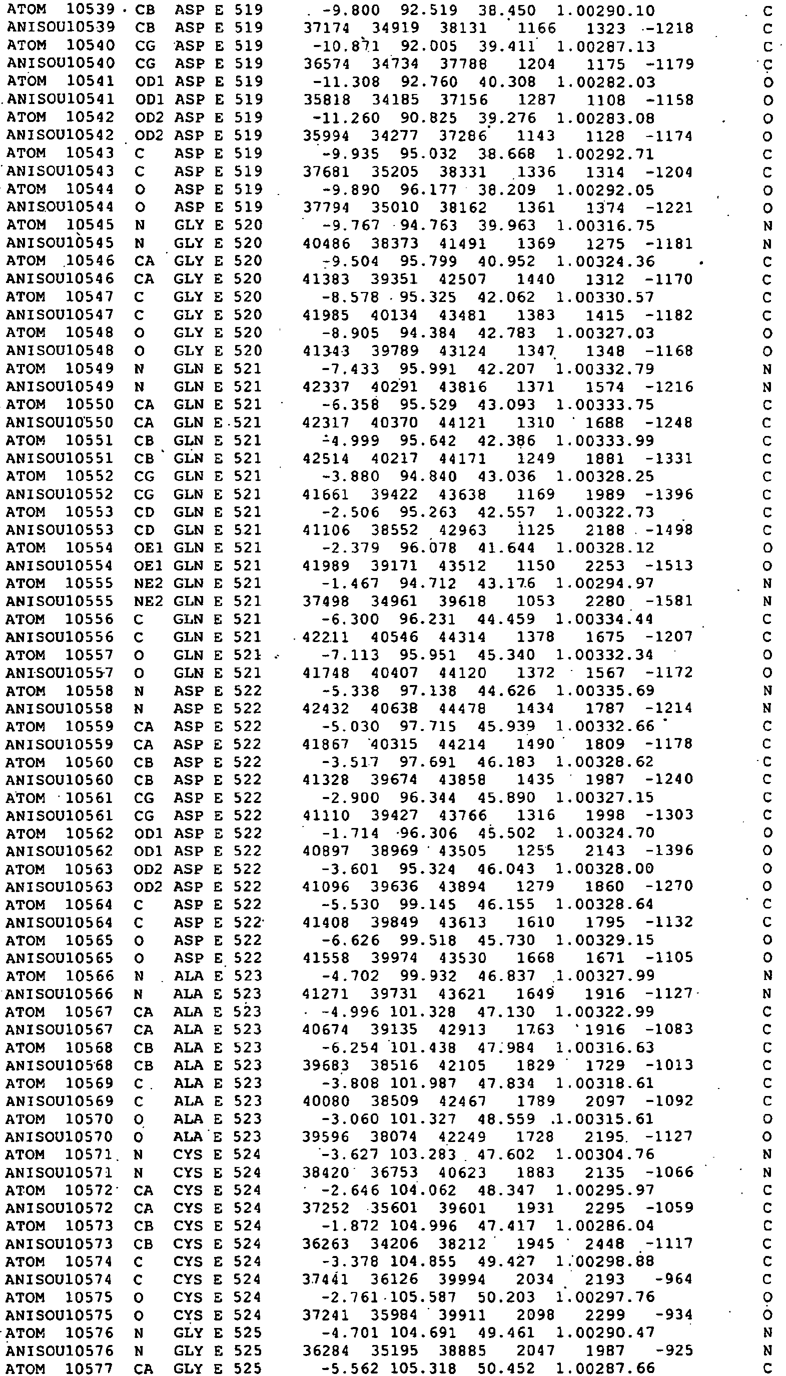

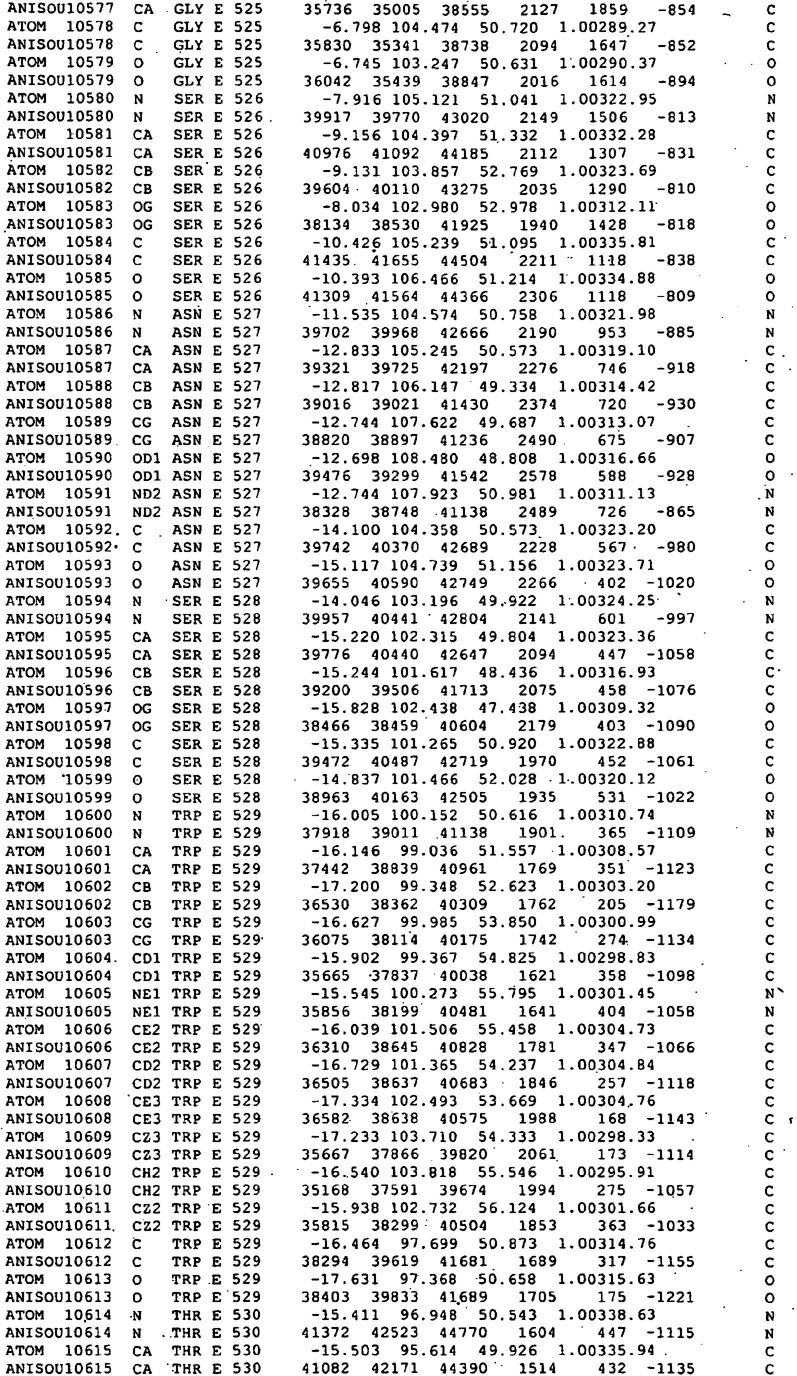

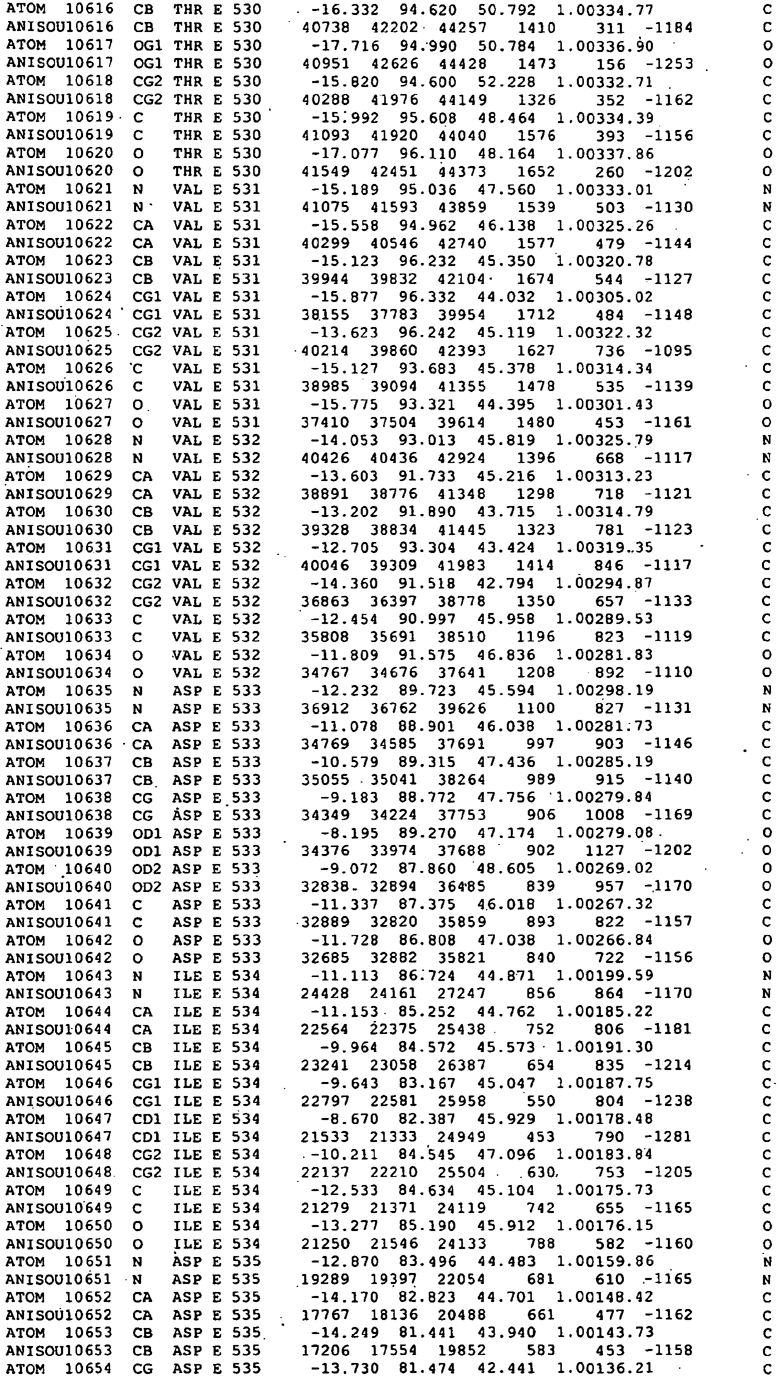

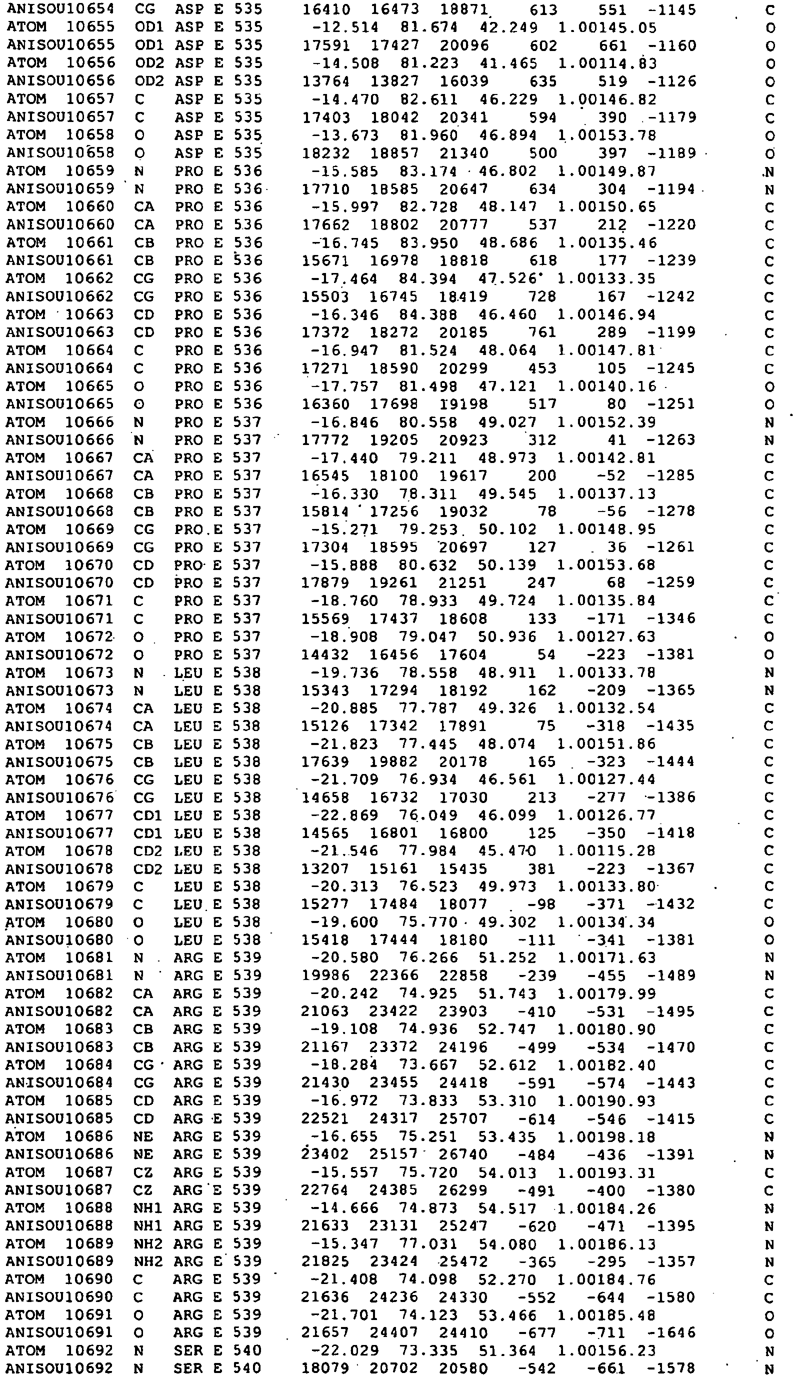

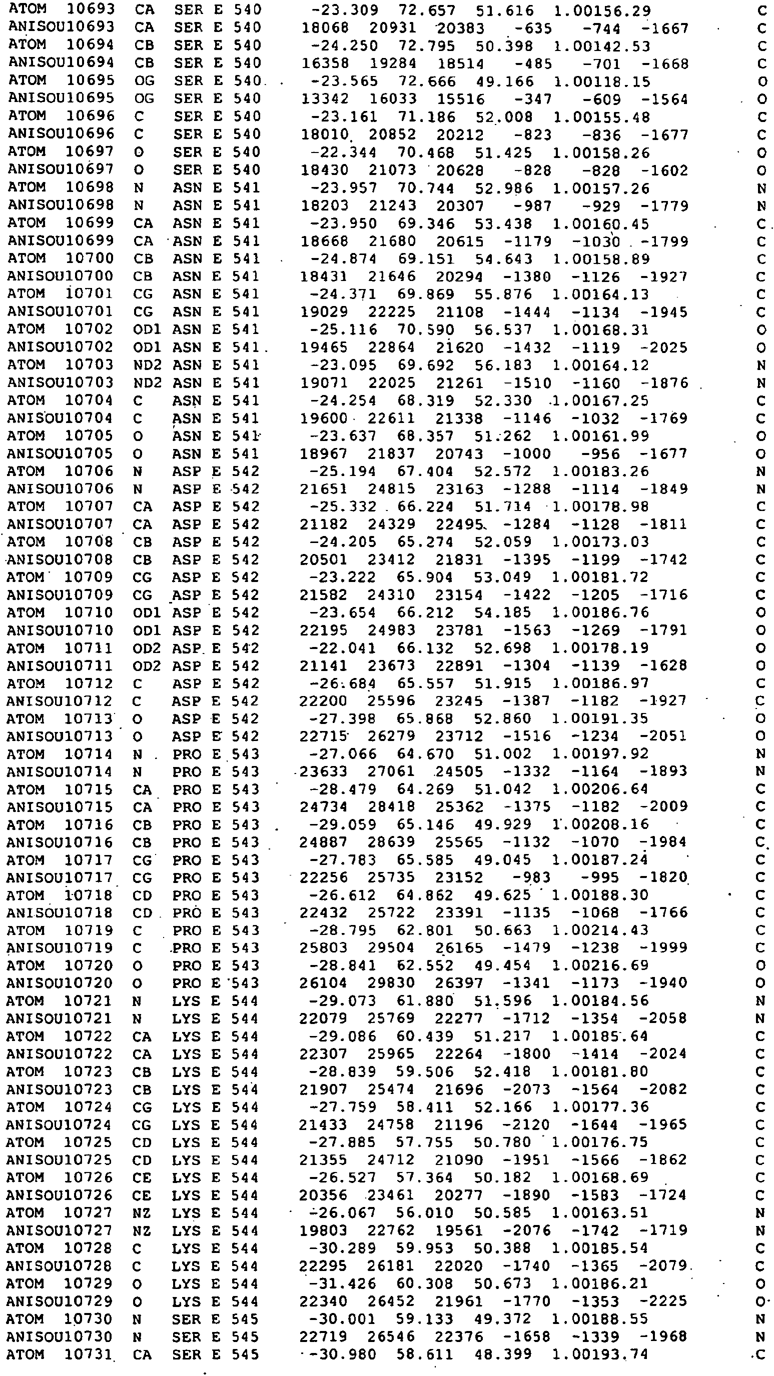

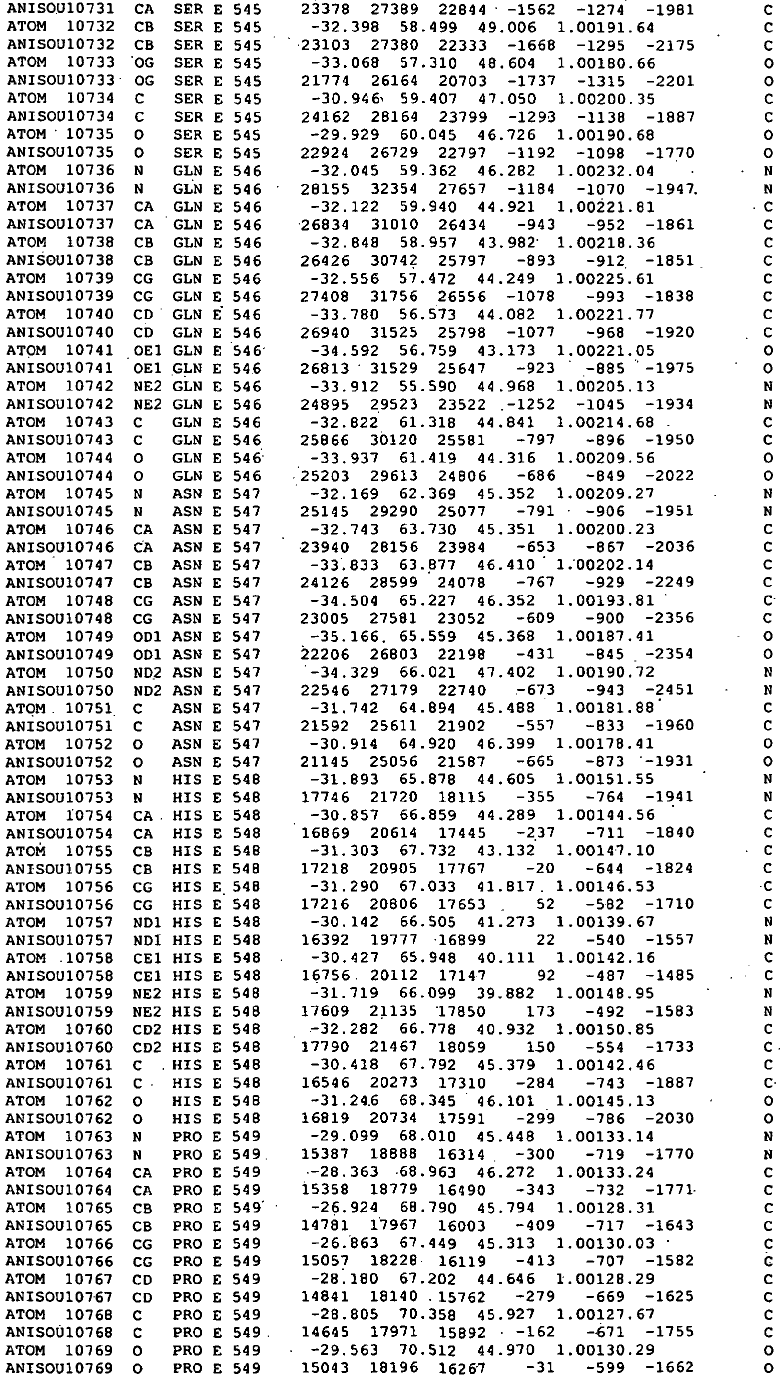

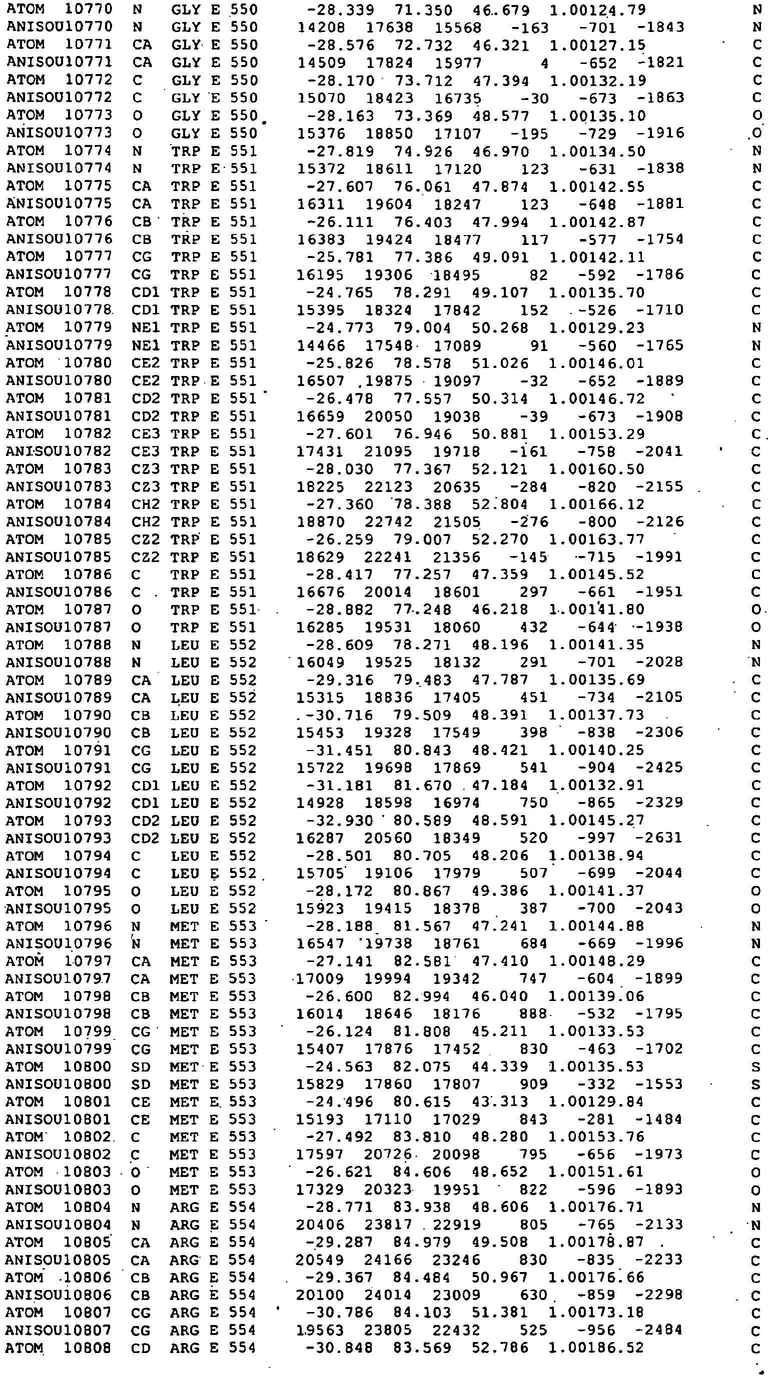

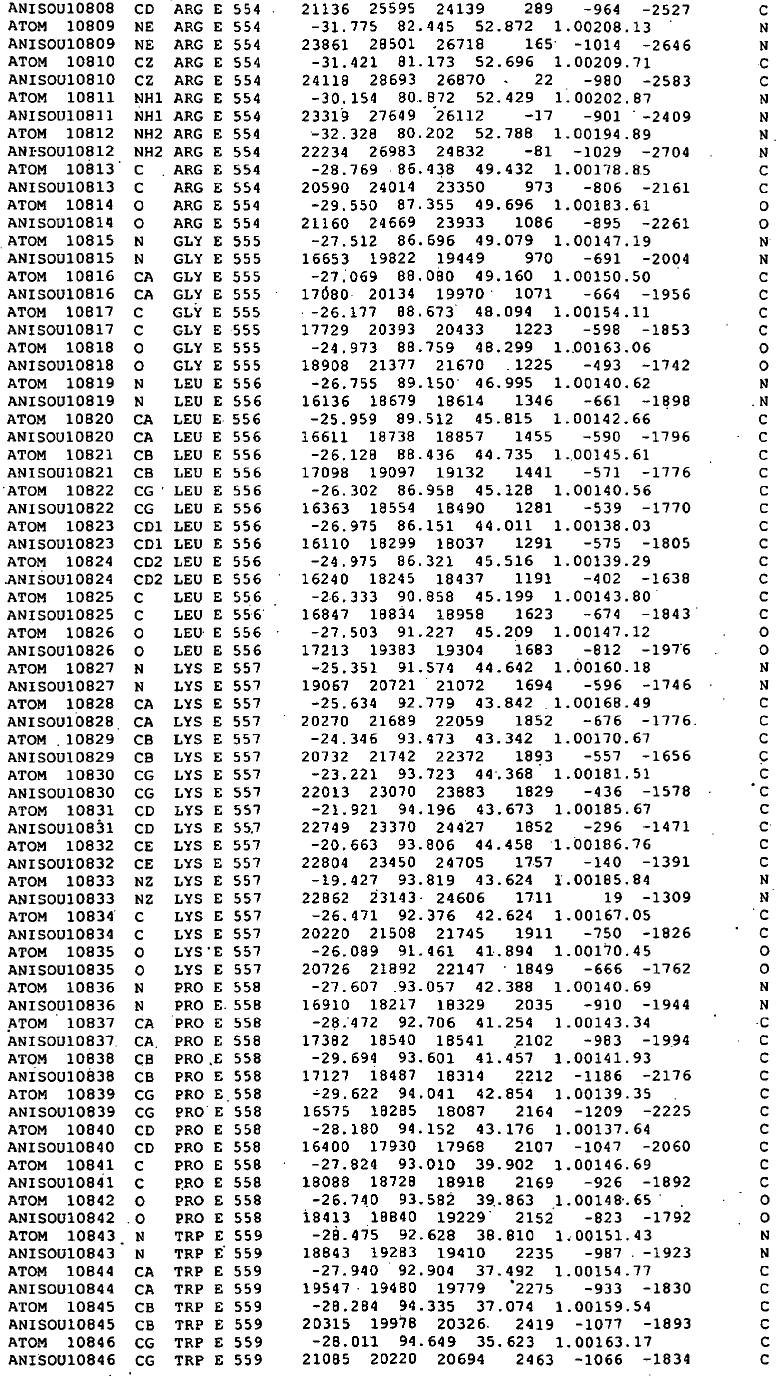

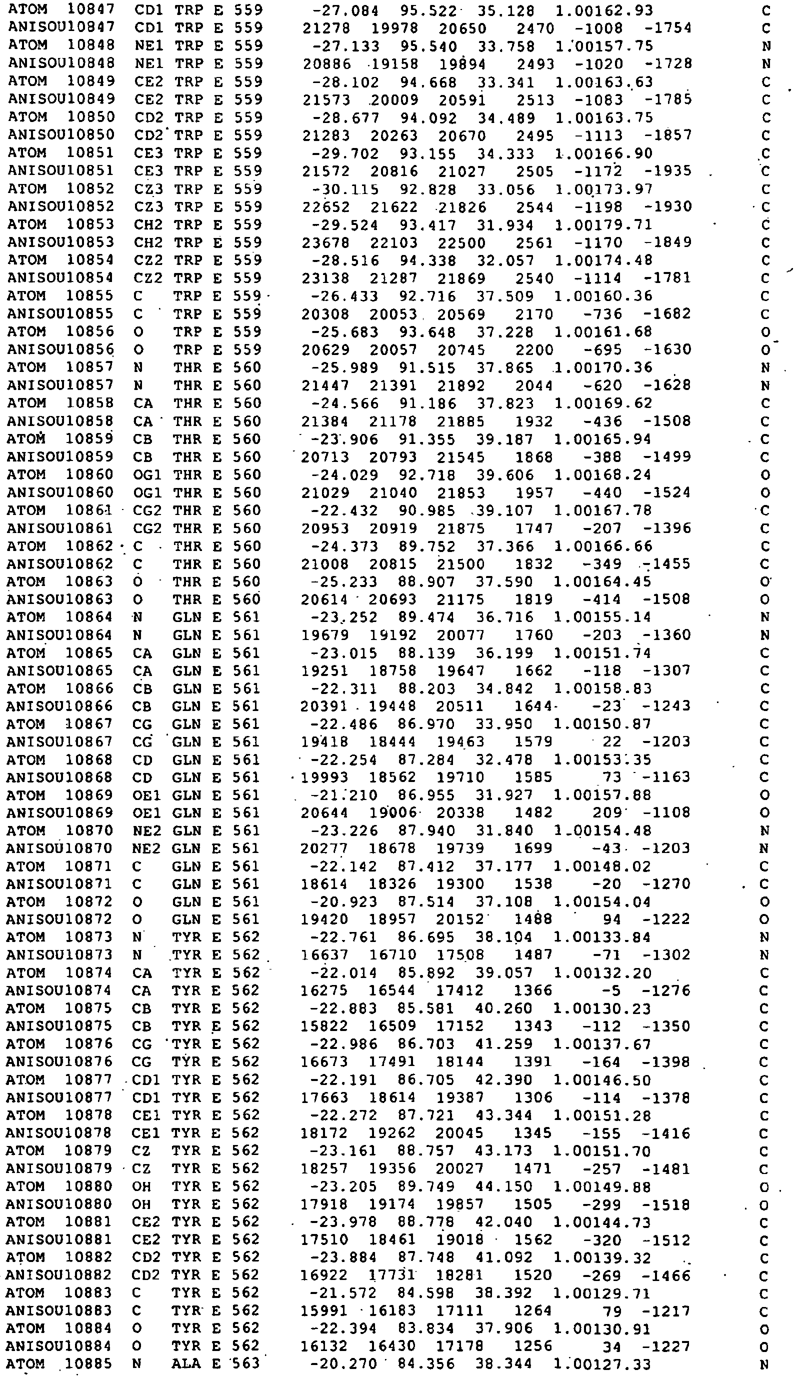

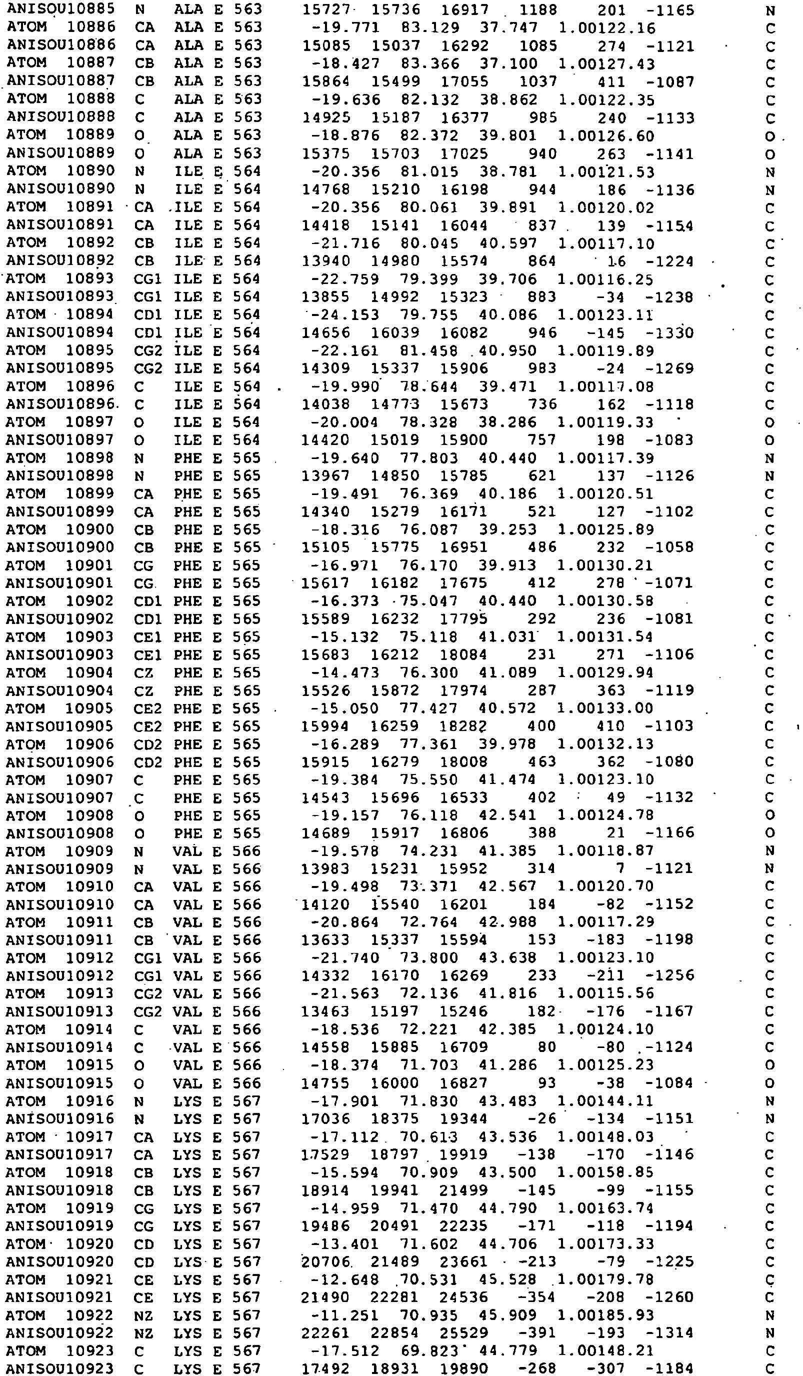

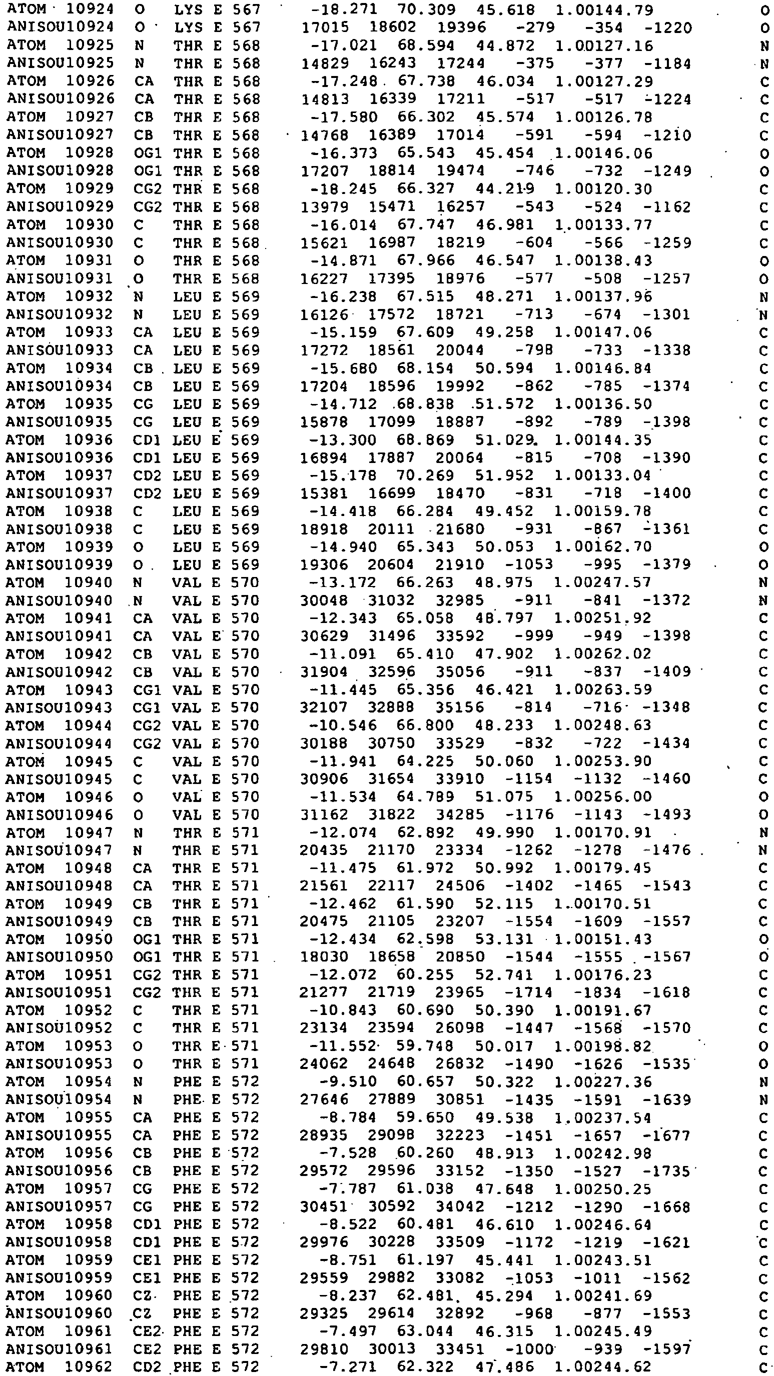

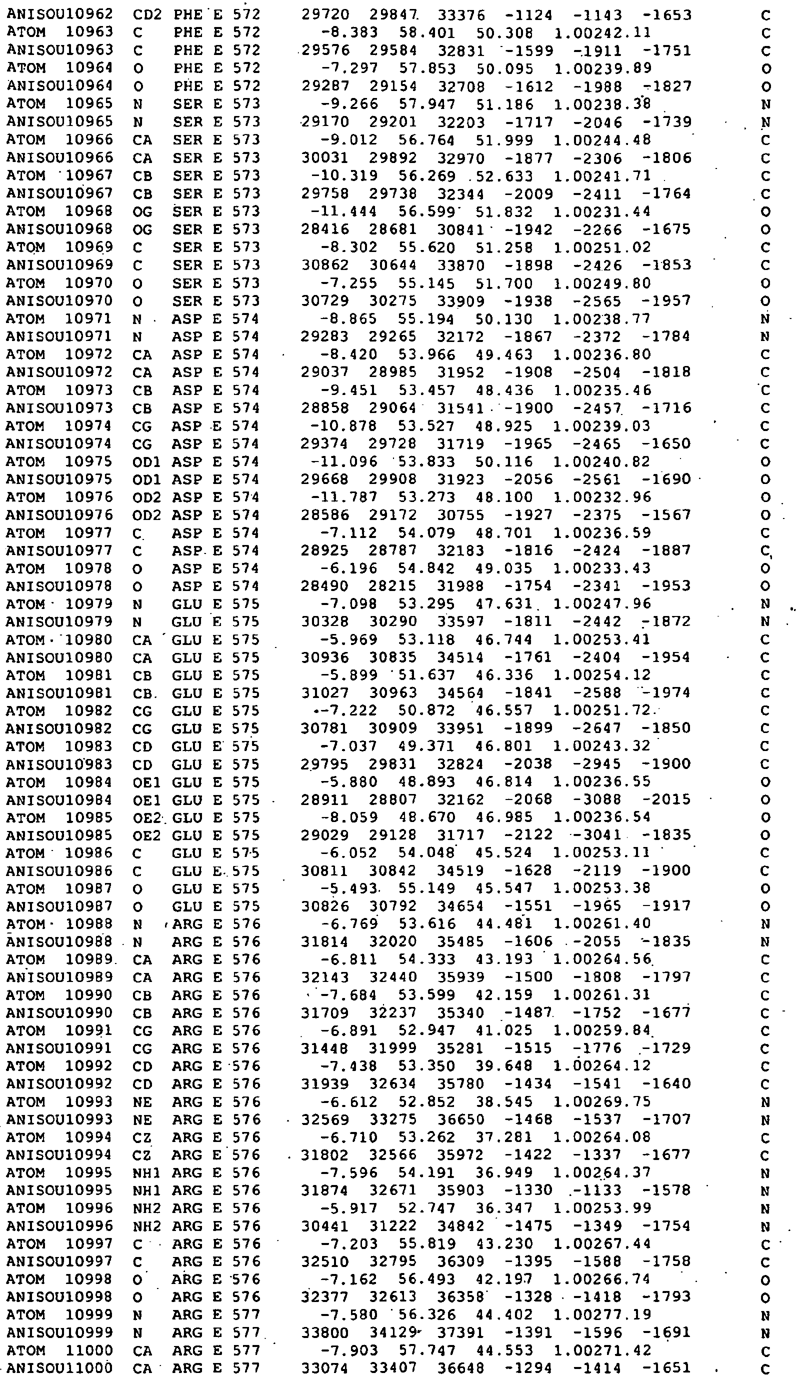

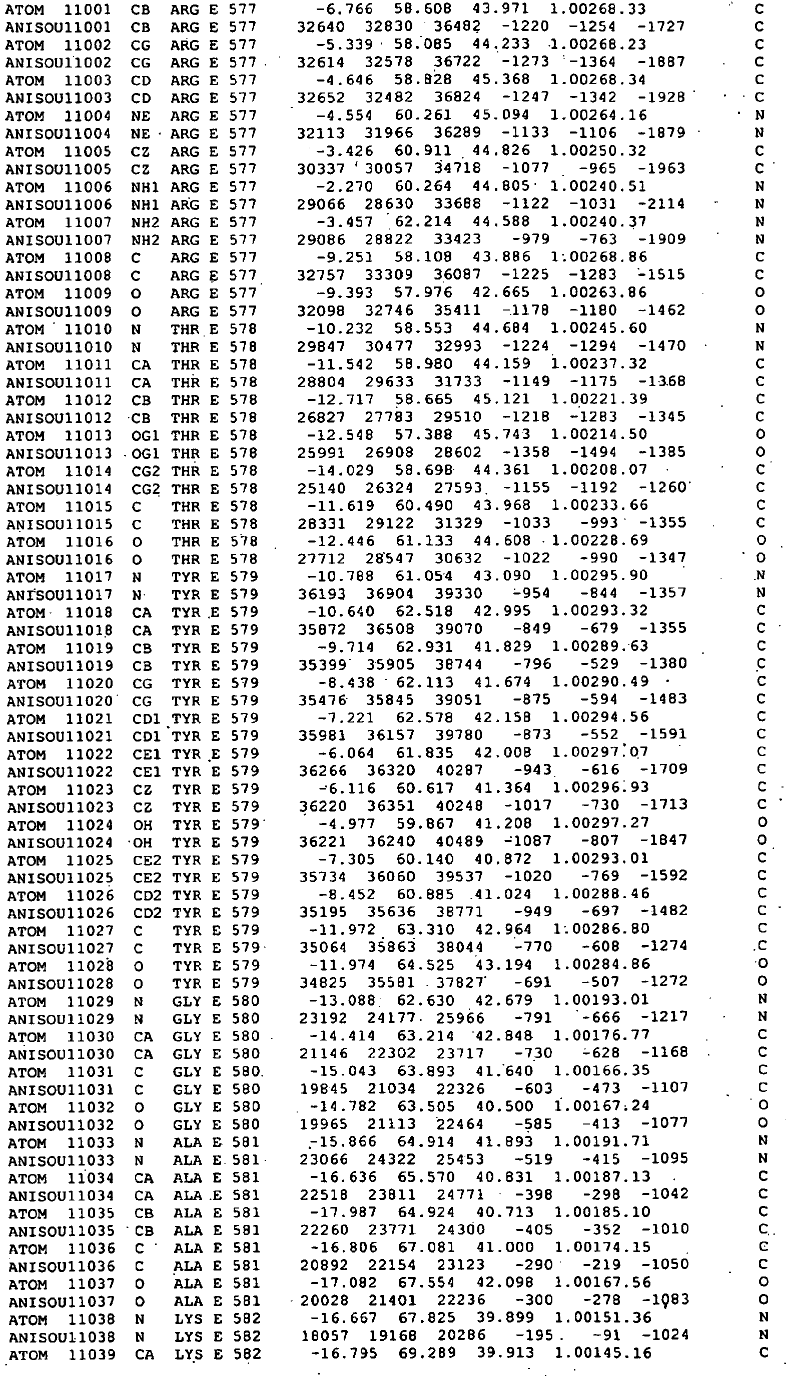

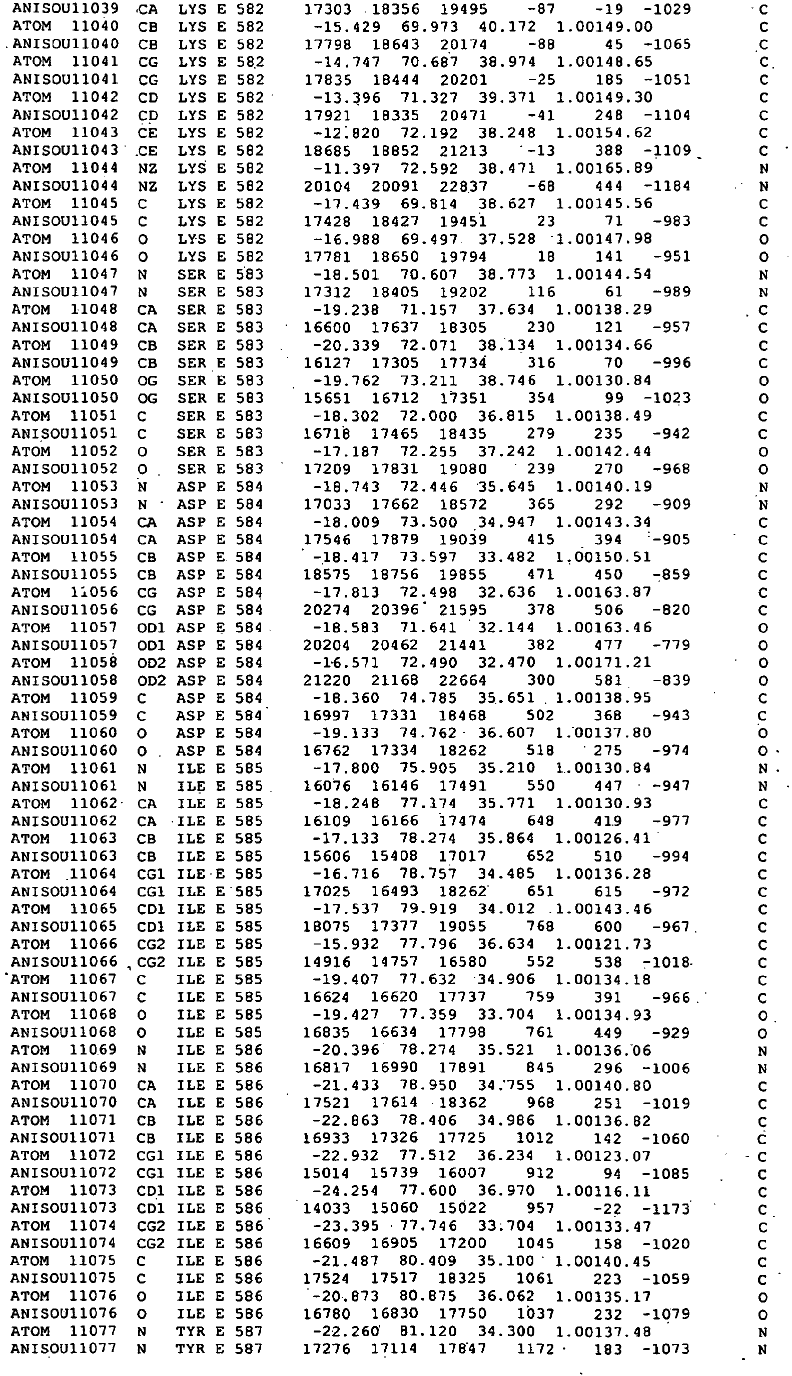

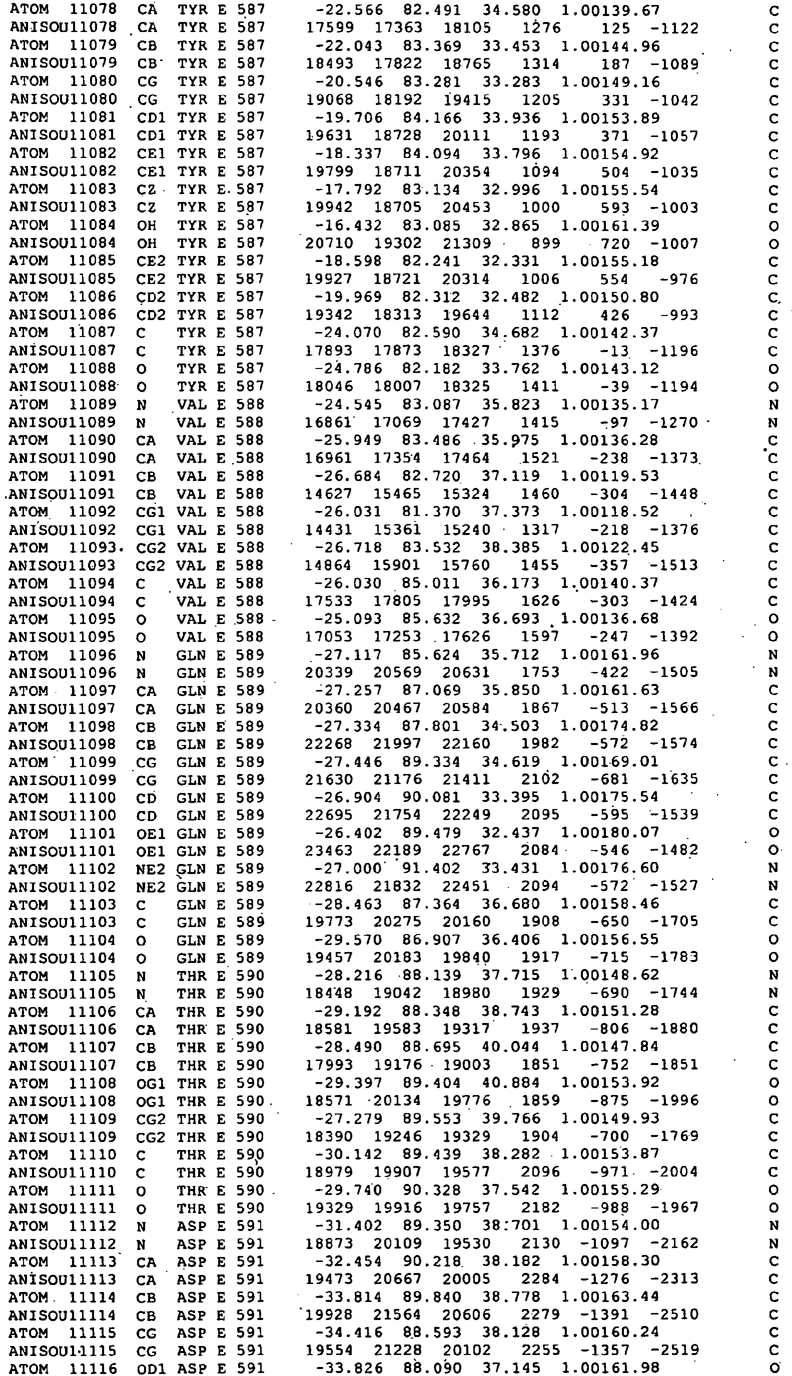

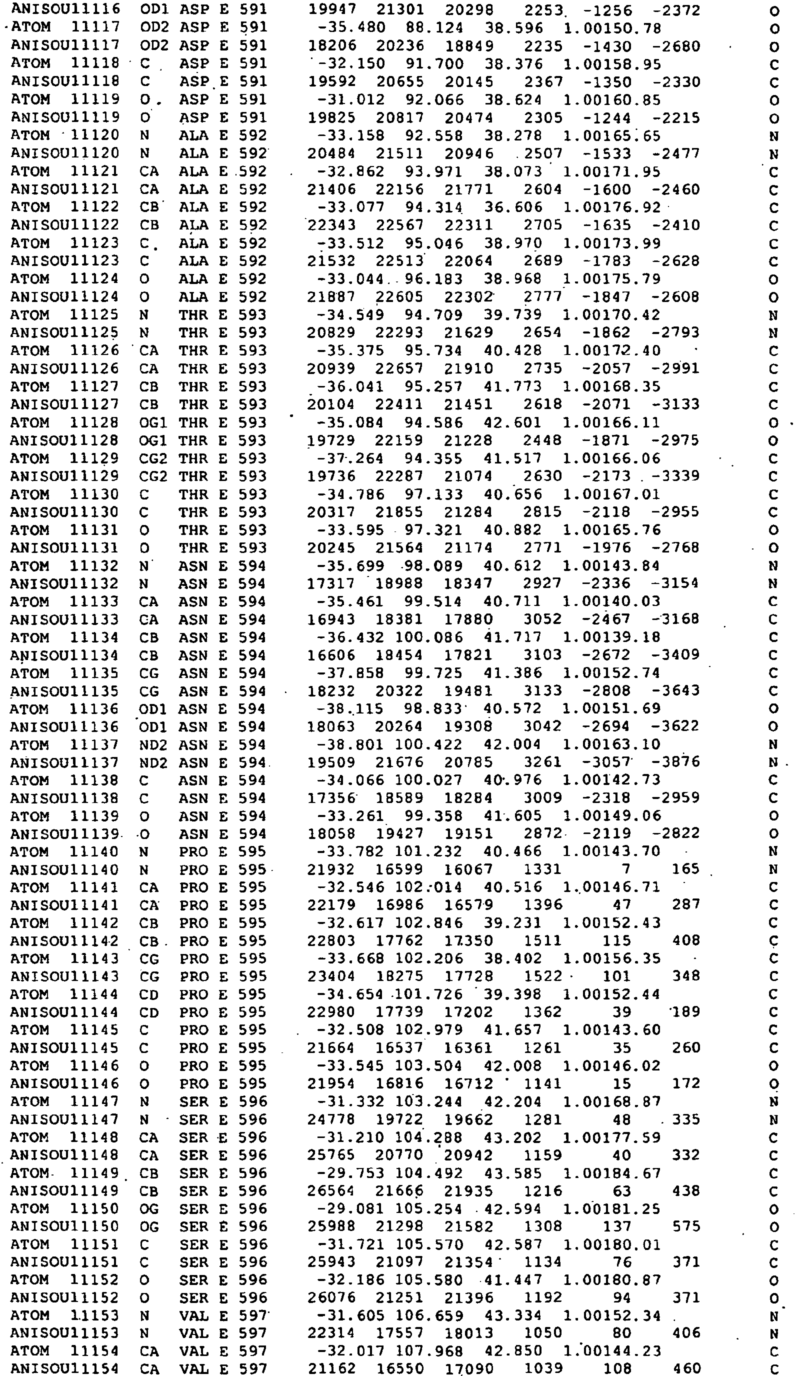

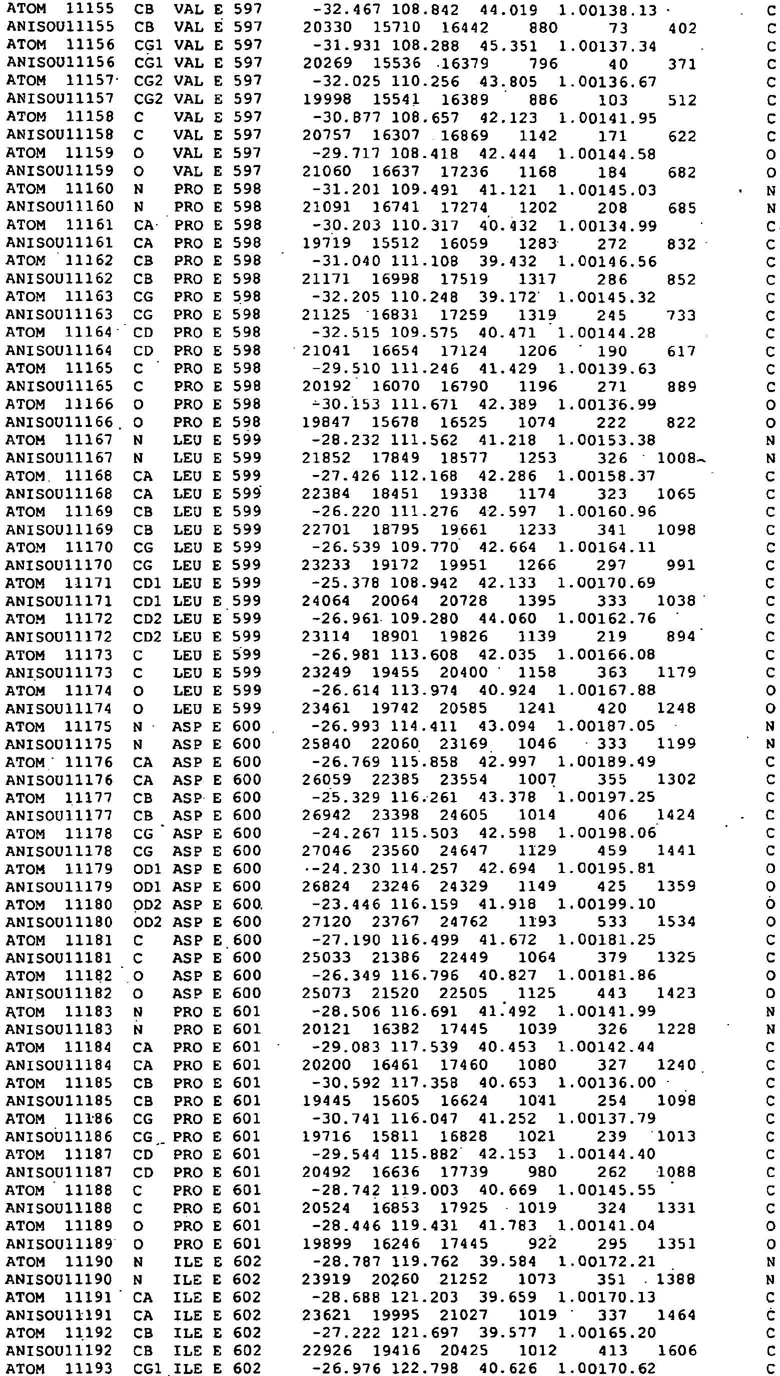

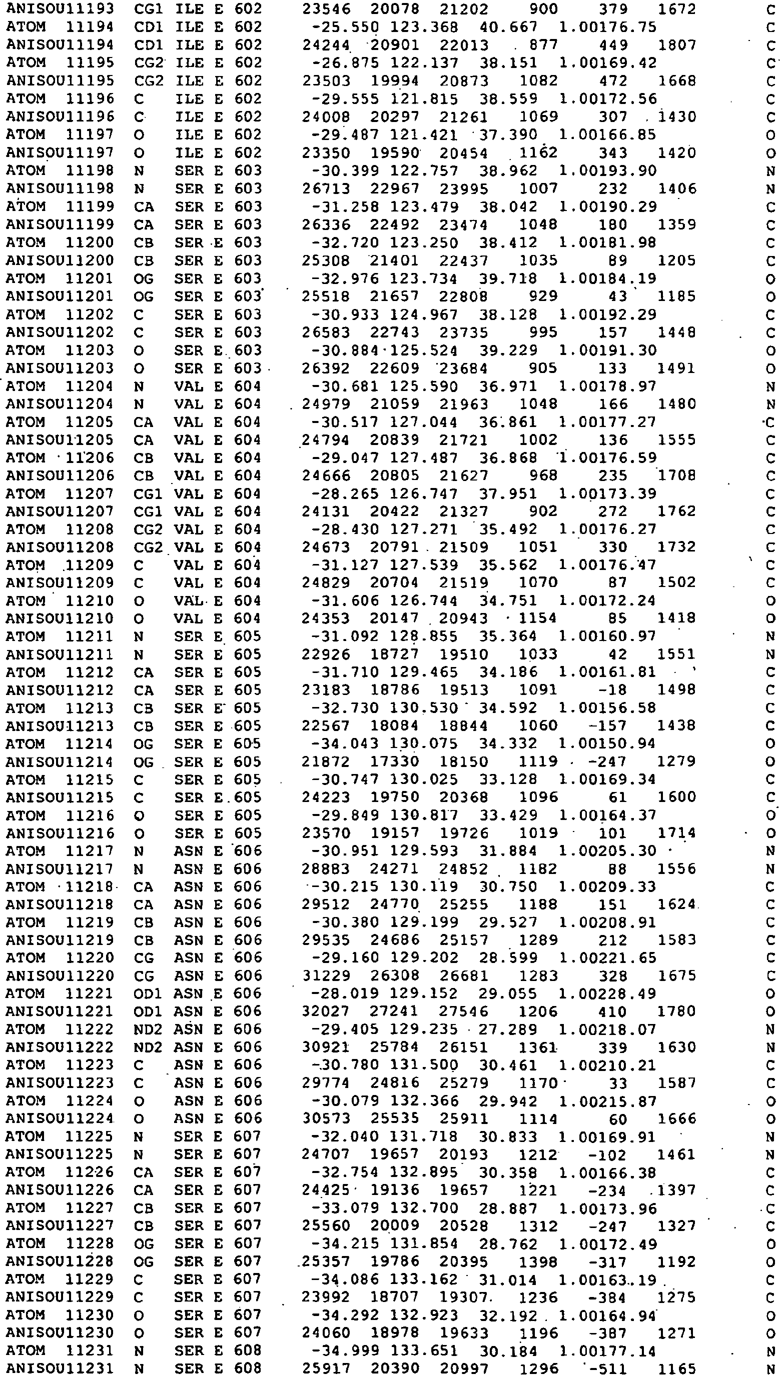

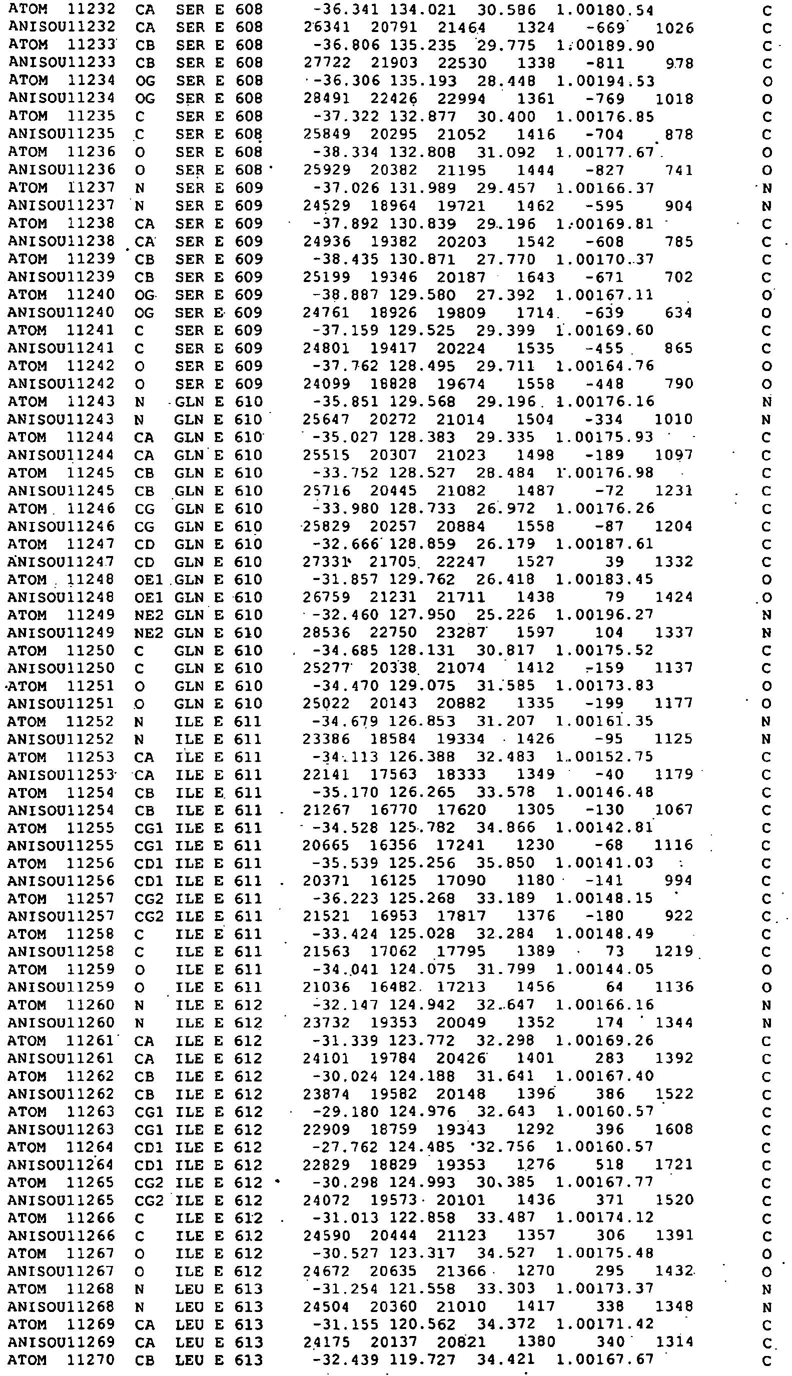

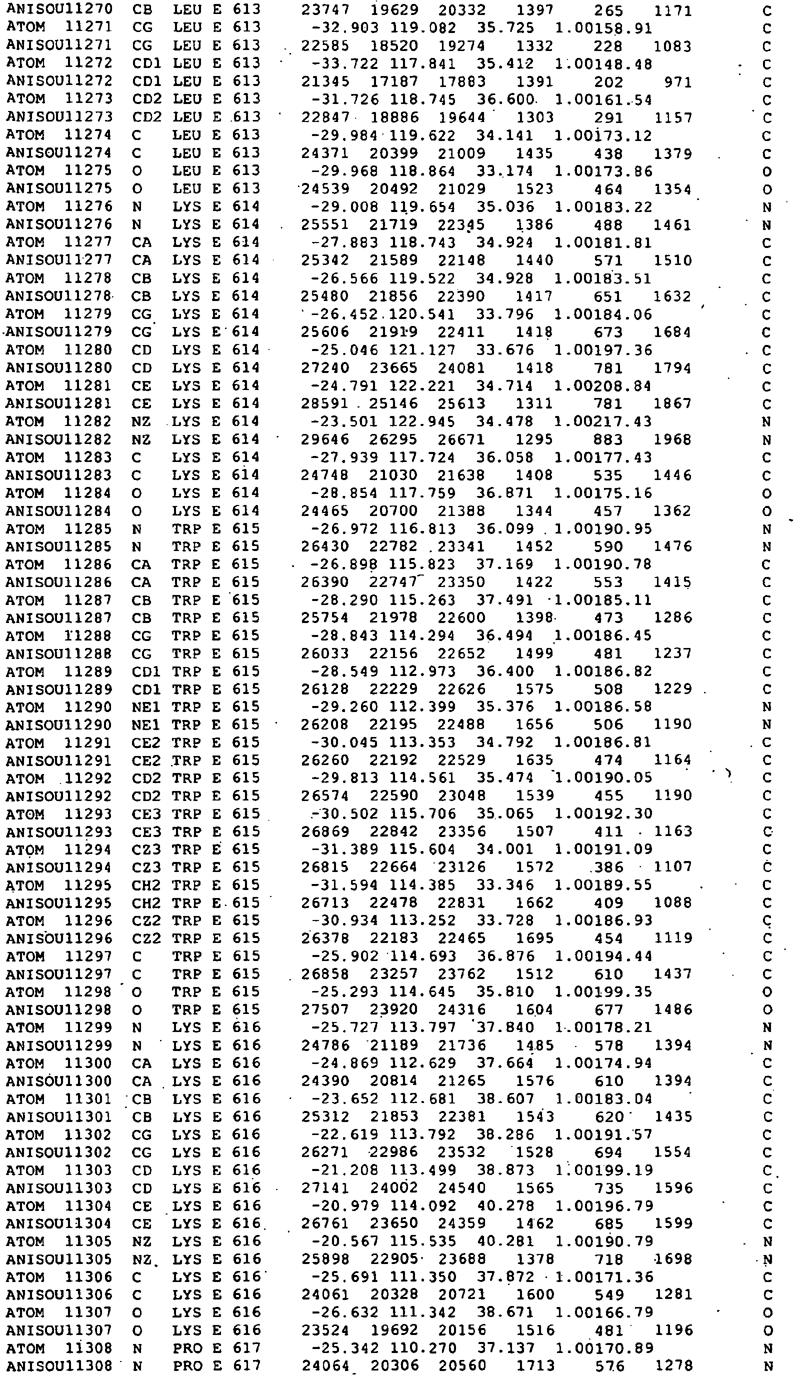

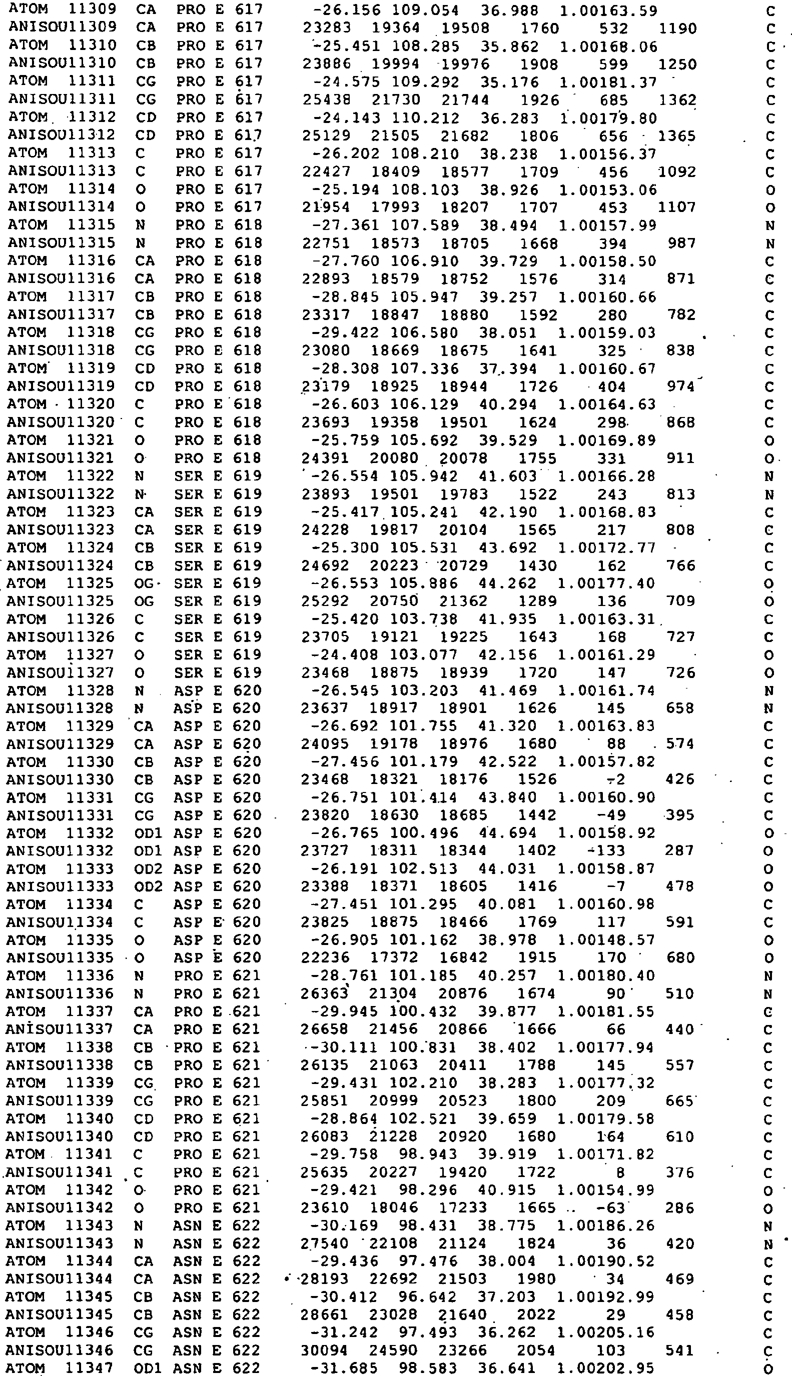

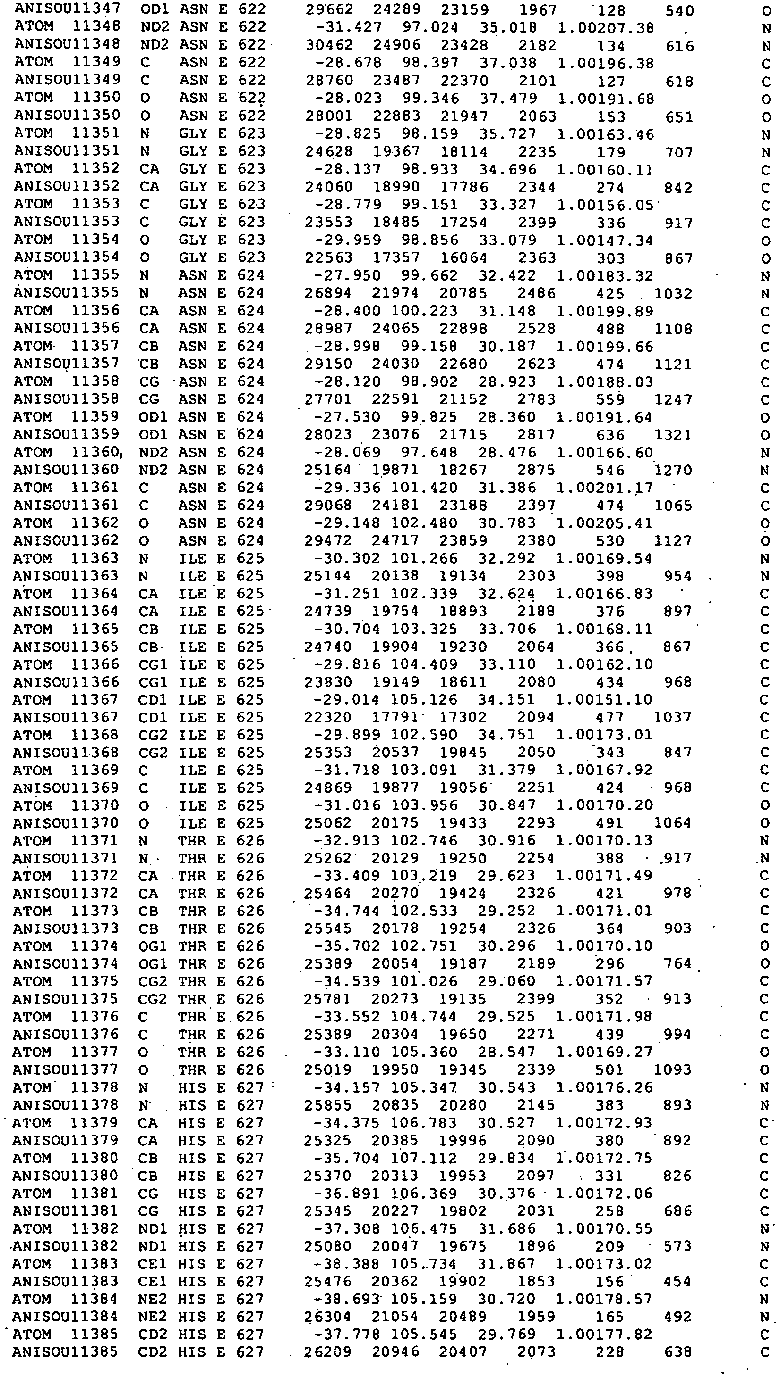

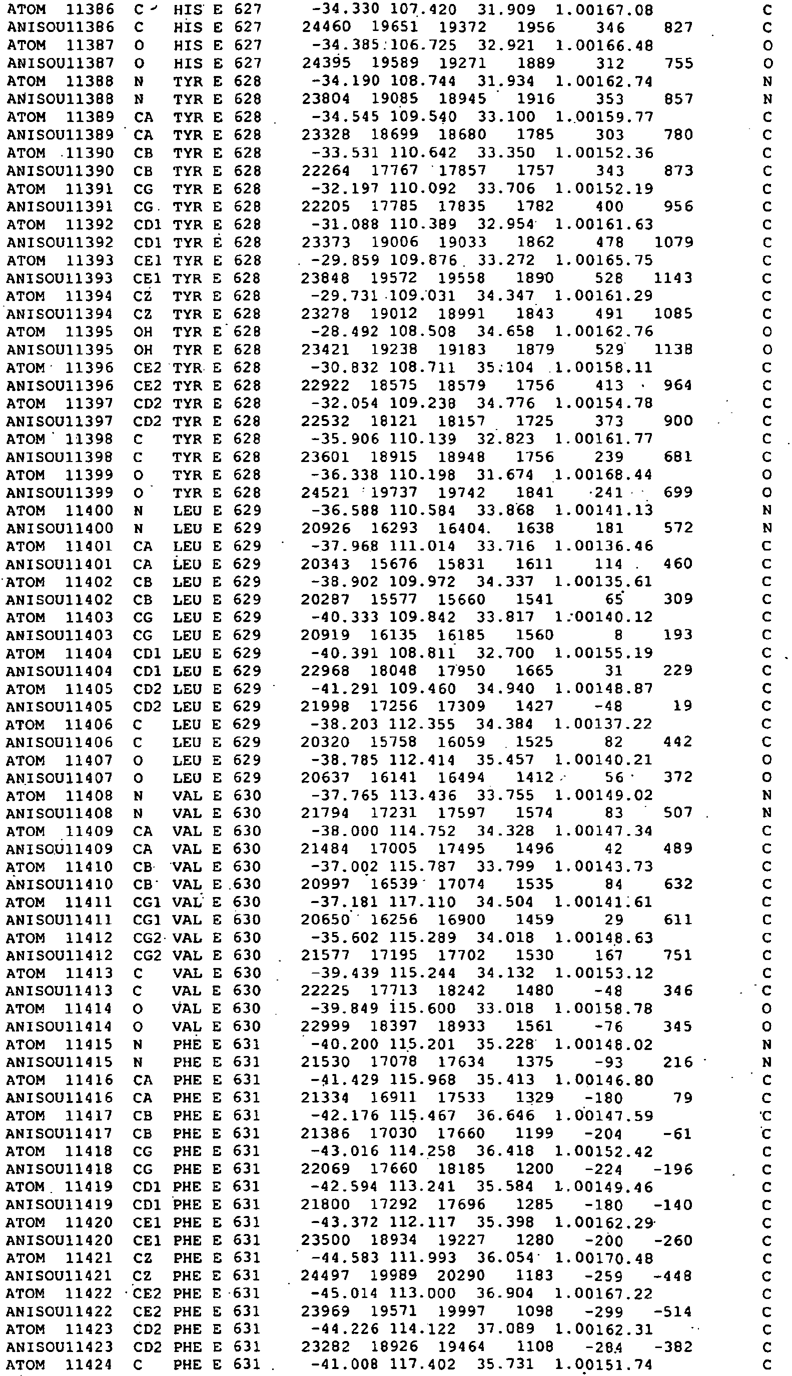

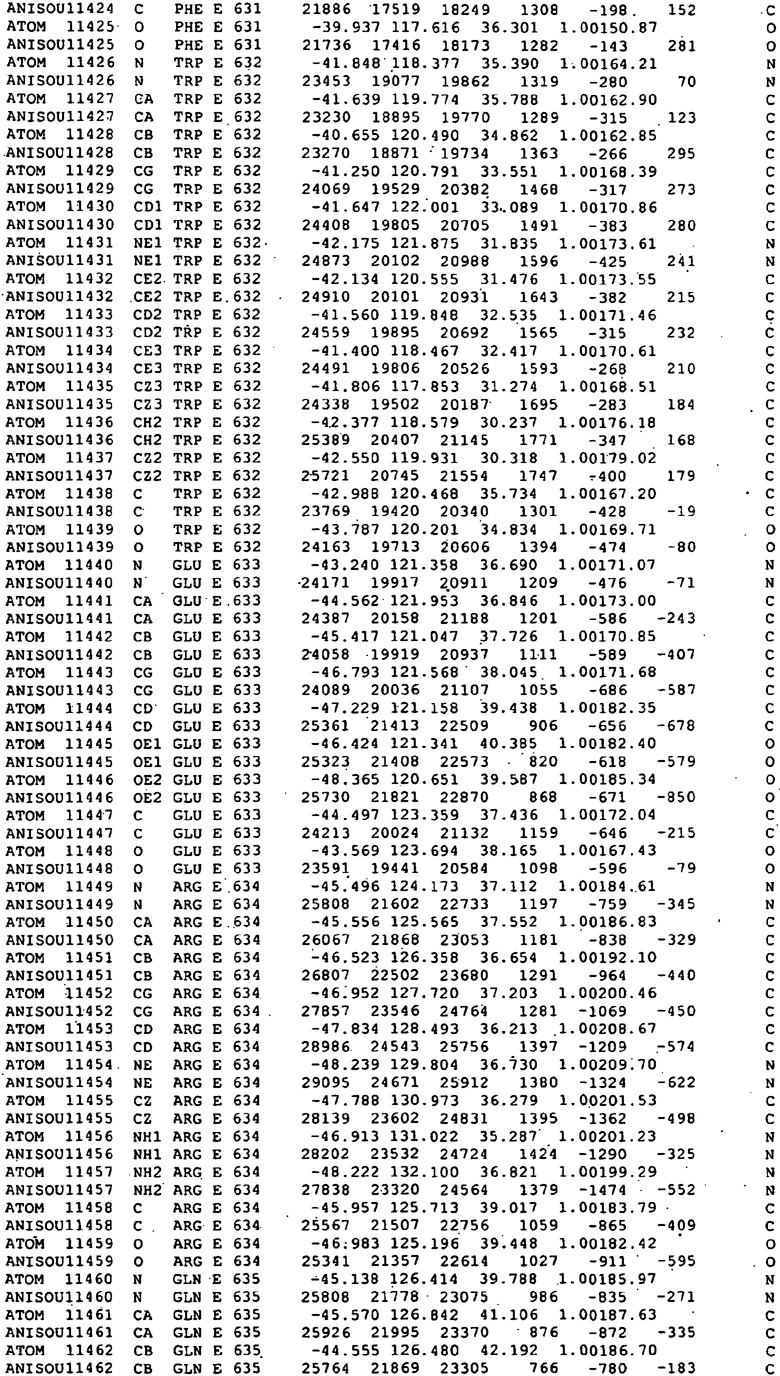

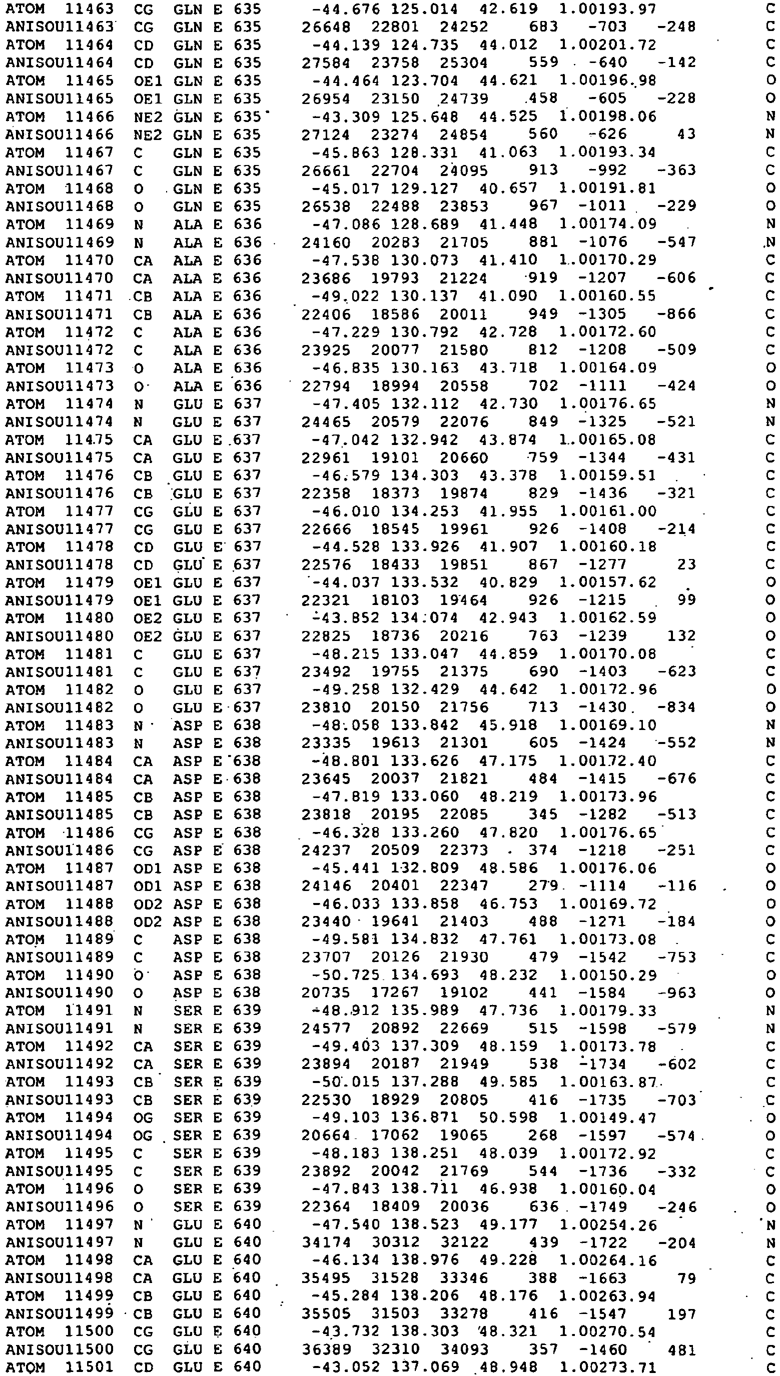

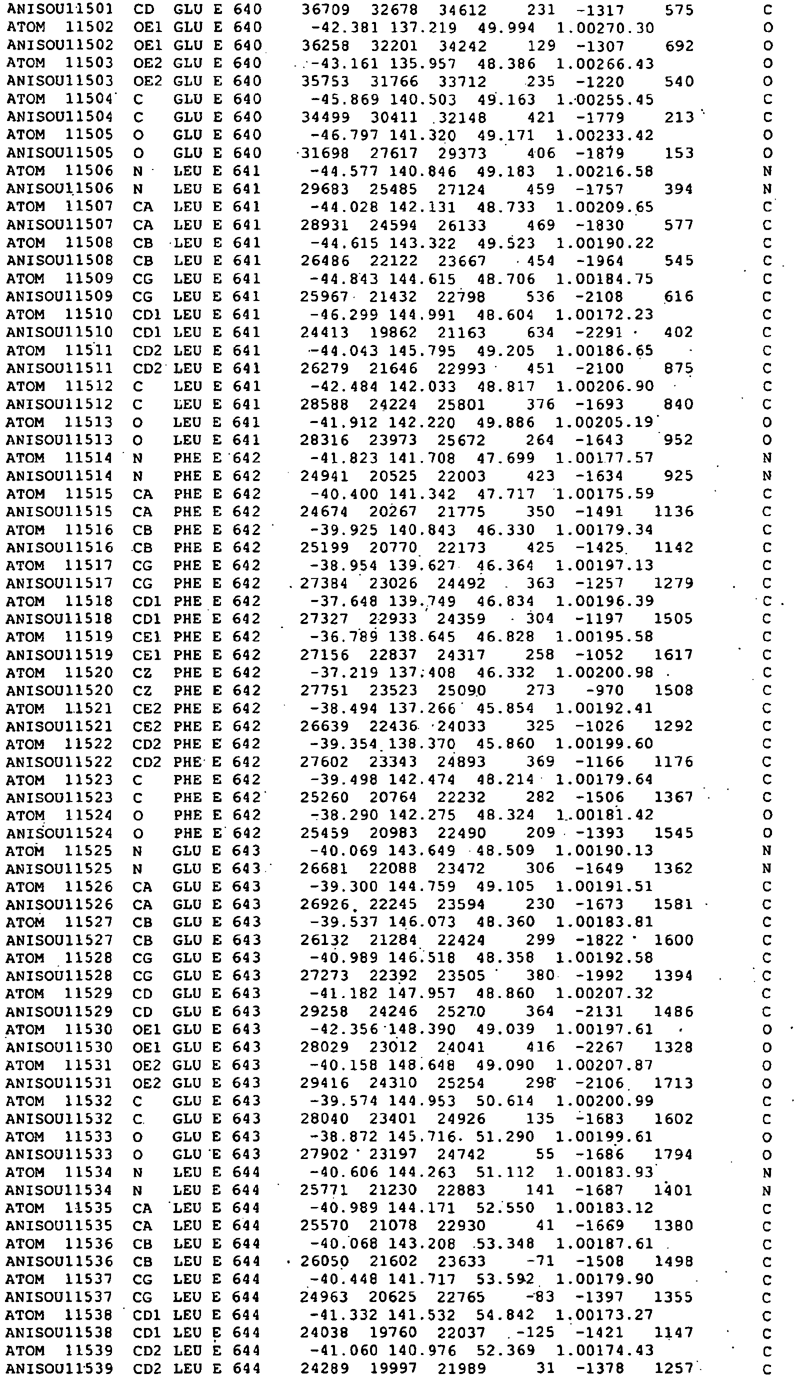

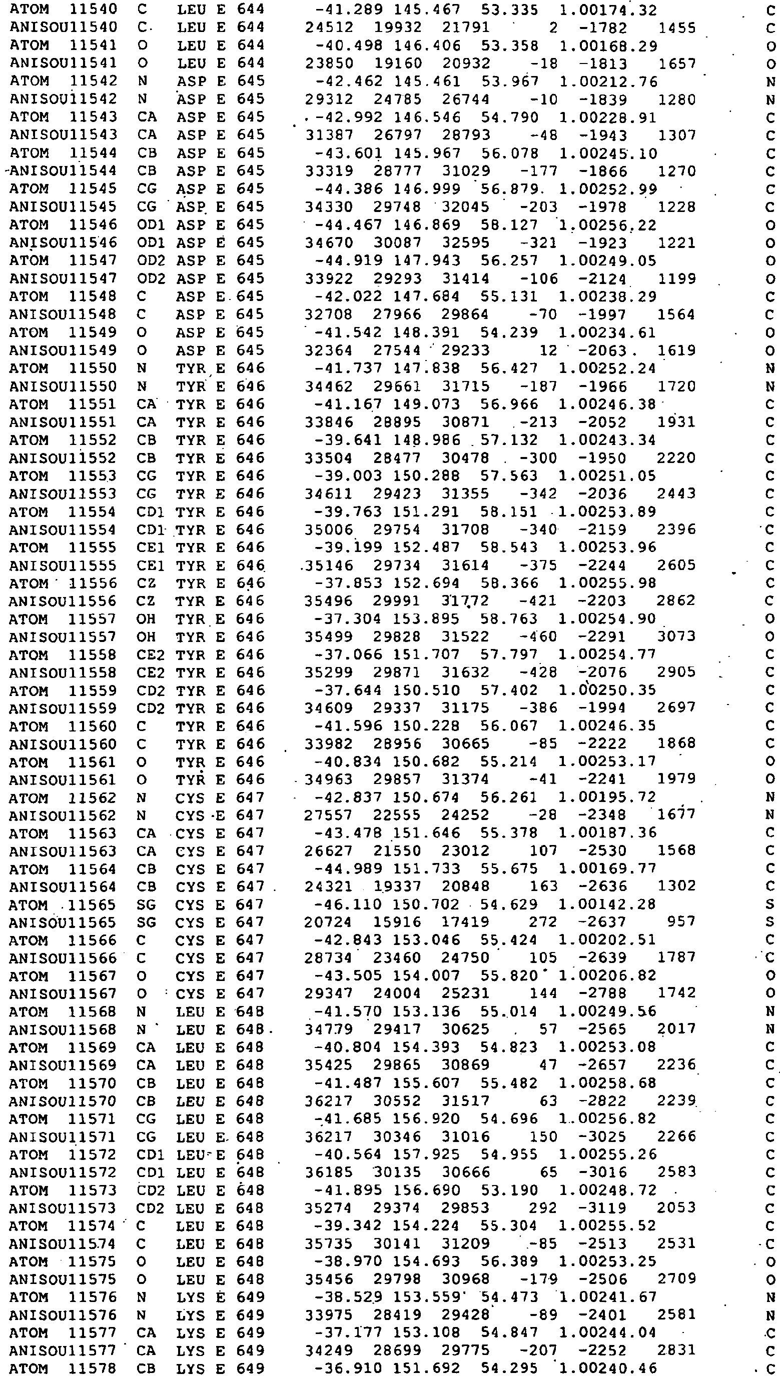

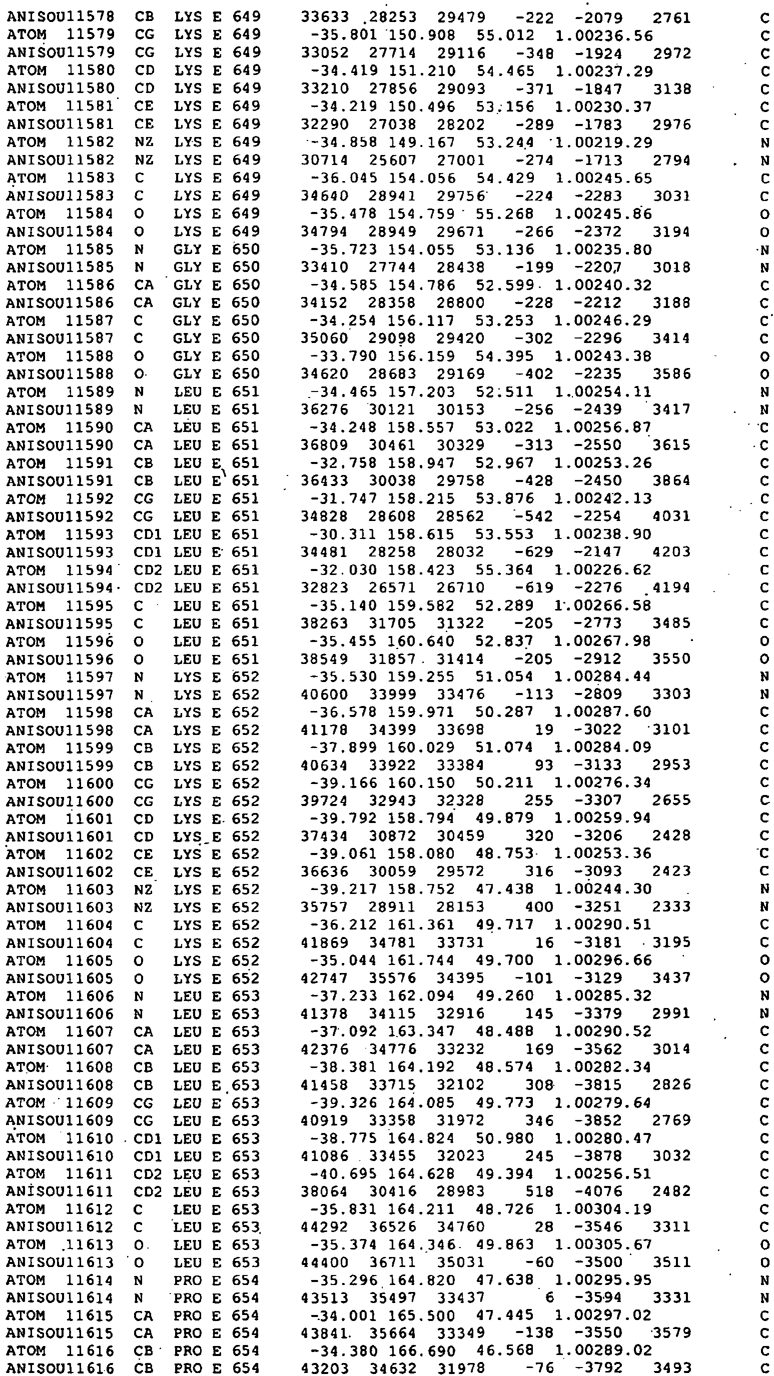

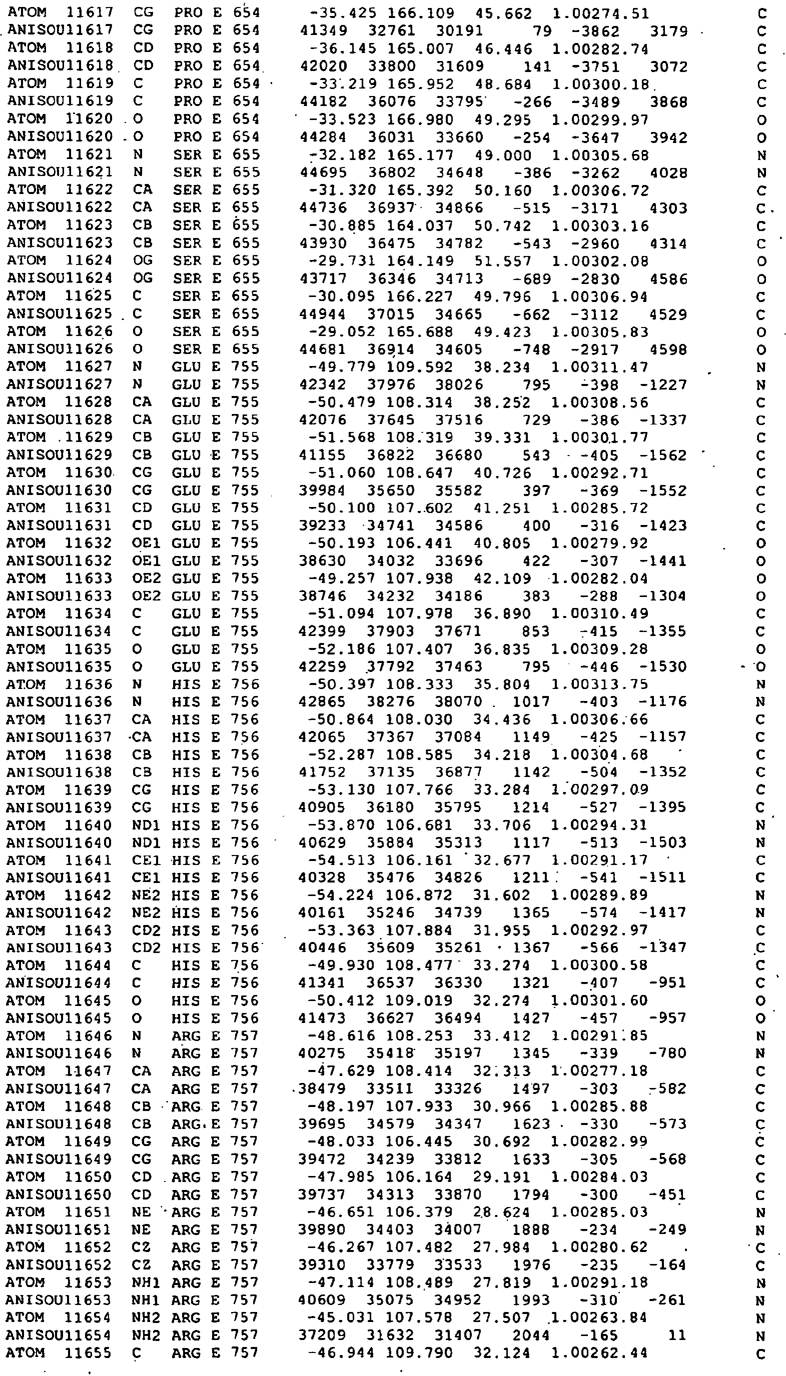

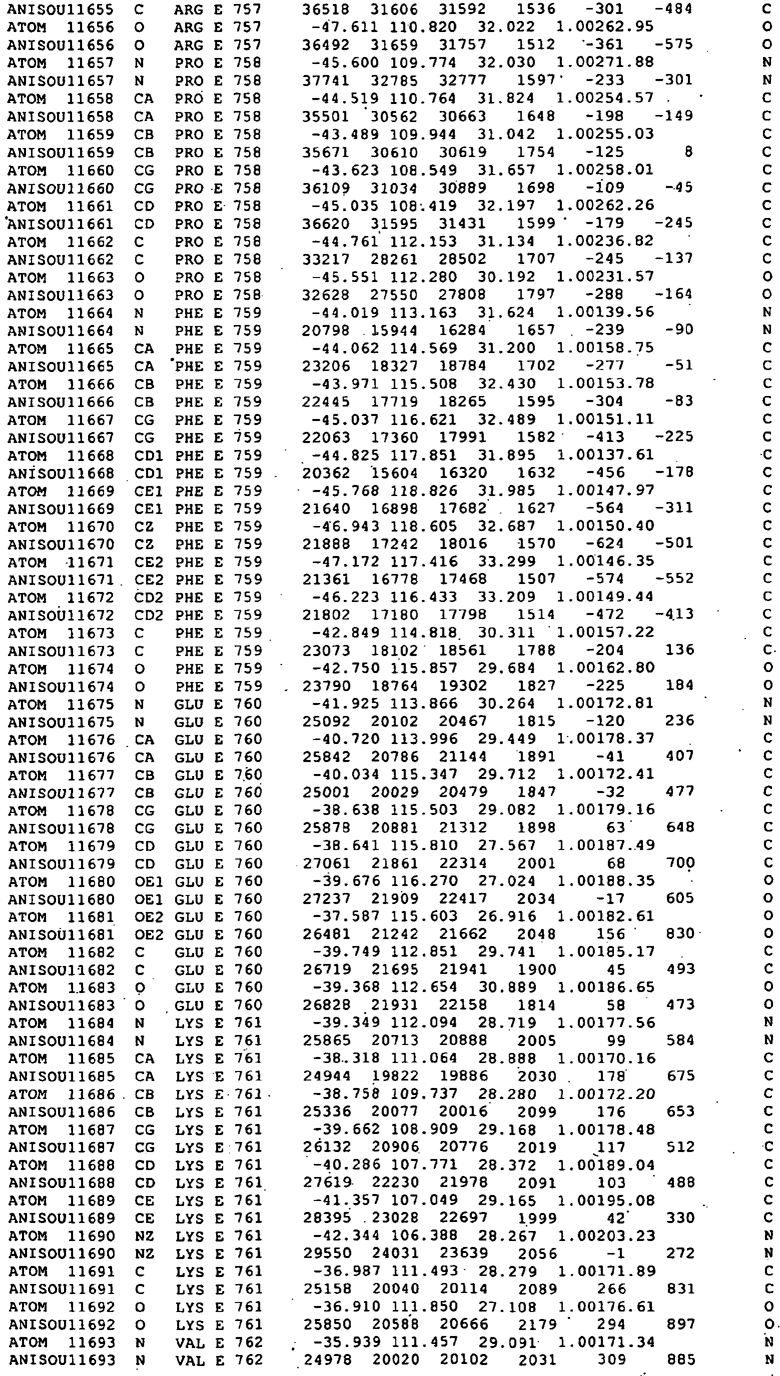

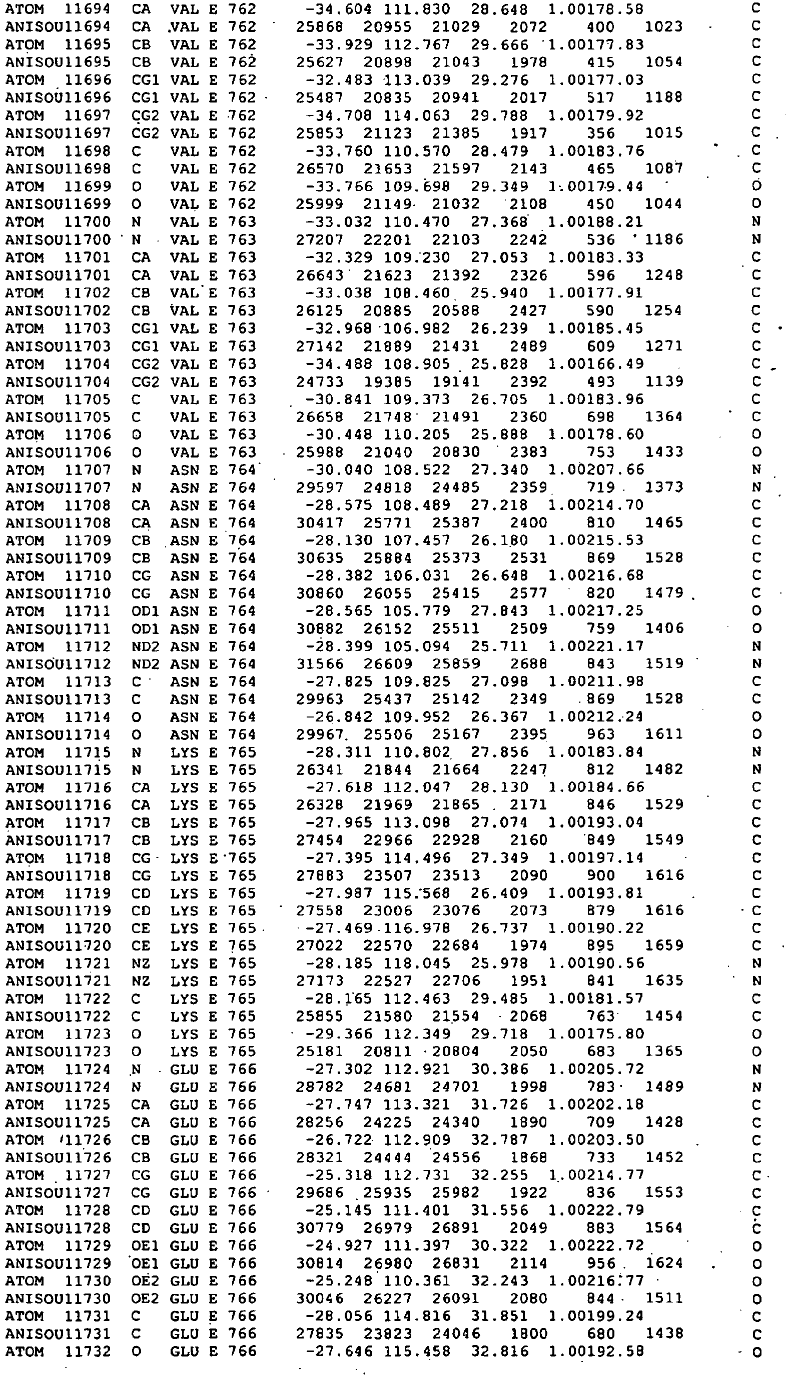

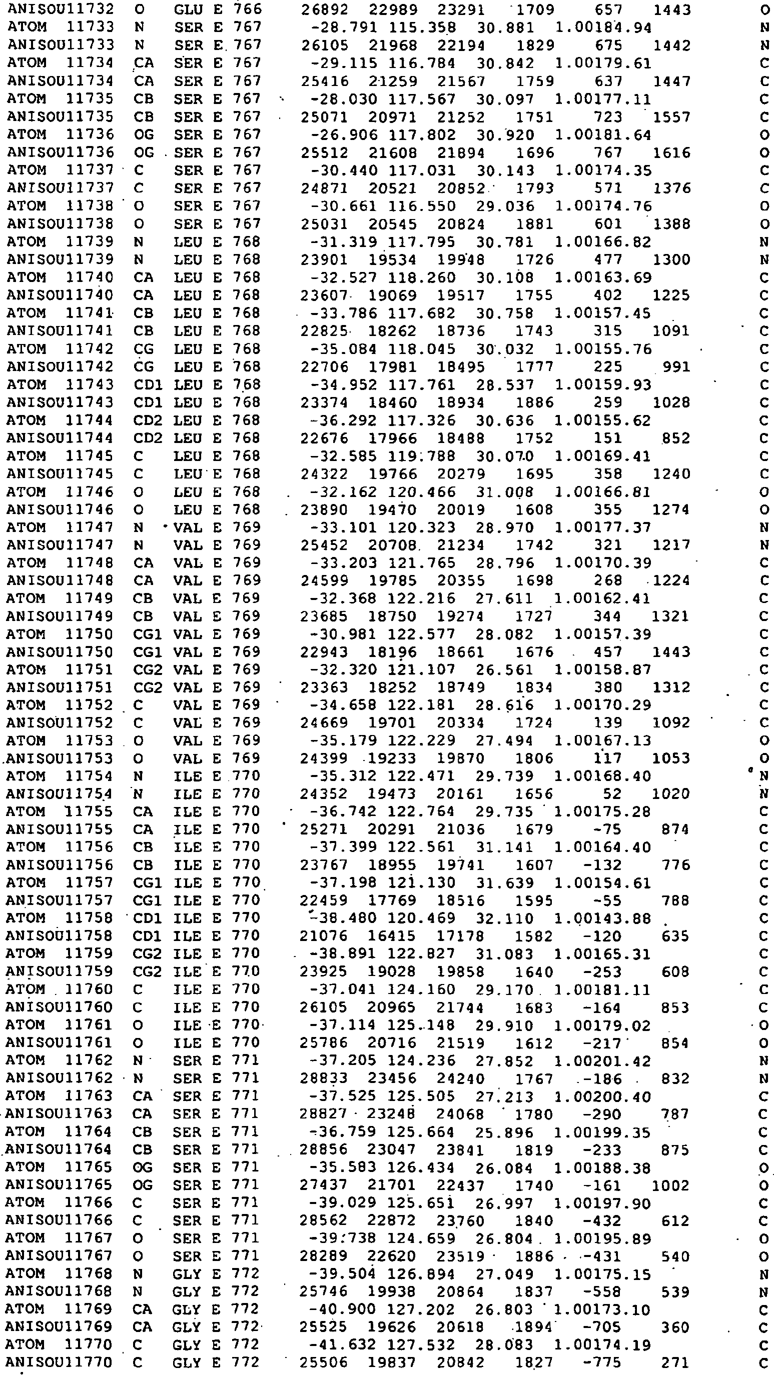

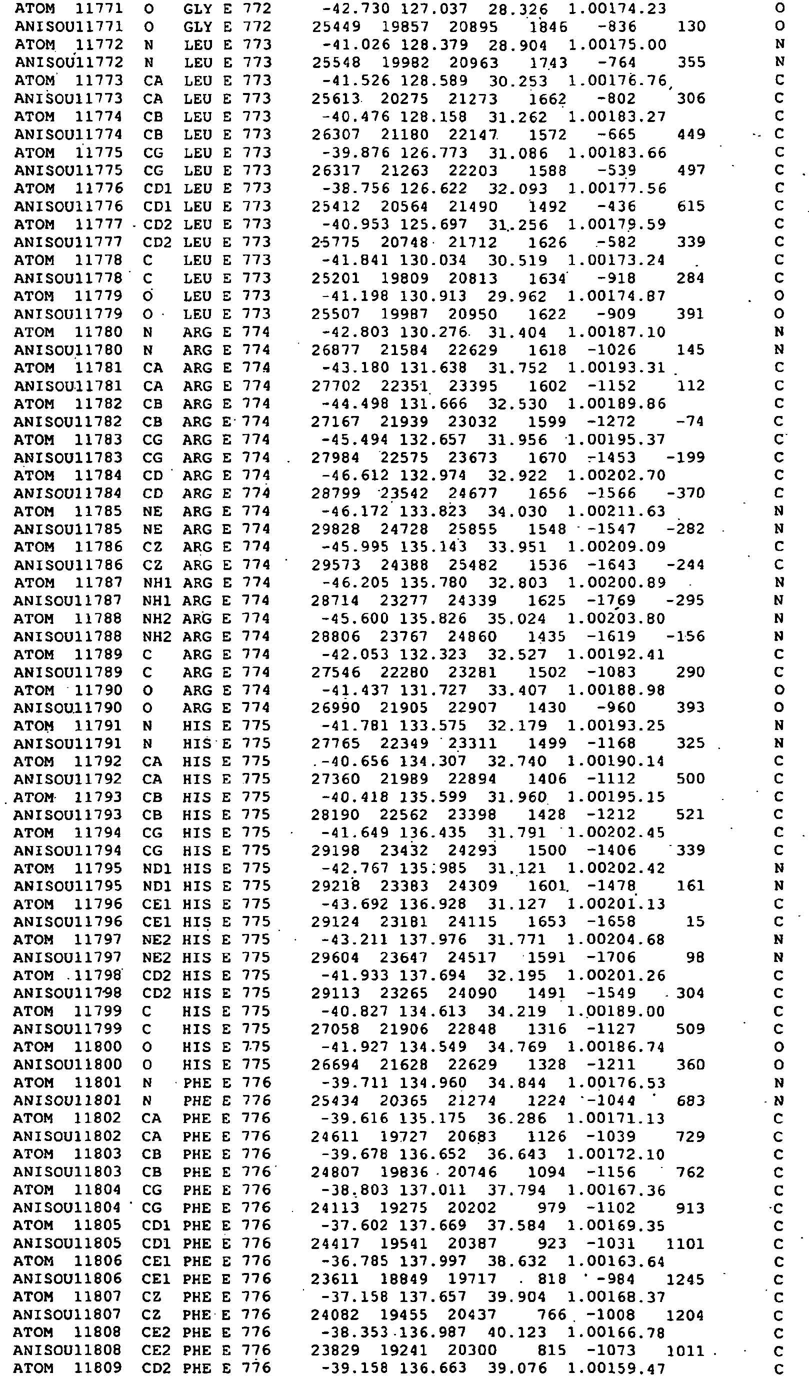

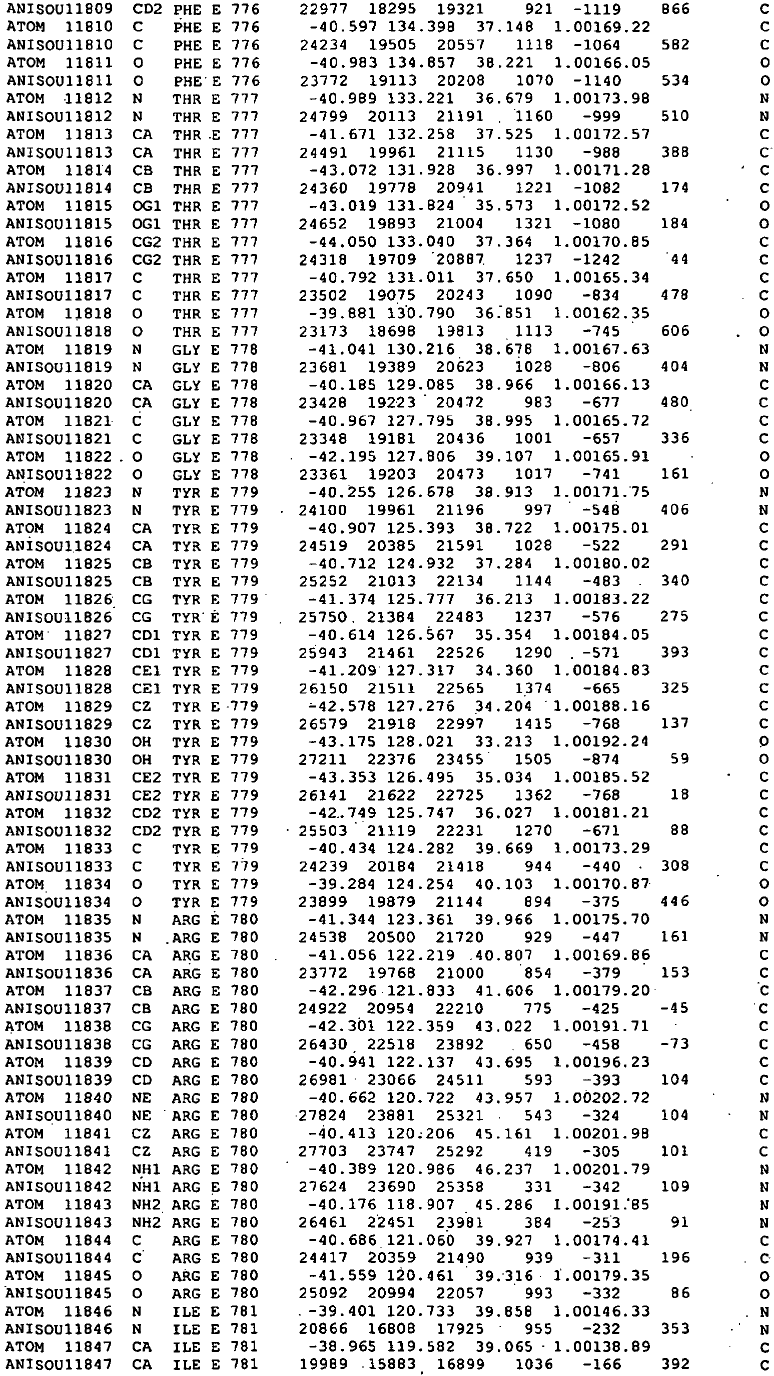

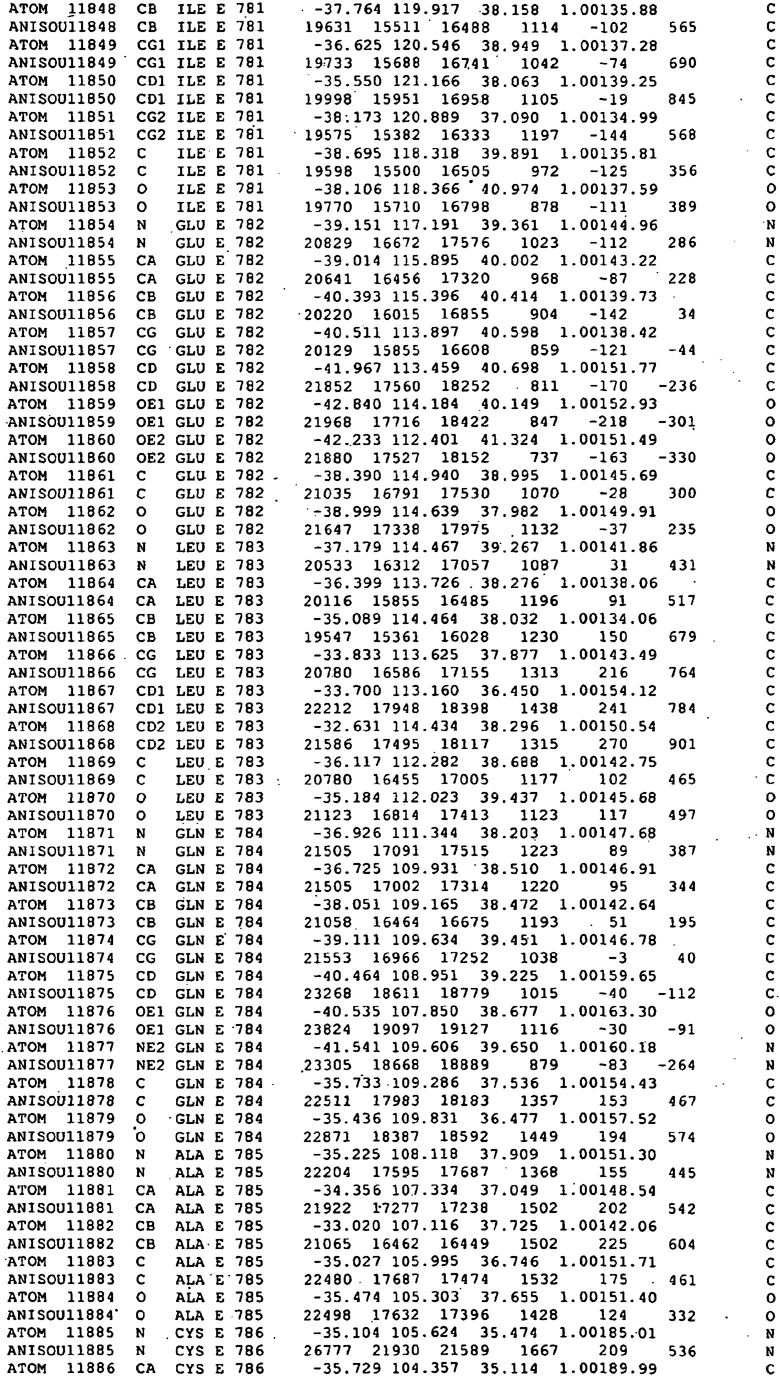

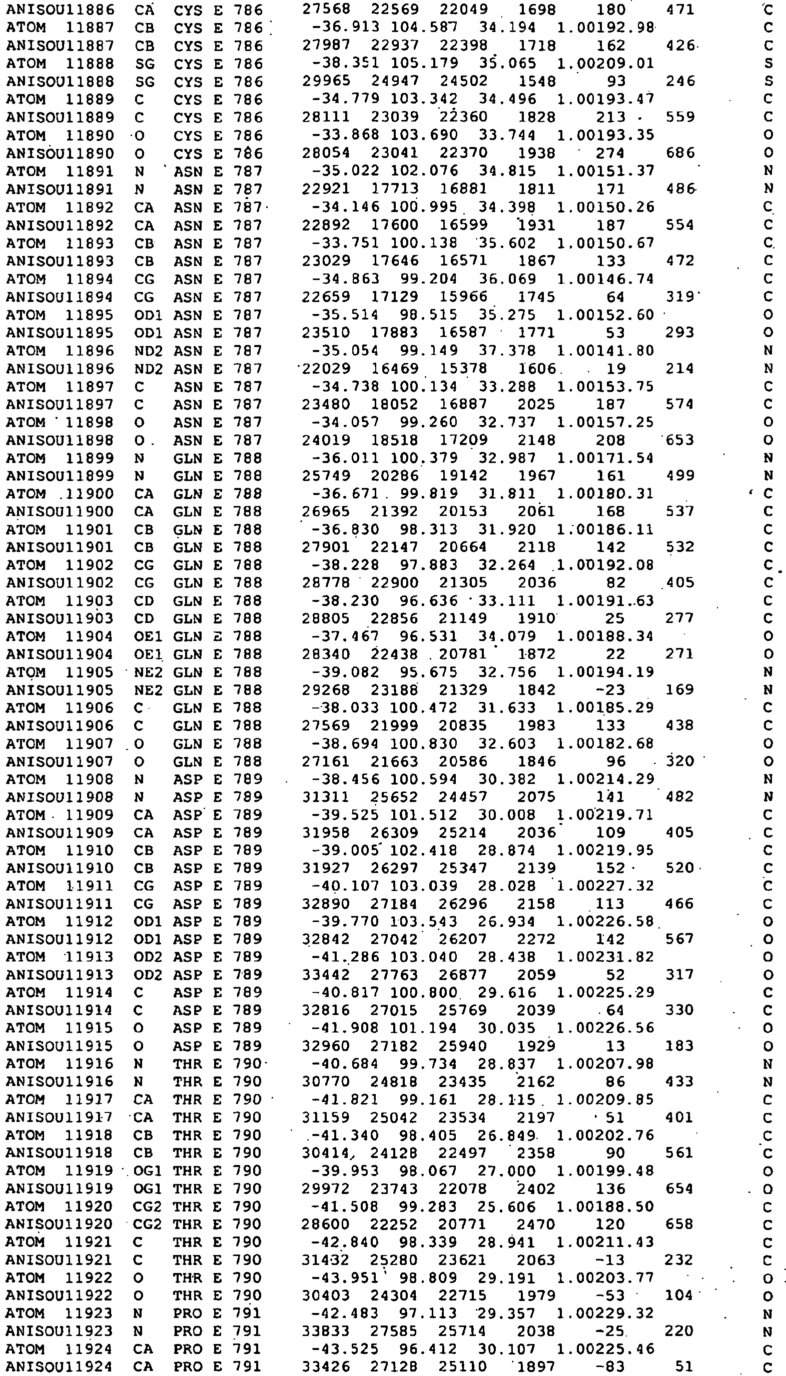

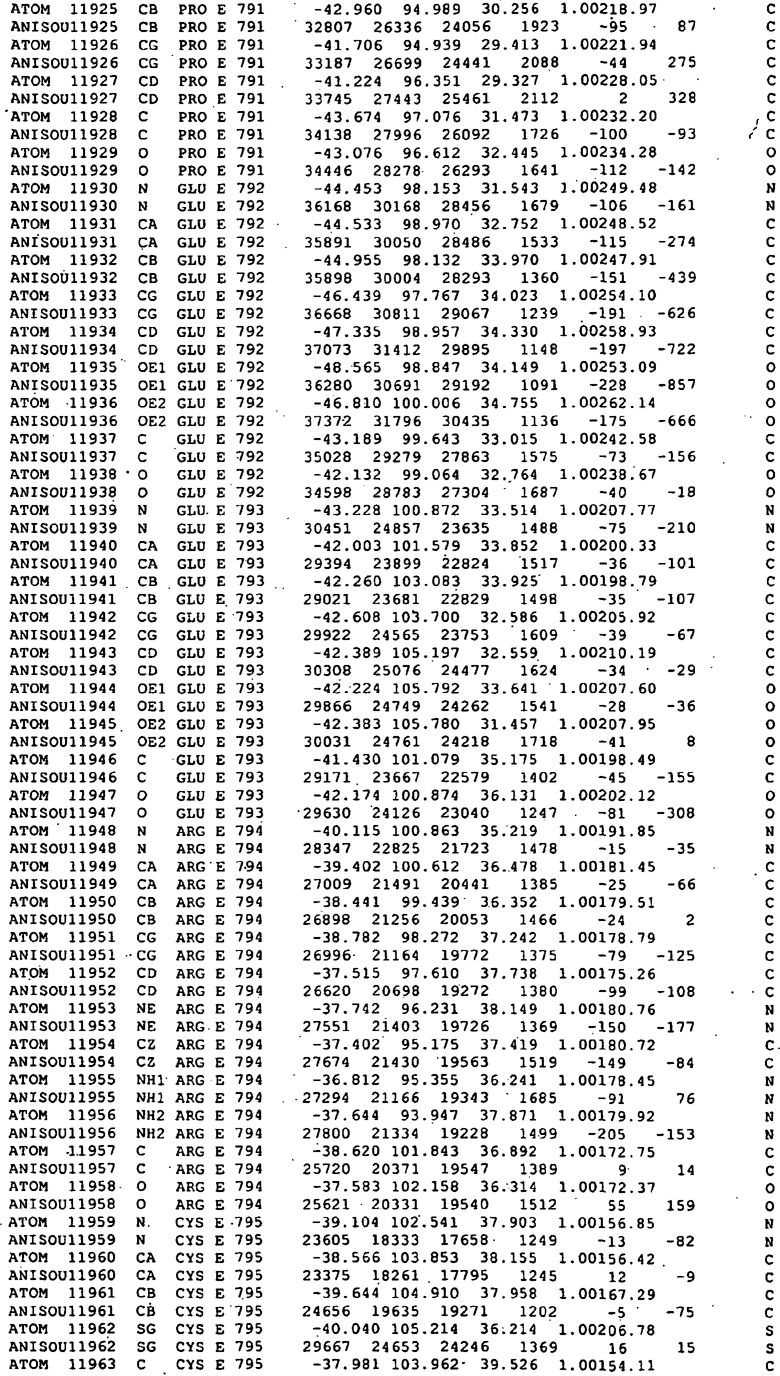

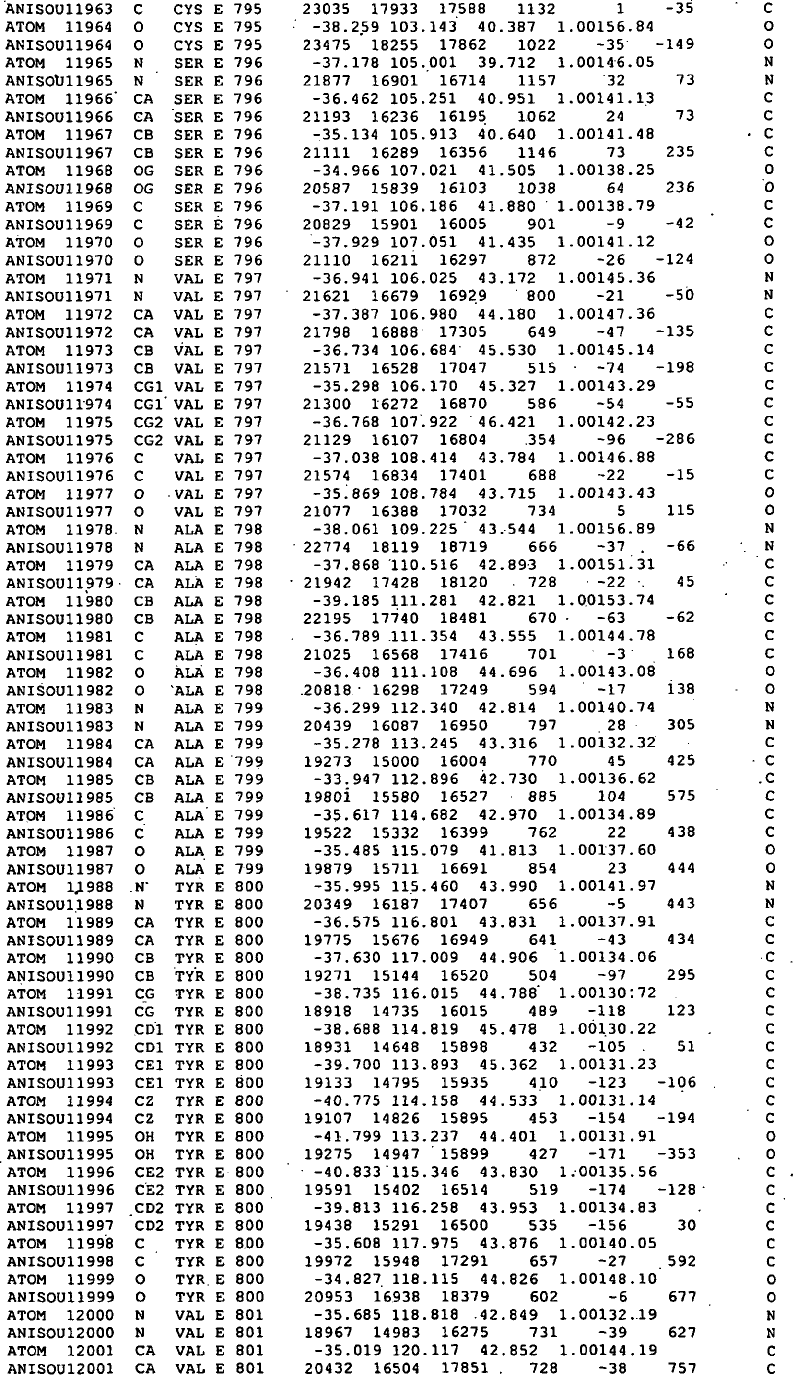

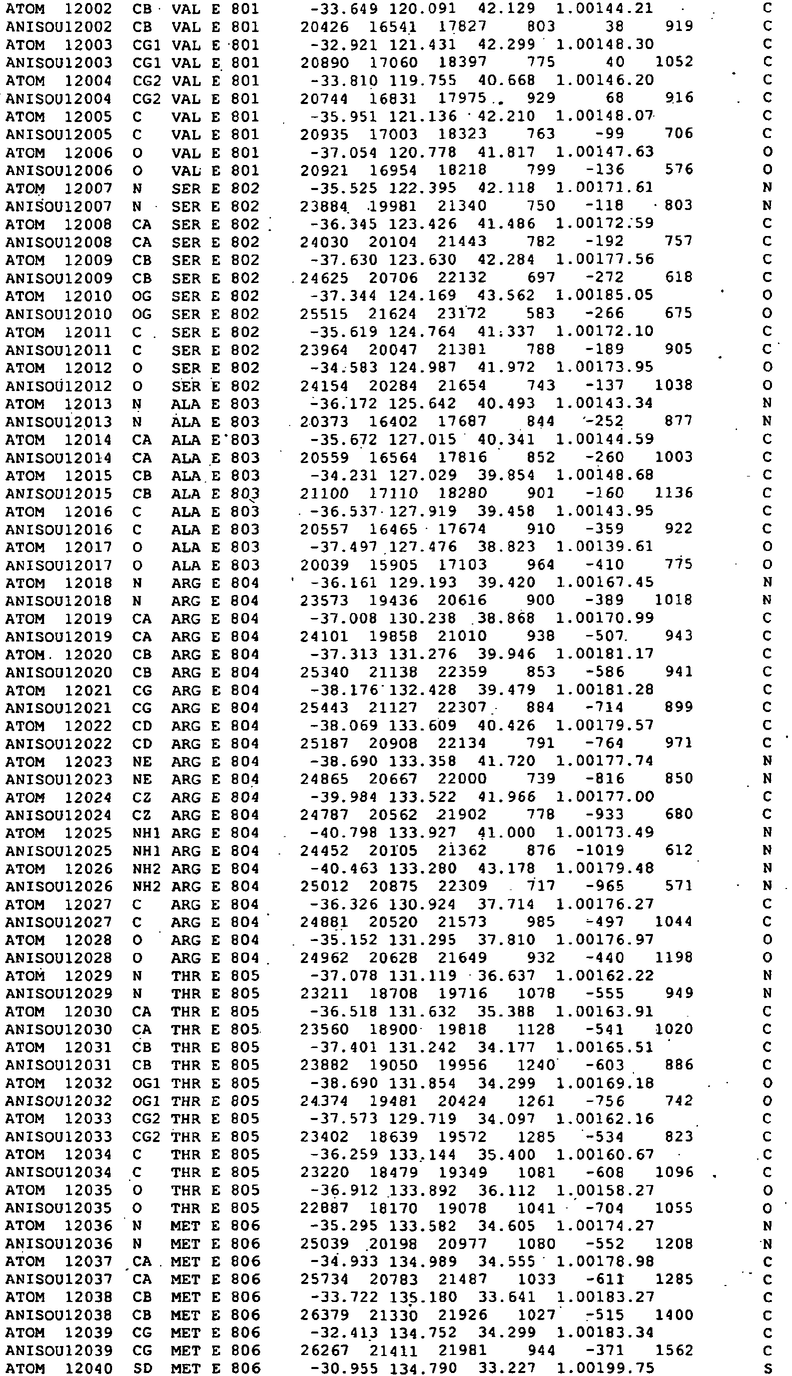

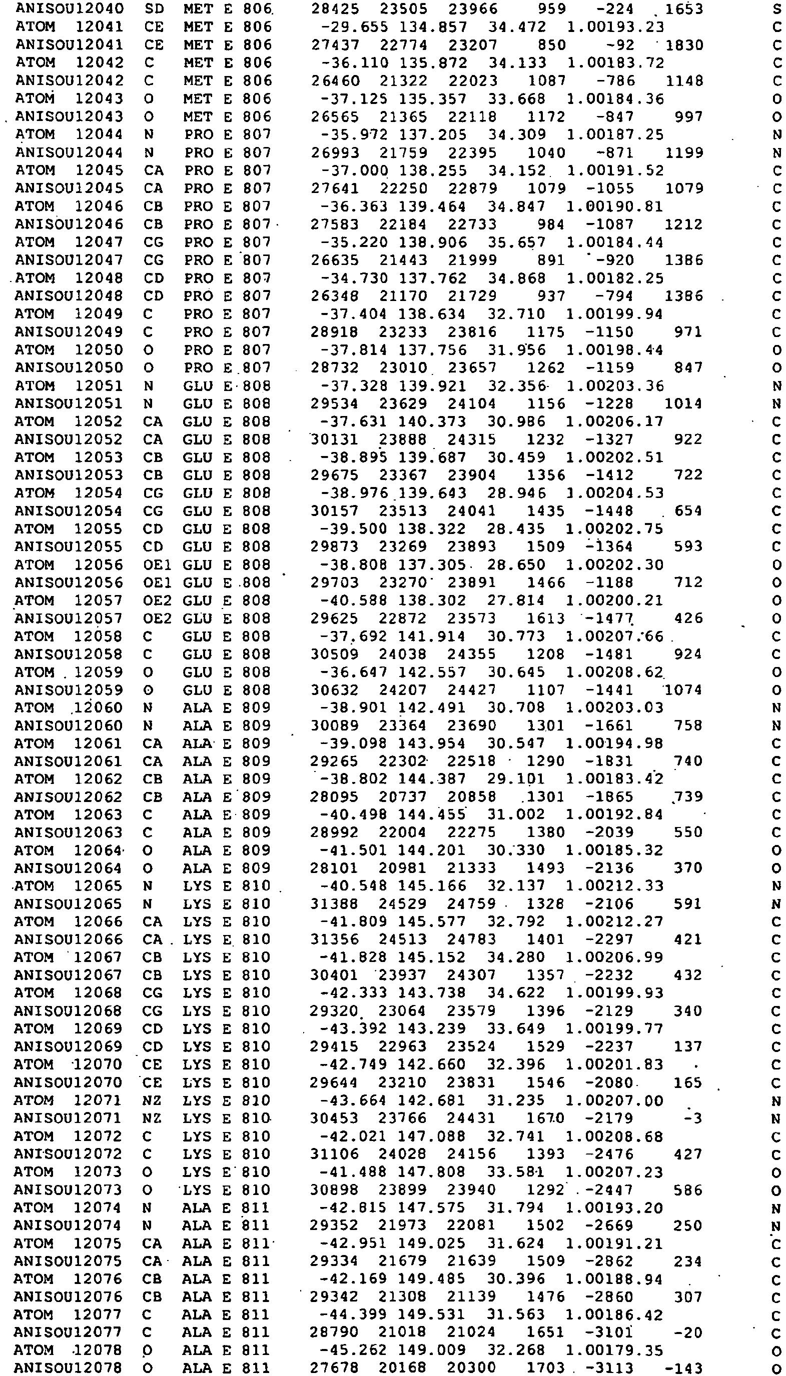

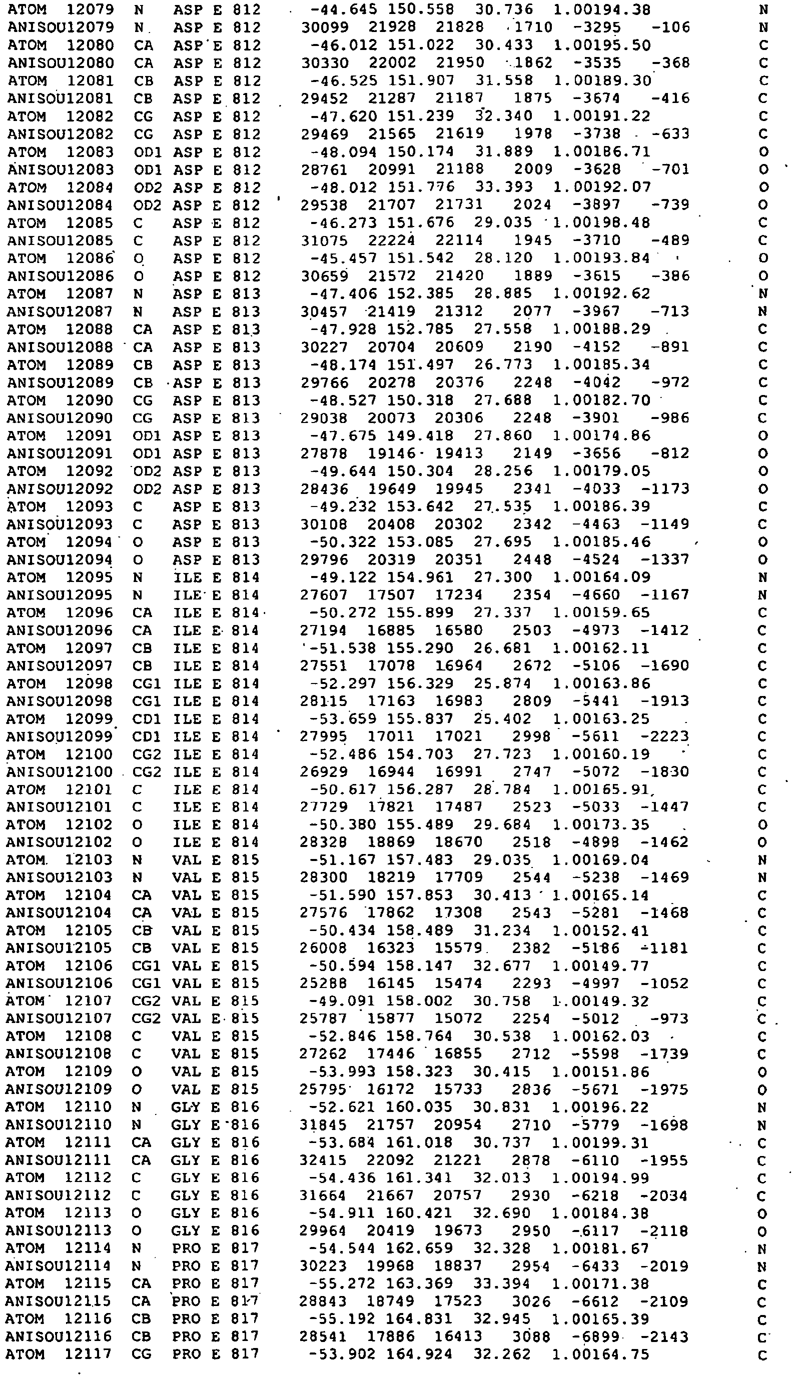

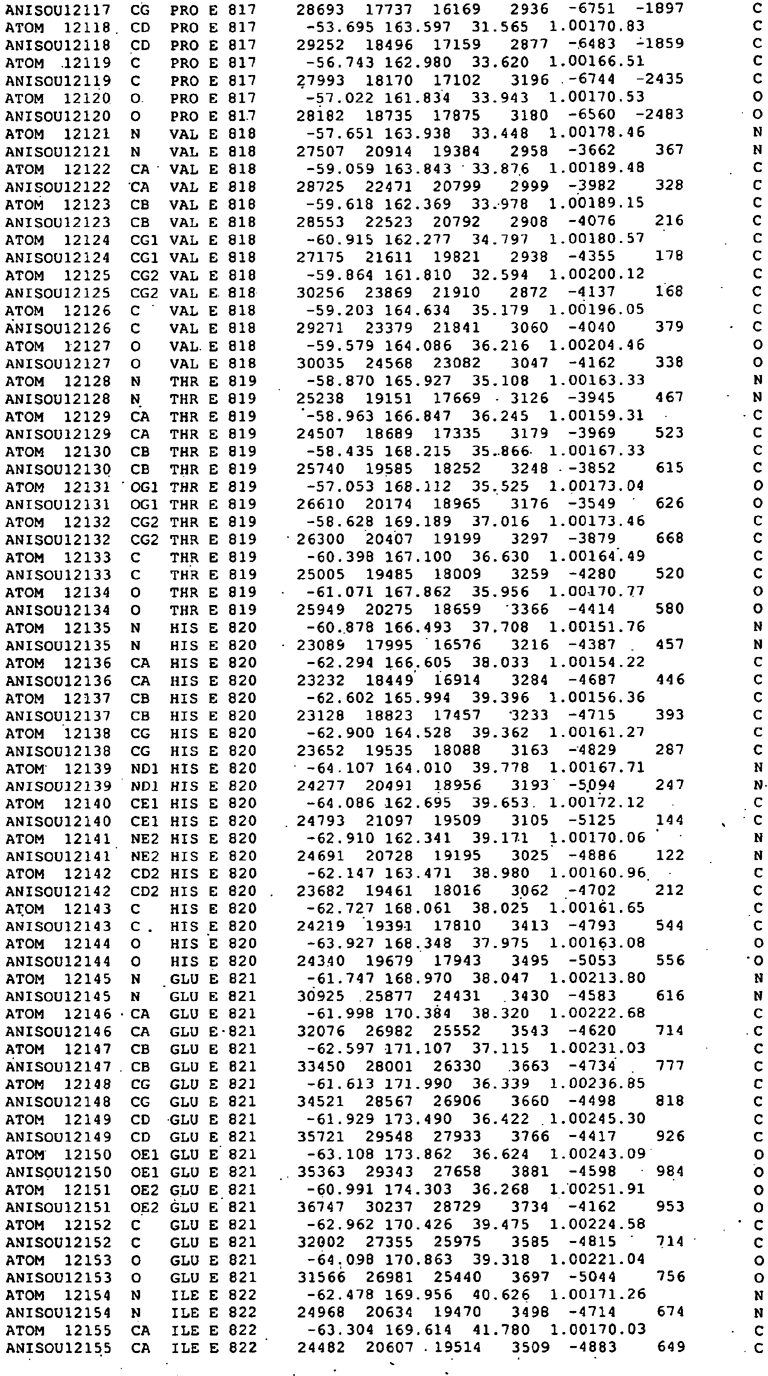

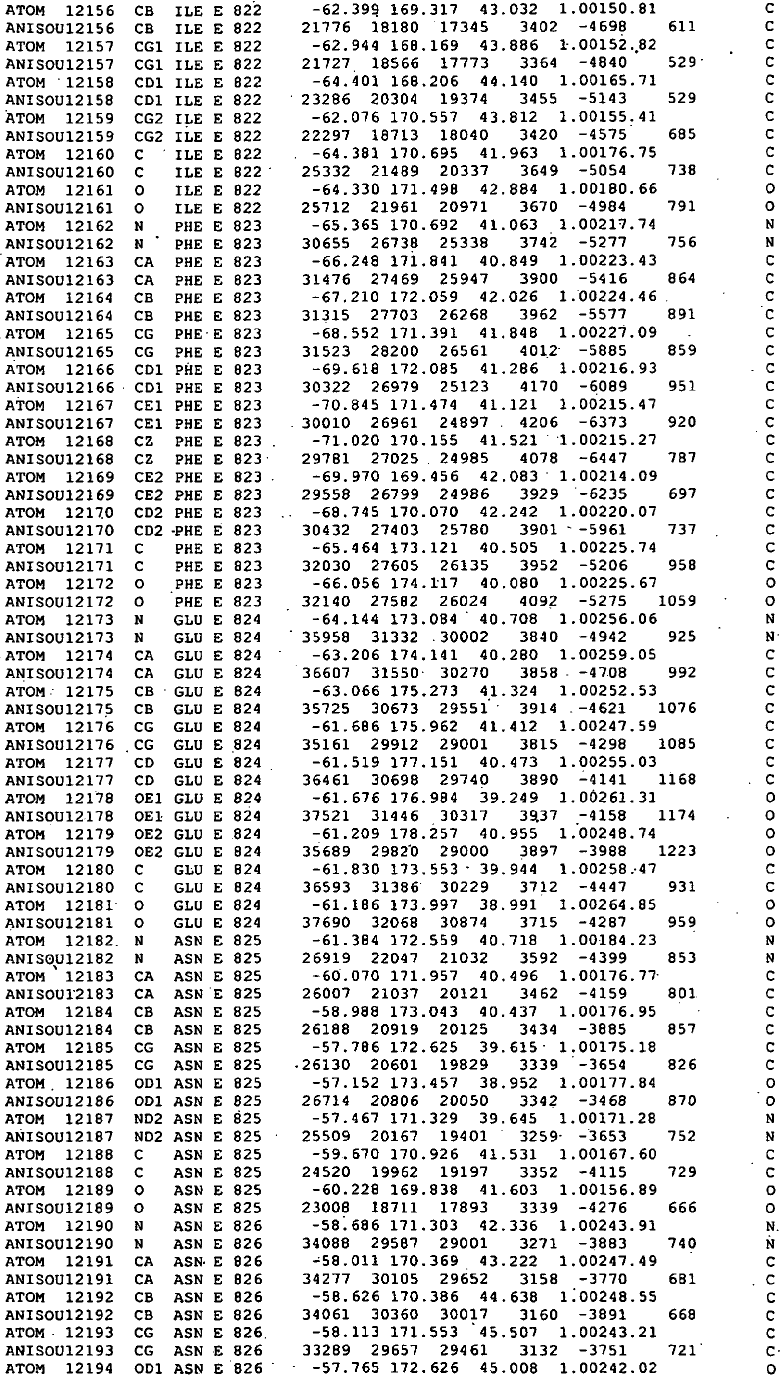

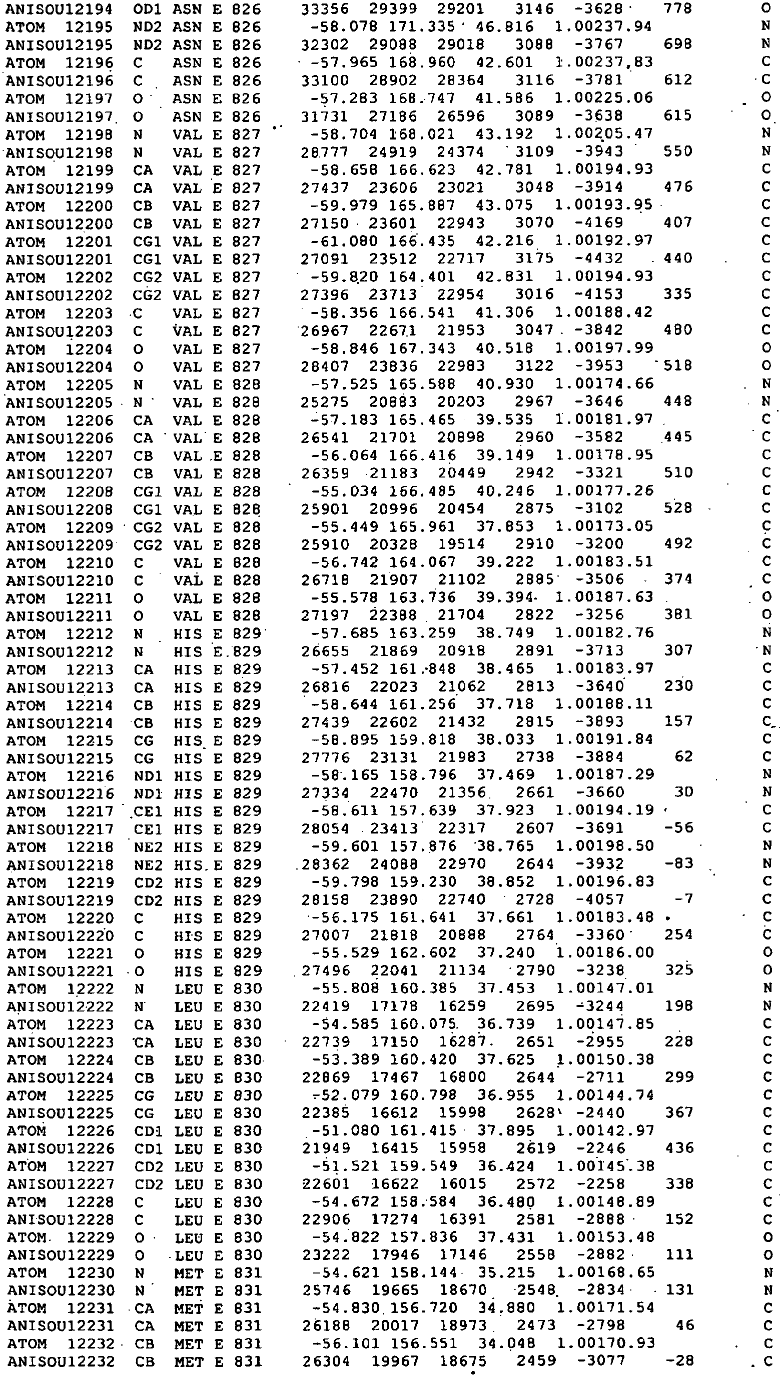

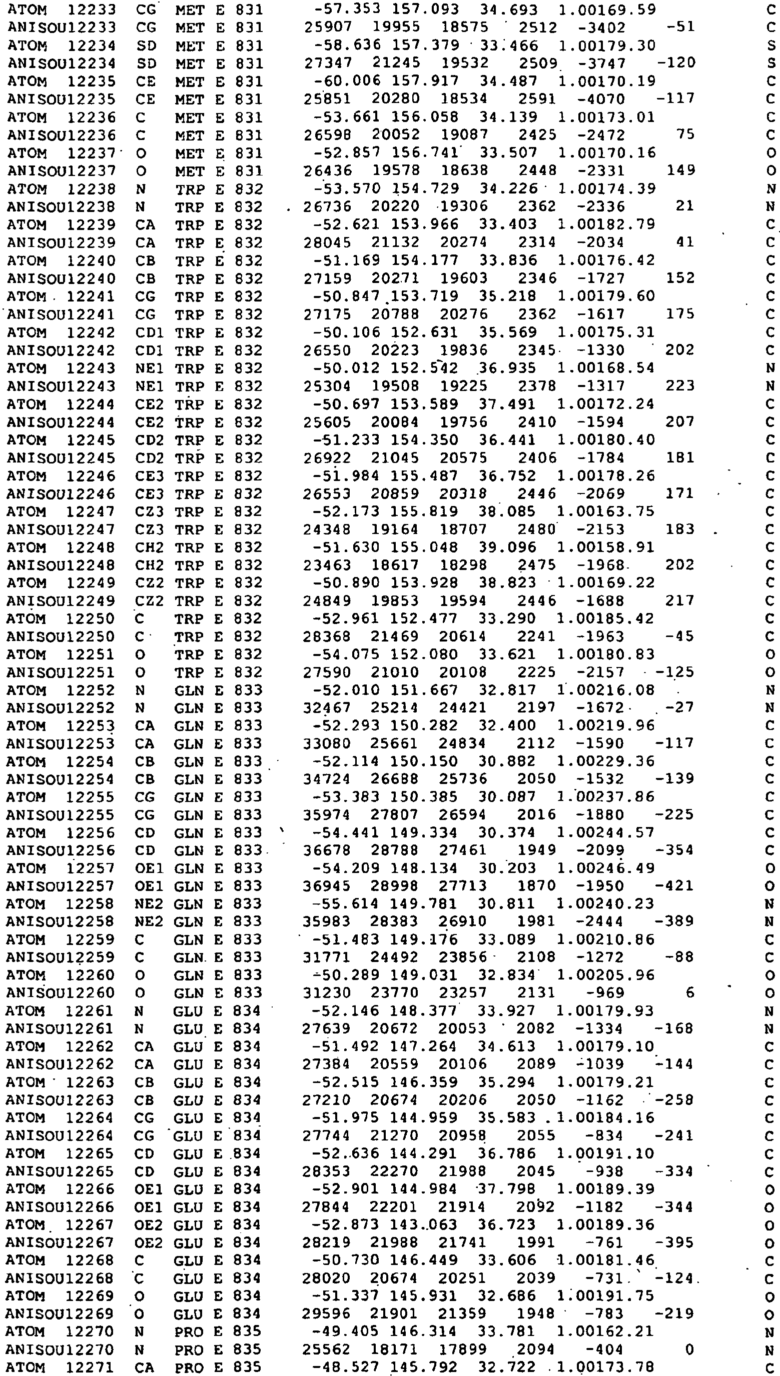

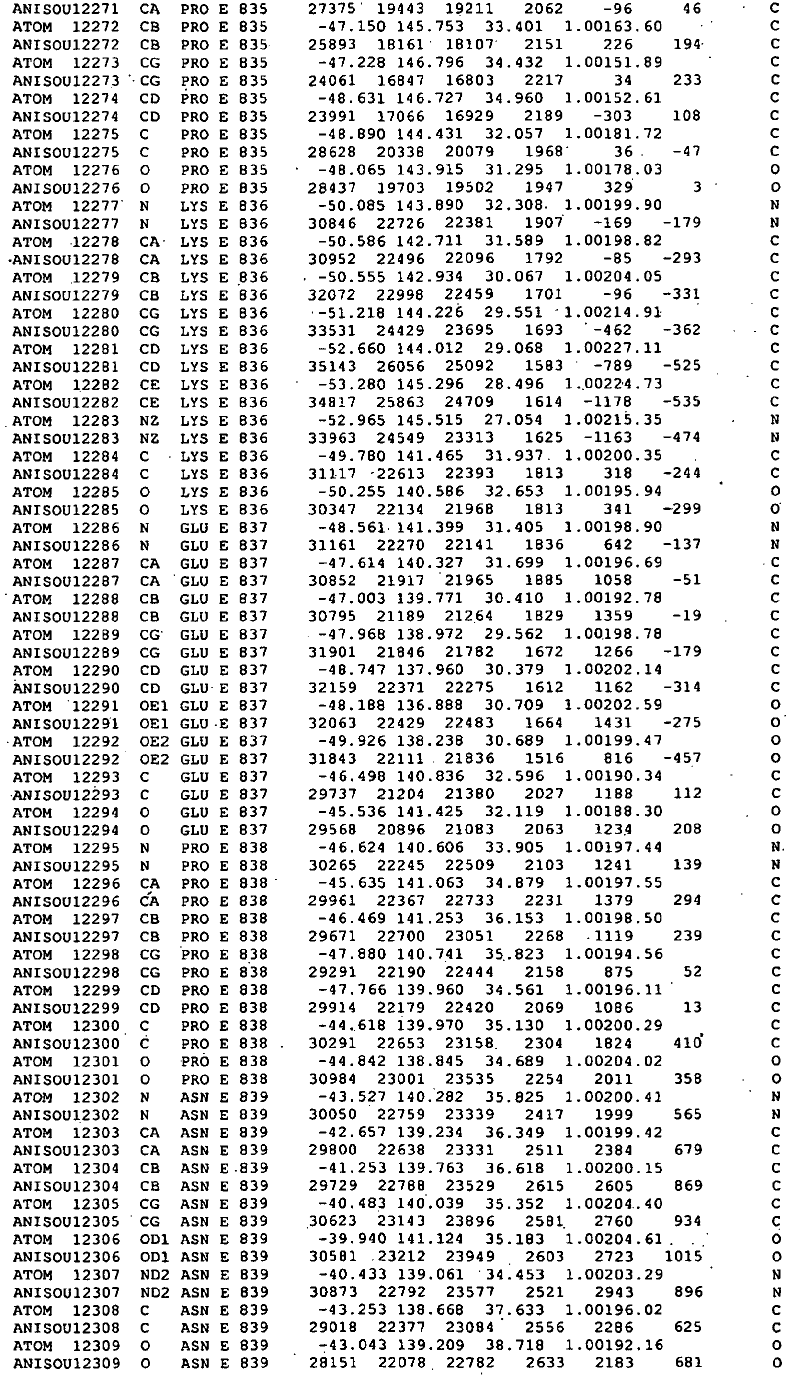

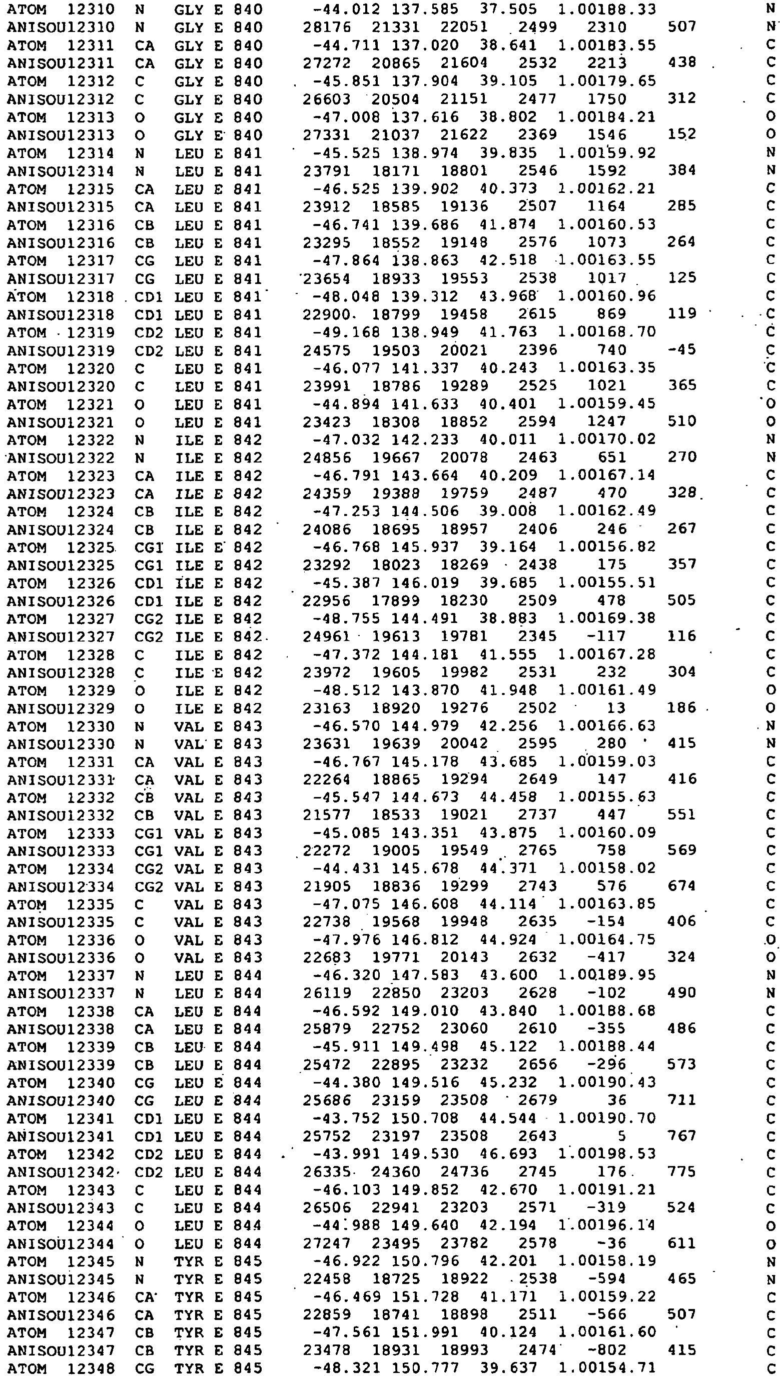

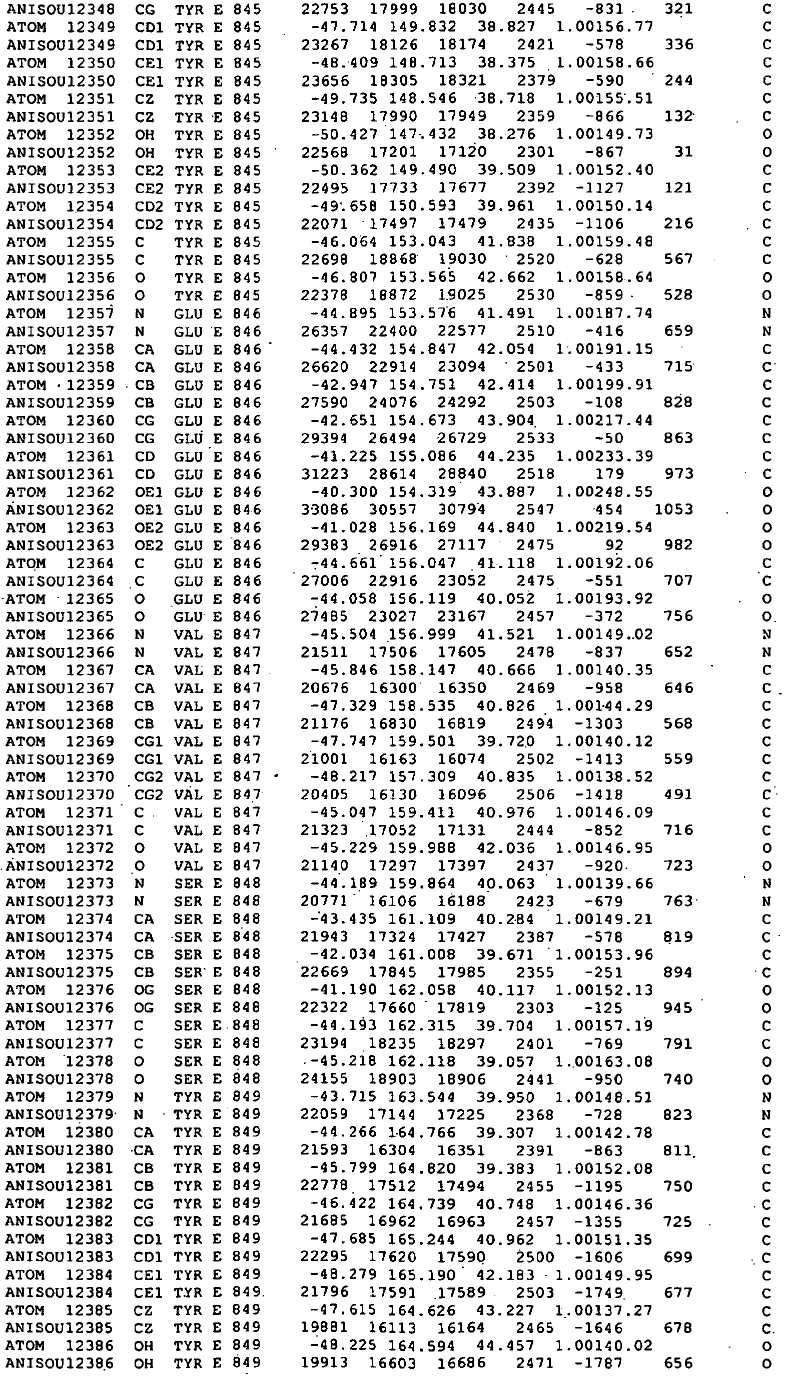

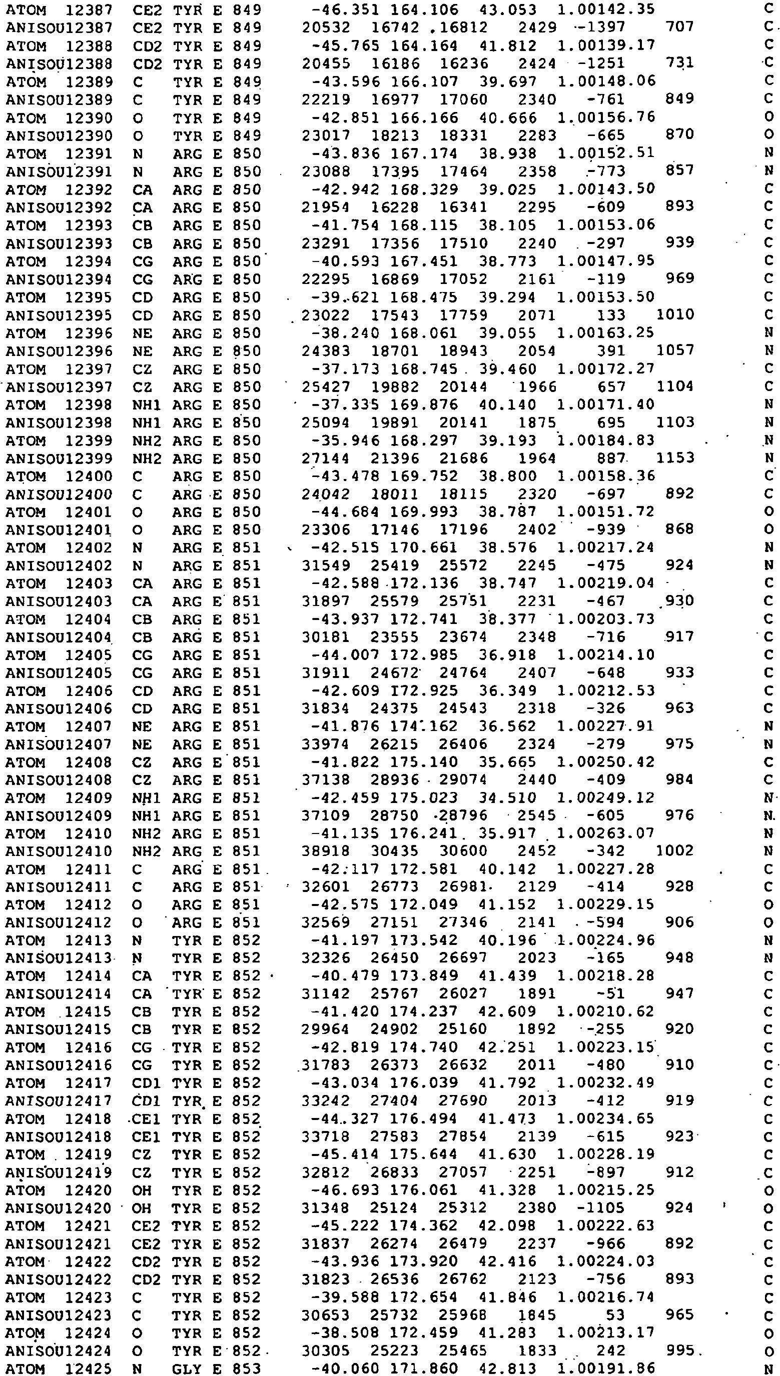

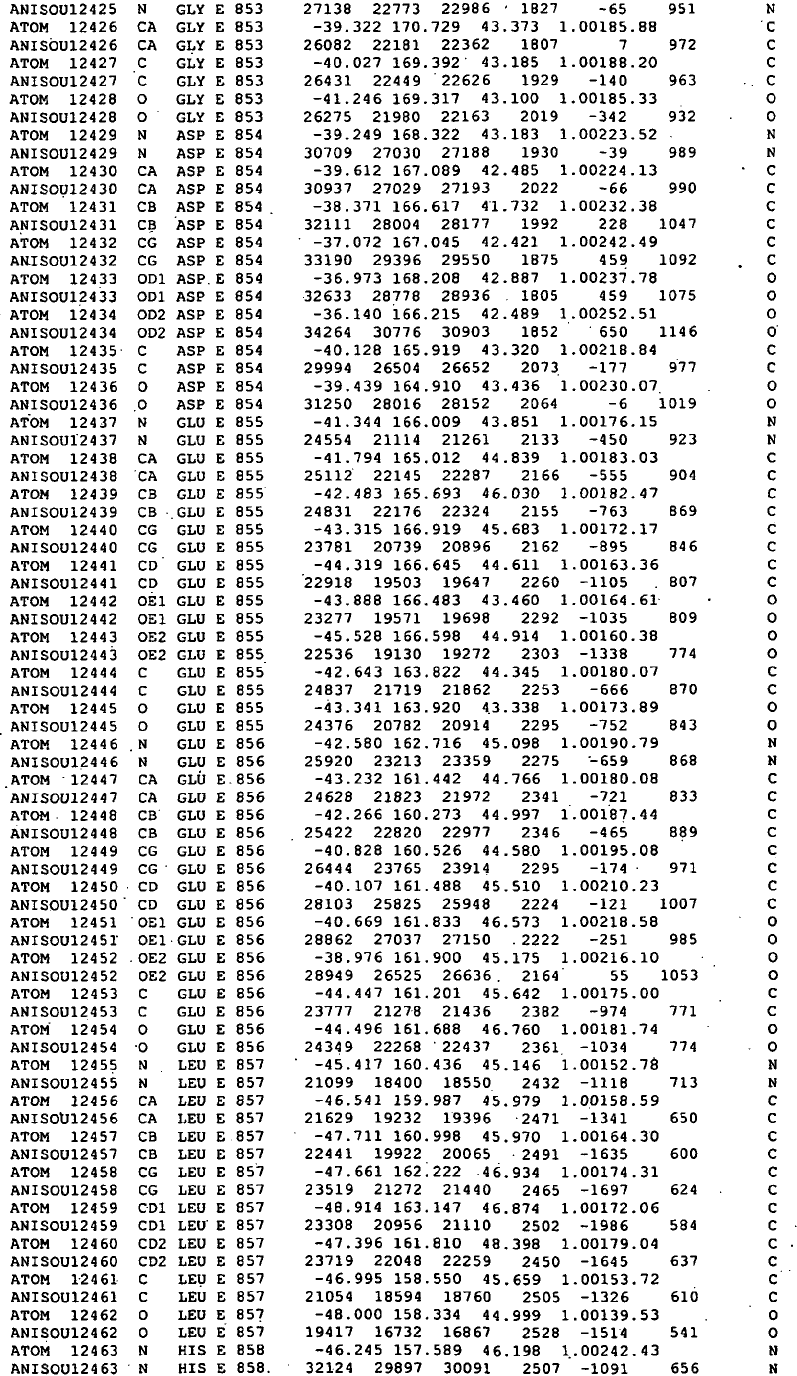

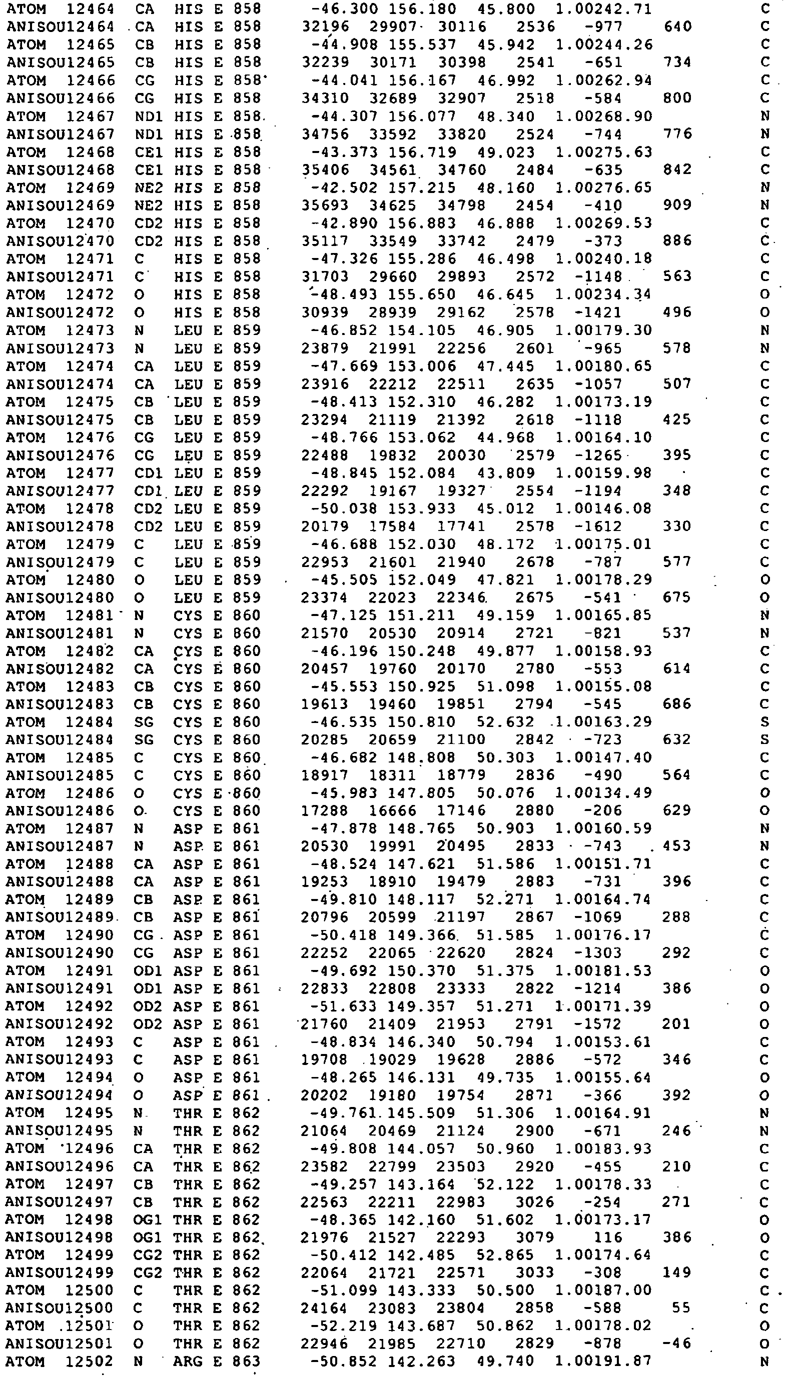

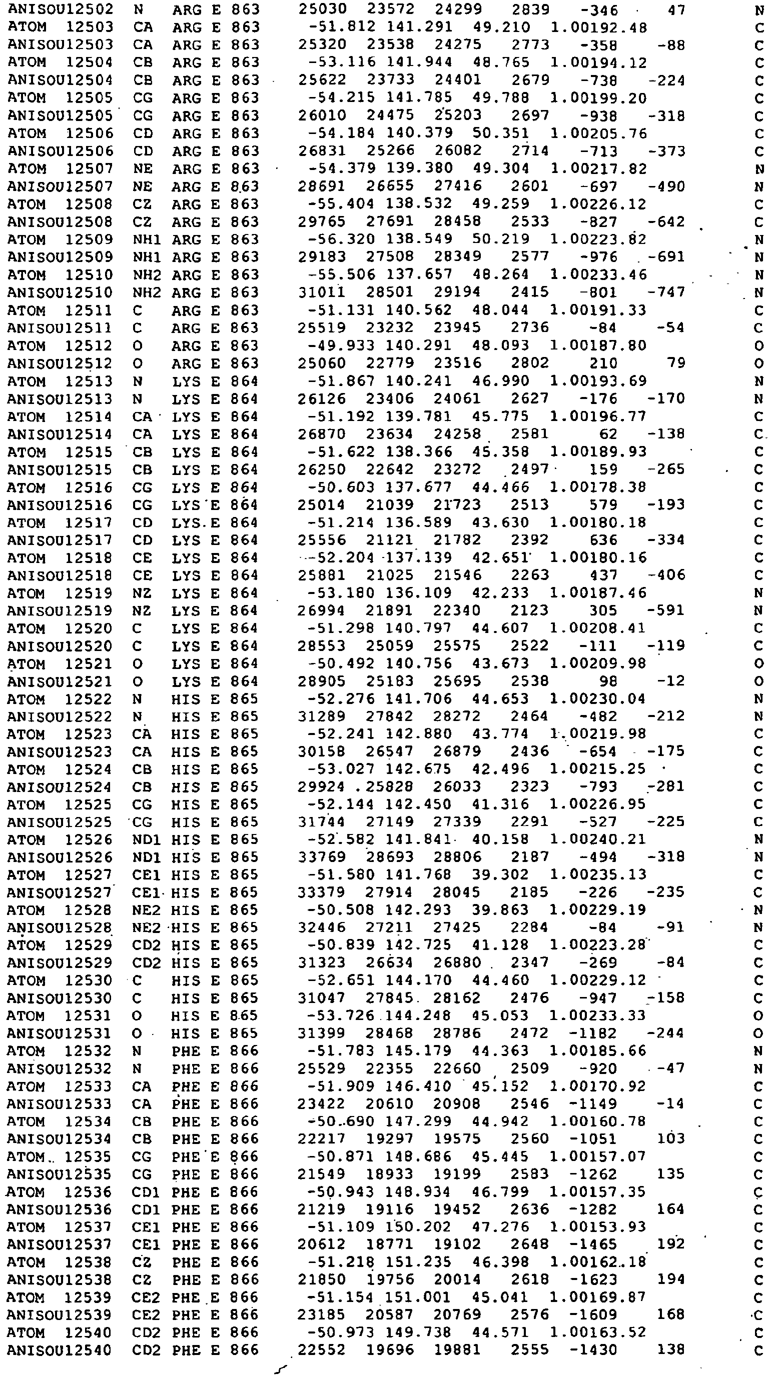

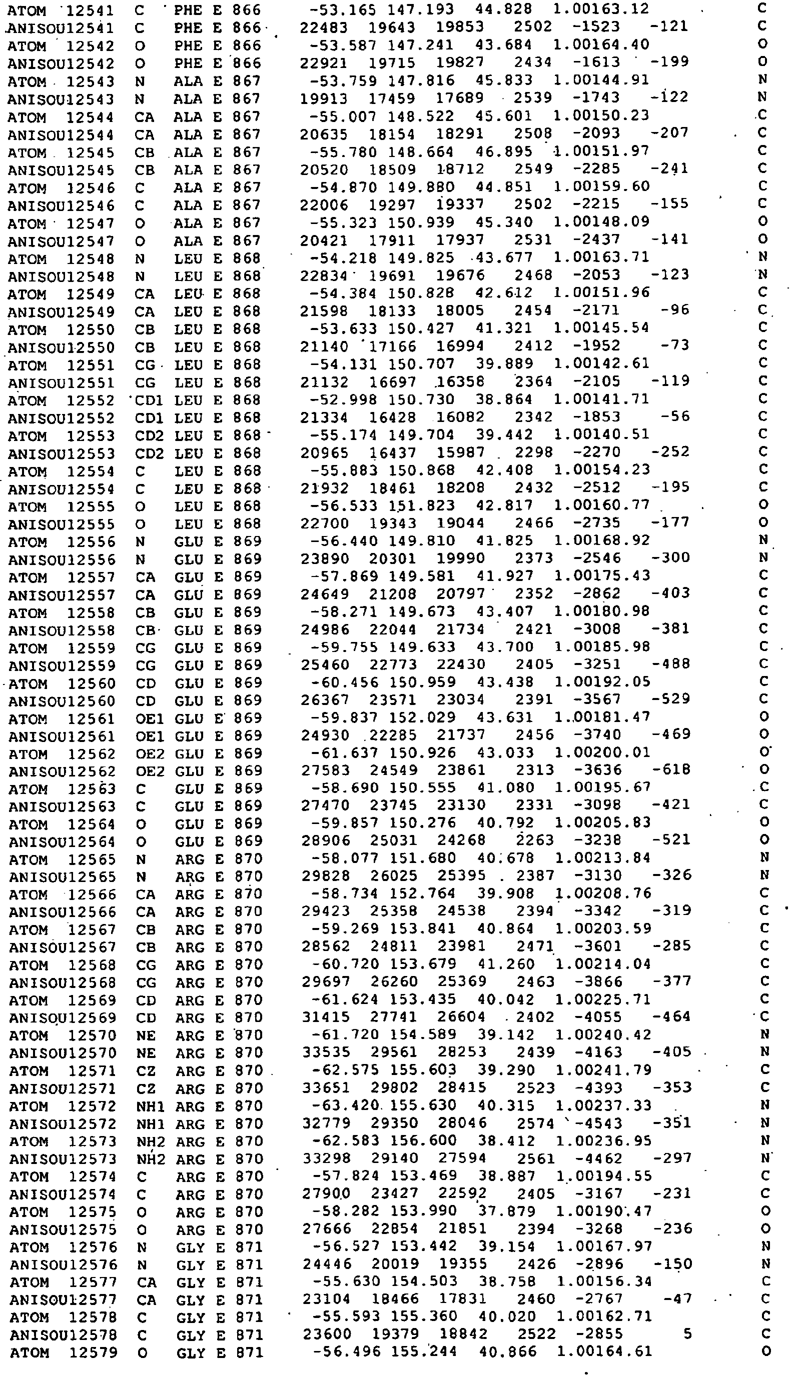

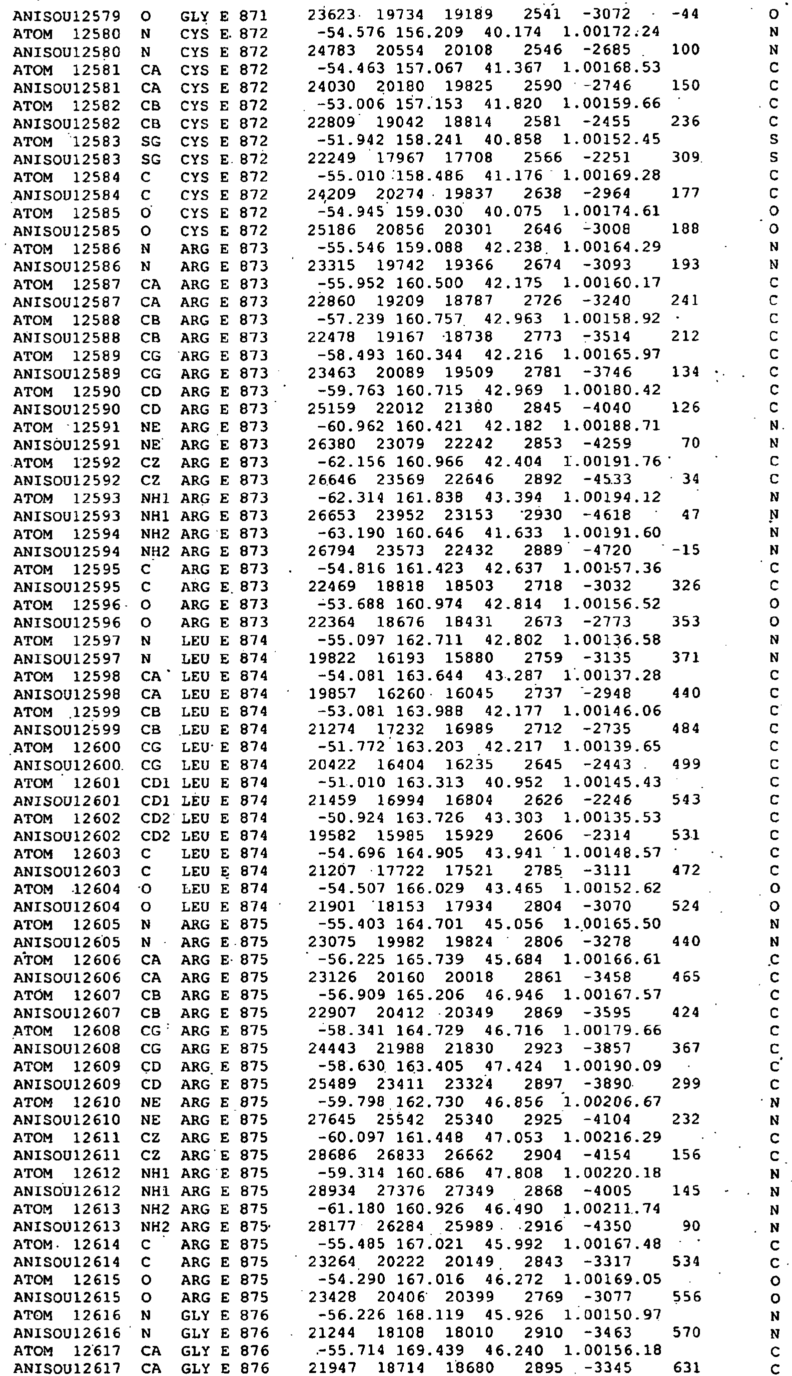

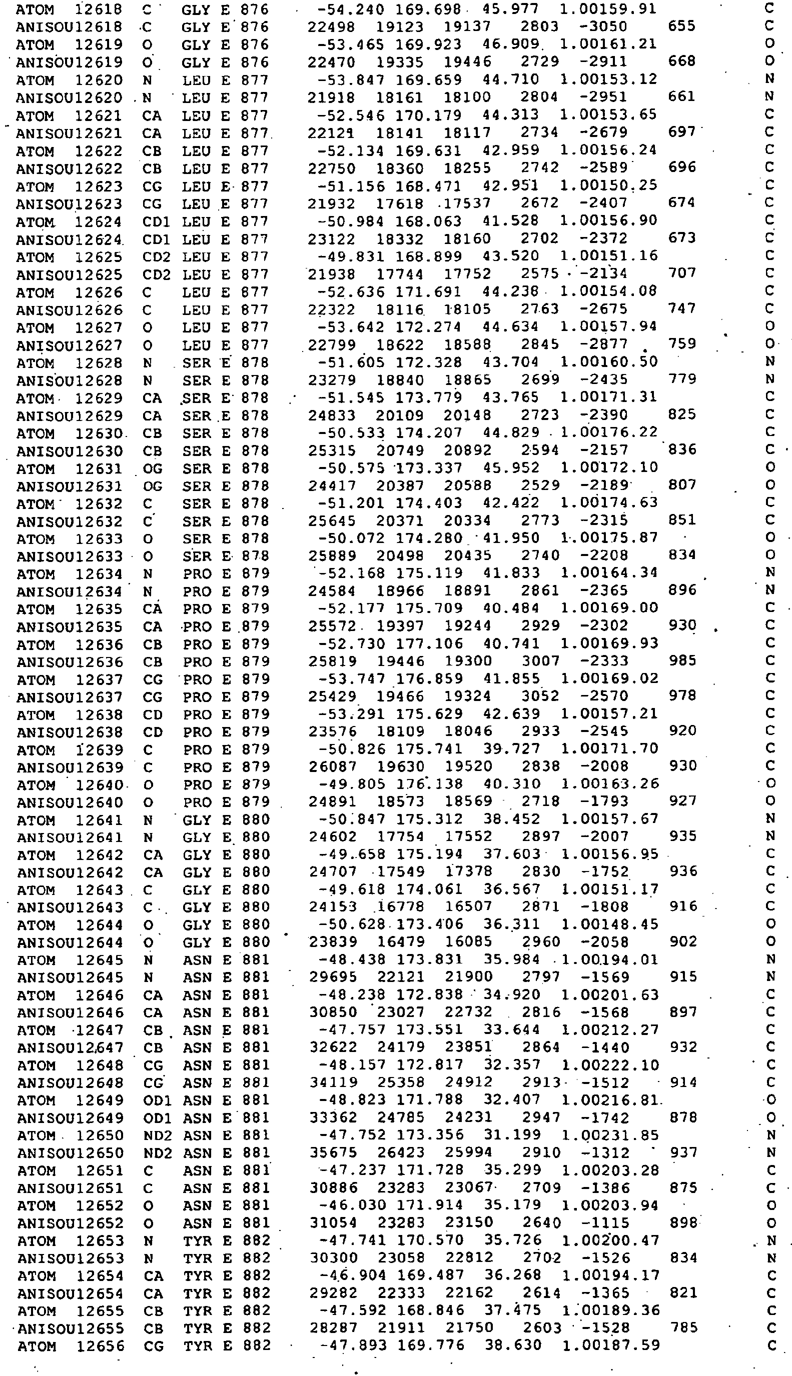

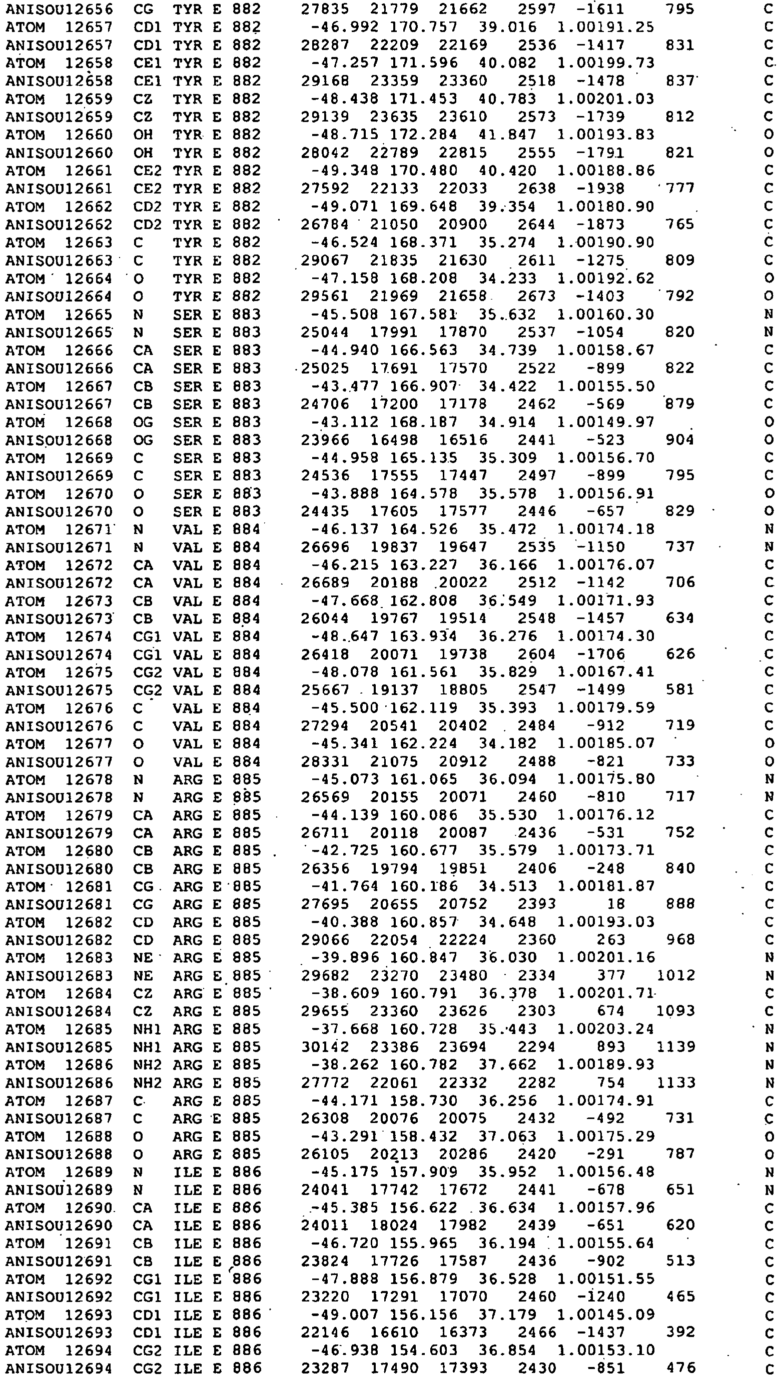

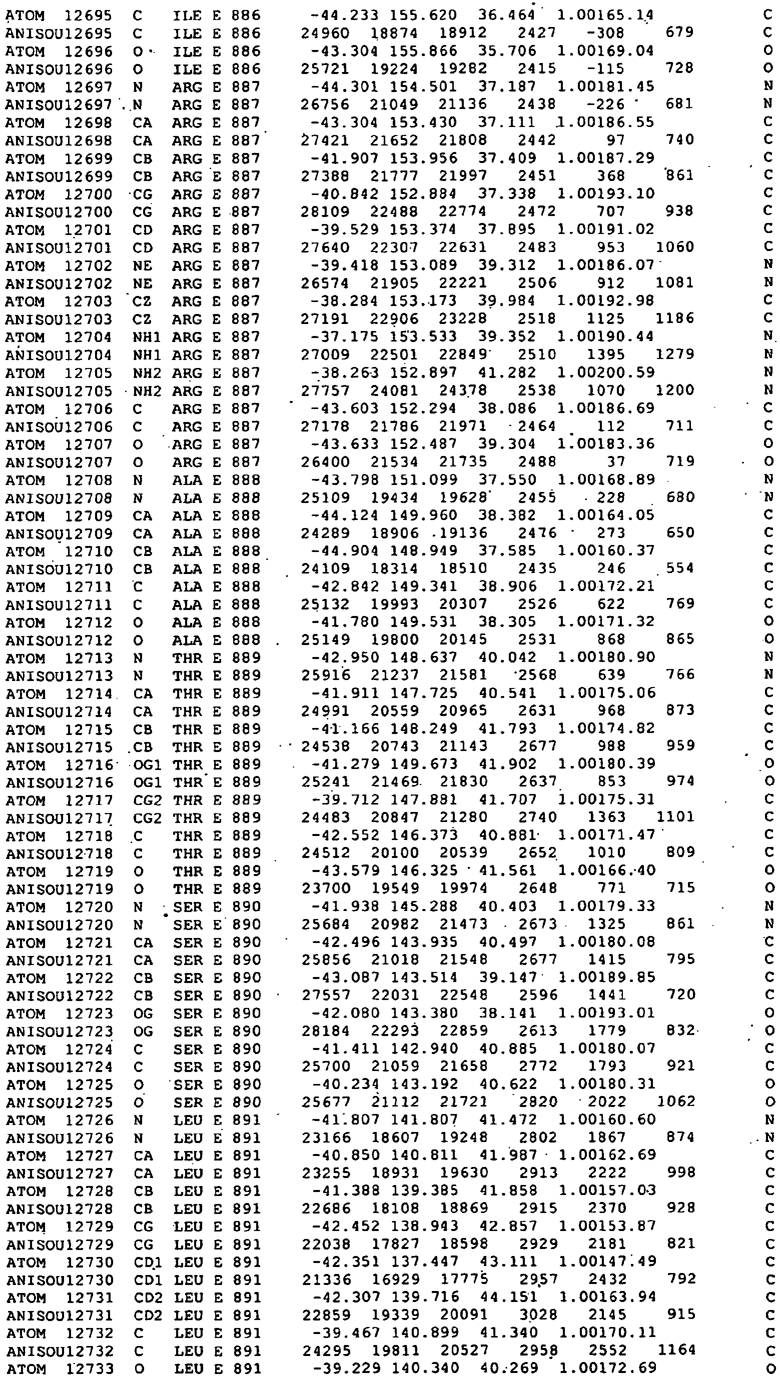

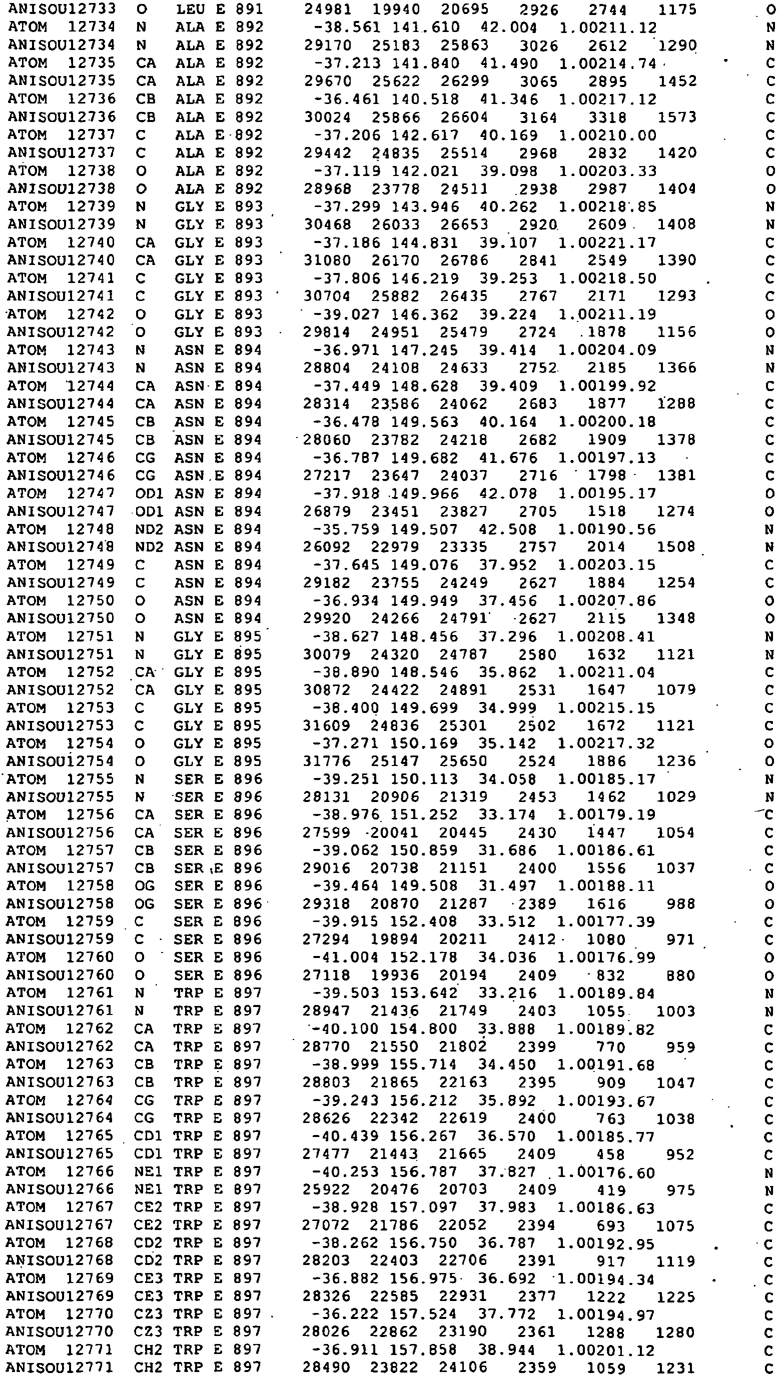

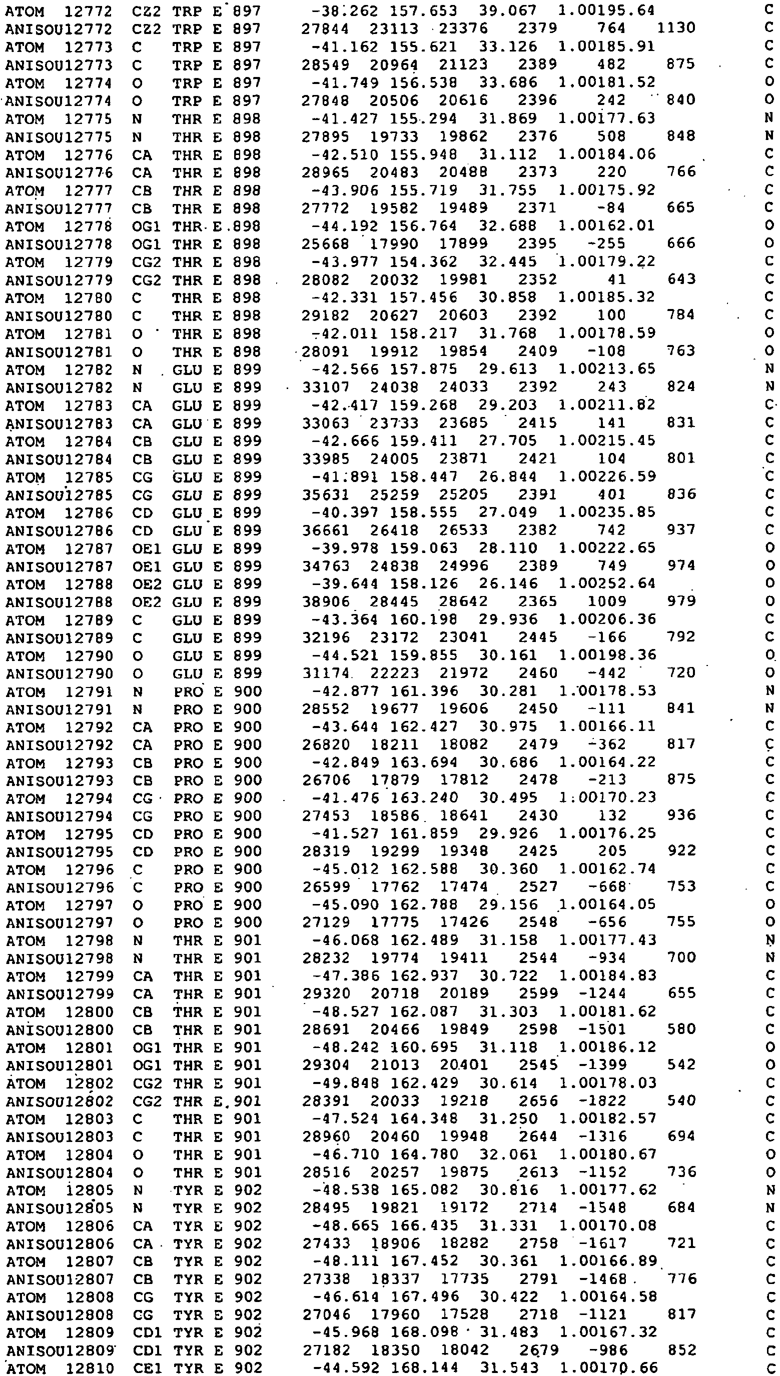

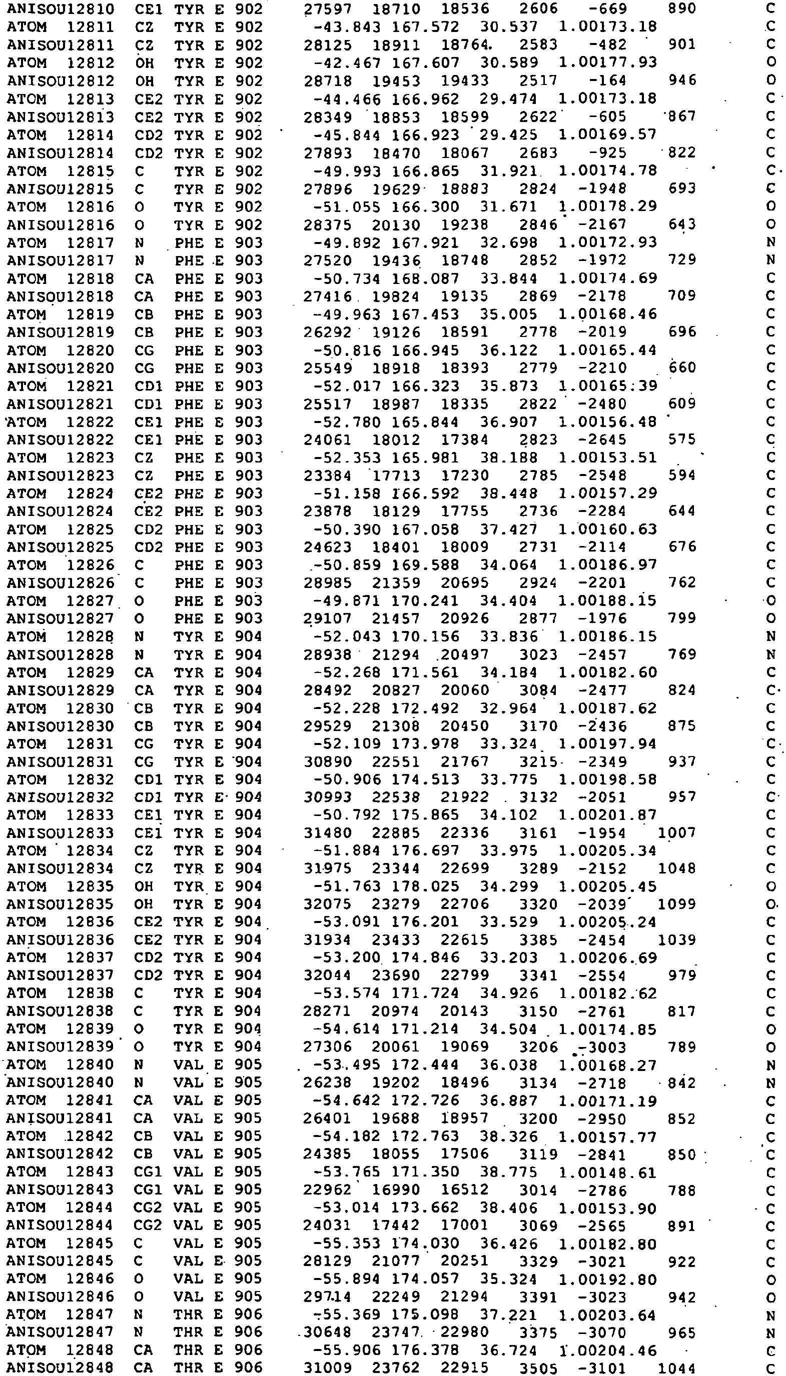

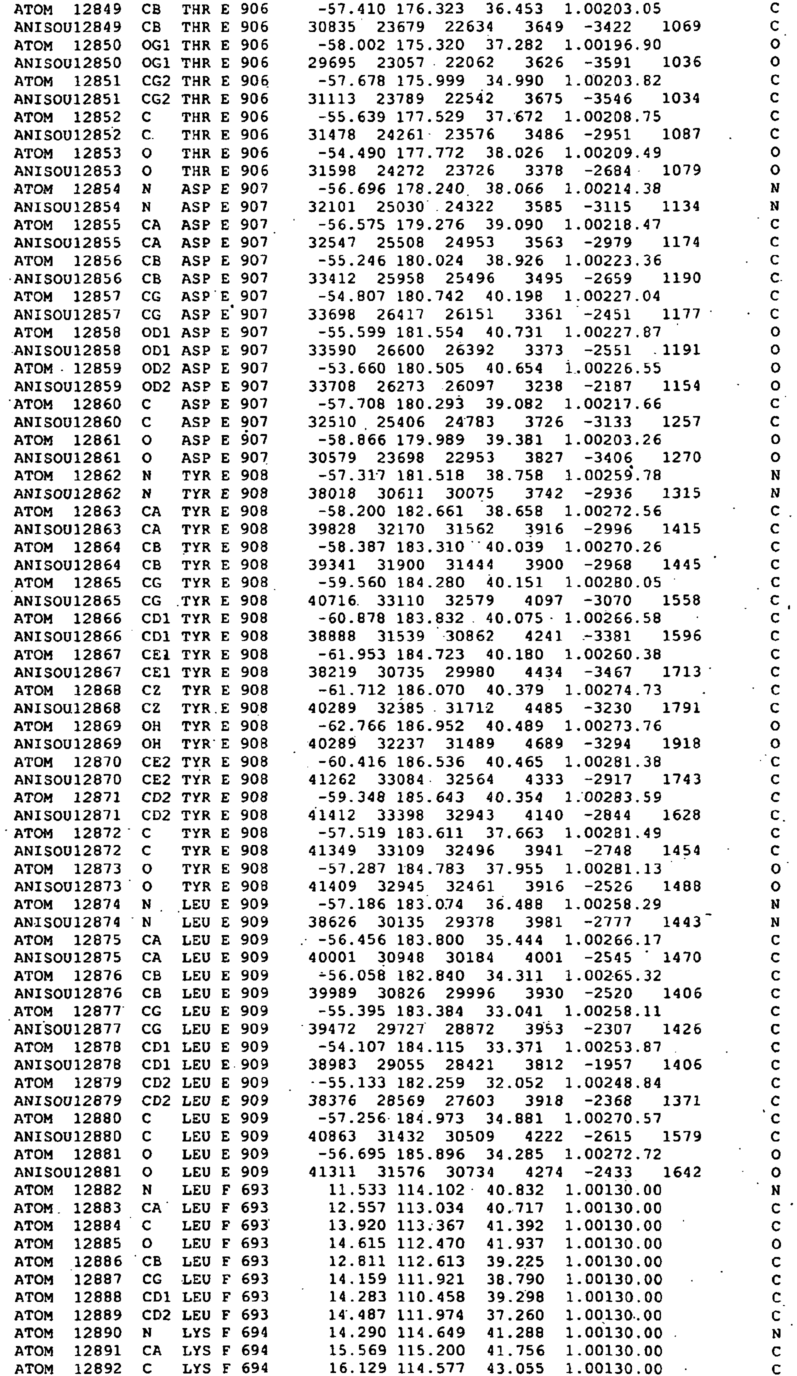

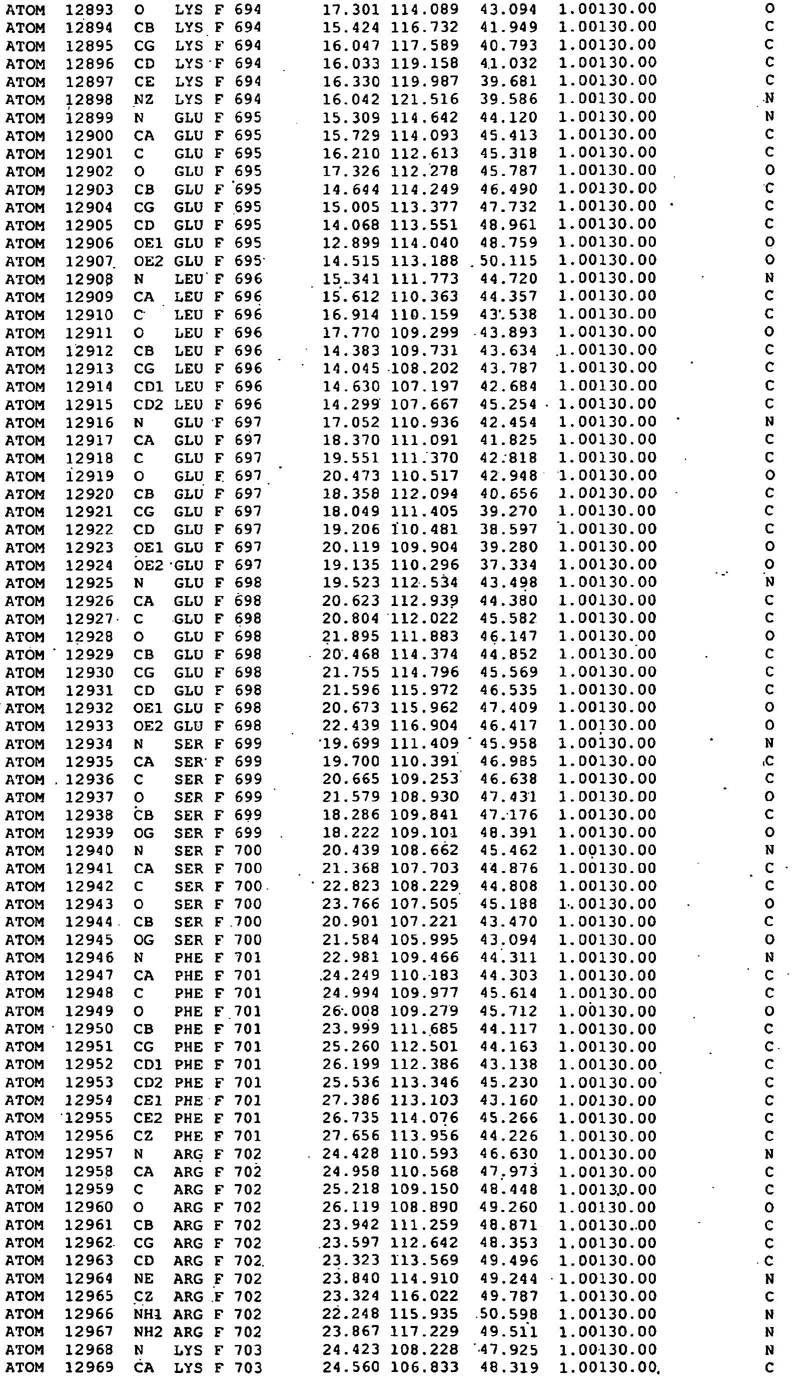

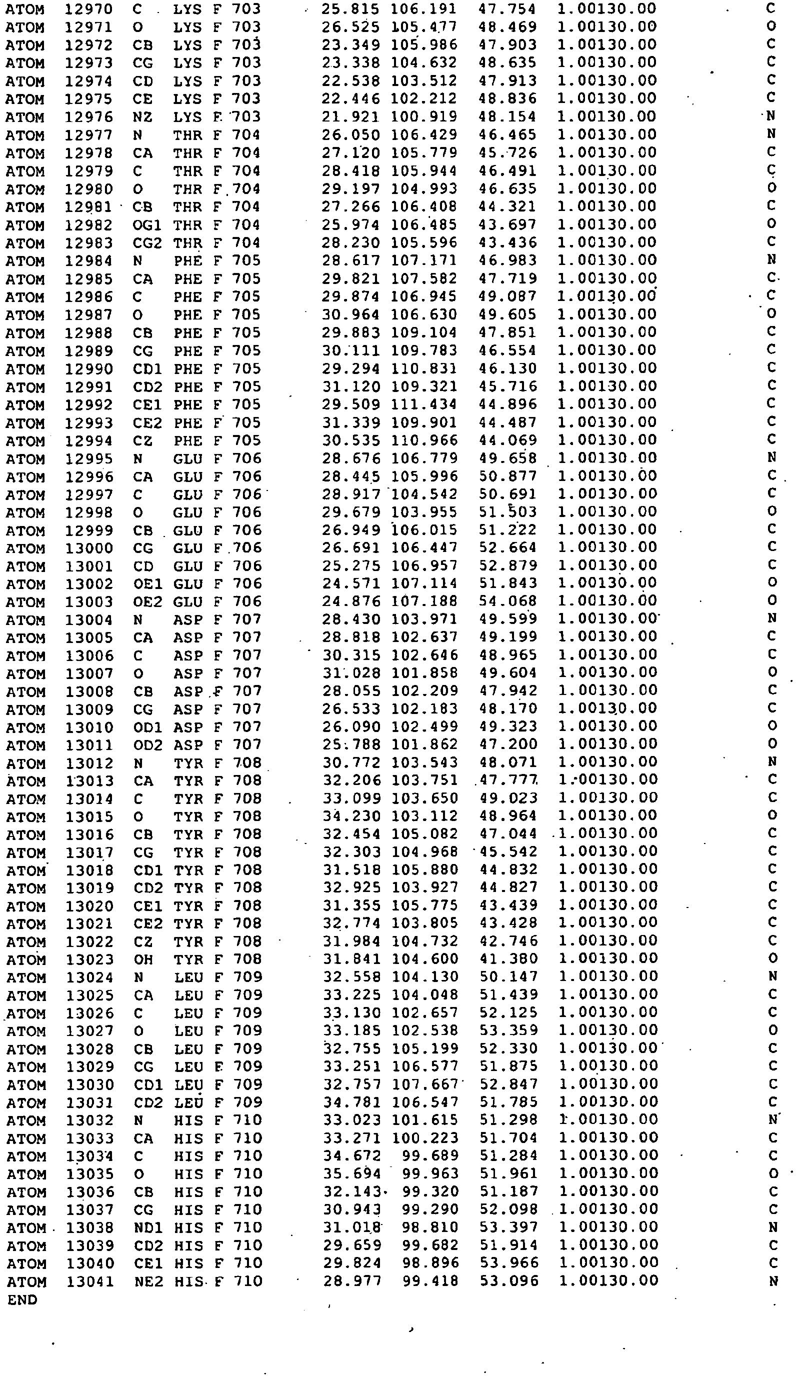

### APPENDIX II : ATOMIC COORDINATES FOR THE MODEL OF THE TERMINAL REGION OF IR α-CHAIN BOUND TO IGF-1R ECTODOM

The structural coordinates for the IR ectodomain containing the C-terminal regio the IR α-chain (Appendix I) were used to place a model of the C-terminal region the IR α-chain in the IGF-1R low affinity binding site for IGF. This model is ori relative to atomic coordinates found in Appendix I and may be used in conjunction with atomic coordinates of Appendixes I and III to VI to design compounds which bind to IR and/or IGF-1R.

| | | | | | | | | | | | |
|---|---|---|---|---|---|---|---|---|---|---|---|
| ATOM | 1 | N | GLU | E | 1 | 48.369 | 108.710 | 21.906 | 1.00 | 0.00 | AAAA |
| ATOM | 5 | CA | GLU | E | 1 | 47.687 | 107.405 | 21.957 | 1.00 | 0.00 | AAAA |
| ATOM | 7 | CB | GLU | E | 1 | 48.614 | 106.206 | 22.178 | 1.00 | 0.00 | AAAA |
| ATOM | 10 | CG | GLU | E | 1 | 49.524 | 106.156 | 23.409 | 1.00 | 0.00 | AAAA |
| ATOM | 13 | CD | GLU | E | 1 | 50.640 | 107.191 | 23.478. | 1.00 | 0.00 | AAAA |
| ATOM | 14 | OE1 | GLU | E | 1 | 51.858 | 106.914 | 23.610 | 1.00 | 0.00 | AAAA |
| ATOM | 15 | OE2 | GLU | E | 1 | 50.351 | 108.398 | 23.355 | 1.00 | 0.00 | AAAA |
| ATOM | 16 | C | GLU | E | 1 | 46.622 | 107.525 | 23.038 | 1.00 | 0.00 | AAAA |
| ATOM | 17 | O | GLU | E | 1 | 46.822 | 108.239 | 24.021 | 1.00 | 0.00 | AAAA |
| ATOM | 18 | N | ILE | E | 2 | 45.449 | 106.915 | 22.892 | 1.00 | 0.00 | AAAA |
| ATOM | 20 | CA | ILE | E | 2 | 44.414 | 106.763 | 23.903 | 1.00 | 0.00 | AAAA |
| ATOM | 22 | CB | ILE | E | 2 | 43.170 | 106.394 | 23.094 | 1.00 | 0.00 | AAAA |
| ATOM | 24 | CG1 | ILE | E | 2 | 43.219 | 105.086 | 22.297 | 1.00 | 0.00 | AAAA |
| ATOM | 27 | CG2 | ILE | E | 2 | 42.590 | 107.531 | 22.243 | 1.00 | 0.00 | AAAA |
| ATOM | 31 | CD1 | ILE | E | 2 | 41.843 | 104.603 | 21.841 | 1.00 | 0.00 | AAAA |
| ATOM | 35 | C | ILE | E | 2 | 44.664 | 105.774 | 25.015 | 1.00 | 0.00 | AAAA |
| ATOM | 36 | O | ILE | E | 2 | 45.549 | 104.910 | 24.974 | 1.00 | 0.00 | AAAA |
| ATOM | 37 | N | CYS | E | 3 | 43.855 | 105.887 | 26.077 | 1.00 | 0.00 | AAAA |
| ATOM | 39 | CA | CYS | E | 3 | 43.628 | 104.854 | 27.066 | 1.00 | 0.00 | AAAA |
| ATOM | 41 | CB | CYS | E | 3 | 44.335 | 105.094 | 28.402 | 1.00 | 0.00 | AAAA |
| ATOM | 44 | SG | CYS | E | 3 | 44.134 | 106.396 | 29.649 | 1.00 | 0.00 | AAAA |
| ATOM | 45 | C | CYS | E | 3 | 42.158 | 104.745 | 27.465 | 1.00 | 0.00 | AAAA |
| ATOM | 46 | O | CYS | E | 3 | 41.347 | 105.663 | 27.481 | 1.00 | 0.00 | AAAA |
| ATOM | 47 | N | GLY | E | 4 | 41.763 | 103.500 | 27.712 | 1.00 | 0.00 | AAAA |
| ATOM | 49 | CA | GLY | E | 4 | 40.380 | 103.159 | 28.012 | 1.00 | 0.00 | AAAA |
| ATOM | 52 | C | GLY | E | 4 | 39.684 | 102.538 | 26.806 | 1.00 | 0.00 | AAAA |
| ATOM | 53 | O | GLY | E | 4 | 40.382 | 102.344 | 25.823 | 1.00 | 0.00 | AAAA |
| ATOM | 54 | N | PRO | E | 5 | 38.427 | 102.117 | 26.958 | 1.00 | 0.00 | AAAA |
| ATOM | 55 | CA | PRO | E | 5 | 37.478 | 102.166 | 28.045 | 1.00 | 0.00 | AAAA |
| ATOM | 57 | CB | PRO | E | 5 | 36.115 | 101.758 | 27.478 | 1.00 | 0.00 | AAAA |
| ATOM | 60 | CG | PRO | E | 5 | 36.453 | 100.995 | 26.205 | 1.00 | 0.00 | AAAA |
| ATOM | 63 | CD | PRO | E | 5 | 37.853 | 101.465 | 25.797 | 1.00 | 0.00 | AAAA |
| ATOM | 66 | C | PRO | E | 5 | 37.807 | 101.258 | 29.216 | 1.00 | 0.00 | AAAA |
| ATOM | 67 | O | PRO | E | 5 | 38.318 | 100.149 | 29.071 | 1.00 | 0.00 | AAAA |
| ATOM | 68 | N | GLY | E | 6 | 37.672 | 101.843 | 30.411 | 1.00 | 0.00 | AAAA |
| ATOM | 70 | CA | GLY | E | 6 | 37.785 | 101.152 | 31.682 | 1.00 | 0.00 | AAAA |
| ATOM | 73 | C | GLY | E | 6 | 38.962 | 101.634 | 32.506 | 1.00 | 0.00 | AAAA |
| ATOM | 74 | O | GLY | E | 6 | 39.774 | 100.885 | 33.053 | 1.00 | 0.00 | AAAA |
| ATOM | 75 | N | ILE | E | 7 | 39.059 | 102.960 | 32.648 | 1.00 | 0.00 | AAAA |
| ATOM | 77 | CA | ILE | E | 7 | 40.037 | 103.690 | 33.424 | 1.00 | 0.00 | AAAA |
| ATOM | 79 | CB | ILE | E | 7 | 40.430 | 104.935 | 32.623 | 1.00 | 0.00 | AAAA |
| ATOM | 81 | CG1 | ILE | E | 7 | 41.054 | 104.602 | 31.262 | 1.00 | 0.00 | AAAA |
| ATOM | 84 | CG2 | ILE | E | 7 | 41.570 | 105.641 | 33.352 | 1.00 | 0.00 | AAAA |
| ATOM | 88 | CD1 | ILE | E | 7 | 41.967 | 103.390 | 31.118 | 1.00 | 0.00 | AAAA |
| ATOM | 92 | C | ILE | E | 7 | 39.429 | 103.925 | 34.794 | 1.00 | 0.00 | AAAA |
| ATOM | 93 | O | ILE | E | 7 | 39.040 | 105.041 | 35.150 | 1.00 | 0.00 | AAAA |
| ATOM | 94 | N | ASP | E | 8 | 39.366 | 102.838 | 35.579 | 1.00 | 0.00 | AAAA |
| ATOM | 96 | CA | ASP | E | 8 | 39.060 | 102.683 | 36.976 | 1.00 | 0.00 | AAAA |
| ATOM | 98 | CB | ASP | E | 8 | 38.265 | 101.376 | 37.095 | 1.00 | 0.00 | AAAA |
| ATOM | 101 | CG | ASP | E | 8 | 37.455 | 101.201 | 38.377 | 1.00 | 0.00 | AAAA |
| ATOM | 102 | OD1 | ASP | E | 8 | 38.149 | 101.314 | 39.407 | 1.00 | 0.00 | AAAA |
| ATOM | 103 | OD2 | ASP | E | 8 | 36.201 | 101.156 | 38.330 | 1.00 | 0.00 | AAAA |
| ATOM | 104 | C | ASP | E | 8 | 40.320 | 102.537 | 37.818 | 1.00 | 0.00 | AAAA |
| ATOM | 105 | O | ASP | E | 8 | 41.092 | 101.599 | 37.695 | 1.00 | 0.00 | AAAA |
| ATOM | 106 | N | ILE | E | 9 | 40.613 | 103.606 | 38.560 | 1.00 | 0.00 | AAAA |
| ATOM | 108 | CA | ILE | E | 9 | 41.780 | 103.766 | 39.404 | 1.00 | 0.00 | AAAA |
| ATOM | 110 | CB | ILE | E | 9 | 42.483 | 105.062 | 38.999 | 1.00 | 0.00 | AAAA |
| ATOM | 112 | CG1 | ILE | E | 9 | 42.589 | 105.279 | 37.494 | 1.00 | 0.00 | AAAA |
| ATOM | 115 | CG2 | ILE | E | 9 | 43.828 | 105.125 | 39.724 | 1.00 | 0.00 | AAAA |
| ATOM | 119 | CD1 | ILE | E | 9 | 43.749 | 106.186 | 37.084 | 1.00 | 0.00 | AAAA |
| ATOM | 123 | C | ILE | E | 9 | 41.230 | 103.711 | 40.821 | 1.00 | 0.00 | AAAA |
| ATOM | 124 | O | ILE | E | 9 | 40.460 | 104.546 | 41.277 | 1.00 | 0.00 | AAAA |
| ATOM | 125 | N | ARG | E | 10 | 41.742 | 102.726 | 41.564 | 1.00 | 0.00 | AAAA |
| ATOM | 127 | CA | ARG | E | 10 | 41.357 | 102.421 | 42.933 | 1.00 | 0.00 | AAAA |
| ATOM | 129 | CB | ARG | E | 10 | 40.335 | 101.280 | 42.911 | 1.00 | 0.00 | AAAA |
| ATOM | 132 | CG | ARG | E | 10 | 38.953 | 101.808 | 42.530 | 1.00 | 0.00 | AAAA |
| ATOM | 135 | CD | ARG | E | 10 | 37.852 | 100.741 | 42.502 | 1.00 | 0.00 | AAAA |
| ATOM | 138 | NE | ARG | E | 10 | 36.783 | 101.075 | 41.552 | 1.00 | 0.00 | AAAA |
| ATOM | 140 | CZ | ARG | E | 10 | 35.457 | 100.941 | 41.567 | 1.00 | 0.00 | AAAA |
| ATOM | 141 | NH1 | ARG | E | 10 | 34.735 | 100.705 | 42.674 | 1.00 | 0.00 | AAAA |
| ATOM | 144 | NH2 | ARG | E | 10 | 34.814 | 101.066 | 40.395 | 1.00 | 0.00 | AAAA |
| ATOM | 147 | C | ARG | E | 10 | 42.585 | 102.057 | 43.751 | 1.00 | 0.00 | AAAA |
| ATOM | 148 | O | ARG | E | 10 | 43.573 | 101.540 | 43.229 | 1.00 | 0.00 | AAAA |
| ATOM | 149 | N | ASN | E | 11 | 42.556 | 102.212 | 45.072 | 1.00 | 0.00 | AAAA |
| ATOM | 151 | CA | ASN | E | 11 | 43.402 | 101.575 | 46.063 | 1.00 | 0.00 | AAAA |
| ATOM | 153 | CB | ASN | E | 11 | 43.473 | 100.051 | 45.930 | 1.00 | 0.00 | AAAA |
| ATOM | 156 | CG | ASN | E | 11 | 43.797 | 99.364 | 47.251 | 1.00 | 0.00 | AAAA |
| ATOM | 157 | OD1 | ASN | E | 11 | 44.928 | 99.121 | 47.667 | 1.00 | 0.00 | AAAA |
| ATOM | 158 | ND2 | ASN | E | 11 | 42.813 | 99.080 | 48.115 | 1.00 | 0.00 | AAAA |
| ATOM | 161 | C | ASN | E | 11 | 44.752 | 102.201 | 46.383 | 1.00 | 0.00 | AAAA |
| ATOM | 162 | O | ASN | E | 11 | 45.093 | 102.409 | 47.546 | 1.00 | 0.00 | AAAA |
| ATOM | 163 | N | ASP | E | 12 | 45.452 | 102.548 | 45.297 | 1.00 | 0.00 | AAAA |
| ATOM | 165 | CA | ASP | E | 12 | 46.855 | 102.923 | 45.286 | 1.00 | 0.00 | AAAA |
| ATOM | 167 | CB | ASP | E | 12 | 47.721 | 101.668 | 45.142 | 1.00 | 0.00 | AAAA |
| ATOM | 170 | CG | ASP | E | 12 | 49.160 | 101.717 | 44.655 | 1.00 | 0.00 | AAAA |
| ATOM | 171 | OD1 | ASP | E | 12 | 49.871 | 102.690 | 44.989 | 1.00 | 0.00 | AAAA |
| ATOM | 172 | OD2 | ASP | E | 12 | 49.686 | 100.742 | 44.060 | 1.00 | 0.00 | AAAA |
| ATOM | 173 | C | ASP | E | 12 | 47.113 | 104.075 | 44.313 | 1.00 | 0.00 | AAAA |
| ATOM | 174 | O | ASP | E | 12 | 46.473 | 104.171 | 43.274 | 1.00 | 0.00 | AAAA |
| ATOM | 175 | N | TYR | E | 13 | 48.061 | 104.949 | 44.645 | 1.00 | 0.00 | AAAA |
| ATOM | 177 | CA | TYR | E | 13 | 48.586 | 106.102 | 43.933 | 1.00 | 0.00 | AAAA |
| ATOM | 179 | CB | TYR | E | 13 | 49.356 | 107.033 | 44.856 | 1.00 | 0.00 | AAAA |
| ATOM | 182 | CG | TYR | E | 13 | 49.829 | 108.342 | 44.265 | 1.00 | 0.00 | AAAA |
| ATOM | 183 | CD1 | TYR | E | 13 | 48.983 | 109.373 | 43.839 | 1.00 | 0.00 | AAAA |
| ATOM | 185 | CD2 | TYR | E | 13 | 51.190 | 108.492 | 43.951 | 1.00 | 0.00 | AAAA |
| ATOM | 187 | CE1 | TYR | E | 13 | 49.466 | 110.583 | 43.329 | 1.00 | 0.00 | AAAA |
| ATOM | 189 | CE2 | TYR | E | 13 | 51.700 | 109.605 | 43.282 | 1.00 | 0.00 | AAAA |
| ATOM | 191 | CZ | TYR | E | 13 | 50.814 | 110.641 | 42.959 | 1.00 | 0.00 | AAAA |
| ATOM | 192 | OH | TYR | E | 13 | 51.240 | 111.688 | 42.207 | 1.00 | 0.00 | AAAA |
| ATOM | 194 | C | TYR | E | 13 | 49.403 | 105.710 | 42.698 | 1.00 | 0.00 | AAAA |
| ATOM | 195 | O | TYR | E | 13 | 49.437 | 106.449 | 41.716 | 1.00 | 0.00 | AAAA |
| ATOM | 196 | N | GLN | E | 14 | 50.065 | 104.555 | 42.653 | 1.00 | 0.00 | AAAA |
| ATOM | 198 | CA | GLN | E | 14 | 50.874 | 104.152 | 41.513 | 1.00 | 0.00 | AAAA |
| ATOM | 200 | CB | GLN | E | 14 | 51.973 | 103.215 | 42.001 | 1.00 | 0.00 | AAAA |
| ATOM | 203 | CG | GLN | E | 14 | 52.947 | 103.666 | 43.102 | 1.00 | 0.00 | AAAA |
| ATOM | 206 | CD | GLN | E | 14 | 53.589 | 105.011 | 42.819 | 1.00 | 0.00 | AAAA |
| ATOM | 207 | OE1 | GLN | E | 14 | 53.891 | 105.461 | 41.721 | 1.00 | 0.00 | AAAA |
| ATOM | 208 | NE2 | GLN | E | 14 | 53.902 | 105.697 | 43.929 | 1.00 | 0.00 | AAAA |
| ATOM | 211 | C | GLN | E | 14 | 50.132 | 103.588 | 40.315 | 1.00 | 0.00 | AAAA |
| ATOM | 212 | O | GLN | E | 14 | 50.621 | 103.517 | 39.181 | 1.00 | 0.00 | AAAA |
| ATOM | 213 | N | GLN | E | 15 | 48.818 | 103.496 | 40.530 | 1.00 | 0.00 | AAAA |
| ATOM | 215 | CA | GLN | E | 15 | 47.818 | 103.134 | 39.548 | 1.00 | 0.00 | AAAA |
| ATOM | 217 | CB | GLN | E | 15 | 46.506 | 102.754 | 40.239 | 1.00 | 0.00 | AAAA |
| ATOM | 220 | CG | GLN | E | 15 | 46.496 | 101.598 | 41.244 | 1.00 | 0.00 | AAAA |
| ATOM | 223 | CD | GLN | E | 15 | 46.641 | 100.211 | 40.639 | 1.00 | 0.00 | AAAA |
| ATOM | 224 | OE1 | GLN | E | 15 | 46.494 | 100.039 | 39.431 | 1.00 | 0.00 | AAAA |
| ATOM | 225 | NE2 | GLN | E | 15 | 46.968 | 99.216 | 41.470 | 1.00 | 0.00 | AAAA |
| ATOM | 228 | C | GLN | E | 15 | 47.532 | 104.227 | 38.537 | 1.00 | 0.00 | AAAA |
| ATOM | 229 | O | GLN | E | 15 | 47.145 | 103.985 | 37.384 | 1.00 | 0.00 | AAAA |
| ATOM | 230 | N | LEU | E | 16 | 47.785 | 105.475 | 38.933 | 1.00 | 0.00 | AAAA |
| ATOM | 232 | CA | LEU | E | 16 | 47.708 | 106.700 | 38.167 | 1.00 | 0.00 | AAAA |
| ATOM | 234 | CB | LEU | E | 16 | 47.877 | 107.951 | 39.035 | 1.00 | 0.00 | AAAA |
| ATOM | 237 | CG | LEU | E | 16 | 46.632 | 108.308 | 39.831 | 1.00 | 0.00 | AAAA |
| ATOM | 239 | CD1 | LEU | E | 16 | 46.326 | 107.440 | 41.048 | 1.00 | 0.00 | AAAA |
| ATOM | 243 | CD2 | LEU | E | 16 | 46.667 | 109.727 | 40.418 | 1.00 | 0.00 | AAAA |
| ATOM | 247 | C | LEU | E | 16 | 48.760 | 106.768 | 37.067 | 1.00 | 0.00 | AAAA |
| ATOM | 248 | O | LEU | E | 16 | 48.548 | 107.4.63 | 36.071 | 1.00 | 0.00 | AAAA |
| ATOM | 249 | N | LYS | E | 17 | 49.730 | 105.848 | 37.041 | 1.00 | 0.00 | AAAA |
| ATOM | 251 | CA | LYS | E | 17 | 50.864 | 105.799 | 36.145 | 1.00 | 0.00 | AAAA |
| ATOM | 253 | CB | LYS | E | 17 | 51.870 | 104.773 | 36.657 | 1.00 | 0.00 | AAAA |
| ATOM | 256 | CG | LYS | E | 17 | 53.272 | 104.936 | 36.056 | 1.00 | 0.00 | AAAA |
| ATOM | 259 | CD | LYS | E | 17 | 54.271 | 103.997 | 36.725 | 1.00 | 0.00 | AAAA |
| ATOM | 262 | CE | LYS | E | 17 | 55.671 | 104.446 | 36.288 | 1.00 | 0.00 | AAAA |
| ATOM | 265 | NZ | LYS | E | 17 | 56.661 | 103.454 | 36.736 | 1.00 | 0.00 | AAAA |
| ATOM | 269 | C | LYS | E | 17 | 50.409 | 105.285 | 34.782 | 1.00 | 0.00 | AAAA |
| ATOM | 270. | O | LYS | E | 17 | 51.156 | 105.480 | 33.827 | 1.00 | 0.00 | AAAA |
| ATOM | 271 | N | ARG | E | 18 | 49.256 | 104.619 | 34.661 | 1.00 | 0.00 | AAAA |
| ATOM | 273 | CA | ARG | E | 18 | 48.700 | 104.289 | 33.368 | 1.00 | 0.00 | AAAA |
| ATOM | 275 | CB | ARG | E | 18 | 47.455 | 103.430 | 33.636 | 1.00 | 0.00 | AAAA |
| ATOM | 278 | CG | ARG | E | 18 | 46.763 | 102.898 | 32.386 | 1.00 | 0.00 | AAAA |
| ATOM | 281 | CD | ARG | E | 18 | 45.562 | 102.026 | 32.735 | 1.00 | 0.00 | AAAA |
| ATOM | 284 | NE | ARG | E | 18 | 44.962 | 101.502 | 31.506 | 1.00 | 0.00 | AAAA |
| ATOM | 286 | CZ | ARG | E | 18 | 43.831 | 100.778 | 31.432 | 1.00 | 0.00 | AAAA |
| ATOM | 287 | NH1 | ARG | E | 18 | 43.110 | 100.509 | 32.523 | 1.00 | 0.00 | AAAA |
| ATOM | 290 | NH2 | ARG | E | 18 | 43.312 | 100.197 | 30.344 | 1.00 | 0.00 | AAAA |
| ATOM | 293 | C | ARG | E | 18 | 48.357 | 105.485 | 32.483 | 1.00 | 0.00 | AAAA |
| ATOM | 294 | O | ARG | E | 18 | 48.161 | 105.294 | 31.282 | 1.00 | 0.00 | AAAA |
| ATOM | 295 | N | LEU | E | 19 | 48.341 | 106.684 | 33.061 | 1.00 | 0.00 | AAAA |
| ATOM | 297 | CA | LEU | E | 19 | 48.010 | 107.944 | 32.406 | 1.00 | 0.00 | AAAA |
| ATOM | 299 | CB | LEU | E | 19 | 47.447 | 108.921 | 33.446 | 1.00 | 0.00 | AAAA |
| ATOM | 302 | CG | LEU | E | 19 | 46.013 | 108.601 | 33.896 | 1.00 | 0.00 | AAAA |
| ATOM | 304 | CD1 | LEU | E | 19 | 45.748 | 109.269 | 35.249 | 1.00 | 0.00 | AAAA |
| ATOM | 308 | CD2 | LEU | E | 19 | 44.951 | 109.166 | 32.959 | 1.00 | 0.00 | AAAA |
| ATOM | 312 | C | LEU | E | 19 | 49.212 | 108.517 | 31.672 | 1.00 | 0.00 | AAAA |
| ATOM | 313 | O | LEU | E | 19 | 49.085 | 109.560 | 31.031 | 1.00 | 0.00 | AAAA |
| ATOM | 314 | N | GLU | E | 20 | 50.403 | 107.928 | 31.794 | 1.00 | 0.00 | AAAA |
| ATOM | 316 | CA | GLU | E | 20 | 51.651 | 108.348 | 31.192 | 1.00 | 0.00 | AAAA |
| ATOM | 318 | CB | GLU | E | 20 | 52.745 | 107.371 | 31.610 | 1.00 | 0.00 | AAAA |
| ATOM | 321 | CG | GLU | E | 20 | 53.539 | 107.501 | 32.916 | 1.00 | 0.00 | AAAA |
| ATOM | 324 | CD | GLU | E | 20 | 54.372 | 108.769 | 33.006 | 1.00 | 0.00 | AAAA |
| ATOM | 325 | OE1 | GLU | E | 20 | 54.355 | 109.445 | 34.059 | 1.00 | 0.00 | AAAA |
| ATOM | 326 | OE2 | GLU | E | 20 | 55.035 | 109.176 | 32.027 | 1.00 | 0.00 | AAAA |
| ATOM | 327 | C | GLU | E | 20 | 51.456 | 108.426 | 29.689 | 1.00 | 0.00 | AAAA |
| ATOM | 328 | O | GLU | E | 20 | 51.677 | 109.471 | 29.080 | 1.00 | 0.00 | AAAA |
| ATOM | 329 | N | ASN | E | 21 | 51.076 | 107.367 | 28.968 | 1.00 | 0.00 | AAAA |
| ATOM | 331 | CA | ASN | E | 21 | 50.930 | 107.316 | 27.531 | 1.00 | 0.00 | AAAA |
| ATOM | 333 | CB | ASN | E | 21 | 51.753 | 106.167 | 26.954 | 1.00 | 0.00 | AAAA |
| ATOM | 336 | CG | ASN | E | 21 | 53.224 | 106.482 | 26.729 | 1.00 | 0.00 | AAAA |
| ATOM | 337 | OD1 | ASN | E | 21 | 54.100 | 106.074 | 27.497 | 1.00 | 0.00 | AAAA |
| ATOM | 338 | ND2 | ASN | E | 21 | 53.590 | 107.156 | 25.645 | 1.00 | 0.00 | AAAA |
| ATOM | 341 | C | ASN | E | 21 | 49.437 | 107.120 | 27.285 | 1.00 | 0.00 | AAAA |
| ATOM | 342 | O | ASN | E | 21 | 48.981 | 105.988 | 27.155 | 1.00 | 0.00 | AAAA |
| ATOM | 343 | N | CYS | E | 22 | 48.721 | 108.246 | 27.337 | 1.00 | 0.00 | AAAA |
| ATOM | 345 | CA | CYS | E | 22 | 47.275 | 108.292 | 27.202 | 1.00 | 0.00 | AAAA |
| ATOM | 347 | CB | CYS | E | 22 | 46.625 | 107.777 | 28.477 | 1.00 | 0.00 | AAAA |
| ATOM | 350 | SG | CYS | E | 22 | 44.839 | 108.007 | 28.617 | 1.00 | 0.00 | AAAA |
| ATOM | 351 | C | CYS | E | 22 | 46.975 | 109.775 | 27.049 | 1.00 | 0.00 | AAAA |
| ATOM | 352 | O | CYS | E | 22 | 46.857 | 110.574 | 27.976 | 1.00 | 0.00 | AAAA |
| ATOM | 353 | N | THR | E | 23 | 46.899 | 110.298 | 25.824 | 1.00 | 0.00 | AAAA |
| ATOM | 355 | CA | THR | E | 23 | 46.560 | 111.652 | 25.452 | 1.00 | 0.00 | AAAA |
| ATOM | 357 | CB | THR | E | 23 | 46.992 | 111.814 | 23.990 | 1.00 | 0.00 | AAAA |
| ATOM | 359 | OG1 | THR | E | 23 | 46.749 | 110.794 | 23.045 | 1.00 | 0.00 | AAAA |
| ATOM | 361 | CG2 | THR | E | 23 | 48.527 | 111.851 | 24.017 | 1.00 | 0.00 | AAAA |
| ATOM | 365 | C | THR | E | 23 | 45.103 | 112.029 | 25.620 | 1.00 | 0.00 | AAAA |
| ATOM | 366 | O | THR | E | 23 | 44.679 | 113.143 | 25.911 | 1.00 | 0.00 | AAAA |
| ATOM | 367 | N | VAL | E | 24 | 44.225 | 111.035 | 25.434 | 1.00 | 0.00 | AAAA |
| ATOM | 369 | CA | VAL | E | 24 | 42.782 | 111.068 | 25.568 | 1.00 | 0.00 | AAAA |
| ATOM | 371 | CB | VAL | E | 24 | 42.053 | 111.387 | 24.266 | 1.00 | 0.00 | AAAA |
| ATOM | 373 | CG1 | VAL | E | 24 | 40.558 | 111.589 | 24.509 | 1.00 | 0.00 | AAAA |
| ATOM | 377 | CG2 | VAL | E | 24 | 42.564 | 112.668 | 23.607 | 1.00 | 0.00 | AAAA |
| ATOM | 381 | C | VAL | E | 24 | 42.256 | 109.837 | 26.301 | 1.00 | 0.00 | AAAA |
| ATOM | 382 | O | VAL | E | 24 | 42.586 | 108.692 | 25.992 | 1.00 | 0.00 | AAAA |
| ATOM | 383 | N | ILE | E | 25 | 41.517 | 110:082 | 27.390 | 1.00 | 0.00 | AAAA |
| ATOM | 385 | CA | ILE | E | 25 | 40.812 | 108.991 | 28.033 | 1.00 | 0.00 | AAAA |
| ATOM | 387 | CB | ILE | E | 25 | 40.487 | 109.269 | 29.503 | 1.00 | 0.00 | AAAA |
| ATOM | 389 | CG1 | ILE | E | 25 | 41.776 | 109.686 | 30.204 | 1.00 | 0.00 | AAAA |
| ATOM | 392 | CG2 | ILE | E | 25 | 39.918 | 108.081 | 30.279 | 1.00 | 0.00 | AAAA |
| ATOM | 396 | CD1 | ILE | E | 25 | 41.751 | 109.878 | 31.713 | 1.00 | 0.00 | AAAA |
| ATOM | 400 | C | ILE | E | 25 | 39.522 | 108.802 | 27.247 | 1.00 | 0.00 | AAAA |
| ATOM | 401 | O | ILE | E | 25 | 38.563 | 109.572 | 27.201 | 1.00 | 0.00 | AAAA |
| ATOM | 402 | N | GLU | E | 26 | 39.428 | 107.711 | 26.490 | 1.00 | 0.00 | AAAA |
| ATOM | 404 | CA | GLU | E | 26 | 38.404 | 107.257 | 25.565 | 1.00 | 0.00 | AAAA |
| ATOM | 406 | CB | GLU | E | 26 | 38.931 | 106.345 | 24.461 | 1.00 | 0.00 | AAAA |
| ATOM | 409 | CG | GLU | E | 26 | 37.962 | 105.709 | 23.462 | 1.00 | 0.00 | AAAA |
| ATOM | 412 | CD | GLU | E | 26 | 36.840 | 106.529 | 22.822 | 1.00 | 0.00 | AAAA |
| ATOM | 413 | OE1 | GLU | E | 26 | 35.723 | 106.017 | 22.607 | 1.00 | 0.00 | AAAA |
| ATOM | 414 | OE2 | GLU | E | 26 | 37.077 | 107.731 | 22.580 | 1.00 | 0.00 | AAAA |
| ATOM | 415 | C | GLU | E | 26 | 37.559 | 106.459 | 26.546 | 1.00 | 0.00 | AAAA |
| ATOM | 416 | O | GLU | E | 26 | 37.672 | 105.231 | 26.633 | 1.00 | 0.00 | AAAA |
| ATOM | 417 | N | GLY | E | 27 | 36.711 | 107.154 | 27.304 | 1.00 | 0.00 | AAAA |
| ATOM | 419 | CA | GLY | E | 27 | 35.837 | 106.672 | 28.349 | 1.00 | 0.00 | AAAA |
| ATOM | 422 | C | GLY | E | 27 | 35.664 | 107.707 | 29.450 | 1.00 | 0.00 | AAAA |
| ATOM | 423 | O | GLY | E | 27 | 36.003 | 108.867 | 29.259 | 1.00 | 0.00 | AAAA |
| ATOM | 424 | N | TYR | E | 28 | 35.414 | 107.226 | 30.675 | 1.00 | 0.00 | AAAA |
| ATOM | 426 | CA | TYR | E | 28 | 35.348 | 107.965 | 31.927 | 1.00 | 0.00 | AAAA |
| ATOM | 428 | CB | TYR | E | 28 | 34.514 | 107.249 | 32.986 | 1.00 | 0.00 | AAAA |
| ATOM | 431 | CG | TYR | E | 28 | 34.666 | 105.778 | 33.310 | 1.00 | 0.00 | AAAA |
| ATOM | 432 | CD1 | TYR | E | 28 | 35.642 | 105.270 | 34.178 | 1.00 | 0.00 | AAAA |
| ATOM | 434 | CD2 | TYR | E | 28 | 33.766 | 104.865 | 32.755 | 1.00 | 0.00 | AAAA |
| ATOM | 436 | CE1 | TYR | E | 28 | 35.769 | 103.915 | 34.479 | 1.00 | 0.00 | AAAA |
| ATOM | 438 | CE2 | TYR | E | 28 | 33.822 | 103.520 | 33.126 | 1.00 | 0.00 | AAAA |
| ATOM | 440 | CZ | TYR | E | 28 | 34.886 | 103.006 | 33.887 | 1.00 | 0.00 | AAAA |
| ATOM | 441 | OH | TYR | E | 28 | 35.063 | 101.664 | 34.021 | 1.00 | 0.00 | AAAA |
| ATOM | 443 | C | TYR | E | 28 | 36.721 | 108.095 | 32.556 | 1.00 | 0.00 | AAAA |
| ATOM | 444 | O | TYR | E | 28 | 37.465 | 107.128 | 32.375 | 1.00 | 0.00 | AAAA |
| ATOM | 445 | N | LEU | E | 29 | 37.002 | 109.028 | 33.473 | 1.00 | 0.00 | AAAA |
| ATOM | 447 | CA | LEU | E | 29 | 38.065 | 108.956 | 34.451 | 1.00 | 0.00 | AAAA |
| ATOM | 449 | CB | LEU | E | 29 | 38.975 | 110.179 | 34.382 | 1.00 | 0.00 | AAAA |
| ATOM | 452 | CG | LEU | E | 29 | 40.085 | 110.293 | 35.425 | 1.00 | 0.00 | AAAA |
| ATOM | 454 | CD1 | LEU | E | 29 | 40.850 | 109.005 | 35.720 | 1.00 | 0.00 | AAAA |
| ATOM | 458 | CD2 | LEU | E | 29 | 41.080 | 111.384 | 35.040 | 1.00 | 0.00 | AAAA |
| ATOM | 462 | C | LEU | E | 29 | 37.349 | 108.843 | 35.788 | 1.00 | 0.00 | AAAA |
| ATOM | 463 | O | LEU | E | 29 | 36.648 | 109.781 | 36.148 | 1.00 | 0.00 | AAAA |
| ATOM | 464 | N | HIS | E | 30 | 37.582 | 107.764 | 36.534 | 1.00 | 0.00 | AAAA |
| ATOM | 466 | CA | HIS | E | 30 | 37.020 | 107.484 | 37.840 | 1.00 | 0.00 | AAAA |
| ATOM | 468 | CB | HIS | E | 30 | 36.043 | 106.311 | 37.783 | 1.00 | 0.00 | AAAA |
| ATOM | 471 | CG | HIS | E | 30 | 35.339 | 106.025 | 39.074 | 1.00 | 0.00 | AAAA |
| ATOM | 472 | ND1 | HIS | E | 30 | 34.909 | 107.042 | 39.929 | 1.00 | 0.00 | AAAA |
| ATOM | 473 | CD2 | HIS | E | 30 | 34.868 | 104.821 | 39.534 | 1.00 | 0.00 | AAAA |
| ATOM | 475 | CE1 | HIS | E | 30 | 34.196 | 106.394 | 40.839 | 1.00 | 0.00 | AAAA |
| ATOM | 477 | NE2 | HIS | E | 30 | 34.111 | 105.063 | 40.660 | 1.00 | 0.00 | AAAA |
| ATOM | 479 | C | HIS | E | 30 | 38.182 | 107.293 | 38.803 | 1.00 | 0.00 | AAAA |
| ATOM | 480 | O | HIS | E | 30 | 38.967 | 106.374 | 38.586 | 1.00 | 0.00 | AAAA |
| ATOM | 481 | N | ILE | E | 31 | 38.432 | 108.127 | 39.819 | 1.00 | 0.00 | AAAA |
| ATOM | 483 | CA | ILE | E | 31 | 39.452 | 108.029 | 40.838 | 1.00 | 0.00 | AAAA |
| ATOM | 485 | CB | ILE | E | 31 | 40.672 | 108.951 | 40.713 | 1.00 | 0.00 | AAAA |
| ATOM | 487 | CG1 | ILE | E | 31 | 41.101 | 109.269 | 39.283 | 1.00 | 0.00 | AAAA |
| ATOM | 490 | CG2 | ILE | E | 31 | 41.780 | 108.442 | 41.637 | 1.00 | 0.00 | AAAA |
| ATOM | 494 | CD1 | ILE | E | 31 | 42.127 | 110.402 | 39.169 | 1.00 | 0.00 | AAAA |
| ATOM | 498 | C | ILE | E | 31 | 38.717 | 108.061 | 42.171 | 1.00 | 0.00 | AAAA |
| ATOM | 499 | O | ILE | E | 31 | 38.170 | 109.126 | 42.438 | 1.00 | 0.00 | AAAA |
| ATOM | 500 | N | LEU | E | 32 | 38.829 | 106.997 | 42.970 | 1.00 | 0.00 | AAAA |
| ATOM | 502 | CA. | LEU | E | 32 | 38.130 | 106.934 | 44.242 | 1.00 | 0.00 | AAAA |
| ATOM | 504 | CB | LEU | E | 32 | 36.742 | 106.303 | 44.271 | 1.00 | 0.00 | AAAA |
| ATOM | 507 | CG | LEU | E | 32 | 36.589 | 104.810 | 43.963 | 1.00 | 0.00 | AAAA |
| ATOM | 509 | CD1 | LEU | E | 32 | 35.167 | 104.332 | 44.269 | 1.00 | 0.00 | AAAA |
| ATOM | 513 | CD2 | LEU | E | 32 | 36.943 | 104.505 | 42.506 | 1.00 | 0.00 | AAAA |
| ATOM | 517 | C | LEU | E | 32 | 39.142 | 106.502 | 45.293 | 1.00 | 0.00 | AAAA |
| ATOM | 518 | O | LEU | E | 32 | 39.641 | 105.372 | 45.296 | 1.00 | 0.00 | AAAA |
| ATOM | 519 | N | LEU | E | 33 | 39.346 | 107.325 | 46.316 | 1.00 | 0.00 | AAAA |
| ATOM | 521 | CA | LEU | E | 33 | 39.709 | 106.976 | 47.679 | 1.00 | 0.00 | AAAA |
| ATOM | 523 | CB | LEU | E | 33 | 38:930 | 105.843 | 48.334 | 1.00 | 0.00 | AAAA |
| ATOM | 526 | CG | LEU | E | 33 | 37.527 | 105.440 | 47.901 | 1.00 | 0.00 | AAAA |
| ATOM | 528 | CD1 | LEU | E | 33 | 37.151 | 103.991 | 48.224 | 1.00 | 0.00 | AAAA |
| ATOM | 532 | CD2 | LEU | E | 33 | 36.563 | 106.518 | 48.408 | 1.00 | 0.00 | AAAA |
| ATOM | 536 | C | LEU | E | 33 | 41.204 | 106.922 | 47.981 | 1.00 | 0.00 | AAAA |
| ATOM | 537 | O | LEU | E | 33 | 41.597 | 106.690 | 49.125 | 1.00 | 0.00 | AAAA |
| ATOM | 538 | N | ILE | E | 34 | 41.984 | 106.947 | 46.901 | 1.00 | 0.00 | AAAA |
| ATOM | 540 | CA | ILE | E | 34 | 43.429 | 107.009 | 46.914 | 1.00 | 0.00 | AAAA |
| ATOM | 542 | CB | ILE | E | 34 | 43.977 | 107.053 | 45.487 | 1.00 | 0.00 | AAAA |
| ATOM | 544 | CG1 | ILE | E | 34 | 43.666 | 105.758 | 44.736 | 1.00 | 0.00 | AAAA |
| ATOM | 547 | CG2 | ILE | E | 34 | 45.430 | 107.466 | 45.286 | 1.00 | 0.00 | AAAA |
| ATOM | 551 | CD1 | ILE | E | 34 | 43.531 | 105.940 | 43.218 | 1.00 | 0.00 | AAAA |
| ATOM | 555 | C | ILE | E | 34 | 44.055 | 108.047 | 47.828 | 1.00 | 0.00 | AAAA |
| ATOM | 556 | O | ILE | E | 34 | 43.588 | 109.174 | 47.773 | 1.00 | 0.00 | AAAA |
| ATOM | 557 | N | SER | E | 35 | 45.114 | 107.716 | 48.573 | 1.00 | 0.00 | AAAA |
| ATOM | 559 | CA | SER | E | 35 | 46.043 | 108.609 | 49.236 | 1.00 | 0.00 | AAAA |
| ATOM | 561 | CB | SER | E | 35 | 46.239 | 108.230 | 50.706 | 1.00 | 0.00 | AAAA |
| ATOM | 564 | OG | SER | E | 35 | 47.058 | 109.157 | 51.359 | 1.00 | 0.00 | AAAA |
| ATOM | 566 | C | SER | E | 35 | 47.374 | 108.487 | 48.502 | 1.00 | 0.00 | AAAA |
| ATOM | 567 | O | SER | E | 35 | 47.819 | 107.385 | 48.205 | 1.00 | 0.00 | AAAA |
| ATOM | 568 | N | LYS | E | 36 | 48.053 | 109.610 | 48.283 | 1.00 | 0.00 | AAAA |
| ATOM | 570 | CA | LYS | E | 36 | 49.342 | 109.675 | 47.625 | 1.00 | 0.00 | AAAA |
| ATOM | 572 | CB | LYS | E | 36 | 49.474 | 111.149 | 47.227 | 1.00 | 0.00 | AAAA |
| ATOM | 575 | CG | LYS | E | 36 | 50.802 | 111.564 | 46.599 | 1.00 | 0.00 | AAAA |
| ATOM | 578 | CD | LYS | E | 36 | 50.618 | 112.908 | 45.898 | 1.00 | 0.00 | AAAA |
| ATOM | 581 | CE | LYS | E | 36 | 51.900 | 113.523 | 45.345 | 1.00 | 0.00 | AAAA |
| ATOM | 584 | NZ | LYS | E | 36 | 52.710 | 114.068 | 46.441 | 1.00 | 0.00 | AAAA |
| ATOM | 588 | C | LYS | E | 36 | 50.389 | 109.260 | 48.644 | 1.00 | 0.00 | AAAA |
| ATOM | 589 | O | LYS | E | 36 | 50.138 | 109.376 | 49.842 | 1.00 | 0.00 | AAAA |
| ATOM | 590 | N | ALA | E | 37 | 51.544 | 108.756 | 48.199 | 1.00 | 0.00 | AAAA |
| ATOM | 592 | CA | ALA | E | 37 | 52.728 | 108.527 | 49.004 | 1.00 | 0.00 | AAAA |
| ATOM | 594 | CB | ALA | E | 37 | 52.718 | 107.066 | 49.474 | 1.00 | 0.00 | AAAA |
| ATOM | 598 | C | ALA | E | 37 | 53.980 | 108.995 | 48.280 | 1.00 | 0.00 | AAAA |
| ATOM | 599 | O | ALA | E | 37 | 54.078 | 108.935 | 47.055 | 1.00 | 0.00 | AAAA |
| ATOM | 600 | N | GLU | E | 38 | 54.984 | 109.409 | 49.055 | 1.00 | 0.00 | AAAA |
| ATOM | 602 | CA | GLU | E | 38 | 56.292 | 109.924 | 48.695 | 1.00 | 0.00 | AAAA |
| ATOM | 604 | CB | GLU | E | 38 | 56.716 | 110.844 | 49.833 | 1.00 | 0.00 | AAAA |
| ATOM | 607 | CG | GLU | E | 38 | 57.819 | 111.835 | 49.466 | 1.00 | 0.00 | AAAA |
| ATOM | 610 | CD | GLU | E | 38 | 57.468 | 112.808 | 48.343 | 1.00 | 0.00 | AAAA |
| ATOM | 611 | OE1 | GLU | E | 38 | 56.289 | 113.223 | 48.206 | 1.00 | 0.00 | AAAA |
| ATOM | 612 | OE2 | GLU | E | 38 | 58.389 | 113.076 | 47.555 | 1.00 | 0.00 | AAAA |
| ATOM | 613 | C | GLU | E | 38 | 57.328 | 108.847 | 48.393 | 1.00 | 0.00 | AAAA |
| ATOM | 614 | O | GLU | E | 38 | 58.522 | 109.040 | 48.570 | 1.00 | 0.00 | AAAA |
| ATOM | 615 | N | ASP | E | 39 | 56.850 | 107.694 | 47.907 | 1.00 | 0.00 | AAAA |
| ATOM | 617 | CA | ASP | E | 39 | 57.655 | 106.532 | 47.592 | 1.00 | 0.00 | AAAA |
| ATOM | 619 | CB | ASP | E | 39 | 56.917 | 105.305 | 48.114 | 1.00 | 0.00 | AAAA |
| ATOM | 622 | CG | ASP | E | 39 | 57.583 | 103.979 | 47.784 | 1.00 | 0.00 | AAAA |
| ATOM | 623 | OD1 | ASP | E | 39 | 57.066 | 103.196 | 46.959 | 1.00 | 0.00 | AAAA |
| ATOM | 624 | OD2 | ASP | E | 39 | 58.642 | 103.698 | 48.384 | 1.00 | 0.00 | AAAA |
| ATOM | 625 | C | ASP | E | 39 | 58.013 | 106.429 | 46.120 | 1.00 | 0.00 | AAAA |
| ATOM | 626 | O | ASP | E | 39 | 58.874 | 105.645 | 45.741 | 1.00 | 0.00 | AAAA |
| ATOM | 627 | N | TYR | E | 40 | 57.575 | 107.401 | 45.297 | 1.00 | 0.00 | AAAA |
| ATOM | 629 | CA | TYR | E | 40 | 58.016 | 107.699 | 43.959 | 1.00 | 0.00 | AAAA |
| ATOM | 631 | CB | TYR | E | 40 | 57.426 | 106.759 | 42.906 | 1.00 | 0.00 | AAAA |
| ATOM | 634 | CG | TYR | E | 40 | 57.698 | 107.025 | 41.440 | 1.00 | 0.00 | AAAA |
| ATOM | 635 | CD1 | TYR | E | 40 | 58.969 | 106.859 | 40.883 | 1.00 | 0.00 | AAAA |
| ATOM | 637 | CD2 | TYR | E | 40 | 56.655 | 107.386 | 40.579 | 1.00 | 0.00 | AAAA |
| ATOM | 639 | CE1 | TYR | E | 40 | 59.276 | 106.968 | 39.513 | 1.00 | 0.00 | AAAA |
| ATOM | 641 | CE2 | TYR | E | 40 | 56.967 | 107.582 | 39.222 | 1.00 | 0.00 | AAAA |
| ATOM | 643 | CZ | TYR | E | 40 | 58.225 | 107.322 | 38.662 | 1.00 | 0.00 | AAAA |
| ATOM | 644 | OH | TYR | E | 40 | 58.437 | 107.512 | 37.335 | 1.00 | 0.00 | AAAA |
| ATOM | 646 | C | TYR | E | 40 | 57.921 | 109.214 | 43.786 | 1.00 | 0.00 | AAAA |
| ATOM | 647 | O | TYR | E | 40 | 57.685 | 109.891 | 44.786 | 1.00 | 0.00 | AAAA |
| ATOM | 648 | N | ARG | E | 41 | 57.989 | 109.642 | 42.527 | 1.00 | 0.00 | AAAA |
| ATOM | 650 | CA | ARG | E | 41 | 57.593 | 110.921 | 41.965 | 1.00 | 0.00 | AAAA |
| ATOM | 652 | CB | ARG | E | 41 | 57.927 | 110.965 | 40.475 | 1.00 | 0.00 | AAAA |
| ATOM | 655 | CG | ARG | E | 41 | 59.402 | 110.719 | 40.168 | 1.00 | 0.00 | AAAA |
| ATOM | 658 | CD | ARG | E | 41 | 59.688 | 110.978 | 38.687 | 1.00 | 0.00 | AAAA |
| ATOM | 661 | NE | ARG | E | 41 | 61.110 | 110.974 | 38.350 | 1.00 | 0.00 | AAAA |
| ATOM | 663 | CZ | ARG | E | 41 | 61.534 | 110.839 | 37.086 | 1.00 | 0.00 | AAAA |
| ATOM | 664 | NH1 | ARG | E | 41 | 60.664 | 110.703 | 36.074 | 1.00 | 0.00 | AAAA |
| ATOM | 667 | NH2 | ARG | E | 41 | 62.853 | 110.794 | 36.858 | 1.00 | 0.00 | AAAA |
| ATOM | 670 | C | ARG | E | 41 | 56.094 | 111.173 | 42.103 | 1.00 | 0.00 | AAAA |
| ATOM | 671 | O | ARG | E | 41 | 55.333 | 110.215 | 42.060 | 1.00 | 0.00 | AAAA |
| ATOM | 672 | N | SER | E | 42 | 55.693 | 112.445 | 42.073 | 1.00 | 0.00 | AAAA |
| ATOM | 674 | CA | SER | E | 42 | 54.329 | 112.950 | 42.031 | 1.00 | 0.00 | AAAA |
| ATOM | 676 | CB | SER | E | 42 | 54.364 | 114.191 | 42.919 | 1.00 | 0.00 | AAAA |
| ATOM | 679 | OG | SER | E | 42 | 55.501 | 114.991 | 42.665 | 1.00 | 0.00 | AAAA |
| ATOM | 681 | C | SER | E | 42 | 53.977 | 113.430 | 40.633 | 1.00 | 0.00 | AAAA |
| ATOM | 682 | O | SER | E | 42 | 54.557 | 114.368 | 40.097 | 1.00 | 0.00 | AAAA |
| ATOM | 683 | N | TYR | E | 43 | 53.069 | 112.717 | 39.942 | 1.00 | 0.00 | AAAA |
| ATOM | 685 | CA | TYR | E | 43 | 52.848 | 112.783 | 38.516 | 1.00 | 0.00 | AAAA |
| ATOM | 687 | CB | TYR | E | 43 | 51.800 | 111.724 | 38.165 | 1.00 | 0.00 | AAAA |
| ATOM | 690 | CG | TYR | E | 43 | 52.200 | 110.307 | 38.481 | 1.00 | 0.00 | AAAA |
| ATOM | 691 | CD1 | TYR | E | 43 | 53.031 | 109.615 | 37.590 | 1.00 | 0.00 | AAAA |
| ATOM | 693 | CD2 | TYR | E | 43 | 51.743 | 109.588 | 39.584 | 1.00 | 0.00 | AAAA |
| ATOM | 695 | CE1 | TYR | E | 43 | 53.461 | 108.317 | 37.900 | 1.00 | 0.00 | AAAA |
| ATOM | 69.7 | CE2 | TYR | E | 43 | 52.182 | 108.287 | 39.866 | 1.00 | 0.00 | AAAA |
| ATOM | 699 | CZ | TYR | E | 43 | 53.081 | 107.592 | 39.040 | 1.00 | 0.00 | AAAA |
| ATOM | 700 | OH | TYR | E | 43 | 53.496 | 106.336 | 39.351 | 1.00 | 0.00 | AAAA |
| ATOM | 702 | C | TYR | E | 43 | 52.259 | 114.090 | 37.991 | 1.00 | 0.00 | AAAA |
| ATOM | 703 | O | TYR | E | 43 | 51.605 | 114.881 | 38.663 | 1.00 | 0.00 | AAAA |
| ATOM | 704 | N | ARG | E | 44 | 52.511 | 114.214 | 36.687 | 1.00 | 0.00 | AAAA |
| ATOM | 706 | CA | ARG | E | 44 | 52.082 | 115.413 | 35.986 | 1.00 | 0.00 | AAAA |
| ATOM | 708 | CB | ARG | E | 44 | 52.976 | 116.635 | 36.188 | 1.00 | 0.00 | AAAA |
| ATOM | 711 | CG | ARG | E | 44 | 52.491 | 117.959 | 35.610 | 1.00 | 0.00 | AAAA |
| ATOM | 714 | CD | ARG | E | 44 | 53.212 | 119.103 | 36.340 | 1.00 | 0.00 | AAAA |
| ATOM | 717 | NE | ARG | E | 44 | 54.656 | 118.906 | 36.346 | 1.00 | 0.00 | AAAA |
| ATOM | 719 | CZ | ARG | E | 44 | 55.580 | 119.346 | 37.207 | 1.00 | 0.00 | AAAA |
| ATOM | 720 | NH1 | ARG | E | 44 | 55.288 | 120.244 | 38.158 | 1.00 | 0.00 | AAAA |
| ATOM | 723 | NH2 | ARG | E | 44 | 56.854 | 118.961 | 37.063 | 1.00 | 0.00 | AAAA |
| ATOM | 726 | C | ARG | E | 44 | 51.823 | 115.062 | 34.529 | 1.00 | 0.00 | AAAA |
| ATOM | 727 | O | ARG | E | 44 | 52.768 | 114.640 | 33.853 | 1.00 | 0.00 | AAAA |
| ATOM | 728 | N | PHE | E | 45 | 50.590 | 115.214 | 34.037 | 1.00 | 0.00 | AAAA |
| ATOM | 730 | CA | PHE | E | 45 | 50.107 | 114.597 | 32.817 | 1.00 | 0.00 | AAAA |
| ATOM | 732 | CB | PHE | E | 45 | 49.087 | 113.478 | 33.079 | 1.00 | 0.00 | AAAA |
| ATOM | 735 | CG | PHE | E | 45 | 49.452 | 112.273 | 33.898 | 1.00 | 0.00 | AAAA |
| ATOM | 736 | CD1 | PHE | E | 45 | 50.424 | 111.380 | 33.433 | 1.00 | 0.00 | AAAA |
| ATOM | 738 | CD2 | PHE | E | 45 | 48.957 | 112.195 | 35.210 | 1.00 | 0.00 | AAAA |
| ATOM | 740 | CE1 | PHE | E | 45 | 50.713 | 110.260 | 34.225 | 1.00 | 0.00 | AAAA |
| ATOM | 742 | CE2 | PHE | E | 45 | 49.314 | 111.065 | 35.941 | 1.00 | 0.00 | AAAA |
| ATOM | 744 | CZ | PHE | E | 45 | 50.100 | 110.009 | 35.456 | 1.00 | 0.00 | AAAA |
| ATOM | 746 | C | PHE | E | 45 | 49.614 | 115.714 | 31.902 | 1.00 | 0.00 | AAAA |
| ATOM | 747 | O | PHE | E | 45 | 48.434 | 116.045 | 31.894 | 1.00 | 0.00 | AAAA |
| ATOM | 748 | N | PRO | E | 46 | 50.530 | 116.396 | 31.216 | 1.00 | 0.00 | AAAA |
| ATOM | 749 | CA | PRO | E | 46 | 50.168 | 117.554 | 30.416 | 1.00 | 0.00 | AAAA |
| ATOM | 751 | CB | PRO | E | 46 | 51.477 | 118.311 | 30.183 | 1.00 | 0.00 | AAAA |
| ATOM | 754 | CG | PRO | E | 46 | 52.545 | 117.226 | 30.234 | 1.00 | 0.00 | AAAA |
| ATOM | 757 | CD | PRO | E | 46 | 51.965 | 116.172 | 31.180 | 1.00 | 0.00 | AAAA |
| ATOM | 760 | C | PRO | E | 46 | 49.537 | 117.185 | 29.080 | 1.00 | 0.00 | AAAA |
| ATOM | 761 | O | PRO | E | 46 | 48.928 | 118.079 | 28.487 | 1.00 | 0.00 | AAAA |
| ATOM | 762 | N | LYS | E | 47 | 49.671 | 115.981 | 28.532 | 1.00 | 0.00 | AAAA |
| ATOM | 764 | CA | LYS | E | 47 | 49.078 | 115.601 | 27.264 | 1.00 | 0.00 | AAAA |
| ATOM | 766 | CB | LYS | E | 47 | 49.851 | 114.529 | 26.500 | 1.00 | 0.00 | AAAA |
| ATOM | 769 | CG | LYS | E | 47 | 51.233 | 115.096 | 26.169 | 1.00 | 0.00 | AAAA |
| ATOM | 772 | CD | LYS | E | 47 | 52.025 | 114.351 | 25.101 | 1.00 | 0.00 | AAAA |
| ATOM | 775 | CE | LYS | E | 47 | 53.365 | 115.058 | 24.907 | 1.00 | 0.00 | AAAA |
| ATOM | 778 | NZ | LYS | E | 47 | 53.945 | 114.460 | 23.694 | 1.00 | 0.00 | AAAA |
| ATOM | 782 | C | LYS | E | 47 | 47.635 | 115.194 | 27.479 | 1.00 | 0.00 | AAAA |
| ATOM | 783 | O | LYS | E | 47 | 46.973 | 114.883 | 26.498 | 1.00 | 0.00 | AAAA |
| ATOM | 784 | N | LEU | E | 48 | 47.139 | 115.130 | 28.724 | 1.00 | 0.00 | AAAA |
| ATOM | 786 | CA | LEU | E | 48 | 45.810 | 114.757 | 29.143 | 1.00 | 0.00 | AAAA |
| ATOM | 788 | CB | LEU | E | 48 | 45.793 | 114.585 | 30.662 | 1.00 | 0.00 | AAAA |
| ATOM | 791 | CG | LEU | E | 48 | 44.711 | 113.772 | 31.383 | 1.00 | 0.00 | AAAA |
| ATOM | 793 | CD1 | LEU | E | 48 | 43.280 | 114.322 | 31.356 | 1.00 | 0.00 | AAAA |
| ATOM | 797 | CD2 | LEU | E | 48 | 44.739 | 112.342 | 30.830 | 1.00 | 0.00 | AAAA |
| ATOM | 801 | C | LEU | E | 48 | 44.859 | 115.863 | 28.746 | 1.00 | 0.00 | AAAA |
| ATOM | 802 | O | LEU | E | 48 | 44.788 | 116.872 | 29.448 | 1.00 | 0.00 | AAAA |
| ATOM | 803 | N | THR | E | 49 | 44.255 | 115.800 | 27.559 | 1.00 | 0.00 | AAAA |
| ATOM | 805 | CA | THR | E | 49 | 43.600 | 116.943 | 26.959 | 1.00 | 0.00 | AAAA |
| ATOM | 807 | CB | THR | E | 49 | 49.174 | 117.342 | 25.599 | 1.00 | 0.00 | AAAA |
| ATOM | 809 | OG1 | THR | E | 49 | 44.212 | 116.197 | 24.776 | 1.00 | 0.00 | AAAA |
| ATOM | 811 | CG2 | THR | E | 49 | 45.504 | 118.084 | 25.769 | 1.00 | 0.00 | AAAA |
| ATOM | 815 | C | THR | E | 49 | 42.097 | 116.742 | 26.966 | 1.00 | 0.00 | AAAA |
| ATOM | 816 | O | THR | E | 49 | 41.398 | 117.744 | 27.083 | 1.00 | 0.00 | AAAA |
| ATOM | 817 | N | VAL | E | 50 | 41.522 | 115.548 | 26.782 | 1.00 | 0.00 | AAAA |
| ATOM | 819 | CA | VAL | E | 50 | 40.113 | 115.222 | 26.784 | 1.00 | 0.00 | AAAA |
| ATOM | 821 | CB | VAL | E | 50 | 39.586 | 115.197 | 25.342 | 1.00 | 0.00 | AAAA |
| ATOM | 823 | CG1 | VAL | E | 50 | 38.130 | 114.752 | 25.217 | 1.00 | 0.00 | AAAA |
| ATOM | 827 | CG2 | VAL | E | 50 | 39.900 | 116.564 | 24.743 | 1.00 | 0.00 | AAAA |
| ATOM | 831 | C | VAL | E | 50 | 39.789 | 113.975 | 27.588 | 1.00 | 0.00 | AAAA |
| ATOM | 832 | O | VAL | E | 50 | 40.626 | 113.082 | 27.747 | 1.00 | 0.00 | AAAA |
| ATOM | 833 | N | ILE | E | 51 | 38.587 | 113.880 | 28.172 | 1.00 | 0.00 | AAAA |
| ATOM | 835 | CA | ILE | E | 51 | 37.892 | 112.700 | 28.649 | 1.00 | 0.00 | AAAA |
| ATOM | 837 | CB | ILE | E | 51 | 37.782 | 112.720 | 30.174 | 1.00 | 0.00 | AAAA |
| ATOM | 839 | CG1 | ILE | E | 51 | 39.113 | 112.936 | 30.895 | 1.00 | 0.00 | AAAA |
| ATOM | 842 | CG2 | ILE | E | 51 | 37.243 | 111.381 | 30.683 | 1.00 | 0.00 | AAAA |
| ATOM | 846 | CD1 | ILE | E | 51 | 38.895 | 113.340 | 32.353 | 1.00 | 0.00 | AAAA |
| ATOM | 850 | C | ILE | E | 51 | 36.567 | 112.830 | 27.916 | 1.00 | 0.00 | AAAA |
| ATOM | 851 | O | ILE | E | 51 | 35.912 | 113.871 | 27.915 | 1.00 | 0.00 | AAAA |
| ATOM | 852 | N | THR | E | 52 | 36.115 | 111.784 | 27.211 | 1.00 | 0.00 | AAAA |
| ATOM | 854 | CA | THR | E | 52 | 35.049 | 111.690 | 26.234 | 1.00 | 0.00 | AAAA |
| ATOM | 856 | CB | THR | E | 52 | 35.322 | 110.508 | 25.315 | 1.00 | 0.00 | AAAA |
| ATOM | 858 | OG1 | THR | E | 52 | 35.426 | 109.315 | 26.054 | 1.00 | 0.00 | AAAA |
| ATOM | 860 | CG2 | THR | E | 52 | 36.660 | 110.673 | 24.608 | 1.00 | 0.00 | AAAA |
| ATOM | 864 | C | THR | E | 52 | 33.730 | 111.514 | 26.970 | 1.00 | 0.00 | AAAA |
| ATOM | 865 | O | THR | E | 52 | 32.633 | 111.678 | 26.427 | 1.00 | 0.00 | AAAA |
| ATOM | 866 | N | GLU | E | 53 | 33.766 | 111.111 | 28.240 | 1.00 | 0.00 | AAAA |
| ATOM | 868 | CA | GLU | E | 53 | 32.650 | 110.926 | 29.142 | 1.00 | 0.00 | AAAA |
| ATOM | 870 | CB | GLU | E | 53 | 32.395 | 109.450 | 29.457 | 1.00 | 0.00 | AAAA |
| ATOM | 873 | CG | GLU | E | 53 | 32.052 | 108.593 | 28.232 | 1.00 | 0.00 | AAAA |
| ATOM | 876 | CD | GLU | E | 53 | 31.642 | 107.143 | 28.459 | 1.00 | 0.00 | AAAA |
| ATOM | 877 | OE1 | GLU | E | 53 | 31.792 | 106.641 | 29.589 | 1.00 | 0.00 | AAAA |
| ATOM | 878 | OE2 | GLU | E | 53 | 31.359 | 106.460 | 27.454 | 1.00 | 0.00 | AAAA |
| ATOM | 879 | C | GLU | E | 53 | 32.726 | 111.922 | 30.290 | 1.00 | 0.00 | AAAA |
| ATOM | 880 | O | GLU | E | 53 | 32.991 | 113.125 | 30.144 | 1.00 | 0.00 | AAAA |
| ATOM | 881 | N | TYR | E | 54 | 32.911 | 111.389 | 31.500 | 1.00 | 0.00 | AAAA |
| ATOM | 883 | CA | TYR | E | 54 | 32.861 | 112.118 | 32.758 | 1.00 | 0.00 | AAAA |
| ATOM | 885 | CB | TYR | E | 54 | 31.673 | 111.648 | 33.589 | 1.00 | 0.00 | AAAA |
| ATOM | 888 | CG | TYR | E | 54 | 31.702 | 110.163 | 33.874 | 1.00 | 0.00 | AAAA |
| ATOM | 889 | CD1 | TYR | E | 54 | 32.394 | 109.700 | 34.991 | 1.00 | 0.00 | AAAA |
| ATOM | 891 | CD2 | TYR | E | 54 | 30.966 | 109.265 | 33.085 | 1.00 | 0.00 | AAAA |
| ATOM | 893 | CE1 | TYR | E | 54 | 32.180 | 108.388 | 35.447 | 1.00 | 0.00 | AAAA |
| ATOM | 895 | CE2 | TYR | E | 54 | 30.854 | 107.927 | 33.462 | 1.00 | 0.00 | AAAA |
| ATOM | 897 | CZ | TYR | E | 54 | 31.393 | 107.504 | 34.681 | 1.00 | 0.00 | AAAA |
| ATOM | 898 | OH | TYR | E | 54 | 31.145 | 106.234 | 35.098 | 1.00 | 0.00 | AAAA |
| ATOM | 900 | C | TYR | E | 54 | 34.143 | 111.907 | 33.552 | 1.00 | 0.00 | AAAA |
| ATOM | 901 | O | TYR | E | 54 | 34.867 | 110.928 | 33.376 | 1.00 | 0.00 | AAAA |
| ATOM | 902 | N | LEU | E | 55 | 34.427 | 112.863 | 34.435 | 1.00 | 0.00 | AAAA |
| ATOM | 904 | CA | LEU | E | 55 | 35.580 | 113.052 | 35.291 | 1.00 | 0.00 | AAAA |
| ATOM | 906 | CB | LEU | E | 55 | 36.130 | 114.464 | 35.084 | 1.00 | 0.00 | AAAA |
| ATOM | 909 | CG | LEU | E | 55 | 37.222 | 114.779 | 36.105 | 1.00 | 0.00 | AAAA |
| ATOM | 911 | CD1 | LEU | E | 55 | 38.514 | 114.021 | 35.794 | 1.00 | 0.00 | AAAA |
| ATOM | 915 | CD2 | LEU | E | 55 | 37.688 | 116.229 | 36.003 | 1.00 | 0.00 | AAAA |
| ATOM | 919 | C | LEU | E | 55 | 34.943 | 112.886 | 36.662 | 1.00 | 0.00 | AAAA |
| ATOM | 920 | O | LEU | E | 55 | 34.315 | 113.828 | 37.141 | 1.00 | 0.00 | AAAA |
| ATOM | 921 | N | LEU | E | 56 | 35.180 | 111.763 | 37.341 | 1.00 | 0.00 | AAAA |
| ATOM | 923 | CA | LEU | E | 56 | 34.689 | 111.513 | 38.687 | 1.00 | 0.00 | AAAA |
| ATOM | 925 | CB | LEU | E | 56 | 33.822 | 110.247 | 38.713 | 1.00 | 0.00 | AAAA |
| ATOM | 928 | CG | LEU | E | 56 | 32.745 | 110.114 | 39.792 | 1.00 | 0.00 | AAAA |
| ATOM | 930 | CD1 | LEU | E | 56 | 31.720 | 109.057 | 39.384 | 1.00 | 0.00 | AAAA |
| ATOM | 934 | CD2 | LEU | E | 56 | 33.373 | 109.888 | 41.164 | 1.00 | 0.00 | AAAA |
| ATOM | 938 | C | LEU | E | 56 | 35.889 | 111.484 | 39.620 | 1.00 | 0.00 | AAAA |
| ATOM | 939 | O | LEU | E | 56 | 36.794 | 110.683 | 39.407 | 1.00 | 0.00 | AAAA |
| ATOM | 940 | N | LEU | E | 57 | 35.892 | 112.238 | 40.723 | 1.00 | 0.00 | AAAA |
| ATOM | 942 | CA | LEU | E | 57 | 36.900 | 112.359 | 41.762 | 1.00 | 0.00 | AAAA |
| ATOM | 944 | CB | LEU | E | 57 | 37.794 | 113.575 | 41.518 | 1.00 | 0.00 | AAAA |
| ATOM | 947 | CG | LEU | E | 57 | 38.559 | 113.751 | 40.200 | 1.00 | 0.00 | AAAA |
| ATOM | 949 | CD1 | LEU | E | 57 | 39.113 | 115.144 | 39.870 | 1.00 | 0.00 | AAAA |
| ATOM | 953 | CD2 | LEU | E | 57 | 39.737 | 112.799 | 40.036 | 1.00 | 0.00 | AAAA |
| ATOM | 957 | C | LEU | E | 57 | 36.232 | 112.344 | 43.134 | 1.00 | 0.00 | AAAA |
| ATOM | 958 | O | LEU | E | 57 | 35.273 | 113.044 | 43.470 | 1.00 | 0.00 | AAAA |
| ATOM | 959 | N | PHE | E | 58 | 36.565 | 111.346 | 43.946 | 1.00 | 0.00 | AAAA |
| ATOM | 961 | CA | PHE | E | 58 | 35.933 | 111.047 | 45.220 | 1.00 | 0.00 | AAAA |
| ATOM | 963 | CB | PHE | E | 58 | 34.952 | 109.892 | 45.021 | 1.00 | 0.00 | AAAA |
| ATOM | 966 | CG | PHE | E | 58 | 33.901 | 109.635 | 46.083 | 1.00 | 0.00 | AAAA |
| ATOM | 967 | CD1 | PHE | E | 58 | 33.244 | 110.674 | 46.731 | 1.00 | 0.00 | AAAA |
| ATOM | 969 | CD2 | PHE | E | 58 | 33.677 | 108.298 | 46.443 | 1.00 | 0.00 | AAAA |
| ATOM | 971 | CE1 | PHE | E | 58 | 32.438 | 110.392 | 47.837 | 1.00 | 0.00 | AAAA |
| ATOM | 973 | CE2 | PHE | E | 58 | 32.909 | 107.995 | 47.575 | 1.00 | 0.00 | AAAA |
| ATOM | 975 | CZ | PHE | E | 58 | 32.313 | 109.058 | 48.251 | 1.00 | 0.00 | AAAA |
| ATOM | 977 | C | PHE | E | 58 | 36.980 | 110.709 | 46.281 | 1.00 | 0.00 | AAAA |
| ATOM | 978 | O | PHE | E | 58 | 37.644 | 109.677 | 46.241 | 1.00 | 0.00 | AAAA |
| ATOM | 979 | N | ARG | E | 59 | 37.062 | 111.584 | 47.287 | 1.00 | 0.00 | AAAA |
| ATOM | 981 | CA | ARG | E | 59 | 37.774 | 111.376 | 48.536 | 1.00 | 0.00 | AAAA |
| ATOM | 983 | CB | ARG | E | 59 | 37.210 | 110.238 | 49.393 | 1.00 | 0.00 | AAAA |
| ATOM | 986 | CG | ARG | E | 59 | 35.684 | 110.145 | 49.537 | 1.00 | 0.00 | AAAA |
| ATOM | 989 | CD | ARG | E | 59 | 35.208 | 109.266 | 50.686 | 1.00 | 0.00 | AAAA |
| ATOM | 992 | NE | ARG | E | 59 | 34.977 | 110.033 | 51.909 | 1.00 | 0.00 | AAAA |
| ATOM | 994 | CZ | ARG | E | 59 | 35.289 | 109.803 | 53.196 | 1.00 | 0.00 | AAAA |
| ATOM | 995 | NH1 | ARG | E | 59 | 36.157 | 108.863 | 53.596 | 1.00 | 0.00 | AAAA |
| ATOM | 998 | NH2 | ARG | E | 59 | 34.780 | 110.576 | 54.165 | 1.00 | 0.00 | AAAA |
| ATOM | 1001 | C | ARG | E | 59 | 39.287 | 111.363 | 48.403 | 1.00 | 0.00 | AAAA |
| ATOM | 1002 | O | ARG | E | 59 | 39.977 | 110.953 | 49.331 | 1.00 | 0.00 | AAAA |
| ATOM | 1003 | N | VAL | E | 60 | 39.876 | 111.712 | 47.267 | 1.00 | 0.00 | AAAA |
| ATOM | 1005 | CA | VAL | E | 60 | 41.260 | 111.459 | 46.909 | 1.00 | 0.00 | AAAA |
| ATOM | 1007 | CB | VAL | E | 60 | 41.388 | 111.452 | 45.388 | 1.00 | 0.00 | AAAA |
| ATOM | 1009 | CG1 | VAL | E | 60 | 42.485 | 110.484 | 44.933 | 1.00 | 0.00 | AAAA |
| ATOM | 1013 | CG2 | VAL | E | 60 | 40.168 | 111.242 | 44.491 | 1.00 | 0.00 | AAAA |
| ATOM | 1017 | C | VAL | E | 60 | 42.194 | 112.427 | 47.628 | 1.00 | 0.00 | AAAA |
| ATOM | 1018 | O | VAL | E | 60 | 41.888 | 113.609 | 47.513 | 1.00 | 0.00 | AAAA |
| ATOM | 1019 | N | ALA | E | 61 | 43.197 | 111.923 | 48.345 | 1.00 | 0.00 | AAAA |
| ATOM | 1021 | CA | ALA | E | 61 | 43.985 | 112.641 | 49.335 | 1.00 | 0.00 | AAAA |
| ATOM | 1023 | CB | ALA | E | 61 | 43.920 | 111.838 | 50.635 | 1.00 | 0.00 | AAAA |
| ATOM | 1027 | C | ALA | E | 61 | 45.432 | 112.797 | 48.879 | 1.00 | 0.00 | AAAA |
| ATOM | 1028 | O | ALA | E | 61 | 46.008 | 111.874 | 48.315 | 1.00 | 0.00 | AAAA |
| ATOM | 1029 | N | GLY | E | 62 | 45.995 | 113.984 | 49.112 | 1.00 | 0.00 | AAAA |
| ATOM | 1031 | CA | GLY | E | 62 | 47.396 | 114.289 | 48.898 | 1.00 | 0.00 | AAAA |
| ATOM | 1034 | C | GLY | E | 62 | 47.722 | 115.374 | 47.882 | 1.00 | 0.00 | AAAA |
| ATOM | 1035 | O | GLY | E | 62 | 48.838 | 115.887 | 47.809 | 1.00 | 0.00 | AAAA |
| ATOM | 1036 | N | LEU | E | 63 | 46.738 | 115.771 | 47.069 | 1.00 | 0.00 | AAAA |
| ATOM | 1038 | CA | LEU | E | 63 | 46.720 | 116.774 | 46.023 | 1.00 | 0.00 | AAAA |
| ATOM | 1040 | CB | LEU | E | 63 | 45.595 | 116.555 | 45.000 | 1.00 | 0.00 | AAAA |
| ATOM | 1043 | CG | LEU | E | 63 | 45.562 | 115.134 | 44.435 | 1.00 | 0.00 | AAAA |
| ATOM | 1045 | CD1 | LEU | E | 63 | 44.488 | 114.992 | 43.355 | 1.00 | 0.00 | AAAA |
| ATOM | 1049 | CD2 | LEU | E | 63 | 46.950 | 114.727 | 43.928 | 1.00 | 0.00 | AAAA |
| ATOM | 1053 | C | LEU | E | 63 | 46.504 | 118.092 | 46.740 | 1.00 | 0.00 | AAAA |
| ATOM | 1054 | O | LEU | E | 63 | 45.547 | 118.271 | 47.488 | 1.00 | 0.00 | AAAA |
| ATOM | 1055 | N | GLU | E | 64 | 47.398 | 119.031 | 46.406 | 1.00 | 0.00 | AAAA |
| ATOM | 1057 | CA | GLU | E | 64 | 47.265 | 120.420 | 46.780 | 1.00 | 0.00 | AAAA |
| ATOM | 1059 | CB | GLU | E | 64 | 48.611 | 121.160 | 46.818 | 1.00 | 0.00 | AAAA |
| ATOM | 1062 | CG | GLU | E | 64 | 48.757 | 122.153 | 47.969 | 1.00 | 0.00 | AAAA |
| ATOM | 1065 | CD | GLU | E | 64 | 49.222 | 121.585 | 49.300 | 1.00 | 0.00 | AAAA |
| ATOM | 1066 | OE1 | GLU | E | 64 | 49.546 | 122.339 | 50.235 | 1.00 | 0.00 | AAAA |
| ATOM | 1067 | OE2 | GLU | E | 64 | 49.238 | 120.337 | 49.420 | 1.00 | 0.00 | AAAA |
| ATOM | 1068 | C | GLU | E | 64 | 46.439 | 121.139 | 45.732 | 1.00 | 0.00 | AAAA |
| ATOM | 1069 | O | GLU | E | 64 | 45.518 | 121.888 | 46.064 | 1.00 | 0.00 | AAAA |
| ATOM | 1070 | N | SER | E | 65 | 46.626 | 120.812 | 44.445 | 1.00 | 0.00 | AAAA |
| ATOM | 1072 | CA | SER | E | 65 | 45.877 | 121.215 | 43.274 | 1.00 | 0.00 | AAAA |
| ATOM | 1074 | CB | SER | E | 65 | 46.679 | 122.315 | 42.578 | 1.00 | 0.00 | AAAA |
| ATOM | 1077 | OG | SER | E | 65 | 45.928 | 123.005 | 41.602 | 1.00 | 0.00 | AAAA |
| ATOM | 1079 | C | SER | E | 65 | 45.546 | 120.073 | 42.326 | 1.00 | 0.00 | AAAA |
| ATOM | 1080 | O | SER | E | 65 | 46.203 | 119.039 | 42.390 | 1.00 | 0.00 | AAAA |
| ATOM | 1081 | N | LEU | E | 66 | 44.465 | 120.226 | 41.555 | 1.00 | 0.00 | AAAA |
| ATOM | 1083 | CA | LEU | E | 66 | 44.251 | 119.356 | 40.407 | 1.00 | 0.00 | AAAA |
| ATOM | 1085 | CB | LEU | E | 66 | 42.772 | 119.133 | 40.095 | 1.00 | 0.00 | AAAA |
| ATOM | 1088 | CG | LEU | E | 66 | 42.013 | 118.269 | 41.104 | 1.00 | 0.00 | AAAA |
| ATOM | 1090 | CD1 | LEU | E | 66 | 40.510 | 118.314 | 40.843 | 1.00 | 0.00 | AAAA |
| ATOM | 1094 | CD2 | LEU | E | 66 | 42.615 | 116.868 | 41.241 | 1.00 | 0.00 | AAAA |
| ATOM | 1098 | C | LEU | E | 66 | 44.915 | 119.886 | 39.145 | 1.00 | 0.00 | AAAA |
| ATOM | 1099 | O | LEU | E | 66 | 45.077 | 119.132 | 38.195 | 1.00 | 0.00 | AAAA |
| ATOM | 1100 | N | GLY | E | 67 | 45.396 | 121.135 | 39.130 | 1.00 | 0.00 | AAAA |
| ATOM | 1102 | CA | GLY | E | 67 | 46.125 | 121.869 | 38.117 | 1.00 | 0.00 | AAAA |
| ATOM | 1105 | C | GLY | E | 67 | 47.610 | 121.570 | 38.215 | 1.00 | 0.00 | AAAA |
| ATOM | 1106 | O | GLY | E | 67 | 48.411 | 122.253 | 37.575 | 1.00 | 0.00 | AAAA |
| ATOM | 1107 | N | ASP | E | 68 | 47.912 | 120.501 | 38.951 | 1.00 | 0.00 | AAAA |
| ATOM | 1109 | CA | ASP | E | 68 | 49.231 | 119.935 | 39.113 | 1.00 | 0.00 | AAAA |
| ATOM | 1111 | CB | ASP | E | 68 | 49.788 | 120.329 | 40.484 | 1.00 | 0.00 | AAAA |
| ATOM | 1114 | CG | ASP | E | 68 | 50.792 | 119.399 | 41.153 | 1.00 | 0.00 | AAAA |
| ATOM | 1115 | OD1 | ASP | E | 68 | 50.373 | 118.496 | 41.899 | 1.00 | 0.00 | AAAA |
| ATOM | 1116 | OD2 | ASP | E | 68 | 52.017 | 119.503 | 40.937 | 1.00 | 0.00 | AAAA |
| ATOM | 1117 | C | ASP | E | 68 | 49.349 | 118.489 | 38.646 | 1.00 | 0.00 | AAAA |
| ATOM | 1118 | O | ASP | E | 68 | 50.415 | 117.921 | 38.424 | 1.00 | 0.00 | AAAA |
| ATOM | 1119 | N | LEU | E | 69 | 48.194 | 117.899 | 38.333 | 1.00 | 0.00 | AAAA |
| ATOM | 1121 | CA | LEU | E | 69 | 47.982 | 116.531 | 37.892 | 1.00 | 0.00 | AAAA |
| ATOM | 1123 | CB | LEU | E | 69 | 46.981 | 115.958 | 38.896 | 1.00 | 0.00 | AAAA |
| ATOM | 1126 | CG | LEU | E | 69 | 46.617 | 114.474 | 38.792 | 1.00 | 0.00 | AAAA |
| ATOM | 1128 | CD1 | LEU | E | 69 | 47.832 | 113.649 | 39.199 | 1.00 | 0.00 | AAAA |
| ATOM | 1132 | CD2 | LEU | E | 69 | 45.443 | 114.127 | 39.718 | 1..00 | 0.00 | AAAA |
| ATOM | 1136 | C | LEU | E | 69 | 47.591 | 116.403 | 36.425 | 1.00 | 0.00 | AAAA |
| ATOM | 1137 | O | LEU | E | 69 | 48.451 | 115.869 | 35.724 | 1.00 | 0.00 | AAAA |
| ATOM | 1138 | N | PHE | E | 70 | 46.474 | 116.978 | 35.989 | 1.00 | 0.00 | AAAA |
| ATOM | 1140 | CA | PHE | E | 70 | 46.047 | 117.258 | 34.635 | 1.00 | 0.00 | AAAA |
| ATOM | 1142 | CB | PHE | E | 70 | 45.067 | 116.180 | 34.150 | 1.00 | 0.00 | AAAA |
| ATOM | 1145 | CG | PHE | E | 70 | 44.019 | 115.700 | 35.126 | 1.00 | 0.00 | AAAA |
| ATOM | 1146 | CD1 | PHE | E | 70 | 44.281 | 114.508 | 35.802 | 1.00 | 0.00 | AAAA |
| ATOM | 1148 | CD2 | PHE | E | 70 | 42.881 | 116.435 | 35.471 | 1.00 | 0.00 | AAAA |
| ATOM | 1150 | CE1 | PHE | E | 70 | 43.398 | 114.045 | 36.778 | 1.00 | 0.00 | AAAA |
| ATOM | 1152 | CE2 | PHE | E | 70 | 42.053 | 116.030 | 36.532 | 1.00 | 0.00 | AAAA |
| ATOM | 1154 | CZ | PHE | E | 70 | 42.226 | 114.754 | 37.083 | 1.00 | 0.00 | AAAA |
| ATOM | 1156 | C | PHE | E | 70 | 45.647 | 118.697 | 34.316 | 1.00 | 0.00 | AAAA |
| ATOM | 1157 | O | PHE | E | 70 | 44.473 | 118.935 | 34.056 | 1.00 | 0.00 | AAAA |
| ATOM | 1158 | N | PRO | E | 71 | 46.525 | 119.699 | 34.309 | 1.00 | 0.00 | AAAA |
| ATOM | 1159 | CA | PRO | E | 71 | 46.135 | 121.074 | 34.087 | 1.00 | 0.00 | AAAA |
| ATOM | 1161 | CB | PRO | E | 71 | 47.349 | 121.923 | 34.469 | 1.00 | 0.00 | AAAA |
| ATOM | 1164 | CG | PRO | E | 71 | 48.545 | 120.981 | 34.364 | 1.00 | 0.00 | AAAA |
| ATOM | 1167 | CD | PRO | E | 71 | 47.930 | 119.621 | 34.679 | 1.00 | 0.00 | AAAA |
| ATOM | 1170 | C | PRO | E | 71 | 45.648 | 121.330 | 32.673 | 1.00 | 0.00 | AAAA |
| ATOM | 1171 | O | PRO | E | 71 | 44.948 | 122.331 | 32.556 | 1.00 | 0.00 | AAAA |
| ATOM | 1172 | N | ASN | E | 72 | 46.143 | 120.658 | 31.633 | 1.00 | 0.00 | AAAA |
| ATOM | 1174 | CA | ASN | E | 72 | 45.856 | 120.868 | 30.230 | 1.00 | 0.00 | AAAA |
| ATOM | 1176 | CB | ASN | E | 72 | 47.113 | 120.482 | 29.452 | 1.00 | 0.00 | AAAA |
| ATOM | 1179 | CG | ASN | E | 72 | 47.561 | 121.560 | 28.480 | 1.00 | 0.00 | AAAA |
| ATOM | 1180 | OD1 | ASN | E | 72 | 47.983 | 122.630 | 28.936 | 1.00 | 0.00 | AAAA |
| ATOM | 1181 | ND2 | ASN | E | 72 | 47.460 | 121.324 | 27.167 | 1.00 | 0.00 | AAAA |
| ATOM | 1184 | C | ASN | E | 72 | 44.634 | 120.082 | 29.772 | 1.00 | 0.00 | AAAA |
| ATOM | 1185 | O | ASN | E | 72 | 44.497 | 119.792 | 28.593 | 1.00 | 0.00 | AAAA |
| ATOM | 1186 | N | LEU | E | 73 | 43.680 | 119.848 | 30.683 | 1.00 | 0.00 | AAAA |
| ATOM | 1188 | CA | LEU | E | 73 | 42.386 | 119.237 | 30.421 | 1.00 | 0.00 | AAAA |
| ATOM | 1190 | CB | LEU | E | 73 | 41.718 | 118.864 | 31.746 | 1.00 | 0.00 | AAAA |
| ATOM | 1193 | CG | LEU | E | 73 | 40.296 | 118.278 | 31.651 | 1.00 | 0.00 | AAAA |
| ATOM | 1195 | CD1 | LEU | E | 73 | 40.227 | 117.057 | 30.728 | 1.00 | 0.00 | AAAA |
| ATOM | 1199 | CD2 | LEU | E | 73 | 39.810 | 118.000 | 33.067 | 1.00 | 0.00 | AAAA |
| ATOM | 1203 | C | LEU | E | 73 | 41.649 | .120.400 | 29.780 | 1.00 | 0.00 | AAAA |
| ATOM | 1204 | O | LEU | E | 73 | 41.457 | 121.439 | 30.405 | 1.00 | 0.00 | AAAA |
| ATOM | 1205 | N | THR | E | 74 | 41.394 | 120.186 | 28.488 | 1.00 | 0.00 | AAAA |
| ATOM | 1207 | CA | THR | E | 74 | 40.707 | 121.141 | 27.636 | 1.00 | 0.00 | AAAA |
| ATOM | 1209 | CB | THR | E | 74 | 41.393 | 121.000 | 26.280 | 1.00 | 0.00 | AAAA |
| ATOM | 1211 | OG1 | THR | E | 74 | 42.773 | 121.164 | 26.524 | 1.00 | 0.00 | AAAA |
| ATOM | 1213 | CG2 | THR | E | 74 | 40.908 | 122.005 | 25.235 | 1.00 | 0.00 | AAAA |
| ATOM | 1217 | C | THR | E | 74 | 39.213 | 120.870 | 27.591 | 1.00 | 0.00 | AAAA |
| ATOM | 1218 | O | THR | E | 74 | 38.465 | 121.841 | 27.728 | 1.00 | 0.00 | AAAA |
| ATOM | 1219 | N | VAL | E | 75 | 38.741 | 119.623 | 27.439 | 1.00 | 0.00 | AAAA |
| ATOM | 1221 | CA | VAL | E | 75 | 37.371 | 119.228 | 27.230 | 1.00 | 0.00 | AAAA |
| ATOM | 1223 | CB | VAL | E | 75 | 37.109 | 118.810 | 25.781 | 1.00 | 0.00 | AAAA |
| ATOM | 1225 | CG1 | VAL | E | 75 | 35.621 | 118.613 | 25.494 | 1.00 | 0.00 | AAAA |
| ATOM | 1229 | CG2 | VAL | E | 75 | 37.775 | 119.734 | 24.754 | 1.00 | 0.00 | AAAA |
| ATOM | 1233 | C | VAL | E | 75 | 37.074 | 118.107 | 28.217 | 1.00 | 0.00 | AAAA |
| ATOM | 1234 | O | VAL | E | 75 | 37.878 | 117.186 | 28.359 | 1.00 | 0.00 | AAAA |
| ATOM | 1235 | N | NIL | E | 76 | 35.824 | 118.108 | 28.687 | 1.00 | 0.00 | AAAA |
| ATOM | 1237 | CA | ILE | E | 76 | 35.151 | 116.928 | 29.171 | 1.00 | 0.00 | AAAA |
| ATOM | 1239 | CB | ILE | E | 76 | 34.910 | 117.117 | 30.672 | 1.00 | 0.00 | AAAA |
| ATOM | 1241 | CG1 | ILE | E | 76 | 36.175 | 117.430 | 31.474 | 1.00 | 0.00 | AAAA |
| ATOM | 1244 | CG2 | ILE | E | 76 | 34.221 | 115.884 | 31.274 | 1.00 | 0.00 | AAAA |
| ATOM | 1248 | CD1 | ILE | E | 76 | 35.939 | 118.003 | 32.871 | 1.00 | 0.00 | AAAA |
| ATOM | 1252 | C | ILE | E | 76 | 33.847 | 116.914 | 28.379 | 1.00 | 0.00 | AAAA |
| ATOM | 1253 | O | ILE | E | 76 | 32.996 | 117.793 | 28.472 | 1.00 | 0.00 | AAAA |
| ATOM | 1254 | N | ARG | E | 77 | 33.725 | 115.921 | 27.492 | 1.00 | 0.00 | AAAA |
| ATOM | 1256 | CA | ARG | E | 77 | 32.812 | 116.035 | 26.379 | 1.00 | 0.00 | AAAA |
| ATOM | 1258 | CB | ARG | E | 77 | 33.451 | 115.283 | 25.207 | 1.00 | 0.00 | AAAA |
| ATOM | 1261 | CG | ARG | E | 77 | 32.654 | 115.266 | 23.906 | 1.00 | 0.00 | AAAA |
| ATOM | 1264 | CD | ARG | E | 77 | 33.400 | 114.742 | 22.675 | 1.00 | 0.00 | AAAA |
| ATOM | 1267 | NE | ARG | E | 77 | 33.338 | 113.287 | 22.489 | 1.00 | 0.00 | AAAA |
| ATOM | 1269 | CZ | ARG | E | 77 | 34.306 | 112.498 | 22.019 | 1.00 | 0.00 | AAAA |
| ATOM | 1270 | NH1 | ARG | E | 77 | 35.497 | 112.963 | 21.615 | 1.00 | 0.00 | AAAA |
| ATOM | 1273 | NH2 | ARG | E | 77 | 34.075 | 111.182 | 21.926 | 1.00 | 0.00 | AAAA |
| ATOM | 1276 | C | ARG | E | 77 | 31.434 | 115.478 | 26.710 | 1.00 | 0.00 | AAAA |
| ATOM | 1277 | O | ARG | E | 77 | 30.458 | 115.864 | 26.071 | 1.00 | 0.00 | AAAA |
| ATOM | 1278 | N | GLY | E | 78 | 31.332 | 114.615 | 27.729 | 1.00 | 0.00 | AAAA |
| ATOM | 1280 | CA | GLY | E | 78 | 30.144 | 114.337 | 28.513 | 1.00 | 0.00 | AAAA |
| ATOM | 1283 | C | GLY | E | 78 | 29.199 | 113.350 | 27.856 | 1.00 | 0.00 | AAAA |
| ATOM | 1284 | O | GLY | E | 78 | 27.980 | 113.458 | 28.035 | 1.00 | 0.00 | AAAA |
| ATOM | 1285 | N | TRP | E | 79 | 29.683 | 112.381 | 27.076 | .1.00 | 0.00 | AAAA |
| ATOM | 1287 | CA | TRP | E | 79 | 28.942 | 111.335 | 26.402 | 1.00 | 0.00 | AAAA |
| ATOM | 1289 | CB | TRP | E | 79 | 29.781 | 110.760 | 25.263 | 1.00 | 0.00 | AAAA |
| ATOM | 1292 | CG | TRP | E | 79 | 30.091 | 111.481 | 23.993 | 1.00 | 0.00 | AAAA |
| ATOM | 1293 | CD1 | TRP | E | 79 | 29.852 | 112.792 | 23.735 | 1.00 | 0.00 | AAAA |
| ATOM | 1295 | CD2 | TRP | E | 79 | 30.566 | 110.917 | 22.729 | 1.00 | 0.00 | AAAA |
| ATOM | 1296 | NE1 | TRP | E | 79 | 30.220 | 113.125 | 22.442 | 1.00 | 0.00 | AAAA |
| ATOM | 1298 | CE2 | TRP | E | 79 | 30.546 | 111.963 | 21.773 | 1.00 | 0.00 | AAAA |
| ATOM | 1299 | CE3 | TRP | E | 79 | 30.894 | 109.633 | 22.279 | 1.00 | 0.00 | AAAA |
| ATOM | 1301 | CZ2 | TRP | E | 79 | 30.843 | 111.706 | 20.428 | 1.00 | 0.00 | AAAA |
| ATOM | 1303 | CZ3 | TRP | E | 79 | 31.178 | 109.372 | 20.931 | 1.00 | 0.00 | AAAA |
| ATOM | 1305 | CH2 | TRP | E | 79 | 31.205 | 110.415 | 20.001 | 1.00 | 0.00 | AAAA |
| ATOM | 1307 | C | TRP | E | 79 | 28.438 | 110.308 | 27.402 | 1.00 | 0.00 | AAAA |
| ATOM | 1308 | O | TRP | E | 79 | 27.819 | 109.324 | 27.005 | 1.00 | 0.00 | AAAA |
| ATOM | 1309 | N | LYS | E | 80 | 28.601 | 110.579 | 28.698 | 1.00 | 0.00 | AAAA |
| ATOM | 1311 | CA | LYS | E | 80 | 27.874 | 110.108 | 29.867 | 1.00 | 0.00 | AAAA |
| ATOM | 1313 | CB | LYS | E | 80 | 28.245 | 108.670 | 30.229 | 1.00 | 0.00 | AAAA |
| ATOM | 1316 | CG | LYS | E | 80 | 27.371 | 107.909 | 31.238 | 1.00 | 0.00 | AAAA |
| ATOM | 1319 | CD | LYS | E | 80 | 27.783 | 106.465 | 31.520 | 1.00 | 0.00 | AAAA |
| ATOM | 1322 | CE | LYS | E | 80 | 26.891 | 105.714 | 32.500 | 1.00 | 0.00 | AAAA |
| ATOM | 1325 | NZ | LYS | E | 80 | 27.250 | 104.328 | 32.841 | 1.00 | 0.00 | AAAA |
| ATOM | 1329 | C | LYS | E | 80 | 28.233 | 111.073 | 30.987 | 1.00 | 0.00 | AAAA |
| ATOM | 1330 | O | LYS | E | 80 | 29.367 | 111.538 | 31.043 | 1.00 | 0.00 | AAAA |
| ATOM | 1331 | N | LEU | E | 81 | 27.311 | 111.305 | 31.920 | 1.00 | 0.00 | AAAA |
| ATOM | 1333 | CA | LEU | E | 81 | 27.413 | 112.071 | 33.150 | 1.00 | 0.00 | AAAA |
| ATOM | 1335 | CB | LEU | E | 81 | 26.474 | 113.272 | 33.021 | 1.00 | 0.00 | AAAA |
| ATOM | 1338 | CG | LEU | E | 81 | 26.535 | 114.109 | 31.747 | 1.00 | 0.00 | AAAA |
| ATOM | 1340 | CD1 | LEU | E | 81 | 25.322 | 115.017 | 31.500 | 1.00 | 0.00 | AAAA |
| ATOM | 1344 | CD2 | LEU | E | 81 | 27.854 | 114.867 | 31.667 | 1.00 | 0.00 | AAAA |
| ATOM | 1348 | C | LEU | E | 81 | 27.126 | 111.118 | 34.300 | 1.00 | 0.00 | AAAA |
| ATOM | 1349 | O | LEU | E | 81 | 26.358 | 110.179 | 34.123 | 1.00 | 0.00 | AAAA |
| ATOM | 1350 | N | PHE | E | 82 | 27.697 | 111.388 | 35.479 | 1.00 | 0.00 | AAAA |
| ATOM | 1352 | CA | PHE | E | 82 | 27.208 | 110.750 | 36.676 | 1.00 | 0.00 | AAAA |
| ATOM | 1354 | CB | PHE | E | 82 | 28.463 | 110.617 | 37.546 | 1.00 | 0.00 | AAAA |
| ATOM | 1357 | CG | PHE | E | 82 | 28.324 | 109.886 | 38.854 | 1.00 | 0.00 | AAAA |
| ATOM | 1358 | CD1 | PHE | E | 82 | 27.916 | 108.544 | 38.865 | 1.00 | 0.00 | AAAA |
| ATOM | 1360 | CD2 | PHE | E | 82 | 28.399 | 110.611 | 40.038 | 1.00 | 0.00 | AAAA |
| ATOM | 1362 | CE1 | PHE | E | 82 | 27.784 | 107.879 | 40.091 | 1.00 | 0.00 | AAAA |
| ATOM | 1364 | CE2 | PHE | E | 82 | 28.228 | 109.923 | 41.244 | 1.00 | 0.00 | AAAA |
| ATOM | 1366 | CZ | PHE | E | 82 | 27.974 | 108.545 | 41.301 | 1.00 | 0.00 | AAAA |
| ATOM | 1368 | C | PHE | E | 82 | 26.235 | 111.715 | 37.323 | 1.00 | 0.00 | AAAA |
| ATOM | 1369 | O | PHE | E | 82 | 26.700 | 112.739 | 37.822 | 1.00 | 0.00 | AAAA |
| ATOM | 1370 | N | TYR | E | 83 | 24.921 | 111.498 | 37.290 | 1.00 | 0.00 | AAAA |
| ATOM | 1372 | CA | TYR | E | 83 | 23.876 | 112.335 | 37.834 | 1.00 | 0.00 | AAAA |
| ATOM | 1374 | CB | TYR | E | 83 | 23.756 | 112.316 | 39.360 | 1.00 | 0.00 | AAAA |
| ATOM | 1377 | CG | TYR | E | 83 | 23.545 | 110.983 | 40.044 | 1.00 | 0.00 | AAAA |
| ATOM | 1378 | CD1 | TYR | E | 83 | 22.248 | 110.554 | 40.342 | 1.00 | 0.00 | AAAA |
| ATOM | 1380 | CD2 | TYR | E | 83 | 24.664 | 110.178 | 40.330 | 1.00 | 0.00 | AAAA |
| ATOM | 1382 | CE1 | TYR | E | 83 | 22.062 | 109.343 | 41.007 | 1.00 | 0.00 | AAAA |
| ATOM | 1384 | CE2 | TYR | E | 83 | 24.437 | 108.903 | 40.861 | 1.00 | 0.00 | AAAA |
| ATOM | 1386 | CZ | TYR | E | 83 | 23.139 | 108.454 | 41.109 | 1.00 | 0.00 | AAAA |
| ATOM | 1387 | OH | TYR | E | 83 | 22.921 | 107.209 | 41.629 | 1.00 | 0.00 | AAAA |
| ATOM | 1389 | C | TYR | E | 83 | 23.873 | 113.750 | 37.280 | 1.00 | 0.00 | AAAA |
| ATOM | 1390 | O | TYR | E | 83 | 23.780 | 114.700 | 38.046 | 1.00 | 0.00 | AAAA |
| ATOM | 1391 | N | ASN | E | 84 | 23.956 | 113.904 | 35.956 | 1.00 | 0.00 | AAAA |
| ATOM | 1393 | CA | ASN | E | 84 | 24.036 | 115.160 | 35.229 | 1.00 | 0.00 | AAAA |
| ATOM | 1395 | CB | ASN | E | 84 | 22.828 | 116.094 | 35.384 | 1.00 | 0.00 | AAAA |
| ATOM | 1398 | CG | ASN | E | 84 | 22.447 | 117.027 | 34.256 | 1.00 | 0.00 | AAAA |
| ATOM | 1399 | OD1 | ASN | E | 84 | 22.262 | 116.604 | 33.111 | 1.00 | 0.00 | AAAA |
| ATOM | 1400 | ND2 | ASN | E | 84 | 22.121 | 118.308 | 34.463 | 1.00 | 0.00 | AAAA |
| ATOM | 1403 | C | ASN | E | 84 | 25.369 | 115.865 | 35.458 | 1.00 | 0.00 | AAAA |
| ATOM | 1404 | O | ASN | E | 84 | 25.614 | 116.855 | 34.780 | 1.00 | 0.00 | AAAA |
| ATOM | 1405 | N | TYR | E | 85 | 26.212 | 115.444 | 36.408 | 1.00 | 0.00 | AAAA |
| ATOM | 1407 | CA | TYR | E | 85 | 27.489 | 116.063 | 36.694 | 1.00 | 0.00 | AAAA |
| ATOM | 1409 | CB | TYR | E | 85 | 27.851 | 115.913 | 38.168 | 1.00 | 0.00 | AAAA |
| ATOM | 1412 | CG | TYR | E | 85 | 26.903 | 116.422 | 39.226 | 1.00 | 0.00 | AAAA |
| ATOM | 1413 | CD1 | TYR | E | 85 | 26.445 | 117.747 | 39.168 | 1.00 | 0.00 | AAAA |
| ATOM | 1415 | CD2 | TYR | E | 85 | 26.428 | 115.571 | 40.233 | 1.00 | 0.00 | AAAA |
| ATOM | 1417 | CE1 | TYR | E | 85 | 25.300 | 118.088 | 39.905 | 1.00 | 0.00 | AAAA |
| ATOM | 1419 | CE2 | TYR | E | 85 | 25.409 | 116.012 | 41.084 | 1.00 | 0.00 | AAAA |
| ATOM | 1421 | CZ | TYR | E | 85 | 24.846 | 117.281 | 40.945 | 1.00 | 0.00 | AAAA |
| ATOM | 1422 | OH | TYR | E | 85 | 23.828 | 117.667 | 41.783 | 1.00 | 0.00 | AAAA |
| ATOM | 1424 | C | TYR | E | 85 | 28.544 | 115.459 | 35.782 | 1.00 | 0.00 | AAAA |
| ATOM | 1425 | O | TYR | E | 85 | 28.787 | 114.255 | 35.846 | 1.00 | 0.00 | AAAA |
| ATOM | 1426 | N | ALA | E | 86 | 29.284 | 116.279 | 35.028 | 1.00 | 0.00 | AAAA |
| ATOM | 1428 | CA | ALA | E | 86 | 30.401 | 115.819 | 34.234 | 1.00 | 0.00 | AAAA |
| ATOM | 1430 | CB | ALA | E | 86 | 30.631 | 116.866 | 33.138 | 1.00 | 0.00 | AAAA |
| ATOM | 1434 | C | ALA | E | 86 | 31.654 | 115.743 | 35.096 | 1.00 | 0.00 | AAAA |
| ATOM | 1435 | O | ALA | E | 86 | 32.632 | 115.067 | 34.774 | 1.00 | 0.00 | AAAA |
| ATOM | 1436 | N | LEU | E | 87 | 31.595 | 116.634 | 36.084 | 1.00 | 0.00 | AAAA |
| ATOM | 1438 | CA | LEU | E | 87 | 32.680 | 116.900 | 37.002 | 1.00 | 0.00 | AAAA |
| ATOM | 1440 | CB | LEU | E | 87 | 33.100 | 118.354 | 36.842 | 1.00 | 0.00 | AAAA |
| ATOM | 1443 | CG | LEU | E | 87 | 34.333 | 118.831 | 37.611 | 1.00 | 0.00 | AAAA |
| ATOM | 1445 | CD1 | LEU | E | 87 | 35.269 | 119.818 | 36.913 | 1.00 | 0.00 | AAAA |
| ATOM | 1449 | CD2 | LEU | E | 87 | 34.055 | 119.392 | 39.006 | 1.00 | 0.00 | AAAA |
| ATOM | 1453 | C | LEU | E | 87 | 32.059 | 116.704 | 38.383 | 1.00 | 0.00 | AAAA |
| ATOM | 1454 | O | LEU | E | 87 | 31.141 | 117.426 | 38.762 | 1.00 | 0.00 | AAAA |
| ATOM | 1455 | N | VAL | E | 88 | 32.496 | 115.743 | 39.193 | 1.00 | 0.00 | AAAA |
| ATOM | 1457 | CA | VAL | E | 88 | 32.279 | 115.653 | 40.632 | 1.00 | 0.00 | AAAA |
| ATOM | 1459 | CB | VAL | E | 88 | 31.536 | 114.347 | 40:918 | 1.00 | 0.00 | AAAA |
| ATOM | 1461 | CG1 | VAL | E | 88 | 31.325 | 113.972 | 42.388 | 1.00 | 0.00 | AAAA |
| ATOM | 1465 | CG2 | VAL | E | 88 | 30.239 | 114.207 | 40.124 | 1.00 | 0.00 | AAAA |
| ATOM | 1469 | C | VAL | E | 88 | 33.682 | 115.646 | 41.216 | 1.00 | 0.00 | AAAA |
| ATOM | 1470 | O | VAL | E | 88 | 34.527 | 114.908 | 40.730 | 1.00 | 0.00 | AAAA |
| ATOM | 1471 | N | ILE | E | 89 | 33.897 | 116.602 | 42.119 | 1.00 | 0.00 | AAAA |
| ATOM | 1473 | CA | ILE | E | 89 | 35.088 | 116.661 | 42.951 | 1.00 | 0.00 | AAAA |
| ATOM | 1475 | CB | ILE | E | 89 | 35.999 | 117.808 | 42.520 | 1.00 | 0.00 | AAAA |
| ATOM | 1477 | CG1 | ILE | E | 89 | 36.583 | 117.575 | 41.121 | 1.00 | 0.00 | AAAA |
| ATOM | 1480 | CG2 | ILE | E | 89 | 37.104 | 118.322 | 43.444 | 1.00 | 0.00 | AAAA |
| ATOM | 1484 | CD1 | ILE | E | 89 | 37.235 | 118.731 | 40.367 | 1.00 | 0.00 | AAAA |
| ATOM | 1488 | C | ILE | E | 89 | 34.529 | 116.791 | 44.362 | 1.00 | 0.00 | AAAA |
| ATOM | 1489 | O | ILE | E | 89 | 33.911 | 117.787 | 44.742 | 1.00 | 0.00 | AAAA |
| ATOM | 1490 | N | PHE | E | 90 | 34.510 | 115.683 | 45.102 | 1.00 | 0.00 | AAAA |
| ATOM | 1492 | CA | PHE | E | 90 | 34.499 | 115.560 | 46.356 | 1.00 | 0.00 | AAAA |
| ATOM | 1494 | CB | PHE | E | 90 | 32.461 | 114.868 | 46.155 | 1.00 | 0.00 | AAAA |
| ATOM | 1497 | CG | PHE | E | 90 | 31.483 | 114.962 | 47.312 | 1.00 | 0.00 | AAAA |
| ATOM | 1498 | CD1 | PHE | E | 90 | 30.575 | 116.031 | 47.317 | 1.00 | 0.00 | AAAA |
| ATOM | 1500 | CD2 | PHE | E | 90 | 31.603 | 114.151 | 48.441 | 1.00 | 0.00 | AAAA |
| ATOM | 1502 | CE1 | PHE | E | 90 | 29.542 | 116.038 | 48.264 | 1.00 | 0.00 | AAAA |
| ATOM | 1504 | CE2 | PHE | E | 90 | 30.673 | 114.248 | 49.486 | 1.00 | 0.00 | AAAA |
| ATOM | 1506 | CZ | PHE | E | 90 | 29.650 | 115.188 | 49.374 | 1.00 | 0.00 | AAAA |
| ATOM | 1508 | C | PHE | E | 90 | 34.605 | 114.938 | 47.491 | 1.00 | 0.00 | AAAA |
| ATOM | 1509 | O | PHE | E | 90 | 35.375 | 113.977 | 47.371 | 1.00 | 0.00 | AAAA |
| ATOM | 1510 | N | GLU | E | 91 | 34.527 | 115.551 | 48.670 | 1.00 | 0.00 | AAAA |
| ATOM | 1512 | CA | GLU | E | 91 | 35.126 | 115.242 | 49.948 | 1.00 | 0.00 | AAAA |
| ATOM | 1514 | CB | GLU | E | 91 | 34.455 | 114.021 | 50.577 | 1.00 | 0.00 | AAAA |
| ATOM | 1517 | CG | GLU | E | 91 | 34.429 | 114.131 | 52.070 | 1.00 | 0.00 | AAAA |
| ATOM | 1520 | CD | GLU | E | 91 | 33.454 | 112.882 | 52.616 | 1.00 | 0.00 | AAAA |
| ATOM | 1521 | OE1 | GLU | E | 91 | 33.213 | 111.945 | 51.830 | 1.00 | 0.00 | AAAA |
| ATOM | 1522 | OE2 | GLU | E | 91 | 33.152 | 112.843 | 53.828 | 1.00 | 0.00 | AAAA |
| ATOM | 1523 | C | GLU | E | 91 | 36.634 | 115.083 | 49.875 | 1.00 | 0.00 | AAAA |
| ATOM | 1524 | O | GLU | E | 91 | 37.196 | 114.286 | 50.620 | 1.00 | 0.00 | AAAA |
| ATOM | 1525 | N | MET | E | 92 | 37.335 | 115.782 | 48.975 | 1.00 | 0.00 | AAAA |
| ATOM | 1527 | CA | MET | E | 92 | 38.756 | 115.617 | 48.756 | 1.00 | 0.00 | AAAA |
| ATOM | 1529 | CB | MET | E | 92 | 39.222 | 115.939 | 47.327 | 1.00 | 0.00 | AAAA |
| ATOM | 1532 | CG | MET | E | 92 | 38.669 | 114.993 | 46.266 | 1.00 | 0.00 | AAAA |
| ATOM | 1535 | SD | MET | E | 92 | 38.836 | 115.434 | 44.517 | 1.00 | 0.00 | AAAA |
| ATOM | 1536 | CE | MET | E | 92 | 40.598 | 115.097 | 44.245 | 1.00 | 0.00 | AAAA |
| ATOM | 1540 | C | MET | E | 92 | 39.373 | 116.625 | 49.713 | 1.00 | 0.00 | AAAA |
| ATOM | 1541 | O | MET | E | 92 | 39.203 | 117.839 | 49.598 | 1.00 | 0.00 | AAAA |
| ATOM | 1542 | N | THR | E | 93 | 40.086 | 116.156 | 50.742 | 1.00 | 0.00 | AAAA |
| ATOM | 1544 | CA | THR | E | 93 | 40.885 | 116.961 | 51.639 | 1.00 | 0.00 | AAAA |
| ATOM | 1546 | CB | THR | E | 93 | 41.088 | 116.103 | 52.889 | 1.00 | 0.00 | AAAA |
| ATOM | 1548 | OG1 | THR | E | 93 | 41.883 | 114.967 | 52.630 | 1.00 | 0.00 | AAAA |
| ATOM | 1550 | CG2 | THR | E | 93 | 39.816 | 115.800 | 53.669 | 1.00 | 0.00 | AAAA |
| ATOM | 1554 | C | THR | E | 93 | 42.247 | 117.256 | 51.040 | 1.00 | 0.00 | AAAA |
| ATOM | 1555 | O | THR | E | 93 | 42.607 | 116.696 | 50.003 | 1.00 | 0.00 | AAAA |
| ATOM | 1556 | N | ASN | E | 94 | 42.930 | 118.232 | 51.651 | 1.00 | 0.00 | AAAA |
| ATOM | 1558 | CA | ASN | E | 94 | 44.165 | 118.801 | 51.153 | 1.00 | 0.00 | AAAA |
| ATOM | 1560 | CB | ASN | E | 94 | 45.254 | 117.747 | 51.015 | 1.00 | 0.00 | AAAA |
| ATOM | 1563 | CG | ASN | E | 94 | 46.634 | 118.391 | 51.024 | 1.00 | 0.00 | AAAA |
| ATOM | 1564 | OD1 | ASN | E | 94 | 47.343 | 118.565 | 52.008 | 1.00 | 0.00 | AAAA |
| ATOM | 1565 | ND2 | ASN | E | 94 | 47.112 | 118.755 | 49.828 | 1.00 | 0.00 | AAAA |
| ATOM | 1568 | C | ASN | E | 94 | 44.013 | 119.667 | 49.909 | 1.00 | 0.00 | AAAA |
| ATOM | 1569 | O | ASN | E | 94 | 44.810 | 120.552 | 49.624 | 1.00 | 0.00 | AAAA |
| ATOM | 1570 | N | LEU | E | 95 | 43.007 | 119.482 | 49.049 | 1.00 | 0.00 | AAAA |
| ATOM | 1572 | CA | LEU | E | 95 | 42.803 | 120.206 | 47.807 | 1.00 | 0.00 | AAAA |
| ATOM | 1574 | CB | LEU | E | 95 | 41.740 | 119.441 | 47.017 | 1.00 | 0.00 | AAAA |
| ATOM | 1577 | CG | LEU | E | 95 | 41.374 | 120.071 | 45.669 | 1.00 | 0.00 | AAAA |
| ATOM | 1579 | CD1 | LEU | E | 95 | 42.460 | 119.771 | 44.630 | 1.00 | 0.00 | AAAA |
| ATOM | 1583 | CD2 | LEU | E | 95 | 39.998 | 119.563 | 45.270 | 1.00 | 0.00 | AAAA |
| ATOM | 1587 | C | LEU | E | 95 | 42.435 | 121.637 | 48.160 | 1.00 | 0.00 | AAAA |
| ATOM | 1588 | O | LEU | E | 95 | 41.451 | 121.853 | 48.872 | 1.00 | 0.00 | AAAA |
| ATOM | 1589 | N | LYS | E | 96 | 43.243 | 122.601 | 47.718 | 1.00 | 0.00 | AAAA |
| ATOM | 1591 | CA | LYS | E | 96 | 43.119 | 123.997 | 48.106 | 1.00 | 0.00 | AAAA |
| ATOM | 1593 | CB | LYS | E | 96 | 44.445 | 124.513 | 48.667 | 1.00 | 0.00 | AAAA |
| ATOM | 1596 | CG | LYS | E | 96 | 44.774 | 123.779 | 49.974 | 1.00 | 0.00 | AAAA |
| ATOM | 1599 | CD | LYS | E | 96 | 46.189 | 124.062 | 50.459 | 1.00 | 0.00 | AAAA |
| ATOM | 1602 | CE | LYS | E | 96 | 46.539 | 123.045 | 51.556 | 1.00 | 0.00 | AAAA |
| ATOM | 1605 | NZ | LYS | E | 96 | 47.890 | 123.166 | 52.137 | 1.00 | 0.00 | AAAA |
| ATOM | 1609 | C | LYS | E | 96 | 42.653 | 124.851 | 46.938 | 1.00 | 0.00 | AAAA |
| ATOM | 1610 | O | LYS | E | 96 | 42.274 | 126.001 | 47.165 | 1.00 | 0.00 | AAAA |
| ATOM | 1611 | N | ASP | E | 97 | 42.617 | 124.332 | 45.718 | 1.00 | 0.00 | AAAA |
| ATOM | 1613 | CA | ASP | E | 97 | 42.202 | 125.027 | 44.515 | 1.00 | 0.00 | AAAA |
| ATOM | 1615 | CB | ASP | E | 97 | 43.176 | 126.109 | 44.050 | 1.00 | 0.00 | AAAA |
| ATOM | 1618 | CG | ASP | E | 97 | 44.674 | 125.829 | 44.053 | 1.00 | 0.00 | AAAA |
| ATOM | 1619 | OD1 | ASP | E | 97 | 45.339 | 126.229 | 45.024 | 1.00 | 0.00 | AAAA |
| ATOM | 1620 | OD2 | ASP | E | 97 | 45.198 | 125.286 | 43.048 | 1.00 | 0.00 | AAAA |
| ATOM | 1621 | C | ASP | E | 97 | 42.069 | 123.933 | 43.467 | 1.00 | 0.00 | AAAA |
| ATOM | 1622 | O | ASP | E | 97 | 42.606 | 122.835 | 43.595 | 1.00 | 0.00 | AAAA |
| ATOM | 1623 | N | ILE | E | 98 | 41.333 | 124.180 | 42.386 | 1.00 | 0.00 | AAAA |
| ATOM | 1625 | CA | ILE | E | 98 | 41.097 | 123.242 | 41.293 | 1.00 | 0.00 | AAAA |
| ATOM | 1627 | CB | ILE | E | 98 | 39.803 | 123.680 | 40.614 | 1.00 | 0.00 | AAAA |
| ATOM | 1629 | CG1 | ILE | E | 98 | 38.586 | 123.579 | 41.545 | 1.00 | 0.00 | AAAA |
| ATOM | 1632 | CG2 | ILE | E | 98 | 39.567 | 123.020 | 39.260 | 1.00 | 0.00 | AAAA |
| ATOM | 1636 | CD1 | ILE | E | 98 | 38.377 | 122.120 | 41.948 | 1.00 | 0.00 | AAAA |
| ATOM | 1640 | C | ILE | E | 98 | 42.287 | 123.390 | 40.367 | 1.00 | 0.00 | AAAA |
| ATOM | 1641 | O | ILE | E | 98 | 43.104 | 122.470 | 40.332 | 1.00 | 0.00 | AAAA |
| ATOM | 1642 | N | GLY | E | 99 | 42.455 | 124.520 | 39.676 | 1.00 | 0.00 | AAAA |
| ATOM | 1644 | CA | GLY | E | 99 | 43.674 | 124.957 | 39.020 | 1.00 | 0.00 | AAAA |
| ATOM | 1647 | C | GLY | E | 99 | 43.777 | 124.591 | 37.539 | 1.00 | 0.00 | AAAA |
| ATOM | 1648 | O | GLY | E | 99 | 44.766 | 124.854 | 36.870 | 1.00 | 0.00 | AAAA |
| ATOM | 1649 | N | LEU | E | 100 | 42.791 | 123.950 | 36.897 | 1.00 | 0.00 | AAAA |
| ATOM | 1651 | CA | LEU | E | 100 | 42.686 | 123.380 | 35.575 | 1.00 | 0.00 | AAAA |
| ATOM | 1653 | CB | LEU | E | 100 | 41.511 | 122.407 | 35.552 | 1.00 | 0.00 | AAAA |
| ATOM | 1656 | CG | LEU | E | 100 | 41.589 | 121.381 | 36.686 | 1.00 | 0.00 | AAAA |
| ATOM | 1658 | CD1 | LEU | E | 100 | 40.243 | 120.677 | 36.899 | 1.00 | 0.00 | AAAA |
| ATOM | 1662 | CD2 | LEU | E | 100 | 42.760 | 120.436 | 36.402 | 1.00 | 0.00 | AAAA |
| ATOM | 1666 | C | LEU | E | 100 | 42.498 | 124.459 | 34.514 | 1.00 | 0.00 | AAAA |
| ATOM | 1667 | O | LEU | E | 100 | 41.541 | 124.369 | 33.744 | 1.00 | 0.00 | AAAA |
| ATOM | 1668 | N | TYR | E | 101 | 43.267 | 125.542 | 34.405 | 1.00 | 0.00 | AAAA |
| ATOM | 1670 | CA | TYR | E | 101 | 42.971 | 126.796 | 33.742 | 1.00 | 0.00 | AAAA |
| ATOM | 1672 | CB | TYR | E | 101 | 44.119 | 127.749 | 34.067 | 1.00 | 0.00 | AAAA |
| ATOM | 1675 | CG | TYR | E | 101 | 45.416 | 127.429 | 33.369 | 1.00 | 0.00 | AAAA |
| ATOM | 1676 | CD1 | TYR | E | 101 | 45.858 | 128.352 | 32.415 | 1.00 | 0.00 | AAAA |
| ATOM | 1678 | CD2 | TYR | E | 101 | 46.276 | 126.413 | 33.808 | 1.00 | 0.00 | AAAA |
| ATOM | 1680 | CE1 | TYR | E | 101 | 47.011 | 128.006 | 31.682 | 1.00 | 0.00 | AAAA |
| ATOM | 1682 | CE2 | TYR | E | 101 | 47.464 | 126.126 | 33.122 | 1.00 | 0.00 | AAAA |
| ATOM | 1684 | CZ | TYR | E | 101 | 47.762 | 126.871 | 31.973 | 1.00 | 0.00 | AAAA |
| ATOM | 1685 | OH | TYR | E | 101 | 48.899 | 126.498 | 31.302 | 1.00 | 0.00 | AAAA |
| ATOM | 1687 | C | TYR | E | 101 | 42.690 | 126.783 | 32.243 | 1.00 | 0.00 | AAAA |
| ATOM | 1688 | O | TYR | E | 101 | 42.391 | 127.811 | 31.652 | 1.00 | 0.00 | AAAA |
| ATOM | 1689 | N | ASN | E | 102 | 42.845 | 125.640 | 31.577 | 1.00 | 0.00 | AAAA |
| ATOM | 1691 | CA | ASN | E | 102 | 42.705 | 125.428 | 30.153 | 1.00 | 0.00 | AAAA |
| ATOM | 1693 | CB | ASN | E | 102 | 43.720 | 124.390 | 29.690 | 1.00 | 0.00 | AAAA |
| ATOM | 1696 | CG | ASN | E | 102 | 45.121 | 124.976 | 29.777 | 1.00 | 0.00 | AAAA |
| ATOM | 1697 | OD1 | ASN | E | 102 | 45.411 | 125.987 | 29.133 | 1.00 | 0.00 | AAAA |
| ATOM | 1698 | ND2 | ASN | E | 102 | 45.986 | 124.294 | 30.515 | 1.00 | 0.00 | AAAA |
| ATOM | 1701 | C | ASN | E | 102 | 41.326 | 124.826 | 29.904 | 1.00 | 0.00 | AAAA |
| ATOM | 1702 | O | ASN | E | 102 | 40.871 | 124.787 | 28.765 | 1.00 | 0.00 | AAAA |
| ATOM | 1703 | N | LEU | E | 103 | 40.579 | 124.504 | 30.961 | 1.00 | 0.00 | AAAA |
| ATOM | 1705 | CA | LEU | E | 103 | 39.213 | 124.048 | 30.835 | 1.00 | 0.00 | AAAA |
| ATOM | 1707 | CB | LEU | E | 103 | 38.932 | 123.410 | 32.199 | 1.00 | 0.00 | AAAA |
| ATOM | 1710 | CG | LEU | E | 103 | 37.653 | 122.605 | 32.385 | 1.00 | 0.00 | AAAA |
| ATOM | 1712 | CD1 | LEU | E | 103 | 37.318 | 121.605 | 31.277 | 1.00 | 0.00 | AAAA |
| ATOM | 1716 | CD2 | LEU | E | 103 | 37.674 | 121.936 | 33.760 | 1.00 | 0.00 | AAAA |
| ATOM | 1720 | C | LEU | E | 103 | 38.319 | 125.224 | 30.479 | 1.00 | 0.00 | AAAA |
| ATOM | 1721 | O | LEU | E | 103 | 38.070 | 126.082 | 31.333 | 1.00 | 0.00 | AAAA |
| ATOM | 1722 | N | ARG | E | 104 | 37.750 | 125.257 | 29.271 | 1.00 | 0.00 | AAAA |
| ATOM | 1724 | CA | ARG | E | 104 | 36.767 | 126.203 | 28.777 | 1.00 | 0.00 | AAAA |
| ATOM | 1726 | CB | ARG | E | 104 | 37.396 | 127.339 | 27.965 | 1.00 | 0.00 | AAAA |
| ATOM | 1729 | CG | ARG | E | 104 | 38.240 | 128.291 | 28.825 | 1.00 | 0.00 | AAAA |
| ATOM | 1732 | CD | ARG | E | 104 | 38.956 | 129.436 | 28.105 | 1.00 | 0.00 | AAAA |
| ATOM | 1735 | NE | ARG | E | 104 | 37.998 | 130.507 | 27.827 | 1.00 | 0.00 | AAAA |
| ATOM | 1737 | CZ | ARG | E | 104 | 38.357 | 131.741 | 27.474 | 1.00 | 0.00 | AAAA |
| ATOM | 1738 | NH1 | ARG | E | 104 | 39.605 | 132.226 | 27.544 | 1.00 | 0.00 | AAAA |
| ATOM | 1741 | NH2 | ARG | E | 104 | 37.339 | 132.552 | 27.157 | 1.00 | 0.00 | AAAA |
| ATOM | 1744 | C | ARG | E | 104 | 35.653 | 125.473 | 28.040 | 1.00 | 0.00 | AAAA |
| ATOM | 1745 | O | ARG | E | 104 | 34.779 | 126.054 | 27.399 | 1.00 | 0.00 | AAAA |
| ATOM | 1746 | N | ASN | E | 105 | 35.641 | 124.142 | 28.066 | 1.00 | 0.00 | AAAA |
| ATOM | 1748 | CA | ASN | E | 105 | 34.729 | 123.228 | 27.390 | 1.00 | 0.00 | AAAA |
| ATOM | 1750 | CB | ASN | E | 105 | 35.282 | 122.614 | 26.107 | 1.00 | 0.00 | AAAA |
| ATOM | 1753 | CG | ASN | E | 105 | 34.201 | 122.042 | 25.199 | 1.00 | 0.00 | AAAA |
| ATOM | 1754 | OD1 | ASN | E | 105 | 33.454 | 121.119 | 25.517 | 1.00 | 0.00 | AAAA |
| ATOM | 1755 | ND2 | ASN | E | 105 | 34.066 | 122.639 | 24.008 | 1.00 | 0.00 | AAAA |
| ATOM | 1758 | C | ASN | E | 105 | 34.364 | 122.249 | 28.495 | 1.00 | 0.00 | AAAA |
| ATOM | 1759 | O | ASN | E | 105 | 35.157 | 121.414 | 28.919 | 1.00 | 0.00 | AAAA |
| ATOM | 1760 | N | ILE | E | 106 | 33.178 | 122.404 | 29.093 | 1.00 | 0.00 | AAAA |
| ATOM | 1762 | CA | ILE | E | 106 | 32.478 | 121.258 | 29.628 | 1.00 | 0.00 | AAAA |
| ATOM | 1764 | CB | ILE | E | 106 | 32.325 | 121.334 | 31.152 | 1.00 | 0.00 | AAAA |
| ATOM | 1766 | CG1 | ILE | E | 106 | 33.593 | 121.693 | 31.917 | 1.00 | 0.00 | AAAA |
| ATOM | 1769 | CG2 | ILE | E | 106 | 31.660 | 120.048 | 31.653 | 1.00 | 0.00 | AAAA |
| ATOM | 1773 | CD1 | ILE | E | 106 | 33.569 | 121.827 | 33.435 | 1.00 | 0.00 | AAAA |
| ATOM | 1777 | C | ILE | E | 106 | 31.108 | 121.340 | 28.971 | 1.00 | 0.00 | AAAA |
| ATOM | 1778 | O | ILE | E | 106 | 30.447 | 122.366 | 29.140 | 1.00 | 0.00 | AAAA |
| ATOM | 1779 | N | THR | E | 107 | 30.641 | 120.302 | 28.282 | 1.00 | 0.00 | AAAA |
| ATOM | 1781 | CA | THR | E | 107 | 29.377 | 120.343 | 27.581 | 1.00 | 0.00 | AAAA |
| ATOM | 1783 | CB | THR | E | 107 | 29.588 | 120.585 | 26.087 | 1.00 | 0.00 | AAAA |
| ATOM | 1785 | OG1 | THR | E | 107 | 28.376 | 120.779 | 25.394 | 1.00 | 0.00 | AAAA |
| ATOM | 1787 | CG2 | THR | E | 107 | 30.271 | 119.410 | 25.380 | 1.00 | 0.00 | AAAA |
| ATOM | 1791 | C | THR | E | 107 | 28.471 | 119.205 | 28.012 | 1.00 | 0.00 | AAAA |
| ATOM | 1792 | O | THR | E | 107 | 28.848 | 118.215 | 28.636 | 1.00 | 0.00 | AAAA |
| ATOM | 1793 | N | ARG | E | 108 | 27.176 | 119.359 | 27.733 | 1.00 | 0.00 | AAAA |
| ATOM | 1795 | CA | ARG | E | 108 | 26.125 | 118.379 | 27.987 | 1.00 | 0.00 | AAAA |
| ATOM | 1797 | CB | ARG | E | 108 | 26.414 | 116.997 | 27.388 | 1.00 | 0.00 | AAAA |
| ATOM | 1800 | CG | ARG | E | 108 | 26.712 | 117.082 | 25.892 | 1.00 | 0.00 | AAAA |
| ATOM | 1803 | CD | ARG | E | 108 | 26.871 | 115.737 | 25.190 | 1.00 | 0.00 | AAAA |
| ATOM | 1806 | NE | ARG | E | 108 | 27.454 | 115.749 | 23.843 | 1.00 | 0.00 | AAAA |
| ATOM | 1808 | CZ | ARG | E | 108 | 27.257 | 114.807 | 22.913 | 1.00 | 0.00 | AAAA |
| ATOM | 1809 | NH1 | ARG | E | 108 | 26.489 | 113.721 | 23.075 | 1.00 | 0.00 | AAAA |
| ATOM | 1812 | NH2 | ARG | E | 108 | 27.855 | 114.997 | 21.727 | 1.00 | 0.00 | AAAA |
| ATOM | 1815 | C | ARG | E | 108 | 25.609 | 118.420 | 29.414 | 1.00 | 0.00 | AAAA |
| ATOM | 1816 | O | ARG | E | 108 | 24.407 | 118.261 | 29.592 | 1.00 | 0.00 | AAAA |
| ATOM | 1817 | N | GLY | E | 109 | 26.471 | 118.458 | 30.436 | 1.00 | 0.00 | AAAA |
| ATOM | 1819 | CA | GLY | E | 109 | 26.208 | 118.235 | 31.846 | 1.00 | 0.00 | AAAA |
| ATOM | 1822 | C | GLY | E | 109 | 26.557 | 119.500 | 32.614 | 1.00 | 0.00 | AAAA |
| ATOM | 1823 | O | GLY | E | 109 | 27.050 | 120.514 | 32.123 | 1.00 | 0.00 | AAAA |
| ATOM | 1824 | N | ALA | E | 110 | 26.360 | 119.393 | 33.924 | 1.00 | 0.00 | AAAA |
| ATOM | 1826 | CA | ALA | E | 110 | 26.723 | 120.313 | 34.977 | 1.00 | 0.00 | AAAA |
| ATOM | 1828 | CB | ALA | E | 110 | 25.535 | 120.164 | 35.925 | 1.00 | 0.00 | AAAA |
| ATOM | 1832 | C | ALA | E | 110 | 27.997 | 119.873 | 35.703 | 1.00 | 0.00 | AAAA |
| ATOM | 1833 | O | ALA | E | 110 | 28.649 | 118.916 | 35.319 | 1.00 | 0.00 | AAAA |
| ATOM | 1834 | N | ILE | E | 111 | 28.325 | 120.494 | 36.838 | 1.00 | 0.00 | AAAA |
| ATOM | 1836 | CA | ILE | E | 111 | 29.500 | 120.346 | 37.683 | 1.00 | 0.00 | AAAA |
| ATOM | 1838 | CB | ILE | E | 111 | 30.585 | 121.406 | 37.504 | 1.00 | 0.00 | AAAA |
| ATOM | 1840 | CG1 | ILE | E | 111 | 30.389 | 122.821 | 38.066 | 1.00 | 0.00 | AAAA |
| ATOM | 1843 | CG2 | ILE | E | 111 | 30.914 | 121.509 | 36.015 | 1.00 | 0.00 | AAAA |
| ATOM | 1847 | CD1 | ILE | E | 111 | 31.732 | 123.532 | 38.277 | 1.00 | 0.00 | AAAA |
| ATOM | 1851 | C | ILE | E | 111 | 29.014 | 120.315 | 39.119 | 1.00 | 0.00 | AAAA |
| ATOM | 1852 | O | ILE | E | 111 | 27.989 | 120.902 | 39.469 | 1.00 | 0.00 | AAAA |
| ATOM | 1853 | N | ARG | E | 112 | 29.739 | 119.653 | 40.019 | 1.00 | 0.00 | AAAA |
| ATOM | 1855 | CA | ARG | E | 112 | 29.544 | 119.697 | 41.458 | 1.00 | 0.00 | AAAA |
| ATOM | 1857 | CB | ARG | E | 112 | 28.605 | 118.565 | 41.889 | 1.00 | 0.00 | AAAA |
| ATOM | 1860 | CG | ARG | E | 112 | 28.274 | 118.643 | 43.384 | 1.00 | 0.00 | AAAA |
| ATOM | 1863 | CD | ARG | E | 112 | 26.835 | 118.155 | 43.600 | 1.00 | 0.00 | AAAA |
| ATOM | 1866 | NE | ARG | E | 112 | 26.384 | 118.062 | 44.992 | 1.00 | 0.00 | AAAA |
| ATOM | 1868 | CZ | ARG | E | 112 | 25.157 | 117.685 | 45.361 | 1.00 | 0.00 | AAAA |
| ATOM | 1869 | NH1 | ARG | E | 112 | 24.193 | 117.230 | 44.552 | 1.00 | 0.00 | AAAA |
| ATOM | 1872 | NH2 | ARG | E | 112 | 24.852 | 117.757 | 46.664 | 1.00 | 0.00 | AAAA |
| ATOM | 1875 | C | ARG | E | 112 | 30.856 | 119.662 | 42.228 | 1.00 | 0.00 | AAAA |
| ATOM | 1876 | O | ARG | E | 112 | 31.725 | 118.825 | 41.997 | 1.00 | 0.00 | AAAA |
| ATOM | 1877 | N | ILE | E | 113 | 31.071 | 120.572 | 43.190 | 1.00 | 0.00 | AAAA |
| ATOM | 1879 | CA | ILE | E | 113 | 32.281 | 120.837 | 43.948 | 1.00 | 0.00 | AAAA |
| ATOM | 1881 | CB | ILE | E | 113 | 33.129 | 121.977 | 43.369 | 1.00 | 0.00 | AAAA |
| ATOM | 1883 | CG1 | ILE | E | 113 | 33.339 | 121.882 | 41.856 | 1.00 | 0.00 | AAAA |
| ATOM | 1886 | CG2 | ILE | E | 113 | 34.378 | 122.253 | 44.192 | 1.00 | 0.00 | AAAA |
| ATOM | 1890 | CD1 | ILE | E | 113 | 34.093 | 123.049 | 41.220 | 1.00 | 0.00 | AAAA |
| ATOM | 1894 | C | ILE | E | 113 | 31.797 | 120.927 | 45.390 | 1.00 | 0.00 | AAAA |
| ATOM | 1895 | O | ILE | E | 113 | 31.461 | 122.075 | 45.685 | 1.00 | 0.00 | AAAA |
| ATOM | 1896 | N | GLU | E | 114 | 31.898 | 119.901 | 46.233 | 1.00 | 0.00 | AAAA |
| ATOM | 1898 | CA | GLU | E | 114 | 31.229 | 119.946 | 47.511 | 1.00 | 0.00 | AAAA |
| ATOM | 1900 | CB | GLU | E | 114 | 29.758 | 119.555 | 47.374 | 1.00 | 0.00 | AAAA |
| ATOM | 1903 | CG | GLU | E | 114 | 28.956 | 119.757 | 48.650 | 1.00 | 0.00 | AAAA |
| ATOM | 1906 | CD | GLU | E | 114 | 27.519 | 119.271 | 48.515 | 1.00 | 0.00 | AAAA |
| ATOM | 1907 | OE1 | GLU | E | 114 | 26.962 | 119.068 | 47.416 | 1.00 | 0.00 | AAAA |
| ATOM | 1908 | OE2 | GLU | E | 114 | 26.817 | 119.040 | 49.521 | 1.00 | 0.00 | AAAA |
| ATOM | 1909 | C | GLU | E | 114 | 31.949 | 119.058 | 48.515 | 1.00 | 0.00 | AAAA |
| ATOM | 1910 | O | GLU | E | 114 | 32.688 | 118.121 | 48.210 | 1.00 | 0.00 | AAAA |
| ATOM | 1911 | N | LYS | E | 115 | 31.906 | 119.425 | 49.792 | 1.00 | 0.00 | AAAA |
| ATOM | 1913 | CA | LYS | E | 115 | 32.441 | 118.826 | 51.002 | 1.00 | 0.00 | AAAA |
| ATOM | 1915 | CB | LYS | E | 115 | 31.748 | 117.486 | 51.213 | 1.00 | 0.00 | AAAA |
| ATOM | 1918 | CG | LYS | E | 115 | 31.101 | 117.281 | 52.586 | 1.00 | 0.00 | AAAA |
| ATOM | 1921 | CD | LYS | E | 115 | 30.775 | 115.828 | 52.927 | 1.00 | 0.00 | AAAA |
| ATOM | 1924 | CE | LYS | E | 115 | 30.800 | 115.478 | 54.410 | 1.00 | 0.00 | AAAA |
| ATOM | 1927 | NZ | LYS | E | 115 | 29.711 | 114.570 | 54.790 | 1.00 | 0.00 | AAAA |
| ATOM | 1931 | C | LYS | E | 115 | 33.955 | 118.823 | 51.175 | 1.00 | 0.00 | AAAA |
| ATOM | 1932 | O | LYS | E | 115 | 34.424 | 118.321 | 52.201 | 1.00 | 0.00 | AAAA |
| ATOM | 1933 | N | ASN | E | 116 | 34.700 | 119.422 | 50.240 | 1.00 | -0.00 | AAAA |
| ATOM | 1935 | CA | ASN | E | 116 | 36.143 | 119.554 | 50.201 | 1.00 | 0.00 | AAAA |
| ATOM | 1937 | CB | ASN | E | 116 | 36.580 | 120.035 | 48.816 | 1.00 | 0.00 | AAAA |
| ATOM | 1940 | CG | ASN | E | 116 | 36.215 | 119.166 | 47.627 | 1.00 | 0.00 | AAAA |
| ATOM | 1941 | OD1 | ASN | E | 116 | 36.629 | 118.016 | 47.518 | 1.00 | 0.00 | AAAA |
| ATOM | 1942 | ND2 | ASN | E | 116 | 35.357 | 119.725 | 46.771 | 1.00 | 0.00 | AAAA |
| ATOM | 1945 | C | ASN | E | 116 | 36.595 | 120.564 | 51.241 | 1.00 | 0.00 | AAAA |
| ATOM | 1946 | O | ASN | E | 116 | 36.335 | 121.748 | 51.046 | 1.00 | 0.00 | AAAA |
| ATOM | 1947 | N | ALA | E | 117 | 37.254 | 120.043 | 52.280 | 1.00 | 0.00 | AAAA |
| ATOM | 1949 | CA | ALA | E | 117 | 37.646 | 120.649 | 53.534 | 1.00 | 0.00 | AAAA |
| ATOM | 1951 | CB | ALA | E | 117 | 38.472 | 119.680 | 54.382 | 1.00 | 0.00 | AAAA |
| ATOM | 1955 | C | ALA | E | 117 | 38.373 | 121.988 | 53.473 | 1.00 | 0.00 | AAAA |
| ATOM | 1956 | O | ALA | E | 117 | 38.105 | 122.880 | 54.273 | 1.00 | 0.00 | AAAA |
| ATOM | 1957 | N | ASP | E | 118 | 39.280 | 122.191 | 52.518 | 1.00 | 0.00 | AAAA |
| ATOM | 1959 | CA | ASP | E | 118 | 40.288 | 123.230 | 52.444 | 1.00 | 0.00 | AAAA |
| ATOM | 1961 | CB | ASP | E | 118 | 41.638 | 122.812 | 53.043 | 1.00 | 0.00 | AAAA |
| ATOM | 1964 | CG | ASP | E | 118 | 42.519 | 123.842 | 53.736 | 1.00 | 0.00 | AAAA |
| ATOM | 1965 | OD1 | ASP | E | 118 | 41.966 | 124.761 | 54.382 | 1.00 | 0.00 | AAAA |
| ATOM | 1966 | OD2 | ASP | E | 118 | 43.742 | 123.635 | 53.710 | 1.00 | 0.00 | AAAA |
| ATOM | 1967 | C | ASP | E | 118 | 40.238 | 123.935 | 51.096 | 1.00 | 0.00 | AAAA |
| ATOM | 1968 | O | ASP | E | 118 | 41.132 | 124.757 | 50.906 | 1.00 | 0.00 | AAAA |
| ATOM | 1969 | N | LEU | E | 119 | 39.243 | 123.723 | 50.234 | 1.00 | 0.00 | AAAA |
| ATOM | 1971 | CA | LEU | E | 119 | 39.181 | 124.210 | 48.869 | 1.00 | 0.00 | AAAA |
| ATOM | 1973 | CB | LEU | E | 119 | 38.332 | 123.189 | 48.112 | 1.00 | 0.00 | AAAA |
| ATOM | 1976 | CG | LEU | E | 119 | 38.049 | 123.468 | 46.637 | 1.00 | 0.00 | AAAA |
| ATOM | 1978 | CD1 | LEU | E | 119 | 39.336 | 123.596 | 45.832 | 1.00 | 0.00 | AAAA |
| ATOM | 1982 | CD2 | LEU | E | 119 | 37.235 | 122.310 | 46.059 | 1.00 | 0.00 | AAAA |
| ATOM | 1986 | C | LEU | E | 119 | 38.671 | 125.641 | 48.780 | 1.00 | 0.00 | AAAA |
| ATOM | 1987 | O | LEU | E | 119 | 37.610 | 125.993 | 49.299 | 1.00 | 0.00 | AAAA |
| ATOM | 1988 | N | CYS | E | 120 | 39.453 | 126.609 | 48.287 | 1.00 | 0.00 | AAAA |
| ATOM | 1990 | CA | CYS | E | 120 | 39.154 | 128.005 | 48.050 | 1.00 | 0.00 | AAAA |
| ATOM | 1992 | CB | CYS | E | 120 | 40.265 | 128.888 | 48.638 | 1.00 | 0.00 | AAAA |
| ATOM | 1995 | SG | CYS | E | 120 | 40.105 | 128.890 | 50.436 | 1.00 | 0.00 | AAAA |
| ATOM | 1996 | C | CYS | E | 120 | 39.084 | 128.155 | 46.537 | 1.00 | 0.00 | AAAA |
| ATOM | 1997 | O | CYS | E | 120 | 39.353 | 127.192 | 45.821 | 1.00 | 0.00 | AAAA |
| ATOM | 1998 | N | TYR | E | 121 | 38.735 | 129.309 | 45.965 | 1.00 | 0.00 | AAAA |
| ATOM | 2000 | CA | TYR | E | 121 | 38.886 | 129.711 | 44.588 | 1.00 | 0.00 | AAAA |
| ATOM | 2002 | CB | TYR | E | 121 | 40.336 | 129.732 | 44.098 | 1.00 | 0.00 | AAAA |
| ATOM | 2005 | CG | TYR | E | 121 | 41.372 | 130.688 | 44.641 | 1.00 | 0.00 | AAAA |
| ATOM | 2006 | CD1 | TYR | E | 121 | 41.491 | 131.952 | 44.063 | 1.00 | 0.00 | AAAA |
| ATOM | 2008 | CD2 | TYR | E | 121 | 42.331 | 130.355 | 45.597 | 1.00 | 0.00 | AAAA |
| ATOM | 2010 | CE1 | TYR | E | 121 | 42.331 | 132.966 | 44.542 | 1.00 | 0.00 | AAAA |
| ATOM | 2012 | CE2 | TYR | E | 121 | 43.209 | 131.338 | 46.060 | 1.00 | 0.00 | AAAA |
| ATOM | 2014 | CZ | TYR | E | 121 | 43.103 | 132.687 | 45.672 | 1.00 | 0.00 | AAAA |
| ATOM | 2015 | OH | TYR | E | 121 | 44.014 | 133.587 | 46.126 | 1.00 | 0.00 | AAAA |
| ATOM | 2017 | C | TYR | E | 121 | 37.895 | 129.130 | 43.596 | 1.00 | 0.00 | AAAA |
| ATOM | 2018 | O | TYR | E | 121 | 38.269 | 128.746 | 42.486 | 1.00 | 0.00 | AAAA |
| ATOM | 2019 | N | LEU | E | 122 | 36.661 | 128.932 | 44.066 | 1.00 | 0.00 | AAAA |
| ATOM | 2021 | CA | LEU | E | 122 | 35.590 | 128.339 | 43.282 | 1.00 | 0.00 | AAAA |
| ATOM | 2023 | CB | LEU | E | 122 | 34.904 | 127.372 | 44.244 | 1.00 | 0.00 | AAAA |
| ATOM | 2026 | CG | LEU | E | 122 | 35.740 | 126.227 | 44.842 | 1.00 | 0.00 | AAAA |
| ATOM | 2028 | CD1 | LEU | E | 122 | 34.927 | 125.403 | 45.827 | 1.00 | 0.00 | AAAA |
| ATOM | 2032 | CD2 | LEU | E | 122 | 36.398 | 125.418 | 43.727 | 1.00 | 0.00 | AAAA |
| ATOM | 2036 | C | LEU | E | 122 | 34.557 | 129.415 | 42.988 | 1.00 | 0.00 | AAAA |
| ATOM | 2037 | O | LEU | E | 122 | 33.909 | 129.336 | 41.949 | 1.00 | 0.00 | AAAA |
| ATOM | 2038 | N | SER | E | 123 | 34.398 | 130.336 | 43.939 | 1.00 | 0.00 | AAAA |
| ATOM | 2040 | CA | SER | E | 123 | 33.561 | 131.508 | 43.816 | 1.00 | 0.00 | AAAA |
| ATOM | 2042 | CB | SER | E | 123 | 33.438 | 132.067 | 45.242 | 1.00 | 0.00 | AAAA |
| ATOM | 2045 | OG | SER | E | 123 | 34.676 | 132.118 | 45.922 | 1.00 | 0.00 | AAAA |
| ATOM | 2047 | C | SER | E | 123 | 34.264 | 132.466 | 42.861 | 1.00 | 0.00 | AAAA |
| ATOM | 2048 | O | SER | E | 123 | 33.647 | 133.159 | 42.058 | 1.00 | 0.00 | AAAA |
| ATOM | 2049 | N | THR | E | 124 | 35.594 | 132.460 | 42.919 | 1.00 | 0.00 | AAAA |
| ATOM | 2051 | CA | THR | E | 124 | 36.510 | 133.292 | 42.160 | 1.00 | 0.00 | AAAA |
| ATOM | 2053 | CB | THR | E | 124 | 37.840 | 133.214 | 42.920 | 1.00 | 0.00 | AAAA |
| ATOM | 2055 | OG1 | THR | E | 124 | 37.567 | 133.088 | 44.303 | 1.00 | 0.00 | AAAA |
| ATOM | 2057 | CG2 | THR | E | 124 | 38.711 | 134.451 | 42.729 | 1.00 | 0.00 | AAAA |
| ATOM | 2061 | C | THR | E | 124 | 36.604 | 132.873 | 40.694 | 1.00 | 0.00 | AAAA |
| ATOM | 2062 | O | THR | E | 124 | 37.164 | 133.617 | 39.900 | 1.00 | 0.00 | AAAA |
| ATOM | 2063 | N | VAL | E | 125 | 36.120 | 131.698 | 40.283 | 1.00 | 0.00 | AAAA |
| ATOM | 2065 | CA | VAL | E | 125 | 35.999 | 131.262 | 38.906 | 1.00 | 0.00 | AAAA |
| ATOM | 2067 | CB | VAL | E | 125 | 36.241 | 129.763 | 38.744 | 1.00 | 0.00 | AAAA |
| ATOM | 2069 | CG1 | VAL | E | 125 | 36.054 | 129.148 | 37.358 | 1.00 | 0.00 | AAAA |
| ATOM | 2073 | CG2 | VAL | E | 125 | 37.641 | 129.369 | 39.219 | 1.00 | 0.00 | AAAA |
| ATOM | 2077 | C | VAL | E | 125 | 34.627 | 131.698 | 38.414 | 1.00 | 0.00 | AAAA |
| ATOM | 2078 | O | VAL | E | 125 | 33.683 | 131.621 | 39.211 | 1.00 | 0.00 | AAAA |
| ATOM | 2079 | N | ASP | E | 126 | 34.495 | 132.315 | 37.242 | 1.00 | 0.00 | AAAA |
| ATOM | 2081 | CA | ASP | E | 126 | 33.187 | 132.331 | 36.615 | 1.00 | 0.00 | AAAA |
| ATOM | 2083 | CB | ASP | E | 126 | 33.111 | 133.678 | 35.886 | 1.00 | 0.00 | AAAA |
| ATOM | 2086 | CG | ASP | E | 126 | 31.847 | 133.881 | 35.068 | 1.00 | 0.00 | AAAA |
| ATOM | 2087 | OD1 | ASP | E | 126 | 30.871 | 133.116 | 35.180 | 1.00 | 0.00 | AAAA |
| ATOM | 2088 | OD2 | ASP | E | 126 | 31.763 | 134.937 | 34.397 | 1.00 | 0.00 | AAAA |
| ATOM | 2089 | C | ASP | E | 126 | 33.088 | 131.137 | 35.678 | 1.00 | 0.00 | AAAA |
| ATOM | 2090 | O | ASP | E | 126 | 33.972 | 130.932 | 34.845 | 1.00 | 0.00 | AAAA |
| ATOM | 2091 | N | TRP | E | 127 | 32.100 | 130.251 | 35.899 | 1.00 | 0.00 | AAAA |
| ATOM | 2093 | CA | TRP | E | 127 | 31.890 | 129.003 | 35.197 | 1.00 | 0.00 | AAAA |
| ATOM | 2095 | CB | TRP | E | 127 | 31.357 | 127.925 | 36.141 | 1.00 | 0.00 | AAAA |
| ATOM | 2098 | CG | TRP | E | 127 | 32.231 | 127.682 | 37.333 | 1.00 | 0.00 | AAAA |
| ATOM | 2099 | CD1 | TRP | E | 127 | 32.118 | 128.346 | 38.515 | 1.00 | 0.00 | AAAA |
| ATOM | 2101 | CD2 | TRP | E | 127 | 33.316 | 126.710 | 37.428 | 1.00 | 0.00 | AAAA |
| ATOM | 2102 | NE1 | TRP | E | 127 | 33.154 | 127.911 | 39.329 | 1.00 | 0.00 | AAAA |
| ATOM | 2104 | CE2 | TRP | E | 127 | 33.898 | 126.938 | 38.702 | 1.00 | 0.00 | AAAA |
| ATOM | 2105 | CE3 | TRP | E | 127 | 33.906 | 125.757 | 36.586 | 1.00 | 0.00 | AAAA |
| ATOM | 2107 | CZ2 | TRP | E | 127 | 35.046 | 126.248 | 39.121 | 1.00 | 0.00 | AAAA |
| ATOM | 2109 | CZ3 | TRP | E | 127 | 35.089 | 125.118 | 36.956 | 1.00 | 0.00 | AAAA |
| ATOM | 2111 | CH2 | TRP | E | 127 | 35.589 | 125.297 | 38.252 | 1.00 | 0.00 | AAAA |
| ATOM | 2113 | C | TRP | E | 127 | 30.872 | 129.148 | 34.087 | 1.00 | 0.00 | AAAA |
| ATOM | 2114 | O | TRP | E | 127 | 30.833 | 128.198 | 33.318 | 1.00 | 0.00 | AAAA |
| ATOM | 2115 | N | SER | E | 128 | 30.117 | 130.254 | 34.001 | 1.00 | 0.00 | AAAA |
| ATOM | 2117 | CA | SER | E | 128 | 29.012 | 130.436 | 33.090 | 1.00 | 0.00 | AAAA |
| ATOM | 2119 | CB | SER | E | 128 | 28.221 | 131.702 | 33.417 | 1.00 | 0.00 | AAAA |
| ATOM | 2122 | OG | SER | E | 128 | 28.938 | 132.906 | 33.211 | 1.00 | 0.00 | AAAA |
| ATOM | 2124 | C | SER | E | 128 | 29.455 | 130.390 | 31.629 | 1.00 | 0.00 | AAAA |
| ATOM | 2125 | O | SER | E | 128 | 28.551 | 130.185 | 30.817 | 1.00 | 0.00 | AAAA |
| ATOM | 2126 | N | LEU | E | 129 | 30.719 | 130.624 | 31.281 | 1.00 | 0.00 | AAAA |
| ATOM | 2128 | CA | LEU | E | 129 | 31.384 | 130.682 | 29.997 | 1.00 | 0.00 | AAAA |
| ATOM | 2130 | CB | LEU | E | 129 | 32.123 | 132.009 | 29.803 | 1.00 | 0.00 | AAAA |
| ATOM | 2133 | CG | LEU | E | 129 | 33.272 | 132.422 | 30.723 | 1.00 | 0.00 | AAAA |
| ATOM | 2135 | CD1 | LEU | E | 129 | 34.320 | 133.241 | 29.978 | 1.00 | 0.00 | AAAA |
| ATOM | 2139 | CD2 | LEU | E | 129 | 32.723 | 133.172 | 31.937 | 1.00 | 0.00 | AAAA |
| ATOM | 2143 | C | LEU | E | 129 | 32.303 | 129.480 | 29.822 | 1.00 | 0.00 | AAAA |
| ATOM | 2144 | O | LEU | E | 129 | 32.970 | 129.314 | 28.804 | 1.00 | 0.00 | AAAA |
| ATOM | 2145 | N | ILE | E | 130 | 32.380 | 128.562 | 30.803 | 1.00 | 0.00 | AAAA |
| ATOM | 2147 | CA | ILE | E | 130 | 33.151 | 127.338 | 30.814 | 1.00 | 0.00 | AAAA |
| ATOM | 2149 | CB | ILE | E | 130 | 33.875 | 127.053 | 32.142 | 1.00 | 0.00 | AAAA |
| ATOM | 2151 | CG1 | ILE | E | 130 | 34.845 | 128.187 | 32.456 | 1.00 | 0.00 | AAAA |
| ATOM | 2154 | CG2 | ILE | E | 130 | 34.578 | 125.703 | 32.243 | 1.00 | 0.00 | AAAA |
| ATOM | 2158 | CD1 | ILE | E | 130 | 35.653 | 128.030 | 33.742 | 1.00 | 0.00 | AAAA |
| ATOM | 2162 | C | ILE | E | 130 | 32.194 | 126.234 | 30.415 | 1.00 | 0.00 | AAAA |
| ATOM | 2163 | O | ILE | E | 130 | 32.556 | 125.279 | 29.719 | 1.00 | 0.00 | AAAA |
| ATOM | 2164 | N | LEU | E | 131 | 30.979 | 126.305 | 30.951 | 1.00 | 0.00 | AAAA |
| ATOM | 2166 | CA | LEU | E | 131 | 29.739 | 125.612 | 30.655 | 1.00 | 0.00 | AAAA |
| ATOM | 2168 | CB | LEU | E | 131 | 28.731 | 125.778 | 31.802 | 1.00 | 0.00 | AAAA |
| ATOM | 2171 | CG | LEU | E | 131 | 29.194 | 125.277 | 33.166 | 1.00 | 0.00 | AAAA |
| ATOM | 2173 | CD1 | LEU | E | 131 | 28.357 | 126.035 | 34.185 | 1.00 | 0.00 | AAAA |
| ATOM | 2177 | CD2 | LEU | E | 131 | 29.021 | 123.758 | 33.196 | 1.00 | 0.00 | AAAA |
| ATOM | 2181 | C | LEU | E | 131 | 29.162 | 125.968 | 29.294 | 1.00 | 0.00 | AAAA |
| ATOM | 2182 | O | LEU | E | 131 | 29.360 | 127.045 | 28.726 | 1.00 | 0.00 | AAAA |
| ATOM | 2183 | N | ASP | E | 132 | 28.279 | 125.100 | 28.813 | 1.00 | 0.00 | AAAA |
| ATOM | 2185 | CA | ASP | E | 132 | 27.434 | 125.282 | 27.650 | 1.00 | 0.00 | AAAA |
| ATOM | 2187 | CB | ASP | E | 132 | 28.118 | 124.528 | 26.514 | 1.00 | 0.00 | AAAA |
| ATOM | 2190 | CG | ASP | E | 132 | 27.325 | 124.408 | 25.218 | 1.00 | 0.00 | AAAA |
| ATOM | 2191 | OD1 | ASP | E | 132 | 27.040 | 125.432 | 24.557 | 1.00 | 0.00 | AAAA |
| ATOM | 2192 | OD2 | ASP | E | 132 | 27.124 | 123.271 | 24.750 | 1.00 | 0.00 | AAAA |
| ATOM | 2193 | C | ASP | E | 132 | 25.950 | 125.074 | 27.898 | 1.00 | 0.00 | AAAA |
| ATOM | 2194 | O | ASP | E | 132 | 25.096 | 125.808 | 27.403 | 1.00 | 0.00 | AAAA |
| ATOM | 2195 | N | ALA | E | 133 | 25.639 | 123.971 | 28.591 | 1.00 | 0.00 | AAAA |
| ATOM | 2197 | CA | ALA | E | 133 | 24.357 | 123.636 | 29.180 | 1.00 | 0.00 | AAAA |
| ATOM | 2199 | CB | ALA | E | 133 | 24.401 | 122.233 | 29.767 | 1.00 | 0.00 | AAAA |
| ATOM | 2203 | C | ALA | E | 133 | 23.975 | 124.643 | 30.256 | 1.00 | 0.00 | AAAA |
| ATOM | 2204 | O | ALA | E | 133 | 24.781 | 125.227 | 30.972 | 1.00 | 0.00 | AAAA |
| ATOM | 2205 | N | VAL | E | 134 | 22.662 | 124.788 | 30.450 | 1.00 | 0.00 | AAAA |
| ATOM | 2207 | CA | VAL | E | 134 | 22.062 | 125.779 | 31.306 | 1.00 | 0.00 | AAAA |
| ATOM | 2209 | CB | VAL | E | 134 | 20.934 | 126.429 | 30.494 | 1.00 | 0.00 | AAAA |
| ATOM | 2211 | CG1 | VAL | E | 134 | 21.417 | 127.097 | 29.205 | 1.00 | 0.00 | AAAA |
| ATOM | 2215 | CG2 | VAL | E | 134 | 19.737 | 125.559 | 30.131 | 1.00 | 0.00 | AAAA |
| ATOM | 2219 | C | VAL | E | 134 | 21.580 | 125.133 | 32.592 | 1.00 | 0.00 | AAAA |
| ATOM | 2220 | O | VAL | E | 134 | 20.719 | 125.658 | 33.283 | 1.00 | 0.00 | AAAA |
| ATOM | 2221 | N | SER | E | 135 | 22.296 | 124.059 | 32.958 | 1.00 | 0.00 | AAAA |
| ATOM | 2223 | CA | SER | E | 135 | 22.137 | 123.132 | 34.062 | 1.00 | 0.00 | AAAA |
| ATOM | 2225 | CB | SER | E | 135 | 22.957 | 121.910 | 33.664 | 1.00 | 0.00 | AAAA |
| ATOM | 2228 | OG | SER | E | 135 | 24.242 | 122.228 | 33.195 | 1.00 | 0.00 | AAAA |
| ATOM | 2230 | C | SER | E | 135 | 22.581 | 123.778 | 35.361 | 1.00 | 0.00 | AAAA |
| ATOM | 2231 | O | SER | E | 135 | 23.423 | 124.673 | 35.388 | 1.00 | 0.00 | AAAA |
| ATOM | 2232 | N | ASN | E | 136 | 22.057 | 123.228 | 36.460 | 1.00 | 0.00 | AAAA |
| ATOM | 2234 | CA | ASN | E | 136 | 22.274 | 123.604 | 37.846 | 1.00 | 0.00 | AAAA |
| ATOM | 2236 | CB | ASN | E | 136 | 21.265 | 123.035 | 38.847 | 1.00 | 0.00 | AAAA |
| ATOM | 2239 | CG | ASN | E | 136 | 19.898 | 123.662 | 38.618 | 1.00 | 0.00 | AAAA |
| ATOM | 2240 | OD1 | ASN | E | 136 | 19.289 | 123.506 | 37.559 | 1.00 | 0.00 | AAAA |
| ATOM | 2241 | ND2 | ASN | E | 136 | 19.352 | 124.350 | 39.628 | 1.00 | 0.00 | AAAA |
| ATOM | 2244 | C | ASN | E | 136 | 23.628 | 123.066 | 38.282 | 1.00 | 0.00 | AAAA |
| ATOM | 2245 | O | ASN | E | 136 | 23.826 | 121.874 | 38.031 | 1.00 | 0.00 | AAAA |
| ATOM | 2246 | N | ASN | E | 137 | 24.418 | 123.979 | 38.842 | 1.00 | 0.00 | AAAA |
| ATOM | 2248 | CA | ASN | E | 137 | 25.779 | 123.742 | 39.262 | 1.00 | 0.00 | AAAA |
| ATOM | 2250 | CB | ASN | E | 137 | 26.854 | 124.537 | 38.508 | 1.00 | 0.00 | AAAA |
| ATOM | 2253 | CG | ASN | E | 137 | 26.767 | 124.086 | 37.060 | 1.00 | 0.00 | AAAA |
| ATOM | 2254 | OD1 | ASN | E | 137 | 27.187 | 122.960 | 36.822 | 1.00 | 0.00 | AAAA |
| ATOM | 2255 | ND2 | ASN | E | 137 | 26.265 | 124.883 | 36.111 | 1.00 | 0.00 | AAAA |
| ATOM | 2258 | C | ASN | E | 137 | 25.904 | 124.026 | 40.756 | 1.00 | 0.00 | AAAA |
| ATOM | 2259 | O | ASN | E | 137 | 25.131 | 124.813 | 41.304 | 1.00 | 0.00 | AAAA |
| ATOM | 2260 | N | TYR | E | 138 | 26.914 | 123.420 | 41.384 | 1.00 | 0.00 | AAAA |
| ATOM | 2262 | CA | TYR | E | 138 | 26.942 | 123.295 | 42.826 | 1.00 | 0.00 | AAAA |
| ATOM | 2264 | CB | TYR | E | 138 | 26.468 | 121.913 | 43.298 | 1.00 | 0.00 | AAAA |
| ATOM | 2267 | CG | TYR | E | 138 | 25.068 | 121.447 | 42.982 | 1.00 | 0.00 | AAAA |
| ATOM | 2268 | CD1 | TYR | E | 138 | 24.630 | 121.079 | 41.701 | 1.00 | 0.00 | AAAA |
| ATOM | 2270 | CD2 | TYR | E | 138 | 24.093 | 121.580 | 43.974 | 1.00 | 0.00 | AAAA |
| ATOM | 2272 | CE1 | TYR | E | 138 | 23.282 | 120.762 | 41.509 | 1.00 | 0.00 | AAAA |
| ATOM | 2274 | CE2 | TYR | E | 138 | 22.727 | 121.294 | 43.805 | 1.00 | 0.00 | AAAA |
| ATOM | 2276 | CZ | TYR | E | 138 | 22.341 | 120.831 | 42.544 | 1.00 | 0.00 | AAAA |
| ATOM | 2277 | OH | TYR | E | 138 | 21.057 | 120.521 | 42.203 | 1.00 | 0.00 | AAAA |
| ATOM | 2279 | C | TYR | E | 138 | 28.358 | 123.600 | 43.308 | 1.00 | 0.00 | AAAA |
| ATOM | 2280 | O | TYR | E | 138 | 29.241 | 122.777 | 43.091 | 1.00 | 0.00 | AAAA |
| ATOM | 2281 | N | ILE | E | 139 | 28.571 | 124.795 | 43.879 | 1.00 | 0.00 | AAAA |
| ATOM | 2283 | CA | ILE | E | 139 | 29.865 | 125.396 | 44.099 | 1.00 | 0.00 | AAAA |
| ATOM | 2285 | CB | ILE | E | 139 | 29.858 | 126.699 | 43.293 | 1.00 | 0.00 | AAAA |
| ATOM | 2287 | CG1 | ILE | E | 139 | 29.537 | 126.469 | 41.820 | 1.00 | 0.00 | AAAA |
| ATOM | 2290 | CG2 | ILE | E | 139 | 31.144 | 127.509 | 43.450 | 1.00 | 0.00 | AAAA |
| ATOM | 2294 | CD1 | ILE | E | 139 | 30.586 | 125.719 | 41.000 | 1.00 | 0.00 | AAAA |
| ATOM | 2298 | C | ILE | E | 139 | 30.059 | 125.566 | 45.601 | 1.00 | 0.00 | AAAA |
| ATOM | 2299 | O | ILE | E | 139 | 31.165 | 125.366 | 46.077 | 1.00 | 0.00 | AAAA |
| ATOM | 2300 | N | VAL | E | 140 | 29.034 | 125.981 | 46.346 | 1.00 | 0.00 | AAAA |
| ATOM | 2302 | CA | VAL | E | 140 | 29.146 | 126.387 | 47.735 | 1.00 | 0.00 | AAAA |
| ATOM | 2304 | CB | VAL | E | 140 | 28.048 | 127.406 | 48.055 | 1.00 | 0.00 | AAAA |
| ATOM | 2306 | CG1 | VAL | E | 140 | 28.384 | 128.094 | 49.375 | 1.00 | 0.00 | AAAA |
| ATOM | 2310 | CG2 | VAL | E | 140 | 27.977 | 128.417 | 46.915 | 1.00 | 0.00 | AAAA |
| ATOM | 2314 | C | VAL | E | 140 | 29.097 | 125.123 | 48.581 | 1.00 | 0.00 | AAAA |
| ATOM | 2315 | O | VAL | E | 140 | 28.447 | 124.142 | 48.237 | 1.00 | 0.00 | AAAA |
| ATOM | 2316 | N | GLY | E | 141 | 29.867 | 125.082 | 49.667 | 1.00 | 0.00 | AAAA |
| ATOM | 2318 | CA | GLY | E | 141 | 29.804 | 123.925 | 50.537 | 1.00 | 0.00 | AAAA |
| ATOM | 2321 | C | GLY | E | 141 | 31.163 | 123.220 | 50.571 | 1.00 | 0.00 | AAAA |
| ATOM | 2322 | O | GLY | E | 141 | 31.261 | 122.025 | 50.297 | 1.00 | 0.00 | AAAA |
| ATOM | 2323 | N | ASN | E | 142 | 32.153 | 124.076 | 50.835 | 1.00 | 0.00 | AAAA |
| ATOM | 2325 | CA | ASN | E | 142 | 33.563 | 123.770 | 50.929 | 1.00 | 0.00 | AAAA |
| ATOM | 2327 | CB | ASN | E | 142 | 34.319 | 123.829 | 49.602 | 1.00 | 0.00 | AAAA |
| ATOM | 2330 | CG | ASN | E | 142 | 33.759 | 122.978 | 48.472 | 1.00 | 0.00 | AAAA |
| ATOM | 2331 | OD1 | ASN | E | 142 | 34.035 | 121.783 | 48.440 | 1.00 | 0.00 | AAAA |
| ATOM | 2332 | ND2 | ASN | E | 142 | 32.972 | 123.603 | 47.589 | 1.00 | 0.00 | AAAA |
| ATOM | 2335 | C | ASN | E | 142 | 34.085 | 124.583 | 52.103 | 1.00 | 0.00 | AAAA |
| ATOM | 2336 | O | ASN | E | 142 | 33.359 | 124.701 | 53.087 | 1.00 | 0.00 | AAAA |
| ATOM | 2337 | N | LYS | E | 143 | 35.325 | 125.066 | 52.012 | 1.00 | 0.00 | AAAA |
| ATOM | 2339 | CA | LYS | E | 143 | 35.770 | 125.933 | 53.090 | 1.00 | 0.00 | AAAA |
| ATOM | 2341 | CB | LYS | E | 143 | 37.277 | 125.927 | 52.836 | 1.00 | 0.00 | AAAA |
| ATOM | 2344 | CG | LYS | E | 143 | 38.085 | 127.016 | 53.548 | 1.00 | 0.00 | AAAA |
| ATOM | 2347 | CD | LYS | E | 143 | 39.584 | 126.844 | 53.335 | 1.00 | 0.00 | AAAA |
| ATOM | 2350 | CE | LYS | E | 143 | 40.540 | 127.908 | 53.889 | 1.00 | 0.00 | AAAA |
| ATOM | 2353 | NZ | LYS | E | 143 | 41.937 | 127.481 | 53.803 | 1.00 | 0.00 | AAAA |
| ATOM | 2357 | C | LYS | E | 143 | 35.145 | 127.311 | 52.979 | 1.00 | 0.00 | AAAA |
| ATOM | 2358 | O | LYS | E | 143 | 34.964 | 127.716 | 51.830 | 1.00 | 0.00 | AAAA |
| ATOM | 2359 | N | PRO | E | 144 | 34.673 | 127.959 | 54.052 | 1.00 | 0.00 | AAAA |
| ATOM | 2360 | CA | PRO | E | 144 | 33.963 | 129.222 | 53.970 | 1.00 | 0.00 | AAAA |
| ATOM | 2362 | CB | PRO | E | 144 | 33.415 | 129.572 | 55.345 | 1.00 | 0.00 | AAAA |
| ATOM | 2365 | CG | PRO | E | 144 | 33.624 | 128.312 | 56.182 | 1.00 | 0.00 | AAAA |
| ATOM | 2368 | CD | PRO | E | 144 | 34.455 | 127.337 | 55.339 | 1.00 | 0.00 | AAAA |
| ATOM | 2371 | C | PRO | E | 144 | 34.673 | 130.432 | 53.377 | 1.00 | 0.00 | AAAA |
| ATOM | 2372 | O | PRO | E | 144 | 35.893 | 130.513 | 53.525 | 1.00 | 0.00 | AAAA |
| ATOM | 2373 | N | PRO | E | 145 | 33.998 | 131.374 | 52.712 | 1.00 | 0.00 | AAAA |
| ATOM | 2374 | CA | PRO | E | 145 | 34.542 | 132.548 | 52.068 | 1.00 | 0.00 | AAAA |
| ATOM | 2376 | CB | PRO | E | 145 | 33.410 | 133.221 | 51.295 | 1.00 | 0.00 | AAAA |
| ATOM | 2379 | CG | PRO | E | 145 | 32.099 | 132.654 | 51.863 | 1.00 | 0.00 | AAAA |
| ATOM | 2382 | CD | PRO | E | 145 | 32.555 | 131.384 | 52.583 | 1.00 | 0.00 | AAAA |
| ATOM | 2385 | C | PRO | E | 145 | 35.331 | 133.493 | 52.964 | 1.00 | 0.00 | AAAA |
| ATOM | 2386 | O | PRO | E | 145 | 36.185 | 134.201 | 52.435 | 1.00 | 0.00 | AAAA |
| ATOM | 2387 | N | LYS | E | 146 | 35.222 | 133.476 | 54.292 | 1.00 | 0.00 | AAAA |
| ATOM | 2389 | CA | LYS | E | 146 | 36.033 | 134.136 | 55.298 | 1.00 | 0.00 | AAAA |
| ATOM | 2391 | CB | LYS | E | 146 | 35.253 | 134.035 | 56.602 | 1.00 | 0.00 | AAAA |
| ATOM | 2394 | CG | LYS | E | 146 | 35.682 | 135.068 | 57.640 | 1.00 | 0.00 | AAAA |
| ATOM | 2397 | CD | LYS | E | 146 | 34.714 | 135.156 | 58.822 | 1.00 | 0.00 | AAAA |
| ATOM | 2400 | CE | LYS | E | 146 | 35.038 | 136.314 | 59.777 | 1.00 | 0.00 | AAAA |
| ATOM | 2403 | NZ | LYS | E | 146 | 35.796 | 135.850 | 60.938 | 1.00 | 0.00 | AAAA |
| ATOM | 2407 | C | LYS | E | 146 | 37.437 | 133.540 | 55.419 | 1.00 | 0.00 | AAAA |
| ATOM | 2408 | O | LYS | E | 146 | 38.394 | 134.309 | 55.448 | 1.00 | 0.00 | AAAA |
| ATOM | 2409 | N | GLU | E | 147 | 37.653 | 132.217 | 55.426 | 1.00 | 0.00 | AAAA |
| ATOM | 2411 | CA | GLU | E | 147 | 38.982 | 131.638 | 55.518 | 1.00 | 0.00 | AAAA |
| ATOM | 2413 | CB | GLU | E | 147 | 38.727 | 130.162 | 55.833 | 1.00 | 0.00 | AAAA |
| ATOM | 2416 | CG | GLU | E | 147 | 37.845 | 129.779 | 57.023 | 1.00 | 0.00 | AAAA |
| ATOM | 2419 | CD | GLU | E | 147 | 38.369 | 130.407 | 58.308 | 1.00 | 0.00 | AAAA |
| ATOM | 2420 | OE1 | GLU | E | 147 | 39.576 | 130.252 | 58.622 | 1.00 | 0.00 | AAAA |
| ATOM | 2421 | OE2 | GLU | E | 147 | 37.524 | 130.979 | 59.020 | 1.00 | 0.00 | AAAA |
| ATOM | 2422 | C | GLU | E | 147 | 39.830 | 131.715 | 54.252 | 1.00 | 0.00 | AAAA |
| ATOM | 2423 | O | GLU | E | 147 | 41.040 | 131.907 | 54.243 | 1.00 | 0.00 | AAAA |
| ATOM | 2424 | N | CYS | E | 148 | 39.124 | 131.735 | 53.126 | 1.00 | 0.00 | AAAA |
| ATOM | 2426 | CA | CYS | E | 148 | 39.677 | 132.009 | 51.808 | 1.00 | 0.00 | AAAA |
| ATOM | 2428 | CB | CYS | E | 148 | 38.730 | 131.556 | 50.696 | 1.00 | 0.00 | AAAA |
| ATOM | 2431 | SG | CYS | E | 148 | 38.328 | 129.802 | 50.838 | 1.00 | 0.00 | AAAA |
| ATOM | 2432 | C | CYS | E | 148 | 39.864 | 133.505 | 51.595 | 1.00 | 0.00 | AAAA |
| ATOM | 2433 | O | CYS | E | 148 | 39.541 | 134.380 | 52.390 | 1.00 | 0.00 | AAAA |
| ATOM | 2434 | N | GLY | E | 149 | 40.542 | 133.842 | 50.492 | 1.00 | 0.00 | AAAA |
| ATOM | 2436 | CA | GLY | E | 149 | 40.766 | 135.225 | 50.104 | 1.00 | 0.00 | AAAA |
| ATOM | 2439 | C | GLY | E | 149 | 40.557 | 135.276 | 48.600 | 1.00 | 0.00 | AAAA |
| ATOM | 2440 | O | GLY | E | 149 | 40.306 | 134.284 | 47.914 | 1.00 | 0.00 | AAAA |
| ATOM | 2441 | N | ASP | E | 150 | 40.866 | 136.463 | 48.076 | 1.00 | 0.00 | AAAA |
| ATOM | 2443 | CA | ASP | E | 150 | 40.810 | 136.848 | 46.685 | 1.00 | 0.00 | AAAA |
| ATOM | 2445 | CB | ASP | E | 150 | 39.469 | 137.530 | 46.404 | 1.00 | 0.00 | AAAA |
| ATOM | 2448 | CG | ASP | E | 150 | 38.972 | 138.682 | 47.272 | 1.00 | 0.00 | AAAA |
| ATOM | 2449 | OD1 | ASP | E | 150 | 37.777 | 138.603 | 47.623 | 1.00 | 0.00 | AAAA |
| ATOM | 2450 | OD2 | ASP | E | 150 | 39.802 | 139.586 | 47.517 | 1.00 | 0.00 | AAAA |
| ATOM | 2451 | C | ASP | E | 150 | 42.045 | 137.531 | 46.129 | 1.00 | 0.00 | AAAA |
| ATOM | 2452 | O | ASP | E | 150 | 42.068 | 138.629 | 45.573 | 1.00 | 0.00 | AAAA |
| ATOM | 2453 | N | LEU | E | 151 | 43.164 | 136.913 | 46.507 | 1.00 | 0.00 | AAAA |
| ATOM | 2455 | CA | LEU | E | 151 | 44.475 | 137.526 | 46.496 | 1.00 | 0.00 | AAAA |
| ATOM | 2457 | CB | LEU | E | 151 | 45.118 | 136.982 | 47.778 | 1.00 | 0.00 | AAAA |
| ATOM | 2460 | CG | LEU | E | 151 | 44.378 | 137.389 | 49.048 | 1.00 | 0.00 | AAAA |
| ATOM | 2462 | CD1 | LEU | E | 151 | 45.073 | 136.820 | 50.287 | 1.00 | 0.00 | AAAA |
| ATOM | 2466 | CD2 | LEU | E | 151 | 44.334 | 138.911 | 49.168 | 1.00 | 0.00 | AAAA |
| ATOM | 2470 | C | LEU | E | 151 | 45.231 | 136.939 | 45.310 | 1.00 | 0.00 | AAAA |
| ATOM | 2471 | O | LEU | E | 151 | 45.657 | 135.788 | 45.375 | 1.00 | 0.00 | AAAA |
| ATOM | 2472 | N | CYS | E | 152 | 45.109 | 137.629 | 44.175 | 1.00 | 0.00 | AAAA |
| ATOM | 2474 | CA | CYS | E | 152 | 45.392 | 137.177 | 42.820 | 1.00 | 0.00 | AAAA |
| ATOM | 2476 | CB | CYS | E | 152 | 44.129 | 137.212 | 41.960 | 1.00 | 0.00 | AAAA |
| ATOM | 2479 | SG | CYS | E | 152 | 42.909 | 136.067 | 42.648 | 1.00 | 0.00 | AAAA |
| ATOM | 2480 | C | CYS | E | 152 | 46.551 | 137.989 | 42.263 | 1.00 | 0.00 | AAAA |
| ATOM | 2481 | O | CYS | E | 152 | 46.989 | 138.895 | 42.970 | 1.00 | 0.00 | AAAA |
| ATOM | 2482 | N | PRO | E | 153 | 47.200 | 137.554 | 41.179 | 1.00 | 0.00 | AAAA |
| ATOM | 2483 | CA | PRO | E | 153 | 48.384 | 138.184 | 40.623 | 1.00 | 0.00 | AAAA |
| ATOM | 2485 | CB | PRO | E | 153 | 48.546 | 137.571 | 39.238 | 1.00 | 0.00 | AAAA |
| ATOM | 2488 | CG | PRO | E | 153 | 48.090 | 136.140 | 39.543 | 1.00 | 0.00 | AAAA |
| ATOM | 2491 | CD | PRO | E | 153 | 46.890 | 136.335 | 40.461 | 1.00 | 0.00 | AAAA |
| ATOM | 2494 | C | PRO | E | 153 | 48.325 | 139.709 | 40.578 | 1.00 | 0.00 | AAAA |
| ATOM | 2495 | O | PRO | E | 153 | 47.531 | 140.244 | 39.800 | 1.00 | 0.00 | AAAA |
| ATOM | 2496 | N | GLY | E | 154 | 49.181 | 140.367 | 41.355 | 1.00 | 0.00 | AAAA |
| ATOM | 2498 | CA | GLY | E | 154 | 49.322 | 141.809 | 41.389 | 1.00 | 0.00 | AAAA |
| ATOM | 2501 | C | GLY | E | 154 | 49.014 | 142.352 | 42.777 | 1.00 | 0.00 | AAAA |
| ATOM | 2502 | O | GLY | E | 154 | 49.466 | 143.457 | 43.090 | 1.00 | 0.00 | AAAA |
| ATOM | 2503 | N | THR | E | 155 | 48.289 | 141.600 | 43.611 | 1.00 | 0.00 | AAAA |
| ATOM | 2505 | CA | THR | E | 155 | 47.946 | 141.981 | 44.966 | 1.00 | 0.00 | AAAA |
| ATOM | 2507 | CB | THR | E | 155 | 47.058 | 140.937 | 45.646 | 1.00 | 0.00 | AAAA |
| ATOM | 2509 | OG1 | THR | E | 155 | 46.100 | 140.414 | 44.764 | 1.00 | 0.00 | AAAA |
| ATOM | 2511 | CG2 | THR | E | 155 | 46.336 | 141.357 | 46.920 | 1.00 | 0.00 | AAAA |
| ATOM | 2515 | C | THR | E | 155 | 49.166 | 142.259 | 45.832 | 1.00 | 0.00 | AAAA |
| ATOM | 2516 | O | THR | E | 155 | 49.120 | 143.125 | 46.700 | 1.00 | 0.00 | AAAA |
| ATOM | 2517 | N | MET | E | 156 | 50.353 | 141.710 | 45.575 | 1.00 | 0.00 | AAAA |
| ATOM | 2519 | CA | MET | E | 156 | 51.624 | 142.131 | 46.120 | 1.00 | 0.00 | AAAA |
| ATOM | 2521 | CB | MET | E | 156 | 52.809 | 141.299 | 45.623 | 1.00 | 0.00 | AAAA |
| ATOM | 2524 | CG | MET | E | 156 | 54.027 | 141.430 | 46.540 | 1.00 | 0.00 | AAAA |
| ATOM | 2527 | SD | MET | E | 156 | 55.331 | 140.280 | 46.020 | 1.00 | 0.00 | AAAA |
| ATOM | 2528 | CE | MET | E | 156 | 56.704 | 141.133 | 46.840 | 1.00 | 0.00 | AAAA |
| ATOM | 2532 | C | MET | E | 156 | 51.983 | 143.595 | 45.916 | 1.00 | 0.00 | AAAA |
| ATOM | 2533 | O | MET | E | 156 | 52.541 | 144.220 | 46.820 | 1.00 | 0.00 | AAAA |
| ATOM | 2534 | N | GLU | E | 157 | 51.658 | 144.241 | 44.782 | 1.00 | 0.00 | AAAA |
| ATOM | 2536 | CA | GLU | E | 157 | 51.913 | 145.618 | 44.427 | 1.00 | 0.00 | AAAA |
| ATOM | 2538 | CB | GLU | E | 157 | 51.843 | 145.689 | 42.903 | 1.00 | 0.00 | AAAA |
| ATOM | 2541 | CG | GLU | E | 157 | 52.395 | 147.012 | 42.370 | 1.00 | 0.00 | AAAA |
| ATOM | 2544 | CD | GLU | E | 157 | 53.828 | 147.311 | 42.773 | 1.00 | 0.00 | AAAA |
| ATOM | 2545 | OE1 | GLU | E | 157 | 54.131 | 148.510 | 42.895 | 1.00 | 0.00 | AAAA |
| ATOM | 2546 | OE2 | GLU | E | 157 | 54.667 | 146.386 | 42.885 | 1.00 | 0.00 | AAAA |
| ATOM | 2547 | C | GLU | E | 157 | 50.869 | 146.494 | 45.105 | 1.00 | 0.00 | AAAA |
| ATOM | 2548 | O | GLU | E | 157 | 51.172 | 147.424 | 45.849 | 1.00 | 0.00 | AAAA |
| ATOM | 2549 | N | GLU | E | 158 | 49.620 | 146.121 | 44.773 | 1.00 | 0.00 | AAAA |
| ATOM | 2551 | CA | GLU | E | 158 | 48.403 | 146.855 | 45.017 | 1.00 | 0.00 | AAAA |
| ATOM | 2553 | CB | GLU | E | 158 | 48.412 | 148.224 | 44.346 | 1.00 | 0.00 | AAAA |
| ATOM | 2556 | CG | GLU | E | 158 | 48.595 | 148.278 | 42.825 | 1.00 | 0.00 | AAAA |
| ATOM | 2559 | CD | GLU | E | 158 | 47.777 | 149.393 | 42.174 | 1.00 | 0.00 | AAAA |
| ATOM | 2560 | OE1 | GLU | E | 158 | 46.985 | 150.144 | 42.783 | 1.00 | 0.00 | AAAA |
| ATOM | 2561 | OE2 | GLU | E | 158 | 47.879 | 149.590 | 40.947 | 1.00 | 0.00 | AAAA |
| ATOM | 2562 | C | GLU | E | 158 | 47.203 | 145.949 | 44.764 | 1.00 | 0.00 | AAAA |
| ATOM | 2563 | O | GLU | E | 158 | 47.064 | 144.992 | 45.517 | 1.00 | 0.00 | AAAA |
| ATOM | 2564 | N | LYS | E | 159 | 46.358 | 146.304 | 43.791 | 1.00 | 0.00 | AAAA |
| ATOM | 2566 | CA | LYS | E | 159 | 45.315 | 145.535 | 43.125 | 1.00 | 0.00 | AAAA |
| ATOM | 2568 | CB | LYS | E | 159 | 44.283 | 146.499 | 42.538 | 1.00 | 0.00 | AAAA |
| ATOM | 2571 | CG | LYS | E | 159 | 44.809 | 147.824 | 41.984 | 1.00 | 0.00 | AAAA |
| ATOM | 2574 | CD | LYS | E | 159 | 43.705 | 148.604 | 41.281 | 1.00 | 0.00 | AAAA |
| ATOM | 2577 | CE | LYS | E | 159 | 44.193 | 149.906 | 40.643 | 1.00 | 0.00 | AAAA |
| ATOM | 2580 | NZ | LYS | E | 159 | 44.923 | 150.896 | 41.449 | 1.00 | 0.00 | AAAA |
| ATOM | 2584 | C | LYS | E | 159 | 45.882 | 144.602 | 42.070 | 1.00 | 0.00 | AAAA |
| ATOM | 2585 | O | LYS | E | 159 | 47.023 | 144.844 | 41.679 | 1.00 | 0.00 | AAAA |
| ATOM | 2586 | N | PRO | E | 160 | 45.171 | 143.540 | 41.716 | 1.00 | 0.00 | AAAA |
| ATOM | 2587 | CA | PRO | E | 160 | 45.696 | 142.625 | 40.730 | 1.00 | 0.00 | AAAA |
| ATOM | 2589 | CB | PRO | E | 160 | 44.850 | 141.354 | 40.901 | 1.00 | 0.00 | AAAA |
| ATOM | 2592 | CG | PRO | E | 160 | 43.520 | 141.830 | 41.472 | 1.00 | 0.00 | AAAA |
| ATOM | 2595 | CD | PRO | E | 160 | 43.885 | 143.097 | 42.234 | 1.00 | 0.00 | AAAA |
| ATOM | 2598 | C | PRO | E | 160 | 45.553 | 143.145 | 39.312 | 1.00 | 0.00 | AAAA |
| ATOM | 2599 | O | PRO | E | 160 | 44.931 | 144.179 | 39.047 | 1.00 | 0.00 | AAAA |
| ATOM | 2600 | N | MET | E | 161 | 46.117 | 142.365 | 38.374 | 1.00 | 0.00 | AAAA |
| ATOM | 2602 | CA | MET | E | 161 | 46.160 | 142.566 | 36.945 | 1.00 | 0.00 | AAAA |
| ATOM | 2604 | CB | MET | E | 161 | 47.423 | 141.828 | 36.494 | 1.00 | 0.00 | AAAA |
| ATOM | 2607 | CG | MET | E | 161 | 48.772 | 142.428 | 36.903 | 1.00 | 0.00 | AAAA |
| ATOM | 2610 | SD | MET | E | 161 | 50.089 | 141.533 | 36.034 | 1.00 | 0.00 | AAAA |
| ATOM | 2611 | CE | MET | E | 161 | 50.181 | 139.980 | 36.956 | 1.00 | 0.00 | AAAA |
| ATOM | 2615 | C | MET | E | 161 | 44.967 | 141.920 | 36.244 | 1.00 | 0.00 | AAAA |
| ATOM | 2616 | O | MET | E | 161 | 44.758 | 142.150 | 35.056 | 1.00 | 0.00 | AAAA |
| ATOM | 2617 | N | CYS | E | 162 | 44.173 | 141.130 | 36.965 | 1.00 | 0.00 | AAAA |
| ATOM | 2619 | CA | CYS | E | 162 | 43.074 | 140.351 | 36.420 | 1.00 | 0.00 | AAAA |
| ATOM | 2621 | CB | CYS | E | 162 | 42.652 | 139.292 | 37.442 | 1.00 | 0.00 | AAAA |
| ATOM | 2624 | SG | CYS | E | 162 | 43.893 | 138.249 | 38.238 | 1.00 | 0.00 | AAAA |
| ATOM | 2625 | C | CYS | E | 162 | 41.887 | 141.154 | 35.919 | 1.00 | 0.00 | AAAA |
| ATOM | 2626 | O | CYS | E | 162 | 41.628 | 142.256 | 36.407 | 1.00 | 0.00 | AAAA |
| ATOM | 2627 | N | GLU | E | 163 | 41.105 | 140.691 | 34.950 | 1.00 | 0.00 | AAAA |
| ATOM | 2629 | CA | GLU | E | 163 | 39.731 | 141.043 | 34.649 | 1.00 | 0.00 | AAAA |
| ATOM | 2631 | CB | GLU | E | 163 | 39.294 | 140.497 | 33.286 | 1.00 | 0.00 | AAAA |
| ATOM | 2634 | CG | GLU | E | 163 | 38.186 | 141.193 | 32.486 | 1.00 | 0.00 | AAAA |
| ATOM | 2637 | CD | GLU | E | 163 | 38.472 | 142.654 | 32.162 | 1.00 | 0.00 | AAAA |
| ATOM | 2638 | OE1 | GLU | E | 163 | 38.776 | 143.015 | 31.003 | 1.00 | 0.00 | AAAA |
| ATOM | 2639 | OE2 | GLU | E | 163 | 38.570 | 143.466 | 33.108 | 1.00 | 0.00 | AAAA |
| ATOM | 2640 | C | GLU | E | 163 | 38.722 | 140.796 | 35.766 | 1.00 | 0.00 | AAAA |
| ATOM | 2641 | O | GLU | E | 163 | 38.809 | 139.836 | 36.522 | 1.00 | 0.00 | AAAA |
| ATOM | 2642 | N | LYS | E | 164 | 37.806 | 141.748 | 35.966 | 1.00 | 0.00 | AAAA |
| ATOM | 2644 | CA | LYS | E | 164 | 36.732 | 141.810 | 36.934 | 1.00 | 0.00 | AAAA |
| ATOM | 2646 | CB | LYS | E | 164 | 36.806 | 143.196 | 37.560 | 1.00 | 0.00 | AAAA |
| ATOM | 2649 | CG | LYS | E | 164 | 36.527 | 144.352 | 36.586 | 1.00 | 0.00 | AAAA |
| ATOM | 2652 | CD | LYS | E | 164 | 36.985 | 145.656 | 37.225 | 1.00 | 0.00 | AAAA |
| ATOM | 2655 | CE | LYS | E | 164 | 36.821 | 146.928 | 36.381 | 1.00 | 0.00 | AAAA |
| ATOM | 2658 | NZ | LYS | E | 164 | 37.604 | 148.010 | 37.001 | 1.00 | 0.00 | AAAA |
| ATOM | 2662 | C | LYS | E | 164 | 35.430 | 141.466 | 36.241 | 1.00 | 0.00 | AAAA |
| ATOM | 2663 | O | LYS | E | 164 | 35.161 | 141.626 | 35.052 | 1.00 | 0.00 | AAAA |
| ATOM | 2664 | N | THR | E | 165 | 34.474 | 141.311 | 37.172 | 1.00 | 0.00 | AAAA |
| ATOM | 2666 | CA | THR | E | 165 | 33.049 | 141.223 | 37.019 | 1.00 | 0.00 | AAAA |
| ATOM | 2668 | CB | THR | E | 165 | 32.544 | 139.790 | 37.179 | 1.00 | 0.00 | AAAA |
| ATOM | 2670 | OG1 | THR- | E | 165 | 33.091 | 139.241 | 38.365 | 1.00 | 0.00 | AAAA |
| ATOM | 2672 | CG2 | THR | E | 165 | 32.912 | 138.778 | 36.100 | 1.00 | 0.00 | AAAA |
| ATOM | 2676 | C | THR | E | 165 | 32.406 | 142.291 | 37.903 | 1.00 | 0.00 | AAAA |
| ATOM | 2677 | O | THR | E | 165 | 33.042 | 142.832 | 38.801 | 1.00 | 0.00 | AAAA |
| ATOM | 2678 | N | THR | E | 166 | 31.106 | 142.532 | 37.748 | 1.00 | 0.00 | AAAA |
| ATOM | 2680 | CA | THR | E | 166 | 30.282 | 143.195 | 38.731 | 1.00 | 0.00 | AAAA |
| ATOM | 2682 | CB | THR | E | 166 | 30.232 | 144.719 | 38.604 | 1.00 | 0.00 | AAAA |
| ATOM . | 2684 | OG1 | THR | E | 166 | 30.008 | 145.382 | 39.823 | 1.00 | 0.00 | AAAA |
| ATOM | 2686 | CG2 | THR | E | 166 | 29.149 | 145.162 | 37.623 | 1.00 | 0.00 | AAAA |
| ATOM | 2690 | C | THR | E | 166 | 28.902 | 142.567 | 38.890 | 1.00 | 0.00 | AAAA |
| ATOM | 2691 | O | THR | E | 166 | 28.536 | 141.706 | 38.094 | 1.00 | 0.00 | AAAA |
| ATOM | 2692 | N | ILE | E | 167 | 28.150 | 142.910 | 39.930 | 1.00 | 0.00 | AAAA |
| ATOM | 2694 | CA | ILE | E | 167 | 26.876 | 142.343 | 40.319 | 1.00 | 0.00 | AAAA |
| ATOM | 2696 | CB | ILE | E | 167 | 26.888 | 140.902 | 40.848 | 1.00 | 0.00 | AAAA |
| ATOM | 2698 | CG1 | ILE | E | 167 | 25.486 | 140.370 | 41.125 | 1.00 | 0.00 | AAAA |
| ATOM | 2701 | CG2 | ILE | E | 167 | 27.887 | 140.643 | 41.978 | 1.00 | 0.00 | AAAA |
| ATOM | 2705 | CD1 | ILE | E | 167 | 25.393 | 138.887 | 41.506 | 1.00 | 0.00 | AAAA |
| ATOM | 2709 | C | ILE | E | 167 | 26.021 | 143.464 | 40.879 | 1.00 | 0.00 | AAAA |
| ATOM | 2710 | O | ILE | E | 167 | 25.830 | 143.606 | 42.083 | 1.00 | 0.00 | AAAA |
| ATOM | 2711 | N | ASN | E | 168 | 25.667 | 144.483 | 40.089 | 1.00 | 0.00 | AAAA |
| ATOM | 2713 | CA | ASN | E | 168 | 25.010 | 145.724 | 40.465 | 1.00 | 0.00 | AAAA |
| ATOM | 2715 | CB | ASN | E | 168 | 23.596 | 145.382 | 40.925 | 1.00 | 0.00 | AAAA |
| ATOM | 2718 | CG | ASN | E | 168 | 22.667 | 146.460 | 40.379 | 1.00 | 0.00 | AAAA |
| ATOM | 2719 | OD1 | ASN | E | 168 | 22.386 | 146.547 | 39.186 | 1.00 | 0.00 | AAAA |
| ATOM | 2720 | ND2 | ASN | E | 168 | 22.068 | 147.317 | 41.210 | 1.00 | 0.00 | AAAA |
| ATOM | 2723 | C | ASN | E | 168 | 25.961 | 146.566 | 41.302 | 1.00 | 0.00 | AAAA |
| ATOM | 2724 | O | ASN | E | 168 | 25.575 | 147.129 | 42.320 | 1.00 | 0.00 | AAAA |
| ATOM | 2725 | N | ASN | E | 169 | 27.253 | 146.619 | 40.946 | 1.00 | 0.00 | AAAA |
| ATOM | 2727 | CA | ASN | E | 169 | 28.281 | 147.489 | 41.486 | 1.00 | 0.00 | AAAA |
| ATOM | 2729 | CB | ASN | E | 169 | 27.814 | 148.947 | 41.589 | 1.00 | 0.00 | AAAA |
| ATOM | 2732 | CG | ASN | E | 169 | 28.911 | 149.969 | 41.365 | 1.00 | 0.00 | AAAA |
| ATOM | 2733 | OD1 | ASN | E | 169 | 29.797 | 150.285 | 42.156 | 1.00 | 0.00 | AAAA |
| ATOM | 2734 | ND2 | ASN | E | 169 | 28.843 | 150.609 | 40.181 | 1.00 | 0.00 | AAAA |
| ATOM | 2737 | C | ASN | E | 169 | 29.050 | 146.973 | 42.700 | 1.00 | 0.00 | AAAA |
| ATOM | 2738 | O | ASN | E | 169 | 30.019 | 147.597 | 43.126 | 1.00 | 0.00 | AAAA |
| ATOM | 2739 | N | GLU | E | 170 | 28.800 | 145.717 | 43.051 | 1.00 | 0.00 | AAAA |
| ATOM | 2741 | CA | GLU | E | 170 | 29.640 | 144.865 | 43.866 | 1.00 | 0.00 | AAAA |
| ATOM | 2743 | CB | GLU | E | 170 | 28.754 | 143.746 | 44.419 | 1.00 | 0.00 | AAAA |
| ATOM | 2746 | CG | GLU | E | 170 | 27.628 | 144.183 | 45.351 | 1.00 | 0.00 | AAAA |
| ATOM | 2749 | CD | GLU | E | 170 | 27.999 | 145.118 | 46.491 | 1.00 | 0.00 | AAAA |
| ATOM | 2750 | OE1 | GLU | E | 170 | 27.200 | 146.069 | 46.666 | 1.00 | 0.00 | AAAA |
| ATOM | 2751 | OE2 | GLU | E | 170 | 29.112 | 145.069 | 47.056 | 1.00 | 0.00 | AAAA |
| ATOM | 2752 | C | GLU | E | 170 | 30.618 | 144.259 | 42.875 | 1.00 | 0.00 | AAAA |
| ATOM | 2753 | O | GLU | E | 170 | 30.270 | 143.294 | 42.195 | 1.00 | 0.00 | AAAA |
| ATOM | 2754 | N | TYR | E | 171 | 31.770 | 144.904 | 42.679 | 1.00 | 0.00 | AAAA |
| ATOM | 2756 | CA | TYR | E | 171 | 32.817 | 144.427 | 41.800 | 1.00 | 0.00 | AAAA |
| ATOM | 2758 | CB | TYR | E | 171 | 33.682 | 145.649 | 41.460 | 1.00 | 0.00 | AAAA |
| ATOM | 2761 | CG | TYR | E | 171 | 33.108 | 146.609 | 40.454 | 1.00 | 0.00 | AAAA |
| ATOM | 2762 | CD1 | TYR | E | 171 | 32.351 | 147.759 | 40.724 | 1.00 | 0.00 | AAAA |
| ATOM | 2764 | CD2 | TYR | E | 171 | 33.418 | 146.356 | 39.114 | 1.00 | 0.00 | AAAA |
| ATOM | 2766 | CE1 | TYR | E | 171 | 31.868 | 148.582 | 39.703 | 1.00 | 0.00 | AAAA |
| ATOM | 2768 | CE2 | TYR | E | 171 | 33.042 | 147.194 | 38.054 | 1.00 | 0.00 | AAAA |
| ATOM | 2770 | CZ | TYR | E | 171 | 32.179 | 148.250 | 38.374 | 1.00 | 0.00 | AAAA |
| ATOM | 2771 | OH | TYR | E | 171 | 31.623 | 148.964 | 37.365 | 1.00 | 0.00 | AAAA |
| ATOM | 2773 | C | TYR | E | 171 | 33.693 | 143.340 | 42.403 | 1.00 | 0.00 | AAAA |
| ATOM | 2774 | O | TYR | E | 171 | 33.932 | 143.386 | 43.607 | 1.00 | 0.00 | AAAA |
| ATOM | 2775 | N | ASN | E | 172 | 34.215 | 142.408 | 41.610 | 1.00 | 0.00 | AAAA |
| ATOM | 2777 | CA | ASN | E | 172 | 35.035 | 141.300 | 42.059 | 1.00 | 0.00 | AAAA |
| ATOM | 2779 | CB | ASN | E | 172 | 34.221 | 140.174 | 42.690 | 1.00 | 0.00 | AAAA |
| ATOM | 2782 | CG | ASN | E | 172 | 33.331 | 139.396 | 41.726 | 1.00 | 0.00 | AAAA |
| ATOM | 2783 | OD1 | ASN | E | 172 | 32.425 | 139.980 | 41.138 | 1.00 | 0.00 | AAAA |
| ATOM | 2784 | ND2 | ASN | E | 172 | 33.575 | 138.100 | 41.526 | 1.00 | 0.00 | AAAA |
| ATOM | 2787 | C | ASN | E | 172 | 35.884 | 140.834 | 40.887 | 1.00 | 0.00 | AAAA |
| ATOM | 2788 | O | ASN | E | 172 | 35.435 | 140.757 | 39.745 | 1.00 | 0.00 | AAAA |
| ATOM | 2789 | N | TYR | E | 173 | 37.114 | 140.387 | 41.184 | 1.00 | 0.00 | AAAA |
| ATOM | 2791 | CA | TYR | E | 173 | 38.129 | 139.928 | 40.260 | 1.00 | 0.00 | AAAA |
| ATOM | 2793 | CB | TYR | E | 173 | 39.519 | 140.255 | 40.805 | 1.00 | 0.00 | AAAA |
| ATOM | 2796 | CG | TYR | E | 173 | 39.956 | 141.688 | 40.664 | 1.00 | 0.00 | AAAA |
| ATOM | 2797 | CD1 | TYR | E | 173 | 40.306 | 142.231 | 39.422 | 1.00 | 0.00 | AAAA |
| ATOM | 2799 | CD2 | TYR | E | 173 | 39.965 | 142.517 | 41.790 | 1.00 | 0.00 | AAAA |
| ATOM | 2801 | CE1 | TYR | E | 173 | 40.530 | 143.603 | 39.313 | 1.00 | 0.00 | AAAA |
| ATOM | 2803 | CE2 | TYR | E | 173 | 40.213 | 143.888 | 41.679 | 1.00 | 0.00 | AAAA |
| ATOM | 2805 | CZ | TYR | E | 173 | 40.452 | 144.454 | 40.417 | 1.00 | 0.00 | AAAA |
| ATOM | 2806 | OH | TYR | E | 173 | 40.677 | 145.793 | 40.291 | 1.00 | 0.00 | AAAA |
| ATOM | 2808 | C | TYR | E | 173 | 38.052 | 138.412 | 40.119 | 1.00 | 0.00 | AAAA |
| ATOM | 2809 | O | TYR | E | 173 | 37.997 | 137.673 | 41.094 | 1.00 | 0.00 | AAAA |
| ATOM | 2810 | N | ARG | E | 174 | 38.138 | 137.875 | 38.900 | 1.00 | 0.00 | AAAA |
| ATOM | 2812 | CA | ARG | E | 174 | 38.110 | 136.452 | 38.614 | 1.00 | 0.00 | AAAA |
| ATOM | 2814 | CB | ARG | E | 174 | 37.149 | 136.097 | 37.484 | 1.00 | 0.00 | AAAA |
| ATOM | 2817 | CG | ARG | E | 174 | 35.759 | 136.709 | 37.643 | 1.00 | 0.00 | AAAA |
| ATOM | 2820 | CD | ARG | E | 174 | 35.087 | 136.227 | 38.928 | 1.00 | 0.00 | AAAA |
| ATOM | 2823 | NE | ARG | E | 174 | 33.677 | 136.631 | 38.927 | 1.00 | 0.00 | AAAA |
| ATOM | 2825 | CZ | ARG | E | 174 | 32.653 | 135.858 | 39.323 | 1.00 | 0.00 | AAAA |
| ATOM | 2826 | NH1 | ARG | E | 174 | 32.899 | 134.677 | 39.906 | 1.00 | 0.00 | AAAA |
| ATOM | 2829 | NH2 | ARG | E | 174 | 31.439 | 136.392 | 39.158 | 1.00 | 0.00 | AAAA |
| ATOM | 2832 | C | ARG | E | 174 | 39.454 | 135.825 | 38.282 | 1.00 | 0.00 | AAAA |
| ATOM | 28333 | O | ARG | E | 174 | 40.224 | 136.196 | 37.408 | 1.00 | 0.00 | AAAA |
| ATOM | 2834 | N | CYS | E | 175 | 39.734 | 134.777 | 39.072 | 1.00 | 0.00 | AAAA |
| ATOM | 2836 | CA | CYS | E | 175 | 40.987 | 134.054 | 39.223 | 1.00 | 0.00 | AAAA |
| ATOM | 2838 | CB | CYS | E | 175 | 41.932 | 134.726 | 40.209 | 1.00 | 0.00 | AAAA |
| ATOM | 2841 | SG | CYS | E | 175 | 41.390 | 136.072 | 41.291 | 1.00 | 0.00 | AAAA |
| ATOM | 2842 | C | CYS | E | 175 | 40.683 | 132.630 | 39.695 | 1.00 | 0.00 | AAAA |
| ATOM | 2843 | O | CYS | E | 175 | 39.804 | 132.340 | 40.500 | 1.00 | 0.00 | AAAA |
| ATOM | 2844 | N | TRP | E | 176 | 41.328 | 131.688 | 38.997 | 1.00 | 0.00 | AAAA |
| ATOM | 2846 | CA | TRP | E | 176 | 41.396 | 130.264 | 39.234 | 1.00 | 0.00 | AAAA |
| ATOM | 2848 | CB | TRP | E | 176 | 42.084 | 129.519 | 38.099 | 1.00 | 0.00 | AAAA |
| ATOM | 2851 | CG | TRP | E | 176 | 41.227 | 129.361 | 36.882 | 1.00 | 0.00 | AAAA |
| ATOM | 2852 | CD1 | TRP | E | 176 | 41.406 | 130.033 | 35.723 | 1.00 | 0.00 | AAAA |
| ATOM | 2854 | CD2 | TRP | E | 176 | 40.296 | 128.276 | 36.563 | 1.00 | 0.00 | AAAA |
| ATOM | 2855 | NE1 | TRP | E | 176 | 40.672 | 129.434 | 34.724 | 1.00 | 0.00 | AAAA |
| ATOM | 2857 | CE2 | TRP | E | 176 | 40.014 | 128.303 | 35.175 | 1.00 | 0.00 | AAAA |
| ATOM | 2858 | CE3 | TRP | E | 176 | 39.813 | 127.116 | 37.194 | 1.00 | 0.00 | AAAA |
| ATOM | 2860 | CZ2 | TRP | E | 176 | 39.237 | 127.366 | 34.495 | 1.00 | 0.00 | AAAA |
| ATOM | 2862 | CZ3 | TRP | E | 176 | 39.002 | 126.172 | 36.556 | 1.00 | 0.00 | AAAA |
| ATOM | 2864 | CH2 | TRP | E | 176 | 38.644 | 126.316 | 35.212 | 1.00 | 0.00 | AAAA |
| ATOM | 2866 | C | TRP | E | 176 | 42.165 | 129.981 | 40.513 | 1.00 | 0.00 | AAAA |
| ATOM | 2867 | O | TRP | E | 176 | 41.896 | 128.992 | 41.193 | 1.00 | 0.00 | AAAA |
| ATOM | 2868 | N | THR | E | 177 | 43.223 | 130.771 | 40.701 | 1.00 | 0.00 | AAAA |
| ATOM | 2870 | CA | THR | E | 177 | 44.222 | 130.527 | 41.733 | 1.00 | 0.00 | AAAA |
| ATOM | 2872 | CB | THR | E | 177 | 45.201 | 129.535 | 41.124 | 1.00 | 0.00 | AAAA |
| ATOM | 2874 | OG1 | THR | E | 177 | 45.861 | 130.116 | 40.024 | 1.00 | 0.00 | AAAA |
| ATOM | 2876 | CG2 | THR | E | 177 | 44.773 | 128.117 | 40.734 | 1.00 | 0.00 | AAAA |
| ATOM | 2880 | C | THR | E | 177 | 44.973 | 131.779 | 42.149 | 1.00 | 0.00 | AAAA |
| ATOM | 2881 | O | THR | E | 177 | 44.788 | 132.797 | 41.470 | 1.00 | 0.00 | AAAA |
| ATOM | 2882 | N | THR | E | 178 | 45.736 | 131.843 | 43.241 | 1.00 | 0.00 | AAAA |
| ATOM | 2884 | CA | THR | E | 178 | 46.701 | 132.865 | 43.601 | 1.00 | 0.00 | AAAA |
| ATOM | 2886 | CB | THR | E | 178 | 47.237 | 132.550 | 44.997 | 1.00 | 0.00 | AAAA |
| ATOM | 2888 | OG1 | THR | E | 178 | 47.617 | 133.699 | 45.716 | 1.00 | 0.00 | AAAA |
| ATOM | 2890 | CG2 | THR | E | 178 | 48.490 | 131.672 | 45.125 | 1.00 | 0.00 | AAAA |
| ATOM | 2894 | C | THR | E | 178 | 47.747 | 133.103 | 42.529 | 1.00 | 0.00 | AAAA |
| ATOM | 2895 | O | THR | E | 178 | 48.584 | 133.998 | 42.629 | 1.00 | 0.00 | AAAA |
| ATOM | 2896 | N | ASN | E | 179 | 47.852 | 132.281 | 41.483 | 1.00 | 0.00 | AAAA |
| ATOM | 2898 | CA | ASN | E | 179 | 48.923 | 132.233 | 40.512 | 1.00 | 0.00 | AAAA |
| ATOM | 2900 | CB | ASN | E | 179 | 49.383 | 130.781 | 40.343 | 1.00 | 0.00 | AAAA |
| ATOM | 2903 | CG | ASN | E | 179 | 50.708 | 130.583 | 39.616 | 1.00 | 0.00 | AAAA |
| ATOM | 2904 | OD1 | ASN | E | 179 | 50.907 | 130.707 | 38.410 | 1.00 | 0.00 | AAAA |
| ATOM | 2905 | ND2 | ASN | E | 179 | 51.687 | 130.177 | 40.429 | 1.00 | 0.00 | AAAA |
| ATOM | 2908 | C | ASN | E | 179 | 48.438 | 132.656 | 39.130 | 1.00 | 0.00 | AAAA |
| ATOM | 2909 | O | ASN | E | 179 | 49.166 | 133.236 | 38.330 | 1.00 | 0.00 | AAAA |
| ATOM | 2910 | N | ARG | E | 180 | 44.182 | 132.388 | 38.772 | 1.00 | 0.00 | AAAA |
| ATOM | 2912 | CA | ARG | E | 180 | 46.689 | 132.622 | 37.434 | 1.00 | 0.00 | AAAA |
| ATOM | 2914 | CB | ARG | E | 180 | 46.408 | 131.336 | 36.649 | 1.00 | 0.00 | AAAA |
| ATOM | 2917 | CG | ARG | E | 180 | 47.770 | 130.673 | 36.435 | 1.00 | 0.00 | AAAA |
| ATOM | 2920 | CD | ARG | E | 180 | 47.651 | 129.537 | 35.419 | 1.00 | 0.00 | AAAA |
| ATOM | 2923 | NE | ARG | E | 180 | 48.886 | 128.740 | 35.402 | 1.00 | 0.00 | AAAA |
| ATOM | 2925 | CZ | ARG | E | 180 | 49.780 | 128.750 | 34.406 | 1.00 | 0.00 | AAAA |
| ATOM | 2926 | NH1 | ARG | E | 180 | 49.829 | 129.584 | 33.362 | 1.00 | 0.00 | AAAA |
| ATOM | 2929 | NH2 | ARG | E | 180 | 50.669 | 127.745 | 34.439 | 1.00 | 0.00 | AAAA |
| ATOM | 2932 | C | ARG | E | 180 | 45.377 | 133.389 | 37.513 | 1.00 | 0.00 | AAAA |
| ATOM | 2933 | O | ARG | E | 180 | 44.511 | 132.926 | 38.239 | 1.00 | 0.00 | AAAA |
| ATOM | 2934 | N | CYS | E | 181 | 45.131 | 134.335 | 36.599 | 1.00 | 0.00 | AAAA |
| ATOM | 2936 | CA | CYS | E | 181 | 43.864 | 134.980 | 36.295 | 1.00 | 0.00 | AAAA |
| ATOM | 2938 | CB | CYS | E | 181 | 44.082 | 136.374 | 35.702 | 1.00 | 0.00 | AAAA |
| ATOM | 2941 | SG | CYS | E | 181 | 45.030 | 137.560 | 36.694 | 1.00 | 0.00 | AAAA |
| ATOM | 2942 | C | CYS | E | 181 | 43.096 | 134.048 | 35.362 | 1.00 | 0.00 | AAAA |
| ATOM | 2943 | O | CYS | E | 181 | 43.729 | 133.198 | 34.754 | 1.00 | 0.00 | AAAA |
| ATOM | 2944 | N | GLN | E | 182 | 41.811 | 134.350 | 35.161 | 1.00 | 0.00 | AAAA |
| ATOM | 2946 | CA | GLN | E | 182 | 40.947 | 133.739 | 34.173 | 1.00 | 0.00 | AAAA |
| ATOM | 2948 | CB | GLN | E | 182 | 39.497 | 133.683 | 34.678 | 1.00 | 0.00 | AAAA |
| ATOM | 2951 | CG | GLN | E | 182 | 38.659 | 132.645 | 33.927 | 1.00 | 0.00 | AAAA |
| ATOM | 2954 | CD | GLN | E | 182 | 37.282 | 132.546 | 34.580 | 1.00 | 0.00 | AAAA |
| ATOM | 2955 | OE1 | GLN | E | 182 | 36.950 | 133.252 | 35.533 | 1.00 | 0.00 | AAAA |
| ATOM | 2956 | NE2 | GLN | E | 182 | 36.517 | 131.537 | 34.159 | 1.00 | 0.00 | AAAA |
| ATOM | 2959 | C | GLN | E | 182 | 40.932 | 134.624 | 32.941 | 1.00 | 0.00 | AAAA |
| ATOM | 2960 | O | GLN | E | 182 | 40.812 | 135.871 | 33.041 | 1.00 | 0.00 | AAAA |
| ATOM | 2961 | OXT | GLN | E | 182 | 41.076 | 134.042 | 31.844 | 1.00 | 0.00 | AAAA |
| ATOM | 2962 | N | LEU | E | 693 | 12.374 | 115.145 | 40.001 | 1.00 | 0.00 | AAAB |
| ATOM | 2965 | CA | LEU | E | 693 | 12.575 | 116.103 | 41.058 | 1.00 | 0.00 | AAAB |
| ATOM | 2967 | CB | LEU | E | 693 | 11.443 | 115.900 | 42.064 | 1.00 | 0.00 | AAAB |
| ATOM | 2970 | CG | LEU | E | 693 | 11.336 | 117.051 | 43.065 | 1.00 | 0.00 | AAAB |
| ATOM | 2972 | CD1 | LEU | E | 693 | 10.435 | 118.148 | 42.505 | 1.00 | 0.00 | AAAB |
| ATOM | 2976 | CD2 | LEU | E | 693 | 10.866 | 116.764 | 44.488 | 1.00 | 0.00 | AAAB |
| ATOM | 2980 | C | LEU | E | 693 | 13.963 | 115.802 | 41.624 | 1.00 | 0.00 | AAAB |
| ATOM | 2981 | O | LEU | E | 693 | 14.192 | 114.732 | 42.186 | 1.00 | 0.00 | AAAB |
| ATOM | 2982 | N | LYS | E | 694 | 14.904 | 116.739 | 41.582 | 1.00 | 0.00 | AAAB |
| ATOM | 2984 | CA | LYS | E | 694 | 16.290 | 116.668 | 42.009 | 1.00 | 0.00 | AAAB |
| ATOM | 2986 | CB | LYS | E | 694 | 16.861 | 118.071 | 41.769 | 1.00 | 0.00 | AAAB |
| ATOM | 2989 | CG | LYS | E | 694 | 18.126 | 118.499 | 42.513 | 1.00 | 0.00 | AAAB |
| ATOM | 2992 | CD | LYS | E | 694 | 17.884 | 119.304 | 43.781 | 1.00 | 0.00 | AAAB |
| ATOM | 2995 | CE | LYS | E | 694 | 18.976 | 119.302 | 44.865 | 1.00 | 0.00 | AAAB |
| ATOM | 2998 | NZ | LYS | E | 694 | 18.527 | 119.931 | 46.119 | 1.00 | 0.00 | AAAB |
| ATOM | 3002 | C | LYS | E | 694 | 16.535 | 116.053 | 43.378 | 1.00 | 0.00 | AAAB |
| ATOM | 3003 | O | LYS | E | 694 | 17.557 | 115.402 | 43.568 | 1.00 | 0.00 | AAAB |
| ATOM | 3004 | N | GLU | E | 695 | 15.636 | 116.284 | 44.341 | 1.00 | 0.00 | AAAB |
| ATOM | 3006 | CA | GLU | E | 695 | 15.757 | 115.722 | 45.671 | 1.00 | 0.00 | AAAB |
| ATOM | 3008 | CB | GLU | E | 695 | 14.686 | 116.335 | 46.577 | 1.00 | 0.00 | AAAB |
| ATOM | 3011 | CG | GLU | E | 695 | 15.304 | 116.972 | 47.819 | 1.00 | 0.00 | AAAB |
| ATOM | 3014 | CD | GLU | E | 695 | 15.972 | 118.305 | 47.515 | 1.00 | 0.00 | AAAB |
| ATOM | 3015 | OE1 | GLU | E | 695 | 15.404 | 119.020 | 46.650 | 1.00 | 0.00 | AAAB |
| ATOM | 3016 | OE2 | GLU | E | 695 | 17.052 | 118.656 | 48.025 | 1.00 | 0.00 | AAAB |
| ATOM | 3017 | C | GLU | E | 695 | 15.700 | 114.200 | 45.708 | 1.00 | 0.00 | AAAB |
| ATOM | 3018 | O | GLU | E | 695 | 16.273 | 113.681 | 46.665 | 1.00 | 0.00 | AAAB |
| ATOM | 3019 | N | LEU | E | 696 | 15.124 | 113.487 | 44.736 | 1.00 | 0.00 | AAAB |
| ATOM | 3021 | CA | LEU | E | 696 | 15.247 | 112.063 | 44.491 | 1.00 | 0.00 | AAAB |
| ATOM | 3023 | CB | LEU | E | 696 | 14.150 | 111.672 | 43.500 | 1.00 | 0.00 | AAAB |
| ATOM | 3026 | CG | LEU | E | 696 | 12.723 | 112.046 | 43.913 | 1.00 | 0.00 | AAAB |
| ATOM | 3028 | CD1 | LEU | E | 696 | 11.772 | 112.137 | 42.716 | 1.00 | 0.00 | AAAB |
| ATOM | 3032 | CD2 | LEU | E | 696 | 12.098 | 111.177 | 45.000 | 1.00 | 0.00 | AAAB |
| ATOM | 3036 | C | LEU | E | 696 | 16.571 | 111.683 | 43.852 | 1.00 | 0.00 | AAAB |
| ATOM | 3037 | O | LEU | E | 696 | 16.988 | 110.552 | 44.082 | 1.00 | 0.00 | AAAB |
| ATOM | 3038 | N | GLU | E | 697 | 17.141 | 112.580 | 43.052 | 1.00 | 0.00 | AAAB |
| ATOM | 3040 | CA | GLU | E | 697 | 18.369 | 112.307 | 42.345 | 1.00 | 0.00 | AAAB |
| ATOM | 3042 | CB | GLU | E | 697 | 18.497 | 113.296 | 41.190 | 1.00 | 0.00 | AAAB |
| ATOM | 3045 | CG | GLU | E | 697 | 17.484 | 113.170 | 40.044 | 1.00 | 0.00 | AAAB |
| ATOM | 3048 | CD | GLU | E | 697 | 17.647 | 111.871 | 39.273 | 1.00 | 0.00 | AAAB |
| ATOM | 3049 | OE1 | GLU | E | 697 | 18.758 | 111.308 | 39.184 | 1.00 | 0.00 | AAAB |
| ATOM | 3050 | OE2 | GLU | E | 697 | 16.639 | 111.348 | 38.746 | 1.00 | 0.00 | AAAB |
| ATOM | 3051 | C | GLU | E | 697 | 19.508 | 112.509 | 43.343 | 1.00 | 0.00 | AAAB |
| ATOM | 3052 | O | GLU | E | 697 | 20.393 | 111.664 | 43.385 | 1.00 | 0.00 | AAAB |
| ATOM | 3053 | N | GLU | E | 698 | 19.383 | 113.493 | 44.233 | 1.00 | 0.00 | AAAB |
| ATOM | 3055 | CA | GLU | E | 698 | 20.198 | 113.877 | 45.373 | 1.00 | 0.00 | AAAB |
| ATOM | 3057 | CB | GLU | E | 698 | 19.493 | 115.112 | 45.947 | 1.00 | 0.00 | AAAB |
| ATOM | 3060 | CG | GLU | E | 698 | 20.279 | 115.724 | 47.100 | 1.00 | 0.00 | AAAB |
| ATOM | 3063 | CD | GLU | E | 698 | 21.510 | 116.495 | 46.637 | 1.00 | 0.00 | AAAB |
| ATOM | 3064 | OE1 | GLU | E | 698 | 21.744 | 116.524 | 45.402 | 1.00 | 0.00 | AAAB |
| ATOM | 3065 | OE2 | GLU | E | 698 | 22.354 | 116.876 | 47.468 | 1.00 | 0.00 | AAAB |
| ATOM | 3066 | C | GLU | E | 698 | 20.251 | 112.680 | 46.319 | 1.00 | 0.00 | AAAB |
| ATOM | 3067 | O | GLU | E | 698 | 21.328 | 112.327 | 46.786 | 1.00 | 0.00 | AAAB |
| ATOM | 3068 | N | SER | E | 699 | 19.087 | 112.084 | 46.579 | 1.00 | 0.00 | AAAB |
| ATOM | 3070 | CA | SER | E | 699 | 18.974 | 110.834 | 47.306 | 1.00 | 0.00 | AAAB |
| ATOM | 3072 | CB | SER | E | 699 | 17.493 | 110.450 | 47.408 | 1.00 | 0.00 | AAAB |
| ATOM | 3075 | OG | SER | E | 699 | 17.259 | 109.530 | 48.443 | 1.00 | 0.00 | AAAB |
| ATOM | 3077 | C | SER | E | 699 | 19.841 | 109.717 | 46.750 | 1.00 | 0.00 | AAAB |
| ATOM | 3078 | O | SER | E | 699 | 20.683 | 109.226 | 47.496 | 1.00 | 0.00 | AAAB |
| ATOM | 3079 | N | SER | E | 700 | 19.627 | 109.335 | 45.494 | 1.00 | 0.00 | AAAB |
| ATOM | 3081 | CA | SER | E | 700 | 20.393 | 108.272 | 44.875 | 1.00 | 0.00 | AAAB |
| ATOM | 3083 | CB | SER | E | 700 | 19.729 | 108.045 | 43.509 | 1.00 | 0.00 | AAAB |
| ATOM | 3086 | OG | SER | E | 700 | 20.154 | 106.960 | 42.725 | 1.00 | 0.00 | AAAB |
| ATOM | 3088 | C | SER | E | 700 | 21.870 | 108.572 | 44.676 | 1.00 | 0.00 | AAAB |
| ATOM | 3089 | O | SER | E | 700 | 22.689 | 107.676 | 44.872 | 1.00 | 0.00 | AAAB |
| ATOM | 3090 | N | PHE | E | 701 | 22.254 | 109.814 | 44.362 | 1.00 | 0.00 | AAAB |
| ATOM | 3092 | CA | PHE | E | 701 | 23.577 | 110.378 | 44.288 | 1.00 | 0.00 | AAAB |
| ATOM | 3094 | CB | PHE | E | 701 | 23.470 | 111.822 | 43.791 | 1.00 | 0.00 | AAAB |
| ATOM | 3097 | CG | PHE | E | 701 | 24.706 | 112.676 | 43.744 | 1.00 | 0.00 | AAAB |
| ATOM | 3098 | CD1 | PHE | E | 701 | 25.765 | 112.281 | 42.911 | 1.00 | 0.00 | AAAB |
| ATOM | 3100 | CD2 | PHE | E | 701 | 24.815 | 113.814 | 44.553 | 1.00 | 0.00 | AAAB |
| ATOM | 3102 | CE1 | PHE | E | 701 | 26.865 | 113.150 | 42.837 | 1.00 | 0.00 | AAAB |
| ATOM | 3104 | CE2 | PHE | E | 701 | 26.001 | 114.555 | 44.601 | 1.00 | 0.00 | AAAB |
| ATOM | 3106 | CZ | PHE | E | 701 | 27.044 | 114.215 | 43.734 | 1.00 | 0.00 | AAAB |
| ATOM | 3108 | C | PHE | E | 701 | 24.490 | 110.171 | 45.488 | 1.00 | 0.00 | AAAB |
| ATOM | 3109 | O | PHE | E | 701 | 25.544 | 109.537 | 45.389 | 1.00 | 0.00 | AAAB |
| ATOM | 3110 | N | ARG | E | 702 | 23.967 | 110.653 | 46.619 | 1.00 | 0.00 | AAAB |
| ATOM | 3112 | CA | ARG | E | 702 | 24.601 | 110.539 | 47.914 | 1.00 | 0.00 | AAAB |
| ATOM | 3114 | CB | ARG | E | 702 | 23.941 | 111.523 | 48.878 | 1.00 | 0.00 | AAAB |
| ATOM | 3117 | CG | ARG | E | 702 | 23.957 | 112.991 | 48.461 | 1.00 | 0.00 | AAAB |
| ATOM | 3120 | CD | ARG | E | 702 | 23.342 | 113.961 | 49.474 | 1.00 | 0.00 | AAAB |
| ATOM | 3123 | NE | ARG | E | 702 | 23.672 | 115.364 | 49.205 | 1.00 | 0.00 | AAAB |
| ATOM | 3125 | CZ | ARG | E | 702 | 24.687 | 116.004 | 49.804 | 1.00 | 0.00 | AAAB |
| ATOM | 3126 | NH1 | ARG | E | 702 | 25.641 | 115.433 | 50.556 | 1.00 | 0.00 | AAAB |
| ATOM | 3129 | NH2 | ARG | E | 702 | 24.760 | 117.323 | 49.555 | 1.00 | 0.00 | AAAB |
| ATOM | 3132 | C | ARG | E | 702 | 24.551 | 109.117 | 48.472 | 1.00 | 0.00 | AAAB |
| ATOM | 3133 | O | ARG | E | 702 | 25.321 | 108.693 | 49.334 | 1.00 | 0.00 | AAAB |
| ATOM | 3134 | N | LYS | E | 703 | 23.707 | 108.290 | 47.861 | 1.00 | 0.00 | AAAB |
| ATOM | 3136 | CA | LYS | E | 703 | 23.672 | 106.853 | 48.043 | 1.00 | 0.00 | AAAB |
| ATOM | 3138 | CB | LYS | E | 703 | 22.251 | 106.326 | 47.844 | 1.00 | 0.00 | AAAB |
| ATOM | 3141 | CG | LYS | E | 703 | 22.020 | 104.918 | 48.392 | 1.00 | 0.00 | AAAB |
| ATOM | 3144 | CD | LYS | E | 703 | 20.531 | 104.636 | 48.585 | 1.00 | 0.00 | AAAB |
| ATOM | 3147 | CE | LYS | E | 703 | 20.215 | 103.155 | 48.369 | 1.00 | 0.00 | AAAB |
| ATOM | 3150 | NZ | LYS | E | 703 | 18.946 | 102.679 | 48.950 | 1.00 | 0.00 | AAAB |
| ATOM | 3154 | C | LYS | E | 703 | 24.707 | 106.099 | 47.218 | 1.00 | 0.00 | AAAB |
| ATOM | 3155 | O | LYS | E | 703 | 25.072 | 104.978 | 47.589 | 1.00 | 0.00 | AAAB |
| ATOM | 3156 | N | THR | E | 704 | 25.012 | 106.518 | 45.988 | 1.00 | 0.00 | AAAB |
| ATOM | 3158 | CA | THR | E | 704 | 26.099 | 105.925 | 45.244 | 1.00 | 0.00 | AAAB |
| ATOM | 3160 | CB | THR | E | 704 | 26.001 | 106.357 | 43.780 | 1.00 | 0.00 | AAAB |
| ATOM | 3162 | OG1 | THR | E | 704 | 24.719 | 106.075 | 43.265 | 1.00 | 0.00 | AAAB |
| ATOM | 3164 | CG2 | THR | E | 704 | 26.887 | 105.572 | 42.813 | 1.00 | 0.00 | AAAB |
| ATOM | 3168 | C | THR | E | 704 | 27.426 | 106.315 | 45.876 | 1.00 | 0.00 | AAAB |
| ATOM | 3169 | O | THR | E | 704 | 28.418 | 105.601 | 45.767 | 1.00 | 0.00 | AAAB |
| ATOM | 3170 | N | PHE | E | 705 | 27.502 | 107.427 | 46.615 | 1.00 | 0.00 | AAAB |
| ATOM | 3172 | CA | PHE | E | 705 | 28.693 | 107.758 | 47.358 | 1.00 | 0.00 | AAAB |
| ATOM | 3174 | CB | PHE | E | 705 | 28.554 | 109.177 | 47.928 | 1.00 | 0.00 | AAAB |
| ATOM | 3177 | CG | PHE | E | 705 | 28.767 | 110.361 | 47.021 | 1.00 | 0.00 | AAAB |
| ATOM | 3178 | CD1 | PHE | E | 705 | 29.400 | 110.288 | 45.766 | 1.00 | 0.00 | AAAB |
| ATOM | 3180 | CD2 | PHE | E | 705 | 28.319 | 111.592 | 47.491 | 1.00 | 0.00 | AAAB |
| ATOM | 3182 | CE1 | PHE | E | 705 | 29.548 | 111.453 | 45.020 | 1.00 | 0.00 | AAAB |
| ATOM | 3184 | CE2 | PHE | E | 705 | 28.508 | 112.781 | 46.766 | 1.00 | 0.00 | AAAB |
| ATOM | 3186 | CZ | PHE | E | 705 | 29.181 | 112.699 | 45.547 | 1.00 | 0.00 | AAAB |
| ATOM | 3188 | C | PHE | E | 705 | 28.902 | 106.788 | 48.507 | 1.00 | 0.00 | AAAB |
| ATOM | 3189 | O | PHE | E | 705 | 29.919 | 106.090 | 48.531 | 1.00 | 0.00 | AAAB |
| ATOM | 3190 | N | GLU | E | 706 | 27.892 | 106.386 | 49.283 | 1.00 | 0.00 | AAAB |
| ATOM | 3192 | CA | GLU | E | 706 | 28.067 | 105.371 | 50.310 | 1.00 | 0.00 | AAAB |
| ATOM | 3194 | CB | GLU | E | 706 | 26.79.9 | 105.558 | 51.153 | 1.00 | 0.00 | AAAB |
| ATOM | 3197 | CG | GLU | E | 706 | 26.805 | 106.801 | 52.052 | 1.00 | 0.00 | AAAB |
| ATOM | 3200 | CD | GLU | E | 706 | 27.949 | 106.854 | 53.051 | 1.00 | 0.00 | AAAB |
| ATOM | 3201 | OE1 | GLU | E | 706 | 28.455 | 105.773 | 53.453 | 1.00 | 0.00 | AAAB |
| ATOM | 3202 | OE2 | GLU | E | 706 | 28.301 | 107.972 | 53.477 | 1.00 | 0.00 | AAAB |
| ATOM | 3203 | C | GLU | E | 706 | 28.141 | 103.911 | 49.903 | 1.00 | 0.00 | AAAB |
| ATOM | 3204 | O | GLU | E | 706 | 28.747 | 103.159 | 50.658 | 1.00 | 0.00 | AAAB |
| ATOM | 3205 | N | ASP | E | 707 | 27.746 | 103.565 | 48.666 | 1.00 | 0.00 | AAAB |
| ATOM | 3207 | CA | ASP | E | 707 | 27.924 | 102.358 | 47.889 | 1.00 | 0.00 | AAAB |
| ATOM | 3209 | CB | ASP | E | 707 | 27.015 | 102.430 | 46.655 | 1.00 | 0.00 | AAAB |
| ATOM | 3212 | CG | ASP | E | 707 | 26.692 | 101.055 | 46.092 | 1.00 | 0.00 | AAAB |
| ATOM | 3213 | OD1 | ASP | E | 707 | 27.196 | 100.568 | 45.062 | 1.00 | 0.00 | AAAB |
| ATOM | 3214 | OD2 | ASP | E | 707 | 25.810 | 100.414 | 46.720 | 1.00 | 0.00 | AAAB |
| ATOM | 3215 | C | ASP | E | 707 | 29.309 | 102.113 | 47.306 | 1.00 | 0.00 | AAAB |
| ATOM | 3216 | O | ASP | E | 707 | 29.785 | 100.988 | 47.194 | 1.00 | 0.00 | AAAB |
| ATOM | 3217 | N | TYR | E | 708 | 30.047 | 103.177 | 46.955 | 1.00 | 0.00 | AAAB |
| ATOM | 3219 | CA | TYR | E | 708 | 31.436 | 103.113 | 46.562 | 1.00 | 0.00 | AAAB |
| ATOM | 3221 | CB | TYR | E | 708 | 31.853 | 104.388 | 45.829 | 1.00 | 0.00 | AAAB |
| ATOM | 3224 | CG | TYR | E | 708 | 31.600 | 104.662 | 44.365 | 1.00 | 0.00 | AAAB |
| ATOM | 3225 | CD1 | TYR | E | 708 | 31.969 | 103.655 | 43.466 | 1.00 | 0.00 | AAAB |
| ATOM | 3227 | CD2 | TYR | E | 708 | 30.851 | 105.757 | 43.931 | 1.00 | 0.00 | AAAB |
| ATOM | 3229 | CE1 | TYR | E | 708 | 31.690 | 103.771 | 42.099 | 1.00 | 0.00 | AAAB |
| ATOM | 3231 | CE2 | TYR | E | 708 | 30.578 | 105.858 | 42.558 | 1.00 | 0.00 | AAAB |
| ATOM | 3233 | CZ | TYR | E | 708 | 30.959 | 104.879 | 41.638 | 1.00 | 0.00 | AAAB |
| ATOM | 3234 | OH | TYR | E | 708 | 30.572 | 104.957 | 40.335 | 1.00 | 0.00 | AAAB |
| ATOM | 3236 | C | TYR | E | 708 | 32.329 | 102.904 | 47.778 | 1.00 | 0.00 | AAAB |
| ATOM | 3237 | O | TYR | E | 708 | 33.463 | 102.481 | 47.549 | 1.00 | 0.00 | AAAB |
| ATOM | 3238 | N | LEU | E | 709 | 31.842 | 103.196 | 48.989 | 1.00 | 0.00 | AAAB |
| ATOM | 3240 | CA | LEU | E | 709 | 32.477 | 102.880 | 50.258 | 1.00 | 0.00 | AAAB |
| ATOM | 3242 | CB | LEU | E | 709 | 32.156 | 103.948 | 51.293 | 1.00 | 0.00 | AAAB |
| ATOM | 3245 | CG | LEU | E | 709 | 32.784 | 105.313 | 50.982 | 1.00 | 0.00 | AAAB |
| ATOM | 3247 | CD1 | LEU | E | 709 | 32.084 | 106.366 | 51.837 | 1.00 | 0.00 | AAAB |
| ATOM | 3251 | CD2 | LEU | E | 709 | 34.300 | 105.290 | 51.063 | 1.00 | 0.00 | AAAB |
| ATOM | 3255 | C | LEU | E | 709 | 31.993 | 101.556 | 50.826 | 1.00 | 0.00 | AAAB |
| ATOM | 3256 | O | LEU | E | 709 | 32.379 | 101.094 | 51.903 | 1.00 | 0.00 | AAAB |
| ATOM | 3257 | N | HIS | E | 710 | 31.306 | 100.793 | 49.972 | 1.00 | 0.00 | AAAB |
| ATOM | 3259 | CA | HIS | E | 710 | 30.889 | 99.415 | 50.165 | 1.00 | 0.00 | AAAB |
| ATOM | 3261 | CB | HIS | E | 710 | 29.458 | 99.461 | 50.695 | 1.00 | 0.00 | AAAB |
| ATOM | 3264 | CG | HIS | E | 710 | 28.947 | 98.245 | 51.410 | 1.00 | 0.00 | AAAB |
| ATOM | 3265 | ND1 | HIS | E | 710 | 29.683 | 97.740 | 52.484 | 1.00 | 0.00 | AAAB |
| ATOM | 3266 | CD2 | HIS | E | 710 | 28.042 | 97.296 | 50.996 | 1.00 | 0.00 | AAAB |
| ATOM | 3268 | CE1 | HIS | E | 710 | 29.144 | 96.535 | 52.697 | 1.00 | 0.00 | AAAB |
| ATOM | 3270 | NE2 | HIS | E | 710 | 28.161 | 96.215 | 51.843 | 1.00 | 0.00 | AAAB |
| ATOM | 3272 | C | HIS | E | 710 | 30.981 | 98.574 | 48.894 | 1.00 | 0.00 | AAAB |
| ATOM | 3273 | OT | HIS | E | 710 | 31.916 | 98.689 | 48.110 | 1.00 | 0.00 | AAAB |
| ATOM | 3274 | O | HIS | E | 710 | 29.949 | 97.703 | 48.714 | 1.00 | 0.00 | AAAB |
| END | | | | | | | | | | | |

### APPENDIX III: ATOMIC COORDINATES FOR THE MODEL OF THI TERMINAL REGION OF IGF-1R α-CHAIN BOUND TO IR ECTODOM

The structural coordinates in Appendixes I and II were used to model the C-term region of the IGF-1R α-chain in the low affinity binding site of IR. This model i oriented relative to atomic coordinates found in Appendixes I and II, and may be in conjunction with atomic coordinates of Appendixes I to II, and IV to VI to des compounds which bind to IR and/or IGF-1R.

| | | | | | | | | | | | |
|---|---|---|---|---|---|---|---|---|---|---|---|
| ATOM | 1 | N | PRO | E | 4 | 42.287 | 117.105 | 18.969 | 1.00 | 0.00 | AAAA |
| ATOM | 4 | CA | PRO | E | 4 | 43.545 | 116.433 | 18.610 | 1.00 | 0.00 | AAAA |
| ATOM | 6 | CB | PRO | E | 4 | 44.552 | 117.508 | 18.212 | 1.00 | 0.00 | AAAA |
| ATOM | 9 | CG | PRO | E | 4 | 44.090 | 118.693 | 19.056 | 1.00 | 0.00 | AAAA |
| ATOM | 12 | CD | PRO | E | 4 | 42.576 | 118.534 | 19.184 | 1.00 | 0.00 | AAAA |
| ATOM | 15 | C | PRO | E | 4 | 44.063 | 115.646 | 19.806 | 1.00 | 0.00 | AAAA |
| ATOM | 16 | O | PRO | E | 4 | 43.706 | 115.923 | 20.955 | 1.00 | 0.00 | AAAA |
| ATOM | 17 | N | GLY | E | 5 | 44.892 | 114.641 | 19.532 | 1.00 | 0.00 | AAAA |
| ATOM | 19 | CA | GLY | E | 5 | 45.530 | 113.809 | 20.537 | 1.00 | 0.00 | AAAA |
| ATOM | 22 | C | GLY | E | 5 | 45.196 | 112.325 | 20.453 | 1.00 | 0.00 | AAAA |
| ATOM | 23 | O | GLY | E | 5 | 44.131 | 112.029 | 19.920 | 1.00 | 0.00 | AAAA |
| ATOM | 24 | N | GLU | E | 6 | 46.112 | 111.448 | 20.871 | 1.00 | 0.00 | AAAA |
| ATOM | 26 | CA | GLU | E | 6 | 45.931 | 110.013 | 20.881 | 1.00 | 0.00 | AAAA |
| ATOM | 28 | CB | GLU | E | 6 | 47.189 | 109.259 | 21.324 | 1.00 | 0.00 | AAAA |
| ATOM | 31 | CG | GLU | E | 6 | 47.264 | 107.893 | 20.631 | 1.00 | 0.00 | AAAA |
| ATOM | 34 | CD | GLU | E | 6 | 47.841 | 107.981 | 19.223 | 1.00 | 0.00 | AAAA |
| ATOM | 35 | OE1 | GLU | E | 6 | 47.348 | 108.785 | 18.403 | 1.00 | 0.00 | AAAA |
| ATOM | 36 | OE2 | GLU | E | 6 | 48.824 | 107.273 | 18.921 | 1.00 | 0.00 | AAAA |
| ATOM | 37 | C | GLU | E | 6 | 44.684 | 109.562 | 21.641 | 1.00 | 0.00 | AAAA |
| ATOM | 38 | O | GLU | E | 6 | 44.489 | 110.002 | 22.774 | 1.00 | 0.00 | AAAA |
| ATOM | 39 | N | VAL | E | 7 | 43.860 | 108.733 | 21.000 | 1.00 | 0.00 | AAAA |
| ATOM | 41 | CA | VAL | E | 7 | 42.618 | 108.214 | 21.535 | 1.00 | 0.00 | AAAA |
| ATOM | 43 | CB | VAL | E | 7 | 41.662 | 107.877 | 20.375 | 1.00 | 0.00 | AAAA |
| ATOM | 45 | CG1 | VAL | E | 7 | 40.270 | 107.549 | 20.914 | 1.00 | 0.00 | AAAA |
| ATOM | 49 | CG2 | VAL | E | 7 | 41.413 | 108.879 | 19.254 | 1.00 | 0.00 | AAAA |
| ATOM | 53 | C | VAL | E | 7 | 42.995 | 106.982 | 22.343 | 1.00 | 0.00 | AAAA |
| ATOM | 54 | O | VAL | E | 7 | 43.561 | 105.978 | 21.905 | 1.00 | 0.00 | AAAA |
| ATOM | 55 | N | CYS | E | 8 | 42.528 | 107.069 | 23.592 | 1.00 | 0.00 | AAAA |
| ATOM | 57 | CA | CYS | E | 8 | 42.745 | 106.114 | 24.653 | 1.00 | 0.00 | AAAA |
| ATOM | 59 | CB | CYS | E | 8 | 43.866 | 106.571 | 25.597 | 1.00 | 0.00 | AAAA |
| ATOM | 62 | SG | CYS | E | 8 | 43.810 | 108.313 | 26.077 | 1.00 | 0.00 | AAAA |
| ATOM | 63 | C | CYS | E | 8 | 41.457 | 105.833 | 25.420 | 1.00 | 0.00 | AAAA |
| ATOM | 64 | O | CYS | E | 8 | 40.537 | 106.641 | 25.410 | 1.00 | 0.00 | AAAA |
| ATOM | 65 | N | PRO | E | 9 | 41.388 | 104.663 | 26.071 | 1.00 | 0.00 | AAAA |
| ATOM | 66 | CA | PRO | E | 9 | 40.189 | 104.265 | 26.783 | 1.00 | 0.00 | AAAA |
| ATOM | 68 | CB | PRO | E | 9 | 40.261 | 102.738 | 26.789 | 1.00 | 0.00 | AAAA |
| ATOM | 71 | CG | PRO | E | 9 | 41.755 | 102.422 | 26.886 | 1.00 | 0.00 | AAAA |
| ATOM | 74 | CD | PRO | E | 9 | 42.325 | 103.569 | 26.046 | 1.00 | 0.00 | AAAA |
| ATOM | 77 | C | PRO | E | 9 | 40.103 | 104.933 | 28.144 | 1.00 | 0.00 | AAAA |
| ATOM | 78 | O | PRO | E | 9 | 41.117 | 105.427 | 28.636 | 1.00 | 0.00 | AAAA |
| ATOM | 79 | N | GLY | E | 10 | 38.948 | 104.954 | 28.814 | 1.00 | 0.00 | AAAA |
| ATOM | 81 | CA | GLY | E | 10 | 38.614 | 105.482 | 30.120 | 1.00 | 0.00 | AAAA |
| ATOM. | 84 | C | GLY | E | 10 | 38.315 | 104.311 | 31.044 | 1.00 | 0.00 | AAAA |
| ATOM | 85 | O | GLY | E | 10 | 37.570 | 103.437 | 30.607 | 1.00 | 0.00 | AAAA |
| ATOM | 86 | N | MET | E | 11 | 38.700 | 104.271 | 32.318 | 1.00 | 0.00 | AAAA |
| ATOM | 88 | CA | MET | E | 11 | 38.758 | 103.128 | 33.208 | 1.00 | 0.00 | AAAA |
| ATOM | 90 | CB | MET | E | 11 | 39.889 | 102.138 | 32.924 | 1.00 | 0.00 | AAAA |
| ATOM | 93 | CG | MET | E | 11 | 41.203 | 102.803 | 32.529 | 1.00 | 0.00 | AAAA |
| ATOM | 96 | SD | MET | E | 11 | 42.647 | 101.713 | 32.601 | 1.00 | 0.00 | AAAA |
| ATOM | 97 | CE | MET | E | 11 | 43.886 | 102.424 | 31.495 | 1.00 | 0.00 | AAAA |
| ATOM | 101 | C | MET | E | 11 | 38.683 | 103.506 | 34.681 | 1.00 | 0.00 | AAAA |
| ATOM | 102 | O | MET | E | 11 | 38.523 | 104.679 | 34.990 | 1.00 | 0.00 | AAAA |
| ATOM | 103 | N | ASP | E | 12 | 38.837 | 102.525 | 35.587 | 1.00 | 0.00 | AAAA |
| ATOM | 105 | CA | ASP | E | 12 | 38.700 | 102.696 | 37.013 | 1.00 | 0.00 | AAAA |
| ATOM | 107 | CB | ASP | E | 12 | 37.542 | 101.884 | 37.586 | 1.00 | 0.00 | AAAA |
| ATOM | 110 | CG | ASP | E | 12 | 37.389 | 101.820 | 39.103 | 1.00 | 0.00 | AAAA |
| ATOM | 111 | OD1 | ASP | E | 12 | 37.987 | 100.837 | 39.604 | 1.00 | 0.00 | AAAA |
| ATOM | 112 | OD2 | ASP | E | 12 | 36.657 | 102.633 | 39.702 | 1.00 | 0.00 | AAAA |
| ATOM | 113 | C | ASP | E | 12 | 40.014 | 102.198 | 37.600 | 1.00 | 0.00 | AAAA |
| ATOM | 114 | O | ASP | E | 12 | 40.424 | 101.087 | 37.280 | 1.00 | 0.00 | AAAA |
| ATOM | 115 | N | ILE | E | 13 | 40.692 | 103.071 | 38.346 | 1.00 | 0.00 | AAAA |
| ATOM | 117 | CA | ILE | E | 13 | 41.991 | 102.815 | 38.929 | 1.00 | 0.00 | AAAA |
| ATOM | 119 | CB | ILE | E | 13 | 43.065 | 103.707 | 38.305 | 1.00 | 0.00 | AAAA |
| ATOM | 121 | CG1 | ILE | E | 13 | 43.095 | 103.615 | 36.780 | 1.00 | 0.00 | AAAA |
| ATOM | 124 | CG2 | ILE | E | 13 | 44.472 | 103.472 | 38.859 | 1.00 | 0.00 | AAAA |
| ATOM | 128 | CD1 | ILE | E | 13 | 42.465 | 104.722 | 35.938 | 1.00 | 0.00 | AAAA |
| ATOM | 132 | C | ILE | E | 13 | 41.760 | 103.016 | 40.420 | 1.00 | 0.00 | AAAA |
| ATOM | 133 | O | ILE | E | 13 | 41.247 | 104.075 | 40.801 | 1.00 | 0.00 | AAAA |
| ATOM | 134 | N | ARG | E | 14 | 42.177 | 102.026 | 41.209 | 1.00 | 0.00 | AAAA |
| ATOM | 136 | CA | ARG | E | 14 | 42.082 | 102.085 | 42.647 | 1.00 | 0.00 | AAAA |
| ATOM | 138 | CB | ARG | E | 14 | 40.917 | 101.277 | 43.216 | 1.00 | 0.00 | AAAA |
| ATOM | 141 | CG | ARG | E | 14 | 39.631 | 101.710 | 42.522 | 1.00 | 0.00 | AAAA |
| ATOM | 144 | CD | ARG | E | 14 | 38.377 | 101.034 | 43.090 | 1.00 | 0.00 | AAAA |
| ATOM | 147 | NE | ARG | E | 14 | 37.215 | 101.224 | 42.219 | 1.00 | 0.00 | AAAA |
| ATOM | 149 | CZ | ARG | E | 14 | 35.907 | 101.058 | 42.431 | 1.00 | 0.00 | AAAA |
| ATOM | 150 | NH1 | ARG | E | 14 | 35.358 | 100.435 | 43.493 | 1.00 | 0.00 | AAAA |
| ATOM | 153 | NH2 | ARG | E | 14 | 35.051 | 101.576 | 41.542 | 1.00 | 0.00 | AAAA |
| ATOM | 156 | C | ARG | E | 14 | 43.420 | 101.671 | 43.257 | 1.00 | 0.00 | AAAA |
| ATOM | 157 | O | ARG | E | 14 | 44.333 | 101.137 | 42.633 | 1.00 | 0.00 | AAAA |
| ATOM | 158 | N | ASN | E | 15 | 43.467 | 102.013 | 44.542 | 1.00 | 0.00 | AAAA |
| ATOM | 160 | CA | ASN | E | 15 | 44.545 | 101.773 | 45.486 | 1.00 | 0.00 | AAAA |
| ATOM | 162 | CB | ASN | E | 15 | 44.885 | 100.292 | 45.525 | 1.00 | 0.00 | AAAA |
| ATOM | 165 | CG | ASN | E | 15 | 45.338 | 99.788 | 46.890 | 1.00 | 0.00 | AAAA |
| ATOM | 166 | OD1 | ASN | E | 15 | 46.374 | 100.194 | 47.407 | 1.00 | 0.00 | AAAA |
| ATOM | 167 | ND2 | ASN | E | 15 | 44.602 | 98.806 | 47.420 | 1.00 | 0.00 | AAAA |
| ATOM | 170 | C | ASN | E | 15 | 45.741 | 102.718 | 45.403 | 1.00 | 0.00 | AAAA |
| ATOM | 171 | O | ASN | E | 15 | 46.129 | 103.376 | 46.364 | 1.00 | 0.00 | AAAA |
| ATOM | 172 | N | ASN | E | 16 | 46.347 | 102.780 | 44.216 | 1.00 | 0.00 | AAAA |
| ATOM | 174 | CA | ASN | E | 16 | 47.461 | 103.642 | 43.856 | 1.00 | 0.00 | AAAA |
| ATOM | 176 | CB | ASN | E | 16 | 48.780 | 103.092 | 44.393 | 1.00 | 0.00 | AAAA |
| ATOM | 179 | CG | ASN | E | 16 | 49.210 | 101.688 | 43.982 | 1.00 | 0.00 | AAAA |
| ATOM | 180 | OD1 | ASN | E | 16 | 48.626 | 100.682 | 44.366 | 1.00 | 0.00 | AAAA |
| ATOM | 181 | ND2 | ASN | E | 16 | 50.277 | 101.551 | 43.202 | 1.00 | 0.00 | AAAA |
| ATOM | 184 | C | ASN | E | 16 | 47.410 | 103.970 | 42.367 | 1.00 | 0.00 | AAAA |
| ATOM | 185 | O | ASN | E | 16 | 47.052 | 103.094 | 41.584 | 1.00 | 0.00 | AAAA |
| ATOM | 186 | N | LEU | E | 17 | 47.697 | 105.211 | 41.978 | 1.00 | 0.00 | AAAA |
| ATOM | 188 | CA | LEU | E | 17 | 47.611 | 105.814 | 40.662 | 1.00 | 0.00 | AAAA |
| ATOM | 190 | CB | LEU | E | 17 | 47.583 | 107.336 | 40.820 | 1.00 | 0.00 | AAAA |
| ATOM | 193 | CG | LEU | E | 17 | 47.415 | 108.262 | 39.622 | 1.00 | 0.00 | AAAA |
| ATOM | 195 | CD1 | LEU | E | 17 | 45.974 | 108.296 | 39.129 | 1.00 | 0.00 | AAAA |
| ATOM | 199 | CD2 | LEU | E | 17 | 47.920 | 109.651 | 39.991 | 1.00 | 0.00 | AAAA |
| ATOM | 203 | C | LEU | E | 17 | 48.728 | 105.372 | 39.726 | 1.00 | 0.00 | AAAA |
| ATOM | 204 | O | LEU | E | 17 | 48.566 | 105.247 | 38.515 | 1.00 | 0.00 | AAAA |
| ATOM | 205 | N | THR | E | 18 | 49.890 | 105.106 | 40.332 | 1.00 | 0.00 | AAAA |
| ATOM | 207 | CA | THR | E | 18 | 51.092 | 104.448 | 39.861 | 1.00 | 0.00 | AAAA |
| ATOM | 209 | CB | THR | E | 18 | 52.179 | 104.489 | 40.946 | 1.00 | 0.00 | AAAA |
| ATOM | 211 | OG1 | THR | E | 18 | 51.717 | 103.781 | 42.072 | 1.00 | 0.00 | AAAA |
| ATOM | 213 | CG2 | THR | E | 18 | 52.610 | 105.864 | 41.445 | 1.00 | 0.00 | AAAA |
| ATOM | 217 | C | THR | E | 18 | 50.942 | 103.076 | 39.223 | 1.00 | 0.00 | AAAA |
| ATOM | 218 | O | THR | E | 18 | 51.848 | 102.615 | 38.533 | 1.00 | 0.00 | AAAA |
| ATOM | 219 | N | ARG | E | 19 | 49.759 | 102.475 | 39.329 | 1.00 | 0.00 | AAAA |
| ATOM | 221 | CA | ARG | E | 19 | 49.367 | 101.313 | 38.564 | 1.00 | 0.00 | AAAA |
| ATOM | 223 | CB | ARG | E | 19 | 48.168 | 100.515 | 39.091 | 1.00 | 0.00 | AAAA |
| ATOM | 226 | CG | ARG | E | 19 | 48.268 | 99.831 | 40.452 | 1.00 | 0.00 | AAAA |
| ATOM | 229 | CD | ARG | E | 19 | 46.929 | 99.479 | 41.100 | 1.00 | 0.00 | AAAA |
| ATOM | 232 | NE | ARG | E | 19 | 46.871 | 98.150 | 41.693 | 1.00 | 0.00 | AAAA |
| ATOM | 234 | CZ | ARG | E | 19 | 45.859 | 97.642 | 42.419 | 1.00 | 0.00 | AAAA |
| ATOM | 235 | NH1 | ARG | E | 19 | 44.671 | 98.274 | 42.385 | 1.00 | 0.00 | AAAA |
| ATOM | 238 | NH2 | ARG | E | 19 | 46.081 | 96.532 | 43.140 | 1.00 | 0.00 | AAAA |
| ATOM | 241 | C | ARG | E | 19 | 49.074 | 101.657 | 37.110 | 1.00 | 0.00 | AAAA |
| ATOM | 242 | O | ARG | E | 19 | 49.195 | 100.814 | 36.225 | 1.00 | 0.00 | AAAA |
| ATOM | 243 | N | LEU | E | 20 | 48.762 | 102.900 | 36.736 | 1.00 | 0.00 | AAAA |
| ATOM | 245 | CA | LEU | E | 20 | 48.770 | 103.341 | 35.363 | 1.00 | 0.00 | AAAA |
| ATOM | 247 | CB | LEU | E | 20 | 48.171 | 104.738 | 35.153 | 1.00 | 0.00 | AAAA |
| ATOM | 250 | CG | LEU | E | 20 | 46.644 | 104.714 | 35.231 | 1.00 | 0.00 | AAAA |
| ATOM | 252 | CD1 | LEU | E | 20 | 46.105 | 106.045 | 35.745 | 1.00 | 0.00 | AAAA |
| ATOM | 256 | CD2 | LEU | E | 20 | 45.927 | 104.169 | 34.000 | 1.00 | 0.00 | AAAA |
| ATOM | 260 | C | LEU | E | 20 | 50.206 | 103.462 | 34.872 | 1.00 | 0.00 | AAAA |
| ATOM | 261 | O | LEU | E | 20 | 50.915 | 104.421 | 35.194 | 1.00 | 0.00 | AAAA |
| ATOM | 262 | N | HIS | E | 21 | 50.614 | 102.451 | 34.110 | 1.00 | 0.00 | AAAA |
| ATOM | 264 | CA | HIS | E | 21 | 52.015 | 102.240 | 33.805 | 1.00 | 0.00 | AAAA |
| ATOM | 266 | CB | HIS | E | 21 | 52.295 | 100.798 | 33.372 | 1.00 | 0.00 | AAAA |
| ATOM | 269 | CG | HIS | E | 21 | 51.886 | 100.533 | 31.945 | 1.00 | 0.00 | AAAA |
| ATOM | 270 | ND1 | HIS | E | 21 | 50.533 | 100.392 | 31.646 | 1.00 | 0.00 | AAAA |
| ATOM | 271 | CD2 | HIS | E | 21 | 52.577 | 100.370 | 30.782 | 1.00 | 0.00 | AAAA |
| ATOM | 273 | CE1 | HIS | E | 21 | 50.481 | 100.145 | 30.335 | 1.00 | 0.00 | AAAA |
| ATOM | 275 | NE2 | HIS | E | 21 | 51.689 | 100.129 | 29.754 | 1.00 | 0.00 | AAAA |
| ATOM | 277 | C | HIS | E | 21 | 52.637 | 103.270 | 32.866 | 1.00 | 0.00 | AAAA |
| ATOM | 278 | O | HIS | E | 21 | 53.837 | 103.539 | 32.894 | 1.00 | 0.00 | AAAA |
| ATOM | 279 | N | GLU | E | 22 | 51.801 | 103.729 | 31.936 | 1.00 | 0.00 | AAAA |
| ATOM | 281 | CA | GLU | E | 22 | 52.048 | 104.597 | 30.797 | 1.00 | 0.00 | AAAA |
| ATOM | 283 | CB | GLU | E | 22 | 52.441 | 103.735 | 29.591 | 1.00 | 0.00 | AAAA |
| ATOM | 286 | CG | GLU | E | 22 | 53.190 | 104.504 | 28.498 | 1.00 | 0.00 | AAAA |
| ATOM | 289 | CD | GLU | E | 22 | 52.360 | 104.433 | 27.221 | 1.00 | 0.00 | AAAA |
| ATOM | 290 | OE1 | GLU | E | 22 | 51.478 | 105.306 | 27.055 | 1.00 | 0.00 | AAAA |
| ATOM | 291 | OE2 | GLU | E | 22 | 52.635 | 103.542 | 26.396 | 1.00 | 0.00 | AAAA |
| ATOM | 292 | C | GLU | E | 22 | 50.721 | 105.324 | 30.716 | 1.00 | 0.00 | AAAA |
| ATOM | 293 | O | GLU | E | 22 | 49.647 | 104.732 | 30.759 | 1.00 | 0.00 | AAAA |
| ATOM | 294 | N | LEU | E | 23 | 50.857 | 106.646 | 30.540 | 1.00 | 0.00 | AAAA |
| ATOM | 296 | CA | LEU | E | 23 | 49.960 | 107.435 | 29.717 | 1.00 | 0.00 | AAAA |
| ATOM | 298 | CB | LEU | E | 23 | 48.896 | 108.130 | 30.574 | 1.00 | 0.00 | AAAA |
| ATOM | 301 | CG | LEU | E | 23 | 47.883 | 107.239 | 31.291 | 1.00 | 0.00 | AAAA |
| ATOM | 303 | CD1 | LEU | E | 23 | 47.066 | 108.007 | 32.334 | 1.00 | 0.00 | AAAA |
| ATOM | 307 | CD2 | LEU | E | 23 | 47.019 | 106.425 | 30.327 | 1.00 | 0.00 | AAAA |
| ATOM | 311 | C | LEU | E | 23 | 50.848 | 108.406 | 28.959 | 1.00 | 0.00 | AAAA |
| ATOM | 312 | O | LEU | E | 23 | 50.817 | 109.608 | 29.230 | 1.00 | 0.00 | AAAA |
| ATOM | 313 | N | GLU | E | 24 | 51.513 | 107.892 | 27.928 | 1.00 | 0.00 | AAAA |
| ATOM | 315 | CA | GLU | E | 24 | 52.132 | 108.646 | 26.863 | 1.00 | 0.00 | AAAA |
| ATOM | 317 | CB | GLU | E | 24 | 53.600 | 108.227 | 26.700 | 1.00 | 0.00 | AAAA |
| ATOM | 320 | CG | GLU | E | 24 | 54.610 | 108.637 | 27.771 | 1.00 | 0.00 | AAAA |
| ATOM | 323 | CD | GLU | E | 24 | 54.963 | 110.123 | 27.751 | 1.00 | 0.00 | AAAA |
| ATOM | 324 | OE1 | GLU | E | 24 | 55.029 | 110.727 | 28.844 | 1.00 | 0.00 | AAAA |
| ATOM | 325 | OE2 | GLU | E | 24 | 55.305 | 110.686 | 26.689 | 1.00 | 0.00 | AAAA |
| ATOM | 326 | C | GLU | E | 24 | 51.327 | 108.348 | 25.609 | 1.00 | 0.00 | AAAA |
| ATOM | 327 | O | GLU | E | 24 | 50.889 | 109.303 | 24.966 | 1.00 | 0.00 | AAAA |
| ATOM | 328 | N | ASN | E | 25 | 51.024 | 107.113 | 25.211 | 1.00 | 0.00 | AAAA |
| ATOM | 330 | CA | ASN | E | 25 | 50.166 | 106.780 | 24.097 | 1.00 | 0.00 | AAAA |
| ATOM | 332 | CB | ASN | E | 25 | 50.381 | 105.294 | 23.773 | 1.00 | 0.00 | AAAA |
| ATOM | 335 | CG | ASN | E | 25 | 51.712 | 104.940 | 23.121 | 1.00 | 0.00 | AAAA |
| ATOM | 336 | OD1 | ASN | E | 25 | 51.927 | 104.998 | 21.917 | 1.00 | 0.00 | AAAA |
| ATOM | 337 | ND2 | ASN | E | 25 | 52.696 | 104.410 | 23.850 | 1.00 | 0.00 | AAAA |
| ATOM | 340 | C | ASN | E | 25 | 48.697 | 107.112 | 24.307 | 1.00 | 0.00 | AAAA |
| ATOM | 341 | O | ASN | E | 25 | 47.808 | 106.341 | 23.949 | 1.00 | 0.00 | AAAA |
| ATOM | 342 | N | CYS | E | 26 | 48.348 | 108.263 | 24.887 | 1.00 | 0.00 | AAAA |
| ATOM | 344 | CA | CYS | E | 26 | 47.020 | 108.637 | 25.346 | 1.00 | 0.00 | AAAA |
| ATOM | 346 | CB | CYS | E | 26 | 46.704 | 107.961 | 26.676 | 1.00 | 0.00 | AAAA |
| ATOM | 349 | SG | CYS | E | 26 | 45.192 | 108.385 | 27.568 | 1.00 | 0.00 | AAAA |
| ATOM | 350 | C | CYS | E | 26 | 46.930 | 110.140 | 25.559 | 1.00 | 0.00 | AAAA |
| ATOM | 351 | O | CYS | E | 26 | 47.899 | 110.770 | 25.993 | 1.00 | 0.00 | AAAA |
| ATOM | 352 | N | SER | E | 27 | 45.820 | 110.742 | 25.125 | 1.00 | 0.00 | AAAA |
| ATOM | 354 | CA | SER | E | 27 | 45.550 | 112.160 | 25.247 | 1.00 | 0.00 | AAAA |
| ATOM | 356 | CB | SER | E | 27 | 46.375 | 112.871 | 24.169 | 1.00 | 0.00 | AAAA |
| ATOM | 359 | OG | SER | E | 27 | 46.206 | 114.267 | 24.265 | 1.00 | 0.00 | AAAA |
| ATOM | 361 | C | SER | E | 27 | 44.074 | 112.514 | 25.305 | 1.00 | 0.00 | AAAA |
| ATOM | 362 | O | SER | E | 27 | 43.774 | 113.478 | 26.008 | 1.00 | 0.00 | AAAA |
| ATOM | 363 | N | VAL | E | 28 | 43.262 | 111.805 | 24.533 | 1.00 | 0.00 | AAAA |
| ATOM | 365 | CA | VAL | E | 28 | 41.828 | 112.060 | 24.477 | 1.00 | 0.00 | AAAA |
| ATOM | 367 | CB | VAL | E | 28 | 41.407 | 112.079 | 23.005 | 1.00 | 0.00 | AAAA |
| ATOM | 369 | CG1 | VAL | E | 28 | 39.915 | 111.922 | 22.752 | 1.00 | 0.00 | AAAA |
| ATOM | 373 | CG2 | VAL | E | 28 | 41.932 | 113.315 | 22.278 | 1.00 | 0.00 | AAAA |
| ATOM | 377 | C | VAL | E | 28 | 41.286 | 110.794 | 25.099 | 1.00 | 0.00 | AAAA |
| ATOM | 378 | O | VAL | E | 28 | 41.158 | 109.745 | 24.475 | 1.00 | 0.00 | AAAA |
| ATOM | 379 | N | ILE | E | 29 | 40.769 | 110.834 | 26.336 | 1.00 | 0.00 | AAAA |
| ATOM | 381 | CA | ILE | E | 29 | 40.192 | 109.743 | 27.097 | 1.00 | 0.00 | AAAA |
| ATOM | 383 | CB | ILE | E | 29 | 40.444 | 109.979 | 28.593 | 1.00 | 0.00 | AAAA |
| ATOM | 385 | CG1 | ILE | E | 29 | 41.940 | 109.852 | 28.862 | 1.00 | 0.00 | AAAA |
| ATOM | 388 | CG2 | ILE | E | 29 | 39.655 | 109.132 | 29.584 | 1.00 | 0.00 | AAAA |
| ATOM | 392 | CD1 | ILE | E | 29 | 42.381 | 110.768 | 29.999 | 1.00 | 0.00 | AAAA |
| ATOM | 396 | C | ILE | E | 29 | 38.729 | 109.603 | 26.711 | 1.00 | 0.00 | AAAA |
| ATOM | 397 | O | ILE | E | 29 | 37.785 | 110.238 | 27.201 | 1.00 | 0.00 | AAAA |
| ATOM | 398 | N | GLU | E | 30 | 38.427 | 108.917 | 25.611 | 1.00 | 0.00 | AAAA |
| ATOM | 400 | CA | GLU | E | 30 | 37.145 | 108.336 | 25.280 | 1.00 | 0.00 | AAAA |
| ATOM | 402 | CB | GLU | E | 30 | 37.270 | 107.595 | 23.944 | 1.00 | 0.00 | AAAA |
| ATOM | 405 | CG | GLU | E | 30 | 35.984 | 107.008 | 23.366 | 1.00 | 0.00 | AAAA |
| ATOM | 408 | CD | GLU | E | 30 | 34.752 | 107.911 | 23.407 | 1.00 | 0.00 | AAAA |
| ATOM | 409 | OE1 | GLU | E | 30 | 33.983 | 107.794 | 24.377 | 1.00 | 0.00 | AAAA |
| ATOM | 410 | OE2 | GLU | E | 30 | 34.647 | 108.720 | 22.452 | 1.00 | 0.00 | AAAA |
| ATOM | 411 | C | GLU | E | 30 | 36.653 | 107.391 | 26.361 | 1.00 | 0.00 | AAAA |
| ATOM | 412 | O | GLU | E | 30 | 37.033 | 106.217 | 26.412 | 1.00 | 0.00 | AAAA |
| ATOM | 413 | N | GLY | E | 31 | 35.817 | 107.954 | 27.231 | 1.00 | 0.00 | AAAA |
| ATOM | 415 | CA | GLY | E | 31 | 35.425 | 107.400 | 28.508 | 1.00 | 0.00 | AAAA |
| ATOM | 418 | C | GLY | E | 31 | 35.605 | 108.333 | 29.693 | 1.00 | 0.00 | AAAA |
| ATOM | 419 | O | GLY | E | 31 | 36.055 | 109.463 | 29.502 | 1.00 | 0.00 | AAAA |
| ATOM | 420 | N | HIS | E | 32 | 35.454 | 107.756 | 30.886 | 1.00 | 0.00 | AAAA |
| ATOM | 422 | CA | HIS | E | 32 | 35.549 | 108.453 | 32.158 | 1.00 | 0.00 | AAAA |
| ATOM | 424 | CB | HIS | E | 32 | 34.421 | 107.932 | 33.048 | 1.00 | 0.00 | AAAA |
| ATOM | 427 | CG | HIS | E | 32 | 34.409 | 106.444 | 33.312 | 1.00 | 0.00 | AAAA |
| ATOM | 428 | ND1 | HIS | E | 32 | 34.347 | 105.495 | 32.298 | 1.00 | 0.00 | AAAA |
| ATOM | 429 | CD2 | HIS | E | 32 | 34.786 | 105.833 | 34.482 | 1.00 | 0.00 | AAAA |
| ATOM | 431 | CE1 | HIS | E | 32 | 34.606 | 104.348 | 32.920 | 1.00 | 0.00 | AAAA |
| ATOM | 433 | NE2 | HIS | E | 32 | 34.797 | 104.479 | 34.241 | 1.00 | 0.00 | AAAA |
| ATOM | 435 | C | HIS | E | 32 | 36.884 | 108.128 | 32.800 | 1.00 | 0.00 | AAAA |
| ATOM | 436 | O | HIS | E | 32 | 37.571 | 107.256 | 32.262 | 1.00 | 0.00 | AAAA |
| ATOM | 437 | N | LEU | E | 33 | 37.179 | 108.739 | 33.946 | 1.00 | 0.00 | AAAA |
| ATOM | 439 | CA | LEU | E | 33 | 38.404 | 108.504 | 34.685 | 1.00 | 0.00 | AAAA |
| ATOM | 441 | CB | LEU | E | 33 | 39.383 | 109.611 | 34.274 | 1.00 | 0.00 | AAAA |
| ATOM | 444 | CG | LEU | E | 33 | 40.827 | 109.284 | 34.619 | 1.00 | 0.00 | AAAA |
| ATOM | 446 | CD1 | LEU | E | 33 | 41.345 | 108.072 | 33.852 | 1.00 | 0.00 | AAAA |
| ATOM | 450 | CD2 | LEU | E | 33 | 41.681 | 110.553 | 34.643 | 1.00 | 0.00 | AAAA |
| ATOM | 454 | C | LEU | E | 33 | 38.081 | 108.462 | 36.176 | 1.00 | 0.00 | AAAA |
| ATOM | 455 | O | LEU | E | 33 | 38.112 | 109.496 | 36.835 | 1.00 | 0.00 | AAAA |
| ATOM | 456 | N | GLN | E | 34 | 37.872 | 107.238 | 36.658 | 1.00 | 0.00 | AAAA |
| ATOM | 458 | CA | GLN | E | 34 | 37.728 | 107.056 | 38.086 | 1.00 | 0.00 | AAAA |
| ATOM | 460 | CB | GLN | E | 34 | 36.679 | 106.013 | 38.452 | 1.00 | 0.00 | AAAA |
| ATOM | 463 | CG | GLN | E | 34 | 35.258 | 106.593 | 38.451 | 1.00 | 0.00 | AAAA |
| ATOM | 466 | CD | GLN | E | 34 | 34.162 | 105.561 | 38.710 | 1.00 | 0.00 | AAAA |
| ATOM | 467 | OE1 | GLN | E | 34 | 32.991 | 105.854 | 38.484 | 1.00 | 0.00 | AAAA |
| ATOM | 468 | NE2 | GLN | E | 34 | 34.472 | 104.314 | 39.060 | 1.00 | 0.00 | AAAA |
| ATOM | 471 | C | GLN | E | 34 | 39.106 | 106.734 | 38.672 | 1.00 | 0.00 | AAAA |
| ATOM | 472 | O | GLN | E | 34 | 39.591 | 105.645 | 38.387 | 1.00 | 0.00 | AAAA |
| ATOM | 473 | N | ILE | E | 35 | 39.656 | 107.617 | 39.509 | 1.00 | 0.00 | AAAA |
| ATOM | 475 | CA | ILE | E | 35 | 40.972 | 107.454 | 40.088 | 1.00 | 0.00 | AAAA |
| ATOM | 477 | CB | ILE | E | 35 | 42.010 | 108.318 | 39.371 | 1.00 | 0.00 | AAAA |
| ATOM | 479 | CG1 | ILE | E | 35 | 41.660 | 109.791 | 39.139 | 1.00 | 0.00 | AAAA |
| ATOM | 482 | CG2 | ILE | E | 35 | 42.540 | 107.598 | 38.133 | 1.00 | 0.00 | AAAA |
| ATOM | 486 | CD1 | ILE | E | 35 | 42.919 | 110.643 | 38.978 | 1.00 | 0.00 | AAAA |
| ATOM | 490 | C | ILE | E | 35 | 40.808 | 107.746 | 41.571 | 1.00 | 0.00 | AAAA |
| ATOM | 491 | O | ILE | E | 35 | 41.108 | 108.823 | 42.076 | 1.00 | 0.00 | AAAA. |
| ATOM | 492 | N | LEU | E | 36 | 40.185 | 106.788 | 42.250 | 1.00 | 0.00 | AAAA |
| ATOM | 494 | CA | LEU | E | 36 | 39.557 | 106.982 | 43.546 | 1.00 | 0.00 | AAAA |
| ATOM | 496 | CB | LEU | E | 36 | 38.067 | 107.315 | 43.500 | 1.00 | 0.00 | AAAA |
| ATOM | 499 | CG | LEU | E | 36 | 37.262 | 106.4.13 | 42.560 | | 0.00 | AAAA |
| ATOM | 501 | CD1 | LEU | E | 36 | 36.883 | 105.083 | 43.211 | 1.00 | 0.00 | AAAA |
| ATOM | 505 | CD2 | LEU | E | 36 | 36.025 | 107.244 | 42.207 | 1.00 | 0.00 | AAAA |
| ATOM | 509 | C | LEU | E | 36 | 36.894 | 105.874 | 44.531 | 1.00 | 0.00 | AAAA |
| ATOM | 510 | O | LEU | E | 36 | 40.284 | 104.776 | 44.144 | 1.00 | 0.00 | AAAA |
| ATOM | 511 | N | LEU | E | 37 | 39.814 | 106.117 | 45.836 | 1.00 | 0.00 | AAAA |
| ATOM | 513 | CA | LEU | E | 37 | 40.203 | 105.296 | 46.976 | 1.00 | 0.00 | AAAA |
| ATOM | 515 | CB | LEU | E | 37 | 39.173 | 104.164 | 46.983 | 1.00 | 0.00 | AAAA |
| ATOM | 518 | CG | LEU | E | 37 | 37.689 | 104.539 | 47.050 | 1.00 | 0.00 | AAAA |
| ATOM | 520 | CD1 | LEU | E | 37 | 36.753 | 103.340 | 46.895 | 1.00 | 0.00 | AAAA |
| ATOM | 524 | CD2 | LEU | E | 37 | 37.236 | 105.366 | 48.252 | 1.00 | 0.00 | AAAA |
| ATOM | 528 | C | LEU | E | 37 | 41.663 | 104.868 | 46.889 | 1.00 | 0.00 | AAAA |
| ATOM | 529 | O | LEU | E | 37 | 41.968 | 103.764 | 46.452 | 1.00 | 0.00 | AAAA |
| ATOM | 530 | N | MET | E | 38 | 42.574 | 105.738 | 47.330 | 1.00 | 0.00 | AAAA |
| ATOM | 532 | CA | MET | E | 38 | 44.026 | 105.664 | 47.290 | 1.00 | 0.00 | AAAA |
| ATOM | 534 | CB | MET | E | 38 | 44.715 | 106.400 | 46.147 | 1.00 | 0.00 | AAAA |
| ATOM | 537 | CG | MET | E | 38 | 44.102 | 105.958 | 44.826 | 1.00 | 0.00 | AAAA |
| ATOM | 540 | SD | MET | E | 38 | 44.934 | 106.518 | 43.316 | 1.00 | 0.00 | AAAA |
| ATOM | 541 | CE | MET | E | 38 | 44.013 | 105.579 | 42.085 | 1.00 | 0.00 | AAAA |
| ATOM | 545 | C | MET | E | 38 | 44.602 | 106.123 | 48.627 | 1.00 | 0.00 | AAAA |
| ATOM | 546 | O | MET | E | 38 | 44.895 | 107.300 | 48.815 | 1.00 | 0.00 | AAAA |
| ATOM | 547 | N | PHE | E | 39 | 44.566 | 105.226 | 49.618 | 1.00 | 0.00 | AAAA |
| ATOM | 549 | CA | PHE | E | 39 | 44.776 | 105.591 | 51.000 | 1.00 | 0.00 | AAAA |
| ATOM | 551 | CB | PHE | E | 39 | 43.872 | 104.797 | 51.953 | 1.00 | 0.00 | AAAA |
| ATOM | 554 | CG | PHE | E | 39 | 42.405 | 104.791 | 51.592 | 1.00 | 0.00 | AAAA |
| ATOM | 555 | CD1 | PHE | E | 39 | 41.901 | 103.665 | 50.929 | 1.00 | 0.00 | AAAA |
| ATOM | 557 | CD2 | PHE | E | 39 | 41.515 | 105.812 | 51.949 | 1.00 | 0.00 | AAAA |
| ATOM | 559 | CE1 | PHE | E | 39 | 40.565 | 103.613 | 50.487 | 1.00 | 0.00 | AAAA |
| ATOM | 561 | CE2 | THE | E | 39 | 40.148 | 105.706 | 51.658 | 1.00 | 0.00 | AAAA |
| ATOM | 563 | CZ | PHE | E | 39 | 39.741 | 104.699 | 50.773 | 1.00 | 0.00 | AAAA |
| ATOM | 565 | C | PHE | E | 39 | 46.187 | 105.398 | 51.514 | 1.00 | 0.00 | AAAA |
| ATOM | 566 | O | PHE | E | 39 | 46.570 | 106.041 | 52.494 | 1.00 | 0.00 | AAAA |
| ATOM | 567 | N | LYS | E | 40 | 46.939 | 104.541 | 50.819 | 1.00 | 0.00 | AAAA |
| ATOM | 569 | CA | LYS | E | 40 | 48.242 | 104.061 | 51.238 | 1.00 | 0.00 | AAAA |
| ATOM | 571 | CB | LYS | E | 40 | 48.469 | 102.647 | 50.721 | 1.00 | 0.00 | AAAA |
| ATOM | 574 | CG | LYS | E | 40 | 49.022 | 102.465 | 49.301 | 1.00 | 0.00 | AAAA |
| ATOM | 577 | CD | LYS | E | 40 | 49.077 | 101.001 | 48.864 | 1.00 | 0.00 | AAAA |
| ATOM | 580 | CE | LYS | E | 40 | 49.380 | 100.855 | 47.375 | 1.00 | 0.00 | AAAA |
| ATOM | 583 | NZ | LYS | E | 40 | 49.247 | 99.480 | 46.885 | 1.00 | 0.00 | AAAA |
| ATOM | 587 | C | LYS | E | 40 | 49.246 | 105.126 | 50.838 | 1.00 | 0.00 | AAAA |
| ATOM | 588 | O | LYS | E | 40 | 50.334 | 105.158 | 51.412 | 1.00 | 0.00 | AAAA |
| ATOM | 589 | N | THR | E | 41 | 48.930 | 105.961 | 49.845 | 1.00 | 0.00 | AAAA |
| ATOM | 591 | CA | THR | E | 41 | 49.643 | 107.095 | 49.296 | 1.00 | 0.00 | AAAA |
| ATOM | 593 | CB | THR | E | 41 | 48.774 | 107.477 | 48.094 | 1.00 | 0.00 | AAAA |
| ATOM | 595 | OG1 | THR | E | 41 | 48.489 | 106.396 | 47.237 | 1.00 | 0.00 | AAAA |
| ATOM | 597 | CG2 | THR | E | 41 | 49.355 | 108.612 | 47.248 | 1.00 | 0.00 | AAAA |
| ATOM | 601 | C | THR | E | 41 | 49.911 | 108.232 | 50.277 | 1.00 | 0.00 | AAAA |
| ATOM | 602 | O | THR | E | 41 | 49.004 | 108.707 | 50.956 | 1.00 | 0.00 | AAAA |
| ATOM | 603 | N | ARG | E | 42 | 51.157 | 108.712 | 50.274 | 1.00 | 0.00 | AAAA |
| ATOM | 605 | CA | ARG | E | 42 | 51.743 | 109.854 | 50.952 | 1.00 | 0.00 | AAAA |
| ATOM | 607 | CB | ARG | E | 42 | 53.199 | 109.503 | 51.254 | 1.00 | 0.00 | AAAA |
| ATOM | 610 | CG | ARG | E | 42 | 53.165 | 108.248 | 52.136 | 1.00 | 0.00 | AAAA |
| ATOM | 613 | CD | ARG | E | 42 | 54.573 | 107.797 | 52.539 | 1.00 | 0.00 | AAAA |
| ATOM | 616 | NE | ARG | E | 42 | 55.321 | 107.404 | 51.336 | 1.00 | 0.00 | AAAA |
| ATOM | 618 | CZ | ARG | E | 42 | 56.645 | 107.286 | 51.159 | 1.00 | 0.00 | AAAA |
| ATOM | 619 | NH1 | ARG | E | 42 | 57.494 | 107.568 | 52.158 | 1.00 | 0.00 | AAAA |
| ATOM | 622 | NH2 | ARG | E | 42 | 57.230 | 106.877 | 50.023 | 1.00 | 0.00 | AAAA |
| ATOM | 625 | C | ARG | E | 42 | 51.780 | 111.000 | 49.955 | 1.00 | 0.00 | AAAA |
| ATOM | 626 | O | ARG | E | 42 | 52.021 | 110.708 | 48.785 | 1.00 | 0.00 | AAAA |
| ATOM | 627 | N | PRO | E | 43 | 51.752 | 112.280 | 50.329 | 1.00 | 0.00 | AAAA |
| ATOM | 628 | CA | PRO | E | 43 | 52.186 | 113.354 | 49.461 | 1.00 | 0.00 | AAAA |
| ATOM | 630 | CB | PRO | E | 43 | 51.817 | 114.618 | 50.239 | 1.00 | 0.00 | AAAA |
| ATOM | 633 | CG | PRO | E | 43 | 51.668 | 114.222 | 51.709 | 1.00 | 0.00 | AAAA |
| ATOM | 636 | CD | PRO | E | 43 | 51.354 | 112.726 | 51.647 | 1.00 | 0.00 | AAAA |
| ATOM | 639 | C | PRO | E | 43 | 53.648 | 113.272 | 49.054 | 1.00 | 0.00 | AAAA |
| ATOM | 640 | O | PRO | E | 43 | 53.964 | 113.626 | 47.920 | 1.00 | 0.00 | AAAA |
| ATOM | 641 | N | GLU | E | 44 | 54.441 | 112.441 | 49.743 | 1.00 | 0.00 | AAAA |
| ATOM | 643 | CA | GLU | E | 44 | 55.836 | 112.192 | 49.464 | 1.00 | 0.00 | AAAA |
| ATOM | 645 | CB | GLU | E | 44 | 56.471 | 111.787 | 50.795 | 1.00 | 0.00 | AAAA |
| ATOM | 648 | CG | GLU | E | 44 | 57.989 | 111.619 | 50.875 | 1.00 | 0.00 | AAAA |
| ATOM | 651 | CD | GLU | E | 44 | 58.647 | 112.929 | 51.301 | 1.00 | 0.00 | AAAA |
| ATOM | 652 | OE1 | GLU | E | 44 | 58.259 | 114.046 | 50.879 | 1.00 | 0.00 | AAAA |
| ATOM | 653 | OE2 | GLU | E | 44 | 59.561 | 112.859 | 52.146 | 1.00 | 0.00 | AAAA |
| ATOM | 654 | C | GLU | E | 44 | 55.985 | 111.130 | 48.373 | 1.00 | 0.00 | AAAA |
| ATOM | 655 | O | GLU | E | 44 | 56.966 | 111.146 | 47.630 | 1.00 | 0.00 | AAAA |
| ATOM | 656 | N | ASP | E | 45 | 54.970 | 110.292 | 48.155 | 1.00 | 0.00 | AAAA |
| ATOM | 658 | CA | ASP | E | 45 | 54.823 | 109.398 | 47.028 | 1.00 | 0.00 | AAAA |
| ATOM | 660 | CB | ASP | E | 45 | 53.797 | 108.286 | 47.271 | 1.00 | 0.00 | AAAA |
| ATOM | 663 | CG | ASP | E | 45 | 54.216 | 107.290 | 48.351 | 1.00 | 0.00 | AAAA |
| ATOM | 664 | OD1 | ASP | E | 45 | 55.233 | 106.608 | 48.119 | 1.00 | 0.00 | AAAA |
| ATOM | 665 | OD2 | ASP | E | 45 | 53.507 | 107.023 | 49.339 | 1.00 | 0.00 | AAAA |
| ATOM | 666 | C | ASP | E | 45 | 54.337 | 110.251 | 45.869 | 1.00 | 0.00 | AAAA |
| ATOM | 667 | O | ASP | E | 45 | 54.988 | 110.408 | 44.836 | 1.00 | 0.00 | AAAA |
| ATOM | 668 | N | PHE | E | 46 | 53.185 | 110.899 | 46.055 | 1.00 | 0.00 | AAAA |
| ATOM | 670 | CA | PHE | E | 46 | 52.290 | 111.610 | 45.166 | 1.00 | 0.00 | AAAA |
| ATOM | 672 | CB | PHE | E | 46 | 51.018 | 112.175 | 45.808 | 1.00 | 0.00 | AAAA |
| ATOM | 675 | CG | PHE | E | 46 | 49.778 | 112.254 | 44.949 | 1.00 | 0.00 | AAAA |
| ATOM | 676 | CD1 | PHE | E | 46 | 49.222 | 113.490 | 44.618 | 1.00 | 0.00 | AAAA |
| ATOM | 678 | CD2 | PHE | E | 46 | 49.274 | 111.079 | 44.384 | 1.00 | 0.00 | AAAA |
| ATOM | 680 | CE1 | PHE | E | 46 | 48.302 | 113.629 | 43.568 | 1.00 | 0.00 | AAAA |
| ATOM | 682 | CE2 | PHE | E | 46 | 48.233 | 111.189 | 43.447 | 1.00 | 0.00 | AAAA |
| ATOM | 684 | CZ | PHE | E | 46 | 47.829 | 112.450 | 42.986 | 1.00 | 0.00 | AAAA |
| ATOM | 686 | C | PHE | E | 46 | 52.966 | 112.689 | 44.346 | 1.00 | 0.00 | AAAA |
| ATOM | 687 | O | PHE | E | 46 | 52.552 | 112.874 | 43.199 | 1.00 | 0.00 | AAAA |
| ATOM | 688 | N | ARG | E | 47 | 54.022 | 113:314 | 44.865 | 1.00 | 0.00 | AAAA |
| ATOM | 690 | CA | ARG | E | 47 | 54.834 | 114.368 | 44.270 | 1.00 | 0.00 | AAAA |
| ATOM | 692 | CB | ARG | E | 47 | 55.946 | 114.792 | 45.229 | 1.00 | 0.00 | AAAA |
| ATOM | 695 | CG | ARG | E | 47 | 56.702 | 113.717 | 46.019 | 1.00 | 0.00 | AAAA |
| ATOM | 698 | CD | ARG | E | 47 | 58.229 | 113.795 | 46.098 | 1.00 | 0.00 | AAAA |
| ATOM | 701 | NE | ARG | E | 47 | 58.759 | 113.439 | 44.785 | 1.00 | 0.00 | AAAA |
| ATOM | 703 | CZ | ARG | E | 47 | 59.091 | 112.185 | 44.474 | 1.00 | 0.00 | AAAA |
| ATOM | 704 | NH1 | ARG | E | 47 | 58.621 | 111.160 | 45.200 | 1.00 | 0.00 | AAAA |
| ATOM | 707 | NH2 | ARG | E | 47 | 59.868 | 111.950 | 43.411 | 1.00 | 0.00 | AAAA |
| ATOM | 710 | C | ARG | E | 47 | 55.445 | 114.065 | 42.918 | 1.00 | 0.00 | AAAA |
| ATOM | 711 | O | ARG | E | 47 | 55.656 | 115.028 | 42.175 | 1.00 | 0.00 | AAAA |
| ATOM | 712 | N | ASP | E | 48 | 55.676 | 112.784 | 42.615 | 1.00 | 0.00 | AAAA |
| ATOM | 714 | CA | ASP | E | 48 | 56.259 | 112.283 | 41.386 | 1.00 | 0.00 | AAAA |
| ATOM | 716 | CB | ASP | E | 48 | 57.007 | 111.009 | 41.777 | 1.00 | 0.00 | AAAA |
| ATOM | 719 | CG | ASP | E | 48 | 57.974 | 110.442 | 40.751 | 1.00 | 0.00 | AAAA |
| ATOM | 720 | OD1 | ASP | E | 48 | 59.109 | 110.938 | 40.605 | 1.00 | 0.00 | AAAA |
| ATOM | 721 | OD2 | ASP | E | 48 | 57.701 | 109.362 | 40.191 | 1.00 | 0.00 | AAAA |
| ATOM | 722 | C | ASP | E | 48 | 55.295 | 112.054 | 40.234 | 1.00 | 0.00 | AAAA |
| ATOM | 723 | O | ASP | E | 48 | 55.653 | 111.763 | 39.096 | 1.00 | 0.00 | AAAA |
| ATOM | 724 | N | LEU | E | 49 | 53.997 | 112.250 | 40.492 | 1.00 | 0.00 | AAAA |
| ATOM | 726 | CA | LEU | E | 49 | 52.936 | 111.959 | 39.544 | 1.00 | 0.00 | AAAA |
| ATOM | 728 | CB | LEU | E | 49 | 51.798 | 111.166 | 40.188 | 1.00 | 0.00 | AAAA |
| ATOM | 731 | CG | LEU | E | 49 | 51.947 | 109.672 | 40.464 | 1.00 | 0.00 | AAAA |
| ATOM | 733 | CD1 | LEU | E | 49 | 51.237 | 109.277 | 41.761 | 1.00 | 0.00 | AAAA |
| ATOM | 737 | CD2 | LEU | E | 49 | 51.522 | 108.796 | 39.282 | 1.00 | 0.00 | AAAA |
| ATOM | 741 | C | LEU | E | 49 | 52.366 | 113.201 | 38.857 | 1.00 | 0.00 | AAAA |
| ATOM | 742 | O | LEU | E | 49 | 51.755 | 114.048 | 39.483 | 1.00 | 0.00 | AAAA |
| ATOM | 743 | N | SER | E | 50 | 52.466 | 113.228 | 37.526 | 1.00 | 0.00 | AAAA |
| ATOM | 745 | CA | SER | E | 50 | 51.819 | 114.229 | 36.697 | 1.00 | 0.00 | AAAA |
| ATOM | 747 | CB | SER | E | 50 | 52.721 | 115.445 | 36.475 | 1.00 | 0.00 | AAAA |
| ATOM | 750 | OG | SER | E | 50 | 52.043 | 116.421 | 35.731 | 1.00 | 0.00 | AAAA |
| ATOM | 752 | C | SER | E | 50 | 51.350 | 113.474 | 35.469 | 1.00 | 0.00 | AAAA |
| ATOM | 753 | O | SER | E | 50 | 52.110 | 112.681 | 34.925 | 1.00 | 0.00 | AAAA |
| ATOM | 754 | N | PHE | E | 51 | 50.201 | 113.752 | 34.849 | 1.00 | 0.00 | AAAA |
| ATOM | 756 | CA | PHE | E | 51 | 49.747 | 113.232 | 33.575 | 1.00 | 0.00 | AAAA |
| ATOM | 758 | CB | PHE | E | 51 | 48.633 | 112.185 | 33.786 | 1.00 | 0.00 | AAAA |
| ATOM | 761 | CG | PHE | E | 51 | 48.917 | 110.851 | 34.437 | 1.00 | 0.00 | AAAA |
| ATOM | 762 | CD1 | PHE | E | 51 | 49.925 | 109.999 | 33.971 | 1.00 | 0.00 | AAAA |
| ATOM | 764 | CD2 | PHE | E | 51 | 48.192 | 110.348 | 35.516 | 1.00 | 0.00 | AAAA |
| ATOM | 766 | CE1 | PHE | E | 51 | 50.141 | 108.716 | 34.471 | 1.00 | 0.00 | AAAA |
| ATOM | 768 | CE2 | PHE | E | 51 | 48.429 | 109.092 | 36.090 | 1.00 | 0.00 | AAAA |
| ATOM | 770 | CZ | PHE | E | 51 | 49.353 | 108.219 | 35.514 | 1.00 | 0.00 | AAAA |
| ATOM | 772 | C | PHE | E | 51 | 49.354 | 114.474 | 32.782 | 1.00 | 0.00 | AAAA |
| ATOM | 773 | O | PHE | E | 51 | 48.154 | 114.647 | 32.630 | 1.00 | 0.00 | AAAA |
| ATOM | 774 | N | PRO | E | 52 | 50.259 | 115.317 | 32.275 | 1.00 | 0.00 | AAAA |
| ATOM | 775 | CA | PRO | E | 52 | 49.941 | 116.531 | 31.546 | 1.00 | 0.00 | AAAA |
| ATOM | 777 | CB | PRO | E | 52 | 51.217 | 117.365 | 31.589 | 1.00 | 0.00 | AAAA |
| ATOM | 780 | CG | PRO | E | 52 | 52.281 | 116.275 | 31.464 | 1.00 | 0.00 | AAAA |
| ATOM | 783 | CD | PRO | E | 52 | 51.688 | 115.113 | 32.266 | 1.00 | 0.00 | AAAA |
| ATOM | 786 | C | PRO | E | 52 | 49.586 | 116.324 | 30.086 | 1.00 | 0.00 | AAAA |
| ATOM | 787 | O | PRO | E | 52 | 49.323 | 117.322 | 29.422 | 1.00 | 0.00 | AAAA |
| ATOM | 788 | N | LYS | E | 53 | 49.690 | 115.145 | 29.466 | 1.00 | 0.00 | AAAA |
| ATOM | 790 | CA | LYS | E | 53 | 49.485 | 114.993 | 28.042 | 1.00 | 0.00 | AAAA |
| ATOM | 792 | CB | LYS | E | 53 | 50.322 | 113.815 | 27.567 | 1.00 | 0.00 | AAAA |
| ATOM | 795 | CG | LYS | E | 53 | 51.820 | 113.977 | 27.837 | 1.00 | 0.00 | AAAA |
| ATOM | 798 | CD | LYS | E | 53 | 52.918 | 113.252 | 27.053 | 1.00 | 0.00 | AAAA |
| ATOM | 801 | CE | LYS | E | 53 | 54.232 | 113.997 | 27.225 | 1.00 | 0.00 | AAAA |
| ATOM | 804 | NZ | LYS | E | 53 | 55.326 | 113.361 | 26.473 | 1.00 | 0.00 | AAAA |
| ATOM | 808 | C | LYS | E | 53 | 48.049 | 114.592 | 27.758 | 1.00 | 0.00 | AAAA |
| ATOM | 809 | O | LYS | E | 53 | 47.717 | 114.505 | 26.581 | 1.00 | 0.00 | AAAA |
| ATOM | 810 | N | LEU | E | 54 | 47.259 | 114.401 | 28.824 | 1.00 | 0.00 | AAAA |
| ATOM | 812 | CA | LEU | E | 54 | 45.832 | 114.230 | 28.710 | 1.00 | 0.00 | AAAA |
| ATOM | 814 | CB | LEU | E | 54 | 45.248 | 113.538 | 29.942 | 1.00 | 0.00 | AAAA |
| ATOM | 817 | CG | LEU | E | 54 | 46.085 | 112.538 | 30.730 | 1.00 | 0.00 | AAAA |
| ATOM | 819 | CD1 | LEU | E | 54 | 45.356 | 112.025 | 31.967 | 1.00 | 0.00 | AAAA |
| ATOM | 823 | CD2 | LEU | E | 54 | 46.480 | 111.363 | 29.837 | 1.00 | 0.00 | AAAA |
| ATOM | 827 | C | LEU | E | 54 | 45.211 | 115.618 | 28.556 | 1.00 | 0.00 | AAAA |
| ATOM | 828 | O | LEU | E | 54 | 45.422 | 116.552 | 29.319 | 1.00 | 0.00 | AAAA |
| ATOM | 829 | N | ILE | E | 55 | 44.314 | 115.794 | 27.582 | 1.00 | 0.00 | AAAA |
| ATOM | 831 | CA | ILE | E | 55 | 43.668 | 117.037 | 27.200 | 1.00 | 0.00 | AAAA |
| ATOM | 833 | CB | ILE | E | 55 | 44.338 | 117.889 | 26.117 | 1.00 | 0.00 | AAAA |
| ATOM | 835 | CG1 | ILE | E | 55 | 44.072 | 117.258 | 24.742 | 1.00 | 0.00 | AAAA |
| ATOM | 838 | CG2 | ILE | E | 55 | 45.821 | 118.177 | 26.334 | 1.00 | 0.00 | AAAA |
| ATOM | 842 | CD1 | ILE | E | 55 | 44.290 | 118.236 | 23.587 | 1.00 | 0.00 | AAAA |
| ATOM | 846 | C | ILE | E | 55 | 42.154 | 116.937 | 27.033 | 1.00 | 0.00 | AAAA |
| ATOM | 847 | O | ILE | E | 55 | 41.414 | 117.907 | 27.111 | 1.00 | 0.00 | AAAA |
| ATOM | 848 | N | MET | E | 56 | 41.607 | 115.734 | 26.846 | 1.00 | 0.00 | AAAA |
| ATOM | 850 | CA | MET | E | 56 | 40.183 | 115.523 | 26.719 | 1.00 | 0.00 | AAAA |
| ATOM | 852 | CB | MET | E | 56 | 39.791 | 115.484 | 25.234 | 1.00 | 0.00 | AAAA |
| ATOM | 855 | CG | MET | E | 56 | 38.368 | 115.189 | 24.785 | 1.00 | 0.00 | AAAA |
| ATOM | 858 | SD | MET | E | 56 | 38.168 | 115.426 | 23.001 | 1.00 | 0.00 | AAAA |
| ATOM | 859 | CE | MET | E | 56 | 36.562 | 114.665 | 22.636 | 1.00 | 0.00 | AAAA |
| ATOM | 863 | C | MET | E | 56 | 39.843 | 114.287 | 27.530 | 1.00 | 0.00 | AAAA |
| ATOM | 864 | O | MET | E | 56 | 40.582 | 113.298 | 27.485 | 1.00 | 0.00 | AAAA |
| ATOM | 865 | N | ILE | E | 57 | 38.778 | 114.383 | 28.341 | 1.00 | 0.00 | AAAA |
| ATOM | 867 | CA | ILE | E | 57 | 38.025 | 113.255 | 28.833 | 1.00 | 0.00 | AAAA |
| ATOM | 869 | CB | ILE | E | 57 | 37.800 | 113.384 | 30.343 | 1.00 | 0.00 | AAAA |
| ATOM | 871 | CG1 | ILE | E | 57 | 39.124 | 113.052 | 31.024 | 1.00 | 0.00 | AAAA |
| ATOM | 874 | CG2 | ILE | E | 57 | 36.713 | 112.526 | 30.975 | 1.00 | 0.00 | AAAA |
| ATOM | 878 | CD1 | ILE | E | 57 | 39.154 | 113.062 | 32.552 | 1.00 | 0.00 | AAAA |
| ATOM | 882 | C | ILE | E | 57 | 36.690 | 113.335 | 28.106 | 1.00 | 0.00 | AAAA |
| ATOM | 883 | O | ILE | E | 57 | 36.259 | 114.451 | 27.810 | 1.00 | 0.00 | AAAA |
| ATOM | 884 | N | THR | E | 58 | 35.980 | 112.258 | 27.782 | 1.00 | 0.00 | AAAA |
| ATOM | 886 | CA | THR | E | 58 | 34.724 | 112.318 | 27.051 | 1.00 | 0.00 | AAAA |
| ATOM | 888 | CB | THR | E | 58 | 34.892 | 111.402 | 25.836 | 1.00 | 0.00 | AAAA |
| ATOM | 890 | OG1 | THR | E | 58 | 36.140 | 111.543 | 25.192 | 1.00 | 0.00 | AAAA |
| ATOM | 892 | CG2 | THR | E | 58 | 33.773 | 111.742 | 24.845 | 1.00 | 0.00 | AAAA |
| ATOM | 896 | C | THR | E | 58 | 33.542 | 112.050 | 27.973 | 1.00 | 0.00 | AAAA |
| ATOM | 897 | O | THR | E | 58 | 32.510 | 112.654 | 27.690 | 1.00 | 0.00 | AAAA |
| ATOM | 898 | N | ASP | E | 59 | 33.707 | 111.277 | 29.049 | 1.00 | 0.00 | AAAA |
| ATOM | 900 | CA | ASP | E | 59 | 32.659 | 111.126 | 30.039 | 1.00 | 0.00 | AAAA |
| ATOM | 902 | CB | ASP | E | 59 | 32.388 | 109.662 | 30.400 | 1.00 | 0.00 | AAAA |
| ATOM | 905 | CG | ASP | E | 59 | 31.936 | 108.673 | 29.340 | 1.00 | 0.00 | AAAA |
| ATOM | 906 | OD1 | ASP | E | 59 | 31.702 | 108.922 | 28.131 | 1.00 | 0.00 | AAAA |
| ATOM | 907 | OD2 | ASP | E | 59 | 31.825 | 107.481 | 29.686 | 1.00 | 0.00 | AAAA |
| ATOM | 908 | C | ASP | E | 59 | 33.008 | 112.093 | 31.163 | 1.00 | 0.00 | AAAA |
| ATOM | 909 | O | ASP | E | 59 | 32.828 | 113.300 | 31.058 | 1.00 | 0.00 | AAAA |
| ATOM | 910 | N | TYR | E | 60 | 33.526 | 111.615 | 32.290 | 1.00 | 0.00 | AAAA |
| ATOM | 912 | CA | TYR | E | 60 | 33.735 | 112.371 | 33.507 | 1.00 | 0.00 | AAAA |
| ATOM | 914 | CB | TYR | E | 60 | 32.580 | 112.137 | 34.473 | 1.00 | 0.00 | AAAA |
| ATOM | 917 | CG | TYR | E | 60 | 32.163 | 110.711 | 34.730 | 1.00 | 0.00 | AAAA |
| ATOM | 918 | CD1 | TYR | E | 60 | 32.658 | 110.036 | 35.856 | 1.00 | 0.00 | AAAA |
| ATOM | 920 | CD2 | TYR | E | 60 | 31.165 | 110.135 | 33.937 | 1.00 | 0.00 | AAAA |
| ATOM | 922 | CE1 | TYR | E | 60 | 32.154 | 108.783 | 36.234 | 1.00 | 0.00 | AAAA |
| ATOM | 924 | CE2 | TYR | E | 60 | 30.596 | 108.914 | 34.304 | 1.00 | 0.00 | AAAA |
| ATOM | 926 | CZ | TYR | E | 60 | 31.103 | 108.286 | 35.443 | 1.00 | 0.00 | AAAA |
| ATOM | 927 | OH | TYR | E | 60 | 30.659 | 107.101 | 35.944 | 1.00 | 0.00 | AAAA |
| ATOM | 929 | C | TYR | E | 60 | 35.049 | 112.103 | 34.236 | 1.00 | 0.00 | AAAA |
| ATOM | 930 | O | TYR | E | 60 | 35.611 | 111.041 | 34.010 | 1.00 | 0.00 | AAAA |
| ATOM | 931 | N | LEU | E | 61 | 35.517 | 113.076 | 35.024 | 1.00 | 0.00 | AAAA |
| ATOM | 933 | CA | LEU | E | 61 | 36.596 | 113.033 | 35.991 | 1.00 | 0.00 | AAAA |
| ATOM | 935 | CB | LEU | E | 61 | 37.357 | 114.365 | 35.921 | 1.00 | 0.00 | AAAA |
| ATOM | 938 | CG | LEU | E | 61 | 38.588 | 114.468 | 36.830 | 1.00 | 0.00 | AAAA |
| ATOM | 940 | CD1 | LEU | E | 61 | 39.547 | 113.297 | 36.635 | 1.00 | 0.00 | AAAA |
| ATOM | 944 | CD2 | LEU | E | 61 | 39.250 | 115.844 | 36.778 | 1.00 | 0.00 | AAAA |
| ATOM | 948 | C | LEU | E | 61 | 36.001 | 112.818 | 37.375 | 1.00 | 0.00 | AAAA. |
| ATOM | 949 | O | LEU | E | 61 | 35.034 | 113.499 | 37.718 | 1.00 | 0.00 | AAAA |
| ATOM | 950 | N | LEU | E | 62 | 36.575 | 111.904 | 38.159 | 1.00 | 0.00 | AAAA |
| ATOM | 952 | CA | LEU | E | 62 | 36.162 | 111.624 | 39.513 | 1.00 | 0.00 | AAAA |
| ATOM | 954 | CB | LEU | E | 62 | 34.916 | 110.749 | 39.587 | 1.00 | 0.00 | AAAA |
| ATOM | 957 | CG | LEU | E | 62 | 34.336 | 110.423 | 40.968 | 1.00 | 0.00 | AAAA |
| ATOM | 959 | CD1 | LEU | E | 62 | 33.817 | 111.615 | 41.767 | 1.00 | 0.00 | AAAA |
| ATOM | 963 | CD2 | LEU | E | 62 | 33.194 | 109.416 | 40.831 | 1.00 | 0.00 | AAAA |
| ATOM | 967 | C | LEU | E | 62 | 37.383 | 111.160 | 40.306 | 1.00 | 0.00 | AAAA |
| ATOM | 968 | O | LEU | E | 62 | 37.837 | 110.019 | 40.241 | 1.00 | 0.00 | AAAA |
| ATOM | 969 | N | LEU | E | 63 | 37.901 | 112.081 | 41.117 | 1.00 | 0.00 | AAAA |
| ATOM | 971 | CA | LEU | E | 63 | 38.681 | 111.675 | 42.264 | 1.00 | 0.00 | AAAA |
| ATOM | 973 | CB | LEU | E | 63 | 39.597 | 112.826 | 42.693 | 1.00 | 0.00 | AAAA |
| ATOM | 976 | CG | LEU | E | 63 | 40.839 | 113.076 | 41.849 | 1.00 | 0.00 | AAAA |
| ATOM | 978 | CD1 | LEU | E | 63 | 40.611 | 113.768 | 40.508 | 1.00 | 0.00 | AAAA |
| ATOM | 982 | CD2 | LEU | E | 63 | 41.935 | 113.802 | 42.624 | 1.00 | 0.00 | AAAA |
| ATOM | 986 | C | LEU | E | 63 | 37.754 | 111.497 | 43.460 | 1.00 | 0.00 | AAAA |
| ATOM | 987 | O | LEU | E | 63 | 36.615 | 111.955 | 43.423 | 1.00 | 0.00 | AAAA |
| ATOM | 988 | N | PHE | E | 64 | 38.262 | 110.933 | 44.560 | 1.00 | 0.00 | AAAA |
| ATOM | 990 | CA | PHE | E | 64 | 37.581 | 110.741 | 45.828 | 1.00 | 0.00 | AAAA |
| ATOM | 992 | CB | PHE | E | 64 | 36.231 | 110.032 | 45.639 | 1.00 | 0.00 | AAAA |
| ATOM | 995 | CG | PHE | E | 64 | 35.474 | 109.816 | 46.918 | 1.00 | 0.00 | AAAA |
| ATOM | 996 | CD1 | PHE | E | 64 | 35.208 | 110.888 | 47.780 | 1.00 | 0.00 | AAAA |
| ATOM | 998 | CD2 | PHE | E | 64 | 35.053 | 108.530 | 47.294 | 1.00 | 0.00 | AAAA |
| ATOM | 1000 | CE1 | PHE | E | 64 | 34.509 | 110.709 | 48.981 | 1.00 | 0.00 | AAAA |
| ATOM | 1002 | CE2 | PHE | E | 64 | 34.361 | 108.411 | 48.505 | 1.00 | 0.00 | AAAA |
| ATOM | 1004 | CZ | PHE | E | 64 | 34.084 | 109.444 | 49.403 | 1.00 | 0.00 | AAAA |
| ATOM | 1006 | C | PHE | E | 64 | 38.555 | 109.960 | 46.696 | 1.00 | 0.00 | AAAA |
| ATOM | 1007 | O | PHE | E | 64 | 38.928 | 108.885 | 46.243 | 1.00 | 0.00 | AAAA |
| ATOM | 1008 | N | ARG | E | 65 | 38.986 | 110.528 | 47.822 | 1.00 | 0.00 | AAAA |
| ATOM | 1010 | CA | ARG | E | 65 | 39.836 | 109.973 | 48.854 | 1.00 | 0.00 | AAAA |
| ATOM | 1012 | CB | ARG | E | 65 | 39.131 | 108.925 | 49.707 | 1.00 | 0.00 | AAAA |
| ATOM | 1015 | CG | ARG | E | 65 | 38.106 | 109.662 | 50.569 | 1.00 | 0.00 | AAAA |
| ATOM | 1018 | CD | ARG | E | 65 | 37.191 | 108.657 | 51.283 | 1.00 | 0.00 | AAAA |
| ATOM | 1021 | NE | ARG | E | 65 | 36.297 | 109.430 | 52.155 | 1.00 | 0.00 | AAAA |
| ATOM | 1023 | CZ | ARG | E | 65 | 35.533 | 108.867 | 53.100 | 1.00 | 0.00 | AAAA |
| ATOM | 1024 | NH1 | ARG | E | 65 | 35.549 | 107.562 | 53.413 | 1.00 | 0.00 | AAAA |
| ATOM | 1027 | NH2 | ARG | E | 65 | 34.638 | 109.629 | 53.737 | 1.00 | 0.00 | AAAA |
| ATOM | 1030 | C | ARG | E | 65 | 41.150 | 109.447 | 48.300 | 1.00 | 0.00 | AAAA |
| ATOM | 1031 | O | ARG | E | 65 | 41.483 | 108.273 | 48.439 | 1.00 | 0.00 | AAAA |
| ATOM | 1032 | N | VAL | E | 66 | 41.808 | 110.279 | 47.498 | 1.00 | 0.00 | AAAA |
| ATOM | 1034 | CA | VAL | E | 66 | 43.181 | 110.114 | 47.054 | 1.00 | 0.00 | AAAA |
| ATOM | 1036 | CB | VAL | E | 66 | 43.399 | 110.773 | 45.689 | 1.00 | 0.00 | AAAA |
| ATOM | 1038 | CG1 | VAL | E | 66 | 44.811 | 110.839 | 45.108 | 1.00 | 0.00 | AAAA |
| ATOM | 1042 | CG2 | VAL | E | 66 | 42.447 | 110.190 | 44.642 | 1.00 | 0.00 | AAAA |
| ATOM | 1046 | C | VAL | E | 66 | 43.967 | 110.882 | 48.107 | 1.00 | 0.00 | AAAA |
| ATOM | 1047 | O | VAL | E | 66 | 43.779 | 112.097 | 48.137 | 1.00 | 0.00 | AAAA |
| ATOM | 1048 | N | TYR | E | 67 | 44.686 | 110.222 | 49.027 | 1.00 | 0.00 | AAAA |
| ATOM | 1050 | CA | TYR | E | 67 | 45.473 | 110.879 | 50.056 | 1.00 | 0.00 | AAAA |
| ATOM | 1052 | CB | TYR | E | 67 | 45.684 | 109.840 | 51.151 | 1.00 | 0.00 | AAAA |
| ATOM | 1055 | CG | TYR | E | 67 | 44.456 | 109.556 | 51.987 | 1.00 | 0.00 | AAAA |
| ATOM | 1056 | CD1 | TYR | E | 67 | 43.199 | 110.176 | 51.912 | 1.00 | 0.00 | AAAA |
| ATOM | 1058 | CD2 | TYR | E | 67 | 44.681 | 108.684 | 53.051 | 1.00 | 0.00 | AAAA |
| ATOM | 1060 | CE1 | TYR | E | 67 | 42.177 | 109.966 | 52.852 | 1.00 | 0.00 | AAAA |
| ATOM | 1062 | CE2 | TYR | E | 67 | 43.695 | 108.487 | 54.025 | 1.00 | 0.00 | AAAA |
| ATOM | 1064 | CZ | TYR | E | 67 | 42.435 | 109.093 | 53.923 | 1.00 | 0.00 | AAAA |
| ATOM | 1065 | OH | TYR | E | 67 | 41.444 | 108.893 | 54.836 | 1.00 | 0.00 | AAAA |
| ATOM | 1067 | C | TYR | E | 67 | 46.810 | 111.239 | 49.419 | 1.00 | 0.00 | AAAA |
| ATOM | 1068 | O | TYR | E | 67 | 47.385 | 110.445 | 48.675 | 1.00 | 0.00 | AAAA |
| ATOM | 1069 | N | GLY | E | 68 | 47.301 | 112.445 | 49.690 | 1.00 | 0.00 | AAAA |
| ATOM | 1071 | CA | GLY | E | 68 | 48.554 | 112.939 | 49.159 | 1.00 | 0.00 | AAAA |
| ATOM | 1074 | C | GLY | E | 68 | 48.294 | 114.036 | 48.136 | 1.00 | 0.00 | AAAA |
| ATOM | 1075 | O | GLY | E | 68 | 49.209 | 114.702 | 47.673 | 1.00 | 0.00 | AAAA |
| ATOM | 1076 | N | LEU | E | 69 | 47.061 | 114.185 | 47.638 | 1.00 | 0.00 | AAAA |
| ATOM | 1078 | CA | LEU | E | 69 | 46.610 | 115.250 | 46.767 | 1.00 | 0.00 | AAAA |
| ATOM | 1080 | CB | LEU | E | 69 | 45.192 | 114.939 | 46.295 | 1.00 | 0.00 | AAAA |
| ATOM | 1083 | CG | LEU | E | 69 | 44.645 | 115.995 | 45.322 | 1.00 | 0.00 | AAAA |
| ATOM | 1085 | CD1 | LEU | E | 69 | 45.087 | 115.685 | 43.894 | 1.00 | 0.00 | AAAA |
| ATOM | 1089 | CD2 | LEU | E | 69 | 43.137 | 115.771 | 45.365 | 1.00 | 0.00 | AAAA |
| ATOM | 1093 | C | LEU | E | 69 | 46.642 | 116.571 | 47.517 | 1.00 | 0.00 | AAAA |
| ATOM | 1094 | O | LEU | E | 69 | 46.160 | 116.764 | 48.630 | 1.00 | 0.00 | AAAA |
| ATOM | 1095 | N | GLU | E | 70 | 47.231 | 117.591 | 46.883 | 1.00 | 0.00 | AAAA |
| ATOM | 1097 | CA | GLU | E | 70 | 46.955 | 118.963 | 47.252 | 1.00 | 0.00 | AAAA |
| ATOM | 1099 | CB | GLU | E | 70 | 48.280 | 119.554 | 47.737 | 1.00 | 0.00 | AAAA |
| ATOM | 1102 | CG | GLU | E | 70 | 48.237 | 120.950 | 48.371 | 1.00 | 0.00 | AAAA |
| ATOM | 1105 | CD | GLU | E | 70 | 49.588 | 121.450 | 48.848 | 1.00 | 0.00 | AAAA |
| ATOM | 1106 | OE1 | GLU | E | 70 | 49.862 | 121.297 | 50.057 | 1.00 | 0.00 | AAAA |
| ATOM | 1107 | OE2 | GLU | E | 70 | 50.393 | 121.897 | 48.007 | 1.00 | 0.00 | AAAA |
| ATOM | 1108 | C | GLU | E | 70 | 46.431 | 119.696 | 46.023 | 1.00 | 0.00 | AAAA |
| ATOM | 1109 | O | GLU | E | 70 | 45.538 | 120.537 | 46.102 | 1.00 | 0.00 | AAAA |
| ATOM | 1110 | N | SER | E | 71 | 47.095 | 119.470 | 44.884 | 1.00 | 0.00 | AAAA |
| ATOM | 1112 | CA | SER | E | 71 | 46.996 | 120.279 | 43.689 | 1.00 | 0.00 | AAAA |
| ATOM | 1114 | CB | SER | E | 71 | 48.407 | 120.744 | 43.338 | 1.00 | 0.00 | AAAA |
| ATOM | 1117 | OG | SER | E | 71 | 48.409 | 121.565 | 42.191 | 1.00 | 0.00 | AAAA |
| ATOM | 1119 | C | SER | E | 71 | 46.389 | 119.369 | 42.619 | 1.00 | 0.00 | AAAA |
| ATOM | 1120 | O | SER | E | 71 | 46.948 | 118.282 | 42.446 | 1.00 | 0.00 | AAAA |
| ATOM | 1121 | N | LEU | E | 72 | 45.405 | 119.815 | 41.845 | 1.00 | 0.00 | AAAA |
| ATOM | 1123 | CA | LEU | E | 72 | 45.002 | 119.247 | 40.573 | 1.00 | 0.00 | AAAA |
| ATOM | 1125 | CB | LEU | E | 72 | 43.501 | 119.388 | 40.301 | 1.00 | 0.00 | AAAA |
| ATOM | 1128 | CG | LEU | E | 72 | 42.524 | 118.913 | 41.377 | 1.00 | 0.00 | AAAA |
| ATOM | 1130 | CD1 | LEU | E | 72 | 41.173 | 119.572 | 41.101 | 1.00 | 0.00 | AAAA |
| ATOM | 1134 | CD2 | LEU | E | 72 | 42.458 | 117.398 | 41.561 | 1.00 | 0.00 | AAAA |
| ATOM | 1138 | C | LEU | E | 72 | 45.789 | 119.870 | 39.427 | 1.00 | 0.00 | AAAA |
| ATOM | 1139 | O | LEU | E | 72 | 45.759 | 119.490 | 38.264 | 1.00 | 0.00 | AAAA |
| ATOM | 1140 | N | LYS | E | 73 | 46.470 | 120.973 | 39.761 | 1.00 | 0.00 | AAAA |
| ATOM | 1142 | CA | LYS | E | 73 | 47.218 | 121.878 | 38.916 | 1.00 | 0.00 | AAAA |
| ATOM | 1144 | CB | LYS | E | 73 | 47.460 | 123.215 | 39.626 | 1.00 | 0.00 | AAAA |
| ATOM | 1147 | CG | LYS | E | 73 | 47.656 | 124.400 | 38.679 | 1.00 | 0.00 | AAAA |
| ATOM | 1150 | CD | LYS | E | 73 | 47.604 | 125.798 | 39.308 | 1.00 | 0.00 | AAAA |
| ATOM | 1153 | CE | LYS | E | 73 | 48.649 | 126.100 | 40.387 | 1.00 | 0.00 | AAAA |
| ATOM | 1156 | NZ | LYS | E | 73 | 48.424 | 127.490 | 40.834 | 1.00 | 0.00 | AAAA |
| ATOM | 1160 | C | LYS | E | 73 | 48.608 | 121.312 | 38.621 | 1.00 | 0.00 | AAAA |
| ATOM | 1161 | O | LYS | E | 73 | 49.256 | 121.711 | 37.649 | 1.00 | 0.00 | AAAA |
| ATOM | 1162 | N | ASP | E | 74 | 49.154 | 120.368 | 39.387 | 1.00 | 0.00 | AAAA |
| ATOM | 1164 | CA | ASP | E | 74 | 50.417 | 119.697 | 39.134 | 1.00 | 0.00 | AAAA |
| ATOM | 1166 | CB | ASP | E | 74 | 51.293 | 119.636 | 40.376 | 1.00 | 0.00 | AAAA |
| ATOM | 1169 | CG | ASP | E | 74 | 51.730 | 121.017 | 40.845 | 1.00 | 0.00 | AAAA |
| ATOM | 1170 | OD1 | ASP | E | 74 | 52.499 | 121.703 | 40.149 | 1.00 | 0.00 | AAAA |
| ATOM | 1171 | OD2 | ASP | E | 74 | 51.238 | 121.391 | 41.938 | 1.00 | 0.00 | AAAA |
| ATOM | 1172 | C | ASP | E | 74 | 50.178 | 118.340 | 38.493 | 1.00 | 0.00 | AAAA |
| ATOM | 1173 | O | ASP | E | 74 | 50.972 | 117.808 | 37.707 | 1.00 | 0.00 | AAAA |
| ATOM | 1174 | N | LEU | E | 75 | 49.067 | 117.687 | 38.833 | 1.00 | 0.00 | AAAA |
| ATOM | 1176 | CA | LEU | E | 75 | 48.633 | 116.383 | 38.352 | 1.00 | 0.00 | AAAA |
| ATOM | 1178 | CB | LEU | E | 75 | 47.406 | 116.120 | 39.226 | 1.00 | 0.00 | AAAA |
| ATOM | 1181 | CG | LEU | E | 75 | 46.724 | 114.756 | 39.256 | 1.00 | 0.00 | AAAA |
| ATOM | 1183 | CD1 | LEU | E | 75 | 47.734 | 113.684 | 39.660 | 1.00 | 0.00 | AAAA |
| ATOM | 1187 | CD2 | LEU | E | 75 | 45.544 | 114.762 | 40.234 | 1.00 | 0.00 | AAAA |
| ATOM | 1191 | C | LEU | E | 75 | 48.333 | 116.470 | 36.865 | 1.00 | 0.00 | AAAA |
| ATOM | 1192 | O | LEU | E | 75 | 48.904 | 115.708 | 36.087 | 1.00 | 0.00 | AAAA |
| ATOM | 1193 | N | PHE | E | 76 | 47.455 | 117.384 | 36.427 | 1.00 | 0.00 | AAAA |
| ATOM | 1195 | CA | PHE | E | 76 | 47.002 | 117.459 | 35.059 | 1.00 | 0.00 | AAAA |
| ATOM | 1197 | CB | PHE | E | 76 | 46.203 | 116.244 | 34.586 | 1.00 | 0.00 | AAAA |
| ATOM | 1200 | CG | PHE | E | 76 | 45.107 | 115.741 | 35.497 | 1.00 | 0.00 | AAAA |
| ATOM | 1201 | CD1 | PHE | E | 76 | 44.878 | 114.370 | 35.557 | 1.00 | 0.00 | AAAA |
| ATOM | 1203 | CD2 | PHE | E | 76 | 44.388 | 116.574 | 36.361 | 1.00 | 0.00 | AAAA |
| ATOM | 1205 | CE1 | PHE | E | 76 | 44.004 | 113.726 | 36.440 | 1.00 | 0.00 | AAAA |
| ATOM | 1207 | CE2 | PHE | E | 76 | 43.660 | 115.942 | 37.365 | 1.00 | 0.00 | AAAA |
| ATOM | 1209 | CZ | PHE | E | 76 | 43.464 | 114.557 | 37.442 | 1.00 | 0.00 | AAAA |
| ATOM | 1211 | C | PHE | E | 76 | 46.353 | 118.774 | 34.649 | 1.00 | 0.00 | AAAA |
| ATOM | 1212 | O | PHE | E | 76 | 45.249 | 118.703 | 34.119 | 1.00 | 0.00 | AAAA |
| ATOM | 1213 | N | PRO | E | 77 | 46.999 | 119.944 | 34.622 | 1.00 | 0.00 | AAAA |
| ATOM | 1214 | CA | PRO | E | 77 | 46.276 | 121.184 | 34.404 | 1.00 | 0.00 | AAAA |
| ATOM | 1216 | CB | PRO | E | 77 | 47.319 | 122.207 | 34.856 | 1.00 | 0.00 | AAAA |
| ATOM | 1219 | CG | PRO | E | 77 | 48.717 | 121.593 | 34.689 | 1.00 | 0.00 | AAAA |
| ATOM | 1222 | CD | PRO | E | 77 | 48.434 | 120.094 | 34.726 | 1.00 | 0.00 | AAAA |
| ATOM | 1225 | C | PRO | E | 77 | 45.957 | 121.421 | 32.935 | 1.00 | 0.00 | AAAA |
| ATOM | 1226 | O | PRO | E | 77 | 45.284 | 122.405 | 32.623 | 1.00 | 0.00 | AAAA |
| ATOM | 1227 | N | ASN | E | 78 | 46.334 | 120.543 | 31.996 | 1.00 | 0.00 | AAAA |
| ATOM | 1229 | CA | ASN | E | 78 | 46.082 | 120.620 | 30.571 | 1.00 | 0.00 | AAAA |
| ATOM | 1231 | CB | ASN | E | 78 | 47.266 | 119.893 | 29.942 | 1.00 | 0.00 | AAAA |
| ATOM | 1234 | CG | ASN | E | 78 | 48.570 | 120.634 | 30.177 | 1.00 | 0.00 | AAAA |
| ATOM | 1235 | OD1 | ASN | E | 78 | 49.249 | 120.316 | 31.148 | 1.00 | 0.00 | AAAA |
| ATOM | 1236 | ND2 | ASN | E | 78 | 48.983 | 121.605 | 29.354 | 1.00 | 0.00 | AAAA |
| ATOM | 1239 | C | ASN | E | 78 | 44.763 | 120.034 | 30.101 | 1.00 | 0.00 | AAAA |
| ATOM | 1240 | O | ASN | E | 78 | 44.468 | 120.047 | 28.911 | 1.00 | 0.00 | AAAA |
| ATOM | 1241 | N | LEU | E | 79 | 43.927 | 119.495 | 30.996 | 1.00 | 0.00 | AAAA |
| ATOM | 1243 | CA | LEU | E | 79 | 42.520 | 119.198 | 30.811 | 1.00 | 0.00 | AAAA |
| ATOM | 1245 | CB | LEU | E | 79 | 41.914 | 118.694 | 32.121 | 1.00 | 0.00 | AAAA |
| ATOM | 1248 | CG | LEU | E | 79 | 42.298 | 117.262 | 32.481 | 1.00 | 0.00 | AAAA |
| ATOM | 1250 | CD1 | LEU | E | 79 | 41.769 | 116.799 | 33.840 | 1.00 | 0.00 | AAAA |
| ATOM | 1254 | CD2 | LEU | E | 79 | 42.074 | 116.146 | 31.458 | 1.00 | 0.00 | AAAA |
| ATOM | 1258 | C | LEU | E | 79 | 41.711 | 120.336 | 30.194 | 1.00 | 0.00 | AAAA |
| ATOM | 1259 | O | LEU | E | 79 | 41.337 | 121.234 | 30.939 | 1.00 | 0.00 | AAAA |
| ATOM | 1260 | N | THR | E | 80 | 41.445 | 120.267 | 28.883 | 1.00 | 0.00 | AAAA |
| ATOM | 1262 | CA | THR | E | 80 | 40.655 | 121.223 | 28.130 | 1.00 | 0.00 | AAAA |
| ATOM | 1264 | CB | THR | E | 80 | 41.405 | 121.264 | 26.792 | 1.00 | 0.00 | AAAA |
| ATOM | 1266 | OG1 | THR | E | 80 | 42.735 | 121.591 | 27.100 | 1.00 | 0.00 | AAAA |
| ATOM | 1268 | CG2 | THR | E | 80 | 40.957 | 122.238 | 25.704 | 1.00 | 0.00 | AAAA |
| ATOM | 1272 | C | THR | E | 80 | 39.175 | 120.915 | 27.982 | 1.00 | 0.00 | AAAA |
| ATOM | 1273 | O | THR | E | 80 | 38.395 | 121.871 | 27.919 | 1.00 | 0.00 | AAAA |
| ATOM | 1274 | N | VAL | E | 81 | 38.776 | 119.672 | 27.714 | 1.00 | 0.00 | AAAA |
| ATOM | 1276 | CA | VAL | E | 81 | 37.462 | 119.143 | 27.417 | 1.00 | 0.00 | AAAA |
| ATOM | 1278 | CB | VAL | E | 81 | 37.491 | 118.485 | 26.035 | 1.00 | 0.00 | AAAA |
| ATOM | 1280 | CG1 | VAL | E | 81 | 36.136 | 117.906 | 25.615 | 1.00 | 0.00 | AAAA |
| ATOM | 1284 | CG2 | VAL | E | 81 | 37.997 | 119.367 | 24.896 | 1.00 | 0.00 | AAAA |
| ATOM | 1288 | C | VAL | E | 81 | 37.186 | 118.071 | 28.469 | 1.00 | 0.00 | AAAA |
| ATOM | 1289 | O | VAL | E | 81 | 37.927 | 117.091 | 28.542 | 1.00 | 0.00 | AAAA |
| ATOM | 1290 | N | ILE | E | 82 | 36.123 | 118.158 | 29.269 | 1.00 | 0.00 | AAAA |
| ATOM | 1292 | CA | ILE | E | 82 | 35.517 | 117.116 | 30.077 | 1.00 | 0.00 | AAAA |
| ATOM | 1294 | CB | ILE | E | 82 | 35.738 | 117.339 | 31.576 | 1.00 | 0.00 | AAAA |
| ATOM | 1296 | CG1 | ILE | E | 82 | 37.219 | 117.346 | 31.957 | 1.00 | 0.00 | AAAA |
| ATOM | 1299 | CG2 | ILE | E | 82 | 34.904 | 116.347 | 32.397 | 1.00 | 0.00 | AAAA |
| ATOM | 1303 | CD1 | ILE | E | 82 | 37.544 | 117.512 | 33.441 | 1.00 | 0.00 | AAAA |
| ATOM | 1307 | C | ILE | E | 82 | 34.108 | 117.099 | 29.504 | 1.00 | 0.00 | AAAA |
| ATOM | 1308 | O | ILE | E | 82 | 33.243 | 117.783 | 30.045 | 1.00 | 0.00 | AAAA |
| ATOM | 1309 | N | ARG | E | 83 | 33.874 | 116.488 | 28.336 | 1.00 | 0.00 | AAAA |
| ATOM | 1311 | CA | ARG | E | 83 | 32.794 | 116.723 | 27.413 | 1.00 | 0.00 | AAAA |
| ATOM | 1313 | CB | ARG | E | 83 | 32.976 | 115.985 | 26.088 | 1.00 | 0.00 | AAAA |
| ATOM | 1316 | CG | ARG | E | 83 | 31.860 | 116.222 | 25.075 | 1.00 | 0.00 | AAAA |
| ATOM | 1319 | CD | ARG | E | 83 | 32.032 | 115.450 | 23.763 | 1.00 | 0.00 | AAAA |
| ATOM | 1322 | NE | ARG | E | 83 | 32.819 | 116.295 | 22.864 | 1.00 | 0.00 | AAAA |
| ATOM | 1324 | CZ | ARG | E | 83 | 33.354 | 115.921 | 21.691 | 1.00 | 0.00 | AAAA |
| ATOM | 1325 | NH1 | ARG | E | 83 | 33.315 | 114.652 | 21.268 | 1.00 | 0.00 | AAAA |
| ATOM | 1328 | NH2 | ARG | E | 83 | 33.883 | 116.909 | 20.961 | 1.00 | 0.00 | AAAA |
| ATOM | 1331 | C | ARG | E | 83 | 31.492 | 116.405 | 28.133 | 1.00 | 0.00 | AAAA |
| ATOM | 1332 | O | ARG | E | 83 | 30.616 | 117.243 | 28.361 | 1.00 | 0.00 | AAAA |
| ATOM | 1333 | N | GLY | E | 84 | 31.294 | 115.169 | 28.609 | 1.00 | 0.00 | AAAA |
| ATOM | 1335 | CA | GLY | E | 84 | 30.187 | 114.673 | 29.386 | 1.00 | 0.00 | AAAA |
| ATOM | 1338 | C | GLY | E | 84 | 29.212 | 113.914 | 28.493 | 1.00 | 0.00 | AAAA |
| ATOM | 1339 | O | GLY | E | 84 | 28.014 | 114.147 | 28.592 | 1.00 | 0.00 | AAAA |
| ATOM | 1340 | N | SER | E | 85 | 29.786 | 113.092 | 27.616 | 1.00 | 0.00 | AAAA |
| ATOM | 1342 | CA | SER | E | 85 | 29.078 | 112.208 | 26.711 | 1.00 | 0.00 | AAAA |
| ATOM | 1344 | CB | SER | E | 85 | 30.043 | 111.733 | 25.625 | 1.00 | 0.00 | AAAA |
| ATOM | 1347 | OG | SER | E | 85 | 30.399 | 112.826 | 24.806 | 1.00 | 0.00 | AAAA |
| ATOM | 1349 | C | SER | E | 85 | 28.405 | 111.038 | 27.430 | 1.00 | 0.00 | AAAA |
| ATOM | 1350 | O | SER | E | 85 | 27.493 | 110.411 | 26.890 | 1.00 | 0.00 | AAAA |
| ATOM | 1351 | N | ARG | E | 86 | 28.722 | 110.875 | 28.710 | 1.00 | 0.00 | AAAA |
| ATOM | 1353 | CA | ARG | E | 86 | 28.037 | 110.100 | 29.722 | 1.00 | 0.00 | AAAA |
| ATOM | 1355 | CB | ARG | E | 86 | 28.440 | 108.634 | 29.951 | 1.00 | 0.00 | AAAA |
| ATOM | 1358 | CG | ARG | E | 86 | 28.336 | 107.708 | 28.740 | 1.00 | 0.00 | AAAA |
| ATOM | 1361 | CD | ARG | E | 86 | 28.648 | 106.269 | 29.162 | 1.00 | 0.00 | AAAA |
| ATOM | 1364 | NE | ARG | E | 86 | 28.726 | 105.347 | 28.022 | 1.00 | 0.00 | AAAA |
| ATOM | 1366 | CZ | ARG | E | 86 | 29.802 | 105.093 | 27.269 | 1.00 | 0.00 | AAAA |
| ATOM | 1367 | NH1 | ARG | E | 86 | 30.971 | 105.702 | 27.538 | 1.00 | 0.00 | AAAA |
| ATOM | 1370 | NH2 | ARG | E | 86 | 29.621 | 104.198 | 26.296 | 1.00 | 0.00 | AAAA |
| ATOM | 1373 | C | ARG | E | 86 | 28.293 | 110.905 | 30.983 | 1.00 | 0.00 | AAAA |
| ATOM | 1374 | O | ARG | E | 86 | 29.357 | 111.519 | 31.065 | 1.00 | 0.00 | AAAA |
| ATOM | 1375 | N | LEU | E | 87 | 27.342 | 110.999 | 31.919 | 1.00 | 0.00 | AAAA |
| ATOM | 1377 | CA | LEU | E | 87 | 27.446 | 111.905 | 33.046 | 1.00 | 0.00 | AAAA |
| ATOM | 1379 | CB | LEU | E | 87 | 26.327 | 112.945 | 32.982 | 1.00 | 0.00 | AAAA |
| ATOM | 1382 | CG | LEU | E | 87 | 26.550 | 114.024 | 31.922 | 1.00 | 0.00 | AAAA |
| ATOM | 1384 | CD1 | LEU | E | 87 | 25.319 | 114.917 | 31.997 | 1.00 | 0.00 | AAAA |
| ATOM | 1388 | CD2 | LEU | E | 87 | 27.835 | 114.832 | 32.131 | 1.00 | 0.00 | AAAA |
| ATOM | 1392 | C | LEU | E | 87 | 27.310 | 111.106 | 34.327 | 1.00 | 0.00 | AAAA |
| ATOM | 1393 | O | LEU | E | 87 | 26.808 | 109.983 | 34.349 | 1.00 | 0.00 | AAAA |
| ATOM | 1394 | N | PHE | E | 88 | 27.769 | 111.655 | 35.455 | 1.00 | 0.00 | AAAA |
| ATOM | 1396 | CA | PHE | E | 88 | 27.831 | 111.098 | 36.785 | 1.00 | 0.00 | AAAA |
| ATOM | 1398 | CB | PHE | E | 88 | 29.219 | 111.161 | 37.410 | 1.00 | 0.00 | AAAA |
| ATOM | 1401 | CG | PHE | E | 88 | 29.423 | 110.497 | 38.751 | 1.00 | 0.00 | AAAA |
| ATOM | 1402 | CD1 | PHE | E | 88 | 29.255 | 111.214 | 39.943 | 1.00 | 0.00 | AAAA |
| ATOM | 1404 | CD2 | PHE | E | 88 | 29.872 | 109.171 | 38.778 | 1.00 | 0.00 | AAAA |
| ATOM | 1406 | CE1 | PHE | E | 88 | 29.587 | 110.639 | 41.184 | 1.00 | 0.00 | AAAA |
| ATOM | 1408 | CE2 | PHE | E | 88 | 30.132 | 108.555 | 40.007 | 1.00 | 0.00 | AAAA |
| ATOM | 1410 | CZ | PHE | E | 88 | 30.002 | 109.310 | 41.179 | 1.00 | 0.00 | AAAA |
| ATOM | 1412 | C | PHE | E | 88 | 26.788 | 111.852 | 37.604 | 1.00 | 0.00 | AAAA |
| ATOM | 1413 | O | PHE | E | 88 | 27.132 | 112.745 | 38.380 | 1.00 | 0.00 | AAAA |
| ATOM | 1414 | N | PHE | E | 89 | 25.497 | 111.651 | 37.345 | 1.00 | 0.00 | AAAA |
| ATOM | 1416 | CA | PHE | E | 89 | 24.276 | 112.148 | 37.950 | 1.00 | 0.00 | AAAA |
| ATOM | 1418 | CB | PHE | E | 89 | 24.266 | 111.816 | 39.438 | 1.00 | 0.00 | AAAA |
| ATOM | 1421 | CG | PHE | E | 89 | 24.403 | 110.330 | 39.696 | 1.00 | 0.00 | AAAA |
| ATOM | 1422 | CD1 | PHE | E | 89 | 23.421 | 109.408 | 39.325 | 1.00 | 0.00 | AAAA |
| ATOM | 1424 | CD2 | PHE | E | 89 | 25.668 | 109.812 | 40.019 | 1.00 | 0.00 | AAAA |
| ATOM | 1426 | CE1 | PHE | E | 89 | 23.615 | 108.041 | 39.525 | 1.00 | 0.00 | AAAA |
| ATOM | 1428 | CE2 | PHE | E | 89 | 25.902 | 108.433 | 40.067 | 1.00 | 0.00 | AAAA |
| ATOM | 1430 | CZ | PHE | E | 89 | 24.892 | 107.514 | 39.750 | 1.00 | 0.00 | AAAA |
| ATOM | 1432 | C | PHE | E | 89 | 24.050 | 113.630 | 37.675 | 1.00 | 0.00 | AAAA |
| ATOM | 1433 | O | PHE | E | 89 | 23.883 | 114.462 | 38.567 | 1.00 | 0.00 | AAAA |
| ATOM | 1434 | N | ASN | E | 90 | 24.260 | 113.916 | 36.389 | 1.00 | 0.00 | AAAA |
| ATOM | 1436 | CA | ASN | E | 90 | 24.333 | 115.201 | 35.708 | 1.00 | 0.00 | AAAA |
| ATOM | 1438 | CB | ASN | E | 90 | 23.472 | 116.346 | 36.247 | 1.00 | 0.00 | AAAA |
| ATOM | 1441 | CG | ASN | E | 90 | 22.951 | 117.308 | 35.196 | 1.00 | 0.00 | AAAA |
| ATOM | 1442 | OD1 | ASN | E | 90 | 21.970 | 116.914 | 34.585 | 1.00 | 0.00 | AAAA |
| ATOM | 1443 | ND2 | ASN | E | 90 | 23.622 | 118.467 | 35.067 | 1.00 | 0.00 | AAAA |
| ATOM | 1446 | C | ASN | E | 90 | 25.771 | 115.700 | 35.633 | 1.00 | 0.00 | AAAA |
| ATOM | 1447 | O | ASN | E | 90 | 26.023 | 116.677 | 34.915 | 1.00 | 0.00 | AAAA |
| ATOM | 1448 | N | TYR | E | 91 | 26.676 | 115.240 | 36.498 | 1.00 | 0.00 | AAAA |
| ATOM | 1450 | CA | TYR | E | 91 | 27.866 | 116.038 | 36.720 | 1.00 | 0.00 | AAAA |
| ATOM | 1452 | CB | TYR | E | 91 | 28.301 | 115.819 | 38.165 | 1.00 | 0.00 | AAAA |
| ATOM | 1455 | CG | TYR | E | 91 | 27.285 | 116.372 | 39.138 | 1.00 | 0.00 | AAAA |
| ATOM | 1456 | CD1 | TYR | E | 91 | 26.881 | 117.710 | 39.058 | 1.00 | 0.00 | AAAA |
| ATOM | 1458 | CD2 | TYR | E | 91 | 26.653 | 115.539 | 40.071 | 1.00 | 0.00 | AAAA |
| ATOM | 1460 | CE1 | TYR | E | 91 | 25.824 | 118.114 | 39.874 | 1.00 | 0.00 | AAAA |
| ATOM | 1462 | CE2 | TYR | E | 91 | 25.618 | 115.972 | 40.914 | 1.00 | 0.00 | AAAA |
| ATOM | 1464 | CZ | TYR | E | 91 | 25.201 | 117.313 | 40.839 | 1.00 | 0.00 | AAAA |
| ATOM | 1465 | OH | TYR | E | 91 | 24.141 | 117.788 | 41.557 | 1.00 | 0.00 | AAAA |
| ATOM | 1467 | C | TYR | E | 91 | 29.013 | 115.490 | 35.870 | 1.00 | 0.00 | AAAA |
| ATOM | 1468 | O | TYR | E | 91 | 29.069 | 114.280 | 35.682 | 1.00 | 0.00 | AAAA |
| ATOM | 1469 | N | ALA | E | 92 | 29.843 | 116.361 | 35.300 | 1.00 | 0:00 | AAAA |
| ATOM | 1471 | CA | ALA | E | 92 | 31.076 | 115.971 | 34.645 | 1.00 | 0.00 | AAAA |
| ATOM | 1473 | CB | ALA | E | 92 | 31.318 | 117.052 | 33.596 | 1.00 | 0.00 | AAAA |
| ATOM | 1477 | C | ALA | E | 92 | 32.256 | 116.074 | 35.605 | 1.00 | 0.00 | AAAA |
| ATOM | 1478 | O | ALA | E | 92 | 33.260 | 115.396 | 35.447 | 1.00 | 0.00 | AAAA |
| ATOM | 1479 | N | LEU | E | 93 | 32.069 | 116.888 | 36.658 | 1.00 | 0.00 | AAAA |
| ATOM | 1481 | CA | LEU | E | 93 | 33.068 | 117.273 | 37.639 | 1.00 | 0.00 | AAAA |
| ATOM | 1483 | CB | LEU | E | 93 | 33.963 | 118.427 | 37.220 | 1.00 | 0.00 | AAAA |
| ATOM | 1486 | CG | LEU | E | 93 | 35.185 | 118.812 | 38.052 | 1.00 | 0.00 | AAAA |
| ATOM | 1488 | CD1 | LEU | E | 93 | 36.007 | 117.616 | 38.553 | 1.00 | 0.00 | AAAA |
| ATOM | 1492 | CD2 | LEU | E | 93 | 36.025 | 119.878 | 37.348 | 1.00 | 0.00 | AAAA |
| ATOM | 1496 | C | LEU | E | 93 | 32.477 | 117.288 | 39.041 | 1.00 | 0.00 | AAAA |
| ATOM | 1497 | O | LEU | E | 93 | 32.150 | 118.378 | 39.510 | 1.00 | 0.00 | AAAA |
| ATOM | 1498 | N | VAL | E | 94 | 32.545 | 116.174 | 39.776 | 1.00 | 0.00 | AAAA |
| ATOM | 1500 | CA | VAL | E | 94 | 32.258 | 116.131 | 41.196 | 1.00 | 0.00 | AAAA |
| ATOM | 1502 | CB | VAL | E | 94 | 31.462 | 114.879 | 41.556 | 1.00 | 0.00 | AAAA |
| ATOM | 1504 | CG1 | VAL | E | 94 | 31.218 | 114.758 | 43.061 | 1.00 | 0.00 | AAAA |
| ATOM | 1508 | CG2 | VAL | E | 94 | 30.135 | 114.721 | 40.829 | 1.00 | 0.00 | AAAA |
| ATOM | 1512 | C | VAL | E | 94 | 33.585 | 116.321 | 41.926 | 1.00 | 0.00 | AAAA |
| ATOM | 1513 | O | VAL | E | 94 | 34.544 | 115.568 | 41.815 | 1.00 | 0.00 | AAAA |
| ATOM | 1514 | N | ILE | E | 95 | 33.684 | 117.448 | 42.646 | 1.00 | 0.00 | AAAA |
| ATOM | 1516 | CA | ILE | E | 95 | 34.745 | 117.707 | 43.591 | 1.00 | 0.00 | AAAA |
| ATOM | 1518 | CB | ILE | E | 95 | 35.430 | 119.045 | 43.304 | 1.00 | 0.00 | AAAA |
| ATOM | 1520 | CG1 | ILE | E | 95 | 35.891 | 119.114 | 41.849 | 1.00 | 0.00 | AAAA |
| ATOM | 1523 | CG2 | ILE | E | 95 | 36.614 | 119.390 | 44.202 | 1.00 | 0.00 | AAAA |
| ATOM | 1527 | CD1 | ILE | E | 95 | 36.387 | 120.481 | 41.375 | 1.00 | 0.00 | AAAA |
| ATOM | 1531 | C | ILE | E | 95 | 34.076 | 117.573 | 44.953 | 1.00 | 0.00 | AAAA |
| ATOM | 1532 | O | ILE | E | 95 | 33.401 | 118.504 | 45.377 | 1.00 | 0.00 | AAAA |
| ATOM | 1533 | N | PHE | E | 96 | 33.977 | 116.361 | 45.512 | 1.00 | 0.00 | AAAA |
| ATOM | 1535 | CA | PHE | E | 96 | 33.315 | 116.185 | 46.781 | 1.00 | 0.00 | AAAA |
| ATOM | 1537 | CB | PHE | E | 96 | 32.559 | 114.856 | 46.796 | 1.00 | 0.00 | AAAA |
| ATOM | 1540 | CG | PHE | E | 96 | 31.816 | 114.390 | 48.031 | 1.00 | 0.00 | AAAA |
| ATOM | 1541 | CD1 | PHE | E | 96 | 30.934 | 115.234 | 48.708 | 1.00 | 0.00 | AAAA |
| ATOM | 1543 | CD2 | PHE | E | 96 | 32.013 | 113.086 | 48.506 | 1.00 | 0.00 | AAAA |
| ATOM | 1545 | CE1 | PHE | E | 96 | 30.398 | 114.811 | 49.929 | 1.00 | 0.00 | AAAA |
| ATOM | 1547 | CE2 | PHE | E | 96 | 31.335 | 112.570 | 49.622 | 1.00 | 0.00 | AAAA |
| ATOM | 1549 | CZ | PHE | E | 96 | 30.569 | 113.483 | 50.363 | 1.00 | 0.00 | AAAA |
| ATOM | 1551 | C | PHE | E | 96 | 34.393 | 115.861 | 47.796 | 1.00 | 0.00 | AAAA |
| ATOM | 1552 | O | PHE | E | 96 | 35.399 | 115.253 | 47.461 | 1.00 | 0.00 | AAAA |
| ATOM | 1553 | N | GLU | E | 97 | 34.351 | 116.368 | 49.037 | 1.00 | 0.00 | AAAA |
| ATOM | 1555 | CA | GLU | E | 97 | 35.099 | 115.935 | 50.198 | 1.00 | 0.00 | AAAA |
| ATOM | 1557 | CB | GLU | E | 97 | 34.860 | 114.483 | 50.601 | 1.00 | 0.00 | AAAA |
| ATOM | 1560 | CG | GLU | E | 97 | 34.919 | 114.151 | 52.095 | 1.00 | 0.00 | AAAA |
| ATOM | 1563 | CD | GLU | E | 97 | 35.042 | 112.647 | 52.319 | 1.00 | 0.00 | AAAA |
| ATOM | 1564 | OE1 | GLU | E | 97 | 34.325 | 112.170 | 53.220 | 1.00 | 0.00 | AAAA |
| ATOM | 1565 | OE2 | GLU | E | 97 | 36.078 | 112.092 | 51.879 | 1.00 | 0.00 | AAAA |
| ATOM | 1566 | C | GLU | E | 97 | 36.576 | 116.278 | 50.104 | 1.00 | 0..00 | AAAA |
| ATOM | 1567 | O | GLU | E | 97 | 37.384 | 115.666 | 50.803 | 1.00 | 0.00 | AAAA |
| ATOM | 1568 | N | MET | E | 98 | 37.079 | 117.085 | 49.164 | 1.00 | 0.00 | AAAA |
| ATOM | 1570 | CA | MET | E | 98 | 38.474 | 117.262 | 48.818 | 1.00 | 0.00 | AAAA |
| ATOM | 1572 | CB | MET | E | 98 | 38.518 | 117.546 | 47.320 | 1.00 | 0.00 | AAAA |
| ATOM | 1575 | CG | MET | E | 98 | 39.745 | 116.892 | 46.690 | 1.00 | 0.00 | AAAA |
| ATOM | 1578 | SD | MET | E | 98 | 39.781 | 116.910 | 44.878 | 1.00 | 0.00 | AAAA |
| ATOM | 1579 | CE | MET | E | 98 | 38.491 | 115.736 | 44.405 | 1.00 | 0.00 | AAAA |
| ATOM | 1583 | C | MET | E | 98 | 39.146 | 118.348 | 49.655 | 1.00 | 0.00 | AAAA |
| ATOM | 1584 | O | MET | E | 98 | 39.552 | 119.379 | 49.125 | 1.00 | 0.00 | AAAA |
| ATOM | 1585 | N | VAL | E | 99 | 39.148 | 118.136 | 50.967 | 1.00 | 0.00 | AAAA |
| ATOM | 1587 | CA | VAL | E | 99 | 39.866 | 118.877 | 51.989 | 1.00 | 0.00 | AAAA |
| ATOM | 1589 | CB | VAL | E | 99 | 39.502 | 118.261 | 53.345 | 1.00 | 0.00 | AAAA |
| ATOM | 1591 | CG1 | VAL | E | 99 | 38.014 | 118.415 | 53.675 | 1.00 | 0.00 | AAAA |
| ATOM | 1595 | CG2 | VAL | E | 99 | 39.801 | 116.766 | 53.358 | 1.00 | 0.00 | AAAA |
| ATOM | 1599 | C | VAL | E | 99 | 41.320 | 119.083 | 51.599 | 1.00 | 0.00 | AAAA |
| ATOM | 1600 | O | VAL | E | 99 | 41.933 | 118.132 | 51.114 | 1.00 | 0.00 | AAAA |
| ATOM | 1601 | N | HIS | E | 100 | 41.822 | 120.321 | 51.625 | 1.00 | 0.00 | AAAA |
| ATOM | 1603 | CA | HIS | E | 100 | 43.163 | 120.804 | 51.398 | 1.00 | 0.00 | AAAA |
| ATOM | 1605 | CB | HIS | E | 100 | 44.242 | 120.050 | 52.180 | 1.00 | 0.00 | AAAA |
| ATOM | 1608 | CG | HIS | E | 100 | 45.581 | 120.727 | 52.067 | 1.00 | 0.00 | AAAA |
| ATOM | 1609 | ND1 | HIS | E | 100 | 45.813 | 121.866 | 52.843 | 1.00 | 0.00 | AAAA |
| ATOM | 1610 | CD2 | HIS | E | 100 | 46.724 | 120.383 | 51.412 | 1.00 | 0.00 | AAAA |
| ATOM | 1612 | CE1 | HIS | E | 100 | 47.073 | 122.209 | 52.542 | 1.00 | 0.00 | AAAA |
| ATOM | 1614 | NE2 | HIS | E | 100 | 47.707 | 121.259 | 51.837 | 1.00 | 0.00 | AAAA |
| ATOM | 1616 | C | HIS | E | 100 | 43.416 | 121.106 | 49.931 | 1.00 | 0.00 | AAAA |
| ATOM | 1617 | O | HIS | E | 100 | 44.542 | 121.201 | 49.455 | 1.00 | 0.00 | AAAA |
| ATOM | 1618 | N | LEU | E | 101 | 42.320 | 121.224 | 49.172 | 1.00 | 0.00 | AAAA |
| ATOM | 1620 | CA | LEU | E | 101 | 42.332 | 121.814 | 47.842 | 1.00 | 0.00 | AAAA |
| ATOM | 1622 | CB | LEU | E | 101 | 41.212 | 121.167 | 47.039 | 1.00 | 0.00 | AAAA |
| ATOM | 1625 | CG | LEU | E | 101 | 41.174 | 121.493 | 45.546 | 1.00 | 0.00 | AAAA |
| ATOM | 1627 | CD1 | LEU | E | 101 | 42.299 | 120.784 | 44.792 | 1.00 | 0.00 | AAAA |
| ATOM | 1631 | CD2 | LEU | E | 101 | 39.903 | 120.955 | 44.889 | 1.00 | 0.00 | AAAA |
| ATOM | 1635 | C | LEU | E | 101 | 41.948 | 123.244 | 48.189 | 1.00 | 0:00 | AAAA |
| ATOM | 1636 | O | LEU | E | 101 | 40.988 | 123.415 | 48.930 | 1.00 | 0.00 | AAAA |
| ATOM | 1637 | N | LYS | E | 102 | 42.623 | 124.259 | 47.639 | 1.00 | 0.00 | AAAA |
| ATOM | 1639 | CA | LYS | E | 102 | 42.345 | 125.653 | 47.893 | 1.00 | 0.00 | AAAA |
| ATOM | 1641 | CB | LYS | E | 102 | 43.237 | 126.108 | 49.041 | 1.00 | 0.00 | AAAA |
| ATOM | 1644 | CG | LYS | E | 102 | 44.754 | 125.916 | 48.898 | 1.00 | 0.00 | AAAA |
| ATOM | 1647 | CD | LYS | E | 102 | 45.191 | 124.541 | 49.388 | 1.00 | 0.00 | AAAA |
| ATOM | 1650 | CE | LYS | E | 102 | 46.704 | 124.481 | 49.199 | 1.00 | 0.00 | AAAA |
| ATOM | 1653 | NZ | LYS | E | 102 | 47.474 | 125.531 | 49.869 | 1.00 | 0.00 | AAAA |
| ATOM | 1657 | C | LYS | E | 102 | 42.357 | 126.423 | 46.572 | 1.00 | 0.00 | AAAA |
| ATOM | 1658 | O | LYS | E | 102 | 42.325 | 127.646 | 46.612 | 1.00 | 0.00 | AAAA |
| ATOM | 1659 | N | GLU | E | 103 | 42.534 | 125.755 | 45.426 | 1.00 | 0.00 | AAAA |
| ATOM | 1661 | CA | GLU | E | 103 | 42.496 | 126.297 | 44.084 | 1.00 | 0.00 | AAAA |
| ATOM | 1663 | CB | GLU | E | 103 | 43.833 | 126.928 | 43.711 | 1.00 | 0.00 | AAAA |
| ATOM | 1666 | CG | GLU | E | 103 | 44.032 | 127.505 | 42.313 | 1.00 | 0.00 | AAAA |
| ATOM | 1669 | CD | GLU | E | 103 | 45.490 | 127.773 | 41.968 | 1.00 | 0.00 | AAAA |
| ATOM | 1670 | OE1 | GLU | E | 103 | 45.791 | 128.255 | 40.850 | 1.00 | 0.00 | AAAA |
| ATOM | 1671 | OE2 | GLU | E | 103 | 46.359 | 127.498 | 42.822 | 1.00 | 0.00 | AAAA |
| ATOM | 1672 | C | GLU | E | 103 | 41.878 | 125.221 | 43.198 | 1.00 | 0.00 | AAAA |
| ATOM | 1673 | O | GLU | E | 103 | 42.090 | 124.035 | 43.464 | 1.00 | 0.00 | AAAA |
| ATOM | 1674 | N | LEU | E | 104 | 41.148 | 125.675 | 42.178 | 1.00 | 0.00 | AAAA |
| ATOM | 1676 | CA | LEU | E | 104 | 40.434 | 124.803 | 41.271 | 1.00 | 0.00 | AAAA |
| ATOM | 1678 | CB | LEU | E | 104 | 39.278 | 125.429 | 40.489 | 1.00 | 0.00 | AAAA |
| ATOM | 1681 | CG | LEU | E | 104 | 38.035 | 125.803 | 41.287 | 1.00 | 0.00 | AAAA |
| ATOM | 1683 | CD1 | LEU | E | 104 | 37.037 | 126.528 | 40.375 | 1.00 | 0.00 | AAAA |
| ATOM | 1687 | CD2 | LEU | E | 104 | 37.374 | 124.729 | 42.152 | 1.00 | 0.00 | AAAA |
| ATOM | 1691 | C | LEU | E | 104 | 41.387 | 124.170 | 40.264 | 1.00 | 0.00 | AAAA |
| ATOM | 1692 | O | LEU | E | 104 | 41.342 | 122.967 | 39.996 | 1.00 | 0.00 | AAAA |
| ATOM | 1693 | N | GLY | E | 105 | 42.347 | 124.892 | 39.671 | 1.00 | 0.00 | AAAA |
| ATOM | 1695 | CA | GLY | E | 105 | 43.559 | 124.221 | 39.239 | 1.00 | 0.00 | AAAA |
| ATOM | 1698 | C | GLY | E | 105 | 43.671 | 123.971 | 37.731 | 1.00 | 0.00 | AAAA |
| ATOM | 1699 | O | GLY | E | 105 | 44.587 | 124.402 | 37.033 | 1.00 | 0.00 | AAAA |
| ATOM | 1700 | N | LEU | E | 106 | 42.601 | 123.427 | 37.167 | 1.00 | 0.00 | AAAA |
| ATOM | 1702 | CA | LEU | E | 106 | 42.442 | 122.946 | 35.801 | 1.00 | 0.00 | AAAA |
| ATOM | 1704 | CB | LEU | E | 106 | 41.280 | 121.953 | 35.713 | 1.00 | 0.00 | AAAA |
| ATOM | 1707 | CG | LEU | E | 106 | 41.448 | 120.774 | 36.662 | 1.00 | 0.00 | AAAA |
| ATOM | 1709 | CD1 | LEU | E | 106 | 40.339 | 119.736 | 36.483 | 1.00 | 0.00 | AAAA |
| ATOM | 1713 | CD2 | LEU | E | 106 | 42.736 | 119.948 | 36.557 | 1.00 | 0.00 | AAAA |
| ATOM | 1717 | C | LEU | E | 106 | 42.123 | 124.135 | 34.894 | 1.00 | 0.00 | AAAA |
| ATOM | 1718 | O | LEU | E | 106 | 41.083 | 124.320 | 34.267 | 1.00 | 0.00 | AAAA |
| ATOM | 1719 | N | TYR | E | 107 | 43.073 | 125.080 | 34.866 | 1.00 | 0.00 | AAAA |
| ATOM | 1721 | CA | TYR | E | 107 | 43.065 | 126.375 | 34.232 | 1.00 | 0.00 | AAAA |
| ATOM | 1723 | CB | TYR | E | 107 | 44.207 | 127.304 | 34.650 | 1.00 | 0.00 | AAAA |
| ATOM | 1726 | CG | TYR | E | 107 | 45.606 | 126.806 | 34.415 | 1.00 | 0.00 | AAAA |
| ATOM | 1727 | CD1 | TYR | E | 107 | 46.145 | 126.807 | 33.124 | 1.00 | 0.00 | AAAA |
| ATOM | 1729 | CD2 | TYR | E | 107 | 46.409 | 126.450 | 35.512 | 1.00 | 0.00 | AAAA |
| ATOM | 1731 | CE1 | TYR | E | 107 | 47.483 | 126.457 | 32.918 | 1.00 | 0.00 | AAAA |
| ATOM | 1733 | CE2 | TYR | E | 107 | 47.756 | 126.114 | 35.323 | 1.00 | 0.00 | AAAA |
| ATOM | 1735 | CZ | TYR | E | 107 | 48.267 | 126.117 | 34.017 | 1.00 | 0.00 | AAAA |
| ATOM | 1736 | OH | TYR | E | 107 | 49.545 | 125.714 | 33.793 | 1.00 | 0.00 | AAAA |
| ATOM | 1738 | C | TYR | E | 107 | 42.887 | 126.434 | 32.720 | 1.00 | 0.00 | AAAA |
| ATOM | 1739 | O | TYR | E | 107 | 42.497 | 127.494 | 32.232 | 1.00 | 0.00 | AAAA |
| ATOM | 1740 | N | ASN | E | 108 | 43.084 | 125.271 | 32.097 | 1.00 | 0.00 | AAAA |
| ATOM | 1742 | CA | ASN | E | 108 | 42.849 | 125.161 | 30.672 | 1.00 | 0.00 | AAAA |
| ATOM | 1744 | | ASN | E | 108 | 43.812 | 124.145 | 30.068 | 1.00 | 0.00 | AAAA |
| ATOM | 1747 | CG | ASN | E | 108 | 45.245 | 124.663 | 29.969 | 1.00 | 0.00 | AAAA |
| ATOM | 1748 | OD1 | ASN | E | 108 | 45.506 | 125.400 | 29.020 | 1.00 | 0.00 | AAAA |
| ATOM | 1749 | ND2 | ASN | E | 108 | 46.194 | 124.224 | 30.792 | 1.00 | 0.00 | AAAA |
| ATOM | 1752 | C | ASN | E | 108 | 41.478 | 124.671 | 30.218 | 1.00 | 0.00 | AAAA |
| ATOM | 1753 | O | ASN | E | 108 | 41.148 | 124.725 | 29.035 | 1.00 | 0.00 | AAAA |
| ATOM | 1754 | N | LEU | E | 109 | 40.686 | 124.208 | 31.181 | 1.00 | 0.00 | AAAA |
| ATOM | 1756 | CA | LEU | E | 109 | 39.358 | 123.632 | 31.033 | 1.00 | 0.00 | AAAA |
| ATOM | 1758 | CB | LEU | E | 109 | 38.926 | 122.936 | 32.317 | 1.00 | 0.00 | AAAA |
| ATOM | 1761 | CG | LEU | E | 109 | 37.557 | 122.249 | 32.341 | 1.00 | 0.00 | AAAA |
| ATOM | 1763 | CD1 | LEU | E | 109 | 37.447 | 121.014 | 31.440 | 1.00 | 0.00 | AAAA |
| ATOM | 1767 | CD2 | LEU | E | 109 | 37.259 | 121.901 | 33.789 | 1.00 | 0.00 | AAAA |
| ATOM | 1771 | C | LEU | E | 109 | 38.351 | 124.661 | 30.545 | 1.00 | 0.00 | AAAA |
| ATOM | 1772 | O | LEU | E | 109 | 38.143 | 125.624 | 31.278 | 1.00 | 0.00 | AAAA |
| ATOM | 1773 | N | MET | E | 110 | 37.716 | 124.377 | 29.414 | 1.00 | 0.00 | AAAA |
| ATOM | 1775 | CA | MET | E | 110 | 36.846 | 125.315 | 28.729 | 1.00 | 0.00 | AAAA |
| ATOM | 1777 | CB | MET | E | 110 | 37.602 | 126.194 | 27.734 | 1.00 | 0.00 | AAAA |
| ATOM | 1780 | CG | MET | E | 110 | 37.858 | 125.554 | 26.370 | 1.00 | 0.00 | AAAA |
| ATOM | 1783 | SD | MET | E | 110 | 38.941 | 126.386 | 25.176 | 1.00 | 0.00 | AAAA |
| ATOM | 1784 | CE | MET | E | 110 | 39.148 | 125.079 | 23.939 | 1.00 | 0.00 | AAAA |
| ATOM | 1788 | C | MET | E | 110 | 35.598 | 124.666 | 28.142 | 1.00 | 0.00 | AAAA |
| ATOM | 1789 | O | MET | E | 110 | 34.735 | 125.299 | 27.542 | 1.00 | 0.00 | AAAA |
| ATOM | 1790 | N | ASN | E | 111 | 35.418 | 123.350 | 28.224 | 1.00 | 0.00 | AAAA |
| ATOM | 1792 | CA | ASN | E | 111 | 34.291 | 122.555 | 27.772 | 1.00 | 0.00 | AAAA |
| ATOM | 1794 | CB | ASN | E | 111 | 34.455 | 122.081 | 26.327 | 1.00 | 0.00 | AAAA |
| ATOM | 1797 | CG | ASN | E | 111 | 33.208 | 121.437 | 25.719 | 1.00 | 0.00 | AAAA |
| ATOM | 1798 | OD1 | ASN | E | 111 | 32.709 | 120.383 | 26.111 | 1.00 | 0.00 | AAAA |
| ATOM | 1799 | ND2 | ASN | E | 111 | 32.538 | 122.169 | 24.835 | 1.00 | 0.00 | AAAA |
| ATOM | 1802 | C | ASN | E | 111 | 33.919 | 121.489 | 28.782 | 1.00 | 0.00 | AAAA |
| ATOM | 1803 | O | ASN | E | 111 | 34.657 | 120.571 | 29.138 | 1.00 | 0.00 | AAAA |
| ATOM | 1804 | N | ILE | E | 112 | 32.643 | 121.684 | 29.134 | 1.00 | 0.00 | AAAA |
| ATOM | 1806 | CA | ILE | E | 112 | 31.625 | 120.806 | 29.688 | 1.00 | 0.00 | AAAA |
| ATOM | 1808 | CB | ILE | E | 112 | 31.465 | 120.949 | 31.201 | 1.00 | 0.00 | AAAA |
| ATOM | 1810 | CG1 | ILE | E | 112 | 32.706 | 120.574 | 32.004 | 1.00 | 0.00 | AAAA |
| ATOM | 1813 | CG2 | ILE | E | 112 | 30.285 | 120.157 | 31.764 | 1.00 | 0.00 | AAAA |
| ATOM | 1817 | CD1 | ILE | E | 112 | 32.772 | 121.057 | 33.455 | 1.00 | 0.00 | AAAA |
| ATOM | 1821 | C | ILE | E | 112 | 30.415 | 121.142 | 28.841 | 1.00 | 0.00 | AAAA |
| ATOM | 1822 | O | ILE | E | 112 | 30.078 | 122.319 | 28.734 | 1.00 | 0.00 | AAAA |
| ATOM | 1823 | N | THR | E | 113 | 29.712 | 120.152 | 28.275 | 1.00 | 0.00 | AAAA |
| ATOM | 1825 | CA | THR | E | 113 | 28.554 | 120.292 | 27.424 | 1.00 | 0.00 | AAAA |
| ATOM | 1827 | CB | THR | E | 113 | 29.009 | 120.342 | 25.964 | 1.00 | 0.00 | AAAA |
| ATOM | 1829 | OG1 | THR | E | 113 | 27.873 | 120.578 | 25.172 | 1.00 | 0.00 | AAAA |
| ATOM | 1831 | CG2 | THR | E | 113 | 29.636 | 119.010 | 25.559 | 1.00 | 0.00 | AAAA |
| ATOM | 1835 | C | THR | E | 113 | 27.419 | 119.375 | 27.848 | 1.00 | 0.00 | AAAA |
| ATOM | 1836 | O | THR | E | 113 | 27.582 | 118.154 | 27.745 | 1.00 | 0.00 | AAAA |
| ATOM | 1837 | N | ARG | E | 114 | 26.282 | 119.883 | 28.328 | 1.00 | 0.00 | AAAA |
| ATOM | 1839 | CA | ARG | E | 114 | 25.132 | 119.075 | 28.685 | 1.00 | 0.00 | AAAA |
| ATOM | 1841 | CB | ARG | E | 114 | 24.774 | 117.934 | 27.733 | 1.00 | 0.00 | AAAA |
| ATOM | 1844 | CG | ARG | E | 114 | 23.336 | 117.435 | 27.924 | 1.00 | 0.00 | AAAA |
| ATOM | 1847 | CD | ARG | E | 114 | 22.759 | 116.677 | 26.738 | 1.00 | 0.00 | AAAA |
| ATOM | 1850 | NE | ARG | E | 114 | 21.466 | 116.106 | 27.121 | 1.00 | 0.00 | AAAA |
| ATOM | 1852 | CZ | ARG | E | 114 | 20.318 | 116.517 | 26.562 | 1.00 | 0.00 | AAAA |
| ATOM | 1853 | NH1 | ARG | E | 114 | 20.226 | 117.628 | 25.824 | 1.00 | 0.00 | AAAA |
| ATOM | 1856 | NH2 | ARG | E | 114 | 19.137 | 115.926 | 26.787 | 1.00 | 0.00 | AAAA |
| ATOM | 1859 | C | ARG | E | 114 | 25.027 | 118.770 | 30.168 | 1.00 | 0.00 | AAAA |
| ATOM | 1860 | O | ARG | E | 114 | 23.886 | 118.705 | 30.627 | 1.00 | 0.00 | AAAA |
| ATOM | 1861 | N | GLY | E | 115 | 26.130 | 118.631 | 30.917 | 1.00 | 0.00 | AAAA |
| ATOM | 1863 | CA | GLY | E | 115 | 26.182 | 118.435 | 32.353 | 1.00 | 0.00 | AAAA |
| ATOM | 1866 | C | GLY | E | 115 | 26.806 | 119.598 | 33.113 | 1.00 | 0.00 | AAAA |
| ATOM | 1867 | O | GLY | E | 115 | 26.903 | 120.704 | 32.588 | 1.00 | 0.00 | AAAA |
| ATOM | 1868 | N | SER | E | 116 | 27.058 | 119.416 | 34.410 | 1.00 | 0.00 | AAAA |
| ATOM | 1870 | CA | SER | E | 116 | 27.426 | 120.433 | 35.378 | 1.00 | 0.00 | AAAA |
| ATOM | 1872 | CB | SER | E | 116 | 26.199 | 120.794 | 36.207 | 1.00 | 0.00 | AAAA |
| ATOM | 1875 | OG | SER | E | 116 | 25.709 | 119.634 | 36.859 | 1.00 | 0.00 | AAAA |
| ATOM | 1877 | C | SER | E | 116 | 28.690 | 120.004 | 36.117 | 1.00 | 0.00 | AAAA |
| ATOM | 1878 | O | SER | E | 116 | 29.384 | 119.045 | 35.770 | 1.00 | 0.00 | AAAA |
| ATOM | 1879 | N | VAL | E | 117 | 29.041 | 120.813 | 37.109 | 1.00 | 0.00 | AAAA |
| ATOM | 1881 | CA | VAL | E | 117 | 30.104 | 120.616 | 38.072 | 1.00 | 0.00 | AAAA. |
| ATOM | 1883 | CB | VAL | E | 117 | 31.031 | 121.808 | 37.874 | 1.00 | 0.00 | AAAA |
| ATOM | 1885 | CG1 | VAL | E | 117 | 32.095 | 121.949 | 38.969 | 1.00 | 0.00 | AAAA |
| ATOM | 1889 | CG2 | VAL | E | 117 | 31.681 | 121.805 | 36.484 | 1.00 | 0.00 | AAAA. |
| ATOM | 1893 | C | VAL | E | 117 | 29.372 | 120.455 | 39.395 | 1.00 | 0.00 | AAAA |
| ATOM | 1894 | O | VAL | E | 117 | 28.229 | 120.874 | 39.583 | 1.00 | 0.00 | AAAA |
| ATOM | 1895 | N | ARG | E | 118 | 30.070 | 119.957 | 40.415 | 1.00 | 0.00 | AAAA |
| ATOM | 1897 | CA | ARG | E | 118 | 29.591 | 119.790 | 41.779 | 1.00 | 0.00 | AAAA |
| ATOM | 1899 | CB | ARG | E | 118 | 28.726 | 118.537 | 41.902 | 1.00 | 0.00 | AAAA |
| ATOM | 1902 | CG | ARG | E | 118 | 28.418 | 118.186 | 43.366 | 1.00 | 0.00 | AAAA |
| ATOM | 1905 | CD | ARG | E | 118 | 27.019 | 117.574 | 43.462 | 1.00 | 0.00 | AAAA |
| ATOM | 1908 | NE | ARG | E | 118 | 26.541 | 117.330 | 44.830 | 1.00 | 0.00 | AAAA |
| ATOM | 1910 | CZ | ARG | E | 118 | 25.336 | 116.936 | 45.260 | 1.00 | 0.00 | AAAA |
| ATOM | 1911 | NH1 | ARG | E | 118 | 24.306 | 116.873 | 44.391 | 1.00 | 0.00 | AAAA |
| ATOM | 1914 | NH2 | ARG | E | 118 | 25.103 | 116.567 | 46.523 | 1.00 | 0.00 | AAAA |
| ATOM | 1917 | C | ARG | E | 118 | 30.778 | 120.081 | 42.672 | 1.00 | 0.00 | AAAA |
| ATOM | 1916 | O | ARG | E | 118 | 31.797 | 119.420 | 42.477 | 1.00 | 0.00 | AAAA |
| ATOM | 1919 | N | ILE | E | 119 | 30.578 | 120.959 | 43.666 | 1.00 | 0.00 | AAAA |
| ATOM | 1921 | CA | ILE | E | 119 | 31.440 | 121.294 | 44.772 | 1.00 | 0.00 | AAAA |
| ATOM | 1923 | CB | ILE | E | 119 | 32.085 | 122.669 | 44.547 | 1.00 | 0.00 | AAAA |
| ATOM | 1925 | CG1 | ILE | E | 119 | 33.344 | 122.583 | 43.698 | 1.00 | 0.00 | AAAA |
| ATOM | 1928 | CG2 | ILE | E | 119 | 32.407 | 123.428 | 45.829 | 1.00 | 0.00 | AAAA |
| ATOM | 1932 | CD1 | ILE | E | 119 | 33.739 | 123.818 | 42.872 | 1.00 | 0.00 | AAAA |
| ATOM | 1936 | C | ILE | E | 119 | 30.646 | 121.108 | 46.055 | 1.00 | 0.00 | AAAA |
| ATOM | 1937 | O | ILE | E | 119 | 29.512 | 121.569 | 46.079 | 1.00 | 0.00 | AAAA |
| ATOM | 1938 | N | GLU | E | 120 | 31.107 | 120.329 | 47.046 | 1.00 | 0.00 | AAAA |
| ATOM | 1940 | CA | GLU | E | 120 | 30.443 | 119.795 | 48.214 | 1.00 | 0.00 | AAAA |
| ATOM | 1942 | CB | GLU | E | 120 | 29.459 | 118.718 | 47.743 | 1.00 | 0.00 | AAAA |
| ATOM | 1945 | CG | GLU | E | 120 | 28.466 | 118.117 | 48.748 | 1.00 | 0.00 | AAAA |
| ATOM | 1948 | CD | GLU | E | 120 | 27.646 | 116.990 | 48.134 | 1.00 | 0.00 | AAAA |
| ATOM | 1949 | OE1 | GLU | E | 120 | 26.633 | 116.674 | 48.789 | 1.00 | 0.00 | AAAA |
| ATOM | 1950 | OE2 | GLU | E | 120 | 27.925 | 116.373 | 47.087 | 1.00 | 0.00 | AAAA |
| ATOM | 1951 | C | GLU | E | 120 | 31.498 | 119.360 | 49.227 | 1.00 | 0.00 | AAAA |
| ATOM | 1952 | O | GLU | E | 120 | 32.055 | 118.267 | 49.238 | 1.00 | 0.00 | AAAA |
| ATOM | 1953 | N | LYS | E | 121 | 31.832 | 120.269 | 50.150 | 1.00 | 0.00 | AAAA |
| ATOM | 1955 | CA | LYS | E | 121 | 32.606 | 119.943 | 51.326 | 1.00 | 0.00 | AAAA |
| ATOM | 1957 | CB | LYS | E | 121 | 32.186 | 118.718 | 52.139 | 1.00 | 0.00 | AAAA |
| ATOM | 1960 | CG | LYS | E | 121 | 30.713 | 118.393 | 52.400 | 1.00 | 0.00 | AAAA |
| ATOM | 1963 | CD | LYS | E | 121 | 30.458 | 117.367 | 53.493 | 1.00 | 0.00 | AAAA |
| ATOM | 1966 | CE | LYS | E | 121 | 29.050 | 116.769 | 53.389 | 1.00 | 0.00 | AAAA |
| ATOM | 1969 | NZ | LYS | E | 121 | 28.828 | 115.926 | 54.569 | 1.00 | 0.00 | AAAA |
| ATOM | 1973 | C | LYS | E | 121 | 34.117 | 120.093 | 51.196 | 1.00 | 0.00 | AAAA |
| ATOM | 1974 | O | LYS | E | 121 | 34.884 | 119.347 | 51.797 | 1.00 | 0.00 | AAAA |
| ATOM | 1975 | N | ASN | E | 122 | 34.535 | 121.077 | 50.400 | 1.00 | 0.00 | AAAA |
| ATOM | 1977 | CA | ASN | E | 122 | 35.911 | 121.370 | 50.045 | 1.00 | 0.00 | AAAA |
| ATOM | 1979 | CB | ASN | E | 122 | 36.156 | 121.595 | 48.553 | 1.00 | 0.00 | AAAA |
| ATOM | 1982 | CG | ASN | E | 122 | 35.699 | 120.461 | 47.637 | 1.00 | 0.00 | AAAA |
| ATOM | 1983 | OD1 | ASN | E | 122 | 36.088 | 119.307 | 47.800 | 1.00 | 0.00 | AAAA |
| ATOM | 1984 | ND2 | ASN | E | 122 | 34.683 | 120.746 | 46.816 | 1.00 | 0.00 | AAAA |
| ATOM | 1987 | C | ASN | E | 122 | 36.281 | 122.618 | 50.831 | 1.00 | 0.00 | AAAA |
| ATOM | 1988 | O | ASN | E | 122 | 36.073 | 123.751 | 50.400 | 1.00 | 0.00 | AAAA |
| ATOM | 1989 | N | ASN | E | 123 | 36.543 | 122.456 | 52.125 | 1.00 | 0.00 | AAAA |
| ATOM | 1991 | CA | ASN | E | 123 | 36.521 | 123.541 | 53.084 | 1.00 | 0.00 | AAAA |
| ATOM | 1993 | CB | ASN | E | 123 | 36.747 | 122.973 | 54.484 | 1.00 | 0.00 | AAAA |
| ATOM | 1996 | CG | ASN | E | 123 | 36.534 | 123.924 | 55.653 | 1.00 | 0.00 | AAAA |
| ATOM | 1997 | OD1 | ASN | E | 123 | 35.380 | 124.223 | 55.972 | 1.00 | 0.00 | AAAA |
| ATOM | 1998 | ND2 | ASN | E | 123 | 37.603 | 124.435 | 56.260 | 1.00 | 0.00 | AAAA |
| ATOM | 2001 | C | ASN | E | 123 | 37.493 | 124.675 | 52.777 | 1.00 | 0.00 | AAAA |
| ATOM | 2002 | O | ASN | E | 123 | 37.170 | 125.796 | 53.170 | 1.00 | 0.00 | AAAA |
| ATOM | 2003 | N | GLU | E | 124 | 38.691 | 124.412 | 52.243 | 1.00 | 0.00 | AAAA |
| ATOM | 2005 | CA | GLU | E | 124 | 39.708 | 125.410 | 51.999 | 1.00 | 0.00 | AAAA |
| ATOM | 2007 | CB | GLU | E | 124 | 41.108 | 124.808 | 52.131 | 1.00 | 0.00 | AAAA |
| ATOM | 2010 | CG | GLU | E | 124 | 42.177 | 125.838 | 52.494 | 1.00 | 0.00 | AAAA |
| ATOM | 2013 | CD | GLU | E | 124 | 42.102 | 126.366 | 53.927 | 1.00 | 0.00 | AAAA |
| ATOM | 2014 | OE1 | GLU | E | 124 | 41.860 | 127.573 | 54.123 | 1.00 | 0.00 | AAAA |
| ATOM | 2015 | OE2 | GLU | E | 124 | 42.374 | 125.572 | 54.853 | 1.00 | 0.00 | AAAA |
| ATOM | 2016 | C | GLU | E | 124 | 39.517 | 126.120 | 50.665 | 1.00 | 0.00 | AAAA |
| ATOM | 2017 | O | GLU | E | 124 | 40.290 | 127.024 | 50.344 | 1.00 | 0.00 | AAAA |
| ATOM | 2018 | N | LEU | E | 125 | 38.483 | 125.796 | 49.890 | 1.00 | 0.00 | AAAA |
| ATOM | 2020 | CA | LEU | E | 125 | 38.358 | 126.143 | 48.488 | 1.00 | 0.00 | AAAA |
| ATOM | 2022 | CB | LEU | E | 125 | 37.589 | 125.045 | 47.756 | 1.00 | 0.00 | AAAA |
| ATOM | 2025 | CG | LEU | E | 125 | 37.552 | 125.093 | 46.219 | 1.00 | 0.00 | AAAA |
| ATOM | 2027 | CD1 | LEU | E | 125 | 38.938 | 124.886 | 45.604 | 1.00 | 0.00 | AAAA |
| ATOM | 2031 | CD2 | LEU | E | 125 | 36.532 | 124.066 | 45.721 | 1.00 | 0.00 | AAAA |
| ATOM | 2035 | C | LEU | E | 125 | 37.607 | 127.463 | 48.321 | 1.00 | 0.00 | AAAA |
| ATOM | 2036 | O | LEU | E | 125 | 36.436 | 127.652 | 98.629 | 1.00 | 0.00 | AAAA |
| ATOM | 2037 | N | CYS | E | 126 | 38.203 | 128.394 | 47.576 | 1.00 | 0.00 | AAAA |
| ATOM | 2039 | CA | CYS | E | 126 | 37.663 | 129.545 | 46.873 | 1.00 | 0.00 | AAAA |
| ATOM | 2041 | CB | CYS | E | 126 | 37.850 | 130.787 | 47.750 | 1.00 | 0.00 | AAAA |
| ATOM | 2044 | SG | CYS | E | 126 | 39.396 | 131.051 | 48.650 | 1.00 | 0.00 | AAAA |
| ATOM | 2045 | C | CYS | E | 126 | 38.267 | 129.576 | 45.485 | 1.00 | 0.00 | AAAA |
| ATOM | 2046 | O | CYS | E | 126 | 38.888 | 128.573 | 45.103 | 1.00 | 0.00 | AAAA |
| ATOM | 2047 | N | TYR | E | 127 | 37.914 | 130.615 | 44.726 | 1.00 | 0.00 | AAAA |
| ATOM | 2049 | CA | TYR | E | 127 | 38.108 | 130.844 | 43.310 | 1.00 | 0.00 | AAAA |
| ATOM | 2051 | CB | TYR | E | 127 | 39.370 | 130.245 | 42.682 | 1.00 | 0.00 | AAAA |
| ATOM | 2054 | CG | TYR | E | 127 | 40.587 | 131.093 | 43.007 | 1.00 | 0.00 | AAAA |
| ATOM | 2055 | CD1 | TYR | E | 127 | 40.810 | 132.332 | 42.418 | 1.00 | 0.00 | AAAA |
| ATOM | 2057 | CD2 | TYR | E | 127 | 41.564 | 130.571 | 43.871 | 1.00 | 0.00 | AAAA |
| ATOM | 2059 | CE1 | TYR | E | 127 | 41.958 | 133.082 | 42.703 | 1.00 | 0.00 | AAAA |
| ATOM | 2061 | CE2 | TYR | E | 127 | 42.714 | 131.297 | 44.201 | 1.00 | 0.00 | AAAA |
| ATOM | 2063 | CZ | TYR | E | 127 | 42.880 | 132.550 | 43.616 | 1.00 | 0.00 | AAAA |
| ATOM | 2064 | OH | TYR | E | 127 | 44.054 | 133.240 | 43.781 | 1.00 | 0.00 | AAAA |
| ATOM | 2066 | C | TYR | E | 127 | 36.865 | 130.665 | 42.454 | 1.00 | 0.00 | AAAA |
| ATOM | 2067 | O | TYR | E | 127 | 36.730 | 131.248 | 41.375 | 1.00 | 0.00 | AAAA |
| ATOM | 2068 | N | LEU | E | 128 | 35.853 | 129.970 | 42.962 | 1.00 | 0.00 | AAAA |
| ATOM | 2070 | CA | LEU | E | 128 | 34.607 | 129.608 | 42.306 | 1.00 | 0.00 | AAAA |
| ATOM | 2072 | CB | LEU | E | 128 | 33.957 | 128.453 | 43.063 | 1.00 | 0.00 | AAAA |
| ATOM | 2075 | CG | LEU | E | 128 | 33.801 | 128.629 | 44.571 | 1.00 | 0.00 | AAAA |
| ATOM | 2077 | CD1 | LEU | E | 128 | 32.360 | 128.391 | 45.023 | 1.00 | 0.00 | AAAA |
| ATOM | 2081 | CD2 | LEU | E | 128 | 34.717 | 127.759 | 45.430 | 1.00 | 0.00 | AAAA |
| ATOM | 2085 | C | LEU | E | 128 | 33.615 | 130.678 | 41.889 | 1.00 | 0.00 | AAAA |
| ATOM | 2086 | O | LEU | E | 128 | 33.020 | 130.669 | 40.813 | 1.00 | 0.00 | AAAA |
| ATOM | 2087 | N | ALA | E | 129 | 33.705 | 131.790 | 42.637 | 1.00 | 0.00 | AAAA |
| ATOM | 2089 | CA | ALA | E | 129 | 33.049 | 133.066 | 42.415 | 1.00 | 0.00 | AAAA |
| ATOM | 2091 | CB | ALA | E | 129 | 32.714 | 133.724 | 43.759 | 1.00 | 0.00 | AAAA |
| ATOM | 2095 | C | ALA | E | 129 | 33.905 | 134.025 | 41.615 | 1.00 | 0.00 | AAAA |
| ATOM | 2096 | O | ALA | E | 129 | 33.362 | 135.040 | 41.173 | 1.00 | 0.00 | AAAA |
| ATOM | 2097 | N | THR | E | 130 | 35.177 | 133.750 | 41.320 | 1.00 | 0.00 | AAAA |
| ATOM | 2099 | CA | THR | E | 130 | 36.089 | 134.748 | 40.789 | 1.00 | 0.00 | AAAA |
| ATOM | 2101 | CB | THR | E | 130 | 37.528 | 134.640 | 41.318 | 1.00 | 0.00 | AAAA |
| ATOM | 2103 | OG1 | THR | E | 130 | 37.423 | 134.550 | 42.722 | 1.00 | 0.00 | AAAA |
| ATOM | 2105 | CG2 | THR | E | 130 | 38.543 | 135.750 | 41.092 | 1.00 | 0.00 | AAAA |
| ATOM | 2109 | C | THR | E | 130 | 36.171 | 134.526 | 39.291 | 1.00 | 0.00 | AAAA |
| ATOM | 2110 | O | THR | E | 130 | 36.604 | 135.372 | 38.512 | 1.00 | 0.00 | AAAA |
| ATOM | 2111 | N | ILE | E | 131 | 35.707 | 133.348 | 38.853 | 1.00 | 0.00 | AAAA |
| ATOM | 2113 | CA | ILE | E | 131 | 35.542 | 132.911 | 37.489 | 1.00 | 0.00 | AAAA |
| ATOM | 2115 | CB | ILE | E | 131 | 35.897 | 131.417 | 37.443 | 1.00 | 0.00 | AAAA |
| ATOM | 2117 | CG1 | ILE | E | 131 | 35.118 | 130.540 | 38.413 | 1.00 | 0.00 | AAAA |
| ATOM | 2120 | CG2 | ILE | E | 131 | 37.402 | 131.151 | 37.423 | 1.00 | 0.00 | AAAA |
| ATOM | 2124 | CD1 | ILE | E | 131 | 34.845 | 129.158 | 37.818 | 1.00 | 0.00 | AAAA |
| ATOM | 2128 | C | ILE | E | 131 | 34.093 | 133.037 | 37.061 | 1.00 | 0.00 | AAAA |
| ATOM | 2129 | O | ILE | E | 131 | 33.248 | 133.193 | 37.937 | 1.00 | 0.00 | AAAA |
| ATOM | 2130 | N | ASP | E | 132 | 33.795 | 133.104 | 35.759 | 1.00 | 0.00 | AAAA |
| ATOM | 2132 | CA | ASP | E | 132 | 32.486 | 132.815 | 35.213 | 1.00 | 0.00 | AAAA |
| ATOM | 2134 | CB | ASP | E | 132 | 32.285 | 133.639 | 33.949 | 1.00 | 0.00 | AAAA |
| ATOM | 2137 | CG | ASP | E | 132 | 30.826 | 133.728 | 33.511 | 1.00 | 0.00 | AAAA |
| ATOM | 2138 | OD1 | ASP | E | 132 | 30.486 | 134.784 | 32.947 | 1.00 | 0.00 | AAAA |
| ATOM | 2139 | OD2 | ASP | E | 132 | 30.069 | 132.760 | 33.775 | 1.00 | 0.00 | AAAA |
| ATOM | 2140 | C | ASP | E | 132 | 32.445 | 131.356 | 34.779 | 1.00 | 0.00 | AAAA |
| ATOM | 2141 | O | ASP | E | 132 | 33.181 | 130.947 | 33.881 | 1.00 | 0.00 | AAAA |
| ATOM | 2142 | N | TRP | E | 133 | 31.647 | 130.501 | 35.415 | 1.00 | 0.00 | AAAA |
| ATOM | 2144 | CA | TRP | E | 133 | 31.340 | 129.185 | 34.890 | 1.00 | 0.00 | AAAA |
| ATOM | 2146 | CB | TRP | E | 133 | 30.379 | 128.570 | 35.913 | 1.00 | 0.00 | AAAA |
| ATOM | 2149 | CG | TRP | E | 133 | 30.900 | 128.404 | 37.309 | 1.00 | 0.00 | AAAA |
| ATOM | 2150 | CD1 | TRP | E | 133 | 30.368 | 129.016 | 38.395 | 1.00 | 0.00 | AAAA |
| ATOM | 2152 | CD2 | TRP | E | 133 | 31.837 | 127.388 | 37.781 | 1.00 | 0.00 | AAAA |
| ATOM | 2153 | NE1 | TRP | E | 133 | 30.933 | 128.441 | 39.515 | 1.00 | 0.00 | AAAA |
| ATOM | 2155 | CE2 | TRP | E | 133 | 31.861 | 127.474 | 39.198 | 1.00 | 0.00 | AAAA |
| ATOM | 2156 | CE3 | TRP | E | 133 | 32.603 | 126.396 | 37.135 | 1.00 | 0.00 | AAAA |
| ATOM | 2158 | CZ2 | TRP | E | 133 | 32.631 | 126.579 | 39.947 | 1.00 | 0.00 | AAAA |
| ATOM | 2160 | CZ3 | TRP | E | 133 | 33.283 | 125.448 | 37.896 | 1.00 | 0.00 | AAAA |
| ATOM | 2162 | CH2 | TRP | E | 133 | 33.309 | 125.539 | 39.291 | 1.00 | 0.00 | AAAA |
| ATOM | 2164 | C | TRP | E | 133 | 30.833 | 129.032 | 33.465 | 1.00 | 0.00 | AAAA |
| ATOM | 2165 | O | TRP | E | 133 | 30.949 | 127.933 | 32.931 | 1.00 | 0.00 | AAAA |
| ATOM | 2166 | N | SER | E | 134 | 30.171 | 130.019 | 32.859 | 1.00 | 0.00 | AAAA |
| ATOM | 2168 | CA | SER | E | 134 | 29.624 | 129.908 | 31.520 | 1.00 | 0.00 | AAAA |
| ATOM | 2170 | CB | SER | E | 134 | 28.573 | 130.987 | 31.245 | 1.00 | 0.00 | AAAA |
| ATOM | 2173 | OG | SER | E | 134 | 29.134 | 132.271 | 31.356 | 1.00 | 0.00 | AAAA |
| ATOM | 2175 | C | SER | E | 134 | 30.740 | 129.872 | 30.479 | 1.00 | 0.00 | AAAA |
| ATOM | 2176 | O | SER | E | 134 | 30.494 | 129.680 | 29.281 | 1.00 | 0.00 | AAAA |
| ATOM | 2177 | N | ARG | E | 135 | 31.992 | 130.054 | 30.886 | 1.00 | 0.00 | AAAA |
| ATOM | 2179 | CA | ARG | E | 135 | 33.110 | 129.928 | 29.975 | 1.00 | 0.00 | AAAA |
| ATOM | 2181 | CB | ARG | E | 135 | 34.218 | 130.788 | 30.562 | 1.00 | 0.00 | AAAA |
| ATOM | 2184 | CG | ARG | E | 135 | 33.957 | 132.285 | 30.385 | 1.00 | 0.00 | AAAA |
| ATOM | 2187 | CD | ARG | E | 135 | 33.902 | 132.948 | 29.013 | 1.00 | 0.00 | AAAA |
| ATOM | 2190 | NE | ARG | E | 135 | 34.399 | 134.317 | 29.039 | 1.00 | 0.00 | AAAA |
| ATOM | 2192 | CZ | ARG | E | 135 | 34.731 | 134.975 | 27.913 | 1.00 | 0.00 | AAAA |
| ATOM | 2193 | NH1 | ARG | E | 135 | 34.407 | 134.424 | 26.734 | 1.00 | 0.00 | AAAA |
| ATOM | 2196 | NH2 | ARG | E | 135 | 35.345 | 136.165 | 27.963 | 1.00 | 0.00 | AAAA |
| ATOM | 2199 | C | ARG | E | 135 | 33.598 | 128.477 | 29.986 | 1.00 | 0.00 | AAAA |
| ATOM | 2200 | O | ARG | E | 135 | 34.426 | 128.082 | 29.178 | 1.00 | 0.00 | AAAA |
| ATOM | 2201 | N | ILE | E | 136 | 33.064 | 127.606 | 30.843 | 1.00 | 0.00 | AAAA |
| ATOM | 2203 | CA | ILE | E | 136 | 33.319 | 126.185 | 30.747 | 1.00 | 0.00 | AAAA |
| ATOM | 2205 | CB | ILE | E | 136 | 33.891 | 125.739 | 32.095 | 1.00 | 0.00 | AAAA |
| ATOM | 2207 | CG1 | ILE | E | 136 | 35.152 | 126.529 | 32.473 | 1.00 | 0.00 | AAAA |
| ATOM | 2210 | CG2 | ILL | E | 136 | 34.340 | 124.283 | 32.130 | 1.00 | 0.00 | AAAA |
| ATOM | 2214 | CD1 | ILE | E | 136 | 34.801 | 127.569 | 33.538 | 1.00 | 0.00 | AAAA |
| ATOM | 2218 | C | ILE | E | 136 | 32.092 | 125.335 | 30.440 | 1.00 | 0.00 | AAAA |
| ATOM | 2219 | O | ILE | E | 136 | 32.269 | 124.425 | 29.626 | 1.00 | 0.00 | AAAA |
| ATOM | 2220 | N | LEU | E | 137 | 30.994 | 125.529 | 31.162 | 1.00 | 0.00 | AAAA |
| ATOM | 2222 | CA | LEU | E | 137 | 29.799 | 124.714 | 31.005 | 1.00 | 0.00 | AAAA |
| ATOM | 2224 | CB | LEU | E | 137 | 29.027 | 124.824 | 32.324 | 1.00 | 0.00 | AAAA |
| ATOM | 2227 | CG | LEU | E | 137 | 29.507 | 124.031 | 33.544 | 1.00 | 0.00 | AAAA |
| ATOM | 2229 | CD1 | LEU | E | 137 | 30.717 | 124.649 | 34.244 | 1.00 | 0.00 | AAAA |
| ATOM | 2233 | CD2 | LEU | E | 137 | 28.360 | 124.010 | 34.550 | 1.00 | 0.00 | AAAA |
| ATOM | 2237 | C | LEU | E | 137 | 28.920 | 125.480 | 30.016 | 1.00 | 0.00 | AAAA |
| ATOM | 2238 | O | LEU | E | 137 | 28.996 | 126.682 | 29.791 | 1.00 | 0.00 | AAAA |
| ATOM | 2239 | N | ASP | E | 138 | 28.010 | 124.772 | 29.339 | 1.00 | 0.00 | AAAA |
| ATOM | 2241 | CA | ASP | E | 138 | 27.028 | 125.207 | 28.369 | 1.00 | 0.00 | AAAA |
| ATOM | 2243 | CB | ASP | E | 138 | 26.579 | 124.070 | 27.457 | 1.00 | 0.00 | AAAA |
| ATOM | 2246 | CG | ASP | E | 138 | 25.213 | 124.121 | 26.790 | 1.00 | 0.00 | AAAA |
| ATOM | 2247 | OD1 | ASP | E | 138 | 25.074 | 124.959 | 25.877 | 1.00 | 0.00 | AAAA |
| ATOM | 2248 | OD2 | ASP | E | 138 | 24.263 | 123.445 | 27.242 | 1.00 | 0.00 | AAAA |
| ATOM | 2249 | C | ASP | E | 138 | 25.838 | 125.846 | 29.054 | 1.00 | 0.00 | AAAA |
| ATOM | 2250 | O | ASP | E | 138 | 25.237 | 126.784 | 28.523 | 1.00 | 0.00 | AAAA |
| ATOM | 2251 | N | SER | E | 139 | 25.367 | 125.339 | 30.198 | 1.00 | 0.00 | AAAA |
| ATOM | 2253 | CA | SER | E | 139 | 24.363 | 125.888 | 31.097 | 1.00 | 0.00 | AAAA |
| ATOM | 2255 | CB | SER | E | 139 | 23.001 | 125.363 | 30.644 | 1.00 | 0.00 | AAAA |
| ATOM | 2258 | OG | SER | E | 139 | 22.868 | 123.992 | 30.956 | 1.00 | 0.00 | AAAA |
| ATOM | 2260 | C | SER | E | 139 | 24.825 | 125.695 | 32.536 | 1.00 | 0.00 | AAAA |
| ATOM | 2261 | O | SER | E | 139 | 25.580 | 124.790 | 32.870 | 1.00 | 0.00 | AAAA |
| ATOM | 2262 | N | VAL | E | 140 | 24.390 | 126.566 | 33.441 | 1.00 | 0.00 | AAAA |
| ATOM | 2264 | CA | VAL | E | 140 | 24.816 | 126.722 | 34.819 | 1.00 | 0.00 | AAAA |
| ATOM | 2266 | CB | VAL | E | 140 | 25.654 | 127.998 | 34.834 | 1.00 | 0.00 | AAAA |
| ATOM | 2268 | CG1 | VAL | E | 140 | 26.917 | 127.827 | 33.978 | 1.00 | 0.00 | AAAA |
| ATOM | 2272 | CG2 | VAL | E | 140 | 25.018 | 129.329 | 34.433 | 1.00 | 0.00 | AAAA |
| ATOM | 2276 | C | VAL | E | 140 | 23.701 | 126.632 | 35.847 | 1.00 | 0.00 | AAAA |
| ATOM | 2277 | O | VAL | E | 140 | 23.862 | 127.163 | 36.953 | 1.00 | 0.00 | AAAA |
| ATOM | 2278 | N | GLU | E | 141 | 22.546 | 126.116 | 35.423 | 1.00 | 0.00 | AAAA |
| ATOM | 2280 | CA | GLU | E | 141 | 21.346 | 126.099 | 36.236 | 1.00 | 0.00 | AAAA |
| ATOM | 2282 | CB | GLU | E | 141 | 20.179 | 125.876 | 35.269 | 1.00 | 0.00 | AAAA |
| ATOM | 2285 | CG | GLU | E | 141 | 18.776 | 126.137 | 35.823 | 1.00 | 0.00 | AAAA |
| ATOM | 2288 | CD | GLU | E | 141 | 18.461 | 127.630 | 35.951 | 1.00 | 0.00 | AAAA |
| ATOM | 2289 | OE1 | GLU | E | 141 | 17.267 | 127.931 | 36.188 | 1.00 | 0.00 | AAAA |
| ATOM | 2290 | OE2 | GLU | E | 141 | 19.270 | 128.495 | 35.577 | 1.00 | 0.00 | AAAA |
| ATOM | 2291 | C | GLU | E | 141 | 21.378 | 124.914 | 37.187 | 1.00 | 0.00 | AAAA |
| ATOM | 2292 | O | GLU | E | 141 | 20.760 | 124.892 | 38.244 | 1.00 | 0.00 | AAAA |
| ATOM | 2293 | N | ASP | E | 142 | 22.029 | 123.836 | 36.732 | 1.00 | 0.00 | AAAA |
| ATOM | 2295 | CA | ASP | E | 142 | 22.099 | 122.583 | 37.465 | 1.00 | 0.00 | AAAA |
| ATOM | 2297 | CB | ASP | E | 142 | 21.969 | 121.373 | 36.532 | 1.00 | 0.00 | AAAA |
| ATOM | 2300 | CG | ASP | E | 142 | 20.723 | 121.402 | 35.665 | 1.00 | 0.00 | AAAA |
| ATOM | 2301 | OD1 | ASP | E | 142 | 19.628 | 121.005 | 36.112 | 1.00 | 0.00 | AAAA |
| ATOM | 2302 | OD2 | ASP | E | 142 | 20.847 | 121.763 | 34.466 | 1.00 | 0.00 | AAAA |
| ATOM | 2303 | C | ASP | E | 142 | 23.444 | 122.407 | 38.141 | 1.00 | 0.00 | AAAA |
| ATOM | 2304 | O | ASP | E | 142 | 23.833 | 121.274 | 38.388 | 1.00 | 0.00 | AAAA |
| ATOM | 2305 | N | ASN | E | 143 | 24.188 | 123.475 | 38.461 | 1.00 | 0.00 | AAAA |
| ATOM | 2307 | CA | ASN | E | 143 | 25.472 | 123.544 | 39.130 | 1.00 | 0.00 | AAAA |
| ATOM | 2309 | CB | ASN | E | 143 | 26.089 | 124.865 | 38.662 | 1.00 | 0.00 | AAAA |
| ATOM | 2312 | CG | ASN | E | 143 | 27.614 | 124.885 | 38.675 | 1.00 | 0.00 | AAAA |
| ATOM | 2313 | OD1 | ASN | E | 143 | 28.253 | 123.867 | 38.407 | 1.00 | 0.00 | AAAA |
| ATOM | 2314 | ND2 | ASN | E | 143 | 28.176 | 126.064 | 38.973 | 1.00 | 0.00 | AAAA |
| ATOM | 2317 | C | ASN | E | 143 | 25.219 | 123.620 | 40.629 | 1.00 | 0.00 | AAAA |
| ATOM | 2318 | O | ASN | E | 143 | 24.322 | 124.245 | 41.180 | 1.00 | 0.00 | AAAA |
| ATOM | 2319 | N | HIS | E | 144 | 25.918 | 122.756 | 41.375 | 1.00 | 0.00 | AAAA |
| ATOM | 2321 | CA | HIS | E | 144 | 25.638 | 122.453 | 42.757 | 1.00 | 0.00 | AAAA |
| ATOM | 2323 | CB | HIS | E | 144 | 25.323 | 120.960 | 42.881 | 1.00 | 0.00 | AAAA |
| ATOM | 2326 | CG | HIS | E | 144 | 24.928 | 120.545 | 44.276 | 1.00 | 0.00 | AAAA |
| ATOM | 2327 | ND1 | HIS | E | 144 | 23.608 | 120.556 | 44.722 | 1.00 | 0.00 | AAAA |
| ATOM | 2328 | CD2 | HIS | E | 144 | 25.685 | 120.256 | 45.377 | 1.00 | 0.00 | AAAA |
| ATOM | 2330 | CE1 | HIS | E | 144 | 23.558 | 120.224 | 46.013 | 1.00 | 0.00 | AAAA |
| ATOM | 2332 | NE2 | HIS | E | 144 | 24.820 | 120.063 | 46.445 | 1.00 | 0.00 | AAAA |
| ATOM | 2334 | C | HIS | E | 144 | 26.902 | 122.704 | 43.563 | 1.00 | 0.00 | AAAA |
| ATOM | 2335 | O | HIS | E | 144 | 27.749 | 121.816 | 43.510 | 1.00 | 0.00 | AAAA |
| ATOM | 2336 | N | ILE | E | 145 | 26.992 | 123.864 | 44.212 | 1.00 | 0.00 | AAAA |
| ATOM | 2338 | CA | ILE | E | 145 | 28.235 | 124.426 | 44.692 | 1.00 | 0.00 | AAAA |
| ATOM | 2340 | CB | ILE | E | 145 | 28.601 | 125.523 | 43.695 | 1.00 | 0.00 | AAAA |
| ATOM | 2342 | CG1 | ILE | E | 145 | 28.763 | 125.190 | 42.207 | 1.00 | 0.00 | AAAA |
| ATOM | 2345 | CG2 | ILE | E | 145 | 29.825 | 126.347 | 44.102 | 1.00 | 0.00 | AAAA |
| ATOM | 2349 | CD1 | ILE | E | 145 | 29.849 | 124.217 | 41.748 | 1.00 | 0.00 | AAAA |
| ATOM | 2353 | C | ILE | E | 145 | 28.028 | 124.895 | 46.124 | 1.00 | 0.00 | AAAA |
| ATOM | 2354 | O | ILE | E | 145 | 27.433 | 125.942 | 46.399 | 1.00 | 0.00 | AAAA |
| ATOM | 2355 | N | VAL | E | 146 | 28.324 | 124.005 | 47.063 | 1.00 | 0.00 | AAAA |
| ATOM | 2357 | CA | VAL | E | 146 | 27.951 | 124.199 | 48.449 | 1.00 | 0.00 | AAAA |
| ATOM | 2359 | CB | VAL | E | 146 | 26.641 | 123.534 | 48.881 | 1.00 | 0.00 | AAAA |
| ATOM | 2361 | CG1 | VAL | E | 146 | 25.450 | 124.223 | 48.213 | 1.00 | 0.00 | AAAA |
| ATOM | 2365 | CG2 | VAL | E | 146 | 26.566 | 122.027 | 48.654 | 1.00 | 0.00 | AAAA |
| ATOM | 2369 | C | VAL | E | 146 | 29.134 | 123.751 | 49.302 | 1.00 | 0.00 | AAAA |
| ATOM | 2370 | O | VAL | E | 146 | 29.887 | 122.914 | 48.806 | 1.00 | 0.00 | AAAA |
| ATOM | 2371 | N | LEU | E | 147 | 29.341 | 124.299 | 50.494 | 1.00 | 0.00 | AAAA |
| ATOM | 2373 | CA | LEU | E | 147 | 30.194 | 123.771 | 51.542 | 1.00 | 0.00 | AAAA |
| ATOM | 2375 | CB | LEU | E | 147 | 29.814 | 122.370 | 52.021 | 1.00 | 0.00 | AAAA |
| ATOM | 2378 | CG | LEU | E | 147 | 28.402 | 122.366 | 52.609 | 1.00 | 0.00 | AAAA |
| ATOM | 2380 | CD1 | LEU | E | 147 | 27.802 | 120.965 | 52.729 | 1.00 | 0.00 | AAAA |
| ATOM | 2384 | CD2 | LEU | E | 147 | 28.421 | 123.059 | 53.975 | 1.00 | 0.00 | AAAA |
| ATOM | 2388 | C | LEU | E | 147 | 31.647 | 124.057 | 51.173 | 1.00 | 0.00 | AAAA |
| ATOM | 2389 | O | LEU | E | 147 | 32.305 | 123.115 | 50.726 | 1.00 | 0.00 | AAAA |
| ATOM | 2390 | N | ASN | E | 148 | 32.143 | 125.291 | 51.298 | 1.00 | 0.00 | AAAA |
| ATOM | 2392 | CA | ASN | E | 148 | 33.458 | 125.709 | 50.864 | 1.00 | 0.00 | AAAA |
| ATOM | 2394 | CB | ASN | E | 148 | 33.530 | 125.767 | 49.334 | 1.00 | 0.00 | AAAA |
| ATOM | 2397 | CG | ASN | E | 148 | 32.502 | 126.669 | 48.668 | 1.00 | 0.00 | AAAA |
| ATOM | 2398 | OD1 | ASN | E | 148 | 32.645 | 127.890 | 48.809 | 1.00 | 0.00 | AAAA |
| ATOM | 2399 | ND2 | ASN | E | 148 | 31.441 | 126.057 | 48.148 | 1.00 | 0.00 | AAAA |
| ATOM | 2402 | C | ASN | E | 148 | 33.848 | 126.899 | 51.724 | 1.00 | 0.00 | AAAA |
| ATOM | 2403 | O | ASN | E | 148 | 33.114 | 127.318 | 52.625 | 1.00 | 0.00 | AAAA |
| ATOM | 2404 | N | LYS | E | 149 | 34.937 | 127.574 | 51.328 | 1.00 | 0.00 | AAAA |
| ATOM | 2406 | CA | LYS | E | 149 | 35.387 | 128.773 | 52.015 | 1.00 | 0.00 | AAAA |
| ATOM | 2408 | CB | LYS | E | 149 | 36.912 | 128.647 | 52.093 | 1.00 | 0.00 | AAAA |
| ATOM | 2411 | CG | LYS | E | 149 | 37.455 | 129.073 | 53.466 | 1.00 | 0.00 | AAAA |
| ATOM | 2414 | CD | LYS | E | 149 | 38.929 | 129.442 | 53.513 | 1.00 | 0.00 | AAAA |
| ATOM | 2417 | CE | LYS | E | 149 | 39.563 | 129.796 | 54.860 | 1.00 | 0.00 | AAAA |
| ATOM | 2420 | NZ | LYS | E | 149 | 39.978 | 128.663 | 55.701 | 1.00 | 0.00 | AAAA |
| ATOM | 2424 | C | LYS | E | 149 | 35.105 | 130.083 | 51.294 | 1.00 | 0.00 | AAAA |
| ATOM | 2425 | O | LYS | E | 149 | 35.349 | 131.174 | 51.804 | 1.00 | 0.00 | AAAA |
| ATOM | 2426 | N | ASP | E | 150 | 34.441 | 130.008 | 50.136 | 1.00 | 0.00 | AAAA |
| ATOM | 2428 | CA | ASP | E | 150 | 33.790 | 131.179 | 49.591 | 1.00 | 0.00 | AAAA |
| ATOM | 2430 | CB | ASP | E | 150 | 33.897 | 130.966 | 48.074 | 1.00 | 0.00 | AAAA |
| ATOM | 2433 | CG | ASP | E | 150 | 33.937 | 132.310 | 47.368 | 1.00 | 0.00 | AAAA |
| ATOM | 2434 | OD1 | ASP | E | 150 | 35.047 | 132.850 | 47.166 | 1.00 | 0.00 | AAAA |
| ATOM | 2435 | OD2 | ASP | E | 150 | 32.809 | 132.769 | 47.082 | 1.00 | 0.00 | AAAA |
| ATOM | 2436 | C | ASP | E | 150 | 32.331 | 131.174 | 50.036 | 1.00 | 0.00 | AAAA |
| ATOM | 2437 | O | ASP | E | 150 | 31.985 | 132.233 | 50.560 | 1.00 | 0.00 | AAAA |
| ATOM | 2438 | N | ASP | E | 151 | 31.662 | 130.023 | 50.098 | 1.00 | 0.00 | AAAA |
| ATOM | 2440 | CA | ASP | E | 151 | 30.343 | 129.839 | 50.661 | 1.00 | 0.00 | AAAA |
| ATOM | 2442 | CB | ASP | E | 151 | 29.985 | 128.365 | 50.448 | 1.00 | 0.00 | AAAA |
| ATOM | 2445 | CG | ASP | E | 151 | 28.723 | 127.859 | 51.147 | 1.00 | 0.00 | AAAA |
| ATOM | 2446 | OD1 | ASP | E | 151 | 27.667 | 128.510 | 50.948 | 1.00 | 0.00 | AAAA |
| ATOM | 2447 | OD2 | ASP | E | 151 | 28.764 | 126.850 | 51.879 | 1.00 | 0.00 | AAAA |
| ATOM | 2448 | C | ASP | E | 151 | 30.194 | 130.252 | 52.120 | 1.00 | 0.00 | AAAA |
| ATOM | 2449 | O | ASP | E | 151 | 29.298 | 131.019 | 52.467 | 1.00 | 0.00 | AAAA |
| ATOM | 2450 | N | ASN | E | 152 | 31.107 | 129.869 | 53.010 | 1.00 | 0.00 | AAAA |
| ATOM | 2452 | CA | ASN | E | 152 | 31.220 | 130.316 | 54.391 | 1.00 | 0.00 | AAAA |
| ATOM | 2454 | CB | ASN | E | 152 | 31.417 | 129.127 | 55.344 | 1.00 | 0.00 | AAAA |
| ATOM | 2457 | CG | ASN | E | 152 | 31.394 | 129.571 | 56.797 | 1.00 | 0.00 | AAAA |
| ATOM | 2458 | OD1 | ASN | E | 152 | 30.362 | 129.767 | 57.442 | 1.00 | 0.00 | AAAA |
| ATOM | 2459 | ND2 | ASN | E | 152 | 32.577 | 129.795 | 57.391 | 1.00 | 0.00 | AAAA |
| ATOM | 2462 | C | ASN | E | 152 | 32.434 | 131.208 | 54.208 | 1.00 | 0.00 | AAAA |
| ATOM | 2463 | O | ASN | E | 152 | 33.510 | 130.654 | 54.036 | 1.00 | 0.00 | AAAA |
| ATOM | 2464 | N | GLU | E | 153 | 32.193 | 132.527 | 54.150 | 1.00 | 0.00 | AAAA |
| ATOM | 2466 | CA | GLU | E | 153 | 33.109 | 133.558 | 53.729 | 1.00 | 0.00 | AAAA |
| ATOM | 2468 | CB | GLU | E | 153 | 32.257 | 134.804 | 53.507 | 1.00 | 0.00 | AAAA |
| ATOM | 2471 | CG | GLU | E | 153 | 33.080 | 135.962 | 52.954 | 1.00 | 0.00 | AAAA |
| ATOM | 2474 | CD | GLU | E | 153 | 32.755 | 137.328 | 53.549 | 1.00 | 0.00 | AAAA |
| ATOM | 2475 | OE1 | GLU | E | 153 | 31.568 | 137.706 | 53.695 | 1.00 | 0.00 | AAAA |
| ATOM | 2476 | OE2 | GLU | E | 153 | 33.721 | 138.076 | 53.833 | 1.00 | 0.00 | AAAA |
| ATOM | 2477 | C | GLU | E | 153 | 34.179 | 133.738 | 54.798 | 1.00 | 0.00 | AAAA |
| ATOM | 2478 | O | GLU | E | 153 | 33.992 | 134.548 | 55.711 | 1.00 | 0.00 | AAAA |
| ATOM | 2479 | N | GLU | E | 154 | 35.280 | 133.006 | 54.619 | 1.00 | 0.00 | AAAA |
| ATOM | 2481 | CA | GLU | E | 154 | 36.487 | 132.974 | 55.418 | 1.00 | 0.00 | AAAA |
| ATOM | 2483 | CB | GLU | E | 154 | 36.409 | 131.779 | 56.362 | 1.00 | 0.00 | AAAA |
| ATOM | 2486 | CG | GLU | E | 154 | 37.351 | 131.836 | 57.575 | 1.00 | 0.00 | AAAA |
| ATOM | 2489 | CD | GLU | E | 154 | 37.511 | 130.471 | 58.231 | 1.00 | 0.00 | AAAA |
| ATOM | 2490 | OE1 | GLU | E | 154 | 38.698 | 130.171 | 58.465 | 1.00 | 0.00 | AAAA |
| ATOM | 2491 | OE2 | GLU | E | 154 | 36.569 | 129.654 | 58.255 | 1.00 | 0.00 | AAAA |
| ATOM | 2492 | C | GLU | E | 154 | 37.645 | 133:136 | 54.434 | 1.00 | 0.00 | AAAA |
| ATOM | 2493 | O | GLU | E | 154 | 38.771 | 133.432 | 54.829 | 1.00 | 0.00 | AAAA |
| ATOM | 2494 | N | CYS | E | 155 | 37.403 | 132.971 | 53.138 | 1.00 | 0.00 | AAAA |
| ATOM | 2496 | CA | CYS | E | 155 | 38.240 | 133.445 | 52.051 | 1.00 | 0.00 | AAAA |
| ATOM | 2498 | CB | CYS | E | 155 | 37.948 | 132.560 | 50.841 | 1.00 | 0.00 | AAAA |
| ATOM | 2501 | SG | CYS | E | 155 | 38.996 | 132.899 | 49.408 | 1.00 | 0.00 | AAAA |
| ATOM | 2502 | C | CYS | E | 155 | 37.805 | 134.861 | 51.683 | 1.00 | 0.00 | AAAA |
| ATOM | 2503 | O | CYS | E | 155 | 36.626 | 135.180 | 51.838 | 1.00 | 0.00 | AAAA |
| ATOM | 2504 | N | GLY | E | 156 | 38.714 | 135.788 | 51.374 | 1.00 | 0.00 | AAAA |
| ATOM | 2506 | CA | GLY | E | 156 | 38.512 | 137.189 | 51.057 | 1.00 | 0.00 | AAAA |
| ATOM | 2509 | C | GLY | E | 156 | 39.044 | 137.428 | 49.651 | 1.00 | 0.00 | AAAA |
| ATOM | 2510 | O | GLY | E | 156 | 38.868 | 136.650 | 48.717 | 1.00 | 0.00 | AAAA |
| ATOM | 2511 | N | ASP | E | 157 | 39.769 | 138.529 | 49.441 | 1.00 | 0.00 | AAAA |
| ATOM | 2513 | CA | ASP | E | 157 | 40.464 | 138.857 | 48.209 | 1.00 | 0.00 | AAAA |
| ATOM | 2515 | CB | ASP | E | 157 | 40.877 | 140.322 | 48.128 | 1.00 | 0.00 | AAAA |
| ATOM | 2518 | CG | ASP | E | 157 | 41.487 | 140.723 | 46.797 | 1.00 | 0.00 | AAAA |
| ATOM | 2519 | OD1 | ASP | E | 157 | 40.933 | 140.481 | 45.706 | 1.00 | 0.00 | AAAA |
| ATOM | 2520 | OD2 | ASP | E | 157 | 42.515 | 141.440 | 46.893 | 1.00 | 0.00 | AAAA |
| ATOM | 2521 | C | ASP | E | 157 | 41.634 | 137.938 | 47.863 | 1.00 | 0.00 | AAAA |
| ATOM | 2522 | O | ASP | E | 157 | 42.322 | 137.482 | 48.778 | 1.00 | 0.00 | AAAA |
| ATOM | 2523 | N | ILE | E | 158 | 41.741 | 137.467 | 46.618 | 1.00 | 0.00 | AAAA |
| ATOM | 2525 | CA | ILE | E | 158 | 42.593 | 136.390 | 46.152 | 1.00 | 0.00 | AAAA |
| ATOM | 2527 | CB | ILE | E | 158 | 41.916 | 135.025 | 46.220 | 1.00 | 0.00 | AAAA |
| ATOM | 2529 | CG1 | ILE | E | 158 | 40.572 | 135.022 | 45.489 | 1.00 | 0.00 | AAAA |
| ATOM | 2532 | CG2 | ILE | E | 158 | 41.699 | 134.620 | 47.673 | 1.00 | 0.00 | AAAA |
| ATOM | 2536 | CD1 | ILE | E | 158 | 39.860 | 133.665 | 45.561 | 1.00 | 0.00 | AAAA |
| ATOM | 2540 | C | ILE | E | 158 | 43.170 | 136.823 | 44.817 | 1.00 | 0.00 | AAAA |
| ATOM | 2541 | O | ILE | E | 158 | 43.968 | 136.048 | 44.285 | 1.00 | 0.00 | AAAA |
| ATOM | 2542 | N | CYS | E | 159 | 42.746 | 137.907 | 44.162 | 1.00 | 0.00 | AAAA |
| ATOM | 2544 | CA | CYS | E | 159 | 43.154 | 138.321 | 42.832 | 1.00 | 0.00 | AAAA |
| ATOM | 2546 | CB | CYS | E | 159 | 42.028 | 137.945 | 41.871 | 1.00 | 0.00 | AAAA |
| ATOM | 2549 | SG | CYS | E | 159 | 42.445 | 138.315 | 40.147 | 1.00 | 0.00 | AAAA |
| ATOM | 2550 | C | CYS | E | 159 | 43.590 | 139.770 | 42.901 | 1.00 | 0.00 | AAAA |
| ATOM | 2551 | O | CYS | E | 159 | 42.720 | 140.639 | 42.776 | 1.00 | 0.00 | AAAA |
| ATOM | 2552 | N | PRO | E | 160 | 44.847 | 140.115 | 43.180 | 1.00 | 0.00 | AAAA |
| ATOM | 2553 | CA | PRO | E | 160 | 45.374 | 141.458 | 43.150 | 1.00 | 0.00 | AAAA |
| ATOM | 2555 | CB | PRO | E | 160 | 46.568 | 141.378 | 44.098 | 1.00 | 0.00 | AAAA |
| ATOM | 2558 | CG | PRO | E | 160 | 47.093 | 139.944 | 44.052 | 1.00 | 0.00 | AAAA |
| ATOM | 2561 | CD | PRO | E | 160 | 45.834 | 139.178 | 43.666 | 1.00 | 0.00 | AAAA |
| ATOM | 2564 | C | PRO | E | 160 | 45.875 | 141.899 | 41.784 | 1.00 | 0.00 | AAAA |
| ATOM | 2565 | O | PRO | E | 160 | 45.890 | 143.102 | 41.535 | 1.00 | 0.00 | AAAA |
| ATOM | 2566 | N | GLY | E | 161 | 46.358 | 140.974 | 40.948 | 1.00 | 0.00 | AAAA |
| ATOM | 2568 | CA | GLY | E | 161 | 46.909 | 141.298 | 39.643 | 1.00 | 0.00 | AAAA |
| ATOM | 2571 | C | GLY | E | 161 | 48.422 | 141.162 | 39.690 | 1.00 | 0.00 | AAAA |
| ATOM | 2572 | O | GLY | E | 161 | 49.013 | 140.374 | 40.429 | 1.00 | 0.00 | AAAA |
| ATOM | 2573 | N | THR | E | 162 | 49.155 | 141.863 | 38.824 | 1.00 | 0.00 | AAAA |
| ATOM | 2575 | CA | THR | E | 162 | 50.541 | 141.582 | 38.514 | 1.00 | 0.00 | AAAA |
| ATOM | 2577 | CB | THR | E | 162 | 50.539 | 141.521 | 36.985 | 1.00 | 0.00 | AAAA |
| ATOM | 2579 | OG1 | THR | E | 162 | 49.939 | 142.609 | 36.298 | 1.00 | 0.00 | AAAA |
| ATOM | 2581 | CG2 | THR | E | 162 | 49.917 | 140.258 | 36.389 | 1.00 | 0.00 | AAAA |
| ATOM | 2585 | C | THR | E | 162 | 51.436 | 142.628 | 39.150 | 1.00 | 0.00 | AAAA |
| ATOM | 2586 | O | THR | E | 162 | 52.657 | 142.604 | 38.971 | 1.00 | 0.00 | AAAA |
| ATOM | 2587 | N | ALA | E | 163 | 50.873 | 143.615 | 39.843 | 1.00 | 0.00 | AAAA |
| ATOM | 2589 | CA | ALA | E | 163 | 51.590 | 144.802 | 40.265 | 1.00 | 0.00 | AAAA |
| ATOM | .2591 | CB | ALA | E | 163 | 51.562 | 145.848 | 39.150 | 1.00 | 0.00 | AAAA |
| ATOM | 2595 | C | ALA | E | 163 | 51.288 | 145.281 | 41.678 | 1.00 | 0.00 | AAAA |
| ATOM | 2596 | O | ALA | E | 163 | 51.622 | 146.423 | 41.989 | 1.00 | 0.00 | AAAA |
| ATOM | 2597 | N | LYS | E | 164 | 50.581 | 144.570 | 42.557 | 1.00 | 0.00 | AAAA |
| ATOM | 2599 | CA | LYS | E | 164 | 50.248 | 144.806 | 43.947 | 1.00 | 0.00 | AAAA |
| ATOM | 2601 | CB | LYS | E | 164 | 51.482 | 145.051 | 44.804 | 1.00 | 0.00 | AAAA |
| ATOM | 2604 | CG | LYS | E | 164 | 52.355 | 143.818 | 45.040 | 1.00 | 0.00 | AAAA |
| ATOM | 2607 | CD | LYS | E | 164 | 51.741 | 142.526 | 45.594 | 1.00 | 0.00 | AAAA |
| ATOM | 2610 | CE | LYS | E | 164 | 52.781 | 141.528 | 46.093 | 1.00 | 0.00 | AAAA |
| ATOM | 2613 | NZ | LYS | E | 164 | 52.107 | 140.282 | 46.501 | 1.00 | 0.00 | AAAA |
| ATOM | 2617 | C | LYS | E | 164 | 49.033 | 145.708 | 44.090 | 1.00 | 0.00 | AAAA |
| ATOM | 2618 | O | LYS | E | 164 | 48.930 | 146.326 | 45.146 | 1.00 | 0.00 | AAAA |
| ATOM | 2619 | N | GLY | E | 165 | 48.171 | 145.777 | 43.078 | 1.00 | 0.00 | AAAA |
| ATOM | 2621 | CA | GLY | E | 165 | 47.054 | 146.689 | 42.931 | 1.00 | 0.00 | AAAA |
| ATOM | 2624 | C | GLY | E | 165 | 45.752 | 145.916 | 43.123 | 1.00 | 0.00 | AAAA |
| ATOM | 2625 | O | GLY | E | 165 | 45.616 | 145.041 | 43.978 | 1.00 | 0.00 | AAAA |
| ATOM | 2626 | N | LYS | E | 166 | 44.783 | 146.164 | 42.231 | 1.00 | 0.00 | AAAA |
| ATOM | 2628 | CA | LYS | E | 166 | 43.646 | 145.291 | 42.032 | 1.00 | 0.00 | AAAA |
| ATOM | 2630 | CB | LYS | E | 166 | 42.508 | 145.611 | 43.001 | 1.00 | 0.00 | AAAA |
| ATOM | 2633 | CG | LYS | E | 166 | 41.200 | 144.843 | 42.865 | 1.00 | 0.00 | AAAA |
| ATOM | 2636 | CD | LYS | E | 166 | 41.329 | 143.362 | 43.235 | 1.00 | 0.00 | AAAA |
| ATOM | 2639 | CE | LYS | E | 166 | 39.944 | 142.719 | 43.231 | 1.00 | 0.00 | AAAA |
| ATOM | 2642 | NZ | LYS | E | 166 | 39.955 | 141.280 | 43.491 | 1.00 | 0.00 | AAAA |
| ATOM | 2646 | C | LYS | E | 166 | 43.226 | 145.234 | 40.564 | 1.00 | 0.00 | AAAA |
| ATOM | 2647 | O | LYS | E | 166 | 43.242 | 144.173 | 39.953 | 1.00 | 0.00 | AAAA |
| ATOM | 2648 | N | THR | E | 167 | 42.789 | 146.375 | 40.024 | 1.00 | 0.00 | AAAA |
| ATOM | 2650 | CA | THR | E | 167 | 42.150 | 146.354 | 38.724 | 1.00 | 0.00 | AAAA |
| ATOM | 2652 | CB | THR | E | 167 | 41.264 | 147.592 | 38.562 | 1.00 | 0.00 | AAAA |
| ATOM | 2654 | OG1 | THR | E | 167 | 42.020 | 148.748 | 38.864 | 1.00 | 0.00 | AAAA |
| ATOM | 2656 | CG2 | THR | E | 167 | 40.055 | 147.412 | 39.467 | 1.00 | 0.00 | AAAA |
| ATOM | 2660 | C | THR | E | 167 | 43.269 | 146.259 | 37.700 | 1.00 | 0.00 | AAAA |
| ATOM | 2661 | O | THR | E | 167 | 44.179 | 147.097 | 37.636 | 1.00 | 0.00 | AAAA |
| ATOM | 2662 | N | ASN | E | 168 | 43.233 | 145.222 | 36.864 | 1.00 | 0.00 | AAAA |
| ATOM | 2664 | CA | ASN | E | 168 | 44.414 | 144.814 | 36.131 | 1.00 | 0.00 | AAAA |
| ATOM | 2666 | CB | ASN | E | 168 | 45.271 | 143.816 | 36.930 | 1.00 | 0.00 | AAAA |
| ATOM | 2669 | CG | ASN | E | 168 | 46.754 | 143.679 | 36.622 | 1.00 | 0.00 | AAAA |
| ATOM | 2670 | OD1 | ASN | E | 168 | 47.158 | 142.647 | 36.100 | 1.00 | 0.00 | AAAA |
| ATOM | 2671 | ND2 | ASN | E | 168 | 47.605 | 144.678 | 36.891 | 1.00 | 0.00 | AAAA |
| ATOM | 2674 | C | ASN | E | 168 | 44.257 | 144.340 | 34.698 | 1.00 | 0.00 | AAAA |
| ATOM | 2675 | O | ASN | E | 168 | 44.903 | 144.862 | 33.784 | 1.00 | 0.00 | AAAA |
| ATOM | 2676 | N | CYS | E | 169 | 43.237 | 143.517 | 34.432 | 1.00 | 0.00 | AAAA |
| ATOM | 2678 | CA | CYS | E | 169 | 42.815 | 142.978 | 33.151 | 1.00 | 0.00 | AAAA |
| ATOM | 2680 | CB | CYS | E | 169 | 42.372 | 141.543 | 33.428 | 1.00 | 0.00 | AAAA |
| ATOM | 2683 | SG | CYS | E | 169 | 41.980 | 140.448 | 32.031 | 1.00 | 0.00 | AAAA |
| ATOM | 2684 | C | CYS | E | 169 | 41.609 | 143.694 | 32.559 | 1.00 | 0.00 | AAAA |
| ATOM | 2685 | O | CYS | E | 169 | 40.769 | 144.047 | 33.379 | 1.00 | 0.00 | AAAA |
| ATOM | 2686 | N | PRO | E | 170 | 41.460 | 143.913 | 31.256 | 1.00 | 0.00 | AAAA |
| ATOM | 2687 | CA | PRO | E | 170 | 40.314 | 144.596 | 30.683 | 1.00 | 0.00 | AAAA |
| ATOM | 2689 | CB | PRO | E | 170 | 40.720 | 144.883 | 29.233 | 1.00 | 0.00 | AAAA |
| ATOM | 2692 | CG | PRO | E | 170 | 41.799 | 143.855 | 28.903 | 1.00 | 0.00 | AAAA |
| ATOM | 2695 | CD | PRO | E | 170 | 42.417 | 143.492 | 30.252 | 1.00 | 0.00 | AAAA |
| ATOM | 2698 | C | PRO | E | 170 | 39.117 | 143.663 | 30.686 | 1.00 | 0.00 | AAAA |
| ATOM | 2699 | O | PRO | E | 170 | 39.171 | 142.589 | 30.084 | 1.00 | 0.00 | AAAA |
| ATOM | 2700 | N | ALA | E | 171 | 38.039 | 144.041 | 31.382 | 1.00 | 0.00 | AAAA |
| ATOM | 2702 | CA | ALA | E | 171 | 36.913 | 143.171 | 31.666 | 1.00 | 0.00 | AAAA |
| ATOM | 2704 | CB | ALA | E | 171 | 36.150 | 143.866 | 32.801 | 1.00 | 0.00 | AAAA |
| ATOM | 2708 | C | ALA | E | 171 | 36.017 | 143.093 | 30.442 | 1.00 | 0.00 | AAAA |
| ATOM | 2709 | O | ALA | E | 171 | 35.814 | 144.121 | 29.804 | 1.00 | 0.00 | AAAA |
| ATOM | 2710 | N | THR | E | 172 | 35.482 | 141.899 | 30.171 | 1.00 | 0.00 | AAAA |
| ATOM | 2712 | CA | THR | E | 172 | 34.414 | 141.599 | 29.229 | 1.00 | 0.00 | AAAA |
| ATOM | 2714 | CB | THR | E | 172 | 34.373 | 140.171 | 28.683 | 1.00 | 0.00 | AAAA |
| ATOM | 2716 | OG1 | THR | E | 172 | 34.232 | 139.164 | 29.670 | 1.00 | 0.00 | AAAA |
| ATOM | 2718 | CG2 | THR | E | 172 | 35.483 | 139.798 | 27.703 | 1.00 | 0.00 | AAAA |
| ATOM | 2722 | C | THR | E | 172 | 33.105 | 142.028 | 29.856 | 1.00 | 0.00 | AAAA |
| ATOM | 2723 | O | THR | E | 172 | 32.967 | 141.913 | 31.075 | 1.00 | 0.00 | AAAA |
| ATOM | 2724 | N | VAL | E | 173 | 32.112 | 142.345 | 29.017 | 1.00 | 0.00 | AAAA |
| ATOM | 2726 | CA | VAL | E | 173 | 30.786 | 142.711 | 29.464 | 1.00 | 0.00 | AAAA |
| ATOM | 2728 | CB | VAL | E | 173 | 30.569 | 144.191 | 29.148 | 1.00 | 0.00 | AAAA |
| ATOM | 2730 | CG1 | VAL | E | 173 | 30.498 | 144.665 | 27.699 | 1.00 | 0.00 | AAAA |
| ATOM | 2734 | CG2 | VAL | E | 173 | 29.421 | 144.851 | 29.906 | 1.00 | 0.00 | AAAA |
| ATOM | 2738 | C | VAL | E | 173 | 29.669 | 141.757 | 29.070 | 1.00 | 0.00 | AAAA |
| ATOM | 2739 | O | VAL | E | 173 | 28.509 | 142.101 | 28.844 | 1.00 | 0.00 | AAAA |
| ATOM | 2740 | N | ILE | E | 174 | 30.014 | 140.470 | 28.983 | 1.00 | 0.00 | AAAA |
| ATOM | 2742 | CA | ILE | E | 174 | 29.086 | 139.458 | 28.528 | 1.00 | 0.00 | AAAA |
| ATOM | 2744 | CB | ILE | E | 174 | 30.039 | 138.314 | 28.205 | 1.00 | 0.00 | AAAA |
| ATOM | 2746 | CG1 | ILE | E | 174 | 31.077 | 138.742 | 27.160 | 1.00 | 0.00 | AAAA |
| ATOM | 2749 | CG2 | ILE | E | 174 | 29.385 | 136.960 | 27.893 | 1.00 | 0.00 | AAAA |
| ATOM | 2753 | CD1 | ILE | E | 174 | 32.105 | 137.760 | 26.597 | 1.00 | 0.00 | AAAA |
| ATOM | 2757 | C | ILE | E | 174 | 28.078 | 139.137 | 29.626 | 1.00 | 0.00 | AAAA |
| ATOM | 2758 | O | ILE | E | 174 | 26.945 | 138.834 | 29.262 | 1.00 | 0.00 | AAAA |
| ATOM | 2759 | N | ASN | E | 175 | 28.460 | 139.205 | 30.900 | 1.00 | 0.00 | AAAA |
| ATOM | 2761 | CA | ASN | E | 175 | 27.657 | 138.922 | 32.075 | 1.00 | 0.00 | AAAA |
| ATOM | 2763 | CB | ASN | E | 175 | 28.645 | 138.861 | 33.242 | 1.00 | 0.00 | AAAA |
| ATOM | 2766 | CG | ASN | E | 175 | 28.121 | 138.132 | 34.469 | 1.00 | 0.00 | AAAA |
| ATOM | 2767 | OD1 | ASN | E | 175 | 28.205 | 138.689 | 35.557 | 1.00 | 0.00 | AAAA |
| ATOM | 2768 | ND2 | ASN | E | 175 | 27.709 | 136.866 | 34.296 | 1.00 | 0.00 | AAAA |
| ATOM | 2771 | C | ASN | E | 175 | 26.610 | 140.008 | 32.278 | 1.00 | 0.00 | AAAA |
| ATOM | 2772 | O | ASN | E | 175 | 25.575 | 139.731 | 32.890 | 1.00 | 0.00 | AAAA |
| ATOM | 2773 | N | GLY | E | 176 | 26.741 | 141.169 | 31.639 | 1.00 | 0.00 | AAAA |
| ATOM | 2775 | CA | GLY | E | 176 | 25.775 | 142.240 | 31.540 | 1.00 | 0.00 | AAAA |
| ATOM | 2778 | C | GLY | E | 176 | 26.191 | 143.472 | 32.339 | 1.00 | 0.00 | AAAA |
| ATOM | 2779 | O | GLY | E | 176 | 25.956 | 144.623 | 31.951 | 1.00 | 0.00 | AAAA |
| ATOM | 2780 | N | GLN | E | 177 | 26.842 | 143.234 | 33.477 | 1.00 | 0.00 | AAAA |
| ATOM | 2782 | CA | GLN | E | 177 | 27.762 | 144.157 | 34.103 | 1.00 | 0.00 | AAAA |
| ATOM | 2784 | CB | GLN | E | 177 | 27.670 | 143.948 | 35.616 | 1.00 | 0.00 | AAAA |
| ATOM | 2787 | CG | GLN | E | 177 | 27.934 | 142.555 | 36.189 | 1.00 | 0.00 | AAAA |
| ATOM | 2790 | CD | GLN | E | 177 | 27.605 | 142.297 | 37.653 | 1.00 | 0.00 | AAAA |
| ATOM | 2791 | OE1 | GLN | E | 177 | 27.631 | 141.181 | 38.167 | 1.00 | 0.00 | AAAA |
| ATOM | 2792 | NE2 | GLN | E | 177 | 27.298 | 143.383 | 38.354 | 1.00 | 0.00 | AAAA |
| ATOM | 2795 | C | GLN | E | 177 | 29.148 | 143.821 | 33.566 | 1.00 | 0.00 | AAAA |
| ATOM | 2796 | O | GLN | E | 177 | 29.417 | 142.751 | 33.042 | 1.00 | 0.00 | AAAA |
| ATOM | 2797 | N | PHE | E | 178 | 30.068 | 144.780 | 33.673 | 1.00 | 0.00 | AAAA |
| ATOM | 2799 | CA | PHE | E | 178 | 31.495 | 144.613 | 33.492 | 1.00 | 0.00 | AAAA |
| ATOM | 2801 | CB | PHE | E | 178 | 32.277 | 145.935 | 33.514 | 1.00 | 0.00 | AAAA |
| ATOM | 2804 | CG | PHE | E | 178 | 32.057 | 146.694 | 32.233 | 1.00 | 0.00 | AAAA |
| ATOM | 2805 | CD1 | PHE | E | 178 | 30.944 | 147.521 | 32.078 | 1.00 | 0.00 | AAAA |
| ATOM | 2807 | CD2 | PHE | E | 178 | 32.897 | 146.464 | 31.124 | 1.00 | 0.00 | AAAA |
| ATOM | 2809 | CE1 | PHE | E | 178 | 30.639 | 148.060 | 30.826 | 1.00 | 0.00 | AAAA |
| ATOM | 2811 | CE2 | PHE | E | 178 | 32.594 | 147.012 | 29.875 | 1.00 | 0.00 | AAAA |
| ATOM | 2813 | CZ | PHE | E | 178 | 31.452 | 147.817 | 29.713 | 1.00 | 0.00 | AAAA |
| ATOM | 2815 | C | PHE | E | 178 | 31.927 | 143.849 | 34.734 | 1.00 | 0.00 | AAAA |
| ATOM | 2816 | O | PHE | E | 178 | 31.761 | 144.293 | 35.861 | 1.00 | 0.00 | AAAA |
| ATOM | 2817 | N | VAL | E | 179 | 32.661 | 142.740 | 34.579 | 1.00 | 0.00 | AAAA |
| ATOM | 2819 | CA | VAL | E | 179 | 33.236 | 141.987 | 35.673 | 1.00 | 0.00 | AAAA |
| ATOM | 2821 | CB | VAL | E | 179 | 32.289 | 140.912 | 36.209 | 1.00 | 0.00 | AAAA |
| ATOM | 2823 | CG1 | VAL | E | 179 | 31.922 | 139.835 | 35.190 | 1.00 | 0.00 | AAAA |
| ATOM | 2827 | CG2 | VAL | E | 179 | 32.800 | 140.399 | 37.549 | 1.00 | 0.00 | AAAA |
| ATOM | 2831 | C | VAL | E | 179 | 34.603 | 141.486 | 35.245 | 1.00 | 0.00 | AAAA |
| ATOM | 2832 | O | VAL | E | 179 | 34.757 | 140.690 | 34.314 | 1.00 | 0.00 | AAAA |
| ATOM | 2833 | N | GLU | E | 180 | 35.626 | 141.868 | 36.019 | 1.00 | 0.00 | AAAA |
| ATOM | 2835 | CA | GLU | E | 180 | 36.987 | 141.338 | 36.014 | 1.00 | 0.00 | AAAA |
| ATOM | 2837 | CB | GLU | E | 180 | 37.791 | 142.475 | 36.643 | 1.00 | 0.00 | AAAA |
| ATOM | 2840 | CG | GLU | E | 180 | 39.261 | 142.294 | 36.277 | 1.00 | 0.00 | AAAA |
| ATOM | 2843 | CD | GLU | E | 180 | 40.171 | 143.391 | 36.789 | 1.00 | 0.00 | AAAA |
| ATOM | 2844 | OE1 | GLU | E | 180 | 39.661 | 144.444 | 37.229 | 1.00 | 0.00 | AAAA |
| ATOM | 2845 | OE2 | GLU | E | 180 | 41.409 | 143.245 | 36.686 | 1.00 | 0.00 | AAAA |
| ATOM | 2846 | C | GLU | E | 180 | 36.928 | 139.974 | 36.694 | 1.00 | 0.00 | AAAA |
| ATOM | 2847 | O | GLU | E | 180 | 36.407 | 139.900 | 37.801 | 1.00 | 0.00 | AAAA |
| ATOM | 2848 | N | ARG | E | 181 | 37.378 | 138.916 | 36.021 | 1.00 | 0.00 | AAAA |
| ATOM | 2850 | CA | ARG | E | 181 | 37.328 | 137.510 | 36.377 | 1.00 | 0.00 | AAAA |
| ATOM | 2852 | CB | ARG | E | 181 | 36.518 | 136.692 | 35.375 | 1.00 | 0.00 | AAAA |
| ATOM | 2855 | CG | ARG | E | 181 | 35.005 | 136.910 | 35.303 | 1.00 | 0.00 | AAAA |
| ATOM | 2858 | CD | ARG | E | 181 | 34.091 | 136.629 | 36.507 | 1.00 | 0.00 | AAAA |
| ATOM | 2861 | NE | ARG | E | 181 | 32.679 | 136.623 | 36.131 | 1.00 | 0.00 | AAAA |
| ATOM | 2863 | CZ | ARG | E | 181 | 31.655 | 136.372 | 36.950 | 1.00 | 0.00 | AAAA |
| ATOM | 2864 | NH1 | ARG | E | 181 | 31.774 | 136.198 | 38.275 | 1.00 | 0.00 | AAAA |
| ATOM | 2867 | NH2 | ARG | E | 181 | 30.451 | 136.288 | 36.372 | 1.00 | 0.00 | AAAA |
| ATOM | 2870 | C | ARG | E | 181 | 38.751 | 136.968 | 36.314 | 1.00 | 0.00 | AAAA |
| ATOM | 2871 | O | ARG | E | 181 | 39.518 | 137.331 | 35.429 | 1.00 | 0.00 | AAAA |
| ATOM | 2872 | N | CYS | E | 182 | 39.100 | 136.068 | 37.237 | 1.00 | 0.00 | AAAA |
| ATOM | 2874 | CA | CYS | E | 182 | 40.424 | 135.477 | 37.296 | 1.00 | 0.00 | AAAA |
| ATOM | 2876 | CB | CYS | E | 182 | 41.464 | 136.379 | 37.954 | 1.00 | 0.00 | AAAA |
| ATOM | 2879 | SG | CYS | E | 182 | 40.840 | 137.622 | 39.105 | 1.00 | 0.00 | AAAA |
| ATOM | 2880 | C | CYS | E | 182 | 40.327 | 134.041 | 37.808 | 1.00 | 0.00 | AAAA |
| ATOM | 2881 | O | CYS | E | 182 | 39.448 | 133.725 | 38.598 | 1.00 | 0.00 | AAAA |
| ATOM | 2882 | N | TRP | E | 183 | 41.120 | 133.179 | 37.169 | 1.00 | 0.00 | AAAA |
| ATOM | 2884 | CA | TRP | E | 183 | 41.028 | 131.733 | 37.322 | 1.00 | 0.00 | AAAA |
| ATOM | 2886 | CB | TRP | E | 183 | 41.363 | 131.158 | 35.953 | 1.00 | 0.00 | AAAA |
| ATOM | 2889 | CG | TRP | E | 183 | 40.341 | 130.256 | 35.352 | 1.00 | 0.00 | AAAA |
| ATOM | 2890 | CD1 | TRP | E | 183 | 39.757 | 130.563 | 34.168 | 1.00 | 0.00 | AAAA |
| ATOM | 2892 | CD2 | TRP | E | 183 | 39.914 | 128.894 | 35.658 | 1.00 | 0.00 | AAAA |
| ATOM | 2893 | NE1 | TRP | E | 183 | 39.039 | 129.472 | 33.709 | 1.00 | 0.00 | AAAA |
| ATOM | 2895 | CE2 | TRP | E | 183 | 39.072 | 128.438 | 34.620 | 1.00 | 0.00 | AAAA |
| ATOM | 2896 | CE3 | TRP | E | 183 | 40.214 | 128.005 | 36.705 | 1.00 | 0.00 | AAAA |
| ATOM | 2898 | CZ2 | TRP | E | 183 | 38.511 | 127.154 | 34.625 | 1.00 | 0.00 | AAAA |
| ATOM | 2900 | CZ3 | TRP | E | 183 | 39.600 | 126.749 | 36.740 | 1.00 | 0.00 | AAAA |
| ATOM | 2902 | CH2 | TRP | E | 183 | 38.729 | 126.324 | 35.736 | 1.00 | 0.00 | AAAA |
| ATOM | 2904 | C | TRP | E | 183 | 41.946 | 131.208 | 38.408 | 1.00 | 0.00 | AAAA |
| ATOM | 2905 | O | TRP | E | 183 | 41.744 | 130.073 | 38.835 | 1.00 | 0.00 | AAAA |
| ATOM | 2906 | N | THR | E | 184 | 42.898 | 132.088 | 38.734 | 1.00 | 0.00 | AAAA |
| ATOM | 2908 | CA | THR | E | 184 | 43.960 | 132.119 | 39.723 | 1.00 | 0.00 | AAAA |
| ATOM | 2910 | CB | THR | E | 184 | 44.985 | 131.004 | 39.492 | 1.00 | 0.00 | AAAA |
| ATOM | 2912 | OG1 | THR | E | 184 | 45.772 | 130.821 | 40.645 | 1.00 | 0.00 | AAAA |
| ATOM | 2914 | CG2 | THR | E | 184 | 45.884 | 131.080 | 38.267 | 1.00 | 0.00 | AAAA |
| ATOM | 2918 | C | THR | E | 184 | 44.481 | 133.543 | 39.838 | 1.00 | 0.00 | AAAA |
| ATOM | 2919 | O | THR | E | 184 | 44.059 | 134.418 | 39.079 | 1.00 | 0.00 | AAAA |
| ATOM | 2920 | N | HIS | E | 185 | 45.425 | 133.732 | 40.765 | 1.00 | 0.00 | AAAA |
| ATOM | 2922 | CA | HIS | E | 185 | 45.932 | 134.952 | 41.360 | 1.00 | 0.00 | AAAA |
| ATOM | 2924 | CB | HIS | E | 185 | 46.967 | 134.459 | 42.366 | 1.00 | 0.00 | AAAA |
| ATOM | 2927 | CG | HIS | E | 185 | 47.913 | 135.552 | 42.782 | 1.00 | 0.00 | AAAA |
| ATOM | 2928 | ND1 | HIS | E | 185 | 48.935 | 136.011 | 41.952 | 1.00 | 0.00 | AAAA |
| ATOM | 2929 | CD2 | HIS | E | 185 | 47.980 | 136.261 | 43.958 | 1.00 | 0.00 | AAAA |
| ATOM | 2931 | CE1 | HIS | E | 185 | 49.469 | 137.047 | 42.599 | 1.00 | 0.00 | AAAA |
| ATOM | 2933 | NE2 | HIS | E | 185 | 49.060 | 137.109 | 43.879 | 1.00 | 0.00 | AAAA |
| ATOM | 2935 | C | HIS | E | 185 | 46.247 | 136.046 | 40.344 | 1.00 | 0.00 | AAAA |
| ATOM | 2936 | O | HIS | E | 185 | 45.868 | 137.210 | 40.443 | 1.00 | 0.00 | AAAA |
| ATOM | 2937 | N | SER | E | 186 | 46.858 | 135.586 | 39.252 | 1.00 | 0.00 | AAAA |
| ATOM | 2939 | CA | SER | E | 186 | 47.464 | 136.374 | 38.195 | 1.00 | 0.00 | AAAA |
| ATOM | 2941 | CB | SER | E | 186 | 48.918 | 136.713 | 38.529 | 1.00 | 0.00 | AAAA |
| ATOM | 2944 | OG | SER | E | 186 | 49.739 | 135.569 | 38.570 | 1.00 | 0.00 | AAAA |
| ATOM | 2946 | C | SER | E | 186 | 47.217 | 135.878 | 36.777 | 1.00 | 0.00 | AAAA |
| ATOM | 2947 | O | SER | E | 186 | 47.949 | 136.210 | 35.845 | 1.00 | 0.00 | AAAA |
| ATOM | 2948 | N | HIS | E | 187 | 46.123 | 135.138 | 36.559 | 1.00 | 0.00 | AAAA |
| ATOM | 2950 | CA | HIS | E | 187 | 45.668 | 134.567 | 35.314 | 1.00 | 0.00 | AAAA |
| ATOM | 2952 | CB | HIS | E | 187 | 45.895 | 133.061 | 35.203 | 1.00 | 0.00 | AAAA |
| ATOM | 2955 | CG | HIS | E | 187 | 45.541 | 132.467 | 33.863 | 1.00 | 0.00 | AAAA |
| ATOM | 2956 | ND1 | HIS | E | 187 | 46.354 | 132.653 | 32.752 | 1.00 | 0.00 | AAAA |
| ATOM | 2957 | CD2 | HIS | E | 187 | 44.422 | 131.784 | 33.463 | 1.00 | 0.00 | AAAA |
| ATOM | 2959 | CE1 | HIS | E | 187 | 45.750 | 131.990 | 31.759 | 1.00 | 0.00 | AAAA |
| ATOM | 2961 | NE2 | HIS | E | 187 | 44.594 | 131.424 | 32.140 | 1.00 | 0.00 | AAAA |
| ATOM | 2963 | C | HIS | E | 187 | 44.192 | 134.950 | 35.189 | 1.00 | 0.00 | AAAA |
| ATOM | 2964 | O | HIS | E | 187 | 43.355 | 134.469 | 35.950 | 1.00 | 0.00 | AAAA |
| ATOM | 2965 | N | CYS | E | 188 | 43.979 | 136.038 | 34.459 | 1.00 | 0.00 | AAAA |
| ATOM | 2967 | CA | CYS | E | 188 | 42.696 | 136.674 | 34.196 | 1.00 | 0.00 | AAAA |
| ATOM | 2969 | CB | CYS | E | 188 | 42.871 | 138.091 | 33.635 | 1.00 | 0.00 | AAAA |
| ATOM | 2972 | SG | CYS | E | 188 | 41.319 | 138.869 | 33.129 | 1.00 | 0.00 | AAAA |
| ATOM | 2973 | C | CYS | E | 188 | 42.019 | 135.715 | 33.219 | 1.00 | 0.00 | AAAA |
| ATOM | 2974 | O | CYS | E | 188 | 42.717 | 135:287 | 32.309 | 1.00 | 0.00 | AAAA |
| ATOM | 2975 | N | GLN | E | 189 | 40.747 | 135.360 | 33.419 | 1.00 | 0.00 | AAAA |
| ATOM | 2977 | CA | GLN | E | 189 | 39.894 | 134.478 | 32.651 | 1.00 | 0.00 | AAAA |
| ATOM | 2979 | CB | GLN | E | 189 | 38.645 | 134.084 | 33.439 | 1.00 | 0.00 | AAAA |
| ATOM | 2982 | CG | GLN | E | 189 | 37.675 | 133.148 | 32.714 | 1.00 | 0.00 | AAAA |
| ATOM | 2985 | CD | GLN | E | 189 | 36.411 | 132.753 | 33.482 | 1.00 | 0.00 | AAAA |
| ATOM | 2986 | OE1 | GLN | E | 189 | 35.815 | 133.701 | 34.000 | 1.00 | 0.00 | AAAA |
| ATOM | 2987 | NE2 | GLN | E | 189 | 36.016 | 131.487 | 33.643 | 1.00 | 0.00 | AAAA |
| ATOM | 2990 | C | GLN | E | 189 | 39.371 | 135.264 | 31.464 | 1.00 | 0.00 | AAAA |
| ATOM | 2991 | O | GLN | E | 189 | 38.775 | 136.354 | 31.662 | 1.00 | 0.00 | AAAA |
| ATOM | 2992 | OXT | GLN | E | 189 | 39.362 | 134.792 | 30.302 | 1.00 | 0.00 | AAAA |
| ATOM | 2993 | N | ALA | E | 681 | 16.135 | 118.091 | 43.243 | 1.00 | 0.00 | AAAB |
| ATOM | 2996 | CA | ALA | E | 681 | 17.074 | 117.185 | 42.614 | 1.00 | 0.00 | AAAB |
| ATOM | 2998 | CB | ALA | E | 681 | 18.474 | 117.804 | 42.728 | 1.00 | 0.00 | AAAB |
| ATOM | 3002 | C | ALA | E | 681 | 16.984 | 115.776 | 43.179 | 1.00 | 0.00 | AAAB |
| ATOM | 3003 | O | ALA | E | 681 | 17.695 | 114.873 | 42.732 | 1.00 | 0.00 | AAAB |
| ATOM | 3004 | N | GLU | E | 682 | 16.128 | 115.578 | 44.182 | 1.00 | 0.00 | AAAB |
| ATOM | 3006 | CA | GLU | E | 682 | 15.890 | 114.440 | 45.040 | 1.00 | 0.00 | AAAB |
| ATOM | 3008 | CB | GLU | E | 682 | 14.506 | 114.475 | 45.695 | 1.00 | 0.00 | AAAB |
| ATOM | 3011 | CG | GLU | E | 682 | 14.464 | 113.595 | 46.940 | 1.00 | 0.00 | AAAB |
| ATOM | 3014 | CD | GLU | E | 682 | 13.532 | 114.268 | 47.941 | 1.00 | 0.00 | AAAB |
| ATOM | 3015 | OE1 | GLU | E | 682 | 13.830 | 114.512 | 49.127 | 1.00 | 0.00 | AAAB |
| ATOM | 3016 | OE2 | GLU | E | 682 | 12.368 | 114.549 | 47.556 | 1.00 | 0.00 | AAAB |
| ATOM | 3017 | C | GLU | E | 682 | 16.220 | 113.108 | 44.394 | 1.00 | 0.00 | AAAB |
| ATOM | 3018 | O | GLU | E | 682 | 17.141 | 112.395 | 44.799 | 1.00 | 0.00 | AAAB |
| ATOM | 3019 | N | LYS | E | 683 | 15.417 | 112.627 | 43.446 | 1.00 | 0.00 | AAAB |
| ATOM | 3021 | CA | LYS | E | 683 | 15.635 | 111.467 | 42.604 | 1.00 | 0.00 | AAAB |
| ATOM | 3023 | CB | LYS | E | 683 | 14.535 | 111.432 | 41.546 | 1.00 | 0.00 | AAAB |
| ATOM | 3026 | CG | LYS | E | 683 | 14.569 | 112.465 | 40.419 | 1.00 | 0.00 | AAAB |
| ATOM | 3029 | CD | LYS | E | 683 | 15.330 | 111.920 | 39.206 | 1.00 | 0.00 | AAAB |
| ATOM | 3032 | CE | LYS | E | 683 | 15.887 | 113.022 | 38.308 | 1.00 | 0.00 | AAAB |
| ATOM | 3035 | NZ | LYS | E | 683 | 16.835 | 112.652 | 37.247 | 1.00 | 0.00 | AAAB |
| ATOM | 3039 | C | LYS | E | 683 | 17.007 | 111.258 | 41.968 | 1.00 | 0.00 | AAAB |
| ATOM | 3040 | O | LYS | E | 683 | 17.333 | 110.162 | 41.521 | 1.00 | 0.00 | AAAB |
| ATOM | 3041 | N | GLU | E | 684 | 17.888 | 112.258 | 41.930 | 1.00 | 0.00 | AAAB |
| ATOM | 3043 | CA | GLU | E | 684 | 19.211 | 112.176 | 41.351 | 1.00 | 0.00 | AAAB |
| ATOM | 3045 | CB | GLU | E | 684 | 19.482 | 113.413 | 40.488 | 1.00 | 0.00 | AAAB |
| ATOM | 3048 | CG | GLU | E | 684 | 20.586 | 113.185 | 39.452 | 1.00 | 0.00 | AAAB |
| ATOM | 3051 | CD | GLU | E | 684 | 20.100 | 112.373 | 38.255 | 1.00 | 0.00 | AAAB |
| ATOM | 3052 | OE1 | GLU | E | 684 | 19.215 | 111.490 | 38.303 | 1.00 | 0.00 | AAAB |
| ATOM | 3053 | OE2 | GLU | E | 684 | 20.813 | 112.538 | 37.246 | 1.00 | 0.00 | AAAB |
| ATOM | 3054 | C | GLU | E | 684 | 20.166 | 112.195 | 42.532 | 1.00 | 0.00 | AAAB |
| ATOM | 3055 | O | GLU | E | 684 | 21.127 | 111.434 | 42.525 | 1.00 | 0.00 | AAAB |
| ATOM | 3056 | N | GLU | E | 685 | 19.874 | 112.963 | 43.588 | 1.00 | 0.00 | AAAB |
| ATOM | 3058 | CA | GLU | E | 685 | 20.587 | 113.150 | 44.834 | 1.00 | 0.00 | AAAB |
| ATOM | 3060 | CB | GLU | E | 685 | 19.925 | 114.297 | 45.592 | 1.00 | 0.00 | AAAB |
| ATOM | 3063 | CG. | GLU | E | 685 | 20.717 | 114.795 | 46.803 | 1.00 | 0.00 | AAAB |
| ATOM | 3066 | CD | GLU | E | 685 | 21.857 | 115.750 | 46.469 | 1.00 | 0.00 | AAAB |
| ATOM | 3067 | OE1 | GLU | E | 685 | 21.890 | 116.334 | 45.370 | 1.00 | 0.00 | AAAB |
| ATOM | 3068 | OE2 | GLU | E | 685 | 22.725 | 115.898 | 47.347 | 1.00 | 0.00 | AAAB |
| ATOM | 3069 | C | GLU | E | 685 | 20.727 | 111.817 | 45.552 | 1.00 | 0.00 | AAAB |
| ATOM | 3070 | O | GLU | E | 685 | 21.763 | 111.498 | 46.126 | 1.00 | 0.00 | AAAB |
| ATOM | 3071 | N | ALA | E | 686 | 19.645 | 111.026 | 45.544 | 1.00 | 0.00 | AAAB |
| ATOM | 3073 | CA | ALA | E | 686 | 19.603 | 109.737 | 46.208 | 1.00 | 0.00 | AAAB |
| ATOM | 3075 | CB | ALA | E | 686 | 18.101 | 109.436 | 46.292 | 1.00 | 0.00 | AAAB |
| ATOM | 3079 | C | ALA | E | 686 | 20.362 | 108.671 | 45.446 | 1.00 | 0.00 | AAAB |
| ATOM | 3080 | O | ALA | E | 686 | 21.156 | 107.955 | 46.067 | 1.00 | 0.00 | AAAB |
| ATOM | 3081 | N | GLU | E | 687 | 20.175 | 108.636 | 44.127 | 1.00 | 0.00 | AAAB |
| ATOM | 3083 | CA | GLU | E | 687 | 20.934 | 107.778 | 43.237 | 1.00 | 0.00 | AAAB |
| ATOM | 3085 | CB | GLU | E | 687 | 20.424 | 107.833 | 41.800 | 1.00 | 0.00 | AAAB |
| ATOM | 3088 | CG | GLU | E | 687 | 19.006 | 107.263 | 41.646 | 1.00 | 0.00 | AAAB |
| ATOM | 3091 | CD | GLU | E | 687 | 18.840 | 105.753 | 41.680 | 1.00 | 0.00 | AAAB |
| ATOM | 3092 | OE1 | GLU | E | 687 | 17.853 | 105.214 | 42.220 | 1.00 | 0.00 | AAAB |
| ATOM | 3093 | OE2 | GLU | E | 687 | 19.742 | 105.083 | 41.127 | 1.00 | 0.00 | AAAB |
| ATOM | 3094 | C | GLU | E | 687 | 22.419 | 108.042 | 43.383 | 1.00 | 0.00 | AAAB |
| ATOM | 3095 | O | GLU | E | 687 | 23.211 | 107.107 | 43.397 | 1.00 | 0.00 | AAAB |
| ATOM | 3096 | N | TYR | E | 688 | 22.864 | 109.294 | 43.574 | 1.00 | 0.00 | AAAB |
| ATOM | 3098 | CA | TYR | E | 688 | 24.210 | 109.788 | 43.805 | 1.00 | 0.00 | AAAB |
| ATOM | 3100 | CB | TYR | E | 688 | 24.164 | 111.305 | 43.650 | 1.00 | 0.00 | AAAB |
| ATOM | 3103 | CG | TYR | E | 688 | 25.361 | 112.134 | 44.065 | 1.00 | 0.00 | AAAB |
| ATOM | 3104 | CD1 | TYR | E | 688 | 26.392 | 112.473 | 43.191 | 1.00 | 0.00 | AAAB |
| ATOM | 3106 | CD2 | TYR | E | 688 | 25.354 | 112.731 | 45.336 | 1.00 | 0.00 | AAAB |
| ATOM | 3108 | CE1 | TYR | E | 688 | 27.428 | 113.341 | 43.552 | 1.00 | 0.00 | AAAB |
| ATOM | 3110 | CE2 | TYR | E | 688 | 26.357 | 113.643 | 45.683 | 1.00 | 0.00 | AAAB |
| ATOM | 3112 | CZ | TYR | E | 688 | 27.453 | 113.869 | 44.845 | 1.00 | 0.00 | AAAB |
| ATOM | 3113 | OH | TYR | E | 688 | 28.416 | 114.772 | 45.192 | 1.00 | 0.00 | AAAB |
| ATOM | 3115 | C | TYR | E | 688 | 24.765 | 109.367 | 45.157 | 1.00 | 0.00 | AAAB |
| ATOM | 3116 | O | TYR | E | 688 | 25.914 | 108.924 | 45.167 | 1.00 | 0.00 | AAAB |
| ATOM | 3117 | N | ARG | E | 689 | 23.925 | 109.491 | 46.183 | 1.00 | 0.00 | AAAB |
| ATOM | 3119 | CA | ARG | E | 689 | 24.289 | 109.183 | 47.554 | 1.00 | 0.00 | AAAB |
| ATOM | 3121 | CB | ARG | E | 689 | 23.160 | 109.618 | 48.483 | 1.00 | 0.00 | AAAB |
| ATOM | 3124 | CG | ARG | E | 689 | 22.945 | 111.075 | 48.873 | 1.00 | 0.00 | AAAB |
| ATOM | 3127 | CD | ARG | E | 689 | 21.708 | 111.403 | 49.716 | 1.00 | 0.00 | AAAB |
| ATOM | 3130 | NE | ARG | E | 689 | 21.700 | 112.848 | 49.981 | 1.00 | 0.00 | AAAB |
| ATOM | 3132 | CZ | ARG | E | 689 | 20.728 | 113.439 | 50.694 | 1.00 | 0.00 | AAAB |
| ATOM | 3133 | NH1 | ARG | E | 689 | 19.650 | 112.705 | 51.020 | 1.00 | 0.00 | AAAB |
| ATOM | 3136 | NH2 | ARG | E | 689 | 20.826 | 114.702 | 51.115 | 1.00 | 0.00 | AAAB |
| ATOM | 3139 | C | ARG | E | 689 | 24.676 | 107.728 | 47.797 | 1.00 | 0.00 | AAAB |
| ATOM | 3140 | O | ARG | E | 689 | 25.307 | 107.383 | 48.796 | 1.00 | 0.00 | AAAB |
| ATOM | 3141 | N | LYS | E | 690 | 24.153 | 106.840 | 46.952 | 1.00 | 0.00 | AAAB |
| ATOM | 3143 | CA | LYS | E | 690 | 24.177 | 105.389 | 46.928 | 1.00 | 0.00 | AAAB |
| ATOM | 3145 | CB | LYS | E | 690 | 23.290 | 104.936 | 45.772 | 1.00 | 0.00 | AAAB |
| ATOM | 3148 | CG | LYS | E | 690 | 22.543 | 103.663 | 46.158 | 1.00 | 0.00 | AAAB |
| ATOM | 3151 | CD | LYS | E | 690 | 21.644 | 103.102 | 45.052 | 1.00 | 0.00 | AAAB |
| ATOM | 3154 | CE | LYS | E | 690 | 20.737 | 101.954 | 45.499 | 1.00 | 0.00 | AAAB |
| ATOM | 3157 | NZ | LYS | E | 690 | 19.573 | 101.760 | 44.619 | 1.00 | 0.00 | AAAB |
| ATOM | 3161 | C | LYS | E | 690 | 25.613 | 104.894 | 46.761 | 1.00 | 0.00 | AAAB |
| ATOM | 3162 | O | LYS | E | 690 | 26.134 | 104.003 | 47.437 | 1.00 | 0.00 | AAAB |
| ATOM | 3163 | N | VAL | E | 691 | 26.323 | 105.494 | 45.813 | 1.00 | 0.00 | AAAB |
| ATOM | 3165 | CA | VAL | E | 691 | 27.664 | 105.163 | 45.343 | 1.00 | 0.00 | AAAB |
| ATOM | 3167 | CB | VAL | E | 691 | 27.943 | 106.042 | 44.130 | 1.00 | 0.00 | AAAB |
| ATOM | 3169 | CG1 | VAL | E | 691 | 29.266 | 105.596 | 43.510 | 1.00 | 0.00 | AAAB |
| ATOM | 3173 | CG2 | VAL | E | 691 | 26.913 | 105.957 | 42.992 | 1.00 | 0.00 | AAAB |
| ATOM | 3177 | C | VAL | E | 691 | 28.606 | 105.566 | 46.467 | 1.00 | 0.00 | AAAB |
| ATOM | 3178 | O | VAL | E | 691 | 29.481 | 104.771 | 46.824 | 1.00 | 0.00 | AAAB |
| ATOM | 3179 | N | PHE | E | 692 | 28.475 | 106.747 | 47.072 | 1.00 | 0.00 | AAAB |
| ATOM | 3181 | CA | PHE | E | 692 | 29.420 | 107.320 | 48.009 | 1.00 | 0.00 | AAAB |
| ATOM | 3183 | CB | PHE | E | 692 | 29.175 | 108.826 | 48.132 | 1.00 | 0.00 | AAAB |
| ATOM | 3186 | CG | PHE | E | 692 | 29.728 | 109.607 | 46.962 | 1.00 | 0.00 | AAAB |
| ATOM | 3187 | CD1 | PHE | E | 692 | 31.099 | 109.872 | 46.868 | 1.00 | 0.00 | AAAB |
| ATOM | 3189 | CD2 | PHE | E | 692 | 28.901 | 110.012 | 45.904 | 1.00 | 0.00 | AAAB |
| ATOM | 3191 | CE1 | PHE | E | 692 | 31.698 | 110.521 | 45.779 | 1.00 | 0.00 | AAAB |
| ATOM | 3193 | CE2 | PHE | E | 692 | 29.483 | 110.668 | 44.806 | 1.00 | 0.00 | AAAB |
| ATOM | 3195 | CZ | PHE | E | 692 | 30.809 | 111.089 | 44.855 | 1.00 | 0.00 | AAAB |
| ATOM | 3197 | C | PHE | E | 692 | 29.381 | 106.720 | 49.403 | 1.00 | 0.00 | AAAB |
| ATOM | 3198 | O | PHE | E | 692 | 30.397 | 106.728 | 50.096 | 1.00 | 0.00 | AAAB |
| ATOM | 3199 | N | GLU | E | 693 | 28.239 | 106.104 | 49.718 | 1.00 | 0.00 | AAAB |
| ATOM | 3201 | CA | GLU | E | 693 | 28.036 | 105.277 | 50.898 | 1.00 | 0.00 | AAAB |
| ATOM | 3203 | CB | GLU | E | 693 | 26.561 | 105.214 | 51.258 | 1.00 | 0.00 | AAAB |
| ATOM | 3206 | CG | GLU | E | 693 | 26.265 | 104.606 | 52.630 | 1.00 | 0.00 | AAAB |
| ATOM | 3209 | CD | GLU | E | 693 | 24.799 | 104.656 | 53.042 | 1.00 | 0.00 | AAAB |
| ATOM | 3210 | OE1 | GLU | E | 693 | 24.489 | 104.374 | 54.223 | 1.00 | 0.00 | AAAB |
| ATOM | 3211 | OE2 | GLU | E | 693 | 23.948 | 104.880 | 52.158 | 1.00 | 0.00 | AAAB |
| ATOM | 3212 | C | GLU | E | 693 | 28.590 | 103.891 | 50.608 | 1.00 | 0.00 | AAAB |
| ATOM | 3213 | O | GLU | E | 693 | 28.984 | 103.181 | 51.532 | 1.00 | 0.00 | AAAB |
| ATOM | 3214 | N | ASN | E | 694 | 28.623 | 103.432 | 49.355 | 1.00 | 0.00 | AAAB |
| ATOM | 3216 | CA | ASN | E | 694 | 29.051 | 102.090 | 48.997 | 1.00 | 0.00 | AAAB |
| ATOM | 3218 | CB | ASN | E | 694 | 28.401 | 101.665 | 47.691 | 1.00 | 0.00 | AAAB |
| ATOM | 3221 | CG | ASN | E | 694 | 28.624 | 100.205 | 47.305 | 1.00 | 0.00 | AAAB |
| ATOM | 3222 | OD1 | ASN | E | 694 | 28.297 | 99.294 | 48.056 | 1.00 | 0.00 | AAAB |
| ATOM | 3223 | ND2 | ASN | E | 694 | 29.200 | 99.995 | 46.116 | 1.00 | 0.00 | AAAB |
| ATOM | 3226 | C | ASN | E | 694 | 30.566 | 102.155 | 48.859 | 1.00 | 0.00 | AAAB |
| ATOM | 3227 | O | ASN | E | 694 | 31.224 | 101.164 | 49.172 | 1.00 | 0.00 | AAAB |
| ATOM | 3228 | N | PHE | E | 695 | 31.164 | 103.297 | 48.511 | 1.00 | 0.00 | AAAB |
| ATOM | 3230 | CA | PHE | E | 695 | 32.596 | 103.516 | 48.563 | 1.00 | 0.00 | AAAB |
| ATOM | 3232 | CB | PHE | E | 695 | 33.007 | 104.774 | 47.795 | 1.00 | 0.00 | AAAB |
| ATOM | 3235 | CG | PHE | E | 695 | 32.921 | 104.913 | 46.287 | 1.00 | 0.00 | AAAB |
| ATOM | 3236 | CD1 | PHE | E | 695 | 33.550 | 103.896 | 45.559 | 1.00 | 0.00 | AAAB |
| ATOM | 3238 | CD2 | PHE | E | 695 | 32.490 | 106.073 | 45.636 | 1.00 | 0.00 | AAAB |
| ATOM | 3240 | CE1 | PHE | E | 695 | 33.581 | 103.876 | 44.159 | 1.00 | 0.00 | AAAB |
| ATOM | 3242 | CE2 | PHE | E | 695 | 32.643 | 106.155 | 44.249 | 1.00 | 0.00 | AAAB |
| ATOM | 3244 | CZ | PHE | E | 695 | 33.120 | 105.043 | 43.550 | 1.00 | 0.00 | AAAB |
| ATOM | 3246 | C | PHE | E | 695 | 33.117 | 103.666 | 49.984 | 1.00 | 0.00 | AAAB |
| ATOM | 3247 | O | PHE | E | 695 | 34.315 | 103.896 | 50.076 | 1.00 | 0.00 | AAAB |
| ATOM | 3248 | N | LEU | E | 696 | 32.347 | 103.612 | 51.076 | 1.00 | 0.00 | AAAB |
| ATOM | 3250 | CA | LEU | E | 696 | 32.930 | 103.503 | 52.399 | 1.00 | 0.00 | AAAB |
| ATOM | 3252 | CB | LEU | E | 696 | 31.796 | 103.787 | 53.377 | 1.00 | 0.00 | AAAB |
| ATOM | 3255 | CG | LEU | E | 696 | 31.062 | 105.120 | 53.186 | 1.00 | 0.00 | AAAB |
| ATOM | 3257 | CD1 | LEU | E | 696 | 29.778 | 105.198 | 54.008 | 1.00 | 0.00 | AAAB |
| ATOM | 3261 | CD2 | LEU | E | 696 | 31.922 | 106.392 | 53.156 | 1.00 | 0.00 | AAAB |
| ATOM | 3265 | C | LEU | E | 696 | 33.519 | 102.138 | 52.699 | 1.00 | 0.00 | AAAB |
| ATOM | 3266 | O | LEU | E | 696 | 34.309 | 101.980 | 53.637 | 1.00 | 0.00 | AAAB |
| ATOM | 3267 | N | HIS | E | 697 | 33.043 | 101.125 | 51.984 | 1.00 | 0.00 | AAAB |
| ATOM | 3269 | CA | HIS | E | 697 | 33.713 | 99.839 | 51.876 | 1.00 | 0.00 | AAAB |
| ATOM | 3271 | CB | HIS | E | 697 | 32.838 | 98.764 | 51.247 | 1.00 | 0.00 | AAAB |
| ATOM | 3274 | CG | HIS | E | 697 | 33.550 | 97.445 | 51.102 | 1.00 | 0.00 | AAAB |
| ATOM | 3275 | ND1 | HIS | E | 697 | 34.151 | 96.962 | 49.943 | 1.00 | 0.00 | AAAB |
| ATOM | 3276 | CD2 | HIS | E | 697 | 33.638 | 96.510 | 52.102 | 1.00 | 0.00 | AAAB |
| ATOM | 3278 | CE1 | HIS | E | 697 | 34.456 | 95.690 | 50.195 | 1.00 | 0.00 | AAAB |
| ATOM | 3280 | NE2 | HIS | E | 697 | 34.060 | 95.372 | 51.439 | 1.00 | 0.00 | AAAB |
| ATOM | 3282 | C | HIS | E | 697 | 34.885 | 100.101 | 50.946 | 1.00 | 0.00 | AAAB |
| ATOM | 3283 | OT | HIS | E | 697 | 34.713 | 100.469 | 49.786 | 1.00 | 0.00 | AAAB |
| ATOM | 3284 | O | HIS | E | 697 | 36.065 | 99.668 | 51.475 | 1.00 | 0.00 | AAAB |
| END | | | | | | | | | | | |

### APPENDIX IV: ATOMIC COORDINATES FOR THE MODEL OF THI TERMINAL REGION OF IGF-1R α-CHAIN BOUND TO IGF-1R ECTODOMAIN

The structural coordinates in Appendixes I to III were used to place a model of the terminal region of the IGF-1R α-chain in the IGF-1R low affinity binding site fo This model is oriented relative to atomic coordinates found in Appendixes I to II may be used in conjunction with atomic coordinates of Appendixes I to III and V VI to design compounds which bind to IR and/or IGF-1R.

| | | | | | | | | | | | |
|---|---|---|---|---|---|---|---|---|---|---|---|
| ATOM | 1 | N | GLU | E | 1 | 49.070 | 107.878 | 22.857 | 1.00 | 0.00 | AAAA |
| ATOM | 5 | CA | GLU | E | 1 | 48.154 | 106.746 | 23.065 | 1.00 | 0.00 | AAAA |
| ATOM | 7 | CB | GLU | E | 1 | 48.683 | 105.892 | 24.217 | 1.00 | 0.00 | AAAA |
| ATOM | 10 | CG | GLU | E | 1 | 48.339 | 104.402 | 24.280 | 1.00 | 0.00 | AAAA |
| ATOM | 13 | CD | GLU | E | 1 | 49.176 | 103.529 | 23.357 | 1.00 | 0.00 | AAAA |
| ATOM | 14 | OE1 | GLU | E | 1 | 50.031 | 104.137 | 22.676 | 1.00 | 0.00 | AAAA |
| ATOM | 15 | OE2 | GLU | E | 1 | 48.954 | 102.311 | 23.184 | 1.00 | 0.00 | AAAA |
| ATOM | 16 | C | GLU | E | 1 | 46.726 | 107.234 | 23.264 | 1.00 | 0.00 | AAAA |
| ATOM | 17 | O | GLU | E | 1 | 46.574 | 108.413 | 23.578 | 1.00 | 0.00 | AAAA |
| ATOM | 18 | N | ILE | E | 2 | 45.739 | 106.338 | 23.177 | 1.00 | 0.00 | AAAA |
| ATOM | 20 | CA | ILE | E | 2 | 44.354 | 106.427 | 23.594 | 1.00 | 0.00 | AAAA |
| ATOM | 22 | CB | ILE | E | 2 | 43.496 | 106.129 | 22.364 | 1.00 | 0.00 | AAAA |
| ATOM | 24 | CG1 | ILE | E | 2 | 43.685 | 107.103 | 21.201 | 1.00 | 0.00 | AAAA |
| ATOM | 27 | CG2 | ILE | E | 2 | 42.001 | 105.959 | 22.637 | 1.00 | 0.00 | AAAA |
| ATOM | 31 | CD | ILE | E | 2 | 43.149 | 108.498 | 21.525 | 1.00 | 0.00 | AAAA |
| ATOM | 35 | C | ILE | E | 2 | 44.102 | 105.343 | 24.633 | 1.00 | 0.00 | AAAA |
| ATOM | 36 | O | ILE | E | 2 | 44.710 | 104.275 | 24.673 | 1.00 | 0.00 | AAAA |
| ATOM | 37 | N | CYS | E | 3 | 43.162 | 105.536 | 25.561 | 1.00 | 0.00 | AAAA |
| ATOM | 39 | CA | CYS | E | 3 | 42.791 | 104.598 | 26.601 | 1.00 | 0.00 | AAAA |
| ATOM | 41 | CB | CYS | E | 3 | 43.672 | 104.572 | 27.851 | 1.00 | 0.00 | AAAA |
| ATOM | 44 | SG | CYS | E | 3 | 43.796 | 106.092 | 28.824 | 1.00 | 0.00 | AAAA |
| ATOM | 45 | C | CYS | E | 3 | 41.309 | 104.578 | 26.946 | 1.00 | 0.00 | AAAA |
| ATOM | 46 | O | CYS | E | 3 | 40.657 | 105.585 | 26.680 | 1.00 | 0.00 | AAAA |
| ATOM | 47 | N | GLY | E | 4 | 40.835 | 103.466 | 27.512 | 1.00 | 0.00 | AAAA |
| ATOM | 49 | CA | GLY | E | 4 | 39.509 | 103.291 | 28.069 | 1.00 | 0.00 | AAAA |
| ATOM | 52 | C | GLY | E | 4 | 38.847 | 102.047 | 27.493 | 1.00 | 0.00 | AAAA |
| ATOM | 53 | O | GLY | E | 4 | 39.522 | 101.225 | 26.876 | 1.00 | 0.00 | AAAA |
| ATOM | 54 | N | PRO | E | 5 | 37.579 | 101.790 | 27.820 | 1.00 | 0.00 | AAAA |
| ATOM | 55 | CA | PRO | E | 5 | 36.629 | 102.631 | 28.520 | 1.00 | 0.00 | AAAA |
| ATOM | 57 | CB | PRO | E | 5 | 35.234 | 102.196 | 28.071 | 1.00 | 0.00 | AAAA |
| ATOM | 60 | CG | PRO | E | 5 | 35.446 | 100.691 | 27.910 | 1.00 | 0.00 | AAAA |
| ATOM | 63 | CD | PRO | E | 5 | 36.888 | 100.584 | 27.411 | 1.00 | 0.00 | AAAA |
| ATOM | 66 | C | PRO | E | 5 | 36.801 | 102.619 | 30.033 | 1.00 | 0.00 | AAAA |
| ATOM | 67 | O | PRO | E | 5 | 37.689 | 101.952 | 30.559 | 1.00 | 0.00 | AAAA |
| ATOM | 68 | N | GLY | E | 6 | 35.852 | 103.300 | 30.680 | 1.00 | 0.00 | AAAA |
| ATOM | 70 | CA | GLY | E | 6 | 35.603 | 103.508 | 32.092 | 1.00 | 0.00 | AAAA |
| ATOM | 73 | C | GLY | E | 6 | 36.694 | 103.108 | 33.075 | 1.00 | 0.00 | AAAA |
| ATOM | 74 | O | GLY | E | 6 | 36.623 | 102.070 | 33.729 | 1.00 | 0.00 | AAAA |
| ATOM | 75 | N | ILE | E | 7 | 37.763 | 103.908 | 33.042 | 1.00 | 0.00 | AAAA |
| ATOM | 77 | CA | ILE | E | 7 | 39.029 | 103.847 | 33.744 | 1.00 | 0.00 | AAAA |
| ATOM | 79 | CB | ILE | E | 7 | 39.996 | 104.953 | 33.320 | 1.00 | 0.00 | AAAA |
| ATOM | 81 | CG1 | ILE | E | 7 | 40.218 | 105.041 | 31.810 | 1.00 | 0.00 | AAAA |
| ATOM | 84 | CG2 | ILE | E | 7 | 41.390 | 104.903 | 33.948 | 1.00 | 0.00 | AAAA |
| ATOM | 88 | CD | ILE | E | 7 | 41.103 | 103.995 | 31.131 | 1.00 | 0.00 | AAAA |
| ATOM | 92 | C | ILE | E | 7 | 38.701 | 103.950 | 35.227 | 1.00 | 0.00 | AAAA |
| ATOM | 93 | O | ILE | E | 7 | 38.571 | 105.022 | 35.814 | 1.00 | 0.00 | AAAA |
| ATOM | 94 | N | ASP | E | 8 | 38.634 | 102.792 | 35.889 | 1.00 | 0.00 | AAAA |
| ATOM | 96 | CA | ASP | E | 8 | 38.570 | 102.773 | 37.335 | 1.00 | 0.00 | AAAA |
| ATOM | 98 | CB | ASP | E | 8 | 37.834 | 101.462 | 37.610 | 1.00 | 0.00 | AAAA |
| ATOM | 101 | CG | ASP | E | 8 | 37.501 | 101.014 | 39.026 | 1.00 | 0.00 | AAAA |
| ATOM | 102 | OD1 | ASP | E | 8 | 37.568 | 99.827 | 39.410 | 1.00 | 0.00 | AAAA |
| ATOM | 103 | OD2 | ASP | E | 8 | 37.027 | 101.869 | 39.806 | 1.00 | 0.00 | AAAA |
| ATOM | 104 | C | ASP | E | 8 | 39.959 | 102.618 | 37.938 | 1.00 | 0.00 | AAAA |
| ATOM | 105 | O | ASP | E | 8 | 40.733 | 101.778 | 37.486 | 1.00 | 0.00 | AAAA |
| ATOM | 106 | N | ILE | E | 9 | 40.322 | 103.595 | 38.773 | 1.00 | 0.00 | AAAA |
| ATOM | 108 | CA | ILE | E | 9 | 41.633 | 103.704 | 39.381 | 1.00 | 0.00 | AAAA |
| ATOM | 110 | CB | ILE | E | 9 | 42.524 | 104.771 | 38.743 | 1.00 | 0.00 | AAAA |
| ATOM | 112 | CG1 | ILE | E | 9 | 43.117 | 104.393 | 37.386 | 1.00 | 0.00 | AAAA |
| ATOM | 115 | CG2 | ILE | E | 9 | 43.631 | 105.253 | 39.682 | 1.00 | 0.00 | AAAA |
| ATOM | 119 | CD | ILE | E | 9 | 43.888 | 105.488 | 36.649 | 1.00 | 0.00 | AAAA |
| ATOM | 123 | C | ILE | E | 9 | 91.200 | 103.831 | 40.835 | 1.00 | 0.00 | AAAA |
| ATOM | 124 | O | ILE | E | 9 | 40.607 | 104.852 | 41.175 | 1.00 | 0.00 | AAAA |
| ATOM | 125 | N | ARG | E | 10 | 41.628 | 102.890 | 41.680 | 1.00 | 0.00 | AAAA |
| ATOM | 127 | CA | ARG | E | 10 | 41.220 | 102.911 | 43.072 | 1.00 | 0.00 | AAAA |
| ATOM | 129 | CB | ARG | E | 10 | 40.188 | 101.813 | 43.329 | 1.00 | 0.00 | AAAA |
| ATOM | 132 | CG | ARG | E | 10 | 38.921 | 101.826 | 42.474 | 1.00 | 0.00 | AAAA |
| ATOM | 135 | CD | ARG | E | 10 | 37.933 | 100.771 | 42.973 | 1.00 | 0.00 | AAAA |
| ATOM | 138 | NE | ARG | E | 10 | 36.643 | 101.139 | 42.390 | 1.00 | 0.00 | AAAA |
| ATOM | 140 | CZ | ARG | E | 10 | 35.416 | 101.043 | 42.918 | 1.00 | 0.00 | AAAA |
| ATOM | 141 | NH1 | ARG | E | 10 | 35.221 | 100.503 | 44.129 | 1.00 | 0.00 | AAAA |
| ATOM | 144 | NH2 | ARG | E | 10 | 34.346 | 101.499 | 42.251 | 1.00 | 0.00 | AAAA |
| ATOM | 147 | C | ARG | E | 10 | 42.435 | 102.657 | 43.953 | 1.00 | 0.00 | AAAA |
| ATOM | 148 | O | ARG | E | 10 | 43.233 | 101.756 | 43.706 | 1.00 | 0.00 | AAAA |
| ATOM | 149 | N | ASN | E | 11 | 42.555 | 103.428 | 45.036 | 1.00 | 0.00 | AAAA |
| ATOM | 151 | CA | ASN | E | 11 | 43.152 | 102.984 | 46.281 | 1.00 | 0.00 | AAAA |
| ATOM | 153 | CB | ASN | E | 11 | 42.620 | 101.636 | 46.768 | 1.00 | 0.00 | AAAA |
| ATOM | 156 | CG | ASN | E | 11 | 42.402 | 101.352 | 48.248 | 1.00 | 0.00 | AAAA |
| ATOM | 157 | OD1 | ASN | E | 11 | 41.459 | 101.788 | 48.904 | 1.00 | 0.00 | AAAA |
| ATOM | 158 | ND2 | ASN | E | 11 | 43.357 | 100.651 | 48.865 | 1.00 | 0.00 | AAAA |
| ATOM | 161 | C | ASN | E | 11 | 44.623 | 103.207 | 46.602 | 1.00 | 0.00 | AAAA |
| ATOM | 162 | O | ASN | E | 11 | 45.023 | 102.976 | 47.740 | 1.00 | 0.00 | AAAA |
| ATOM | 163 | N | ASP | E | 12 | 45.397 | 103.416 | 45.535 | 1.00 | 0.00 | AAAA |
| ATOM | 165 | CA | ASP | E | 12 | 46.842 | 103.360 | 45.431 | 1.00 | 0.00 | AAAA |
| ATOM | 167 | CB | ASP | E | 12 | 47.302 | 101.928 | 45.155 | 1.00 | 0.00 | AAAA |
| ATOM | 170 | CG | ASP | E | 12 | 48.771 | 101.724 | 44.816 | 1.00 | 0.00 | AAAA |
| ATOM | 171 | OD1 | ASP | E | 12 | 49.117 | 101.262 | 43.707 | 1.00 | 0.00 | AAAA |
| ATOM | 172 | OD2 | ASP | E | 12 | 49.625 | 102.177 | 45.610 | 1.00 | 0.00 | AAAA |
| ATOM | 173 | C | ASP | E | 12 | 47.318 | 104.347 | 44.374 | 1.00 | 0.00 | AAAA |
| ATOM | 174 | O | ASP | E | 12 | 46.716 | 104.455 | 43.309 | 1.00 | 0.00 | AAAA |
| ATOM | 175 | N | TYR | E | 13 | 48.388 | 105.110 | 44.614 | 1.00 | 0.00 | AAAA |
| ATOM | 177 | CA | TYR | E | 13 | 48.869 | 106.137 | 43.714 | 1.00 | 0.00 | AAAA |
| ATOM | 179 | CB | TYR | E | 13 | 49.579 | 107.126 | 44.639 | 1.00 | 0.00 | AAAA |
| ATOM | 182 | CG | TYR | E | 13 | 50.119 | 108.413 | 44.063 | 1.00 | 0.00 | AAAA |
| ATOM | 183 | CD1 | TYR | E | 13 | 49.310 | 109.553 | 44.134 | 1.00 | 0.00 | AAAA |
| ATOM | 185 | CD2 | TYR | E | 13 | 51.463 | 108.548 | 43.692 | 1.00 | 0.00 | AAAA |
| ATOM. | 187 | CE1 | TYR | E | 13 | 49.743 | 110.751 | 43.554 | 1.00 | 0.00 | AAAA |
| ATOM | 189 | CE2 | TYR | E | 13 | 51.955 | 109.763 | 43.199 | 1.00 | 0.00 | AAAA |
| ATOM | 191 | CZ | TYR | E | 13 | 51.042 | 110.812 | 43.035 | 1.00 | 0.00 | AAAA |
| ATOM | 192 | OH | TYR | E | 13 | 51.547 | 111.967 | 42.516 | 1.00 | 0.00 | AAAA |
| ATOM | 194 | C | TYR | E | 13 | 49.702 | 105.627 | 42.546 | 1.00 | 0.00 | AAAA |
| ATOM | 195 | O | TYR | E | 13 | 49.870 | 106.375 | 41.586 | 1.00 | 0.00 | AAAA |
| ATOM | 196 | N | GLN | E | 14 | 50.188 | 104.384 | 42.519 | 1.00 | 0.00 | AAAA |
| ATOM | 198 | CA | GLN | E | 14 | 51.010 | 103.789 | 41.485 | 1.00 | 0.00 | AAAA |
| ATOM | 200 | CB | GLN | E | 14 | 51.690 | 102.495 | 41.931 | 1.00 | 0.00 | AAAA |
| ATOM | 203 | CG | GLN | E | 14 | 52.374 | 102.573 | 43.296 | 1.00 | 0.00 | AAAA |
| ATOM | 206 | CD | GLN | E | 14 | 53.590 | 103.487 | 43.319 | 1.00 | 0.00 | AAAA |
| ATOM | 207 | OE1 | GLN | E | 14 | 53.714 | 104.348 | 42.449 | 1.00 | 0.00 | AAAA |
| ATOM | 208 | NE2 | GLN | E | 14 | 54.494 | 103.218 | 44.264 | 1.00 | 0.00 | AAAA |
| ATOM | 211 | C | GLN | E | 14 | 50.169 | 103.455 | 40.260 | 1.00 | 0.00 | AAAA |
| ATOM | 212 | O | GLN | E | 14 | 50.650 | 103.503 | 39.130 | 1.00 | 0.00 | AAAA |
| ATOM | 213 | N | GLN | E | 15 | 48.857 | 103.322 | 40.467 | 1.00 | 0.00 | AAAA |
| ATOM | 215 | CA | GLN | E | 15 | 47.875 | 102.953 | 39.466 | 1.00 | 0.00 | AAAA |
| ATOM | 217 | CB | GLN | E | 15 | 46.497 | 102.643 | 40.051 | 1.00 | 0.00 | AAAA |
| ATOM | 220 | CG | GLN | E | 15 | 46.492 | 101.759 | 41.300 | 1.00 | 0.00 | AAAA |
| ATOM | 223 | CD | GLN | E | 15 | 46.969 | 100.332 | 41.070 | 1.00 | 0.00 | AAAA |
| ATOM | 224 | OE1 | GLN | E | 15 | 46.791 | 99.749 | 40.003 | 1.00 | 0.00 | AAAA |
| ATOM | 225 | NE2 | GLN | E | 15 | 47.762 | 99.756 | 41.977 | 1.00 | 0.00 | AAAA |
| ATOM | 228 | C | GLN | E | 15 | 47.746 | 104.084 | 38.457 | 1.00 | 0.00 | AAAA |
| ATOM | 229 | O | GLN | E | 15 | 47.230 | 103.789 | 37.381 | 1.00 | 0.00 | AAAA |
| ATOM | 230 | N | LEU | E | 16 | 48.202 | 105.288 | 38.810 | 1.00 | 0.00 | AAAA |
| ATOM | 232 | CA | LEU | E | 16 | 48.050 | 106.459 | 37.968 | 1.00 | 0.00 | AAAA |
| ATOM | 234 | CB | LEU | E | 16 | 48.449 | 107.746 | 38.689 | 1.00 | 0.00 | AAAA |
| ATOM | 237 | CG | LEU | E | 16 | 47.456 | 108.284 | 39.719 | 1.00 | 0.00 | AAAA |
| ATOM | 239 | CD1 | LEU | E | 16 | 48.157 | 109.329 | 40.588 | 1.00 | 0.00 | AAAA |
| ATOM | 243 | CD2 | LEU | E | 16 | 46.121 | 108.769 | 39.152 | 1.00 | 0.00 | AAAA |
| ATOM | 247 | C | LEU | E | 16 | 48.754 | 106.332 | 36.624 | 1.00 | 0.00 | AAAA |
| ATOM | 248 | O | LEU | E | 16 | 48.299 | 106.803 | 35.583 | 1.00 | 0.00 | AAAA |
| ATOM | 249 | N | LYS | E | 17 | 49.894 | 105.639 | 36.656 | 1.00 | 0.00 | AAAA |
| ATOM | 251 | CA | LYS | E | 17 | 50.872 | 105.355 | 35.624 | 1.00 | 0.00 | AAAA |
| ATOM | 253 | CB | LYS | E | 17 | 52.017 | 104.482 | 36.136 | 1.00 | 0.00 | AAAA |
| ATOM | 256 | CG | LYS | E | 17 | 53.223 | 105.185 | 36.761 | 1.00 | 0.00 | AAAA |
| ATOM | 259 | CD | LYS | E | 17 | 54.336 | 104.256 | 37.246 | 1.00 | 0.00 | AAAA |
| ATOM | 262 | CE | LYS | E | 17 | 53.924 | 103.184 | 38.257 | 1.00 | 0.00 | AAAA |
| ATOM | 265 | NZ | LYS | E | 17 | 55.128 | 102.419 | 38.620 | 1.00 | 0.00 | AAAA |
| ATOM | 269 | C | LYS | E | 17 | 50.364 | 104.734 | 34.330 | 1.00 | 0.00 | AAAA |
| ATOM | 270 | O | LYS | E | 17 | 51.049 | 104.780 | 33.311 | 1.00 | 0.00 | AAAA |
| ATOM | 271 | N | ARG | E | 18 | 49.187 | 104.107 | 34.285 | 1.00 | 0.00 | AAAA |
| ATOM | 273 | CA | ARG | E | 18 | 48.513 | 103.661 | 33.082 | 1.00 | 0.00 | AAAA |
| ATOM | 275 | CB | ARG | E | 18 | 47.284 | 102.861 | 33.514 | 1.00 | 0.00 | AAAA |
| ATOM | 278 | CG | ARG | E | 18 | 46.491 | 102.207 | 32.382 | 1.00 | 0.00 | AAAA |
| ATOM | 281 | CD | ARG | E | 18 | 45.196 | 101.516 | 32.808 | 1.00 | 0.00 | AAAA |
| ATOM | 284 | NE | ARG | E | 18 | 44.278 | 101.280 | 31.695 | 1.00 | 0.00 | AAAA |
| ATOM | 286 | CZ | ARG | E | 18 | 42.945 | 101.166 | 31.615 | 1.00 | 0.00 | AAAA |
| ATOM | 287 | NH1 | ARG | E | 18 | 42.156 | 101.186 | 32.698 | 1.00 | 0.00 | AAAA |
| ATOM | 290 | NH2 | ARG | E | 18 | 42.376 | 101.114 | 30.403 | 1.00 | 0.00 | AAAA |
| ATOM | 293 | C | ARG | E | 18 | 48.150 | 104.804 | 32.146 | 1.00 | 0.00 | AAAA |
| ATOM | 294 | O | ARG | E | 18 | 48.028 | 104.594 | 30.940 | 1.00 | 0.00 | AAAA |
| ATOM | 295 | N | LEU | E | 19 | 48.066 | 106.044 | 32.635 | 1.00 | 0.00 | AAAA |
| ATOM | 297 | CA | LEU | E | 19 | 47.613 | 107.187 | 31.870 | 1.00 | 0.00 | AAAA |
| ATOM | 299 | CB | LEU | E | 19 | 46.880 | 108.177 | 32.776 | 1.00 | 0.00 | AAAA |
| ATOM | 302 | CG | LEU | E | 19 | 45.590 | 107.590 | 33.349 | 1.00 | 0.00 | AAAA |
| ATOM | 304 | CD1 | LEU | E | 19 | 45.032 | 108.656 | 34.290 | 1.00 | 0.00 | AAAA |
| ATOM | 308 | CD2 | LEU | E | 19 | 44.534 | 107.288 | 32.285 | 1.00 | 0.00 | AAAA |
| ATOM | 312 | C | LEU | E | 19 | 48.816 | 107.930 | 31.305 | 1.00 | 0.00 | AAAA |
| ATOM | 313 | O | LEU | E | 19 | 48.601 | 109.036 | 30.815 | 1.00 | 0.00 | AAAA |
| ATOM | 314 | N | GLU | E | 20 | 50.046 | 107.428 | 31.438 | 1.00 | 0.00 | AAAA |
| ATOM | 316 | CA | GLU | E | 20 | 51.319 | 107.967 | 31.003 | 1.00 | 0.00 | AAAA |
| ATOM | 318 | CB | GLU | E | 20 | 52.445 | 106.950 | 31.195 | 1.00 | 0.00 | AAAA |
| ATOM | 321 | CG | GLU | E | 20 | 53.445 | 107.345 | 32.282 | 1.00 | 0.00 | AAAA |
| ATOM | 324 | CD | GLU | E | 20 | 54.376 | 108.503 | 31.954 | 1.00 | 0.00 | AAAA |
| ATOM | 325 | OE1 | GLU | E | 20 | 55.068 | 108.459 | 30.915 | 1.00 | 0.00 | AAAA |
| ATOM | 326 | OE2 | GLU | E | 20 | 54.381 | 109.521 | 32.679 | 1.00 | 0.00 | AAAA |
| ATOM | 327 | C | GLU | E | 20 | 51.449 | 108.609 | 29.629 | 1.00 | 0.00 | AAAA |
| ATOM | 328 | O | GLU | E | 20 | 52.259 | 109.511 | 29.434 | 1.00 | 0.00 | AAAA |
| ATOM | 329 | N | ASN | E | 21 | 50.661 | 108.169 | 28.646 | 1.00 | 0.00 | AAAA |
| ATOM | 331 | CA | ASN | E | 21 | 50.762 | 108.661 | 27.286 | 1.00 | 0.00 | AAAA |
| ATOM | 333 | CB | ASN | E | 21 | 51.788 | 107.924 | 26.424 | 1.00 | 0.00 | AAAA |
| ATOM | 336 | CG | ASN | E | 21 | 51.935 | 106.442 | 26.737 | 1.00 | 0.00 | AAAA |
| ATOM | 337 | OD1 | ASN | E | 21 | 50.977 | 105.726 | 26.453 | 1.00 | 0.00 | AAAA |
| ATOM | 338 | ND2 | ASN | E | 21 | 53.043 | 105.907 | 27.254 | 1.00 | 0.00 | AAAA |
| ATOM | 341 | C | ASN | E | 21 | 49.418 | 108.713 | 26.573 | 1.00 | 0.00 | AAAA |
| ATOM | 342 | O | ASN | E | 21 | 49.324 | 109.062 | 25.399 | 1.00 | 0.00 | AAAA |
| ATOM | 343 | N | CYS | E | 22 | 48.346 | 108.565 | 27.355 | 1.00 | 0.00 | AAAA |
| ATOM | 345 | CA | CYS | E | 22 | 46.951 | 108.619 | 26.969 | 1.00 | 0.00 | AAAA |
| ATOM | 347 | CB | CYS | E | 22 | 46.168 | 107.857 | 28.039 | 1.00 | 0.00 | AAAA |
| ATOM | 350 | SG | CYS | E | 22 | 44.419 | 107.627 | 27.636 | 1.00 | 0.00 | AAAA |
| ATOM | 351 | C | CYS | E | 22 | 46.470 | 110.047 | 26.759 | 1.00 | 0.00 | AAAA |
| ATOM | 352 | O | CYS | E | 22 | 46.420 | 110.828 | 27.707 | 1.00 | 0.00 | AAAA |
| ATOM | 353 | N | THR | E | 23 | 46.405 | 110.464 | 25.492 | 1.00 | 0.00 | AAAA |
| ATOM | 355 | CA | THR | E | 23 | 46.087 | 111.822 | 25.100 | 1.00 | 0.00 | AAAA |
| ATOM | 357 | CB | THR | E | 23 | 46.558 | 112.017 | 23.658 | 1.00 | 0.00 | AAAA |
| ATOM | 359 | OG1 | THR | E | 23 | 46.071 | 111.110 | 22.695 | 1.00 | 0.00 | AAAA |
| ATOM | 361 | CG2 | THR | E | 23 | 48.088 | 112.007 | 23.665 | 1.00 | 0.00 | AAAA |
| ATOM | 365 | C | THR | E | 23 | 44.589 | 112.096 | 25.116 | 1.00 | 0.00 | AAAA |
| ATOM | 366 | O | THR | E | 23 | 44.163 | 113.093 | 25.693 | 1.00 | 0.00 | AAAA |
| ATOM | 367 | N | VAL | E | 24 | 43.773 | 111.194 | 24.567 | 1.00 | 0.00 | AAAA |
| ATOM | 369 | CA | VAL | E | 24 | 42.352 | 111.151 | 24.852 | 1.00 | 0.00 | AAAA |
| ATOM | 371 | CB | VAL | E | 24 | 41.499 | 111.354 | 23.599 | 1.00 | 0.00 | AAAA |
| ATOM | 373 | CG1 | VAL | E | 24 | 39.997 | 111.375 | 23.884 | 1.00 | 0.00 | AAAA |
| ATOM | 377 | CG2 | VAL | E | 24 | 41.894 | 112.631 | 22.859 | 1.00 | 0.00 | AAAA |
| ATOM | 381 | C | VAL | E | 24 | 41.964 | 109.905 | 25.635 | 1.00 | 0.00 | AAAA |
| ATOM | 382 | O | VAL | E | 24 | 42.354 | 108.817 | 25.215 | 1.00 | 0.00 | AAAA |
| ATOM | 383 | N | ILE | E | 25 | 41.243 | 110.009 | 26.753 | 1.00 | 0.00 | AAAA |
| ATOM | 385 | CA | ILE | E | 25 | 40.579 | 108.893 | 27.398 | 1.00 | 0.00 | AAAA |
| ATOM | 387 | CB | ILE | E | 25 | 40.423 | 109.306 | 28.863 | 1.00 | 0.00 | AAAA |
| ATOM | 389 | CG1 | ILE | E | 25 | 41.772 | 109.491 | 29.559 | 1.00 | 0.00 | AAAA |
| ATOM | 392 | CG2 | ILE | E | 25 | 39.627 | 108.286 | 29.677 | 1.00 | 0.00 | AAAA |
| ATOM | 396 | CD | ILE | E | 25 | 41.721 | 109.718 | 31.070 | 1.00 | 0.00 | AAAA |
| ATOM | 400 | C | ILE | E | 25 | 39.264 | 108.744 | 26.645 | 1.00 | 0.00 | AAAA |
| ATOM | 401 | O | ILE | E | 25 | 38.507 | 109.711 | 26.656 | 1.00 | 0.00 | AAAA |
| ATOM | 402 | N | GLU | E | 26 | 38.979 | 107.584 | 26.048 | 1.00 | 0.00 | AAAA |
| ATOM | 404 | CA | GLU | E | 26 | 37.850 | 107.186 | 25.232 | 1.00 | 0.00 | AAAA |
| ATOM | 406 | CB | GLU | E | 26 | 38.396 | 106.368 | 24.062 | 1.00 | 0.00 | AAAA |
| ATOM | 409 | CG | GLU | E | 26 | 38.527 | 107.244 | 22.816 | 1.00 | 0.00 | AAAA |
| ATOM | 412 | CD | GLU | E | 26 | 37.183 | 107.302 | 22.104 | 1.00 | 0.00 | AAAA |
| ATOM | 413 | OE1 | GLU | E | 26 | 36.085 | 107.494 | 22.670 | 1.00 | 0.00 | AAAA |
| ATOM | 414 | OE2 | GLU | E | 26 | 37.138 | 107.228 | 20.857 | 1.00 | 0.00 | AAAA |
| ATOM | 415 | C | GLU | E | 26 | 37.075 | 106.389 | 26.272 | 1.00 | 0.00 | AAAA |
| ATOM | 416 | O | GLU | E | 26 | 37.087 | 105.161 | 26.324 | 1.00 | 0.00 | AAAA |
| ATOM | 417 | N | GLY | E | 27 | 36.306 | 107.133 | 27.070 | 1.00 | 0.00 | AAAA |
| ATOM | 419 | CA | GLY | E | 27 | 35.552 | 106.766 | 28.251 | 1.00 | 0.00 | AAAA |
| ATOM | 422 | C | GLY | E | 27 | 35.566 | 107.834 | 29.337 | 1.00 | 0.00 | AAAA |
| ATOM | 423 | O | GLY | E | 27 | 35.809 | 109.020 | 29.127 | 1.00 | 0.00 | AAAA. |
| ATOM | 424 | N | TYR | E | 28 | 35.193 | 107.402 | 30.544 | 1.00 | 0.00 | AAAA |
| ATOM | 426 | CA | TYR | E | 28 | 35.263 | 108.221 | 31.737 | 1.00 | 0.00 | AAAA |
| ATOM | 428 | CB | TYR | E | 28 | 34.044 | 108.087 | 32.651 | 1.00 | 0.00 | AAAA |
| ATOM | 431 | CG | TYR | E | 28 | 33.789 | 106.738 | 33.278 | 1.00 | 0.00 | AAAA |
| ATOM | 432 | CD1 | TYR | E | 28 | 34.545 | 106.283 | 34.365 | 1.00 | 0.00 | AAAA |
| ATOM | 434 | CD2 | TYR | E | 28 | 32.804 | 105.865 | 32.801 | 1.00 | 0.00 | AAAA |
| ATOM | 436 | CE1 | TYR | E | 28 | 34.416 | 104.996 | 34.900 | 1.00 | 0.00 | AAAA |
| ATOM | 438 | CE2 | TYR | E | 28 | 32.705 | 104.589 | 33.371 | 1.00 | 0.00 | AAAA |
| ATOM | 440 | CZ | TYR | E | 28 | 33.498 | 104.066 | 34.399 | 1.00 | 0.00 | AAAA |
| ATOM | 441 | OH | TYR | E | 28 | 33.400 | 102.772 | 34.819 | 1.00 | 0.00 | AAAA |
| ATOM | 443 | C | TYR | E | 28 | 36.480 | 107.822 | 32.559 | 1.00 | 0.00 | AAAA |
| ATOM | 444 | O | TYR | E | 28 | 37.188 | 106.848 | 32.319 | 1.00 | 0.00 | AAAA |
| ATOM | 445 | N | LEU | E | 29 | 36.718 | 108.680 | 33.555 | 1.00 | 0.00 | AAAA |
| ATOM | 447 | CA | LEU | E | 29 | 37.807 | 108.513 | 34.497 | 1.00 | 0.00 | AAAA |
| ATOM | 449 | CB | LEU | E | 29 | 38.906 | 109.531 | 34.192 | 1.00 | 0.00 | AAAA |
| ATOM | 452 | CG | LEU | E | 29 | 40.160 | 109.599 | 35.065 | 1.00 | 0.00 | AAAA |
| ATOM | 454 | CD1 | LEU | E | 29 | 40.869 | 108.244 | 35.075 | 1.00 | 0.00 | AAAA |
| ATOM | 458 | CD2 | LEU | E | 29 | 41.089 | 110.750 | 34.683 | 1.00 | 0.00 | AAAA |
| ATOM | 462 | C | LEU | E | 29 | 37.279 | 108.704 | 35.912 | 1.00 | 0.00 | AAAA |
| ATOM | 463 | O | LEU | E | 29 | 36.893 | 109.780 | 36.364 | 1.00 | 0.00 | AAAA |
| ATOM | 464 | N | HIS | E | 30 | 37.275 | 107.621 | 36.693 | 1.00 | 0.00 | AAAA |
| ATOM | 466 | CA | HIS | E | 30 | 37.140 | 107.535 | 38.134 | 1.00 | 0.00 | AAAA |
| ATOM | 468 | CB | HIS | E | 30 | 36.429 | 106.229 | 38.489 | 1.00 | 0.00 | AAAA |
| ATOM | 471 | CG | HIS | E | 30 | 34.941 | 106.206 | 38.277 | 1.00 | 0.00 | AAAA |
| ATOM | 472 | ND1 | HIS | E | 30 | 34.150 | 105.077 | 38.484 | 1.00 | 0.00 | AAAA |
| ATOM | 473 | CD2 | HIS | E | 30 | 34.160 | 107.216 | 37.780 | 1.00 | 0.00 | AAAA |
| ATOM | 475 | CE1 | HIS | E | 30 | 32.939 | 105.378 | 38.007 | 1.00 | 0.00 | AAAA |
| ATOM | 477 | NE2 | HIS | E | 30 | 32.911 | 106.654 | 37.590 | 1.00 | 0.00 | AAAA |
| ATOM | 479 | C | HIS | E | 30 | 38.510 | 107.354 | 38.772 | 1.00 | 0.00 | AAAA |
| ATOM | 480 | O | HIS | E | 30 | 39.228 | 106.376 | 38.575 | 1.00 | 0.00 | AAAA |
| ATOM | 481 | N | ILE | E | 31 | 38.890 | 108.336 | 39.593 | 1.00 | 0.00 | AAAA |
| ATOM | 483 | CA | ILE | E | 31 | 40.000 | 108.226 | 40.519 | 1.00 | 0.00 | AAAA |
| ATOM | 485 | CB | ILE | E | 31 | 40.944 | 109.397 | 40.243 | 1.00 | 0.00 | AAAA |
| ATOM | 487 | CG1 | ILE | E | 31 | 41.510 | 109.443 | 38.824 | 1.00 | 0.00 | AAAA |
| ATOM | 490 | CG2 | ILE | E | 31 | 42.069 | 109.490 | 41.274 | 1.00 | 0.00 | AAAA |
| ATOM | 494 | CD | ILE | E | 31 | 42.200 | 110.760 | 38.465 | 1.00 | 0.00 | AAAA |
| ATOM | 498 | C | ILE | E | 31 | 39.370 | 108.289 | 41.903 | 1.00 | 0.00 | AAAA |
| ATOM | 499 | O | ILE | E | 31 | 38.851 | 109.336 | 42.282 | 1.00 | 0.00 | AAAA |
| ATOM | 500 | N | LEU | E | 32 | 39.480 | 107.213 | 42.684 | 1.00 | 0.00 | AAAA |
| ATOM | 502 | CA | LEU | E | 32 | 39.007 | 107.256 | 44.053 | 1.00 | 0.00 | AAAA |
| ATOM | 504 | CB | LEU | E | 32 | 37.591 | 106.702 | 44.201 | 1.00 | 0.00 | AAAA |
| ATOM | 507 | CG | LEU | E | 32 | 37.260 | 105.284 | 43.736 | 1.00 | 0.00 | AAAA |
| ATOM | 509 | CD1 | LEU | E | 32 | 36.121 | 104.635 | 44.523 | 1.00 | 0.00 | AAAA |
| ATOM | 513 | CD2 | LEU | E | 32 | 36.883 | 105.247 | 42.254 | 1.00 | 0.00 | AAAA |
| ATOM | 517 | C | LEU | E | 32 | 40.000 | 106.730 | 45.081 | 1.00 | 0.00 | AAAA |
| ATOM | 518 | O | LEU | E | 32 | 40.800 | 205.832 | 44.830 | 1.00 | 0.00 | AAAA |
| ATOM | 519 | N | LEU | E | 33 | 39.787 | 107.213 | 46.307 | 1.00 | 0.00 | AAAA |
| ATOM | 521 | CA | LEU | E | 33 | 40.258 | 106.642 | 47.552 | 1.00 | 0.00 | AAAA |
| ATOM | 523 | CB | LEU | E | 33 | 39.505 | 105.386 | 47.991 | 1.00 | 0.00 | AAAA |
| ATOM | 526 | CG | LEU | E | 33 | 37.976 | 105.376 | 47.945 | 1.00 | 0.00 | AAAA |
| ATOM | 528 | CD1 | LEU | E | 33 | 37.357 | 104.201 | 48.702 | 1.00 | 0.00 | AAAA |
| ATOM | 532 | CD2 | LEU | E | 33 | 37.402 | 106.744 | 48.312 | 1.00 | 0.00 | AAAA |
| ATOM | 536 | C | LEU | E | 33 | 41.757 | 106.491 | 47.774 | 1.00 | 0.00 | AAAA |
| ATOM | 537 | O | LEU | E | 33 | 42.229 | 105.698 | 48.585 | 1.00 | 0.00 | AAAA |
| ATOM | 538 | N | ILE | E | 34 | 42.535 | 107.366 | 47.133 | 1.00 | 0.00 | AAAA |
| ATOM | 540 | CA | ILE | E | .34 | 43.983 | 107.295 | 47.180 | 1.00 | 0.00 | AAAA |
| ATOM | 542 | CB | ILE | E | 34 | 44.625 | 107.772 | 45.877 | 1.00 | 0.00 | AAAA |
| ATOM | 544 | CG1 | ILE | E | 34 | 44.035 | 107.097 | 44.638 | 1.00 | 0.00 | AAAA |
| ATOM | 547 | CG2 | ILE | E | 34 | 46.149 | 107.664 | 45.833 | 1.00 | 0.00 | AAAA |
| ATOM | 551 | CD | ILE | E | 34 | 43.414 | 108.116 | 43.684 | 1.00 | 0.00 | AAAA |
| ATOM | 555 | C | ILE | E | 34 | 44.386 | 108.183 | 48.347 | 1.00 | 0.00 | AAAA |
| ATOM | 556 | O | ILE | E | 34 | 43.893 | 109.296 | 48.520 | 1.00 | 0.00 | AAAA |
| ATOM | 557 | N | SER | E | 35 | 45.341 | 107.774 | 49.185 | 1.00 | 0.00 | AAAA |
| ATOM | 559 | CA | SER | E | 35 | 46.202 | 108.575 | 50.033 | 1.00 | 0.00 | AAAA |
| ATOM | 561 | CB | SER | E | 35 | 46.156 | 108.097 | 51.484 | 1.00 | 0.00 | AAAA |
| ATOM | 564 | OG | SER | E | 35 | 44.820 | 108.076 | 51.933 | 1.00 | 0.00 | AAAA |
| ATOM | 566 | C | SER | E | 35 | 47.609 | 108.471 | 49.462 | 1.00 | 0.00 | AAAA |
| ATOM | 567 | O | SER | E | 35 | 48.057 | 107.435 | 48.978 | 1.00 | 0.00 | AAAA |
| ATOM | 568 | N | LYS | E | .36 | 48.319 | 109.596 | 49.575 | 1.00 | 0.00 | AAAA |
| ATOM | 570 | CA | LYS | E | 36 | 49.651 | 109.837 | 49.057 | 1.00 | 0.00 | AAAA |
| ATOM | 572 | CB | LYS | E | 36 | 49.594 | 111.182 | 48.332 | 1.00 | 0.00 | AAAA |
| ATOM | 575 | CG | LYS | E | 36 | 50.696 | 111.516 | 47.327 | 1.00 | 0.00 | AAAA |
| ATOM | 578 | CD | LYS | E | 36 | 51.251 | 112.941 | 47.354 | 1.00 | 0.00 | AAAA |
| ATOM | 581 | CE | LYS | E | 36 | 52.463 | 113.153 | 46.447 | 1.00 | 0.00 | AAAA |
| ATOM | 584 | NZ | LYS | E | 36 | 53.014 | 114.516 | 46.503 | 1.00 | 0.00 | AAAA |
| ATOM | 588 | C | LYS | E | 36 | 50.745 | 109.826 | 50.116 | 1.00 | 0.00 | AAAA |
| ATOM | 589 | O | LYS | E | 36 | 50.603 | 110.362 | 51.212 | 1.00 | 0.00 | AAAA |
| ATOM | 590 | N | ALA | E | 37 | 51.909 | 109.303 | 49.726 | 1.00 | 0.00 | AAAA |
| ATOM | 592 | CA | ALA | E | 37 | 53.220 | 109.403 | 50.335 | 1.00 | 0.00 | AAAA |
| ATOM | 594 | CB | ALA | E | 37 | 53.918 | 108.074 | 50.039 | 1.00 | 0.00 | AAAA |
| ATOM | 598 | C | ALA | E | 37 | 53.953 | 110.561 | 49.673 | 1.00 | 0.00 | AAAA |
| ATOM | 599 | O | ALA | E | 37 | 54.290 | 110.493 | 48.494 | 1.00 | 0.00 | AAAA |
| ATOM | 600 | N | GLU | E | 38 | 54.057 | 111.663 | 50.420 | 1.00 | 0.00 | AAAA |
| ATOM | 602 | CA | GLU | E | 38 | 54.812 | 112.858 | 50.105 | 1.00 | 0.00 | AAAA |
| ATOM | 604 | CB | GLU | E | 38 | 54.558 | 113.978 | 51.116 | 1.00 | 0.00 | AAAA |
| ATOM | 607 | CG | GLU | E | 38 | 54.884 | 115.408 | 50.687 | 1.00 | 0.00 | AAAA |
| ATOM | 610 | CD | GLU | E | 38 | 54.373 | 115.817 | 49.313 | 1.00 | 0.00 | AAAA |
| ATOM | 611 | OE1 | GLU | E | 38 | 53.151 | 115.724 | 49.067 | 1.00 | 0.00 | AAAA |
| ATOM | 612 | OE2 | GLU | E | 38 | 55.153 | 116.342 | 48.488 | 1.00 | 0.00 | AAAA |
| ATOM | 613 | C | GLU | E | 38 | 56.299 | 112.652 | 49.854 | 1.00 | 0.00 | AAAA |
| ATOM | 614 | O | GLU | E | 38 | 56.942 | 113.163 | 48.940 | 1.00 | 0.00 | AAAA |
| ATOM | 615 | N | ASP | E | 39 | 56.857 | 111.693 | 50.596 | 1.00 | 0.00 | AAAA |
| ATOM | 617 | CA | ASP | E | 39 | 58.231 | 111.254 | 50.455 | 1.00 | 0.00 | AAAA |
| ATOM | 619 | CB | ASP | E | 39 | 58.530 | 110.310 | 51.621 | 1.00 | 0.00 | AAAA |
| ATOM | 622 | CG | ASP | E | 39 | 59.971 | 109.856 | 51.803 | 1.00 | 0.00 | AAAA |
| ATOM | 623 | OD1 | ASP | E | 39 | 60.374 | 108.688 | 51.610 | 1.00 | 0.00 | AAAA |
| ATOM | 624 | OD2 | ASP | E | 39 | 60.807 | 110.676 | 52.239 | 1.00 | 0.00 | AAAA |
| ATOM | 625 | C | ASP | E | 39 | 58.597 | 110.571 | 49.144 | 1.00 | 0.00 | AAAA |
| ATOM | 626 | O | ASP | E | 39 | 59.712 | 110.723 | 48.652 | 1.00 | 0.00 | AAAA |
| ATOM | 627 | N | TYR | E | 40 | 57.521 | 110.001 | 48.596 | 1.00 | 0.00 | AAAA |
| ATOM | 629 | CA | TYR | E | 40 | 57.504 | 109.220 | 47.376 | 1.00 | 0.00 | AAAA |
| ATOM | 631 | CB | TYR | E | 40 | 56.461 | 108.108 | 47.481 | 1.00 | 0.00 | AAAA |
| ATOM | 634 | CG | TYR | E | 40 | 56.734 | 107.057 | 46.430 | 1.00 | 0.00 | AAAA |
| ATOM | 635 | CD1 | TYR | E | 40 | 55.765 | 106.955 | 45.425 | 1.00 | 0.00 | AAAA |
| ATOM | 637 | CD2 | TYR | E | 40 | 57.777 | 106.129 | 46.523 | 1.00 | 0.00 | AAAA |
| ATOM | 639 | CE1 | TYR | E | 40 | 55.930 | 105.973 | 44.441 | 1.00 | 0.00 | AAAA |
| ATOM | 641 | CE2 | TYR | E | 40 | 57.920 | 105.153 | 45.528 | 1.00 | 0.00 | AAAA |
| ATOM | 643 | CZ | TYR | E | 40 | 57.024 | 105.101 | 44.454 | 1.00 | 0.00 | AAAA |
| ATOM | 644 | OH | TYR | E | 40 | 57.158 | 104.188 | 43.449 | 1.00 | 0.00 | AAAA |
| ATOM | 646 | C | TYR | E | 40 | 57.226 | 110.145 | 46.199 | 1.00 | 0.00 | AAAA |
| ATOM | 647 | O | TYR | E | 40 | 56.430 | 111.071 | 46.344 | 1.00 | 0.00 | AAAA |
| ATOM | 648 | N | ARG | E | 41 | 57.798 | 109.804 | 45.043 | 1.00 | 0.00 | AAAA |
| ATOM | 650 | CA | ARG | E | 41 | 57.623 | 110.401 | 43.734 | 1.00 | 0.00 | AAAA |
| ATOM | 652 | CB | ARG | E | 41 | 58.197 | 109.509 | 42.631 | 1.00 | 0.00 | AAAA |
| ATOM | 655 | CG | ARG | E | 41 | 57.562 | 108.130 | 42.462 | 1.00 | 0.00 | AAAA |
| ATOM | 658 | CD | ARG | E | 41 | 58.291 | 107.232 | 41.462 | 1.00 | 0.00 | AAAA |
| ATOM | 661 | NE | ARG | E | 41 | 59.523 | 106.639 | 41.984 | 1.00 | 0.00 | AAAA |
| ATOM | 663 | CZ | ARG | E | 41 | 60.435 | 105.977 | 41.260 | 1.00 | 0.00 | AAAA |
| ATOM | 664 | NH1 | ARG | E | 41 | 60.339 | 105.836 | 39.931 | 1.00 | 0.00 | AAAA |
| ATOM | 667 | NH2 | ARG | E | 41 | 61.422 | 105.336 | 41.900 | 1.00 | 0.00 | ..AAAA |
| ATOM | 670 | C | ARG | E | 41 | 56.242 | 110.856 | 43.283 | 1.00 | 0.00 | AAAA |
| ATOM | 671 | O | ARG | E | 41 | 55.333 | 110.031 | 43.332 | 1.00 | 0.00 | AAAA |
| ATOM | 672 | N | SER | E | 42 | 56.109 | 112.081 | 42.771 | 1.00 | 0.00 | AAAA |
| ATOM | 674 | CA | SER | E | 42 | 54.910 | 112.626 | 42.164 | 1.00 | 0.00 | AAAA |
| ATOM | 676 | CB | SER | E | 42 | 54.954 | 114.139 | 42.378 | 1.00 | 0.00 | AAAA |
| ATOM | 679 | OG | SER | E | 42 | 56.129 | 114.856 | 42.071 | 1.00 | 0.00 | AAAA |
| ATOM | 681 | C | SER | E | 42 | 54.848 | 112.335 | 40.672 | 1.00 | 0.00 | AAAA |
| ATOM | 682 | O | SER | E | 42 | 55.879 | 112.268 | 40.006 | 1.00 | 0.00 | AAAA |
| ATOM | 683 | N | TYR | E | 43 | 53.631 | 112.107 | 40.172 | 1.00 | 0.00 | AAAA |
| ATOM | 685 | CA | TYR | E | 43 | 53.294 | 111.881 | 38.781 | 1.00 | 0.00 | AAAA |
| ATOM | 687 | CB | TYR | E | 43 | 52.445 | 110.613 | 38.674 | 1.00 | 0.00 | AAAA |
| ATOM | 690 | CG | TYR | E | 43 | 53.080 | 109.335 | 39.165 | 1.00 | 0.00 | AAAA |
| ATOM | 691 | CD1 | TYR | E | 43 | 54.306 | 108.905 | 38.645 | 1.00 | 0.00 | AAAA |
| ATOM | 693 | CD2 | TYR | E | 43 | 52.487 | 108.559 | 40.170 | 1.00 | 0.00 | AAAA |
| ATOM | 695 | CE1 | TYR | E | 43 | 54.967 | 107.780 | 39.153 | 1.00 | 0.00 | AAAA |
| ATOM | 697 | CE2 | TYR | E | 43 | 53.126 | 107.435 | 40.707 | 1.00 | 0.00 | AAAA |
| ATOM | 699 | CZ | TYR | E | 43 | 54.337 | 107.015 | 40.142 | 1.00 | 0.00 | AAAA |
| ATOM | 700 | OH | TYR | E | 43 | 54.830 | 105.782 | 40.449 | 1.00 | 0.00 | AAAA |
| ATOM | 702 | C | TYR | E | 43 | 52.531 | 113.089 | 38.256 | 1.00 | 0.00 | AAAA |
| ATOM | 703 | O | TYR | E | 43 | 52.046 | 113.878 | 39.064 | 1.00 | 0.00 | AAAA |
| ATOM | 704 | N | ARG | E | 44 | 52.345 | 113.213 | 36.940 | 1.00 | 0.00 | AAAA |
| ATOM | 706 | CA | ARG | E | 44 | 51.792 | 114.293 | 36.147 | 1.00 | 0.00 | AAAA |
| ATOM | 708 | CB | ARG | E | 44 | 52.630 | 115.558 | 36.336 | 1.00 | 0.00 | AAAA |
| ATOM | 711 | CG | ARG | E | 44 | 52.331 | 116.698 | 35.362 | 1.00 | 0.00 | AAAA |
| ATOM | 714 | CD | ARG | E | 44 | 53.154 | 117.944 | 35.687 | 1.00 | 0.00 | AAAA |
| ATOM | 717 | NE | ARG | E | 44 | 53.172 | 118.914 | 34.592 | 1.00 | 0.00 | AAAA |
| ATOM | 719 | CZ | ARG | E | 44 | 54.057 | 118.919 | 33.586 | 1.00 | 0.00 | AAAA |
| ATOM | 720 | NH1 | ARG | E | 44 | 54.930 | 117.903 | 33.543 | 1.00 | 0.00 | AAAA |
| ATOM | 723 | NH2 | ARG | E | 44 | 54.146 | 119.876 | 32.651 | 1.00 | 0.00 | AAAA |
| ATOM | 726 | C | ARG | E | 44 | 51.586 | 113.792 | 34.724 | 1.00 | 0.00 | AAAA |
| ATOM | 727 | O | ARG | E | 44 | 52.389 | 113.016 | 34.213 | 1.00 | 0.00 | AAAA |
| ATOM | 728 | N | PHE | E | 45 | 50.442 | 114.166 | 34.145 | 1.00 | 0.00 | AAAA |
| ATOM | 730 | CA | PHE | E | 45 | 49.914 | 113.648 | 32.899 | 1.00 | 0.00 | AAAA |
| ATOM | 732 | CB | PHE | E | 45 | 48.735 | 112.682 | 33.017 | 1.00 | 0.00 | AAAA |
| ATOM | 735 | CG | PHE | E | 45 | 49.131 | 111.579 | 33.970 | 1.00 | 0.00 | AAAA |
| ATOM | 736 | CD1 | PHE | E | 45 | 48.967 | 111.824 | 35.340 | 1.00 | 0.00 | AAAA |
| ATOM | 738 | CD2 | PHE | E | 45 | 49.770 | 110.430 | 33.492 | 1.00 | 0.00 | AAAA |
| ATOM | 740 | CE1 | PHE | E | 45 | 49.523 | 110.877 | 36.210 | 1.00 | 0.00 | AAAA |
| ATOM | 742 | | PHE | E | 45 | 50.276 | 109.502 | 34.410 | 1.00 | 0.00 | AAAA |
| ATOM | 744 | CZ | PHE | E | 45 | 50.183 | 109.716 | 35.791 | 1.00 | 0.00 | AAAA |
| ATOM | 746 | C | PHE | E | 45 | 49.601 | 114.869 | 32.046 | 1.00 | 0.00 | AAAA |
| ATOM | 747 | O | PHE | E | 45 | 48.424 | 115.220 | 31.995 | 1.00 | 0.00 | AAAA |
| ATOM | 748 | N | PRO | E | 46 | 50.580 | 115.555 | 31.454 | 1.00 | 0.00 | AAAA |
| ATOM | 749 | CA | PRO | E | 46 | .50.400 | 116.772 | 30.687 | 1.00 | 0.00 | AAAA |
| ATOM | 751 | CB | PRO | E | 46 | 51.715 | 117.548 | 30.760 | 1.00 | 0.00 | AAAA |
| ATOM | 754 | CG | PRO | E | 46 | 52.724 | 116.401 | 30.815 | 1.00 | 0.00 | AAAA |
| ATOM | 757 | CD | PRO | E | 46 | 52.000 | 115.299 | 31.591 | 1.00 | 0.00 | AAAA |
| ATOM | 760 | C | PRO | E | 46 | 49.953 | 116.450 | 29.268 | 1.00 | 0.00 | AAAA |
| ATOM | 761 | O | PRO | E | 46 | 49.531 | 117.400 | 28.612 | 1.00 | 0.00 | AAAA |
| ATOM | 762 | N | LYS | E | 47 | 50.238 | 115.245 | 28.769. | 1.00 | 0.00 | AAAA |
| ATOM | 764 | CA | LYS | E | 47 | 49.906 | 114.937 | 27.393 | 1.00 | 0.00 | AAAA |
| ATOM | 766 | CB | LYS | E | 47 | 50.637 | 113.633 | 27.066 | 1.00 | 0.00 | AAAA |
| ATOM | 769 | CG | LYS | E | 47 | 52.135 | 113.742 | 27.352 | 1.00 | 0.00 | AAAA |
| ATOM | 772 | CD | LYS | E | 47 | 52.714 | 112.327 | 27.380 | 1.00 | 0.00 | AAAA |
| ATOM | 775 | CE | LYS | E | 47 | 54.073 | 112.156 | 28.061 | 1.00 | 0.00 | AAAA |
| ATOM | 778 | NZ | LYS | E | 47 | 54.498 | 110.756 | 28.205 | 1.00 | 0.00 | AAAA |
| ATOM | 782 | C | LYS | E | 47 | 48.414 | 114.701 | 27.205 | 1.00 | 0.00 | AAAA |
| ATOM | 783 | O | LYS | E | 47 | 47.950 | 114.578 | 26.073 | 1.00 | 0.00 | AAAA |
| ATOM | 784 | N | LEU | E | 48 | 47.662 | 114.542 | 28.296 | 1.00 | 0.00 | AAAA |
| ATOM | 786 | CA | LEU | E | 48 | 46.224 | 114.402 | 28.411 | 1.00 | 0.00 | AAAA |
| ATOM | 788 | CB | LEU | E | 48 | 45.945 | 113.901 | 29.828 | 1.00 | 0.00 | AAAA |
| ATOM | 791 | CG | LEU | E | 48 | 44.590 | 113.243 | 30.090 | 1.00 | 0.00 | AAAA |
| ATOM | 793 | CD1 | LEU | E | 48 | 44.703 | 112.310 | 31.297 | 1.00 | 0.00 | AAAA |
| ATOM | 797 | CD2 | LEU | E | 48 | 43.623 | 114.349 | 30.518 | 1.00 | 0.00 | AAAA |
| ATOM | 801 | C | LEU | E | 48 | 45.520 | 115.724 | 28.141 | 1.00 | 0.00 | AAAA |
| ATOM | 802 | O | LEU | E | 48 | 45.913 | 116.724 | 28.739 | 1.00 | 0.00 | AAAA |
| ATOM | 803 | N | THR | E | 49 | 44.470 | 115.750 | 27.318 | 1.00 | 0.00 | AAAA |
| ATOM | 805 | CA | THR | E | 49 | 43.825 | 116.887 | 26.692 | 1.00 | 0.00 | AAAA |
| ATOM | 807 | CB | THR | E | 49 | 44.351 | 117.107 | 25.273 | 1.00 | 0.00 | AAAA |
| ATOM | 809 | OG1 | THR | E | 49 | 44.134 | 116.006 | 24.419 | 1.00 | 0.00 | AAAA |
| ATOM | 811 | CG2 | THR | E | 49 | 45.756 | 117.706 | 25.334 | 1.00 | 0.00 | AAAA |
| ATOM | 815 | C | THR | E | 49 | 42.306 | 116.822 | 26.771 | 1.00 | 0.00 | AAAA |
| ATOM | 816 | O | THR | E | 49 | 41.723 | 117.862 | 27.072 | 1.00 | 0.00 | AAAA |
| ATOM | 817 | N | VAL | E | 50 | 41.733 | 115.637 | 26.547 | 1.00 | 0.00 | AAAA |
| ATOM | 819 | CA | VAL | E | 50 | 40.304 | 115.400 | 26.562 | 1.00 | 0.00 | AAAA |
| ATOM | 821 | CB | VAL | E | 50 | 39.698 | 115.425 | 25.159 | 1.00 | 0.00 | AAAA |
| ATOM | 823 | CG1 | VAL | E | 50 | 38.174 | 115.295 | 25.167 | 1.00 | 0.00 | AAAA |
| ATOM | 827 | CG2 | VAL | E | 50 | 40.076 | 116.614 | 24.275 | 1.00 | 0.00 | AAAA |
| ATOM | 831 | C | VAL | E | 50 | 39.980 | 114.120 | 27.319 | 1.00 | 0.00 | AAAA |
| ATOM | 832 | O | VAL | E | 50 | 40.718 | 113.140 | 27.252 | 1.00 | 0.00 | AAAA |
| ATOM | 833 | N | ILE | E | 51 | 38.848 | 114.201 | 28.023 | 1.00 | 0.00 | AAAA |
| ATOM | 835 | CA | ILE | E | 51 | 38.111 | 113.064 | 28.535 | 1.00 | 0.00 | AAAA |
| ATOM | 837 | CB | ILE | E | 51 | 37.955 | 113.033 | 30.056 | 1.00 | 0.00 | AAAA |
| ATOM | 839 | CG1 | ILE | E | 51 | 39.297 | 112.998 | 30.786 | 1.00 | 0.00 | AAAA |
| ATOM | 842 | CG2 | ILE | E | 51 | 37.097 | 111.874 | 30.563 | 1.00 | 0.00 | AAAA |
| ATOM | 846 | CD | ILE | E | 51 | 39.404 | 113.259 | 32.289 | 1.00 | 0.00 | AAAA |
| ATOM | 850 | C | ILE | E | 51 | 36.765 | 113.109 | 27.827 | 1.00 | 0.00 | AAAA |
| ATOM | 851 | O | ILE | E | 51 | 36.115 | 114.152 | 27.837 | 1.00 | 0.00 | AAAA |
| ATOM | 852 | N | THR | E | 52 | 36.277 | 112.036 | 27.200 | 1.00 | 0.00 | AAAA |
| ATOM | 854 | CA | THR | E | 52 | 35.056 | 112.103 | 26.423 | 1.00 | 0.00 | AAAA |
| ATOM | 856 | CB | THR | E | 52 | 35.084 | 110.967 | 25.400 | 1.00 | 0.00 | AAAA |
| ATOM | 858 | OG1 | THR | E | 52 | 35.009 | 109.701 | 26.017 | 1.00 | 0.00 | AAAA |
| ATOM | 860 | CG2 | THR | E | 52 | 36.258 | 110.925 | 24.421 | 1.00 | 0.00 | AAAA |
| ATOM | 864 | C | THR | E | 52 | 33.777 | 112.147 | 27.247 | 1.00 | 0.00 | AAAA |
| ATOM | 865 | O | THR | E | 52 | 32.849 | 112.907 | 26.976 | 1.00 | 0.00 | AAAA |
| ATOM | 866 | N | GLU | E | 53 | 33.640 | 111.205 | 28.183 | 1.00 | 0.00 | AAAA |
| ATOM | 868 | CA | GLU | E | 53 | 32.500 | 111.142 | 29.075 | 1.00 | 0.00 | AAAA |
| ATOM | 870 | CB | GLU | E | 53 | 32.434 | 109.682 | 29.525 | 1.00 | 0.00 | AAAA |
| ATOM | 873 | CG | GLU | E | 53 | 31.989 | 108.840 | 28.327 | 1.00 | 0.00 | AAAA |
| ATOM | 876 | CD | GLU | E | 53 | 31.782 | 107.351 | 28.560 | 1.00 | 0.00 | AAAA |
| ATOM | 877 | OE1 | GLU | E | 53 | 31.399 | 106.867 | 29.647 | 1.00 | 0.00 | AAAA |
| ATOM | 878 | OE2 | GLU | E | 53 | 32.077 | 106.587 | 27.615 | 1.00 | 0.00 | AAAA |
| ATOM | 879 | C | GLU | E | 53 | 32.564 | 112.089 | 30.265 | 1.00 | 0.00 | AAAA |
| ATOM | 880 | O | GLU | E | 53 | 31.949 | 113.144 | 30.126 | 1.00 | 0.00 | AAAA |
| ATOM | 881 | N | TYR | E | 54 | 33.088 | 111.648 | 31.412 | 1.00 | 0.00 | AAAA |
| ATOM | 883 | CA | TYR | E | 54 | 33.107 | 112.329 | 32.690 | 1.00 | 0.00 | AAAA |
| ATOM | 885 | CB | TYR | E | 54 | 31.766 | 112.092 | 33.388 | 1.00 | 0.00 | AAAA |
| ATOM | 888 | CG | TYR | E | 54 | 31.607 | 110.684 | 33.910 | 1.00 | 0.00 | AAAA |
| ATOM | 889 | CD1 | TYR | E | 54 | 31.987 | 110.383 | 35.223 | 1.00 | 0.00 | AAAA |
| ATOM | 891 | CD2 | TYR | E | 54 | 31.043 | 109.669 | 33.127 | 1.00 | 0.00 | AAAA |
| ATOM | 893 | CE1 | TYR | E | 54 | 31.734 | 109.100 | 35.724 | 1.00 | 0.00 | AAAA |
| ATOM | 895 | CE2 | TYR | E | 54 | 30.878 | 108.366 | 33.611 | 1.00 | 0.00 | AAAA |
| ATOM | 897 | CZ | TYR | E | 54 | 31.214 | 108.067 | 34.936 | 1.00 | 0.00 | AAAA |
| ATOM | 898 | OH | TYR | E | 54 | 31.017 | 106.844 | 35.509 | 1.00 | 0.00 | AAAA |
| ATOM | 900 | C | TYR | E | 54 | 34.324 | 111.982 | 33.535 | 1.00 | 0.00 | AAAA |
| ATOM | 901 | O | TYR | E | 54 | 34.978 | 110.948 | 33.417 | 1.00 | 0.00 | AAAA |
| ATOM | 902 | N | LEU | E | 55 | 34.655 | 112.885 | 34.462 | 1.00 | 0.00 | AAAA |
| ATOM | 904 | CA | LEU | E | 55 | 35.615 | 112.830 | 35.545 | 1.00 | 0.00 | AAAA |
| ATOM | 906 | CB | LEU | E | 55 | 36.603 | 113.979 | 35.342 | 1.00 | 0.00 | AAAA |
| ATOM | 909 | CG | LEU | E | 55 | 37.656 | 114.149 | 36.437 | 1.00 | 0.00 | AAAA |
| ATOM | 911 | CD1 | LEU | E | 55 | 38.376 | 112.904 | 36.956 | 1.00 | 0.00 | AAAA |
| ATOM | 915 | CD2 | LEU | E | 55 | 38.743 | 115.098 | 35.930 | 1.00 | 0.00 | AAAA |
| ATOM | 919 | C | LEU | E | 55 | 34.849 | 112.899 | 36.859 | 1.00 | 0.00 | AAAA |
| ATOM | 920 | O | LEU | E | 55 | 34.087 | 113.814 | 37.166 | 1.00 | 0.00 | AAAA |
| ATOM | 921 | N | LEU | E | 56 | 35.081 | 111.878 | 37.687 | 1.00 | 0.00 | AAAA |
| ATOM | 923 | CA | LEU | E | 56 | 34.719 | 111.758 | 39.085 | 1.00 | 0.00 | AAAA |
| ATOM | 925 | CB | LEU | E | 56 | 33.819 | 110.586 | 39.475 | 1.00 | 0.00 | AAAA |
| ATOM | 928 | CG | LEU | E | 56 | 33.505 | 110.476 | 40.967 | 1.00 | 0.00 | AAAA |
| ATOM | 930 | CD1 | LEU | E | 56 | 31.982 | 110.556 | 41.078 | 1.00 | 0.00 | AAAA |
| ATOM | 934 | CD2 | LEU | E | 56 | 33.985 | 109.198 | 41.658 | 1.00 | 0.00 | AAAA |
| ATOM | 938 | C | LEU | E | 56 | 35.995 | 111.695 | 39.913 | 1.00 | 0.00 | AAAA |
| ATOM | 939 | O | LEU | E | 56 | 36.803 | 110.811 | 39.636 | 1.00 | 0.00 | AAAA |
| ATOM | 940 | N | LEU | E | 57 | 36.116 | 112.581 | 40.905 | 1.00 | 0.00 | AAAA |
| ATOM | 942 | CA | LEU | E | 57 | 37.247 | 112.625 | 41.810 | 1.00 | 0.00 | AAAA |
| ATOM | 944 | CB | LEU | E | 57 | 38.134 | 113.842 | 41.544 | 1.00 | 0.00 | AAAA |
| ATOM | 947 | CG | LEU | E | 57 | 39.512 | 113.584 | 40.935 | 1.00 | 0.00 | AAAA |
| ATOM | 949 | CD1 | LEU | E | 57 | 40.101 | 114.837 | 40.285 | 1.00 | 0.00 | AAAA |
| ATOM | 953 | CD2 | LEU | E | 57 | 40.453 | 112.846 | 41.888 | 1.00 | 0.00 | AAAA |
| ATOM | 957 | C | LEU | E | 57 | 36.742 | 112.597 | 43.245 | 1.00 | 0.00 | AAAA |
| ATOM | 958 | O | LEU | E | 57 | 36.012 | 113.539 | 43.550 | 1.00 | 0.00 | AAAA |
| ATOM | 959 | N | PHE | E | 58 | 37.013 | 111.581 | 44.068 | 1.00 | 0.00 | AAAA |
| ATOM | 961 | CA | PHE | E | 58 | 36.377 | 111.377 | 45.354 | 1.00 | 0.00 | AAAA |
| ATOM | 963 | CB | PHE | E | 58 | 35.196 | 110.412 | 45.243 | 1.00 | 0.00 | AAAA |
| ATOM | 966 | CG | PHE | E | 58 | 34.613 | 109.749 | 46.469 | 1.00 | 0.00 | AAAA |
| ATOM | 967 | CD1 | PHE | E | 58 | 34.157 | 110.538 | 47.532 | 1.00 | 0.00 | AAAA |
| ATOM | 969 | CD2 | PHE | E | 58 | 34.356 | 108.373 | 46.453 | 1.00 | 0.00 | AAAA |
| ATOM | 971 | CE1 | PHE | E | 58 | 33.513 | 109.946 | 48.625 | 1.00 | 0.00 | AAAA |
| ATOM | 973 | CE2 | PHE | E | 58 | 33.844 | 107.769 | 47.608 | 1.00 | 0.00 | AAAA |
| ATOM | 975 | CZ | PHE | E | 58 | 33.398 | 108.551 | 48.680 | 1.00 | 0.00 | AAAA |
| ATOM | 977 | C | PHE | E | 58 | 37.403 | 110.977 | 46.404 | 1.00 | 0.00 | AAAA |
| ATOM | 978 | O | PHE | E | 58 | 38.063 | 109.945 | 46.316 | 1.00 | 0.00 | AAAA |
| ATOM | 979 | N | ARG | E | 59 | 37.458 | 111.779 | 47.471 | 1.00 | 0.00 | AAAA |
| ATOM | 981 | CA | ARG | E | 59 | 37.997 | 111.422 | 48.767 | 1.00 | 0.00 | AAAA |
| ATOM | 983 | CB | ARG | E | 59 | 37.150 | 110.352 | 49.459 | 1.00 | 0.00 | AAAA |
| ATOM | 986 | CG | ARG | E | 59 | 37.359 | 109.996 | 50.931 | 1.00 | 0.00 | AAAA |
| ATOM | 989 | CD | ARG | E | 59 | 36.258 | 109.056 | 51.420 | 1.00 | 0.00 | AAAA |
| ATOM | 992 | NE | ARG | E | 59 | 36.528 | 108.517 | 52.753 | 1.00 | 0.00 | AAAA |
| ATOM | 994 | CZ | ARG | E | 59 | 35.749 | 108.671 | 53.833 | 1.00 | 0.00 | AAAA |
| ATOM | 995 | NH1 | ARG | E | 59 | 34.750 | 109.564 | 53.832 | 1.00 | 0.00 | AAAA |
| ATOM | 998 | NH2 | ARG | E | 59 | 35.931 | 108.009 | 54.983 | 1.00 | 0.00 | AAAA |
| ATOM | 1001 | C | ARG | E | 59 | 39.481 | 111.092 | 48.690 | 1.00 | 0.00 | AAAA |
| ATOM | 1002 | O | ARG | E | 59 | 39.963 | 110.249 | 49.445 | 1.00 | 0.00 | AAAA |
| ATOM | 1003 | N | VAL | E | 60 | 40.180 | 111.739 | 47.755 | 1.00 | 0.00 | AAAA |
| ATOM | 1005 | CA | VAL | E | 60 | 41.600 | 111.585 | 47.509 | 1.00 | 0.00 | AAAA |
| ATOM | 1007 | CB | VAL | E | 60 | 41.935 | 111.884 | 46.047 | 1.00 | 0.00 | AAAA |
| ATOM | 1009 | CG1 | VAL | E | 60 | 43.408 | 111.557 | 45.808 | 1.00 | 0.00 | AAAA |
| ATOM | 1013 | CG2 | VAL | E | 60 | 41.109 | 111.102 | 45.025 | 1.00 | 0.00 | AAAA |
| ATOM | 1017 | C | VAL | E | 60 | 42.298 | 112.550 | 48.456 | 1.00 | 0.00 | AAAA |
| ATOM | 1018 | O | VAL | E | 60 | 41.803 | 113.621 | 48.803 | 1.00 | 0.00 | AAAA |
| ATOM | 1019 | N | ALA | E | 61 | 43.436 | 112.123 | 49.006 | 1.00 | 0.00 | AAAA |
| ATOM | 1021 | CA | ALA | E | 61 | 44.327 | 112.885 | 49.859 | 1.00 | 0.00 | AAAA |
| ATOM | 1023 | CB | ALA | E | 61 | 44.452 | 112.272 | 51.254 | 1.00 | 0.00 | AAAA |
| ATOM | 1027 | C | ALA | E | 61 | 45.680 | 113.025 | 49.174 | 1.00 | 0.00 | AAAA |
| ATOM | 1028 | O | ALA | E | 61 | 46.156 | 112.060 | 48.580 | 1.00 | 0.00 | AAAA |
| ATOM | 1029 | N | GLY | E | 62 | 46.344 | 114.175 | 49.314 | 1.00 | 0.00 | AAAA |
| ATOM | 1031 | CA | GLY | E | 62 | 47.741 | 114.294 | 48.944 | 1.00 | 0.00 | AAAA |
| ATOM | 1034 | C | GLY | E | 62 | 47.851 | 115.151 | 47.692 | 1.00 | 0.00 | AAAA |
| ATOM | 1035 | O | GLY | E | 62 | 48.958 | 115.608 | 47.417 | 1.00 | 0.00 | AAAA |
| ATOM | 1036 | N | LEU | E | 63 | 46.801 | 115.429 | 46.915 | 1.00 | 0.00 | AAAA |
| ATOM | 1038 | CA | LEU | E | 63 | 46.821 | 116.414 | 45.853 | 1.00 | 0.00 | AAAA. |
| ATOM | 1040 | CB | LEU | E | 63 | 45.772 | 115.993 | 44.823 | 1.00 | 0.00 | AAAA |
| ATOM | 1043 | CG | LEU | E | 63 | 45.942 | 114.594 | 44.230 | 1.00 | 0.00 | AAAA |
| ATOM | 1045 | CD1 | LEU | E | 63 | 44.804 | 114.155 | 43.308 | 1.00 | 0.00 | AAAA |
| ATOM | 1049 | CD2 | LEU | E | 63 | 47.280 | 114.372 | 43.527 | 1.00 | 0.00 | AAAA |
| ATOM | 1053 | C | LEU | E | 63 | 46.348 | 117.714 | 46.488 | 1.00 | 0.00 | AAAA |
| ATOM | 1054 | O | LEU | E | 63 | 45.233 | 117.874 | 46.979 | 1.00 | 0.00 | AAAA |
| ATOM | 1055 | N | GLU | E | 64 | 47.261 | 118.683 | 46.388 | 1.00 | 0.00 | AAAA |
| ATOM | 1057 | CA | GLU | E | 64 | 47.020 | 120.054 | 46.793 | 1.00 | 0.00 | AAAA |
| ATOM | 1059 | CB | GLU | E | 64 | 48.346 | 120.695 | 47.205 | 1.00 | 0.00 | AAAA |
| ATOM | 1062 | CG | GLU | E | 64 | 48.315 | 121.927 | 48.108 | 1.00 | 0.00 | AAAA |
| ATOM | 1065 | CD | GLU | E | 64 | 49.707 | 122.374 | 48.530 | 1.00 | 0.00 | AAAA |
| ATOM | 1066 | OE1 | GLU | E | 64 | 50.042 | 122.273 | 49.731 | 1.00 | 0.00 | AAAA |
| ATOM | 1067 | OE2 | GLU | E | 64 | 50.452 | 122.779 | 47.611 | 1.00 | 0.00 | AAAA |
| ATOM | 1068 | C | GLU | E | 64 | 46.381 | 120.815 | 45.641 | 1.00 | 0.00 | AAAA |
| ATOM | 1069 | O | GLU | E | 64 | 45.634 | 121.767 | 45.854 | 1.00 | 0.00 | AAAA |
| ATOM | 1070 | N | SER | E | 65 | 46.599 | 120.407 | 44.388 | 1.00 | 0.00 | AAAA |
| ATOM | 1072 | CA | SER | E | 65 | 45.979 | 121.026 | 43.235 | 1.00 | 0.00 | AAAA |
| ATOM | 1074 | CB | SER | E | 65 | 46.759 | 122.256 | 42.769 | 1.00 | 0.00 | AAAA |
| ATOM | 1077 | OG | SER | E | 65 | 46.030 | 123.141 | 41.949 | 1.00 | 0.00 | AAAA |
| ATOM | 1079 | C | SER | E | 65 | 45.924 | 119.935 | 42.174 | 1.00 | 0.00 | AAAA |
| ATOM | 1080 | O | SER | E | 65 | 46.889 | 119.201 | 41.980 | 1.00 | 0.00 | AAAA |
| ATOM | 1081 | N | LEU | E | 66 | 44.820 | 119.793 | 41.436 | 1.00 | 0.00 | AAAA |
| ATOM | 1083 | CA | LEU | E | 66 | 44.722 | 118.920 | 40.284 | 1.00 | 0.00 | AAAA |
| ATOM | 1085 | CB | LEU | E | 66 | 43.253 | 118.747 | 39.896 | 1.00 | 0.00 | AAAA |
| ATOM | 1088 | CG | LEU | E | 66 | 42.295 | 118.357 | 41.022 | 1.00 | 0.00 | AAAA |
| ATOM | 1090 | CD1 | LEU | E | 66 | 40.875 | 118.346 | 40.452 | 1.00 | 0.00 | AAAA |
| ATOM | 1094 | CD2 | LEU | E | 66 | 42.591 | 116.967 | 41.586 | 1.00 | 0.00 | AAAA |
| ATOM | 1098 | C | LEU | E | 66 | 45.585 | 119.366 | 39.112 | 1.00 | 0.00 | AAAA |
| ATOM | 1099 | O | LEU | E | 66 | 45.710 | 118.557 | 38.194 | 1.00 | 0.00 | AAAA |
| ATOM | 1100 | N | GLY | E | 67 | 46.134 | 120.582 | 39.151 | 1.00 | 0.00 | AAAA |
| ATOM | 1102 | CA | GLY | E | 67 | 46.877 | 120.993 | 37.977 | 1.00 | 0.00 | AAAA |
| ATOM | 1105 | C | GLY | E | 67 | 48.290 | 120.427 | 37.958 | 1.00 | 0.00 | AAAA |
| ATOM | 1106 | O | GLY | E | 67 | 48.950 | 120.368 | 36.923 | 1.00 | 0.00 | AAAA |
| ATOM | 1107 | N | ASP | E | 68 | 48.751 | 119.952 | 39.117 | 1.00 | 0.00 | AAAA |
| ATOM | 1109 | CA | ASP | E | 68 | 49.998 | 119.248 | 39.341 | 1.00 | 0.00 | AAAA |
| ATOM | 1111 | CB | ASP | E | 68 | 50.492 | 119.612 | 40.742 | 1.00 | 0.00 | AAAA |
| ATOM | 1114 | CG | ASP | E | 68 | 51.090 | 121.009 | 40.824 | 1.00 | 0.00 | AAAA |
| ATOM | 1115 | OD1 | ASP | E | 68 | 50.479 | 121.925 | 41.415 | 1.00 | 0.00 | AAAA |
| ATOM | 1116 | OD2 | ASP | E | 68 | 52.192 | 121.311 | 40.317 | 1.00 | 0.00 | AAAA |
| ATOM | 1117 | C | ASP | E | 68 | 49.811 | 117.744 | 39.207 | 1.00 | 0.00 | AAAA |
| ATOM | 1118 | O | ASP | E | 68 | 50.625 | 116.961 | 39.694 | 1.00 | 0.00 | AAAA |
| ATOM | 1119 | N | LEU | E | 69 | 48.735 | 117.344 | 38.527 | 1.00 | 0.00 | AAAA |
| ATOM | 1121 | CA | LEU | E | 69 | 48.498 | 116.006 | 38.024 | 1.00 | 0.00 | AAAA |
| ATOM | 1123 | CB | LEU | E | 69 | 47.538 | 115.324 | 39.000 | 1.00 | 0.00 | AAAA |
| ATOM | 1126 | CG | LEU | E | 69 | 47.117 | 113.887 | 38.697 | 1.00 | 0.00 | AAAA |
| ATOM | 1128 | CD1 | LEU | E | 69 | 48.238 | 112.850 | 38.772 | 1.00 | 0.00 | AAAA |
| ATOM | 1132 | CD2 | LEU | E | 69 | 45.948 | 113.386 | 39.547 | 1.00 | 0.00 | AAAA |
| ATOM | 1136 | C | LEU | E | 69 | 47.967 | 116.164 | 36.606 | 1.00 | 0.00 | AAAA |
| ATOM | 1137 | O | LEU | E | 69 | 48.380 | 115.424 | 35.717 | 1.00 | 0.00 | AAAA |
| ATOM | 1138 | N | PHE | E | 70 | 47.051 | 117.091 | 36.318 | 1.00 | 0.00 | AAAA |
| ATOM | 1140 | CA | PHE | E | 70 | 46.496 | 117.388 | 35.013 | 1.00 | 0.00 | AAAA |
| ATOM | 1142 | CB | PHE | E | 70 | 45.043 | 116.918 | 35.099 | 1.00 | 0.00 | AAAA |
| ATOM | 1145 | CG | PHE | E | 70 | 44.847 | 115.479 | 35.511 | 1.00 | 0.00 | AAAA |
| ATOM | 1146 | CD1 | PHE | E | 70 | 45.598 | 114.477 | 34.884 | 1.00 | 0.00 | AAAA |
| ATOM | 1148 | CD2 | PHE | E | 70 | 43.819 | 115.104 | 36.385 | 1.00 | 0.00 | AAAA |
| ATOM | 1150 | CE1 | PHE | E | 70 | 45.416 | 113.117 | 35.158 | 1.00 | 0.00 | AAAA |
| ATOM | 1152 | CE2 | PHE | E | 70 | 43.618 | 113.744 | 36.650 | 1.00 | 0.00 | AAAA |
| ATOM | 1154 | CZ | PHE | E | 70 | 44.472 | 112.765 | 36.130 | 1.00 | 0.00 | AAAA |
| ATOM | 1156 | C | PHE | E | 70 | 46.500 | 118.869 | 34.661 | 1.00 | 0.00 | AAAA |
| ATOM | 1157 | O | PHE | E | 70 | 45.523 | 119.574 | 34.901 | 1.00 | 0.00 | AAAA |
| ATOM | 1158 | N | PRO | E | 71 | 47.625 | 119.427 | 34.206 | 1.00 | 0.00 | AAAA |
| ATOM | 1159 | CA | PRO | E | 71 | 47.642 | 120.800 | 33.743 | 1.00 | 0.00 | AAAA |
| ATOM | 1161 | CB | PRO | E | 71 | 49.142 | 121.089 | 33.657 | 1.00 | 0.00 | AAAA |
| ATOM | 1164 | CG | PRO | E | 71 | 49.788 | 119.724 | 33.415 | 1.00 | 0.00 | AAAA |
| ATOM | 1167 | CD | PRO | E | 71 | 48.838 | 118.669 | 33.982 | 1.00 | 0.00 | AAAA |
| ATOM | 1170 | C | PRO | E | 71 | 46.977 | 121.078 | 32.403 | 1.00 | 0.00 | AAAA |
| ATOM | 1171 | O | PRO | E | 71 | 46.651 | 122.243 | 32.192 | 1.00 | 0.00 | AAAA |
| ATOM | 1172 | N | ASN | E | 72 | 46.653 | 120.091 | 31.563 | 1.00 | 0.00 | AAAA |
| ATOM | 1174 | CA | ASN | E | 72 | 46.169 | 120.399 | 30.232 | 1.00 | 0.00 | AAAA |
| ATOM | 1176 | CB | ASN | E | 72 | 47.339 | 120.253 | 29.260 | 1.00 | 0.00 | AAAA |
| ATOM | 1179 | CG | ASN | E | 72 | 47.265 | 121.152 | 28.035 | 1.00 | 0.00 | AAAA |
| ATOM | 1180 | OD1 | ASN | E | 72 | 47.043 | 120.706 | 26.911 | 1.00 | 0.00 | AAAA |
| ATOM | 1181 | ND2 | .ASN | E | 72 | 47.495 | 122.462 | 28.153 | 1.00 | 0.00 | AAAA |
| ATOM | 1184 | C | ASN | E | 72 | 44.845 | 119.750 | 29.849 | 1.00 | 0.00 | AAAA |
| ATOM | 1185 | O | ASN | E | 72 | 44.591 | 119.559 | 28.662 | 1.00 | 0.00 | AAAA |
| ATOM | 1186 | N | LEU | E | 73 | 44.056 | 119.252 | 30.804 | 1.00 | 0.00 | AAAA |
| ATOM | 1188 | CA | LEU | E | 73 | 42.725 | 118.711 | 30.619 | 1.00 | 0.00 | AAAA |
| ATOM | 1190 | CB | LEU | E | 73 | 42.379 | 118.014 | 31.935 | 1.00 | 0.00 | AAAA |
| ATOM | 1193 | CG | LEU | E | 73 | 40.939 | 117.505 | 32.000 | 1.00 | 0.00 | AAAA |
| ATOM | 1195 | CD1 | LEU | E | 73 | 40.512 | 116.359 | 31.083 | 1.00 | 0.00 | AAAA |
| ATOM | 1199 | CD2 | LEU | E | 73 | 40.663 | 116.998 | 33.415 | 1.00 | 0.00 | AAAA |
| ATOM | 1203 | C | LEU | E | 73 | 41.813 | 119.834 | 30.147 | 1.00 | 0.00 | AAAA |
| ATOM | 1204 | O | LEU | E | 73 | 41.570 | 120.776 | 30.898 | 1.00 | 0.00 | AAAA |
| ATOM | 1205 | N | THR | E | 74 | 41.369 | 119.923 | 28.891 | 1.00 | 0.00 | AAAA |
| ATOM | 1207 | CA | THR | E | 74 | 40.874 | 121.143 | 28.287 | 1.00 | 0.00 | AAAA |
| ATOM | 1209 | CB | THR | E | 74 | 41.553 | 121.326 | 26.930 | 1..00 | 0.00 | AAAA |
| ATOM | 1211 | OG1 | THR | E | 74 | 42.956 | 121.210 | 27.012 | 1.00 | 0.00 | AAAA |
| ATOM | 1213 | CG2 | THR | E | 74 | 41.250 | 122.690 | 26.308 | 1.00 | 0.00 | AAAA |
| ATOM | 1217 | C | THR | E | 74 | 39.380 | 120.989 | 28.036 | 1.00 | 0.00 | AAAA |
| ATOM | 1218 | O | THR | E | 74 | 38.555 | 121.893 | 28.153 | 1.00 | 0.00 | AAAA |
| ATOM | 1219 | N | VAL | E | 75 | 39.000 | 119.735 | 27.778 | 1.00 | 0.00 | AAAA |
| ATOM | 1221 | CA | VAL | E | 75 | 37.621 | 119.390 | 27.500 | 1.00 | 0.00 | AAAA |
| ATOM | 1223 | CB | VAL | E | 75 | 37.448 | 119.149 | 26.001 | 1.00 | 0.00 | AAAA |
| ATOM | 1225 | CG1 | VAL | E | 75 | 36.028 | 118.701 | 25.653 | 1.00 | 0.00 | AAAA |
| ATOM | 1229 | CG2 | VAL | E | 75 | 37.986 | 120.315 | 25.171 | 1.00 | 0.00 | AAAA |
| ATOM | 1233 | C | VAL | E | 75 | 37.228 | 118.165 | 28.314 | 1.00 | 0.00 | AAAA |
| ATOM | 1234 | O | VAL | E | 75 | 38.042 | 117.253 | 28.441 | 1.00 | 0.00 | AAAA |
| ATOM | 1235 | N | ILE | E | 76 | 36.058 | 118.163 | 28.957 | 1.00 | 0.00 | AAAA |
| ATOM | 1237 | CA | ILE | E | 76 | 35.363 | 117.023 | 29.520 | 1.00 | 0.00 | AAAA |
| ATOM | 1239 | CB | ILE | E | 76 | 35.223 | 117.188 | 31.033 | 1.00 | 0.00 | AAAA |
| ATOM | 1241 | CG1 | ILE | E | 76 | 36.531 | 117.638 | 31.686 | 1.00 | 0.00 | AAAA |
| ATOM | 1244 | CG2 | ILE | E | 76 | 34.565 | 115.945 | 31.634 | 1.00 | 0.00 | AAAA |
| ATOM | 1248 | CD | ILE | E | 76 | 36.523 | 117.790 | 33.208 | 1.00 | 0.00 | AAAA |
| ATOM | 1252 | C | ILE | E | 76 | 34.105 | 116.950 | 28.667 | 1.00 | 0.00 | AAAA |
| ATOM | 1253 | O | ILE | E | 76 | 33.204 | 117.733 | 28.966 | 1.00 | 0.00 | AAAA |
| ATOM | 1254 | N | ARG | E | 77 | 34.100 | 116.237 | 27.539 | 1.00 | 0.00 | AAAA |
| ATOM | 1256 | CA | ARG | E | 77 | 33.252 | 116.522 | 26.399 | 1.00 | 0.00 | AAAA |
| ATOM | 1258 | CB | ARG | E | 77 | 33.702 | 115.648 | 25.228 | 1.00 | 0.00 | AAAA |
| ATOM | 1261 | CG | ARG | E | 77 | 33.065 | 115.833 | 23.851 | 1.00 | 0.00 | AAAA |
| ATOM | 1264 | CD | ARG | E | 77 | 33:823 | 115.167 | 22.702 | 1.00 | 0.00 | AAAA |
| ATOM | 1267 | NE | ARG | E | 77 | 33.016 | 114.337 | 21.809 | 1.00 | 0.00 | AAAA |
| ATOM | 1269 | CZ | ARG | E | 77 | 33.278 | 113.072 | 21.450 | 1.00 | 0.00 | AAAA |
| ATOM | 1270 | NH1 | ARG | E | 77 | 34.464 | 112.500 | 21.700 | 1.00 | 0.00 | AAAA |
| ATOM | 1273 | NH2 | ARG | E | 77 | 32.354 | 112.352 | 20.801 | 1.00 | 0.00 | AAAA |
| ATOM | 1276 | C | ARG | E | 77 | 31.767 | 116.322 | 26.666 | 1.00 | 0.00 | AAAA |
| ATOM | 1277 | O | ARG | E | 77 | 31.050 | 117.280 | 26.384 | 1.00 | 0.00 | AAAA |
| ATOM | 1278 | N | GLY | E | 78 | 31.375 | 115.277 | 27.398 | 1.00 | 0.00 | AAAA |
| ATOM | 1280 | CA | GLY | E | 78 | 30.128 | 115.132 | 28.121 | 1.00 | 0.00 | AAAA |
| ATOM | 1283 | C | GLY | E | 78 | 29.242 | 114.056 | 27.511 | 1.00 | 0.00 | AAAA |
| ATOM | 1284 | O | GLY | E | 78 | 28.016 | 113.995 | 27.573 | 1.00 | 0.00 | AAAA |
| ATOM | 1285 | N | TRP | E | 79 | 29.884 | 113.125 | 26.801 | 1.00 | 0.00 | AAAA |
| ATOM | 1287 | CA | TRP | E | 79 | 29.398 | 112.069 | 25.936 | 1.00 | 0.00 | AAAA |
| ATOM | 1289 | CB | TRP | E | 79 | 30.619 | 111.462 | 25.243 | 1.00 | 0.00 | AAAA |
| ATOM | 1292 | CG | TRP | E | 79 | 30.316 | 110..666 | 24.015 | 1.00 | 0.00 | AAAA |
| ATOM | 1293 | CD1 | TRP | E | 79 | 30.248 | 111.199 | 22.775 | 1.00 | 0.00 | AAAA |
| ATOM | 1295 | CD2 | TRP | E | 79 | 30.219 | 109.224 | 23.810 | 1.00 | 0.00 | AAAA |
| ATOM | 1296 | NE1 | TRP | E | 79 | 29.827 | 110.250 | 21.864 | 1.00 | 0.00 | AAAA |
| ATOM | 1298 | CE2 | TRP | E | 79 | 30.077 | 109.009 | 22.415 | 1.00 | 0.00 | AAAA |
| ATOM | 1299 | CE3 | TRP | E | 79 | 30.433 | 108.111 | 24.639 | 1.00 | 0.00 | AAAA |
| ATOM | 1301 | CZ2 | TRP | E | 79 | 29.954 | 107.717 | 21.890 | 1.00 | 0.00 | AAAA |
| ATOM | 1303 | CZ3 | TRP | E | 79 | 30.448 | 106.809 | 24.125 | 1.00 | 0.00 | AAAA |
| ATOM | 1305 | CH2 | TRP | E | 79 | 30.143 | 106.643 | 22.769 | 1.00 | 0.00 | AAAA |
| ATOM | 1307 | C | TRP | E | 79 | 28.577 | 111.008 | 26.653 | 1.00 | 0.00 | AAAA |
| ATOM | 1308 | O | TRP | E | 79 | 27.673 | 110.366 | 26.124 | 1.00 | 0.00 | AAAA |
| ATOM | 1309 | N | LYS | E | 80 | 28.838 | 110.869 | 27.955 | 1.00 | 0.00 | AAAA |
| ATOM | 1311 | CA | LYS | E | 80 | 28.085 | 110.142 | 28.958 | 1.00 | 0.00 | AAAA |
| ATOM | 1313 | CB | LYS | E | 80 | 28.597 | 108.708 | 29.104 | 1.00 | 0.00 | AAAA |
| ATOM | 1316 | CG | LYS | E | 80 | 27.718 | 107.793 | 29.956 | 1.00. | 0.00 | AAAA |
| ATOM | 1319 | CD | LYS | E | 80 | 28.461 | 106.646 | 30.641 | 1.00 | 0.00 | AAAA |
| ATOM | 1322 | CE | LYS | E | 80 | 28.677 | 105.546 | 29.600 | 1.00 | 0.00 | AAAA |
| ATOM | 1325 | NZ | LYS | E | 80 | 29.794 | 104.667 | 29.980 | 1.00 | 0.00 | AAAA |
| ATOM | 1329 | C | LYS | E | 80 | 28.303 | 110.937 | 30.238 | 1.00 | 0.00 | AAAA |
| ATOM | 1330 | O | LYS | E | 80 | 29.395 | 111.403 | 30.553 | 1.00 | 0.00 | AAAA |
| ATOM | 1331 | N | LEU | E | 81 | 27.245 | 111.186 | 31.013 | 1.00 | 0.00 | AAAA |
| ATOM | 1333 | CA | LEU | E | 81 | 27.343 | 111.951 | 32.240 | 1.00 | 0.00 | AAAA |
| ATOM | 1335 | CB | LEU | E | 81 | 26.445 | 113.187 | 32.289 | 1.00 | 0.00 | AAAA |
| ATOM | 1338 | CG | LEU | E | 81 | 26.645 | 114.171 | 31.135 | 1.00 | 0.00 | AAAA |
| ATOM | 1340 | CD1 | LEU | E | 81 | 25.415 | 115.076 | 31.053 | 1.00 | 0.00 | AAAA |
| ATOM | 1344 | CD2 | LEU | E | 81 | 27.944 | 114.973 | 31.207 | 1.00 | 0.00 | AAAA |
| ATOM | 1348 | C | LEU | E | 81 | 27.049 | 111.036 | 33.420 | 1.00 | 0.00 | AAAA |
| ATOM | 1349 | O | LEU | E | 81 | 26.411 | 110.011 | 33.194 | 1.00 | 0.00 | AAAA |
| ATOM | 1350 | N | PHE | E | 82 | 27.611 | 111.369 | 34.585 | 1.00 | 0.00 | AAAA |
| ATOM | 1352 | CA | PHE | E | 82 | 27.397 | 110.610 | 35.800 | 1.00 | 0.00 | AAAA |
| ATOM | 1354 | CB | PHE | E | 82 | 28.654 | 110.781 | 36.655 | 1.00 | 0.00 | AAAA |
| ATOM | 1357 | CG | PHE | E | 82 | 28.957 | 109.814 | 37.774 | 1.00 | 0.00 | AAAA |
| ATOM | 1358 | CD1 | PHE | E | 82 | 29.100 | 108.430 | 37.616 | 1.00 | 0.00 | AAAA |
| ATOM | 1360 | CD2 | PHE | E | 82 | 28.937 | 110.346 | 39.069 | 1.00 | 0.00 | AAAA |
| ATOM | 1362 | CE1 | PHE | E | 82 | 29.160 | 107.556 | 38.708 | 1.00 | 0.00 | AAAA. |
| ATOM | 1364 | CE2 | PHE | E | 82 | 29.055 | 109.500 | 40.178 | 1.00 | 0.00 | AAAA |
| ATOM | 1366 | CZ | PHE | E | 82 | 29.208 | 108.122 | 39.988 | 1.00 | 0.00 | AAAA |
| ATOM | 1368 | C | PHE | E | 82 | 26.208 | 111.288 | 36.465 | 1.00 | 0.00 | AAAA |
| ATOM | 1369 | O | PHE | E | 82 | 26.382 | 112.188 | 37.283 | 1.00 | 0.00 | AAAA |
| ATOM | 1370 | N | TYR | E | 83 | 24.958 | 110.941 | 36.148 | 1.00 | 0.00 | AAAA |
| ATOM | 1372 | CA | TYR | E | 83 | 23.760 | 111.456 | 36.779 | 1.00 | 0.00 | AAAA |
| ATOM | 1374 | CB | TYR | E | 83 | 23.749 | 111.039. | 38.250 | 1.00 | 0.00 | AAAA |
| ATOM | 1377 | CG | TYR | E | 83 | 23.542 | 109.557 | 38.449 | 1.00 | 0.00 | AAAA |
| ATOM | 1378 | CD1 | TYR | E | 83 | 22.281 | 108.961 | 38.341 | 1.00 | 0.00 | AAAA |
| ATOM | 1380 | CD2 | TYR | E | 83 | 24.652 | 108.794 | 38.835 | 1.00 | 0.00 | AAAA |
| ATOM | 1382 | CE1 | TYR | E | 83 | 22.195 | 107.599 | 38.651 | 1.00 | 0.00 | AAAA |
| ATOM | 1384 | CE2 | TYR | E | 83 | 24.594 | 107.409 | 39.029 | 1.00 | 0.00 | AAAA |
| ATOM | 1386 | CZ | TYR | E | 83 | 23.335 | 106.818 | 38.881 | 1.00 | 0.00 | AAAA |
| ATOM | 1387 | OH | TYR | E | 83 | 23.248 | 105.466 | 39.037 | 1.00 | 0.00 | AAAA |
| ATOM | 1389 | C | TYR | E | 83 | 23.500 | 112.937 | 36.545 | 1.00 | 0.00 | AAAA |
| ATOM | 1390 | O | TYR | E | 83 | 22.925 | 113.570 | 37.428 | 1.00 | 0.00 | AAAA |
| ATOM | 1391 | N | ASN | E | 84 | 23.868 | 113.437 | 35.362 | 1.00 | 0.00 | AAAA |
| ATOM | 1393 | CA | ASN | E | 84 | 23.846 | 114.833 | 34.976 | 1.00 | 0.00 | AAAA |
| ATOM | 1395 | CB | ASN | E | 84 | 22.676 | 115.605 | 35.586 | 1.00 | 0.00 | AAAA |
| ATOM | 1398 | CG | ASN | E | 84 | 22.407 | 116.897 | 34.827 | 1.00 | 0.00 | AAAA |
| ATOM | 1399 | OD1 | ASN | E | 84 | 22.349 | 116.954 | 33.600 | 1.00 | 0.00 | AAAA |
| ATOM | 1400 | ND2 | ASN | E | 84 | 22.117 | 117.974 | 35.560 | 1.00 | 0.00 | AAAA |
| ATOM | 1403 | C | ASN | E | 84 | 25.181 | 115.552 | 35.119 | 1.00 | 0.00 | AAAA |
| ATOM | 1404 | O | ASN | E | 84 | 25.381 | 116.552 | 34.436 | 1.00 | 0.00 | AAAA |
| ATOM | 1405 | N | TYR | E | 85 | 26.038 | 115.161 | 36.064 | 1.00 | 0.00 | AAAA |
| ATOM | 1407 | CA | TYR | E | 85 | 27.281 | 115.782 | 36.480 | 1.00 | 0.00 | AAAA |
| ATOM | 1409 | CB | TYR | E | 85 | 27.476 | 115.517 | 37.973 | 1.00 | .0.00 | AAAA |
| ATOM | 1412 | CG | TYR | E | 85 | 26.413 | 116.086 | 38.884 | 1.00 | 0.00 | AAAA |
| ATOM | 1413 | CD1 | TYR | E | 85 | 26.074 | 117.444 | 38.846 | 1.00 | 0.00 | AAAA |
| ATOM | 1415 | CD2 | TYR | E | 85 | 25.704 | 115.195 | 39.697 | 1.00 | 0.00 | AAAA |
| ATOM | 1417 | CE1 | TYR | E | 85 | 25.140 | 117.966 | 39.750 | 1.00 | 0.00 | AAAA. |
| ATOM | 1419 | CE2 | TYR | E | 85 | 24.761 | 115.712 | 40.593 | 1.00 | 0.00 | AAAA |
| ATOM | 1421 | CZ | TYR | E | 85 | 24.465 | 117.080 | 40.597 | 1.00 | 0.00 | AAAA |
| ATOM | 1422 | OH | TYR | E | 85 | 23.657 | 117.611 | 41.559 | 1.00 | 0.00 | AAAA |
| ATOM | 1424 | C | TYR | E | 85 | 28.420 | 115.304 | 35.593 | 1.00 | 0.00 | AAAA |
| ATOM | 1425 | O | TYR | E | 85 | 28.658 | 114.098 | 35.561 | 1.00 | 0.00 | AAAA |
| ATOM | 1426 | N | ALA | E | 86 | 29.186 | 116.240 | 35.027 | 1.00 | 0.00 | AAAA |
| ATOM | 1428 | CA | ALA | E | 86 | 30.276 | 115.964 | 34.113 | 1.00 | 0.00 | AAAA |
| ATOM | 1430 | CB | ALA | E | 86 | 30.307 | 117.052 | 33.038 | 1.00 | 0.00 | AAAA |
| ATOM | 1434 | C | ALA | E | 86 | 31.621 | 115.982 | 34.825 | 1.00 | 0.00 | AAAA |
| ATOM | 1435 | O | ALA | E | 86 | 32.516 | 115.245 | 34.417 | 1.00 | 0.00 | AAAA |
| ATOM | 1436 | N | LEU | E | 87 | 31.677 | 116.751 | 35.914 | 1.00 | 0.00 | AAAA |
| ATOM | 1438 | CA | LEU | E | 87 | 32.851 | 116.809 | 36.763 | 1.00 | 0.00 | AAAA |
| ATOM | 1440 | CB | LEU | E | 87 | 33.796 | 117.970 | 36.451 | 1.00 | 0.00 | AAAA |
| ATOM | 1443 | CG | LEU | E | 87 | 34.966 | 118.202 | 37.408 | 1.00 | 0.00. | AAAA |
| ATOM | 1445 | CD1 | LEU | E | 87 | 36.042 | 117.116 | 37.418 | 1.00 | 0.00 | AAAA |
| ATOM | 1449 | CD2 | LEU | E | 87 | 35.601 | 119.571 | 37.159 | 1.00 | 0.00 | AAAA |
| ATOM | 1453 | C | LEU | E | 87 | 32.348 | 116.838 | 38.199 | 1.00 | 0.00 | AAAA |
| ATOM | 1454 | O | LEU | E | 87 | 31.600 | 117.768 | 38.490 | 1.00 | 0.00 | AAAA |
| ATOM | 1455 | N | VAL | E | 88 | 32.693 | 115.810 | 38.979 | 1.00 | 0.00 | AAAA |
| ATOM | 1457 | CA | VAL | E | 88 | 32.380 | 115.796 | 40.394 | 1.00 | 0.00 | AAAA |
| ATOM | 1459 | CB | VAL | E | 88 | 31.641 | 114.505 | 40.746 | 1.00 | 0.00 | AAAA |
| ATOM | 1461 | CG1 | VAL | E | 88 | 31.247 | 114.337 | 42.214 | 1.00 | 0.00 | AAAA |
| ATOM | 1465 | CG2 | VAL | E | 88 | 30.396 | 114.111 | 39.950 | 1.00 | 0.00 | AAAA |
| ATOM | 1469 | C | VAL | E | 88 | 33.690 | 115.772 | 41.167 | 1.00 | 0.00 | AAAA |
| ATOM | 1470 | O | VAL | E | 88 | 34.560 | 114.983 | 40.803 | 1.00 | 0.00 | AAAA |
| ATOM | 1471 | N | ILE | E | 89 | 33.844 | 116.709 | 42.105 | 1.00 | 0.00 | AAAA |
| ATOM | 1473 | CA | ILE | E | 89 | 34.884 | 116.759 | 43.113 | 1.00 | 0.00 | AAAA |
| ATOM | 1475 | CB | ILE | E | 89 | 35.707 | 118.017 | 42.831 | 1.00 | 0.00 | AAAA |
| ATOM | 1477 | CG1 | ILE | E | 89 | 36.351 | 117.955 | 41.444 | 1.00 | 0.00 | AAAA |
| ATOM | 1480 | CG2 | ILE | E | 89 | 36.806 | 118.220 | 43.874 | 1.00 | 0.00 | AAAA |
| ATOM | 1484 | CD | ILE | E | 89 | 37.053 | 119.255 | 41.054 | 1.00 | 0.00 | AAAA |
| ATOM | 1488 | C | ILE | E | 89 | 34.189 | 116.654 | 44.462 | 1.00 | 0.00 | AAAA |
| ATOM | 1489 | O | ILE | E | 89 | 33.289 | 117.455 | 44.708 | 1.00 | 0.00 | AAAA |
| ATOM | 1490 | N | PHE | E | 90 | 34.531 | 115.636 | 45.255 | 1.00 | 0.00 | AAAA |
| ATOM | 1492 | CA | PHE | E | 90 | 33.920 | 115.459 | 46.557 | 1.00 | 0.00 | AAAA |
| ATOM | 1494 | CB | PHE | E | 90 | 32.734 | 114.500 | 46.458 | 1.00 | 0.00 | AAAA |
| ATOM | 1497 | CG | PHE | E | 90 | 31.816 | 114.338 | 47.645 | 1.00 | 0.00 | AAAA |
| 3.ATOM | 1498 | CD1 | PHE | E | 90 | 31.050 | 115.438 | 48.052 | 1.00 | 0.00 | AAAA |
| ATOM | 1500 | CD2 | PHE | E | 90 | 31.694 | 113.152 | 48.380 | 1.00 | 0.00 | AAAA |
| ATOM | 1502 | CE1 | PHE | E | 90 | 30.086 | 115.348 | 49.062 | 1.00 | 0.00 | AAAA |
| ATOM | 1504 | CE2 | PHE | E | 90 | 30.780 | 113.037 | 49.434 | 1.00 | 0.00 | AAAA |
| ATOM | 1506 | CZ | PHE | E | 90 | 29.995 | 114.145 | 49.774 | 1.00 | 0.00 | AAAA |
| ATOM | 1508 | C | PHE | E | 90 | 34.879 | 115.106 | 47.685 | 1.00 | 0.00 | AAAA |
| ATOM | 1509 | O | PHE | E | 90 | 35.684 | 114.218 | 47.412 | 1.00 | 0.00 | AAAA |
| ATOM | 1510 | N | GLU | E | 91 | 34.644 | 115.668 | 48.873 | 1.00 | 0.00 | AAAA |
| ATOM | 1512 | CA | GLU | E | 91 | 35.193 | 115.296 | 50.162 | 1.00 | 0.00 | AAAA. |
| ATOM | 1514 | CB | GLU | E | 91 | 34.712 | 113.919 | 50.620 | 1.00 | 0.00 | AAAA |
| ATOM | 1517 | CG | GLU | E | 91 | 34.798 | 113.796 | 52.142 | 1.00 | 0.00 | AAAA |
| ATOM | 1520 | CD | GLU | E | 91 | 33.848 | 112.763 | 52.731 | 1.00 | 0.00 | AAAA |
| ATOM | 1521 | OE1 | GLU | E | 91 | 33.853 | 111.589 | 52.304 | 1.00 | 0.00 | AAAA |
| ATOM | 1522 | OE2 | GLU | E | 91 | 33.097 | 113.183 | 53.638 | 1.00 | 0.00 | AAAA |
| ATOM | 1523 | C | GLU | E | 91 | 36.704 | 115.482 | 50.182 | 1.00 | 0.00 | AAAA |
| ATOM | 1524 | O | GLU | E | 91 | 37.387 | 114.756 | 50.900 | 1.00 | 0.00 | AAAA |
| ATOM | 1525 | N | MET | E | 92 | 37.289 | 116.339 | 49.341 | 1.00 | 0.00 | AAAA |
| ATOM | 1527 | CA | MET | E | 92 | 38.716 | 116.218 | 49.126 | 1.00 | 0.00 | AAAA |
| ATOM | 1529 | CB | MET | E | 92 | 39.121 | 116.931 | 47.836 | 1.00 | 0.00 | AAAA |
| ATOM | 1532 | CG | MET | E | 92 | 38.490 | 116.489 | 46.516 | 1.00 | 0.00 | AAAA |
| ATOM | 1535 | SD | MET | E | 92 | 39.154 | 114.976 | 45.776 | 1.00 | 0.00 | AAAA |
| ATOM | 1536 | CE | MET | E | 92 | 40.650 | 115.569 | 44.948 | 1.00 | 0.00 | AAAA |
| ATOM | 1540 | C | MET | E | 92 | 39.560 | 116.860 | 50.217 | 1.00 | 0.00 | AAAA |
| ATOM | 1541 | O | MET | E | 92 | 39.455 | 118.071 | 50.399 | 1.00 | 0.00 | AAAA |
| ATOM | 1542 | N | THR | E | 93 | 40.448 | 116.126 | 50.893 | 1.00 | 0.00 | AAAA |
| ATOM | 1544 | CA | THR | E | 93 | 41.503 | 116.601 | 51.765 | 1.00 | 0.00 | AAAA |
| ATOM | 1546 | CB | THR | E | 93 | 41.946 | 115.424 | 52.635 | 1.00 | 0.00 | AAAA |
| ATOM | 1548 | OG1 | THR | E | 93 | 40.796 | 114.786 | 53.145 | 1.00 | 0.00 | AAAA |
| ATOM | 1550 | CG2 | THR | E | 93 | 42.815 | 115.790 | 53.839 | 1.00 | 0.00 | AAAA |
| ATOM | 1554 | C | THR | E | 93 | 42.651 | 117.129 | 50.917 | 1.00 | 0.00 | AAAA |
| ATOM | 1555 | O | THR | E | 93 | 43.058 | 116.515 | 49.932 | 1.00 | 0.00 | AAAA |
| ATOM | 1556 | N | ASN | E | 94 | 43.315 | 118.196 | 51.368 | 1.00 | 0.00 | AAAA |
| ATOM | 1558 | CA | ASN | E | 94 | 44.480 | 118.873 | 50.836 | 1.00 | 0.00 | AAAA |
| ATOM | 1560 | CB | ASN | E | 94 | 45.628 | 117.951 | 50.425 | 1.00 | 0.00 | AAAA |
| ATOM | 1563 | CG | ASN | E | 94 | 46.059 | 117.029 | 51.556 | 1.00 | 0.00 | AAAA |
| ATOM | 1564 | OD1 | ASN | E | 94 | 45.658 | 115.869 | 51.515 | 1.00 | 0.00 | AAAA |
| ATOM | 1565 | ND2 | ASN | E | 94 | 46.709 | 117.480 | 52.632 | 1.00 | 0.00 | AAAA |
| ATOM | 1568 | C | ASN | E | 94 | 44.137 | 119.911 | 49.777 | 1.00 | 0.00 | AAAA |
| ATOM | 1569 | O | ASN | E | 94 | 44.876 | 120.860 | 49.519 | 1.00 | 0.00 | AAAA |
| ATOM | 1570 | N | LEU | E | 95 | 43.013 | 119.703 | 49.087 | 1.00 | 0.00 | AAAA |
| ATOM | 1572 | CA | LEU | E | 95 | 42.668 | 120.432 | 47.883 | 1.00 | 0.00 | AAAA |
| ATOM | 1574 | CB | LEU | E | 95 | 41.475 | 119.770 | 47.191 | 1.00 | 0.00 | AAAA |
| ATOM | 1577 | CG | LEU | E | 95 | 41.121 | 120.351 | 45.821 | 1.00 | 0.00 | AAAA |
| ATOM | 1579 | CD1 | LEU | E | 95 | 42.108 | 120.030 | 44.699 | 1.00 | 0.00 | AAAA |
| ATOM | 1583 | CD2 | LEU | E | 95 | 39.669 | 120.154 | 45.385 | 1.00 | 0.00 | AAAA |
| ATOM | 1587 | C | LEU | E | 95 | 42.340 | 121.881 | 48.213 | 1.00 | 0.00 | AAAA |
| ATOM | 1588 | O | LEU | E | 95 | 41.326 | 122.178 | 48.841 | 1.00 | 0.00 | AAAA |
| ATOM | 1589 | N | LYS | E | 96 | 43.125 | 122.839 | 47.716 | 1.00 | 0.00 | AAAA. |
| ATOM | 1591 | CA | LYS | E | 96 | 43.210 | 124.200 | 48.205 | 1.00 | 0.00 | AAAA |
| ATOM | 1593 | CB | LYS | E | 96 | 44.710 | 124.362 | 48.455 | 1.00 | 0.00 | AAAA |
| ATOM | 1596 | CG | LYS | E | 96 | 45.141 | 125.482 | 49.402 | 1.00 | 0.00 | AAAA |
| ATOM | 1599 | CD | LYS | E | 96 | 44.619 | 125.273 | 50.824 | 1.00 | 0.00 | AAAA |
| ATOM | 1602 | CE | LYS | E | 96 | 45.232 | 126.215 | 51.860 | 1.00 | 0.00 | AAAA |
| ATOM | 1605 | NZ | LYS | E | 96 | 44.978 | 125.684 | 53.208 | 1.00 | 0.00 | AAAA |
| ATOM | 1609 | C | LYS | E | 96 | 42.635 | 125.175 | 47.186 | 1.00 | 0.00 | AAAA |
| ATOM | 1610 | O | LYS | E | 96 | 41.897 | 126.090 | 47.544 | 1.00 | 0.00 | AAAA |
| ATOM | 1611 | N | ASP | E | 97 | 42.878 | 124.936 | 45.895 | 1.00 | 0.00 | AAAA |
| ATOM | 1613 | CA | ASP | E | 97 | 42.355 | 125.517 | 44.675 | 1.00 | 0.00 | AAAA |
| ATOM | 1615 | CB | ASP | E | 97 | 43.425 | 126.510 | 44.220 | 1.00 | 0.00 | AAAA |
| ATOM | 1618 | CG | ASP | E | 97 | 44.451 | 126.107 | 43.171 | 1.00 | 0.00 | AAAA |
| ATOM | 1619 | OD1 | ASP | E | 97 | 45.481 | 125.499 | 43.534 | 1.00 | 0.00 | AAAA |
| ATOM | 1620 | OD2 | ASP | E | 97 | 44.214 | 126.319 | 41.962 | 1.00 | 0.00 | AAAA |
| ATOM | 1621 | C | ASP | E | 97 | 42.239 | 124.327 | 43.733 | 1.00 | 0.00 | AAAA |
| ATOM | 1622 | O | ASP | E | 97 | 42.867 | 123.311 | 44.019 | 1.00 | 0.00 | AAAA |
| ATOM | 1623 | N | ILE | E | 98 | 41.344 | 124.358 | 42.742 | 1.00 | 0.00 | AAAA |
| ATOM | 1625 | CA | ILE | E | 98 | 41.073 | 123.272 | 41.822 | 1.00 | 0.00 | AAAA |
| ATOM | 1627 | CB | ILE | E | 98 | 39.891 | 123.553 | 40.892 | 1.00 | 0.00 | AAAA |
| ATOM | 1629 | CG1 | ILE | E | 98 | 38.603 | 123.900 | 41.641 | 1.00 | 0.00 | AAAA |
| ATOM | 1632 | CG2 | ILE | E | 98 | 39.591 | 122.441 | 39.887 | 1.00 | 0.00 | AAAA |
| ATOM | 1636 | CD | ILE | E | 98 | 38.050 | 122.675 | 42.369 | 1.00 | 0.00 | AAAA |
| ATOM | 1640 | C | ILE | E | 98 | 42.310 | 122.856 | 41.040 | 1.00 | 0.00 | AAAA |
| ATOM | 1641 | O | ILE | E | 98 | 42.789 | 121.741 | 41.231 | 1.00 | 0.00 | AAAA |
| ATOM | 1642 | N | GLY | E | 99 | 42.778 | 123.797 | 40.216 | 1.00 | 0.00 | AAAA |
| ATOM | 1644 | CA | GLY | E | 99 | 43.998 | 123.603 | 39.458 | 1.00 | 0.00 | AAAA |
| ATOM | 1647 | C | GLY | E | 99 | 43.822 | 123.246 | 37.989 | 1.00 | 0.00 | AAAA |
| ATOM | 1648 | O | GLY | E | 99 | 44.768 | 123.174 | 37.208 | 1.00 | 0.00 | AAAA |
| ATOM | 1649 | N | LEU | E | 100 | 42.606 | 122.964 | 37.515 | 1.00 | 0.00 | AAAA |
| ATOM | 1651 | CA | LEU | E | 100 | 42.448 | 122.549 | 36.135 | 1.00 | 0.00 | AAAA |
| ATOM | 1653 | CB | LEU | E | 100 | 41.166 | 121.726 | 36.003 | 1.00 | 0.00 | AAAA |
| ATOM | 1656 | CG | LEU | E | 100 | 41.193 | 120.395 | 36.755 | 1.00 | 0.00 | AAAA |
| ATOM | 1658 | CD1 | LEU | E | 100 | 39.766 | 119.852 | 36.673 | 1.00 | 0.00 | AAAA |
| ATOM | 1662 | CD2 | LEU | E | 100 | 42.256 | 119.378 | 36.341 | 1.00 | 0.00 | AAAA |
| ATOM | 1666 | C | LEU | E | 100 | 42.261 | 123.758 | 35.228 | 1.00 | 0.00 | AAAA |
| ATOM | 1667 | O | LEU | E | 100 | 41.184 | 124.003 | 34.689 | 1.00 | 0.00 | AAAA |
| ATOM | 1668 | N | TYR | E | 101 | 43.292 | 124.601 | 35.133 | 1.00 | 0.00 | AAAA |
| ATOM | 1670 | CA | TYR | E | 101 | 43.347 | 125.950 | 34.606 | 1.00 | 0.00 | AAAA |
| ATOM | 1672 | CB | TYR | E | 101 | 44.654 | 126.608 | 35.051 | 1.00 | 0.00 | AAAA |
| ATOM | 1675 | CG | TYR | E | 101 | 45.875 | 125.903 | 34.510 | 1.00 | 0.00 | AAAA |
| ATOM | 1676 | CD1 | TYR | E | 101 | 46.542 | 124.940 | 35.277 | 1.00 | 0.00 | AAAA |
| ATOM | 1678 | CD2 | TYR | E | 101 | 46.342 | 126.157 | 33:215 | 1.00 | 0.00 | AAAA |
| ATOM | 1680 | CE1 | TYR | E | 101 | 47.770 | 124.384 | 34.898 | 1.00 | 0.00 | AAAA |
| ATOM | 1682 | CE2 | TYR | E | 101 | 47.618 | 125.704 | 32.860 | 1.00 | 0.00 | AAAA |
| ATOM | 1684 | CZ | TYR | E | 101 | 48.295 | 124.776 | 33.662 | 1.00 | 0.00 | AAAA |
| ATOM | 1685 | OH | TYR | E | 101 | 49.539 | 124.318 | 33.340 | 1.00 | 0.00 | AAAA |
| ATOM | 1687 | C | TYR | E | 101 | 43.034 | 126.178 | 33.134 | 1.00 | 0.00 | AAAA |
| ATOM | 1688 | O | TYR | E | 101 | 42.450 | 127.186 | 32.743 | 1.00 | 0.00 | AAAA |
| ATOM | 1689 | N | ASN | E | 102 | 43.266 | 125.120 | 32.353 | 1.00 | 0.00 | AAAA |
| ATOM | 1691 | CA | ASN | E | 102 | 43.049 | 125.064 | 30.921 | 1.00 | 0.00 | AAAA |
| ATOM | 1693 | CB | ASN | E | 102 | 44.064 | 124.115 | 30.282 | 1.00 | 0.00 | AAAA |
| ATOM | 1696 | CG | ASN | E | 102 | 44.382 | 124.568 | 28.865 | 1.00 | 0.00 | AAAA |
| ATOM | 1697 | OD1 | ASN | E | 102 | 45.128 | 125.539 | 28.759 | 1.00 | 0.00 | AAAA |
| ATOM | 1698 | ND2 | ASN | E | 102 | 44.216 | 123.747 | 27.825 | 1.00 | 0.00 | AAAA |
| ATOM | 1701 | C | ASN | E | 102 | 41.630 | 124.669 | 30.539 | 1.00 | 0.00 | AAAA |
| ATOM | 1702 | O | ASN | E | 102 | 41.195 | 124.787 | 29.396 | 1.00 | .0.00 | AAAA |
| ATOM | 1703 | N | LEU | E | 103 | 40.883 | 124.114 | 31.497 | 1.00 | 0.00 | AAAA |
| ATOM | 1705 | CA | LEU | E | 103 | 39.508 | 123.681 | 31.351 | 1.00 | 0.00 | AAAA |
| ATOM | 1707 | CB | LEU | E | 103 | 39.083 | 123.075 | 32.690 | 1.00 | 0.00 | AAAA |
| ATOM | 1710 | CG | LEU | E | 103 | 37.736 | 122.374 | 32.864 | 1.00 | 0.00 | AAAA |
| ATOM | 1712 | CD1 | LEU | E | 103 | 37.510 | 121.233 | 31.871 | 1.00 | 0.00 | AAAA |
| ATOM | 1716 | CD2 | LEU | E | 103 | 37.579 | 121.872 | 34.300 | 1.00 | 0.00 | AAAA |
| ATOM | 1720 | C | LEU | E | 103 | 38.541 | 124.711 | 30.784 | 1.00 | 0.00 | AAAA |
| ATOM | 1721 | O | LEU | E | 103 | 38.269 | 125.712 | 31.443 | 1.00 | 0.00 | AAAA |
| ATOM | 1722 | N | ARG | E | 104 | 38.145 | 124.516 | 29.523 | 1.00 | 0.00 | AAAA |
| ATOM | 1724 | CA | ARG | E | 104 | 37.395 | 125.400 | 28.653 | 1.00 | 0.00 | AAAA |
| ATOM | 1726 | CB | ARG | E | 104 | 38.332 | 126.083 | 27.656 | 1.00 | 0.00 | AAAA |
| ATOM | 1729 | CG | ARG | E | 104 | 39.251 | 127.187 | 28.181 | 1.00 | 0.00 | AAAA |
| ATOM | 1732 | CD | ARG | E | 104 | 38.421 | 128.393 | 28.624 | 1.00 | 0.00 | AAAA |
| .ATOM | 1735 | NE | ARG | E | 104 | 39.246 | 129.516 | 29.073 | 1.00 | 0.00 | AAAA |
| ATOM | 1737 | CZ | ARG | E | 104 | 39.147 | 130.814 | 28.758 | 1.00 | 0.00 | AAAA |
| ATOM | 1738 | NH1 | ARG | E | 104 | 38.272 | 131.309 | 27.872 | 1.00 | 0.00 | AAAA |
| ATOM | 1741 | NH2 | ARG | E | 104 | 39.857 | 131.687 | 29.486 | 1.00 | 0.00 | AAAA |
| ATOM | 1744 | C | ARG | E | 104 | 36.152 | 124.810 | 28.001 | 1.00 | 0.00 | AAAA |
| ATOM | 1745 | O | ARG | E | 104 | 35.460 | 125.619 | 27.388 | 1.00 | 0.00 | AAAA |
| ATOM | 1746 | N | ASN | E | 105 | 35.850 | 123.510 | 27.988 | 1.00 | 0.00 | AAAA |
| ATOM | 1748 | CA | ASN | E | 105 | 34.623 | 122.995 | 27.415 | 1.00 | 0.00 | AAAA |
| ATOM | 1750 | CB | ASN | E | 105 | 34.875 | 122.496 | 25.991 | 1.00 | 0.00 | AAAA |
| ATOM | 1753 | CG | ASN | E | 105 | 33.591 | 122.383 | 25.180 | 1.00 | 0.00 | AAAA |
| ATOM | 1754 | OD1 | ASN | E | 105 | 32.822 | 121.430 | 25.279 | 1.00 | 0.00 | AAAA |
| ATOM | 1755 | ND2 | ASN | E | 105 | 33.342 | 123.412 | 24.366 | 1.00 | 0.00 | AAAA |
| ATOM | 1758 | C | ASN | E | 105 | 34.051 | 121.884 | 28.285 | 1.00 | 0.00 | AAAA |
| ATOM | 1759 | O | ASN | E | 105 | 34.691 | 120.838 | 28.363 | 1.00 | 0.00 | AAAA |
| ATOM | 1760 | N | ILE | E | 106 | 32.933 | 122.119 | 28.975 | 1.00 | 0.00 | AAAA |
| ATOM | 1762 | CA | ILE | E | 106 | 32.057 | 121.075 | 29.467 | 1.00 | 0.00 | AAAA |
| ATOM | 1764 | CB | ILE | E | 106 | 31.725 | 121.143 | 30.957 | 1.00 | 0.00 | AAAA |
| ATOM | 1766 | CG1 | ILE | E | 106 | 33.063 | 120.884 | 31.650 | 1.00 | 0.00 | AAAA |
| ATOM | 1769 | CG2 | ILE | E | 106 | 30.705 | 120.096 | 31.407 | 1.00 | 0.00 | AAAA. |
| ATOM | 1773 | CD | ILE | E | 106 | 33.071 | 121.279 | 33.127 | 1.00 | 0.00 | AAAA |
| ATOM | 1777 | C | ILE | E | 106 | 30.741 | 121.331 | 28.745 | 1.00 | 0.00 | AAAA |
| ATOM | 1778 | O | ILE | E | 106 | 30.117 | 122.368 | 28.959 | 1.00 | 0.00 | AAAA |
| ATOM | 1779 | N | THR | E | 107 | 30.263 | 120.314 | 28.025 | 1.00 | 0.00 | AAAA |
| ATOM | 1781 | CA | THR | E | 107 | 28.977 | 120.419 | 27.366 | 1.00 | 0.00 | AAAA |
| ATOM | 1783 | CB | THR | E | 107 | 29.137 | 120.259 | 25.854 | 1.00 | 0.00 | AAAA |
| ATOM | 1785 | OG1 | THR | E | 107 | 29.764 | 121.459 | 25.460 | 1.00 | 0.00 | AAAA |
| ATOM | 1787 | CG2 | THR | E | 107 | 27.894 | 120.158 | 24.970 | 1.00 | 0.00 | AAAA |
| ATOM | 1791 | C | THR | E | 107 | 28.175 | 119.256 | 27.934 | 1.00 | 0.00 | AAAA |
| ATOM | 1792 | O | THR | E | 107 | 28.702 | 118.200 | 28.277 | 1.00 | 0.00 | AAAA |
| ATOM | 1793 | N | ARG | E | 108 | 26.864 | 119.510 | 27.938 | 1.00 | 0.00 | AAAA |
| ATOM | 1795 | CA | ARG | E | 108 | 25.703 | 118.654 | 28.080 | 1.00 | 0.00 | AAAA |
| ATOM | 1797 | CB | ARG | E | 108 | 25.860 | 117.352 | 27.296 | 1.00 | 0.00 | AAAA |
| ATOM | 1800 | CG | ARG | E | 108 | 24.517 | 116.637 | 27.137 | 1.00 | 0.00 | AAAA |
| ATOM | 1803 | CD | ARG | E | 108 | 24.680 | 115.270 | 26.474 | 1.00 | 0.00 | AAAA |
| ATOM | 1806 | NE | ARG | E | 108 | 25.458 | 115.335 | 25.236 | 1.00 | 0.00 | AAAA |
| ATOM | 1808 | CZ | ARG | E | 108 | 26.039 | 114.260 | 24.689 | 1.00 | 0.00 | AAAA |
| ATOM | 1809 | NH1 | ARG | E | 108 | 25.850 | 113.031 | 25.189 | 1.00 | 0.00 | AAAA |
| ATOM | 1812 | NH2 | ARG | E | 108 | 26.866 | 114.422 | 23.646 | 1.00 | 0.00 | AAAA |
| ATOM | 1815 | C | ARG | E | 108 | 25.385 | 118.495 | 29.560 | 1.00 | 0.00 | AAAA |
| ATOM | 1816 | O | ARG | E | 108 | 24.223 | 118.262 | 29.883 | 1.00 | 0.00 | AAAA |
| ATOM | 1817 | N | GLY | E | 109 | 26.409 | 118.547 | 30.415 | 1.00 | 0.00 | AAAA |
| ATOM | 1819 | CA | GLY | E | 109 | 26.341 | 118.286 | 31.840 | 1.00 | 0.00 | AAAA |
| ATOM | 1822 | C | GLY | E | 109 | 26.829 | 119.448 | 32.694 | 1.00 | 0.00 | AAAA |
| ATOM | 1823 | O | GLY | E | 109 | 27.441 | 120.412 | 32.237 | 1.00 | 0.00 | AAAA |
| ATOM | 1824 | N | ALA | E | 110 | 26.486 | 119.343 | 33.980 | 1.00 | 0.00 | AAAA |
| ATOM | 1826 | CA | ALA | E | 110 | 26.811 | 120.331 | 34.989 | 1.00 | 0.00 | AAAA |
| ATOM | 1828 | CB | ALA | E | 110 | 25.539 | 120.472 | 35.824 | 1.00 | 0.00 | AAAA |
| ATOM | 1832 | C | ALA | E | 110 | 28.024 | 119.849 | 35.772 | 1.00 | 0.00 | AAAA |
| ATOM | 1833 | O | ALA | E | 110 | 28.708 | 118.907 | 35.378 | 1.00 | 0.00 | AAAA |
| ATOM | 1834 | N | ILE | E | 111 | 28.238 | 120.422 | 36.959 | 1.00 | 0.00 | AAAA |
| ATOM | 1836 | CA | ILE | E | 111 | 29.353 | 120.141 | 37.841 | 1.00 | 0.00 | AAAA |
| ATOM | 1838 | CB | ILE | E | 111 | 30.608 | 120.938 | 37.485 | 1.00 | 0.00 | AAAA |
| ATOM | 1840 | CG1 | ILE | E | 111 | 30.503 | 122.427 | 37.819 | 1.00 | 0.00 | AAAA |
| ATOM | 1843 | CG2 | ILE | E | 111 | 31.284 | 120.644 | 36.145 | 1.00 | 0.00 | AAAA |
| ATOM | 1847 | CD | ILE | E | 111 | 31.858 | 123.111 | 38.003 | 1.00 | 0.00 | AAAA |
| ATOM | 1851 | C | ILE | E | 111 | 28.992 | 120.146 | 39.319 | 1.00 | 0.00 | AAAA |
| ATOM | 1852 | O | ILE | E | 111 | 28.066 | 120.855 | 39.705 | 1.00 | 0.00 | AAAA |
| ATOM | 1853 | N | ARG | E | 112 | 29.696 | 119.346 | 40.124 | 1.00 | 0.00 | AAAA |
| ATOM | 1855 | CA | ARG | E | 112 | 29.573 | 119.336 | 41.568 | 1.00 | 0.00 | AAAA |
| ATOM | 1857 | CB | ARG | E | 112 | 28.774 | 118.114 | 42.020 | 1.00 | 0.00 | AAAA |
| ATOM | 1860 | CG | ARG | E | 112 | 28.251 | 118.132 | 43.457 | 1.00 | 0.00 | AAAA |
| ATOM | 1863 | CD | ARG | E | 112 | 26.851 | 117.525 | 43.557 | 1.00 | 0.00 | AAAA |
| ATOM | 1866 | NE | ARG | E | 112 | 26.375 | 117.363 | 44.931 | 1.00 | 0.00 | AAAA |
| ATOM | 1868 | CZ | ARG | E | 112 | 25.105 | 117.374 | 45.360 | 1.00 | 0.00 | AAAA |
| ATOM | 1869 | NH1 | ARG | E | 112 | 24.016 | 117.301 | 44.583 | 1.00 | 0.00 | AAAA |
| ATOM | 1872 | NH2 | ARG | E | 112 | 24.900 | 117.448 | 46.682 | 1.00 | 0.00 | AAAA |
| ATOM | 1875 | C | ARG | E | 112 | 30.910 | 119.480 | 42.283 | 1.00 | 0.00 | AAAA |
| ATOM | 1876 | O | ARG | E | 112 | 31.772 | 118.659 | 41.981 | 1.00 | 0.00 | AAAA |
| ATOM | 1877 | N | ILE | E | 113 | 31.178 | 120.546 | 43.040 | 1.00 | 0.00 | AAAA |
| ATOM | 1879 | CA | ILE | E | 113 | 32.304 | 120.764 | 43.925 | 1.00 | 0.00 | AAAA |
| ATOM | 1881 | CB | ILE | E | 113 | 33.291 | 121.799 | 43.383 | 1.00 | 0.00 | AAAA |
| ATOM | 1883 | CG1 | ILE | E | 113 | 33.613 | 121.550 | 41.910 | 1.00 | 0.00 | AAAA |
| ATOM | 1886 | CG2 | ILE | E | 113 | 34.559 | 121.899 | 44.232 | 1.00 | 0.00 | AAAA |
| ATOM | 1890 | CD | ILE | E | 113 | 34.188 | 122.801 | 41.244 | 1.00 | 0.00 | AAAA |
| ATOM | 1894 | C | ILE | E | 113 | 31.792 | 121.143 | 45.307 | 1.00 | 0.00 | AAAA |
| ATOM | 1895 | O | ILE | E | 113 | 31.408 | 122.282 | 45.558 | 1.00 | 0.00 | AAAA |
| ATOM | 1896 | N | GLU | E | 114 | 31.773 | 120.148 | 46.198 | 1.00 | 0.00 | AAAA |
| ATOM | 1898 | CA | GLU | E | 114 | 31.117 | 120.058 | 47.487 | 1.00 | 0.00 | AAAA |
| ATOM | 1900 | CB | GLU | E | 114 | 29.680 | 119.598 | 47.240 | 1.00 | 0.00 | AAAA |
| ATOM | 1903 | CG | GLU | E | 114 | 28.838 | 119.292 | 48.479 | 1.00 | 0.00 | AAAA |
| ATOM | 1906 | CD | GLU | E | 114 | 27.645 | 118.400 | 48.167 | 1.00 | 0.00 | AAAA |
| ATOM | 1907 | OE1 | GLU | E | 114 | 27.671 | 117.667 | 47.154 | 1.00 | 0.00 | AAAA |
| ATOM | 1908 | OE2 | GLU | E | 114 | 26.703 | 118.363 | 48.989 | 1.00 | 0.00 | AAAA |
| ATOM | 1909 | C | GLU | E | 114 | 31.916 | 119.252 | 48.501 | 1.00 | 0.00 | AAAA |
| ATOM | 1910 | O | GLU | E | 114 | 32.553 | 118.309 | 48.036 | 1.00 | 0.00 | AAAA |
| ATOM | 1911 | N | LYS | E | 115 | 32.024 | 119.692 | 49.757 | 1.00 | 0.00 | AAAA |
| ATOM | 1913 | CA | LYS | E | 115 | 32.488 | 118.954 | 50.916 | 1.00 | 0.00 | AAAA |
| ATOM | 1915 | CB | LYS | E | 115 | 31.847 | 117.581 | 51.121 | 1.00 | 0.00 | AAAA |
| ATOM | 1918 | CG | LYS | E | 115 | 31.610 | 117.164 | 52.572 | 1.00 | 0.00 | AAAA |
| ATOM | 1921 | CD | LYS | E | 115 | 30.973 | 115.783 | 52.734 | 1.00 | 0.00 | AAAA |
| ATOM | 1924 | CE | LYS | E | 115 | 30.718 | 115.456 | 54.205 | 1.00 | 0.00 | AAAA |
| ATOM | 1927 | NZ | LYS | E | 115 | 30.568 | 114.007 | 54.409 | 1.00 | 0.00 | AAAA |
| ATOM | 1931 | C | LYS | E | 115 | 34.001 | 119.003 | 51.070 | 1.00 | 0.00 | AAAA |
| ATOM | 1932 | O | LYS | E | 115 | 34.543 | 118.209 | 51.836 | 1.00 | 0.00 | AAAA |
| ATOM | 1933 | N | ASN | E | 116 | 34.619 | 119.989 | 50.414 | 1.00 | 0.00 | AAAA |
| ATOM | 1935 | CA | ASN | E | 116 | 36.048 | 120.204 | 50.302 | 1.00 | 0.00 | AAAA |
| ATOM | 1937 | CB | ASN | E | 116 | 36.340 | 120.742 | 48.900 | 1.00 | 0.00 | AAAA |
| ATOM | 1940 | CG | ASN | E | 116 | 36.066 | 119.767 | 47.765 | 1.00 | 0.00 | AAAA |
| ATOM | 1941 | OD1 | ASN | E | 116 | 36.624 | 118.682 | 47.615 | 1.00 | 0.00 | AAAA |
| ATOM | 1942 | ND2 | ASN | E | 116 | 34.984 | 120.020 | 47.024 | 1.00 | 0:00 | AAAA |
| ATOM | 1945 | C | ASN | E | 116 | 36.358 | 121.347 | 51.257 | 1.00 | 0.00 | AAAA |
| ATOM | 1946 | O | ASN | E | 116 | 36.108 | 122.510 | 50.952 | 1.00 | 0.00 | AAAA |
| ATOM | 1947 | N | ALA | E | 117 | 36.716 | 120.958 | 52.483 | 1.00 | 0.00 | AAAA |
| ATOM | 1949 | CA | ALA | E | 117 | 36.652 | 121.788 | 53.669 | 1.00 | 0.00 | AAAA |
| ATOM | 1951 | CB | ALA | E | 117 | 36.901 | 120.900 | 54.889 | 1.00 | 0.00 | AAAA |
| ATOM | 1955 | C | ALA | E | 117 | 37.655 | 122.921 | 53.834 | 1.00 | 0.00 | AAAA |
| ATOM | 1956 | O | ALA | E | 117 | 37.483 | 123.877 | 54.586 | 1.00 | 0.00 | AAAA |
| ATOM | 1957 | N | ASP | E | 118 | 38.693 | 122.866 | 52.995 | 1.00 | 0.00 | AAAA |
| ATOM | 1959 | CA | ASP | E | 118 | 39.830 | 123.758 | 52.892 | 1.00 | 0.00 | AAAA |
| ATOM | 1961 | CB | ASP | E | 118 | 41.102 | 123.084 | 53.406 | 1.00 | 0.00 | AAAA |
| ATOM | 1964 | CG | ASP | E | 118 | 42.073 | 124.048 | 54.074 | 1.00 | 0.00 | AAAA |
| ATOM | 1965 | OD1 | ASP | E | 118 | 43.265 | 123.946 | 53.716 | 1.00 | 0.00 | AAAA |
| ATOM | 1966 | OD2 | ASP | E | 118 | 41.719 | 124.948 | 54.866 | 1.00 | 0.00 | AAAA |
| ATOM | 1967 | C | ASP | E | 118 | 40.057 | 124.272 | 51.477 | 1.00 | 0.00 | AAAA |
| ATOM | 1968 | O | ASP | E | 118 | 41.121 | 124.785 | 51.138 | 1.00 | 0.00 | AAAA |
| ATOM | 1969 | N | LEU | E | 119 | 39.028 | 124.322 | 50.629 | 1.00 | 0.00 | AAAA |
| ATOM | 1971 | CA | LEU | E | 119 | 39.002 | 124.666 | 49.222 | 1.00 | 0.00 | AAAA |
| ATOM | 1973 | CB | LEU | E | 119 | 38.348 | 123.617 | 48.322 | 1.00 | 0.00 | AAAA |
| ATOM | 1976 | CG | LEU | E | 119 | 38.041 | 123.952 | 46.861 | 1.00 | 0.00 | AAAA |
| ATOM | 1978 | CD1 | LEU | E | 119 | 39.360 | 124.329 | 46.188 | 1.00 | 0.00 | AAAA |
| ATOM | 1982 | CD2 | LEU | E | 119 | 37.356 | 122.782 | 46.154 | 1.00 | 0.00 | AAAA |
| ATOM | 1986 | C | LEU | E | 119 | 38.281 | 125.995 | 49.047 | 1.00 | 0.00 | AAAA |
| ATOM | 1987 | O | LEU | E | 119 | 37.192 | 126.192 | 49.579 | 1.00 | 0.00 | AAAA |
| ATOM | 1988 | N | CYS | E | 120 | 38.990 | 126.932 | 48.414 | 1.00 | 0.00 | AAAA |
| ATOM | 1990 | CA | CYS | E | 120 | 38.401 | 128.140 | 47.871 | 1.00 | 0.00 | AAAA |
| ATOM | 1992 | CB | CYS | E | 120 | 38.375 | 129.227 | 48.946 | 1.00 | 0.00 | AAAA |
| ATOM | 1995 | SG | CYS | E | 120 | 36.921 | 130.300 | 49.047 | 1.00 | 0.00 | AAAA |
| ATOM | 1996 | C | CYS | E | 120 | 38.961 | 128.568 | 46.522 | 1.00 | 0.00 | AAAA |
| ATOM | 1997 | O | CYS | E | 120 | 39.676 | 127.789 | 45.896 | 1.00 | 0.00 | AAAA |
| ATOM | 1998 | N | TYR | E | 121 | 38.658 | 129.796 | 46.094 | 1.00 | 0.00 | AAAA |
| ATOM | 2000 | CA | TYR | E | 121 | 38.851 | 130.339 | 44.765 | 1.00 | 0.00 | AAAA |
| ATOM | 2002 | CB | TYR | E | 121 | 40.280 | 130.169 | 44.249 | 1.00 | 0.00 | AAAA |
| ATOM | 2005 | CG | TYR | E | 121 | 41.222 | 131.158 | 44.894 | 1.00 | 0.00 | AAAA |
| ATOM | 2006 | CD1 | TYR | E | 121 | 41.932 | 130.845 | 46.059 | 1.00 | 0.00 | AAAA |
| ATOM | 2008 | CD2 | TYR | E | 121 | 41.363 | 132.460 | 44.401 | 1.00 | 0.00 | AAAA |
| ATOM | 2010 | CE1 | TYR | E | 121 | 42.870 | 131.708 | 46.636 | 1.00 | 0.00 | AAAA |
| ATOM | 2012 | CE2 | TYR | E | 121 | 42.349 | 133.318 | 44.903 | 1.00 | 0.00 | AAAA |
| ATOM | 2014 | CZ | TYR | E | 121 | 43.025 | 132.984 | 46.084 | 1.00 | 0.00 | AAAA |
| ATOM | 2015 | OH | TYR | E | 121 | 43.895 | 133.891 | 46.616 | 1.00 | 0.00 | AAAA |
| ATOM | 2017 | C | TYR | E | 121 | 37.891 | 129.603 | 43.841 | 1.00 | 0.00 | AAAA |
| ATOM | 2018 | O | TYR | E | 121 | 38.256 | 128.854 | 42.938 | 1.00 | 0.00 | AAAA |
| ATOM | 2019 | N | LEU | E | 122 | 36.604 | 129.762 | 44.158 | 1.00 | 0.00 | AAAA. |
| ATOM | 2021 | CA | LEU | E | 122 | 35.402 | 129.177 | 43.599 | 1.00 | 0.00 | AAAA. |
| ATOM | 2023 | CB | LEU | E | 122 | 34.907 | 128.106 | 44.571 | 1.00 | 0.00 | AAAA |
| ATOM | 2026 | CG | LEU | E | 122 | 35.664 | 126.777 | 44.546 | 1.00 | 0.00 | AAAA |
| ATOM | 2028 | CD1 | LEU | E | 122 | 35.063 | 125.877 | 45.625 | 1.00 | 0.00 | AAAA |
| ATOM | 2032 | CD2 | LEU | E | 122 | 35.722 | 126.051 | 43.201 | 1.00 | 0.00 | AAAA |
| ATOM | 2036 | C | LEU | E | 122 | 34.457 | 130.305 | 43.208 | 1.00 | 0.00 | AAAA |
| ATOM | 2037 | O | LEU | E | 122 | 33.845 | 130.126 | 42.157 | 1.00 | 0.00 | AAAA |
| ATOM | 2038 | N | SER | E | 123 | 34.503 | 131.465 | 43.867 | 1.00 | 0.00 | AAAA |
| ATOM | 2040 | CA | SER | E | 123 | 33.728 | 132.612 | 43.437 | 1.00 | 0.00 | AAAA |
| ATOM | 2042 | CB | SER | E | 123 | 33.342 | 133.258 | 44.767 | 1.00 | 0.00 | AAAA |
| ATOM | 2045 | OG | SER | E | 123 | 32.393 | 134.298 | 44.689 | 1.00 | 0.00 | AAAA |
| ATOM | 2047 | C | SER | E | 123 | 34.404 | 133.531 | 42.428 | 1.00 | 0.00 | AAAA |
| ATOM | 2048 | O | SER | E | 123 | 33.929 | 134.562 | 41.956 | 1.00 | 0.00 | AAAA. |
| ATOM | 2049 | N | THR | E | 124 | 35.625 | 133.150 | 42.047 | 1.00 | 0.00 | AAAA |
| ATOM | 2051 | CA | THR | E | 124 | 36.546 | 133.965 | 41.280 | 1.00 | 0.00 | AAAA |
| ATOM | 2053 | CB | THR | E | 124 | 37.889 | 133.903 | 42.007 | 1.00 | 0.00 | AAAA |
| ATOM | 2055 | OG1 | THR | E | 124 | 38.324 | 132.580 | 42.223 | 1.00 | 0.00 | AAAA |
| ATOM | 2057 | CG2 | THR | E | 124 | 37.831 | 134.644 | 43.344 | 1.00 | 0.00 | AAAA |
| ATOM | 2061 | C | THR | E | 124 | 36.695 | 133.448 | 39.856 | 1.00 | 0.00 | AAAA |
| ATOM | 2062 | O | THR | E | 124 | 37.075 | 134.230 | 38.988 | 1.00 | 0.00 | AAAA |
| ATOM | 2063 | N | VAL | E | 125 | 36.193 | 132.261 | 39.512 | 1.00 | 0.00 | AAAA |
| ATOM | 2065 | CA | VAL | E | 125 | 36.160 | 131.876 | 38.115 | 1.00 | 0.00 | AAAA. |
| ATOM | 2067 | CB | VAL | E | 125 | 36.555 | 130.400 | 38.130 | 1.00 | 0.00 | AAAA |
| ATOM | 2069 | CG1 | VAL | E | 125 | 36.443 | 129.608 | 36.827 | 1.00 | 0.00 | AAAA |
| ATOM | 2073 | CG2 | VAL | E | 125 | 37.858 | 130.022 | 38.836 | 1.00 | 0.00 | AAAA |
| ATOM | 2077 | C | VAL | E | 125 | 34.737 | 132.087 | 37.619 | 1.00 | 0.00 | AAAA |
| ATOM | 2078 | O | VAL | E | 125 | 33.794 | 132.088 | 38.407 | 1.00 | 0.00 | AAAA |
| ATOM | 2079 | N | ASP | E | 126 | 34.609 | 132.487 | 36.351 | 1.00 | 0.00 | AAAA |
| ATOM | 2081 | CA | ASP | E | 126 | 33.330 | 132.563 | 35.675 | 1.00 | 0.00 | AAAA |
| ATOM | 2083 | CB | ASP | E | 126 | 33.389 | 133.719 | 34.675 | 1.00 | 0.00 | AAAA |
| ATOM | 2086 | CG | ASP | E | 126 | 32.229 | 133.805 | 33.693 | 1.00 | 0.00 | AAAA |
| ATOM | 2087 | OD1 | ASP | E | 126 | 31.168 | 133.337 | 34.160 | 1.00 | 0.00 | AAAA |
| ATOM | 2088 | OD2 | ASP | E | 126 | 32.374 | 134.329 | 32.568 | 1.00 | 0.00 | AAAA |
| ATOM | 2089 | C | ASP | E | 126 | 33.059 | 131.185 | 35.089 | 1.00 | 0.00 | AAAA |
| ATOM | 2090 | O | ASP | E | 126 | 33.703 | 130.853 | 34.096 | 1.00 | 0.00 | AAAA |
| ATOM | 2091 | N | TRP | E | 127 | 32.228 | 130.304 | 35.652 | 1.00 | 0.00 | AAAA |
| ATOM | 2093 | CA | TRP | E | 127 | 31.935 | 128.955 | 35.211 | 1.00 | 0.00 | AAAA |
| ATOM | 2095 | CB | TRP | E | 127 | 31.313 | 128.317 | 36.453 | 1.00 | 0.00 | AAAA. |
| ATOM | 2098 | CG | TRP | E | 127 | 32.139 | 128.298 | 37.698 | 1.00 | 0.00 | AAAA |
| ATOM | 2099 | CD1 | TRP | E | 127 | 32.087 | 129.169 | 38.731 | 1.00 | 0.00 | AAAA |
| ATOM | 2101 | CD2 | TRP | E | 127 | 33.266 | 127.409 | 37.964 | 1.00 | 0.00 | AAAA |
| ATOM | 2102 | NE1 | TRP | E | 127 | 33.000 | 128.808 | 39.701 | 1.00 | 0.00 | AAAA |
| ATOM | 2104 | CE2 | TRP | E | 127 | 33.708 | 127.699 | 39.281 | 1.00 | 0.00 | AAAA |
| ATOM | 2105 | CE3 | TRP | E | 127 | 33.861 | 126.331 | 37.289 | 1.00 | 0.00 | AAAA |
| ATOM | 2107 | CZ2 | TRP | E | 127 | 34.729 | 126.936 | 39.858 | 1.00 | 0.00 | AAAA |
| ATOM | 2109 | CZ3 | TRP | E | 127 | 34.998 | 125.707 | 37.815 | 1.00 | 0.00 | AAAA |
| ATOM | 2111 | CH2 | TRP | E | 127 | 35.434 | 125.980 | 39.116 | 1.00 | 0.00 | AAAA |
| ATOM | 2113 | C | TRP | E | 127 | 30.953 | 128.983 | 34.048 | 1.00 | 0.00 | AAAA |
| ATOM | 2114 | O | TRP | E | 127 | 30.810 | 127.943 | 33.410 | 1.00 | 0.00 | AAAA |
| ATOM | 2115 | N | SER | E | 128 | 30.264 | 130.085 | 33.739 | 1.00 | 0.00 | AAAA |
| ATOM | 2117 | CA | SER | E | 128 | 29.441 | 130.300 | 32.567 | 1.00 | 0.00 | AAAA |
| ATOM | 2119 | CB | SER | E | 128 | 28.660 | 131.593 | 32.801 | 1.00 | 0.00 | AAAA |
| ATOM | 2122 | OG | SER | E | 128 | 27.605 | 131.743 | 31.878 | 1.00 | 0.00 | AAAA |
| ATOM | 2124 | C | SER | E | 128 | 30.327 | 130.405 | 31.334 | 1.00 | 0.00 | AAAA |
| ATOM | 2125 | O | SER | E | 128 | 29.793 | 130.344 | 30.228 | 1.00 | 0.00 | AAAA |
| ATOM | 2126 | N | LEU | E | 129 | 31.647 | 130.340 | 31.518 | 1.00 | 0.00 | AAAA |
| ATOM | 2128 | CA | LEU | E | 129 | 32.570 | 130.442 | 30.405 | 1.00 | 0.00 | AAAA |
| ATOM | 2130 | CB | LEU | E | 129 | 33.841 | 131.044 | 31.006 | 1.00 | 0.00 | AAAA |
| ATOM | 2133 | CG | LEU | E | 129 | 34.772 | 131.643 | 29.951 | 1.00 | 0.00 | AAAA |
| ATOM | 2135 | CD1 | LEU. | E | 129 | 34.024 | 132.612 | 29.035 | 1.00 | 0.00 | AAAA |
| ATOM | 2139 | CD2 | LEU | E | 129 | 35.789 | 132.513 | 30.689 | 1.00 | 0.00 | AAAA |
| ATOM | 2143 | C | LEU | E | 129 | 32.905 | 129.038 | 29.924 | 1.00 | 0.00 | AAAA |
| ATOM | 2144 | O | LEU | E | 129 | 33.371 | 128.958 | 28.789 | 1.00 | 0.00 | AAAA |
| ATOM | 2145 | N | ILE | E | 130 | 32.765 | 128.030 | 30.788 | 1.00 | 0.00 | AAAA |
| ATOM | 2147 | CA | ILE | E | 130 | 33.082 | 126.635 | 30.559 | 1.00 | 0.00 | AAAA |
| ATOM | 2149 | CB | ILE | E | 130 | 33.653 | 126.040 | 31.846 | 1.00 | 0.00 | AAAA |
| ATOM | 2151 | CG1 | ILE | E | 130 | 34.883 | 126.780 | 32.370 | 1.00 | 0.00 | AAAA |
| ATOM | 2154 | CG2 | ILE | E | 130 | 33.945 | 124.539 | 31.797 | 1.00 | 0.00 | AAAA |
| ATOM | 2158 | CD | ILE | E | 130 | 35.200 | 126.686 | 33.862 | 1.00 | 0.00 | AAAA |
| ATOM | 2162 | C | ILE | E | 130 | 31.739 | 125.959 | 30.322 | 1.00 | 0.00 | AAAA |
| ATOM | 2163 | O | ILE | E | 130 | 31.612 | 125.314 | 29.284 | 1.00 | 0.00 | AAAA |
| ATOM | 2164 | N | LEU | E | 131 | 30.686 | 126.158 | 31.120 | 1.00 | 0.00 | AAAA |
| ATOM | 2166 | CA | LEU | E | 131 | 29.377 | 125.539 | 31.080 | 1.00 | 0.00 | AAAA |
| ATOM | 2168 | CB | LEU | E | 131 | 28.904 | 125.267 | 32.508 | 1.00 | 0.00 | AAAA |
| ATOM | 2171 | CG | LEU | E | 131 | 29.751 | 124.225 | 33.240 | 1.00 | 0.00 | AAAA |
| ATOM | 2173 | CD1 | LEU | E | 131 | 30.758 | 124.728 | 34.276 | 1.00 | 0.00 | AAAA |
| ATOM | 2177 | CD2 | LEU | E | 131 | 28.867 | 123.202 | 33.955 | 1.00 | 0.00 | AAAA |
| ATOM | 2181 | C | LEU | E | 131 | 28.276 | 126.359 | 30.423 | 1.00 | 0.00 | AAAA |
| ATOM | 2182 | O | LEU | E | 131 | 28.279 | 127.577 | 30.259 | 1.00 | 0.00 | AAAA |
| ATOM | 2183 | N | ASP | E | 132 | 27.230 | 125.599 | 30.091 | 1.00 | 0.00 | AAAA |
| ATOM | 2185 | CA | ASP | E | 132 | 25.957 | 126.129 | 29.643 | 1.00 | 0.00 | AAAA |
| ATOM | 2187 | CB | ASP | E | 132 | 25.528 | 125.516 | 28.310 | 1.00 | 0.00 | AAAA |
| ATOM | 2190 | CG | ASP | E | 132 | 24.095 | 125.688 | 27.825 | 1.00 | 0.00 | AAAA |
| ATOM | 2191 | OD1 | ASP | E | 132 | 23.837 | 126.695 | 27.131 | 1.00 | 0.00 | AAAA |
| ATOM | 2192 | OD2 | ASP | E | 132 | 23.254 | 124.852 | 28.221 | 1.00 | 0.00 | AAAA |
| ATOM | 2193 | C | ASP | E | 132 | 24.890 | 125.797 | 30.676 | 1.00 | 0.00 | AAAA |
| ATOM | 2194 | O | ASP | E | 132 | 24.026 | 126.605 | 31.011 | 1.00 | 0.00 | AAAA |
| ATOM | 2195 | N | ALA | E | 133 | 24.987 | 124.583 | 31.222 | 1.00 | 0.00 | AAAA |
| ATOM | 2197 | CA | ALA | E | 133 | 24.024 | 123.956 | 32.105 | 1.00 | 0.00 | AAAA |
| ATOM | 2199 | CB | ALA | E | 133 | 24.386 | 122.489 | 32.346 | 1.00 | 0.00 | AAAA |
| ATOM | 2203 | C | ALA | E | 133 | 24.030 | 124.597 | 33.485 | 1.00 | 0.00 | AAAA |
| ATOM | 2204 | O | ALA | E | 133 | 23.684 | 123.925 | 34.455 | 1.00 | 0.00 | AAAA |
| ATOM | 2205 | N | VAL | E | 134 | 24.544 | 125.819 | 33.640 | 1.00 | 0.00 | AAAA |
| ATOM | 2207 | CA | VAL | E | 134 | 24.929 | 126.461 | 34.882 | 1.00 | 0.00 | AAAA |
| ATOM | 2209 | CB | VAL | E | 134 | 25.725 | 127.747 | 34.659 | 1.00 | 0.00 | AAAA |
| ATOM | 2211 | CG1 | VAL | E | 134 | 27.115 | 127.552 | 34.052 | 1.00 | 0.00 | AAAA |
| ATOM | 2215 | CG2 | VAL | E | 134 | 24.961 | 128.818 | 33.881 | 1.00 | 0.00 | AAAA |
| ATOM | 2219 | C | VAL | E | 134 | 23.782 | 126.728 | 35.845 | 1.00 | 0.00 | AAAA |
| ATOM | 2220 | O | VAL | E | 134 | 23.987 | 127.131 | 36.988 | 1.00 | 0.00 | AAAA |
| ATOM | 2221 | N | SER | E | 135 | 22.544 | 126.553 | 35.375 | 1.00 | 0.00 | AAAA |
| ATOM | 2223 | CA | SER | E | 135 | 21.327 | 126.602 | 36.160 | 1.00 | 0.00 | AAAA |
| ATOM | 2225 | CB | SER | E | 135 | 20.134 | 126.704 | 35.209 | 1.00 | 0.00 | AAAA |
| ATOM | 2228 | OG | SER | E | 135 | 20.082 | 125.661 | 34.261 | 1.00 | 0.00 | AAAA |
| ATOM | 2230 | C | SER | E | 135 | 21.125 | 125.437 | 37.118 | 1.00 | 0.00 | AAAA |
| ATOM | 2231 | O | SER | E | 135 | 20.400 | 125.525 | 38.106 | 1.00 | 0.00 | AAAA |
| ATOM | 2232 | N | ASN | E | 136 | 21.902 | 124.355 | 37.032 | 1.00 | 0.00 | AAAA |
| ATOM | 2234 | CA | ASN | E | 136 | 21.819 | 123.223 | 37.933 | 1.00 | 0.00 | AAAA |
| ATOM | 2236 | CB | ASN | E | 136 | 21.053 | 122.074 | 37.277 | 1.00 | 0.00 | AAAA |
| ATOM | 2239 | CG | ASN | E | 136 | 19.586 | 121.947 | 37.664 | 1.00 | 0.00 | AAAA |
| ATOM | 2240 | OD1 | ASN | E | 136 | 19.201 | 121.867 | 38.828 | 1.00 | 0.00 | AAAA |
| ATOM | 2241 | ND2 | ASN | E | 136 | 18.677 | 121.880 | 36.688 | 1.00 | 0.00 | AAAA |
| ATOM | 2244 | C | ASN | E | 136 | 23.194 | 122.881 | 38.488 | 1.00 | 0.00 | AAAA |
| ATOM | 2245 | O | ASN | E | 136 | 23.384 | 121.767 | 38.971 | 1.00 | 0.00 | AAAA |
| ATOM | 2246 | N | ASN | E | 137 | 24.095 | 123.864 | 38.541 | 1.00 | 0.00 | AAAA |
| ATOM | 2248 | CA | ASN | E | 137 | 25.438 | 123.702 | 39.059 | 1.00 | 0.00 | AAAA |
| ATOM | 2250 | CB | ASN | E | 137 | 26.342 | 124.807 | 38.508 | 1.00 | 0.00 | AAAA |
| ATOM | 2253 | CG | ASN | E | 137 | 27.222 | 124.455 | 37.317 | 1.00 | 0.00 | AAAA |
| ATOM | 2254 | OD1 | ASN | E | 137 | 27.344 | 123.328 | 36.844 | 1.00 | 0.00 | AAAA |
| ATOM | 2255 | ND2 | ASN | E | 137 | 27.913 | 125.437 | 36.734 | 1.00 | 0.00 | AAAA |
| ATOM | 2258 | C | ASN | E | 137 | 25.380 | 123.799 | 40.577 | 1.00 | 0.00 | AAAA |
| ATOM | 2259 | O | ASN | E | 137 | 24.682 | 124.566 | 41.236 | 1.00 | 0.00 | AAAA |
| ATOM | 2260 | N | TYR | E | 138 | 26.311 | 123.065 | 41.190 | 1.00 | 0.00 | AAAA |
| ATOM | 2262 | CA | TYR | E | 138 | 26.504 | 122.948 | 42.621 | 1.00 | 0.00 | AAAA |
| ATOM | 2264 | CB | TYR | E | 138 | 25.981 | 121.570 | 43.026 | 1.00 | 0.00 | AAAA |
| ATOM | 2267 | CG | TYR | E | 138 | 25.378 | 121.462 | 44.406 | 1.00 | 0.00 | AAAA |
| ATOM | 2268 | CD1 | TYR | E | 138 | 24.218 | 122.205 | 44.656 | 1.00 | 0.00 | .AAAA |
| ATOM | 2270 | CD2 | TYR | E | 138 | 25.853 | 120.578 | 45.383 | 1.00 | 0.00 | AAAA |
| ATOM | 2272 | CE1 | TYR | E | 138 | 23.475 | 122.040 | 45.831 | 1.00 | 0.00 | AAAA |
| ATOM | 2274 | CE2 | TYR | E | 138 | 25.118 | 120.421 | 46.565 | 1.00 | 0.00 | AAAA |
| ATOM | 2276 | CZ | TYR | E | 138 | 23.991 | 121.208 | 46.832 | 1.00 | 0.00 | AAAA |
| ATOM | 2277 | OH | TYR | E | 138 | 23.326 | 121.075 | 48.015 | 1.00 | 0.00 | AAAA |
| ATOM | 2279 | C | TYR | E | 138 | 27.936 | 123.193 | 43.078 | 1.00 | 0.00 | AAAA |
| ATOM. | 2280 | O | TYR | E | 138 | 28.792 | 122.313 | 43.122 | 1.00 | 0.00 | AAAA |
| ATOM | 2281 | N | ILE | E | 139 | 28.292 | 124.464 | 43.279 | 1.00 | 0.00 | AAAA |
| ATOM | 2283 | CA | ILE | E | 139 | 29.619 | 124.908 | 43.655 | 1.00 | 0.00 | AAAA |
| ATOM | 2285 | CB | ILE | E | 139 | 30.340 | 125.673 | 42.545 | 1.00 | 0.00 | AAAA |
| ATOM | 2287 | CG1 | ILE | E | 139 | 30.196 | 125.085 | 41.140 | 1.00 | 0.00 | AAAA |
| ATOM | 2290 | CG2 | ILE | E | 139 | 31.835 | 125.824 | 42.822 | 1.00 | 0.00 | AAAA |
| ATOM | 2294 | CD | ILE | E | 139 | 30.477 | 126.012 | 39.957 | 1.00 | 0.00 | AAAA |
| ATOM | 2298 | C | ILE | E | 139 | 29.473 | 125.452 | 45.070 | 1.00 | 0.00 | AAAA |
| ATOM | 2299 | O | ILE | E | 139 | 29.852 | 126.601 | 45.286 | 1.00 | 0.00 | AAAA |
| ATOM | 2300 | N | VAL | E | 140 | 28.762 | 124.809 | 45.999 | 1.00 | 0.00 | AAAA |
| ATOM | 2302 | CA | VAL | | 140 | 28.317 | 125.436 | 47.227 | 1.00 | 0.00 | AAAA |
| ATOM | 2304 | CB | VAL | E | 140 | 26.976 | 126.157 | 47.086 | 1.00 | 0.00 | AAAA |
| ATOM | 2306 | CG1 | VAL | E | 140 | 27.078 | 127.468 | 46.305 | 1.00 | 0.00 | AAAA |
| ATOM | 2310 | CG2 | VAL | E | 140 | 25.873 | 125.293 | 46.473 | 1.00 | 0.00 | AAAA |
| ATOM | 2314 | C | VAL | E | 140 | 28.346 | 124.373 | 48.316 | 1.00 | 0.00 | AAAA |
| ATOM | 2315 | O | VAL | E | 140 | 27.744 | 123.317 | 48.135 | 1.00 | 0.00 | AAAA |
| ATOM | 2316 | N | GLY | E | 141 | 29.140 | 124.562 | 49.371 | 1.00 | 0.00 | AAAA |
| ATOM | 2318 | CA | GLY | E | 141 | 29.289 | 123.605 | 50.449 | 1.00 | 0.00 | AAAA |
| ATOM | 2321 | C | GLY | E | 141 | 30.724 | 123.130 | 50.621 | 1.00 | 0.00 | AAAA |
| ATOM | 2322 | O | GLY | E | 141 | 30.918 | 121.917. | 50.609 | 1.00 | 0.00 | AAAA |
| ATOM | 2323 | N | ASN | E | 142 | 31.658 | 124.084 | 50.658 | 1.00 | 0.00 | AAAA |
| ATOM | 2325 | CA | ASN | E | 142 | 33.087 | 123.859 | 50.738 | 1.00 | 0.00 | AAAA |
| ATOM | 2327 | CB | ASN | E | 142 | 33.657 | 124.342 | 49.405 | 1.00 | 0.00 | AAAA |
| ATOM | 2330 | CG | ASN | E | 142 | 33.365 | 123.446 | 48.209 | 1.00 | 0.00 | AAAA |
| ATOM | 2331 | OD1 | ASN | E | 142 | 33.521 | 122.227 | 48.203 | 1.00 | 0.00 | AAAA |
| ATOM | 2332 | ND2 | ASN | E | 142 | 32.725 | 124.064 | 47.215 | 1.00 | 0.00 | AAAA |
| ATOM | 2335 | C | ASN | E | 142 | 33.541 | 124.717 | 51.910 | 1.00 | 0.00 | AAAA |
| ATOM | 2336 | O | ASN | E | 142 | 32.869 | 124.744 | 52.938 | 1.00 | 0.00 | AAAA |
| ATOM | 2337 | N | LYS | E | 143 | 34.722 | 125.337 | 51.856 | 1.00 | 0.00 | AAAA |
| ATOM | 2339 | CA | LYS | E | 143 | 35.258 | 126.160 | 52.922 | 1.00 | 0.00 | AAAA |
| ATOM | 2341 | CB | LYS | E | 143 | 36.644 | 126.627 | 52.478 | 1.00 | 0.00 | AAAA |
| ATOM | 2344 | CG | LYS | E | 143 | 37.486 | 127.304 | 53.560 | 1.00 | 0.00 | AAAA |
| ATOM | 2347 | CD | LYS | E | 143 | 38.978 | 127.512 | 53.296 | 1.00 | 0.00 | AAAA |
| ATOM | 2350 | CE | LYS | E | 143 | 39.820 | 127.715 | 54.556 | 1.00 | 0.00 | AAAA |
| ATOM | 2353 | NZ | LYS | E | 143 | 41.227 | 127.384 | 54.278 | 1.00 | 0.00 | AAAA |
| ATOM | 2357 | C | LYS | E | 143 | 34.403 | 127.396 | 53.165 | 1.00 | 0.00 | AAAA |
| ATOM | 2358 | O | LYS | E | 143 | 33.924 | 128.032 | 52.229 | 1.00 | 0.00 | AAAA |
| ATOM | 2359 | N | PRO | E | 144 | 34.083 | 127.785 | 54.402 | 1.00 | 0.00 | AAAA |
| ATOM | 2360 | CA | PRO | E | 144 | 33.185 | 128.903 | 54.613 | 1.00 | 0.00 | AAAA |
| ATOM | 2362 | CB | PRO | E | 144 | 32.740 | 128.759 | 56.070 | 1.00 | 0.00 | AAAA |
| ATOM | 2365 | CG | PRO | E | 144 | 33.937 | 128.047 | 56.701 | 1.00 | 0.00 | AAAA |
| ATOM | 2368 | CD | PRO | E | 144 | 34.474 | 127.108 | 55.621 | 1.00 | 0.00 | AAAA |
| ATOM | 2371 | C | PRO | E | 144 | 33.873 | 130.227 | 54.314 | 1.00 | 0.00 | AAAA |
| ATOM | 2372 | O | PRO | E | 144 | 35.080 | 130.449 | 54.264 | 1.00 | 0.00 | AAAA |
| ATOM | 2373 | N | PRO | E | 145 | 33.055 | 131.270 | 54.161 | 1.00 | 0.00 | AAAA |
| ATOM | 2374 | CA | PRO | E | 145 | 33.438 | 132.604 | 53.744 | 1.00 | 0.00 | AAAA |
| ATOM | 2376 | CB | PRO | E | 145 | 32.182 | 133.302 | 53.224 | 1.00 | 0.00 | AAAA |
| ATOM | 2379 | CG | PRO | E | 145 | 31.073 | 132.519 | 53.926 | 1.00 | 0.00 | AAAA |
| ATOM | 2382 | CD | PRO | E | 145 | 31.614 | 131.110 | 54.167 | 1.00 | 0.00 | AAAA |
| ATOM | 2385 | C | PRO | E | 145 | 33.967 | 133.462 | 54.885 | 1.00 | 0.00 | AAAA |
| ATOM | 2386 | O | PRO | E | 145 | 34.597 | 134.491 | 54.655 | 1.00 | 0.00 | AAAA |
| ATOM | 2387 | N | LYS | E | 146 | 33.727 | 133.064 | 56.138 | 1.00 | 0.00 | AAAA |
| ATOM | 2389 | CA | LYS | E | 146 | 34.332 | 133.665 | 57.309 | 1.00 | 0.00 | AAAA |
| ATOM | 2391 | CB | LYS | E | 146 | 33.491 | 133.208 | 58.501 | 1.00 | 0.00 | AAAA |
| ATOM | 2394 | CG | LYS | E | 146 | 33.787 | 133.781 | 59.888 | 1.00 | 0.00 | AAAA |
| ATOM | 2397 | CD | LYS | E | 146 | 32.677 | 133.469 | 60.891 | 1.00 | 0.00 | AAAA |
| ATOM | 2400 | CE | LYS | E | 146 | 32.906 | 134.244 | 62.189 | 1.00 | 0.00 | AAAA |
| ATOM | 2403 | NZ | LYS | E | 146 | 31.921 | 134.027 | 63.259 | 1.00 | 0.00 | AAAA |
| ATOM | 2407 | C | LYS | E | 146 | 35.785 | 133.249 | 57.489 | 1.00 | 0.00 | AAAA |
| ATOM | 2408 | O | LYS | E | 146 | 36.481 | 133.715 | 58.389 | 1.00 | 0.00 | AAAA |
| ATOM | 2409 | N | GLU | E | 147 | 36.210 | 132.349 | 56.600 | 1.00 | 0.00 | AAAA |
| ATOM | 2411 | CA | GLU | E | 147 | 37.517 | 131.747 | 56.430 | 1.00 | 0.00 | AAAA |
| ATOM | 2413 | CB | GLU | E | 147 | 37.380 | 130.331 | 56.990 | 1.00 | 0.00 | AAAA |
| ATOM | 2416 | CG | GLU | E | 147 | 36.906 | 130.172 | 58.435 | 1.00 | 0.00 | AAAA |
| ATOM | 2419 | CD | GLU | E | 147 | 37.822 | 130.760 | 59.498 | 1.00 | 0.00 | AAAA |
| ATOM | 2420 | OE1 | GLU | E | 147 | 37.340 | 131.172 | 60.575 | 1.00 | 0.00 | AAAA |
| ATOM | 2421 | OE2 | GLU | E | 147 | 39.058 | 130.865 | 59.343 | 1.00 | 0.00 | AAAA |
| ATOM | 2422 | C | GLU | E | 147 | 38.001 | 131.739 | 54.988 | 1.00 | 0.00 | AAAA |
| ATOM | 2423 | O | GLU | E | 147 | 39.002 | 131.051 | 54.800 | 1.00 | 0.00 | AAAA |
| ATOM | 2424 | N | CYS | E | 148 | 37.405 | 132.417 | 54.003 | 1.00 | 0.00 | AAAA |
| ATOM | 2426 | CA | CYS | E | 148 | 37.970 | 132.591 | 52.679 | 1.00 | 0.00 | AAAA |
| ATOM | 2428 | CB | CYS | E | 148 | 37.239 | 131.619 | 51.753 | 1.00 | 0.00 | AAAA |
| ATOM | 2431 | SG | CYS | E | 148 | 37.678 | 131.922 | 50.024 | 1.00 | 0.00 | AAAA |
| ATOM | 2432 | C | CYS | E | 148 | 37.668 | 134.059 | 52.410 | 1.00 | 0.00 | AAAA |
| ATOM | 2433 | O | CYS | E | 148 | 36.512 | 134.471 | 52.324 | 1.00 | 0.00 | AAAA |
| ATOM | 2434 | N | GLY | E | 149 | 38.701 | 134.860 | 52.140 | 1.00 | 0.00 | AAAA |
| ATOM | 2436 | CA | GLY | E | 149 | 38.582 | 136.158 | 51.509 | 1.00 | 0.00 | AAAA |
| ATOM | 2439 | C | GLY | E | 149 | 39.347 | 135.996 | 50.204 | 1.00 | 0.00 | AAAA |
| ATOM | 2440 | O | GLY | E | 149 | 40.434 | 135.424 | 50.144 | 1.00 | 0.00 | AAAA |
| ATOM | 2441 | N | ASP | E | 150 | 38.703 | 136.477 | 49.139 | 1.00 | 0.00 | AAAA |
| ATOM | 2443 | CA | ASP | E | 150 | 39.207 | 136.286 | 47.793 | 1.00 | 0.00 | AAAA |
| ATOM | 2445 | CB | ASP | E | 150 | 38.069 | 136.427 | 46.783 | 1.00 | 0.00 | AAAA |
| ATOM | 2448 | CG | ASP | E | 150 | 36.983 | 135.362 | 46.838 | 1.00 | 0.00 | AAAA |
| ATOM | 2449 | OD1 | ASP | E | 150 | 37.287 | 134.212 | 46.457 | 1.00 | 0.00 | AAAA |
| ATOM | 2450 | OD2 | ASP | E | 150 | 35.826 | 135.627 | 47.233 | 1.00 | 0.00 | AAAA |
| ATOM | 2451 | C | ASP | E | 150 | 40.256 | 137.322 | 47.418 | 1.00 | 0.00 | AAAA |
| ATOM | 2452 | O | ASP | E | 150 | 40.153 | 138.487 | 47.794 | 1.00 | 0.00 | AAAA |
| ATOM | 2453 | N | LEU | E | 151 | 41.297 | 136.845 | 46.730 | 1.00 | 0.00 | AAAA |
| ATOM | 2455 | CA | LEU | E | 151 | 42.579 | 137.496 | 46.552 | 1.00 | 0.00 | AAAA |
| ATOM | 2457 | CB | LEU | E | 151 | 43.491 | 137.303 | 47.764 | 1.00 | 0.00 | AAAA |
| ATOM | 2460 | CG | LEU | E | 151 | 44.849 | 138.005 | 47.810 | 1.00 | 0.00 | AAAA |
| ATOM | 2462 | CD1 | LEU | E | 151 | 44.750 | 139.506 | 48.084 | 1.00 | 0.00 | AAAA |
| ATOM | 2466 | CD2 | LEU | E | 151 | 45.789 | 137.509 | 48.910 | 1.00 | 0.00 | AAAA |
| ATOM | 2470 | C | LEU | E | 151 | 43.216 | 137.104 | 45.226 | 1.00 | 0.00 | AAAA |
| ATOM | 2471 | O | LEU | E | 151 | 43.916 | 136.100 | 45.112 | 1.00 | 0.00 | AAAA |
| ATOM | 2472 | N | CYS | E | 152 | 42.749 | 137.748 | 44.154 | 1.00 | 0.00 | AAAA |
| ATOM | 2474 | CA | CYS | E | 152 | 43.157 | 137.500 | 42.785 | 1.00 | 0.00 | AAAA |
| ATOM | 2476 | CB | CYS | E | 152 | 41.975 | 137.989 | 41.949 | 1.00 | 0.00 | AAAA |
| ATOM | 2479 | SG | CYS | E | 152 | 40.739 | 136.684 | 41.730 | 1.00 | 0.00 | AAAA |
| ATOM | 2480 | C | CYS | E | 152 | 44.393 | 138.296 | 42.394 | 1.00 | 0.00 | AAAA |
| ATOM | 2481 | O | CYS | E | 152 | 44.620 | 139.333 | 43.014 | 1.00 | 0.00 | AAAA |
| ATOM | 2482 | N | PRO | E | 153 | 45.276 | 137.837 | 41.504 | 1.00 | 0.00 | AAAA |
| ATOM | 2483 | CA | PRO | E | 153 | 46.590 | 138.357 | 41.184 | 1.00 | 0.00 | AAAA |
| ATOM | 2485 | CB | PRO | E | 153 | 47.376 | 137.451 | 40.235 | 1.00 | 0.00 | AAAA |
| ATOM | 2488 | CG | PRO | E | 153 | 46.283 | 136.516 | 39.717 | 1.00 | 0.00 | AAAA |
| ATOM | 2491 | CD | PRO | E | 153 | 45.049 | 136.731 | 40.595 | 1.00 | 0.00 | AAAA |
| ATOM | 2494 | C | PRO | E | 153 | 46.352 | 139.740 | 40.592 | 1.00 | 0.00 | AAAA |
| ATOM | 2495 | O | PRO | E | 153 | 45.809 | 139.788 | 39.491 | 1.00 | 0.00 | AAAA |
| ATOM | 2496 | N | GLY | E | 154 | 46.746 | 140.812 | 41.283 | 1.00 | 0.00 | AAAA |
| ATOM | 2498 | CA | GLY | E | 154 | 46.528 | 142.158 | 40.794 | 1.00 | 0.00 | AAAA |
| ATOM | 2501 | C | GLY | E | 154 | 45.719 | 143.068 | 41.707 | 1.00 | 0.00 | AAAA |
| ATOM | 2502 | O | GLY | E | 154 | 45.340 | 144.151 | 41.264 | 1.00 | 0.00 | AAAA |
| ATOM | 2503 | N | THR | E | 155 | 45.301 | 142.583 | 42.878 | 1.00 | 0.00 | AAAA |
| ATOM | 2505 | CA | THR | E | 155 | 44.398 | 143.226 | 43.812 | 1.00 | 0.00 | AAAA |
| ATOM | 2507 | CB | THR | E | 155 | 43.098 | 142.432 | 43.938 | 1.00 | 0.00 | AAAA |
| ATOM | 2509 | OG1 | THR | E | 155 | 43.232 | 141.141 | 44.490 | 1.00 | 0.00 | AAAA |
| ATOM | 2511 | CG2 | THR | E | 155 | 42.363 | 142.291 | 42.604 | 1.00 | 0.00 | AAAA |
| ATOM | 2515 | C | THR | E | 155 | 44.984 | 143.511 | 45.187 | 1.00 | 0.00 | AAAA |
| ATOM | 2516 | O | THR | E | 155 | 44.274 | 143.591 | 96.187 | 1.00 | 0.00 | AAAA |
| ATOM | 2517 | N | MET | E | 156 | 46.317 | 143.546 | 45.253 | 1.00 | 0.00 | AAAA |
| ATOM | 2519 | CA | MET | E | 156 | 47.152 | 143.675 | 46.431 | 1.00 | 0.00 | AAAA |
| ATOM | 2521 | CB | MET | E | 156 | 47.425 | 142.312 | 47.068 | 1.00 | 0.00 | AAAA |
| ATOM | 2524 | CG | MET | E | 156 | 48.169 | 141.252 | 46.253 | 1.00 | 0.00 | AAAA |
| ATOM | 2527 | SD | MET | E | 156 | 46.983 | 140.237 | 45.337 | 1.00 | 0.00 | AAAA |
| ATOM | 2528 | CE | MET | E | 156 | 47.770 | 138.622 | 45.118 | 1.00 | 0.00 | AAAA |
| ATOM | 2532 | C | MET | E | 156 | 48.419 | 144.399 | 45.999 | 1.00 | 0.00 | AAAA |
| ATOM | 2533 | O | MET | E | 156 | 49.246 | 144.918 | 46.746 | 1.00 | 0.00 | AAAA |
| ATOM | 2534 | N | GLU | E | 157 | 48.650 | 144.381 | 44.684 | 1.00 | 0.00 | AAAA |
| ATOM | 2536 | CA | GLU | E | 157 | 49.803 | 144.968 | 44.032 | 1.00 | 0.00 | AAAA |
| ATOM | 2538 | CB | GLU | E | 157 | 51.042 | 144.073 | 44.051 | 1.00 | 0.00 | AAAA |
| ATOM | 2541 | CG | GLU | E | 157 | 52.311 | 144.497 | 43.311 | 1.00 | 0.00 | AAAA |
| ATOM | 2544 | CD | GLU | E | 157 | 53.250 | 145.340 | 44.162 | 1.00 | 0.00 | AAAA |
| ATOM | 2545 | OE1 | GLU | E | 157 | 52.808 | 146.413 | 44.626 | 1.00 | 0.00 | AAAA |
| ATOM | 2546 | OE2 | GLU | E | 157 | 54.338 | 144.861 | 44.549 | 1.00 | 0.00 | AAAA |
| ATOM | 2547 | C | GLU | E | 157 | 49.375 | 145.170 | 42.585 | 1.00 | 0.00 | AAAA |
| ATOM | 2548 | O | GLU | E | 157 | 48.592 | 144.421 | 42.005 | 1.00 | 0.00 | AAAA |
| ATOM | 2549 | N | GLU | E | 158 | 49.819 | 146.231 | 41.908 | 1.00 | 0.00 | AAAA |
| ATOM | 2551 | CA | GLU | E | 158 | 49.415 | 146.636 | 40.577 | 1.00 | 0.00 | AAAA |
| ATOM | 2553 | CB | GLU | E | 158 | 49.765 | 148.120 | 40.452 | 1.00 | 0.00 | AAAA |
| ATOM | 2556 | CG | GLU | E | 158 | 49.213 | 148.826 | 39.214 | 1.00 | 0.00 | AAAA |
| ATOM | 2559 | CD | GLU | E | 158 | 47.718 | 149.109 | 39.233 | 1.00 | 0.00 | AAAA |
| ATOM | 2560 | OE1 | GLU | E | 158 | 47.053 | 148.776 | 40.237 | 1.00 | 0.00 | AAAA |
| ATOM | 2561 | OE2 | GLU | E | 158 | 47.134 | 149.741 | 38.326 | 1.00 | 0.00 | AAAA |
| ATOM | 2562 | C | GLU | E | 158 | 50.300 | 145.862 | 39.611 | 1.00 | 0.00 | AAAA |
| ATOM | 2563 | O | GLU | E | 158 | 51.362 | 146.240 | 39.121 | 1.00 | 0.00 | AAAA |
| ATOM | 2564 | N | LYS | E | 159 | 49.899 | 144.609 | 39.382 | 1.00 | 0.00 | AAAA |
| ATOM | 2566 | CA | LYS | E | 159 | 50.444 | 143.644 | 38.449 | 1.00 | 0.00 | AAAA |
| ATOM | 2568 | CB | LYS | E | 159 | 51.738 | 142.923 | 38.826 | 1.00 | 0.00 | AAAA |
| ATOM | 2571 | CG | LYS | E | 159 | 51.648 | 142.072 | 40.093 | 1.00 | 0.00 | AAAA. |
| ATOM | 2574 | CD | LYS | E | 159 | 53.029 | 141.505 | 40.420 | 1.00 | 0.00 | AAAA |
| ATOM | 2577 | CE | LYS | E | 159 | 53.101 | 140.713 | 41.726 | 1.00 | 0.00 | AAAA |
| ATOM | 2580 | NZ | LYS | E | 159 | 54.465 | 140.517 | 42.241 | 1.00 | 0.00 | AAAA |
| ATOM | 2584 | C | LYS | E | 159 | 49.426 | 142.761 | 37.741 | 1.00 | 0.00 | AAAA |
| ATOM | 2585 | O | LYS | E | 159 | 49.205 | 141.631 | 38.170 | 1.00 | 0.00 | AAAA |
| ATOM | 2586 | N | PRO | E | 160 | 48.634 | 143.292 | 36.806 | 1.00 | 0.00 | AAAA |
| ATOM | 2587 | CA | PRO | E | 160 | 47.566 | 142.598 | 36.113 | 1.00 | 0.00 | AAAA |
| ATOM | 2589 | CB | PRO | E | 160 | 46.798 | 143.698 | 35.379 | 1.00 | 0.00 | AAAA |
| ATOM | 2592 | CG | PRO | E | 160 | 47.749 | 144.878 | 35.179 | 1.00 | 0.00 | AAAA |
| ATOM | 2595 | CD | PRO | E | 160 | 48.745 | 144.650 | 36.317 | 1.00 | 0.00 | AAAA |
| ATOM | 2598 | C | PRO | E | 160 | 48.019 | 141.576 | 35.079 | 1.00 | 0.00 | AAAA |
| ATOM | 2599 | O | PRO | E | 160 | 48.830 | 141.759 | 34.174 | 1.00 | 0.00 | AAAA |
| ATOM | 2600 | N | MET | E | 161 | 47.365 | 140.415 | 35.154 | 1.00 | 0.00 | AAAA |
| ATOM | 2602 | CA | MET | E | 161 | 47.486 | 139.259 | 34.290 | 1.00 | 0.00 | AAAA |
| ATOM | 2604 | CB | MET | E | 161 | 47.570 | 138.014 | 35.174 | 1.00 | 0.00 | AAAA |
| ATOM | 2607 | CG | MET | E | 161 | 48.114 | 136.835 | 34.366 | 1.00 | 0.00 | AAAA |
| ATOM | 2610 | SD | MET | E | 161 | 48.118 | 135.336 | 35.380 | 1.00 | 0.00 | AAAA |
| ATOM | 2611 | CE | MET | E | 161 | 49.453 | 135.713 | 36.542 | 1.00 | 0.00 | AAAA |
| ATOM | 2615 | C | MET | E | 161 | 46.330 | 139.202 | 33.301 | 1.00 | 0.00 | AAAA |
| ATOM | 2616 | O | MET | E | 161 | 46.354 | 138.457 | 32.324 | 1.00 | 0.00 | AAAA |
| ATOM | 2617 | N | CYS | E | 162 | 45.226 | 139.916 | 33.540 | 1.00 | 0.00 | AAAA |
| ATOM | 2619 | CA | CYS | E | 162 | 44.019 | 139.865 | 32.742 | 1.00 | 0.00 | AAAA |
| ATOM | 2621 | CB | CYS | E | 162 | 43.245 | 138.561 | 32.936 | 1.00 | 0.00 | AAAA |
| ATOM | 2624 | SG | CYS | E | 162 | 42.636 | 138.283 | 34.618 | 1.00 | 0.00 | AAAA |
| ATOM | 2625 | C | CYS | E | 162 | 43.205 | 141.132 | 32.960 | 1.00 | 0.00 | AAAA |
| ATOM | 2626 | O | CYS | E | 162 | 43.788 | 142.125 | 33.389 | 1.00 | 0.00 | AAAA |
| ATOM | 2627 | N | GLU | E | 163 | 41.956 | 141.230 | 32.500 | 1.00 | 0.00 | AAAA |
| ATOM | 2629 | CA | GLU | E | 163 | 41.088 | 142.390 | 32.517 | 1.00 | 0.00 | AAAA |
| ATOM | 2631 | CB | GLU | E | 163 | 40.197 | 142.358 | 31.274 | 1.00 | 0.00 | AAAA |
| ATOM | 2634 | CG | GLU | E | 163 | 39.455 | 143.673 | 31.032 | 1.00 | 0.00 | AAAA |
| ATOM | 2637 | CD | GLU | E | 163 | 40.223 | 144.670 | 30.176 | 1.00 | 0.00 | AAAA |
| ATOM | 2638 | OE1 | GLU | E | 163 | 40.745 | 144.314 | 29.098 | 1.00 | 0.00 | AAAA |
| ATOM | 2639 | OE2 | GLU | E | 163 | 40.453 | 145.823 | 30.598 | 1.00 | 0.00 | AAAA |
| ATOM | 2640 | C | GLU | E | 163 | 40.140 | 142.234 | 33.698 | 1.00 | 0.00 | AAAA |
| ATOM | 2641 | O | GLU | E | 163 | 39.687 | 141.153 | 34.065 | 1.00 | 0.00 | AAAA |
| ATOM | 2642 | N | LYS | E | 164 | 39.778 | 143.400 | 34.238 | 1.00 | 0.00 | AAAA |
| ATOM | 2644 | CA | LYS | E | 164 | 38.774 | 143.557 | 35.271 | 1.00 | 0.00 | AAAA |
| ATOM | 2646 | CB | LYS | E | 164 | 38.854 | 144.995 | 35.784 | 1.00 | 0.00 | AAAA |
| ATOM | 2649 | CG | LYS | E | 164 | 40.129 | 145.234 | 36.593 | 1.00 | 0.00 | AAAA |
| ATOM | 2652 | CD | LYS | E | 164 | 40.099 | 146.521 | 37.419 | 1.00 | 0.00 | AAAA |
| ATOM | 2655 | CE | LYS | E | 164 | 41.160 | 146.572 | 38.519 | 1.00 | 0.00 | AAAA |
| ATOM | 2658 | NZ | LYS | E | 164 | 40.977 | 147.718 | 39.422 | 1.00 | 0.00 | AAAA |
| ATOM | 2662 | C | LYS | E | 164 | 37.376 | 143.420 | 34.684 | 1.00 | 0.00 | AAAA |
| ATOM | 2663 | O | LYS | E | 164 | 36.992 | 144.197 | 33.811 | 1.00 | 0.00 | AAAA |
| ATOM | 2664 | N | THR | E | 165 | 36.684 | 142.350 | 35.081 | 1.00 | 0.00 | AAAA |
| ATOM | 2666 | CA | THR | E | 165 | 35.410 | 141.984 | 34.496 | 1.00 | 0.00 | AAAA |
| ATOM | 2668 | CB | THR | E | 165 | 35.362 | 140.547 | 33.975 | 1.00 | 0.00 | AAAA |
| ATOM | 2670 | OG1 | THR | E | 165 | 35.903 | 139.671 | 34.938 | 1.00 | 0.00 | AAAA |
| ATOM | 2672 | CG2 | THR | E | 165 | 36.056 | 140.428 | 32.618 | 1.00 | 0.00 | AAAA |
| ATOM | 2676 | C | THR | E | 165 | 34.355 | 142.213 | 35.568 | 1.00 | 0.00 | AAAA |
| ATOM | 2677 | O | THR | E | 165 | 34.798 | 142.280 | 36.714 | 1.00 | 0.00 | AAAA |
| ATOM | 2678 | N | THR | E | 166 | 33.041 | 142.338 | 35.371 | 1.00 | 0.00 | AAAA |
| ATOM | 2680 | CA | THR | E | 166 | 32.119 | 142.731 | 36.418 | 1.00 | 0.00 | AAAA |
| ATOM | 2682 | CB | THR | E | 166 | 31.343 | 143.909 | 35.831 | 1.00 | 0.00 | AAAA |
| ATOM | 2684 | OG1 | THR | E | 166 | 30.621 | 143.483 | 34.696 | 1.00 | 0.00 | AAAA |
| ATOM | 2686 | CG2 | THR | E | 166 | 32.188 | 145.081 | 35.328 | 1.00 | 0.00 | AAAA |
| ATOM | 2690 | C | THR | E | 166 | 31.212 | 141.605 | 36.893 | 1.00 | 0.00 | AAAA |
| ATOM | 2691 | O | THR | E | 166 | 30.130 | 141.910 | 37.391 | 1.00 | 0.00 | AAAA |
| ATOM | 2692 | N | ILE | E | 167 | 31.626 | 140.352 | 36.691 | 1.00 | 0.00 | AAAA |
| ATOM | 2694 | CA | ILE | E | 167 | 30.916 | 139.118 | 36.960 | 1.00 | 0.00 | AAAA |
| ATOM | 2696 | CB | ILE | E | 167 | 31.594 | 138.000 | 36.166 | 1.00 | 0.00 | AAAA |
| ATOM | 2698 | CG1 | ILE | E | 167 | 31.725 | 138.198 | 34.656 | 1.00 | 0.00 | AAAA |
| ATOM | 2701 | CG2 | ILE | E | 167 | 31.010 | 136.607 | 36.401 | 1.00 | 0.00 | AAAA |
| ATOM | 2705 | CD | ILE | E | 167 | 32.982 | 137.554 | 34.068 | 1.00 | 0.00 | AAAA |
| ATOM | 2709 | C | ILE | E | 167 | 30.808 | 138.928 | 38.466 | 1.00 | 0.00 | AAAA |
| ATOM | 2710 | O | ILE | E | 167 | 31.667 | 139.382 | 39.218 | 1.00 | 0.00 | AAAA |
| ATOM | 2711 | N | ASN | E | 168 | 29.722 | 138.335 | 38.970 | 1.00 | 0.00 | AAAA |
| ATOM | 2713 | CA | ASN | E | 168 | 29.206 | 138.239 | 40.321 | 1.00 | 0.00 | AAAA |
| ATOM | 2715 | CB | ASN | E | 168 | 30.170 | 137.498 | 41.248 | 1.00 | 0.00 | AAAA |
| ATOM | 2718 | CG | ASN | E | 168 | 30.211 | 136.013 | 40.919 | 1.00 | 0.00 | AAAA |
| ATOM | 2719 | OD1 | ASN | E | 168 | 29.343 | 135.467 | 40.241 | 1.00 | 0.00 | AAAA |
| ATOM | 2720 | ND2 | ASN | E | 168 | 31.210 | 135.248 | 41.363 | 1.00 | 0.00 | AAAA |
| ATOM | 2723 | C | ASN | E | 168 | 28.816 | 139.609 | 40.856 | 1.00 | 0.00 | AAAA |
| ATOM | 2724 | O | ASN | E | 168 | 28.757 | 139.679 | 42.082 | 1.00 | 0.00 | AAAA |
| ATOM | 2725 | N | ASN | E | 169 | 28.831 | 140.692 | 40.074 | 1.00 | 0.00 | AAAA |
| ATOM | 2727 | CA | ASN | E | 169 | 28.601 | 142.076 | 40.437 | 1.00 | 0.00 | AAAA |
| ATOM | 2729 | CB | ASN | E | 169 | 27.255 | 142.388 | 41.092 | 1.00 | 0.00 | AAAA |
| ATOM | 2732 | CG | ASN | E | 169 | 27.083 | 142.339 | 42.604 | 1.00 | 0.00 | AAAA |
| ATOM | 2733 | OD1 | ASN | E | 169 | 28.056 | 142.311 | 43.355 | 1.00 | 0.00 | AAAA |
| ATOM | 2734 | ND2 | ASN | E | 169 | 25.834 | 142.459 | 43.062 | 1.00 | 0.00 | AAAA |
| ATOM | 2737 | C | ASN | E | 169 | 29.759 | 142.862 | 41.008 | 1.00 | 0.00 | AAAA |
| ATOM | 2738 | O | ASN | E | 169 | 29.537 | 144.038 | 41.362 | 1.00 | 0.00 | AAAA |
| ATOM | 2739 | N | GLU | E | 170 | 30.921 | 142.232 | 41.113 | 1.00 | 0.00 | AAAA |
| ATOM | 2741 | CA | GLU | E | 170 | 32.168 | 142.821 | 41.555 | 1.00 | 0.00 | AAAA |
| ATOM | 2743 | CB | GLU | E | 170 | 32.852 | 141.900 | 42.567 | 1.00 | 0.00 | AAAA |
| ATOM | 2746 | CG | GLU | E | 170 | 33.584 | 142.741 | 43.613 | 1.00 | 0.00 | AAAA |
| ATOM | 2749 | CD | GLU | E | 170 | 34.643 | 142.009 | 44.425 | 1.00 | 0.00 | AAAA |
| ATOM | 2750 | OE1 | GLU | E | 170 | 35.801 | 142.470 | 44.326 | 1.00 | 0.00 | AAAA |
| ATOM | 2751 | OE2 | GLU | E | 170 | 34.317 | 141.090 | 45.207 | 1.00 | 0.00 | AAAA |
| ATOM | 2752 | C | GLU | E | 170 | 33.179 | 142.983 | 40.428 | 1.00 | 0.00 | AAAA |
| ATOM | 2753 | O | GLU | E | 170 | 33.594 | 142.022 | 39.784 | 1.00 | 0.00 | AAAA |
| ATOM | 2754 | N | TYR | E | 171 | 33.615 | 144.229 | 40.234 | 1.00 | 0.00 | AAAA |
| ATOM | 2756 | CA | TYR | E | 171 | 34.660 | 144.523 | 39.273 | 1.00 | 0.00 | AAAA |
| ATOM | 2758 | CB | TYR | E | 171 | 34.624 | 146.043 | 39.105 | 1.00 | 0.00 | AAAA |
| ATOM | 2761 | CG | TYR | E | 171 | 35.597 | 146.676 | 38.138 | 1.00 | 0.00 | AAAA |
| ATOM | 2762 | CD1 | TYR | E | 171 | 35.707 | 146.339 | 36.784 | 1.00 | 0.00 | AAAA |
| ATOM | 2764 | CD2 | TYR | E | 171 | 36.255 | 147.818 | 38.608 | 1.00 | 0.00 | AAAA |
| ATOM | 2766 | CE1 | TYR | E | 171 | 36.489 | 147.158 | 35.963 | 1.00 | 0.00 | AAAA |
| ATOM | 2768 | CE2 | TYR | E | 171 | 36.982 | 148.684 | 37.781 | 1.00 | 0.00 | AAAA |
| ATOM | 2770 | CZ | TYR | E | 171 | 37.084 | 148.337 | 36.429 | 1.00 | 0.00 | AAAA |
| ATOM | 2771 | OH | TYR | E | 171 | 37.802 | 149.151 | 35.602 | 1.00 | 0.00 | AAAA |
| ATOM | 2773 | C | TYR | E | 171 | 36.047 | 144.098 | 39.733 | 1.00 | 0.00 | AAAA |
| ATOM | 2774 | O | TYR | E | 171 | 36.693 | 144.706 | 40.584 | 1.00 | 0.00 | AAAA |
| ATOM | 2775 | N | ASN | E | 172 | 36.579 | 143.007 | 39.177 | 1.00 | 0.00 | AAAA |
| ATOM | 2777 | CA | ASN | E | 172 | 37.727 | 142.224 | 39.584 | 1.00 | 0.00 | AAAA |
| ATOM | 2779 | CB | ASN | E | 172 | 37.306 | 141.409 | 40.808 | 1.00 | 0.00 | AAAA |
| ATOM | 2782 | CG | ASN | E | 172 | 38.420 | 141.100 | 41.798 | 1.00 | 0.00 | AAAA |
| ATOM | 2783 | OD1 | ASN | E | 172 | 39.592 | 140.929 | 41.467 | 1.00 | 0.00 | AAAA |
| ATOM | 2784 | ND2 | ASN | E | 172 | 38.089 | 141.225 | 43.086 | 1.00 | 0.00 | AAAA |
| ATOM | 2787 | C | ASN | E | 172 | 38.197 | 141.295 | 38.474 | 1.00 | 0.00 | AAAA |
| ATOM | 2788 | O | ASN | E | 172 | 37.492 | 141.053 | 37.496 | 1.00 | 0.00 | AAAA |
| ATOM | 2789 | N | TYR | E | 173 | 39.440 | 140.818 | 38.567 | 1.00 | 0.00 | AAAA |
| ATOM | 2791 | CA | TYR | E | 173 | 40.083 | 139.926 | 37.624 | 1.00 | 0.00 | AAAA |
| ATOM | 2793 | CB | TYR | E | 173 | 41.584 | 140.044 | 37.887 | 1.00 | 0.00 | AAAA |
| ATOM | 2796 | CG | TYR | E | 173 | 42.299 | 141.368 | 37.759 | 1.00 | 0.00 | AAAA |
| ATOM | 2797 | CD1 | TYR | E | 173 | 42.845 | 141.809 | 36.548 | 1.00 | 0.00 | AAAA |
| ATOM | 2799 | CD2 | TYR | E | 173 | 42.409 | 142.179 | 38.895 | 1.00 | 0.00 | AAAA |
| ATOM | 2801 | CE1 | TYR | E | 173 | 43.501 | 143.045 | 36.559 | 1.00 | 0.00 | AAAA |
| ATOM | 2803 | CE2 | TYR | E | 173 | 43.049 | 143.424 | 38.884 | 1.00 | 0.00 | AAAA |
| ATOM | 2805 | CZ | TYR | E | 173 | 43.623 | 143.867 | 37.686 | 1.00 | 0.00 | AAAA |
| ATOM | 2806 | OH | TYR | E | 173 | 44.354 | 145.017 | 37.625 | 1.00. | 0.00 | AAAA |
| ATOM | 2808 | C | TYR | E | 173 | 39.582 | 138.540 | 38.002 | 1.00 | 0.00 | AAAA |
| ATOM | 2809 | O | TYR | E | 173 | 39.576 | 138.107 | 39.152 | 1.00 | 0.00 | AAAA |
| ATOM | 2810 | N | ARG | E | 174 | 39.172 | 137.712 | 37.037 | 1.00 | 0.00 | AAAA |
| ATOM | 2812 | CA | ARG | E | 174 | 38.736 | 136.341 | 37.214 | 1.00 | 0.00 | AAAA |
| ATOM | 2814 | CB | ARG | E | 174 | 37.880 | 135.747 | 36.095 | 1.00 | 0.00 | AAAA |
| ATOM | 2817 | CG | ARG | E | 174 | 36.598 | 136.522 | 35.796 | 1.00 | 0.00 | AAAA |
| ATOM | 2820 | CD | ARG | E | 174 | 35.654 | 136.706 | 36.985 | 1.00 | 0.00 | AAAA |
| ATOM | 2823 | NE | ARG | E | 174 | 35.431 | 138.117 | 37.300 | 1.00 | 0.00 | AAAA |
| ATOM | 2825 | CZ | ARG | E | 174 | 34.644 | 138.596 | 38.274 | 1.00 | 0.00 | AAAA |
| ATOM | 2826 | NH1 | ARG | E | 174 | 34.041 | 137.774 | 39.143 | 1.00 | 0.00 | AAAA |
| ATOM | 2829 | NH2 | ARG | E | 174 | 34.479 | 139.914 | 38.446 | 1.00 | 0.00 | AAAA |
| ATOM | 2832 | C | ARG | E | 174 | 40.015 | 135.552 | 37.455 | 1.00 | 0.00 | AAAA |
| ATOM | 2833 | O | ARG | E | 174 | 40.878 | 135.554 | 36.580 | 1.00 | 0.00 | AAAA |
| ATOM | 2834 | N | CYS | E | 175 | 40.110 | 134.792 | 38.549 | 1.00 | 0.00 | AAAA |
| ATOM | 2836 | CA | CYS | E | 175 | 41.316 | 134.052 | 38.865 | 1.00 | 0.00 | AAAA |
| ATOM | 2838 | CB | CYS | E | 175 | 42.257 | 134.904 | 39.717 | 1.00 | 0.00 | AAAA |
| ATOM | 2841 | SG | CYS | E | 175 | 41.721 | 134.921 | 41.446 | 1.00 | 0.00 | AAAA |
| ATOM | 2842 | C | CYS | E | 175 | 41.021 | 132.627 | 39.314 | 1.00 | 0.00 | AAAA |
| ATOM | 2843 | O | CYS | E | 175 | 40.094 | 132.412 | 40.091 | 1.00 | 0.00 | AAAA |
| ATOM | 2844 | N | TRP | E | 176 | 41.846 | 131.641 | 38.952 | 1.00 | 0.00 | AAAA |
| ATOM | 2846 | CA | TRP | E | 176 | 41.774 | 130.323 | 39.550 | 1.00 | 0.00 | AAAA |
| ATOM | 2848 | CB | TRP | E | 176 | 42.420 | 129.386 | 38.530 | 1.00 | 0.00 | AAAA |
| ATOM | 2851 | CG | TRP | E | 176 | 41.541 | 129.041 | 37.371 | 1.00 | 0.00 | AAAA |
| ATOM | 2852 | CD1 | TRP | E | 176 | 41.679 | 129.602 | 36.149 | 1.00 | 0.00 | AAAA |
| ATOM | 2854 | CD2 | TRP | E | 176 | 40.558 | 127.973 | 37.219 | 1.00 | 0.00 | AAAA |
| ATOM | 2855 | NE1 | TRP | E | 176 | 40.874 | 128.941 | 35.243 | 1.00 | 0.00 | AAAA |
| ATOM | 2857 | CE2 | TRP | E | 176 | 40.118 | 127.970 | 35.869 | 1.00 | 0.00 | AAAA |
| ATOM | 2858 | CE3 | TRP | E | 176 | 40.074 | 126.948 | 38.048 | 1.00 | 0.00 | AAAA |
| ATOM | 2860 | CZ2 | TRP | E | 176 | 39.233 | 127.020 | 35.348 | 1.00 | 0.00 | AAAA |
| ATOM | 2862 | CZ3 | TRP | E | 176 | 39.079 | 126.084 | 37.577 | 1.00 | 0.00 | AAAA |
| ATOM | 2864 | CH2 | TRP | E | 176 | 38.686 | 126.083 | 36.233 | 1.00 | 0.00 | AAAA |
| ATOM | 2866 | C | TRP | E | 176 | 42.434 | 130.204 | 40.917 | 1.00 | 0.00 | AAAA |
| ATOM | 2867 | O | TRP | E | 176 | 41.990 | 129.451 | 41.781 | 1.00 | 0.00 | AAAA |
| ATOM | 2868 | N | THR | E | 177 | 43.454 | 131.039 | 41.126 | 1.00 | 0.00 | AAAA |
| ATOM | 2870 | CA | THR | E | 177 | 44.308 | 131.247 | 42.278 | 1.00 | 0.00 | AAAA |
| ATOM | 2872 | CB | THR | E | 177 | 45.026 | 129.955 | 42.670 | 1.00 | 0.00 | AAAA |
| ATOM | 2874 | OG1 | THR | E | 177 | 45.840 | 129.965 | 43.821 | 1.00 | 0.00 | AAAA |
| ATOM | 2876 | CG2 | THR | E | 177 | 45.937 | 129.263 | 41.656 | 1.00 | 0.00 | AAAA |
| ATOM | 2880 | C | THR | E | 177 | 45.147 | 132.506 | 42.110 | 1.00 | 0.00 | AAAA |
| ATOM | 2881 | O | THR | E | 177 | 45.212 | 133.059 | 41.014 | 1.00 | 0.00 | AAAA |
| ATOM | 2882 | N | THR | E | 178 | 45.961 | 132.849 | 43.111 | 1.00 | 0.00 | AAAA |
| ATOM | 2884 | CA | THR | E | 178 | 46.892 | 133.919 | 43.411 | 1.00 | 0.00 | AAAA |
| ATOM | 2886 | CB | THR | E | 178 | 47.522 | 133.758 | 44.794 | 1.00 | 0.00 | AAAA |
| ATOM | 2888 | OG1 | THR | E | 178 | 46.630 | 133.107 | 45.673 | 1.00 | 0.00 | AAAA |
| ATOM | 2890 | CG2 | THR | E | 178 | 47.718 | 135.062 | 45.567 | 1.00 | 0.00 | AAAA |
| ATOM | 2894 | C | THR | E | 178 | 47.881 | 134.233 | 42.297 | 1.00 | 0.00 | AAAA |
| ATOM | 2895 | O | THR | E | 178 | 48.604 | 135.225 | 42.360 | 1.00 | 0.00 | AAAA |
| ATOM | 2896 | N | ASN | E | 179 | 48.129 | 133.340 | 41.335 | 1.00 | 0.00 | AAAA |
| ATOM | 2898 | CA | ASN | E | 179 | 49.158 | 133.362 | 40.316 | 1.00 | 0.00 | AAAA |
| ATOM | 2900 | CB | ASN | E | 179 | 50.475 | 132.752 | 40.794 | 1.00 | 0.00 | AAAA |
| ATOM | 2903 | CG | ASN | E | 179 | 50.323 | 131.256 | 41.030 | 1.00 | 0.00 | AAAA |
| ATOM | 2904 | OD1 | ASN | E | 179 | 49.284 | 130.770 | 41.470 | 1.00 | 0.00 | AAAA |
| ATOM | 2905 | ND2 | ASN | E | 179 | 51.354 | 130.482 | 40.683 | 1.00 | 0.00 | AAAA |
| ATOM | 2908 | C | ASN | E | 179 | 48.703 | 132.831 | 38.964 | 1.00 | 0.00 | AAAA |
| ATOM | 2909 | O | ASN | E | 179 | 49.504 | 132.324 | 38.181 | 1.00 | 0.00 | AAAA |
| ATOM | 2910 | N | ARG | E | 180 | 47.402 | 132.811 | 38.663 | 1.00 | 0.00 | AAAA |
| ATOM | 2912 | CA | ARG | E | 180 | 46.767 | 132.463 | 37.407 | 1.00 | 0.00 | AAAA |
| ATOM | 2914 | CB | ARG | E | 180 | 46.438 | 130.970 | 37.358 | 1.00 | 0.00 | AAAA |
| ATOM | 2917 | CG | ARG | E | 180 | 47.571 | 129.967 | 37.143 | 1.00 | 0.00 | AAAA |
| ATOM | 2920 | CD | ARG | E | 180 | 47.178 | 128.497 | 37.288 | 1.00 | 0.00 | AAAA |
| ATOM | 2923 | NE | ARG | E | 180 | 48.237 | 127.517 | 37.052 | 1.00 | 0.00 | AAAA |
| ATOM | 2925 | CZ | ARG | E | 180 | 48.471 | 126.318 | 37.604 | 1.00 | 0.00 | AAAA |
| ATOM | 2926 | NH1 | ARG | E | 180 | 47.584 | 125.898 | 38.517 | 1.00 | 0.00 | AAAA |
| ATOM | 2929 | NH2 | ARG | E | 180 | 49.534 | 125.585 | 37.244 | 1.00 | 0.00 | AAAA |
| ATOM | 2932 | C | ARG | E | 180 | 45.488 | 133.280 | 3.7.287 | 1.00 | 0.00 | AAAA |
| ATOM | 2933 | O | ARG | E | 180 | 44.714 | 133.297 | 38.242 | 1.00 | 0.00 | AAAA |
| ATOM | 2934 | N | CYS | E | 181 | 45.217 | 133.955' | 36.169 | 1.00 | 0.00 | AAAA |
| ATOM | 2936 | CA | CYS | E | 181 | 43.925 | 134.451 | 35.736 | 1.00 | 0.00 | AAAA |
| ATOM | 2938 | CB | CYS | E | 181 | 44.091 | 135.651 | 34.802 | 1.00 | 0.00 | AAAA |
| ATOM | 2941 | SG | CYS | E | 181 | 44.054 | 137.248 | 35.653 | 1.00 | 0.00 | AAAA |
| ATOM | 2942 | C | CYS | E | 181 | 43.216 | 133.337 | 34.979 | 1.00 | 0.00 | AAAA |
| ATOM | 2943 | O | CYS | E | 181 | 43.800 | 132.285 | 34.729 | 1.00 | 0.00 | AAAA |
| ATOM | 2944 | N | GLN | E | 182 | 41.935 | 133.479 | 34.635 | 1.00 | 0.00 | AAAA |
| ATOM | 2946 | CA | GLN | E | 182 | 41.191 | 132.494 | 33.877 | 1.00 | 0.00 | AAAA |
| ATOM | 2948 | CB | GLN | E | 182 | 39.680 | 132.729 | 33.913 | 1.00 | 0.00 | AAAA |
| ATOM | 2951 | CG | GLN | E | 182 | 38.816 | 131.582 | 33.388 | 1.00 | 0.00 | AAAA |
| ATOM | 2954 | CD | GLN | E | 182 | 37.347 | 131.686 | 33.770 | 1.00 | 0.00 | AAAA |
| ATOM | 2955 | OE1 | GLN | E | 182 | 36.967 | 132.646 | 34.438 | 1.00 | 0.00 | AAAA |
| ATOM | 2956 | NE2 | GLN | E | 182 | 36.534 | 130.664 | 33.489 | 1.00 | 0.00 | AAAA |
| ATOM | 2959 | C | GLN | E | 182 | 41.734 | 132.582 | 32.458 | 1.00 | 0.00 | AAAA |
| ATOM | 2960 | O1 | GLN | E | 182 | 42.264 | 133.624 | 32.016 | 1.00 | 0.00 | AAAA |
| ATOM | 2961 | 02 | GLN | E | 182 | 41.482 | 131.649 | 31.666 | 1.00 | 0.00 | AAAA |
| ATOM | 2962 | N | ALA | E | 681 | 17.923 | 119.007 | 35.300 | 1.00 | 0.00 | AAAB |
| ATOM | 2965 | CA | ALA | E | 681 | 17.782 | 117.757 | 36.018 | 1.00 | 0.00 | AAAB |
| ATOM | 2967 | CB | ALA | E | 681 | 18.958 | 116.782 | 35.957 | 1.00 | 0.00 | AAAB |
| ATOM | 2971 | C | ALA | E | 681 | 17.279 | 117.961 | 37.440 | 1.00 | 0.00 | AAAB |
| ATOM | 2972 | O | ALA | E | 681 | 17.556 | 118.999 | 38.038 | 1.00 | 0.00 | AAAB |
| ATOM | 2973 | N | GLU | E | 682 | 16.537 | 116.986 | 37.970 | 1.00 | 0.00 | AAAB |
| ATOM | 2975 | CA | GLU | E | 682 | 16.020 | 117.062 | 39.322 | 1.00 | 0.00 | AAAB |
| ATOM | 2977 | CB | GLU | E | 682 | 14.668 | 117.772 | 39.387 | 1.00 | 0.00 | AAAB |
| ATOM | 2980 | CG | GLU | E | 682 | 14.223 | 118.158 | 40.798 | 1.00 | 0.00 | AAAB |
| ATOM | 2983 | CD | GLU | E | 682 | 13.132 | 119.217 | 40.730 | .1.00 | 0.00 | AAAB |
| ATOM | 2984 | OE1 | GLU | E | 682 | 11.974 | 118.948 | 40.345 | 1.00 | 0.00 | AAAB |
| ATOM | 2985 | OE2 | GLU | E | 682 | 13.524 | 120.373 | 41.003 | 1.00 | 0.00 | AAAB |
| ATOM | 2986 | C | GLU | E | 682 | 16.133 | 115.769 | 40.116 | 1.00 | 0.00 | AAAB |
| ATOM | 2987 | O | GLU | E | 682 | 16.908 | 115.702 | 41.069 | 1.00 | 0.00 | AAAB |
| ATOM | 2988 | N | LYS | E | 683 | 15.341 | 114.747 | 39.786 | 1.00 | 0.00 | AAAB |
| ATOM | 2990 | CA | LYS | E | 683 | 15.385 | 113.424 | 40.377 | 1.00 | 0.00 | AAAB |
| ATOM | 2992 | CB | LYS | E | 683 | 14.300 | 112.488 | 39.843 | 1.00 | 0.00 | AAAB |
| ATOM | 2995 | CG | LYS | E | 683 | 14.113 | 111.110 | 40.480 | 1.00 | 0.00 | AAAB |
| ATOM | 2998 | CD | LYS | E | 683 | 13.575 | 111.290 | 41.900 | 1.00 | 0.00 | AAAB |
| ATOM | 3001 | CE | LYS | E | 683 | 12.842 | 110.112 | 42.542 | 1.00 | 0.00 | AAAB |
| ATOM | 3004 | NZ | LYS | E | 683 | 12.249 | 110.767 | 43.719 | 1.00 | 0.00 | AAAB |
| ATOM | 3008 | C | LYS | E | 683 | 16.737 | 112.737 | 40.246 | 1.00 | 0.00 | AAAB |
| ATOM | 3009 | O | LYS | E | 683 | 16.990 | 111.974 | 41.176 | 1.00 | 0.00 | AAAB |
| ATOM | 3010 | N | GLU | E | 684 | 17.582 | 113.010 | 39.249 | 1.00 | 0.00 | AAAB |
| ATOM | 3012 | CA | GLU | E | 684 | 18.914 | 112.496 | 39.001 | 1.00 | 0.00 | AAAB |
| ATOM | 3014 | CB | GLU | E | 684 | 19.423 | 112.860 | 37.607 | 1.00 | 0.00 | AAAB |
| ATOM | 3017 | CG | GLU | E | 684 | 19.715 | 111.883 | 36.467 | 1.00 | 0.00 | AAAB |
| ATOM | 3020 | CD | GLU | E | 684 | 18.498 | 111.610 | 35.596 | 1.00 | 0.00 | AAAB |
| ATOM | 3021 | OE1 | GLU | E | 684 | 18.712 | 111.167 | 34.446 | 1.00 | 0.00 | AAAB |
| ATOM | 3022 | OE2 | GLU | E | 684 | 17.310 | 111.814 | 35.928 | 1.00 | 0.00 | AAAB |
| ATOM | 3023 | C | GLU | E | 684 | 19.868 | 112.864 | 40.129 | 1.00 | 0.00 | AAAB |
| ATOM | 3024 | O | GLU | E | 684 | 20.759 | 112.047 | 40.348 | 1.00 | 0.00 | AAAB |
| ATOM | 3025 | N | GLU | E | 685 | 19.649 | 113.988 | 40.814 | 1.00 | 0.00 | AAAB |
| ATOM | 3027 | CA | GLU | E | 685 | 20.504 | 114.427 | 41.899 | 1.00 | 0.00 | AAAB |
| ATOM | 3029 | CB | GLU | E | 685 | 20.089 | 115.872 | 42.179 | 1.00 | 0.00 | AAAB |
| ATOM | 3032 | CG | GLU | E | 685 | 21.043 | 116.531 | 43.176 | 1.00 | 0.00 | AAAB |
| ATOM | 3035 | CD | GLU | E | 685 | 20.776 | 116.524 | 44.675 | 1.00 | 0.00 | AAAB |
| ATOM | 3036 | OE1 | GLU | E | 685 | 19.576 | 116.382 | 44.995 | 1.00 | 0.00 | AAAB |
| ATOM | 3037 | OE2 | GLU | E | 685 | 21.692 | 116.380 | 45.513 | 1.00 | 0.00 | AAAB |
| ATOM | 3038 | C | GLU | E | 685 | 20.104 | 113.605 | 43.116 | 1.00 | 0.00 | AAAB |
| ATOM | 3039 | O | GLU | E | 685 | 21.069 | 113.259 | 43.796 | 1.00 | 0.00 | AAAB |
| ATOM | 3040 | N | ALA | E | 686 | 18.863 | 113.184 | 43.371 | 1.00 | 0.00 | AAAB |
| ATOM | 3042 | CA | ALA | E | 686 | 18.557 | 112.332 | 44.502 | 1.00 | 0.00 | AAAB |
| ATOM | 3044 | CB | ALA | E | 686 | 17.042 | 112.353 | 44.697 | 1.00 | 0.00 | AAAB |
| ATOM | 3048 | C | ALA | E | 686 | 19.002 | 110.880 | 44.399 | 1.00 | 0.00 | AAAB |
| ATOM | 3049 | O | ALA | E | 686 | 19.181 | 110.238 | 45.432 | 1.00 | 0.00 | AAAB |
| ATOM | 3050 | N | GLU | E | 687 | 19.278 | 110.426 | 43.174 | 1.00 | 0.00 | AAAB |
| ATOM | 3052 | CA | GLU | E | 687 | 19.769 | 109.087 | 42.922 | 1.00 | 0.00 | AAAB |
| ATOM | 3054 | CB | GLU | E | 687 | 19.362 | 108.693 | 41.502 | 1.00 | 0.00 | AAAB |
| ATOM | 3057 | CG | GLU | E | 687 | 19.345 | 107.186 | 41.245 | 1.00 | 0.00 | AAAB |
| ATOM | 3060 | CD | GLU | E | 687 | 18.262 | 106.316 | 41.867 | 1.00 | 0.00 | AAAB |
| ATOM | 3061 | OE1 | GLU | E | 687 | 17.379 | 106.882 | 42.546 | 1.00 | 0.00 | AAAB |
| ATOM | 3062 | OE2 | GLU | E | 687 | 18.321 | 105.078 | 41.697 | 1.00 | 0.00 | AAAB |
| ATOM | 3063 | C | GLU | E | 687 | 21.289 | 109.115 | 42.988 | 1.00 | 0.00 | AAAB |
| ATOM | 3064 | O | GLU | E | 687 | 21.854 | 108.124 | 43.445 | 1.00 | 0.00 | AAAB |
| ATOM | 3065 | N | TYR | E | 688 | 21.947 | 110.230 | 42.664 | 1.00 | 0.00 | AAAB |
| ATOM | 3067 | CA | TYR | E | 688 | 23.392 | 110.358 | 42.655 | 1.00 | 0.00 | AAAB |
| ATOM | 3069 | CB | TYR | E | 688 | 23.663 | 111.735 | 42.046 | 1.00 | 0.00 | AAAB |
| ATOM | 3072 | CG | TYR | E | 688 | 24.951 | 112.401 | 42.466 | 1.00 | 0.00 | AAAB |
| ATOM | 3073 | CD1 | TYR | E | 688 | 26.129 | 112.231 | 41.730 | 1.00 | 0.00 | AAAB |
| ATOM | 3075 | CD2 | TYR | E | 688 | 24.843 | 113.385 | 43.458 | 1.00 | 0.00 | AAAB |
| ATOM | 3077 | CE1 | TYR | E | 688 | 27.273 | 112.958 | 42.085 | 1.00 | 0.00 | AAAB |
| ATOM | 3079 | CE2 | TYR | E | 688 | 26.000 | 114.089 | 43.812 | 1.00 | 0.00 | AAAB |
| ATOM | 3081 | CZ | TYR | E | 688 | 27.198 | 113.907 | 43.111 | 1.00 | 0.00 | AAAB |
| ATOM | 3082 | OH | TYR | E | 688 | 28.305 | 114.655 | 43.386 | 1.00 | 0.00 | AAAB |
| ATOM | 3084 | C | TYR | E | 688 | 24.017 | 110.203 | 44.033 | 1.00 | 0.00 | AAAB |
| ATOM | 3085 | O | TYR | E | 688 | 25.193 | 109.852 | 44.098 | 1.00 | 0.00 | AAAB |
| ATOM | 3086 | N | ARG | E | 689 | 23.313 | 110.537 | 45.118 | 1.00 | 0.00 | AAAB |
| ATOM | 3088 | CA | ARG | E | 689 | 23.887 | 110.504 | 46.448 | 1.00 | 0.00 | AAAB |
| ATOM | 3090 | CB | ARG | E | 689 | 23.124 | 111.503. | 47.319 | 1.00 | 0.00 | AAAB |
| ATOM | 3093 | CG | ARG | E | 689 | 23.362 | 112.960 | 46.922 | 1.00 | 0.00 | AAAB |
| ATOM | 3096 | CD | ARG | E | 689 | 22.723 | 113.984 | 47.860 | 1.00 | 0.00 | AAAB |
| ATOM | 3099 | NE | ARG | E | 689 | 21.433 | 114.424 | 47.329 | 1.00 | 0.00 | AAAB |
| ATOM | 3101 | CZ | ARG | E | 689 | 20.191 | 114.215 | 47.788 | 1.00 | 0.00 | AAAB |
| ATOM | 3102 | NH1 | ARG | E | 689 | 19.912 | 113.334 | 48.759 | 1.00 | 0.00 | AAAB |
| ATOM | 3105 | NH2 | ARG | E | 689 | 19.153 | 114.828 | 47.203 | 1.00 | 0.00 | AAAB |
| ATOM | 3108 | C | ARG | E | 689 | 24.054 | 109.153 | 47.127 | 1.00 | 0.00 | AAAB |
| ATOM | 3109 | O | ARG | E | 689 | 24.430 | 109.059 | 48.293 | 1.00 | 0.00 | AAAB |
| ATOM | 3110 | N | LYS | E | 690 | 23.846 | 108.084 | 46.354 | 1.00 | 0.00 | AAAB |
| ATOM | 3112 | CA | LYS | E | 690 | 23.921 | 106.690 | 46.742 | 1.00 | 0.00 | AAAB |
| ATOM | 3114 | CB | LYS | E | 690 | 22.820 | 106.096 | 45.864 | 1.00 | 0.00 | AAAB |
| ATOM | 3117 | CG | LYS | E | 690 | 21.380 | 106.487 | 46.198 | 1.00 | 0.00 | AAAB |
| ATOM | 3120 | CD | LYS | E | 690 | 20.345 | 105.679 | 45.416 | 1.00 | 0.00 | AAAB |
| ATOM | 3123 | CE | LYS | E | 690 | 18.891 | 105.955 | 45.804 | 1.00 | 0.00 | AAAB |
| ATOM | 3126 | NZ | LYS | E | 690 | 17.870 | 105.471 | 44.861 | 1.00 | 0.00 | AAAB |
| ATOM | 3130 | C | LYS | E | 690 | 25.256 | 106.136 | 46.263 | 1.00 | 0.00 | AAAB |
| ATOM | 3131 | O | LYS | E | 690 | 25.640 | 105.068 | 46.735 | 1.00 | 0.00 | AAAB |
| ATOM | 3132 | N | VAL | E | 691 | 26.005 | 106.840 | 45.413 | 1.00 | 0.00 | AAAB |
| ATOM | 3134 | CA | VAL | E | 691 | 27.215 | 106.403 | 44.745 | 1.00 | 0.00 | AAAB |
| ATOM | 3136 | CB | VAL | E | 691 | 27.645 | 107.330 | 43.608 | 1.00 | 0.00 | AAAB |
| ATOM | 3138 | CG1 | VAL | E | 691 | 26.644 | 107.290 | 42.453 | 1.00 | 0.00 | AAAB |
| ATOM | 3142 | CG2 | VAL | E | 691 | 28.001 | 108.755 | 44.034 | 1.00 | 0.00 | AAAB |
| ATOM | 3146 | C | VAL | E | 691 | 28.377 | 106.309 | 45.722 | 1.00 | 0.00 | AAAB |
| ATOM | 3147 | O | VAL | E | 691 | 29.226 | 105.431 | 45.583 | 1.00 | 0.00 | AAAB |
| ATOM | 3148 | N | PHE | E | 692 | 28.350 | 107.168 | 46.744 | 1.00 | 0.00 | AAAB |
| ATOM | 3150 | CA | PHE | E | 692 | 29.416 | 107.301 | 47.716 | 1.00 | 0.00 | AAAB |
| ATOM | 3152 | CB | PHE | E | 692 | 29.492 | 108.731 | 48.252 | 1.00 | 0.00 | AAAB |
| ATOM | 3155 | CG | PHE | E | 692 | 29.600 | 109.908 | 47.312 | 1.00 | 0.00 | AAAB |
| ATOM | 3156 | CD1 | PHE | E | 692 | 30.710 | 109.991 | 46.464 | 1.00 | 0.00 | AAAB |
| ATOM | 3158 | CD2 | PHE | E | 692 | 28.563 | 110.841 | 47.191 | 1.00 | 0.00 | AAAB |
| ATOM | 3160 | CE1 | PHE | E | 692 | 30.744 | 110.998 | 45.491 | 1.00 | 0.00 | AAAB |
| ATOM | 3162 | CE2 | PHE | E | 692 | 28.625 | 111.845 | 46.217 | 1.00 | 0.00 | AAAB |
| ATOM | 3164 | CZ | PHE | E | 692 | 29.702 | 111.920 | 45.326 | 1.00 | 0.00 | AAAB |
| ATOM | 3166 | C | PHE | E | 692 | 29.418 | 106.172 | 48.738 | 1.00 | 0.00 | AAAB |
| ATOM | 3167 | O | PHE | E | 692 | 30.424 | 105.534 | 49.042 | 1.00 | 0.00 | AAAB |
| ATOM | 3168 | N | GLU | E | 693 | 28.208 | 105.870 | 49.213 | 1.00 | 0.00 | AAAB |
| ATOM | 3170 | CA | GLU | E | 693 | 27.881 | 104.654 | 49.929 | 1.00 | 0.00 | AAAB |
| ATOM | 3172 | CB | GLU | E | 693 | 26.387 | 104.535 | 50.235 | 1.00 | 0.00 | AAAB |
| ATOM | 3175 | CG | GLU | E | 693 | 25.822 | 104.112 | 51.591 | 1.00 | 0.00 | AAAB |
| ATOM | 3178 | CD | GLU | E | 693 | 25.804 | 102.620 | 51.897 | 1.00 | 0.00 | AAAB |
| ATOM | 3179 | OE1 | GLU | E | 693 | 25.410 | 101.836 | 51.007 | 1.00 | 0.00 | AAAB |
| ATOM | 3180 | OE2 | GLU | E | 693 | 25.981 | 102.298 | 53.092 | 1.00 | 0.00 | AAAB |
| ATOM | 3181 | C | GLU | E | 693 | 28.440 | 103.435 | 49.211 | 1.00 | 0.00 | AAAB |
| ATOM | 3182 | O | GLU | E | 693 | 28.963 | 102.519 | 49.843 | 1.00 | 0.00 | AAAB |
| ATOM | 3183 | N | ASN | E | 694 | 28.498 | 103.434 | 47.878 | 1.00 | 0.00 | AAAB |
| ATOM | 3185 | CA | ASN | E | 694 | 28.838 | 102.280 | 47.070 | 1.00 | 0.00 | AAAB |
| ATOM | 3187 | CB | ASN | E | 694 | 28.168 | 102.412 | 45.702 | 1.00 | 0.00 | AAAB |
| ATOM | 3190 | CG | ASN | E | 694 | 28.076 | 100.996 | 45.152 | 1.00 | 0.00 | AAAB |
| ATOM | 3191 | OD1 | ASN | E | 694 | 29.013 | 100.465 | 44.558 | 1.00 | 0.00 | AAAB |
| ATOM | 3192 | ND2 | ASN | E | 694 | 26.932 | 100.326 | 45.309 | 1.00 | 0.00 | AAAB |
| ATOM | 3195 | C | ASN | E | 694 | 30.338 | 102.318 | 46.815 | 1.00 | 0.00 | AAAB |
| ATOM | 3196 | O | ASN | E | 694 | 30.909 | 101.293 | 46.447 | 1.00 | 0.00 | AAAB |
| ATOM | 3197 | N | PHE | E | 695 | 30.990 | 103.470 | 46.987 | 1.00 | 0.00 | AAAB |
| ATOM | 3199 | CA | PHE | E | 695 | 32.381 | 103.665 | 46.627 | 1.00 | 0.00 | AAAB |
| ATOM | 3201 | CB | PHE | E | 695 | 32.600 | 105.041 | 45.998 | 1.00 | 0.00 | AAAB |
| ATOM | 3204 | CG | PHE | E | 695 | 32.287 | 105.265 | 44.538 | 1.00 | 0.00 | AAAB |
| ATOM | 3205 | CD1 | PHE | E | 695 | 32.698 | 104.340 | 43.570 | 1.00 | 0.00 | AAAB |
| ATOM | 3207 | CD2 | PHE | E | 695 | 31.653 | 106.428 | 44.082 | 1.00 | 0.00 | AAAB |
| ATOM | 3209 | CE1 | PHE | E | 695 | 32.502 | 104.539 | 42.199 | 1.00 | 0.00 | AAAB |
| ATOM | 3211 | CE2 | PHE | E | 695 | 31.379 | 106.604 | 42.720 | 1.00 | 0.00 | AAAB |
| ATOM | 3213 | CZ | PHE | E | 695 | 31.864 | 105.707 | 41.761 | 1.00 | 0.00 | AAAB |
| ATOM | 3215 | C | PHE | E | 695 | 33.298 | 103.311 | 47.789 | 1.00 | 0.00 | AAAB |
| ATOM | 3216 | O | PHE | E | 695 | 34.367 | 102.780 | 47.492 | 1.00 | 0.00 | AAAB |
| ATOM | 3217 | N | LEU | E | 696 | 32.751 | 103.351 | 49.006 | 1.00 | 0.00 | AAAB |
| ATOM | 3219 | CA | LEU | E | 696 | 33.462 | 102.960 | 50.206 | 1.00 | 0.00 | AAAB |
| ATOM | 3221 | CB | LEU | E | 696 | 32.847 | 103.732 | 51.373 | 1.00 | 0.00 | AAAB |
| ATOM | 3224 | CG | LEU | E | 696 | 33.186 | 105.223 | 51.398 | 1.00 | 0.00 | AAAB |
| ATOM | 3226 | CD1 | LEU | E | 696 | 32.448 | 105.964 | 52.513 | 1.00 | 0.00 | AAAB |
| ATOM | 3230 | CD2 | LEU | E | 696 | 34.700 | 105.421 | 51.475 | 1.00 | 0.00 | AAAB |
| ATOM | 3234 | C | LEU | E | 696 | 33.178 | 101.539 | 50.669 | 1.00 | 0.00 | AAAB |
| ATOM | 3235 | O | LEU | E | 696 | 33.956 | 101.054 | 51.486 | 1.00 | 0.00 | AAAB |
| ATOM | 3236 | N | HIS | E | 697 | 32.194 | 100.870 | 50.061 | 1.00 | 0.00 | AAAB |
| ATOM | 3238 | CA | HIS | E | 697 | 31.947 | 99.445 | 50.125 | 1.00 | 0.00 | AAAB |
| ATOM | 3240 | CB | HIS | E | 697 | 30.439 | 99.268 | 49.943 | 1.00 | 0.00 | AAAB |
| ATOM | 3243 | CG | HIS | E | 697 | 29.958 | 97.845 | 49.877 | 1.00 | 0.00 | AAAB |
| ATOM | 3244 | ND1 | HIS | E | 697 | 29.997 | 97.045 | 48.735 | 1.00 | 0.00 | AAAB |
| ATOM | 3245 | CD2 | HIS | E | 697 | 29.393 | 97.099 | 50.877 | 1.00 | 0.00 | AAAB |
| ATOM | 3247 | CE1 | HIS | E | 697 | 29.403 | 95.884 | 49.023 | 1.00 | 0.00 | AAAB |
| ATOM | 3249 | NE2 | HIS | E | 697 | 29.052 | 95.883 | 50.319 | 1.00 | 0.00 | AAAB |
| ATOM | 3251 | C | HIS | E | 697 | 32.756 | 98.760 | 49.033 | 1.00 | 0.00 | AAAB |
| ATOM | 3252 | OT | HIS | E | 697 | 33.348 | 99.476 | 48.229 | 1.00 | 0.00 | AAAB |
| ATOM | 3253 | O | HIS | E | 697 | 32.849 | 97.400 | 48.998 | 1.00 | 0.00 | AAAB |
| END | | | | | | | | | | | |

### APPENDIX V : ATOMIC COORDINATES FOR THE MODEL OF T INSULIN MIMETIC PEPTIDE, S519C16, BOUND TO IR ECTODOMA

The structural coordinates in Appendixes I and III were used to model the insulin mimetic peptide, S519C16, in the low affinity insulin binding site of IR. This m is oriented relative to atomic coordinates found in Appendixes I and IV, and may used in conjunction with atomic coordinates of Appendixes I to IV and VI to des compounds which bind to IR and/or IGF-1R.

| | | | | | | | | | | | |
|---|---|---|---|---|---|---|---|---|---|---|---|
| ATOM | 1 | N | PRO | E | 4 | 49.870 | 109.907 | 14.773 | 1.00 | 0.00 | AAAA |
| ATOM | 4 | CA | PRO | E | 4 | 48.806 | 110.022 | 15.790 | 1.00 | 0.00 | AAAA |
| ATOM | 6 | CB | PRO | E | 4 | 47.761 | 108.948 | 15.497 | 1.00 | 0.00 | AAAA |
| ATOM | 9 | CG | PRO | E | 4 | 47.825 | 108.849 | 13.974 | 1.00 | 0.00 | AAAA |
| ATOM | 12 | CD | PRO | E | 4 | 49.299 | 109.135 | 13.661 | 1.00 | 0.00 | AAAA |
| ATOM | 15 | C | PRO | E | 4 | 49.329 | 109.809 | 17.194 | 1.00 | 0.00 | AAAA |
| ATOM | 16 | O | PRO | E | 4 | 50.547 | 109.602 | 17.268 | 1.00 | 0.00 | AAAA |
| ATOM | 17 | N | GLY | E | 5 | 48.561 | 109.700 | 18.276 | 1.00 | 0.00 | AAAA |
| ATOM | 19 | CA | GLY | E | 5 | 48.913 | 109.260 | 19.610 | 1.00 | 0.00 | AAAA |
| ATOM | 22 | C | GLY | E | 5 | 48.149 | 108.050 | 20.138 | 1.00 | 0.00 | AAAA |
| ATOM | 23 | O | GLY | E | 5 | 47.356 | 107.406 | 19.452 | 1.00 | 0.00 | AAAA |
| ATOM | 24 | N | GLU | E | 6 | 48.331 | 107.669 | 21.404 | 1.00 | 0.00 | AAAA |
| ATOM | 26 | CA | GLU | E | 6 | 47.807 | 106.465 | 22.008 | 1.00 | 0.00 | AAAA |
| ATOM | 28 | CB | GLU | E | 6 | 48.711 | 106.138 | 23.196 | 1.00 | 0.00 | AAAA |
| ATOM | 31 | CG | GLU | E | 6 | 48.420 | 104.861 | 23.994 | 1.00 | 0.00 | AAAA |
| ATOM | 34 | CD | GLU | E | 6 | 48.678 | 103.565 | 23.229 | 1.00 | 0.00 | AAAA |
| ATOM | 35 | OE1 | GLU | E | 6 | 48.156 | 102.498 | 23.615 | 1.00 | 0.00 | AAAA |
| ATOM | 36 | OE2 | GLU | E | 6 | 49.290 | 103.562 | 22.130 | 1.00 | 0.00 | AAAA |
| ATOM | 37 | C | GLU | E | 6 | 46.475 | 106.832 | 22.658 | 1.00 | 0.00 | AAAA |
| ATOM | 38 | O | GLU | E | 6 | 46.299 | 107.961 | 23.105 | 1.00 | 0.00 | AAAA |
| ATOM | 39 | N | VAL | E | 7 | 45.570 | 105.856 | 22.787 | 1.00 | 0.00 | AAAA |
| ATOM | 41 | CA | VAL | E | 7 | 44.297 | 105.971 | 23.471 | 1.00 | 0.00 | AAAA |
| ATOM | 43 | CB | VAL | E | 7 | 43.190 | 106.374 | 22.501 | 1.00 | 0.00 | AAAA |
| ATOM | 45 | CG1 | VAL | E | 7 | 43.385 | 107.771 | 21.914 | 1.00 | 0.00 | AAAA |
| ATOM | 49 | CG2 | VAL | E | 7 | 42.877 | 105.350 | 21.417 | 1.00 | 0.00 | AAAA |
| ATOM | 53 | C | VAL | E | 7 | 43.999 | 104.750 | 24.331 | 1.00 | 0.00 | AAAA |
| ATOM | 54 | O | VAL | E | 7 | 44.568 | 103.683 | 24.094 | 1.00 | 0.00 | AAAA |
| ATOM | 55 | N | CYS | E | 8 | 43.128 | 104.866 | 25.334 | 1.00 | 0.00 | AAAA |
| ATOM | 57 | CA | CYS | E | 8 | 43.010 | 104.027 | 26.504 | 1.00 | 0.00 | AAAA |
| ATOM | 59 | CB | CYS | E | 8 | 44.071 | 104.391 | 27.540 | 1.00 | 0.00 | AAAA |
| ATOM | 62 | SG | CYS | E | 8 | 44.033 | 105.964 | 28.421 | 1.00 | 0.00 | AAAA |
| ATOM | 63 | C | CYS | E | 8 | 41.589 | 104.057 | 27.046 | 1.00 | 0.00 | AAAA |
| ATOM | 64 | O | CYS | E | 8 | 41.014 | 105.132 | 26.917 | 1.00 | 0.00 | AAAA |
| ATOM | 65 | N | PRO | E | 9 | 40.933 | 103.005 | 27.544 | 1.00 | 0.00 | AAAA |
| ATOM | 66 | CA | PRO | E | 9 | 39.495 | 102.995 | 27.733 | 1.00 | 0.00 | AAAA |
| ATOM | 68 | CB | PRO | E | 9 | 39.180 | 101.498 | 27.736 | 1.00 | 0.00 | AAAA |
| ATOM | 71 | CG | PRO | E | 9 | 40.434 | 100.875 | 28.358 | 1.00 | 0.00 | AAAA |
| ATOM | 74 | CD | PRO | E | 9 | 41.587 | 101.749 | 27.875 | 1.00 | 0.00 | AAAA |
| ATOM | 77 | C | PRO | E | 9 | 38.995 | 103.681 | 29.008 | 1.00 | 0.00 | AAAA |
| ATOM | 78 | O | PRO | E | 9 | 39.744 | 104.365 | 29.707 | 1.00 | 0.00 | AAAA |
| ATOM | 79 | N | GLY | E | 10 | 37.699 | 103.554 | 29.302 | 1.00 | 0.00 | AAAA |
| ATOM | 81 | CA | GLY | E | 10 | 37.044 | 104.101 | 30.471 | 1.00 | 0.00 | AAAA |
| ATOM | 84 | C | GLY | E | 10 | 37.169 | 103.032 | 31.544 | 1.00 | 0.00 | AAAA |
| ATOM | 85 | O | GLY | E | 10 | 36.692 | 101.917 | 31.321 | 1.00 | 0.00 | AAAA |
| ATOM | 86 | N | MET | E | 11 | 37.704 | 103.460 | 32.683 | 1.00 | 0.00 | AAAA |
| ATOM | 88 | CA | MET | E | 11 | 38.254 | 102.668 | 33.762 | 1.00 | 0.00 | AAAA |
| ATOM | 90 | CB | MET | E | 11 | 39.643 | 102.261 | 33.289 | 1.00 | 0.00 | AAAA |
| ATOM | 93 | CG | MET | E | 11 | 40.620 | 103.431 | 33.384 | 1.00 | 0.00 | AAAA |
| ATOM | 96 | SD | MET | E | 11 | 42.321 | 102.930 | 33.019 | 1.00 | 0.00 | AAAA |
| ATOM | 97 | CE | MET | E | 11 | 42.302 | 102.853 | 31.212 | 1.00 | 0.00 | AAAA |
| ATOM | 101 | C | MET | E | 11 | 38.096 | 103.335 | 35.123 | 1.00 | 0.00 | AAAA |
| ATOM | 102 | O | MET | E | 11 | 37.733 | 104.497 | 35.288 | 1.00 | 0.00 | AAAA |
| ATOM | 103 | N | ASP | E | 12 | 38.372 | 102.611 | 36.211 | 1.00 | 0.00 | AAAA |
| ATOM | 105 | CA | ASP | E | 12 | 38.492 | 103.164 | 37.542 | 1.00 | 0.00 | AAAA |
| ATOM | 107 | CB | ASP | E | 12 | 37.424 | 102.825 | 38.585 | 1.00 | 0.00 | AAAA |
| ATOM | 110 | CG | ASP | E | 12 | 37.605 | 101.548 | 39.391 | 1.00 | 0.00 | AAAA |
| ATOM | 111 | OD1 | ASP | E | 12 | 38.022 | 100.535 | 38.787 | 1.00 | 0.00 | AAAA |
| ATOM | 112 | OD2 | ASP | E | 12 | 37.110 | 101.570 | 40.544 | 1.00 | 0.00 | AAAA |
| ATOM | 113 | C | ASP | E | 12 | 39.890 | 102.857 | 38.056 | 1.00 | 0.00 | AAAA |
| ATOM | 114 | O | ASP | E | 12 | 40.594 | 101.926 | 37.666 | 1.00 | 0.00 | AAAA |
| ATOM | 115 | N | ILE | E | 13 | 40.365 | 103.799 | 38.876 | 1.00 | 0.00 | AAAA |
| ATOM | 117 | CA | ILE | E | 13 | 41.710 | 103.947 | 39.392 | 1.00 | 0.00 | AAAA |
| ATOM | 119 | CB | ILE | E | 13 | 42.323 | 105.038 | 38.510 | 1.00 | 0.00 | AAAA |
| ATOM | 121 | CG1 | ILE | E | 13 | 42.689 | 104.701 | 37.071 | 1.00 | 0.00 | AAAA |
| ATOM | 124 | CG2 | ILE | E | 13 | 43.483 | 105.714 | 39.234 | 1.00 | 0.00 | AAAA |
| ATOM | 128 | CD1 | ILE | E | 13 | 43.796 | 103.638 | 37.018 | 1.00 | 0.00 | AAAA |
| ATOM | 132 | C | ILE | E | 13 | 41.594 | 104.006 | 40.910 | 1.00 | 0.00 | AAAA |
| ATOM | 133 | O | ILE | E | 13 | 41.289 | 105.053 | 41.475 | 1.00 | 0.00 | AAAA |
| ATOM | 134 | N | ARG | E | 14 | 41.988 | 102.918 | 41.577 | 1.00 | 0.00 | AAAA |
| ATOM | 136 | CA | ARG | E | 14 | 41.842 | 102.751 | 43.010 | 1.00 | 0.00 | AAAA |
| ATOM | 138 | CB | ARG | E | 14 | 41.026 | 101.480 | 43.231 | 1.00 | 0.00 | AAAA |
| ATOM | 141 | CG | ARG | E | 14 | 39.571 | 101.384 | 42.752 | 1.00 | 0.00 | AAAA |
| ATOM | 144 | CD | ARG | E | 14 | 38.794 | 100.186 | 43.303 | 1.00 | 0.00 | AAAA |
| ATOM | 147 | NE | ARG | E | 14 | 37.433 | 100.168 | 42.766 | 1.00 | 0.00 | AAAA |
| ATOM | 149 | CZ | ARG | E | 14 | 36.424 | 99.530 | 43.378 | 1.00 | 0.00 | AAAA |
| ATOM | 150 | NH1 | ARG | E | 14 | 36.417 | 98.858 | 44.528 | 1.00 | 0.00 | AAAA |
| ATOM | 153 | NH2 | ARG | E | 14 | 35.241 | 99.617 | 42.753 | 1.00 | 0.00 | AAAA |
| ATOM | 156 | C | ARG | E | 14 | 43.206 | 102.603 | 43.682 | 1.00 | 0.00 | AAAA |
| ATOM | 157 | O | ARG | E | 14 | 44.159 | 102.045 | 43.128 | 1.00 | 0.00 | AAAA |
| ATOM | 158 | N | ASN | E | 15 | 43.332 | 103.019 | 44.939 | 1.00 | 0.00 | AAAA |
| ATOM | 160 | CA | ASN | E | 15 | 44.424 | 102.910 | 45.874 | 1.00 | 0.00 | AAAA |
| ATOM | 162 | CB | ASN | E | 15 | 44.772 | 101.422 | 45.984 | 1.00 | 0.00 | AAAA |
| ATOM | 165 | CG | ASN | E | 15 | 45.653 | 101.126 | 47.193 | 1.00 | 0.00 | AAAA |
| ATOM | 166 | OD1 | ASN | E | 15 | 46.869 | 100.994 | 47.118 | 1.00 | 0.00 | AAAA |
| ATOM | 167 | ND2 | ASN | E | 15 | 44.964 | 100.875 | 48.310 | 1.00 | 0.00 | AAAA |
| ATOM | 170 | C | ASN | E | 15 | 45.575 | 103.833 | 45.511 | 1.00 | 0.00 | AAAA |
| ATOM | 171 | O | ASN | E | 15 | 46.132 | 104.560 | 46.328 | 1.00 | 0.00 | AAAA |
| ATOM | 172 | N | ASN | E | 16 | 46.007 | 103.906 | 44.243 | 1.00 | 0.00 | AAAA |
| ATOM | 174 | CA | ASN | E | 16 | 47.046 | 104.782 | 43.743 | 1.00 | 0.00 | AAAA |
| ATOM | 176 | CB | ASN | E | 16 | 48.451 | 104.271 | 44.050 | 1.00 | 0.00 | AAAA |
| ATOM | 179 | CG | ASN | E | 16 | 49.537 | 105.335 | 44.075 | 1.00 | 0.00 | AAAA |
| ATOM | 180 | OD1 | ASN | E | 16 | 49.819 | 105.806 | 42.972 | 1.00 | 0.00 | AAAA |
| ATOM | 181 | ND2 | ASN | E | 16 | 50.197 | 105.622 | 45.203 | 1.00 | 0.00 | AAAA |
| ATOM | 184 | C | ASN | E | 16 | 46.781 | 104.985 | 42.264 | 1.00 | 0.00 | AAAA |
| ATOM | 185 | O | ASN | E | 16 | 45.774 | 104.461 | 41.786 | 1.00 | 0.00 | AAAA |
| ATOM | 186 | N | LEU | E | 17 | 47.534 | 105.767 | 41.485 | 1.00 | 0.00 | AAAA |
| ATOM | 188 | CA | LEU | E | 17 | 47.412 | 106.042 | 40.075 | 1.00 | 0.00 | AAAA |
| ATOM | 190 | CB | LEU | E | 17 | 47.753 | 107.510 | 39.789 | 1.00 | 0.00 | AAAA |
| ATOM | 193 | CG | LEU | E | 17 | 47.018 | 108.598 | 40.570 | 1.00 | 0.00 | AAAA |
| ATOM | 195 | CD1 | LEU | E | 17 | 47.231 | 109.965 | 39.915 | 1.00 | 0.00 | AAAA |
| ATOM | 199 | CD2 | LEU | E | 17 | 45.499 | 108.441 | 40.661 | 1.00 | 0.00 | AAAA |
| ATOM | 203 | C | LEU | E | 17 | 48.258 | 105.135 | 39.189 | 1.00 | 0.00 | PAPS |
| ATOM | 204 | O | LEU | E | 17 | 48.512 | 105.425 | 38.031 | 1.00 | 0.00 | AAAA |
| ATOM | 205 | N | THR | E | 18 | 48.776 | 104.052 | 39.780 | 1.00 | 0.00 | AAAA |
| ATOM | 207 | CA | THR | E | 18 | 49.828 | 103.286 | 39.138 | 1.00 | 0.00 | AAAA |
| ATOM | 209 | CB | THR | E | 18 | 50.429 | 102.262 | 40.099 | 1.00 | 0.00 | AAAA |
| ATOM | 211 | OG1 | THR | E | 18 | 49.484 | 101.493 | 40.806 | 1.00 | 0.00 | AAAA |
| ATOM | 213 | CG2 | THR | E | 18 | 51.073 | 102.933 | 41.318 | 1.00 | 0.00 | AAAA |
| ATOM | 217 | C | THR | E | 18 | 49.454 | 102.575 | 37.844 | 1.00 | 0.00 | AAAA |
| ATOM | 218 | O | THR | E | 18 | 50.298 | 102.349 | 36.982 | 1.00 | 0.00 | AAAA |
| ATOM | 219 | N | ARG | E | 19 | 48.152 | 102.256 | 37.731 | 1.00 | 0.00 | AAAA |
| ATOM | 221 | CA | ARG | E | 19 | 47.554 | 101.667 | 36.553 | 1.00 | 0.00 | AAAA |
| ATOM | 223 | CB | ARG | E | 19 | 46.273 | 100.940 | 36.950 | 1.00 | 0.00 | AAAA |
| ATOM | 226 | CG | ARG | E | 19 | 45.503 | 100.154 | 35.893 | 1.00 | 0.00 | AAAA |
| ATOM | 229 | CD | ARG | E | 19 | 44.328 | 99.410 | 36.534 | 1.00 | 0.00 | AAAA |
| ATOM | 232 | NE | ARG | E | 19 | 43.528 | 98.762 | 35.493 | 1.00 | 0.00 | AAAA |
| ATOM | 234 | CZ | ARG | E | 19 | 42.200 | 98.911 | 35.428 | 1.00 | 0.00 | AAAA |
| ATOM | 235 | NH1 | ARG | E | 19 | 41.495 | 99.937 | 35.931 | 1.00 | 0.00 | AAAA |
| ATOM | 238 | NH2 | ARG | E | 19 | 41.536 | 97.897 | 34.870 | 1.00 | 0.00 | AAAA |
| ATOM | 241 | C | ARG | E | 19 | 47.372 | 102.683 | 35.438 | 1.00 | 0.00 | AAAA |
| ATOM | 242 | O | ARG | E | 19 | 47.202 | 102.269 | 34.287 | 1.00 | 0.00 | AAAA |
| ATOM | 243 | N | LEU | E | 20 | 47.318 | 103.986 | 35.735 | 1.00 | 0.00 | AAAA |
| ATOM | 245 | CA | LEU | E | 20 | 47.232 | 105.070 | 34.785 | 1.00 | 0.00 | AAAA |
| ATOM | 247 | CB | LEU | E | 20 | 46.421 | 106.210 | 35.405 | 1.00 | 0.00 | AAAA |
| ATOM | 250 | CG | LEU | E | 20 | 45.669 | 107.062 | 34.380 | 1.00 | 0.00 | AAAA |
| ATOM | 252 | CD1 | LEU | E | 20 | 44.378 | 106.358 | 33.957 | 1.00 | 0.00 | AAAA |
| ATOM | 256 | CD2 | LEU | E | 20 | 45.302 | 108.463 | 34.854 | 1.00 | 0.00 | AAAA |
| ATOM | 260 | C | LEU | E | 20 | 48.638 | 105.577 | 34.502 | 1.00 | 0.00 | AAAA |
| ATOM | 261 | O | LEU | E | 20 | 48.876 | 105.957 | 33.355 | 1.00 | 0.00 | AAAA |
| ATOM | 262 | N | HIS | E | 21 | 49.600 | 105.488 | 35.422 | 1.00 | 0.00 | AAAA |
| ATOM | 264 | CA | HIS | E | 21 | 51.041 | 105.601 | 35.319 | 1.00 | 0.00 | AAAA |
| ATOM | 266 | CB | HIS | E | 21 | 51.599 | 105.731 | 36.734 | 1.00 | 0.00 | AAAA |
| ATOM | 269 | CG | HIS | E | 21 | 52.881 | 106.508 | 36.863 | 1.00 | 0.00 | AAAA |
| ATOM | 270 | ND1 | HIS | E | 21 | 54.119 | 105.870 | 36.900 | 1.00 | 0.00 | AAAA |
| ATOM | 271 | CD2 | HIS | E | 21 | 52.996 | 107.879 | 36.825 | 1.00 | 0.00 | AAAA |
| ATOM | 273 | CE1 | HIS | E | 21 | 54.925 | 106.925 | 37.088 | 1.00 | 0.00 | AAAA |
| ATOM | 275 | NE2 | HIS | E | 21 | 54.335 | 108.131 | 37.013 | 1.00 | 0.00 | AAAA |
| ATOM | 277 | C | HIS | E | 21 | 51.782 | 104.607 | 34.434 | 1.00 | 0.00 | AAAA |
| ATOM | 278 | O | HIS | E | 21 | 52.852 | 104.969 | 33.939 | 1.00 | 0.00 | AAAA |
| ATOM | 279 | N | GLU | E | 22 | 51.241 | 103.399 | 34.269 | 1.00 | 0.00 | AAAA |
| ATOM | 281 | CA | GLU | E | 22 | 51.557 | 102.394 | 33.268 | 1.00 | 0.00 | AAAA |
| ATOM | 283 | CB | GLU | E | 22 | 51.126 | 101.010 | 33.754 | 1.00 | 0.00 | AAAA |
| ATOM | 286 | CG | GLU | E | 22 | 51.891 | 99.814 | 33.184 | 1.00 | 0.00 | AAAA |
| ATOM | 289 | CD | GLU | E | 22 | 53.405 | 99.774 | 33.404 | 1.00 | 0.00 | AAAA |
| ATOM | 290 | OE1 | GLU | E | 22 | 54.083 | 99.000 | 32.707 | 1.00 | 0.00 | AAAA |
| ATOM | 291 | OE2 | GLU | E | 22 | 53.814 | 100.633 | 34.210 | 1.00 | 0.00 | AAAA |
| ATOM | 292 | C | GLU | E | 22 | 51.002 | 102.649 | 31.880 | 1.00 | 0.00 | AAAA |
| ATOM | 293 | O | GLU | E | 22 | 51.445 | 102.007 | 30.926 | 1.00 | 0.00 | AAAA |
| ATOM | 294 | N | LEU | E | 23 | 50.155 | 103.675 | 31.729 | 1.00 | 0.00 | AAAA |
| ATOM | 296 | CA | LEU | E | 23 | 49.512 | 104.204 | 30.543 | 1.00 | 0.00 | AAAA |
| ATOM | 298 | CB | LEU | E | 23 | 48.012 | 103.899 | 30.500 | 1.00 | 0.00 | AAAA |
| ATOM | 301 | CG | LEU | E | 23 | 47.555 | 102.454 | 30.625 | 1.00 | 0.00 | AAAA |
| ATOM | 303 | CD1 | LEU | E | 23 | 46.091 | 102.338 | 31.054 | 1.00 | 0.00 | AAAA |
| ATOM | 307 | CD2 | LEU | E | 23 | 47.767 | 101.712 | 29.301 | 1.00 | 0.00 | AAAA |
| ATOM | 311 | C | LEU | E | 23 | 49.803 | 105.689 | 30.382 | 1.00 | 0.00 | AAAA |
| ATOM | 312 | O | LEU | E | 23 | 48.877 | 106.400 | 29.982 | 1.00 | 0.00 | AAAA |
| ATOM | 313 | N | GLU | E | 24 | 50.942 | 106.249 | 30.795 | 1.00 | 0.00 | AAAA |
| ATOM | 315 | CA | GLU | E | 24 | 51.161 | 107.667 | 30.924 | 1.00 | 0.00 | AAAA |
| ATOM | 317 | CB | GLU | E | 24 | 52.499 | 107.896 | 31.630 | 1.00 | 0.00 | AAAA |
| ATOM | 320 | CG | GLU | E | 24 | 52.805 | 109.314 | 32.119 | 1.00 | 0.00 | AAAA |
| ATOM | 323 | CD | GLU | E | 24 | 53.512 | 110.306 | 31.204 | 1.00 | 0.00 | AAAA |
| ATOM | 324 | OE1 | GLU | E | 24 | 54.159 | 109.799 | 30.258 | 1.00 | 0.00 | AAAA |
| ATOM | 325 | OE2 | GLU | E | 24 | 53.419 | 111.526 | 31.431 | 1.00 | 0.00 | AAAA |
| ATOM | 326 | C | GLU | E | 24 | 51.097 | 108.390 | 29.584 | 1.00 | 0.00 | AAAA |
| ATOM | 327 | O | GLU | E | 24 | 50.711 | 109.554 | 29.505 | .1.00 | 0.00 | AAAA |
| ATOM | 328 | N | ASN | E | 25 | 51.490 | 107.723 | 28.501 | 1.00 | 0.00 | AAAA |
| ATOM | 330 | CA | ASN | E | 25 | 51.478 | 108.191 | 27.124 | 1.00 | 0.00 | AAAA |
| ATOM | 332 | CB | ASN | E | 25 | 52.327 | 107.315 | 26.204 | 1.00 | 0.00 | AAAA |
| ATOM | 335 | CG | ASN | E | 25 | 52.678 | 108.080 | 24.943 | 1.00 | 0.00 | AAAA |
| ATOM | 336 | OD1 | ASN | E | 25 | 52.978 | 109.275 | 24.946 | 1.00 | 0.00 | AAAA |
| ATOM | 337 | ND2 | ASN | E | 25 | 52.798 | 107.495 | 23.746 | 1.00 | 0.00 | AAAA |
| ATOM | 340 | C | ASN | E | 25 | 50.084 | 108.371 | 26.541 | 1.00 | 0.00 | AAAA |
| ATOM | 341 | O | ASN | E | 25 | 49.917 | 108.934 | 25.463 | 1.00 | 0.00 | AAAA |
| ATOM | 342 | N | CYS | E | 26 | 49.028 | 108.014 | 27.287 | 1.00 | 0.00 | AAAA |
| ATOM | 344 | CA | CYS | E | 26 | 47.686 | 108.047 | 26.732 | 1.00 | 0.00 | AAAA |
| ATOM | 346 | CB | CYS | E | 26 | 46.766 | 107.376 | 27.746 | 1.00 | 0.00 | AAAA |
| ATOM | 349 | SG | CYS | E | 26 | 45.054 | 107.210 | 27.180 | 1.00 | 0.00 | AAAA |
| ATOM | 350 | C | CYS | E | 26 | 47.228 | 109.476 | 26.457 | 1.00 | 0.00 | AAAA |
| ATOM | 351 | O | CYS | E | 26 | 47.476 | 110.305 | 27.326 | 1.00 | 0.00 | AAAA |
| ATOM | 352 | N | SER | E | 27 | 46.459 | 109.781 | 25.404 | 1.00 | 0.00 | AAAA |
| ATOM | 354 | CA | SER | E | 27 | 46.102 | 111.110 | 24.956 | 1.00 | 0.00 | AAAA |
| ATOM | 356 | CB | SER | E | 27 | 46.615 | 111.378 | 23.546 | 1.00 | 0.00 | AAAA |
| ATOM | 359 | OG | SER | E | 27 | 45.884 | 110.753 | 22.508 | 1.00 | 0.00 | AAAA |
| ATOM | 361 | C | SER | E | 27 | 44.630 | 111.430 | 25.155 | 1.00 | 0.00 | AAAA. |
| ATOM | 362 | O | SER | E | 27 | 44.285 | 112.578 | 25.416 | 1.00 | 0.00 | AAAA |
| ATOM | 363 | N | VAL | E | 28 | 43.861 | 110.338 | 25.231 | 1.00 | 0.00 | AAAA |
| ATOM | 365 | CA | VAL | E | 28 | 42.415 | 110.322 | 25.365 | 1.00 | 0.00 | AAAA |
| ATOM | 367 | CB | VAL | E | 28 | 41.625 | 110.419 | 24.062 | 1.00 | 0.00 | AAAA |
| ATOM | 369 | CG1 | VAL | E | 28 | 40.110 | 110.342 | 24.285 | 1.00 | 0.00 | AAAA |
| ATOM | 373 | CG2 | VAL | E | 28 | 41.933 | 111.675 | 23.236 | 1.00 | 0.00 | AAAA |
| ATOM | 377 | C | VAL | E | 28 | 42.076 | 109.122 | 26.233 | 1.00 | 0.00 | AAAA |
| ATOM | 378 | O | VAL | E | 28 | 42.290 | 107.967 | 25.866 | 1.00 | 0.00 | AAAA |
| ATOM | 379 | N | ILE | E | 29 | 41.433 | 109.317 | 27.380 | 1.00 | 0.00 | AAAA |
| ATOM | 381 | CA | ILE | E | 29 | 40.766 | 108.287 | 28.161 | 1.00 | 0.00 | AAAA |
| ATOM | 383 | CB | ILE | E | 29 | 40.688 | 108.639 | 29.643 | 1.00 | 0.00 | AAAA |
| ATOM | 385 | CG1 | ILE | E | 29 | 42.114 | 108.499 | 30.185 | 1.00 | 0.00 | AAAA |
| ATOM | 388 | CG2 | ILE | E | 29 | 39.726 | 107.782 | 30.462 | 1.00 | 0.00 | AAAA |
| ATOM | 392 | CD1 | ILE | E | 29 | 42.579 | 109.253 | 31.432 | 1.00 | 0.00 | AAAA |
| ATOM | 396 | C | ILE | E | 29 | 39.369 | 108.163 | 27.561 | 1.00 | 0.00 | AAAA |
| ATOM | 397 | O | ILE | E | 29 | 38.535 | 109.061 | 27.717 | 1.00 | 0.00 | AAAA |
| ATOM | 398 | N | GLU | E | 30 | 39.130 | 107.178 | 26.689 | 1.00 | 0.00 | AAAA |
| ATOM | 400 | CA | GLU | E | 30 | 38.155 | 107.116 | 25.621 | 1.00 | 0.00 | AAAA |
| ATOM | 402 | CB | GLU | E | 30 | 38.534 | 105.885 | 24.790 | 1.00 | 0.00 | AAAA |
| ATOM | 405 | CG | GLU | E | 30 | 39.630 | 106.201 | 23.774 | 1.00 | 0.00 | AAAA |
| ATOM | 408 | CD | GLU | E | 30 | 39.084 | 106.609 | 22.414 | 1.00 | 0.00 | AAAA |
| ATOM | 409 | OE1 | GLU | E | 30 | 38.386 | 107.643 | 22.379 | 1.00 | 0.00 | AAAA |
| ATOM | 410 | OE2 | GLU | E | 30 | 39.227 | 105.822 | 21.455 | 1.00 | 0.00 | AAAA |
| ATOM | 411 | C | GLU | E | 30 | 36.761 | 106.855 | 26.165 | 1.00 | 0.00 | AAAA |
| ATOM | 412 | O | GLU | E | 30 | 35.745 | 106.739 | 25.478 | 1.00 | 0.00 | AAAA |
| ATOM | 413 | N | GLY | E | 31 | 36.586 | 106.798 | 27.486 | 1.00 | 0.00 | AAAA |
| ATOM | 415 | CA | GLY | E | 31 | 35.359 | 106.795 | 28.244 | 1.00 | 0.00 | AAAA |
| ATOM | 418 | C | GLY | E | 31 | 35.304 | 107.732 | 29.445 | 1.00 | 0.00 | AAAA |
| ATOM | 419 | O | GLY | E | 31 | 35.479 | 108.947 | 29.329 | 1.00 | 0.00 | AAAA |
| ATOM | 420 | N | HIS | E | 32 | 35.180 | 107.122 | 30.626 | 1.00 | 0.00 | AAAA |
| ATOM | 422 | CA | HIS | E | 32 | 35.150 | 107.748 | 31.926 | 1.00 | 0.00 | AAAA |
| ATOM | 424 | CB | HIS | E | 32 | 34.028 | 107.093 | 32.740 | 1.00 | 0.00 | AAAA |
| ATOM | 427 | CG | HIS | E | 32 | 34.069 | 105.650 | 33.139 | 1.00 | 0.00 | AAAA |
| ATOM | 428 | ND1 | HIS | E | 32 | 34.065 | 104.567 | 32.258 | 1.00 | 0.00 | AAAA |
| ATOM | 429 | CD2 | HIS | E | 32 | 33.975 | 105.120 | 34.396 | 1.00 | 0.00 | AAAA |
| ATOM | 431 | CE1 | HIS | E | 32 | 33.848 | 103.459 | 32.965 | 1.00 | 0.00 | AAAA |
| ATOM | 433 | NE2 | HIS | E | 32 | 33.819 | 103.753 | 34.279 | 1.00 | 0.00 | AAAA |
| ATOM | 435 | C | HIS | E | 32 | 36.473 | 107.449 | 32.618 | 1.00 | 0.00 | AAAA |
| ATOM | 436 | O | .HIS | E | 32 | 37.329 | 106.634 | 32.260 | 1.00 | 0.00 | AAAA |
| ATOM | 437 | N | LEU | E | 33 | 36.644 | 108.172 | 33.726 | 1.00 | 0.00 | AAAA |
| ATOM | 439 | CA | LEU | E | 33 | 37.617 | 107.920 | 34.776 | 1.00 | 0.00 | AAAA |
| ATOM | 441 | CB | LEU | E | 33 | 38.891 | 108.728 | 34.569 | 1.00 | 0.00 | AAAA |
| ATOM | 444 | CG | LEU | E | 33 | 40.003 | 108.783 | 35.611 | 1.00 | 0.00 | AAAA |
| ATOM | 446 | CD1 | LEU | E | 33 | 40.533 | 107.386 | 35.938 | 1.00 | 0.00 | AAAA |
| ATOM | 450 | CD2 | LEU | E | 33 | 41.145 | 109.757 | 35.324 | 1.00 | 0.00 | AAAA |
| ATOM | 454 | C | LEU | E | 33 | 36.984 | 108.125 | 36.153 | 1.00 | 0.00 | AAAA |
| ATOM | 455 | O | LEU | E | 33 | 36.389 | 109.164 | 36.430 | 1.00 | 0.00 | AAAA |
| ATOM | 456 | N | GLN | E | 34 | 37.193 | 107.164 | 37.057 | 1.00 | 0.00 | AAAA |
| ATOM | 458 | CA | GLN | E | 34 | 36.872 | 107.234 | 38.463 | 1.00 | 0.00 | AAAA |
| ATOM | 460 | CB | GLN | E | 34 | 35.854 | 106.145 | 38.831 | 1.00 | 0.00 | AAAA |
| ATOM | 463 | CG | GLN | E | 34 | 34.449 | 106.463 | 38.312 | 1.00 | 0.00 | AAAA |
| ATOM | 466 | CD | GLN | E | 34 | 33.473 | 105.297 | 38.354 | 1.00 | 0.00 | AAAA |
| ATOM | 467 | OE1 | GLN | E | 34 | 32.369 | 105.241 | 37.838 | 1.00 | 0.00 | AAAA |
| ATOM | 468 | NE2 | GLN | E | 34 | 33.884 | 104.219 | 39.035 | 1.00 | 0.00 | AAAA |
| ATOM | 471 | C | GLN | E | 34 | 38.128 | 107.009 | 39.289 | 1.00 | 0.00 | AAAA |
| ATOM | 472 | O | GLN | E | 34 | 38.615 | 105.882 | 39.387 | 1.00 | 0.00 | AAAA |
| ATOM | 473 | N | ILE | E | 35 | 38.684 | 108.066 | 39.881 | 1.00 | 0.00 | AAAA |
| ATOM | 475 | CA | ILE | E | 35 | 39.662 | 107.918 | 40.942 | 1.00 | 0.00 | AAAA |
| ATOM | 477 | CB | ILE | E | 35 | 40.577 | 109.145 | 40.978 | 1.00 | 0.00 | AAAA |
| ATOM | 479 | CG1 | ILE | E | 35 | 41.426 | 109.422 | 39.737 | 1.00 | 0.00 | AAAA |
| ATOM | 482 | CG2 | ILE | E | 35 | 41.553 | 109.155 | 42.150 | 1.00 | 0.00 | AAAA |
| ATOM | 486 | CD1 | ILE | E | 35 | 41.720 | 110.915 | 39.587 | 1.00 | 0.00 | AAAA |
| ATOM | 490 | C | ILE | E | 35 | 38.906 | 107.850 | 42.258 | 1.00 | 0.00 | AAAA |
| ATOM | 491 | O | ILE | E | 35 | 38.042 | 108.671 | 42.512 | 1.00 | 0.00 | AAAA |
| ATOM | 492 | N | LEU | E | 36 | 39.338 | 106.886 | 43.076 | 1.00 | 0.00 | AAAA |
| ATOM | 494 | CA | LEU | E | 36 | 38.783 | 106.799 | 44.417 | 1.00 | 0.00 | AAAA |
| ATOM | 496 | CB | LEU | E | 36 | 37.456 | 106.049 | 44.349 | 1.00 | 0.00 | AAAA |
| ATOM | 499 | CG | LEU | E | 36 | 37.480 | 104.639 | 43.773 | 1.00 | 0.00 | AAAA |
| ATOM | 501 | CD1 | LEU | E | 36 | 36.853 | 103.669 | 44.781 | 1.00 | 0.00 | AAAA |
| ATOM | 505 | CD2 | LEU | E | 36 | 36.861 | 104.543 | 42.382 | 1.00 | 0.00 | AAAA |
| ATOM | 509 | C | LEU | E | 36 | 39.731 | 106.198 | 45.448 | 1.00 | 0.00 | AAAA |
| ATOM | 510 | O | LEU | E | 36 | 40.570 | 105.350 | 45.159 | 1.00 | 0.00 | AAAA |
| ATOM | 511 | N | LEU | E | 37 | 39.556 | 106.651 | 46.693 | 1.00 | 0.00 | AAAA |
| ATOM | 513 | CA | LEU | E | 37 | 39.946 | 105.942 | 47.886 | 1.00 | 0.00 | AAAA |
| ATOM | 515 | CB | LEU | E | 37 | 39.394 | 104.511 | 47.933 | 1.00 | 0.00 | AAAA |
| ATOM | 518 | CG | LEU | E | 37 | 39.454 | 104.215 | 49.101 | 1.00 | 0.00 | AAAA |
| ATOM | 520 | CD1 | LEU | E | 37 | 37.110 | 104.909 | 48.900 | 1.00 | 0.00 | AAAA |
| ATOM | 524 | CD2 | LEU | E | 37 | 38.253 | 102.704 | 49.230 | 1.00 | 0.00 | AAAA |
| ATOM | 528 | C | LEU | E | 37 | 41.421 | 106.104 | 48.225 | 1.00 | 0.00 | AAAA |
| ATOM | 529 | O | LEU | E | 37 | 42.064 | 105.149 | 48.644 | 1.00 | 0.00 | AAAA |
| ATOM | 530 | N | MET | E | 38 | 42.007 | 107.311 | 48.134 | 1.00 | 0.00 | AAAA |
| ATOM | 532 | CA | MET | E | 38 | 43.422 | 107.586 | 48.221 | 1.00 | 0.00 | AAAA |
| ATOM | 534 | CB | MET | E | 38 | 43.925 | 108.250 | 46.937 | 1.00 | 0.00 | AAAA |
| ATOM | 537 | CG | MET | E | 38 | 43.749 | 107.566 | 45.578 | 1.00 | 0.00 | AAAA |
| ATOM | 540 | SD | MET | E | 38 | 44.413 | 108.352 | 44.096 | 1.00 | 0.00 | AAAA |
| ATOM | 541 | CE | MET | E | 38 | 46.115 | 108.539 | 44.696 | 1.00 | 0.00 | AAAA |
| ATOM | 545 | C | MET | E | 38 | 43.688 | 108.509 | 49.401 | 1.00 | 0.00 | AAAA |
| ATOM | 546 | O | MET | E | 38 | 42.970 | 109.504 | 49.561 | 1.00 | 0.00 | AAAA |
| ATOM | 547 | N | PHE | E | 39 | 44.696 | 108.234 | 50.224 | 1.00 | 0.00 | AAAA |
| ATOM | 549 | CA | PHE | E | 39 | 44.855 | 108.908 | 51.494 | 1.00 | 0.00 | AAAA |
| ATOM | 551 | CB | PHE | E | 39 | 43.664 | 108.524 | 52.383 | 1.00 | 0.00 | AAAA |
| ATOM | 554 | CG | PHE | E | 39 | 43.603 | 107.042 | 52.664 | 1.00 | 0.00 | AAAA |
| ATOM | 555 | CD1 | PHE | E | 39 | 42.838 | 106.148 | 51.911 | 1.00 | 0.00 | AAAA |
| ATOM | 557 | CD2 | PHE | E | 39 | 43.916 | 106.586 | 53.950 | 1.00 | 0.00 | AAAA |
| ATOM | 559 | CE1 | PHE | E | 39 | 42.685 | 104.808 | 52.290 | 1.00 | 0.00 | AAAA |
| ATOM | 561 | CE2 | PHE | E | 39 | 43.653 | 105.285 | 54.414 | 1.00 | 0.00 | AAAA |
| ATOM | 563 | CZ | PHE | E | 39 | 43.175 | 104.335 | 53.508 | 1.00 | 0.00 | AAAA |
| ATOM | 565 | C | PHE | E | 39 | 46.239 | 108.734 | 52.123 | 1.00 | 0.00 | AAAA |
| ATOM | 566 | O | PHE | E | 39 | 46.429 | 109.017 | 53.303 | 1.00 | 0.00 | AAAA |
| ATOM | 567 | N | LYS | E | 40 | 47.205 | 108.190 | 51.388 | 1.00 | 0.00 | AAAA |
| ATOM | 569 | CA | LYS | E | 40 | 48.575 | 107.902 | 51.776 | 1.00 | 0.00 | AAAA |
| ATOM | 571 | CB | LYS | E | 40 | 48.779 | 106.395 | 51.850 | 1.00 | 0.00 | AAAA |
| ATOM | 574 | CG | LYS | E | 40 | 47.960 | 105.790 | 52.999 | 1.00 | 0.00 | AAAA |
| ATOM | 577 | CD | LYS | E | 40 | 48.790 | 105.886 | 54.287 | 1.00 | 0.00 | AAAA |
| ATOM | 580 | CE | LYS | E | 40 | 47.999 | 105.339 | 55.474 | 1.00 | 0.00 | AAAA |
| ATOM | 583 | NZ | LYS | E | 40 | 48.706 | 105.484 | 56.759 | 1.00 | 0.00 | AAAA |
| ATOM | 587 | C | LYS | E | 40 | 49.564 | 108.479 | 50.773 | 1.00 | 0.00 | AAAA |
| ATOM | 588 | O | LYS | E | 40 | 50.776 | 108.436 | 50.974 | 1.00 | 0.00 | AAAA |
| ATOM | 589 | N | THR | E | 41 | 49.115 | 109.039 | 49.650 | 1.00 | 0.00 | AAAA |
| ATOM | 591 | CA | THR | E | 41 | 49.823 | 109.777 | 48.625 | 1.00 | 0.00 | AAAA |
| ATOM | 593 | CB | THR | E | 41 | 49.004 | 109.716 | 47.334 | 1.00 | 0.00 | AAAA |
| ATOM | 595 | OG1 | THR | E | 41 | 48.822 | 108.361 | 46.976 | 1.00 | 0.00 | AAAA |
| ATOM | 597 | CG2 | THR | E | 41 | 49.633 | 110.325 | 46.074 | 1.00 | 0.00 | AAAA |
| ATOM | 601 | C | THR | E | 41 | 49.823 | 111.145 | 49.289 | 1.00 | 0.00 | AAAA |
| ATOM | 602 | O | THR | E | 41 | 48.795 | 111.741 | 49.580 | 1.00 | 0.00 | AAAA |
| ATOM | 603 | N | ARG | E | 42 | 51.010 | 111.734 | 49.486 | 1.00 | 0.00 | AAAA |
| ATOM | 605 | CA | ARG | E | 42 | 51.176 | 113.007 | 50.154 | 1.00 | 0.00 | AAAA |
| ATOM | 607 | CB | ARG | E | 42 | 52.635 | 113.071 | 50.601 | 1.00 | 0.00 | AAAA |
| ATOM | 610 | CG | ARG | E | 42 | 53.126 | 111.923 | 51.474 | 1.00 | 0.00 | AAAA |
| ATOM | 613 | CD | ARG | E | 42 | 54.647 | 112.005 | 51.607 | 1.00 | 0.00 | AAAA |
| ATOM | 616 | NE | ARG | E | 42 | 55.221 | 111.542 | 50.347 | 1.00 | 0.00 | AAAA |
| ATOM | 618 | CZ | ARG | E | 42 | 56.498 | 111.121 | 50.250 | 1.00 | 0.00 | AAAA |
| ATOM | 619 | NH1 | ARG | E | 42 | 57.342 | 111.120 | 51.292 | 1.00 | 0.00 | AAAA |
| ATOM | 622 | NH2 | ARG | E | 42 | 56.816 | 110.805 | 48.997 | 1.00 | 0.00 | AAAA |
| ATOM | 625 | C | ARG | E | 42 | 50.848 | 114.122 | 49.183 | 1.00 | 0.00 | AAAA |
| ATOM | 626 | O | ARG | E | 42 | 50.941 | 113.938 | 47.974 | 1.00 | 0.00 | AAAA |
| ATOM | 627 | N | PRO | E | 43 | 50.583 | 115.345 | 49.645 | 1.00 | 0.00 | AAAA |
| ATOM | 628 | CA | PRO | E | 43 | 50.641 | 116.546 | 48.831 | 1.00 | 0.00 | AAAA |
| ATOM | 630 | CB | PRO | E | 43 | 50.806 | 117.662 | 49.861 | 1.00 | 0.00 | AAAA |
| ATOM | 633 | CG | PRO | E | 43 | 50.043 | 117.160 | 51.096 | 1.00 | 0.00 | AAAA |
| ATOM | 636 | CD | PRO | E | 43 | 50.236 | 115.643 | 51.015 | 1.00 | 0.00 | AAAA |
| ATOM | 639 | C | PRO | E | 43 | 51.697 | 116.684 | 47.743 | 1.00 | 0.00 | AAAA |
| ATOM | 640 | O | PRO | E | 43 | 51.352 | 116.966 | 46.593 | 1.00 | 0.00 | AAAA |
| ATOM | 641 | N | GLU | E | 44 | 52.965 | 116.424 | 48.071 | 1.00 | 0.00 | AAAA |
| ATOM | 643 | CA | GLU | E | 44 | 54.088 | 116.421 | 47.152 | 1.00. | 0.00 | AAAA |
| ATOM | 645 | CB | GLU | E | 44 | 55.390 | 116.402 | 47.960 | 1.00 | 0.00 | AAAA |
| ATOM | 648 | CG | GLU | E | 44 | 55.479 | 115.556 | 49.228 | 1.00 | 0.00 | AAAA |
| ATOM | 651 | CD | GLU | E | 44 | 54.891 | 116.190 | 50.472 | 1.00 | 0.00 | AAAA |
| ATOM | 652 | OE1 | GLU | E | 44 | 53.670 | 116.126 | 50.746 | 1.00 | 0.00 | AAAA |
| ATOM | 653 | OE2 | GLU | E | 44 | 55.750 | 116.689 | 51.227 | 1.00 | 0.00 | AAAA |
| ATOM | 654 | C | GLU | E | 44 | 54.110 | 115.284 | 46.145 | 1.00 | 0.00 | AAAA |
| ATOM | 655 | O | GLU | E | 44 | 54.561 | 115.479 | 45.017 | 1.00 | 0.00 | AAAA |
| ATOM | 656 | N | ASP | E | 45 | 53.619 | 114.120 | 46.557 | 1.00 | 0.00 | AAAA |
| ATOM | 658 | CA | ASP | E | 45 | 53.370 | 113.047 | 45.617 | 1.00 | 0.00 | AAAA |
| ATOM | 660 | CB | ASP | E | 45 | 52.904 | 111.709 | 46.173 | 1.00 | 0.00 | AAAA |
| ATOM | 663 | CG | ASP | E | 45 | 53.824 | 111.293 | 47.312 | 1.00 | 0.00 | AAAA |
| ATOM | 664 | OD1 | ASP | E | 45 | 55.067 | 111.256 | 47.145 | 1.00 | 0.00 | AAAA |
| ATOM | 665 | OD2 | ASP | E | 45 | 53.341 | 110.950 | 48.417 | 1.00 | 0.00 | AAAA |
| ATOM | 666 | C | ASP | E | 45 | 52.321 | 113.408 | 44.576 | 1.00 | 0.00 | AAAA |
| ATOM | 667 | O | ASP | E | 45 | 52.444 | 113.141 | 43.383 | 1.00 | 0.00 | AAAA |
| ATOM | 668 | N | PHE | E | 46 | 51.241 | 114.099 | 44.942 | 1.00 | 0.00 | AAAA |
| ATOM | 670 | CA | PHE | E | 46 | 50.147 | 114.518 | 44.076 | 1.00 | 0.00 | AAAA |
| ATOM | 672 | CB | PHE | E | 46 | 48.982 | 114.957 | 44.964 | 1.00 | 0.00 | AAAA |
| ATOM | 675 | CG | PHE | E | 46 | 47.989 | 113.948 | 45.474 | 1.00 | 0.00 | AAAA |
| ATOM | 676 | CD1 | PHE | E | 46 | 47.427 | 113.050 | 44.549 | 1.00 | 0.00 | AAAA |
| ATOM | 678 | CD2 | PHE | E | 46 | 47.693 | 113.855 | 46.839 | 1.00 | 0.00 | AAAA |
| ATOM | 680 | CE1 | PHE | E | 46 | 46.623 | 112.008 | 45.018 | 1.00 | 0.00 | AAAA |
| ATOM | 682 | CE2 | PHE | E | 46 | 46.814 | 112.858 | 47.281 | 1.00 | 0.00 | AAAA |
| ATOM | 684 | CZ | PHE | E | 46 | 46.257 | 111.968 | 46.367 | 1.00 | 0.00 | AAAA |
| ATOM | 686 | C | PHE | E | 46 | 50.461 | 115.677 | 43.146 | 1.00 | 0.00 | AAAA |
| ATOM | 687 | O | PHE | E | 46 | 49.592 | 116.180 | 42.441 | 1.00 | 0.00 | AAAA |
| ATOM | 688 | N | ARG | E | 47 | 51.692 | 116.197 | 43.103 | 1.00 | 0.00 | AAAA |
| ATOM | 690 | CA | ARG | E | 47 | 52.158 | 117.084 | 42.049 | 1.00 | 0.00 | AAAA |
| ATOM | 692 | CB | ARG | E | 47 | 52.313 | 118.520 | 42.550 | 1.00 | 0.00 | AAAA |
| ATOM | 695 | CG | ARG | E | 47 | 53.087 | 118.739 | 43.847 | 1.00 | 0.00 | AAAA |
| ATOM | 698 | CD | ARG | E | 47 | 53.290 | 120.208 | 44.230 | 1.00 | 0.00 | AAAA |
| ATOM | 701 | NE | ARG | E | 47 | 53.865 | 120.332 | 45.570 | 1.00 | 0.00 | AAAA |
| ATOM | 703 | CZ | ARG | E | 47 | 53.309 | 120.843 | 46.675 | 1.00 | 0.00 | AAAA |
| ATOM | 704 | NH1 | ARG | E | 47 | 52.062 | 121.341 | 46.712 | 1.00 | 0.00 | AAAA |
| ATOM | 707 | NH2 | ARG | E | 47 | 54.087 | 120.868 | 47.767 | 1.00 | 0.00 | AAAA |
| ATOM | 710 | C | ARG | E | 47 | 53.424 | 116.524 | 41.394 | 1.00 | 0.00 | AAAA |
| ATOM | 711 | O | ARG | E | 47 | 53.806 | 116.977 | 40.316 | 1.00 | 0.00 | AAAA |
| ATOM | 712 | N | ASP | E | 48 | 54.064 | 115.523 | 42.008 | 1.00 | 0.00 | AAAA |
| ATOM | 714 | CA | ASP | E | 48 | 55.074 | 114.669 | 41.421 | 1.00 | 0.00 | AAAA |
| ATOM | 716 | CB | ASP | E | 48 | 56.020 | 114.063 | 42.466 | 1.00 | 0.00 | AAAA |
| ATOM | 719 | CG | ASP | E | 48 | 57.097 | 113.109 | 41.978 | 1.00 | 0.00 | AAAA |
| ATOM | 720 | OD1 | ASP | E | 48 | 56.965 | 111.921 | 42.364 | 1.00 | 0.00 | AAAA |
| ATOM | 721 | OD2 | ASP | E | 48 | 58.069 | 113.504 | 41.297 | 1.00 | 0.00 | AAAA |
| ATOM | 722 | C | ASP | E | 48 | 54.445 | 113.601 | 40.521 | 1.00 | 0.00 | AAAA |
| ATOM | 723 | O | ASP | E | 48 | 55.040 | 113.067 | 39.595 | 1.00 | 0.00 | AAAA |
| ATOM | 724 | N | LEU | E | 49 | 53.136 | 113.391 | 40.614 | 1.00 | 0.00 | AAAA |
| ATOM | 726 | CA | LEU | E | 49 | 52.324 | 112.745 | 39.594 | 1.00 | 0.00 | AAAA |
| ATOM | 728 | CB | LEU | E | 49 | 51.164 | 111.980 | 40.247 | 1.00 | 0.00 | AAAA |
| ATOM | 731 | CG | LEU | E | 49 | 51.624 | 110.640 | 40.806 | 1.00 | 0.00 | AAAA |
| ATOM | 733 | CD1 | LEU | E | 49 | 50.613 | 109.990 | 41.751 | 1.00 | 0.00 | AAAA |
| ATOM | 737 | CD2 | LEU | E | 49 | 51.940 | 109.677 | 39.661 | 1.00 | 0.00 | AAAA |
| ATOM | 741 | C | LEU | E | 49 | 51.835 | 113.938 | 38.788 | 1.00 | 0.00 | AAAA |
| ATOM | 742 | O | LEU | E | 49 | 51.186 | 114.812 | 39.341 | 1.00 | 0.00 | AAAA |
| ATOM | 743 | N | SER | E | 50 | 52.078 | 113.922 | 37.470 | 1.00 | 0.00 | AAAA |
| ATOM | 745 | CA | SER | E | 50 | 51.611 | 114.917 | 36.521 | 1.00 | 0.00 | AAAA |
| ATOM | 747 | CB | SER | E | 50 | 52.558 | 116.116 | 36.455 | 1.00 | 0.00 | AAAA |
| ATOM | 750 | OG | SER | E | 50 | 52.154 | 117.153 | 35.590 | 1.00 | 0.00 | AAAA |
| ATOM | 752 | C | SER | E | 50 | 51.411 | 114.210 | 35.188 | 1.00 | 0.00 | AAAA |
| ATOM | 753 | O | SER | E | 50 | 52.356 | 113.544 | 34.786 | 1.00 | 0.00 | AAAA |
| ATOM | 754 | N | PHE | E | 51 | 50.349 | 114.399 | 34.405 | 1.00 | 0.00 | AAAA |
| ATOM | 756 | CA | PHE | E | 51 | 50.001 | 113.691 | 33.191 | 1.00 | 0.00 | AAAA |
| ATOM | 758 | CB | PHE | E | 51 | 48.809 | 112.732 | 33.318 | 1.00 | 0.00 | AAAA |
| ATOM | 761 | CG | PHE | E | 51 | 49.153 | 111.543 | 34.192 | 1.00 | 0.00 | AAAA |
| ATOM | 762 | CD1 | PHE | E | 51 | 49.343 | 110.280 | 33.630 | 1.00 | 0.00 | AAAA |
| ATOM | 764 | CD2 | PHE | E | 51 | 49.186 | 111.711 | 35.581 | 1.00 | 0.00 | AAAA |
| ATOM | 766 | CE1 | PHE | E | 51 | 49.711 | 109.209 | 34.441 | 1.00 | 0.00 | AAAA |
| ATOM | 768 | CE2 | PHE | E | 51 | 49.541 | 110.611 | 36.373 | 1.00 | 0.00 | AAAA |
| ATOM | 770 | CZ | PHE | E | 51 | 49.756 | 109.335 | 35.840 | 1.00 | 0.00 | AAAA |
| ATOM | 772 | C | PHE | E | 51 | 49.725 | 114.738 | 32.124 | 1.00 | 0.00 | AAAA |
| ATOM | 773 | O | PHE | E | 51 | 48.594 | 115.045 | 31.765 | 1.00 | 0.00 | AAAA |
| ATOM | 774 | N | PRO | E | 52 | 50.758 | 115.344 | 31.528 | 1.00 | 0.00 | AAAA |
| ATOM | 775 | CA | PRO | E | 52 | 50.650 | 116.301 | 30.451 | 1.00 | 0.00 | AAAA |
| ATOM | 777 | CB | PRO | E | 52 | 51.976 | 117.062 | 30.448 | 1.00 | 0.00 | AAAA |
| ATOM | 780 | CG | PRO | E | 52 | 52.929 | 115.910 | 30.777 | 1.00 | 0.00 | AAAA |
| ATOM | 783 | CD | PRO | E | 52 | 52.152 | 115.107 | 31.831 | 1.00 | 0.00 | AAAA |
| ATOM | 786 | C | PRO | E | 52 | 50.430 | 115.726 | 29.053 | 1.00 | 0.00 | AAAA |
| ATOM | 787 | O | PRO | E | 52 | 50.337 | 116.542 | 28.143 | 1.00 | 0.00 | AAAA |
| ATOM | 788 | N | LYS | E | 53 | 50.399 | 114.420 | 28.779 | 1.00 | 0.00 | AAAA |
| ATOM | 790 | CA | LYS | E | 53 | 49.885 | 114.021 | 27.481 | 1.00 | 0.00 | AAAA |
| ATOM | 792 | CB | LYS | E | 53 | 50.247 | 112.570 | 27.140 | 1.00 | 0.00 | AAAA |
| ATOM | 795 | CG | LYS | E | 53 | 51.700 | 112.328 | 26.730 | 1.00 | 0.00 | AAAA |
| ATOM | 798 | CD | LYS | E | 53 | 52.780 | 112.125 | 27.797 | 1.00 | 0.00 | AAAA |
| ATOM | 801 | CE | LYS | E | 53 | 54.179 | 111.767 | 27.310 | 1.00 | 0.00 | AAAA |
| ATOM | 804 | NZ | LYS | E | 53 | 55.027 | 111.577 | 28.496 | 1.00 | 0.00 | AAAA |
| ATOM | 808 | C | LYS | E | 53 | 48.390 | 114.179 | 27.302 | 1.00 | 0.00 | AAAA |
| ATOM | 809 | O | LYS | E | 53 | 47.887 | 114.345 | 26.186 | 1.00 | 0.00 | AAAA |
| ATOM | 810 | N | LEU | E | 54 | 47.645 | 113.998 | 28.395 | 1.00 | 0.00 | AAAA |
| ATOM | 812 | CA | LEU | E | 54 | 46.197 | 113.869 | 28.402 | 1.00 | 0.00 | AAAA |
| ATOM | 814 | CB | LEU | E | 54 | 45.689 | 113.492 | 29.792 | 1.00 | 0.00 | AAAA |
| ATOM | 817 | CG | LEU | E | 54 | 44.211 | 113.113 | 29.910 | 1.00 | 0.00 | AAAA |
| ATOM | 819 | CD1 | LEU | E | 54 | 43.741 | 111.924 | 29.063 | 1.00 | 0.00 | AAAA |
| ATOM | 823 | CD2 | LEU | E | 54 | 44.062 | 112.832 | 31.403 | 1.00 | 0:00 | AAAA |
| ATOM | 827 | C | LEU | E | 54 | 45.492 | 115.153 | 27.974 | 1.00 | 0.00 | AAAA |
| ATOM | 828 | O | LEU | E | 54 | 45.691 | 116.212 | 28.563 | 1.00 | 0.00 | AAAA |
| ATOM | 829 | N | ILE | E | 55 | 44.589 | 115.028 | 26.994 | 1.00 | 0.00 | AAAA |
| ATOM | 831 | CA | ILE | E | 55 | 43.871 | 116.120 | 26.375 | 1.00 | 0.00 | AAAA |
| ATOM | 833 | CB | ILE | E | 55 | 44.115 | 116.170 | 24.867 | 1.00 | 0.00 | AAAA |
| ATOM | 835 | CG1 | ILE | E | 55 | 45.559 | 115.881 | 24.432 | 1.00 | 0.00 | AAAA |
| ATOM | 838 | CG2 | ILE | E | 55 | 43.558 | 117.411 | 24.178 | 1.00 | 0.00 | AAAA |
| ATOM | 842 | CD1 | ILE | E | 55 | 45.786 | 115.788 | 22.929 | 1.00 | 0.00 | AAAA |
| ATOM | 846 | C | ILE | E | 55 | 42.367 | 116.077 | 26.595 | 1.00 | 0.00 | AAAA |
| ATOM | 847 | O | ILE | E | 55 | 41.713 | 117.111 | 26.741 | 1.00 | 0.00 | AAAA |
| ATOM | 848 | N | MET | E | 56 | 41.809 | 114.878 | 26.791 | 1.00 | 0.00 | AAAA |
| ATOM | 850 | CA | MET | E | 56 | 40.386 | 114.643 | 26.925 | 1.00 | 0.00 | AAAA |
| ATOM | 852 | CB | MET | E | 56 | 39.730 | 114.576 | 25.553 | 1.00 | 0.00 | AAAA |
| ATOM | 855 | CG | MET | E | 56 | 38.247 | 114.185 | 25.523 | 1.00 | 0.00 | AAAA |
| ATOM | 858 | SD | MET | E | 56 | 37.297 | 114.968 | 24.196 | 1.00 | 0.00 | AAAA |
| ATOM | 859 | CE | MET | E | 56 | 37.248 | 113.636 | 22.965 | 1.00 | 0.00 | AAAA |
| ATOM | 863 | C | MET | E | 56 | 40.115 | 113.453 | 27.845 | 1.00 | 0.00 | AAAA |
| ATOM | 864 | O | MET | E | 56 | 40.625 | 112.356 | 27.634 | 1.00 | 0.00 | AAAA |
| ATOM | 865 | N | ILE | E | 57 | 39.295 | 113.623 | 28.881 | 1.00 | 0.00 | AAAA |
| ATOM | 867 | CA | ILE | E | 57 | 38.459 | 112.598 | 29.486 | 1.00 | 0.00 | AAAA |
| ATOM | 869 | CB | ILE | E | 57 | 38.436 | 112.771 | 31.002 | 1.00 | 0.00 | AAAA |
| ATOM | 871 | CG1 | ILE | E | 57 | 39.836 | 112.604 | 31.584 | 1.00 | 0.00 | AAAA |
| ATOM | 874 | CG2 | ILE | E | 57 | 37.500 | 111.778 | 31.684 | 1.00 | 0.00 | AAAA |
| ATOM | 878 | CD1 | ILE | E | 57 | 40.149 | 113.053 | 33.009 | 1.00 | 0.00 | AAAA |
| ATOM | 882 | C | ILE | E | 57 | 37.121 | 112.760 | 28.785 | 1.00 | 0.00 | AAAA |
| ATOM | 883 | O | ILE | E | 57 | 36.824 | 113.935 | 28.582 | 1.00 | 0.00 | AAAA |
| ATOM | 884 | N | THR | E | 58 | 36.479 | 111.723 | 28.245 | 1.00 | 0.00 | AAAA |
| ATOM | 886 | CA | THR | E | 58 | 35.410 | 111.788 | 27.263 | 1.00 | 0.00 | AAAA |
| ATOM | 888 | CB | THR | E | 58 | 35.300 | 110.510 | 26.430 | 1.00 | 0.00 | AAAA |
| ATOM | 890 | OG1 | THR | E | 58 | 36.605 | 110.278 | 25.954 | 1.00 | 0.00 | AAAA |
| ATOM | 892 | CG2 | THR | E | 58 | 34.392 | 110.615 | 25.201 | 1.00 | 0.00 | AAAA |
| ATOM | 896 | C | THR | E | 58 | 34.122 | 112.114 | 28.012 | 1.00 | 0.00 | AAAA |
| ATOM | 897 | O | THR | E | 58 | 33.504 | 113.166 | 27.861 | 1.00 | 0.00 | AAAA |
| ATOM | 898 | N | ASP | E | 59 | 33.734 | 111.187 | 28.892 | 1.00 | 0.00 | AAAA |
| ATOM | 900 | CA | ASP | E | 59 | 32.442 | 111.046 | 29.529 | 1.00 | 0.00 | AAAA |
| ATOM | 902 | CB | ASP | E | 59 | 32.075 | 109.575 | 29.734 | 1.00 | 0.00 | AAAA |
| ATOM | 905 | CG | ASP | E | 59 | 31.901 | 108.722 | 28.487 | 1.00 | 0.00 | AAAA |
| ATOM | 906 | OD1 | ASP | E | 59 | 31.403 | 109.141 | 27.416 | 1.00 | 0.00 | AAAA |
| ATOM | 907 | OD2 | ASP | E | 59 | 32.213 | 107.509 | 28.537 | 1.00 | 0.00 | AAAA |
| ATOM | 908 | C | ASP | E | 59 | 32.429 | 111.897 | 30.798 | 1.00 | 0.00 | AAAA |
| ATOM | 909 | O | ASP | E | 59 | 32.012 | 113.048 | 30.703 | 1.00 | 0.00 | AAAA |
| ATOM | 910 | N | TYR | E | 60 | 32.987 | 111.322 | 31.866 | 1.00 | 0.00 | AAAA |
| ATOM | 912 | CA | TYR | E | 60 | 33.081 | 111.966 | 33.154 | 1.00 | 0.00 | AAAA |
| ATOM | 914 | CB | TYR | E | 60 | 31.990 | 111.608 | 34.173 | 1.00 | 0.00 | AAAA |
| ATOM | 917 | CG | TYR | E | 60 | 31.789 | 110.138 | 34.421 | 1.00 | 0.00 | AAAA |
| ATOM | 918 | CD1 | TYR | E | 60 | 32.164 | 109.585 | 35.647 | 1.00 | 0.00 | AAAA |
| ATOM | 920 | CD2 | TYR | E | 60 | 31.041 | 109.333 | 33.556 | 1.00 | 0.00 | AAAA |
| ATOM | 922 | CE1 | TYR | E | 60 | 31.983 | 108.238 | 35.960 | 1.00 | 0.00 | AAAA |
| ATOM | 924 | CE2 | TYR | E | 60 | 30.743 | 108.011 | 33.918 | 1.00 | 0.00 | AAAA |
| ATOM | 926 | CZ | TYR | E | 60 | 31.219 | 107.427 | 35.105 | 1.00 | 0.00 | AAAA |
| ATOM | 927 | OH | TYR | E | 60 | 30.886 | 106.166 | 35.489 | 1.00 | 0.00 | AAAA |
| ATOM | 929 | C | TYR | E | 60 | 34.490 | 111.766 | 33.711 | 1.00 | 0.00 | AAAA |
| ATOM | 930 | O | TYR | E | 60 | 35.092 | 110.715 | 33.552 | 1.00 | 0.00 | AAAA |
| ATOM | 931 | N | LEU | E | 61 | 34.925 | 112.784 | 34.460 | 1.00 | 0.00 | AAAA |
| ATOM | 933 | CA | LEU | E | 61 | 36.022 | 112.779 | 35.406 | 1.00 | 0.00 | AAAA |
| ATOM | 935 | CB | LEU | E | 61 | 36.992 | 113.957 | 35.209 | 1.00 | 0.00 | AAAA |
| ATOM | 938 | CG | LEU | E | 61 | 38.081 | 114.083 | 36.279 | 1.00 | 0.00 | AAAA |
| ATOM | 940 | CD1 | LEU | E | 61 | 39.037 | 112.923 | 36.531 | 1.00 | 0.00 | AAAA |
| ATOM | 944 | CD2 | LEU | E | 61 | 38.908 | 115.352 | 36.073 | 1.00 | 0.00 | AAAA |
| ATOM | 948 | C | LEU | E | 61 | 35.373 | 112.909 | 36.772 | 1.00 | 0.00 | AAAA |
| ATOM | 949 | O | LEU | E | 61 | 34.646 | 113.859 | 37.082 | 1.00 | 0.00 | AAAA |
| ATOM | 950 | N | LEU | E | 62 | 35.649 | 111.941 | 37.639 | 1.00 | 0.00 | AAAA |
| ATOM | 952 | CA | LEU | E | 62 | 35.225 | 111.726 | 39.005 | 1.00 | 0.00 | AAAA |
| ATOM | 954 | CB | LEU | E | 62 | 34.187 | 110.603 | 39.036 | 1.00 | 0.00 | AAAA |
| ATOM | 957 | CG | LEU | E | 62 | 33.471 | 110.419 | 40.380 | 1.00 | 0.00 | AAAA |
| ATOM | 959 | CD1 | LEU | E | 62 | 32.006 | 110.237 | 40.007 | 1.00 | 0.00 | AAAA |
| ATOM | 963 | CD2 | LEU | E | 62 | 34.106 | 109.276 | 41.188 | 1.00 | 0.00 | AAAA |
| ATOM | 967 | C | LEU | E | 62 | 36.379 | 111.355 | 39.933 | 1.00 | 0.00 | AAAA |
| ATOM | 968 | O | LEU | E | 62 | 37.168 | 110.481 | 39.589 | 1.00 | 0.00 | AAAA |
| ATOM | 969 | N | LEU | E | 63 | 36.535 | 112.154 | 40.987 | 1.00 | 0.00 | AAAA |
| ATOM | 971 | CA | LEU | E | 63 | 37.418 | 111.955 | 42.122 | 1.00 | 0.00 | AAAA |
| ATOM | 973 | CB | LEU | E | 63 | 38.305 | 113.167 | 42.336 | 1.00 | 0.00 | AAAA |
| ATOM | 976 | CG | LEU | E | 63 | 39.578 | 113.440 | 41.522 | 1.00 | 0.00 | AAAA |
| ATOM | 978 | CD1 | LEU | E | 63 | 39.381 | 114.138 | 40.175 | 1.00 | 0.00 | AAAA |
| ATOM | 982 | CD2 | LEU | E | 63 | 40.533 | 114.211 | 42.431 | 1.00 | 0.00 | AAAA |
| ATOM | 986 | C | LEU | E | 63 | 36.492 | 111.851 | 43.331 | 1.00 | 0.00 | AAAA |
| ATOM | 987 | O | LEU | E | 63 | 35.564 | 112.644 | 43.454 | 1.00 | 0.00 | AAAA |
| ATOM | 988 | N | PHE | E | 64 | 36.768 | 110.829 | 44.129 | 1.00 | 0.00 | AAAA |
| ATOM | 990 | CA | PHE | E | 64 | 35.982 | 110.373 | 45.262 | 1.00 | 0.00 | AAAA |
| ATOM | 992 | CB | PHE | E | 64 | 34.942 | 109.323 | 44.858 | 1.00 | 0.00 | AAAA |
| ATOM | 995 | CG | PHE | E | 64 | 34.313 | 108.470 | 45.934 | 1.00 | 0.00 | AAAA |
| ATOM | 996 | CD1 | PHE | E | 64 | 33.919 | 109.000 | 47.161 | 1.00 | 0.00 | AAAA |
| ATOM | 998 | CD2 | PHE | E | 64 | 34.094 | 107.102 | 45.701 | 1.00 | 0.00 | AAAA |
| ATOM | 1000 | CE1 | PHE | E | 64 | 33.424 | 108.187 | 48.196 | 1.00 | 0.00 | AAAA |
| ATOM | 1002 | CE2 | PHE | E | 64 | 33.581 | 106.265 | 46.695 | 1.00 | 0.00 | AAAA |
| ATOM | 1004 | CZ | PHE | E | 64 | 33.288 | 106.809 | 47.950 | 1.00 | 0.00 | AAAA |
| ATOM | 1006 | C | PHE | E | 64 | 36.972 | 109.995 | 46.357 | 1.00 | 0.00 | AAAA |
| ATOM | 1007 | O | PHE | E | 64 | 37.348 | 108.821 | 46.355 | 1.00 | 0.00 | AAAA |
| ATOM | 1008 | N | ARG | E | 65 | 37.234 | 110.802 | 47.386 | 1.00 | 0.00 | AAAA |
| ATOM | 1010 | CA | ARG | E | 65 | 38.011 | 110.463 | 48.564 | 1.00 | 0.00 | AAAA |
| ATOM | 1012 | CB | ARG | E | 65 | 37.456 | 109.235 | 49.299 | 1.00 | 0.00 | AAAA |
| ATOM | 1015 | CG | ARG | E | 65 | 37.891 | 109.106 | 50.754 | 1.00 | 0.00 | AAAA |
| ATOM | 1018 | CD | ARG | E | 65 | 37.314 | 107.861 | 51.427 | 1.00 | 0.00 | AAAA |
| ATOM | 1021 | NE | ARG | E | 65 | 37.503 | 107.968 | 52.881 | 1.00 | 0.00 | AAAA |
| ATOM | 1023 | CZ | ARG | E | 65 | 36.685 | 108.224 | 53.914 | 1.00 | 0.00 | AAAA |
| ATOM | 1024 | NH1 | ARG | E | 65 | 35.475 | 108.727 | 53.628 | 1.00 | 0.00 | AAAA |
| ATOM | 1027 | NH2 | ARG | E | 65 | 37.179 | 108.150 | 55.148 | 1.00 | 0.00 | AAAA |
| ATOM | 1030 | C | ARG | E | 65 | 39.527 | 110.503 | 48.434 | 1.00 | 0.00 | AAAA |
| ATOM | 1031 | O | ARG | E | 65 | 40.302 | 109.678 | 48.901 | 1.00 | 0.00 | AAAA |
| ATOM | 1032 | N | VAL | E | 66 | 40.053 | 111.521 | 47.741 | 1.00 | 0.00 | AAAA |
| ATOM | 1034 | CA | VAL | E | 66 | 41.454 | 111.721 | 47.418 | 1.00 | 0.00 | AAAA |
| ATOM | 1036 | CB | VAL | E | 66 | 41.588 | 112.118 | 45.952 | 1:00 | 0.00 | AAAA |
| ATOM | 1038 | CG1 | VAL | E | 66 | 43.040 | 112.168 | 45.485 | 1.00 | 0.00 | AAAA |
| ATOM | 1042 | CG2 | VAL | E | 66 | 40.891 | 111.149 | 44.990 | 1.00 | 0.00 | AAAA |
| ATOM | 1046 | C | VAL | E | 66 | 41.951 | 112.739 | 48.432 | 1.00 | 0.00 | AAAA |
| ATOM | 1047 | O | VAL | E | 66 | 41.792 | 113.935 | 48.182 | 1.00 | 0.00 | AAAA |
| ATOM | 1048 | N | TYR | E | 67 | 42.298 | 112.328 | 49.662 | 1.00 | 0.00 | AAAA |
| ATOM | 1050 | CA | TYR | E | 67 | 42.747 | 113.100 | 50.800 | 1.00 | 0.00 | AAAA |
| ATOM | 1052 | CB | TYR | E | 67 | 42.465 | 112.349 | 52.099 | 1.00 | 0.00 | AAAA |
| ATOM | 1055 | CG | TYR | E | 67 | 41.025 | 112.066 | 52.463 | 1.00 | 0.00 | AAAA |
| ATOM | 1056 | CD1 | TYR | E | 67 | 39.905 | 112.758 | 51.989 | 1.00 | 0.00 | AAAA |
| ATOM | 1058 | CD2 | TYR | E | 67 | 40.772 | 111.293 | 53.602 | 1.00 | 0.00 | AAAA |
| ATOM | 1060 | CE1 | TYR | E | 67 | 38.634 | 112.470 | 52.491 | 1.00 | 0.00 | AAAA |
| ATOM | 1062 | CE2 | TYR | E | 67 | 39.512 | 110.935 | 54.098 | 1.00 | 0.00 | AAAA |
| ATOM | 1064 | CZ | TYR | E | 67 | 38.395 | 111.537 | 53.498 | 1.00 | 0.00 | AAAA |
| ATOM | 1065 | OH | TYR | E | 67 | 37.123 | 111.231 | 53.912 | 1.00 | 0.00 | AAAA |
| ATOM | 1067 | C | TYR | E | 67 | 44.239 | 113.362 | 50.730 | 1.00 | 0.00 | AAAA |
| ATOM | 1068 | O | TYR | E | 67 | 45.050 | 112.455 | 50.552 | 1.00 | 0.00 | AAAA |
| ATOM | 1069 | N | GLY | E | 68 | 44.626 | 114.636 | 50.892 | 1.00 | 0.00 | AAAA |
| ATOM | 1071 | CA | GLY | E | 68 | 45.979 | 115.142 | 50.833 | 1.00 | 0.00 | AAAA |
| ATOM | 1074 | C | GLY | E | 68 | 46.343 | 116.026 | 49.642 | 1.00 | 0.00 | AAAA |
| ATOM | 1075 | O | GLY | E | 68 | 47.441 | 116.561 | 49.560 | 1.00 | 0.00 | AAAA |
| ATOM | 1076 | N | LEU | E | 69 | 45.407 | 116.197 | 48.715 | 1.00 | 0.00 | AAAA |
| ATOM | 1078 | CA | LEU | E | 69 | 45.566 | 116.893 | 47.447 | 1.00 | 0.00 | AAAA |
| ATOM | 1080 | CB | LEU | E | 69 | 44.421 | 116.502 | 46.517 | 1.00 | 0.00 | AAAA |
| ATOM | 1083 | CG | LEU | E | 69 | 44.367 | 117.070 | 45.098 | 1.00 | 0.00 | AAAA |
| ATOM | 1085 | CD1 | LEU | E | 69 | 45.551 | 116.605 | 44.243 | 1.00 | 0.00 | AAAA |
| ATOM | 1089 | CD2 | LEU | E | 69 | 43.055 | 116.813 | 44.357 | 1.00 | 0.00 | AAAA |
| ATOM | 1093 | C | LEU | E | 69 | 45.546 | 118.376 | 47.769 | 1.00 | 0.00 | AAAA |
| ATOM | 1094 | O | LEU | E | 69 | 44.560 | 118.876 | 48.314 | 1.00 | 0.00 | AAAA |
| ATOM | 1095 | N | GLU | E | 70 | 46.628 | 119.076 | 47.437 | 1.00 | 0.00 | AAAA |
| ATOM | 1097 | CA | GLU | E | 70 | 46.606 | 120.486 | 47.788 | 1.00 | 0.00 | AAAA |
| ATOM | 1099 | CB | GLU | E | 70 | 48.034 | 120.953 | 48.067 | 1.00 | 0.00 | AAAA |
| ATOM | 1102 | CG | GLU | E | 70 | 48.274 | 122.276 | 48.800 | 1.00 | 0.00 | AAAA |
| ATOM | 1105 | CD | GLU | E | 70 | 49.691 | 122.265 | 49.366 | 1.00 | 0.00 | AAAA |
| ATOM | 1106 | OE1 | GLU | E | 70 | 50.618 | 122.496 | 48.558 | 1.00 | 0.00 | AAAA |
| ATOM | 1107 | OE2 | GLU | E | 70 | 49.876 | 122.038 | 50.577 | 1.00 | 0.00 | AAAA |
| ATOM | 1108 | C | GLU | E | 70 | 46.010 | 121.342 | 46.677 | 1.00 | 0.00 | AAAA |
| ATOM | 1109 | O | GLU | E | 70 | 45.267 | 122.273 | 46.970 | 1.00 | 0.00 | AAAA |
| ATOM | 1110 | N | SER | E | 71 | 46.300 | 121.036 | 45.408 | 1.00 | 0.00 | AAAA |
| ATOM | 1112 | CA | SER | E | 71 | 45.693 | 121.640 | 44.243 | 1.00 | 0.00 | AAAA |
| ATOM | 1114 | CB | SER | E | 71 | 46.359 | 122.984 | 43.917 | 1.00 | 0.00 | AAAA |
| ATOM | 1117 | OG | SER | E | 71 | 45.581 | 123.731 | 43.019 | 1.00 | 0.00 | AAAA |
| ATOM | 1119 | C | SER | E | 71 | 45.769 | 120.562 | 43.181 | 1.00 | 0.00 | AAAA |
| ATOM | 1120 | O | SER | E | 71 | 46.635 | 119.693 | 43.167 | 1.00 | 0.00 | AAAA |
| ATOM | 1121 | N | LEU | E | 72 | 44.832 | 120.529 | 42.226 | 1.00 | 0.00 | AAAA |
| ATOM | 1123 | CA | LEU | E | 72 | 44.759 | 119.592 | 41.118 | 1.00 | 0.00 | AAAA |
| ATOM | 1125 | CB | LEU | E | 72 | 43.281 | 119.504 | 40.742 | 1.00 | 0.00 | AAAA |
| ATOM | 1128 | CG | LEU | E | 72 | 42.898 | 118.258 | 39.936 | 1.00 | 0.00 | AAAA |
| ATOM | 1130 | CD1 | LEU | E | 72 | 42.942 | 116.934 | 40.698 | 1.00 | 0.00 | AAAA |
| ATOM | 1134 | CD2 | LEU | E | 72 | 41.487 | 118.441 | 39.366 | 1.00 | 0.00 | AAAA |
| ATOM | 1138 | C | LEU | E | 72 | 45.557 | 119.992 | 39.893 | 1.00 | 0.00 | AAAA |
| ATOM | 1139 | O | LEU | E | 72 | 45.792 | 119.156 | 39.023 | 1.00 | 0.00 | AAAA |
| ATOM | 1140 | N | LYS | E | 73 | 46.097 | 121.208 | 39.775 | 1.00 | 0.00 | AAAA |
| ATOM | 1142 | CA | LYS | E | 73 | 46.671 | 121.808 | 38.579 | 1.00 | 0.00 | AAAA |
| ATOM | 1144 | CB | LYS | E | 73 | 47.155 | 123.222 | 38.877 | 1.00 | 0.00 | AAAA |
| ATOM | 1147 | CG | LYS | E | 73 | 48.286 | 123.396 | 39.886 | 1.00 | 0.00 | AAAA |
| ATOM | 1150 | CD | LYS | E | 73 | 48.731 | 124.859 | 39.983 | 1.00 | 0.00 | AAAA |
| ATOM | 1153 | CE | LYS | E | 73 | 50.140 | 125.008 | 40.577 | 1.00 | 0.00 | AAAA |
| ATOM | 1156 | NZ | LYS | E | 73 | 50.295 | 126.427 | 40.914 | 1.00 | 0.00 | AAAA |
| ATOM | 1160 | C | LYS | E | 73 | 47.853 | 121.100 | 37.939 | 1.00 | 0.00 | AAAA |
| ATOM | 1161 | O | LYS | E | 73 | 48.045 | 121.139 | 36.727 | 1.00 | 0.00 | AAAA |
| ATOM | 1162 | N | ASP | E | 74 | 48.649 | 120.378 | 38.727 | 1.00 | 0.00 | AAAA |
| ATOM | 1164 | CA | ASP | E | 74 | 49.798 | 119.694 | 38.164 | 1.00 | 0.00 | AAAA |
| ATOM | 1166 | CB | ASP | E | 74 | 51.031 | 119.664 | 39.071 | 1.00 | 0.00 | AAAA |
| ATOM | 1169 | CG | ASP | E | 74 | 51.593 | 121.054 | 39.358 | 1.00 | 0.00 | AAAA |
| ATOM | 1170 | OD1 | ASP | E | 74 | 52.248 | 121.654 | 38.476 | 1.00 | 0.00 | AAAA |
| ATOM | 1171 | OD2 | ASP | E | 74 | 51.605 | 121.309 | 40.576 | 1.00 | 0.00 | AAAA |
| ATOM | 1172 | C | ASP | E | 74 | 49.431 | 118.245 | 37.889 | 1.00 | 0.00 | AAAA |
| ATOM | 1173 | O | ASP | E | 74 | 50.209 | 117.612 | 37.178 | 1.00 | 0.00 | AAAA |
| ATOM | 1174 | N | LEU | E | 75 | 48.309 | 117.679 | 38.355 | 1.00 | 0.00 | AAAA |
| ATOM | 1176 | CA | LEU | E | 75 | 47.900 | 116.305 | 38.195 | 1.00 | 0.00 | AAAA |
| ATOM | 1178 | CB | LEU | E | 75 | 46.983 | 115.977 | 39.379 | 1.00 | 0.00 | AAAA |
| ATOM | 1181 | CG | LEU | E | 75 | 46.915 | 114.478 | 39.676 | 1.00 | 0.00 | AAAA |
| ATOM | 1183 | CD1 | LEU | E | 75 | 48.097 | 114.057 | 40.539 | 1.00 | 0.00 | AAAA |
| ATOM | 1187 | CD2 | LEU | E | 75 | 45.544 | 114.062 | 40.219 | 1.00 | 0.00 | AAAA |
| ATOM | 1191 | C | LEU | E | 75 | 47.164 | 116.111 | 36.875 | 1.00 | 0.00 | AAAA |
| ATOM | 1192 | O | LEU | E | 75 | 47.513 | 115.193 | 36.147 | 1.00 | 0.00 | AAAA |
| ATOM | 1193 | N | PHE | E | 76 | 46.362 | 117.121 | 36.516 | 1.00 | 0.00 | AAAA |
| ATOM | 1195 | CA | PHE | E | 76 | 45.538 | 117.188 | 35.333 | 1.00 | 0.00 | AAAA |
| ATOM | 1197 | CB | PHE | E | 76 | 44.104 | 116.880 | 35.750 | 1.00 | 0.00 | AAAA |
| ATOM | 1200 | CG | PHE | E | 76 | 43.760 | 115.418 | 35.940 | 1.00 | 0.00 | AAAA |
| ATOM | 1201 | CD1 | PHE | E | 76 | 44.136 | 114.434 | 35.023 | 1.00 | 0.00 | AAAA |
| ATOM | 1203 | CD2 | PHE | E | 76 | 43.197 | 115.043 | 37.172 | 1.00 | 0.00 | AAAA |
| ATOM | 1205 | CE1 | PHE | E | 76 | 43.982 | 113.064 | 35.287 | 1.00 | 0.00 | AAAA |
| ATOM | 1207 | CE2 | PHE | E | 76 | 42.989 | 113.679 | 37.415 | 1.00 | 0.00 | AAAA |
| ATOM | 1209 | CZ | PHE | E | 76 | 43.439 | 112.703 | 36.520 | 1.00 | 0.00 | AAAA |
| ATOM | 1211 | C | PHE | E | 76 | 45.729 | 118.536 | 34.654 | 1.00 | 0.00 | AAAA |
| ATOM | 1212 | O | PHE | E | 76 | 44.769 | 119.303 | 34.733 | 1.00 | 0.00 | AAAA |
| ATOM | 1213 | N | PRO | E | 77 | 46.916 | 118.782 | 34.095 | 1.00 | 0.00 | AAAA |
| ATOM | 1214 | CA | PRO | E | 77 | 47.312 | 120.122 | 33.701 | 1.00 | 0.00 | AAAA |
| ATOM | 1216 | CB | PRO | E | 77 | 48.847 | 120.057 | 33.729 | 1.00 | 0.00 | AAAA |
| ATOM | 1219 | CG | PRO | E | 77 | 49.174 | 118.613 | 33.340 | 1.00 | 0.00 | AAAA |
| ATOM | 1222 | CD | PRO | E | 77 | 47.973 | 117.829 | 33.882 | 1.00 | 0.00 | AAAA |
| ATOM | 1225 | C | PRO | E | 77 | 46.904 | 120.536 | 32.305 | 1.00 | 0.00 | AAAA |
| ATOM | 1226 | O | PRO | E | 77 | 46.917 | 121.745 | 32.057 | 1.00 | 0.00 | AAAA |
| ATOM | 1227 | N | ASN | E | 78 | 46.652 | 119.604 | 31.388 | 1.00 | 0.00 | AAAA |
| ATOM | 1229 | CA | ASN | E | 78 | 46.552 | 119.712 | 29.950 | 1.00 | 0.00 | AAAA |
| ATOM | 1231 | CB | ASN | E | 78 | 47.703 | 118.937 | 29.298 | 1.00 | 0.00 | AAAA |
| ATOM | 1234 | CG | ASN | E | 78 | 47.979 | 119.217 | 27.824 | 1.00 | 0.00 | AAAA |
| ATOM | 1235 | OD1 | ASN | E | 78 | 47.982 | 120.361 | 27.388 | 1.00 | 0.00 | AAAA |
| ATOM | 1236 | ND2 | ASN | E | 78 | 48.216 | 118.198 | 27.001 | 1.00 | 0.00 | AAAA |
| ATOM | 1239 | C | ASN | E | 78 | 45.206 | 119.186 | 29.455 | 1.00 | 0.00 | AAAA |
| ATOM | 1240 | O | ASN | E | 78 | 44.805 | 119.174 | 28.291 | 1.00 | 0.00 | AAAA |
| ATOM | 1241 | N | LEU | E | 79 | 44.348 | 118.831 | 30.407 | 1.00 | 0.00 | AAAA |
| ATOM | 1243 | CA | LEU | E | 79 | 42.995 | 118.336 | 30.244 | 1.00 | 0.00 | AAAA |
| ATOM | 1245 | CB | LEU | E | 79 | 42.369 | 117.949 | 31.582 | 1.00 | 0.00 | AAAA |
| ATOM | 1248 | CG | LEU | E | 79 | 40.961 | 117.354 | 31.581 | 1.00 | 0.00 | AAAA |
| ATOM | 1250 | CD1 | LEU | E | 79 | 40.770 | 116.040 | 30.814 | 1.00 | 0.00 | AAAA |
| ATOM | 1254 | CD2 | LEU | E | 79 | 40.472 | 117.231 | 33.019 | 1.00 | 0.00 | AAAA |
| ATOM | 1258 | C | LEU | E | 79 | 42.172 | 119.502 | 29.699 | 1.00 | 0.00 | AAAA |
| ATOM | 1259 | O | LEU | E | 79 | 41.818 | 120.476 | 30.375 | 1.00 | 0.00 | AAAA |
| ATOM | 1260 | N | THR | E | 80 | 41.910 | 119.539 | 28.392 | 1.00 | 0.00 | AAAA |
| ATOM | 1262 | CA | THR | E | 80 | 41.279 | 120.652 | 27.726 | 1.00 | 0.00 | AAAA |
| ATOM | 1264 | CB | THR | E | 80 | 41.824 | 120.623 | 26.300 | 1.00 | 0.00 | AAAA |
| ATOM | 1266 | OG1 | THR | E | 80 | 43.238 | 120.599 | 26.322 | 1.00 | 0.00 | AAAA |
| ATOM | 1268 | CG2 | THR | E | 80 | 41.523 | 121.863 | 25.464 | 1.00 | 0.00 | AAAA |
| ATOM | 1272 | C | THR | E | 80 | 39.754 | 120.643 | 27.668 | 1.00 | 0.00 | AAAA |
| ATOM | 1273 | O | THR | E | 80 | 39.185 | 121.720 | 27.759 | 1.00 | 0.00 | AAAA |
| ATOM | 1274 | N | VAL | E | 81 | 39.149 | 119.474 | 27.430 | 1.00 | 0.00 | AAAA |
| ATOM | 1276 | CA | VAL | E | 81 | 37.705 | 119.353 | 27.411 | 1.00 | 0.00 | AAAA |
| ATOM | 1278 | CB | VAL | E | 81 | 37.169 | 119.337 | 25.983 | 1.00 | 0.00 | AAAA |
| ATOM | 1280 | CG1 | VAL | E | 81 | 37.586 | 120.527 | 25.132 | 1.00 | 0.00 | AAAA |
| ATOM | 1284 | CG2 | VAL | E | 81 | 37.583 | 118.020 | 25.326 | 1.00 | 0.00 | AAAA |
| ATOM | 1288 | C | VAL | E | 81 | 37.173 | 118.189 | 28.237 | 1.00 | 0.00 | AAAA |
| ATOM | 1289 | O | VAL | E | 81 | 37.922 | 117.351 | 28.733 | 1.00 | 0.00 | AAAA |
| ATOM | 1290 | N | ILE | E | 82 | 35.844 | 118.179 | 28.361 | 1.00 | 0.00 | AAAA |
| ATOM | 1292 | CA | ILE | E | 82 | 35.063 | 116.996 | 28.669 | 1.00 | 0.00 | AAAA |
| ATOM | 1294 | CB | ILE | E | 82 | 34.707 | 116.899 | 30.151 | 1.00 | 0.00 | AAAA |
| ATOM | 1296 | CG1 | ILE | E | 82 | 35.894 | 117.136 | 31.091 | 1.00 | 0.00 | AAAA |
| ATOM | 1299 | CG2 | ILE | E | 82 | 33.923 | 115.600 | 30.383 | 1.00 | 0.00 | AAAA |
| ATOM | 1303 | CD1 | ILE | E | 82 | 35.668 | 116.734 | 32.548 | 1.00 | 0.00 | AAAA |
| ATOM | 1307 | C | ILE | E | 82 | 33.870 | 117.042 | 27.736 | 1.00 | 0.00 | AAAA |
| ATOM | 1308 | O | ILE | E | 82 | 33.131 | 118.019 | 27.834 | 1.00 | 0.00 | AAAA |
| ATOM | 1309 | N | ARG | E | 83 | 33.697 | 116.022 | 26.885 | 1.00 | 0.00 | AAAA |
| ATOM | 1311 | CA | ARG | E | 83 | 32.573 | 115.918 | 25.968 | 1.00 | 0.00 | AAAA |
| ATOM | 1313 | CB | ARG | E | 83 | 32.979 | 114.902 | 24.888 | 1.00 | 0.00 | AAAA |
| ATOM | 1316 | CG | ARG | E | 83 | 33.961 | 115.435 | 23.847 | 1.00 | 0.00 | AAAA |
| ATOM | 1319 | CD | ARG | E | 83 | 33.372 | 116.321 | 22.747 | 1.00 | 0.00 | AAAA |
| ATOM | 1322 | NE | ARG | E | 83 | 34.420 | 116.496 | 21.745 | 1.00 | 0.00 | AAAA |
| ATOM | 1324 | CZ | ARG | E | 83 | 34.865 | 117.682 | 21.295 | 1.00 | 0.00 | AAAA |
| ATOM | 1325 | NH1 | ARG | E | 83 | 34.717 | 118.781 | 22.052 | 1.00 | 0.00 | AAAA |
| ATOM | 1328 | NH2 | ARG | E | 83 | 35.477 | 117.757 | 20.109 | 1.00 | 0.00 | AAAA |
| ATOM | 1331 | C | ARG | E | 83 | 31.226 | 115.688 | 26.619 | 1.00 | 0.00 | AAAA |
| ATOM | 1332 | O | ARG | E | 83 | 30.263 | 116.306 | 26.147 | 1.00 | 0.00 | AAAA |
| ATOM | 1333 | N | GLY | E | 84 | 31.116 | 114.700 | 27.502 | 1.00 | 0.00 | AAAA |
| ATOM | 1335 | CA | GLY | E | 84 | 29.928 | 114.403 | 28.277 | 1.00 | 0.00 | AAAA |
| ATOM | 1338 | C | GLY | E | 84 | 29.017 | 113.312 | 27.737 | 1.00 | 0.00 | AAAA |
| ATOM | 1339 | O | GLY | E | 84 | 27.827 | 113.328 | 28.049 | 1.00 | 0.00 | AAAA |
| ATOM | 1340 | N | SER | E | 85 | 29.476 | 112.544 | 26.753 | 1.00 | 0.00 | AAAA |
| ATOM | 1342 | CA | SER | E | 85 | 28.647 | 111.649 | 25.958 | 1.00 | 0.00 | AAAA |
| ATOM | 1344 | CB | SER | E | 85 | 29.542 | 111.043 | 24.869 | 1.00 | 0.00 | AAAA |
| ATOM | 1347 | OG | SER | E | 85 | 30.816 | 110.609 | 25.255 | 1.00 | 0.00 | AAAA |
| ATOM | 1349 | C | SER | E | 85 | 28.005 | 110.523 | 26.757 | 1.00 | 0.00 | AAAA |
| ATOM | 1350 | O | SER | E | 85 | 26.940 | 110.091 | 26.322 | 1.00 | 0.00 | AAAA |
| ATOM | 1351 | N | ARG | E | 86 | 28.561 | 110.134 | 27.914 | 1.00 | 0.00 | AAAA |
| ATOM | 1353 | CA | ARG | E | 86 | 27.962 | 109.452 | 29.043 | 1.00 | 0.00 | AAAA |
| ATOM | 1355 | CB | ARG | E | 86 | 28.307 | 107.973 | 29.156 | .1.00 | 0.00 | AAAA |
| ATOM | 1358 | CG | ARG | E | 86 | 27.749 | 106.948 | 28.160 | 1.00 | 0.00 | AAAA |
| ATOM | 1361 | CD | ARG | E | 86 | 28.436 | 106.910 | 26.793 | 1.00 | 0.00 | AAAA |
| ATOM | 1364 | NE | ARG | E | 86 | 29.844 | 106.567 | 26.996 | 1.00 | 0.00 | AAAA |
| ATOM | 1366 | CZ | ARG | E | 86 | 30.529 | 105.447 | 26.730 | 1.00 | 0.00 | AAAA |
| ATOM | 1367 | NH1 | ARG | E | 86 | 29.904 | 104.421 | 26.129 | 1.00 | 0.00 | AAAA |
| ATOM | 1370 | NH2 | ARG | E | 86 | 31.856 | 105.379 | 26.881 | 1.00 | 0.00 | AAAA |
| ATOM | 1373 | C | ARG | E | 86 | 28.280 | 110.260 | 30.291 | 1.00 | 0.00 | AAAA |
| ATOM | 1374 | O | ARG | E | 86 | 29.307 | 110.930 | 30.354 | 1.00 | 0.00 | AAAA |
| ATOM | 1375 | N | LEU | E | 87 | 27.338 | 110.280 | 31.245 | 1.00 | 0.00 | AAAA |
| ATOM | 1377 | CA | LEU | E | 87 | 27.405 | 111.136 | 32.409 | 1.00 | 0.00 | AAAA |
| ATOM | 1379 | CB | LEU | E | 87 | 26.438 | 112.316 | 32.266 | 1.00 | 0.00 | AAAA |
| ATOM | 1382 | CG | LEU | E | 87 | 26.510 | 113.218 | 31.027 | 1.00 | 0.00 | AAAA |
| ATOM | 1384 | CD1 | LEU | E | 87 | 25.228 | 114.029 | 30.836 | 1.00 | 0.00 | AAAA |
| ATOM | 1388 | CD2 | LEU | E | 87 | 27.800 | 114.025 | 31.168 | 1.00 | 0.00 | AAAA |
| ATOM | 1392 | C | LEU | E | 87 | 27.172 | 110.232 | 33.614 | 1.00 | 0.00 | AAAA |
| ATOM | 1393 | O | LEU | E | 87 | 26.515 | 109.198 | 33.568 | 1.00 | 0.00 | AAAA |
| ATOM | 1394 | N | PHE | E | 88 | 27.606 | 110.715 | 34.784 | 1.00 | 0.00 | AAAA |
| ATOM | 1396 | CA | PHE | E | 88 | 27.410 | 110.253 | 36.137 | 1.00 | 0.00 | AAAA |
| ATOM | 1398 | CB | PHE | E | 88 | 28.670 | 110.400 | 36.999 | 1.00 | 0.00 | AAAA |
| ATOM | 1401 | CG | PHE | E | 88 | 28.621 | 109.616 | 38.287 | 1.00 | 0.00 | AAAA |
| ATOM | 1402 | CD1 | PHE | E | 88 | 29.052 | 108.289 | 38.373 | 1.00 | 0.00 | AAAA |
| ATOM | 1404 | CD2 | PHE | E | 88 | 28.362 | 110.377 | 39.436 | 1.00 | 0.00 | AAAA |
| ATOM | 1406 | CE1 | PHE | E | 88 | 29.096 | 107.670 | 39.631 | 1.00 | 0.00 | AAAA |
| ATOM | 1408 | CE2 | PHE | E | 88 | 28.463 | 109.770 | 40.692 | 1.00 | 0.00 | AAAA |
| ATOM | 1410 | CZ | PHE | E | 88 | 28.781 | 108.410 | 40.779 | 1.00 | 0.00 | AAAA |
| ATOM | 1412 | C | PHE | E | 88 | 26.297 | 111.080 | 36.744 | 1.00 | 0.00 | AAAA |
| ATOM | 1413 | O | PHE | E | 88 | 26.535 | 112.220 | 37.155 | 1.00 | 0.00 | AAAA |
| ATOM | 1414 | N | PHE | E | 89 | 25.034 | 110.641 | 36.635 | 1.00 | 0.00 | AAAA |
| ATOM | 1416 | CA | PHE | E | 89 | 23.820 | 111.306 | 37.070 | 1.00 | 0.00 | AAAA |
| ATOM | 1418 | CB | PHE | E | 89 | 23.735 | 111.296 | 38.592 | 1.00 | 0.00 | AAAA |
| ATOM | 1421 | CG | PHE | E | 89 | 23.749 | 109.919 | 39.219 | 1.00 | 0.00 | AAAA |
| ATOM | 1422 | CD1 | PHE | E | 89 | 22.586 | 109.168 | 39.379 | 1.00 | 0.00 | AAAA |
| ATOM | 1424 | CD2 | PHE | E | 89 | 24.989 | 109.414 | 39.644 | 1.00 | 0.00 | AAAA |
| ATOM | 1426 | CE1 | PHE | E | 89 | 22.649 | 107.896 | 39.973 | 1.00 | 0.00 | AAAA |
| ATOM | 1428 | CE2 | PHE | E | 89 | 24.989 | 108.171 | 40.275 | 1.00 | 0.00 | AAAA |
| ATOM | 1430 | CZ | PHE | E | 89 | 23.853 | 107.370 | 40.446 | 1.00 | 0.00 | AAAA |
| ATOM | 1432 | C | PHE | E | 89 | 23.671 | 112.721 | 36.546 | 1.00 | 0.00 | AAAA |
| ATOM | 1433 | O | PHE | E | 89 | 23.060 | 113.597 | 37.158 | 1.00 | 0.00 | AAAA |
| ATOM | 1434 | N | ASN | E | 90 | 24.262 | 113.008 | 35.380 | 1.00 | 0.00 | AAAA |
| ATOM | 1436 | CA | ASN | E | 90 | 24.311 | 114.301 | 34.721 | 1.00 | 0.00 | AAAA |
| ATOM | 1438 | CB | ASN | E | 90 | 22.963 | 115.000 | 34.649 | 1.00 | 0.00 | AAAA |
| ATOM | 1441 | CG | ASN | E | 90 | 22.591 | 115.640 | 33.308 | 1.00 | 0.00 | AAAA |
| ATOM | 1442 | OD1 | ASN | E | 90 | 22.220 | 114.915 | 32.394 | 1.00 | 0.00 | AAAA |
| ATOM | 1443 | ND2 | ASN | E | 90 | 22.885 | 116.902 | 33.039 | 1.00 | 0.00 | AAAA |
| ATOM | 1446 | C | ASN | E | 90 | 25.397 | 115.326 | 35.027 | 1.00 | 0.00 | AAAA |
| ATOM | 1447 | O | ASN | E | 90 | 25.455 | 116.327 | 34.321 | 1.00 | 0.00 | AAAA |
| ATOM | 1448 | N | TYR | E | 91 | 26.255 | 114.992 | 35.987 | 1.00 | 0.00 | AAAA |
| ATOM | 1450 | CA | TYR | E | 91 | 27.437 | 115.776 | 36.276 | 1.00 | 0.00 | AAAA |
| ATOM | 1452 | CB | TYR | E | 91 | 27.787 | 115.522 | 37.737 | 1.00 | 0.00 | AAAA |
| ATOM | 1455 | CG | TYR | E | 91 | 26.672 | 115.865 | 38.699 | 1.00 | 0.00 | AAAA |
| ATOM | 1456 | CD1 | TYR | E | 91 | 26.311 | 117.218 | 38.790 | 1.00 | 0.00 | AAAA |
| ATOM | 1458 | CD2 | TYR | E | 91 | 25.977 | 114.828 | 39.337 | 1.00 | 0.00 | AAAA |
| ATOM | 1460 | CE1 | TYR | E | 91 | 25.222 | 117.520 | 39.616 | 1.00 | 0.00 | AAAA |
| ATOM | 1462 | CE2 | TYR | E | 91 | 24.875 | 115.156 | 40.139 | 1.00 | 0.00 | AAAA |
| ATOM | 1464 | CZ | TYR | E | 91 | 24.545 | 116.507 | 40.295 | 1.00 | 0.00 | AAAA |
| ATOM | 1465 | OH | TYR | E | 91 | 23.484 | 116.869 | 41.084 | 1.00 | 0.00 | AAAA |
| ATOM | 1467 | C | TYR | E | 91 | 28.498 | 115.225 | 35.336 | 1.00 | 0.00 | AAAA |
| ATOM | 1468 | O | TYR | E | 91 | 28.881 | 114.067 | 35.431 | 1.00 | 0.00 | AAAA |
| ATOM | 1469 | N | ALA | E | 92 | 29.066 | 116.020 | 34.419 | 1.00 | 0.00 | AAAA |
| ATOM | 1471 | CA | ALA | E | 92 | 30.269 | 115.649 | 33.688 | 1.00 | 0.00 | AAAA |
| ATOM | 1473 | CB | ALA | E | 92 | 30.490 | 116.664 | 32.569 | 1.00 | 0.00 | AAAA |
| ATOM | 1477 | C | ALA | E | 92 | 31.563 | 115.705 | 34.489 | 1.00 | 0.00 | AAAA |
| ATOM | 1478 | O | ALA | E | 92 | 32.511 | 114.954 | 34.271 | 1.00 | 0.00 | AAAA |
| ATOM | 1479 | N | LEU | E | 93 | 31.666 | 116.568 | 35.503 | 1.00 | 0.00 | AAAA |
| ATOM | 1481 | CA | LEU | E | 93 | 32.729 | 116.705 | 36.481 | 1.00 | 0.00 | AAAA |
| ATOM | 1483 | CB | LEU | E | 93 | 33.332 | 118.109 | 36.382 | 1.00 | 0.00 | AAAA |
| ATOM | 1486 | CG | LEU | E | 93 | 34.666 | 118.303 | 37.099 | 1.00 | 0.00 | AAAA |
| ATOM | 1488 | CD1 | LEU | E | 93 | 35.766 | 117.395 | 36.576 | 1.00 | 0.00 | AAAA |
| ATOM | 1492 | CD2 | LEU | E | 93 | 35.125 | 119.765 | 37.025 | 1.00 | 0.00 | AAAA |
| ATOM | 1496 | C | LEU | E | 93 | 32.124 | 116.350 | 37.834 | 1.00 | 0.00 | AAAA |
| ATOM | 1497 | O | LEU | E | 93 | 31.207 | 117.024 | 38.303 | 1.00 | 0.00 | AAAA |
| ATOM | 1498 | N | VAL | E | 94 | 32.806 | 115.495 | 38.599 | 1.00 | 0.00 | AAAA |
| ATOM | 1500 | CA | VAL | E | 94 | 32.530 | 115.256 | 40.003 | 1.00 | 0.00 | AAAA |
| ATOM | 1502 | CB | VAL | E | 94 | 31.832 | 113.910 | 40.167 | 1.00 | 0.00 | AAAA |
| ATOM | 1504 | CG1 | VAL | E | 94 | 31.487 | 113.579 | 41.620 | 1.00 | 0.00 | AAAA |
| ATOM | 1508 | CG2 | VAL | E | 94 | 30.583 | 113.825 | 39.280 | 1.00 | 0.00 | AAAA |
| ATOM | 1512 | C | VAL | E | 94 | 33.790 | 115.376 | 40.850 | 1.00 | 0.00 | AAAA |
| ATOM | 1513 | O | VAL | E | 94 | 34.710 | 114.567 | 40.706 | 1.00 | 0.00 | AAAA |
| ATOM | 1514 | N | ILE | E | 95 | 33.829 | 116.385 | 41.720 | 1.00 | 0.00 | AAAA |
| ATOM | 1516 | CA | ILE | E | 95 | 34.911 | 116.683 | 42.633 | 1.00 | 0.00 | AAAA |
| ATOM | 1518 | CB | ILE | E | 95 | 35.496 | 118.086 | 42.448 | 1.00 | 0.00 | AAAA |
| ATOM | 1520 | CG1 | ILE | E | 95 | 35.950 | 118.351 | 41.005 | 1.00 | 0.00 | AAAA |
| ATOM | 1523 | CG2 | ILE | E | 95 | 36.515 | 118.549 | 43.479 | 1.00 | 0.00 | AAAA |
| ATOM | 1527 | CD1 | ILE | E | 95 | 37.221 | 117.644 | 40.537 | 1.00 | 0.00 | AAAA |
| ATOM | 1531 | C | ILE | E | 95 | 34.258 | 116.537 | 44.000 | 1.00 | 0.00 | AAAA |
| ATOM | 1532 | O | ILE | E | 95 | 33.760 | 117.501 | 44.568 | 1.00 | 0.00 | AAAA |
| ATOM | 1533 | N | PHE | E | 96 | 34.288 | 115.272 | 44.433 | 1.00 | 0.00 | AAAA |
| ATOM | 1535 | CA | PHE | E | 96 | 33.537 | 114.837 | 45.593 | 1.00 | 0.00 | AAAA |
| ATOM | 1537 | CB | PHE | E | 96 | 32.661 | 113.739 | 44.990 | 1.00 | 0.00 | AAAA |
| ATOM | 1540 | CG | PHE | E | 96 | 31.666 | 113.137 | 45.968 | 1.00 | 0.00 | AAAA |
| ATOM | 1541 | CD1 | PHE | E | 96 | 30.573 | 113.857 | 46.446 | 1.00 | 0.00 | AAAA |
| ATOM | 1543 | CD2 | PHE | E | 96 | 31.912 | 111.872 | 46.517 | 1.00 | 0.00 | AAAA |
| ATOM | 1545 | CE1 | PHE | E | 96 | 29.805 | 113.316 | 47.480 | 1.00 | 0.00 | AAAA |
| ATOM | 1547 | CE2 | PHE | E | 96 | 31.115 | 111.283 | 47.516 | 1.00 | 0.00 | AAAA |
| ATOM | 1549 | CZ | PHE | E | 96 | 30.036 | 112.032 | 48.014 | 1.00 | 0.00 | AAAA |
| ATOM | 1551 | C | PHE | E | 96 | 34.481 | 114.281 | 46.646 | 1.00 | 0.00 | AAAA |
| ATOM | 1552 | O | PHE | E | 96 | 35.366 | 113.489 | 46.307 | 1.00 | 0.00 | AAAA |
| ATOM | 1553 | N | GLU | E | 97 | 34.375 | 114.667 | 47.917 | 1.00 | 0.00 | AAAA |
| ATOM | 1555 | CA | GLU | E | 97 | 35.000 | 114.142 | 49.117 | 1.00 | 0.00 | AAAA |
| ATOM | 1557 | CB | GLU | E | 97 | 34.626 | 112.685 | 49.379 | 1.00 | 0.00 | AAAA |
| ATOM | 1560 | CG | GLU | E | 97 | 35.236 | 111.966 | 50.574 | 1.00 | 0.00 | AAAA |
| ATOM | 1563 | CD | GLU | E | 97 | 34.511 | 112.339 | 51.856 | 1.00 | 0.00 | AAAA |
| ATOM | 1564 | OE1 | GLU | E | 97 | 35.136 | 112.994 | 52.713 | 1.00 | 0.00 | AAAA |
| ATOM | 1565 | OE2 | GLU | E | 97 | 33.426 | 111.755 | 52.082 | 1.00 | 0.00 | AAAA |
| ATOM | 1566 | C | GLU | E | 97 | 36.508 | 114.396 | 49.122 | 1.00 | 0.00 | AAAA |
| ATOM | 1567 | O | GLU | E | 97 | 37.263 | 113.597 | 48.565 | 1.00 | 0.00 | AAAA |
| ATOM | 1568 | N | MET | E | 98 | 36.973 | 115.543 | 49.631 | 1.00 | 0.00 | AAAA |
| ATOM | 1570 | CA | MET | E | 98 | 38.372 | 115.904 | 49.594 | 1.00 | 0.00 | AAAA |
| ATOM | 1572 | CB | MET | E | 98 | 38.704 | 116.722 | 48.354 | 1.00 | 0.00 | AAAA |
| ATOM | 1575 | CG | MET | E | 98 | 39.648 | 116.006 | 47.388 | 1.00 | 0.00 | AAAA |
| ATOM | 1578 | SD | MET | E | 98 | 39.506 | 116.547 | 45.669 | 1.00 | 0.00 | AAAA |
| ATOM | 1579 | CE | MET | E | 98 | 38.007 | 115.631 | 45.242 | 1.00 | 0.00 | AAAA |
| ATOM | 1583 | C | MET | E | 98 | 38.688 | 116.680 | 50.877 | 1.00 | 0.00 | AAAA |
| ATOM | 1584 | O | MET | E | 98 | 37.794 | 117.414 | 51.301 | 1.00 | 0.00 | AAAA |
| ATOM | 1585 | N | VAL | E | 99 | 39.860 | 116.532 | 51.488 | 1.00 | 0.00 | AAAA |
| ATOM | 1587 | CA | VAL | E | 99 | 40.523 | 117.402 | 52.436 | 1.00 | 0.00 | AAAA |
| ATOM | 1589 | CB | VAL | E | 99 | 40.732 | 116.743 | 53.804 | 1.00 | 0.00 | AAAA |
| ATOM | 1591 | CG1 | VAL | E | 99 | 39.442 | 116.446 | 54.579 | 1.00 | 0.00 | AAAA |
| ATOM | 1595 | CG2 | VAL | E | 99 | 41.604 | 115.499 | 53.882 | 1.00 | 0.00 | AAAA |
| ATOM | 1599 | C | VAL | E | 99 | 41.911 | 117.706 | 51.889 | 1.00 | 0.00 | AAAA |
| ATOM | 1600 | O | VAL | E | 99 | 42.436 | 116.998 | 51.040 | 1.00 | 0.00 | AAAA |
| ATOM | 1601 | N | HIS | E | 100 | 42.355 | 118.889 | 52.332 | 1.00 | 0.00 | AAAA |
| ATOM | 1603 | CA | HIS | E | 100 | 43.620 | 119.558 | 52.124 | 1.00 | 0.00 | AAAA |
| ATOM | 1605 | CB | HIS | E | 100 | 44.792 | 118.582 | 52.184 | 1.00 | 0.00 | AAAA |
| ATOM | 1608 | CG | HIS | E | 100 | 46.153 | 119.227 | 52.202 | 1.00 | 0.00 | AAAA |
| ATOM | 1609 | ND1 | HIS | E | 100 | 46.477 | 120.242 | 53.104 | 1.00 | 0.00 | AAAA |
| ATOM | 1610 | CD2 | HIS | E | 100 | 47.056 | 119.324 | 51.182 | 1.00 | 0.00 | AAAA |
| ATOM | 1612 | CE1 | HIS | E | 100 | 47.536 | 120.906 | 52.622 | 1.00 | 0.00 | AAAA |
| ATOM | 1614 | NE2 | HIS | E | 100 | 48.005 | 120.251 | 51.547 | 1.00 | 0.00 | AAAA |
| ATOM | 1616 | C | HIS | E | 100 | 43.590 | 120.474 | 50.911 | 1.00 | 0.00 | AAAA |
| ATOM | 1617 | O | HIS | E | 100 | 44.309 | 121.462 | 50.794 | 1.00 | 0.00 | AAAA |
| ATOM | 1618 | N | LEU | E | 101 | 42.636 | 120.190 | 50.007 | 1.00 | 0.00 | AAAA |
| ATOM | 1620 | CA | LEU | E | 101 | 42.438 | 120.955 | 48.795 | 1.00 | 0.00 | AAAA |
| ATOM | 1622 | CB | LEU | E | 101 | 41.358 | 120.247 | 47.983 | 1.00 | 0.00 | AAAA |
| ATOM | 1625 | CG | LEU | E | 101 | 41.016 | 120.826 | 46.613 | 1.00 | 0.00 | AAAA |
| ATOM | 1627 | CD1 | LEU | E | 101 | 41.845 | 120.256 | 45.465 | 1.00 | 0.00 | AAAA |
| ATOM | 1631 | CD2 | LEU | E | 101 | 39.515 | 120.650 | 46.341 | 1.00 | 0.00 | AAAA |
| ATOM | 1635 | C | LEU | E | 101 | 42.019 | 122.380 | 49.148 | 1.00 | 0.00 | AAAA |
| ATOM | 1636 | O | LEU | E | 101 | 41.124 | 122.565 | 49.967 | 1.00 | 0.00 | AAAA |
| ATOM | 1637 | N | LYS | E | 102 | 42.730 | 123.385 | 48.630 | 1.00 | 0.00 | AAAA |
| ATOM | 1639 | CA | LYS | E | 102 | 42.729 | 124.774 | 49.037 | 1.00 | 0.00 | AAAA |
| ATOM | 1641 | CB | LYS | E | 102 | 43.939 | 125.126 | 49.890 | 1.00 | 0.00 | AAAA |
| ATOM | 1644 | CG | LYS | E | 102 | 45.287 | 125.115 | 49.154 | 1.00 | 0.00 | AAAA |
| ATOM | 1647 | CD | LYS | E | 102 | 46.169 | 126.291 | 49.566 | 1.00 | 0.00 | AAAA |
| ATOM | 1650 | CE | LYS | E | 102 | 47.467 | 126.295 | 48.745 | 1.00 | 0.00 | AAAA |
| ATOM | 1653 | NZ | LYS | E | 102 | 48.329 | 127.401 | 49.198 | 1.00 | 0.00 | AAAA |
| ATOM | 1657 | C | LYS | E | 102 | 42.479 | 125.726 | 47.876 | 1.00 | 0.00 | AAAA |
| ATOM | 1658 | O | LYS | E | 102 | 42.084 | 126.875 | 48.024 | 1.00 | 0.00 | AAAA |
| ATOM | 1659 | N | GLU | E | 103 | 42.865 | 125.365 | 46.642 | 1.00 | 0.00 | AAAA |
| ATOM | 1661 | CA | GLU | E | 103 | 42.539 | 126.008 | 45.393 | 1.00 | 0.00 | AAAA |
| ATOM | 1663 | CB | GLU | E | 103 | 43.561 | 127.065 | 44.993 | 1.00 | 0.00 | AAAA |
| ATOM | 1666 | CG | GLU | E | 103 | 45.031 | 126.773 | 45.282 | 1.00 | 0.00 | AAAA |
| ATOM | 1669 | CD | GLU | E | 103 | 45.875 | 127.937 | 45.819 | 1.00 | 0.00 | AAAA |
| ATOM | 1670 | OE1 | GLU | E | 103 | 45.335 | 128.895 | 46.417 | 1.00 | 0.00 | AAAA |
| ATOM | 1671 | OE2 | GLU | E | 103 | 47.101 | 127.771 | 45.683 | 1.00 | 0.00 | AAAA |
| ATOM | 1672 | C | GLU | E | 103 | 42.358 | 124.822 | 44.450 | 1.00 | 0.00 | AAAA |
| ATOM | 1673 | O | GLU | E | 103 | 43.020 | 123.794 | 44.511 | 1.00 | 0.00 | AAAA |
| ATOM | 1674 | N | LEU | E | 104 | 41.393 | 124.930 | 43.536 | 1.00 | 0.00 | AAAA |
| ATOM | 1676 | CA | LEU | E | 104 | 40.992 | 123.847 | 42.667 | 1.00 | 0.00 | AAAA |
| ATOM | 1678 | CB | LEU | E | 104 | 39.669 | 124.215 | 41.997 | 1.00 | 0.00 | AAAA |
| ATOM | 1681 | CG | LEU | E | 104 | 39.122 | 123.146 | 41.049 | 1.00 | 0.00 | AAAA |
| ATOM | 1683 | CD1 | LEU | E | 104 | 38.871 | 121.791 | 41.708 | 1.00 | 0.00 | AAAA |
| ATOM | 1687 | CD2 | LEU | E | 104 | 37.833 | 123.585 | 40.339 | 1.00 | 0.00 | AAAA |
| ATOM | 1691 | C | LEU | E | 104 | 42.058 | 123.373 | 41.685 | 1.00 | 0.00 | AAAA |
| ATOM | 1692 | O | LEU | E | 104 | 42.534 | 122.251 | 41.866 | 1.00 | 0.00 | AAAA |
| ATOM | 1693 | N | GLY | E | 105 | 42.511 | 124.193 | 40.743 | 1.00 | 0.00 | AAAA |
| ATOM | 1695 | CA | GLY | E | 105 | 43.666 | 123.883 | 39.918 | 1.00 | 0.00 | AAAA |
| ATOM | 1698 | C | GLY | E | 105 | 43.416 | 123.742 | 38.428 | 1.00 | 0.00 | AAAA |
| ATOM | 1699 | O | GLY | E | 105 | 44.350 | 123.922 | 37.658 | 1.00 | 0.00 | AAAA |
| ATOM | 1700 | N | LEU | E | 106 | 42.202 | 123.295 | 38.085 | 1.00 | 0.00 | AAAA |
| ATOM | 1702 | CA | LEU | E | 106 | 41.785 | 122.869 | 36.763 | 1.00 | 0.00 | AAAA |
| ATOM | 1704 | CB | LEU | E | 106 | 40.705 | 121.811 | 37.019 | 1.00 | 0.00 | AAAA |
| ATOM | 1707 | CG | LEU | E | 106 | 40.517 | 120.887 | 35.821 | 1.00 | 0.00 | AAAA |
| ATOM | 1709 | CD1 | LEU | E | 106 | 41.641 | 119.862 | 35.723 | 1.00 | 0.00 | AAAA |
| ATOM | 1713 | CD2 | LEU | E | 106 | 39.176 | 120.152 | 35.876 | 1.00 | 0.00 | AAAA |
| ATOM | 1717 | C | LEU | E | 106 | 41.363 | 124.028 | 35.867 | 1.00 | 0.00 | AAAA |
| ATOM | 1718 | O | LEU | E | 106 | 40.240 | 124.107 | 35.383 | 1.00 | 0.00 | AAAA |
| ATOM | 1719 | N | TYR | E | 107 | 42.232 | 125.020 | 35.654 | 1.00 | 0.00 | AAAA |
| ATOM | 1721 | CA | TYR | E | 107 | 41.924 | 126.261 | 34.959 | 1.00 | 0.00 | AAAA |
| ATOM | 1723 | CB | TYR | E | 107 | 42.822 | 127.392 | 35.465 | 1.00 | 0.00 | AAAA |
| ATOM | 1726 | CG | TYR | E | 107 | 44.302 | 127.118 | 35.589 | 1.00 | 0.00 | AAAA |
| ATOM | 1727 | CD1 | TYR | E | 107 | 45.170 | 127.127 | 34.483 | 1.00 | 0.00 | AAAA |
| ATOM | 1729 | CD2 | TYR | E | 107 | 44.882 | 127.091 | 36.866 | 1.00 | 0.00 | AAAA |
| ATOM | 1731. | CE1 | TYR | E | 107 | 46.539 | 126.918 | 34.670 | 1.00 | 0.00 | AAAA |
| ATOM | 1733 | CE2 | TYR | E | 107 | 46.254 | 126.886 | 37.078 | 1.00 | 0.00 | AAAA |
| ATOM | 1735 | CZ | TYR | E | 107 | 47.078 | 126.762 | 35.960 | 1.00 | 0.00 | AAAA |
| ATOM | 1736 | OH | TYR | E | 107 | 48.420 | 126.550 | 36.105 | 1.00 | 0.00 | AAAA |
| ATOM | 1738 | C | TYR | E | 107 | 42.091 | 126.015 | 33.465 | 1.00 | 0.00 | AAAA |
| ATOM | 1739 | O | TYR | E | 107 | 41.451 | 126.704 | 32.684 | 1.00 | 0.00 | AAAA |
| ATOM | 1740 | N | ASN | E | 108 | 42.861 | 124.967 | 33.161 | 1.00 | 0.00 | AAAA |
| ATOM | 1742 | CA | ASN | E | 108 | 43.095 | 124.393 | 31.857 | 1.00 | 0.00 | AAAA |
| ATOM | 1744 | CB | ASN | E | 108 | 44.150 | 123.293 | 31.972 | 1.00 | 0.00 | AAAA |
| ATOM | 1747 | CG | ASN | E | 108 | 43.787 | 122.300 | 33.060 | 1.00 | 0.00 | AAAA |
| ATOM | 1748 | OD1 | ASN | E | 108 | 44.105 | 122.473 | 34.233 | 1.00 | 0.00 | AAAA |
| ATOM | 1749 | ND2 | ASN | E | 108 | 43.130 | 121.182 | 32.755 | 1.00 | 0.00 | AAAA |
| ATOM | 1752 | C | ASN | E | 108 | 41.887 | 123.712 | 31.229 | 1.00 | 0.00 | AAAA |
| ATOM | 1753 | O | ASN | E | 108 | 41.803 | 123.587 | 30.020 | 1.00 | 0.00 | AAAA |
| ATOM | 1754 | N | LEU | E | 109 | 40.880 | 123.299 | 32.019 | 1.00 | 0.00 | AAAA |
| ATOM | 1756 | CA | LEU | E | 109 | 39.619 | 122.831 | 31.496 | 1.00 | 0.00 | AAAA |
| ATOM | 1758 | CB | LEU | E | 109 | 38.842 | 122.083 | 32.582 | 1.00 | 0.00 | AAAA |
| ATOM | 1761 | CG | LEU | E | 109 | 37.469 | 121.502 | 32.223 | 1.00 | 0.00 | AAAA |
| ATOM | 1763 | CD1 | LEU | E | 109 | 37.279 | 120.776 | 30.894 | 1.00 | 0.00 | AAAA |
| ATOM | 1767 | CD2 | LEU | E | 109 | 36.918 | 120.585 | 33.321 | 1.00 | 0.00 | AAAA |
| ATOM | 1771 | C | LEU | E | 109 | 38.779 | 123.933 | 30.868 | 1.00 | 0.00 | AAAA |
| ATOM | 1772 | O | LEU | E | 109 | 38.227 | 124.825 | 31.502 | 1.00 | 0.00 | AAAA |
| ATOM | 1773 | N | MET | E | 110 | 38.822 | 123.859 | 29.536 | 1.00 | 0.00 | AAAA |
| ATOM | 1775 | CA | MET | E | 110 | 38.466 | 124.928 | 28.629 | 1.00 | 0.00 | AAAA |
| ATOM | 1777 | CB | MET | E | 110 | 39.278 | 124.831 | 27.337 | 1.00 | 0.00 | AAAA |
| ATOM | 1780 | CG | MET | E | 110 | 39.088 | 125.979 | 26.346 | 1.00 | 0.00 | AAAA |
| ATOM | 1783 | SD | MET | E | 110 | 40.436 | 126.096 | 25.133 | 1.00 | 0.00 | AAAA |
| ATOM | 1784 | CE | MET | E | 110 | 39.902 | 127.490 | 24.113 | 1.00 | 0.00 | AAAA |
| ATOM | 1788 | C | MET | E | 110 | 36.966 | 124.916 | 28.380 | 1.00 | 0.00 | AAAA |
| ATOM | 1789 | O | MET | E | 110 | 36.380 | 125.997 | 28.371 | 1.00 | 0.00 | AAAA |
| ATOM | 1790 | N | ASN | E | 111 | 36.436 | 123.729 | 28.073 | 1.00 | 0.00 | AAAA |
| ATOM | 1792 | CA | ASN | E | 111 | 35.070 | 123.484 | 27.657 | 1.00 | 0.00 | AAAA |
| ATOM | 1794 | CB | ASN | E | 111 | 35.107 | 123.327 | 26.133 | 1.00 | 0.00 | AAAA |
| ATOM | 1797 | CG | ASN | E | 111 | 33.800 | 123.137 | 25.384 | 1.00 | 0.00 | AAAA |
| ATOM | 1798 | OD1 | ASN | E | 111 | 33.407 | 122.035 | 25.028 | 1.00 | 0.00 | AAAA |
| ATOM | 1799 | ND2 | ASN | E | 111 | 33.024 | 124.218 | 25.240 | 1.00 | 0.00 | AAAA |
| ATOM | 1802 | C | ASN | E | 111 | 34.485 | 122.276 | 28.378 | 1.00 | 0.00 | AAAA |
| ATOM | 1803 | O | ASN | E | 111 | 34.944 | 121.142 | 28.298 | 1.00 | 0.00 | AAAA |
| ATOM | 1804 | N | ILE | E | 112 | 33.342 | 122.463 | 29.053 | 1.00 | 0.00 | AAAA |
| ATOM | 1806 | CA | ILE | E | 112 | 32.426 | 121.414 | 29.465 | 1.00 | 0.00 | AAAA |
| ATOM | 1808 | CB | ILE | E | 112 | 32.215 | 121.540 | 30.974 | 1.00 | 0.00 | AAAA |
| ATOM | 1810 | CG1 | ILE | E | 112 | 33.501 | 121.459 | 31.798 | 1.00 | 0.00 | AAAA |
| ATOM | 1813 | CG2 | ILE | E | 112 | 31.239 | 120.487 | 31.503 | 1.00 | 0.00 | AAAA |
| ATOM | 1817 | CD1 | ILE | E | 112 | 33.213 | 121.586 | 33.288 | 1.00 | 0.00 | AAAA |
| ATOM | 1821 | C | ILE | E | 112 | 31.124 | 121.583 | 28.690 | 1.00 | 0.00 | AAAA |
| ATOM | 1822 | O | ILE | E | 112 | 30.583 | 122.675 | 28.526 | 1.00 | 0.00 | AAAA |
| ATOM | 1823 | N | THR | E | 113 | 30.728 | 120.419 | 28.178 | 1.00 | 0.00 | AAAA |
| ATOM | 1825 | CA | THR | E | 113 | 29.511 | 120.149 | 27.441 | 1.00 | 0.00 | AAAA |
| ATOM | 1827 | CB | THR | E | 113 | 29.817 | 120.115 | 25.939 | 1.00 | 0.00 | AAAA |
| ATOM | 1829 | OG1 | THR | E | 113 | 30.957 | 119.348 | 25.618 | 1.00 | 0.00 | AAAA |
| ATOM | 1831 | CG2 | THR | E | 113 | 30.022 | 121.438 | 25.209 | 1.00 | 0.00 | AAAA |
| ATOM | 1835 | C | THR | E | 113 | 28.780 | 118.982 | 28.099 | 1.00 | 0.00 | AAAA |
| ATOM | 1836 | O | THR | E | 113 | 29.290 | 118.168 | 28.858 | 1.00 | 0.00 | AAAA |
| ATOM | 1837 | N | ARG | E | 114 | 27.458 | 119.009 | 27.913 | 1.00 | 0.00 | AAAA |
| ATOM | 1839 | CA | ARG | E | 114 | 26.467 | 117.975 | 28.146 | 1.00 | 0.00 | AAAA |
| ATOM | 1841 | CB | ARG | E | 114 | 26.777 | 116.628 | 27.485 | 1.00 | 0.00 | AAAA |
| ATOM | 1844 | CG | ARG | E | 114 | 26.425 | 116.687 | 25.998 | 1.00 | 0.00 | AAAA |
| ATOM | 1847 | CD | ARG | E | 114 | 26.795 | 115.411 | 25.242 | 1.00 | 0.00 | AAAA |
| ATOM | 1850 | NE | ARG | E | 114 | 28.070 | 115.596 | 24.550 | 1.00 | 0.00 | AAAA |
| ATOM | 1852 | CZ | ARG | E | 114 | 28.563 | 114.754 | 23.629 | 1.00 | 0.00 | AAAA |
| ATOM | 1853 | NH1 | ARG | E | 114 | 27.824 | 113.716 | 23.209 | 1.00 | 0.00 | AAAA |
| ATOM | 1856 | NH2 | ARG | E | 114 | 29.818 | 114.967 | 23.228 | 1.00 | 0.00 | AAAA |
| ATOM | 1859 | C | ARG | E | 114 | 26.051 | 117.790 | 29.597 | 1.00 | 0.00 | AAAA |
| ATOM | 1860 | O | ARG | E | 114 | 24.865 | 117.583 | 29.839 | 1.00 | 0.00 | AAAA |
| ATOM | 1861 | N | GLY | E | 115 | 26.966 | 117.858 | 30.560 | 1.00 | 0.00 | AAAA |
| ATOM | 1863 | CA | GLY | E | 115 | 26.666 | 117.770 | 31.979 | 1.00 | 0.00 | AAAA |
| ATOM | 1866 | C | GLY | E | 115 | 27.201 | 118.974 | 32.743 | 1.00 | 0.00 | AAAA |
| ATOM | 1867 | O | GLY | E | 115 | 27.875 | 119.806 | 32.140 | 1.00 | 0.00 | AAAA |
| ATOM | 1868 | N | SER | E | 116 | 26.892 | 119.079 | 34.035 | 1.00 | 0.00 | AAAA |
| ATOM | 1870 | CA | SER | E | 116 | 27.252 | 120.211 | 34.853 | 1.00 | 0.00 | AAAA |
| ATOM | 1872 | CB | SER | E | 116 | 26.036 | 120.756 | 35.594 | 1.00 | 0.00 | AAAA |
| ATOM | 1875 | OG | SER | E | 116 | 25.463 | 119.804 | 36.466 | 1.00 | 0.00 | AAAA |
| ATOM | 1877 | C | SER | E | 116 | 28.440 | 119.830 | 35.730 | 1.00 | 0.00 | AAAA |
| ATOM | 1878 | O | SER | E | 116 | 28.964 | 118.725 | 35.594 | 1.00 | 0.00 | AAAA |
| ATOM | 1879 | N | VAL | E | 117 | 28.820 | 120.684 | 36.680 | 1.00 | 0.00 | AAAA |
| ATOM | 1881 | CA | VAL | E | 117 | 29.871 | 120.446 | 37.664 | 1.00 | 0.00 | AAAA |
| ATOM | 1883 | CB | VAL | E | 117 | 30.829 | 121.618 | 37.847 | 1.00 | 0.00 | AAAA |
| ATOM | 1885 | CG1 | VAL | E | 117 | 31.989 | 121.287 | 38.788 | 1.00 | 0.00 | AAAA |
| ATOM | 1889 | CG2 | VAL | E | 117 | 31.351 | 122.107 | 36.487 | 1.00 | 0.00 | AAAA |
| ATOM | 1893 | C | VAL | E | 117 | 29.161 | 120.233 | 39.000 | 1.00 | 0.00 | AAAA |
| ATOM | 1894 | O | VAL | E | 117 | 28.289 | 121.028 | 39.342 | 1.00 | 0.00 | AAAA |
| ATOM | 1895 | N | ARG | E | 118 | 29.611 | 119.198 | 39.709 | 1.00 | 0.00 | AAAA |
| ATOM | 1897 | CA | ARG | E | 118 | 29.429 | 118.983 | 41.135 | 1.00 | 0.00 | AAAA |
| ATOM | 1899 | CB | ARG | E | 118 | 28.765 | 117.656 | 41.513 | 1.00 | 0.00 | AAAA |
| ATOM | 1902 | CG | ARG | E | 118 | 28.211 | 117.464 | 42.926 | 1.00 | 0.00 | AAAA |
| ATOM | 1905 | CD | ARG | E | 118 | 26.700 | 117.229 | 42.889 | 1.00 | 0.00 | AAAA |
| ATOM | 1908 | NE | ARG | E | 118 | 26.190 | 117.062 | 44.249 | 1.00 | 0.00 | AAAA |
| ATOM | 1910 | CZ | ARG | E | 118 | 24.932 | 116.894 | 44.671 | 1.00 | 0.00 | AAAA |
| ATOM | 1911 | NH1 | ARG | E | 118 | 23.871 | 117.006 | 43.863 | 1.00 | 0.00 | AAAA |
| ATOM | 1914 | NH2 | ARG | E | 118 | 24.643 | 116.545 | 45.933 | 1.00 | 0.00 | AAAA |
| ATOM | 1917 | C | ARG | E | 118 | 30.801 | 119.066 | 41.785 | 1.00 | 0.00 | AAAA |
| ATOM | 1918 | O | ARG | E | 118 | 31.637 | 118.186 | 41.568 | 1.00 | 0.00 | AAAA |
| ATOM | 1919 | N | ILE | E | 119 | 31.020 | 120.143 | 42.548 | 1.00 | 0.00 | AAAA |
| ATOM | 1921 | CA | ILE | E | 119 | 32.012 | 120.401 | 43.569 | 1.00 | 0.00 | AAAA |
| ATOM | 1923 | CB | ILE | E | 119 | 32.468 | 121.843 | 43.346 | 1.00 | 0.00 | AAAA |
| ATOM | 1925 | CG1 | ILE | E | 119 | 33.405 | 121.917 | 42.136 | 1.00 | 0.00 | AAAA |
| ATOM | 1928 | CG2 | ILE | E | 119 | 33.134 | 122.428 | 44.585 | 1.00 | 0.00 | AAAA |
| ATOM | 1932 | CD1 | ILE | E | 119 | 33.265 | 123.240 | 41.382 | 1.00 | 0.00 | AAAA |
| ATOM | 1936 | C | ILE | E | 119 | 31.370 | 120.144 | 44.925 | 1.00 | 0.00 | AAAA |
| ATOM | 1937 | O | ILE | E | 119 | 30.422 | 120.871 | 45.226 | 1.00 | 0.00 | AAAA |
| ATOM | 1938 | N | GLU | E | 120 | 31.739 | 119.088 | 45.651 | 1.00 | 0.00 | AAAA |
| ATOM | 1940 | CA | GLU | E | 120 | 31.065 | 118.665 | 46.858 | 1.00 | 0.00 | AAAA |
| ATOM | 1942 | CB | GLU | E | 120 | 29.847 | 117.805 | 46.509 | 1.00 | 0.00 | AAAA |
| ATOM | 1945 | CG | GLU | E | 120 | 29.171 | 117.233 | 47.758 | 1.00 | 0.00 | AAAA |
| ATOM | 1948 | CD | GLU | E | 120 | 27.750 | 116.690 | 47.674 | 1.00 | 0.00 | AAAA |
| ATOM | 1949 | OE1 | GLU | E | 120 | 27.213 | 116.608 | 46.548 | 1.00 | 0.00 | AAAA |
| ATOM | 1950 | OE2 | GLU | E | 120 | 27.199 | 116.473 | 48.771 | 1.00 | 0.00 | AAAA |
| ATOM | 1951 | C | GLU | E | 120 | 31.993 | 118.157 | 47.953 | 1.00 | 0.00 | AAAA |
| ATOM | 1952 | O | GLU | E | 120 | 32.660 | 117.149 | 47.728 | 1.00 | 0.00 | AAAA |
| ATOM | 1953 | N | LYS | E | 121 | 32.130 | 118.731 | 49.151 | 1.00 | 0.00 | AAAA |
| ATOM | 1955 | CA | LYS | E | 121 | 32.749 | 118.166 | 50.337 | 1.00 | 0.00 | AAAA |
| ATOM | 1957 | CB | LYS | E | 121 | 32.230 | 116.754 | 50.635 | 1.00 | 0.00 | AAAA |
| ATOM | 1960 | CG | LYS | E | 121 | 32.334 | 116.331 | 52.100 | 1.00 | 0.00 | AAAA |
| ATOM | 1963 | CD | LYS | E | 121 | 31.847 | 114.921 | 52.450 | 1.00 | 0.00 | AAAA |
| ATOM | 1966 | CE | LYS | E | 121 | 32.250 | 114.562 | 53.870 | 1.00 | 0.00 | AAAA |
| ATOM | 1969 | NZ | LYS | E | 121 | 32.148 | 113.115 | 54.111 | 1.00 | 0.00 | AAAA |
| ATOM | 1973 | C | LYS | E | 121 | 34.261 | 118.320 | 50.250 | 1.00 | 0.00 | AAAA |
| ATOM | 1974 | O | LYS | E | 121 | 35.012 | 117.344 | 50.304 | 1.00 | 0.00 | AAAA |
| ATOM | 1975 | N | ASN | E | 122 | 34.692 | 119.517 | 49.862 | 1.00 | 0.00 | AAAA |
| ATOM | 1977 | CA | ASN | E | 122 | 36.065 | 119.889 | 49.583 | 1.00 | 0.00 | AAAA |
| ATOM | 1979 | CB | ASN | E | 122 | 36.192 | 120.583 | 48.226 | 1.00 | 0.00 | AAAA |
| ATOM | 1982 | CG | ASN | E | 122 | 35.820 | 119.784 | 46.979 | 1.00 | 0.00 | AAAA |
| ATOM | 1983 | OD1 | ASN | E | 122 | 35.836 | 120.327 | 45.879 | 1.00 | 0.00 | AAAA |
| ATOM | 1984 | ND2 | ASN | E | 122 | 35.516 | 118.484 | 47.040 | 1.00 | 0.00 | AAAA |
| ATOM | 1987 | C | ASN | E | 122 | 36.478 | 120.772 | 50.750 | 1.00 | 0.00 | AAAA |
| ATOM | 1988 | O | ASN | E | 122 | 36.377 | 122.001 | 50.723 | 1.00 | 0.00 | AAAA |
| ATOM | 1989 | N | ASN | E | 123 | 36.958 | 120.156 | 51.830 | 1.00 | 0.00 | AAAA |
| ATOM | 1991 | CA | ASN | E | 123 | 37.376 | 120.837 | 53.039 | 1.00 | 0.00 | AAAA |
| ATOM | 1993 | CB | ASN | E | 123 | 37.587 | 119.910 | 54.232 | 1.00 | 0.00 | AAAA |
| ATOM | 1996 | CG | ASN | E | 123 | 37.744 | 120.593 | 55.580 | 1.00 | 0.00 | AAAA |
| ATOM | 1997 | OD1 | ASN | E | 123 | 38.865 | 120.833 | 56.022 | 1.00 | 0.00 | AAAA |
| ATOM | 1998 | ND2 | ASN | E | 123 | 36.648 | 121.040 | 56.195 | 1.00 | 0.00 | AAAA |
| ATOM | 2001 | C | ASN | E | 123 | 38.658 | 121.622 | 52.773 | 1.00 | 0.00 | AAAA |
| ATOM | 2002 | O | ASN | E | 123 | 39.667 | 120.984 | 52.503 | 1.00 | 0.00 | AAAA |
| ATOM | 2003 | N | GLU | E | 124 | 38.612 | 122.939 | 52.918 | 1.00 | 0.00 | AAAA |
| ATOM | 2005 | CA | GLU | E | 124 | 39.684 | 123.916 | 52.793 | 1.00 | 0.00 | AAAA |
| ATOM | 2007 | CB | GLU | E | 124 | 41.067 | 123.497 | 53.313 | 1.00 | 0.00 | AAAA |
| ATOM | 2010 | CG | GLU | E | 124 | 41.168 | 123.366 | 54.834 | 1.00 | 0.00 | AAAA |
| ATOM | 2013 | CD | GLU | E | 124 | 41.374 | 124.750 | 55.439 | 1.00 | 0.00 | AAAA |
| ATOM | 2014 | OE1 | GLU | E | 124 | 40.467 | 125.257 | 56.126 | 1.00 | 0.00 | AAAA |
| ATOM | 2015 | OE2 | GLU | E | 124 | 42.389 | 125.403 | 55.148 | 1.00 | 0.00 | AAAA |
| ATOM | 2016 | C | GLU | E | 124 | 39.675 | 124.701 | 51.496 | 1.00 | 0.00 | AAAA |
| ATOM | 2017 | O | GLU | E | 124 | 40.462 | 125.644 | 51.435 | 1.00 | 0.00 | AAAA |
| ATOM | 2018 | N | LEU | E | 125 | 38.841 | 124.359 | 50.511 | 1.00 | 0.00 | AAAA |
| ATOM | 2020 | CA | LEU | E | 125 | 38.863 | 124.939 | 49.176 | 1.00 | 0.00 | AAAA |
| ATOM | 2022 | CB | LEU | E | 125 | 38.179 | 123.945 | 48.238 | 1.00 | 0.00 | AAAA |
| ATOM | 2025 | CG | LEU | E | 125 | 37.991 | 124.311 | 46.763 | 1.00 | 0.00 | AAAA |
| ATOM | 2027 | CD1 | LEU | E | 125 | 39.323 | 124.467 | 46.026 | 1.00 | 0.00 | AAAA |
| ATOM | 2031 | CD2 | LEU | E | 125 | 37.004 | 123.421 | 46.021 | 1.00 | 0.00 | AAAA |
| ATOM | 2035 | C | LEU | E | 125 | 38.196 | 126.305 | 49.042 | 1.00 | 0.00 | AAAA |
| ATOM | 2036 | O | LEU | E | 125 | 37.071 | 126.485 | 49.493 | 1.00 | 0.00 | AAAA |
| ATOM | 2037 | N | CYS | E | 126 | 38.820 | 127.219 | 48.301 | 1.00 | 0.00 | AAAA |
| ATOM | 2039 | CA | CYS | E | 126 | 38.316 | 128.551 | 48.033 | 1.00 | 0.00 | AAAA |
| ATOM | 2041 | CB | CYS | E | 126 | 38.683 | 129.566 | 49.115 | 1.00 | 0.00 | AAAA |
| ATOM | 2044 | SG | CYS | E | 126 | 40.344 | 129.668 | 49.831 | 1.00 | 0.00 | AAAA |
| ATOM | 2045 | C | CYS | E | 126 | 38.842 | 129.007 | 46.677 | 1.00 | 0.00 | AAAA |
| ATOM | 2046 | O | CYS | E | 126 | 39.725 | 128.317 | 46.168 | 1.00 | 0.00 | AAAA |
| ATOM | 2047 | N | TYR | E | 127 | 38.363 | 130.119 | 46.125 | 1.00 | 0.00 | AAAA |
| ATOM | 2049 | CA | TYR | E | 127 | 38.496 | 130.610 | 44.771 | 1.00 | 0.00 | AAAA |
| ATOM | 2051 | CB | TYR | E | 127 | 39.917 | 130.754 | 44.216 | 1.00 | 0.00 | AAAA |
| ATOM | 2054 | CG | TYR | E | 127 | 40.749 | 131.749 | 44.998 | 1.00 | 0.00 | AAAA |
| ATOM | 2055 | CD1 | TYR | E | 127 | 40.748 | 133.045 | 44.475 | 1.00 | 0.00 | AAAA |
| ATOM | 2057 | CD2 | TYR | E | 127 | 41.505 | 131.418 | 46.131 | 1.00 | 0.00 | AAAA |
| ATOM | 2059 | CE1 | TYR | E | 127 | 41.538 | 134.031 | 45.074 | 1.00 | 0.00 | AAAA |
| ATOM | 2061 | CE2 | TYR | E | 127 | 42.284 | 132.392 | 46.762 | 1.00 | 0.00 | AAAA |
| ATOM | 2063 | CZ | TYR | E | 127 | 42.273 | 133.681 | 46.220 | 1.00 | 0.00 | AAAA |
| ATOM | 2064 | OH | TYR | E | 127 | 42.763 | 134.749 | 46.911 | 1.00 | 0.00 | AAAA |
| ATOM | 2066 | C | TYR | E | 127 | 37.487 | 129.940 | 43.841 | 1.00 | 0.00 | AAAA |
| ATOM | 2067 | O | TYR | E | 127 | 37.846 | 129.150 | 42.975 | 1.00 | 0.00 | AAAA |
| ATOM | 2068 | N | LEU | E | 128 | 36.212 | 130.246 | 44.082 | 1.00 | 0.00 | AAAA |
| ATOM | 2070 | CA | LEU | E | 128 | 35.124 | 129.483 | 43.492 | 1.00 | 0.00 | AAAA |
| ATOM | 2072 | CB | LEU | E | 128 | 34.611 | 128.401 | 44.453 | 1.00 | 0.00 | AAAA |
| ATOM | 2075 | CG | LEU | E | 128 | 35.440 | 127.150 | 44.709 | 1.00 | 0.00 | AAAA |
| ATOM | 2077 | CD1 | LEU | E | 128 | 34.794 | 126.205 | 45.722 | 1.00 | 0.00 | AAAA |
| ATOM | 2081 | CD2 | LEU | E | 128 | 35.866 | 126.455 | 43.411 | 1.00 | 0.00 | AAAA |
| ATOM | 2085 | C | LEU | E | 128 | 34.074 | 130.467 | 43.010 | 1.00 | 0.00 | AAAA |
| ATOM | 2086 | O | LEU | E | 128 | 33.706 | 130.431 | 41.844 | 1.00 | 0.00 | AAAA |
| ATOM | 2087 | N | ALA | E | 129 | 33:675 | 131.429 | 43.842 | 1.00 | 0.00 | AAAA |
| ATOM | 2089 | CA | ALA | E | 129 | 32.920 | 132.635 | 43.576 | 1.00 | 0.00 | AAAA |
| ATOM | 2091 | CB | ALA | E | 129 | 32.301 | 133.312 | 44.798 | 1.00 | 0.00 | AAAA |
| ATOM | 2095 | C | ALA | E | 129 | 33.709 | 133.638 | 42.753 | 1.00 | 0.00 | AAAA |
| ATOM | 2096 | O | ALA | E | 129 | 33.139 | 134.531 | 42.123 | 1.00 | 0.00 | AAAA |
| ATOM | 2097 | N | THR | E | 130 | 35.027 | 133.492 | 42.652 | 1.00 | 0.00 | AAAA |
| ATOM | 2099 | CA | THR | E | 130 | 35.876 | 134.424 | 41.937 | 1.00 | 0.00 | AAAA |
| ATOM | 2101 | CB | THR | E | 130 | 37.198 | 134.283 | 42.702 | 1.00 | 0.00 | AAAA |
| ATOM | 2103 | OG1 | THR | E | 130 | 37.717 | 132.978 | 42.591 | 1.00 | 0.00 | AAAA |
| ATOM | 2105 | CG2 | THR | E | 130 | 37.216 | 134.621 | 44.188 | 1.00 | 0.00 | AAAA |
| ATOM | 2109 | C | THR | E | 130 | 35.987 | 134.006 | 40.484 | 1.00 | 0.00 | AAAA |
| ATOM | 2110 | O | THR | E | 130 | 36.337 | 134.894 | 39.713 | 1.00 | 0.00 | AAAA |
| ATOM | 2111 | N | ILE | E | 131 | 35.794 | 132.721 | 40.169 | 1.00 | 0.00 | AAAA |
| ATOM | 2113 | CA | ILE | E | 131 | 35.740 | 132.279 | 38.794 | 1.00 | 0.00 | AAAA |
| ATOM | 2115 | CB | ILE | E | 131 | 36.034 | 130.784 | 38.669 | 1.00 | 0.00 | AAAA |
| ATOM | 2117 | CG1 | ILE | E | 131 | 37.412 | 130.394 | 39.198 | 1.00 | 0.00 | AAAA |
| ATOM | 2120 | CG2 | ILE | E | 131 | 35.858 | 130.247 | 37.244 | 1.00 | 0.00 | AAAA |
| ATOM | 2124 | CD1 | ILE | E | 131 | 37.531 | 128.914 | 39.560 | 1.00 | 0.00 | AAAA |
| ATOM | 2128 | C | ILE | E | 131 | 34.391 | 132.619 | 38.163 | 1.00 | 0.00 | AAAA |
| ATOM | 2129 | O | ILE | E | 131 | 33.337 | 132.277 | 38.675 | 1.00 | 0.00 | AAAA |
| ATOM | 2130 | N | ASP | E | 132 | 34.464 | 133.284 | 37.014 | 1.00 | 0.00 | AAAA |
| ATOM | 2132 | CA | ASP | E | 132 | 33.394 | 133.199 | 36.036 | 1.00 | 0.00 | AAAA |
| ATOM | 2134 | CB | ASP | E | 132 | 33.401 | 134.343 | 35.015 | 1.00 | 0.00 | AAAA |
| ATOM | 2137 | CG | ASP | E | 132 | 32.255 | 134.449 | 34.018 | 1.00 | 0.00 | AAAA |
| ATOM | 2138 | OD1 | ASP | E | 132 | 32.336 | 135.224 | 33.041 | 1.00 | 0.00 | AAAA |
| ATOM | 2139 | OD2 | ASP | E | 132 | 31.187 | 133.844 | 34.218 | 1.00 | 0.00 | AAAA |
| ATOM | 2140 | C | ASP | E | 132 | 33.183 | 131.856 | 35.354 | 1.00 | 0.00 | AAAA |
| ATOM | 2141 | O | ASP | E | 132 | 34.053 | 131.512 | 34.555 | 1.00 | 0.00 | AAAA |
| ATOM | 2142 | N | TRP | E | 133 | 32.131 | 131.071 | 35.566 | 1.00 | 0.00 | AAAA |
| ATOM | 2144 | CA | TRP | E | 133 | 31.979 | 129.748 | 35.005 | 1.00 | 0.00 | AAAA |
| ATOM | 2146 | CB | TRP | E | 133 | 31.171 | 128.839 | 35.929 | 1.00 | 0.00 | AAAA |
| ATOM | 2149 | CG | TRP | E | 133 | 31.869 | 128.658 | 37.236 | 1.00 | 0.00 | AAAA |
| ATOM | 2150 | CD1 | TRP | E | 133 | 31.707 | 129.314 | 38.412 | 1.00 | 0.00 | AAAA |
| ATOM | 2152 | CD2 | TRP | E | 133 | 32.948 | 127.689 | 37.464 | 1.00 | 0.00 | AAAA |
| ATOM | 2153 | NE1 | TRP | E | 133 | 32.705 | 128.910 | 39.274 | 1.00 | 0.00 | AAAA |
| ATOM | 2155 | CE2 | TRP | E | 133 | 33.462 | 127.874 | 38.769 | 1.00 | 0.00 | AAAA |
| ATOM | 2156 | CE3 | TRP | E | 133 | 33.449 | 126.621 | 36.705 | 1.00 | 0.00 | AAAA |
| ATOM | 2158 | CZ2 | TRP | E | 133 | 34.467 | 127.048 | 39.294 | 1.00 | 0.00 | AAAA |
| ATOM | 2160 | CZ3 | TRP | E | 133 | 34.484 | 125.848 | 37.252 | 1.00 | 0.00 | AAAA |
| ATOM | 2162 | CH2 | TRP | E | 133 | 35.086 | 126.067 | 38.500 | 1.00 | 0.00 | AAAA |
| ATOM | 2164 | C | TRP | E | 133 | 31.087 | 129.805 | 33.770 | 1.00 | 0.00 | AAAA |
| ATOM | 2165 | O | TRP | E | 133 | 31.016 | 128.906 | 32.937 | 1.00 | 0.00 | AAAA |
| ATOM | 2166 | N | SER | E | 134 | 30.513 | 130.946 | 33.377 | 1.00 | 0.00 | AAAA |
| ATOM | 2168 | CA | SER | E | 134 | 29.701 | 131.174 | 32.195 | 1.00 | 0.00 | AAAA |
| ATOM | 2170 | CB | SER | E | 134 | 28.718 | 132.322 | 32.407 | 1.00 | 0.00 | AAAA |
| ATOM | 2173 | OG | SER | E | 134 | 29.330 | 133.567 | 32.130 | 1.00 | 0.00 | AAAA |
| ATOM | 2175 | C | SER | E | 134 | 30.466 | 131.126 | 30.881 | 1.00 | 0.00 | AAAA |
| ATOM | 2176 | O | SER | E | 134 | 29.976 | 130.739 | 29.816 | 1.00 | 0.00 | AAAA |
| ATOM | 2177 | N | ARG | E | 135 | 31.774 | 131.284 | 31.042 | 1.00 | 0.00 | AAAA |
| ATOM | 2179 | CA | ARG | E | 135 | 32.752 | 131.163 | 29.986 | 1.00 | 0:00 | AAAA |
| ATOM | 2181 | CB | ARG | E | 135 | 33.985 | 132.039 | 30.215 | 1.00 | 0.00 | AAAA |
| ATOM | 2184 | CG | ARG | E | 135 | 33.647 | 133.341 | 30.939 | 1.00 | 0.00 | AAAA |
| ATOM | 2187 | CD | ARG | E | 135 | 34.888 | 134.232 | 30.829 | 1.00 | 0.00 | AAAA |
| ATOM | 2190 | NE | ARG | E | 135 | 34.662 | 135.486 | 31.541 | 1.00 | 0.00 | AAAA |
| ATOM | 2192 | CZ | ARG | E | 135 | 35.557 | 136.485 | 31.620 | 1.00 | 0.00 | AAAA |
| ATOM | 2193 | NH1 | ARG | E | 135 | 36.711 | 136.510 | 30.955 | 1.00 | 0.00 | AAAA |
| ATOM | 2196 | NH2 | ARG | E | 135 | 35.209 | 137.525 | 32.402 | 1.00 | 0.00 | AAAA |
| ATOM | 2199 | C | ARG | E | 135 | 33.232 | 129.735 | 29.724 | 1.00 | 0.00 | AAAA |
| ATOM | 2200 | O | ARG | E | 135 | 34.118 | 129.584 | 28.892 | 1.00 | 0.00 | AAAA |
| ATOM | 2201 | N | ILE | E | 136 | 32.718 | 128.741 | 30.450 | 1.00 | 0.00 | AAAA |
| ATOM | 2203 | CA | ILE | E | 136 | 33.120 | 127.354 | 30.362 | 1.00 | 0.00 | AAAA |
| ATOM | 2205 | CB | ILE | E | 136 | 33.985 | 127.011 | 31.576 | 1.00 | 0.00 | AAAA |
| ATOM | 2207 | CG1 | ILE | E | 136 | 35.331 | 127.718 | 31.625 | 1.00 | 0.00 | AAAA |
| ATOM | 2210 | CG2 | ILE | E | 136 | 34.237 | 125.525 | 31.867 | 1.00 | 0.00 | AAAA |
| ATOM | 2214 | CD1 | ILE | E | 136 | 35.639 | 128.505 | 32.903 | 1.00 | 0.00 | AAAA |
| ATOM | 2218 | C | ILE | E | 136 | 31.857 | 126.510 | 30.259 | 1.00 | 0.00 | AAAA |
| ATOM | 2219 | O | ILE | E | 136 | 31.779 | 125.594 | 29.442 | 1.00 | 0.00 | AAAA |
| ATOM | 2220 | N | LEU | E | 137 | 30.785 | 126.819 | 30.999 | 1.00 | 0.00 | AAAA |
| ATOM | 2222 | CA | LEU | E | 137 | 29.458 | 126.246 | 30.905 | 1.00 | 0.00 | AAAA |
| ATOM | 2224 | CB | LEU | E | 137 | 29.025 | 125.863 | 32.329 | 1.00 | 0.00 | AAAA |
| ATOM | 2227 | CG | LEU | E | 137 | 29.928 | 124.729 | 32.821 | 1.00 | 0.00 | AAAA |
| ATOM | 2229 | CD1 | LEU | E | 137 | 30.839 | 125.074 | 34.000 | 1.00 | 0.00 | AAAA |
| ATOM | 2233 | CD2 | LEU | E | 137 | 29.181 | 123.457 | 33.241 | 1.00 | 0.00 | AAAA |
| ATOM | 2237 | C | LEU | E | 137 | 28.453 | 127.245 | 30.368 | 1.00 | 0.00 | AAAA |
| ATOM | 2238 | O | LEU | E | 137 | 28.474 | 128.411 | 30.753 | 1.00 | 0.00 | AAAA |
| ATOM | 2239 | N | ASP | E | 138 | 27.543 | 126.824 | 29.488 | 1.00 | 0.00 | AAAA |
| ATOM | 2241 | CA | ASP | E | 138 | 26.403 | 127.566 | 28.975 | 1.00 | 0.00 | AAAA |
| ATOM | 2243 | CB | ASP | E | 138 | 25.939 | 126.789 | 27.736 | 1.00 | 0.00 | AAAA |
| ATOM | 2246 | CG | ASP | E | 138 | 25.052 | 125.569 | 27.879 | 1.00 | 0.00 | AAAA |
| ATOM | 2247 | OD1 | ASP | E | 138 | 25.394 | 124.645 | 28.643 | 1.00 | 0.00 | AAAA |
| ATOM | 2248 | OD2 | ASP | E | 138 | 24.110 | 125.486 | 27.057 | 1.00 | 0.00 | AAAA |
| ATOM | 2249 | C | ASP | E | 138 | 25.208 | 127.592 | 29.913 | 1.00 | 0.00 | AAAA |
| ATOM | 2250 | O | ASP | E | 138 | 24.290 | 128.346 | 29.588 | 1.00 | 0.00 | AAAA |
| ATOM | 2251 | N | SER | E | 139 | 25.146 | 126.803 | 30.986 | 1.00 | 0.00 | AAAA |
| ATOM | 2253 | CA | SER | E | 139 | 24.116 | 126.720 | 32.013 | 1.00 | 0.00 | AAAA |
| ATOM | 2255 | CB | SER | E | 139 | 23.126 | 125.679 | 31.500 | 1.00 | 0.00 | AAAA |
| ATOM | 2258 | OG | SER | E | 139 | 22.028 | 125.288 | 32.297 | 1.00 | 0.00 | AAAA |
| ATOM | 2260 | C | SER | E | 139 | 24.776 | 126.405 | 33.354 | 1.00 | 0.00 | AAAA |
| ATOM | 2261 | O | SER | E | 139 | 25.398 | 125.362 | 33.515 | 1.00 | 0.00 | AAAA |
| ATOM | 2262 | N | VAL | E | 140 | 24.827 | 127.384 | 34.262 | 1.00 | 0.00 | AAAA |
| ATOM | 2264 | CA | VAL | E | 140 | 25.624 | 127.477 | 35.462 | 1.00 | 0.00 | AAAA |
| ATOM | 2266 | CB | VAL | E | 140 | 26.345 | 128.819 | 35.331 | 1.00 | 0.00 | AAAA |
| ATOM | 2268 | CG1 | VAL | E | 140 | 27.339 | 128.837 | 34.172 | 1.00 | 0.00 | AAAA |
| ATOM | 2272 | CG2 | VAL | E | 140 | 25.356 | 129.992 | 35.377 | 1.00 | 0.00 | AAAA |
| ATOM | 2276 | C | VAL | E | 140 | 24.718 | 127.416 | 36.688 | 1.00 | 0.00 | AAAA |
| ATOM | 2277 | O | VAL | E | 140 | 25.243 | 127.350 | 37.801 | 1.00 | 0.00 | AAAA |
| ATOM | 2278 | N | GLU | E | 141 | 23.403 | 127.350 | 36.533 | 1.00 | 0.00 | AAAA |
| ATOM | 2280 | CA | GLU | E | 141 | 22.419 | 127.163 | 37.586 | 1.00 | 0.00 | AAAA |
| ATOM | 2282 | CB | GLU | E | 141 | 21.132 | 127.809 | 37.079 | 1.00 | 0.00 | AAAA |
| ATOM | 2285 | CG | GLU | E | 141 | 20.318 | 127.146 | 35.971 | 1.00 | 0.00 | AAAA |
| ATOM | 2288 | CD | GLU | E | 141 | 20.706 | 127.459 | 34.533 | 1.00 | 0.00 | AAAA |
| ATOM | 2289 | OE1 | GLU | E | 141 | 19.782 | 127.441 | 33.683 | 1.00 | 0.00 | AAAA |
| ATOM | 2290 | OE2 | GLU | E | 141 | 21.901 | 127.656 | 34.223 | 1.00 | 0.00 | AAAA |
| ATOM | 2291 | C | GLU | E | 141 | 22.155 | 125.695 | 37.892 | 1.00 | 0.00 | AAAA |
| ATOM | 2292 | O | GLU | E | 141 | 21.458 | 125.410 | 38.862 | 1.00 | 0.00 | AAAA |
| ATOM | 2293 | N | ASP | E | 142 | 22.591 | 124.799 | 37.005 | 1.00 | 0.00 | AAAA |
| ATOM | 2295 | CA | ASP | E | 142 | 22.459 | 123.380 | 37.235 | 1.00 | 0.00 | AAAA |
| ATOM | 2297 | CB | ASP | E | 142 | 22.059 | 122.856 | 35.862 | 1.00 | 0.00 | AAAA |
| ATOM | 2300 | CG | ASP | E | 142 | 21.638 | 121.392 | 35.859 | 1.00 | 0.00 | AAAA |
| ATOM | 2301 | OD1 | ASP | E | 142 | 20.759 | 120.992 | 36.658 | 1.00 | 0.00 | AAAA |
| ATOM | 2302 | OD2 | ASP | E | 142 | 22.197 | 120.659 | 35.006 | 1.00 | 0.00 | AAAA |
| ATOM | 2303 | C | ASP | E | 142 | 23.792 | 122.843 | 37.762 | 1.00 | 0.00 | AAAA |
| ATOM | 2304 | O | ASP | E | 142 | 23.820 | 121.626 | 37.955 | 1.00 | 0.00 | AAAA |
| ATOM | 2305 | N | ASN | E | 143 | 24.798 | 123.698 | 37.941 | 1.00 | 0.00 | AAAA |
| ATOM | 2307 | CA | ASN | E | 143 | 25.965 | 123.434 | 38.762 | 1.00 | 0.00 | AAAA |
| ATOM | 2309 | CB | ASN | E | 143 | 26.963 | 124.576 | 38.566 | 1.00 | 0.00 | AAAA |
| ATOM | 2312 | CG | ASN | E | 143 | 27.913 | 124.488 | 37.374 | 1.00 | 0.00 | AAAA |
| ATOM | 2313 | OD1 | ASN | E | 143 | 28.127 | 123.398 | 36.855 | 1.00 | 0.00 | AAAA |
| ATOM | 2314 | ND2 | ASN | E | 143 | 28.451 | 125.630 | 36.924 | 1.00 | 0.00 | AAAA |
| ATOM | 2317 | C | ASN | E | 143 | 25.581 | 123.397 | 40.237 | 1.00 | 0.00 | AAAA |
| ATOM | 2318 | O | ASN | E | 143 | 24.858 | 124.291 | 40.672 | 1.00 | 0.00 | AAAA |
| ATOM | 2319 | N | HIS | E | 144 | 26.123 | 122.490 | 41.045 | 1.00 | 0.00 | AAAA |
| ATOM | 2321 | CA | HIS | E | 144 | 25.916 | 122.343 | 42.475 | 1.00 | 0.00 | AAAA |
| ATOM | 2323 | CB | HIS | E | 144 | 25.497 | 120.912 | 42.798 | 1.00 | 0.00 | AAAA |
| ATOM | 2326 | CG | HIS | E | 144 | 24.914 | 120.604 | 44.154 | 1.00 | 0.00 | AAAA |
| ATOM | 2327 | ND1 | HIS | E | 144 | 23.577 | 120.902 | 44.413 | 1.00 | 0.00 | AAAA |
| ATOM | 2328 | CD2 | HIS | E | 144 | 25.406 | 119.917 | 45.228 | 1.00 | 0.00 | AAAA |
| ATOM | 2330 | CE1 | HIS | E | 144 | 23.363 | 120.467 | 45.659 | 1.00 | 0.00 | AAAA |
| ATOM | 2332 | NE2 | HIS | E | 144 | 24.404 | 119.798 | 46.180 | 1.00 | 0.00 | AAAA |
| ATOM | 2334 | C | HIS | E | 144 | 27.335 | 122.578 | 43.000 | 1.00 | 0.00 | AAAA |
| ATOM | 2335 | O | HIS | E | 144 | 28.262 | 121.808 | 42.795 | 1.00 | 0.00 | AAAA |
| ATOM | 2336 | N | ILE | E | 145 | 27.486 | 123.607 | 43.843 | 1.00 | 0.00 | AAAA |
| ATOM | 2338 | CA | ILE | E | 145 | .28.813 | 123.931 | 44.322 | 1.00 | 0.00 | AAAA |
| ATOM | 2340 | CB | ILE | E | 145 | 29.418 | 125.100 | 43.532 | 1.00 | 0.00 | AAAA |
| ATOM | 2342 | CG1 | ILE | E | 145 | 29.551 | 124.701 | 42.065 | 1.00 | 0.00 | AAAA |
| ATOM | 2345 | CG2 | ILE | E | 145 | 30.761 | 125.552 | 44.092 | 1.00 | 0.00 | AAAA |
| ATOM | 2349 | CD1 | ILE | E | 145 | 30.096 | 125.741 | 41.089 | 1.00 | 0.00 | AAAA |
| ATOM | 2353 | C | ILE | E | 145 | 28.546 | 124.127 | 45.807 | 1.00 | 0.00 | AAAA |
| ATOM | 2354 | O | ILE | E | 145 | 27.830 | 125.033 | 46.202 | 1.00 | 0.00 | AAAA |
| ATOM | 2355 | N | VAL | E | 146 | 29.028 | 123.217 | 46.658 | 1.00 | 0.00 | AAAA |
| ATOM | 2357 | CA | VAL | E | 146 | 28.677 | 123.060 | 48.055 | 1.00 | 0.00 | AAAA |
| ATOM | 2359 | CB | VAL | E | 146 | 27.519 | 122.077 | 48.179 | 1.00 | 0.00 | AAAA |
| ATOM | 2361 | CG1 | VAL | E | 146 | 26.188 | 122.496 | 47.546 | 1.00 | 0.00 | AAAA |
| ATOM | 2365 | CG2 | VAL | E | 146 | 27.819 | 120.657 | 47.688 | 1.00 | 0.00 | AAAA |
| ATOM | 2369 | C | VAL | E | 146 | 29.869 | 122.606 | 48.894 | 1.00 | 0.00 | AAAA |
| ATOM | 2370 | O | VAL | E | 146 | 30.753 | 121.957 | 48.352 | 1.00 | 0.00 | AAAA |
| ATOM | 2371 | N | LEU | E | 147 | 29.914 | 123.001 | 50.169 | 1.00 | 0.00 | AAAA |
| ATOM | 2373 | CA | LEU | E | 147 | 30.756 | 122.513 | 51.243 | 1.00 | 0.00 | AAAA |
| ATOM | 2375 | CB | LEU | E | 147 | 30.546 | 121.023 | 51.445 | 1.00 | 0.00 | AAAA |
| ATOM | 2378 | CG | LEU | E | 147 | 29.140 | 120.475 | 51.684 | 1.00 | 0.00 | AAAA |
| ATOM | 2380 | CD1 | LEU | E | 147 | 29.050 | 118.940 | 51.718 | 1.00 | 0.00 | AAAA |
| ATOM | 2384 | CD2 | LEU | E | 147 | 28.505 | 121.026 | 52.962 | 1.00 | 0.00 | AAAA |
| ATOM | 2388 | C | LEU | E | 147 | 32.220 | 122.873 | 50.997 | 1.00 | 0.00 | AAAA |
| ATOM | 2389 | O | LEU | E | 147 | 32.892 | 122.059 | 50.362 | 1.00 | 0.00 | AAAA |
| ATOM | 2390 | N | ASN | E | 148 | 32.621 | 124.135 | 51.135 | 1.00 | 0.00 | AAAA |
| ATOM | 2392 | CA | ASN | E | 148 | 33.866 | 124.760 | 50.737 | 1.00 | 0.00 | AAAA |
| ATOM | 2394 | CBB | ASN | E | 148 | 33.913 | 125.014 | 49.226 | 1.00 | 0.00 | AAAA |
| ATOM | 2397 | CG | ASN | E | 148 | 32.700 | 125.778 | 48.731 | 1.00 | 0.00 | AAAA |
| ATOM | 2398 | OD1 | ASN | E | 148 | 32.723 | 127.001 | 48.886 | 1.00 | 0.00 | AAAA |
| ATOM | 2399 | ND2 | ASN | E | 148 | 31.719 | 125.105 | 48.126 | 1.00 | 0.00 | AAAA |
| ATOM | 2402 | C | ASN | E | 148 | 34.043 | 125.965 | 51.647 | 1.00 | 0.00 | AAAA |
| ATOM | 2403 | O | ASN | E | 148 | 33.086 | 126.542 | 52.169 | 1.00 | 0.00 | AAAA |
| ATOM | 2404 | N | LYS | E | 149 | 35.259 | 126.496 | 51.702 | 1.00 | 0.00 | AAAA |
| ATOM | 2406 | CA | LYS | E | 149 | 35.733 | 127.597 | 52.522 | 1.00 | 0.00 | AAAA |
| ATOM | 2408 | CB | LYS | E | 149 | 37.171 | 127.239 | 52.875 | 1.00 | 0.00 | AAAA |
| ATOM | 2411 | CG | LYS | E | 149 | 37.684 | 128.025 | 54.080 | 1.00 | 0.00 | AAAA |
| ATOM | 2414 | CD | LYS | E | 149 | 39.200 | 127.922 | 54.227 | 1.00 | 0.00 | AAAA |
| ATOM | 2417 | CE | LYS | E | 149 | 39.920 | 128.538 | 55.432 | 1.00 | 0.00 | AAAA |
| ATOM | 2420 | NZ | LYS | E | 149 | 41.140 | 127.828 | 55.819 | 1.00 | 0.00 | AAAA |
| ATOM | 2424 | C | LYS | E | 149 | 35.732 | 128.905 | 51.739 | 1.00 | 0.00 | AAAA |
| ATOM | 2425 | O | LYS | E | 149 | 36.224 | 129.934 | 52.200 | 1.00 | 0.00 | AAAA |
| ATOM | 2426 | N | ASP | E | 150 | 35.025 | 128.949 | 50.605 | 1.00 | 0.00 | AAAA |
| ATOM | 2428 | CA | ASP | E | 150 | 35.089 | 130.123 | 49.745 | 1.00 | 0.00 | AAAA |
| ATOM | 2430 | CB | ASP | E | 150 | 34.555 | 129.600 | 48.419 | 1.00 | 0.00 | AAAA |
| ATOM | 2433 | CG | ASP | E | 150 | 34.756 | 130.587 | 47.273 | 1.00 | 0.00 | AAAA |
| ATOM | 2434 | OD1 | ASP | E | 150 | 35.934 | 130.855 | 46.922 | 1.00 | 0.00 | AAAA |
| ATOM | 2435 | OD2 | ASP | E | 150 | 33.707 | 130.967 | 46.717 | 1.00 | 0.00 | AAAA |
| ATOM | 2436 | C | ASP | E | 150 | 34.353 | 131.350 | 50.262 | 1.00 | 0.00 | AAAA |
| ATOM | 2437 | O | ASP | E | 150 | 34.959 | 132.395 | 50.048 | 1.00 | 0.00 | AAAA |
| ATOM | 2438 | N | ASP | E | 151 | 33.272 | 131.183 | 51.029 | 1.00 | 0.00 | AAAA |
| ATOM | 2440 | CA | ASP | E | 151 | 32.620 | 132.271 | 51.725 | 1.00 | 0.00 | AAAA |
| ATOM | 2442 | CB | ASP | E | 151 | 31.117 | 132.117 | 51.520 | 1.00 | 0.00 | AAAA |
| ATOM | 2445 | CG | ASP | E | 151 | 30.309 | 131.195 | 52.418 | 1.00 | 0.00 | AAAA |
| ATOM | 2446 | OD1 | ASP | E | 151 | 30.737 | 130.040 | 52.641 | 1.00 | 0.00 | AAAA |
| ATOM | 2447 | OD2 | ASP | E | 151 | 29.162 | 131.503 | 52.815 | 1.00 | 0.00 | AAAA |
| ATOM | 2448 | C | ASP | E | 151 | 32.967 | 132.535 | 53.187 | 1.00 | 0.00 | AAAA |
| ATOM | 2449 | O | ASP | E | 151 | 32.478 | 133:452 | 53.854 | 1.00 | 0.00 | AAAA |
| ATOM | 2450 | N | ASN | E | 152 | 33.810 | 131.639 | 53.694 | 1.00 | 0.00 | AAAA |
| ATOM | 2452 | CA | ASN | E | 152 | 34.498 | 131.852 | 54.952 | 1.00 | 0.00 | AAAA |
| ATOM | 2454 | CB | ASN | E | 152 | 35.165 | 130.529 | 55.340 | 1.00 | 0.00 | AAAA |
| ATOM | 2457 | CG | ASN | E | 152 | 35.563 | 130.444 | 56.802 | 1.00 | 0.00 | AAAA |
| ATOM | 2458 | OD1 | ASN | E | 152 | 36.486 | 131.129 | 57.251 | 1.00 | 0.00 | AAAA |
| ATOM | 2459 | ND2 | ASN | E | 152 | 34.899 | 129.595 | 57.582 | 1.00 | 0.00 | AAAA |
| ATOM | 2462 | C | ASN | E | 152 | 35.644 | 132.851 | 54.914 | 1.00 | 0.00 | AAAA |
| ATOM | 2463 | O | ASN | E | 152 | 36.451 | 132.923 | 53.984 | 1.00 | 0.00 | AAAA |
| ATOM | 2464 | N | GLU | E | 153 | 35.704 | 133.663 | 55.965 | 1.00 | 0.00 | AAAA |
| ATOM | 2466 | CA | GLU | E | 153 | 36.475 | 134.884 | 56.066 | 1.00 | 0.00 | AAAA |
| ATOM | 2468 | CB | GLU | E | 153 | 36.176 | 135.523 | 57.425 | 1.00 | 0.00 | AAAA |
| ATOM | 2471 | CG | GLU | E | 153 | 36.656 | 136.967 | 57.506 | 1.00 | 0.00 | AAAA |
| ATOM | 2474 | CD | GLU | E | 153 | 36.018 | 137.911 | 56.499 | 1.00 | 0.00 | AAAA |
| ATOM | 2475 | OE1 | GLU | E | 153 | 36.773 | 138.493 | 55.679 | 1.00 | 0.00 | AAAA |
| ATOM | 2476 | OE2 | GLU | E | 153 | 34.786 | 138.037 | 56.622 | 1.00 | 0.00 | AAAA |
| ATOM | 2477 | C | GLU | E | 153 | 37.969 | 134.599 | 56.166 | 1.00 | 0.00 | AAAA |
| ATOM | 2478 | O | GLU | E | 153 | 38.787 | 135.416 | 55.755 | 1.00 | 0.00 | AAAA |
| ATOM | 2479 | N | GLU | E | 154 | 38.387 | 133.400 | 56.576 | 1.00 | 0.00 | AAAA |
| ATOM | 2481 | CA | GLU | E | 154 | 39.726 | 132.897 | 56.770 | 1.00 | 0.00 | AAAA |
| ATOM | 2483 | CB | GLU | E | 154 | 39.628 | 131.638 | 57.642 | 1.00 | 0.00 | AAAA |
| ATOM | 2486 | CG | GLU | E | 154 | 40.873 | 131.175 | 58.405 | 1.00 | 0.00 | AAAA |
| ATOM | 2489 | CD | GLU | E | 154 | 41.419 | 132.203 | 59.384 | 1.00 | 0.00 | AAAA |
| ATOM | 2490 | OE1 | GLU | E | 154 | 42.292 | 133.054 | 59.112 | 1.00 | 0.00 | AAAA |
| ATOM | 2491 | OE2 | GLU | E | 154 | 40.782 | 132.261 | 60.459 | 1.00 | 0.00 | AAAA |
| ATOM | 2492 | C | GLU | E | 154 | 40.413 | 132.604 | 55.441 | 1.00 | 0.00 | AAAA |
| ATOM | 2493 | O | GLU | E | 154 | 41.644 | 132.545 | 55.489 | 1.00 | 0.00 | AAAA |
| ATOM | 2494 | N | CYS | E | 155 | 39.741 | 132.519 | 54.300 | 1.00 | 0.00 | AAAA |
| ATOM | 2496 | CA | CYS | E | 155 | 40.263 | 132.807 | 52.974 | 1.00 | 0.00 | AAAA |
| ATOM | 2498 | CB | CYS | E | 155 | 40.016 | 131.564 | 52.108 | 1.00 | 0.00 | AAAA |
| ATOM | 2501 | SG | CYS | E | 155 | 40.637 | 131.596 | 50.414 | 1.00 | 0.00 | AAAA |
| ATOM | 2502 | C | CYS | E | 155 | 39.694 | 134.090 | 52.386 | 1.00 | 0.00 | AAAA |
| ATOM | 2503 | O | CYS | E | 155 | 38.623 | 134.479 | 52.852 | 1.00 | 0.00 | AAAA |
| ATOM | 2504 | N | GLY | E | 156 | 40.494 | 134.809 | 51.602 | 1.00 | 0.00 | AAAA |
| ATOM | 2506 | CA | GLY | E | 156 | 40.245 | 136.164 | 51.141 | 1.00 | 0.00 | AAAA |
| ATOM | 2509 | C | GLY | E | 156 | 39.980 | 136.139 | 49.642 | 1.00 | 0.00 | AAAA |
| ATOM | 2510 | O | GLY | E | 156 | 40.757 | 135.598 | 48.870 | 1.00 | 0.00 | AAAA |
| ATOM | 2511 | N | ASP | E | 157 | 38.837 | 136.632 | 49.145 | 1.00 | 0.00 | AAAA |
| ATOM | 2513 | CA | ASP | E | 157 | 38.269 | 136.697 | 47.815 | 1.00 | 0.00 | AAAA |
| ATOM | 2515 | CB | ASP | E | 157 | 36.814 | 137.160 | 47.898 | 1.00 | 0.00 | AAAA |
| ATOM | 2518 | CG | ASP | E | 157 | 36.493 | 138.603 | 48.257 | 1.00 | 0.00 | AAAA |
| ATOM | 2519 | OD1 | ASP | E | 157 | 36.857 | 138.940 | 49.407 | 1.00 | 0.00 | AAAA |
| ATOM | 2520 | OD2 | ASP | E | 157 | 35.795 | 139.302 | 47.507 | 1.00 | 0.00 | AAAA |
| ATOM | 2521 | C | ASP | E | 157 | 38.984 | 137.665 | 46.879 | 1.00 | 0.00 | AAAA |
| ATOM | 2522 | O | ASP | E | 157 | 38.651 | 137.609 | 45.697 | 1.00 | 0.00 | AAAA |
| ATOM | 2523 | N | ILE | E | 158 | 39.888 | 138.513 | 47.377 | 1.00 | 0.00 | AAAA |
| ATOM | 2525 | CA | ILE | E | 158 | 40.834 | 139.350 | 46.654 | 1.00 | 0.00 | AAAA |
| ATOM | 2527 | CB | ILE | E | 158 | 41.528 | 140.273 | 47.661 | 1.00 | 0.00 | AAAA |
| ATOM | 2529 | CG1 | ILE | E | 158 | 42.605 | 139.576 | 48.487 | 1.00 | 0.00 | AAAA |
| ATOM | 2532 | CG2 | ILE | E | 158 | 40.486 | 141:049 | 48.459 | 1.00 | 0.00 | AAAA |
| ATOM | 2536 | CD1 | ILE | E | 158 | 43.181 | 140.374 | 49.661 | 1.00 | 0.00 | AAAA |
| ATOM | 2540 | C | ILE | E | 158 | 41.783 | 138.472 | 45.847 | 1.00 | 0.00 | AAAA |
| ATOM | 2541 | O | ILE | E | 158 | 42.158 | 137.341 | 46.137 | 1.00 | 0.00 | AAAA |
| ATOM | 2542 | N | CYS | E | 159 | 42.229 | 139.018 | 44.711 | 1.00 | 0.00 | AAAA |
| ATOM | 2544 | CA | CYS | E | 159 | 43.193 | 138.480 | 43.768 | 1.00 | 0.00 | AAAA |
| ATOM | 2546 | CB | CYS | E | 159 | 42.530 | 137.529 | 42.771 | 1.00 | 0.00 | AAAA |
| ATOM | 2549 | SG | CYS | E | 159 | 41.071 | 138.227 | 41.961 | 1.00 | 0.00 | AAAA |
| ATOM | 2550 | C | CYS | E | 159 | 43.930 | 139.660 | 43.144 | 1.00 | 0.00 | AAAA |
| ATOM | 2551 | O | CYS | E | 159 | 43.985 | 139.787 | 41.932 | 1.00 | 0.00 | AAAA |
| ATOM | 2552 | N | PRO | E | 160 | 44.461 | 140.601 | 43.936 | 1.00 | 0.00 | AAAA |
| ATOM | 2553 | CA | PRO | E | 160 | 44.921 | 141.885 | 43.452 | 1.00 | 0.00 | AAAA |
| ATOM | 2555 | CB | PRO | E | 160 | 45.159 | 142.740 | 44.702 | 1.00 | 0.00 | AAAA |
| ATOM | 2558 | CG | PRO | E | 160 | 45.433 | 141.756 | 45.842 | 1.00 | 0.00 | AAAA |
| ATOM | 2561 | CD | PRO | E | 160 | 44.669 | 140.519 | 45.362 | 1.00 | 0.00 | AAAA |
| ATOM | 2564 | C | PRO | E | 160 | 46.215 | 141.894 | 42.646 | 1.00 | 0.00 | AAAA |
| ATOM | 2565 | O | PRO | E | 160 | 46.562 | 142.940 | 42.098 | 1.00 | 0.00 | AAAA |
| ATOM | 2566 | N | GLY | E | 161 | 47.048 | 140.849 | 42.668 | 1.00 | 0.00 | AAAA |
| ATOM | 2568 | CA | GLY | E | 161 | 48.421 | 140.909 | 42.222 | 1.00 | 0.00 | AAAA |
| ATOM | 2571 | C | GLY | E | 161 | 49.306 | 140.849 | 43.462 | 1.00 | 0.00 | AAAA |
| ATOM | 2572 | O | GLY | E | 161 | 49.332 | 139.851 | 44.182 | 1.00 | 0.00 | AAAA |
| ATOM | 2573 | N | THR | E | 162 | 49.954 | 141.964 | 43.809 | 1.00 | 0.00 | AAAA |
| ATOM | 2575 | CA | THR | E | 162 | 50.826 | 142.184 | 44.950 | 1.00 | 0.00 | AAAA |
| ATOM | 2577 | CB | THR | E | 162 | 52.299 | 142.226 | 44.528 | 1.00 | 0.00 | AAAA |
| ATOM | 2579 | OG1 | THR | E | 162 | 53.151 | 142.501 | 45.615 | 1.00 | 0.00 | AAAA |
| ATOM | 2581 | CG2 | THR | E | 162 | 52.758 | 143.111 | 43.368 | 1.00 | 0.00 | AAAA |
| ATOM | 2585 | C | THR | E | 162 | 50.331 | 143.346 | 45.792 | 1.00 | 0.00 | AAAA |
| ATOM | 2586 | O | THR | E | 162 | 50.454 | 143.327 | 47.015 | 1.00 | 0.00 | AAAA |
| ATOM | 2587 | N | ALA | E | 163 | 49.747 | 144.342 | 45.125 | 1.00 | 0.00 | AAAA |
| ATOM | 2589 | CA | ALA | E | 163 | 49.097 | 145.406 | 45.867 | 1.00 | 0.00 | AAAA |
| ATOM | 2591 | CB | ALA | E | 163 | 50.040 | 146.600 | 46.001 | 1.00 | 0.00 | AAAA |
| ATOM | 2595 | C | ALA | E | 163 | 47.795 | 145.792 | 45.184 | 1.00 | 0.00 | AAAA |
| ATOM | 2596 | O | ALA | E | 163 | 47.543 | 145.608 | 43.988 | 1.00 | 0.00 | AAAA |
| ATOM | 2597 | N | LYS | E | 164 | 46.862 | 146.340 | 45.970 | 1.00 | 0.00 | AAAA |
| ATOM | 2599 | CA | LYS | E | 164 | 45.646 | 147.004 | 45.562 | 1.00 | 0.00 | AAAA |
| ATOM | 2601 | CB | LYS | E | 164 | 44.673 | 146.909 | 46.744 | 1.00 | 0.00 | AAAA |
| ATOM | 2604 | CG | LYS | E | 164 | 44.291 | 145.507 | 47.218 | 1.00 | 0.00 | AAAA |
| ATOM | 2607 | CD | LYS | E | 164 | 43.459 | 145.667 | 48.496 | 1.00 | 0.00 | AAAA |
| ATOM | 2610 | CE | LYS | E | 164 | 42.852 | 144.343 | 48.944 | 1.00 | 0.00 | AAAA |
| ATOM | 2613 | NZ | LYS | E | 164 | 42.165 | 144.554 | 50.225 | 1.00 | 0.00 | AAAA |
| ATOM | 2617 | C | LYS | E | 164 | 46.058 | 148.449 | 45.332 | 1.00 | 0.00 | AAAA |
| ATOM | 2618 | O | LYS | E | 164 | 46.859 | 149.041 | 46.055 | 1.00 | 0.00 | AAAA |
| ATOM | 2619 | N | GLY | E | 165 | 45.694 | 149.012 | 44.186 | 1.00 | 0.00 | AAAA |
| ATOM | 2621 | CA | GLY | E | 165 | 46.140 | 150.281 | 43.635 | 1.00 | 0.00 | AAAA |
| ATOM | 2624 | C | GLY | E | 165 | 46.866 | 150.136 | 42.299 | 1.00 | 0.00 | AAAA |
| ATOM | 2625 | O | GLY | E | 165 | 47.015 | 151.142 | 41.602 | 1.00 | 0.00 | AAAA |
| ATOM | 2626 | N | LYS | E | 166 | 47.239 | 148.887 | 42.003 | 1.00 | 0.00 | AAAA |
| ATOM | 2628 | CA | LYS | E | 166 | 47.939 | 148.486 | 40.798 | 1.00 | 0.00 | AAAA |
| ATOM | 2630 | CB | LYS | E | 166 | 49.428 | 148.811 | 40.914 | 1.00 | 0.00 | AAAA |
| ATOM | 2633 | CG | LYS | E | 166 | 50.257 | 148.162 | 42.023 | 1.00 | 0.00 | AAAA |
| ATOM | 2636 | CD | LYS | E | 166 | 51.662 | 148.753 | 42.188 | 1.00 | 0.00 | AAAA |
| ATOM | 2639 | CE | LYS | E | 166 | 52.408 | 148.118 | 43.372 | 1.00 | 0.00 | AAAA |
| ATOM | 2642 | NZ | LYS | E | 166 | 53.723 | 148.738 | 43.591 | 1.00 | 0.00 | AAAA |
| ATOM | 2646 | C | LYS | E | 166 | 47.675 | 147.038 | 40.412 | 1.00 | 0.00 | AAAA |
| ATOM | 2647 | O | LYS | E | 166 | 48.535 | 146.170 | 40.353 | 1.00 | 0.00 | AAAA |
| ATOM | 2648 | N | THR | E | 167 | 46.394 | 146.656 | 40.349 | 1.00 | 0.00 | AAAA |
| ATOM | 2650 | CA | THR | E | 167 | 46:045 | 145.309 | 39.952 | 1.00 | 0.00 | AAAA |
| ATOM | 2652 | CB | THR | E | 167 | 44.588 | 145.033 | 40.353 | 1.00 | 0.00 | AAAA |
| ATOM | 2654 | OG1 | THR | E | 167 | 44.429 | 145.214 | 41.746 | 1.00 | 0.00 | AAAA |
| ATOM | 2656 | CG2 | THR | E | 167 | 43.984 | 143.650 | 40.122 | 1.00 | 0.00 | AAAA |
| ATOM | 2660 | C | THR | E | 167 | 46.098 | 145.226 | 38.439 | 1.00 | 0.00 | AAAA |
| ATOM | 2661 | O | THR | E | 167 | 45.594 | 146.093 | 37.721 | 1.00 | 0.00 | AAAA |
| ATOM | 2662 | N | ASN | E | 168 | 46.843 | 144.270 | 37.868 | 1.00 | 0.00 | AAAA |
| ATOM | 2664 | CA | ASN | E | 168 | 47.156 | 144.361 | 36.452 | 1.00 | 0.00 | AAAA |
| ATOM | 2666 | CB | ASN | E | 168 | 48.595 | 143.875 | 36.254 | 1.00 | 0.00 | AAAA |
| ATOM | 2669 | CG | ASN | E | 168 | 49.225 | 144.229 | 34.920 | 1.00 | 0.00 | AAAA |
| ATOM | 2670 | OD1 | ASN | E | 168 | 49.480 | 145.408 | 34.693 | 1.00 | 0.00 | AAAA |
| ATOM | 2671 | ND2 | ASN | E | 168 | 49.686 | 143.265 | 34.105 | 1.00 | 0.00 | AAAA |
| ATOM | 2674 | C | ASN | E | 168 | 46.122 | 143.507 | 35.736 | 1.00 | 0.00 | AAAA |
| ATOM | 2675 | O | ASN | E | 168 | 46.496 | 142.548 | 35.060 | 1.00 | 0.00 | AAAA |
| ATOM | 2676 | N | CYS | E | 169 | 44.855 | 143.829 | 35.999 | 1.00 | 0.00 | AAAA |
| ATOM | 2678 | CA | CYS | E | 169 | 43.730 | 143.109 | 35.454 | 1.00 | 0.00 | AAAA |
| ATOM | 2680 | CB | CYS | E | 169 | 43.589 | 141.662 | 35.922 | 1.00 | 0.00 | AAAA |
| ATOM | 2683 | SG | CYS | E | 169 | 42.360 | 140.650 | 35.064 | 1.00 | 0.00 | AAAA |
| ATOM | 2684 | C | CYS | E | 169 | 42.471 | 143.935 | 35.707 | 1.00 | 0.00 | AAAA |
| ATOM | 2685 | O | CYS | E | 169 | 42.239 | 144.309 | 36.857 | 1.00 | 0.00 | AAAA |
| ATOM | 2686 | N | PRO | E | 170 | 41.797 | 144.359 | 34.640 | 1.00 | 0.00 | AAAA |
| ATOM | 2687 | CA | PRO | E | 170 | 40.648 | 145.238 | 34.748 | 1.00 | 0.00 | AAAA |
| ATOM | 2689 | CB | PRO | E | 170 | 40.345 | 145.820 | 33.362 | 1.00 | 0.00 | AAAA |
| ATOM | 2692 | CG | PRO | E | 170 | 40.949 | 144.752 | 32.454 | 1.00 | 0.00 | AAAA |
| ATOM | 2695 | CD | PRO | E | 170 | 41.998 | 143.984 | 33.252 | 1.00 | 0.00 | AAAA |
| ATOM | 2698 | C | PRO | E | 170 | 39.427 | 144.504 | 35.287 | 1.00 | 0.00 | AAAA |
| ATOM | 2699 | O | PRO | E | 170 | 39.081 | 143.393 | 34.884 | 1.00 | 0.00 | AAAA |
| ATOM | 2700 | N | ALA | E | 171 | 38.784 | 145.141 | 36.261 | 1.00 | 0.00 | AAAA |
| ATOM | 2702 | CA | ALA | E | 171 | 37.708 | 144.609 | 37.084 | 1.00 | 0.00 | AAAA |
| ATOM | 2704 | CB | ALA | E | 171 | 37.646 | 145.383 | 38.404 | 1.00 | 0.00 | AAAA |
| ATOM | 2708 | C | ALA | E | 171 | 36.405 | 144.856 | 36.333 | 1.00 | 0.00 | AAAA |
| ATOM | 2709 | O | ALA | E | 171 | 36.261 | 145.794 | 35.552 | 1.00 | 0.00 | AAAA |
| ATOM | 2710 | N | THR | E | 172 | 35.432 | 143.996 | 36.639 | 1.00 | 0.00 | AAAA |
| ATOM | 2712 | CA | THR | E | 172 | 34.069 | 143.974 | 36.153 | 1.00 | 0.00 | AAAA |
| ATOM | 2714 | CB | THR | E | 172 | 33.732 | 142.622 | 35.523 | 1.00 | 0.00 | AAAA |
| ATOM | 2716 | OG1 | THR | E | 172 | 33.505 | 141.646 | 36.503 | 1.00 | 0.00 | AAAA |
| ATOM | 2718 | CG2 | THR | E | 172 | 34.825 | 142.019 | 34.639 | 1.00 | 0.00 | AAAA |
| ATOM | 2722 | C | THR | E | 172 | 33.113 | 144.317 | 37.298 | 1.00 | 0.00 | AAAA |
| ATOM | 2723 | O | THR | E | 172 | 33.500 | 144.232 | 38.459 | 1.00 | 0.00 | AAAA |
| ATOM | 2724 | N | VAL | E | 173 | 31.845 | 144.590 | 36.997 | 1.00 | 0.00 | AAAA |
| ATOM | 2726 | CA | VAL | E | 173 | 30.775 | 144.920 | 37.924 | 1.00 | 0.00 | AAAA |
| ATOM | 2728 | CB | VAL | E | 173 | 30.766 | 146.373 | 38.397 | 1.00 | 0.00 | AAAA |
| ATOM | 2730 | CG1 | VAL | E | 173 | 30.586 | 147.394 | 37.269 | 1.00 | 0.00 | AAAA |
| ATOM | 2734 | CG2 | VAL | E | 173 | 29.762 | 146.630 | 39.514 | 1.00 | 0.00 | AAAA |
| ATOM | 2738 | C | VAL | E | 173 | 29.480 | 144.313 | 37.419 | 1.00 | 0.00 | AAAA |
| ATOM | 2739 | O | VAL | E | 173 | 29.187 | 144.313 | 36.216 | 1.00 | 0.00 | AAAA |
| ATOM | 2740 | N | ILE | E | 174 | 28.689 | 143.723 | 38.314 | 1.00 | 0.00 | AAAA |
| ATOM | 2742 | CA | ILE | E | 174 | 27.443 | 142.995 | 38.153 | 1.00 | 0.00 | AAAA |
| ATOM | 2744 | CB | ILE | E | 174 | 27.657 | 141.492 | 37.998 | 1.00 | 0.00 | AAAA |
| ATOM | 2746 | CG1 | ILE | E | 174 | 28.076 | 140.587 | 39.155 | 1.00 | 0.00 | AAAA |
| ATOM | 2749 | CG2 | ILE | E | 174 | 28.548 | 141.252 | 36.777 | 1.00 | 0.00 | AAAA |
| ATOM | 2753 | CD1 | ILE | E | 174 | 27.414 | 139.205 | 39.027 | 1.00 | 0.00 | AAAA |
| ATOM | 2757 | C | ILE | E | 174 | 26.389 | 143.399 | 39.167 | 1.00 | 0.00 | AAAA |
| ATOM | 2758 | O | ILE | E | 174 | 25.236 | 143.510 | 38.772 | 1.00 | 0.00 | AAAA |
| ATOM | 2759 | N | ASN | E | 175 | 26.774 | 143.567 | 40.438 | 1.00 | 0.00 | AAAA |
| ATOM | 2761 | CA | ASN | E | 175 | 25.936 | 143.876 | 41.568 | 1.00 | 0.00 | AAAA |
| ATOM | 2763 | CB | ASN | E | 175 | 25.053 | 142.667 | 41.859 | 1.00 | 0.00 | AAAA |
| ATOM | 2766 | CG | ASN | E | 175 | 24.003 | 142.705 | 42.967 | 1.00 | 0.00 | AAAA |
| ATOM | 2767 | OD1 | ASN | E | 175 | 24.255 | 142.050 | 43.983 | 1.00 | 0.00 | AAAA |
| ATOM | 2768 | ND2 | ASN | E | 175 | 22.910 | 143.458 | 42.892 | 1.00 | 0.00 | AAAA |
| ATOM | 2771 | C | ASN | E | 175 | 26.690 | 144.404 | 42.774 | 1.00 | 0.00 | AAAA |
| ATOM | 2772 | O | ASN | E | 175 | 27.107 | 143.651 | 43.657 | 1.00 | 0.00 | AAAA |
| ATOM | 2773 | N | GLY | E | 176 | 27.064 | 145.690 | 42.848 | 1.00 | 0.00 | AAAA |
| ATOM | 2775 | CA | GLY | E | 176 | 27.661 | 146.329 | 43.996 | 1.00 | 0.00 | AAAA |
| ATOM | 2778 | C | GLY | E | 176 | 29.125 | 146.689 | 43.750 | 1.00 | 0.00 | AAAA |
| ATOM | 2779 | O | GLY | E | 176 | 29.275 | 147.601 | 42.940 | 1.00 | 0.00 | AAAA |
| ATOM | 2780 | N | GLN | E | 177 | 30.117 | 146.123 | 44.432 | 1.00 | 0.00 | AAAA |
| ATOM | 2782 | CA | GLN | E | 177 | 31.523 | 146.470 | 44.263 | 1.00 | 0.00 | AAAA |
| ATOM | 2784 | CB | GLN | E | 177 | 32.258 | 145.770 | 45.409 | 1.00 | 0.00 | AAAA |
| ATOM | 2787 | CG | GLN | E | 177 | 31.869 | 146.276 | 46.802 | 1.00 | 0.00 | AAAA |
| ATOM | 2790 | CD | GLN | E | 177 | 32.511 | 145.610 | 48.019 | 1.00 | 0.00 | AAAA |
| ATOM | 2791 | OE1 | GLN | E | 177 | 32.372 | 146.099 | 49.130 | 1.00 | 0.00 | AAAA |
| ATOM | 2792 | NE2 | GLN | E | 177 | 33.242 | 144.499 | 47.944 | 1.00 | 0.00 | AAAA |
| ATOM | 2795 | C | GLN | E | 177 | 32.151 | 145.837 | 43.041 | 1.00 | 0.00 | AAAA |
| ATOM | 2796 | O | GLN | E | 177 | 31.812 | 144.702 | 42..690 | 1.00 | 0.00 | AAAA |
| ATOM | 2797 | N | PHE | E | 178 | 33.213 | 146.411 | 42.469 | 1.00 | 0.00 | AAAA |
| ATOM | 2799 | CA | PHE | E | 178 | 33.983 | 145.892 | 41.358 | 1.00 | 0.00 | AAAA |
| ATOM | 2801 | CB | PHE | E | 178 | 34.931 | 146.994 | 40.894 | 1.00 | 0.00 | AAAA |
| ATOM | 2804 | CG | PHE | E | 178 | 34.339 | 147.804 | 39.760 | 1.00 | 0.00 | AAAA |
| ATOM | 2805 | CD1 | PHE | E | 178 | 34.522 | 147.445 | 38.412 | 1.00 | 0.00 | AAAA |
| ATOM | 2807 | CD2 | PHE | E | 178 | 33.622 | 148.976 | 40.007 | 1.00 | 0.00 | AAAA |
| ATOM | 2809 | CE1 | PHE | E | 178 | 33.954 | 148.173 | 37.362 | 1.00 | 0.00 | AAAA |
| ATOM | 2811 | CE2 | PHE | E | 178 | 33.120 | 149.777 | 38.968 | 1.00 | 0.00 | AAAA |
| ATOM | 2813 | CZ | PHE | E | 178 | 33.299 | 149.383 | 37.631 | 1.00 | 0.00 | AAAA |
| ATOM | 2815 | C | PHE | E | 178 | 34.824 | 144.652 | 41.620 | 1.00 | 0.00 | AAAA |
| ATOM | 2816 | O | PHE | E | 178 | 35.722 | 144.616 | 42.456 | 1.00 | 0.00 | AAAA |
| ATOM | 2817 | N | VAL | E | 179 | 34.570 | 143.660 | 40.758 | 1.00 | 0.00 | AAAA |
| ATOM | 2819 | CA | VAL | E | 179 | 35.092 | 142.313 | 40.837 | 1.00 | 0.00 | AAAA |
| ATOM | 2821 | CB | VAL | E | 179 | 34.016 | 141.276 | 41.167 | 1.00 | 0.00 | AAAA |
| ATOM | 2823 | CG1 | VAL | E | 179 | 33.384 | 141.362 | 42.551 | 1.00 | 0.00 | AAAA |
| ATOM | 2827 | CG2 | VAL | E | 179 | 32.825 | 141.299 | 40.204 | 1.00 | 0.00 | AAAA |
| ATOM | 2831 | C | VAL | E | 179 | 35.946 | 141.875 | 39.652 | 1.00 | 0.00 | AAAA |
| ATOM | 2832 | O | VAL | E | 179 | 35.556 | 142.102 | 38.514 | 1.00 | 0.00 | AAAA |
| ATOM | 2833 | N | GLU | E | 180 | 37.177 | 141.454 | 39.923 | 1.00 | 0.00 | AAAA |
| ATOM | 2835 | CA | GLU | E | 180 | 38.086 | 140.694 | 39.087 | 1.00 | 0.00 | AAAA |
| ATOM | 2837 | CB | GLU | E | 180 | 39.509 | 140.709 | 39.640 | 1.00 | 0.00 | AAAA |
| ATOM | 2840 | CG | GLU | E | 180 | 40.188 | 142.085 | 39.581 | 1.00 | 0.00 | AAAA |
| ATOM | 2843 | CD | GLU | E | 180 | 39.880 | 142.904 | 40.824 | 1.00 | 0.00 | AAAA |
| ATOM | 2844 | OE1 | GLU | E | 180 | 39.217 | 142.423 | 41.766 | 1.00 | 0.00 | AAAA |
| ATOM | 2845 | OE2 | GLU | E | 180 | 40.413 | 144.028 | 40.946 | 1.00 | 0.00 | AAAA |
| ATOM | 2846 | C | GLU | E | 180 | 37.606 | 139.254 | 39.142 | 1.00 | 0.00 | AAAA |
| ATOM | 2847 | O | GLU | E | 180 | 37.371 | 138.760 | 40.246 | 1.00 | 0.00 | AAAA |
| ATOM | 2848 | N | ARG | E | 181 | 37.487 | 138.585 | 37.992 | 1.00 | 0.00 | AAAA |
| ATOM | 2850 | CA | ARG | E | 181 | 37.267 | 137.163 | 37.802 | 1.00 | 0.00 | AAAA |
| ATOM | 2852 | CB | ARG | E | 181 | 36.271 | 136.842 | 36.700 | 1.00 | 0.00 | AAAA |
| ATOM | 2855 | CG | ARG | E | 181 | 34.986 | 137.664 | 36.872 | 1.00 | 0.00 | AAAA |
| ATOM | 2858 | CD | ARG | E | 181 | 34.297 | 137.293 | 38.184 | 1.00 | 0.00 | AAAA |
| ATOM | 2861 | NE | ARG | E | 181 | 32.870 | 137.612 | 38.090 | 1.00 | 0.00 | AAAA |
| ATOM | 2863 | CZ | ARG | E | 181 | 32.030 | 137.607 | 39.127 | 1.00 | 0.00 | AAAA |
| ATOM | 2864 | NH1 | ARG | E | 181 | 32.406 | 137.432 | 40.407 | 1.00 | 0.00 | AAAA |
| ATOM | 2867 | NH2 | ARG | E | 181 | 30.719 | 137.737 | 38.885 | 1.00 | 0.00 | AAAA |
| ATOM | 2870 | C | ARG | E | 181 | 38.633 | 136.498 | 37.692 | 1.00 | 0.00 | AAAA |
| ATOM | 2871 | O | ARG | E | 181 | 39.560 | 136.988 | 37.058 | 1.00 | 0.00 | AAAA |
| ATOM | 2872 | N | CYS | E | 182 | 38.968 | 135.493 | 38.512 | 1.00 | 0.00 | AAAA |
| ATOM | 2874 | CA | CYS | E | 182 | 40.302 | 135.076 | 38.882 | 1.00 | 0.00 | AAAA |
| ATOM | 2876 | CB | CYS | E | 182 | 41.031 | 136.071 | 39.775 | 1.00 | 0.00 | AAAA |
| ATOM | 2879 | SG | CYS | E | 182 | 40.095 | 136.594 | 41.229 | 1.00 | 0.00 | AAAA |
| ATOM | 2880 | C | CYS | E | 182 | 40.260 | 133.620 | 39.340 | 1.00 | 0.00 | AAAA |
| ATOM | 2881 | O | CYS | E | 182 | 39.356 | 133.153 | 40.023 | 1.00 | 0.00 | AAAA |
| ATOM | 2882 | N | TRP | E | 183 | 41.315 | 132.878 | 39.021 | 1.00 | 0.00 | AAAA |
| ATOM | 2884 | CA | TRP | E | 183 | 41.465 | 131.460 | 39.334 | 1.00 | 0.00 | AAAA |
| ATOM | 2886 | CB | TRP | E | 183 | 42.383 | 130.850 | 38.277 | 1.00 | 0.00 | AAAA |
| ATOM | 2889 | CG | TRP | E | 183 | 41.989 | 131.012 | 36.847 | 1.00 | 0.00 | AAAA |
| ATOM | 2890 | CD1 | TRP | E | 183 | 42.640 | 131.778 | 35.942 | 1.00 | 0.00 | AAAA |
| ATOM | 2892 | CD2 | TRP | E | 183 | 40.913 | 130.311 | 36.159 | 1.00 | 0.00 | AAAA |
| ATOM | 2893 | NE1 | TRP | E | 183 | 42.118 | 131.511 | 34.692 | 1.00 | 0.00 | AAAA |
| ATOM | 2895 | CE2 | TRP | E | 183 | 41.057 | 130.624 | 34.773 | 1.00 | 0.00 | AAAA |
| ATOM | 2896 | CE3 | TRP | E | 183 | 39.879 | 129.430 | 36.493 | 1.00 | 0.00 | AAAA |
| ATOM | 2898 | CZ2 | TRP | E | 183 | 40.229 | 130.066 | 33.801 | 1.00 | 0.00 | AAAA |
| ATOM | 2900 | CZ3 | TRP | E | 183 | . 39.063 | 128.884 | 35.490 | 1.00 | 0.00 | AAAA |
| ATOM | 2902 | CH2 | TRP | E | 183 | 39.269 | 129.099 | 34.120 | 1.00 | 0.00 | AAAA |
| ATOM | 2904 | C | TRP | E | 183 | 42.163 | 131.252 | 40.662 | 1.00 | 0.00 | AAAA |
| ATOM | 2905 | O | TRP | E | 183 | 41.792 | 130.292 | 41.339 | 1.00 | 0.00 | AAAA |
| ATOM | 2906 | N | THR | E | 184 | 43.176 | 132.082 | 40.931 | 1.00 | 0.00 | AAAA |
| ATOM | 2908 | CA | THR | E | 184 | 43.898 | 132.166 | 42.184 | 1.00 | 0.00 | AAAA |
| ATOM | 2910 | CB | THR | E | 184 | 45.026 | 131.148 | 42.171 | 1.00 | 0.00 | AAAA |
| ATOM | 2912 | OG1 | THR | E | 184 | 45.869 | 131.294 | 41.052 | 1.00 | 0.00 | AAAA |
| ATOM | 2914 | CG2 | THR | E | 184 | 44.604 | 129.683 | 42.318 | 1.00 | 0.00 | AAAA |
| ATOM | 2918 | C | THR | E | 184 | 44.303 | 133.613 | 42.419 | 1.00 | 0.00 | AAAA |
| ATOM | 2919 | O | THR | E | 184 | 44.047 | 134.463 | 41.570 | 1.00 | 0.00 | AAAA |
| ATOM | 2920 | N | HIS | E | 185 | 44.903 | 133.856 | 43.584 | 1.00 | 0.00 | AAAA |
| ATOM | 2922 | CA | HIS | E | 185 | 45.510 | 135.086 | 44.090 | 1.00 | 0.00 | AAAA |
| ATOM | 2924 | CB | HIS | E | 185 | 46.226 | 134.942 | 45.427 | 1.00 | 0.00 | AAAA |
| ATOM | 2927 | CG | HIS | E | 185 | 46.728 | 136.209 | 46.063 | 1.00 | 0.00 | AAAA |
| ATOM | 2928 | ND1 | HIS | E | 185 | 47.954 | 136.836 | 45.790 | 1.00 | 0.00 | AAAA |
| ATOM | 2929 | CD2 | HIS | E | 185 | 46.050 | 137.027 | 46.939 | 1.00 | 0.00 | AAAA |
| ATOM | 2931 | CE1 | HIS | E | 185 | 47.966 | 137.973 | 46.487 | 1.00 | 0.00 | AAAA |
| ATOM | 2933 | NE2 | HIS | E | 185 | 46.854 | 138.117 | 47.221 | 1.00 | 0.00 | AAAA |
| ATOM | 2935 | C | HIS | E | 185 | 46.139 | 136.020 | 43.062 | 1.00 | 0.00 | AAAA |
| ATOM | 2936 | O | HIS | E | 185 | 46.111 | 137.229 | 43.265 | 1.00 | 0.00 | AAAA |
| ATOM | 2937 | N | SER | E | 186 | 46.824 | 135.539 | 42.018 | 1.00 | 0.00 | AAAA |
| ATOM | 2939 | CA | SER | E | 186 | 47.435 | 136.354 | 40.987 | 1.00 | 0.00 | AAAA |
| ATOM | 2941 | CB | SER | E | 186 | 48.837 | 136.728 | 41.457 | 1.00 | 0.00 | AAAA |
| ATOM | 2944 | OG | SER | E | 186 | 49.629 | 135.628 | 41.823 | 1.00 | 0.00 | AAAA |
| ATOM | 2946 | C | SER | E | 186 | 47.336 | 135.724 | 39.605 | 1.00 | 0.00 | AAAA |
| ATOM | 2947 | O | SER | E | 186 | 48.245 | 135.959 | 38.811 | 1.00 | 0.00 | AAAA |
| ATOM | 2948 | N | HIS | E | 187 | 46.269 | 134.968 | 39.336 | 1.00 | 0.00 | AAAA |
| ATOM | 2950 | CA | HIS | E | 187 | 45.940 | 134.380 | 38.051 | 1.00 | 0.00 | AAAA |
| ATOM | 2952 | CB | HIS | E | 187 | 46.225 | 132.881 | 38.081 | 1.00 | 0.00 | AAAA |
| ATOM | 2955 | CG | HIS | E | 187 | 46.134 | 132.173 | 36.758 | 1.00 | 0.00 | AAAA |
| ATOM | 2956 | ND1 | HIS | E | 187 | 46.306 | 130.786 | 36.686 | 1.00 | 0.00 | AAAA |
| ATOM | 2957 | CD2 | HIS | E | 187 | 45.933 | 132.675 | 35.498 | 1.00 | 0.00 | AAAA |
| ATOM | 2959 | CE1 | HIS | E | 187 | 45.983 | 130.466 | 35.435 | 1.00 | 0.00 | AAAA |
| ATOM | 2961 | NE2 | HIS | E | 187 | 45.808 | 131.568 | 34.687 | 1.00 | 0.00 | AAAA |
| ATOM | 2963 | C | HIS | E | 187 | 44.522 | 134.835 | 37.747 | 1.00 | 0.00 | AAAA |
| ATOM | 2964 | O | HIS | E | 187 | 43.547 | 134.297 | 38.261 | 1.00 | 0.00 | AAAA |
| ATOM | 2965 | N | CYS | E | 188 | 44.446 | 135.875 | 36.919 | 1.00 | 0.00 | AAAA |
| ATOM | 2967 | CA | CYS | E | 188 | 43.252 | 136.545 | 36.434 | 1.00 | 0.00 | AAAA |
| ATOM | 2969 | CB | CYS | E | 188 | 43.554 | 137.997 | 36.062 | 1.00 | 0.00 | AAAA |
| ATOM | 2972 | SG | CYS | E | 188 | 42.090 | 139.034 | 36.283 | 1.00 | 0.00 | AAAA |
| ATOM | 2973 | C | CYS | E | 188 | 42.631 | 135.682 | 35.346 | 1.00 | 0.00 | AAAA |
| ATOM | 2974 | O | CYS | E | 188 | 43.418 | 135.148 | 34.555 | 1.00 | 0.00 | AAAA |
| ATOM | 2975 | N | GLN | E | 189 | 41.306 | 135.581 | 35.268 | 1.00 | 0.00 | AAAA |
| ATOM | 2977 | CA | GLN | E | 189 | 40.653 | 134.892 | 34.178 | 1.00 | 0.00 | AAAA |
| ATOM | 2979 | CB | GLN | E | 189 | 39.257 | 134.612 | 34.745 | 1.00 | 0.00 | AAAA |
| ATOM | 2982 | CG | GLN | E | 189 | 38.519 | 133.518 | 33.969 | 1.00 | 0.00 | AAAA |
| ATOM | 2985 | CD | GLN | E | 189 | 37.166 | 133.031 | 34.477 | 1.00 | 0.00 | AAAA |
| ATOM | 2986 | OE1 | GLN | E | 189 | 36.799 | 133.457 | 35.570 | 1.00 | 0.00 | AAAA |
| ATOM | 2987 | NE2 | GLN | E | 189 | 36.502 | 132.109 | 33.783 | 1.00 | 0.00 | AAAA |
| ATOM | 2990 | C | GLN | E | 189 | 40.648 | 135.496 | 32.778 | 1.00 | 0.00 | AAAA |
| ATOM | 2991 | O | GLN | E | 189 | 40.179 | 136.647 | 22.683 | 1.00 | 0.00 | AAAA |
| ATOM | 2992 | OXT | GLN | E | 189 | 41.088 | 134.809 | 31.832 | 1.00 | 0.00 | AAAA |
| ATOM | 2993 | N | GLY | S | 1 | 17.662 | 116.623 | 46.924 | 1.00 | 0.00 | AAAB |
| ATOM | 2996 | CA | GLY | S | 1 | 17.149 | 116.360 | 45.594 | 1.00 | 0.00 | AAAB |
| ATOM | 2999 | C | GLY | S | 1 | 16.750 | 114.912 | 45.404 | 1.00 | 0.00 | AAAB |
| ATOM | 3000 | O | GLY | S | 1 | 17.002 | 114.108 | 46.313 | 1.00 | 0.00 | AAAB |
| ATOM | 3001 | N | SER | S | 2 | 16.159 | 114.649 | 44.242 | 1.00 | 0.00 | AAAB |
| ATOM | 3003 | CA | SER | S | 2 | 15.751 | 113.313 | 43.877 | 1.00 | 0.00 | AAAB |
| ATOM | 3005 | CB | SER | S | 2 | 14.588 | 113.463 | 42.899 | 1.00 | 0.00 | AAAB |
| ATOM | 3008 | OG | SER | S | 2 | 14.878 | 114.233 | 41.758 | 1.00 | 0.00 | AAAB |
| ATOM | 3010 | C | SER | S | 2 | 16.905 | 112.546 | 43.235 | 1.00 | 0.00 | AAAB |
| ATOM | 3011 | O | SER | S | 2 | 17.040 | 111.325 | 43.338 | 1.00 | 0.00 | AAAB |
| ATOM | 3012 | N | LEU | S | 3 | 17.770 | 113.253 | 42.503 | 1.00 | 0.00 | AAAB |
| ATOM | 3014 | CA | LEU | S | 3 | 18.915 | 112.775 | 41.772 | 1.00 | 0.00 | AAAB |
| ATOM | 3016 | CB | LEU | S | 3 | 19.249 | 113.909 | 40.800 | 1.00 | 0.00 | AAAB |
| ATOM | 3019 | CG | LEU | S | 3 | 20.268 | 113.685 | 39.688 | 1.00 | 0.00 | AAAB |
| ATOM | 3021 | CD1 | LEU | S | 3 | 21.712 | 113.879 | 40.152 | 1.00 | 0.00 | AAAB |
| ATOM | 3025 | CD2 | LEU | S | 3 | 20.046 | 112.386 | 38.920 | 1.00 | 0.00 | AAAB |
| ATOM | 3029 | C | LEU | S | 3 | 20.028 | 112.561 | 42.789 | 1.00 | 0.00 | AAAB |
| ATOM | 3030 | O | LEU | S | 3 | 20.719 | 111.553 | 42.695 | 1.00 | 0.00 | AAAB |
| ATOM | 3031 | N | ASP | S | 4 | 20.104 | 113.366 | 43.840 | 1.00 | 0.00 | AAAB |
| ATOM | 3033 | CA | ASP | S | 4 | 21.003 | 113.309 | 44.971 | 1.00 | 0.00 | AAAB |
| ATOM | 3035 | CB | ASP | S | 4 | 20.825 | 114.402 | 46.022 | 1.00 | 0.00 | AAAB |
| ATOM | 3038 | CG | ASP | S | 4 | 21.228 | 115.782 | 45.530 | 1.00 | 0.00 | AAAB |
| ATOM | 3039 | OD1 | ASP | S | 4 | 20.951 | 116.166 | 44.370 | 1.00 | 0.00 | AAAB |
| ATOM | 3040 | OD2 | ASP | S | 4 | 22.021 | 116.384 | 46.284 | 1.00 | 0.00 | AAAB |
| ATOM | 3041 | C | ASP | S | 4 | 20.870 | 111.990 | 45.736 | 1.00 | 0.00 | AAAB |
| ATOM | 3042 | O | ASP | S | 4 | 21.881 | 111.432 | 46.145 | 1.00 | 0.00 | AAAB |
| ATOM | 3043 | N | GLU | S | 5 | 19.661 | 111.452 | 45.935 | 1.00 | 0.00 | AAAB |
| ATOM | 3045 | CA | GLU | S | 5 | 19.345 | 110.244 | 46.661 | 1.00 | 0.00 | AAAB |
| ATOM | 3047 | CB | GLU | S | 5 | 17.825 | 110.074 | 46.688 | 1.00 | 0.00 | AAAB |
| ATOM | 3050 | CG | GLU | S | 5 | 17.343 | 108.963 | 47.623 | 1.00 | 0.00 | AAAB |
| ATOM | 3053 | | GLU | S | 5 | 17.696 | 109.186 | 49.087 | 1.00 | 0.00 | AAAB |
| ATOM | 3054 | OE1 | GLU | S | 5 | 18.161 | 108.181 | 49.664 | 1.00 | 0.00 | AAAB |
| ATOM | 3055 | OE2 | GLU | S | 5 | 17.244 | 110.127 | 49.768 | 1.00 | 0.00 | AAAB |
| ATOM | 3056 | C | GLU | S | 5 | 19.965 | 109.026 | 45.993 | 1.00 | 0.00 | AAAB |
| ATOM | 3057 | O | GLU | S | 5 | 20.696 | 108.285 | 46.652 | 1.00 | 0.00 | AAAB |
| ATOM | 3058 | N | SER | S | 6 | 19.871 | 108.822 | 44.681 | 1.00 | 0.00 | AAAB |
| ATOM | 3060 | CA | SER | S | 6 | 20.523 | 107.777 | 43.916 | 1.00 | 0.00 | AAAB |
| ATOM | 3062 | CB | SER | S | 6 | 19.833 | 107.679 | 42.551 | 1.00 | 0.00 | AAAB |
| ATOM | 3065 | OG | SER | S | 6 | 19.881 | 108.805 | 41.696 | 1.00 | 0.00 | AAAB |
| ATOM | 3067 | C | SER | S | 6 | 22.001 | 108.047 | 43.696 | 1.00 | 0.00 | AAAB |
| ATOM | 3068 | O | SER | S | 6 | 22.744 | 107.079 | 43.571 | 1.00 | 0.00 | AAAB |
| ATOM | 3069 | N | PHE | S | 7 | 22.490 | 109.293 | 43.768 | 1.00 | 0.00 | AAAB |
| ATOM | 3071 | CA | PHE | S | 7 | 23.883 | 109.666 | 43.613 | 1.00 | 0.00 | AAAB |
| ATOM | 3073 | CB | PHE | S | 7 | 23.902 | 111.170 | 43.340 | 1.00 | 0.00 | AAAB |
| ATOM | 3076 | CG | PHE | S | 7 | 25.089 | 112.096 | 43.472 | 1.00 | 0.00 | AAAB |
| ATOM | 3077 | CD1 | PHE | S | 7 | 25.876 | 112.420 | 42.356 | 1.00 | 0.00 | AAAB |
| ATOM | 3079 | CD2 | PHE | S | 7 | 25.393 | 112.552 | 44.756 | 1.00 | 0.00 | AAAB |
| ATOM | 3081 | CE1 | PHE | S | 7 | 27.002 | 113.244 | 42.469 | 1.00 | 0.00 | AAAB |
| ATOM | 3083 | CE2 | PHE | S | 7 | 26.486 | 113.427 | 44.849 | 1.00 | 0.00 | AAAB |
| ATOM | 3085 | CZ | PHE | S | 7 | 27.279 | 113.750 | 43.742 | 1.00 | 0.00 | AAAB |
| ATOM | 3087 | C | PHE | S | 7 | 24.685 | 109.380 | 44.880 | 1.00 | 0.00 | AAAB |
| ATOM | 3088 | O | PHE | S | 7 | 25.725 | 108.729 | 44.858 | 1.00 | 0.00 | AAAB |
| ATOM | 3089 | N | TYR | S | 8 | 24.099 | 109.587 | 46.062 | 1.00 | 0.00 | AAAB |
| ATOM | 3091 | CA | TYR | S | 8 | 24.596 | 109.130 | 47.339 | 1.00 | 0.00 | AAAB |
| ATOM | 3093 | CB | TYR | S | 8 | 23.833 | 109.747 | 48.520 | 1.00 | 0.00 | AAAB |
| ATOM | 3096 | CG | TYR | S | 8 | 24.171 | 111.212 | 48.608 | 1.00 | 0.00 | AAAB |
| ATOM | 3097 | CD1 | TYR | S | 8 | 25.470 | 111.741 | 48.653 | 1.00 | 0.00 | AAAB |
| ATOM | 3099 | CD2 | TYR | S | 8 | 23.147 | 112.140 | 48.818 | 1.00 | 0.00 | AAAB |
| ATOM | 3101 | CE1 | TYR | S | 8 | 25.691 | 113.122 | 48.801 | 1.00 | 0.00 | AAAB |
| ATOM | 3103 | CE2 | TYR | S | 8 | 23.341 | 113.521 | 49.022 | 1.00 | 0.00 | AAAB |
| ATOM | 3105 | CZ | TYR | S | 8 | 24.646 | 114.022 | 49.059 | 1.00 | 0.00 | AAAB |
| ATOM | 3106 | OH | TYR | S | 8 | 24.947 | 115.343 | 49.215 | 1.00 | 0.00 | AAAB |
| ATOM | 3108 | C | TYR | S | 8 | 24.647 | 107.610 | 47.423 | 1.00 | 0.00 | AAAB |
| ATOM | 3109 | O | TYR | S | 8 | 25.584 | 107.019 | 47.930 | 1.00 | 0.00 | AAAB |
| ATOM | 3110 | N | ASP | S | 9 | 23.575 | 107.016 | 46.882 | 1.00 | 0.00 | AAAB |
| ATOM | 3112 | CA | ASP | S | 9 | 23.308 | 105.608 | 47.124 | 1.00 | 0.00 | AAAB |
| ATOM | 3114 | CB | ASP | S | 9 | 21.871 | 105.322 | 46.691 | 1.00 | 0.00 | AAAB |
| ATOM | 3117 | CG | ASP | S | 9 | 21.633 | 103.835 | 46.455 | 1.00 | 0.00 | AAAB |
| ATOM | 3118 | OD1 | ASP | S | 9 | 21.249 | 103.120 | 47.411 | 1.00 | 0.00 | AAAB |
| ATOM | 3119 | OD2 | ASP | S | 9 | 21.580 | 103.386 | 45.288 | 1.00 | 0.00 | AAAB |
| ATOM | 3120 | C | ASP | S | 9 | 24.380 | 104.761 | 46.444 | 1.00 | 0.00 | AAAB |
| ATOM | 3121 | O | ASP | S | 9 | 24.840 | 103.731 | 46.922 | 1.00 | 0.00 | AAAB |
| ATOM | 3122 | N | TRP | S | 10 | 24.762 | 105.120 | 45.215 | 1.00 | 0.00 | AAAB |
| ATOM | 3124 | CA | TRP | S | 10 | 25.732 | 104.485 | 44.354 | 1.00 | 0.00 | AAAB |
| ATOM | 3126 | CB | TRP | S | 10 | 25.762 | 105.218 | 43.009 | 1.00 | 0.00 | AAAB |
| ATOM | 3129 | CG | TRP | S | 10 | 26.382 | 104.477 | 41.864 | 1.00 | 0.00 | AAAB |
| ATOM | 3130 | CD1 | TRP | S | 10 | 27.650 | 104.611 | 41.417 | 1.00 | 0.00 | AAAB |
| ATOM | 3132 | CD2 | TRP | S | 10 | 25.718 | 103.538 | 40.969 | 1.00 | 0.00 | AAAB |
| ATOM | 3133 | NE1 | TRP | S | 10 | 27.826 | 103.769 | 40.341 | 1.00 | 0.00 | AAAB |
| ATOM | 3135 | CE2 | TRP | S | 10 | 26.664 | 103.108 | 40.005 | 1.00 | 0.00 | AAAB |
| ATOM | 3136 | CE3 | TRP | S | 10 | 24.373 | 103.206 | 40.762 | 1.00 | 0.00 | AAAB |
| ATOM | 3138 | CZ2 | TRP | S | 10 | 26.324 | 102.165 | 39.025 | 1.00 | 0.00 | AAAB |
| ATOM | 3140 | CZ3 | TRP | S | 10 | 23.997 | 102.320 | 39.742 | 1.00 | 0.00 | AAAB |
| ATOM | 3142 | CH2 | TRP | S | 10 | 24.976 | 101.815 | 38.879 | 1.00 | 0.00 | AAAB |
| ATOM | 3144 | C | TRP | S | 10 | 27.134 | 104.457 | 44.966 | 1.00 | 0.00 | AAAB |
| ATOM | 3145 | O | TRP | S | 10 | 27.805 | 103.458 | 44.751 | 1.00 | 0.00 | AAAB |
| ATOM | 3146 | N | PHE | S | 11 | 27.511 | 105.477 | 45.743 | 1.00 | 0.00 | AAAB |
| ATOM | 3148 | CA | PHE | S | 11 | 28.770 | 105.553 | 46.448 | 1.00 | 0.00 | AAAB |
| ATOM | 3150 | CB | PHE | S | 11 | 29.022 | 106.939 | 47.045 | 1.00 | 0.00 | AAAB |
| ATOM | 3153 | CG | PHE | S | 11 | 29.494 | 107.920 | 46.000 | 1.00 | 0.00 | AAAB |
| ATOM | 3154 | CD1 | PHE | S | 11 | 30.643 | 107.811 | 45.208 | 1.00 | 0.00 | AAAB |
| ATOM | 3156 | CD2 | PHE | S | 11 | 28.834 | 109.152 | 45.930 | 1.00 | 0.00 | AAAB |
| ATOM | 3158 | CE1 | PHE | S | 11 | 30.944 | 108.769 | 44.234 | 1.00 | 0.00 | AAAB |
| ATOM | 3160 | CE2 | PHE | S | 11 | 29.156 | 110.142 | 45.007 | 1.00 | 0.00 | AAAB |
| ATOM | 3162 | CZ | PHE | S | 11 | 30.203 | 109.952 | 44.091 | 1.00 | 0.00 | AAAB |
| ATOM | 3164 | C | PHE | S | 11 | 28.977 | 104.483 | 47.511 | 1.00 | 0.00 | AAAB |
| ATOM | 3165 | O | PHE | S | 11 | 30.050 | 103.902 | 47.600 | 1.00 | 0.00 | AAAB |
| ATOM | 3166 | N | GLU | S | 12 | 27.919 | 104.243 | 48.288 | 1.00 | 0.00 | AAAB |
| ATOM | 3168 | CA | GLU | S | 12 | 27.813 | 103.267 | 49.355 | 1.00 | 0.00 | AAAB |
| ATOM | 3170 | CB | GLU | S | 12 | 26.416 | 103.456 | 49.953 | 1.00 | 0.00 | AAAB |
| ATOM | 3173 | CG | GLU | S | 12 | 26.059 | 102.910 | 51.336 | 1.00 | 0.00 | AAAB |
| ATOM | 3176 | CD | GLU | S | 12 | 26.436 | 103.918 | 52.410 | 1.00 | 0.00 | AAAB |
| ATOM | 3177 | OE1 | GLU | S | 12 | 27.447 | 103.567 | 53.058 | 1.00 | 0.00 | AAAB |
| ATOM | 3178 | OE2 | GLU | S | 12 | 26.017 | 105.096 | 52.368 | 1.00 | 0.00 | AAAB |
| ATOM | 3179 | C | GLU | S | 12 | 27.932 | 101.868 | 48.775 | 1.00 | 0.00 | AAAB |
| ATOM | 3180 | O | GLU | S | 12 | 28.336 | 100.883 | 49.387 | 1.00 | 0.00 | AAAB |
| ATOM | 3181 | N | ARG | S | 13 | 27.385 | 101.601 | 47.581 | 1.00 | 0.00 | AAAB |
| ATOM | 3183 | CA | ARG | S | 13 | 27.348 | 100.332 | 46.883 | 1.00 | 0.00 | AAAB |
| ATOM | 3185 | CB | ARG | S | 13 | 26.114 | 100.412 | 45.984 | 1.00 | 0.00 | AAAB |
| ATOM | 3188 | CG | ARG | S | 13 | 24.749 | 100.535 | 46.648 | 1.00 | 0.00 | AAAB |
| ATOM | 3191 | CD | ARG | S | 13 | 23.692 | 101.029 | 45.656 | 1.00 | 0.00 | AAAB |
| ATOM | 3194 | NE | ARG | S | 13 | 23.313 | 100.016 | 44.670 | 1.00 | 0.00 | AAAB |
| ATOM | 3196 | CZ | ARG | S | 13 | 22.251 | 100.151 | 43.853 | 1.00 | 0.00 | AAAB |
| ATOM | 3197 | NH1 | ARG | S | 13 | 21.428 | 101.206 | 43.949 | 1.00 | 0.00 | AAAB |
| ATOM | 3200 | NH2 | ARG | S | 13 | 21.890 | 99.206 | 42.975 | 1.00 | 0.00 | AAAB |
| ATOM | 3203 | C | ARG | S | 13 | 28.666 | 100.136 | 46.143 | 1.00 | 0.00 | AAAB |
| ATOM | 3204 | O | ARG | S | 13 | 28.870 | 99.025 | 45.653 | 1.00 | 0.00 | AAAB |
| ATOM | 3205 | N | GLN | S | 14 | 29.504 | 101.157 | 45.957 | 1.00 | 0.00 | AAAB |
| ATOM | 3207 | CA | GLN | S | 14 | 30.874 | 101.035 | 45.512 | 1.00 | 0.00 | AAAB |
| ATOM | 3209 | CB | GLN | S | 14 | 31.300 | 102.367 | 44.873 | 1.00 | 0.00 | AAAB |
| ATOM | 3212 | CG | GLN | S | 14 | 32.656 | 102.523 | 44.192 | 1.00 | 0.00 | AAAB |
| ATOM | 3215 | CD | GLN | S | 14 | 32.761 | 103.686 | 43.214 | 1.00 | 0.00 | AAAB |
| ATOM | 3216 | OE1 | GLN | S | 14 | 32.179 | 104.765 | 43.363 | 1.00 | 0.00 | AAAB |
| ATOM | 3217 | NE2 | GLN | S | 14 | 33.337 | 103.430 | 42.045 | 1.00 | 0.00 | AAAB |
| ATOM | 3220 | C | GLN | S | 14 | 31.761 | 100.684 | 46.697 | 1.00 | 0.00 | AAAB |
| ATOM | 3221 | O | GLN | S | 14 | 32.749 | 99.976 | 46.506 | 1.00 | 0.00 | AAAB |
| ATOM | 3222 | N | LEU | S | 15 | 31.442 | 101.224 | 47.871 | 1.00 | 0.00 | AAAB |
| ATOM | 3224 | CA | LEU | S | 15 | 32.149 | 101.101 | 49.130 | 1.00 | 0.00 | AAAB |
| ATOM | 3226 | CB | LEU | S | 15 | 31.576 | 102.168 | 50.065 | 1.00 | 0.00 | AAAB |
| ATOM | 3229 | CG | LEU | S | 15 | 32.162 | 103.575 | 49.903 | 1.00 | 0.00 | AAAB |
| ATOM | 3231 | CD1 | LEU | S | 15 | 31.246 | 104.575 | 50.604 | 1.00 | 0.00 | AAAB |
| ATOM | 3235 | CD2 | LEU | S | 15 | 33.636 | 103.682 | 50.321 | 1.00 | 0.00 | AAAB |
| ATOM | 3239 | C | LEU | S | 15 | 32.086 | 99.712 | 49.748 | 1.00 | 0.00 | AAAB |
| ATOM | 3240 | O | LEU | S | 15 | 33.102 | 99.193 | 50.208 | 1.00 | 0.00 | AAAB |
| ATOM | 3241 | N | GLY | S | 16 | 31.000 | 98.958 | 49.642 | 1.00 | 0.00 | AAAB |
| ATOM | 3243 | CA | GLY | S | 16 | 30.804 | 97.554 | 49.957 | 1.00 | 0.00 | AAAB |
| ATOM | 3246 | C | GLY | S | 16 | 30.299 | 96.944 | 48.659 | 1.00 | 0.00 | AAAB |
| ATOM | 3247 | OT | GLY | S | 16 | 29.300 | 97.433 | 48.132 | 1.00 | 0.00 | AAAB |
| ATOM | 3248 | O | GLY | S | 16 | 30.865 | 95.751 | 48.306 | 1.00 | 0.00 | AAAB |
| END | | | | | | | | | | | |

### APPENDIX VI : ATOMIC COORDINATES FOR THE MODEL OF THE INSULIN MIMETIC PEPTIDE, S519C16, BOUND TO IGF-1R ECTODOMAIN

The structural coordinates in Appendixes II, IV and V were used to model the in mimetic peptide, S519C16, in the low affinity IGF binding site of IGF-1R. This model is oriented relative to atomic coordinates found in Appendixes II and IV, may be used in conjunction with atomic coordinates of Appendixes I to V to des compounds which bind to IR and/or IGF-1R.

| | | | | | | | | | | | |
|---|---|---|---|---|---|---|---|---|---|---|---|
| ATOM | 1 | N | GLU | E | 1 | 47.633 | 108.413 | 22.919 | 1.00 | 0.00 | AAAA |
| ATOM | 5 | CA | GLU | E | 1 | 46.896 | 107.146 | 23.098 | 1.00 | 0.00 | AAAA |
| ATOM | 7 | CB | GLU | E | 1 | 47.463 | 106.107 | 24.052 | 1.00 | 0.00 | AAAA |
| ATOM | 10 | CG | GLU | E | 1 | 46.904 | 104.685 | 23.925 | 1.00 | 0.00 | AAAA |
| ATOM | 13 | CD | GLU | E | 1 | 46.764 | 104.221 | 22.483 | 1.00 | 0.00 | AAAA |
| ATOM | 14 | OE1 | GLU | E | 1 | 45.723 | 104.589 | 21.911 | 1.00 | 0.00 | AAAA |
| ATOM | 15 | OE2 | GLU | E | 1 | 47.692 | 103.685 | 21.833 | 1.00 | 0.00 | AAAA |
| ATOM | 16 | C | GLU | E | 1 | 45.441 | 107.445 | 23.433 | 1.00 | 0.00 | AAAA |
| ATOM | 17 | O | GLU | E | 1 | 45.211 | 108.425 | 24.140 | 1.00 | 0.00 | AAAA |
| ATOM | 18 | N | ILE | E | 2 | 44.512 | 106.718 | 22.814 | 1.00 | 0.00 | AAAA |
| ATOM | 20 | CA | ILE | E | 2 | 43.075 | 106.704 | 23.021 | 1.00 | 0.00 | AAAA |
| ATOM | 22 | CB | ILE | E | 2 | 42.273 | 106.585 | 21.732 | 1.00 | 0.00 | AAAA |
| ATOM | 24 | CG1 | ILE | E | 2 | 42.665 | 107.652 | 20.707 | 1.00 | 0.00 | AAAA |
| ATOM | 27 | CG2 | ILE | E | 2 | 40.764 | 106.578 | 21.975 | 1.00 | 0.00 | AAAA |
| ATOM | 31 | CD1 | ILE | E | 2 | 41.907 | 107.609 | 19.383 | 1.00 | 0.00 | AAAA |
| ATOM | 35 | C | ILE | E | 2 | 42.768 | 105.529 | 23.940 | 1.00 | 0.00 | AAAA |
| ATOM | 36 | O | ILE | E | 2 | 42.786 | 104.387 | 23.485 | 1.00 | 0.00 | AAAA |
| ATOM | 37 | N | CYS | E | 3 | 42.592 | 105.774 | 25.243 | 1.00 | 0.00 | AAAA |
| ATOM | 39 | CA | CYS | E | 3 | 42.648 | 104.813 | 26.321 | 1.00 | 0.00 | AAAA |
| ATOM | 41 | CB | CYS | E | 3 | 43.472 | 105.395 | 27.467 | 1.00 | 0.00 | AAAA |
| ATOM | 44 | SG | CYS | E | 3 | 45.267 | 105.504 | 27.229 | 1.00 | 0.00 | AAAA |
| ATOM | 45 | C | CYS | E | 3 | 41.210 | 104.517 | 26.736 | 1.00 | 0.00 | AAAA |
| ATOM | 46 | O | CYS | E | 3 | 40.465 | 105.297 | 27.314 | 1.00 | 0.00 | AAAA |
| ATOM | 47 | N | GLY | E | 4 | 40.716 | 103.322 | 26.422 | 1.00 | 0.00 | AAAA |
| ATOM | 49 | CA | GLY | E | 4 | 39.365 | 102.848 | 26.664 | 1.00 | 0.00 | AAAA |
| ATOM | 52 | C | GLY | E | 4 | 39.384 | 101.366 | 26.999 | 1.00 | 0.00 | AAAA |
| ATOM | 53 | O | GLY | E | 4 | 40.377 | 100.679 | 26.786 | 1.00 | 0.00 | AAAA |
| ATOM | 54 | N | PRO | E | 5 | 38.258 | 100.837 | 27.491 | 1.00 | 0.00 | AAAA |
| ATOM | 55 | CA | PRO | E | 5 | 37.106 | 101.557 | 27.981 | 1.00 | 0.00 | AAAA |
| ATOM | 57 | CB | PRO | E | 5 | 35.965 | 100.542 | 28.080 | 1.00 | 0.00 | AAAA |
| ATOM | 60 | CG | PRO | E | 5 | 36.678 | 99.196 | 28.126 | 1.00 | 0.00 | AAAA |
| ATOM | 63 | CD | PRO | E | 5 | 38.035 | 99.402 | 27.457 | 1.00 | 0.00 | AAAA |
| ATOM | 66 | C | PRO | E | 5 | 37.397 | 102.047 | 29.402 | 1.00 | 0.00 | AAAA |
| ATOM | 67 | O | PRO | E | 5 | 38.294 | 101.581 | 30.100 | 1.00 | 0.00 | AAAA |
| ATOM | 68 | N | GLY | E | 6 | 36.458 | 102.867 | 29.861 | 1.00 | 0.00 | AAAA |
| ATOM | 70 | CA | GLY | E | 6 | 36.054 | 103.426 | 31.137 | 1.00 | 0.00 | AAAA |
| ATOM | 73 | C | GLY | E | 6 | 37.022 | 103.219 | 32.291 | 1.00 | 0.00 | AAAA |
| ATOM | 74 | O | GLY | E | 6 | 37.014 | 102.129 | 32.851 | 1.00 | 0.00 | AAAA |
| ATOM | 75 | N | ILE | E | 7 | 37.911 | 104.154 | 32.642 | 1.00 | 0.00 | AAAA |
| ATOM | 77 | CA | ILE | E | 7 | 39.124 | 103.878 | 33.385 | 1.00 | 0.00 | AAAA |
| ATOM | 79 | CB | ILE | E | 7 | 40.273 | 104.786 | 32.936 | 1.00 | 0.00 | AAAA |
| ATOM | 81 | CG1 | ILE | E | 7 | 40.321 | 104.877 | 31.409 | 1.00 | 0.00 | AAAA |
| ATOM | 84 | CG2 | ILE | E | 7 | 41.575 | 104.241 | 33.525 | 1.00 | 0.00 | AAAA |
| ATOM | 88 | CD1 | ILE | E | 7 | 41.608 | 105.401 | 30.785 | 1.00 | 0.00 | AAAA |
| ATOM | 92 | C | ILE | E | 7 | 38.845 | 103.920 | 34.882 | 1.00 | 0.00 | AAAA |
| ATOM | 93 | O | ILE | E | 7 | 38.207 | 104.862 | 35.344 | 1.00 | 0.00 | AAAA |
| ATOM | 94 | N | ASP | E | 8 | 39.239 | 102.899 | 35.648 | 1.00 | 0.00 | AAAA |
| ATOM | 96 | CA | ASP | E | 8 | 39.005 | 102.807 | 37.075 | 1.00 | 0.00 | AAAA |
| ATOM | 98 | CB | ASP | E | 8 | 38.294 | 101.467 | 37.275 | 1.00 | 0.00 | AAAA |
| ATOM | 101 | CG | ASP | E | 8 | 38.112 | 101.078 | 38.740 | 1.00 | 0.00 | AAAA |
| ATOM | 102 | OD1 | ASP | E | 8 | 37.104 | 101.512 | 39.331 | 1.00 | 0.00 | AAAA |
| ATOM | 103 | OD2 | ASP | E | 8 | 38.869 | 100.226 | 39.245 | 1.00 | 0.00 | AAAA |
| ATOM | 104 | C | ASP | E | 8 | 40.437 | 102.853 | 37.597 | 1.00 | 0.00 | AAAA |
| ATOM | 105 | O | ASP | E | 8 | 41.292 | 102.030 | 37.263 | 1.00 | 0.00 | AAAA |
| ATOM | 106 | N | ILE | E | 9 | 40.769 | 103.888 | 38.377 | 1.00 | 0.00 | AAAA |
| ATOM | 108 | CA | ILE | E | 9 | 41.963 | 104.106 | 39.153 | 1.00 | 0.00 | AAAA |
| ATOM | 110 | CB | ILE | E | 9 | 42.669 | 105.388 | 38.700 | 1.00 | 0.00 | AAAA |
| ATOM | 112 | CG1 | ILE | E | 9 | 42.792 | 105.823 | 37.240 | 1.00 | 0.00 | AAAA |
| ATOM | 115 | CG2 | ILE | E | 9 | 43.980 | 105.565 | 39.459 | 1.00 | 0.00 | AAAA |
| ATOM | 119 | CD1 | ILE | E | 9 | 43.930 | 105.148 | 36.473 | 1.00 | 0.00 | AAAA |
| ATOM | 123 | C | ILE | E | 9 | 41.449 | 104.111 | 40.579 | 1.00 | 0.00 | AAAA |
| ATOM | 124 | O | ILE | E | 9 | 40.467 | 104.797 | 40.848 | 1.00 | 0.00 | AAAA |
| ATOM | 125 | N | ARG | E | 10 | 42.004 | 103.235 | 41.433 | 1.00 | 0.00 | AAAA |
| ATOM | 127 | CA | ARG | E | 10 | 41.583 | 103.161 | 42.817 | 1.00 | 0.00 | AAAA |
| ATOM | 129 | CB | ARG | E | 10 | 40.320 | 102.336 | 43.089 | 1.00 | 0.00 | AAAA |
| ATOM | 132 | CG | ARG | E | 10 | 40.480 | 100.920 | 42.527 | 1.00 | 0.00 | AAAA |
| ATOM | 135 | CD | ARG | E | 10 | 39.223 | 100.125 | 42.849 | 1.00 | 0.00 | AAAA |
| ATOM | 138 | NE | ARG | E | 10 | 38.032 | 100.549 | 42.113 | 1.00 | 0.00 | AAAA |
| ATOM | 140 | CZ | ARG | E | 10 | 36.752 | 100.379 | 42.458 | 1.00 | 0.00 | AAAA |
| ATOM | 141 | NH1 | ARG | E | 10 | 36.286 | 99.990 | 43.661 | 1.00 | 0.00 | AAAA |
| ATOM | 144 | NH2 | ARG | E | 10 | 35.838 | 100.628 | 41.519 | 1.00 | 0.00 | AAAA |
| ATOM | 147 | C | ARG | E | 10 | 42.742 | 102.998 | 43.783 | 1.00 | 0.00 | AAAA |
| ATOM | 148 | O | ARG | E | 10 | 43.704 | 102.305 | 43.453 | 1.00 | 0.00 | AAAA |
| ATOM | 149 | | ASN | E | 11 | 42.628 | 103.529 | 45.003 | 1.00 | 0.00 | AAAA |
| ATOM | 151 | CA | ASN | E | 11 | 43.292 | 103.211 | 46.248 | 1.00 | 0.00 | AAAA |
| ATOM | 153 | CB | ASN | E | 11 | 42.952 | 101.822 | 46.771 | 1.00 | 0.00 | AAAA |
| ATOM | 156 | CG | ASN | E | 11 | 41.484 | 101.579 | 47.114 | 1.00 | 0.00 | AAAA |
| ATOM | 157 | OD1 | ASN | E | 11 | 40.831 | 102.177 | 47.958 | 1.00 | 0.00 | AAAA |
| ATOM | 158 | ND2 | ASN | E | 11 | 40.943 | 100.635 | 46.334 | 1.00 | 0.00 | AAAA |
| ATOM | 161 | C | ASN | E | 11 | 44.780 | 103.446 | 46.484 | 1.00 | 0.00 | AAAA |
| ATOM | 162 | O | ASN | E | 11 | 45.388 | 103.627 | 47.531 | 1.00 | 0.00 | AAAA |
| ATOM | 163 | N | ASP | E | 12 | 45.462 | 103.391 | 45.341 | 1.00 | 0.00 | AAAA |
| ATOM | 165 | CA | ASP | E | 12 | 46.898 | 103.374 | 45.147 | 1.00 | 0.00 | AAAA |
| ATOM | 167 | CB | ASP | E | 12 | 47.192 | 101.891 | 44.932 | 1.00 | 0.00 | AAAA |
| ATOM | 170 | CG | ASP | E | 12 | 48.540 | 101.626 | 44.294 | 1.00 | 0.00 | AAAA |
| ATOM | 171 | OD1 | ASP | E | 12 | 49.540 | 102.350 | 44.503 | 1.00 | 0.00 | AAAA |
| ATOM | 172 | OD2 | ASP | E | 12 | 48.673 | 100.646 | 43.534 | 1.00 | 0.00 | AAAA |
| ATOM | 173 | C | ASP | E | 12 | 47.191 | 104.272 | 43.957 | 1.00 | 0.00 | AAAA |
| ATOM | 174 | O | ASP | E | 12 | 46.579 | 104.051 | 42.909 | 1.00 | 0.00 | AAAA |
| ATOM | 175 | N | TYR | E | 13 | 48.066 | 105.260 | 44.139 | 1.00 | 0.00 | AAAA |
| ATOM | 177 | CA | TYR | E | 13 | 48.608 | 106.331 | 43.323 | 1.00 | 0.00 | AAAA |
| ATOM | 179 | CB | TYR | E | 13 | 49.458 | 107.224 | 44.228 | 1.00 | 0.00 | AAAA |
| ATOM | 182 | CG | TYR | E | 13 | 49.798 | 108.613 | 43.741 | 1.00 | 0.00 | AAAA |
| ATOM | 183 | CD1 | TYR | E | 13 | 48.881 | 109.644 | 43.950 | 1.00 | 0.00 | AAAA |
| ATOM | 185 | CD2 | TYR | E | 13 | 51.087 | 108.954 | 43.321 | 1.00 | 0.00 | AAAA |
| ATOM | 187 | CE1 | TYR | E | 13 | 49.148 | 110.978 | 43.598 | 1.00 | 0.00 | AAAA |
| ATOM | 189 | CE2 | TYR | E | 13 | 51.384 | 110.284 | 43.013 | 1.00 | 0.00 | AAAA |
| ATOM | 191 | CZ | TYR | E | 13 | 50.445 | 111.318 | 43.183 | 1.00 | 0.00 | AAAA |
| ATOM | 192 | OH | TYR | E | 13 | 50.893 | 112.593 | 43.039 | 1.00 | 0.00 | AAAA |
| ATOM | 194 | C | TYR | E | 13 | 49.525 | 105.670 | 42.311 | 1.00 | 0.00 | AAAA |
| ATOM | 195 | O | TYR | E | 13 | 49.753 | 106.311 | 41.283 | 1.00 | 0.00 | AAAA |
| ATOM | 196 | N | GLN | E | 14 | 50.046 | 104.442 | 42.462 | 1.00 | 0.00 | AAAA |
| ATOM | 198 | CA | GLN | E | 14 | 50.891 | 103.807 | 41.472 | 1.00 | 0.00 | AAAA |
| ATOM | 200 | CB | GLN | E | 14 | 51.542 | 102.592 | 42.131 | 1.00 | 0.00 | AAAA |
| ATOM | 203 | CG | GLN | E | 14 | 52.626 | I01.871 | 41.330 | 1.00 | 0.00 | AAAA |
| ATOM | 206 | CD | GLN | E | 14 | 5.898 | 102.655 | 41.055 | 1.00 | 0.00 | AAAA |
| ATOM | 207 | OE1 | GLN | E | 14 | 54.682 | 102.256 | 40.192 | 1.00 | 0.00 | AAAA |
| ATOM | 208 | NE2 | GLN | E | 14 | 54.213 | 103.712 | 41.810 | 1.00 | 0.00 | AAAA |
| ATOM | 211 | C | GLN | E | 14 | 50.086 | 103.335 | 40.260 | 1.00 | 0.00 | AAAA |
| ATOM | 212 | O | GLN | E | 14 | 50.675 | 103.138 | 39.196 | 1.00 | 0.00 | AAAA |
| ATOM | 213 | N | GLN | E | 15 | 48.766 | 103.271 | 40.392 | 1.00 | 0.00 | AAAA |
| ATOM | 215 | CA | GLN | E | 15 | 47.890 | 102.794 | 39.338 | 1.00 | 0.00 | AAAA |
| ATOM | 217 | CB | GLN | E | 15 | 46.551 | 102.366 | 39.947 | 1.00 | 0.00 | AAAA |
| ATOM | 220 | CG | GLN | E | 15 | 46.567 | 101.189 | 40.925 | 1.00 | 0.00 | AAAA |
| ATOM | 223 | CD | GLN | E | 15 | 47.480 | 100.131 | 40.306 | 1.00 | 0.00 | AAAA |
| ATOM | 224 | OE1 | GLN | E | 15 | 47.295 | 99.759 | 39.142 | 1.00 | 0.00 | AAAA |
| ATOM | 225 | NE2 | GLN | E | 15 | 48.459 | 99.707 | 41.105 | 1.00 | 0.00 | AAAA |
| ATOM | 228 | C | GLN | E | 15 | 47.606 | 103.957 | 38.397 | 1.00 | 0.00 | AAAA |
| ATOM | 229 | O | GLN | E | 15 | 47.285 | 103.628 | 37.261 | 1.00 | 0.00 | AAAA |
| ATOM | 230 | N | LEU | E | 16 | 47.740 | 105.236 | 38.754 | 1.00 | 0.00 | AAAA |
| ATOM | 232 | CA | LEU | E | 16 | 47.780 | 106.417 | 37.909 | 1.00 | 0.00 | AAAA |
| ATOM | 234 | CB | LEU | E | 16 | 47.952 | 107.698 | 38.712 | 1.00 | 0.00 | AAAA |
| ATOM | 237 | CG | LEU | E | 16 | 46.812 | 108.161 | 39.610 | 1.00 | 0.00 | AAAA |
| ATOM | 239 | CD1 | LEU | E | 16 | 47.348 | 109.073 | 40.715 | 1.00 | 0.00 | AAAA |
| ATOM | 243 | CD2 | LEU | E | 16 | 45.547 | 108.743 | 38.973 | 1.00 | 0.00 | AAAA |
| ATOM | 247 | C | LEU | E | 16 | 48.827 | 106.316 | 36.808 | 1.00 | 0.00 | AAAA |
| ATOM | 248 | O | LEU | E | 16 | 48.634 | 106.928 | 35.754 | 1.00 | 0.00 | AAAA |
| ATOM | 249 | N | LYS | E | 17 | 49.885 | 105.509 | 36.910 | 1.00 | 0.00 | AAAA |
| ATOM | 251 | CA | LYS | E | 17 | 50.873 | 105.232 | 35.889 | 1.00 | 0.00 | AAAA |
| ATOM | 253 | CB | LYS | E | 17 | 51.992 | 104.285 | 36.328 | 1.00 | 0.00 | AAAA |
| ATOM | 256 | CG | LYS | E | 17 | 52.979 | 104.872 | 37.342 | 1.00 | 0.00 | AAAA |
| ATOM | 259 | CD | LYS | E | 17 | 54.081 | 103.839 | 37.523 | 1.00 | 0.00 | AAAA |
| ATOM | 262 | CE | LYS | E | 17 | 55.316 | 104.405 | 38.222 | 1.00 | 0.00 | AAAA |
| ATOM | 265 | NZ | LYS | E | 17 | 56.346 | 103.378 | 38.474 | 1.00 | 0.00 | AAAA |
| ATOM | 269 | C | LYS | E | 17 | 50.333 | 104.786 | 34.539 | 1.00 | 0.00 | AAAA |
| ATOM | 270 | O | LYS | E | 17 | 50.978 | 105.055 | 33.535 | 1.00 | 0.00 | AAAA |
| ATOM | 271 | N | ARG | E | 18 | 49.123 | 104.211 | 34.520 | 1.00 | 0.00 | AAAA |
| ATOM | 273 | CA | ARG | E | 18 | 48.276 | 103.882 | 33.393 | 1.00 | 0.00 | AAAA |
| ATOM | 275 | CB | ARG | E | 18 | 47.167 | 102.980 | 33.928 | 1.00 | 0.00 | AAAA |
| ATOM | 278 | CG | ARG | E | 18 | 46.147 | 102.436 | 32.914 | 1.00 | 0.00 | AAAA |
| ATOM | 281 | CD | ARG | E | 18 | 44.857 | 101.793 | 33.399 | 1.00 | 0.00 | AAAA |
| ATOM | 284 | NE | ARG | E | 18 | 44.006 | 101.456 | 32.257 | 1.00 | 0.00 | AAAA |
| ATOM | 286 | CZ | ARG | E | 18 | 42.778 | 100.921 | 32.308 | 1.00 | 0.00 | AAAA |
| ATOM | 287 | NH1 | ARG | E | 18 | 42.163 | 100.658 | 33.465 | 1.00 | 0.00 | AAAA |
| ATOM | 290 | NH2 | ARG | E | 18 | 42.016 | 100.584 | 31.250 | 1.00 | 0.00 | AAAA |
| ATOM | 293 | C | ARG | E | 18 | 47.787 | 105.074 | 32.578 | 1.00 | 0.00 | AAAA |
| ATOM | 294 | O | ARG | E | 18 | 47.489 | 104.803 | 31.418 | 1.00 | 0.00 | AAAA |
| ATOM | 295 | N | LEU | E | 19 | 47.965 | 106.318 | 33.027 | 1.00 | 0.00 | AAAA |
| ATOM | 297 | CA | LEU | E | 19 | 47.663 | 107.540 | 32.300 | 1.00 | 0.00 | AAAA |
| ATOM | 299 | CB | LEU | E | 19 | 47.060 | 108.594 | 33.211 | 1.00 | 0.00 | AAAA |
| ATOM | 302 | CG | LEU | E | 19 | 45.848 | 108.111 | 34.017 | 1.00 | 0.00 | AAAA |
| ATOM | 304 | CD1 | LEU | E | 19 | 45.311 | 109.260 | 34.883 | 1.00 | 0.00 | AAAA |
| ATOM | 308 | CD2 | LEU | E | 19 | 44.746 | 107.493 | 33.168 | 1.00 | 0.00 | AAAA |
| ATOM | 312 | C | LEU | E | 19 | 48.866 | 108.194 | 31.623 | 1.00 | 0.00 | AAAA |
| ATOM | 313 | O | LEU | E | 19 | 48.620 | 109.020 | 30.751 | 1.00 | 0.00 | AAAA |
| ATOM | 314 | N | GLU | E | 20 | 50.097 | 107.701 | 31.752 | 1.00 | 0.00 | AAAA |
| ATOM | 316 | CA | GLU | E | 20 | 51.344 | 108.069 | 31.109 | 1.00 | 0.00 | AAAA |
| ATOM | 318 | CB | GLU | E | 20 | 52.439 | 107.016 | 31.262 | 1.00 | 0.00 | AAAA |
| ATOM | 321 | CG | GLU | E | 20 | 53.822 | 107.230 | 30.646 | 1.00 | 0.00 | AAAA |
| ATOM | 324 | CD | GLU | E | 20 | 54.492 | 108.516 | 31.108 | 1.00 | 0.00 | AAAA |
| ATOM | 325 | OE1 | GLU | E | 20 | 55.275 | 109.091 | 30.324 | 1.00 | 0.00 | AAAA |
| ATOM | 326 | OE2 | GLU | E | 20 | 54.338 | 108.841 | 32.300 | 1.00 | 0.00 | AAAA |
| ATOM | 327 | C | GLU | E | 20 | 51.213 | 108.538 | 29.671 | 1.00 | 0.00 | AAAA |
| ATOM | 328 | O | GLU | E | 20 | 51.791 | 109.561 | 29.314 | 1.00 | 0.00 | AAAA |
| ATOM | 329 | N | ASN | E | 21 | 50.513 | 107.811 | 28.784 | 1.00 | 0.00 | AAAA |
| ATOM | 331 | CA | ASN | E | 21 | 50.455 | 108.023 | 27.360 | 1.00 | 0.00 | AAAA |
| ATOM | 333 | CB | ASN | E | 21 | 51.017 | 106.779 | 26.660 | 1.00 | 0.00 | AAAA |
| ATOM | 336 | CG | ASN | E | 21 | 52.348 | 106.363 | 27.261 | 1.00 | 0.00 | AAAA |
| ATOM | 337 | OD1 | ASN | E | 21 | 53.337 | 107.043 | 26.958 | 1.00 | 0.00 | AAAA |
| ATOM | 338 | ND2 | ASN | E | 21 | 52.504 | 105.304 | 28.050 | 1.00 | 0.00 | AAAA |
| ATOM | 341 | C | ASN | E | 21 | 49.038 | 108.246 | 26.857 | 1.00 | 0.00 | AAAA |
| ATOM | 342 | O | ASN | E | 21 | 48.837 | 108.338 | 25.645 | 1.00 | 0.00 | AAAA |
| ATOM | 343 | N | CYS | E | 22 | 48.045 | 108.494 | 27.720 | 1.00 | 0.00 | AAAA |
| ATOM | 345 | CA | CYS | E | 22 | 46.654 | 108.653 | 27.347 | 1.00 | 0.00 | AAAA |
| ATOM | 347 | CB | CYS | E | 22 | 45.815 | 108.170 | 28.534 | 1.00 | 0.00 | AAAA |
| ATOM | 350 | SG | CYS | E | 22 | 45.882 | 106.411 | 28.952 | 1.00 | 0.00 | AAAA |
| ATOM | 351 | C | CYS | E | 22 | 46.386 | 110.129 | 27.110 | 1.00 | 0.00 | AAAA |
| ATOM | 352 | O | CYS | E | 22 | 46.526 | 110.970 | 28.001 | 1.00 | 0.00 | AAAA |
| ATOM | 353 | N | THR | E | 23 | 46.052 | 110.575 | 25.898 | 1.00 | 0.00 | AAAA |
| ATOM | 355 | CA | THR | E | 23 | 45.918 | 111.959 | 25.467 | 1.00 | 0.00 | AAAA |
| ATOM | 357 | CB | THR | E | 23 | 46.599 | 112.128 | 24.107 | 1.00 | 0.00 | AAAA |
| ATOM | 359 | OG1 | THR | E | 23 | 46.436 | 110.966 | 23.327 | 1.00 | 0.00 | AAAA |
| ATOM | 361 | CG2 | THR | E | 23 | 48.079 | 112.474 | 24.320 | 1.00 | 0.00 | AAAA |
| ATOM | 365 | C | THR | E | 23 | 44.423 | 112.176 | 25.346 | 1.00 | 0.00 | AAAA |
| ATOM | 366 | O | THR | E | 23 | 43.917 | 113.226 | 25.721 | 1.00 | 0.00 | AAAA |
| ATOM | 367 | N | VAL | E | 24 | 43.619 | 111.194 | 24.934 | 1.00 | 0.00 | AAAA |
| ATOM | 369 | CA | VAL | E | 24 | 42.191 | 110.981 | 24.917 | 1.00 | 0.00 | AAAA |
| ATOM | 371 | CB | VAL | E | 24 | 41.709 | 110.821 | 23.471 | 1.00 | 0.00 | AAAA |
| ATOM | 373 | CG1 | VAL | E | 24 | 40.217 | 110.515 | 23.392 | 1.00 | 0.00 | AAAA |
| ATOM | 377 | CG2 | VAL | E | 24 | 42.164 | 111.972 | 22.580 | 1.00 | 0.00 | AAAA |
| ATOM | 381 | C | VAL | E | 24 | 42.068 | 109.748 | 25.806 | 1.00 | 0.00 | AAAA |
| ATOM | 382 | O | VAL | E | 24 | 42.834 | 108.800 | 25.709 | 1.00 | 0.00 | AAAA |
| ATOM | 383 | | ILE | E | 25 | 41.118 | 109.841 | 26.743 | 1.00 | 0.00 | AAAA |
| ATOM | 385 | CA | ILE | E | 25 | 40.515 | 108.766 | 27.507 | 1.00 | 0.00 | AAAA |
| ATOM | 387 | CB | ILE | E | 25 | 40.724 | 109.133 | 28.976 | 1.00 | 0.00 | AAAA |
| ATOM | 389 | CG1 | ILE | E | 25 | 42.223 | 109.115 | 29.245 | 1.00 | 0.00 | AAAA |
| ATOM | 392 | CG2 | ILE | E | 25 | 39.877 | 108.341 | 29.982 | 1.00 | 0.00 | AAAA |
| ATOM | 396 | CD1 | ILE | E | 25 | 42.639 | 109.805 | 30.547 | 1.00 | 0.00 | AAAA |
| ATOM | 400 | C | ILE | E | 25 | 39.081 | 108.508 | 27.079 | 1.00 | 0.00 | AAAA |
| ATOM | 401 | O | ILE | E | 25 | 38.154 | 109.248 | 27.418 | 1.00 | 0.00 | AAAA |
| ATOM | 402 | N | GLU | E | 26 | 38.887 | 107.430 | 26.321 | 1.00 | 0.00 | AAAA |
| ATOM | 404 | CA | GLU | E | 26 | 37.637 | 106.994 | 25.737 | 1.00 | 0.00 | AAAA |
| ATOM | 406 | CB | GLU | E | 26 | 38.008 | 106.066 | 24.579 | 1.00 | 0.00 | AAAA |
| ATOM | 409 | CG | GLU | E | 26 | 37.323 | 106.457 | 23.268 | 1.00 | 0.00 | AAAA |
| ATOM | 412 | CD | GLU | E | 26 | 35.809 | 106.542 | 23.442 | 1.00 | 0.00 | AAAA |
| ATOM | 413 | OE1 | GLU | E | 26 | 35.315 | 105.406 | 23.354 | 1.00 | 0.00 | AAAA |
| ATOM | 414 | OE2 | GLU | E | 26 | 35.199 | 107.628 | 23.347 | 1.00 | 0.00 | AAAA |
| ATOM | 415 | C | GLU | E | 26 | 36.849 | 106.266 | 26.828 | 1.00 | 0.00 | AAAA |
| ATOM | 416 | O | GLU | E | 26 | 36.842 | 105.059 | 27.046 | 1.00 | 0.00 | AAAA |
| ATOM | 417 | N | GLY | E | 27 | 35.937 | 107.022 | 27.453 | 1.00 | 0.00 | AAAA |
| ATOM | 419 | CA | GLY | E | 27 | 35.080 | 106.702 | 28.583 | 1.00 | 0.00 | AAAA |
| ATOM | 422 | C | GLY | E | 27 | 35.311 | 107.692 | 29.720 | 1.00 | 0.00 | AAAA |
| ATOM | 423 | O | GLY | E | 27 | 35.676 | 108.825 | 29.444 | 1.00 | 0.00 | AAAA |
| ATOM | 424 | N | TYR | E | 28 | 34.995 | 107.288 | 30.951 | 1.00 | 0.00 | AAAA |
| ATOM | 426 | CA | TYR | E | 28 | 35.213 | 108.059 | 32.164 | 1.00 | 0.00 | AAAA |
| ATOM | 428 | CB | TYR | E | 28 | 34.168 | 107.637 | 33.198 | 1.00 | 0.00 | AAAA |
| ATOM | 431 | CG | TYR | E | 28 | 33.941 | 106.237 | 33.713 | 1.00 | 0.00 | AAAA |
| ATOM | 432 | CD1 | TYR | E | 28 | 34.942 | 105.550 | 34.421 | 1.00 | 0.00 | AAAA |
| ATOM | 434 | CD2 | TYR | E | 28 | 32.691 | 105.617 | 33.585 | 1.00 | 0.00 | AAAA |
| ATOM | 436 | CE1 | TYR | E | 28 | 34.671 | 104.275 | 34.912 | 1.00 | 0.00 | AAAA |
| ATOM | 438 | CE2 | TYR | E | 28 | 32.406 | 104.363 | 34.137 | 1.00 | 0.00 | AAAA |
| ATOM | 440 | CZ | TYR | E | 28 | 33.435 | 103.624 | 34.722 | 1.00 | 0.00 | AAAA |
| ATOM | 441 | OH | TYR | E | 28 | 33.196 | 102.386 | 35.245 | 1.00 | 0.00 | AAAA |
| ATOM | 443 | C | TYR | E | 28 | 36.579 | 107.829 | 32.814 | 1.00 | 0.00 | AAAA |
| ATOM | 444 | O | TYR | E | 28 | 37.223 | 106.845 | 32.480 | 1.00 | 0.00 | AAAA |
| ATOM | 445 | N | LEU | E | 29 | 36.912 | 108.681 | 33.797 | 1.00 | 0.00 | AAAA |
| ATOM | 447 | CA | LEU | E | 29 | 38.030 | 108.462 | 34.690 | 1.00 | 0.00 | AAAA |
| ATOM | 449 | CB | LEU | E | 29 | 39.142 | 109.424 | 34.250 | 1.00 | 0.00 | AAAA |
| ATOM | 452 | CG | LEU | E | 29 | 40.422 | 109.291 | 35.078 | 1.00 | 0.00 | AAAA |
| ATOM | 454 | CD1 | LEU | E | 29 | 41.193 | 107.982 | 34.883 | 1.00 | 0.00 | AAAA |
| ATOM | 458 | CD2 | LEU | E | 29 | 41.312 | 110.484 | 34.716 | 1.00 | 0.00 | AAAA |
| ATOM | 462 | C | LEU | E | 29 | 37.416 | 108.558 | 36.081 | 1.00 | 0.00 | AAAA |
| ATOM | 463 | O | LEU | E | 29 | 37.020 | 109.627 | 36.528 | 1.00 | 0.00 | AAAA |
| ATOM | 464 | N | HIS | E | 30 | 37.435 | 107.434 | 36.794 | 1.00 | 0.00 | AAAA |
| ATOM | 466 | CA | HIS | E | 30 | 37.124 | 107.259 | 38.197 | 1.00 | 0.00 | AAAA |
| ATOM | 468 | CB | HIS | E | 30 | 36.331 | 105.996 | 38.531 | 1.00 | 0.00 | AAAA |
| ATOM | 471 | CG | HIS | E | 30 | 34.863 | 105.911 | 38.252 | 1.00 | 0.00 | AAAA |
| ATOM | 472 | ND1 | HIS | E | 30 | 34.111 | 104.775 | 38.545 | 1.00 | 0.00 | AAAA |
| ATOM | 473 | CD2 | HIS | E | 30 | 34.143 | 106.782 | 37.480 | 1.00 | 0.00 | AAAA |
| ATOM | 475 | CE1 | HIS | E | 30 | 32.964 | 105.024 | 37.896 | 1.00 | 0.00 | AAAA |
| ATOM | 477 | NE2 | HIS | E | 30 | 32.881 | 106.243 | 37.328 | 1.00 | 0.00 | AAAA |
| ATOM | 479 | C | HIS | E | 30 | 38.430 | 107.224 | 38.965 | 1.00 | 0.00 | AAAA |
| ATOM | 480 | O | HIS | E | 30 | 39.319 | 106.520 | 38.491 | 1.00 | 0.00 | AAAA |
| ATOM | 481 | N | ILE | E | 31 | 38.489 | 107.931 | 40.098 | 1.00 | 0.00 | AAAA |
| ATOM | 483 | CA | ILE | E | 31 | 39.703 | 108.005 | 40.895 | 1.00 | 0.00 | AAAA |
| ATOM | 485 | CB | ILE | E | 31 | 40.519 | 109.246 | 40.506 | 1.00 | 0.00 | AAAA |
| ATOM | 487 | CG1 | ILE | E | 31 | 40.741 | 109.381 | 38.996 | 1.00 | 0.00 | AAAA |
| ATOM | 490 | CG2 | ILE | E | 31 | 41.954 | 109.122 | 41.016 | 1.00 | 0.00 | AAAA |
| ATOM | 494 | CD1 | ILE | E | 31 | 41.638 | 110.504 | 38.487 | 1.00 | 0.00 | AAAA |
| ATOM | 498 | C | ILE | E | 31 | 39.154 | 107.964 | 42.312 | 1.00 | 0.00 | AAAA |
| ATOM | 499 | O | ILE | E | 31 | 38.653 | 108.993 | 42.764 | 1.00 | 0.00 | AAAA |
| ATOM | 500 | N | LEU | E | 32 | 39.278 | 106.829 | 43.012 | 1.00 | 0.00 | AAAA |
| ATOM | 502 | CA | LEU | E | 32 | 38.717 | 106.579 | 44.324 | 1.00 | 0.00 | AAAA |
| ATOM | 504 | CB | LEU | E | 32 | 37.943 | 105.269 | 44.192 | 1.00 | 0.00 | AAAA |
| ATOM | 507 | CG | LEU | E | 32 | 37.074 | 105.115 | 42.944 | 1.00 | 0.00 | AAAA |
| ATOM | 509 | CD1 | LEU | E | 32 | 36.610 | 103.663 | 42.977 | 1.00 | 0.00 | AAAA |
| ATOM | 513 | CD2 | LEU | E | 32 | 35.858 | 106.047 | 42.934 | 1.00 | 0.00 | AAAA |
| ATOM | 517 | C | LEU | E | 32 | 39.853 | 106.298 | 45.306 | 1.00 | 0.00 | AAAA |
| ATOM | 518 | O | LEU | E | 32 | 40.705 | 105.444 | 45.079 | 1.00 | 0.00 | AAAA |
| ATOM | 519 | N | LEU | E | 33 | 39.790 | 106.976 | 46.455 | 1.00 | 0.00 | AAAA |
| ATOM | 521 | CA | LEU | E | 33 | 40.296 | 106.662 | 47.774 | 1.00 | 0.00 | AAAA |
| ATOM | 523 | CB | LEU | E | 33 | 39.544 | 105.424 | 48.266 | 1.00 | 0.00 | AAAA |
| ATOM | 526 | CG | LEU | E | 33 | 38.029 | 105.559 | 48.414 | 1.00 | 0.00 | AAAA |
| ATOM | 528 | CD1 | LEU | E | 33 | 37.223 | 104.262 | 48.367 | 1.00 | 0.00 | AAAA |
| ATOM | 532 | CD2 | LEU | E | 33 | 37.682 | 106.303 | 49.706 | 1.00 | 0.00 | AAAA |
| ATOM | 536 | C | LEU | E | 33 | 41.818 | 106.644 | 47.867 | 1.00 | 0.00 | AAAA |
| ATOM | 537 | O | LEU | E | 33 | 42.370 | 105.759 | 48.526 | 1.00 | 0.00 | AAAA |
| ATOM | 538 | N | ILE | E | 34 | 42.522 | 107.527 | 47.157 | 1.00 | 0.00 | AAAA. |
| ATOM | 540 | CA | ILE | E | 34 | 43.948 | 107.370 | 46.940 | 1.00 | 0.00 | AAAA |
| ATOM | 542 | CB | ILE | E | 34 | 44.344 | 107.668 | 45.503 | 1.00 | 0.00 | AAAA |
| ATOM | 544 | CG1 | ILE | E | 34 | 43.595 | 106.857 | 44.443 | 1.00 | 0.00 | AAAA |
| ATOM | 547 | CG2 | ILE | E | 34 | 45.832 | 107.456 | 45.185 | 1.00 | 0.00 | AAAA |
| ATOM | 551 | CD1 | ILE | E | 34 | 43.920 | 107.022 | 42.965 | 1.00 | 0.00 | AAAA |
| ATOM | 555 | C | ILE | E | 34 | 44.644 | 108.226 | 47.997 | 1.00 | 0.00 | AAAA |
| ATOM | 556 | O | ILE | E | 34 | 44.387 | 109.426 | 48.061 | 1.00 | 0.00 | AAAA |
| ATOM | 557 | N | SER | E | 35 | 45.488 | 107.564 | 48.780 | 1.00 | 0.00 | AAAA |
| ATOM | 559 | CA | SER | E | 35 | 46.430 | 108.035 | 49.770 | 1.00 | 0.00 | AAAA |
| ATOM | 561 | CB | SER | E | 35 | 46.798 | 106.804 | 50.614 | 1.00 | 0.00 | AAAA |
| ATOM | 564 | OG | SER | E | 35 | 47.606 | 107.157 | 51.714 | 1.00 | 0.00 | AAAA |
| ATOM | 566 | C | SER | E | 35 | 47.633 | 108.509 | 48.959 | 1.00 | 0.00 | AAAA |
| ATOM | 567 | O | SER | E | 35 | 48.024 | 107.857 | 47.992 | 1.00 | 0.00 | AAAA |
| ATOM | 568 | N | LYS | E | 36 | 48.224 | 109.622 | 49.403 | 1.00 | 0.00 | AAAA |
| ATOM | 570 | CA | LYS | E | 36 | 49.431 | 110.204 | 48.853 | 1.00 | 0.00 | AAAA |
| ATOM | 572 | CB | LYS | E | 36 | 49.269 | 111.016 | 47.568 | 1.00 | 0.00 | AAAA |
| ATOM | 575 | CG | LYS | E | 36 | 50.614 | 111.354 | 46.920 | 1.00 | 0.00 | AAAA |
| ATOM | 578 | CD | LYS | E | 36 | 50.829 | 112.837 | 47.224 | 1.00 | 0.00 | AAAA |
| ATOM | 581 | CE | LYS | E | 36 | 52.069 | 113.467 | 46.598 | 1.00 | 0.00 | AAAA |
| ATOM | 584 | NZ | LYS | E | 36 | 52.354 | 114.841 | 47.050 | 1.00 | 0.00 | AAAA |
| ATOM | 588 | C | LYS | E | 36 | 50.158 | 111.047 | 49.897 | 1.00 | 0.00 | AAAA |
| ATOM | 589 | O | LYS | E | 36 | 49.661 | 112.024 | 50.461 | 1.00 | 0.00 | AAAA |
| ATOM | 590 | N | ALA | E | 37 | 51.370 | 110.565 | 50.144 | 1.00 | 0.00 | AAAA |
| ATOM | 592 | CA | ALA | E | 37 | 52.393 | 111.029 | 51.068 | 1.00 | 0.00 | AAAA |
| ATOM | 594 | CB | ALA | E | 37 | 53.156 | 109.804 | 51.562 | 1.00 | 0.00 | AAAA |
| ATOM | 598 | C | ALA | E | 37 | 53.383 | 111.835 | 50.229 | 1.00 | 0.00 | AAAA |
| ATOM | 599 | O | ALA | E | 37 | 53.451 | 111.630 | 49.021 | 1.00 | 0.00 | AAAA |
| ATOM | 600 | N | GLU | E | 38 | 54.247 | 112.639 | 50.848 | 1.00 | 0.00 | AAAA |
| ATOM | 602 | CA | GLU | E | 38 | 55.349 | 113.402 | 50.303 | 1.00 | 0.00 | AAAA |
| ATOM | 604 | CB | GLU | E | 38 | 55.673 | 114.475 | 51.352 | 1.00 | 0.00 | AAAA |
| ATOM | 607 | CG | GLU | E | 38 | 56.358 | 115.740 | 50.838 | 1.00 | 0.00 | AAAA |
| ATOM | 610 | CD | GLU | E | 38 | 55.514 | 116.605 | 49.925 | 1.00 | 0.00 | AAAA |
| ATOM | 611 | OE1 | GLU | E | 38 | 56.016 | 116.879 | 48.812 | 1.00 | 0.00 | AAAA |
| ATOM | 612 | OE2 | GLU | E | 38 | 54.406 | 116.949 | 50.381 | 1.00 | 0.00 | AAAA |
| ATOM | 613 | C | GLU | E | 38 | 56.536 | 112.506 | 49.967 | 1.00 | 0.00 | AAAA |
| ATOM | 614 | O | GLU | E | 38 | 57.502 | 112.961 | 49.360 | 1.00 | 0.00 | AAAA |
| ATOM | 615 | N | ASP | E | 39 | 56.486 | 111.192 | 50.234 | 1.00 | 0.00 | AAAA |
| ATOM | 617 | CA | ASP | E | 39 | 57.446 | 110.219 | 49.738 | 1.00 | 0.00 | AAAA |
| ATOM | 619 | CB | ASP | E | 39 | 57.442 | 108.945 | 50.588 | 1.00 | 0.00 | AAAA |
| ATOM | 622 | CG | ASP | E | 39 | 56.708 | 107.715 | 50.068 | 1.00 | 0.00 | AAAA |
| ATOM | 623 | OD1 | ASP | E | 39 | 57.395 | 106.769 | 49.640 | 1.00 | 0.00 | AAAA |
| ATOM | 624 | OD2 | ASP | E | 39 | 55.475 | 107.837 | 49.886 | 1.00 | 0.00 | AAAA |
| ATOM | 625 | C | ASP | E | 39 | 57.106 | 109.966 | 48.283 | 1.00 | 0.00 | AAAA |
| ATOM | 626 | O | ASP | E | 39 | 58.028 | 109.562 | 47.573 | 1.00 | 0.00 | AAAA |
| ATOM | 627 | N | TYR | E | 40 | 55.955 | 110.285 | 47.688 | 1.00 | 0.00 | AAAA |
| ATOM | 629 | CA | TYR | E | 40 | 55.799 | 110.465 | 46.260 | 1.00 | 0.00 | AAAA |
| ATOM | 631 | CB | TYR | E | 40 | 54.320 | 110.372 | 45.889 | 1.00 | 0.00 | AAAA |
| ATOM | 634 | CG | TYR | E | 40 | 53.904 | 108.969 | 45.483 | 1.00 | 0.00 | AAAA |
| ATOM | 635 | CD1 | TYR | E | 40 | 54.353 | 108.506 | 44.243 | 1.00 | 0.00 | AAAA |
| ATOM | 637 | CD2 | TYR | E | 40 | 53.127 | 108.122 | 46.286 | 1.00 | 0.00 | AAAA |
| ATOM | 639 | CE1 | TYR | E | 40 | 54.039 | 107.205 | 43.827 | 1.00 | 0.00 | AAAA |
| ATOM | 641 | CE2 | TYR | E | 40 | 52.724 | 106.880 | 45.795 | 1.00 | 0.00 | AAAA |
| ATOM | 643 | CZ | TYR | E | 40 | 53.150 | 106.400 | 44.553 | 1.00 | 0.00 | AAAA |
| ATOM | 644 | OH | TYR | E | 40 | 52.939 | 105.129 | 44.096 | 1.00 | 0.00 | AAAA |
| ATOM | 646 | C | TYR | E | 40 | 56.251 | 111.862 | 45.860 | 1.00 | 0.00 | AAAA |
| ATOM | 647 | O | TYR | E | 40 | -55.689 | 112.836 | 46.358 | 1.00 | 0.00 | AAAA |
| ATOM | 648 | N | ARG | E | 41 | 57.125 | 112.018 | 44.858 | 1.00 | 0.00 | AAAA |
| ATOM | 650 | CA | ARG | E | 41 | 57.229 | 113.222 | 44.066 | 1.00 | 0.00 | AAAA |
| ATOM | 652 | CB | ARG | E | 41 | 58.476 | 113.211 | 43.167 | 1.00 | 0.00 | AAAA |
| ATOM | 655 | CG | ARG | E | 41 | 59.775 | 112.956 | 43.931 | 1.00 | 0.00 | AAAA |
| ATOM | 658 | CD | ARG | E | 41 | 61.086 | 112.907 | 43.148 | 1.00 | 0.00 | AAAA |
| ATOM | 661 | NE | ARG | E | 41 | 62.060 | 112.390 | 44.118 | 1.00 | 0.00 | AAAA |
| ATOM | 663 | CZ | ARG | E | 41 | 63.081 | 113.049 | 44.681 | 1.00 | 0.00 | AAAA |
| ATOM | 664 | NH1 | ARG | E | 41 | 63.494 | 114.239 | 44.217 | 1.00 | 0.00 | AAAA |
| ATOM | 667 | NH2 | ARG | E | 41 | 63.694 | 112.656 | 45.812 | 1.00 | 0.00 | AAAA |
| ATOM | 670 | C | ARG | E | 41 | 55.980 | 113.302 | 43.202 | 1.00 | 0.00 | AAAA |
| ATOM | 671 | O | ARG | E | 41 | 55.465 | 112.292 | 42.732 | 1.00 | 0.00 | AAAA |
| ATOM | 672 | N | SER | E | 42 | 55.427 | 114.493 | 43.019 | 1.00 | 0.00 | AAAA |
| ATOM | 674 | CA | SER | E | 42 | 54.076 | 114.650 | 42.516 | 1.00 | 0.00 | AAAA |
| ATOM | 676 | CB | SER | E | 42 | 53.596 | 116.059 | 42.888 | 1.00 | 0.00 | AAAA |
| ATOM | 679 | OG | SER | E | 42 | 54.220 | 117.121 | 42.199 | 1.00 | 0.00 | AAAA |
| ATOM | 681 | C | SER | E | 42 | 53.922 | 114.389 | 41.017 | 1.00 | 0.00 | AAAA |
| ATOM | 682 | O | SER | E | 42 | 54.769 | 114.637 | 40.161 | 1.00 | 0.00 | AAAA |
| ATOM | 683 | N | TYR | E | 43 | 52.818 | 113.715 | 40.701 | 1.00 | 0.00 | AAAA |
| ATOM | 685 | CA | TYR | E | 43 | 52.427 | 113.194 | 39.407 | 1.00 | 0.00 | AAAA |
| ATOM | 687 | CB | TYR | E | 43 | 51.470 | 112.019 | 39.602 | 1.00 | 0.00 | AAAA |
| ATOM | 690 | CG | TYR | E | 43 | 52.184 | 110.721 | 39.887 | 1.00 | 0.00 | AAAA |
| ATOM | 691 | CD1 | TYR | E | 43 | 53.523 | 110.597 | 40.271 | 1.00 | 0.00 | AAAA |
| ATOM | 693 | CD2 | TYR | E | 43 | 51.439 | 109.549 | 39.717 | 1.00 | 0.00 | AAAA |
| ATOM | 695 | CE1 | TYR | E | 43 | 54.125 | 109.350 | 40.514 | 1.00 | 0.00 | AAAA |
| ATOM | 697 | CE2 | TYR | E | 43 | 52.029 | 108.294 | 39.904 | 1.00 | 0.00 | AAAA |
| ATOM | 699 | CZ | TYR | E | 43 | 53.365 | 108:200 | 40.297 | 1.00 | 0.00 | AAAA |
| ATOM | 700 | OH | TYR | E | 43 | 53.950 | 106.968 | 40.299 | 1.00 | 0.00 | AAAA |
| ATOM | 702 | C | TYR | E | 43 | 51.691 | 114.318 | 38.696 | 1.00 | 0.00 | AAAA |
| ATOM | 703 | O | TYR | E | 43 | 50.870 | 115.004 | 39.301 | 1.00 | 0.00 | AAAA |
| ATOM | 704 | N | ARG | E | 44 | 51.858 | 114.466 | 37.378 | 1.00 | 0.00 | AAAA |
| ATOM | 706 | CA | ARG | E | 44 | 51.275 | 115.466 | 36.512 | 1.00 | 0.00 | AAAA |
| ATOM | 708 | CB | ARG | E | 44 | 52.150 | 116.705 | 36,716 | 1.00 | 0.00 | AAAA |
| ATOM | 711 | CG | ARG | E | 44 | 51.778 | 117.915 | 35.858 | 1.00 | 0.00 | AAAA |
| ATOM | 714 | CD | ARG | E | 44 | 52.684 | 119.132 | 36.013 | 1.00 | 0.00 | AAAA |
| ATOM | 717 | NE | ARG | E | 44 | 52.510 | 120.144 | 34.974 | 1.00 | 0.00 | AAAA |
| ATOM | 719 | CZ | ARG | E | 44 | 52.126 | 121.415 | 35.129 | 1.00 | 0.00 | AAAA |
| ATOM | 720 | NH1 | ARG | E | 44 | 51.873 | 121.950 | 36.339 | 1.00 | 0.00 | AAAA |
| ATOM | 723 | NH2 | ARG | E | 44 | 51.924 | 122.219 | 34.082 | 1.00 | 0.00 | AAAA |
| ATOM | 726 | C | ARG | E | 44 | 51.146 | 114.910 | 35.094 | 1.00 | 0.00 | AAAA |
| ATOM | 727 | O | ARG | E | 44 | 52.166 | 114.332 | 34.713 | 1.00 | 0.00 | AAAA |
| ATOM | 728 | N | PHE | E | 45 | 49.993 | 115.039 | 34.436 | 1.00 | 0.00 | AAAA |
| ATOM | 730 | CA | PHE | E | 45 | 49.740 | 114.454 | 33.142 | 1.00 | 0.00 | AAAA |
| ATOM | 732 | CB | PHE | E | 45 | 48.641 | 113.384 | 33.224 | 1.00 | 0.00 | AAAA |
| ATOM | 735 | CG | PHE | E | 45 | 49.160 | 112.220 | 34.033 | 1.00 | 0.00 | AAAA |
| ATOM | 736 | CD1 | PHE | E | 45 | 50.056 | 111.305 | 33.479 | 1.00 | 0.00 | AAAA |
| ATOM | 738 | CD2 | PHE | E | 45 | 48.721 | 112.018 | 35.352 | 1.00 | 0.00 | AAAA |
| ATOM | 740 | CE1 | PHE | E | 45 | 50.504 | 110.198 | 34.224 | 1.00 | 0.00 | AAAA |
| ATOM | 742 | CE2 | PHE | E | 45 | 49.174 | 110.922 | 36.089 | 1.00 | 0.00 | AAAA |
| ATOM | 744 | CZ | PHE | E | 45 | 50.028 | 109.970 | 35.521 | 1.00 | 0.00 | AAAA |
| ATOM | 746 | C | PHE | E | 45 | 49.482 | 115.494 | 32.069 | 1.00 | 0.00 | AAAA |
| ATOM | 747 | O | PHE | E | 45 | 48.339 | 115.790 | 31.709 | 1.00 | 0.00 | AAAA |
| ATOM | 748 | N | PRO | E | 46 | 50.492 | 116.116 | 31.458 | 1.00 | 0.00 | AAAA |
| ATOM | 749 | CA | PRO | E | 46 | 50.362 | 117.143 | 30.447 | 1.00 | 0.00 | AAAA |
| ATOM | 751 | CB | PRO | E | 46 | 51.796 | 117.580 | 30.167 | 1.00 | 0.00 | AAAA |
| ATOM | 754 | CG | PRO | E | 46 | 52.721 | 116.421 | 30.536 | 1.00 | 0.00 | AAAA |
| ATOM | 757 | CD | PRO | E | 46 | 51.883 | 115.718 | 31.616 | 1.00 | 0.00 | AAAA |
| ATOM | 760 | C | PRO | E | 46 | 49.705 | 116.759 | 29.137 | 1.00 | 0.00 | AAAA |
| ATOM | 761 | O | PRO | E | 46 | 49.213 | 117.608 | 28.400 | 1.00 | 0.00 | AAAA |
| ATOM | 762 | N | LYS | E | 47 | 49.936 | 115.515 | 28.710 | 1.00 | 0.00 | AAAA |
| ATOM | 764 | CA | LYS | E | 47 | 49.546 | 115.094 | 27.375 | 1.00 | 0.00 | AAAA |
| ATOM | 766 | CB | LYS | E | 47 | 50.272 | 113.801 | 27.016 | 1.00 | 0.00 | AAAA |
| ATOM | 769 | CG | LYS | E | 47 | 51.781 | 113.772 | 27.270 | 1.00 | 0.00 | AAAA |
| ATOM | 772 | CD | LYS | E | 47 | 52.459 | 112.578 | 26.584 | 1.00 | 0.00 | AAAA |
| ATOM | 775 | CE | LYS | E | 47 | 53.966 | 112.782 | 26.715 | 1.00 | 0.00 | AAAA |
| ATOM | 778 | NZ | LYS | E | 47 | 54.881 | 111.744 | 26.204 | 1.00 | 0.00 | AAAA |
| ATOM | 782 | C | LYS | E | 47 | 48.044 | 114.828 | 27.336 | 1.00 | 0.00 | AAAA |
| ATOM | 783 | O | LYS | E | 47 | 47.435 | 114.898 | 26.274 | 1.00 | 0.00 | AAAA |
| ATOM | 784 | N | LEU | E | 48 | 47.501 | 114.417 | 28.488 | 1.00 | 0.00 | AAAA |
| ATOM | 786 | CA | LEU | E | 48 | 46.096 | 114.199 | 28.777 | 1.00 | 0.00 | AAAA |
| ATOM | 788 | CB | LEU | E | 48 | 46.045 | 113.577 | 30.174 | 1.00 | 0.00 | AAAA |
| ATOM | 791 | CG | LEU | E | 48 | 44.724 | 113.047 | 30.740 | 1.00 | 0.00 | AAAA |
| ATOM | 793 | CD1 | LEU | E | 48 | 45.137 | 112.061 | 31.842 | 1.00 | 0.00 | AAAA |
| ATOM | 797 | CD2 | LEU | E | 48 | 43.783 | 114.138 | 31.243 | 1.00 | 0.00 | AAAA |
| ATOM | 801 | C | LEU | E | 48 | 45.340 | 115.501 | 28.573 | 1.00 | 0.00 | AAAA |
| ATOM | 802 | O | LEU | E | 48 | 45.662 | 116.457 | 29.266 | 1.00 | 0.00 | AAAA |
| ATOM | 803 | N | THR | E | 49 | 44.359 | 115.495 | 27.675 | 1.00 | 0.00 | AAAA |
| ATOM | 805 | CA | THR | E | 49 | 43.647 | 116.693 | 27.265 | 1.00 | 0.00 | AAAA |
| ATOM | 807 | CB | THR | E | 49 | 44.050 | 117.181 | 25.882 | 1.00 | 0.00 | AAAA |
| ATOM | 809 | OG1 | THR | E | 49 | 44.098 | 116.070 | 25.011 | 1.00 | 0.00 | AAAA |
| ATOM | 811 | CG2 | THR | E | 49 | 45.408 | 117.903 | 25.848 | 1.00 | 0.00 | AAAA |
| ATOM | 815 | C | THR | E | 49 | 42.149 | 116.477 | 27.334 | 1.00 | 0.00 | AAAA |
| ATOM | 816 | O | THR | E | 49 | 41.386 | 117.434 | 27.445 | 1.00 | 0.00 | AAAA |
| ATOM | 817 | N | VAL | E | 50 | 41.668 | 115.228 | 27.297 | 1.00 | 0.00 | AAAA |
| ATOM | 819 | CA | VAL | E | 50 | 40.291 | 114.820 | 27.157 | 1.00 | 0.00 | AAAA |
| ATOM | 821 | CB | VAL | E | 50 | 39.819 | 115.042 | 25.720 | 1.00 | 0.00 | AAAA |
| ATOM | 823 | CG1 | VAL | E | 50 | 40.504 | 114.172 | 24.661 | 1.00 | 0.00 | AAAA |
| ATOM | 827 | CG2 | VAL | E | 50 | 38.306 | 114.959 | 25.580 | 1.00 | 0.00 | AAAA |
| ATOM | 831 | C | VAL | E | 50 | 39.985 | 113.477 | 27.797 | 1.00 | 0.00 | AAAA |
| ATOM | 832 | O | VAL | E | 50 | 40.725 | 112.512 | 27.647 | 1.00 | 0.00 | AAAA |
| ATOM | 833 | N | ILE | E | 51 | 38.878 | 113.517 | 28.547 | 1.00 | 0.00 | AAAA |
| ATOM | 835 | CA | ILE | E | 51 | 38.061 | 112.445 | 29.071 | 1.00 | 0.00 | AAAA |
| ATOM | 837 | CB | ILE | E | 51 | 37.685 | 112.819 | 30.510 | 1.00 | 0.00 | AAAA |
| ATOM | 839 | CG1 | ILE | E | 51 | 38.880 | 113.095 | 31.439 | 1.00 | 0.00 | AAAA |
| ATOM | 842 | CG2 | ILE | E | 51 | 36.891 | 111.694 | 31.172 | 1.00 | 0.00 | AAAA |
| ATOM | 846 | CD1 | ILE | E | 51 | 39.938 | 111.998 | 31.388 | 1.00 | 0.00 | AAAA |
| ATOM | 850 | C | ILE | E | 51 | 36.792 | 112.607 | 28.249 | 1.00 | 0.00 | AAAA |
| ATOM | 851 | O | ILE | E | 51 | 36.303 | 113.731 | 28.161 | 1.00 | 0.00 | AAAA |
| ATOM | 852 | N | THR | E | 52 | 36.233 | 111.563 | 27.643 | 1.00 | 0.00 | AAAA |
| ATOM | 854 | CA | THR | E | 52 | 35.116 | 111.741 | 26.741 | 1.00 | 0.00 | AAAA |
| ATOM | 856 | CB | THR | E | 52 | 35.079 | 110.690 | 25.629 | 1.00 | 0.00 | AAAA |
| ATOM | 858 | OG1 | THR | E | 52 | 35.086 | 109.339 | 26.021 | 1.00 | 0.00 | AAAA |
| ATOM | 860 | CG2 | THR | E | 52 | 36.279 | 110.905 | 24.710 | 1.00 | 0.00 | AAAA |
| ATOM | 864 | C | THR | E | 52 | 33.816 | 111.548 | 27.507 | 1.00 | 0.00 | AAAA |
| ATOM | 865 | O | THR | E | 52 | 32.795 | 111.944 | 26.946 | 1.00 | 0.00 | AAAA |
| ATOM | 866 | N | GLU | E | 53 | 33.699 | 110.964 | 28.702 | 1.00 | 0.00 | AAAA |
| ATOM | 868 | CA | GLU | E | 53 | 32.499 | 110.771 | 29.499 | 1.00 | 0.00 | AAAA |
| ATOM | 870 | CB | GLU | E | 53 | 32.188 | 109.302 | 29.769 | 1.00 | 0.00 | AAAA |
| ATOM | 873 | CG | GLU | E | 53 | 31.801 | 108.488 | 28.530 | 1.00 | 0.00 | AAAA |
| ATOM | 876 | CD | GLU | E | 53 | 31.353 | 107.071 | 28.850 | 1.00 | 0.00 | AAAA |
| ATOM | 877 | OE1 | GLU | E | 53 | 31.321 | 106.790 | 30.073 | 1.00 | 0.00 | AAAA |
| ATOM | 878 | OE2 | GLU | E | 53 | 30.774 | 106.332 | 28.033 | 1.00 | 0.00 | AAAA |
| ATOM | 879 | C | GLU | E | 53 | 32.742 | 111.680 | 30.696 | 1.00 | 0.00 | AAAA |
| ATOM | 880 | O | GLU | E | 53 | 32.732 | 112.889 | 30.502 | 1.00 | 0.00 | AAAA |
| ATOM | 881 | N | TYR | E | 54 | 32.950 | 111.105 | 31.878 | 1.00 | 0.00 | AAAA |
| ATOM | 883 | CA | TYR | E | 54 | 32.831 | 111.847 | 33.119 | 1.00 | 0.00 | AAAA |
| ATOM | 885 | CB | TYR | E | 54 | 31.459 | 111.723 | 33.786 | 1.00 | 0.00 | AAAA |
| ATOM | 888 | CG | TYR | E | 54 | 30.980 | 110.456 | 34.462 | 1.00 | 0.00 | AAAA |
| ATOM | 889 | CD1 | TYR | E | 54 | 31.723 | 110.031 | 35.574 | 1.00 | 0.00 | AAAA |
| ATOM | 891 | CD2 | TYR | E | 54 | 29.864 | 109.715 | 34.045 | 1.00 | 0.00 | AAAA |
| ATOM | 893 | CE1 | TYR | E | 54 | 31.327 | 108.877 | 36.251 | 1.00 | 0.00 | AAAA |
| ATOM | 895 | CE2 | TYR | E | 54 | 29.432 | 108.564 | 34.723 | 1.00 | 0.00 | AAAA |
| ATOM | 897 | CZ | TYR | E | 54 | 30.261 | 108.122 | 35.759 | 1.00 | 0.00 | AAAA |
| ATOM | 898 | OH | TYR | E | 54 | 30.095 | 106.896 | 36.354 | 1.00 | 0.00 | AAAA |
| ATOM | 900 | C | TYR | E | 54 | 34.108 | 111.655 | 33.925 | 1.00 | 0.00 | AAAA |
| ATOM | 901 | O | TYR | E | 54 | 34.845 | 110.691 | 33.724 | 1.00 | 0.00 | AAAA |
| ATOM | 902 | N | LEU | E | 55 | 34.437 | 112.527 | 34.880 | 1.00 | 0.00 | AAAA |
| ATOM | 904 | CA | LEU | E | 55 | 35.536 | 112.401 | 35.816 | 1.00 | 0.00 | AAAA |
| ATOM | 906 | CB | LEU | E | 55 | 36.618 | 113.454 | 35.543 | 1.00 | 0.00 | AAAA |
| ATOM | 909 | CG | LEU | E | 55 | 37.800 | 113.661 | 36.479 | 1.00 | 0.00 | AAAA |
| ATOM | 911 | CD1 | LEU | E | 55 | 38.630 | 112.480 | 37.004 | 1.00 | 0.00 | AAAA |
| ATOM | 915 | CD2 | LEU | E | 55 | 38.859 | 114.648 | 35.978 | 1.00 | 0.00 | AAAA |
| ATOM | 919 | C | LEU | E | 55 | 34.908 | 112.488 | 37.209 | 1.00 | 0.00 | AAAA |
| ATOM | 920 | O | LEU | E | 55 | 34.241 | 113.464 | 37.540 | 1.00 | 0.00 | AAAA |
| ATOM | 921 | N | LEU | E | 56 | 35.205 | 111.447 | 37.972 | 1.00 | 0.00 | AAAA |
| ATOM | 923 | CA | LEU | E | 56 | 34.696 | 111.221 | 39.318 | 1.00 | 0.00 | AAAA |
| ATOM | 925 | CB | LEU | E | 56 | 33.636 | 110.119 | 39.211 | 1.00 | 0.00 | AAAA |
| ATOM | 928 | CG | LEU | E | 56 | 33.025 | 109.723 | 40.558 | 1.00 | 0.00 | AAAA |
| ATOM | 930 | CD1 | LEU | E | 56 | 31.507 | 109.701 | 40.398 | 1.00 | 0.00 | AAAA |
| ATOM | 934 | CD2 | LEU | E | 56 | 33.541 | 108.372 | 41.038 | 1.00 | 0.00 | AAAA |
| ATOM | 938 | C | LEU | E | 56 | 35.871 | 111.037 | 40.259 | 1.00 | 0.00 | AAAA |
| ATOM | 939 | O | LEU | E | 56 | 36.454 | 109.956 | 40.251 | 1.00 | 0.00 | AAAA |
| ATOM | 940 | N | LEU | E | 57 | 36.189 | 112.053 | 41.055 | 1.00 | 0.00 | AAAA |
| ATOM | 942 | CA | LEU | E | 57 | 37.198 | 111.914 | 42.087 | 1.00 | 0.00 | AAAA |
| ATOM | 944 | CB | LEU | E | 57 | 37.944 | 113.249 | 42.067 | 1.00 | 0.00 | AAAA |
| ATOM | 947 | CG | LEU | E | 57 | 38.955 | 113.493 | 40.948 | 1.00 | 0.00 | AAAA |
| ATOM | 949 | CD1 | LEU | E | 57 | 38.814 | 114.831 | 40.214 | 1.00 | 0.00 | AAAA |
| ATOM | 953 | CD2 | LEU | E | 57 | 40.343 | 113.292 | 41.551 | 1.00 | 0.00 | AAAA |
| ATOM | 957 | C | LEU | E | 57 | 36.469 | 111.720 | 43.405 | 1.00 | 0.00 | AAAA |
| ATOM | 958 | O | LEU | E | 57 | 35.621 | 112.574 | 43.645 | 1.00 | 0.00 | AAAA |
| ATOM | 959 | N | PHE | E | 58 | 36.761 | 110.649 | 44.153 | 1.00 | 0.00 | AAAA |
| ATOM | 961 | CA | PHE | E | 58 | 36.074 | 110.362 | 45.395 | 1.00 | 0.00 | AAAA |
| ATOM | 963 | CB | PHE | E | 58 | 35.306 | 109.030 | 45.376 | 1.00 | 0.00 | AAAA |
| ATOM | 966 | CG | PHE | E | 58 | 34.647 | 108.629 | 46.667 | 1.00 | 0.00 | AAAA |
| ATOM | 967 | CD1 | PHE | E | 58 | 33.911 | 109.567 | 47.400 | 1.00 | 0.00 | AAAA |
| ATOM | 969 | CD2 | PHE | E | 58 | 34.883 | 107.345 | 47.197 | 1.00 | 0.00 | AAAA |
| ATOM | 971 | CE1 | PHE | E | 58 | 33.216 | 109.171 | 48.547 | 1.00 | 0.00 | AAAA |
| ATOM | 973 | CE2 | PHE | E | 58 | 34.233 | 106.984 | 48.372 | 1.00 | 0.00 | AAAA |
| ATOM | 975 | CZ | PHE | E | 58 | 33.369 | 107.865 | 49.034 | 1.00 | 0.00 | AAAA |
| ATOM | 977 | C | PHE | E | 58 | 37.172 | 110.225 | 46.439 | 1.00 | 0.00 | AAAA |
| ATOM | 978 | O | PHE | E | 58 | 37.937 | 109.266 | 46.414 | 1.00 | 0.00 | AAAA |
| ATOM | 979 | N | ARG | E | 59 | 37.363 | 111.191 | 47.345 | 1.00 | 0.00 | AAAA |
| ATOM | 981 | CA | ARG | E | 59 | 38.127 | 111.125 | 48.571 | 1.00 | 0.00 | AAAA |
| ATOM | 983 | CB | ARG | E | 59 | 37.434 | 110.078 | 49.454 | 1.00 | 0.00 | AAAA |
| ATOM | 986 | CG | ARG | E | 59 | 37.644 | 110.223 | 50.959 | 1.00 | 0.00 | AAAA |
| ATOM | 989 | CD | ARG | E | 59 | 36.792 | 109.233 | 51.745 | 1.00 | 0.00 | AAAA |
| ATOM | 992 | NE | ARG | E | 59 | 37.712 | 108.253 | 52.330 | 1.00 | 0.00 | AAAA |
| ATOM | 994 | CZ | ARG | E | 59 | 37.374 | 107.154 | 53.020 | 1.00 | 0.00 | AAAA |
| ATOM | 995 | NH1 | ARG | E | 59 | 36.091 | 106.834 | 53.238 | 1.00 | 0.00 | AAAA |
| ATOM | 998 | NH2 | ARG | E | 59 | 38.369 | 106.330 | 53.357 | 1.00 | 0.00 | AAAA |
| ATOM | 1001 | C | ARG | E | 59 | 39.597 | 110.813 | 48.355 | 1.00 | 0.00 | AAAA |
| ATOM | 1002 | O | ARG | E | 59 | 40.267 | 110.117 | 49.120 | 1.00 | 0.00 | AAAA |
| ATOM | 1003 | N | VAL | E | 60 | 40.137 | 111.435 | 47.310 | 1.00 | 0.00 | AAAA |
| ATOM | 1005 | CA | VAL | E | 60 | 41.569 | 111.539 | 47.106 | 1.00 | 0.00 | AAAA |
| ATOM | 1007 | CB | VAL | E | 60 | 41.728 | 111.920 | 45.638 | 1.00 | 0.00 | AAAA |
| ATOM | 1009 | CG1 | VAL | E | 60 | 43.179 | 112.005 | 45.171 | 1.00 | 0.00 | AAAA |
| ATOM | 1013 | CG2 | VAL | E | 60 | 40.843 | 111.091 | 44.705 | 1.00 | 0.00 | AAAA |
| ATOM | 1017 | C | VAL | E | 60 | 42.224 | 112.550 | 48.032 | 1.00 | 0.00 | AAAA |
| ATOM | 1018 | O | VAL | E | 60 | 41.750 | 113.691 | 48.121 | 1.00 | 0.00 | AAAA |
| ATOM | 1019 | N | ALA | E | 61 | 43.221 | 112.194 | 48.851 | 1.00 | 0.00 | AAAA |
| ATOM | 1021 | CA | ALA | E | 61 | 43.983 | 112.984 | 49.799 | 1.00 | 0.00 | AAAA |
| ATOM | 1023 | CB | ALA | E | 61 | 43.969 | 112.089 | 51.044 | 1.00 | 0.00 | AAAA |
| ATOM | 1027 | C | ALA | E | 61 | 45.429 | 113.029 | 49.332 | 1.00 | 0.00 | AAAA |
| ATOM | 1028 | O | ALA | E | 61 | 45.925 | 112.063 | 48.753 | 1.00 | 0.00 | AAAA |
| ATOM | 1029 | N | GLY | E | 62 | 46.032 | 114.194 | 49.587 | 1.00 | 0.00 | AAAA |
| ATOM | 1031 | CA | GLY | E | 62 | 47.451 | 114.360 | 49.360 | 1.00 | 0.00 | AAAA |
| ATOM | 1034 | C | GLY | E | 62 | 47.773 | 115.345 | 48.244 | 1.00 | 0.00 | AAAA |
| ATOM | 1035 | O | GLY | E | 62 | 48.891 | 115.830 | 48.171 | 1.00 | 0.00 | AAAA |
| ATOM | 1036 | N | LEU | E | 63 | 46.835 | 115.636 | 47.347 | 1.00 | 0.00 | AAAA |
| ATOM | 1038 | CA | LEU | E | 63 | 47.022 | 116.603 | 46.287 | 1.00 | 0.00 | AAAA |
| ATOM | 1040 | CB | LEU | E | 63 | 45.815 | 116.650 | 45.356 | 1.00 | 0.00 | AAAA |
| ATOM | 1043 | CG | LEU | E | 63 | 45.632 | 115.349 | 44.582 | 1.00 | 0.00 | AAAA |
| ATOM | 1045 | CD1 | LEU | E | 63 | 44.185 | 115.344 | 44.071 | 1.00 | 0.00 | AAAA |
| ATOM | 1049 | CD2 | LEU | E | 63 | 46.575 | 115.058 | 43.410 | 1.00 | 0.00 | AAAA |
| ATOM | 1053 | C | LEU | E | 63 | 47.071 | 118.020 | 46.845 | 1.00 | 0.00 | AAAA |
| ATOM | 1054 | O | LEU | E | 63 | 46.428 | 118.293 | 47.857 | 1.00 | 0.00 | AAAA |
| ATOM | 1055 | N | GLU | E | 64 | 47.729 | 118.997 | 46.219 | 1.00 | 0.00 | AAAA |
| ATOM | 1057 | CA | GLU | E | 64 | 47.731 | 120.410 | 46.541 | 1.00 | 0.00 | AAAA |
| ATOM | 1059 | CB | GLU | E | 64 | 49.043 | 121.140 | 46.235 | 1.00 | 0.00 | AAAA |
| ATOM | 1062 | CG | GLU | E | 64 | 49.276 | 122.364 | 47.119 | 1.00 | 0.00 | AAAA |
| ATOM | 1065 | CD | GLU | E | 64 | 49.814 | 121.986 | 48.488 | 1.00 | 0.00 | AAAA |
| ATOM | 1066 | OE1 | GLU | E | 64 | 50.021 | 122.963 | 49.247 | 1.00 | 0.00 | AAAA |
| ATOM | 1067 | OE2 | GLU | E | 64 | 49.981 | 120.820 | 48.904 | 1.00 | 0.00 | AAAA |
| ATOM | 1068 | C | GLU | E | 64 | 46.607 | 121.090 | 45.763 | 1.00 | 0.00 | AAAA |
| ATOM | 1069 | O | GLU | E | 64 | 45.870 | 121.971 | 46.184 | 1.00 | 0.00 | AAAA |
| ATOM | 1070 | N | SER | E | 65 | 46.549 | 120.646 | 44.508 | 1.00 | 0.00 | AAAA |
| ATOM | 1072 | CA | SER | E | 65 | 45.814 | 121.181 | 43.383 | 1.00 | 0.00 | AAAA |
| ATOM | 1074 | CB | SER | E | 65 | 46.602 | 122.327 | 42.752 | 1.00 | 0:00 | AAAA |
| ATOM | 1077 | OG | SER | E | 65 | 46.126 | 122.838 | 41.526 | 1.00 | 0.00 | AAAA |
| ATOM | 1079 | C | SER | E | 65 | 45.438 | 120.062 | 42.422 | 1.00 | 0.00 | AAAA |
| ATOM | 1080 | O | SER | E | 65 | 46.249 | 119.224 | 42.024 | 1.00 | 0.00 | AAAA |
| ATOM | 1081 | N | LEU | E | 66 | 44.241 | 120.225 | 41.853 | 1.00 | 0.00 | AAAA |
| ATOM | 1083 | CA | LEU | E | 66 | 43.875 | 119.377 | 40.739 | 1.00 | 0.00 | AAAA |
| ATOM | 1085 | CB | LEU | E | 66 | 42.345 | 119.427 | 40.627 | 1.00 | 0.00 | AAAA |
| ATOM | 1088 | CG | LEU | E | 66 | 41.779 | 118.171 | 39.933 | 1.00 | 0.00 | AAAA |
| ATOM | 1090 | CD1 | LEU | E | 66 | 41.987 | 116.898 | 40.767 | 1.00 | 0.00 | AAAA |
| ATOM | 1094 | CD2 | LEU | E | 66 | 40.337 | 118.227 | 39.506 | 1.00 | 0.00 | AAAA |
| ATOM | 1098 | C | LEU | E | 66 | 44.516 | 119.881 | 39.454 | 1.00 | 0.00 | AAAA |
| ATOM | 1099 | O | LEU | E | 66 | 44.672 | 119.010 | 38.606 | 1.00 | 0.00 | AAAA |
| ATOM | 1100 | N | GLY | E | 67 | 45.051 | 121.106 | 39.371 | 1.00 | 0.00 | AAAA |
| ATOM | 1102 | CA | GLY | E | 67 | 45.770 | 121.520 | 38.191 | 1.00 | 0.00 | AAAA |
| ATOM | 1105 | C | GLY | E | 67 | 47.268 | 121.252 | 38.318 | 1.00 | 0.00 | AAAA |
| ATOM | 1106 | O | GLY | E | 67 | 47.962 | 121.426 | 37.315 | 1.00 | 0.00 | AAAA |
| ATOM | 1107 | N | ASP | E | 68 | 47.743 | 120.682 | 39.426 | 1.00 | 0.00 | AAAA |
| ATOM | 1109 | CA | ASP | E | 68 | 49.039 | 120.062 | 39.617 | 1.00 | 0.00 | AAAA |
| ATOM | 1111 | CB | ASP | E | 68 | 49.421 | 120.171 | 41.096 | 1.00 | 0.00 | AAAA |
| ATOM | 1114 | CG | ASP | E | 68 | 50.782 | 119.742 | 41.633 | 1.00 | 0.00 | AAAA |
| ATOM | 1115 | OD1 | ASP | E | 68 | 50.817 | 118.696 | 42.316 | 1.00 | 0.00 | AAAA |
| ATOM | 1116 | OD2 | ASP | E | 68 | 51.754 | 120.512 | 41.531 | 1.00 | 0.00 | AAAA |
| ATOM | 1117 | C | ASP | E | 68 | 49.073 | 118.586 | 39.251 | 1.00 | 0.00 | AAAA |
| ATOM | 1118 | O | ASP | E | 68 | 50.106 | 117.944 | 39.110 | 1.00 | 0.00 | AAAA |
| ATOM | 1119 | N | LEU | E | 69 | 47.904 | 118.050 | 38.908 | 1.00 | 0.00 | AAAA |
| ATOM | 1121 | CA | LEU | E | 69 | 47.586 | 116.760 | 38.319 | 1.00 | 0.00 | AAAA |
| ATOM | 1123 | CB | LEU | E | 69 | 46.552 | 115.988 | 39.139 | 1.00 | 0.00 | AAAA |
| ATOM | 1126 | CG | LEU | E | 69 | 46.174 | 114.604 | 38.589 | 1.00 | 0.00 | AAAA |
| ATOM | 1128 | CD1 | LEU | E | 69 | 47.279 | 113.561 | 38.711 | 1.00 | 0.00 | AAAA |
| ATOM | 1132 | CD2 | LEU | E | 69 | 45.042 | 113.932 | 39.373 | 1.00 | 0.00 | AAAA |
| ATOM | 1136 | C | LEU | E | 69 | 47.154 | 116.908 | 36.866 | 1.00 | 0.00 | AAAA |
| ATOM | 1137 | O | LEU | E | 69 | 47.663 | 116.183 | 36.018 | 1.00 | 0.00 | AAAA |
| ATOM | 1138 | N | PHE | E | 70 | 46.180 | 117.778 | 36.612 | 1.00 | 0.00 | AAAA |
| ATOM | 1140 | CA | PHE | E | 70 | 45.556 | 117.933 | 35.318 | 1.00 | 0.00 | AAAA |
| ATOM | 1142 | CB | PHE | E | 70 | 44.165 | 117.303 | 35.469 | 1.00 | 0.00 | AAAA |
| ATOM | 1145 | CG | PHE | E | 70 | 43.965 | 115.823 | 35.645 | 1.00 | 0.00 | AAAA |
| ATOM | 1146 | CD1 | PHE | E | 70 | 44.666 | 114.838 | 34.940 | 1.00 | 0.00 | AAAA |
| ATOM | 1148 | CD2 | PHE | E | 70 | 42.973 | 115.368 | 36.524 | 1.00 | 0.00 | AAAA |
| ATOM | 1150 | CE1 | PHE | E | 70 | 44.557 | 113.469 | 35.231 | 1.00 | 0.00 | AAAA |
| ATOM | 1152 | CE2 | PHE | E | 70 | 42.830 | 113.993 | 36.793 | 1.00 | 0.00 | AAAA |
| ATOM | 1154 | CZ | PHE | E | 70 | 43.576 | 113.013 | 36.129 | 1.00 | 0.00 | AAAA |
| ATOM | 1156 | C | PHE | E | 70 | 45.575 | 119.373 | 34.830 | 1.00 | 0.00 | AAAA |
| ATOM | 1157 | O | PHE | E | 70 | 44.527 | 119.989 | 34.612 | 1.00 | 0.00 | AAAA |
| ATOM | 1158 | N | PRO | E | 71 | 46.760 | 119.854 | 34.441 | 1.00 | 0.00 | AAAA |
| ATOM | 1159 | CA | PRO | E | 71 | 46.893 | 121.193 | 33.914 | 1.00 | 0.00 | AAAA |
| ATOM | 1161 | CB | PRO | E | 71 | 48.398 | 121.488 | 33.954 | 1.00 | 0.00 | AAAA |
| ATOM | 1164 | CG | PRO | E | 71 | 49.129 | 120.152 | 33.902 | 1.00 | 0.00 | AAAA |
| ATOM | 1167 | CD | PRO | E | 71 | 48.071 | 119.234 | 34.521 | 1.00 | 0.00 | AAAA |
| ATOM | 1170 | C | PRO | E | 71 | 46.412 | 121.335 | 32.476 | 1.00 | 0.00 | AAAA |
| ATOM | 1171 | O | PRO | E | 71 | 46.080 | 122.456 | 32.083 | 1.00 | 0.00 | AAAA |
| ATOM | 1172 | N | ASN | E | 72 | 46.335 | 120.276 | 31.675 | 1.00 | 0.00 | AAAA |
| ATOM | 1174 | CA | ASN | E | 72 | 46.026 | 120.290 | 30.251 | 1.00 | 0.00 | AAAA |
| ATOM | 1176 | CB | ASN | E | 72 | 47.179 | 119.653 | 29.467 | 1.00 | 0.00 | AAAA |
| ATOM | 1179 | CG | ASN | E | 72 | 48.379 | 120.583 | 29.376 | 1.00 | 0.00 | AAAA |
| ATOM | 1180 | OD1 | ASN | E | 72 | 49.143 | 120.820 | 30.308 | 1.00. | 0.00 | AAAA |
| ATOM | 1181 | ND2 | ASN | E | 72 | 48.486 | 121.297 | 28.252 | 1.00 | 0.00 | AAAA |
| ATOM | 1184 | C | ASN | E | 72 | 44.656 | 119.744 | 29.882 | 1.00 | 0.00 | AAAA |
| ATOM | 1185 | O | ASN | E | 72 | 44.259 | 119.663 | 28.717 | 1.00 | 0.00 | AAAA |
| ATOM | 1186 | N | LEU | E | 73 | 43.857 | 119.469 | 30.915 | 1.00 | 0.00 | AAAA |
| ATOM | 1188 | CA | LEU | E | 73 | 42.455 | 119.094 | 30.853 | 1.00 | 0.00 | AAAA |
| ATOM | 1190 | CB | LEU | E | 73 | 41.953 | 118.917 | 32.286 | 1.00 | 0.00 | AAAA |
| ATOM | 1193 | CG | LEU | E | 73 | 40.527 | 118.381 | 32.432 | 1.00 | 0.00 | AAAA |
| ATOM | 1195 | CD1 | LEU | E | 73 | 40.210 | 117.021 | 31.813 | 1.00 | 0.00 | AAAA |
| ATOM | 1199 | CD2 | LEU | E | 73 | 40.042 | 118.343 | 33.880 | 1.00 | 0.00 | AAAA |
| ATOM | 1203 | C | LEU | E | 73 | 41.745 | 120.225 | 30.123 | 1.00 | 0.00 | AAAA |
| ATOM | 1204 | O | LEU | E | 73 | 41.484 | 121.279 | 30.709 | 1.00 | 0.00 | AAAA |
| ATOM | 1205 | N | THR | E | 74 | 41.433 | 120.058 | 28.839 | 1.00 | 0.00 | AAAA |
| ATOM | 1207 | CA | THR | E | 74 | 41.064 | 121.078 | 27.873 | 1.00 | 0.00 | AAAA |
| ATOM | 1209 | CB | THR | E | 74 | 41.601 | 120.678 | 26.496 | 1.00 | 0.00 | AAAA |
| ATOM | 1211 | OG1 | THR | E | 74 | 43.006 | 120.511 | 26.542 | 1.00 | 0.00 | AAAA |
| ATOM | 1213 | CG2 | THR | E | 74 | 41.202 | 121.633 | 25.369 | 1.00 | 0.00 | AAAA |
| ATOM | 1217 | C | THR | E | 74 | 39.539 | 121.096 | 27.875 | 1.00 | 0.00 | AAAA |
| ATOM | 1218 | O | THR | E | 74 | 38.935 | 122.168 | 27.930 | 1.00 | 0.00 | AAAA |
| ATOM | 1219 | N | VAL | E | 75 | 38.932 | 119.920 | 27.714 | 1.00 | 0.00 | AAAA |
| ATOM | 1221 | CA | VAL | E | 75 | 37.502 | 119.709 | 27.575 | 1.00 | 0.00 | AAAA |
| ATOM | 1223 | CB | VAL | E | 75 | 37.257 | 119.799 | 26.069 | 1.00 | 0.00 | AAAA |
| ATOM | 1225 | CG1 | VAL | E | 75 | 37.943 | 118.776 | 25.163 | 1.00 | 0.00 | AAAA |
| ATOM | 1229 | CG2 | VAL | E | 75 | 35.785 | 119.896 | 25.645 | 1.00 | 0.00 | AAAA |
| ATOM | 1233 | C | VAL | E | 75 | 37.146 | 118.361 | 28.182 | 1.00 | 0.00 | AAAA |
| ATOM | 1234 | O | VAL | E | 75 | 37.987 | 117.475 | 28.252 | 1.00 | 0.00 | AAAA |
| ATOM | 1235 | N | ILE | E | 76 | 35.869 | 118.200 | 28.577 | 1.00 | 0.00 | AAAA |
| ATOM | 1237 | CA | ILE | E | 76 | 35.241 | 116.902 | 28.769 | 1.00 | 0.00 | AAAA |
| ATOM | 1239 | CB | ILE | E | 76 | 34.839 | 116.776 | 30.242 | 1.00 | 0.00 | AAAA |
| ATOM | 1241 | CG1 | ILE | E | 76 | 35.901 | 117.104 | 31.284 | 1.00 | 0.00 | AAAA |
| ATOM | 1244 | CG2 | ILE | E | 76 | 34.204 | 115.417 | 30.578 | 1.00 | 0.00 | AAAA |
| ATOM | 1248 | CD1 | ILE | E | 76 | 35.478 | 117.251 | 32.740 | 1.00 | 0.00 | AAAA |
| ATOM | 1252 | C | ILE | E | 76 | 34.074 | 116.935 | 27.794 | 1.00 | 0.00 | AAAA |
| ATOM | 1253 | O | ILE | E | 76 | 33.399 | 117.956 | 27.731 | 1.00 | 0.00 | AAAA |
| ATOM | 1254 | N | ARG | E | 77 | 33.916 | 115.935 | 26.923 | 1.00 | 0.00 | AAAA |
| ATOM | 1256 | CA | ARG | E | 77 | 32.859 | 115.867 | 25.931 | 1.00 | 0.00 | AAAA |
| ATOM | 1258 | CB | ARG | E | 77 | 33.112 | 114.737 | 24.933 | 1.00 | 0.00 | AAAA |
| ATOM | 1261 | CG | ARG | E | 77 | 34.536 | 114.574 | 24.414 | 1.00 | 0.00 | AAAA |
| ATOM | 1264 | CD | ARG | E | 77 | 34.797 | 115.734 | 23.453 | 1.00 | 0.00 | AAAA |
| ATOM | 1267 | NE | ARG | E | 77 | 36.102 | 115.493 | 22.846 | 1.00 | 0.00 | AAAA |
| ATOM | 1269 | CZ | ARG | E | 77 | 36.906 | 116.494 | 22.438 | 1.00 | 0.00 | AAAA |
| ATOM | 1270 | NH1 | ARG | E | 77 | 36.584 | 117.800 | 22.488 | 1.00 | 0.00 | AAAA |
| ATOM | 1273 | NH2 | ARG | E | 77 | 38.093 | 116.183 | 21.924 | 1.00 | 0.00 | AAAA |
| ATOM | 1276 | C | ARG | E | 77 | 31.499 | 115.631 | 26.563 | 1.00 | 0.00 | AAAA |
| ATOM | 1277 | O | ARG | E | 77 | 30.505 | 116.165 | 26.073 | 1.00 | 0.00 | AAAA |
| ATOM | 1278 | N | GLY | E | 78 | 31.438 | 114.952 | 27.715 | 1.00 | 0.00 | AAAA |
| ATOM | 1280 | CA | GLY | E | 78 | 30.226 | 114.730 | 28.471 | 1.00 | 0.00 | AAAA |
| ATOM | 1283 | C | GLY | E | 78 | 29.352 | 113.707 | 27.761 | 1.00 | 0.00 | AAAA |
| ATOM | 1284 | O | GLY | E | 78 | 28.132 | 113.857 | 27.671 | 1.00 | 0.00 | AAAA |
| ATOM | 1285 | N | TRP | E | 79 | 29.873 | 112.597 | 27.231 | 1.00 | 0.00 | AAAA |
| ATOM | 1287 | CA | TRP | E | 79 | 29.151 | 111.681 | 26.364 | 1.00 | 0.00 | AAAA |
| ATOM | 1289 | CB | TRP | E | 79 | 30.100 | 110.834 | 25.528 | 1.00 | 0.00 | AAAA |
| ATOM | 1292 | CG | TRP | E | 79 | 29.596 | 109.970 | 24.419 | 1.00 | 0.00 | AAAA |
| ATOM | 1293 | CD1 | TRP | E | 79 | 29.104 | 110.512 | 23.277 | 1.00 | 0.00 | AAAA |
| ATOM | 1295 | CD2 | TRP | E | 79 | 29.490 | 108.525 | 24.282 | 1.00 | 0.00 | AAAA |
| ATOM | 1296 | NE1 | TRP | E | 79 | 28.663 | 109.498 | 22.447 | 1.00 | 0.00 | AAAA |
| ATOM | 1298 | CE2 | TRP | E | 79 | 28.943 | 108.270 | 22.991 | 1.00 | 0.00 | AAAA |
| ATOM | 1299 | CE3 | TRP | E | 79 | 29.833 | 107.408 | 25.055 | 1.00 | 0.00 | AAAA |
| ATOM | 1301 | CZ2 | TRP | E | 79 | 28.839 | 106.979 | 22.462 | 1.00 | 0.00 | AAAA |
| ATOM | 1303 | CZ3 | TRP | E | 79 | 29.602 | 106.109 | 24.587 | 1.00 | 0.00 | AAAA |
| ATOM | 1305 | CH2 | TRP | E | 79 | 29.056 | 105.894 | 23.317 | 1.00 | 0.00 | AAAA |
| ATOM | 1307 | C | TRP | E | 79 | 28.327 | 110.649 | 27.122 | 1.00 | 0.00 | AAAA |
| ATOM | 1308 | O | TRP | E | 79 | 27.347 | 110.126 | 26.591 | 1.00 | 0.00 | AAAA |
| ATOM | 1309 | N | LYS | E | 80 | 28.583 | 110.440 | 28.413 | 1.00 | 0.00 | AAAA |
| ATOM | 1311 | CA | LYS | E | 80 | 27.715 | 109.941 | 29.461 | 1.00 | 0.00 | AAAA |
| ATOM | 1313 | CB | LYS | E | 80 | 27.854 | 108.428 | 29.614 | 1.00 | 0.00 | AAAA |
| ATOM | 1316 | CG | LYS | E | 80 | 26.871 | 107.751 | 30.571 | 1.00 | 0.00 | AAAA |
| ATOM | 1319 | CD | LYS | E | 80 | 26.286 | 106.416 | 30.083 | 1.00 | 0.00 | AAAA |
| ATOM | 1322 | CE | LYS | E | 80 | 27.353 | 105.326 | 30.183 | 1.00 | 0.00 | AAAA |
| ATOM | 1325 | NZ | LYS | E | 80 | 27.566 | 104.960 | 31.586 | 1.00 | 0.00 | AAAA |
| ATOM | 1329 | C | LYS | E | 80 | 28.044 | 110.740 | 30.710 | 1.00 | 0.00 | AAAA |
| ATOM | 1330 | O | LYS | E | 80 | 29.099 | 111.360 | 30.804 | 1.00 | 0.00 | AAAA |
| ATOM | 1331 | N | LEU | E | 81 | 27.068 | 110.876 | 31.603 | 1.00 | 0.00 | AAAA |
| ATOM | 1333 | CA | LEU | E | 81 | 27.097 | 111.735 | 32.768 | 1.00 | 0.00 | AAAA |
| ATOM | 1335 | CB | LEU | E | 81 | 26.336 | 113.038 | 32.500 | 1.00 | 0.00 | AAAA |
| ATOM | 1338 | CG | LEU | E | 81 | 26.816 | 113.828 | 31.284 | 1.00 | 0.00 | AAAA |
| ATOM | 1340 | CD1 | LEU | E | 81 | 25.937 | 115.041 | 30.979 | 1.00 | 0.00 | AAAA |
| ATOM | 1344 | CD2 | LEU | E | 81 | 28.208 | 114.424 | 31.449 | 1.00 | 0.00 | AAAA |
| ATOM | 1348 | C | LEU | E | 81 | 26.639 | 111.083 | 34.064 | 1.00 | 0.00 | AAAA |
| ATOM | 1349 | O | LEU | E | 81 | 26.023 | 110.019 | 33.987 | 1.00 | 0.00 | AAAA |
| ATOM | 1350 | N | PHE | E | 82 | 27.109 | 111.568 | 35.219 | 1.00 | 0.00 | AAAA |
| ATOM | 1352 | CA | PHE | E | 82 | 26.696 | 111.263 | 36.573 | 1.00 | 0.00 | AAAA |
| ATOM | 1354 | CB | PHE | E | 82 | 28.000 | 111.179 | 37.355 | 1.00 | 0.00 | AAAA |
| ATOM | 1357 | CG | PHE | E | 82 | 27.918 | 110.650 | 38.775 | -1.00 | 0.00 | AAAA |
| ATOM | 1358 | CD1 | PHE | E | 82 | 27.956 | 109.267 | 39.008 | 1.00 | 0.00 | AAAA |
| ATOM | 1360 | CD2 | PHE | E | 82 | 27.899 | 111.525 | 39.870 | 1.00 | 0.00 | AAAA |
| ATOM | 1362 | CE1 | PHE | E | 82 | 28.049 | 108.813 | 40.324 | 1.00 | 0.00 | AAAA |
| ATOM | 1364 | CE2 | PHE | E | 82 | 28.025 | 111.087 | 41.195 | 1.00 | 0.00 | AAAA |
| ATOM | 1366 | CZ | PHE | E | 82 | 28.079 | 109.703 | 41.404 | 1.00 | 0.00 | AAAA |
| ATOM | 1368 | C | PHE | E | 82 | 25.805 | 112.379 | 37.096 | 1.00 | 0.00 | AAAA |
| ATOM | 1369 | O | PHE | E | 82 | 26.374 | 113.374 | 37.555 | 1.00 | 0.00 | AAAA |
| ATOM | 1370 | N | TYR | E | 83 | 24.488 | 112.217 | 37.030 | 1.00 | 0.00 | AAAA |
| ATOM | 1372 | CA | TYR | E | 83 | 23.493 | 113.122 | 37.567 | 1.00 | 0.00 | AAAA |
| ATOM | 1374 | CB | TYR | E | 83 | 23.625 | 113.385 | 39.063 | 1.00 | 0.00 | AAAA |
| ATOM | 1377 | CG | TYR | E | 83 | 23.312 | 112.211 | 39.954 | 1.00 | 0.00 | AAAA |
| ATOM | 1378 | CD1 | TYR | E | 83 | 22.044 | 111.937 | 40.481 | 1.00 | 0.00 | AAAA |
| ATOM | 1380 | CD2 | TYR | E | 83 | 24.414 | 111.437 | 40.337 | 1.00 | 0.00 | AAAA |
| ATOM | 1382 | CE1 | TYR | E | 83 | 21.884 | 110.809 | 41.279 | 1.00 | 0.00 | AAAA |
| ATOM | 1384 | CE2 | TYR | E | 83 | 24.305 | 110.373 | 41.250 | 1.00 | 0.00 | AAAA |
| ATOM | 1386 | CZ | TYR | E | 83 | 22.994 | 110.034 | 41.632 | 1.00 | 0.00 | AAAA |
| ATOM | 1387 | OH | TYR | E | 83 | 22.769 | 108.824 | 42.210 | 1.00 | 0.00 | AAAA |
| ATOM | 1389 | C | TYR | E | 83 | 23.362 | 114.448 | 36.817 | 1.00 | 0.00 | AAAA |
| ATOM | 1390 | O | TYR | E | 83 | | 115.366 | 37.239 | 1.00 | 0.00 | AAAA |
| ATOM | 1391 | N | ASN | E | 84 | 24.057 | 114.583 | 35.684 | 1.00 | 0.00 | AAAA |
| ATOM | 1393 | CA | ASN | E | 84 | 24.197 | 115.734 | 34.811 | 1.00 | 0.00 | AAAA |
| ATOM | 1395 | CB | ASN | E | 84 | 23.097 | 116.797 | 34.703 | 1.00 | 0.00 | AAAA |
| ATOM | 1398 | CG | ASN | E | 84 | 23.076 | 117.656 | 33.449 | 1.00 | 0.00 | AAAA |
| ATOM | 1399 | OD1 | ASN | E | 84 | 23.003 | 117.071 | 32.373 | 1.00 | 0.00 | AAAA |
| ATOM | 1400 | ND2 | ASN | E | 84 | 23.247 | 118.964 | 33.613 | 1.00 | 0.00 | AAAA |
| ATOM | 1403 | C | ASN | E | 84 | 25.635 | 116.196 | 34.905 | 1.00 | 0.00 | AAAA |
| ATOM | 1404 | O | ASN | E | 84 | 26.084 | 116.885 | 33.988 | 1.00 | 0.00 | AAAA |
| ATOM | 1405 | N | TYR | E | 85 | 26.407 | 115.899 | 35.957 | 1.00 | 0.00 | AAAA |
| ATOM | 1407 | CA | TYR | E | 85 | 27.739 | 116.339 | 36.330 | 1.00 | 0.00 | AAAA |
| ATOM | 1409 | CB | TYR | E | 85 | 28.014 | 116.207 | 37.823 | 1.00 | 0.00 | AAAA |
| ATOM | 1412 | CG | TYR | E | 85 | 26.863 | 116.783 | 38.611 | 1.00 | 0.00 | AAAA |
| ATOM | 1413 | CD1 | TYR | E | 85 | 26.434 | 118.102 | 38.480 | 1.00 | 0.00 | AAAA |
| ATOM | 1415 | CD2 | TYR | E | 85 | 26.331 | 116.050 | 39.680 | 1.00 | 0.00 | AAAA |
| ATOM | 1417 | CE1 | TYR | E | 85 | 25.429 | 118.653 | 39.283 | 1.00 | 0.00 | AAAA |
| ATOM | 1419 | CE2 | TYR | E | 85 | 25.221 | 116.552 | 40.379 | 1.00 | 0.00 | AAAA |
| ATOM | 1421 | CZ | TYR | E | 85 | 24.735 | 117.848 | 40.187 | 1.00 | 0.00 | AAAA |
| ATOM | 1422 | OH | TYR | E | 85 | 23.718 | 118.310 | 40.975 | 1.00 | 0.00 | AAAA |
| ATOM | 1424 | C | TYR | E | 85 | 28.743 | 115.516 | 35.542 | 1.00 | 0.00 | AAAA |
| ATOM | 1425 | O | TYR | E | 85 | 28.760 | 114.287 | 35.633 | 1.00 | 0.00 | AAAA |
| ATOM | 1426 | N | ALA | E | 86 | 29.593 | 116.175 | 34.762 | 1.00 | 0.00 | AAAA |
| ATOM | 1428 | CA | ALA | E | 86 | 30.739 | 115.557 | 34.117 | 1.00 | 0.00 | AAAA |
| ATOM | 1430 | CB | ALA | E | 86 | 31.004 | 116.528 | 32.977 | 1.00 | 0.00 | AAAA |
| ATOM | 1434 | C | ALA | E | 86 | 31.985 | 115.648 | 34.994 | 1.00 | 0.00 | AAAA |
| ATOM | 1435 | O | ALA | E | 86 | 32.967 | 114.963 | 34.726 | 1.00 | 0.00 | AAAA |
| ATOM | 1436 | N | LEU | E | 87 | 31.965 | 116.514 | 36.018 | 1.00 | 0.00 | AAAA |
| ATOM | 1438 | CA | LEU | E | 87 | 33.125 | 116.835 | 36.825 | 1.00 | 0.00 | AAAA |
| ATOM | 1440 | CB | LEU | E | 87 | 33.787 | 118.132 | 36.366 | 1.00 | 0.00 | AAAA |
| ATOM | 1443 | CG | LEU | E | 87 | 35.203 | 118.350 | 36.905 | 1.00 | 0.00 | AAAA |
| ATOM | 1445 | CD1 | LEU | E | 87 | 36.246 | 117.288 | 36.577 | 1.00 | 0.00 | AAAA |
| ATOM | 1449 | CD2 | LEU | E | 87 | 35.826 | 119.729 | 36.643 | 1.00 | 0.00 | AAAA |
| ATOM | 1453 | C | LEU | E | 87 | 32.762 | 116.885 | 38.302 | 1.00 | 0.00 | AAAA |
| ATOM | 1454 | O | LEU | E | 87 | 32.223 | 117.888 | 38.754 | 1.00 | 0.00 | AAAA |
| ATOM | 1455 | N | VAL | E | 88 | 32.915 | 115.788 | 39.055 | 1.00 | 0.00 | AAAA |
| ATOM | 1457 | CA | VAL | E | 88 | 32.501 | 115.483 | 40.409 | 1.00 | 0.00 | AAAA |
| ATOM | 1459 | CB | VAL | E | 88 | 31.640 | 114.220 | 40.432 | 1.00 | 0.00 | AAAA |
| ATOM | 1461 | CG1 | VAL | E | 88 | 30.824 | 114.215 | 41.729 | 1.00 | 0.00 | AAAA |
| ATOM | 1465 | CG2 | VAL | E | 88 | 30.629 | 113.988 | 39.310 | 1.00 | 0.00 | AAAA |
| ATOM | 1469 | C | VAL | E | 88 | 33.760 | 115.442 | 41.266 | 1.00 | 0.00 | AAAA |
| ATOM | 1470 | O | VAL | E | 88 | 34.556 | 114.512 | 41.127 | 1.00 | 0.00 | AAAA |
| ATOM | 1471 | N | ILE | E | 89 | 33.950 | 116.511 | 42.046 | 1.00 | 0.00 | AAAA |
| ATOM | 1473 | CA | ILE | E | 89 | 35.099 | 116.692 | 42.901 | 1.00 | 0.00 | AAAA |
| ATOM | 1475 | CB | ILE | E | 89 | 35.956 | 117.898 | 42.517 | 1.00 | 0.00 | AAAA |
| ATOM | 1477 | CG1 | ILE | E | 89 | 36.273 | 117.782 | 41.025 | 1.00 | 0.00 | AAAA |
| ATOM | 1480 | CG2 | ILE | E | 89 | 37.264 | 117.914 | 43.308 | 1.00 | 0.00 | AAAA |
| ATOM | 1484 | CD1 | ILE | E | 89 | 36.777 | 119.037 | 40.294 | 1.00 | 0.00 | AAAA |
| ATOM | 1488 | C | ILE | E | 89 | 34.474 | 116.678 | 44.291 | 1.00 | 0.00 | AAAA |
| ATOM | 1489 | O | ILE | E | 89 | 34.094 | 117.745 | 44.749 | 1.00 | 0.00 | AAAA |
| ATOM | 1490 | N | PHE | E | 90 | 34.563 | 115.527 | 44.968 | 1.00 | 0.00 | AAAA |
| ATOM | 1492 | CA | PHE | E | 90 | 33.857 | 115.272 | 46.207 | 1.00 | 0.00 | AAAA |
| ATOM | 1494 | CB | PHE | E | 90 | 32.620 | 114.461 | 45.833 | 1.00 | 0.00 | AAAA |
| ATOM | 1497 | CG | PHE | E | 90 | 31.658 | 114.181 | 46.964 | 1.00 | 0.00 | AAAA |
| ATOM | 1498 | CD1 | PHE | E | 90 | 30.924 | 115.221 | 47.548 | 1.00 | 0.00 | AAAA |
| ATOM | 1500 | CD2 | PHE | E | 90 | 31.577 | 112.878 | 47.475 | 1.00 | 0.00 | AAAA |
| ATOM | 1502 | CE1 | PHE | E | 90 | 29.981 | 114.961 | 48.545 | 1.00 | 0.00 | AAAA |
| ATOM | 1504 | CE2 | PHE | E | 90 | 30.711 | 112.610 | 48.536 | 1.00 | 0.00 | AAAA |
| ATOM | 1506 | CZ | PHE | E | 90 | 29.836 | 113.625 | 48.931 | 1.00 | 0.00 | AAAA |
| ATOM | 1508 | C | PHE | E | 90 | 34.714 | 114.635 | 47.298 | 1.00 | 0.00 | AAAA |
| ATOM | 1509 | O | PHE | E | 90 | 35.357 | 113.599 | 47.135 | 1.00 | 0.00 | AAAA |
| ATOM | 1510 | N | GLU | E | 91 | 34.715 | 115.296 | 48.455 | 1.00 | 0.00 | AAAA |
| ATOM | 1512 | CA | GLU | E | 91 | 35.081 | 114.685 | 49.721 | 1.00 | 0.00 | AAAA |
| ATOM | 1514 | CB | GLU | E | 91 | 34.312 | 113.419 | 50.108 | 1.00 | 0.00 | AAAA |
| ATOM | 1517 | CG | GLU | E | 91 | 34.251 | 113.159 | 51.611 | 1.00 | 0.00 | AAAA |
| ATOM | 1520 | CD | GLU | E | 91 | 33.397 | 111.952 | 51.973 | 1.00 | 0.00 | AAAA |
| ATOM | 1521 | OE1 | GLU | E | 91 | 33.808 | 111.319 | 52.970 | 1.00 | 0.00 | AAAA |
| ATOM | 1522 | OE2 | GLU | E | 91 | 32.261 | 111.746 | 51.489 | 1.00 | 0.00 | AAAA |
| ATOM | 1523 | C | GLU | E | 91 | 36.592 | 114.647 | 49.880 | 1.00 | 0.00 | AAAA |
| ATOM | 1524 | O | GLU | E | 91 | 37.078 | 113.828 | 50.648 | 1.00 | 0.00 | AAAA |
| ATOM | 1525 | N | MET | E | 92 | 37.326 | 115.398 | 49.053 | 1.00 | 0.00 | AAAA |
| ATOM | 1527 | CA | MET | E | 92 | 38.767 | 115.348 | 49.009 | 1.00 | 0.00 | AAAA |
| ATOM | 1529 | CB | MET | E | 92 | 39.182 | 115.865 | 47.632 | 1.00 | 0.00 | AAAA |
| ATOM | 1532 | CG | MET | E | 92 | 38.630 | 115.016 | 46.489 | 1.00 | 0.00 | AAAA |
| ATOM | 1535 | SD | MET | E | 92 | 38.988 | 115.661 | 44.839 | 1.00 | 0.00 | AAAA |
| ATOM | 1536 | CE | MET | E | 92 | 40.797 | 115.658 | 44.653 | 1.00 | 0.00 | AAAA |
| ATOM | 1540 | C | MET | E | 92 | 39.453 | 116.246 | 50.033 | 1.00 | 0.00 | AAAA |
| ATOM | 1541 | O | MET | E | 92 | 38.776 | 117.018 | 50.706 | 1.00 | 0.00 | AAAA |
| ATOM | 1542 | N | THR | E | 93 | 40.767 | 116.156 | 50.243 | 1.00 | 0.00 | AAAA |
| ATOM | 1544 | CA | THR | E | 93 | 41.519 | 116.877 | 51.250 | 1.00 | 0.00 | AAAA |
| ATOM | 1546 | CB | THR | E | 93 | 41.330 | 116.148 | 52.584 | 1.00 | 0.00 | AAAA |
| ATOM | 1548 | OG1 | THR | E | 93 | 42.024 | 116.793 | 53.628 | 1.00 | 0.00 | AAAA |
| ATOM | 1550 | CG2 | THR | E | 93 | 41.749 | 114.679 | 52.705 | 1.00 | 0.00 | AAAA |
| ATOM | 1554 | C | THR | E | 93 | 42.927 | 117.267 | 50.821 | 1.00 | 0.00 | AAAA |
| ATOM | 1555 | O | THR | E | 93 | 43.509 | 116.575 | 49.996 | 1.00 | 0.00 | AAAA |
| ATOM | 1556 | N | ASN | E | 94 | 43.427 | 118.358 | 51.397 | 1.00 | 0.00 | AAAA |
| ATOM | 1558 | CA | ASN | E | 94 | 44.692 | 119.011 | 51.112 | 1.00 | 0.00 | AAAA |
| ATOM | 1560 | CB | ASN | E | 94 | 45.922 | 118.099 | 50.998 | 1.00 | 0.00 | AAAA |
| ATOM | 1563 | CG | ASN | E | 94 | 47.267 | 118.780 | 51.210 | 1.00 | 0.00 | AAAA |
| ATOM | 1564 | OD1 | ASN | E | 94 | 47.718 | 119.016 | 52.319 | 1.00 | 0.00 | AAAA |
| ATOM | 1565 | ND2 | ASN | E | 94 | 47.913 | 119.140 | 50.093 | 1.00 | 0.00 | AAAA |
| ATOM | 1568 | C | ASN | E | 94 | 44.603 | 120.026 | 49.985 | 1.00 | 0.00 | AAAA |
| ATOM | 1569 | O | ASN | E | 94 | 45.528 | 120.793 | 49.719 | 1.00 | 0.00 | AAAA |
| ATOM | 1570 | N | LEU | E | 95 | 43.561 | 119.804 | 49.179 | 1.00 | 0.00 | AAAA |
| ATOM | 1572 | CA | LEU | E | 95 | 43.275 | 120.427 | 47.905 | 1.00 | 0.00 | AAAA |
| ATOM | 1574 | CB | LEU | E | 95 | 42.098 | 119.654 | 47.305 | 1.00 | 0.00 | AAAA |
| ATOM | 1577 | CG | LEU | E | 95 | 41.742 | 120.125 | 45.896 | 1.00 | 0.00 | AAAA |
| ATOM | 1579 | CD1 | LEU | E | 95 | 42.698 | 119.681 | 44.789 | 1.00 | 0.00 | AAAA |
| ATOM | 1583 | CD2 | LEU | E | 95 | 40.293 | 119.706 | 45.611 | 1.00 | 0.00 | AAAA |
| ATOM | 1587 | C | LEU | E | 95 | 42.814 | 121.835 | 48.267 | 1.00 | 0.00 | AAAA |
| ATOM | 1588 | O | LEU | E | 95 | 41.674 | 122.103 | 48.633 | 1.00 | 0.00 | AAAA |
| ATOM | 1589 | N | LYS | E | 96 | 43.754 | 122.780 | 48.169 | 1.00 | 0.00 | AAAA |
| ATOM | 1591 | CA | LYS | E | 96 | 43.555 | 124.183 | 48.465 | 1.00 | 0.00 | AAAA |
| ATOM | 1593 | CB | LYS | E | 96 | 44.947 | 124.697 | 48.814 | 1.00 | 0.00 | AAAA |
| ATOM | 1596 | CG | LYS | E | 96 | 45.395 | 124.337 | 50.230 | 1.00 | 0.00 | AAAA |
| ATOM | 1599 | CD | LYS | E | 96 | 46.917 | 124.209 | 50.305 | 1.00 | 0.00 | AAAA |
| ATOM | 1602 | CE | LYS | E | 96 | 47.480 | 123.329 | 51.417 | 1.00 | 0.00 | AAAA |
| ATOM | 1605 | NZ. | LYS | E | 96 | 48.927 | 123.548 | 51.567 | 1.00 | 0.00 | AAAA |
| ATOM | 1609 | C | LYS | E | 96 | 42.923 | 124.949 | 47.313 | 1.00 | 0.00 | AAAA |
| ATOM | 1610 | O | LYS | E | 96 | 42.409 | 126.062 | 47.474 | 1.00 | 0.00 | AAAA |
| ATOM | 1611 | N | ASP | E | 97 | 43.082 | 124.393 | 46.113 | 1.00 | 0.00 | AAAA |
| ATOM | 1613 | CA | ASP | E | 97 | 42.664 | 124.979 | 44.849 | 1.00 | 0.00 | AAAA |
| ATOM | 1615 | CB | ASP | E | 97 | 43.703 | 126.027 | 44.475 | 1.00 | 0.00 | AAAA |
| ATOM | 1618 | CG | ASP | E | 97 | 44.969 | 125.519 | 43.797 | 1.00 | 0.00 | AAAA |
| ATOM | 1619 | OD1 | ASP | E | 97 | 46.079 | 125.940 | 44.191 | 1.00 | 0.00 | AAAA |
| ATOM | 1620 | OD2 | ASP | E | 97 | 44.950 | 124.859 | 42.736 | 1.00 | 0.00 | AAAA |
| ATOM | 1621 | C | ASP | E | 97 | 42.240 | 123.936 | 43.827 | 1.00 | 0.00 | AAAA |
| ATOM | 1622 | O | ASP | E | 97 | 42.579 | 122.756 | 43.950 | 1.00 | 0.00 | AAAA |
| ATOM | 1623 | N | ILE | E | 98 | 41.551 | 124.405 | 42.786 | 1.00 | 0.00 | AAAA |
| ATOM | 1625 | CA | ILE | E | 98 | 41.265 | 123.601 | 41.615 | 1.00 | 0.00 | AAAA |
| ATOM | 1627 | CB | ILE | E | 98 | 39.966 | 124.175 | 41.026 | 1.00 | 0.00 | AAAA |
| ATOM | 1629 | CG1 | ILE | E | 98 | 38.765 | 124.011 | 41.954 | 1.00 | 0.00 | AAAA |
| ATOM | 1632 | CG2 | ILE | E | 98 | 39.688 | 123.624 | 39.630 | 1.00 | 0.00 | AAAA |
| ATOM | 1636 | CD1 | ILE | E | 98 | 38.317 | 122.594 | 42.310 | 1.00 | 0.00 | AAAA |
| ATOM | 1640 | C | ILE | E | 98 | 42.445 | 123.607 | 40.657 | 1.00 | 0.00 | AAAA |
| ATOM | 1641 | O | ILE | E | 98 | 43.043 | 122.556 | 40.417 | 1.00 | 0.00 | AAAA |
| ATOM | 1642 | N | GLY | E | 99 | 42.747 | 124.710 | 39.968 | 1.00 | 0.00 | AAAA |
| ATOM | 1644 | CA | GLY | E | 99 | 43.969 | 124.840 | 39.201 | 1.00 | 0.00 | AAAA |
| ATOM | 1647 | C | GLY | E | 99 | 43.722 | 124.577 | 37.713 | 1.00 | 0.00 | AAAA |
| ATOM | 1648 | O | GLY | E | 99 | 44.674 | 124.794 | 36.965 | 1.00 | 0.00 | AAAA |
| ATOM | 1649 | N | LEU | E | 100 | 42.509 | 124.258 | 37.255 | 1.00 | 0.00 | AAAA |
| ATOM | 1651 | CA | LEU | E | 100 | 42.437 | 123.595 | 35.967 | 1.00 | 0.00 | AAAA |
| ATOM | 1653 | CB | LEU | E | 100 | 41.102 | 122.840 | 35.981 | 1.00 | 0.00 | AAAA |
| ATOM | 1656 | CG | LEU | E | 100 | 40.958 | 121.629 | 36.904 | 1.00 | 0.00 | AAAA |
| ATOM | 1658 | CD1 | LEU | E | 100 | 39.563 | 121.001 | 36.883 | 1.00 | 0.00 | AAAA |
| ATOM | 1662 | CD2 | LEU | E | 100 | 42.076 | 120.597 | 36.793 | 1.00 | 0.00 | AAAA |
| ATOM | 1666 | C | LEU | E | 100 | 42.417 | 124.513 | 34.758 | 1.00 | 0.00 | AAAA |
| ATOM | 1667 | O | LEU | E | 100 | 41.497 | 124.370 | 33.946 | 1.00 | 0.00 | AAAA |
| ATOM | 1668 | N | TYR | E | 101 | 43.296 | 125.495 | 34.594 | 1.00 | 0.00 | AAAA |
| ATOM | 1670 | CA | TYR | E | 101 | 43.365 | 126.543 | 33.595 | 1.00 | 0.00 | AAAA |
| ATOM | 1672 | CB | TYR | E | 101 | 44.633 | 127.383 | 33.748 | 1.00 | 0.00 | AAAA |
| ATOM | 1675 | CG | TYR | E | 101 | 45.895 | 126.672 | 33.330 | 1.00 | 0.00 | AAAA |
| ATOM | 1676 | CD1 | TYR | E | 101 | 46.573 | 127.070 | 32.172 | 1.00 | 0.00 | AAAA |
| ATOM | 1678 | CD2 | TYR | E | 101 | 46.446 | 125.636 | 34.094 | 1.00 | 0.00 | AAAA |
| ATOM | 1680 | CE1 | TYR | E | 101 | 47.762 | 126.446 | 31.786 | 1.00 | 0.00 | AAAA |
| ATOM | 1682 | CE2 | TYR | E | 101 | 47.670 | 125.058 | 33.752 | 1.00 | 0.00 | AAAA |
| ATOM | 1684 | CZ | TYR | E | 101 | 48.363 | 125.494 | 32.614 | 1.00 | 0.00 | AAAA |
| ATOM | 1685 | OH | TYR | E | 101 | 49.592 | 124.946 | 32.359 | 1.00 | 0.00 | AAAA |
| ATOM | 1687 | C | TYR | E | 101 | 43.009 | 126.271 | 32.143 | 1.00 | 0.00 | AAAA |
| ATOM | 1688 | O | TYR | E | 101 | 42.421 | 127.099 | 31.439 | 1.00 | 0.00 | AAAA |
| ATOM | 1689 | N | ASN | E | 102 | 43.267 | 125.065 | 31.647 | 1.00 | 0.00 | AAAA |
| ATOM | 1691 | CA | ASN | E | 102 | 43.032 | 124.721 | 30.256 | 1.00 | 0.00 | AAAA |
| ATOM | 1693 | CB | ASN | E | 102 | 43.855 | 123.510 | 29.801 | 1.00 | 0.00 | AAAA |
| ATOM | 1696 | CG | ASN | E | 102 | 45.115 | 123.917 | 29.056 | 1.00 | 0.00 | AAAA |
| ATOM | 1697 | OD1 | ASN | E | 102 | 44.990 | 124.483 | 27.966 | 1.00 | 0.00 | AAAA |
| ATOM | 1698 | ND2 | ASN | E | 102 | 46.284 | 123.757 | 29.667 | 1.00 | 0.00 | AAAA |
| ATOM | 1701 | C | ASN | E | 102 | 41.578 | 124.403 | 29.938 | 1.00 | 0.00 | AAAA |
| ATOM | 1702 | O | ASN | E | 102 | 41.213 | 124.501 | 28.773 | 1.00 | 0.00 | AAAA |
| ATOM | 1703 | N | LEU | E | 103 | 40.785 | 124.158 | 30.987 | 1.00 | 0.00 | AAAA |
| ATOM | 1705 | CA | LEU | E | 103 | 39.383 | 123.791 | 31.032 | 1.00 | 0.00 | AAAA |
| ATOM | 1707 | CB | LEU | E | 103 | 38.913 | 123.181 | 32.353 | 1.00 | 0.00 | AAAA |
| ATOM | 1710 | CG | LEU | E | 103 | 37.552 | 122.488 | 32.432 | 1.00 | 0.00 | AAAA |
| ATOM | 1712 | CD1 | LEU | E | 103 | 37.453 | 121.434 | 31.339 | 1.00 | 0.00 | AAAA |
| ATOM | 1716 | CD2 | LEU | E | 103 | 37.281 | 121.847 | 33.791 | 1.00 | 0.00 | AAAA |
| ATOM | 1720 | C | LEU | E | 103 | 38.593 | 125.056 | 30.783 | 1.00 | 0.00 | AAAA |
| ATOM | 1721 | O | LEU | E | 103 | 38.249 | 125.733 | 31.756 | 1.00 | 0.00 | AAAA |
| ATOM | 1722 | N | ARG | E | 104 | 38.279 | 125.251 | 29.502 | 1.00 | 0.00 | AAAA |
| ATOM | 1724 | CA | ARG | E | 104 | 37.315 | 126.233 | 29.055 | 1.00 | 0.00 | AAAA |
| ATOM | 1726 | CB | ARG | E | 104 | 38.164 | 127.263 | 28.315 | 1.00 | 0.00 | AAAA |
| ATOM | 1729 | CG | ARG | E | 104 | 38.847 | 128.293 | 29.221 | 1.00 | 0.00 | AAAA |
| ATOM | 1732 | CD | ARG | E | 104 | 40.066 | 129.004 | 28.637 | 1.00 | 0.00 | AAAA |
| ATOM | 1735 | NE | ARG | E | 104 | 40.165 | 130.335 | 29.233 | 1.00 | 0.00 | AAAA |
| ATOM | 1737 | CZ | ARG | E | 104 | 39.807 | 131.569 | 28.841 | 1.00 | 0.00 | AAAA |
| ATOM | 1738 | NH1 | ARG | E | 104 | 39.122 | 131.728 | 27.711 | 1.00 | 0.00 | AAAA |
| ATOM | 1741 | NH2 | ARG | E | 104 | 40.015 | 132.586 | 29.695 | 1.00 | 0.00 | AAAA |
| ATOM | 1744 | C | ARG | E | 104 | 36.061 | 125.750 | 28.327 | 1.00 | 0.00 | AAAA |
| ATOM | 1745 | O | ARG | E | 104 | 35.172 | 126.542 | 28.026 | 1.00 | 0.00 | AAAA |
| ATOM | 1746 | N | ASN | E | 105 | 35.804 | 124.449 | 28.274 | 1.00 | 0.00 | AAAA |
| ATOM | 1748 | CA | ASN | E | 105 | 34.571 | 123.880 | 27.769 | 1.00 | 0.00 | AAAA |
| ATOM | 1750 | CB | ASN | E | 105 | 34.615 | 123.671 | 26.256 | 1.00 | 0.00 | AAAA |
| ATOM | 1753 | CG | ASN | E | 105 | 33.291 | 123.273 | 25.608 | 1.00 | 0.00 | AAAA |
| ATOM | 1754 | OD1 | ASN | E | 105 | 32.941 | 122.103 | 25.498 | 1.00 | 0.00 | AAAA |
| ATOM | 1755 | ND2 | ASN | E | 105 | 32.462 | 124.252 | 25.229 | 1.00 | 0.00 | AAAA |
| ATOM | 1758 | C | ASN | E | 105 | 34.317 | 122.611 | 28.560 | 1.00 | 0.00 | AAAA |
| ATOM | 1759 | O | ASN | E | 105 | 35.184 | 121.829 | 28.967 | 1.00 | 0.00 | AAAA |
| ATOM | 1760 | N | ILE | E | 106 | 33.043 | 122.330 | 28.807 | 1.00 | 0.00 | AAAA |
| ATOM | 1762 | CA | ILE | E | 106 | 32.490 | 121.005 | 29.027 | 1.00 | 0.00 | AAAA |
| ATOM | 1764 | CB | ILE | E | 106 | 32.250 | 120.793 | 30.517 | 1.00 | 0.00 | AAAA |
| ATOM | 1766 | CG1 | ILE E | | 106 | 33.521 | 120.630 | 31.353 | 1.00 | 0.00 | AAAA |
| ATOM | 1769 | CG2 | ILE | E | 106 | 31.486 | 119.478 | 30.692 | 1.00 | 0.00 | AAAA |
| ATOM | 1773 | CD1 | ILE | E | 106 | 33.292 | 120.386 | 32.839 | 1.00 | 0.00 | AAAA |
| ATOM | 1777 | C | ILE | E | 106 | 31:244 | 120.914 | 28.150 | 1.00 | 0.00 | AAAA |
| ATOM | 1778 | O | ILE | E | 106 | 30.251 | 121.622 | 28.314 | 1.00 | 0.00 | AAAA |
| ATOM | 1779 | N | THR | E | 107 | 31.339 | 120.163 | 27.050 | 1.00 | 0.00 | AAAA |
| ATOM | 1781 | CA | THR | E | 107 | 30.425 | 120.123 | 25.923 | 1.00 | 0.00 | AAAA |
| ATOM | 1783 | CB | THR | E | 107 | 31.061 | 119.288 | 24.816 | 1.00 | 0.00 | AAAA |
| ATOM | 1785 | OG1 | THR | E | 107 | 32.428 | 119.617 | 24.834 | 1.00 | 0.00 | AAAA |
| ATOM | 1787 | CG2 | THR | E | 107 | 30.437 | 119.644 | 23.463 | 1.00 | 0.00 | AAAA |
| ATOM | 1791 | C | THR | E | 107 | 28.992 | 119.696 | 26.203 | 1.00 | 0.00 | AAAA |
| ATOM | 1792 | O | THR | E | 107 | 28.130 | 120.229 | 25.500 | 1.00 | 0.00 | AAAA |
| ATOM | 1793 | N | ARG | E | 108 | 28.816 | 118.779 | 27.148 | 1.00 | 0.00 | AAAA |
| ATOM | 1795 | CA | ARG | E | 108 | 27.573 | 118.391 | 27.782 | 1.00 | 0.00 | AAAA |
| ATOM | 1797 | CB | ARG | E | 108 | 27.134 | 116.992 | 27.336 | 1.00 | 0.00 | AAAA |
| ATOM | 1800 | CG | ARG | E | 108 | 26.950 | 116.995 | 25.816 | 1.00 | 0.00 | AAAA |
| ATOM | 1803 | CD | ARG | E | 108 | 26.471 | 115.661 | 25.243 | 1.00 | 0.00 | AAAA |
| ATOM | 1806 | NE | ARG | E | 108 | 25.102 | 115.498 | 25.721 | 1.00 | 0.00 | AAAA |
| ATOM | 1808 | CZ | ARG | E | 108 | 24.630 | 114.570 | 26.564 | 1.00 | 0.00 | AAAA |
| ATOM | 1809 | NH1 | ARG | E | 108 | 25.414 | 113.625 | 27.105 | 1.00 | 0.00 | AAAA |
| ATOM | 1812 | NH2 | ARG | E | 108 | 23.326 | 114.574 | 26.847 | 1.00 | 0.00 | AAAA |
| ATOM | 1815 | C | ARG | E | 108 | 27.755 | 118.258 | 29.285 | 1.00 | 0.00 | AAAA |
| ATOM | 1816 | O | ARG | E | 108 | 28.762 | 117.713 | 29.736 | 1.00 | 0.00 | AAAA |
| ATOM | 1817 | N | GLY | E | 109 | 26.802 | 118.694 | 30.117 | 1.00 | 0.00 | AAAA |
| ATOM | 1819 | CA | GLY | E | 109 | 26.809 | 118.588 | 31.561 | 1.00 | 0.00 | AAAA |
| ATOM | 1822 | C | GLY | E | 109 | 26.986 | 119.858 | 32.392 | 1.00 | 0.00 | AAAA |
| ATOM | 1823 | O | GLY | E | 109 | 26.879 | 120.979 | 31.898 | 1.00 | 0.00 | AAAA |
| ATOM | 1824 | N | ALA | E | 110 | 27.332 | 119.651 | 33.659 | 1.00 | 0.00 | AAAA |
| ATOM | 1826 | CA | ALA | E | 110 | 27.839 | 120.607 | 34.634 | 1.00 | 0.00 | AAAA |
| ATOM | 1828 | CB | ALA | E | 110 | 26.673 | 121.245 | 35.383 | 1.00 | 0.00 | AAAA |
| ATOM | 1832 | C | ALA | E | 110 | 28.864 | 119.965 | 35.547 | 1.00 | 0.00 | AAAA |
| ATOM | 1833 | O | ALA | E | 110 | 29.509 | 118.972 | 35.191 | 1.00 | 0.00 | AAAA |
| ATOM | 1834 | N | ILE | E | 111 | 29.010 | 120.481 | 36.773 | 1.00 | 0.00 | AAAA |
| ATOM | 1836 | CA | ILE | E | 111 | 30.048 | 120.189 | 37.737 | 1.00 | 0.00 | AAAA |
| ATOM | 1838 | CB | ILE | E | 111 | 31.272 | 121.107 | 37.591 | 1.00 | 0.00 | AAAA |
| ATOM | 1840 | CG1 | ILE | E | 111 | 30.929 | 122.553 | 37.945 | 1.00 | 0.00 | AAAA |
| ATOM | 1843 | CG2 | ILE | E | 111 | 31.831 | 121.188 | 36.166 | 1.00 | 0.00 | AAAA |
| ATOM | 1847 | CD1 | ILE | E | 111 | 31.513 | 123.076 | 39.259 | 1.00 | 0.00 | AAAA |
| ATOM | 1851 | C | ILE | E | 111 | 29.406 | 120.228 | 39.125 | 1.00 | 0.00 | AAAA |
| ATOM | 1852 | O | ILE | E | 111 | 28.457 | 120.960 | 39.388 | 1.00 | 0.00 | AAAA |
| ATOM | 1853 | N | ARG | E | 112 | 29.987 | 119.528 | 40.094 | 1.00 | 0.00 | AAAA |
| ATOM | 1855 | CA | ARG | E | 112 | 29.603 | 119.441 | 41.492 | 1.00 | 0.00 | AAAA |
| ATOM | 1857 | CB | ARG | E | 112 | 28.463 | 118.444 | 41.735 | 1.00 | 0.00 | AAAA |
| ATOM | 1860 | CG | ARG | E | 112 | 28.054 | 118.328 | 43.202 | 1.00 | 0.00 | AAAA |
| ATOM | 1863 | CD | ARG | E | 112 | 26.549 | 118.042 | 43.234 | 1.00 | 0.00 | AAAA |
| ATOM | 1866 | NE | ARG | E | 112 | 26.127 | 118.169 | 44.632 | 1.00 | 0.00 | AAAA |
| ATOM | 1868 | CZ | ARG | E | 112 | 24.821 | 118.303 | 44.918 | 1.00 | 0.00 | AAAA |
| ATOM | 1869 | NH1 | ARG | E | 112 | 23.846 | 118.387 | 44.008 | 1.00 | 0.00 | AAAA |
| ATOM | 1872 | NH2 | ARG | E | 112 | 24.508 | 118.250 | 46.217 | 1.00 | 0.00 | AAAA |
| ATOM | 1875 | C | ARG | E | 112 | 30.822 | 119.343 | 42.398 | 1.00 | 0.00 | AAAA |
| ATOM | 1876 | O | ARG | E | 112 | 31.503 | 118.322 | 42.416 | 1.00 | 0.00 | AAAA |
| ATOM | 1877 | | ILE | E | 113 | 31.159 | 120.378 | 43.169 | 1.00 | 0.00 | AAAA |
| ATOM | 1879 | CA | ILE | E | 113 | 32.391 | 120.632 | 43.896 | 1.00 | 0.00 | AAAA |
| ATOM | 1881 | CB | ILE | E | 113 | 33.392 | 121.586 | 43.260 | 1.00 | 0.00 | AAAA |
| ATOM | 1883 | CG1 | ILE | E | 113 | 33.549 | 121.345 | 41.753 | 1.00 | 0.00 | AAAA |
| ATOM | 1886 | CG2 | ILE | E | 113 | 34.694 | 121.683 | 44.055 | 1.00 | 0.00 | AAAA |
| ATOM | 1890 | CD1 | ILE | E | 113 | 34.606 | 122.214 | 41.075 | 1.00 | 0.00 | AAAA |
| ATOM | 1894 | C | ILE | E | 113 | 31.939 | 120.933 | 45.311 | 1.00 | 0.00 | AAAA |
| ATOM | 1895 | O | ILE | E | 113 | 31.756 | 122.103 | 45.655 | 1.00 | 0.00 | AAAA |
| ATOM | 1896 | N | GLU | E | 114 | 31.755 | 119.937 | 46.183 | 1.00 | 0.00 | AAAA |
| ATOM | 1898 | CA | GLU | E | 114 | 31.211 | 120.037 | 47.524 | 1.00 | 0.00 | AAAA |
| ATOM | 1900 | CB | GLU | E | 114 | 29.680 | 120.015 | 47.511 | 1.00 | 0.00 | AAAA |
| ATOM | 1903 | CG | GLU | E | 114 | 28.944 | 118.713 | 47.198 | 1.00 | 0.00 | AAAA |
| ATOM | 1906 | CD | GLU | E | 114 | 27.724 | 118.381 | 48.039 | 1.00 | 0.00 | AAAA |
| ATOM | 1907 | OE1 | GLU | E | 114 | 26.800 | 117.824 | 47.402 | 1.00 | 0.00 | AAAA |
| ATOM | 1908 | OE2 | GLU | E | 114 | 27.697 | 118.743 | 49.229 | 1.00 | 0.00 | AAAA |
| ATOM | 1909 | C | GLU | E | 114 | 31.877 | 119.058 | 48.468 | 1.00 | 0.00 | AAAA |
| ATOM | 1910 | O | GLU | E | 114 | 32.455 | 118.083 | 47.982 | 1.00 | 0.00 | AAAA |
| ATOM | 1911 | N | LYS | E | 115 | 31.684 | 119.275 | 49.773 | 1.00 | 0.00 | AAAA |
| ATOM | 1913 | CA | LYS | E | 115 | 32.145 | 118.435 | 50.863 | 1.00 | 0.00 | AAAA |
| ATOM | 1915 | CB | LYS | E | 115 | 31.580 | 117.013 | 50.816 | 1.00 | 0.00 | AAAA |
| ATOM | 1918 | CG | LYS | E | 115 | 31.676 | 116.117 | 52.061 | 1.00 | 0.00 | AAAA |
| ATOM | 1921 | CD | LYS | E | 115 | 31.013 | 114.767 | 51.812 | 1.00 | 0.00 | AAAA |
| ATOM | 1924 | CE | LYS | E | 115 | 30.909 | 114.060 | 53.167 | 1.00 | 0.00 | AAAA |
| ATOM | 1927 | NZ | LYS | E | 115 | 30.386 | 112.696 | 53.048 | 1.00 | 0.00 | AAAA |
| ATOM | 1931 | C | LYS | E | 115 | 33.641 | 118.528 | 51.137 | 1.00 | 0.00 | AAAA |
| ATOM | 1932 | O | LYS | E | 115 | 34.182 | 117.873 | 52.028 | 1.00 | 0.00 | AAAA |
| ATOM | 1933 | N | ASN | E | 116 | 34.408 | 119.315 | 50.390 | 1.00 | 0.00 | AAAA |
| ATOM | 1935 | CA | ASN | E | 116 | 35.836 | 119.497 | 50.493 | 1.00 | 0.00 | AAAA |
| ATOM | 1937 | CB | ASN | E | 116 | 36.522 | 119.625 | 49.138 | 1.00 | 0.00 | AAAA |
| ATOM | 1940 | CG | ASN | E | 116 | 35.982 | 118.815 | 47.966 | 1.00 | 0.00 | AAAA |
| ATOM | 1941 | OD1 | ASN | E | 116 | 36.377 | 117.684 | 47.690 | 1.00 | 0.00 | AAAA |
| ATOM | 1942 | ND2 | ASN | E | 116 | 35.112 | 119.500 | 47.225 | 1.00 | 0.00 | AAAA |
| ATOM | 1945 | C | ASN | E | 116 | 36.088 | 120.719 | 51.366 | 1.00 | 0.00 | AAAA |
| ATOM | 1946 | O | ASN | E | 116 | 36.339 | 121.815 | 50.859 | 1.00 | 0.00 | AAAA |
| ATOM | 1947 | N | ALA | E | 117 | 36.083 | 120.515 | 52.687 | 1.00 | 0.00 | AAAA |
| ATOM | 1949 | CA | ALA | E | 117 | 36.155 | 121.573 | 53.676 | 1.00 | 0.00 | AAAA |
| ATOM | 1951 | CB | ALA | E | 117 | 35.760 | 120.995 | 55.034 | 1.00 | 0.00 | AAAA |
| ATOM | 1955 | C | ALA | E | 117 | 37.475 | 122.339 | 53.723 | 1.00 | 0.00 | AAAA |
| ATOM | 1956 | O | ALA | E | 117 | 37.609 | 123.374 | 54.383 | 1.00 | 0.00 | AAAA |
| ATOM | 1957 | N | ASP | E | 118 | 38.410 | 121.955 | 52.853 | 1.00 | 0.00 | AAAA |
| ATOM | 1959 | CA | ASP | E | 118 | 39.717 | 122.554 | 52.659 | 1.00 | 0.00 | AAAA |
| ATOM | 1961 | CB | ASP | E | 118 | 40.777 | 121.541 | 52.207 | 1.00 | 0.00 | AAAA |
| ATOM | 1964 | CG | ASP | E | 118 | 41.182 | 120.616 | 53.348 | 1.00 | 0.00 | AAAA |
| ATOM | 1965 | OD1 | ASP | E | 118 | 41.687 | 121.088 | 54.395 | 1.00 | 0.00 | AAAA |
| ATOM | 1966 | OD2 | ASP | E | 118 | 41.026 | 119.386 | 53.227 | 1.00 | 0.00 | AAAA |
| ATOM | 1967 | C | ASP | E | 118 | 39.733 | 123.529 | 51.495 | 1.00 | 0.00 | AAAA |
| ATOM | 1968 | O | ASP | E | 118 | 40.660 | 124.314 | 51.338 | 1.00 | 0.00 | AAAA |
| ATOM | 1969 | N | LEU | E | 119 | 38.707 | 123.406 | 50.646 | 1.00 | 0.00 | AAAA |
| ATOM | 1971 | CA | LEU | E | 119 | 38.742 | 123.838 | 49.261 | 1.00 | 0.00 | AAAA |
| ATOM | 1973 | CB | LEU | E | 119 | 38.144 | 122.750 | 48.366 | 1.00 | 0.00 | AAAA |
| ATOM | 1976 | CG | LEU | E | 119 | 37.969 | 123.134 | 46.890 | 1.00 | 0.00 | AAAA |
| ATOM | 1978 | CD1 | LEU | E | 119 | 39.307 | 123.313 | 46.174 | 1.00 | 0.00 | AAAA |
| ATOM | 1982 | CD2 | LEU | E | 119 | .37.103 | 122.111 | 46.150 | 1.00 | 0.00 | AAAA |
| ATOM | 1986 | C | LEU | E | 119 | 38.203 | 125.249 | 49.076 | 1.00 | 0.00 | AAAA |
| ATOM | 1987 | O | LEU | E | 119 | 37.210 | 125.580 | 49.718 | 1.00 | 0.00 | AAAA |
| ATOM | 1988 | N | CYS | E | 120 | 38.828 | 126.091 | 48.243 | 1.00 | 0.00 | AAAA |
| ATOM | 1990 | CA | CYS | E | 120 | 38.376 | 127.356 | 47.727 | 1.00 | 0.00 | AAAA |
| ATOM | 1992 | CB | CYS | E | 120 | 38.760 | 128.474 | 48.694 | 1.00 | 0.00 | AAAA |
| ATOM | 1995 | SG | CYS | E | 120 | 40.431 | 128.546 | 49.387 | 1.00 | 0.00 | AAAA |
| ATOM | 1996 | C | CYS | E | 120 | 38.756 | 127.485 | 46.251 | 1.00 | 0.00 | AAAA |
| ATOM | 1997 | O | CYS | E | 120 | 39.207 | 126.482 | 45.717 | 1.00. | 0.00 | AAAA |
| ATOM | 1998 | N | TYR | E | 121 | 38.646 | 128.707 | 45.725 | 1.00 | 0.00 | AAAA |
| ATOM | 2000 | CA | TYR | E | 121 | 38.951 | 129.163 | 44.374 | 1.00 | 0.00 | AAAA |
| ATOM | 2002 | CB | TYR | E | 121 | 40.399 | 128.967 | 43.921 | 1.00 | 0.00 | AAAA |
| ATOM | 2005 | CG | TYR | E | 121 | 41.364 | 129.993 | 44.462 | 1.00 | 0.00 | AAAA |
| ATOM | 2006 | CD1 | TYR | E | 121 | 42.040 | 129.794 | 45.673 | 1.00 | 0.00 | AAAA |
| ATOM | 2008 | CD2 | TYR | E | 121 | 41.538 | 131.278 | 43.918 | 1.00 | 0.00 | AAAA |
| ATOM | 2010 | CE1 | TYR | E | 121 | 43.005 | 130.680 | 46.174 | 1.00 | 0.00 | AAAA |
| ATOM | 2012 | CE2 | TYR | E | 121 | 42.515 | 132.173 | 44.367 | 1.00 | 0.00 | AAAA |
| ATOM | 2014 | CZ | TYR | E | 121 | 43.287 | 131.865 | 45.490 | 1.00 | 0.00 | AAAA |
| ATOM | 2015 | OH | TYR | E | 121 | 44.365 | 132.590 | 45.907 | 1.00 | 0.00 | AAAA |
| ATOM | 2017 | C | TYR | E | 121 | 37.841 | 128.778 | 43.411 | 1.00 | 0.00 | AAAA |
| ATOM | 2018 | O | TYR | E | 121 | 38.200 | 128.413 | 42.286 | 1.00 | 0.00 | AAAA |
| ATOM | 2019 | N | LEU | E | 122 | 36.566 | 128.889 | 43.792 | 1.00 | 0.00 | AAAA |
| ATOM | 2021 | CA | LEU | E | 122 | 35.407 | 128.436 | 43.053 | 1.00 | 0.00 | AAAA |
| ATOM | 2023 | CB | LEU | E | 122 | 34.724 | 127.368 | 43.919 | 1.00 | 0.00 | AAAA |
| ATOM | 2026 | CG | LEU | E | 122 | 35.532 | 126.278 | 44.617 | 1.00 | 0.00 | AAAA |
| ATOM | 2028 | CD1 | LEU | E | 122 | 34.808 | 125.723 | 45.837 | 1.00 | 0.00 | AAAA |
| ATOM | 2032 | CD2 | LEU | E | 122 | 35.808 | 125.044 | 43.749 | 1.00 | 0.00 | AAAA |
| ATOM | 2036 | C | LEU | E | 122 | 34.498 | 129.583 | 42.658 | 1.00 | 0.00 | AAAA |
| ATOM | 2037 | O | LEU | E | 122 | 34.226 | 129.733 | 41.469 | 1.00 | 0.00 | AAAA |
| ATOM | 2038 | N | SER | E | 123 | 34.343 | 130.544 | 43.574 | 1.00 | 0.00 | AAAA |
| ATOM | 2040 | CA | SER | E | 123 | 33.625 | 131.781 | 43.339 | 1.00 | 0.00 | AAAA |
| ATOM | 2042 | CB | SER | E | 123 | 33.225 | 132.470 | 44.647 | 1.00 | 0.00 | AAAA |
| ATOM | 2045 | OG | SER | E | 123 | 34.056 | 132.117 | 45.729 | 1.00 | 0.00 | AAAA |
| ATOM | 2047 | C | SER | E | 123 | 34.385 | 132.801 | 42.515 | 1.00 | 0.00 | AAAA |
| ATOM | 2048 | O | SER | E | 123 | 33.743 | 133.723 | 42.010 | 1.00 | 0.00 | AAAA |
| ATOM | 2049 | N | THR | E | 124 | 35.696 | 132.615 | 42.355 | 1.00 | 0.00 | AAAA |
| ATOM | 2051 | CA | THR | E | 124 | 36.695 | 133.399 | 41.643 | 1.00 | 0.00 | AAAA |
| ATOM | 2053 | CB | THR | E | 124 | 38.076 | 133.009 | 42.156 | 1.00 | 0.00 | AAAA |
| ATOM | 2055 | OG1 | THR | E | 124 | 38.210 | 131.608 | 42.226 | 1.00 | 0.00 | AAAA |
| ATOM | 2057 | CG2 | THR | E | 124 | 38.376 | 133.568 | 43.555 | 1.00 | 0.00 | AAAA |
| ATOM | 2061 | C | THR | E | 124 | 36.644 | 133.168 | 40.140 | 1.00 | 0.00 | AAAA |
| ATOM | 2062 | O | THR | E | 124 | 37.297 | 133.866 | 39.370 | 1.00 | 0.00 | AAAA |
| ATOM | 2063 | N | VAL | E | 125 | 36.036 | 132.056 | 39.721 | 1.00 | 0.00 | AAAA |
| ATOM | 2065 | CA | VAL | E | 125 | 35.989 | 131.552 | 38.368 | 1.00 | 0.00 | AAAA |
| ATOM | 2067 | CB | VAL | E | 125 | 36.249 | 130.050 | 38.360 | 1.00 | 0.00 | AAAA |
| ATOM | 2069 | CG1 | VAL | E | 125 | 36.112 | 129.283 | 37.046 | 1.00 | 0.00 | AAAA |
| ATOM | 2073 | CG2 | VAL | E | 125 | 37.640 | 129.788 | 38.933 | 1.00 | 0.00 | AAAA |
| ATOM | 2077 | C | VAL | E | 125 | 34.711 | 132.055 | 37.702 | 1.00 | 0.00 | AAAA |
| ATOM | 2078 | O | VAL | E | 125 | 33.605 | 132.026 | 38.240 | 1.00 | 0.00 | AAAA |
| ATOM | 2079 | N | ASP | E | 126 | 34.818 | 132.459 | 36.439 | 1.00 | 0.00 | AAAA |
| ATOM | 2081 | CA | ASP | E | 126 | 33.706 | 132.567 | 35.520 | 1.00 | 0.00 | AAAA |
| ATOM | 2083 | CB | ASP | E | 126 | 34.105 | 133.474 | 34.351 | 1.00 | 0.00 | AAAA |
| ATOM | 2086 | CG | ASP | E | 126 | 32.853 | 133.725 | 33.536 | 1.00 | 0.00 | AAAA |
| ATOM | 2087 | OD1 | ASP | E | 126 | 31.742 | 133.197 | 33.809 | 1.00 | 0.00 | AAAA |
| ATOM | 2088 | OD2 | ASP | E | 126 | 32.982 | 134.556 | 32.612 | 1.00 | 0.00 | AAAA |
| ATOM | 2089 | C | ASP | E | 126 | 33.325 | 131.182 | 34.991 | 1.00 | 0.00 | AAAA |
| ATOM | 2090 | O | ASP | E | 126 | 34.084 | 130.563 | 34.256 | 1.00 | 0.00 | AAAA |
| ATOM | 2091 | N | TRP | E | 127 | 32.135 | 130.761 | 35.398 | 1.00 | 0.00 | AAAA |
| ATOM | 2093 | CA | TRP | E | 127 | 31.622 | 129.459 | 35.006 | 1.00 | 0.00 | AAAA |
| ATOM | 2095 | CB | TRP | E | 127 | 30.783 | 128.806 | 36.094 | 1.00 | 0.00 | AAAA |
| ATOM | 2098 | CG | TRP | E | 127 | 31.578 | 128.455 | 37.320 | 1.00 | 0.00 | AAAA |
| ATOM | 2099 | CD1 | TRP | E | 127 | 31.705 | 129.174 | 38.462 | 1.00 | 0.00 | AAAA |
| ATOM | 2101 | CD2 | TRP | E | 127 | 32.419 | 127.286 | 37.526 | 1.00 | 0.00 | AAAA |
| ATOM | 2102 | NE1 | TRP | E | 127 | 32.657 | 128.621 | 39.299 | 1.00 | 0.00 | AAAA |
| ATOM | 2104 | CE2 | TRP | E | 127 | 33.146 | 127.464 | 38.737 | 1.00 | 0.00 | AAAA |
| ATOM | 2105 | CE3 | TRP | E | 127 | 32.849 | 126.207 | 36.741 | 1.00 | 0.00 | AAAA |
| ATOM | 2107 | CZ2 | TRP | E | 127 | 34.104 | 126.533 | 39.165 | 1.00 | 0.00 | AAAA |
| ATOM | 2109 | CZ3 | TRP | E | 127 | 33.644 | 125.170 | 37.233 | 1.00 | 0.00 | AAAA |
| ATOM | 2111 | CH2 | TRP | E | 127 | 34.274 | 125.327 | 38.477 | 1.00 | 0.00 | AAAA |
| ATOM | 2113 | C | TRP | E | 127 | 30.783 | 129.493 | 33.743 | 1.00 | 0.00 | AAAA |
| ATOM | 2114 | O | TRP | E | 127 | 30.477 | 128.443 | 33.174 | 1.00 | 0.00 | AAAA |
| ATOM | 2115 | N | SER | E | 128 | 30.360 | 130.684 | 33.298 | 1.00 | 0.00 | AAAA |
| ATOM | 2117 | CA | SER | E | 128 | 29.626 | 130.859 | 32.062 | 1.00 | 0.00 | AAAA |
| ATOM | 2119 | CB | SER | E | 128 | 28.773 | 132.126 | 32.157 | 1.00 | 0.00 | AAAA |
| ATOM | 2122 | OG | SER | E | 128 | 29.533 | 133.319 | 32.058 | 1.00 | 0.00 | AAAA |
| ATOM | 2124 | C | SER | E | 128 | -30.487 | 130.697 | 30.816 | 1.00 | 0.00 | AAAA |
| ATOM | 2125 | O | SER | E | 128 | 29.936 | 130.500 | 29.736 | 1.00 | 0.00 | AAAA |
| ATOM | 2126 | N | LEU | E | 129 | 31.814 | 130.717 | 30.969 | 1.00 | 0.00 | AAAA |
| ATOM | 2128 | CA | LEU | E | 129 | 32.831 | 130.558 | 29.960 | 1.00 | 0.00 | AAAA |
| ATOM | 2130 | CB | LEU | E | 129 | 34.165 | 131.005 | 30.564 | 1.00 | 0.00 | AAAA |
| ATOM | 2133 | CG | LEU | E | 129 | 35.034 | 131.884 | 29.664 | 1.00 | 0.00 | AAAA |
| ATOM | 2135 | CD1 | LEU | E | 129 | 35.973 | 132.719 | 30.536 | 1.00 | 0.00 | AAAA |
| ATOM | 2139 | CD2 | LEU | E | 129 | 35.669 | 131.040 | 28.557 | 1.00 | 0.00 | AAAA |
| ATOM | 2143 | C | LEU | E | 129 | 32.789 | 129.089 | 29.579 | 1.00 | 0.00 | AAAA |
| ATOM | 2144 | O | LEU | E | 129 | 32.843 | 128.734 | 28.406 | 1.00 | 0.00 | AAAA |
| ATOM | 2145 | N | ILE | E | 130 | 32.740 | 128.220 | 30.594 | 1.00 | 0.00 | AAAA |
| ATOM | 2147 | CA | ILE | E | 130 | 32.780 | 126.769 | 30.580 | 1.00 | 0.00 | AAAA |
| ATOM | 2149 | CB | ILE | E | 130 | 33.408 | 126.303 | 31.893 | 1.00 | 0.00 | AAAA |
| ATOM | 2151 | CG1 | ILE | E | 130 | 34.594 | 127.147 | 32.364 | 1.00 | 0.00 | AAAA |
| ATOM | 2154 | CG2 | ILE | E | 130 | 33.759 | 124.812 | 31.861 | 1.00 | 0.00 | AAAA |
| ATOM | 2158 | CD1 | ILE | E | 130 | 35.152 | 126.657 | 33.704 | 1.00 | 0.00 | AAAA |
| ATOM | 2162 | C | ILE | E | 130 | 31.388 | 126.224 | 30.316 | 1.00 | 0.00 | AAAA |
| ATOM | 2163 | O | ILE | E | 130 | 31.085 | 125.701 | 29.250 | 1.00 | 0.00 | AAAA |
| ATOM | 2164 | N | LEU | E | 131 | 30.487 | 126.281 | 31.300 | 1.00 | 0.00 | AAAA |
| ATOM | 2166 | CA | LEU | E | 131 | 29.168 | 125.666 | 31.258 | 1.00 | 0.00 | AAAA |
| ATOM | 2168 | CB | LEU | E | 131 | 28.791 | 125.276 | 32.689 | 1.00 | 0.00 | AAAA |
| ATOM | 2171 | CG | LEU | E | 131 | 29.720 | 124.255 | 33.343 | 1.00 | 0.00 | AAAA |
| ATOM | 2173 | CD1 | LEU | E | 131 | 29.131 | 124.007 | 34.733 | 1.00 | 0.00 | AAAA |
| ATOM | 2177 | CD2 | LEU | E | 131 | 29.839 | 122.925 | 32.604 | 1.00 | 0.00 | AAAA |
| ATOM | 2181 | C | LEU | E | 131 | 28.135 | 126.656 | 30.753 | 1.00 | 0.00 | AAAA |
| ATOM | 2182 | O | LEU | E | 131 | 28.255 | 127.869 | 30.851 | 1.00 | 0.00 | AAAA |
| ATOM | 2183 | N | ASP | E | 132 | 27.037 | 126.064 | 30.279 | 1.00 | 0.00 | AAAA |
| ATOM | 2185 | CA | ASP | E | 132 | 25.843 | 126.728 | 29.781 | 1.00 | 0.00 | AAAA |
| ATOM | 2187 | CB | ASP | E | 132 | 25.353 | 126.106 | 28.474 | 1.00 | 0.00 | AAAA |
| ATOM | 2190 | CG | ASP | E | 132 | 24.097 | 126.786 | 27.946 | 1.00 | 0.00 | AAAA |
| ATOM | 2191 | OD1 | ASP | E | 132 | 24.153 | 128.018 | 27.747 | 1.00 | 0.00 | AAAA |
| ATOM | 2192 | OD2 | ASP | E | 132 | 23.059 | 126.104 | 27.854 | 1.00 | 0.00 | AAAA |
| ATOM | 2193 | C | ASP | E | 132 | 24.719 | 126.576 | 30.795 | 1.00 | 0.00 | AAAA |
| ATOM | 2194 | O | ASP | E | 132 | 24.036 | 127.502 | 31.214 | 1.00 | 0.00 | AAAA |
| ATOM | 2195 | N | ALA | E | 133 | 24.794 | 125.397 | 31.416 | 1.00 | 0.00 | AAAA |
| ATOM | 2197 | CA | ALA | E | 133 | 24.074 | 124.852 | 32.542 | 1.00 | 0.00 | AAAA |
| ATOM | 2199 | CB | ALA | E | 133 | 24.489 | 123.386 | 32.491 | 1.00 | 0.00 | AAAA |
| ATOM | 2203 | C | ALA | E | 133 | 24.531 | 125.528 | 33.835 | 1.00 | 0.00 | AAAA |
| ATOM | 2204 | O | ALA | E | 133 | 24.651 | 124.873 | 34.866 | 1.00 | 0.00 | AAAA |
| ATOM | 2205 | N | VAL | E | 134 | 24.808 | 126.828 | 33.840 | 1.00 | 0.00 | AAAA |
| ATOM | 2207 | CA | VAL | E | 134 | 25.356 | 127.658 | 34.897 | 1.00 | 0.00 | AAAA |
| ATOM | 2209 | CB | VAL | E | 134 | 25.689 | 129.087 | 34.463 | 1.00 | 0.00 | AAAA |
| ATOM | 2211 | CG1 | VAL | E | 134 | 26.878 | 129.192 | 33.507 | 1.00 | 0.00 | AAAA |
| ATOM | 2215 | CG2 | VAL | E | 134 | 24.487 | 129.862 | 33.941 | 1.00 | 0.00 | AAAA |
| ATOM | 2219 | C | VAL | E | 134 | 24.605 | 127.667 | 36.225 | 1.00 | 0.00 | AAAA |
| ATOM | 2220 | O | VAL | E | 134 | 25.130 | 128.027 | 37.279 | 1.00 | 0.00 | AAAA |
| ATOM | 2221 | N | SER | E | 135 | 23.355 | 127.195 | 36.317 | 1.00 | 0.00 | AAAA |
| ATOM | 2223 | CA | SER | E | 135 | 22.551 | 127.070 | 37.508 | 1.00 | 0.00 | AAAA |
| ATOM | 2225 | CB | SER | E | 135 | 21.065 | 127.318 | 37.235 | 1.00 | 0.00 | AAAA |
| ATOM | 2228 | OG | SER | E | 135 | 20.938 | 128.602 | 36.670 | 1.00 | 0.00 | AAAA |
| ATOM | 2230 | C | SER | E | 135 | 22.596 | 125.663 | 38.085 | 1.00 | 0.00 | AAAA |
| ATOM | 2231 | O | SER | E | 135 | 22.231 | 125.455 | 39.238 | 1.00 | 0.00 | AAAA |
| ATOM | 2232 | N | ASN | E | 136 | 23.163 | 124.732 | 37.299 | 1.00 | 0.00 | AAAA |
| ATOM | 2234 | CA | ASN | E | 136 | 23.409 | 123.348 | 37.636 | 1.00 | 0.00 | AAAA |
| ATOM | 2236 | CB | ASN | E | 136 | 23.095 | 122.426 | 36.453 | 1.00 | 0.00 | AAAA |
| ATOM | 2239 | CG | ASN | E | 136 | 22.940 | 120.970 | 36.851 | 1.00 | 0.00 | AAAA |
| ATOM | 2240 | OD1 | ASN | E | 136 | 23.702 | 120.127 | 36.383 | 1.00 | 0.00 | AAAA |
| ATOM | 2241 | ND2 | ASN | E | 136 | 21.866 | 120.575 | 37.539 | 1.00 | 0.00 | AAAA |
| ATOM | 2244 | C | ASN | E | 136 | 24.852 | 123.103 | 38.062 | 1.00 | 0.00 | AAAA |
| ATOM | 2245 | O | ASN | E | 136 | 25.196 | 122.049 | 38.591 | 1.00 | 0.00 | AAAA |
| ATOM | 2246 | N | ASN | E | 137 | 25.673 | 124.150 | 37.904 | 1.00 | 0.00 | AAAA |
| ATOM | 2248 | CA | ASN | E | 137 | 27.020 | 124.235 | 38.415 | 1.00 | 0.00 | AAAA |
| ATOM | 2250 | CB | ASN | E | 137 | 27.574 | 125.592 | 37.965 | 1.00 | 0.00 | AAAA |
| ATOM | 2253 | CG | ASN | E | 137 | 28.998 | 126.003 | 38.305 | 1.00 | 0.00 | AAAA |
| ATOM | 2254 | OD1 | ASN | E | 137 | 29.978 | 125.430 | 37.824 | 1.00 | 0.00 | AAAA |
| ATOM | 2255 | ND2 | ASN | E | 137 | 29.139 | 126.914 | 39.277 | 1.00 | 0.00 | AAAA |
| ATOM | 2258 | C | ASN | E | 137 | 26.846 | 124.387 | 39.917 | 1.00 | 0.00 | AAAA |
| ATOM | 2259 | O | ASN | E | 137 | 26.309 | 125.389 | 40.382 | 1.00 | 0.00 | AAAA |
| ATOM | 2260 | N | TYR | E | 138 | 27.378 | 123.413 | 40.664 | 1.00 | 0.00 | AAAA |
| ATOM | 2262 | CA | TYR | E | 138 | 27.205 | 123.405 | 42.103 | 1.00 | 0.00 | AAAA |
| ATOM | 2264 | CB | TYR | E | 138 | 26.523 | 122.072 | 42.405 | 1.00 | 0.00 | AAAA |
| ATOM | 2267 | CG | TYR | E | 138 | 26.069 | 121.810 | 43.827 | 1.00 | 0.00 | AAAA |
| ATOM | 2268 | CD1 | TYR | E | 138 | 24.714 | 121.758 | 44.139 | 1.00 | 0.00 | AAAA |
| ATOM | 2270 | CD2 | TYR | E | 138 | 27.037 | 121.626 | 44.822 | 1.00 | 0.00 | AAAA |
| ATOM | 2272 | CE1 | TYR | E | 138 | 24.216 | 121.522 | 45.433 | 1.00 | 0.00 | AAAA |
| ATOM | 2274 | CE2 | TYR | E | 138 | 26.560 | 121.438 | 46.127 | 1.00 | 0.00 | AAAA |
| ATOM | 2276 | CZ | TYR | E | 138 | 25.195 | 121.397 | 46.420 | 1.00 | 0.00 | AAAA |
| ATOM | 2277 | OH | TYR | E | 138 | 24.779 | 121.153 | 47.698 | 1.00 | 0.00 | AAAA |
| ATOM | 2279 | C | TYR | E | 138 | 28.548 | 123.509 | 42.803 | 1.00 | 0.00 | AAAA |
| ATOM | 2280 | O | TYR | E | 138 | 29.329 | 122.570 | 42.667 | 1.00 | 0.00 | AAAA |
| ATOM | 2281 | N | ILE | E | 139 | 28.787 | 124.563 | 43.580 | 1.00 | 0.00 | AAAA |
| ATOM | 2283 | CA | ILE | E | 139 | 30.048 | 124.916 | 11.188 | 1.00 | 0.00 | AAAA |
| ATOM | 2285 | CB | ILE | E | 139 | 30.918 | 125.746 | 43.243 | 1.00 | 0.00 | AAAA |
| ATOM | 2287 | CG1 | ILE | E | 139 | 30.242 | 127.061 | 44.851 | 1.00 | 0.00 | AAAA |
| ATOM | 2290 | CG2 | ILE | E | 139 | 31.479 | 124.940 | 42.073 | 1.00 | 0.00 | AAAA |
| ATOM | 2294 | CD1 | ILE | E | 139 | 31.200 | 128.226 | 42.633 | 1.00 | 0.00 | AAAA |
| ATOM | 2298 | C | ILE | E | 139 | 29.781 | 125.430 | 45.595 | 1.00 | 0.00 | AAAA |
| ATOM | 2299 | O | ILE | E | 139 | 30.496 | 126.321 | 46.037 | 1.00 | 0.00 | AAAA |
| ATOM | 2300 | N | VAL | E | 140 | 28.776 | 124.838 | 46.256 | 1.00 | 0.00 | AAAA |
| ATOM | 2302 | CA | VAL | E | 140 | 28.356 | 125.146 | 47.608 | 1.00 | 0.00 | AAAA |
| ATOM | 2304 | CB | VAL | E | 140 | 26.837 | 125.212 | 47.805 | 1.00 | 0.00 | AAAA |
| ATOM | 2306 | CG1 | VAL | E | 140 | 26.441 | 125.688 | 49.200 | 1.00 | 0.00 | AAAA |
| ATOM | 2310 | CG2 | VAL | E | 140 | 26.247 | 126.034 | 46.653 | 1.00 | 0.00 | AAAA |
| ATOM | 2314 | C | VAL | E | 140 | 28.960 | 124.054 | 48.482 | 1.00 | 0.00 | AAAA |
| ATOM | 2315 | O | VAL | E | 140 | 28.977 | 122.901 | 48.063 | 1.00 | 0.00 | AAAA |
| ATOM | 2316 | N | GLY | E | 141 | 29.468 | 124.304 | 49.688 | 1.00 | 0.00 | AAAA |
| ATOM | 2318 | CA | GLY | E | 141 | 29.864 | 123.221 | 50.567 | 1.00 | 0.00 | AAAA |
| ATOM | 2321 | C | GLY | E | 141 | 31.360 | 122.964 | 50.715 | 1.00 | 0.00 | AAAA |
| ATOM | 2322 | O | GLY | E | 141 | 31.830 | 121.842 | 50.850 | 1.00 | 0.00 | AAAA |
| ATOM | 2323 | N | ASN | E | 142 | 32.170 | 124.021 | 50.682 | 1.00 | 0.00 | AAAA |
| ATOM | 2325 | CA | ASN | E | 142 | 33.611 | 124.000 | 50.840 | 1.00 | 0.00 | AAAA |
| ATOM | 2327 | CB | ASN | E | 142 | 34.312 | 124.136 | 49.483 | 1.00 | 0.00 | AAAA |
| ATOM. | 2330 | CG | ASN | E | 142 | 33.878 | 123.134 | 48.428 | 1.00 | 0.00 | AAAA |
| ATOM | 2331 | OD1 | ASN | E | 142 | 34.218 | 121.956 | 48.443 | 1.00 | 0.00 | AAAA |
| ATOM | 2332 | ND2 | ASN | E | 142 | 33.186 | 123.544 | 47.364 | 1.00 | 0.00 | AAAA |
| ATOM | 2335 | C | ASN | E | 142 | 33.975 | 125.110 | 51.818 | 1.00 | 0.00 | AAAA |
| ATOM | 2336 | O | ASN | E | 142 | 33.116 | 125.527 | 52.581 | 1.00 | 0.00 | AAAA |
| ATOM | 2337 | N | LYS | E | 143 | 35.216 | 125.612 | 51.851 | 1.00 | 0.00 | AAAA |
| ATOM | 2339 | CA | LYS | E | 143 | 35.754 | 126.616 | 52.734 | 1.00 | 0.00 | AAAA |
| ATOM | 2341 | CB | LYS | E | 143 | 37.246 | 126.318 | 52.882 | 1.00 | 0.00 | AAAA |
| ATOM | 2344 | CG | LYS | E | 143 | 37.756 | 126.848 | 54.218 | 1.00 | 0.00 | AAAA |
| ATOM | 2347 | CD | LYS | E | 143 | 39.224 | 126.482 | 54.462 | 1.00 | 0.00 | AAAA |
| ATOM | 2350 | CE | LYS | E | 143 | 39.609 | 126.650 | 55.927 | 1.00 | 0.00 | AAAA |
| ATOM | 2353 | NZ | LYS | E | 143 | 41.063 | 126.687 | 56.164 | 1.00 | 0.00 | AAAA |
| ATOM | 2357 | C | LYS | E | 143 | 35.452 | 127.987 | 52.155 | 1.00 | 0.00 | AAAA |
| ATOM | 2358 | O | LYS | E | 143 | 35.584 | 128.157 | 50.945 | 1.00 | 0.00 | AAAA |
| ATOM | 2359 | N | PRO | E | 144 | 34.982 | 128.969 | 52.931 | 1.00 | 0.00 | AAAA |
| ATOM | 2360 | CA | PRO | E | 144 | 34.557 | 130.291 | 52.490 | 1.00 | 0.00 | AAAA |
| ATOM | 2362 | CB | PRO | E | 144 | 33.567 | 130.799 | 53.533 | 1.00 | 0.00 | AAAA |
| ATOM | 2365 | CG | PRO | E | 144 | 34.098 | 130.121 | 54.794 | 1.00 | 0.00 | AAAA |
| ATOM | 2368 | CD | PRO | E | 144 | 34.683 | 128.783 | 54.331 | 1.00 | 0.00 | AAAA |
| ATOM | 2371 | C | PRO | E | 144 | 35.717 | 131.256 | 52.249 | 1.00 | 0.00 | AAAA |
| ATOM | 2372 | O | PRO | E | 144 | 36.705 | 131.243 | 52.972 | 1.00 | 0.00 | AAAA |
| ATOM | 2373 | N | PRO | E | 145 | 35.615 | 132.180 | 51.287 | 1.00 | 0.00 | AAAA |
| ATOM | 2374 | CA | PRO | E | 145 | 36.637 | 133.139 | 50.943 | 1.00 | 0.00 | AAAA |
| ATOM | 2376 | CB | PRO | E | 145 | 36.006 | 134.065 | 49.904 | 1.00 | 0.00 | AAAA |
| ATOM | 2379 | CG | PRO | E | 145 | 34.967 | 133.179 | 49.208 | 1.00 | 0.00 | AAAA |
| ATOM | 2382 | CD | PRO | E | 145 | 34.574 | 132.161 | 50.278 | 1.00 | 0.00 | AAAA |
| ATOM | 2385 | C | PRO | E | 145 | 37.240 | 133.994 | 52.047 | 1.00 | 0.00 | AAAA |
| ATOM | 2386 | O | PRO | E | 145 | 38.424 | 134.301 | 52.027 | 1.00 | 0.00 | AAAA |
| ATOM | 2387 | N | LYS | E | 146 | 36.480 | 134.318 | 53.098 | 1.00 | 0.00 | AAAA |
| ATOM | 2389 | CA | LYS | E | 146 | 36.906 | 134.937 | 54.337 | 1.00 | 0.00 | AAAA |
| ATOM | 2391 | CB | LYS | E | 146 | 35.679 | 135.330 | 55.157 | 1.00 | 0.00 | AAAA |
| ATOM | 2394 | CG | LYS | E | 146 | 34.781 | 134.157 | 55.535 | 1.00 | 0.00 | AAAA |
| ATOM | 2397 | CD | LYS | E | 146 | 33.459 | 134.520 | 56.221 | 1.00 | 0.00 | AAAA |
| ATOM | 2400 | CE | LYS | E | 146 | 32.507 | 135.265 | 55.289 | 1.00 | 0.00 | AAAA |
| ATOM | 2403 | NZ | LYS | E | 146 | 31.417 | 135.865 | 56.081 | 1.00 | 0.00 | AAAA |
| ATOM | 2407 | C | LYS | E | 146 | 37.876 | 134.141 | 55.199 | 1.00 | 0.00 | AAAA |
| ATOM | 2408 | O | LYS | E | 146 | 38.765 | 134.711 | 55.836 | 1.00 | 0.00 | AAAA |
| ATOM | 2409 | N | GLU | E | 147 | 37.781 | 132.815 | 55.100 | 1.00 | 0.00 | AAAA |
| ATOM | 2411 | CA | GLU | E | 147 | 38.472 | 131.835 | 55.917 | 1.00 | 0.00 | AAAA |
| ATOM | 2413 | CB | GLU | E | 147 | 37.555 | 131.280 | 57.009 | 1.00 | 0.00 | AAAA |
| ATOM | 2416 | CG | GLU | E | 147 | 38.225 | 130.530 | 58.161 | 1.00 | 0.00 | AAAA |
| ATOM | 2419 | CD | GLU | E | 147 | 39.171 | 131.343 | 59.039 | 1.00 | 0.00 | AAAA |
| ATOM | 2420 | OE1 | GLU | E | 147 | 38.854 | 132.506 | 59.365 | 1.00 | 0.00 | AAAA |
| ATOM | 2421 | OE2 | GLU | E | 147 | 40.216 | 130.786 | 59.432 | 1.00 | 0.00 | AAAA |
| ATOM | 2422 | C | GLU | E | 147 | 39.304 | 130.909 | 55.044 | 1.00 | 0.00 | AAAA |
| ATOM | 2423 | O | GLU | E | 147 | 39.820 | 129.870 | 55.446 | 1.00 | 0.00 | AAAA |
| ATOM | 2424 | N | CYS | E | 148 | 39.453 | 131.339 | 53.795 | 1.00 | 0.00 | AAAA |
| ATOM | 2426 | CA | CYS | E | 148 | 40.179 | 130.656 | 52.740 | 1.00 | 0.00 | AAAA |
| ATOM | 2428 | CB | CYS | E | 148 | 39.244 | 129.700 | 51.986 | 1.00 | 0.00 | AAAA |
| ATOM | 2431 | SG | CYS | E | 148 | 39.979 | 128.178 | 51.345 | 1.00 | 0.00 | AAAA |
| ATOM | 2432 | C | CYS | E | 148 | 40.815 | 131.719 | 51.855 | 1.00 | 0.00 | AAAA |
| ATOM | 2433 | O | CYS | E | 148 | 40.934 | 132.900 | 52.183 | 1.00 | 0.00 | AAAA |
| ATOM | 2434 | N | GLY | E | 149 | 41.378 | 131.304 | 50.724 | 1.00 | 0.00 | AAAA |
| ATOM | 2436 | CA | GLY | E | 149 | 41.969 | 132.161 | 49.712 | 1.00 | 0.00 | AAAA |
| ATOM | 2439 | C | GLY | E | 149 | 41.014 | 132.477 | 48.573 | 1.00 | 0.00 | AAAA |
| ATOM | 2440 | O | GLY | E | 149 | 40.055 | 131.769 | 48.271 | 1.00 | 0.00 | AAAA |
| ATOM | 2441 | N | ASP | E | 150 | 41.233 | 133.637 | 47.945 | 1.00 | 0.00 | AAAA |
| ATOM | 2443 | CA | ASP | E | 150 | 40.496 | 134.253 | 46.852 | 1.00 | 0.00 | AAAA |
| ATOM | 2445 | CB | ASP | E | 150 | 39.269 | 134.979 | 47.400 | 1.00 | 0.00 | AAAA |
| ATOM | 2448 | CG | ASP | E | 150 | 38.744 | 136.194 | 46.644 | 1.00 | 0.00 | AAAA |
| ATOM | 2449 | OD1 | ASP | E | 150 | 37.884 | 135.975 | 45.767 | 1.00 | 0.00 | AAAA |
| ATOM | 2450 | OD2 | ASP | E | 150 | 39.120 | 137.329 | 47.015 | 1.00 | 0.00 | AAAA |
| ATOM | 2451 | C | ASP | E | 150 | 41.396 | 135.079 | 45.952 | 1.00 | 0.00 | AAAA |
| ATOM | 2452 | O | ASP | E | 150 | 41.153 | 135.345 | 44.774 | 1.00 | 0.00 | AAAA |
| ATOM | 2453 | N | LEU | E | 151 | 42.458 | 135.612 | 46.561 | 1.00 | 0.00 | AAAA |
| ATOM | 2455 | CA | LEU | E | 151 | 43.414 | 136.510 | 45.947 | 1.00 | 0.00 | AAAA |
| ATOM | 2457 | CB | LEU | E | 151 | 44.395 | 137.037 | 46.996 | 1.00 | 0.00 | AAAA |
| ATOM. | 2460 | CG | LEU | E | 151 | 45.371 | 138.152 | 46.601 | 1.00 | 0.00 | AAAA |
| ATOM | 2462 | CD1 | LEU | E | 151 | 44.825 | 139.477 | 46.054 | 1.00 | 0.00 | AAAA |
| ATOM | 2466 | CD2 | LEU | E | 151 | 46.279 | 138.474 | 47.782 | 1.00 | 0.00 | AAAA |
| ATOM | 2470 | C | LEU | E | 151 | 44.143 | 135.919 | 44.757 | 1.00 | 0.00 | AAAA |
| ATOM | 2471 | O | LEU | E | 151 | 44.836 | 134.906 | 44.903 | 1.00 | 0.00 | AAAA |
| ATOM | 2472 | N | CYS | E | 152 | 44.022 | 136.546 | 43.581 | 1.00 | 0.00 | AAAA |
| ATOM | 2474 | CA | CYS | E | 152 | 44.681 | 136.122 | 42.360 | 1.00 | 0.00 | AAAA |
| ATOM | 2476 | CB | CYS | E | 152 | 43.900 | 136.752 | 41.211 | 1.00 | 0.00 | AAAA |
| ATOM | 2479 | SG | CYS | E | 152 | 42.188 | 136.218 | 40.974 | 1.00 | 0.00 | AAAA |
| ATOM | 2480 | C | CYS | E | 152 | 46.120 | 136.615 | 42.404 | 1.00 | 0.00 | AAAA |
| ATOM | 2481 | O | CYS | E | 152 | 46.414 | 137.655 | 42.990 | 1.00 | 0.00 | AAAA |
| ATOM | 2482 | N | PRO | E | 153 | 47.044 | 135.980 | 41.672 | 1.00 | 0.00 | AAAA |
| ATOM | 2483 | CA | PRO | E | 153 | 48.442 | 136.338 | 41.541 | 1.00 | 0.00 | AAAA |
| ATOM | 2485 | CB | PRO | E | 153 | 49.122 | 135.306 | 40.643 | 1.00 | 0.00 | AAAA |
| ATOM | 2488 | CG | PRO | E | 153 | 47.948 | 134.634 | 39.930 | 1.00 | 0.00 | AAAA |
| ATOM | 2491 | CD | PRO | E | 153 | 46.715 | 134.885 | 40.789 | 1.00 | 0.00 | AAAA |
| ATOM | 2494 | C | PRO | E | 153 | 48.715 | 137.724 | 40.973 | 1.00 | 0.00 | AAAA |
| ATOM | 2495 | O | PRO | E | 153 | 47.900 | 138.342 | 40.285 | 1.00 | 0.00 | AAAA |
| ATOM | 2496 | N | GLY | E | 154 | 49.917 | 138.257 | 41.230 | 1.00 | 0.00 | AAAA |
| ATOM | 2498 | CA | GLY | E | 154 | 50.513 | 139.490 | 40.754 | 1.00 | 0.00 | AAAA |
| ATOM | 2501 | C | GLY | E | 154 | 51.173 | 140.137 | 41.956 | 1.00 | 0.00 | AAAA |
| ATOM | 2502 | O | GLY | E | 154 | 50.548 | 140.197 | 43.016 | 1.00 | 0.00 | AAAA |
| ATOM | 2503 | N | THR | E | 155 | 52.346 | 140.758 | 41.803 | 1.00 | 0.00 | AAAA |
| ATOM | 2505 | CA | THR | E | 155 | 53.126 | 141.425 | 42.831 | 1.00 | 0.00 | AAAA |
| ATOM | 2507 | CB | THR | E | 155 | 54.220 | 140.451 | 43.268 | 1.00 | 0.00 | AAAA |
| ATOM | 2509 | OG1 | THR | E | 155 | 55.032 | 140.051 | 42.183 | 1.00 | 0.00 | AAAA |
| ATOM | 2511 | CG2 | THR | E | 155 | 53.813 | 139.216 | 44.075 | 1.00 | 0.00 | AAAA |
| ATOM | 2515 | C | THR | E | 155 | 53.622 | 142.818 | 42.473 | 1.00 | 0.00 | AAAA |
| ATOM | 2516 | O | THR | E | 155 | 54.220 | 143.495 | 43.306 | 1.00 | 0.00 | AAAA |
| ATOM | 2517 | N | MET | E | 156 | 53.433 | 143.381 | 41.279 | 1.00 | 0.00 | AAAA |
| ATOM | 2519 | CA | MET | E | 156 | 53.937 | 144.643 | 40.764 | 1.00 | 0.00 | AAAA |
| ATOM | 2521 | CB | MET | E | 156 | 53.993 | 144.593 | 39.238 | 1.00 | 0.00 | AAAA |
| ATOM | 2524 | CG | MET | E | 156 | 54.742 | 145.703 | 38.498 | 1.00 | 0.00 | AAAA |
| ATOM | 2527 | SD | MET | E | 156 | 54.817 | 145.413 | 36.709 | 1.00 | 0.00 | AAAA |
| ATOM | 2528 | CE | MET | E | 156 | 55.785 | 146.802 | 36.064 | 1.00 | 0.00 | AAAA |
| ATOM | 2532 | C | MET | E | 156 | 53.102 | 145.846 | 41.163 | 1.00 | 0.00 | AAAA |
| ATOM | 2533 | O | MET | E | 156 | 53.566 | 146.985 | 41.279 | 1.00 | 0.00 | AAAA |
| ATOM | 2534 | N | GLU | E | 157 | 51.839 | 145.533 | 41.479 | 1.00 | 0.00 | AAAA |
| ATOM | 2536 | CA | GLU | E | 157 | 50.785 | 146.404 | 41.954 | 1.00 | 0.00 | AAAA |
| ATOM | 2538 | CB | GLU | E | 157 | 50.105 | 147.076 | 40.751 | 1.00 | 0.00 | AAAA |
| ATOM | 2541 | CG | GLU | E | 157 | 49.149 | 148.252 | 40.914 | 1.00 | 0.00 | AAAA |
| ATOM | 2544 | CD | GLU | E | 157 | 49.430 | 149.370 | 41.908 | 1.00 | 0.00 | AAAA |
| ATOM | 2545 | OE1 | GLU | E | 157 | 49.449 | 150.558 | 41.506 | 1.00 | 0.00 | AAAA |
| ATOM | 2546 | OE2 | GLU | E | 157 | 49.315 | 149.038 | 43.103 | 1.00 | 0.00 | AAAA |
| ATOM | 2547 | C | GLU | E | 157 | 49.913 | 145.695 | 42.975 | 1.00 | 0.00 | AAAA |
| ATOM | 2548 | O | GLU | E | 157 | 50.246 | 144.544 | 43.274 | 1.00 | 0.00 | AAAA |
| ATOM | 2549 | N | GLU | E | 158 | 48.947 | 146.386 | 43.580 | 1.00 | 0.00 | AAAA |
| ATOM | 2551 | CA | GLU | E | 158 | 47.851 | 145.772 | 44.301 | 1.00 | 0.00 | AAAA |
| ATOM | 2553 | CB | GLU | E | 158 | 47.158 | 146.876 | 45.102 | 1.00 | 0.00 | AAAA |
| ATOM | 2556 | CG | GLU | E | 158 | 47.813 | 147.261 | 46.435 | 1.00 | 0.00 | AAAA |
| ATOM | 2559 | CD | GLU | E | 158 | 47.522 | 146.233 | 47.513 | 1.00 | 0.00 | AAAA |
| ATOM | 2560 | OE1 | GLU | E | 158 | 46.927 | 146.541 | 48.568 | 1.00 | 0.00 | AAAA |
| ATOM | 2561 | OE2 | GLU | E | 158 | 47.821 | 145.030 | 47.334 | 1.00 | 0.00 | AAAA |
| ATOM | 2562 | C | GLU | E | 158 | 46.880 | 145.153 | 43.312 | 1.00 | 0.00 | AAAA |
| ATOM | 2563 | O | GLU | E | 158 | 46.154 | 144.234 | 43.707 | 1.00 | 0.00 | AAAA |
| ATOM | 2564 | N | LYS | E | 159 | 46.863 | 145.509 | 42.028 | 1.00 | 0.00 | AAAA |
| ATOM | 2566 | CA | LYS | E | 159 | 46.160 | 144.855 | 40.943 | 1.00 | 0.00 | AAAA |
| ATOM | 2568 | CB | LYS | E | 159 | 46.213 | 145.846 | 39.779 | 1.00 | 0.00 | AAAA |
| ATOM | 2571 | CG | LYS | E | 159 | 45.169 | 146.950 | 40.007 | 1.00 | 0.00 | AAAA |
| ATOM | 2574 | CD | LYS | E | 159 | 44.883 | 147.665 | 38.693 | 1.00 | 0.00 | AAAA |
| ATOM | 2577 | CE | LYS | E | 159 | 43.634 | 148.540 | 38.687 | 1.00 | 0.00 | AAAA |
| ATOM | 2580 | NZ | LYS | E | 159 | 42.415 | 147.725 | 38.519 | 1.00 | 0.00 | AAAA |
| ATOM | 2584 | C | LYS | E | 159 | 46.765 | 143.503 | 40.627 | 1.00 | 0.00 | AAAA |
| ATOM | 2585 | O | LYS | E | 159 | 47.970 | 143.344 | 40.792 | 1.00 | 0.00 | AAAA |
| ATOM | 2586 | N | PRO | E | 160 | 46.002 | 142.517 | 40.134 | 1.00 | 0.00 | AAAA |
| ATOM | 2587 | CA | PRO | E | 160 | 46.510 | 141.216 | 39.738 | 1.00 | 0.00 | AAAA |
| ATOM | 2589 | CB | PRO | E | 160 | 45.233 | 140.384 | 39.868 | 1.00 | 0.00 | AAAA |
| ATOM | 2592 | CG | PRO | E | 160 | 44.110 | 141.312 | 39.415 | 1.00 | 0.00 | AAAA |
| ATOM | 2595 | CD | PRO | E | 160 | 44.569 | 142.648 | 39.999 | 1.00 | 0.00 | AAAA |
| ATOM | 2598 | C | PRO | E | 160 | 46.991 | 141.214 | 38.289 | 1.00 | 0.00 | AAAA |
| ATOM | 2599 | O | PRO | E | 160 | 46.768 | 142.098 | 37.465 | 1.00 | 0.00 | AAAA |
| ATOM | 2600 | N | MET | E | 161 | 47.879 | 140.246 | 38.045 | 1.00 | 0.00 | AAAA |
| ATOM | 2602 | CA | MET | E | 161 | 48.479 | 139.930 | 36.766 | 1.00 | 0.00 | AAAA |
| ATOM | 2604 | CB | MET | E | 161 | 49.669 | 138.988 | 36.940 | 1.00 | 0.00 | AAAA |
| ATOM | 2607 | CG | MET | E | 161 | 49.286 | 137.517 | 37.065 | 1.00 | 0.00 | AAAA |
| ATOM | 2610 | SD | MET | E | 161 | 50.731 | 136.459 | 37.344 | 1.00 | 0.00 | AAAA |
| ATOM | 2611 | CE | MET | E | 161 | 50.207 | 134.880 | 36.622 | 1.00 | 0.00 | AAAA |
| ATOM | 2615 | C | MET | E | 161 | 47.488 | 139.414 | 35.733 | 1.00 | 0.00 | AAAA |
| ATOM | 2616 | O | MET | E | 161 | 47.922 | 139.142 | 34.618 | 1.00 | 0.00 | AAAA |
| ATOM | 2617 | N | CYS | E | 162 | 46.192 | 139.275 | 36.018 | 1.00 | 0.00 | AAAA |
| ATOM | 2619 | CA | CYS | E | 162 | 45.124 | 138.755 | 35.187 | 1.00 | 0.00 | AAAA |
| ATOM | 2621 | CB | CYS | E | 162 | 44.086 | 137.993 | 36.015 | 1.00 | 0.00 | AAAA |
| ATOM | 2624 | SG | CYS | E | 162 | 44.637 | 136.772 | 37.234 | 1.00 | 0.00 | AAAA |
| ATOM | 2625 | C | CYS | E | 162 | 44.534 | 139.945 | 34.450 | 1.00 | 0.00 | AAAA |
| ATOM | 2626 | O | CYS | E | 162 | 44.505 | 141.037 | 35.020 | 1.00 | 0.00 | AAAA |
| ATOM | 2627 | N | GLU | E | 163 | 44.197 | 139.810 | 33.171 | 1.00 | 0.00 | AAAA |
| ATOM | 2629 | CA | GLU | E | 163 | 43.363 | 140.752 | 32.453 | 1.00 | 0.00 | AAAA |
| ATOM | 2631 | CB | GLU | E | 163 | 43.452 | 140.296 | 30.995 | 1.00 | 0.00 | AAAA |
| ATOM | 2634 | CG | GLU | E | 163 | 42.773 | 141.217 | 29.972 | 1.00 | 0.00 | AAAA |
| ATOM | 2637 | CD | GLU | E | 163 | 43.457 | 142.569 | 29.814 | 1.00 | 0.00 | AAAA |
| ATOM | 2638 | OE1 | GLU | E | 163 | 42.867 | 143.565 | 29.352 | 1.00 | 0.00 | AAAA |
| ATOM | 2639 | OE2 | GLU | E | 163 | 44.597 | 142.764 | 30.289 | 1.00 | 0.00 | AAAA |
| ATOM | 2640 | C | GLU | E | 163 | 41.950 | 140.743 | 33.037 | 1.00 | 0.00 | AAAA |
| ATOM | 2641 | O | GLU | E | 163 | 41.435 | 139.704 | 33.424 | 1.00 | 0.00 | AAAA |
| ATOM | 2642 | N | LYS | E | 164 | 41.296 | 141.901 | 33.090 | 1.00 | 0.00 | AAAA |
| ATOM | 2644 | CA | LYS | E | 164 | 39.955 | 142.107 | 33.602 | 1.00 | 0.00 | AAAA |
| ATOM | 2646 | CB | LYS | E | 164 | 39.705 | 143.564 | 33.981 | 1.00 | 0.00 | AAAA |
| ATOM | 2649 | CG | LYS | E | 164 | 38.888 | 143.751 | 35.255 | 1.00 | 0.00 | AAAA |
| ATOM | 2652 | CD | LYS | E | 164 | 38.699 | 145.223 | 35.602 | 1.00 | 0.00 | AAAA |
| ATOM | 2655 | CE | LYS | E | 164 | 38.099 | 145.498 | 36.980 | 1.00 | 0.00 | AAAA |
| ATOM | 2658 | NZ | LYS | E | 164 | 37.976 | 146.934 | 37.277 | 1.00 | 0.00 | AAAA |
| ATOM | 2662 | C | LYS | E | 164 | 39.010 | 141.809 | 32.444 | 1.00 | 0.00 | AAAA |
| ATOM | 2663 | O | LYS | E | 164 | 39.226 | 142.151 | 31.280 | 1.00 | 0.00 | AAAA |
| ATOM | 2664 | N | THR | E | 165 | 37.851 | 141.215 | 32.724 | 1.00 | 0.00 | AAAA |
| ATOM | 2666 | CA | THR | E | 165 | 37.016 | 140.421 | 31.847 | 1.00 | 0.00 | AAAA |
| ATOM | 2668 | CB | THR | E | 165 | 37.560 | 139.000 | 31.988 | 1.00 | 0.00 | AAAA |
| ATOM | 2670 | OG1 | THR | E | 165 | 37.803 | 138.541 | 33.297 | 1.00 | 0.00 | AAAA |
| ATOM | 2672 | CG2 | THR | E | 165 | 38.818 | 138.786 | 31.132 | 1.00 | 0.00 | AAAA |
| ATOM | 2676 | C | THR | E | 165 | 35.557 | 140.619 | 32.245 | 1.00 | 0.00 | AAAA |
| ATOM | 2677 | O | THR | E | 165 | 35.232 | 140.789 | 33.416 | 1.00 | 0.00 | AAAA |
| ATOM | 2678 | N | THR | E | 166 | 34.671 | 140.384 | 31.279 | 1.00 | 0.00 | AAAA |
| ATOM | 2680 | CA | THR | E | 166 | 33.290 | 140.829 | 31.258 | 1.00 | 0.00 | AAAA |
| ATOM | 2682 | CB | THR | E | 166 | 32.706 | 140.539 | 29.871 | 1.00 | 0.00 | AAAA |
| ATOM | 2684 | OG1 | THR | E | 166 | 32.764 | 139.170 | 29.539 | 1.00 | 0.00 | AAAA |
| ATOM | 2686 | CG2 | THR | E | 166 | 33.315 | 141.395 | 28.762 | 1.00 | 0.00 | AAAA |
| ATOM | 2690 | C | THR | E | 166 | 32.392 | 140.143 | 32.284 | 1.00 | 0.00 | AAAA |
| ATOM | 2691 | O | THR | E | 166 | 32.462 | 138.948 | 32.553 | 1.00 | 0.00 | AAAA |
| ATOM | 2692 | N | ILE | E | 167 | 31.542 | 140.963 | 32.899 | 1.00 | 0.00 | AAAA |
| ATOM | 2694 | CA | ILE | E | 167 | 30.475 | 140.515 | 33.764 | 1.00 | 0.00 | AAAA |
| ATOM | 2696 | CB | ILE | E | 167 | 30.888 | 140.249 | 35.211 | 1.00 | 0.00 | AAAA |
| ATOM | 2698 | CG1 | ILE | E | 167 | 29.942 | 139.318 | 35.966 | 1.00 | 0.00 | AAAA |
| ATOM | 2701 | CG2 | ILE | E | 167 | 31.325 | 141.505 | 35.976 | 1.00 | 0.00 | AAAA |
| ATOM | 2705 | CD1 | ILE | E | 167 | 30.270 | 139.161 | 37.447 | 1.00 | 0.00 | AAAA |
| ATOM | 2709 | C | ILE | E | 167 | 29.149 | 141.190 | 33.429 | 1.00 | 0.00 | AAAA |
| ATOM | 2710 | O | ILE | E | 167 | 28.198 | 141.154 | 34.203 | 1.00 | 0.00 | AAAA |
| ATOM | 2711 | N | ASN | E | 168 | 29.106 | 141.739 | 32.213 | 1.00 | 0.00 | AAAA |
| ATOM | 2713 | CA | ASN | E | 168 | 28.029 | 142.443 | 31.550 | 1.00 | 0.00 | AAAA |
| ATOM | 2715 | CB | ASN | E | 168 | 26.640 | 141.812 | 31.751 | 1.00 | 0.00 | AAAA |
| ATOM | 2718 | CG | ASN | E | 168 | 25.499 | 142.254 | 30.855 | 1.00 | 0.00 | AAAA |
| ATOM | 2719 | OD1 | ASN | E | 168 | 24.913 | 143.314 | 31.043 | 1.00 | 0.00 | AAAA |
| ATOM | 2720 | ND2 | ASN | E | 168 | 24.891 | 141.357 | 30.075 | 1.00 | 0.00 | AAAA |
| ATOM | 2723 | C | ASN | E | 168 | 28.030 | 143.906 | 31.952 | 1.00 | 0.00 | AAAA |
| ATOM | 2724 | O | ASN | E | 168 | 27.606 | 144.227 | 33.060 | 1.00 | 0.00 | AAAA |
| ATOM | 2725 | N | ASN | E | 169 | 28.587 | 144.815 | 31.143 | 1.00 | 0.00 | AAAA |
| ATOM | 2727 | CA | ASN | E | 169 | 28.893 | 146.220 | 31.319 | 1.00 | 0.00 | AAAA |
| ATOM | 2729 | CB | ASN | E | 169 | 27.762 | 147.159 | 31.739 | 1.00 | 0.00 | AAAA |
| ATOM | 2732 | CG | ASN | E | 169 | 27.552 | 148.226 | 30.672 | 1.00 | 0.00 | AAAA |
| ATOM | 2733 | OD1 | ASN | E | 169 | 28.490 | 148.952 | 30.345 | 1.00 | 0.00 | AAAA |
| ATOM | 2734 | ND2 | ASN | E | 169 | 26.300 | 148.282 | 30.204 | 1.00 | 0.00 | AAAA |
| ATOM | 2737 | C | ASN | E | 169 | 30.121 | 146.302 | 32.221 | 1.00 | 0.00 | AAAA |
| ATOM | 2738 | O | ASN | E | 169 | 31.178 | 146.846 | 31.923 | 1.00 | 0.00 | AAAA |
| ATOM | 2739 | N | GLU | E | 170 | 30.047 | 145.784 | 33.453 | 1.00 | 0.00 | AAAA |
| ATOM | 2741 | CA | GLU | E | 170 | 31.100 | 145.612 | 34.432 | 1.00 | 0.00 | AAAA |
| ATOM | 2743 | CB | GLU | E | 170 | 30.439 | 145.251 | 35.768 | 1.00 | 0.00 | AAAA |
| ATOM | 2746 | CG | GLU | E | 170 | 31.450 | 145.151 | 36.909 | 1.00 | 0.00 | AAAA |
| ATOM | 2749 | CD | GLU | E | 170 | 31.920 | 146.476 | 37.476 | 1.00 | 0.00 | AAAA |
| ATOM | 2750 | OE1 | GLU | E | 170 | 31.280 | 146.950 | 38.443 | 1.00 | 0.00 | AAAA |
| ATOM | 2751 | OE2 | GLU | E | 170 | 32.782 | 147.032 | 36.769 | 1.00 | 0.00 | AAAA |
| ATOM | 2752 | C | GLU | E | 170 | 32.118 | 144.573 | 33.966 | 1.00 | 0.00 | AAAA |
| ATOM | 2753 | O | GLU | E | 170 | 31.823 | 143.716 | 33.141 | 1.00 | 0.00 | AAAA |
| ATOM | 2754 | N | TYR | E | 171 | 33.292 | 144.694 | 34.577 | 1.00 | 0.00 | AAAA |
| ATOM | 2756 | CA | TYR | E | 171 | 34.461 | 143.838 | 34.454 | 1.00 | 0.00 | AAAA |
| ATOM | 2758 | CB | TYR | E | 171 | 35.482 | 144.571 | 33.592 | 1.00 | 0.00 | AAAA |
| ATOM | 2761 | CG | TYR | E | 171 | 35.258 | 144.879 | 32.124 | 1.00 | 0.00 | AAAA |
| ATOM | 2762 | CD1 | TYR | E | 171 | 35.784 | 144.065 | 31.112 | 1.00 | 0.00 | AAAA |
| ATOM | 2764 | CD2 | TYR | E | 171 | 34.395 | 145.902 | 31.731 | 1.00 | 0.00 | AAAA |
| ATOM | 2766 | CE1 | TYR | E | 171 | 35.577 | 144.380 | 29.763 | 1.00 | 0.00 | AAAA |
| ATOM | 2768 | CE2 | TYR | E | 171 | 34.152 | 146.160 | 30.373 | 1.00 | 0.00 | AAAA |
| ATOM | 2770 | CZ | TYR | E | 171 | 34.794 | 145.455 | 29.349 | 1.00 | 0.00 | AAAA |
| ATOM | 2771 | OH | TYR | E | 171 | 34.683 | 145.691 | 28.008 | 1.00 | 0.00 | AAAA |
| ATOM | 2773 | C | TYR | E | 171 | 34.971 | 143.323 | 35.788 | 1.00 | 0.00 | AAAA |
| ATOM | 2774 | O | TYR | E | 171 | 34.743 | 143.907 | 36.844 | 1.00 | 0.00 | AAAA |
| ATOM | 2775 | N | ASN | E | 172 | 35.531 | 142.113 | 35.724 | 1.00 | 0.00 | AAAA |
| ATOM | 2777 | CA | ASN | E | 172 | 35.940 | 141.348 | 36.883 | 1.00 | 0.00 | AAAA |
| ATOM | 2779 | CB | ASN | E | 172 | 34.849 | 140.306 | 37.137 | 1.00 | 0.00 | AAAA |
| ATOM | 2782 | CG | ASN | E | 172 | 34.796 | 139.815 | 38.574 | 1.00 | 0.00 | AAAA |
| ATOM | 2783 | OD1 | ASN | E | 172 | 35.678 | 139.071 | 39.001 | 1.00 | 0.00 | AAAA |
| ATOM | 2784 | ND2 | ASN | E | 172 | 33.775 | 140.197 | 39.339 | 1.00 | 0.00 | AAAA |
| ATOM | 2787 | C | ASN | E | 172 | 37.298 | 140.738 | 36.536 | 1.00 | 0.00 | AAAA |
| ATOM | 2788 | O | ASN | E | 172 | 37.620 | 140.537 | 35.365 | 1.00 | 0.00 | AAAA |
| ATOM | 2789 | N | TYR | E | 173 | 38.159 | 140.463 | 37.514 | 1.00 | 0.00 | AAAA |
| ATOM | 2791 | CA | TYR | E | 173 | 39.325 | 139.629 | 37.292 | 1.00 | 0.00 | AAAA |
| ATOM | 2793 | CB | TYR | E | 173 | 40.479 | 140.070 | 38.197 | 1.00 | 0.00 | AAAA |
| ATOM | 2796 | CG | TYR | E | 173 | 41.024 | 141.447 | 37.908 | 1.00 | 0.00 | AAAA |
| ATOM | 2797 | CD1 | TYR | E | 173 | 41.877 | 141.706 | 36.827 | 1.00 | 0.00 | AAAA |
| ATOM | 2799 | CD2 | TYR | E | 173 | 40.725 | 142.532 | 38.732 | | 0.00 | AAAA |
| ATOM | 2801 | CE1 | TYR | E | 173 | 42.376 | 142.987 | 36.572 | 1.00 | 0.00 | AAAA |
| ATOM | 2803 | CE2 | TYR | E | 173 | 41.200 | 143.825 | 38.461 | 1.00 | 0.00 | AAAA |
| ATOM | 2805 | CZ | TYR | E | 173 | 42.061 | 144.074 | 37.386 | 1.00 | 0.00 | AAAA |
| ATOM | 2806 | OH | TYR | E | 173 | 42.605 | 145.300 | 37.128 | 1.00 | 0.00 | AAAA |
| ATOM | 2808 | C | TYR | E | 173 | 38.991 | 138.220 | 37.748 | 1.00 | 0.00 | AAAA |
| ATOM | 2809 | O | TYR | E | 173 | 38.528 | 137.972 | 38.861 | 1.00 | 0.00 | AAAA |
| ATOM | 2810 | N | ARG | E | 174 | 39.293 | 137.204 | 36.946 | 1.00 | 0.00 | AAAA |
| ATOM | 2812 | CA | ARG | E | 174 | 39.107 | 135.782 | 37.200 | 1.00 | 0.00 | AAAA |
| ATOM | 2814 | CB | ARG | E | 174 | 38.335 | 135.115 | 36.070 | 1.00 | 0.00 | AAAA |
| ATOM | 2817 | CG | ARG | E | 174 | 36.981 | 135.763 | 35.754 | 1.00 | 0.00 | AAAA |
| ATOM | 2820 | CD | ARG | E | 174 | 36.103 | 135.886 | 37.002 | 1.00 | 0.00 | AAAA |
| ATOM | 2823 | NE | ARG | E | 174 | 34.776 | 136.301 | 36.537 | 1.00 | 0.00 | AAAA |
| ATOM | 2825 | CZ | ARG | E | 174 | 33.699 | 136.086 | 37.310 | 1.00 | 0.00 | AAAA |
| ATOM | 2826 | NH1 | ARG | E | 174 | 33.689 | 135.742 | 38.599 | 1.00 | 0.00 | AAAA |
| ATOM | 2829 | NH2 | ARG | E | 174 | 32.527 | 136.156 | 36.653 | 1.00 | 0.00 | AAAA |
| ATOM | 2832 | C | ARG | E | 174 | 40.418 | 135.030 | 37.421 | 1.00 | 0.00 | AAAA |
| ATOM | 2833 | O | ARG | E | 174 | 41.346 | 135.259 | 36.656 | 1.00 | 0.00 | AAAA |
| ATOM | 2834 | N | CYS | E | 175 | 40.381 | 134.049 | 38.329 | 1.00 | 0.00 | AAAA |
| ATOM | 2836 | CA | CYS | E | 175 | 41.481 | 133.136 | 38.550 | 1.00 | 0.00 | AAAA |
| ATOM | 2838 | CB | CYS | E | 175 | 42.594 | 133.834 | 39.341 | 1.00 | 0.00 | AAAA |
| ATOM | 2841 | SG | CYS | E | 175 | 42.276 | 134.179 | 41.085 | 1.00 | 0.00 | AAAA |
| ATOM | 2842 | C | CYS | E | 175 | 40.964 | 131.824 | 39.131 | 1.00 | 0.00 | AAAA |
| ATOM | 2843 | O | CYS | E | 175 | 39.952 | 131.785 | 39.817 | 1.00 | 0.00 | AAAA |
| ATOM | 2844 | N | TRP | E | 176 | 41.796 | 130.801 | 38.889 | 1.00 | 0.00 | AAAA |
| ATOM | 2846 | CA | TRP | E | 176 | 41.568 | 129.442 | 39.340 | 1.00 | 0.00 | AAAA |
| ATOM | 2848 | CB | TRP | E | 176 | 41.780 | 128.327 | 38.315 | 1.00 | 0.00 | AAAA |
| ATOM | 2851 | CG | TRP | E | 176 | 40.960 | 128.128 | 37.078 | 1.00 | 0.00 | AAAA |
| ATOM | 2852 | CD1 | TRP | E | 176 | 41.296 | 128.530 | 35.835 | 1.00 | 0.00 | AAAA |
| ATOM | 2854 | CD2 | TRP | E | 176 | 39.715 | 127.386 | 36.960 | 1.00 | 0.00 | AAAA |
| ATOM | 2855 | NE1 | TRP | E | 176 | 40.400 | 127.943 | 34.955 | 1.00 | 0.00 | AAAA |
| ATOM | 2857 | CE2 | TRP | E | 176 | 39.382 | 127.262 | 35.587 | 1.00 | 0.00 | AAAA |
| ATOM | 2858 | CE3 | TRP | E | 176 | 38.899 | 126.673 | 37.862 | 1.00 | 0.00 | AAAA |
| ATOM | 2860 | CZ2 | TRP | E | 176 | 38.293 | 126.508 | 35.128 | 1.00 | 0.00 | AAAA |
| ATOM | 2862 | CZ3 | TRP | E | 176 | 37.877 | 125.832 | 37.417 | 1.00 | 0.00 | AAAA |
| ATOM | 2864 | CH2 | TRP | E | 176 | 37.540 | 125.788 | 36.061 | 1.00 | 0.00 | AAAA |
| ATOM | 2866 | C | TRP | E | 176 | 42.350 | 129.022 | 40.574 | 1.00 | 0.00 | AAAA |
| ATOM | 2867 | O | TRP | E | 176 | 42.107 | 127.945 | 41.109 | 1.00 | 0.00 | AAAA |
| ATOM | 2868 | N | THR | E | 177 | 43.370 | 129.783 | 40.974 | 1.00 | 0.00 | AAAA |
| ATOM | 2870 | CA | THR | E | 177 | 44.344 | 129.547 | 42.025 | 1.00 | 0.00 | AAAA |
| ATOM | 2872 | CB | THR | E | 177 | 45.192 | 128.395 | 41.487 | 1.00 | 0.00 | AAAA |
| ATOM | 2874 | OG1 | THR | E | 177 | 46.400 | 128.260 | 42.207 | 1.00 | 0.00 | AAAA |
| ATOM | 2876 | CG2 | THR | E | 177 | 45.613 | 128.480 | 40.017 | 1.00 | 0.00 | AAAA |
| ATOM | 2880 | C | THR | E | 177 | 45.108 | 130.848 | 42.188 | 1.00 | 0.00 | AAAA |
| ATOM | 2881 | O | THR | E | 177 | 45.101 | 131.751 | 41.351 | 1.00 | 0.00 | AAAA |
| ATOM | 2882 | N | THR | E | 178 | 45.886 | 130.922 | 43.271 | 1.00 | 0.00 | AAAA |
| ATOM | 2884 | CA | THR | E | 178 | 46.973 | 131.869 | 43.440 | 1.00 | 0.00 | AAAA |
| ATOM | 2886 | CB | THR | E | 178 | 47.379 | 131.604 | 44.896 | 1.00 | 0.00 | AAAA |
| ATOM | 2888 | OG1 | THR | E | 178 | 47.923 | 132.853 | 45.288 | 1.00 | 0.00 | AAAA |
| ATOM | 2890 | CG2 | THR | E | 178 | 48.246 | 130.381 | 45.218 | 1.00 | 0.00 | AAAA |
| ATOM | 2894 | C | THR | E | 178 | 48.063 | 131.724 | 42.395 | 1.00 | 0.00 | AAAA |
| ATOM | 2895 | O | THR | E | 178 | 48.903 | 132.627 | 42.330 | 1.00 | 0.00 | AAAA |
| ATOM | 2896 | N | ASN | E | 179 | 48.080 | 130.696 | 41.548 | 1.00 | 0.00 | AAAA |
| ATOM | 2898 | CA | ASN | E | 179 | 49.174 | 130.337 | 40.661 | 1.00 | 0.00 | AAAA |
| ATOM | 2900 | CB | ASN | E | 179 | 49.230 | 128.816 | 40.541 | 1.00 | 0.00 | AAAA |
| ATOM | 2903 | CG | ASN | E | 179 | 50.562 | 128.178 | 40.155 | 1.00 | 0.00 | AAAA |
| ATOM | 2904 | OD1 | ASN | E | 179 | 50.887 | 128.259 | 38.971 | 1.00 | 0.00 | AAAA |
| ATOM | 2905 | ND2 | ASN | E | 179 | 51.440 | 127.692 | 41.023 | 1.00 | 0.00 | AAAA |
| ATOM | 2908 | C | ASN | E | 179 | 49.007 | 130.890 | 39.254 | 1.00 | 0.00 | AAAA |
| ATOM | 2909 | O | ASN | E | 179 | 49.913 | 131.531 | 38.729 | 1.00 | 0.00 | AAAA |
| ATOM | 2910 | N | ARG | E | 180 | 47.800 | 130.752 | 38.707 | 1.00 | 0.00 | AAAA |
| ATOM | 2912 | CA | ARG | E | 180 | 47.366 | 130.999 | 37.343 | 1.00 | 0.00 | AAAA |
| ATOM | 2914 | CB | ARG | E | 180 | 47.455 | 129.702 | 36.545 | 1.00 | 0.00 | AAAA |
| ATOM | 2917 | CG | ARG | E | 180 | 48.876 | 129.205 | 36.319 | 1.00 | 0.00 | AAAA |
| ATOM | 2920 | CD | ARG | E | 180 | 49.140 | 128.105 | 35.292 | 1.00 | 0.00 | AAAA |
| ATOM | 2923 | NE | ARG | E | 180 | 50.532 | 127.658 | 35.260 | 1.00 | 0.00 | AAAA |
| ATOM | 2925 | CZ | ARG | E | 180 | 51.474 | 128.207 | 34.488 | 1.00 | 0.00 | AAAA |
| ATOM | 2926 | NH1 | ARG | E | 180 | 51.168 | 129.151 | 33.580 | 1.00 | 0.00 | AAAA |
| ATOM | 2929 | NH2 | ARG | E | 180 | 52.762 | 127.939 | 34.763 | 1.00 | 0.00 | AAAA |
| ATOM | 2932 | C | ARG | E | 180 | 45.966 | 131.618 | 37.287 | 1.00 | 0.00 | AAAA |
| ATOM | 2933 | O | ARG | | 180 | 45.060 | 131.407 | 38.077 | 1.00 | 0.00 | AAAA |
| ATOM | 2934 | N | CYS | E | 181 | 45.717 | 132.307 | 36.166 | 1.00 | 0.00 | AAAA |
| ATOM | 2936 | CA | CYS | E | 181 | 44.433 | 132.936 | 35.923 | 1.00 | 0.00 | AAAA |
| ATOM | 2938 | CB | CYS | E | 181 | 44.660 | 134.366 | 35.430 | 1.00 | 0.00 | AAAA |
| ATOM | 2941 | SG | CYS | E | 181 | 45.842 | 135.489 | 36.220 | 1.00 | 0.00 | AAAA |
| ATOM | 2942 | C | CYS | E | 181 | 43.646 | 132.170 | 34.872 | 1.00 | 0.00 | AAAA |
| ATOM | 2943 | O | CYS | E | 181 | 44.128 | 131.102 | 34.495 | 1.00 | 0.00 | AAAA |
| ATOM | 2944 | N | GLN | E | 182 | 42.408 | 132.584 | 34.635 | 1.00 | 0.00 | AAAA |
| ATOM | 2946 | CA | GLN | E | 182 | 41.424 | 131.752 | 33.973 | 1.00 | 0.00 | AAAA |
| ATOM | 2948 | CB | GLN | E | 182 | 40.051 | 131.989 | 34.607 | 1.00 | 0.00 | AAAA |
| ATOM | 2951 | CG | GLN | E | 182 | 38.977 | 131.034 | 34.061 | 1.00 | 0.00 | AAAA |
| ATOM | 2954 | CD | GLN | E | 182 | 37.523 | 131.313 | 34.409 | 1.00 | 0.00 | AAAA |
| ATOM | 2955 | OE1 | GLN | E | 182 | 37.260 | 132.194 | 35.221 | 1.00 | 0.00 | AAAA |
| ATOM | 2956 | NE2 | GLN | E | 182 | 36.661 | 130.539 | 33.754 | 1.00 | 0.00 | AAAA |
| ATOM | 2959 | C | GLN | E | 182 | 41.407 | 132.220 | 32.527 | 1.00 | 0.00 | AAAA |
| ATOM | 2960 | O | GLN | E | 182 | 41.360 | 133.414 | 32.153 | 1.00 | 0.00 | AAAA |
| ATOM | 2961 | OXT | GLN | E | 182 | 41.488 | 131.357 | 31.629 | 1.00 | 0.00 | AAAA |
| ATOM | 2962 | N | GLY | S | 1 | 17.998 | 117.086 | 49.079 | 1.00 | 0.00 | AAAB |
| ATOM | 2965 | CA | GLY | S | 1 | 16.850 | 116.723 | 48.282 | 1.00 | 0.00 | AAAB |
| ATOM | 2968 | C | GLY | S | 1 | 16.891 | 115.235 | 47.957 | 1.00 | 0.00 | AAAB |
| ATOM | 2969 | O | GLY | S | 1 | 17.879 | 114.603 | 48.316 | 1.00 | 0.00 | AAAB |
| ATOM | 2970 | N | SER | S | 2 | 15.987 | 114.820 | 47.062 | 1.00 | 0.00 | AAAB |
| ATOM | 2972 | CA | SER | S | 2 | 15.984 | 113.459 | 46.568 | 1.00 | 0.00 | AAAB |
| ATOM | 2974 | CB | SER | S | 2 | 14.737 | 113.237 | 45.709 | 1.00 | 0.00 | AAAB |
| ATOM | 2977 | OG | SER | S | 2 | 14.726 | 114.054 | 44.561 | 1.00 | 0.00 | AAAB |
| ATOM | 2979 | C | SER | S | 2 | 17.249 | 113.022 | 45.849 | 1.00 | 0.00 | AAAB |
| ATOM | 2980 | O | SER | S | 2 | 17.879 | 112.013 | 46.158 | 1.00 | 0.00 | AAAB |
| ATOM | 2981 | N | LEU | S | 3 | 17.644 | 113.907 | 44.933 | 1.00 | 0.00 | AAAB |
| ATOM | 2983 | CA | LEU | S | 3 | 18.738 | 113.830 | 43.983 | 1.00 | 0.00 | AAAB |
| ATOM | 2985 | CB | LEU | S | 3 | 18.719 | 114.998 | 42.990 | 1.00 | 0.00 | AAAB |
| ATOM | 2988 | CG | LEU | S | 3 | 19.714 | 114.822 | 41.847 | 1.00 | 0.00 | AAAB |
| ATOM | 2990 | CD1 | LEU | S | 3 | 19.234 | 115.335 | 40.485 | 1.00 | 0.00 | AAAB |
| ATOM | 2994 | CD2 | LEU | S | 3 | 21.026 | 115.566 | 42.118 | 1.00 | 0.00 | AAAB |
| ATOM | 2998 | C | LEU | S | 3 | 20.005 | 113.849 | 44.818 | 1.00 | 0.00 | AAAB |
| ATOM | 2999 | O | LEU | S | 3 | 20.933 | 113.099 | 44.529 | 1.00 | 0.00 | AAAB |
| ATOM | 3000 | N | ASP | S | 4 | 20.101 | 114.704 | 45.847 | 1.00 | 0.00 | AAAB |
| ATOM | 3002 | CA | ASP | S | 4 | 21.288 | 114.841 | 46.658 | 1.00 | 0.00 | AAAB |
| ATOM | 3004 | CB | ASP | S | 4 | 21.316 | 116.024 | 47.624 | 1.00 | 0.00 | AAAB |
| ATOM | 3007 | CG | ASP | S | 4 | 20.981 | 117.409 | 47.059 | 1.00 | 0.00 | AAAB |
| ATOM | 3008 | OD1 | ASP | S | 4 | 19.807 | 117.825 | 46.997 | 1.00 | 0.00 | AAAB |
| ATOM | 3009 | OD2 | ASP | S | 4 | 21.947 | 118.132 | 46.726 | 1.00 | 0.00 | AAAB |
| ATOM | 3010 | C | ASP | S | 4 | 21.447 | 113.591 | 47.520 | 1.00 | 0.00 | AAAB |
| ATOM | 3011 | O | ASP | S | 4 | 22.551 | 113.051 | 47.542 | 1.00 | 0.00 | AAAB |
| ATOM | 3012 | N | GLU | S | 5 | 20.353 | 113.109 | 48.115 | 1.00 | 0.00 | AAAB |
| ATOM | 3014 | CA | GLU | S | 5 | 20.224 | 111.887 | 48.891 | 1.00 | 0.00 | AAAB |
| ATOM | 3016 | CB | GLU | S | 5 | 18.829 | 111.725 | 49.496 | 1.00 | 0.00 | AAAB |
| ATOM | 3019 | CG | GLU | S | 5 | 18.594 | 112.509 | 50.789 | 1.00 | 0.00 | AAAB |
| ATOM | 3022 | CD | GLU | S | 5 | 19.304 | 112.025 | 52.050 | 1.00 | 0.00 | AAAB |
| ATOM | 3023 | OE1 | GLU | S | 5 | 19.797 | 110.882 | 52.021 | 1.00 | 0.00 | AAAB |
| ATOM | 3024 | OE2 | GLU | S | 5 | 19.375 | 112.792 | 53.031 | 1.00 | 0.00 | AAAB |
| ATOM | 3025 | C | GLU | S | 5 | 20.654 | 110.699 | 48.052 | 1.00 | 0.00 | AAAB |
| ATOM | 3026 | O | GLU | S | 5 | 21.404 | 109.857 | 48.551 | 1.00 | 0.00 | AAAB |
| ATOM | 3027 | N | SER | S | 6 | 20.196 | 110.615 | 46.798 | 1.00 | 0.00 | AAAB |
| ATOM | 3029 | CA | SER | S | 6 | 20.667 | 109.554 | 45.919 | 1.00 | 0.00 | AAAB |
| ATOM | 3031 | CB | SER | S | 6 | 19.785 | 109.582 | 44.664 | 1.00 | 0.00 | AAAB |
| ATOM | 3034 | OG | SER | S | 6 | 20.231 | 108.654 | 43.700 | 1.00 | 0.00 | AAAB |
| ATOM | 3036 | C | SER | S | 6 | 22.159 | 109.582 | 45.651 | 1.00 | 0.00 | AAAB |
| ATOM | 3037 | O | SER | S | 6 | 22.893 | 108.596 | 45.703 | 1.00 | 0.00 | AAAB |
| ATOM | 3038 | N | PHE | S | 7 | 22.650 | 110.774 | 45.287 | 1.00 | 0.00 | AAAB |
| ATOM | 3040 | CA | PHE | S | 7 | 24.036 | 111.153 | 45.123 | 1.00 | 0.00 | AAAB |
| ATOM | 3042 | CB | PHE | S | 7 | 24.209 | 112.569 | 44.582 | 1.00 | 0.00 | AAAB |
| ATOM | 3045 | CG | PHE | S | 7 | 25.603 | 113.148 | 44.436 | 1.00 | 0.00 | AAAB |
| ATOM | 3046 | CD1 | PHE | S | 7 | 26.218 | 113.292 | 43.189 | 1.00 | 0.00 | AAAB |
| ATOM | 3048 | CD2 | PHE | S | 7 | 26.110 | 113.922 | 45.492 | 1.00 | 0.00 | AAAB |
| ATOM | 3050 | CE1 | PHE | S | 7 | 27.379 | 114.055 | 43.022 | 1.00 | 0.00 | AAAB |
| ATOM | 3052 | CE2 | PHE | S | 7 | 27.290 | 114.653 | 45.326 | 1.00 | 0.00 | AAAB |
| ATOM | 3054 | CZ | PHE | S | 7 | 27.941 | 114.763 | 44.093 | 1.00 | 0.00 | AAAB |
| ATOM | 3056 | C | PHE | S | 7 | 24.961 | 110.790 | 46.280 | 1.00 | 0.00 | AAAB |
| ATOM | 3057 | O | PHE | S | 7 | 26.010 | 110.168 | 46.065 | 1.00 | 0.00 | AAAB |
| ATOM | 3058 | N | TYR | S | 8 | 24.562 | 111.148 | 47.499 | 1.00 | 0.00 | AAAB |
| ATOM | 3060 | CA | TYR | S | 8 | 25.276 | 110.675 | 48.665 | 1.00 | 0.00 | AAAB |
| ATOM | 3062 | CB | TYR | S | 8 | 24.796 | 111.433 | 49.905 | 1.00 | 0.00 | AAAB |
| ATOM | 3065 | CG | TYR | S | 8 | 24.873 | 112.934 | 49.753 | 1.00 | 0.00 | AAAB |
| ATOM | 3066 | CD1 | TYR | S | 8 | 23.816 | 113.715 | 50.220 | 1.00 | 0.00 | AAAB |
| ATOM | 3068 | CD2 | TYR | S | 8 | 25.985 | 113.515 | 49.129 | 1.00 | 0.00 | AAAB |
| ATOM | 3070 | CE1 | TYR | S | 8 | 23.870 | 115.089 | 49.939 | 1.00 | 0.00 | AAAB |
| ATOM | 3072 | CE2 | TYR | S | 8 | 25.991 | 114.884 | 48.829 | 1.00 | 0.00 | AAAB |
| ATOM | 3074 | CZ | TYR | S | 8 | 24.937 | 115.696 | 49.259 | 1.00 | 0.00 | AAAB |
| ATOM | 3075 | OH | TYR | S | 8 | 24.950 | 117.052 | 49.109 | 1.00 | 0.00 | AAAB |
| ATOM | 3077 | C | TYR | S | 8 | 25.264 | 109.196 | 49.028 | 1.00 | 0.00 | AAAB |
| ATOM | 3078 | O | TYR | S | 8 | 26.227 | 108.684 | 49.604 | 1.00 | 0.00 | AAAB |
| ATOM | 3079 | N | ASP | S | 9 | 24.193 | 108.510 | 48.662 | 1.00 | 0.00 | AAAB |
| ATOM | 3081 | CA | ASP | S | 9 | 23.976 | 107.083 | 48.799 | 1.00 | 0.00 | AAAB |
| ATOM | 3083 | CB | ASP | S | 9 | 22.555 | 106.642 | 48.414 | 1.00 | 0.00 | AAAB |
| ATOM | 3086 | CG | ASP | S | 9 | 22.358 | 105.142 | 48.582 | 1.00 | 0.00 | AAAB |
| ATOM | 3087 | OD1 | ASP | S | 9 | 22.141 | 104.639 | 49.705 | 1.00 | 0.00 | AAAB |
| ATOM | 3088 | OD2 | ASP | S | 9 | 22.118 | 104.503 | 47.529 | 1.00 | 0.00 | AAAB |
| ATOM | 3089 | C | ASP | S | 9 | 25.063 | 106.321 | 48.039 | 1.00 | 0.00 | AAAB |
| ATOM | 3090 | O | ASP | S | 9 | 25.670 | 105.472 | 48.697 | 1.00 | 0.00 | AAAB |
| ATOM | 3091 | N | TRP | S | 10 | 25.376 | 106.655 | 46.785 | 1.00 | 0.00 | AAAB |
| ATOM | 3093 | CA | TRP | S | 10 | 26.385 | 106.003 | 45.978 | 1.00 | 0.00 | AAAB |
| ATOM | 3095 | CB | TRP | S | 10 | 26.361 | 106.642 | 44.589 | 1.00 | 0.00 | AAAB |
| ATOM | 3098 | CG | TRP | S | 10 | 27.236 | 105.924 | 43.613 | 1.00 | 0.00 | AAAB |
| ATOM | 3099 | CD1 | TRP | S | 10 | 28.301 | 106.450 | 42.960 | 1.00 | 0.00 | AAAB |
| ATOM | 3101 | CD2 | TRP | S | 10 | 26.995 | 104.576 | 43.116 | 1.00 | 0.00 | AAAB |
| ATOM | 3102 | NE1 | TRP | S | 10 | 28.757 | 105.440 | 42.128 | 1.00 | 0.00 | AAAB |
| ATOM | 3104 | CE2 | TRP | S | 10 | 28.104 | 104.235 | 42.299 | 1.00 | 0.00 | AAAB |
| ATOM | 3105 | CE3 | TRP | S | 10 | 26.071 | 103.568 | 43.432 | 1.00 | 0.00 | AAAB |
| ATOM | 3107 | CZ2 | TRP | S | 10 | 28.263 | 102.949 | 41.777 | 1.00 | 0.00 | AAAB |
| ATOM | 3109 | CZ3 | TRP | S | 10 | 26.255 | 102.310 | 42.841 | 1.00 | 0.00 | AAAB |
| ATOM | 3111 | CH2 | TRP | S | 10 | 27.296 | 101.956 | 41.979 | 1.00 | 0.00 | AAAB |
| ATOM | 3113 | C | TRP | S | 10 | 27.792 | 106.182 | 46.524 | 1.00 | 0.00 | AAAB |
| ATOM | 3114 | O | TRP | S | 10 | 28.595 | 105.250 | 46.500 | 1.00 | 0.00 | AAAB |
| ATOM | 3115 | N | PHE | S | 11 | 28.064 | 107.366 | 47.075 | 1.00 | 0.00 | AAAB |
| ATOM | 3117 | CA | PHE | S | 11 | 29.356 | 107.606 | 47.674 | 1.00 | 0.00 | AAAB |
| ATOM | 3119 | CB | PHE | S | 11 | 29.666 | 109.103 | 47.643 | 1.00 | 0.00 | AAAB |
| ATOM | 3122 | CG | PHE | S | 11 | 30.048 | 109.723 | 46.311 | 1.00 | 0.00 | AAAB |
| ATOM | 3123 | CD1 | PHE | S | 11 | 31.183 | 109.217 | 45.665 | 1.00 | 0.00 | AAAB |
| ATOM | 3125 | CD2 | PHE | S | 11 | 29.305 | 110.745 | 45.721 | 1.00 | 0.00 | AAAB |
| ATOM | 3127 | CE1 | PHE | S | 11 | 31.524 | 109.669 | 44.387 | 1.00 | 0.00 | AAAB |
| ATOM | 3129 | CE2 | PHE | S | 11 | 29.690 | 111.303 | 44.503 | 1.00 | 0.00 | AAAB |
| ATOM | 3131 | CZ | PHE | S | 11 | 30.834 | 110.786 | 43.876 | 1.00 | 0.00 | AAAB |
| ATOM | 3133 | C | PHE | S | 11 | 29.595 | 107.026 | 49.059 | 1.00 | 0.00 | AAAB |
| ATOM | 3134 | O | PHE | S | 11 | 30.719 | 106.642 | 49.363 | 1.00 | 0.00 | AAAB |
| ATOM | 3135 | N | GLU | S | 12 | 28.537 | 106.689 | 49.803 | 1.00 | 0.00 | AAAB |
| ATOM | 3137 | CA | GLU | S | 12 | 28.574 | 105.790 | 50.937 | 1.00 | 0.00 | AAAB |
| ATOM | 3139 | CB | GLU | S | 12 | 27.259 | 105.930 | 51.687 | 1.00 | 0.00 | AAAB |
| ATOM | 3142 | CG | GLU | S | 12 | 27.340 | 106.672 | 53.026 | 1.00 | 0.00 | AAAB |
| ATOM | 3145 | CD | GLU | S | 12 | 28.111 | 105.983 | 54.146 | 1.00 | 0.00 | AAAB |
| ATOM | 3146 | OE1 | GLU | S | 12 | 28.826 | 106.740 | 54.836 | 1.00 | 0.00 | AAAB |
| ATOM | 3147 | OE2 | GLU | S | 12 | 27.867 | 104.778 | 54.374 | 1.00 | 0.00 | AAAB |
| ATOM | 3148 | C | GLU | S | 12 | 28.675 | 104.296 | 50.629 | 1.00 | 0.00 | AAAB |
| ATOM | 3149 | O | GLU | S | 12 | 29.448 | 103.671 | 51.361 | 1.00 | 0.00 | AAAB |
| ATOM | 3150 | N | ARG | S | 13 | 28.024 | 103.807 | 49.581 | 1.00 | 0.00 | AAAB |
| ATOM | 3152 | CA | ARG | S | 13 | 27.935 | 102.444 | 49.090 | 1.00 | 0.00 | AAAB |
| ATOM | 3154 | CB | ARG | S | 13 | 26.627 | 102.439 | 48.300 | 1.00 | 0.00 | AAAB |
| ATOM | 3157 | CG | ARG | S | 13 | 25.453 | 102.062 | 49.209 | 1.00 | 0.00 | AAAB |
| ATOM | 3160 | CD | ARG | S | 13 | 24.136 | 102.247 | 48.461 | 1.00 | 0.00 | AAAB |
| ATOM | 3163 | NE | ARG | S | 13 | 23.804 | 101.093 | 47.628 | 1.00 | 0.00 | AAAB |
| ATOM | 3165 | CZ | ARG | S | 13 | 23.066 | 101.146 | 46.504 | 1.00 | 0.00 | AAAB |
| ATOM | 3166 | NH1 | ARG | S | 13 | 22.364 | 102.222 | 46.132 | 1.00 | 0.00 | AAAB |
| ATOM | 3169 | NH2 | ARG | S | 13 | 22.900 | 99.983 | 45.854 | 1.00 | 0.00 | AAAB |
| ATOM | 3172 | C | ARG | S | 13 | 29.137 | 102.055 | 48.254 | 1.00 | 0.00 | AAAB |
| ATOM | 3173 | O | ARG | S | 13 | 29.361 | 100.880 | 47.975 | 1.00 | 0.00 | AAAB |
| ATOM | 3174 | N | GLN | S | 14 | 30.070 | 102.962 | 47.962 | 1.00 | 0.00 | AAAB |
| ATOM | 3176 | CA | GLN | S | 14 | 31.373 | 102.811 | 47.337 | 1.00 | 0.00 | AAAB |
| ATOM | 3178 | CB | GLN | S | 14 | 32.004 | 104.160 | 46.987 | 1.00 | 0.00 | AAAB |
| ATOM | 3181 | CG | GLN | S | 14 | 32.823 | 104.254 | 45.699 | 1.00 | 0.00 | AAAB |
| ATOM | 3184 | CD | GLN | S | 14 | 32.027 | 104.275 | 44.405 | 1.00 | 0.00 | AAAB |
| ATOM | 3185 | OE1 | GLN | S | 14 | 32.550 | 104.306 | 43.296 | 1.Q0 | 0.00 | AAAB |
| ATOM | 3186 | NE2 | GLN | S | 14 | 30.692 | 104.243 | 44.441 | 1.00 | 0.00 | AAAB |
| ATOM | 3189 | C | GLN | S | 14 | 32.373 | 102.062 | 48.216 | 1.00 | 0.00 | AAAB |
| ATOM | 3190 | O | GLN | S | 14 | 33.487 | 101.757 | 47.810 | 1.00 | 0.00 | AAAB |
| ATOM | 3191 | N | LEU | S | 15 | 32.007 | 101.783 | 49.468 | 1.00 | 0.00 | AAAB |
| ATOM | 3193 | CA | LEU | S | 15 | 32.843 | 101.099 | 50.439 | 1.00 | 0.00 | AAAB |
| ATOM | 3195 | CB | LEU | S | 15 | 33.383 | 102.021 | 51.521 | 1.00 | 0.00 | AAAB |
| ATOM | 3198 | CG | LEU | S | 15 | 34.217 | 103.220 | 51.062 | 1.00 | 0.00 | AAAB |
| ATOM | 3200 | CD1 | LEU | S | 15 | 33.367 | 104.456 | 51.328 | 1.00 | 0.00 | AAAB |
| ATOM | 3204 | CD2 | LEU | S | 15 | 35.525 | 103.226 | 51.852 | 1.00 | 0.00 | AAAB |
| ATOM | 3208 | C | LEU | S | 15 | 32.029 | 100.006 | 51.134 | 1.00 | 0.00 | AAAB |
| ATOM | 3209 | O | LEU | S | 15 | 30.809 | 100.006 | 50.968 | 1.00 | 0.00 | AAAB |
| ATOM | 3210 | N | GLY | S | 16 | 32.756 | 99.105 | 51.803 | 1.00 | 0.00 | AAAB |
| ATOM | 3212 | CA | GLY | S | 16 | 32.298 | 97.946 | 52.535 | 1.00 | 0.00 | AAAB |
| ATOM | 3215 | C | GLY | S | 16 | 32.538 | 98.149 | 54.022 | 1.00 | 0.00 | AAAB |
| ATOM | 3216 | OT | GLY | S | 16 | 32.465 | 99.225 | 54.601 | 1.00 | 0.00 | AAAB |
| ATOM | 3217 | O | GLY | S | 16 | 32.707 | 96.960 | 54.683 | 1.00 | 0.00 | AAAB |
| END | | | | | | | | | | | |

## Claims

1. A computer-assisted method of identifying, designing or screening for a compound that can potentially interact with insulin receptor (IR) and/or insulin-like growth factor-1 receptor (IGF-1R), comprising performing structure-based identification, design or screening of a compound based on the compound's interactions with a structure defined by the atomic coordinates of one or more of Appendixes I to VI, or a subset of atomic coordinates of one or more thereof at least representing the C-terminal region of the α-chain of IR, the C-terminal region of the α-chain of IGF-1R, or a mimetic of the C-terminal region of the α-chain of IR and/or IGF-1R,
wherein the atomic coordinates define one or more regions of the low affinity binding site of IR for insulin, and/or the low affinity binding site of IGF-1R for IGF, comprising the C-terminal region of the α-chain of IR, the C-terminal region of the α-chain of IGF-1R, or a mimetic of the C-terminal region of the α-chain of IR and/or IGF-1R,
wherein the C-terminal region of the α-chain of IR comprises amino acids 693 to 710 of IR α-chain (SEQ ID NO: 13),
wherein the C-terminal region of the α-chain of IGF-1R comprises amino acids 681 to 697 of IGF-1R α-chain (SEQ ID NO: 15), and
wherein the mimetic of the C-terminal region of the α-chain of IR and/or IGF-1R is S519C16 (SEQ ID NO: 18).

2. The method according to claim 1, comprising identifying, designing or screening for a compound which interacts with the three-dimensional structure of;
(i) the low affinity insulin binding site of IR, the structure being defined by the atomic coordinates shown in one or more of Appendixes I, III and V; and/or
(ii) the low affinity insulin-like growth factor (IGF) binding site of IGF-1 R, the structure being defined by the atomic coordinates shown in one or more of Appendixes II, IV and VI,
wherein interaction of the compound with the structure is favoured energetically,
and/or further comprising synthesising or obtaining an identified or designed candidate compound and determining the ability of the candidate compound to interact with IR and/or IGF-1R.

3. The method according to claim 1, wherein the atomic coordinates defining the low affinity insulin binding site of IR further comprise the leucine-rich repeat 1 (L1) domain and/or the cysteine-rich (CR) domain of the IR ectodomain, optionally wherein the atomic coordinates define portions of the molecular surface of the central β-sheet of the L1 domain and portions of the molecular surface of the second leucine-rich repeat (LRR) which contain Phe39 and/or the loop in the fourth LRR rung of the L1 domain, and/or wherein the atomic coordinates define module 6 of the CR domain of IR.

4. The method according to any preceding claim, wherein the atomic coordinates further define one or more amino acid sequences selected from IR amino acid residues 1-156, 157-310, 594 and 794, optionally wherein the one or more amino acids selected from IR amino acid residues 1-156 comprise at least one amino acid selected from Arg14, Asn15, Gln34, Leu36, Leu37, Phe39, Pro43, Phe46, Leu62, Phe64, Leu87, Phe88, Phe89, Asn90, Phe96, Glu97, Arg118, G1u120 and His144, and/or wherein the one or more amino acids selected from IR amino acid residues 157-310 comprise at least one of the amino acid sequences selected from 192-310, 227-303 and 259-284.

5. The method according to claim 1, wherein the atomic coordinates defining the low affinity IGF binding site of IGF-1R further comprise the L1 domain and/or the CR domain of IGF-1R ectodomain, optionally wherein the atomic coordinates define the central β-sheet of the L1 domain, and/or that part of the second LRR containing Ser35, and/or the loop in the fourth LRR rung of the L1 domain, and/or wherein the atomic coordinates define module 6 of the CR domain of IGF-1R.

6. The method of claim 2, wherein the compound substitutes for the C-terminal region of the α-chain of IR and/or the C-terminal region of the α-chain of IGF-1R in the formation of the low affinity binding site of IR or IGF-1R.

7. The method according to any preceding claim, wherein a candidate compound for interacting with IR and/or IGF-1R is chemically modified as a result of structure-based evaluation, optionally wherein the chemical modification is designed to either:
(i) reduce the potential for the candidate compound to bind to IR whilst maintaining binding to IGF-1R; or
(ii) reduce the potential for the candidate compound to bind to IGF-1R, whilst maintaining binding to IR.

8. A computer-assisted method of redesigning a compound which is known to bind to IR and/or IGF-1R comprising performing structure-based evaluation of the compound based on the compound's interactions with a structure defined by the atomic coordinates of one or more of Appendixes I to VI, or a subset of atomic coordinates of one or more thereof at least representing the C-terminal region of the α-chain of IR, the C-terminal region of the α-chain of IGF-1R, or a mimetic of the C-terminal region of the α-chain of IR and/or IGF-1R, and redesigning or chemically modifying the compound as a result of the evaluation,
wherein the atomic coordinates define one or more regions of the low affinity binding site of IR for insulin, and/or the low affinity binding site of IGF-1R for IGF, comprising the C-terminal region of the α-chain of IR, the C-terminal region of the α-chain of IGF-1R, or a mimetic of the C-terminal region of the α-chain of IR and/or IGF-1R,
wherein the C-terminal region of the α-chain of IR comprises amino acids 693 to 710 of IR α-chain (SEQ ID NO: 13),
wherein the C-terminal region of the α-chain of IGF-1R comprises amino acids 681 to 697 of IGF-1R α-chain (SEQ ID NO: 15), and
wherein the mimetic of the C-terminal region of the α-chain of IR and/or IGF-1R is S519C16 (SEQ ID NO: 18).

9. The method according to claim 8, wherein the compound is redesigned or chemically modified to (i) improve affinity for binding to IR, and/or (ii) lower affinity for binding to IGF-1R, or wherein the compound is redesigned or chemically modified to (i) improve affinity for binding to IGF-1R, and/or (ii) lower affinity for binding to IR,
optionally wherein the compound is redesigned or modified so as to lower the affinity to IR or IGF-1R by virtue of the structural differences between IR and IGF-1R at or in the vicinity of the C-terminal region of the α-chain of IR and the C-terminal region of the α-chain of IGF-1R.

10. A computer-assisted method of identifying a compound that potentially interacts with IR and/or IGF-1R, which method comprises fitting the structure of:
(i) the low affinity insulin binding site of IR, the structure being defined by the atomic coordinates shown in one or more of Appendixes I, III and V;
(ii) the low affinity IGF binding site of IGF-1R, the structure being defined by the atomic coordinates shown in one or more of Appendixes II, IV and VI; and/or
(iii) the C-terminal region of the α-chain of IR, the C-terminal region of the α-chain of IGF-1R, or a mimetic of the C-terminal region of the α-chain of IR and/or IGF-1R, the structure being defined by a subset of atomic coordinates shown in one or more of Appendixes I to VI,
to the structure of a candidate compound,
wherein the atomic coordinates define one or more regions of the low affinity binding site of IR for insulin, and/or the low affinity binding site of IGF-1R for IGF, comprising the C-terminal region of the α-chain of IR, the C-terminal region of the α-chain of IGF-1R, or a mimetic of the C-terminal region of the α-chain of IR and/or IGF-1R,
wherein the C-terminal region of the α-chain of IR comprises amino acids 693 to 710 of IR α-chain (SEQ ID NO: 13),
wherein the C-terminal region of the α-chain of IGF-1R comprises amino acids 681 to 697 of IGF-1R α-chain (SEQ ID NO: 15), and
wherein the mimetic of the C-terminal region of the α-chain of IR and/or IGF-1R is S519C16 (SEQ ID NO: 18).

11. A computer-assisted method for identifying a compound able to interact with IR and/or IGF-1R using a programmed computer comprising a processor, which method comprises the steps of:
(a) generating, using computer methods, a set of atomic coordinates of a structure that possesses energetically favourable interactions with the atomic coordinates of:
(i) the low affinity insulin binding site of IR, the structure being defined by the atomic coordinates shown in one or more of Appendixes I, III and V;
(ii) the low affinity IGF binding site of IGF-1R, the structure being defined by the atomic coordinates shown in one or more of Appendixes II, IV and VI; and/or
(iii) the C-terminal region of the α-chain of IR, the C-terminal region of the α-chain of IGF-1R, or a mimetic of the C-terminal region of the α-chain of IR and/or IGF-1R, the structure being defined by a subset of atomic coordinates shown in one or more of Appendixes I to VI,
which coordinates are entered into the computer thereby generating a criteria data set;
(b) comparing, using the processor, the criteria data set to a computer database of chemical structures;
(c) selecting from the database, using computer methods, chemical structures which are complementary or similar to a region of the criteria data set; and optionally,
(d) outputting, to an output device, the selected chemical structures which are complementary to or similar to a region of the criteria data set,
wherein the atomic coordinates define one or more regions of the low affinity binding site of IR for insulin, and/or the low affinity binding site of IGF-1R for IGF, comprising the C-terminal region of the α-chain of IR, the C-terminal region of the α-chain of IGF-1R, or a mimetic of the C-terminal region of the α-chain of IR and/or IGF-1R,
wherein the C-terminal region of the α-chain of IR comprises amino acids 693 to 710 of IR α-chain (SEQ ID NO: 13),
wherein the C-terminal region of the α-chain of IGF-1R comprises amino acids 681 to 697 of IGF-1R α-chain (SEQ ID NO: 15), and
wherein the mimetic of the C-terminal region of the α-chain of IR and/or IGF-1R is S519C16 (SEQ ID NO: 18).

12. A computer-assisted method for identifying potential mimetics of IR and/or IGF-1R using a programmed computer comprising a processor, the method comprising the steps of:
(a) generating a criteria data set from a set of atomic coordinates of:
(i) the low affinity insulin binding site of IR, the structure being defined by the atomic coordinates shown in one or more of Appendixes I, III and V;
(ii) the low affinity IGF binding site of IGF-1R, the structure being defined by the atomic coordinates shown in one or more of Appendixes II, IV and VI; and/or
(iii) the C-terminal region of the α-chain of IR, the C-terminal region of the α-chain of IGF-1R, or a mimetic of the C-terminal region of the α-chain of IR and/or IGF-1R, the structure being defined by a subset of atomic coordinates shown in one or more of Appendixes I to VI,
which coordinates are entered into the computer;
(b)
(i) comparing, using the processor, the criteria data set to a computer database of chemical structures stored in a computer data storage system and selecting from the database, using computer methods, chemical structures having a region that is structurally similar to the criteria data set; or
(ii) constructing, using computer methods, a model of a chemical structure having a region that is structurally similar to the criteria data set; and, optionally,
(c) outputting to an output device:
(i) the selected chemical structures from step (b)(i) having a region similar to the criteria data set; or
(ii) the constructed model from step (b)(ii),
wherein the atomic coordinates define one or more regions of the low affinity binding site of IR for insulin, and/or the low affinity binding site of IGF-1R for IGF, comprising the C-terminal region of the α-chain of IR, the C-terminal region of the α-chain of IGF-1R, or a mimetic of the C-terminal region of the α-chain of IR and/or IGF-1R,
wherein the C-terminal region of the α-chain of IR comprises amino acids 693 to 710 of IR α-chain (SEQ ID NO: 13),
wherein the C-terminal region of the α-chain of IGF-1R comprises amino acids 681 to 697 of IGF-1R α-chain (SEQ ID NO: 15), and
wherein the mimetic of the C-terminal region of the α-chain of IR and/or IGF-1R is S519C16 (SEQ ID NO: 18).

13. A method for evaluating the ability of a compound to interact with IR and/or IGF-1R, the method comprising the steps of:
(a) employing computational means to perform a fitting operation between the compound and the binding surface of a computer model of the low affinity binding site for insulin on IR ectodomain, and/or the low affinity binding site for IGF on IGF-1R ectodomain, using atomic coordinates wherein the root mean square deviation between the atomic coordinates and atomic coordinates of one or more of Appendixes I to VI or a subset of atomic coordinates of one or more thereof at least representing the C-terminal region of the α-chain of IR, the C-terminal region of the α-chain of IGF-1R, or a mimetic of the C-terminal region of the α-chain of IR and/or IGF-1R, is not more than 1.5 Å; and
(b) analysing the results of the fitting operation to quantify the association between the compound and the binding surface model,
wherein the atomic coordinates define one or more regions of the low affinity binding site of IR for insulin, and/or the low affinity binding site of IGF-1R for IGF, comprising the C-terminal region of the α-chain of IR, the C-terminal region of the α-chain of IGF-1R, or a mimetic of the C-terminal region of the α-chain of IR and/or IGF-1R,
wherein the C-terminal region of the α-chain of IR comprises amino acids 693 to 710 of IR α-chain (SEQ ID NO: 13),
wherein the C-terminal region of the α-chain of IGF-1R comprises amino acids 681 to 697 of IGF-1R α-chain (SEQ ID NO: 15), and
wherein the mimetic of the C-terminal region of the α-chain of IR and/or IGF-1R is S519C16 (SEQ ID NO: 18).

14. A method of using molecular replacement to obtain structural information about a molecule or a molecular complex of unknown structure, comprising the steps of:
(i) generating an X-ray diffraction pattern of the crystallized molecule or molecular complex; and
(ii) applying the atomic coordinates of one or more of Appendixes I to VI, or a subset of atomic coordinates of one or more thereof at least representing the C-terminal region of the α-chain of IR, the C-terminal region of the α-chain of IGF-1R, or a mimetic of the C-terminal region of the α-chain of IR and/or IGF-1R, to the X-ray diffraction pattern to generate a three-dimensional electron density map of at least a region of the molecule or molecular complex whose structure is unknown,
wherein the atomic coordinates define one or more regions of the low affinity binding site of IR for insulin, and/or the low affinity binding site of IGF-1R for IGF, comprising the C-terminal region of the α-chain of IR, the C-terminal region of the α-chain of IGF-1R, or a mimetic of the C-terminal region of the α-chain of IR and/or IGF-1R,
wherein the C-terminal region of the α-chain of IR comprises amino acids 693 to 710 of IR α-chain (SEQ ID NO: 13),
wherein the C-terminal region of the α-chain of IGF-1R comprises amino acids 681 to 697 of IGF-1R α-chain (SEQ ID NO: 15), and
wherein the mimetic of the C-terminal region of the α-chain of IR and/or IGF-1R is S519C16 (SEQ ID NO: 18).

## Patentansprüche

1. Rechnergestütztes Verfahren zum Identifizieren, Gestalten oder Screenen einer Verbindung, die möglicherweise mit Insulinrezeptor (IR) und/oder insulinähnlichem Wachstumsfaktor-1-Rezeptor (IGF-1R) in Wechselwirkung treten kann, umfassend Durchführen von strukturbasiertem Identifizieren, Gestalten oder Screenen einer Verbindung, basierend auf den Wechselwirkungen der Verbindung mit einer Struktur, die durch die Atomkoordinaten von einem oder mehreren der Anhänge I bis VI oder durch eine Teilmenge von Atomkoordinaten von einem oder mehreren davon definiert ist, die mindestens die C-terminale Region der α-Kette von IR, die C-terminale Region der α-Kette von IGF-1 R oder ein Mimetikum der C-terminalen Region der α-Kette von IR und/oder IGF-1 R repräsentieren,
wobei die Atomkoordinaten eine oder mehrere Regionen der gering affinen Bindungsstelle von IR für Insulin und/oder der gering affinen Bindungsstelle von IGF-1 R für IGF definieren, umfassend die C-terminale Region der α-Kette von IR, die C-terminale Region der α-Kette von IGF-1 R oder ein Mimetikum der C-terminalen Region der α-Kette von IR und/oder IGF-1 R,
wobei die C-terminale Region der α-Kette von IR die Aminosäuren 693 bis 710 der IR- α-Kette (SEQ ID NO: 13) umfasst,
wobei die C-terminale Region der α-Kette von IGF-1 R die Aminosäuren 681 bis 697 der IGF-1 R- α-Kette (SEQ ID NO: 15) umfasst, und
wobei das Mimetikum der C-terminalen Region der α-Kette von IR und/oder IGF-1R S519C16 (SEQ ID NO: 18) ist.

2. Verfahren nach Anspruch 1, umfassend Identifizieren, Gestalten oder Screenen einer Verbindung, die in Wechselwirkung tritt mit der dreidimensionalen Struktur von:
(i) der gering affinen Bindungsstelle von IR für Insulin, wobei die Struktur durch die Atomkoordinaten definiert ist, die in einem oder mehreren der Anhänge I, III und V dargestellt sind; und/oder
(ii) der gering affinen Bindungsstelle von IGF-1R für den insulinähnlichen Wachstumsfaktor (IGF), wobei die Struktur durch die Atomkoordinaten definiert ist, die in einem oder mehreren der Anhänge II, IV und VI dargestellt sind,
wobei die Wechselwirkung der Verbindung mit der Struktur energetisch günstig ist,
und/oder ferner umfassend die Synthese oder die Gewinnung einer identifizierten oder gestalteten Kandidatenverbindung und Bestimmen der Fähigkeit der Kandidatenverbindung mit IR und/oder IGF-1R in Wechselwirkung zu treten.

3. Verfahren nach Anspruch 1, wobei die Atomkoordinaten, die die gering affine Bindungsstelle von IR für Insulin definieren, ferner die Leucin-reiche Wiederholung 1 enthaltende (L1) Domäne und/oder die Cystein-reiche (CR) Domäne der IR-Ektodomäne umfassen, optional wobei die Atomkoordinaten Abschnitte der molekularen Oberfläche des zentralen β-Faltblatts der L1-Domäne und Abschnitte der molekularen Oberfläche der zweiten Leucin-reichen Wiederholung (LRR, leucin-rich repeat), welche Phe39 und/oder die Schleife in der vierten LRR-Querverbindung der L1-Domäne enthalten, definieren, und/oder wobei die Atomkoordinaten Modul 6 der CR-Domäne von IR definieren.

4. Verfahren nach einem der vorhergehenden Ansprüche, wobei die Atomkoordinaten ferner eine oder mehrere Aminosäuresequenzen definieren, ausgewählt aus IR-Aminosäureresten 1-156, 157-310, 594 und 794, optional wobei die eine oder die mehreren Aminosäuren aus den IR-Aminosäureresten 1-156 ausgewählt sind, welche mindestens eine Aminosäure umfassen, ausgewählt aus Arg14, Asn15, Gln34, Leu36, Leu37, Phe39, Pro43, Phe46, Leu62, Phe64, Leu87, Phe88, Phe89, Asn90, Phe96, Glu97, Arg118, Glu120 und His144, und/oder wobei die eine oder die mehreren Aminosäuren aus IR-Aminosäureresten 157-310 ausgewählt sind, umfassend mindestens eine der Aminosäuresequenzen, ausgewählt aus 192-310, 227-303 und 259-284.

5. Verfahren nach Anspruch 1, wobei die Atomkoordinaten, die die gering affine Bindungsstelle von IGF-1 R für IGF definieren, ferner die L1-Domäne und/oder die CR-Domäne der IGF-1 R-Ektodomäne umfassen, optional wobei die Atomkoordinaten das zentrale β-Faltblatt der L1-Domäne und/oder den Teil der zweiten LRR, enthaltend Ser35, und/oder die Schleife in der vierten LRR-Querverbindung der L1-Domäne definieren, und/oder wobei die Atomkoordinaten Modul 6 der CR-Domäne von IGF-1R definieren.

6. Verfahren nach Anspruch 2, wobei die Verbindung die C-terminale Region der α-Kette von IR und/oder die C-terminale Region der α-Kette von IGF-1R bei der Bildung der gering affinen Bindungsstelle von IR oder IGF-1R ersetzt.

7. Verfahren nach einem der vorhergehenden Ansprüche, wobei eine Kandidatenverbindung für die Wechselwirkung mit IR und/oder IGF-1R infolge von strukturbasierter Evaluierung chemisch modifiziert ist, optional wobei die chemische Modifikation gestaltet ist, um entweder:
(i) das Potenzial der Kandidatenverbindung zu verringern, um sich an IR zu binden, während die Bindung an IGF-1 R aufrechterhalten bleibt; oder
(ii) das Potenzial der Kandidatenverbindung zu verringern, um sich an IGF-1 R zu binden, während die Bindung an IR aufrechterhalten bleibt.

8. Rechnergestütztes Verfahren zum Neugestalten einer Verbindung, von welcher bekannt ist, dass sie an IR und/oder IGF-1 R bindet, umfassend Durchführen von strukturbasierter Evaluierung der Verbindung, basierend auf den Wechselwirkungen der Verbindung mit einer Struktur, die durch die Atomkoordinaten von einem oder mehreren der Anhänge I bis VI oder durch eine Teilmenge von Atomkoordinaten von einem oder mehreren davon definiert ist, die mindestens die C-terminale Region der α-Kette von IR, die C-terminale Region der α-Kette von IGF-1 R oder ein Mimetikum der C-terminalen Region der α-Kette von IR und/oder IGF-1 R repräsentieren, und Neugestalten oder chemisches Modifizieren der Verbindung infolge der Evaluierung,
wobei die Atomkoordinaten eine oder mehrere Regionen der gering affinen Bindungsstelle von IR für Insulin und/oder der gering affinen Bindungsstelle von IGF-1 R für IGF definieren, umfassend die C-terminale Region der α-Kette von IR, die C-terminale Region der α-Kette von IGF-1R oder ein Mimetikum der C-terminalen Region der α-Kette von IR und/oder IGF-1R,
wobei die C-terminale Region der α-Kette von IR die Aminosäuren 693 bis 710 der IR- α-Kette (SEQ ID NO: 13) umfasst,
wobei die C-terminale Region der α-Kette von IGF-1R die Aminosäuren 681 bis 697 der IGF-1R- α-Kette (SEQ ID NO: 15) umfasst, und
wobei das Mimetikum der C-terminalen Region der α-Kette von IR und/oder IGF-1R S519C16 (SEQ ID NO: 18) ist.

9. Verfahren nach Anspruch 8, wobei die Verbindung neugestaltet oder chemisch modifiziert ist, um (i) die Affinität für die Bindung an IR zu erhöhen, und/oder (ii) die Affinität für die Bindung an IGF-1R zu verringern, oder wobei die Verbindung neu gestaltet oder chemisch modifiziert ist, um (i) die Affinität für die Bindung an IGF-1R zu erhöhen, und/oder (ii) die Affinität für die Bindung an IR zu verringern,
optional wobei die Verbindung neu gestaltet oder modifiziert ist, um so die Affinität für IR oder IGF-1R aufgrund der strukturellen Unterschiede zwischen IR und IGF-1R an oder in der Nähe der C-terminalen Region der α-Kette von IR und der C-terminalen Region der α-Kette von IGF-1R zu verringern.

10. Rechnergestütztes Verfahren zum Identifizieren einer Verbindung, die möglicherweise mit IR und/oder IGF-1R in Wechselwirkung treten kann, wobei das Verfahren umfasst, dass die Struktur von:
(i) der gering affinen Bindungsstelle von IR, wobei die Struktur durch die in einem oder mehreren der Anhänge I, III und V dargestellten Atomkoordinaten, definiert ist;
(ii) der gering affinen Bindungsstelle von IGF-1 R für IGF, wobei die Struktur durch die in einem oder mehreren der Anhänge II, IV und VI dargestellten Atomkoordinaten definiert ist; und/oder
(iii) der C-terminalen Region der α-Kette von IR, der C-terminalen Region der α-Kette von IGF-1 R oder einem Mimetikum der C-terminalen Region der α-Kette von IR und/oder IGF-IR, wobei die Struktur durch eine Teilmenge von in einem oder mehreren der Anhänge I bis VI dargestellten Atomkoordinaten definiert wird,
auf die Struktur der Kandidatenverbindung angewandt wird,
wobei die Atomkoordinaten eine oder mehrere Regionen der gering affinen Bindungsstelle von IR für Insulin und/oder der gering affinen Bindungsstelle von IGF-1R für IGF definieren, umfassend die C-terminale Region der α-Kette von IR, die C-terminale Region der α-Kette von IGF-1R oder ein Mimetikum der C-terminalen Region der α-Kette von IR und/oder IGF-1R,
wobei die C-terminale Region der α-Kette von IR die Aminosäuren 693 bis 710 der IR- α-Kette (SEQ ID NO: 13) umfasst,
wobei die C-terminale Region der α-Kette von IGF-1R die Aminosäuren 681 bis 697 der IGF-1R- α-Kette (SEQ ID NO: 15) umfasst, und
wobei das Mimetikum der C-terminalen Region der α-Kette von IR und/oder IGF-1R S519C16 (SEQ ID NO: 18) ist.

11. Rechnergestütztes Verfahren zum Identifizieren einer Verbindung, die die Fähigkeit besitzt, mit IR und/oder IGF-1R in Wechselwirkung zu treten, unter Verwendung eines programmierten Rechners, umfassend einen Prozessor, wobei das Verfahren die Folgenden Schritte umfasst:
(a) mittels rechnergestützter Verfahren Erzeugen einer Reihe von Atomkoordinaten, die eine Struktur aufweisen, welche energetisch günstige Wechselwirkungen besitzt mit den Atomkoordinaten:
(i) der gering affinen Bindungsstelle von IR für Insulin, wobei die Struktur durch die in einem oder mehreren der Anhänge I, III und V dargestellten Atomkoordinaten, definiert ist;
(ii) der gering affinen Bindungsstelle von IGF-IR für IGF, wobei die Struktur durch die in einem oder mehreren der Anhänge II, IV und VI dargestellten Atomkoordinaten definiert ist; und/oder
(iii) der C-terminalen Region der α-Kette von IR, der C-terminalen Region der α-Kette von IGF-1 R oder einem Mimetikum der C-terminalen Region der α-Kette von IR und/oder IGF-1 R, wobei die Struktur durch eine Teilmenge von in einem oder mehreren der Anhänge I bis VI dargestellten Atomkoordinaten definiert ist,
wobei die Koordinaten in den Rechner eingegeben werden, wodurch ein Datensatz von Kriterien erzeugt wird;
(b) mithilfe des Prozessors Vergleichen des Datensatzes von Kriterien mit einer Rechnerdatenbank mit chemischen Strukturen;
(c) mithilfe von rechnergestützten Verfahren Auswählen chemischer Strukturen, welche komplementär oder ähnlich zu einer Region mit den im Datensatz enthaltenen Kriterien sind, aus der Datenbank; und optional
(d) an ein Ausgabegerät Ausgeben der ausgewählten chemischen Strukturen, welche komplementär oder ähnlich zu einer Region mit den im Datensatz enthaltenen Kriterien sind,
wobei die Atomkoordinaten eine oder mehrere Regionen der gering affinen Bindungsstelle von IR für Insulin und/oder der gering affinen Bindungsstelle von IGF-1R für IGF definieren, umfassend die C-terminale Region der α-Kette von IR, die C-terminale Region der α-Kette von IGF-1R oder ein Mimetikum der C-terminalen Region der α-Kette von IR und/oder IGF-1 R,
wobei die C-terminale Region der α-Kette von IR die Aminosäuren 693 bis 710 der IR- α-Kette (SEQ ID NO: 13) umfasst,
wobei die C-terminale Region der α-Kette von IGF-1 R die Aminosäuren 681 bis 697 der IGF-1 R- α-Kette (SEQ ID NO: 15) umfasst, und
wobei das Mimetikum der C-terminalen Region der α-Kette von IR und/oder IGF-1R S519C16 (SEQ ID NO: 18) ist.

12. Rechnergestütztes Verfahren zum Identifizieren möglicher Mimetika von IR und/oder IGF-1 R unter Verwendung eines programmierten Rechners, umfassend einen Prozessor, wobei das Verfahren die Folgenden Schritte umfasst:
(a) Erzeugen eines Datensatzes von Kriterien aus einer Reihe von Atomkoordinaten:
(i) der gering affinen Bindungsstelle von IR für Insulin, wobei die Struktur durch die in einem oder mehreren der Anhänge I, III und V dargestellten Atomkoordinaten, definiert ist;
(ii) der gering affinen Bindungsstelle von IGF-1R für IGF, wobei die Struktur durch die in einem oder mehreren der Anhänge II, IV und VI dargestellten Atomkoordinaten definiert ist; und/oder
(iii) der C-terminalen Region der α-Kette von IR, der C-terminalen Region der α-Kette von IGF-1R oder einem Mimetikum der C-terminalen Region von IR und/oder IGF-1 R, wobei die Struktur durch eine Teilmenge von in einem oder mehreren der Anhänge I bis VI dargestellten Atomkoordinaten definiert ist,
wobei die Koordinaten in den Rechner eingegeben werden;
(b)
(i) mithilfe des Prozessors Vergleichen des Datensatzes von Kriterien mit einer rechnerbasierten Datenbank mit chemischen Strukturen, die in einem Datenspeicherungssystem eines Rechners gespeichert sind, und mithilfe von rechnergestützten Verfahren auswählen chemischer Strukturen, die eine Region aufweisen, welche strukturell ähnlich zu den Kriterien des Datensatzes sind, aus der Datenbank; oder
(ii) mithilfe von rechnergestützten Verfahren Konstruieren eines Modells einer chemischen Struktur mit einer Region, die strukturell ähnlich zu den Kriterien des Datensatzes ist; und optional
(c) an ein Ausgabegerät Ausgeben:
(i) der ausgewählten chemischen Strukturen aus Schritt (b) (i) mit einer Region, die ähnlich zu den Kriterien des Datensatzes ist; oder
(ii) des konstruierten Modells aus Schritt (b) (ii),
wobei die Atomkoordinaten eine oder mehrere Regionen der gering affinen Bindungsstelle von IR für Insulin und/oder der gering affinen Bindungsstelle von IGF-1R für IGF definieren, umfassend die C-terminale Region der α-Kette von IR, die C-terminale Region der α-Kette von IGF-1 R oder ein Mimetikum der C-terminalen Region der α-Kette von IR und/oder IGF-1 R,
wobei die C-terminale Region der α-Kette von IR die Aminosäuren 693 bis 710 der IR- α-Kette (SEQ ID NO: 13) umfasst,
wobei die C-terminale Region der α-Kette von IGF-1 R die Aminosäuren 681 bis 697 der IGF-IR- α-Kette (SEQ ID NO: 15) umfasst, und
wobei das Mimetikum der C-terminalen Region der α-Kette von IR und/oder IGF-IR S519C16 (SEQ ID NO: 18) ist.

13. Verfahren zum Evaluieren der Fähigkeit einer Verbindung, mit IR und/oder IGF-1R in Wechselwirkung zu treten, wobei das Verfahren die Folgenden Schritte umfasst:
(a) Anwenden rechnerischer Mittel, um einen Anpassungsvorgang zwischen der Verbindung und der Bindungsoberfläche nach einem rechnerischen Modell für die Bindungsstelle mit geringer Affinität für Insulin an der IR-Ektodomäne und/oder der Bindungsstelle mit geringer Affinität für IGF an der IGF-1R-Ektodomäne mithilfe von Atomkoordinaten durchzuführen, wobei die Standardabweichung zwischen diesen Atomkoordinaten und den Atomkoordinaten, die in einem oder mehreren der Anhänge I-VI dargestellt sind, oder einer Teilmenge der Atomkoordinaten, die in einem oder mehreren davon dargestellt ist, die mindestens die C-terminale Region der α-Kette von IR, der C-terminalen Region der α-Kette von IGF-1R oder ein Mimetikum der C-terminalen Region der α-Kette von IR und/oder IGF-1R repräsentieren, um nicht mehr als 1,5 Å abweicht; und
(b) Analysieren der Ergebnisse des Anpassungsvorgangs, um die Assoziation zwischen der Verbindung und dem Model der Oberflächenbindung zu quantifizieren,
wobei die Atomkoordinaten eine oder mehrere Regionen der gering affinen Bindungsstelle von IR für Insulin und/oder der gering affinen Bindungsstelle von IGF-1R für IGF definieren, umfassend die C-terminale Region der α-Kette von IR, die C-terminale Region der α-Kette von IGF-1R oder ein Mimetikum der C-terminalen Region der α-Kette von IR und/oder IGF-1R,
wobei die C-terminale Region der α-Kette von IR die Aminosäuren 693 bis 710 der IR- α-Kette (SEQ ID NO: 13) umfasst,
wobei die C-terminale Region der α-Kette von IGF-1R die Aminosäuren 681 bis 697 der IGF-1 R- α-Kette (SEQ ID NO: 15) umfasst, und
wobei das Mimetikum der C-terminalen Region der α-Kette von IR und/oder IGF-1R S519C16 (SEQ ID NO: 18) ist.

14. Verfahren zum Verwenden von Molekularaustausch, um Strukturinformationen über ein Molekül oder einen molekularen Komplex mit unbekannter Struktur zu erhalten, umfassend die Folgenden Schritte:
(i) Erzeugen eines Röntgenbeugungsdiagramms des kristallisierten Moleküls oder molekularen Komplexes; und
(ii) Anwenden der Atomkoordinaten von einem oder mehreren der Anhänge I bis VI oder einer Teilmenge von Atomkoordinaten von einem oder mehreren davon, die mindestens die C-terminale Region der α-Kette von IR, die C-terminale Region der α-Kette von IGF-1R oder ein Mimetikum der C-terminalen Region der α-Kette von IR und/oder IGF-1R repräsentieren, auf das Röntgenbeugungsdiagramm, um eine dreidimensionale Elektronendichtekarte von mindestens einer Region des Moleküls oder des molekularen Komplexes zu erzeugen, dessen Struktur unbekannt ist,
wobei die Atomkoordinaten eine oder mehrere Regionen der gering affinen Bindungsstelle von IR für Insulin und/oder der gering affinen Bindungsstelle von IGF-1R für IGF definieren, umfassend die C-terminale Region der α-Kette von IR, die C-terminale Region der α-Kette von IGF-1R oder ein Mimetikum der C-terminalen Region der α-Kette von IR und/oder IGF-1R,
wobei die C-terminale Region der α-Kette von IR die Aminosäuren 693 bis 710 der IR- α-Kette (SEQ ID NO: 13) umfasst,
wobei die C-terminale Region der α-Kette von IGF-1R die Aminosäuren 681 bis 697 der IGF-1R- α-Kette (SEQ ID NO: 15) umfasst, und
wobei das Mimetikum der C-terminalen Region der α-Kette von IR und/oder IGF-1R S519C16 (SEQ ID NO: 18) ist.

## Revendications

1. Procédé assisté par ordinateur d'identification, de conception ou de criblage d'un composé qui peut potentiellement interagir avec un récepteur d'insuline (IR) et/ou un récepteur de facteur de croissance 1 analogue à l'insuline (IGF-1R), comprenant la réalisation d'une identification, d'une conception ou d'un criblage basé sur la structure d'un composé sur la base des interactions du composé avec une structure définie par les coordonnées atomiques d'une ou plusieurs des annexes I à VI, ou un sous-ensemble de coordonnées atomiques d'une ou plusieurs d'entre elles représentant au moins la région C-terminale de la chaîne a de l'IR, la région C-terminale de la chaîne α de l'IGF-1R, ou un mimétique de la région C-terminale de la chaîne a de l'IR et/ou IGF-1R,
dans lequel les coordonnées atomiques définissent une ou plusieurs régions du site de liaison de faible affinité de l'IR pour l'insuline et/ou du site de liaison de faible affinité de l'IGF-1 R pour l'IGF, comprenant la région C-terminale de la chaîne a de l'IR, la région C-terminale de la chaîne a de l'IGF-1R, ou un mimétique de la région C-terminale de la chaîne a de l'IR et/ou de l'IGF-1 R,
dans lequel la région C-terminale de la chaîne a de l'IR comprend les acides aminés 693 à 710 de la chaîne a de l'IR (SEQ ID N°13),
dans lequel la région C-terminale de la chaîne a de l'IGF-1 R comprend les acides aminés 681 à 697 de la chaîne α de l'IGF-1 R (SEQ ID N°15), et
dans lequel le mimétique de la région C-terminale de la chaîne a de l'IR et/ou de l'IGF-1 R, est S519C16 (SEQ ID N° 18).

2. Procédé selon la revendication 1, comprenant l'identification, la conception ou le criblage d'un composé qui interagit avec la structure tridimensionnelle :
(i) du site de liaison à l'insuline de faible affinité de l'IR, la structure étant définie par les coordonnées atomiques montrées dans une ou plusieurs des annexes I, III et V ; et/ou
(ii) du site de liaison au facteur de croissance analogue à l'insuline (IGF) de faible affinité de l'IGF-1 R, la structure étant définie par les coordonnées atomiques montrées dans une ou plusieurs des annexes II, IV et VI,
dans lequel l'interaction du composé avec la structure est favorisée énergétiquement,
et/ou comprenant en outre la synthèse ou l'obtention d'un composé candidat identifié ou conçu et la détermination de la capacité du composé candidat à interagir avec l'IR et/ou l'IGF-1 R.

3. Procédé selon la revendication 1, dans lequel les coordonnées atomiques définissant le site de liaison à l'insuline de faible affinité de l'IR comprennent en outre le domaine de la répétition riche en leucine 1 (L1) et/ou le domaine riche en cystéine (CR) de l'ectodomaine de l'IR, facultativement dans lequel les coordonnées atomiques définissent des parties de la surface moléculaire du feuillet β central du domaine L1 et des parties de la surface moléculaire de la seconde répétition riche en leucine (LRR) qui contiennent Phe39 et/ou la boucle dans le quatrième barreau LRR du domaine L1 et/ou dans lequel les coordonnées atomiques définissent le module 6 du domaine CR de l'IR.

4. Procédé selon l'une quelconque des revendications précédentes, dans lequel les coordonnées atomiques définissent en outre une ou plusieurs séquences d'acides aminés choisies parmi les résidus d'acides aminés de l'IR 1 à 156, 157 à 310, 594 et 794, facultativement dans lequel le ou les acides aminés choisis parmi les résidus d'acides aminés de l'IR 1 à 156 comprennent au moins un acide aminé choisi parmi Arg14, Asn15, Gln34, Leu36, Leu37, Phe39, Pro43, Phe46, Leu62, Phe64, Leu87, Phe88, Phe89, Asn90, Phe96, Glu97, Arg118, Glu120 et His144, et/ou dans lequel le un ou les acides aminés choisis parmi les résidus d'acides aminés de l'IR 157 à 310 comprennent au moins l'une des séquences d'acides aminés choisies parmi 192 à 310, 227 à 303 et 259 à 284.

5. Procédé selon la revendication 1, dans lequel les coordonnées atomiques définissant le site de liaison à l'IGF de faible affinité de l'IGF-1R comprennent en outre le domaine L1 et/ou le domaine CR de l'ectodomaine de l'IGF-1 R, facultativement dans lequel les coordonnées atomiques définissent le feuillet β central du domaine L1 et/ou cette partie de la seconde LRR contenant Ser35 et/ou la boucle dans le quatrième barreau LRR du domaine L1 et/ou dans lequel les coordonnées atomiques définissent le module 6 du domaine CR de l'IGF-1R.

6. Procédé de la revendication 2, dans lequel le composé remplace la région C-terminale de la chaîne α de l'IR et/ou la région C-terminale de la chaîne α de l'IGF-1 R dans la formation du site de liaison de faible affinité de l'IR ou de l'IGF-1R.

7. Procédé selon l'une quelconque des revendications précédentes, dans lequel un composé candidat d'interaction avec l'IR et/ou l'IGF-1 R est modifié chimiquement par suite d'une évaluation basée sur la structure, facultativement dans lequel la modification chimique est conçue pour :
(i) soit réduire le potentiel du composé candidat à se lier à l'IR tout en maintenant une liaison à l'IGF-1 R ;
(ii) soit réduire le potentiel du composé candidat à se lier à l'IGF-1 R, tout en maintenant une liaison à l'IR.

8. Procédé assisté par ordinateur de reconception d'un composé qui est connu pour se lier à l'IR et/ou à l'IGF-1 R, comprenant la réalisation d'une évaluation basée sur la structure du composé sur la base des interactions du composé avec une structure définie par les coordonnées atomiques d'une ou plusieurs des annexes I à VI, ou d'un sous-ensemble de coordonnées atomiques d'une ou plusieurs d'entre elles représentant au moins la région C-terminale de la chaîne α de l'IR, la région C-terminale de la chaîne a de l'IGF-1 R, ou un mimétique de la région C-terminale de la chaîne α de l'IR et/ou l'IGF-1 R, et la reconception ou la modification chimique du composé par suite de l'évaluation,
dans lequel les coordonnées atomiques définissent une ou plusieurs régions du site de liaison de faible affinité de l'IR pour l'insuline, et/ou du site de liaison de faible affinité de l'IGF-1 R pour l'IGF, comprenant la région C-terminale de la chaîne α de l'IR, la région C-terminale de la chaîne a de l'IGF-1 R, ou un mimétique de la région C-terminale de la chaîne a de l'IR et/ou de l'IGF-1 R,
dans lequel la région C-terminale de la chaîne a de l'IR comprend les acides aminés 693 à 710 de la chaîne a de l'IR (SEQ ID N°13),
dans lequel la région C-terminale de la chaîne α de l'IGF-1 R comprend les acides aminés 681 à 697 de la chaîne α de l'IGF-1 R (SEQ ID N°15), et
dans lequel le mimétique de la région C-terminale de la chaîne α de l'IR et/ou de l'IGF-1 R est S519C16 (SEQ ID N° 18).

9. Procédé selon la revendication 8, dans lequel le composé est reconçu ou modifié chimiquement pour (i) améliorer l'affinité de liaison à l'IR, et/ou (ii) diminuer l'affinité de liaison à l'IGF-1 R, ou dans lequel le composé est reconçu ou modifié chimiquement pour (i) améliorer l'affinité de liaison à l'IGF-1 R,
et/ou (ii) diminuer l'affinité de liaison à l'IR,
facultativement dans lequel le composé est reconçu ou modifié de façon à diminuer l'affinité pour l'IR ou de l'IGF-1 R en vertu des différences structurelles entre l'IR et l'IGF-1 R dans ou au voisinage de la région C-terminale de la chaîne α de l'IR et la région C-terminale de la chaîne α de l'IGF-1 R.

10. Procédé assisté par ordinateur d'identification d'un composé qui interagit potentiellement avec l'IR et/ou l'IGF-1R, lequel procédé comprend l'ajustement de la structure :
(i) du site de liaison à l'insuline de faible affinité de l'IR, la structure étant définie par les coordonnées atomiques montrées dans une ou plusieurs des annexes I, III et V ;
(ii) du site de liaison à l'IGF de faible affinité de l'IGF-1 R, la structure étant définie par les coordonnées atomiques montrées dans une ou plusieurs des annexes II, IV et VI ; et/ou
(iii) de la région C-terminale de la chaîne α de l'IR, la région C-terminale de la chaîne α de l'IGF-1 R, ou d'un mimétique de la région C-terminale de la chaîne α de l'IR et/ou de l'IGF-1 R, la structure étant définie par un sous-ensemble de coordonnées atomiques montrées dans une ou plusieurs des annexes I à VI,
à la structure d'un composé candidat,
dans lequel les coordonnées atomiques définissent une ou plusieurs régions du site de liaison de faible affinité de l'IR pour l'insuline et/ou du site de liaison de faible affinité de l'IGF-1 R pour l'IGF, comprenant la région C-terminale de la chaîne α de l'IR, la région C-terminale de la chaîne α de l'IGF-1 R ou un mimétique de la région C-terminale de la chaîne α de l'IR et/ou de l'IGF-1 R,
dans lequel la région C-terminale de la chaîne α de l'IR comprend les acides aminés 693 à 710 de la chaîne α de l'IR (SEQ ID N°13),
dans lequel la région C-terminale de la chaîne α de l'IGF-1 R comprend les acides aminés 681 à 697 de la chaîne α de l'IGF-1 R (SEQ ID N°15), et
dans lequel le mimétique de la région C-terminale de la chaîne α de l'IR et/ou de l'IGF-1 R est S519C16 (SEQ ID N° 18).

11. Procédé assisté par ordinateur d'identification d'un composé capable d'interagir avec l'IR et/ou l'IGF-1R utilisant un ordinateur programmé comprenant un processeur, lequel procédé comprend les étapes consistant à :
(a) générer, à l'aide de procédés informatiques, un ensemble de coordonnées atomiques d'une structure qui possède des interactions énergétiquement favorables avec les coordonnées atomiques :
(i) du site de liaison à l'insuline de faible affinité de l'IR, la structure étant définie par les coordonnées atomiques montrées dans une ou plusieurs des annexes I, III et V ;
(ii) du site de liaison à l'IGF de faible affinité de l'IGF-1 R, la structure étant définie par les coordonnées atomiques montrées dans une ou plusieurs des annexes II, IV et VI ; et/ou
(iii) de la région C-terminale de la chaîne α de l'IR, la région C-terminale de la chaîne α de l'IGF-1 R, ou d'un mimétique de la région C-terminale de la chaîne α de l'IR et/ou de l'IGF-1 R, la structure étant définie par un sous-ensemble de coordonnées atomiques montrées dans une ou plusieurs des annexes I à VI,
lesquelles coordonnées sont entrées dans l'ordinateur générant ainsi un ensemble de données de critères ;
(b) comparer, à l'aide du processeur, l'ensemble de données de critères à une base de données informatique de structures chimiques ;
(c) sélectionner dans la base de données, à l'aide de procédés informatiques, des structures chimiques qui sont complémentaires de ou similaires à une région de l'ensemble de données de critères ; et facultativement,
(d) produire en sortie, dans un dispositif de sortie, les structures chimiques sélectionnées qui sont complémentaires de ou similaires à une région de l'ensemble de données de critères,
dans lequel les coordonnées atomiques définissent une ou plusieurs régions du site de liaison de faible affinité de l'IR pour l'insuline et/ou du site de liaison de faible affinité de l'IGF-1 R pour l'IGF, comprenant la région C-terminale de la chaîne α de l'IR, la région C-terminale de la chaîne α de l'IGF-1R ou un mimétique de la région C-terminale de la chaîne α de l'IR et/ou de l'IGF-1 R,
dans lequel la région C-terminale de la chaîne α de l'IR comprend les acides aminés 693 à 710 de la chaîne α de l'IR (SEQ ID N°13),
dans lequel la région C-terminale de la chaîne α de l'IGF-1 R comprend les acides aminés 681 à 697 de la chaîne α de l'IGF-1 R (SEQ ID N°15), et
dans lequel le mimétique de la région C-terminale de la chaîne α de l'IR et/ou de l'IGF-1 R est S519C16 (SEQ ID N°18).

12. Procédé assisté par ordinateur d'identification de mimétiques potentiels de l'IR et/ou de l'IGF-1 R utilisant un ordinateur programmé comprenant un processeur, le procédé comprenant les étapes consistant à :
(a) générer un ensemble de données de critères à partir d'un ensemble de coordonnées atomiques :
(i) du site de liaison à l'insuline de faible affinité de l'IR, la structure étant définie par les coordonnées atomiques montrées dans une ou plusieurs des annexes I, III et V ;
(ii) du site de liaison à l'IGF de faible affinité de l'IGF-1 R, la structure étant définie par les coordonnées atomiques montrées dans une ou plusieurs des annexes II, IV et VI ; et/ou
(iii) de la région C-terminale de la chaîne α de l'IR, de la région C-terminale de la chaîne α de l'IGF-1 R, ou d'un mimétique de la région C-terminale de la chaîne α de l'IR et/ou de l'IGF-1 R, la structure étant définie par un sous-ensemble de coordonnées atomiques montrées dans une ou plusieurs des annexes I à VI,
lesquelles coordonnées sont entrées dans l'ordinateur ;
(b)
(i) comparer, à l'aide du processeur, l'ensemble de données de critères à une base de données informatique de structures chimiques stockée dans un système de stockage de données informatiques et sélectionner dans la base de données, en utilisant des procédés informatiques, des structures chimiques ayant une région qui est structurellement similaire à l'ensemble de données de critères ; ou
(ii) construire, à l'aide de procédés d'ordinateur, un modèle d'une structure chimique ayant une région qui est structurellement similaire à l'ensemble de données de critères ; et facultativement,
(c) produire en sortie dans un dispositif de sortie :
(i) les structures chimiques sélectionnées de l'étape (b) (i) ayant une région similaire à l'ensemble de données de critères ; ou
(ii) le modèle construit de l'étape (b) (ii),
dans lequel les coordonnées atomiques définissent une ou plusieurs régions du site de liaison de faible affinité de l'IR pour l'insuline et/ou du site de liaison de faible affinité de l'IGF-1 R pour l'IGF, comprenant la région C-terminale de la chaîne α de l'IR, la région C-terminale de la chaîne α de l'IGF-1 R ou un mimétique de la région C-terminale de la chaîne α de l'IR et/ou de l'IGF-1 R,
dans lequel la région C-terminale de la chaîne α de l'IR comprend les acides aminés 693 à 710 de la chaîne α de l'IR (SEQ ID N°13),
dans lequel la région C-terminale de la chaîne α de l'IGF-1 R comprend les acides aminés 681 à 697 de la chaîne α de l'IGF-1 R (SEQ ID N°15), et
dans lequel le mimétique de la région C-terminale de la chaîne α de l'IR et/ou de l'IGF-1 R est S519C16 (SEQ ID N° 18).

13. Procédé d'évaluation de la capacité d'un composé à interagir avec l'IR et/ou l'IGF-1 R, le procédé comprenant les étapes consistant à :
(a) employer des moyens de calcul pour réaliser une opération d'ajustement entre le composé et la surface de liaison d'un modèle informatique du site de liaison de faible affinité pour l'insuline sur l'ectodomaine de l'IR, et/ou le site de liaison de faible affinité pour l'IGF sur l'ectodomaine de l'IGF-1 R, en utilisant des coordonnées atomiques dans lesquelles l'écart moyen quadratique entre les coordonnées atomiques et des coordonnées atomiques d'une ou plusieurs des annexes I à VI ou un sous-ensemble de coordonnées atomiques d'une ou plusieurs d'entre elles représentant au moins la région C-terminale de la chaîne α de l'IR, la région C-terminale de la chaîne α de l'IGF-1R, ou un mimétique de la région C-terminale de la chaîne α de l'IR et/ou de l'IGF-1 R, n'est pas supérieur à 1,5 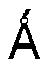 ; et
(b) analyser les résultats de l'opération d'ajustement pour quantifier l'association entre le composé et le modèle de surface de liaison,
dans lequel les coordonnées atomiques définissent une ou plusieurs régions du site de liaison de faible affinité de l'IR pour l'insuline et/ou du site de liaison de faible affinité de l'IGF-1 R pour l'IGF, comprenant la région C-terminale de la chaîne α de l'IR, la région C-terminale de la chaîne α de l'IGF-1 R ou un mimétique de la région C-terminale de la chaîne α de l'IR et/ou de l'IGF-1 R,
dans lequel la région C-terminale de la chaîne α de l'IR comprend les acides aminés 693 à 710 de la chaîne α de l'IR (SEQ ID N°13),
dans lequel la région C-terminale de la chaîne α de l'IGF-1 R comprend les acides aminés 681 à 697 de la chaîne α de l'IGF-1 R (SEQ ID N°15), et
dans lequel le mimétique de la région C-terminale de la chaîne α de l'IR et/ou de l'IGF-1R est S519C16 (SEQ ID N°18).

14. Procédé d'utilisation d'un substitut moléculaire pour obtenir des informations structurelles à propos d'une molécule ou d'un complexe moléculaire de structure inconnue, comprenant les étapes consistant à :
(i) générer un diagramme de diffraction des rayons X de la molécule ou du complexe moléculaire cristallisé ; et
(ii) appliquer les coordonnées atomiques d'une ou plusieurs des annexes I à VI, ou d'un sous-ensemble de coordonnées atomiques d'une ou plusieurs d'entre elles représentant au moins la région C-terminale de la chaîne α de l'IR, la région C-terminale de la chaîne α de l'IGF-1 R, ou un mimétique de la région C-terminale de la chaîne α de l'IR et/ou l'IGF-1 R, au diagramme de diffraction des rayons X pour générer une carte de densité électronique tridimensionnelle d'au moins une région de la molécule ou du complexe moléculaire dont la structure est inconnue,
dans lequel les coordonnées atomiques définissent une ou plusieurs régions du site de liaison de faible affinité de l'IR pour l'insuline et/ou du site de liaison de faible affinité de l'IGF-1 R pour l'IGF, comprenant la région C-terminale de la chaîne α de l'IR, la région C-terminale de la chaîne α de l'IGF-1 R ou un mimétique de la région C-terminale de la chaîne α de l'IR et/ou de l'IGF-1 R,
dans lequel la région C-terminale de la chaîne α de l'IR comprend les acides aminés 693 à 710 de la chaîne α de l'IR (SEQ ID N°13),
dans lequel la région C-terminale de la chaîne α de l'IGF-1 R comprend les acides aminés 681 à 697 de la chaîne α de l'IGF-1 R (SEQ ID N°15), et
dans lequel le mimétique de la région C-terminale de la chaîne α de l'IR et/ou de l'IGF-1 R est S519C16 (SEQ ID N° 18).


### SEQUENCE LISTING

<110> Walter and Eliza Hall Institute of Medical Research
<120> Structure of the C-terminal region of the insulin receptor alpha-chain and of the insulin-like growth factor receptor alpha-chain
<130> A/16/231
<150> 61/214.472 <151> 2009-04-22
<160> 19
<170> PatentIn version 3.5
<210> 1
   <211> 917
   <212> PRT
   <213> Homo sapiens
<400> 1
   His Leu Tyr Pro Gly Glu Val Cys Pro Gly Met Asp Ile Arg Asn Asn
   1               5                   10                  15
   Leu Thr Arg Leu His Glu Leu Glu Asn Cys Ser Val Ile Glu Gly His
               20                  25                  30
   Leu Gln Ile Leu Leu Met Phe Lys Thr Arg Pro Glu Asp Phe Arg Asp
           35                  40                  45
   Leu Ser Phe Pro Lys Leu Ile Met Ile Thr Asp Tyr Leu Leu Leu Phe
       50                  55                  60
   Arg Val Tyr Gly Leu Glu Ser Leu Lys Asp Leu Phe Pro Asn Leu Thr
   65                  70                  75                  80
   Val Ile Arg Gly Ser Arg Leu Phe Phe Asn Tyr Ala Leu Val Ile Phe
                   85                  90                  95
   Glu Met Val His Leu Lys Glu Leu Gly Leu Tyr Asn Leu Met Asn Ile
               100                 105                 110
   Thr Arg Gly Ser Val Arg Ile Glu Lys Asn Asn Glu Leu Cys Tyr Leu
           115                 120                 125
   Ala Thr Ile Asp Trp Ser Arg Ile Leu Asp Ser Val Glu Asp Asn His
       130                 135                 140
   Ile Val Leu Asn Lys Asp Asp Asn Glu Glu Cys Gly Asp Ile Cys Pro
   145                 150                 155                 160
   Gly Thr Ala Lys Gly Lys Thr Asn Cys Pro Ala Thr Val Ile Asn Gly
                   165                 170                 175
   Gln Phe Val Glu Arg Cys Trp Thr His Ser His Cys Gln Lys Val Cys
               180                 185                 190
   Pro Thr Ile Cys Lys Ser His Gly Cys Thr Ala Glu Gly Leu Cys Cys
           195                 200                 205
   His Ser Glu Cys Leu Gly Asn Cys Ser Gln Pro Asp Asp Pro Thr Lys
       210                 215                 220
   Cys Val Ala Cys Arg Asn Phe Tyr Leu Asp Gly Arg Cys Val Glu Thr
   225                 230                 235                 240
   Cys Pro Pro Pro Tyr Tyr His Phe Gln Asp Trp Arg Cys Val Asn Phe
                   245                 250                 255
   Ser Phe Cys Gln Asp Leu His His Lys Cys Lys Asn Ser Arg Arg Gln
               260                 265                 270
   Gly Cys His Gln Tyr Val Ile His Asn Asn Lys Cys Ile Pro Glu Cys
           275                 280                 285
   Pro Ser Gly Tyr Thr Met Asn Ser Ser Asn Leu Leu Cys Thr Pro Cys
       290                 295                 300
   Leu Gly Pro Cys Pro Lys Val Cys His Leu Leu Glu Gly Glu Lys Thr
   305                 310                 315                 320
   Ile Asp Ser Val Thr Ser Ala Gln Glu Leu Arg Gly Cys Thr Val Ile
                   325                 330                 335
   Asn Gly Ser Leu Ile Ile Asn Ile Arg Gly Gly Asn Asn Leu Ala Ala
               340                 345                 350
   Glu Leu Glu Ala Asn Leu Gly Leu Ile Glu Glu Ile Ser Gly Tyr Leu
           355                 360                 365
   Lys Ile Arg Arg Ser Tyr Ala Leu Val Ser Leu Ser Phe Phe Arg Lys
       370                 375                 380
   Leu Arg Leu Ile Arg Gly Glu Thr Leu Glu Ile Gly Asn Tyr Ser Phe
   385                 390                 395                 400
   Tyr Ala Leu Asp Asn Gln Asn Leu Arg Gln Leu Trp Asp Trp Ser Lys
                   405                 410                 415
   His Asn Leu Thr Ile Thr Gln Gly Lys Leu Phe Phe His Tyr Asn Pro
               420                 425                 430
   Lys Leu Cys Leu Ser Glu Ile His Lys Met Glu Glu Val Ser Gly Thr
           435                 440                 445
   Lys Gly Arg Gln Glu Arg Asn Asp Ile Ala Leu Lys Thr Asn Gly Asp
       450                 455                 460
   Gln Ala Ser Cys Glu Asn Glu Leu Leu Lys Phe Ser Tyr Ile Arg Thr
   465                 470                 475                 480
   Ser Phe Asp Lys Ile Leu Leu Arg Trp Glu Pro Tyr Trp Pro Pro Asp
                   485                 490                 495
   Phe Arg Asp Leu Leu Gly Phe Met Leu Phe Tyr Lys Glu Ala Pro Tyr
               500                 505                 510
   Gln Asn Val Thr Glu Phe Asp Gly Gln Asp Ala Cys Gly Ser Asn Ser
           515                 520                 525
   Trp Thr Val Val Asp Ile Asp Pro Pro Leu Arg Ser Asn Asp Pro Lys
       530                 535                 540
   Ser Gln Asn His Pro Gly Trp Leu Met Arg Gly Leu Lys Pro Trp Thr
   545                 550                 555                 560
   Gln Tyr Ala Ile Phe Val Lys Thr Leu Val Thr Phe Ser Asp Glu Arg
   565 570 575
   Arg Thr Tyr Gly Ala Lys Ser Asp Ile Ile Tyr Val Gln Thr Asp Ala
   580 585 590
   Thr Asn Pro Ser Val Pro Leu Asp Pro Ile Ser Val Ser Asn Ser Ser
   595 600 605
   Ser Gln Ile Ile Leu Lys Trp Lys Pro Pro Ser Asp Pro Asn Gly Asn
   610 615 620
   Ile Thr His Tyr Leu Val Phe Trp Glu Arg Gln Ala Glu Asp Ser Glu
   625 630 635 640
   Leu Phe Glu Leu Asp Tyr Cys Leu Lys Gly Leu Lys Leu Pro Ser Arg
   645 650 655
   Thr Trp Ser Pro Pro Phe Glu Ser Glu Asp Ser Gln Lys His Asn Gln
   660 665 670
   Ser Glu Tyr Glu Asp Ser Ala Gly Glu Cys Cys Ser Cys Pro Lys Thr
   675 680 685
   Asp Ser Gln Ile Leu Lys Glu Leu Glu Glu Ser Ser Phe Arg Lys Thr
   690 695 700
   Phe Glu Asp Tyr Leu His Asn Val Val Phe Val Pro Arg Pro Ser Arg
   705 710 715 720
   Lys Arg Arg Ser Leu Gly Asp Val Gly Asn Val Thr Val Ala Val Pro
   725 730 735
   Thr Val Ala Ala Phe Pro Asn Thr Ser Ser Thr Ser Val Pro Thr Ser
   740 745 750
   Pro Glu Glu His Arg Pro Phe Glu Lys Val Val Asn Lys Glu Ser Leu
   755 760 765
   Val Ile Ser Gly Leu Arg His Phe Thr Gly Tyr Arg Ile Glu Leu Gln
   770 775 780
   Ala Cys Asn Gln Asp Thr Pro Glu Glu Arg Cys Ser Val Ala Ala Tyr
   785 790 795 800
   Val Ser Ala Arg Thr Met Pro Glu Ala Lys Ala Asp Asp Ile Val Gly
   805 810 815
   Pro Val Thr His Glu Ile Phe Glu Asn Asn Val Val His Leu Met Trp
   820 825 830
   Gln Glu Pro Lys Glu Pro Asn Gly Leu Ile Val Leu Tyr Glu Val Ser
   835 840 845
   Tyr Arg Arg Tyr Gly Asp Glu Glu Leu His Leu Cys Asp Thr Arg Lys
   850 855 860
   His Phe Ala Leu Glu Arg Gly Cys Arg Leu Arg Gly Leu Ser Pro Gly
   865 870 875 880
   Asn Tyr Ser Val Arg Ile Arg Ala Thr Ser Leu Ala Gly Asn Gly Ser
   885 890 895
   Trp Thr Glu Pro Thr Tyr Phe Tyr Val Thr Asp Tyr Leu Asp Val Pro
   900 905 910
   Ser Asn Ile Ala Lys
   915
<210> 2
   <211> 929
   <212> PRT
   <213> Homo sapiens
<400> 2
   His Leu Tyr Pro Gly Glu Val Cys Pro Gly Met Asp Ile Arg Asn Asn
   1               5                   10                  15
   Leu Thr Arg Leu His Glu Leu Glu Asn Cys Ser Val Ile Glu Gly His
               20                  25                  30
   Leu Gln Ile Leu Leu Met Phe Lys Thr Arg Pro Glu Asp Phe Arg Asp
           35                  40                  45
   Leu Ser Phe Pro Lys Leu Ile Met Ile Thr Asp Tyr Leu Leu Leu Phe
       50                  55                  60
   Arg Val Tyr Gly Leu Glu Ser Leu Lys Asp Leu Phe Pro Asn Leu Thr
   65                  70                  75                  80
   Val Ile Arg Gly Ser Arg Leu Phe Phe Asn Tyr Ala Leu Val Ile Phe
                   85                  90                  95
   Glu Met Val His Leu Lys Glu Leu Gly Leu Tyr Asn Leu Met Asn Ile
               100                 105                 110
   Thr Arg Gly Ser Val Arg Ile Glu Lys Asn Asn Glu Leu Cys Tyr Leu
           115                 120                 125
   Ala Thr Ile Asp Trp Ser Arg Ile Leu Asp Ser Val Glu Asp Asn His
       130                 135                 140
   Ile Val Leu Asn Lys Asp Asp Asn Glu Glu Cys Gly Asp Ile Cys Pro
   145                 150                 155                 160
   Gly Thr Ala Lys Gly Lys Thr Asn Cys Pro Ala Thr Val Ile Asn Gly
                   165                 170                 175
   Gln Phe Val Glu Arg Cys Trp Thr His Ser His Cys Gln Lys Val Cys
               180                 185                 190
   Pro Thr Ile Cys Lys Ser His Gly Cys Thr Ala Glu Gly Leu Cys Cys
           195                 200                 205
   His Ser Glu Cys Leu Gly Asn Cys Ser Gln Pro Asp Asp Pro Thr Lys
       210                 215                 220
   Cys Val Ala Cys Arg Asn Phe Tyr Leu Asp Gly Arg Cys Val Glu Thr
   225                 230                 235                 240
   Cys Pro Pro Pro Tyr Tyr His Phe Gln Asp Trp Arg Cys Val Asn Phe
                   245                 250                 255
   Ser Phe Cys Gln Asp Leu His His Lys Cys Lys Asn Ser Arg Arg Gln
               260                 265                 270
   Gly Cys His Gln Tyr Val Ile His Asn Asn Lys Cys Ile Pro Glu Cys
           275                 280                 285
   Pro Ser Gly Tyr Thr Met Asn Ser Ser Asn Leu Leu Cys Thr Pro Cys
       290                 295                 300
   Leu Gly Pro Cys Pro Lys Val Cys His Leu Leu Glu Gly Glu Lys Thr
   305                 310                 315                 320
   Ile Asp Ser Val Thr Ser Ala Gln Glu Leu Arg Gly Cys Thr Val Ile
                   325                 330                 335
   Asn Gly Ser Leu Ile Ile Asn Ile Arg Gly Gly Asn Asn Leu Ala Ala
               340                 345                 350
   Glu Leu Glu Ala Asn Leu Gly Leu Ile Glu Glu Ile Ser Gly Tyr Leu
           355                 360                 365
   Lys Ile Arg Arg Ser Tyr Ala Leu Val Ser Leu Ser Phe Phe Arg Lys
       370                 375                 380
   Leu Arg Leu Ile Arg Gly Glu Thr Leu Glu Ile Gly Asn Tyr Ser Phe
   385                 390                 395                 400
   Tyr Ala Leu Asp Asn Gln Asn Leu Arg Gln Leu Trp Asp Trp Ser Lys
                   405                 410                 415
   His Asn Leu Thr Ile Thr Gln Gly Lys Leu Phe Phe His Tyr Asn Pro
               420                 425                 430
   Lys Leu Cys Leu Ser Glu Ile His Lys Met Glu Glu Val Ser Gly Thr
           435                 440                 445
   Lys Gly Arg Gln Glu Arg Asn Asp Ile Ala Leu Lys Thr Asn Gly Asp
       450                 455                 460
   Gln Ala Ser Cys Glu Asn Glu Leu Leu Lys Phe Ser Tyr Ile Arg Thr
   465                 470                 475                 480
   Ser Phe Asp Lys Ile Leu Leu Arg Trp Glu Pro Tyr Trp Pro Pro Asp
                   485                 490                 495
   Phe Arg Asp Leu Leu Gly Phe Met Leu Phe Tyr Lys Glu Ala Pro Tyr
               500                 505                 510
   Gln Asn Val Thr Glu Phe Asp Gly Gln Asp Ala Cys Gly Ser Asn Ser
           515                 520                 525
   Trp Thr Val Val Asp Ile Asp Pro Pro Leu Arg Ser Asn Asp Pro Lys
       530                 535                 540
   Ser Gln Asn His Pro Gly Trp Leu Met Arg Gly Leu Lys Pro Trp Thr
   545                 550                 555                 560
   Gln Tyr Ala Ile Phe Val Lys Thr Leu Val Thr Phe Ser Asp Glu Arg
                   565                 570                 575
   Arg Thr Tyr Gly Ala Lys Ser Asp Ile Ile Tyr Val Gln Thr Asp Ala
               580                 585                 590
   Thr Asn Pro Ser Val Pro Leu Asp Pro Ile Ser Val Ser Asn Ser Ser
           595                 600                 605
   Ser Gln Ile Ile Leu Lys Trp Lys Pro Pro Ser Asp Pro Asn Gly Asn
       610                 615                 620
   Ile Thr His Tyr Leu Val Phe Trp Glu Arg Gln Ala Glu Asp Ser Glu
   625                 630                 635                 640
   Leu Phe Glu Leu Asp Tyr Cys Leu Lys Gly Leu Lys Leu Pro Ser Arg
   645 650 655
   Thr Trp Ser Pro Pro Phe Glu Ser Glu Asp Ser Gln Lys His Asn Gln
   660 665 670
   Ser Glu Tyr Glu Asp Ser Ala Gly Glu Cys Cys Ser Cys Pro Lys Thr
   675 680 685
   Asp Ser Gln Ile Leu Lys Glu Leu Glu Glu Ser Ser Phe Arg Lys Thr
   690 695 700
   Phe Glu Asp Tyr Leu His Asn Val Val Phe Val Pro Arg Lys Thr Ser
   705 710 715 720
   Ser Gly Thr Gly Ala Glu Asp Pro Arg Pro Ser Arg Lys Arg Arg Ser
   725 730 735
   Leu Gly Asp Val Gly Asn Val Thr Val Ala Val Pro Thr Val Ala Ala
   740 745 750
   Phe Pro Asn Thr Ser Ser Thr Ser Val Pro Thr Ser Pro Glu Glu His
   755 760 765
   Arg Pro Phe Glu Lys Val Val Asn Lys Glu Ser Leu Val Ile Ser Gly
   770 775 780
   Leu Arg His Phe Thr Gly Tyr Arg Ile Glu Leu Gln Ala Cys Asn Gln
   785 790 795 800
   Asp Thr Pro Glu Glu Arg Cys Ser Val Ala Ala Tyr Val Ser Ala Arg
   805 810 815
   Thr Met Pro Glu Ala Lys Ala Asp Asp Ile Val Gly Pro Val Thr His
   820 825 830
   Glu Ile Phe Glu Asn Asn Val Val His Leu Met Trp Gln Glu Pro Lys
   835 840 845
   Glu Pro Asn Gly Leu Ile Val Leu Tyr Glu Val Ser Tyr Arg Arg Tyr
   850 855 860
   Gly Asp Glu Glu Leu His Leu Cys Asp Thr Arg Lys His Phe Ala Leu
   865 870 875 880
   Glu Arg Gly Cys Arg Leu Arg Gly Leu Ser Pro Gly Asn Tyr Ser Val
   885 890 895
   Arg Ile Arg Ala Thr Ser Leu Ala Gly Asn Gly Ser Trp Thr Glu Pro
   900 905 910
   Thr Tyr Phe Tyr Val Thr Asp Tyr Leu Asp Val Pro Ser Asn Ile Ala
   915 920 925
   Lys
<210> 3
   <211> 1372
   <212> PRT
   <213> Mus musculus
<400> 3
   Met Gly Phe Gly Arg Gly Cys Glu Thr Thr Ala Val Pro Leu Leu Val
   1               5                   10                  15
   Ala Val Ala Ala Leu Leu Val Gly Thr Ala Gly His Leu Tyr Pro Gly
               20                  25                  30
   Glu Val Cys Pro Gly Met Asp Ile Arg Asn Asn Leu Thr Arg Leu His
           35                  40                  45
   Glu Leu Glu Asn Cys Ser Val Ile Glu Gly His Leu Gln Ile Leu Leu
       50                  55                  60
   Met Phe Lys Thr Arg Pro Glu Asp Phe Arg Asp Leu Ser Phe Pro Lys
   65                  70                  75                  80
   Leu Ile Met Ile Thr Asp Tyr Leu Leu Leu Phe Arg Val Tyr Gly Leu
                   85                  90                  95
   Glu Ser Leu Lys Asp Leu Phe Pro Asn Leu Thr Val Ile Arg Gly Ser
               100                 105                 110
   Arg Leu Phe Phe Asn Tyr Ala Leu Val Ile Phe Glu Met Val His Leu
           115                 120                 125
   Lys Glu Leu Gly Leu Tyr Asn Leu Met Asn Ile Thr Arg Gly Ser Val
       130                 135                 140
   Arg Ile Glu Lys Asn Asn Glu Leu Cys Tyr Leu Ala Thr Ile Asp Trp
   145                 150                 155                 160
   Ser Arg Ile Leu Asp Ser Val Glu Asp Asn Tyr Ile Val Leu Asn Lys
                   165                 170                 175
   Asp Asp Asn Glu Glu Cys Gly Asp Val Cys Pro Gly Thr Ala Lys Gly
               180                 185                 190
   Lys Thr Asn Cys Pro Ala Thr Val Ile Asn Gly Gln Phe Val Glu Arg
           195                 200                 205
   Cys Trp Thr His Ser His Cys Gln Lys Val Cys Pro Thr Ile Cys Lys
       210                 215                 220
   Ser His Gly Cys Thr Ala Glu Gly Leu Cys Cys His Lys Glu Cys Leu
   225                 230                 235                 240
   Gly Asn Cys Ser Glu Pro Asp Asp Pro Thr Lys Cys Val Ala Cys Arg
                   245                 250                 255
   Asn Phe Tyr Leu Asp Gly Gln Cys Val Glu Thr Cys Pro Pro Pro Tyr
               260                 265                 270
   Tyr His Phe Gln Asp Trp Arg Cys Val Asn Phe Ser Phe Cys Gln Asp
           275                 280                 285
   Leu His Phe Lys Cys Arg Asn Ser Arg Lys Pro Gly Cys His Gln Tyr
       290                 295                 300
   Val Ile His Asn Asn Lys Cys Ile Pro Glu Cys Pro Ser Gly Tyr Thr
   305                 310                 315                 320
   Met Asn Ser Ser Asn Leu Met Cys Thr Pro Cys Leu Gly Pro Cys Pro
                   325                 330                 335
   Lys Val Cys Gln Ile Leu Glu Gly Glu Lys Thr Ile Asp Ser Val Thr
               340                 345                 350
   Ser Ala Gln Glu Leu Arg Gly Cys Thr Val Ile Asn Gly Ser Leu Ile
           355                 360                 365
   Ile Asn Ile Arg Gly Gly Asn Asn Leu Ala Ala Glu Leu Glu Ala Asn
       370                 375                 380
   Leu Gly Leu Ile Glu Glu Ile Ser Gly Phe Leu Lys Ile Arg Arg Ser
   385                 390                 395                 400
   Tyr Ala Leu Val Ser Leu Ser Phe Phe Arg Lys Leu His Leu Ile Arg
                   405                 410                 415
   Gly Glu Thr Leu Glu Ile Gly Asn Tyr Ser Phe Tyr Ala Leu Asp Asn
               420                 425                 430
   Gln Asn Leu Arg Gln Leu Trp Asp Trp Ser Lys His Asn Leu Thr Ile
           435                 440                 445
   Thr Gln Gly Lys Leu Phe Phe His Tyr Asn Pro Lys Leu Cys Leu Ser
       450                 455                 460
   Glu Ile His Lys Met Glu Glu Val Ser Gly Thr Lys Gly Arg Gln Glu
   465                 470                 475                 480
   Arg Asn Asp Ile Ala Leu Lys Thr Asn Gly Asp Gln Ala Ser Cys Glu
                   485                 490                 495
   Asn Glu Leu Leu Lys Phe Ser Phe Ile Arg Thr Ser Phe Asp Lys Ile
               500                 505                 510
   Leu Leu Arg Trp Glu Pro Tyr Trp Pro Pro Asp Phe Arg Asp Leu Leu
           515                 520                 525
   Gly Phe Met Leu Phe Tyr Lys Glu Ala Pro Tyr Gln Asn Val Thr Glu
       530                 535                 540
   Phe Asp Gly Gln Asp Ala Cys Gly Ser Asn Ser Trp Thr Val Val Asp
   545                 550                 555                 560
   Ile Asp Pro Pro Gln Arg Ser Asn Asp Pro Lys Ser Gln Thr Pro Ser
                   565                 570                 575
   His Pro Gly Trp Leu Met Arg Gly Leu Lys Pro Trp Thr Gln Tyr Ala
               580                 585                 590
   Ile Phe Val Lys Thr Leu Val Thr Phe Ser Asp Glu Arg Arg Thr Tyr
           595                 600                 605
   Gly Ala Lys Ser Asp Ile Ile Tyr Val Gln Thr Asp Ala Thr Asn Pro
       610                 615                 620
   Ser Val Pro Leu Asp Pro Ile Ser Val Ser Asn Ser Ser Ser Gln Ile
   625                 630                 635                 640
   Ile Leu Lys Trp Lys Pro Pro Ser Asp Pro Asn Gly Asn Ile Thr His
                   645                 650                 655
   Tyr Leu Val Tyr Trp Glu Arg Gln Ala Glu Asp Ser Glu Leu Phe Glu
               660                 665                 670
   Leu Asp Tyr Cys Leu Lys Gly Leu Lys Leu Pro Ser Arg Thr Trp Ser
           675                 680                 685
   Pro Pro Phe Glu Ser Asp Asp Ser Gln Lys His Asn Gln Ser Glu Tyr
       690                 695                 700
   Asp Asp Ser Ala Ser Glu Cys Cys Ser Cys Pro Lys Thr Asp Ser Gln
   705                 710                 715                 720
   Ile Leu Lys Glu Leu Glu Glu Ser Ser Phe Arg Lys Thr Phe Glu Asp
                   725                 730                 735
   Tyr Leu His Asn Val Val Phe Val Pro Arg Pro Ser Arg Lys Arg Arg
               740                 745                 750
   Ser Leu Glu Glu Val Gly Asn Val Thr Ala Thr Thr Leu Thr Leu Pro
           755                 760                 765
   Asp Phe Pro Asn Val Ser Ser Thr Ile Val Pro Thr Ser Gln Glu Glu
       770                 775                 780
   His Arg Pro Phe Glu Lys Val Val Asn Lys Glu Ser Leu Val Ile Ser
   785                 790                 795                 800
   Gly Leu Arg His Phe Thr Gly Tyr Arg Ile Glu Leu Gln Ala Cys Asn
                   805                 810                 815
   Gln Asp Ser Pro Asp Glu Arg Cys Ser Val Ala Ala Tyr Val Ser Ala
               820                 825                 830
   Arg Thr Met Pro Glu Ala Lys Ala Asp Asp Ile Val Gly Pro Val Thr
           835                 840                 845
   His Glu Ile Phe Glu Asn Asn Val Val His Leu Met Trp Gln Glu Pro
       850                 855                 860
   Lys Glu Pro Asn Gly Leu Ile Val Leu Tyr Glu Val Ser Tyr Arg Arg
   865                 870                 875                 880
   Tyr Gly Asp Glu Glu Leu His Leu Cys Val Ser Arg Lys His Phe Ala
                   885                 890                 895
   Leu Glu Arg Gly Cys Arg Leu Arg Gly Leu Ser Pro Gly Asn Tyr Ser
               900                 905                 910
   Val Arg Val Arg Ala Thr Ser Leu Ala Gly Asn Gly Ser Trp Thr Glu
           915                 920                 925
   Pro Thr Tyr Phe Tyr Val Thr Asp Tyr Leu Asp Val Pro Ser Asn Ile
       930                 935                 940
   Ala Lys Ile Ile Ile Gly Pro Leu Ile Phe Val Phe Leu Phe Ser Val
   945                 950                 955                 960
   Val Ile Gly Ser Ile Tyr Leu Phe Leu Arg Lys Arg Gln Pro Asp Gly
                   965                 970                 975
   Pro Met Gly Pro Leu Tyr Ala Ser Ser Asn Pro Glu Tyr Leu Ser Ala
               980                 985                 990
   Ser Asp Val Phe Pro Ser Ser Val  Tyr Val Pro Asp Glu  Trp Glu Val
           995                 1000                1005
   Pro Arg  Glu Lys Ile Thr Leu  Leu Arg Glu Leu Gly  Gln Gly Ser
       1010                1015                1020
   Phe Gly  Met Val Tyr Glu Gly  Asn Ala Lys Asp Ile  Ile Lys Gly
       1025                1030                1035
   Glu Ala  Glu Thr Arg Val Ala  Val Lys Thr Val Asn  Glu Ser Ala
   1040 1045 1050
   Ser Leu Arg Glu Arg Ile Glu Phe Leu Asn Glu Ala Ser Val Met
   1055 1060 1065
   Lys Gly Phe Thr Cys His His Val Val Arg Leu Leu Gly Val Val
   1070 1075 1080
   Ser Lys Gly Gln Pro Thr Leu Val Val Met Glu Leu Met Ala His
   1085 1090 1095
   Gly Asp Leu Lys Ser His Leu Arg Ser Leu Arg Pro Asp Ala Glu
   1100 1105 1110
   Asn Asn Pro Gly Arg Pro Pro Pro Thr Leu Gln Glu Met Ile Gln
   1115 1120 1125
   Met Thr Ala Glu Ile Ala Asp Gly Met Ala Tyr Leu Asn Ala Lys
   1130 1135 1140
   Lys Phe Val His Arg Asp Leu Ala Ala Arg Asn Cys Met Val Ala
   1145 1150 1155
   His Asp Phe Thr Val Lys Ile Gly Asp Phe Gly Met Thr Arg Asp
   1160 1165 1170
   Ile Tyr Glu Thr Asp Tyr Tyr Arg Lys Gly Gly Lys Gly Leu Leu
   1175 1180 1185
   Pro Val Arg Trp Met Ser Pro Glu Ser Leu Lys Asp Gly Val Phe
   1190 1195 1200
   Thr Ala Ser Ser Asp Met Trp Ser Phe Gly Val Val Leu Trp Glu
   1205 1210 1215
   Ile Thr Ser Leu Ala Glu Gln Pro Tyr Gln Gly Leu Ser Asn Glu
   1220 1225 1230
   Gln Val Leu Lys Phe Val Met Asp Gly Gly Tyr Leu Asp Pro Pro
   1235 1240 1245
   Asp Asn Cys Pro Glu Arg Leu Thr Asp Leu Met Arg Met Cys Trp
   1250 1255 1260
   Gln Phe Asn Pro Lys Met Arg Pro Thr Phe Leu Glu Ile Val Asn
   1265 1270 1275
   Leu Leu Lys Asp Asp Leu His Pro Ser Phe Pro Glu Val Ser Phe
   1280 1285 1290
   Phe Tyr Ser Glu Glu Asn Lys Ala Pro Glu Ser Glu Glu Leu Glu
   1295 1300 1305
   Met Glu Phe Glu Asp Met Glu Asn Val Pro Leu Asp Arg Ser Ser
   1310 1315 1320
   His Cys Gln Arg Glu Glu Ala Gly Gly Arg Glu Gly Gly Ser Ser
   1325 1330 1335
   Leu Ser Ile Lys Arg Thr Tyr Asp Glu His Ile Pro Tyr Thr His
   1340 1345 1350
   Met Asn Gly Gly Lys Lys Asn Gly Arg Val Leu Thr Leu Pro Arg
   1355 1360 1365
   Ser Asn Pro Ser
   1370
<210> 4
   <211> 443
   <212> PRT
   <213> Macaca mulatta
<400> 4
   Met Met Ser Phe Glu Leu Asp Asn Leu Ala Ala Glu Leu Glu Ala Asn
   1 5 10 15
   Leu Gly Leu Ile Glu Glu Ile Ser Gly Tyr Leu Lys Ile Arg Arg Ser
   20 25 30
   Tyr Ala Leu Val Ser Leu Ser Phe Phe Arg Lys Leu Arg Leu Ile Arg
   35 40 45
   Gly Glu Thr Leu Glu Ile Gly Asn Tyr Ser Phe Tyr Ala Leu Asp Asn
   50 55 60
   Gln Asn Leu Arg Gln Leu Trp Asp Trp Ser Lys His Asn Leu Thr Ile
   65 70 75 80
   Thr Gln Gly Lys Leu Phe Phe His Tyr Asn Pro Lys Leu Cys Leu Ser
   85 90 95
   Glu Ile His Lys Met Glu Glu Val Ser Gly Thr Lys Gly Arg Gln Glu
   100 105 110
   Arg Asn Asp Ile Ala Leu Lys Thr Asn Gly Asp Gln Ala Ser Cys Glu
   115 120 125
   Asn Glu Leu Leu Lys Phe Ser Tyr Ile Arg Thr Ser Phe Asp Lys Ile
   130 135 140
   Leu Leu Arg Trp Glu Pro Tyr Trp Pro Pro Asp Phe Arg Asp Leu Leu
   145 150 155 160
   Gly Phe Met Leu Phe Tyr Lys Glu Ala Pro Tyr Gln Asn Val Thr Glu
   165 170 175
   Phe Asp Gly Gln Asp Ala Cys Gly Ser Asn Ser Trp Thr Val Val Asp
   180 185 190
   Ile Asp Pro Pro Leu Arg Ser Asn Asp Pro Lys Ser Gln Asn His Pro
   195 200 205
   Gly Trp Leu Met Arg Gly Leu Lys Pro Trp Thr Gln Tyr Ala Ile Phe
   210 215 220
   Val Lys Thr Leu Val Thr Phe Ser Asp Glu Arg Arg Thr Tyr Gly Ala
   225 230 235 240
   Lys Ser Asp Ile Ile Tyr Val Gln Thr Asp Ala Thr Asn Pro Ser Val
   245 250 255
   Pro Leu Asp Pro Ile Ser Val Ser Asn Ser Ser Ser Gln Ile Ile Leu
   260 265 270
   Lys Trp Lys Pro Pro Ser Asp Pro Asn Gly Asn Ile Thr His Tyr Leu
   275 280 285
   Val Phe Trp Glu Arg Gln Ala Glu Asp Ser Glu Leu Phe Glu Leu Asp
   290 295 300
   Tyr Cys Leu Lys Gly Leu Lys Leu Pro Ser Arg Thr Trp Ser Pro Pro
   305 310 315 320
   Phe Glu Ser Glu Asp Ser Gln Lys His Asn Gln Ser Glu Tyr Glu Asp
   325 330 335
   Ser Ala Gly Glu Cys Cys Ser Cys Pro Lys Thr Asp Ser Gln Ile Leu
   340 345 350
   Lys Glu Leu Glu Glu Ser Ser Phe Arg Lys Thr Phe Glu Asp Tyr Leu
   355 360 365
   His Asn Val Val Phe Val Pro Arg Lys Thr Ser Ser Gly Thr Gly Ala
   370 375 380
   Glu Asp Pro Arg Tyr Asp Ser Pro Val Arg Pro Leu Val Pro Ala Pro
   385 390 395 400
   Cys Arg Ala Gly Gly Val Pro Gly Arg Arg Leu Gly Glu Arg Arg Gly
   405 410 415
   Phe Cys Gly Phe Leu His Ala Ala Gly Cys Cys Ala Gly Asp Glu Met
   420 425 430
   Leu His Gln Phe Arg Asn Pro Met Pro Ser Leu
   435 440
<210> 5
   <211> 1279
   <212> PRT
   <213> Bos taurus
<400> 5
   Met Asp Ile Arg Asn Asn Leu Thr Arg Leu His Glu Leu Ala Asn Cys
   1               5                   10                  15
   Ser Val Ile Glu Gly His Leu Gln Ile Leu Leu Met Phe Lys Thr Arg
               20                  25                  30
   Pro Glu Asp Phe Arg Asp Leu Ser Phe Pro Lys Leu Ile Met Ile Thr
           35                  40                  45
   Asp Tyr Leu Leu Leu Phe Arg Val Tyr Gly Leu Glu Ser Leu Lys Asp
       50                  55                  60
   Leu Phe Pro Asn Leu Thr Val Ile Arg Gly Ser Arg Leu Phe Phe Asn
   65                  70                  75                  80
   Tyr Ala Leu Val Ile Phe Glu Met Val His Leu Lys Glu Leu Gly Leu
                   85                  90                  95
   Tyr Asn Leu Met Asn Ile Thr Arg Gly Ser Val Arg Ile Glu Lys Asn
               100                 105                 110
   Asn Glu Leu Cys Tyr Leu Ala Thr Ile Asp Trp Ser Arg Ile Leu Asp
           115                 120                 125
   Ser Val Glu Asp Asn Tyr Ile Val Leu Asn Lys Asp Asp Asn Glu Glu
       130                 135                 140
   Cys Gly Asp Ile Cys Pro Gly Thr Ala Lys Gly Lys Thr Asn Cys Pro
   145                 150                 155                 160
   Ala Thr Val Ile Asn Gly Gln Phe Val Glu Arg Cys Trp Thr His Ser
                   165                 170                 175
   His Cys Gln Lys Gly Pro Pro Ser Ala Ile Pro Gly Ala Ala Cys His
               180                 185                 190
   Ala Val Thr Arg Ser Pro Pro Gly His Thr Pro Ser Ser Val Arg Gly
           195                 200                 205
   Pro Ser His Thr Ala Ala Ala Arg Gly Gly Pro His Thr Arg Phe Leu
       210                 215                 220
   Leu Phe Phe Asn Phe Phe Gln Thr Pro Ile Leu Cys Gly Pro Ala Leu
   225                 230                 235                 240
   Gln Gly Leu Asn Pro Arg Lys Gly Pro Pro Pro Gly Ala Pro Gly Ala
                   245                 250                 255
   Asp Arg Pro Ala Ala Val Thr Ala Arg Ala Pro Val Gly Arg Ala Glu
               260                 265                 270
   Pro Arg Ala Pro Glu Gly Arg Gly Gln Ser Pro Ser Ser Thr Pro Ala
           275                 280                 285
   His Trp Leu Ser Ala Arg Ala Ala Leu Arg Leu Pro Pro Pro Pro Gly
       290                 295                 300
   Pro Asp Ser Thr Glu Arg Ser Ala Pro Arg Ala Leu Cys Phe Ser Ala
   305                 310                 315                 320
   Ala Ala Gly Leu Arg Gly Ala Gly Leu Leu Pro Pro Asn Tyr Ser Phe
                   325                 330                 335
   Tyr Ala Leu Asp Asn Gln Asn Leu Arg Gln Leu Trp Asp Trp Ser Lys
               340                 345                 350
   His Asn Leu Thr Ile Thr Gln Gly Lys Leu Phe Phe His Tyr Asn Pro
           355                 360                 365
   Lys Leu Cys Leu Ser Glu Ile His Lys Met Glu Glu Val Ser Gly Thr
       370                 375                 380
   Lys Gly Arg Gln Glu Arg Asn Asp Ile Ala Leu Lys Thr Asn Gly Asp
   385                 390                 395                 400
   Gln Ala Ser Cys Glu Asn Glu Leu Leu Lys Phe Ser Tyr Ile Arg Thr
                   405                 410                 415
   Ser Tyr Asp Lys Ile Leu Leu Lys Trp Glu Pro Tyr Trp Pro Pro Asp
               420                 425                 430
   Phe Arg Asp Leu Leu Gly Phe Met Leu Phe Tyr Lys Glu Ala Pro Tyr
           435                 440                 445
   Gln Asn Val Thr Glu Phe Asp Gly Gln Asp Ala Cys Gly Ser Asn Ser
       450                 455                 460
   Trp Thr Val Val Asp Ile Asp Pro Pro Thr Arg Ser Asn Asp Pro Lys
   465                 470                 475                 480
   Ser Gln Asn His Pro Gly Trp Leu Met Arg Gly Leu Lys Pro Trp Thr
                   485                 490                 495
   Gln Tyr Ala Ile Phe Val Lys Thr Leu Val Thr Phe Ser Asp Glu Arg
   500 505 510
   Arg Thr Tyr Gly Ala Lys Ser Asp Ile Ile Tyr Val Gln Thr Asp Ala
   515 520 525
   Thr Asn Pro Ser Val Pro Leu Asp Pro Ile Ser Val Ser Asn Ser Ser
   530 535 540
   Ser Gln Ile Ile Leu Lys Trp Lys Pro Pro Ser Asp Pro Asn Gly Asn
   545 550 555 560
   Ile Thr His Tyr Leu Val Phe Trp Glu Arg Gln Ala Glu Asp Ser Glu
   565 570 575
   Leu Tyr Glu Leu Asp Tyr Cys Leu Lys Gly Leu Lys Leu Pro Ser Arg
   580 585 590
   Thr Trp Ser Pro Pro Phe Glu Ser Glu Gly Ser Gln Lys His Asn Gln
   595 600 605
   Ser Glu Tyr Glu Glu Ser Ala Gly Glu Cys Cys Ser Cys Pro Lys Thr
   610 615 620
   Asp Ser Gln Ile Leu Lys Glu Leu Glu Glu Ser Ser Phe Arg Lys Thr
   625 630 635 640
   Phe Glu Asp Tyr Leu His Asn Val Val Phe Ile Pro Arg Pro Ser Arg
   645 650 655
   Lys Arg Arg Ala Leu Gly Asp Val Gly Asn Val Thr Ala Ala Val Pro
   660 665 670
   Thr Ala Leu Gly Leu Pro Asn Thr Ser Ser Thr Ser Thr Pro Met Ser
   675 680 685
   Ser Glu Glu His Arg Pro Phe Glu Lys Val Val Asn Lys Glu Ser Leu
   690 695 700
   Val Ile Ser Gly Leu Arg His Phe Thr Gly Tyr Arg Ile Glu Leu Gln
   705 710 715 720
   Ala Cys Asn Gln Asp Ser Pro Glu Glu Arg Cys Ser Val Ala Ala Tyr
   725 730 735
   Val Ser Ala Arg Thr Met Pro Glu Ala Lys Ala Asp Asp Ile Val Gly
   740 745 750
   Pro Val Thr His Glu Ile Phe Glu Asn Asn Val Val His Leu Met Trp
   755 760 765
   Gln Glu Pro Lys Glu Pro Asn Gly Leu Ile Val Leu Tyr Glu Val Ser
   770 775 780
   Tyr Arg Arg Tyr Gly Glu Glu Glu Leu His Leu Cys Val Ser Arg Arg
   785 790 795 800
   His Tyr Ala Leu Glu Arg Gly Cys Arg Leu Arg Gly Leu Leu Pro Gly
   805 810 815
   Asn Tyr Ser Val Arg Val Arg Ala Thr Ser Leu Ala Gly Asn Gly Ser
   820 825 830
   Trp Thr Glu Ala Thr Tyr Phe Tyr Val Thr Asp Tyr Leu Asp Val Pro
   835 840 845
   Ser Asn Ile Ala Lys Ile Ile Ile Gly Pro Leu Ile Phe Val Phe Leu
   850 855 860
   Phe Ser Val Val Ile Gly Ser Ile Cys Leu Phe Leu Arg Lys Arg Gln
   865 870 875 880
   Pro Asp Gly Pro Leu Gly Pro Leu Tyr Ala Ser Ser Asn Pro Glu Tyr
   885 890 895
   Leu Ser Ala Ser Asp Val Phe Pro Cys Ser Val Tyr Val Pro Asp Glu
   900 905 910
   Trp Glu Val Pro Arg Glu Lys Ile Thr Leu Leu Arg Glu Leu Gly Gln
   915 920 925
   Gly Ser Phe Gly Met Val Tyr Glu Gly Asn Ala Arg Asp Ile Val Lys
   930 935 940
   Gly Glu Ala Glu Thr Arg Val Ala Val Lys Thr Val Asn Glu Ser Ala
   945 950 955 960
   Ser Leu Arg Glu Arg Ile Glu Phe Leu Asn Glu Ala Ser Val Met Lys
   965 970 975
   Gly Phe Thr Cys His His Val Val Arg Leu Leu Gly Val Val Ser Lys
   980 985 990
   Gly Gln Pro Thr Leu Val Val Met Glu Leu Met Ala His Gly Asp Leu
   995 1000 1005
   Lys Ser Tyr Leu Arg Ser Leu Arg Pro Glu Ala Glu Asn Asn Pro
   1010 1015 1020
   Gly Arg Pro Pro Pro Thr Leu Gln Glu Met Ile Gln Met Ala Ala
   1025 1030 1035
   Glu Ile Ala Asp Gly Met Ala Tyr Leu Asn Ala Lys Lys Phe Val
   1040 1045 1050
   His Arg Asp Leu Ala Ala Arg Asn Cys Met Val Ala His Asp Phe
   1055 1060 1065
   Thr Val Lys Ile Gly Asp Phe Gly Met Thr Arg Asp Ile Tyr Glu
   1070 1075 1080
   Thr Asp Tyr Tyr Arg Lys Gly Gly Lys Gly Leu Leu Pro Val Arg
   1085 1090 1095
   Trp Met Ala Pro Glu Ser Leu Lys Asp Gly Val Phe Thr Thr Ser
   1100 1105 1110
   Ser Asp Met Trp Ser Phe Gly Val Val Leu Trp Glu Ile Thr Ser
   1115 1120 1125
   Leu Ala Glu Gln Pro Tyr Gln Gly Leu Ser Asn Glu Gln Val Leu
   1130 1135 1140
   Lys Phe Val Met Asp Gly Gly Tyr Leu Asp Gln Pro Asp Asn Cys
   1145 1150 1155
   Pro Glu Arg Val Thr Asp Leu Met Arg Met Cys Trp Gln Phe Asn
   1160 1165 1170
   Pro Lys Met Arg Pro Thr Phe Leu Glu Ile Val Asp Leu Leu Lys
   1175 1180 1185
   Asp Asp Leu His Pro Ser Phe Pro Glu Val Ser Phe Phe His Ser
   1190 1195 1200
   Glu Glu Asn Lys Ala Pro Glu Ser Glu Glu Leu Glu Met Glu Phe
   1205 1210 1215
   Glu Asp Met Glu Ser Val Pro Leu Asp Arg Ala Ser His Ala Gln
   1220 1225 1230
   Arg Glu Glu Ala Gly Gly Arg Asp Gly Gly Ser Ala Leu Gly Leu
   1235 1240 1245
   Lys Arg Asn Tyr Asp Glu His Ile Pro Tyr Thr His Met Asn Gly
   1250 1255 1260
   Gly Lys Lys Asn Gly Arg Ile Leu Thr Leu Pro Arg Ser Asn Pro
   1265 1270 1275
   Ser
<210> 6
   <211> 906
   <212> PRT
   <213> Homo sapiens
<400> 6
   Glu Ile Cys Gly Pro Gly Ile Asp Ile Arg Asn Asp Tyr Gln Gln Leu
   1               5                   10                  15
   Lys Arg Leu Glu Asn Cys Thr Val Ile Glu Gly Tyr Leu His Ile Leu
               20                  25                  30
   Leu Ile Ser Lys Ala Glu Asp Tyr Arg Ser Tyr Arg Phe Pro Lys Leu
           35                  40                  45
   Thr Val Ile Thr Glu Tyr Leu Leu Leu Phe Arg Val Ala Gly Leu Glu
       50                  55                  60
   Ser Leu Gly Asp Leu Phe Pro Asn Leu Thr Val Ile Arg Gly Trp Lys
   65                  70                  75                  80
   Leu Phe Tyr Asn Tyr Ala Leu Val Ile Phe Glu Met Thr Asn Leu Lys
                   85                  90                  95
   Asp Ile Gly Leu Tyr Asn Leu Arg Asn Ile Thr Arg Gly Ala Ile Arg
               100                 105                 110
   Ile Glu Lys Asn Ala Asp Leu Cys Tyr Leu Ser Thr Val Asp Trp Ser
           115                 120                 125
   Leu Ile Leu Asp Ala Val Ser Asn Asn Tyr Ile Val Gly Asn Lys Pro
       130                 135                 140
   Pro Lys Glu Cys Gly Asp Leu Cys Pro Gly Thr Met Glu Glu Lys Pro
   145                 150                 155                 160
   Met Cys Glu Lys Thr Thr Ile Asn Asn Glu Tyr Asn Tyr Arg Cys Trp
                   165                 170                 175
   Thr Thr Asn Arg Cys Gln Lys Met Cys Pro Ser Thr Cys Gly Lys Arg
               180                 185                 190
   Ala Cys Thr Glu Asn Asn Glu Cys Cys His Pro Glu Cys Leu Gly Ser
   195 200 205
   Cys Ser Ala Pro Asp Asn Asp Thr Ala Cys Val Ala Cys Arg His Tyr
   210 215 220
   Tyr Tyr Ala Gly Val Cys Val Pro Ala Cys Pro Pro Asn Thr Tyr Arg
   225 230 235 240
   Phe Glu Gly Trp Arg Cys Val Asp Arg Asp Phe Cys Ala Asn Ile Leu
   245 250 255
   Ser Ala Glu Ser Ser Asp Ser Glu Gly Phe Val Ile His Asp Gly Glu
   260 265 270
   Cys Met Gln Glu Cys Pro Ser Gly Phe Ile Arg Asn Gly Ser Gln Ser
   275 280 285
   Met Tyr Cys Ile Pro Cys Glu Gly Pro Cys Pro Lys Val Cys Glu Glu
   290 295 300
   Glu Lys Lys Thr Lys Thr Ile Asp Ser Val Thr Ser Ala Gln Met Leu
   305 310 315 320
   Gln Gly Cys Thr Ile Phe Lys Gly Asn Leu Leu Ile Asn Ile Arg Arg
   325 330 335
   Gly Asn Asn Ile Ala Ser Glu Leu Glu Asn Phe Met Gly Leu Ile Glu
   340 345 350
   Val Val Thr Gly Tyr Val Lys Ile Arg His Ser His Ala Leu Val Ser
   355 360 365
   Leu Ser Phe Leu Lys Asn Leu Arg Leu Ile Leu Gly Glu Glu Gln Leu
   370 375 380
   Glu Gly Asn Tyr Ser Phe Tyr Val Leu Asp Asn Gln Asn Leu Gln Gln
   385 390 395 400
   Leu Trp Asp Trp Asp His Arg Asn Leu Thr Ile Lys Ala Gly Lys Met
   405 410 415
   Tyr Phe Ala Phe Asn Pro Lys Leu Cys Val Ser Glu Ile Tyr Arg Met
   420 425 430
   Glu Glu Val Thr Gly Thr Lys Gly Arg Gln Ser Lys Gly Asp Ile Asn
   435 440 445
   Thr Arg Asn Asn Gly Glu Arg Ala Ser Cys Glu Ser Asp Val Leu His
   450 455 460
   Phe Thr Ser Thr Thr Thr Ser Lys Asn Arg Ile Ile Ile Thr Trp His
   465 470 475 480
   Arg Tyr Arg Pro Pro Asp Tyr Arg Asp Leu Ile Ser Phe Thr Val Tyr
   485 490 495
   Tyr Lys Glu Ala Pro Phe Lys Asn Val Thr Glu Tyr Asp Gly Gln Asp
   500 505 510
   Ala Cys Gly Ser Asn Ser Trp Asn Met Val Asp Val Asp Leu Pro Pro
   515 520 525
   Asn Lys Asp Val Glu Pro Gly Ile Leu Leu His Gly Leu Lys Pro Trp
   530 535 540
   Thr Gln Tyr Ala Val Tyr Val Lys Ala Val Thr Leu Thr Met Val Glu
   545 550 555 560
   Asn Asp His Ile Arg Gly Ala Lys Ser Glu Ile Leu Tyr Ile Arg Thr
   565 570 575
   Asn Ala Ser Val Pro Ser Ile Pro Leu Asp Val Leu Ser Ala Ser Asn
   580 585 590
   Ser Ser Ser Gln Leu Ile Val Lys Trp Asn Pro Pro Ser Leu Pro Asn
   595 600 605
   Gly Asn Leu Ser Tyr Tyr Ile Val Arg Trp Gln Arg Gln Pro Gln Asp
   610 615 620
   Gly Tyr Leu Tyr Arg His Asn Tyr Cys Ser Lys Asp Lys Ile Pro Ile
   625 630 635 640
   Arg Lys Tyr Ala Asp Gly Thr Ile Asp Ile Glu Glu Val Thr Glu Asn
   645 650 655
   Pro Lys Thr Glu Val Cys Gly Gly Glu Lys Gly Pro Cys Cys Ala Cys
   660 665 670
   Pro Lys Thr Glu Ala Glu Lys Gln Ala Glu Lys Glu Glu Ala Glu Tyr
   675 680 685
   Arg Lys Val Phe Glu Asn Phe Leu His Asn Ser Ile Phe Val Pro Arg
   690 695 700
   Pro Glu Arg Lys Arg Arg Asp Val Met Gln Val Ala Asn Thr Thr Met
   705 710 715 720
   Ser Ser Arg Ser Arg Asn Thr Thr Ala Ala Asp Thr Tyr Asn Ile Thr
   725 730 735
   Asp Pro Glu Glu Leu Glu Thr Glu Tyr Pro Phe Phe Glu Ser Arg Val
   740 745 750
   Asp Asn Lys Glu Arg Thr Val Ile Ser Asn Leu Arg Pro Phe Thr Leu
   755 760 765
   Tyr Arg Ile Asp Ile His Ser Cys Asn His Glu Ala Glu Lys Leu Gly
   770 775 780
   Cys Ser Ala Ser Asn Phe Val Phe Ala Arg Thr Met Pro Ala Glu Gly
   785 790 795 800
   Ala Asp Asp Ile Pro Gly Pro Val Thr Trp Glu Pro Arg Pro Glu Asn
   805 810 815
   Ser Ile Phe Leu Lys Trp Pro Glu Pro Glu Asn Pro Asn Gly Leu Ile
   820 825 830
   Leu Met Tyr Glu Ile Lys Tyr Gly Ser Gln Val Glu Asp Gln Arg Glu
   835 840 845
   Cys Val Ser Arg Gln Glu Tyr Arg Lys Tyr Gly Gly Ala Lys Leu Asn
   850 855 860
   Arg Leu Asn Pro Gly Asn Tyr Thr Ala Arg Ile Gln Ala Thr Ser Leu
   865 870 875 880
   Ser Gly Asn Gly Ser Trp Thr Asp Pro Val Phe Phe Tyr Val Gln Ala
   885 890 895
   Lys Thr Gly Tyr Glu Asn Phe Ile His Leu
   900 905
<210> 7
   <211> 1369
   <212> PRT
   <213> Mus musculus
<400> 7
   Met Lys Ser Gly Ser Gly Gly Gly Ser Pro Thr Ser Leu Trp Gly Leu
   1               5                   10                  15
   Val Phe Leu Ser Ala Ala Leu Ser Leu Trp Pro Thr Ser Gly Glu Ile
               20                  25                  30
   Cys Gly Pro Gly Ile Asp Ile Arg Asn Asp Tyr Gln Gln Leu Lys Arg
           35                  40                  45
   Leu Glu Asn Cys Thr Val Ile Glu Gly Phe Leu His Ile Leu Leu Ile
       50                  55                  60
   Ser Lys Ala Glu Asp Tyr Arg Ser Tyr Arg Phe Pro Lys Leu Thr Val
   65                  70                  75                  80
   Ile Thr Glu Tyr Leu Leu Leu Phe Arg Val Ala Gly Leu Glu Ser Leu
                   85                  90                  95
   Gly Asp Leu Phe Pro Asn Leu Thr Val Ile Arg Gly Trp Lys Leu Phe
               100                 105                 110
   Tyr Asn Tyr Ala Leu Val Ile Phe Glu Met Thr Asn Leu Lys Asp Ile
           115                 120                 125
   Gly Leu Tyr Asn Leu Arg Asn Ile Thr Arg Gly Ala Ile Arg Ile Glu
       130                 135                 140
   Lys Asn Ala Asp Leu Cys Tyr Leu Ser Thr Ile Asp Trp Ser Leu Ile
   145                 150                 155                 160
   Leu Asp Ala Val Ser Asn Asn Tyr Ile Val Gly Asn Lys Pro Pro Lys
                   165                 170                 175
   Glu Cys Gly Asp Leu Cys Pro Gly Thr Leu Glu Glu Lys Pro Met Cys
               180                 185                 190
   Glu Lys Thr Thr Ile Asn Asn Glu Tyr Asn Tyr Arg Cys Trp Thr Thr
           195                 200                 205
   Asn Arg Cys Gln Lys Met Cys Pro Ser Val Cys Gly Lys Arg Ala Cys
       210                 215                 220
   Thr Glu Asn Asn Glu Cys Cys His Pro Glu Cys Leu Gly Ser Cys His
   225                 230                 235                 240
   Thr Pro Asp Asp Asn Thr Thr Cys Val Ala Cys Arg His Tyr Tyr Tyr
                   245                 250                 255
   Lys Gly Val Cys Val Pro Ala Cys Pro Pro Gly Thr Tyr Arg Phe Glu
               260                 265                 270
   Gly Trp Arg Cys Val Asp Arg Asp Phe Cys Ala Asn Ile Pro Asn Ala
           275                 280                 285
   Glu Ser Ser Asp Ser Asp Gly Phe Val Ile His Asp Asp Glu Cys Met
       290                 295                 300
   Gln Glu Cys Pro Ser Gly Phe Ile Arg Asn Ser Thr Gln Ser Met Tyr
   305                 310                 315                 320
   Cys Ile Pro Cys Glu Gly Pro Cys Pro Lys Val Cys Gly Asp Glu Glu
                   325                 330                 335
   Lys Lys Thr Lys Thr Ile Asp Ser Val Thr Ser Ala Gln Met Leu Gln
               340                 345                 350
   Gly Cys Thr Ile Leu Lys Gly Asn Leu Leu Ile Asn Ile Arg Arg Gly
           355                 360                 365
   Asn Asn Ile Ala Ser Glu Leu Glu Asn Phe Met Gly Leu Ile Glu Val
       370                 375                 380
   Val Thr Gly Tyr Val Lys Ile Arg His Ser His Ala Leu Val Ser Leu
   385                 390                 395                 400
   Ser Phe Leu Lys Asn Leu Arg Leu Ile Leu Gly Glu Glu Gln Leu Glu
                   405                 410                 415
   Gly Asn Tyr Ser Phe Tyr Val Leu Asp Asn Gln Asn Leu Gln Gln Leu
               420                 425                 430
   Trp Asp Trp Asn His Arg Asn Leu Thr Val Arg Ser Gly Lys Met Tyr
           435                 440                 445
   Phe Ala Phe Asn Pro Lys Leu Cys Val Ser Glu Ile Tyr Arg Met Glu
       450                 455                 460
   Glu Val Thr Gly Thr Lys Gly Arg Gln Ser Lys Gly Asp Ile Asn Thr
   465                 470                 475                 480
   Arg Asn Asn Gly Glu Arg Ala Ser Cys Glu Ser Asp Val Leu Arg Phe
                   485                 490                 495
   Thr Ser Thr Thr Thr Trp Lys Asn Arg Ile Ile Ile Thr Trp His Arg
               500                 505                 510
   Tyr Arg Pro Pro Asp Tyr Arg Asp Leu Ile Ser Phe Thr Val Tyr Tyr
           515                 520                 525
   Lys Glu Ala Pro Phe Lys Asn Val Thr Glu Tyr Asp Gly Gln Asp Ala
       530                 535                 540
   Cys Gly Ser Asn Ser Trp Asn Met Val Asp Val Asp Leu Pro Pro Asn
   545                 550                 555                 560
   Lys Glu Gly Glu Pro Gly Ile Leu Leu His Gly Leu Lys Pro Trp Thr
                   565                 570                 575
   Gln Tyr Ala Val Tyr Val Lys Ala Val Thr Leu Thr Met Val Glu Asn
               580                 585                 590
   Asp His Ile Arg Gly Ala Lys Ser Glu Ile Leu Tyr Ile Arg Thr Asn
           595                 600                 605
   Ala Ser Val Pro Ser Ile Pro Leu Asp Val Leu Ser Ala Ser Asn Ser
       610                 615                 620
   Ser Ser Gln Leu Ile Val Lys Trp Asn Pro Pro Thr Leu Pro Asn Gly
   625 630 635 640
   Asn Leu Ser Tyr Tyr Ile Val Arg Trp Gln Arg Gln Pro Gln Asp Gly
   645 650 655
   Tyr Leu Tyr Arg His Asn Tyr Cys Ser Lys Asp Lys Ile Pro Ile Arg
   660 665 670
   Lys Tyr Ala Asp Gly Thr Ile Asp Val Glu Glu Val Thr Glu Asn Pro
   675 680 685
   Lys Thr Glu Val Cys Gly Gly Asp Lys Gly Pro Cys Cys Ala Cys Pro
   690 695 700
   Lys Thr Glu Ala Glu Lys Gln Ala Glu Lys Glu Glu Ala Glu Tyr Arg
   705 710 715 720
   Lys Val Phe Glu Asn Phe Leu His Asn Ser Ile Phe Val Pro Arg Pro
   725 730 735
   Glu Arg Arg Arg Arg Asp Val Met Gln Val Ala Asn Thr Thr Met Ser
   740 745 750
   Ser Arg Ser Arg Asn Thr Thr Val Ala Asp Thr Tyr Asn Ile Thr Asp
   755 760 765
   Pro Glu Glu Phe Glu Thr Glu Tyr Pro Phe Phe Glu Ser Arg Val Asp
   770 775 780
   Asn Lys Glu Arg Thr Val Ile Ser Asn Leu Arg Pro Phe Thr Leu Tyr
   785 790 795 800
   Arg Ile Asp Ile His Ser Cys Asn His Glu Ala Glu Lys Leu Gly Cys
   805 810 815
   Ser Ala Ser Asn Phe Val Phe Ala Arg Thr Met Pro Ala Glu Gly Ala
   820 825 830
   Asp Asp Ile Pro Gly Pro Val Thr Trp Glu Pro Arg Pro Glu Asn Ser
   835 840 845
   Ile Phe Leu Lys Trp Pro Glu Pro Glu Asn Pro Asn Gly Leu Ile Leu
   850 855 860
   Met Tyr Glu Ile Lys Tyr Gly Ser Gln Val Glu Asp Gln Arg Glu Cys
   865 870 875 880
   Val Ser Arg Gln Glu Tyr Arg Lys Tyr Gly Gly Ala Lys Leu Asn Arg
   885 890 895
   Leu Asn Pro Gly Asn Tyr Thr Ala Arg Ile Gln Ala Thr Ser Leu Ser
   900 905 910
   Gly Asn Gly Ser Trp Thr Asp Pro Val Phe Phe Tyr Val Pro Ala Lys
   915 920 925
   Thr Thr Tyr Glu Asn Phe Met His Leu Ile Ile Ala Leu Pro Val Ala
   930 935 940
   Ile Leu Leu Ile Val Gly Gly Leu Val Ile Met Leu Tyr Val Phe His
   945 950 955 960
   Arg Lys Arg Asn Asn Ser Arg Leu Gly Asn Gly Val Leu Tyr Ala Ser
   965 970 975
   Val Asn Pro Glu Tyr Phe Ser Ala Ala Asp Val Tyr Val Pro Asp Glu
   980 985 990
   Trp Glu Val Ala Arg Glu Lys Ile Thr Met Asn Arg Glu Leu Gly Gln
   995 1000 1005
   Gly Ser Phe Gly Met Val Tyr Glu Gly Val Ala Lys Gly Val Val
   1010 1015 1020
   Lys Asp Glu Pro Glu Thr Arg Val Ala Ile Lys Thr Val Asn Glu
   1025 1030 1035
   Ala Ala Ser Met Arg Glu Arg Ile Glu Phe Leu Asn Glu Ala Ser
   1040 1045 1050
   Val Met Lys Glu Phe Asn Cys His His Val Val Arg Leu Leu Gly
   1055 1060 1065
   Val Val Ser Gln Gly Gln Pro Thr Leu Val Ile Met Glu Leu Met
   1070 1075 1080
   Thr Arg Gly Asp Leu Lys Ser Tyr Leu Arg Ser Leu Arg Pro Glu
   1085 1090 1095
   Val Glu Gln Asn Asn Leu Val Leu Ile Pro Pro Ser Leu Ser Lys
   1100 1105 1110
   Met Ile Gln Met Ala Gly Glu Ile Ala Asp Gly Met Ala Tyr Leu
   1115 1120 1125
   Asn Ala Asn Lys Phe Val His Arg Asp Leu Ala Ala Arg Asn Cys
   1130 1135 1140
   Met Val Ala Glu Asp Phe Thr Val Lys Ile Gly Asp Phe Gly Met
   1145 1150 1155
   Thr Arg Asp Ile Tyr Glu Thr Asp Tyr Tyr Arg Lys Gly Gly Lys
   1160 1165 1170
   Gly Leu Leu Pro Val Arg Trp Met Ser Pro Glu Ser Leu Lys Asp
   1175 1180 1185
   Gly Val Phe Thr Thr His Ser Asp Val Trp Ser Phe Gly Val Val
   1190 1195 1200
   Leu Trp Glu Ile Ala Thr Leu Ala Glu Gln Pro Tyr Gln Gly Leu
   1205 1210 1215
   Ser Asn Glu Gln Val Leu Arg Phe Val Met Glu Gly Gly Leu Leu
   1220 1225 1230
   Asp Lys Pro Asp Asn Cys Pro Asp Met Leu Phe Glu Leu Met Arg
   1235 1240 1245
   Met Cys Trp Gln Tyr Asn Pro Lys Met Arg Pro Ser Phe Leu Glu
   1250 1255 1260
   Ile Ile Gly Ser Ile Lys Asp Glu Met Glu Pro Ser Phe Gln Glu
   1265 1270 1275
   Val Ser Phe Tyr Tyr Ser Glu Glu Asn Lys Pro Pro Glu Pro Glu
   1280 1285 1290
   Glu Leu Glu Met Glu Pro Glu Asn Met Glu Ser Val Pro Leu Asp
   1295 1300 1305
   Pro Ser Ala Ser Ser Ala Ser Leu Pro Leu Pro Glu Arg His Ser
   1310 1315 1320
   Gly His Lys Ala Glu Asn Gly Pro Gly Pro Gly Val Leu Val Leu
   1325 1330 1335
   Arg Ala Ser Phe Asp Glu Arg Gln Pro Tyr Ala His Met Asn Gly
   1340 1345 1350
   Gly Arg Ala Asn Glu Arg Ala Leu Pro Leu Pro Gln Ser Ser Thr
   1355 1360 1365
   Cys
<210> 8
   <211> 1367
   <212> PRT
   <213> Macaca mulatta
<400> 8
   Met Lys Ser Gly Ser Gly Glu Gly Ser Pro Thr Ser Leu Trp Gly Leu
   1 5 10 15
   Leu Phe Leu Ser Ala Ala Leu Ser Leu Trp Pro Thr Ser Gly Glu Ile
   20 25 30
   Cys Gly Pro Gly Ile Asp Ile Arg Asn Asp Tyr Gln Gln Leu Lys Arg
   35 40 45
   Leu Glu Asn Cys Thr Val Ile Glu Gly Tyr Leu His Ile Leu Leu Ile
   50 55 60
   Ser Lys Ala Glu Asp Tyr Arg Ser Tyr Arg Phe Pro Lys Leu Thr Val
   65 70 75 80
   Ile Thr Glu Tyr Leu Leu Leu Phe Arg Val Ala Gly Leu Glu Ser Leu
   85 90 95
   Gly Asp Leu Phe Pro Asn Leu Thr Val Ile Arg Gly Trp Lys Leu Phe
   100 105 110
   Tyr Asn Tyr Ala Leu Val Ile Phe Glu Met Thr Asn Leu Lys Asp Ile
   115 120 125
   Gly Leu Tyr Asn Leu Arg Asn Ile Thr Arg Gly Ala Ile Arg Ile Glu
   130 135 140
   Lys Asn Ala Asp Leu Cys Tyr Leu Ser Thr Val Asp Trp Ser Leu Ile
   145 150 155 160
   Leu Asp Ala Val Ser Asn Asn Tyr Ile Val Gly Asn Lys Pro Pro Lys
   165 170 175
   Glu Cys Gly Asp Leu Cys Pro Gly Thr Met Glu Glu Lys Pro Met Cys
   180 185 190
   Glu Lys Thr Thr Ile Asn Asn Glu Tyr Asn Tyr Arg Cys Trp Thr Thr
   195 200 205
   Asn Arg Cys Gln Lys Met Cys Pro Ser Ala Cys Gly Lys Arg Ala Cys
   210 215 220
   Thr Glu Asn Asn Glu Cys Cys His Pro Glu Cys Leu Gly Ser Cys Ser
   225 230 235 240
   Ala Pro Asp Asn Asp Thr Ala Cys Val Ala Cys Arg His Tyr Tyr Tyr
   245 250 255
   Ala Gly Val Cys Val Pro Ala Cys Pro Pro Asn Thr Tyr Arg Phe Glu
   260 265 270
   Gly Trp Arg Cys Val Asp Arg Asp Phe Cys Ala Asn Ile Leu Ser Ala
   275 280 285
   Glu Ser Ser Asp Ser Glu Gly Phe Val Ile His Asp Gly Glu Cys Met
   290 295 300
   Gln Glu Cys Pro Ser Gly Phe Ile Arg Asn Gly Ser Gln Ser Met Tyr
   305 310 315 320
   Cys Ile Pro Cys Glu Gly Pro Cys Pro Lys Val Cys Glu Glu Glu Lys
   325 330 335
   Lys Thr Lys Thr Ile Asp Ser Val Thr Ser Ala Gln Met Leu Gln Gly
   340 345 350
   Cys Thr Ile Phe Lys Gly Asn Leu Leu Ile Asn Ile Arg Arg Gly Asn
   355 360 365
   Asn Ile Ala Ser Glu Leu Glu Asn Phe Met Gly Leu Ile Glu Val Val
   370 375 380
   Thr Gly Tyr Val Lys Ile Arg His Ser His Ala Leu Val Ser Leu Ser
   385 390 395 400
   Phe Leu Lys Asn Leu Arg Leu Ile Leu Gly Glu Glu Gln Leu Glu Gly
   405 410 415
   Asn Tyr Ser Phe Tyr Val Leu Asp Asn Gln Asn Leu Gln Gln Leu Trp
   420 425 430
   Asp Trp Asp His Arg Asn Leu Thr Ile Lys Ala Gly Lys Met Tyr Phe
   435 440 445
   Ala Phe Asn Pro Lys Leu Cys Val Ser Glu Ile Tyr Arg Met Glu Glu
   450 455 460
   Val Thr Gly Thr Lys Gly Arg Gln Ser Lys Gly Asp Ile Asn Thr Arg
   465 470 475 480
   Asn Asn Gly Glu Arg Ala Ser Cys Glu Ser Asp Val Leu His Phe Thr
   485 490 495
   Ser Thr Thr Thr Trp Lys Asn Arg Ile Ile Ile Thr Trp His Arg Tyr
   500 505 510
   Arg Pro Pro Asp Tyr Arg Asp Leu Ile Ser Phe Thr Val Tyr Tyr Lys
   515 520 525
   Glu Ala Pro Phe Lys Asn Val Thr Glu Tyr Asp Gly Gln Asp Ala Cys
   530 535 540
   Gly Ser Asn Ser Trp Asn Met Val Asp Val Asp Leu Pro Pro Asn Lys
   545 550 555 560
   Asp Val Glu Pro Gly Ile Leu Leu His Gly Leu Lys Pro Trp Thr Gln
   565 570 575
   Tyr Ala Val Tyr Val Lys Ala Val Thr Leu Thr Met Val Glu Asn Asp
   580 585 590
   His Ile Arg Gly Ala Lys Ser Glu Ile Leu Tyr Ile Arg Thr Asn Ala
   595 600 605
   Ser Val Pro Ser Ile Pro Leu Asp Val Leu Ser Ala Ser Asn Ser Ser
   610 615 620
   Ser Gln Leu Ile Val Lys Trp Asn Pro Pro Ser Leu Pro Asn Gly Asn
   625 630 635 640
   Leu Ser Tyr Tyr Ile Val Arg Trp Gln Arg Gln Pro Gln Asp Gly Tyr
   645 650 655
   Leu Tyr Arg His Asn Tyr Cys Ser Lys Asp Lys Ile Pro Ile Arg Lys
   660 665 670
   Tyr Ala Asp Gly Thr Ile Asp Ile Glu Glu Val Thr Glu Asn Pro Lys
   675 680 685
   Thr Glu Val Cys Gly Gly Glu Lys Gly Pro Cys Cys Ala Cys Pro Lys
   690 695 700
   Thr Glu Ala Glu Lys Gln Ala Glu Lys Glu Glu Ala Glu Tyr Arg Lys
   705 710 715 720
   Val Phe Glu Asn Phe Leu His Asn Ser Ile Phe Val Pro Arg Pro Glu
   725 730 735
   Arg Lys Arg Arg Asp Val Met Gln Val Ala Asn Thr Thr Met Ser Ser
   740 745 750
   Arg Ser Arg Asn Thr Thr Val Ala Asp Thr Tyr Asn Ile Thr Asp Leu
   755 760 765
   Glu Glu Leu Glu Thr Glu Tyr Pro Phe Phe Glu Ser Arg Val Asp Asn
   770 775 780
   Lys Glu Arg Thr Val Ile Ser Asn Leu Arg Pro Phe Thr Leu Tyr Arg
   785 790 795 800
   Ile Asp Ile His Ser Cys Asn His Glu Ala Glu Lys Leu Gly Cys Ser
   805 810 815
   Ala Ser Asn Phe Val Phe Ala Arg Thr Met Pro Ala Glu Gly Ala Asp
   820 825 830
   Asp Ile Pro Gly Pro Val Thr Trp Glu Pro Arg Pro Glu Asn Ser Ile
   835 840 845
   Phe Leu Lys Trp Pro Glu Pro Glu Asn Pro Asn Gly Leu Ile Leu Met
   850 855 860
   Tyr Glu Ile Lys Tyr Gly Ser Gln Val Glu Asp Gln Arg Glu Cys Val
   865 870 875 880
   Ser Arg Gln Glu Tyr Arg Lys Tyr Gly Gly Ala Lys Leu Asn Arg Leu
   885 890 895
   Asn Pro Gly Asn Tyr Thr Ala Arg Ile Gln Ala Thr Ser Leu Ser Gly
   900 905 910
   Asn Gly Ser Trp Thr Asp Pro Val Phe Phe Tyr Val Gln Ala Lys Thr
   915 920 925
   Gly Tyr Glu Asn Phe Ile His Leu Ile Ile Ala Leu Pro Val Ala Val
   930 935 940
   Leu Leu Ile Val Gly Gly Leu Val Ile Met Leu Tyr Val Phe His Arg
   945 950 955 960
   Lys Arg Asn Asn Ser Arg Leu Gly Asn Gly Val Leu Tyr Ala Ser Val
   965 970 975
   Asn Pro Glu Tyr Phe Ser Ala Ala Asp Val Tyr Val Pro Asp Glu Trp
   980 985 990
   Glu Val Ala Arg Glu Lys Ile Thr Met Ser Arg Glu Leu Gly Gln Gly
   995 1000 1005
   Ser Phe Gly Met Val Tyr Glu Gly Val Ala Lys Gly Val Val Lys
   1010 1015 1020
   Asp Glu Pro Glu Thr Arg Val Ala Ile Lys Thr Val Asn Glu Ala
   1025 1030 1035
   Ala Ser Met Arg Glu Arg Ile Glu Phe Leu Asn Glu Ala Ser Val
   1040 1045 1050
   Met Lys Glu Phe Asn Cys His His Val Val Arg Leu Leu Gly Val
   1055 1060 1065
   Val Ser Gln Gly Gln Pro Thr Leu Val Ile Met Glu Leu Met Thr
   1070 1075 1080
   Arg Gly Asp Leu Lys Ser Tyr Leu Arg Ser Leu Arg Pro Glu Met
   1085 1090 1095
   Glu Asn Asn Pro Val Leu Ala Pro Pro Ser Leu Ser Lys Met Ile
   1100 1105 1110
   Gln Met Ala Gly Glu Ile Ala Asp Gly Met Ala Tyr Leu Asn Ala
   1115 1120 1125
   Asn Lys Phe Val His Arg Asp Leu Ala Ala Arg Asn Cys Met Val
   1130 1135 1140
   Ala Glu Asp Phe Thr Val Lys Ile Gly Asp Phe Gly Met Thr Arg
   1145 1150 1155
   Asp Ile Tyr Glu Thr Asp Tyr Tyr Arg Lys Gly Gly Lys Gly Leu
   1160 1165 1170
   Leu Pro Val Arg Trp Met Ser Pro Glu Ser Leu Lys Asp Gly Val
   1175 1180 1185
   Phe Thr Thr Tyr Ser Asp Val Trp Ser Phe Gly Val Val Leu Trp
   1190 1195 1200
   Glu Ile Ala Thr Leu Ala Glu Gln Pro Tyr Gln Gly Leu Ser Asn
   1205 1210 1215
   Glu Gln Val Leu Arg Phe Val Met Glu Gly Gly Leu Leu Asp Lys
   1220 1225 1230
   Pro Asp Asn Cys Pro Asp Met Leu Phe Glu Leu Met Arg Met Cys
   1235 1240 1245
   Trp Gln Tyr Asn Pro Lys Met Arg Pro Ser Phe Leu Glu Ile Ile
   1250 1255 1260
   Ser Ser Ile Lys Asp Glu Met Glu Pro Gly Phe Arg Glu Val Ser
   1265 1270 1275
   Phe Tyr Tyr Ser Glu Glu Asn Lys Leu Pro Glu Pro Glu Glu Leu
   1280 1285 1290
   Asp Leu Glu Pro Glu Asn Met Glu Ser Val Pro Leu Asp Pro Ser
   1295 1300 1305
   Ala Ser Ser Ser Ser Leu Pro Leu Pro Asp Arg His Ser Gly His
   1310 1315 1320
   Lys Ala Glu Asn Gly Pro Gly Pro Gly Val Leu Val Leu Arg Ala
   1325 1330 1335
   Ser Phe Asp Glu Arg Gln Pro Tyr Ala His Met Asn Gly Gly Arg
   1340 1345 1350
   Lys Asn Glu Arg Ala Leu Pro Leu Pro Gln Ser Ser Thr Cys
   1355 1360 1365
<210> 9
   <211> 1204
   <212> PRT
   <213> Bos taurus
<400> 9
   Met Gln Ser Thr Cys Ser Leu Pro Gln Arg Asn Ser Gln His Val Thr
   1               5                   10                  15
   Leu Val Ile Gln Ala Leu Gly Pro Arg Arg Val Ala Gly Gly Leu Gly
               20                  25                  30
   Val Pro Gly Gly Gly Pro Ser Ala Gln Arg Pro His Thr Leu Pro Val
           35                  40                  45
   Pro Thr Val Cys Pro Ser Ala Cys Gly Lys Arg Ala Cys Thr Glu Thr
       50                  55                  60
   His Glu Cys Cys His Pro Glu Cys Leu Gly Ser Cys Ser Ala Pro Asp
   65                  70                  75                  80
   Asn Ala Thr Ala Cys Val Ala Cys Arg His Tyr Tyr Tyr Ala Gly Val
                   85                  90                  95
   Cys Val Pro Ser Cys Pro Pro Asn Thr Tyr Arg Phe Glu Gly Trp Arg
               100                 105                 110
   Cys Val Asp Arg Asp Phe Cys Ala Asn Ile Pro Asn Ala Glu Ser Ser
           115                 120                 125
   Asp Ser Glu Gly Phe Val Ile His Asp Gly Glu Cys Met Gln Glu Cys
       130                 135                 140
   Pro Ser Gly Phe Ile Arg Asn Gly Ser Gln Ser Met Tyr Cys Ile Pro
   145                 150                 155                 160
   Cys Glu Gly Pro Cys Pro Lys Val Cys Glu Glu Glu Lys Lys Thr Lys
                   165                 170                 175
   Thr Ile Asp Ser Val Thr Ser Ala Gln Met Leu Gln Gly Cys Thr Ile
   180 185 190
   Phe Lys Gly Asn Leu Leu Ile Asn Ile Arg Arg Gly Asn Asn Ile Ala
   195 200 205
   Ser Glu Leu Glu Asn Phe Met Gly Leu Ile Glu Val Val Thr Gly Tyr
   210 215 220
   Val Lys Ile Arg His Ser His Ala Leu Val Ser Leu Ser Phe Leu Lys
   225 230 235 240
   Asn Leu Arg Gln Ile Leu Gly Glu Glu Gln Leu Glu Gly Asn Tyr Ser
   245 250 255
   Phe Tyr Val Leu Asp Asn Gln Asn Leu Gln Gln Leu Trp Asp Trp Asp
   260 265 270
   His Arg Asn Leu Thr Ile Lys Ala Gly Lys Met Tyr Phe Ala Phe Asn
   275 280 285
   Pro Lys Leu Cys Val Ser Glu Ile Tyr Arg Met Glu Glu Val Thr Gly
   290 295 300
   Thr Lys Gly Arg Gln Ser Lys Gly Asp Ile Asn Thr Arg Asn Asn Gly
   305 310 315 320
   Glu Arg Ala Ser Cys Glu Ser Asp Val Leu His Phe Thr Ser Thr Thr
   325 330 335
   Thr Ser Lys Asn Arg Ile Ile Ile Thr Trp His Arg Tyr Arg Pro Pro
   340 345 350
   Asp Tyr Arg Asp Leu Ile Ser Phe Thr Val Tyr Tyr Lys Glu Ala Pro
   355 360 365
   Phe Lys Asn Val Thr Glu Tyr Asp Gly Gln Asp Ala Cys Gly Ser Asn
   370 375 380
   Ser Trp Asn Met Val Asp Val Asp Leu Pro Pro Asn Lys Asp Val Glu
   385 390 395 400
   Pro Gly Ile Leu Leu His Gly Leu Lys Pro Trp Thr Gln Tyr Ala Val
   405 410 415
   Tyr Val Lys Ala Val Thr Leu Thr Met Val Glu Asn Asp His Ile Arg
   420 425 430
   Gly Ala Lys Ser Glu Ile Leu Tyr Ile Arg Thr Asn Ala Ser Val Pro
   435 440 445
   Ser Ile Pro Leu Asp Val Leu Ser Ala Ser Asn Ser Ser Ser Gln Leu
   450 455 460
   Ile Val Lys Trp Asn Pro Pro Ser Leu Pro Asn Gly Asn Leu Ser Tyr
   465 470 475 480
   Tyr Ile Val Arg Trp Gln Arg Gln Pro Gln Asp Ser Tyr Leu Tyr Arg
   485 490 495
   His Asn Tyr Cys Ser Lys Asp Lys Ile Pro Ile Arg Lys Tyr Ala Asp
   500 505 510
   Gly Thr Ile Asp Val Glu Glu Val Thr Glu Asn Pro Lys Thr Glu Val
   515 520 525
   Cys Gly Gly Glu Lys Gly Pro Cys Cys Ala Cys Pro Lys Thr Glu Ala
   530 535 540
   Glu Lys Gln Ala Glu Lys Glu Glu Ala Glu Tyr Arg Lys Val Phe Glu
   545 550 555 560
   Asn Phe Leu His Asn Ala Ile Phe Val Pro Arg Pro Glu Arg Lys Arg
   565 570 575
   Arg Glu Val Met Gln Ile Ala Asn Thr Thr Met Ser Ser Arg Ser Arg
   580 585 590
   Asn Thr Thr Val Leu Asp Thr Tyr Asn Ile Thr Asp Pro Glu Glu Leu
   595 600 605
   Glu Thr Glu Tyr Pro Phe Phe Glu Ser Arg Val Asp Asn Lys Glu Arg
   610 615 620
   Thr Val Ile Ser Asn Leu Arg Pro Phe Thr Leu Tyr Arg Ile Asp Ile
   625 630 635 640
   His Ser Cys Asn His Glu Ala Glu Lys Leu Gly Cys Ser Ala Ser Asn
   645 650 655
   Phe Val Phe Ala Arg Thr Met Pro Ala Glu Gly Ala Asp Asp Ile Pro
   660 665 670
   Gly Pro Val Thr Trp Glu Pro Arg Pro Glu Asn Ser Ile Phe Leu Lys
   675 680 685
   Trp Pro Glu Pro Glu Asn Pro Asn Gly Leu Ile Leu Met Tyr Glu Ile
   690 695 700
   Lys Tyr Gly Ser Gln Val Glu Asp Gln Arg Glu Cys Val Ser Arg Gln
   705 710 715 720
   Glu Tyr Arg Lys Tyr Gly Gly Ala Lys Leu Asn Arg Leu Asn Pro Gly
   725 730 735
   Asn Tyr Thr Ala Arg Ile Gln Ala Thr Ser Leu Ser Gly Asn Gly Ser
   740 745 750
   Trp Thr Asp Pro Val Phe Phe Tyr Val Gln Ala Lys Thr Thr Tyr Glu
   755 760 765
   Asn Phe Ile His Leu Met Ile Ala Leu Pro Ile Ala Val Leu Leu Ile
   770 775 780
   Val Gly Gly Leu Val Ile Met Leu Tyr Val Phe His Arg Lys Arg Asn
   785 790 795 800
   Ser Ser Arg Leu Gly Asn Gly Val Leu Tyr Ala Ser Val Asn Pro Glu
   805 810 815
   Tyr Phe Ser Ala Ala Asp Val Tyr Val Pro Asp Glu Trp Glu Val Ala
   820 825 830
   Arg Glu Lys Ile Thr Met Ser Arg Glu Leu Gly Gln Gly Ser Phe Gly
   835 840 845
   Met Val Tyr Glu Gly Val Ala Lys Gly Val Val Lys Asp Glu Pro Glu
   850 855 860
   Thr Arg Val Ala Ile Lys Thr Val Asn Glu Ala Ala Ser Met Arg Glu
   865 870 875 880
   Arg Ile Glu Phe Leu Asn Glu Ala Ser Val Met Lys Glu Phe Asn Cys
   885 890 895
   His His Val Val Arg Leu Leu Gly Val Val Ser Gln Gly Gln Pro Thr
   900 905 910
   Leu Val Ile Met Glu Leu Met Thr Arg Gly Asp Leu Lys Ser Tyr Leu
   915 920 925
   Arg Ser Leu Arg Pro Glu Met Glu Asn Asn Pro Val Leu Ala Pro Pro
   930 935 940
   Ser Leu Ser Lys Met Ile Gln Met Ala Gly Glu Ile Ala Asp Gly Met
   945 950 955 960
   Ala Tyr Leu Asn Ala Asn Lys Phe Val His Arg Asp Leu Ala Ala Arg
   965 970 975
   Asn Cys Met Val Ala Glu Asp Phe Thr Val Lys Ile Gly Asp Phe Gly
   980 985 990
   Met Thr Arg Asp Ile Tyr Glu Thr Asp Tyr Tyr Arg Lys Gly Gly Lys
   995 1000 1005
   Gly Leu Leu Pro Val Arg Trp Met Ser Pro Glu Ser Leu Lys Asp
   1010 1015 1020
   Gly Val Phe Thr Thr His Ser Asp Val Trp Ser Phe Gly Val Val
   1025 1030 1035
   Leu Trp Glu Ile Ala Thr Leu Ala Glu Gln Pro Tyr Gln Gly Leu
   1040 1045 1050
   Ser Asn Glu Gln Val Leu Arg Phe Val Met Glu Gly Gly Leu Leu
   1055 1060 1065
   Asp Lys Pro Asp Asn Cys Pro Asp Met Leu Phe Glu Leu Met Arg
   1070 1075 1080
   Met Cys Trp Gln Tyr Asn Pro Lys Met Arg Pro Ser Phe Leu Glu
   1085 1090 1095
   Ile Ile Ser Ser Val Lys Asp Glu Met Glu Ala Gly Phe Arg Glu
   1100 1105 1110
   Val Ser Phe Tyr Tyr Ser Glu Glu Asn Lys Pro Pro Glu Pro Glu
   1115 1120 1125
   Glu Leu Asp Leu Glu Pro Glu Asn Met Glu Ser Val Pro Leu Asp
   1130 1135 1140
   Pro Ser Ala Ser Ser Ala Ser Leu Pro Leu Pro Asp Arg His Ser
   1145 1150 1155
   Gly His Lys Ala Glu Asn Gly Pro Gly Pro Gly Val Leu Val Leu
   1160 1165 1170
   Arg Ala Ser Phe Asp Glu Arg Gln Pro Tyr Ala His Met Asn Gly
   1175 1180 1185
   Gly Arg Lys Asn Glu Arg Ala Leu Pro Leu Pro Gln Ser Ser Thr
   1190 1195 1200
   Cys
<210> 10
   <211> 502
   <212> PRT
   <213> Homo sapiens
<400> 10
   His Leu Tyr Pro Gly Glu Val Cys Pro Gly Met Asp Ile Arg Asn Asn
   1 5 10 15
   Leu Thr Arg Leu His Glu Leu Glu Asn Cys Ser Val Ile Glu Gly His
   20 25 30
   Leu Gln Ile Leu Leu Met Phe Lys Thr Arg Pro Glu Asp Phe Arg Asp
   35 40 45
   Leu Ser Phe Pro Lys Leu Ile Met Ile Thr Asp Tyr Leu Leu Leu Phe
   50 55 60
   Arg Val Tyr Gly Leu Glu Ser Leu Lys Asp Leu Phe Pro Asn Leu Thr
   65 70 75 80
   Val Ile Arg Gly Ser Arg Leu Phe Phe Asn Tyr Ala Leu Val Ile Phe
   85 90 95
   Glu Met Val His Leu Lys Glu Leu Gly Leu Tyr Asn Leu Met Asn Ile
   100 105 110
   Thr Arg Gly Ser Val Arg Ile Glu Lys Asn Asn Glu Leu Cys Tyr Leu
   115 120 125
   Ala Thr Ile Asp Trp Ser Arg Ile Leu Asp Ser Val Glu Asp Asn His
   130 135 140
   Ile Val Leu Asn Lys Asp Asp Asn Glu Glu Cys Gly Asp Ile Cys Pro
   145 150 155 160
   Gly Thr Ala Lys Gly Lys Thr Asn Cys Pro Ala Thr Val Ile Asn Gly
   165 170 175
   Gln Phe Val Glu Arg Cys Trp Thr His Ser His Cys Gln Lys Val Cys
   180 185 190
   Pro Thr Ile Cys Lys Ser His Gly Cys Thr Ala Glu Gly Leu Cys Cys
   195 200 205
   His Ser Glu Cys Leu Gly Asn Cys Ser Gln Pro Asp Asp Pro Thr Lys
   210 215 220
   Cys Val Ala Cys Arg Asn Phe Tyr Leu Asp Gly Arg Cys Val Glu Thr
   225 230 235 240
   Cys Pro Pro Pro Tyr Tyr His Phe Gln Asp Trp Arg Cys Val Asn Phe
   245 250 255
   Ser Phe Cys Gln Asp Leu His His Lys Cys Lys Asn Ser Arg Arg Gln
   260 265 270
   Gly Cys His Gln Tyr Val Ile His Asn Asn Lys Cys Ile Pro Glu Cys
   275 280 285
   Pro Ser Gly Tyr Thr Met Asn Ser Ser Asn Leu Leu Cys Thr Pro Cys
   290 295 300
   Leu Gly Pro Cys Pro Lys Val Cys His Leu Leu Glu Gly Glu Lys Thr
   305 310 315 320
   Ile Asp Ser Val Thr Ser Ala Gln Glu Leu Arg Gly Cys Thr Val Ile
   325 330 335
   Asn Gly Ser Leu Ile Ile Asn Ile Arg Gly Gly Asn Asn Leu Ala Ala
   340 345 350
   Glu Leu Glu Ala Asn Leu Gly Leu Ile Glu Glu Ile Ser Gly Tyr Leu
   355 360 365
   Lys Ile Arg Arg Ser Tyr Ala Leu Val Ser Leu Ser Phe Phe Arg Lys
   370 375 380
   Leu Arg Leu Ile Arg Gly Glu Thr Leu Glu Ile Gly Asn Tyr Ser Phe
   385 390 395 400
   Tyr Ala Leu Asp Asn Gln Asn Leu Arg Gln Leu Trp Asp Trp Ser Lys
   405 410 415
   His Asn Leu Thr Thr Thr Gln Gly Lys Leu Phe Phe His Tyr Asn Pro
   420 425 430
   Lys Leu Cys Leu Ser Glu Ile His Lys Met Glu Glu Val Ser Gly Thr
   435 440 445
   Lys Gly Arg Gln Glu Arg Asn Asp Ile Ala Leu Lys Thr Asn Gly Asp
   450 455 460
   Lys Ala Ser Cys Glu Asn Glu Leu Leu Lys Phe Ser Tyr Ile Arg Thr
   465 470 475 480
   Ser Phe Asp Lys Ile Ser Asp Asp Asp Asp Lys Glu Gln Lys Leu Ile
   485 490 495
   Ser Glu Glu Asp Leu Asn
   500
<210> 11
   <211> 16
   <212> PRT
   <213> Homo sapiens
<400> 11
   Thr Phe Glu Asp Tyr Leu His Asn Val Val Phe Val Pro Arg Pro Ser
   1 5 10 15
<210> 12
   <211> 16
   <212> PRT
   <213> Homo sapiens
<400> 12
   Thr Phe Glu Asp Tyr Leu His Asn Val Val Ala Val Pro Arg Pro Ser
   1 5 10 15
<210> 13
   <211> 18
   <212> PRT
   <213> Homo sapiens
<400> 13
   Leu Lys Glu Leu Glu Glu Ser Ser Phe Arg Lys Thr Phe Glu Asp Tyr
   1               5                   10                  15
   Leu His
<210> 14
   <211> 16
   <212> PRT
   <213> Homo sapiens
<400> 14
   Val Phe Glu Asn Phe Leu His Asn Ser Ile Phe Val Pro Arg Pro Glu
   1               5                   10                  15
<210> 15
   <211> 17
   <212> PRT
   <213> Homo sapiens
<400> 15
   Ala Glu Lys Glu Glu Ala Glu Tyr Arg Lys Val Phe Glu Asn Phe Leu
   1               5                   10                  15
   His
<210> 16
   <211> 36
   <212> PRT
   <213> Homo sapiens
<400> 16
   Ser Leu Glu Glu Glu Trp Ala Gln Val Glu Cys Glu Val Tyr Gly Arg
   1               5                   10                  15
   Gly Cys Pro Ser Gly Ser Leu Asp Glu Ser Phe Tyr Asp Trp Phe Glu
               20                  25                  30
   Arg Gln Leu Gly
           35
<210> 17
   <211> 20
   <212> PRT
   <213> Homo sapiens
<400> 17
   Ser Leu Glu Glu Glu Trp Ala Gln Val Glu Cys Glu Val Tyr Gly Arg
   1 5 10 15
   Gly Cys Pro Ser
   20
<210> 18
   <211> 16
   <212> PRT
   <213> Homo sapiens
<400> 18
   Gly Ser Leu Asp Glu Ser Phe Tyr Asp Trp Phe Glu Arg Gln Leu Gly
   1 5 10 15
<210> 19
   <211> 5
   <212> PRT
   <213> Artificial Sequence
<220>
   <223> Conserved motif of insulin-mimetic peptides
<220>
   <221> MISC_FEATURE
   <222> (3)..(3)
   <223> Xaa = any amino acid
<400> 19
   Phe Tyr Xaa Trp Phe
   1 5